# EUROPEAN PATENT SPECIFICATION

(11) **EP 1 032 674 B1**
(45) Date of publication and mention of the grant of the patent: **24.01.2007**
(21) Application number: 98954662.7
(22) Date of filing: 20.11.1998
(51) Int. Cl.: C12N 15/31, C12N 15/62, C07K 14/295, C07K 16/12, C07K 19/00, A01K 67/027, A61K 39/118, G01N 33/53, C12Q 1/68

(54) **(CHLAMYDIA PNEUMONIAE) GENOMIC SEQUENCE AND POLYPEPTIDES, FRAGMENTS THEREOF AND USES THEREOF, IN PARTICULAR FOR THE DIAGNOSIS, PREVENTION AND TREATMENT OF INFECTION**
CHLAMYDIA PNEUMONIAE GENOMISCHE SEQUENZEN UND POLYPEPTIDEN, FRAGMENTEN UND ANWENDUNGEN DAVON FÜR NACHWEIS, PREVENTION UND HEILUNG
SEQUENCE GENOMIQUE ET POLYPEPTIDES DE (CHLAMYDIA PNEUMONIAE), LEURS FRAGMENTS ET LEURS UTILISATIONS, EN PARTICULIER POUR LE DIAGNOSTIC, LA PREVENTION OU LE TRAITEMENT D'UNE INFECTION

(30) Priority: 21.11.1997 FR 9714673; 04.11.1998 US 107078 P
(43) Date of publication of application: 06.09.2000
(73) Proprietor: Serono Genetics Institute S.A., 91030 Evry Cedex (FR)
(72) Inventor: GRIFFAIS, Rémy, F-92120 Montrouge (FR); HOISETH, Susan K., Fairport, NY 14450 (US); ZAGURSKY, Robert J., Victor, NY 14564 (US); METCALF, Benjamin J., Rochester, NY 14618 (US); PEEK, Joel A., Pittsford, NY 14534 (US); SANKARAN, Banumathi, Penfield, NY 14526 (US); FLETCHER, Leah Diane, Geneseo, NY 14454 (US)
(74) Representative: Warcoin, Jacques
(86) International application number: PCT/IB1998/001890
(87) International publication number: WO 1999/027105

(56) References cited:
- EP-A- 0 784 059
- PEREZ MELGOSA M ET AL: "Isolation and characterization of a gene encoding a Chlamydia pneumoniae 76-kilodalton protein containing a species-specific epitope." INFECT IMMUN, MAR 1994, 62 (3) P880-6, XP002076845 UNITED STATES
- TOMB J -F ET AL: "THE COMPLETE GENOME SEQUENCE OF THE GASTRIC PATHOGEN HELICOBACTER PYLORI" NATURE, vol. 388, no. 6642, 7 August 1997 (1997-08-07), pages 539-547, TABEL, XP002062106
- KORNAK JM ET AL: "Sequence analysis of the gene encoding the Chlamydia pneumoniae DnaK protein homolog." INFECT IMMUN, FEB 1991, 59 (2) P721-5, XP002076846 UNITED STATES
- WATSON MW ET AL: "The CrP operon of Chlamydia psittaci and Chlamydia pneumoniae." MICROBIOLOGY, OCT 1995, 141 ( PT 10) P2489-97, XP002076847 ENGLAND
- LOBAU S ET AL: "Molecular cloning, sequence analysis, and functional characterization of the lipopolysaccharide biosynthetic gene kdtA encoding 3-deoxy-alpha-D-manno-octulosonic acid transferase of Chlamydia pneumoniae strain TW-183." MOL MICROBIOL, NOV 1995, 18 (3) P391-9, XP002076848 ENGLAND
- PETERSON EM ET AL: "Characterization of the murine antibody response to peptides representing the variable domains of the major outer membrane protein of Chlamydia pneumoniae." INFECT IMMUN, AUG 1996, 64 (8) P3354-9, XP002076849 UNITED STATES
- "http://www.ncbi.nlm.nih.gov/cgi-bin/Entre z/framik?db=Genome&gi=140" KALMAN S. ET AL.,December 1998 (1998-12), XP002104860

## Description

The subject of the invention is the genomic sequence and the nucleotide sequences encoding polypeptides of *Chlamydia pneumoniae,* such as cellular envelope polypeptides, which are secreted or specific, or which are involved in metabolism, in the replication process or in virulence, polypeptides encoded by such sequences. The invention also relates to methods of detecting these nucleic acids and kits for diagnosing *Chlamydia pneumoniae* infection. The invention also relates to a screening assay using the said nucleotide sequences or the said polypeptides.

Comparative analysis of the sequence of the gene encoding the ribosomal 16S RNA has been widely used for the phylogenetic study of prokaryotes. This approach has made it possible to classify the Chlamydiae among the eubacteria, among which they represent a well-isolated group, with, nevertheless, a very weak link with the planctomyces. The Chlamydiae thus exhibit some unique characteristics within the eubacteria, in particular their development cycle and the structure of their membranes. They have a unique two-phase cell cycle: the elementary body, a small extracellular form, attaches to the host and is phagocytosed; in the phagosome, it is converted to the replicative intracellular form, the reticulate body. The Chlamydiae are obligate intracellular bacteria which multiply in eukaryotic cells at the expense of their energy reserves and nucleotide pools; they are responsible for a wide variety of diseases in mammals and birds. The Chlamydiae are the only members of the order Chlamydiales, of the family Chlamydiaceae and of the genus Chlamydia. Within the genus *Chlamydia,* four species are currently described: *Chlamydia trachomatis, Chlamydia psittaci, Chlamydia pneumoniae* and *Chlamydia pecorum.* These bacteria are grouped together and share biological and biochemical properties. Among them, only the first three infect humans, *Chlamydia pecorum* being a pathogen of ruminants.

The species *Chlamydia psittaci* infects many animals, in particular birds, and is transmissible to humans. It is responsible for atypical pneumonia, for hepatic and renal dysfunction, for endocarditis and for conjunctivitis.

The species *Chlamydia trachomatis* is the best characterized. Besides a murine strain, it is divided into two groups which are distinguishable by the nature of the diseases for which they are responsible: trachoma, genital attack and venereal lymphogranulomatosis. There are fifteen human serotypes of *Chlamydia trachomatis* (A, K) and LGV (L1, L2, L3). Strains A to C are mainly found in eye infections, whereas strains D to K and LGV are essentially responsible for genital entry infections. It should be mentioned that the LGV strains are responsible for systemic diseases. Historically, it was in 1906 that Halberstaeder and Von Provaseck discovered, in trachoma patients, the presence of inclusions in the cytoplasm of the cells derived from conjunctival scrapings. In 1940, Rake and Jones described these same inclusions in certain cells obtained by puncturing the ganglia from a patient suffering from venereal granulomatosis. Characterization of the *Chlamydia trachomatis* microorganism was only successfully carried out in 1957, after a series of isolations in cell cultures.

It was in 1983 that *Chlamydia pneumoniae* was recognized as a human pathogen (Grayston JT et al., 1986); since then, special attention has been paid to this bacterium and it is estimated (Gaydos CA et al., 1994) that 10% of pneumonias, and 5% of bronchitides and sinusites are attributable to *Chlamydia pneumoniae* (Aldous MB et al., 1992). More recently, the association of this bacterium with the pathogenesis of asthmatic disease and of cardiovascular impairments is increasingly of interest.

Serological studies have made it possible to observe that *Chlamydia pneumoniae* infection is common in children between 5 and 16 years of age. Before this age, it is rare to find antibodies; the increase in the number of individuals carrying antibodies is then correlated with age up to 20 years. Accordingly, 50% of adults are carriers of antibodies, it being possible for this prevalence to be as high as 75%. These figures are all the more striking since a first infection induces antibody levels of which the persistence over time is limited to 3 or at most 5 years, which suggests frequent reinfection during the entire lifespan. The annual seroconversion rate is about 8% between 8 and 12 years and about 6% between 12 and 16 years (Haidl et al., 1994). Before the age of 15 years, the seroprevalence of the disease is identical between both sexes. After this age, men are more frequently infected than women; this is true in all regions worldwide where such studies have been carried out.

These infections are geographically highly widespread, as shown by numerous studies carried out throughout the world (Kanamoto Y et al., 1991; Tong CY et al., 1993). Developed countries of the north such as Canada, Denmark and Norway have the lowest infection rates; conversely, the highest prevalence rates are found in the less developed countries of tropical regions where the infection may occur before the age of 5 years.

Humans are the only known reservoir for *Chlamydia pneumoniae* and it is probable that the infection is caused by direct transmission, respiratory secretions probably being responsible for this low-yield transmission (Aldous et al., 1992). The chain of transmission may also appear to be indirect (Kleemola M et al., 1988), suggesting that the infection is caused by an effective transmission, but also that asymptomatic carriers exist, which could explain the high prevalence of the disease. Other studies (Mordhorst CH et al., 1992) show that the efficiency of the transmission varies according to the individuals and list cases of infection affecting all or the majority of members of one family or of a group of families. The period of incubation is several weeks, significantly longer in this regard than that of many other respiratory pathogenic agents. Although under conditions of high relative humidity the infectivity of *Chlamydia pneumoniae* in the open air decreases rapidly, suggesting a direct mode of transmission under these conditions, it is probable that the transmission occurs in some cases indirectly since the microorganism can survive for up to 30 hours in a hostile environment (Falsey et al., 1993).

Clinical manifestations due to *Chlamydia pneumoniae* are essentially respiratory diseases. Pneumonia and bronchitis are the most frequent because they are clinically patent: since etiological diagnosis is evoked in this case, the infectious agent is identified. The asymptomatic diseases are probably numerous (Grayston JT et al., 1992; Grayston JT et al., 1986; Thom DH et al., 1990). The disease then progresses via bronchitis or pneumonia; fever is absent at the time of examination but is sometimes reported by the patient. The degree of seriousness of the disease is variable and in hospitalized patients, it is common to observe pleural effusion; a generalized infection may also be observed and, in severe cases, anatomicopathological examination shows *Chlamydia pneumoniae* diseases.

Other syndromes such as sinusitis (Hashiguchi K et al., 1992), purulent otitis media (Ogawa H et al., 1992), or pharyngitis (Huovinen P et al., 1989) have been described, as well as infections with respiratory impairments similar to asthma (Hahn DL et al., 1991). *Chlamydia pneumoniae* has also been associated with sarcoidosis, with erythema nodosum (Sundelof et al., 1993) and one case of Guillain-Barré syndrome has even been described (Haidl et al., 1992). The involvement of *Chlamydia pneumoniae* in Reiter's syndrome has also been evaluated (Braun J et al., 1994).

The association of *Chlamydia pneumoniae* with coronary diseases and with myocardial infarction was first suspected from the observation of the high antibody level in 71% of patients having a heart disease (Shor A et al., 1992; Kuo CC et al., 1993; Puolakkainen M et al., 1993; Thomas GN et al., 1997). Studies carried out in several countries have shown similar results in patients with atheromatous impairments (Shor A et al., 1992; Kuo CC et al., 1993; Puolakkainen M et al., 1993; Grayston JT et al., 1996; Casas-Ciria J et al., 1996; Thomas GN et al., 1997; Jackson LA et al., 1997) and in patients with carotid impairments. Anatomicopathological and microbiological studies have detected *Chlamydia pneumoniae* in the vessels. The electron microscope has made it possible to visualize the bacterium (Ladany S et al., 1989), which has in fact been demonstrated by other techniques such as PCR (Campbell LA et al., 1992; Kuo CC et al., 1993; Kuo CC et al., 1988). It also appears that the bacterium is more frequently found in old atheromatous lesions. Other studies carried out on young subjects from 15 to 35 years have given the opportunity to study the coronary arteries of people without atherosclerosis, this observation not being possible in older subjects (the onset of the atheromatous disease is early). In these young subjects, the PCR studies did not find *Chlamydia pneumoniae* in subjects free of atheromatous disease, but revealed the presence of *Chlamydia pneumoniae* in two of the eleven subjects who showed early lesions and in six of the seven subjects who developed atheroma plaques. These studies therefore show that the atheroma plaque is very strongly correlated with the presence of *Chlamydia pneumoniae,* but the role played by the bacterium in vascular pathology is not yet defined.

The data relating to controlled clinical studies analysing the effect of treatments in *Chlamydia pneumoniae* infections are limited in number. Unlike penicillin, ampicillin or the sulphamides, erythromycin, tetracycline or doxycycline show an antibiotic activity *in vitro* against *Chlamydia pneumoniae.* However, a treatment at high doses should be continued for several weeks in order to avoid a recurrence of the infection. Accordingly, the use of two new macrolides, clarithromycin and azithromycin, whose diffusion, bioavailability and half-life allow shorter and better tolerated cures, is nowadays preferred. In the absence of definitive proof based on the results of clinical studies, an effective, without recurrences, and well-tolerated treatment of *Chlamydia pneumoniae* infections therefore remains desirable.

An even more important need up until now relates to a specific and sensitive diagnosis, which can be carried out conveniently and rapidly, allowing early screening for the infection. Methods based on *Chlamydia pneumoniae* culture are slow and require a considerable know-how because of the difficulty involved in the collection, preservation and storage of the strain under appropriate conditions. Methods based on antigen detection (EIA, DFA) or on nucleic acid amplification (PCR) provide tests which are more suitable for laboratory practice. A reliable, sensitive and convenient test, which allows distinction between serogroups and a fortiori between *Chlamydia pneumoniae* species is therefore highly desirable.

This is all the more important since the symptoms of *Chlamydia pneumoniae* infection appear slowly, since all the pathologies associated with these infections have not yet been identified, and since, as has been mentioned above, an association is suspected between these infections and serious chronic infections, asthma or atherosclerosis.

No vaccine is yet available against *Chlamydia pneumoniae:* this is due to the labile nature of the antigens specific to the strain, which has so far prevented their specific identification.

Although the number of studies and of animal models developed is high, the antigens used have not induced sufficient protective immunity to lead to the development of human vaccines. In the case of *Chlamydia pneumoniae,* the role of the immune defense in the physiology and pathology of the disease should probably be understood in order to develop satisfactory vaccines.

More detailed information relating to the biology of these strains, their interactions with their hosts, the associated phenomena of infectivity and those of escaping the immune defenses of the host in particular, and finally their involvement in the development of the these associated pathologies, will allow a better understanding of these mechanisms. In the light of the preceding text which shows in particular the limitations of the means of controlling *Chlamydia pneumoniae* infection, it is therefore at present essential, on the one hand, to develop molecular tools, in particular from a better genetic knowledge of *Chlamydia pneumoniae,* but also to develop new preventive and therapeutic treatments, new diagnostic methods and new vaccine strategies which are specific, effective and tolerated. This is precisely the object of the present invention.

The subject of the present invention is the nucleotide sequence having the sequence SEQ ID No. 1 of the *Chlamydia pneumoniae* genome. However, the invention is not limited to SEQ ID No. 1, but encompasses genomes and nucleotides encoding polypeptides of strain variants, polymorphisims, allelic variants, and mutants.

Thus, the subject of the present invention encompasses nucleotide sequences characterized in that they are chosen from:
a) the nucleotide sequence of SEQ ID No. 1, a nucleotide sequence exhibiting at least 99.9% identity with the sequence SEQ ID No. 1, the nucleotide sequence of the genomic DNA contained within ATCC Deposit No. 1360-VR,
b) a nucleotide sequence exhibiting at least 80% identity to the sequence SEQ ID No. 1;
c) a polynucleotide sequence that hybridizes to the totality of the nucleotide sequence of a) under conditions of high stringency.

(i) By way of example and not limitation, procedures using conditions of high stringency are as follows: Prehybridization of filters containing DNA is carried out for 8 h to overnight at 65EC in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65EC, the preferred hybridization temperature, in prehybridization mixture containing 100 µg/ml denatured salmon sperm DNA and 5-20 X 10⁶ cpm of ³²P-labeled probe. Alternatively, the hybridization step can be performed at 65EC in the presence of SSC buffer, 1 x SSC corresponding to 0.15M NaCl and 0.05 M Na citrate. Subsequently, filter washes can be done at 37EC for I h in a solution containing 2X SSC, 0.01% PVP, 0.01% Ficoll, and 0.01% BSA, followed by a wash in 0.1X SSC at 50EC for 45 min. Alternatively, filter washes can be performed in a solution containing 2 x SSC and 0.1% SDS, or 0.5 x SSC and 0.1% SDS, or 0.1 x SSC and 0.1% SDS at 68EC for 15 minute intervals. Following the wash steps, the hybridized probes are detectable by autoradiography. Other conditions of high stringency which may be used are well known in the art and as cited in Sambrook et al., 1989, Molecular Cloning, A Laboratory Manual, Second Edition, Cold Spring Harbor Press, N.Y., pp. 9.47-9.57; and Ausubel et al., 1989, Current Protocols in Molecular Biology, Green Publishing Associates and Wiley Interscience, N.Y. are incorporated herein in their entirety.
(ii) By way of example and not limitation, procedures using conditions of intermediate stringency are as follows: Filters containing DNA are prehybridized, and then hybridized at a temperature of 60EC in the presence of a 5 x SSC buffer and labeled probe. Subsequently, filters washes are performed in a solution containing 2x SSC at 50EC and the hybridized probes are detectable by autoradiography. Other conditions of intermediate stringency which may be used are well known in the art and as cited in Sambrook et al., 1989, Molecular Cloning, A Laboratory Manual, Second Edition, Cold Spring Harbor Press, N.Y., pp. 9.47-957; and Ausubel et al., 1989, Current Protocols in Molecular Biology, Green Publishing Associates and Wiley Interscience, N.Y. are incorporated herein in their entirety.

Nucleotide sequence, polynucleotide or nucleic acid are understood to mean, according to the present invention, either a double-stranded DNA, a single-stranded DNA or products of transcription of the said DNAs.

It should be understood that the present invention does not relate to the genomic nucleotide sequences of *Chlamydia pneumoniae* taken in their natural environment, that is to say in the natural state. They are sequences which may have been isolated, purified or partially purified, by separation methods such as, for example, ion-exchange chromatography, molecular size exclusion chromatography or affinity chromatography, or alternatively fractionation techniques based on solubility in various solvents, or by genetic engineering methods such as amplification, cloning or subcloning, it being possible for the sequences of the invention to be carried by vectors.

The nucleotide sequence SEQ ID No. I was obtained by sequencing the *Chlamydia pneumoniae* genome by the method of directed sequencing after fluorescent automated sequencing of the inserts of clones and assembling of these sequences of nucleotide fragments (inserts) by means of softwares (cf. Examples). In spite of the high precision of the sequence SEQ ID No. 1, it is possible that it does not perfectly, 1009b represent the nucleotide sequence of the *Chlamydia pneumoniae* genome and that a few rare sequencing errors or uncertainties still remain in the sequence SEQ ID No. 1. In the present invention, the presence of an uncertainty for a nucleotide is designated by "N" in the sequence listing below. These few rare errors or uncertainties could be easily detected and corrected by persons skilled in the art using the entire chromosome according to the invention and standard amplification, cloning and sequencing methods, it being possible for the sequences obtained to be easily compared, in particular by means of a computer software and using computer-readable media for recording the sequences according to the invention as described, for example, below. After correcting these possible rare errors or uncertainties, the corrected nucleotide sequence obtained would still exhibit at least 99.9% identity with the sequence SEQ ID No. 1. Such rare sequencing uncertainties are not present within the DNA contained within ATCC Deposit No. 1360-VR and whatever rare sequence uncertainties that exist within SEQ ID No. 1 can routinely be corrected utilizing the DNA of the ATCC deposit.

Homologous nucleotide sequence for the purposes of the present invention is understood to mean a nucleotide sequence having a percentage identity with the bases of the nucleotide sequence SEQ ID No. 1 of at least 80%, preferably 90% and 95%, this percentage being purely statistical and it being possible for the differences between the two nucleotide sequences to be distributed randomly and over their entire length. The said homologous sequences exhibiting a percentage identity with the bases of the nucleotide sequence SEQ ID No. 1 of at least 80%, preferably 90% and 95%, may comprise, for example, the sequences corresponding to the genomic sequence or to the sequences of its representative fragments of a bacterium belonging to the Chlamydia family, including the species *Chlamydia trachomatis, Chlamydia psittaci* and *Chlamydia pecorum* mentioned above, as well as the sequences corresponding to the genomic sequence or to the sequences of its representative fragments of a bacterium belonging to the variants of the species *Chlamydia pneumoniae.* In the present invention, the terms family and genus are mutually interchangeable, the terms variant, serotype, strain and subspecies are also mutually interchangeable. These homologous sequences may thus correspond to variations linked to mutations within the same species or between species and may correspond in particular to truncations, substitutions, deletions and/or additions of at least one nucleotide. The said homologous sequences may also correspond to variations linked to the degeneracy of the genetic code or to a bias in the genetic code which is specific to the family, to the species or to the variant and which are likely to be present in *Chlamydia.*

Protein and/or nucleic acid sequence homologies may be evaluated using any of the variety of sequence comparison algorithms and programs known in the art. Such algorithms and programs include, but are by no means limited to, TBLASTN, BLASTP, FASTA, TFASTA, and CLUSTALW (Pearson and Lipman, 1988, *Proc. Natl. Acad. Sci. USA 85(8)*:2444-2448; Altschul *et al.,* 1990, *J. Mol. Biol. 215(3)*:403-410; Thompson *et al.,* 1994, *Nucleic Acids Res. 22(2)*:4673-4680; Higgins *et al.,* 1996, *Methods Enzymol. 266*:383-402; Altschul *et al.,* 1990, *J. Mol. Biol. 215(3)*:403-410; Altschul *et al.,* 1993, *Nature Genetics 3*:266-272).

In a particularly preferred embodiment, protein and nucleic acid sequence homologies are evaluated using the Basic Local Alignment Search Tool ("BLAST') which is well known in the art (see, *e.g.,* Karlin and Altschul, 1990, *Proc. Natl. Acad*. *Sci. USA 87*:2267-2268; Altschul *et al.,* 1990, *J. Mol. Biol. 215*:403-410; Altschul *et al.,* 1993, *Nature Genetics 3*:266-272; Altschul *et al.,* 1997, *Nuc. Acids Res. 25*:3389-3402). In particular, five specific BLAST programs are used to perform the following task:
(1)BLASTP and BLAST3 compare an amino acid query sequence against a protein sequence database;
(2)BLASTN compares a nucleotide query sequence against a nucleotide sequence database;
(3)BLASTX compares the six-frame conceptual translation products of a query nucleotide sequence (both strands) against a protein sequence database;
(4)TBLASTN compares a query protein sequence against a nucleotide sequence database translated in all six reading frames (both strands); and
(5)TBLASTX compares the six-frame translations of a nucleotide query sequence against the six-frame translations of a nucleotide sequence database.
The BLAST programs identify homologous sequences by identifying similar segments, which are referred to herein as "high-scoring segment pairs," between a query amino or nucleic acid sequence and a test sequence which is preferably obtained from a protein or nucleic acid sequence database. High-scoring segment pairs are preferably identified (*i.e.,* aligned) by means of a scoring matrix, many of which are known in the art. Preferably, the scoring matrix used is the BLOSUM62 matrix (Gonnet *et al.,* 1992, *Science 256*:1443-1445; Henikoff and Henikoff, 1993, *Proteins 17*:49-61). Less preferably, the PAM or PAM250 matrices may also be used (see, *e.g.,* Schwartz and Dayhoff, eds., 1978, *Matrices for Detecting Distance Relationships: Atlas of Protein Sequence and Structure,* Washington: National Biomedical Research Foundation)

The BLAST programs evaluate the statistical significance of all high-scoring segment pairs identified, and preferably selects those segments which satisfy a user-specified threshold of significance, such as a user-specified percent homology. Preferably, the statistical significance of a high-scoring segment pair is evaluated using the statistical significance formula of Karlin (see, *e.g.,* Karlin and Altschul, 1990, *Proc. Natl. Acad Sci. USA 87*:2267-2268).

Nucleotide sequence complementary to a sequence of the invention is understood to mean any DNA whose nucleotides are complementary to those of the sequence of the invention, and whose orientation is reversed (antiparallel sequence).

The present invention further discloses fragments of the sequences of a) through f), above. Representative fragments of the sequences according to the invention will be understood to mean any nucleotide fragment having at least 8 successive nucleotides, preferably at least 12 successive nucleotides, and still more preferably at least 15 or at least 20 successive nucleotides of the sequence from which it is derived. It is understood that such fragments refer only to portions of SEQ ID No. 1 that are not currently listed in a publicly available database.

Among these representative fragments, those capable of hybridizing under stringent conditions with a nucleotide sequence according to the invention can be cited. Hybridization under stringent conditions means that the temperature and ionic strength conditions are chosen such that they allow hybridization to be maintained between two complementary DNA fragments.

By way of illustration, high stringency conditions for the hybridization step for the purposes of defining the nucleotide fragments described above, are advantageously the following.

The hybridization is carried out at a preferred temperature of 65EC in the presence of SSC buffer, 1 x SSC corresponding to 0.15 M NaCl and 0.05 M Na citrate. The washing steps may be, for example, the following:
2 × SSC, 0.1% SDS at room temperature followed by three washes with 1 × SSC, 0.1% SDS; 0.5 × SSC, 0.1% SDS; 0.1 × SSC, 0.1% SDS at 68EC for 15 minutes.

Intermediate stringency conditions, using, for example, a temperature of 60EC in the presence of a 5 × SSC buffer, or of low stringency, for example a temperature of 50EC in the presence of a 5 × SSC buffer, respectively require a lower overall complementarity for the hybridization between the two sequences.

The stringent hybridization conditions described above for a polynucleotide of about 300 bases in size will be adapted by persons skilled in the art for larger- or smaller-sized oligonucleotides, according to the teaching of Sambrook et al., 1989.

Among the representative fragments, those which can be used as primer or probe in methods which make it possible to obtain homologous sequences or their representative fragments, or to reconstitute a genomic fragment found to be incomplete in the sequence SEQ ID No. 1 or carrying an error or an uncertainly, can be also cited these methods, such as the polymerase chain reaction (PCR), cloning and sequencing of nucleic acid being well known to persons skilled in the art. These homologous nucleotide sequences correspond to mutations or to inter- or intra-species variations, as well as the complete genomic sequence or one of its representative fragments capable of being reconstituted.

Among the said representative fragments, those which can be used as primer or probe in methods allowing diagnosis of the presence of *Chlamydia pneumoniae* or one of its associated microorganisms as defined below can be also cited.

The representative fragments capable of modulating, regulating, inhibiting or inducing the expression of a gene of *Chlamydia pneumoniae* or one of its associated microorganisms, and/or capable of modulating the replication cycle of *Chlamydia pneumoniae* or one of its associated microorganisms in the host cell and/or organism, can be also cited. Replication cycle is intended to designate invasion, multiplication, intracellular localization, in particular retention in the vacuole and inhibition of the process of fusion to the lysosome, and propagation of *Chlamydia pneumoniae* or one of its associated microorganisms from host cells to host cells.

Among the said representative fragments, those corresponding to nucleotide sequences corresponding to open reading frames, called ORF sequences (ORF for open reading frame), and encoding polypeptides, are finally described.

The representative fragments may be obtained, for example, by specific amplification, such as PCR, or after digestion, with appropriate restriction enzymes, of nucleotide sequences according to the invention; these methods are in particular described in the manual by Sambrook et al., 1989. The said representative fragments may also be obtained by chemical synthesis when they are not too large in size and according to methods well known to persons skilled in the art. For example, such fragments can be obtained by isolating fragments of the genomic DNA of ATCC Deposit No. 1360-VR or a clone insert present at this ATCC Deposit No. 1360-VR.

The representative fragments may be used, for example, as primer, to reconstitute some of the said representative fragments, in particular those in which a portion of the sequence is likely to be missing or imperfect, by methods well known to persons skilled in the art such as amplification, cloning or sequencing techniques.

Modified nucleotide sequence will be understood to mean any nucleotide sequence obtained by mutagenesis according to techniques well known to persons skilled in the art, and exhibiting modifications in relation to the normal sequences, for example mutations in the regulatory and/or promoter sequences for the expression of a polypeptide, in particular leading to a modification of the level of expression of the said polypeptide or to a modulation of the replicative cycle.

Modified nucleotide sequence will also be understood to mean any nucleotide sequence encoding a modified polypeptide as defined below.

The present invention also describes *Chlamydia pneumoniae* nucleotide sequences chosen from a nucleotide sequence of an open reading frame (ORF), that is, the ORF2 to ORF1297 sequences.

The ORF2 to ORF1297 nucleotide sequences are defined in Tables 1 and 2, *infra,* by their position on the sequence SEQ ID No. 1. For example, the ORF2 sequence is defined by the nucleotide sequence between the nucleotides at position 42 and 794 on the sequence SEQ ID No. 1, ends included. ORF2 to ORF1297 have been identified via homology analyses as well as via analyses of potential ORF start sites, as discussed in the examples below. It is to be understood that each identified ORF comprises a nucleotide sequence that spans the contiguous nucleotide sequence from the ORF stop codon immediately 3' to the stop codon of the preceding ORF and through the 5' codon to the next stop codon of SEQ ID No.:1 in-frame to the ORF nucleotide sequence. Table 2, *infra,* lists the beginning, end and potential start site of each of ORFs 1-1297. In one embodiment, the ORF comprises the contiguous nucleotide sequence spanning from the potential ORF start site downstream (that is, 3') to the ORF stop codon (or the ORF codon immediately adjacent to and upstream of the ORF stop codon). ORF2 to ORF1297 encode the polypeptides No. 2 to No. 1291 and No. 6844 to No. 6849, respectively.

Upon introduction of minor frameshifts, certain individual ORFs can comprise larger "combined" ORFs. A list of such putative "combined" ORFs is shown in Table 3, below. For example, a combined ORF can comprise ORF 25, ORF 26 and ORF 27, including intervening in-frame, nucleotide sequences. The order of ORFs (5' to 3'), within each "combined" ORF is as listed. It is to be understood that when ORF2 to ORF1297 are referred to herein, such reference is also meant to include "combined" ORFs.

**Table 3**

| |
|---|
| ORF 25, ORF 26, ORF 27; |
| ORF 28, ORF 29, ORF 30; |
| ORF 31, ORF 32; |
| ORF 33, ORF 35; |
| ORF 466, ORF 467; |
| ORF 468, ORF 469; |
| ORF 477, ORF 476, ORF 474; |
| ORF 480, ORF 482; |
| ORF 483, ORF 485, ORF 486, ORF 500; |
| ORF 503, ORF 504, ORF 505; |
| ORF 506, ORF 507; |
| ORF 1211, ORF 647; |
| ORF 1286, ORF 1039; |
| ORF 691, ORF 690; |
| ORF 105, ORF 106; |
| ORF 170, ORF 171;ORF 394, ORF 393; |
| ORF 453, ORF 452, ORF 451; |
| ORF 526, ORF 525; |
| ORF 757, ORF 756, ORF 755; |
| ORF 856, ORF 855; |
| ORF 958, ORF 957; |
| ORF 915, ORF 914, ORF 913; |
| ORF 543, ORF 544; |
| ORF 1266, ORF 380; |
| ORF 745, ORF 744; |
| ORF 777, ORF 776; |
| ORF 343, ORF 1297, and representative fragments. |

Table 1 also depicts the results of homology searches that compared the sequences of the polypeptides encoded by each of the ORFs to sequences present in public published databases.

The invention also cites the polypeptides comprising a polypeptide chosen from:
a) a polypeptide encoded by a polynucleotide sequence in SEQ ID No. (*e.g.,* any polypeptide encoded by a polynucleotide sequence corresponding to ORF2 to ORF1297 and/or representative fragments thereof);
b) a polypeptide homologous to a polypeptide as defined in a);
c) a polypeptide encoded by a polynucleotide sequence that hybridizes to SEQ ID No. 1 or ORF2 to ORF1297 under high or intermediate stringency;
d) a fragment of at least 5 amino acids of a polypeptide as defined in a), b) or c);
e) a biologically active fragment of a polypeptide as defined in a), b), c) or d); and
f) a modified polypeptide of a polypeptide as defined in a), b), c),d) or e).

In the present description, the terms polypeptide, peptide and protein are interchangeable.

Homologous polypeptide will be understood to designate the polypeptides exhibiting, in relation to the natural polypeptide, certain modifications such as in particular a deletion, addition or substitution of at least one amino acid, a truncation, an extension, a chimeric fusion, and/or a mutation, or polypeptides exhibiting post-translational modifications. Among the homologous polypeptides, those whose amino acid sequence exhibits at least 80%, preferably 9096, homology or identity with the amino acid sequences of the natural polypeptides are preferred. In the case of a substitution, one or more consecutive or nonconsecutive amino acids are replaced by "equivalent" amino acids. The expression "equivalent" amino acid is intended here to designate any amino acid capable of being substituted for one of the amino acids in the basic structure without, however, essentially modifying the biological activities of the corresponding peptides and as will be defined later.

Protein and/or nucleic acid sequence homologies may be evaluated using any of the variety of sequence comparison algorithms and programs known in the art. Such algorithms and programs include, but are by no means limited to, TBLASTN, BLASTP, FASTA, TFASTA, and CLUSTALW (Pearson and Lipman, 1988, *Proc. Natl. Acad. Sci. USA 85(8)*:2444-2448; Altschul *et al.,* 1990, *J. Mol. Biol. 215(3)*:403-410; Thompson *et al.*, 1994, *Nucleic Acids Res. 22(2)*:4673-4680; Higgins *et al.*, 1996, *Methods Enzymol. 266*:383-402; Altschul *et al.*, 1990, *J. Mol. Biol. 215(3)*:403-410; Altschul *et al.*, 1993, *Nature Genetics 3*:266-272).

Equivalent amino acids may be determined either based on their structural homology with the amino acids for which they are substituted, or on results of comparative tests of biological activity between the various polypeptides which may be carried out.

By way of example, there may be mentioned the possibilities of substitutions which may be carried out without resulting in a substantial modification of the biological activity of the corresponding modified polypeptides; the replacements, for example, of leucine with valine or isoleucine, of aspartic acid with glutamic acid, of glutamine with asparagine, of arginine with lysine, and the like, the reverse substitutions naturally being feasible under the same conditions.

The homologous polypeptides also correspond to the polypeptides encoded by the homologous nucleotide sequences as defined above and thus comprise in the present definition the mutated polypeptides or polypeptides corresponding to inter- or intra-species variations which may exist in *Chlamydia,* and which correspond in particular to truncations, substitutions, deletions and/or additions of at least one amino acid residue.

Biologically active fragment of a polypeptide will be understood to designate in particular a polypeptide fragment, as defined below, exhibiting at least one of the characteristics of the polypeptides, in particular in that it is:
- capable of eliciting an immune response directed against *Chlamydia pneumoniae;* and/or
- capable of being recognized by an antibody specific for a polypeptide according to the invention; and/or
- capable of binding to a polypeptide or to a nucleotide sequence of *Chlamydia pneumoniae*; and/or
- capable of modulating, regulating, inducing or inhibiting the expression of a gene of *Chlamydia pneumoniae* or one of its associated microorganisms, and/or capable of modulating the replication cycle of *Chlamydia pneumoniae* or one of its associated microorganisms in the host cell and/or organism; and/or
- capable of generally exerting an even partial physiological activity, such as for example a structural activity (cellular envelope, ribosome), an enzymatic (metabolic) activity, a transport activity, an activity in the secretion or in the virulence.

A polypeptide fragment is understood to designate a polypeptide comprising a minimum of 5 amino acids, preferably 10 amino acids or preferably 15 amino acids. It is to be understood that such fragments refer only to portions of polypeptides encoded by ORF2 to ORF1297 that are not currently listed in a publicly available database.

The polypeptide fragments may correspond to isolated or purified fragments which are naturally present in *Chlamydia pneumoniae* or which are secreted by *Chlamydia pneumoniae,* or may correspond to fragments capable of being obtained by cleaving the said polypeptide with a proteolytic enzyme, such as trypsin or chymotrypsin or collagenase, or with a chemical reagent, such as cyanogen bromide (CNBr) or alternatively by placing the said polypeptide in a highly acidic environment, for example at pH 2.5. Such polypeptide fragments may be equally well prepared by chemical synthesis, using hosts transformed with an expression vector according to the invention containing a nucleic acid allowing the expression of the said fragments, placed under the control of appropriate elements for regulation and/or expression.

"Modified polypeptide" of a polypeptide is understood to designate a polypeptide obtained by genetic recombination or by chemical synthesis as will be described below, exhibiting at least one modification in relation to the normal sequence. These modifications may in particular affect amino acids responsible for a specificity or for the efficiency of the activity, or responsible for the structural conformation, for the charge or for the hydrophobicity, and for the capacity for multimerization and for membrane insertion of the polypeptide. It is thus possible to create polypeptides with an equivalent, an increased or a reduced activity, and with an equivalent, a narrower or a broader specificity. Among the modified polypeptides, there may be mentioned the polypeptides in which up to 5 amino acids may be modified, truncated at the N- or C-terminal end, or alternatively deleted, or else added.

As is indicated, the modifications of the polypeptide may have in particular the objective:
- of making it capable of modulating, regulating, inhibiting or inducing the expression of a gene of *Chlamydia,* in particular of *Chlamydia pneumoniae* and its variants, or one of its associated microorganisms, and/or capable of modulating the replication cycle of *Chlamydia,* in particular of *Chlamydia pneumoniae* and its variants, or one of its associated microorganisms, in the host cell and/or organism,
- of allowing its use in methods of biosynthesis or of biodegradation, or its incorporation into vaccine compositions.
- of modifying its bioavailability as a compound for therapeutic use.

The said modified polypeptides may also be used on any cell or microorganism for which the said modified polypeptides will be capable of modulating, regulating, inhibiting or inducing gene expression, or of modulating the growth or the replication cycle of the said cell or of the said microorganism. The methods allowing demonstration of the said modulations on eukaryotic or prokaryotic cells are well known to persons skilled in the art. The said cells or microorganisms will be chosen, in particular, from tumour cells or infectious microorganisms and the said modified polypeptides may be used for the prevention or treatment of pathologies linked to the presence of the said cells or of the said microorganisms. It is also clearly understood that the nucleotide sequences encoding the said modified polypeptides may be used for the said modulations, for example by the intermediacy of vectors according to the invention and which are described below, so as to prevent or to treat the said pathologies.

The above modified polypeptides may be obtained using combinatory chemistry, in which it is possible to systematically vary portions of the polypeptide before testing them on models, cell cultures or microorganisms for example, so as to select the compounds which are the most active or which exhibit the desired properties.

Chemical synthesis also has the advantage of being able to use:
- nonnatural amino acids, or
- nonpeptide bonds.

Accordingly, in order to extend the life of the polypeptides, it may be advantageous to use nonnatural amino acids, for example in the D form, or alternatively amino acid analogues, in particular sulphur-containing forms for example.

Finally, the structure of the polypeptides, its homologous or modified forms, as well as the corresponding fragments may be integrated into chemical structures of the polypeptide type and the like. Accordingly, it may be advantageous to provide at the N- and C-terminal ends compounds which are not recognized by proteases.

Described below are ORF nucleotide sequences encoding polypeptides exhibiting particularly preferable characteristics. For each group of preferred ORFS described below, it is to be understood that in addition to the individual ORFs listed, in instances wherein such ORFS are present as part of "combined" ORFs, the "combined" ORFs are also to be included within the preferred group.

More particularly, the invention describes nucleotide sequences, characterized in that they encode a polypeptide of the cellular envelope, preferably of the outer cellular envelope of *Chlamydia pneumoniae* or one of its representative fragments, such as for example the predominant proteins of the outer membrane, the adhesion proteins or the proteins entering into the composition of the Chlamydia wall. Among these sequences, the sequences comprising a nucleotide sequence chosen from the following sequences are most preferred:
ORF15; ORF25; ORF26; ORF27; ORF28; ORF29; ORF30; ORF31; ORF32; ORF33; ORF35; ORF68; ORF124; ORF275; ORF291; ORF294; ORF327; ORF342; ORF364; ORF374; ORF380; ORF414; ORF439; ORF466; ORF467; ORF468; ORF469; ORF470; ORF472; ORF474; ORF476; ORF477; ORF478; ORF479; ORF480; ORF482; ORF485; ORF500; ORF501; ORF503; ORF504; ORF505; ORF506; ORF520; ORF578; ORF580; ORF581; ORF595; ORF596; ORF597; ORF737; ORF830; ORF834; ORF836; ORF893; ORF917; ORF932; ORF976; ORF1035; ORF1045; ORF1090 and one of their representative fragments.

The structure of the cytoplasmic membranes and of the wall of bacteria is dependent on the associated proteins. The structure of the cytoplasmic membrane makes it impermeable to water, to water-soluble substances and to small-sized molecules (ions, small inorganic molecules, peptides or proteins). To enter into or to interfere with a cell or a bacterium, a ligand must establish a special relationship with a protein anchored in the cytoplasmic membrane (the receptor). These proteins which are anchored on the membrane play an important role in metabolism since they control the exchanges in the bacterium. These exchanges apply to molecules of interest for the bacterium (small molecules such as sugars and small peptides) as well as undesirable molecules for the bacterium such as antibiotics or heavy metals.

The double lipid layer structure of the membrane requires the proteins which are inserted therein to have hydrophobic domains of about twenty amino acids forming an alpha helix. Predominantly hydrophobic and potentially transmembrane regions may be predicted from the primary sequence of the proteins, itself deduced from the nucleotide sequence. The presence of one or more putative transmembrane domains raises the possibility for a protein to be associated with the cytoplasmic membrane and to be able to play an important metabolic role therein or alternatively for the protein thus exposed to be able to exhibit potentially protective epitopes.

If the proteins inserted into the membrane exhibit several transmembrane domains capable of interacting with one another via electrostatic bonds, it then becomes possible for these proteins to form pores which go across the membrane which becomes permeable for a number of substances. It should be noted that proteins which do not have transmembrane domains may also be anchored by the intermediacy of fatty acids in the cytoplasmic membrane, it being possible for the breaking of the bond between the protein and its anchor in some cases to be responsible for the release of the peptide outside the bacterium.

The invention describes nucleotide sequences, characterized in that they encode a *Chlamydia pneumoniae* transmembrane polypeptide or one of its representative fragments, having between I and 3 transmembrane domains and in that they comprise a nucleotide sequence chosen from the following sequences:
ORF2; ORF3; ORF6; ORF9; ORF10; ORF11; ORF13; ORF14; ORF16; ORF18; ORF19; ORF20; ORF21; ORF22; ORF25; ORF27; ORF28; ORF29; ORF30; ORF31; ORF32; ORF33; ORF34; ORF35; ORF37; ORF39; ORF41; ORF42; ORF44; ORF45; ORF46; ORF47; ORF48; ORF49; ORF50; ORF53; ORF54; ORF56; ORF57; ORF59; ORF60; ORF61; ORF62; ORF63; ORF64; ORF65; ORF66; ORF69;; ORF72; ORF73; ORF74; ORF76; ORF77; ORF78; ORF19; ORF80; ORF82; ORF84; ORF85; ORF86; ORF88; ORF89; ORF90; ORF91; ORF92; ORF93; ORF95; ORF96; ORF98; ORF99; ORF100; ORF101; ORF102; ORF103; ORF104; ORF105; ORF106; ORF107; ORF108; ORF114; ORF117; ORF118; ORF122; ORF123; ORF124; ORF125; ORF129; ORF130; ORF131; ORF132; ORF133; ORF134; ORF135; ORF137; ORF138; ORF139; ORF140; ORF141; ORF142; ORF143; ORF145; ORF146; ORF147; ORF150; ORF151; ORF152; ORF156; ORF157; ORF158; ORF159; ORF160; ORF161; ORF162; ORF164; ORF166; ORF167; ORF170; ORF173; ORF175; ORF176; ORF178; ORF179; ORF180; ORF182; ORF183; ORF184; ORF185; ORF186; ORF187; ORF188; ORF189; ORF190; ORF191; ORF192; ORF194; ORF195; ORF196; ORF197; ORF198; ORF199; ORF200; ORF201; ORF202; ORF205; ORF207; ORF208; ORF209; ORF210; ORF212; ORF215; ORF219; ORF220; ORF224; ORF226; ORF227; ORF228; ORF231; ORF232; ORF233; ORF234; ORF235; ORF236; ORF238; ORF239; ORF240; ORF241; ORF242; ORF244; ORF247; ORF251; ORF252; ORF253; ORF255; ORF256; ORF257; ORF258; ORF260; ORF262; ORF263; ORF266; ORF267; ORF268; ORF269; ORF270; ORF273; ORF274; ORF276; ORF278; ORF279; ORF280; ORF281; ORF282; ORF283; ORF284; ORF286; ORF287; ORF289; ORF290; ORF291; ORF293; ORF294; ORF297; ORF304; ORF305; ORF307; ORF308; ORF309; ORF310; ORF311; ORF313; ORF314; ORF315; ORF316; ORF318; ORF319; ORF320; ORF321; ORF322; ORF323; ORF324; ORF325; ORF326; ORF331; ORF332; ORF336; ORF338; ORF339; ORF341; ORF344; ORF345; ORF346; ORF350; ORF352; ORF353; ORF356; ORF357; ORF358; ORF359; ORF360; ORF362; ORF365; ORF366; ORF367; ORF370; ORF372; ORF373; ORF376; ORF377; ORF378; ORF379; ORF381; ORF382; ORF383; ORF384; ORF385; ORF386; ORF387; ORF390; ORF392; ORF393; ORF394; ORF396; ORF398; ORF399; ORF400; ORF404; ORF408; ORF410; ORF411; ORF413; ORF416; ORF417; ORF418; ORF420; ORF422; ORF424; ORF427; ORF428; ORF429; ORF430; ORF431; ORF433; ORF434; ORF437; ORF440; ORF441; ORF442; ORF443; ORF444; ORF445; ORF447; ORF450; ORF451; ORF452; ORF455; ORF456; ORF459; ORF460; ORF461; ORF462; ORF463; ORF464; ORF465; ORF467; ORF469; ORF471; ORF474; ORF475; ORF476; ORF477; ORF479; ORF482; ORF483; ORF484; ORF485; ORF486; ORF487; ORF488; ORF491; ORF493; ORF494; ORF497; ORF498; ORF499; ORF503; ORF508; ORF509; ORF510; ORF512; ORF514; ORF515; ORF516; ORF517; ORF518; ORF520; ORF521; ORF523; ORF525; ORF527; ORF528; ORF529; ORF530; ORF531; ORF533; ORF534; ORF535; ORF536; ORF537; ORF540; ORF541; ORF543; ORF544; ORF545; ORF546; ORF548; ORF549; ORF551; ORF553; ORF554; ORF555; ORF556; ORF557; ORF558; ORF559; ORF560; ORF562; ORF563; ORF564; ORF565; ORF566; ORF569; ORF571; ORF573; ORF576; ORF577; ORF581; ORF583; ORF584; ORF585; ORF586; ORF588; ORF591; ORF592; ORF594; ORF595; ORF596; ORF597; ORF599; ORF600; ORF603; ORF605; ORF608; ORF614; ORF615; ORF620; ORF621; ORF622; ORF623; ORF624; ORF625; ORF629; ORF630; ORF631; ORF633; ORF634; ORF637; ORF642; ORF644; ORF645; ORF647; ORF648; ORF652; ORF654; ORF655; ORF657; ORF658; ORF659; ORF660; ORF661; ORF664; ORF665; ORF666; ORF667; ORF670; ORF671; ORF672; ORF673; ORF674; ORF676; ORF679; ORF681; ORF684; ORF687; ORF688; ORF689; ORF690; ORF693; ORF694; ORF695; ORF696; ORF697; ORF698; ORF699; ORF700; ORF701; ORF703; ORF705; ORF706; ORF707; ORF708; ORF710; ORF712; ORF715; ORF716; ORF717; ORF718; ORF719; ORF721; ORF722; ORF723; ORF725; ORF726; ORF727; ORF728; ORF729; ORF730; ORF731; ORF733; ORF736; ORF737; ORF738; ORF740; ORF741; ORF742; ORF743; ORF747; ORF748; ORF750; ORF752; ORF754; ORF755; ORF756; ORF757; ORF759; ORF760; ORF761; ORF762; ORF763; ORF764; ORF765; ORF766; ORF767; ORF768; ORF772; ORF774; ORF775; ORF777; ORF781; ORF783; ORF788; ORF791; ORF792; ORF793; ORF794; ORF795; ORF796; ORF797; ORF798; ORF799; ORF802; ORF803; ORF806; ORF807; ORF808; ORF809; ORF810; ORF811; ORF813; ORF814; ORF815; ORF816; ORF817; ORF819; ORF820; ORF821; ORF823; ORF824; ORF827; ORF829; ORF830; ORF831; ORF833; ORF834; ORF835; ORF837; ORF844; ORF845; ORF846; ORF847; ORF848; ORF849; ORF850; ORF851; ORF852; ORF854; ORF855; ORF856; ORF857; ORF859; ORF860; ORF862; ORF865; ORF866; ORF868; ORF869; ORF870; ORF871; ORF872; ORF874; ORF877; ORF878; ORF879; ORF880; ORF881; ORF882; ORF884; ORF885; ORF888; ORF889; ORF890; ORF891; ORF892; ORF894; ORF895; ORF896; ORF897; ORF899; ORF900; ORF902; ORF903; ORF904; ORF905; ORF909; ORF910; ORF912; ORF913; ORF914; ORF915; ORF917; ORF918; ORF919; ORF921; ORF923; ORF924; ORF926; ORF927; ORF928; ORF929; ORF930; ORF931; ORF937; ORF938; ORF939; ORF941; ORF943; ORF948; ORF951; ORF952; ORF953; ORF958; ORF960; ORF963; ORF964; ORF965; ORF968; ORF970; ORF974; ORF975; ORF977; ORF979; ORF980; ORF981; ORF983; ORF984; ORF985; ORF987; ORF989; ORF992; ORF993; ORF997; ORF998; ORF999; ORF1001; ORF1002; ORF1004; ORF1005; ORF1009; ORF1013; ORF1014; ORF1015; ORF1016; ORF1019; ORF1021; ORF1023; ORF1024; ORF1029; ORF1031; ORF1033; ORF1034; ORF1039; ORF1041; ORF1042; ORF1045; ORF1047; ORFI049; ORF1051; ORF1052; ORF1053; ORF1054; ORF1056; ORF1059; ORF1061; ORF1062; ORF1063; ORF1064; ORF1065; ORF1067; ORF1075; ORF1077; ORF1078; ORF1079; ORF1080; ORF1081; ORF1089; ORF1095; ORF1097; ORF1098; ORF1099; ORF1101; ORF1102; ORF1103; ORF1106; ORF1107; ORF1108; ORF1109; ORF1110; ORF1113; ORF1116; ORF1118; ORF1119; ORF1121; ORF1123; ORF1124; ORF1126; ORF1128; ORF1130; ORF1131; ORF1133; ORF1134; ORF1136; ORF1137 and one of their representative fragments.

The invention describes the nucleotide sequences, characterized in that they encode a *Chlamydia pneumoniae* transmembrane polypeptide or one of its representative fragments, having between 4 and 6 transmembrane domains and in that they comprise a nucleotide sequence chosen from the following sequences:
ORF5; ORF7; ORF8; ORF15; ORF36; ORF38; ORF51; ORF55; ORF58; ORF67; ORF70; ORF81; ORF97; ORF110; ORF111; ORF115; ORF119; ORF126; ORF128; ORF148; ORF155; ORF163; ORF165; ORF168; ORF169; ORF171; ORF172; ORF174; ORF177; ORF181; ORF193; ORF203; ORF213; ORF214; ORF216; ORF217; ORF221; ORF222; ORF225; ORF229; ORF243; ORF246; ORF248; ORF254; ORF261; ORF285; ORF288; ORF292; ORF296; ORF298; ORF299; ORF301; ORF303; ORF317; ORF328; ORF329; ORF351; ORF354; ORF355; ORF364; ORF371; ORF374; ORF375; ORF391; ORF395; ORF401; ORF403; ORF405; ORF409; ORF414; ORF419; ORF421; ORF423; ORF425; ORF438; ORF448; ORF453; ORF458; ORF466; ORF468; ORF470; ORF480; ORF489; ORF490; ORF496; ORF501; ORF504; ORF505; ORF506; ORF511; ORF513; ORF519; ORF526; ORF532; ORF538; ORF539; ORF547; ORF550; ORF561; ORF568; ORF570; ORF574; ORF578; ORF579; ORF580; ORF582; ORF589; ORF593; ORF598; ORF601; ORF604; ORF610; ORF613; ORF617; ORF626; ORF632; ORF635; ORF638; ORF640; ORF641; ORF646; ORF649; ORF650; ORF651; ORF686; ORF711; ORF724; ORF732; ORF734; ORF744; ORF745; ORF749; ORF751; ORF769; ORF770; ORF771; ORF773; ORF776; ORF779; ORF780; ORF785; ORF787; ORF789; ORF801; ORF805; ORF812; ORF822; ORF825; ORF826; ORF839; ORF841; ORF843; ORF853; ORF861; ORF875; ORF876; ORF886; ORF893; ORF898; ORF906; ORF907; ORF908; ORF920; ORF922; ORF925; ORF933; ORF935; ORF936; ORF944; ORF946; ORF947; ORF954; ORF959; ORF961; ORF966; ORF967; ORF972; ORF978; ORF995; ORF996; ORF1000; ORF1003; ORF1010; ORY-1011; ORF1012; ORF1017; ORF1020; ORF1030; ORF1036; ORF1038; ORF1043; ORF1046; ORF1048; ORF1050; ORF1058; ORF1071; ORF1073; ORF1084; ORF1085; ORF1086; ORF1087; ORF1091; ORF1092; ORF1094; ORF1096; ORF1100; ORF1104; ORF1111; ORF1112; ORF1114; ORF1117; ORF1122; ORF1125 and one of their representative fragments.

The invention also describes the nucleotide sequences, characterized in that they encode a *Chlamydia pneumoniae* transmembrane polypeptide or one of its representative fragments, having at least 7 transmembrane domains and in that they comprise a nucleotide sequence chosen from the following sequences:
ORF17; ORF52; ORF68; ORF83; ORF87; ORF109; ORF112; ORF113; ORF120; ORF121; ORF127; ORF153; ORF204; ORF211; ORF218; ORF223; ORF275; ORF277; ORF295; ORF300; ORF302; ORF306; ORF327; ORF335; ORF342; ORF343; ORF347; ORF349; ORF361; ORF363; ORF369; ORF380; ORF388; ORF389; ORF397; ORF415; ORF432; ORF439; ORF446; ORF449; ORF472; ORF478; ORF500; ORF522; ORF524; ORF567; ORF575; ORF602; ORF606; ORF609; ORF636; ORF639; ORF643; ORF653; ORF668; ORF692; ORF702; ORF704; ORF713; ORF720; ORF778; ORF784; ORF800; ORF836; ORF838; ORF842; ORF864; ORF867; ORF883; ORF901; ORF916; ORF932; ORF934; ORF940; ORF942; ORF950; ORF956; ORF971; ORF973; ORF976; ORF988; ORF994; ORF1018; ORF1028; ORF1035; ORF1037; ORF1044; ORF1055; ORF1057; ORF1068; ORF1069; ORF1070; ORF1072; ORF1082; ORF1088; ORF1105; ORF1132; ORF1135 and one of their representative fragments.

The invention describes the nucleotide sequences, characterized in that they encode a *Chlamydia pneumoniae* surface exposed polypeptide *(e.g.,* an outer membrane protein) or one of its representative fragments, said nucleotide sequences comprising a nucleotide sequence chosen from the following sequences:
ORF 15, ORF 25, ORF 26, ORF 27, ORF 28, ORF 29, ORF 30, ORF 31, ORF 32, ORF 33, ORF 35, ORF 36, ORF 1257, ORF 280, ORF 291, ORF 314, ORF 354, ORF 380, ORF 1266, ORF 466, ORF 467, ORF 468, ORF 469, ORF 470, ORF 472, ORF 474, ORF 476, ORF 477, ORF 478, ORF 479, ORF 480, ORF 482, ORF 483, ORF 485, ORF 486, ORF 500, ORF 501, ORF 503, ORF 504, ORF 505, ORF 506, ORF 507, ORF 1268, ORF 1269, ORF 543, ORF 544, ORF 578, ORF 579, ORF 580, ORF 581, ORF 595, ORF 596, ORF 597, ORF 1271, ORF 633, ORF 637, ORF 699, ORF 706, ORF 737, ORF 744, ORF 1273, ORF 751, ORF 775, ORF 776, ORF 777, ORF 793, ORF 815, ORF 830, ORF 1221, ORF 849, ORF 851. ORF 852, ORF 874, ORF 891, ORF 922, ORF 940, ORF 1231, ORF 1281, ORF 1035, ORF 1079, ORF 1087 ORF 1108, and one of their representative fragments.

The invention describes the nucleotide sequences, characterized in that they encode a *Chlamydia pneumoniae* lipoprotein or one of its representative fragments, said nucleotide sequences comprising a nucleotide sequence chosen from the following sequences:
ORF 3, ORF 10, ORF 11, ORF 16, ORF 1254, ORF 1255, ORF 38, ORF 1256, ORF 62, ORF 85, ORF 1258, ORF 115, ORF 1151, ORF 151, ORF 1259, ORF 173, ORF 1261, ORF 186, ORF 194, ORF 205, ORF 214, ORF 216, ORF 217, ORF 238, ORF 1177, ORF 280, ORF 291, ORF 317, ORF 327, ORF 354, ORF 364, ORF 367, ORF 414, ORF 432, ORF 1192, ORF 460, ORF 1267, ORF 1268, ORF 520, ORF 536, ORF 1270, ORF 576, ORF 597, ORF 603, ORF 609, ORF 637, ORF 1272, ORF 652, ORF 1213, ORF 699, ORF 705, ORF 706, ORF 708, ORF 711, ORF 727, ORF 1274, ORF 800, ORF 814, ORF 825, ORF 829, ORF 830, ORF 831, ORF 844, ORF 849, ORF 1275, ORF 1276, ORF 1277, ORF 872, ORF 878, ORF 880, ORF 891, ORF 892, ORF 1278, ORF 1279, ORF 1280, ORF 941, ORF 942, ORF 1282, ORF 1283, ORF 952, ORF 988, ORF 998, ORF 1009, ORF 1285, ORF 1235, ORF 1028, ORF 1056, ORF 1070, ORF 1287, ORF 1087, ORF 1288, ORF 1289, ORF 1098, ORF 1246, ORF 1291, ORF 1108, ORF 1109, ORF 1112, ORF 1133, and one of their representative fragments.

The invention describes the nucleotide sequences, characterized in that they encode a *Chlamydia pneumoniae* polypeptide involved in lipopolysaccharide (LPS) biosynthesis, said nucleotide sequences comprising a nucleotide sequence chosen from the following sequences: ORF 316, ORF 564, ORF 610, ORF 647, ORF 1211, ORF 688, ORF 924, and one of their representative fragments.

The invention describes additional LPS-related nucleotide sequences to the invention, characterized in that they encode:
(a) a *Chlamydia pneumoniae* KDO (3-deoxy-D-manno-octulosonic acid)-related polypeptide or one of its representative fragments, said nucleotide sequences comprising a nucleotide sequence chosen from the following sequences: ORF 177, ORF 1156, ORF 245, ORF 767, and one of their representative fragments;
(b) a *Chlamydia pneumoniae* phosphomannomutase-related polypeptide or one of its representative fragments, said nucleotide sequences comprising a nucleotide sequence chosen from the following sequences: ORF 74, and one of its representative fragments;
(c) a *Chlamydia pneumoniae* phosphoglucomutase-related polypeptide or one of its representative fragments, said nucleotide sequences comprising a nucleotide sequence chosen from the following sequences: ORF 1286, ORF 1039, and one of their representative fragments; and
(d) a *Chlamydia pneumoniae* lipid A component-related polypeptide or one of its representative fragments, said nucleotide sequences comprising a nucleotide sequence chosen from the following sequences: ORF 689, ORF 690, ORF 691, ORF 1037, and one of their representative fragments.

The invention describes nucleotide sequences, characterized in that they encode a *Chlamydia pneumoniae* polypeptide containing RGD (Arg-Gly-Asp) attachment sites or one of its representative fragments.
(a) RGD-containing proteins that are outer membrane proteins, are more likely to play a role in cell attachment. ORFs that encoded a protein containing an RGD sequence and also were classified as outer membrane proteins are ORF 468 and its representative fragments.
(b)An RGD-encoding ORF that showed homology to cds1, cds2, and copN type III virulence loci in *Chlamydia psittaci* (Hsia, R. et al. (1997), Type III secretion genes identity a putative virulence locus of Chlamydia. Molecular Microbiology 25:351-359) is ORF 350, and its representative fragments.
(c) The outer membrane of Chlamydia is made of cysteine-rich proteins that form a network of both intra and inter molecular disulfide links. This contributes to the integrity of the membrane since Chlamydia lacks the peptidoglycan layer that other gram-negative bacteria have. Cysteine-rich proteins that have the RGD sequence are also considered to be potential vaccine candidates. Cysteine-rich proteins were defined as proteins that had more than 3.0% cysteine in their primary amino acid sequence, above the mean genomic ORF cysteine content. The corresponding ORFs are: ORF 1290, ORF 1294, ORF 1296, and one of their representative fragments.
(d) The outer membrane of Chlamydia may also contain small proteins that have cysteines in their N- and C-terminus that may contribute to the network formed by disulfide linkages. These proteins may be anchored in the outer membrane via their N-terminus and may have their C-terminus exposed, which then can interact with the host cells. Alternatively, these proteins may be anchored in the outer membrane via both N-and C-terminus and may have regions in the middle that may be exposed which can in turn interact with the host cells. ORFs encoding polypeptides that contain cysteines in their first 30 amino acids and also contain an RGD sequence are: ORF 105, ORF 106, ORF 114, ORF 170, ORF 171, ORF 1264, ORF 268, ORF 1265, ORF 350, ORF 393, ORF 394, ORF 451, ORF 452, ORF 453, ORF 473, ORF 499, ORF 515, ORF 519, ORF 525, ORF 526, ORF 538, ORF 611, ORF 645, ORF 686, ORF 700, ORF 746, ORF 755, ORF 756, ORF 757, ORF 789, ORF 814, ORF 855, ORF 856, ORF 878, ORF 957, ORF 958, ORF 989, ORF 1290, and one of their representative fragments.
(e) RGD-containing ORFs homologous to RGD-containing ORFs from *Chlamydia trachomatis* are:
   ORF 114, ORF 468, ORF 755, ORF 756, ORF 757, ORF 855, ORF 856, ORF 905, ORF 913, ORF 914, ORF 915, and one of their representative fragments.

The invention describes nucleotide sequences, characterized in that they encode a *Chlamydia pneumoniae* Type III or other, non-type III secreted polypeptide or one of its representative fragments, said nucleotide sequences comprising a nucleotide sequence chosen from the following sequences:

ORF 25, ORF 28, ORF 29, ORF 33, ORF 308, ORF 309, ORF 343, ORF 344, ORF 345, ORF 367, ORF 414, ORF 415, ORF 480, ORF 550, ORF 579, ORF 580, ORF 581, ORF 597, ORF 699, ORF 744, ORF 751, ORF 776, ORF 866, ORF 874, ORF 883, ORF 884, ORF 888, ORF 891, ORF 1293, and one of their representative fragments.

The invention describes nucleotide sequences, characterized in that they encode a *Chlamydia pneumoniae* cell wall anchored surface polypeptide or one of its representative fragments, said nucleotide sequences comprising a nucleotide sequence chosen from the following sequences: ORF 267, ORF 271, ORF 419, ORF 590, ORF 932, ORF 1292, ORF 1295, and one of their representative fragments.

The invention describes nucleotide sequences, characterized in that they encode *Chlamydia pneumoniae* polypeptides not found in *Chlamydia trachomatis* (Blastp. P>e⁻¹⁰), said nucleotide sequences comprising a nucleotide sequence chosen from the following sequences: ORF 7, ORF 8, ORF 9, ORF 16, ORF 17, ORF 18, ORF 19, ORF 20, ORF 21, ORF 22, ORF 1254, ORF 23, ORF 1255. ORF 24, ORF 1139, ORF 1140, ORF 46, ORF 47, ORF 51, ORF 60, ORF 1256, ORF 61, ORF 62, ORF 63, ORF 64, ORF 1257, ORF 65, ORF 66, ORF 67, ORF 68, ORF 1143, ORF 1145, ORF 83, ORF 84, ORF 1146, ORF 85, ORF 86, ORF 87, ORF 1258, ORF 116, ORF 117, ORF 125, ORF 1148, ORF 143, ORF 1150, ORF 1151, ORF 144, ORF 145, ORF 147, ORF 148, ORF 149, ORF 150, ORF 152, ORF 1259, ORF 162, ORF 166, ORF 1154. ORF 167, ORF 1261, ORF 1156, ORF 1157, ORF 178, ORF 179, ORF 1158, ORF 182, ORF 183, ORF 184, ORF 185, ORF 1159, ORF 186, ORF 1160. ORF 187, ORF 188, ORF 189, ORF 190, ORF 1161, ORF 1162, ORF 191, ORF 192, ORF 194, ORF 195, ORF 1163, ORF 196, ORF 201, ORF 202, ORF 209, ORF 212, ORF 221, ORF 224, ORF 1167, ORF 226, ORF 227, ORF 228, ORF 229, ORF 230, ORF 231, ORF 232, ORF 1169, ORF 1170, ORF 1171, ORF 234, ORF 235, ORF 236, ORF 1172, ORF 243, ORF 251, ORF 252, ORF 1176, ORF 253, ORF 255, ORF 254, ORF 256, ORF 1177, ORF 1178, ORF 262, ORF 263, ORF 1264, ORF 278, ORF 279, ORF 1180, ORF 280, ORF 290, ORF 291, ORF 292, ORF 296, ORF 1181, ORF 297, ORF 298, ORF 300, ORF 1265, ORF 322, ORF 324, ORF 325, ORF 370, ORF 1186, ORF 371, ORF 372, ORF 1187, ORF 373, ORF 378, ORF 1266, ORF 382, ORF 383, ORF 384, ORF 385, ORF 386, ORF 1188, ORF 1189, ORF 391, ORF 392, ORF 398, ORF 400, ORF 403, ORF 1191, ORF 423, ORF 435, ORF 445, ORF 450, ORF 1193, ORF 456, ORF 460, ORF 461, ORF 465, ORF 1196, ORF 471, ORF 473, ORF 475, ORF 481, ORF 484, ORF 487, ORF 488, ORF 489, ORF 490, ORF 491, ORF 492, ORF 493, ORF 494, ORF 495, ORF 496, ORF 497, ORF 498, ORF 499, ORF 502, ORF 1267, ORF 1268, ORF 508, ORF 510, ORF 509, ORF 512, ORF 515, ORF 519, ORF 1197, ORF 521, ORF 1198, ORF 522, ORF 524, ORF 528, ORF 534, ORF 537, ORF 1269, ORF 1270, ORF 548, ORF 551, ORF 557, ORF 1201, ORF 1203, ORF 562, ORF 566, ORF 593, ORF 595, ORF 600, ORF 1271, ORF 604, ORF 611, ORF 612, ORF 614, ORF 616, ORF 625, ORF 627, ORF 628, ORF 629, ORF 631, ORF 641, ORF 1272, ORF 648, ORF 1212, ORF 663, ORF 685, ORF 707, ORF 714, ORF 715, ORF 716, ORF 717, ORF 722, ORF 746, ORF 1273, ORF 761, ORF 764, ORF 770, ORF 1217; ORF 783, ORF 1274, ORF 803, ORF 815, ORF 1220, ORF 835, ORF 1221, ORF 844, ORF 845, ORF 846, ORF 847, ORF 848, ORF 849, ORF 850, ORF 851, ORF 1275, ORF 852, ORF 862, ORF 1276, ORF 1277, ORF 873, ORF 1223, ORF 892, ORF 919, ORF 1225, ORF 1278, ORF 926, ORF 1228, ORF 1229, ORF 1230, ORF 1279, ORF 1281, ORF 1282, ORF 1283, ORF 948, ORF 950, ORF 949, ORF 951. ORF 980, ORF 982, ORF 1233, ORF 999, ORF 1000, ORF 1001, ORF 1002, ORF 1008, ORF 1285, ORF 1235, ORF 1016, ORF 1019, ORF 1027, ORF 1036, ORF 1241, ORF 1048, ORF 1049, ORF 1050, ORF 1053, ORF 1054, ORF 1064, ORF 1076, ORF 1091, ORF 1288, ORF 1093, ORF 1289, ORF 1101, ORF 1103, ORF 1245, ORF 1246, ORF 1247, ORF 1290, ORF 1291, ORF 1115, ORF 1116, ORF 1118, ORF 1120, ORF 1249, ORF 1121, ORF 1250, ORF 1126, ORF 1251, ORF 1127, ORF 1128, ORF 1130, ORF 1129, ORF 1131, ORF 1136, ORF 1253, ORF 1292, ORF 1294, ORF 1295, ORF 1296, and one of their representative fragments.

The invention also describes nucleotide sequences, characterized in that they encode a *Chlamydia pneumoniae* polypeptide or one of its representative fragments which is involved in the intermediate metabolism, in particular in the metabolism of sugars and/or of cofactors, such as for example triose phosphate isomerase or pyruvate kinase, and in that they comprise a nucleotide sequence chosen from the following sequences:
ORF2; ORF55; ORF56; ORF69; ORF75; ORF80: ORF100; ORF110; ORF114; ORF120; ORF121; ORF157; ORF160; ORF161; ORF172; ORF180; ORF181; ORF198; ORF200; ORF225; ORF248; ORF249; ORF276; ORF277; ORF318; ORF319; ORF320; ORF323; ORF331; ORF347; ORF375; ORF376; ORF381; ORF393; ORF394; ORF395; ORF396; ORF409; ORF446; ORF447; ORF448; ORF449; ORF513; ORF516; ORF571; ORF647; ORF662; ORF697; ORF718; ORF793; ORF794; ORF808; ORF809; ORF838; ORF839; ORF840; ORF853; ORF854; ORF918; ORF923; ORF929; ORF931; ORF938; ORF939; ORP958; ORF959; ORF960; ORF966; ORF995; ORF1021; ORF1040; ORF1041; ORF1042; ORF1085; ORF1100; ORF1102; ORF1117; ORF1118; ORF1119; ORF1120; ORF1135 and one of their representative fragments.

The invention also describes nucleotide sequences, characterized in that they encode a *Chlamydia pneumoniae* polypeptide or one of its representative fragments which is involved in the intermediate metabolism of nucleotides or nucleic acids, such as for example CTP synthetase or GMP synthetase, and in that they comprise a nucleotide sequence chosen from the following sequences:
ORF77; ORF78; ORF138; ORF189; ORF190; ORF233; ORF246; ORF338; ORF412; ORF421; ORF438; ORF607; ORF648; ORF657; ORF740; ORF783; ORF967; ORF989; ORF990; ORF992; ORF1011; ORF1058; ORF1059; ORF1073; ORF1074 and one of their representative fragments.

The invention also describes the nucleotide sequences, characterized in that they encode a *Chlamydia pneumoniae* polypeptide or one of its representative fragments which is involved in the metabolism of nucleic acids, such as for example DNA polymerases or DNA topoisomerases, and in that they comprise a nucleotide sequence chosen from the following sequences:
ORF14; ORF59; ORF70; ORF71; ORF97; ORF113; ORF137; ORF141; ORF169; ORF285; ORF287; ORF288; ORF313; ORF326; ORF358; ORF411; ORF443; ORF548; ORF569; ORF601; ORF651; ORF654; ORF658; ORF659; ORF664; ORF665; ORF694; ORF698; ORF704; ORF760; ORF762; ORF763; ORF786; ORF787; ORF788; ORF801; ORF802; ORF812; ORF819; ORF822; ORF870; ORF897; ORF898; ORF902; ORF908; ORF916; ORF954; ORF955; ORF961; ORF983; ORF996; ORF1007; ORF1012; ORF1013; ORF1014; ORF1015; ORF1038; ORF1137 and one of their representative fragments.

The invention also describes nucleotide sequences, characterized in that they encode a *Chlamydia pneumoniae* polypeptide or one of its representative fragments which is involved in the metabolism of amino acids or polypeptides, such as for example serine hydroxymethyl transferase or the proteins which load amino acids onto transfer RNAs, and in that they comprise a nucleotide sequence chosen from the following sequences:
ORF99; ORF111; ORF127; ORF134; ORF140; ORF174; ORF175; ORF176; ORF353; ORF377; ORF404; ORF523; ORF539; ORF559; ORF561; ORF586; ORF598; ORF609; ORF636; ORF687; ORF700; ORF701; ORF759; ORF790; ORF857; ORF861; ORF904; ORF936; ORF952; ORF962; ORF963; ORF964; ORF965; ORF991; ORF1003; ORF1004; ORF1005; ORF1018; ORF1067; ORF1110; ORF1111; ORF1112; ORF1114; ORF1121; ORF1122; ORF1123; ORF1124; ORF1125 and one of their representative fragments.

The invention also describes nucleotide sequences, characterized in that they encode a *Chlamydia pneumoniae* polypeptide or one of its representative fragments which is involved in the metabolism of polypeptides, such as for example protein kinases or proteases, and in that they comprise a nucleotide sequence chosen from the following sequences:
ORF4; ORF44; ORF45; ORF48; ORF54; ORF112; ORF130; ORF155; ORF163; ORF212; ORF257; ORF307; ORF343; ORF405; ORF416; ORF458; ORF540; ORF541; ORF542; ORF543; ORF544; ORF560; ORF594; ORF652; ORF699; ORF723; ORF747; ORF817; ORF827; ORF871; ORF909; ORF910; ORF911; ORF912; ORF1023; ORF1051; ORF1052; ORF1081 and one of their representative fragments.

The invention also describes nucleotide sequences, characterized in that they encode a *Chlamydia pneumoniae* polypeptide or one of its representative fragments which is involved in the metabolism of fatty acids, such as for example succinyl-CoA-synthesizing proteins or phosphatidylserine synthetase, and in that they comprise a nucleotide sequence chosen from the following sequences:
ORF76; ORF284; ORF308; ORF309; ORF310; ORF311; ORF312; ORF425; ORF433; ORF565; ORF688; ORF690; ORF691; ORF767; ORF797; ORF894; ORF895; ORF994; ORF1020; ORF1030; ORF1033; ORF1034; ORF1046; ORF1047; ORF1057 and one of their representative fragments.

The invention also describes nucleotide sequences, characterized in that they encode a *Chlamydia pneumoniae* polypeptide or one of its representative fragments which is involved in the synthesis of the wall, such as for example KDO transferase, and the proteins responsible for the attachment of certain sugars onto the exposed proteins, and in that they comprise a nucleotide sequence chosen from the following sequences:
ORF49; ORF50; ORF177; ORF178; ORF245; ORF610: ORF972; ORF974; ORF978; ORF1037 and one of their representative fragments.

The invention also describes nucleotide sequences, characterized in that they encode a *Chlamydia pneumoniae* polypeptide or one of its representative fragments which is involved in the transcription, translation and/or maturation process, such as for example initiation factors, RNA polymerases or certain chaperone proteins, and in that they comprise a nucleotide sequence chosen from the following sequences:
ORF90; ORF92; ORF131; ORF151; ORF199; ORF333; ORF334; ORF336; ORF379; ORF589; ORF590; ORF619; ORF630; ORF649; ORF739; ORF741; ORF806; ORF821; ORF843; ORF968; ORF971; ORF1061 and one of their representative fragments.

The invention also describes nucleotide sequences, characterized in that they encode a *Chlamydia pneumoniae* ribosomal polypeptide or one of its representative fragments, such as for example the ribosomal proteins L21, L27 and S10, and in that they comprise a nucleotide sequence chosen from the following sequences:
ORF93; ORF94; ORF95; ORF136; ORF259; ORF332; ORF348; ORF583; ORF584; ORF588; ORF591; ORF592; ORF663; ORF666; ORF667; ORF669; ORF670; ORF671; ORF672; ORF673; ORF674; ORF675; ORF676; ORF677; ORF678; ORF679; ORF680; ORF681; ORF683; ORF684; ORF738; ORF781; ORF1008; ORF1024; ORF1025; ORF1066 and one of their representative fragments.

The invention also describes nucleotide sequences, characterized in that they encode a *Chlamydia pneumoniae* transport polypeptide or one of its representative fragments, such as for example the proteins for transporting amino acids, sugars and certain oligopeptides, and in that they comprise a nucleotide sequence chosen from the following sequences:
ORF40; ORF41; ORF52; ORF105; ORF106; ORF107; ORF109; ORF133; ORF210; ORF211; ORF214; ORF215; ORF216; ORF217; ORF218; ORF219; ORF220; ORF223; ORF242; ORF260; ORF293; ORF299; ORF366; ORF369; ORF575; ORF602; ORF638; ORF639; ORF640; ORF643; ORF653; ORF702; ORF703; ORF724; ORF732; ORF855; ORF856; ORF901; ORF906; ORF933; ORF942; ORF1043; ORF1086; ORF1105 and one of their representative fragments.

The invention also describes nucleotide sequences, characterized in that they encode a *Chlamydia pneumoniae* polypeptide or one of its representative fragments which is involved in the virulence process, such as for example the proteins analogous to the *Escherichia coli* vacB protein, and in that they comprise a nucleotide sequence chosen from the following sequences:
ORF546; ORF550; ORF778; ORF779; ORF886 and one of their representative fragments.

The invention also describes nucleotide sequences, characterized in that they encode a *Chlamydia pneumoniae* polypeptide or one of its representative fragments which is involved in the secretory system and/or which is secreted, such as for example proteins homologous to proteins in the secretory system of certain bacteria such as the Salmonellae or the Yersiniae, and in that they comprise a nucleotide sequence chosen from the following sequences:
ORF751; ORF874; ORF875; ORF876; ORF883; ORF884; ORF885 and one of their representative fragments.

The invention also describes a nucleotide sequence, characterized in that they encode a polypeptide specific to *Chlamydia pneumoniae* or one of its representative fragments (with a Blast E value of >10⁻⁵), and in that they comprise a nucleotide sequence chosen from the following sequences:
ORF7; ORF8; ORF17; ORF18; ORF19; ORF20; ORF22; ORF23; ORF24; ORF51; ORF60; ORF63; ORF65; ORF66; ORF67; ORF83; ORF84; ORF86; ORF87; ORF125; ORF143; ORF144; ORF179; ORF182; ORF184; ORF185; ORF187; ORF221; ORF252; ORF254;; ORF278; ORF279; ORF387; ORF388; ORF397; ORF1048; ORF1049; ORF1050; ORF1128; ORF1130; ORF1131 and one of their representative fragments.

In general, in the present invention, the functional group to which a polypeptide belongs, as well as its corresponding nucleotide sequence, may be determined either by comparative analogy with sequences already known, or by the use of standard techniques of biochemistry, of cytology combined with the techniques of genetic engineering such as immunoaffinity, localization by immunolabelling, differential extraction, measurement of enzymatic activity, study of the activity inducing or repressing expression or the study of expression in *E. coli.*

It is clearly understood, on the one hand, that, in the present invention, the nucleotide sequences (ORF) and the amino acid sequences (No. 2 to No. 1291 and No. 6844 to No. 6848) which are listed by functional group, are not exhaustive within the group considered. Moreover, it is also clearly understood that, in the present invention, a nucleotide sequence (ORF) mentioned within a given functional group may also be part of another group taking into account, for example, the interrelationship between the groups listed. Accordingly, and as an example of this interrelationship, an exported and/or secreted polypeptide as well as its coding nucleotide sequence may also be involved in the *Chlamydia pneumoniae* virulence process by modifying the defense mechanism of the infected host cell, or a transmembrane polypeptide or its coding nucleotide sequence is also part of the polypeptides or coding nucleotide sequences of the cellular envelope.

The present invention also describes the cited nucleotide and/or polypeptide sequences recorded on a medium, called recording medium, whose type and nature facilitate the reading, the analysis and the exploitation of the said sequences. These media may of course also contain other information extracted from the present invention, such as in particular the analogies with already known sequences, such as those mentioned in Table 1 of the present description, and/or may contain, in addition, information relating to the nucleotide and/or polypeptide sequences of other microorganisms so as to facilitate the comparative analysis and the exploitation of the results obtained.

Among these recording media, computer-readable media, such as magnetic, optical, electrical and hybrid media such as, for example, floppy disks, CD-ROMs or recording cassettes, are preferred in particular.

The invention also describes nucleotide sequences which can be used as primer or probe, for the detection and/or amplification of nucleic acid sequences.

These nucleotide sequences may thus be used to amplify nucleotide sequences, in particular by the PCR technique (polymerase chain reaction) (Erlich, 1989; Innis et al., 1990; Rolfs et al., 1991, and White et al., 1997).

These oligodeoxyribonucleotide or oligoribonucleotide primers correspond to representative nucleotide fragments, and are advantageously at least 8 nucleotides, preferably at least 12 nucleotides, 15 nucleotides and still more preferably at least 20 nucleotides long.

Other techniques for amplifying the target nucleic acid may be advantageously used as alternatives to PCR.

These nucleotide sequences, in particular the primers, may also be used in other methods for amplifying a target nucleic acid, such as:
- the TAS (Transcription-based Amplification System) technique described by Kwoh et al. in 1989;
- the 3SR (Self-Sustained Sequence Replication) technique described by Guatelli et al. in 1990;
- the NASBA (Nucleic Acid Sequence Based Amplification) technique described by Kievitis et al. in 1991;
- the SDA (Strand Displacement Amplification) technique (Walker et al., 1992);
- the TMA (Transcription Mediated Amplification) technique.

These polynucleotides may also be used in techniques for amplifying or for modifying the nucleic acid serving as probe, such as:
- the LCR (Ligase Chain Reaction) technique described by Landegren et al. in 1988 and perfected by Barany et al. in 1991, which uses a thermostable ligase;
- the RCR (Repair Chain Reaction) technique described by Segev in 1992;
- the CPR (Cycling Probe Reaction) technique described by Duck et al. in 1990;
- the Q-beta-replicase amplification technique described by Miele et al. in 1983 and perfected in particular by Chu et al. in 1986, Lizardi et al. in 1988, and then by Burg et al. as well as by Stone et al. in 1996.

The present invention discloses both hybridization probes and primers. In general, the complementary probes should be of a length sufficient to form a stable hybrid complex with the target sequences . Primers, while complementary to the target sequences need not form stable hybridization complexes with the target sequences alone. Rather, primers form stable complexes with the target sequences in the presence of polymerase to permit extension of the primer.

In the case where the target polynucleotide to be detected is possibly an RNA, for example an mRNA, it will be possible to use, prior to the use of an amplification reaction with the aid of at least one primer to the use of a method of detection with the aid of at least one probe, a reverse transcriptase-type enzyme so as to obtain a cDNA from the RNA contained in the biological sample. The cDNA obtained will then serve as target for the primer(s) or the probe(s) used in the amplification or detection method.

The detection probe will be chosen so that it hybridizes with the target sequence or the amplicon generated from the target sequence. Such a detection probe will advantageously have as sequence a sequence of at least 12 nucleotides, in particular of at least 20 nucleotides, and preferably at least 100 nucleotides.

The invention also cites the nucleotide sequences which can be used as probe or primer labelled with a radioactive compound or with a nonradioactive compound.

The nonlabelled nucleotide sequences may be used directly as probes or primers; however, the sequences are generally labelled with a radioactive element (³²P, ³⁵S, ³H, ¹²⁵I) or with a nonradioactive molecule (biotin, acetylaminofluorene, digoxigenin, 5-bromo-deoxyuridine, fluorescein) so as to obtain probes which can be used in numerous applications.

Examples of nonradioactive labelling of nucleotide sequences are described, for example, in French patent No. 78,10975 or by Urdea et al. or by Sanchez-Pescador et al. in 1988.

In the latter case, one of the labelling methods described in patents FR-2 422 956 and FR-2 518 755 may also be used.

The hybridization technique may be performed in various ways (Matthews et al., 1988). The most common method consists in immobilizing the nucleic acid extracted from *Chlamydia pneumoniae* cells on a support (such as nitrocellulose, nylon, polystyrene) and in incubating, under well-defined conditions, the target nucleic acid immobilized with the probe. After hybridization, the excess probe is removed and the hybrid molecules formed are detected by the appropriate method (measurement of the radioactivity, of the fluorescence or of the enzymatic activity linked to the probe).

The invention also cites these nucleotide sequences, characterized in that they are covalently or noncovalently immobilized on a support.

According to another advantageous embodiment of the nucleic sequences according to the invention, the latter may be used immobilized on a support and may thus serve to capture, through specific hybridization, the target nucleic acid obtained from the biological sample to be tested. If necessary, the solid support is separated from the sample and the hybridization complex formed between the so-called capture probe and the target nucleic acid is then detected by means of a second probe, called detection probe, labelled with an easily detectable element.

The nucleotide sequences according to the invention may also be used in new analytical systems, DNA chips, which allow sequencing, the study of mutations and of the expression of genes, and which are currently of interest given their very small size and their high capacity in terms of number of analyses.

The principle of the operation of these chips is based on molecular probes, most often oligonucleotides, which are attached onto a miniaturized surface, generally of the order of a few square centimetres. During an analysis, a sample containing fragments of a target nucleic acid to be analysed, for example DNA or RNA labelled, for example, after amplification, is deposited onto the DNA chip in which the support has been coated beforehand with probes. Bringing the labelled target sequences into contact with the probes leads to the formation, through hybridization, of a duplex according to the rule of pairing defined by J.D. Watson and F. Crick. After a washing step, analysis of the surface of the chip allows the effective hybridizations to be located by means of the signals emitted by the labels tagging the target. A hybridization fingerprint results from this analysis which, by appropriate computer processing, will make it possible to determine information such as the presence of specific fragments in the sample, the determination of sequences and the presence of mutations.

The chip consists of a multitude of molecular probes, precisely organized or arrayed on a solid support whose surface is miniaturized. It is at the centre of a system where other elements (imaging system, microcomputer) allow the acquisition and interpretation of a hybridization fingerprint.

The hybridization supports are provided in the form of flat or porous surfaces (pierced with wells) composed of various materials. The choice of a support is determined by its physicochemical properties, or more precisely, by the relationship between the latter and the conditions under which the support will be placed during the synthesis or the attachment of the probes or during the use of the chip. It is therefore necessary, before considering the use of a particular support (R.S. Matson et al., 1994), to consider characteristics such as its stability to pH, its physical strength, its reactivity and its chemical stability as well as its capacity to nonspecifically bind nucleic acids. Materials such as glass, silicon and polymers are commonly used. Their surface is, in a first step, called "functionalization", made reactive towards the groups which it is desired to attach thereon. After the functionalization, so-called spacer molecules are grafted onto the activated surface. Used as intermediates between the surface and the probe, these molecules of variable size render unimportant the surface properties of the supports, which often prove to be problematic for the synthesis or the attachment of the probes and for the hybridization.

Among the hybridization supports, there may be mentioned glass which is used, for example, in the method of in situ synthesis of oligonucleotides by photochemical addressing developed by the company Affymettix (E.L. Sheldon, 1993), the glass surface being activated by silane. Genosensor Consortium (P. Mérel, 1994) also uses glass slides carrying wells 3 mm apart, this support being activated with epoxysilane.

Polymers or silicon may also be mentioned among these hybridization supports. For example, the Andrein Mirzabekov team has developed a chip consisting of polyacrylamide squares polymerized on a silanized glass surface (G. Yershov et al., 1996). Several teams use silicon, in particular the IFOS laboratory of Ecole Centrale of Lyon which uses a silicon semiconductor substrate which is p-doped by introducing it into its crystalline structure atoms whose valency is different from that of silicon. Various types of metals, in particular gold and platinum, may also be used as support (Genosensor Consortium (K. Beattie et al., 1993)).

The probes may be synthesized directly in situ on the supports of the DNA chips. This in situ synthesis may be carried out by photochemical addressing (developed by the company Affymax (Amsterdam, Holland) and exploited industrially by its subsidiary Affymetrix (United States)) or based on the VLSIPS (very large scale immobilized polymer synthesis) technology (S.P.A. Fodor et al., 1991) which is based on a method of photochemically directed combinatory synthesis and the principle of which combines solid-phase chemistry, the use of photolabile protecting groups and photolithography.

The probes may be attached to the DNA chips in various ways such as electrochemical addressing, automated addressing or the use of probe printers (T. Livache et al., 1994; G. Yershov et al., 1996; J. Derisi et al., 1996, and S. Borman, 1996).

The revealing of the hybridization between the probes, deposited or synthesized in situ on the supports of the DNA chips, and the sample to be analysed, may be determined, for example, by measurement of fluorescent signals, by radioactive counting or by electronic detection.

The use of fluorescent molecules such as fluorescein constitutes the most common method of labelling the samples. It allows direct or indirect revealing of the hybridization and allows the use of various fluorochromes.

Affymetrix currently provides an apparatus or a scanner designed to read its Gene Chip^{™} chips. It makes it possible to detect the hybridizations by scanning the surface of the chip in confocal microscopy (RJ. Lipshutz et al., 1995). Other methods of detecting fluorescent signals have been tested: coupling of an epifluorescence microscope and a CCD camera (G. Yershov et al., 1996), the use of an optical fibre collecting system (E.L. Sheldon, 1993). A conventional method consists in carrying out an end labelling, with phosphorus 32, of the target sequences, by means of an appropriate apparatus, the Phosphorimager (marketed by Molecular Dynamics). The electronic detection is based on the principle that the hybridization of two nucleic acid molecules is accompanied by physical phenomena which can be quantified under certain conditions (system developed by Ecole Centrale of Lyon and called GEN-FET (GEN field effect transistor)). Genosensor Consortium and the company Beckman Instruments who are developing an electronic chip or Permittivity Chips^{™} may also be mentioned (K. Beattie et al., 1993).

The nucleotide sequences according to the invention may thus be used in DNA chips to carry out the analysis of mutations. This analysis is based on the production of chips capable of analysing each base of a nucleotide sequence according to the invention.

The nucleotide sequences according to the invention may also be used in DNA chips to carry out the analysis of the expression of the *Chlamydia pneumoniae* genes. This analysis of the expression of *Chlamydia pneumoniae* genes is based on the use of chips where probes of the invention, chosen for their specificity to characterize a given gene, are present (DJ. Lockhart et al., 1996; D.D. Shoemaker et al., 1996). For the methods of analysis of gene expression using the DNA chips, reference may, for example, be made to the methods described by DJ. Lockhart et al. (1996) and Sosnowsky et al. (1997) for the synthesis of probes in situ or for the addressing and the attachment of previously synthesized probes. The target sequences to be analysed are labelled and in general fragmented into sequences of about 50 to 100 nucleotides before being hybridized onto the chip. After washing as described, for example, by DJ. Lockhart et al. (1996) and application of different electric fields (Sosnowsky et al., 1997), the labelled compounds are detected and quantified, the hybridizations being carried out at least in duplicate. Comparative analyses of the signal intensities obtained with respect to the same probe for different samples and/or for different probes with the same sample, determine the differential expression of RNA or of DNA derived from the sample.

The nucleotide sequences according to the invention may, in addition, be used in DNA chips where other nucleotide probes specific for other microorganisms are also present, and may allow the carrying out of a serial test allowing rapid identification of the presence of a microorganism in a sample.

Accordingly, the subject of the invention is also the nucleotide sequences according to the invention, characterized in that they are immobilized on a support of a DNA chip.

The DNA chips, characterized in that they contain at least one nucleotide sequence according to the invention, immobilized on the support of the said chip, also form part of the invention.

The said chips will preferably contain several probes or nucleotide sequences of the invention of different length and/or corresponding to different genes so as to identify, with greater certainty, the specificity of the target sequences or the desired mutation in the sample to be analysed.

Accordingly, the analyses carried out by means of primers and/or probes, immobilized on supports such as DNA chips, will make it possible, for example, to identify, in samples, mutations linked to variations such as intraspecies variations. These variations may be correlated or associated with pathologies specific to the variant identified and will make it possible to select the appropriate treatment.

The invention also cites a DNA chip according to the invention, characterized in that it contains, in addition, at least one nucleotide sequence of a microorganism different from *Chlamydia pneumoniae,* immobilized on the support of the said chip; preferably, the different microorganism will be chosen from an associated microorganism, a bacterium of the *Chlamydia* family, and a variant of the species *Chlamydia pneumoniae.*

The present invention discloses a vector for the cloning and/or the expression of a sequence, characterized in that it contains a nucleotide sequence encoding a polypeptide of the cellular, preferably outer, envelope of *Chlamydia pneumoniae* or one of its representative fragments, are preferred. In a specific embodiment, the vectors contain a nucleotide sequence encoding a *Chlamydia pneumoniae* secreted polypeptide or one of its representative fragments or encoding a transport polypeptide, a surface exposed polypeptide, a lipoprotein or one of its representative fragments, a polypeptide involved in lipopolysaccharide (LPS) biosynthesis, a Type III and non-Type III secreted polypeptide, a polypeptide containing RGD attachment sites, a cell wall anchored surface polypeptide, a polypeptide not found in *Chlamydia trachomatis,* a ribosomal polypeptide or a polypeptide involved in secretion, transcription, translation, maturation of proteins, a polypeptide involved in the synthesis of the wall, a polypeptide involved in the virulence, a polypeptide involved in the intermediate metabolism, in particular in the metabolism of sugars and/or of cofactors, a polypeptide involved in the metabolism of nucleotides, of amino acids, of nucleic acids or of fatty acids of *Chlamydia pneumoniae* or one of their representative fragments, or a polypeptide specific to *Chlamydia pneumoniae.*

These vectors comprise the elements necessary to allow the expression and/or the secretion of the said nucleotide sequences in a given host cell. The vector should, in this case, comprise a promoter, signals for initiation and for termination of translation, as well as appropriate regions for regulation of transcription. It should be capable of being stably maintained in the host cell and may optionally possess particular signals specifying the secretion of the translated protein. These different elements are chosen according to the host cell used. To this effect, the nucleotide sequences according to the invention may be inserted into autonomously-replicating vectors within the chosen host, or integrative vectors in the chosen host.

Any of the standard methods known to those skilled in the art for the insertion of DNA fragments into a vector may be used to construct expression vectors containing a chimeric gene consisting of appropriate transcriptional/translational control signals and the protein coding sequences. These methods may include *in vitro* recombinant DNA and synthetic techniques and *in vivo* recombinants (genetic recombination).

Expression of a polypeptide, peptide or derivative, or analogs thereof encoded by a polynucleotide sequence in SEQ ID No. 1 or ORFs contained within SEQ ID No. I may be regulated by a second nucleic acid sequence so that the protein or peptide is expressed in a host transformed with the recombinant DNA molecule. For example, expression of a protein or peptide may be controlled by any promoter/enhancer element known in the art. Promoters which may be used to control expression include, but are not limited to, the CMV promoter, the SV40 early promoter region (Bernoist and Chambon, 1981, Nature 290:304-310), the promoter contained in the 3' long terminal repeat of Rous sarcoma virus (Yamamoto, *et al.,* 1980, Cell 22:787-797), the herpes thymidine kinase promoter (Wagner *et al.,* 1981, Proc. Natl. Acad. Sci. U.S.A. 78:1441-1445), the regulatory sequences of the metallothionein gene (Brinster *et al.,* 1982, Nature 296:39-42); prokaryotic expression vectors such as the 3-lactamase promoter (Villa-Kamaroff, *et al.,* 1978, Proc. Natl. Acad. Sci. U.S.A. 75:3727-3731), or the *tac* promoter (DeBoer, *et al.,* 1983, Proc. Natl. Acad. Sci. U.S.A. 80:21-25); see also "Useful proteins from recombinant bacteria" in Scientific American, 1980, 242:74-94; plant expression vectors comprising the nopaline synthetase promoter region (Herrera-Estrella *et al.,* 1983, Nature 303:209-213) or the cauliflower mosaic virus 35S RNA promoter (Gardner, *et al.,* 1981, Nucl. Acids Res. 9:2871), and the promoter of the photosynthetic enzyme ribulose biphosphate carboxylase (Herrera-Estrella *et al.,* 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift *et al.,* 1984, Cell 38:639-646; Omitz *et al.,* 1986, Cold Spring Harbor Symp. Quant Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl *et al.,* 1984, Cell 38:647-658; Adames *et al.,* 1985, Nature 318:533-538; Alexander *et al.,* 1987, Mol. Cell. Biol. 7:1436-1444), mouse mammary tumor virus control region which is active in testicular, breast, lymphoid and mast cells (Leder *et al.,* 1986, Cell 45:485-495), albumin gene control region which is active in liver (Pinkert *et al.,* 1987, Genes and Devel. 1:268-276), alpha-fetoprotein gene control region which is active in liver (Krumlauf *et al.,* 1985, Mol. Cell. Biol. 5:1639-1648; Hammer *et al.,* 1987, Science 235:53-58; alpha 1-antitrypsin gene control region which is active in the liver (Kelsey *et al.,* 1987, Genes and Devel. 1:161-171), beta-globin gene control region which is active in myeloid cells (Mogram *et al.,* 1985, Nature 315:338-340; Kollias *et al.,* 1986, Cell 46:89-94; myelin basic protein gene control region which is active in oligodendrocyte cells in the brain (Readhead *et al.,* 1987, Cell 48:703-712); myosin light chain-2 gene control region which is active in skeletal muscle (Sani, 1985, Nature 314:283-286), and gonadotropic releasing hormone gene control region which is active in the hypothalamus (Mason *et al.,* 1986, Science 234:1372-1378).

The vectors are, for example, vectors of plasmid or viral origin. In a specific embodiment, a vector is used that comprises a promoter operably linked to a protein or peptide-encoding a nucleic acid sequence in SEQ ID No. 1, or ORFs contained within SEQ ID No. 1, one or more origins of replication, and, optionally, one or more selectable markers (*e.g.,* an antibiotic resistance gene). Expression vectors comprise regulatory sequences that control gene expression, including gene expression in a desired host cell. Preferred vectors for the expression of the polypeptides of the invention include the pET-type plasmid vectors (Promega) or pBAD plasmid vectors (Invitrogen). Furthermore, these vectors are useful for transforming host cells so as to clone or express the nucleotide sequences of the invention.

Expression can also be achieved using targeted homologous recombination to activate *Chlamydia pneumoniae* genes present in the cloned genomic DNA. A heterologous regulatory element may be inserted into a stable cell line or cloned microorganism, such that it is operatively linked with an endogenous *Chlamydia pneumoniae* gene present in the cloned genome, using techniques, such as targeted homologous recombination, which are well known to those of skill in the art (*See, e.g.,* Chappel, U.S. Patent No. 4,215,051 and Skoultchi, WO 91/06667).

Expression vector/host cell systems containing inserts of polynucleotide sequences in SEQ ID No. 1 or ORFs within SEQ ID No. 1, which encode polypeptides, peptides or derivatives, or analogs thereof, can be identified by three general approaches: (a) nucleic acid hybridization, (b) presence or absence of "marker" gene functions, and (c) expression of inserted sequences. In the first approach, the presence of a polynucleotide sequence inserted in an expression vector can be detected by nucleic acid hybridization using probes comprising sequences that are homologous to an inserted polynucleotide sequence. In the second approach, the recombinant vector/host system can be identified and selected based upon the presence or absence of certain "marker" gene functions (*e.g.,* thymidine kinase activity, resistance to antibiotics, transformation phenotype, occlusion body formation in baculovirus, etc.) caused by the insertion of a polynucleotide sequence in the vector. For example, if the polynucleotide sequence in SEQ ID No. 1 or ORFs within SEQ ID No. 1 is inserted within the marker gene sequence of the vector, recombinants containing the insert can be identified by the absence of the marker gene function. In the third approach, recombinant expression vectors can be identified by assaying the product of the polynucleotide sequence expressed by the recombinant. Such assays can be based, for example, on the physical or functional properties of the expressed polypeptide in *in vitro* assay systems, *e.g.*, binding with antibody, promotion of cell proliferation.

Once a particular recombinant DNA molecule is identified and isolated, several methods known in the art may be used to propagate it. The clones identified may be introduced into an appropriate host cell by standard methods, such as for example lipofection, electroporation, and heat shock. Once a suitable host system and growth conditions are established, recombinant expression vectors can be propagated and prepared in quantity.

The invention also cites the host cells transformed by these vectors. These cells may be obtained by introducing into host cells a nucleotide sequence inserted into a vector as defined above, and then culturing the said cells under conditions allowing the replication and/or the expression of the transfected nucleotide sequence.

The host cell may be chosen from eukaryotic or prokaryotic systems, such as for example bacterial cells (Olins and Lee, 1993), but also yeast cells (Buckholz, 1993), as well as animal cells, in particular cultures of mammalian cells (Edwards and Aruffo, 1993), and in particular Chinese hamster ovary (CHO) cells, but also insect cells in which methods using baculoviruses for example may be used (Luckow, 1993).

Furthermore, a host cell strain may be chosen which modulates the expression of the inserted sequences, or modifies and processes the gene product in the specific fashion desired. Expression from certain promoters can be elevated in the presence of certain inducers; thus, expression of the genetically engineered polypeptide may be controlled. Furthermore, different host cells have characteristic and specific mechanisms for the translational and post-translational processing and modification (*e.g.,* glycosylation, phosphorylation) of proteins. Appropriate cell lines or host systems can be chosen to ensure the desired modification and processing of the foreign protein expressed. For example, expression in a bacterial system can be used to produce an unglycosylated core protein product. Expression in yeast will produce a glycosylated product. Expression in mammalian cells can be used to ensure "native" glycosylation of a heterologous protein. Furthermore, different vector/host expression systems may effect processing reactions to different extents.

A preferred host cell for the expression of the proteins consists of prokaryotic cells, such as Gram bacteria. A further preferred host cell is a bacterium belonging to the *Chlamydia* family, more preferably belonging to the species *Chlamydia pneumoniae* or chosen from a microorganism associated with the species *Chlamydia pneumoniae.*

In other specific embodiments, the polypeptides, peptides or derivatives, or analogs thereof may be expressed as a fusion, or chimeric protein product (comprising the protein, fragment, analog, or derivative joined via a peptide bond to a heterologous protein sequence (of a different protein)). Such a chimeric product can be made by ligating the appropriate nucleic acid sequences encoding the desired amino acid sequences to each other by methods known in the art, in the proper coding frame, and expressing the chimeric product by methods commonly known in the art. Alternatively, such a chimeric product may be made by protein synthetic techniques, *e.g.*, by use of a peptide synthesizer.

Genomic sequences can be cloned and expressed as translational gene products (*i.e.,* peptides, polypeptides, and proteins) or transcriptional gene products (*i.e.*, antisense and ribozymes).

The invention further cites the intracellular production of an antisense nucleic acid sequence of SEQ ID No. I by transcription from an exogenous sequence. For example, a vector can be introduced *in vivo* such that it is taken up by a cell, within which cell the vector or a portion thereof is transcribed, producing an antisense nucleic acid (RNA) of the invention. Such a vector would contain a sequence encoding an antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the an antisense RNA can be by any promoter known in the art to act in mammalian, preferably human, cells. Such promoters can be inducible or constitutive. Such promoters include but are not limited to: the CMV promoter, the SV40 early promoter region (Bernoist and Chambon, 1981, Nature 290:304-310), the promoter contained in the 3N long terminal repeat of Rous sarcoma virus (Yamamoto *et al.,* 1980, Cell 22:787-797), the herpes thymidine kinase promoter (Wagner *et al.,* 1981, Proc. Natl. Acad. Sci. U.SA. 78:1441-1445), the regulatory sequences of the metallothionein gene (Brinster *et al.,* 1982, Nature 296:39-42), etc.

In a specific embodiment, the antisense oligonucleotide comprises catalytic RNA, or a ribozyme (see, *e.g.,* PCT International Publication WO 90/11364, published October 4, 1990; Sarver *et al.,* 1990, Science 247:1222-1225). In another embodiment, the oligonucleotide is a 2N-0-methylribonucleotide (Inoue *et al.,* 1987, Nucl. Acids Res. 15:6131-6148), or a chimeric RNA-DNA analog (Inoue *et al.,* 1987, FEBS Lett. 215:327-330).

In another embodiment, the antisense nucleic acids comprise a sequence complementary to at least a portion of an RNA transcript of a polynucleotide sequence in SEQ ID No. 1. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acid sequence, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA transcribed from SEQ ID No. 1 may contain and still form a stable duplex (or triplex, as the case may be). One skilled in the art can ascertain a tolerable degree of mismatch by use of standard procedures to determine the melting point of the hybridized complex.

The invention also cites animals, except humans, comprising one of the above-described transformed cells.

The production of transgenic animals overexpressing one or more of the *Chlamydia pneumoniae* genes will be preferably carried out on rats, mice or rabbits according to methods well known to persons skilled in the art such as viral or nonviral transfections. The transgenic animals overexpressing one or more of the said genes may be obtained by transfection of multiple copies of the said genes under the control of a powerful promoter of a ubiquitous nature, or which is selective for one type of tissue. The transgenic animals may also be obtained by homologous recombination on embryonic stem cells, transfer of these stem cells to embryos, selection of the chimeras affected at the level of the reproductive lines, and growth of the said chimeras.

The transformed cells as well as the transgenic animals can be used in methods of preparing the recombinant polypeptide.

It is now possible to produce recombinant polypeptides in a relatively large quantity by genetic engineering using the cells transformed with expression vectors or using transgenic animals.

Are also disclosed methods of preparing a polypeptide as defined above in recombinant form, characterized in that they use a vector and/or a cell transformed with a vector and/or a transgenic animal comprising one of the said transformed cells.

Among the said methods of preparing such a polypeptide in recombinant form, the methods of preparation using a vector, and/or a cell transformed with the said vector and/or a transgenic animal comprising one of the said transformed cells, containing a nucleotide sequence encoding a polypeptide of the cellular envelope of *Chlamydia pneumoniae* or one of its representative fragments, more preferably encoding a polypeptide of the outer cellular envelope of *Chlamydia pneumoniae* or one of its fragment, are preferred.

Among the said methods of preparing such a polypeptide in recombinant form, the methods of preparation using a vector, and/or a cell transformed with the said vector and/or a transgenic animal comprising one of the said transformed cells, containing a nucleotide sequence encoding a *Chlamydia pneumoniae* secreted polypeptide or one of its representative fragments or encoding a transport polypeptide, a surface exposed polypeptide, a lipoprotein or one of its representative fragments, a polypeptide involved in lipopolysaccharide biosynthesis, a Type III or other secreted polypeptide, a polypeptide containing RGD attachment sites, a cell wall anchored surface polypeptide, a polypeptide not found in *Chlamydia trachomatis,* a ribosomal polypeptide or a polypeptide involved in secretion, transcription, translation, maturation of proteins, a polypeptide involved in the synthesis of the wall, a polypeptide involved in the virulence, a polypeptide involved in the intermediate metabolism, in particular in the metabolism of sugars and/or of cofactors, a polypeptide involved in the metabolism of nucleotides, of amino acids, of nucleic acids or of fatty acids of *Chlamydia pneumoniae* or one of their representative fragments, or a polypeptide specific to *Chlamydia pneumoniae,* are also preferred.

The recombinant polypeptides obtained as indicated above may be provided either in glycosylated or non-glycosylated form and may or may not have the natural tertiary structure.

A preferred variant consists in producing a recombinant polypeptide fused to a "carrier" protein (chimeric protein). The advantage of this system is that it allows a stabilization and a reduction in proteolysis of the recombinant product, an increase in solubility during renaturation in vitro and/or a simplification of purification when the fusion partner has affinity for a specific ligand.

More particularly, it is disclosed a method of preparing a polypeptide of the invention comprising the following steps:
a) culture of the transformed cells under conditions allowing the expression of a recombinant polypeptide having a nucleic acid sequence as defined above;
b) where appropriate, recovery of the said recombinant polypeptide.

When the method of preparing a polypeptide uses a transgenic animal, the recombinant polypeptide is then extracted from the said animal.

Is also cited a method of preparing a synthetic polypeptide, using an amino acid sequence of such a polypeptides.

Polypeptides as defined above may also be prepared by conventional techniques in the field of peptide synthesis under conditions suitable to produce the polypeptides encoded by the polynucleotide of the invention. This synthesis may be carried out in and recovered from a homogeneous solution or on a solid phase.

For example, the synthesis technique in a homogeneous solution described by Houbenweyl in 1974 may be used.

This method of synthesis consists in successively condensing, in pairs, the successive amino acids in the required order, or in condensing amino acids and fragments previously formed and already containing several amino acids in the appropriate order, or alternatively several fragments thus previously prepared, it being understood that care will have been taken to protect beforehand all the reactive functional groups carried by these amino acids or fragments, with the exception of the amine functional groups of one and the carboxyl functional groups of the other or vice versa, which should normally take part in the formation of the peptide bonds, in particular after activation of the carboxyl functional group, according to methods well known in peptide synthesis.

According to another preferred technique, the one described by Merrifield is used.

To manufacture a peptide chain according to the Merrifield method, a highly porous polymer resin is used, onto which the first C-terminal amino acid of the chain is attached. This amino acid is attached onto a resin via its carboxyl group and its amine functional group is protected. The amino acids which will constitute the peptide chain are thus attached, one after another, onto the amine group, each time deprotected beforehand, of the portion of the peptide chain already formed, and which is attached to the resin. When the entire peptide chain desired is formed, the protecting groups are removed from the various amino acids constituting the peptide chain and the peptide is detached from the resin with the aid of an acid.

The invention cites, in addition, hybrid (fusion) polypeptides having at least one polypeptide or one of its representative fragments as defined above and a sequence of a polypeptide capable of eliciting an immune response in humans or animals.

Advantageously, the antigenic determinant is such that it is capable of eliciting a humoral and/or cellular response. An antigenic determinant may be identified by screening expression libraries of the *Chlamydia pneumoniae* genome with antibodies contained in the serum of patients infected with a bacterium belonging to the species *Chlamydia pneumoniae.* An antigenic determinant may comprise a polypeptide or one of its representative fragments, according to the invention, in glycosylated form, used in order to obtain immunogenic compositions capable of inducing the synthesis of antibodies directed against multiple epitopes.

These hybrid molecules may consist, in part, of a carrier molecule for polypeptides or for their representative fragments according to the invention, combined with a portion which may be immunogenic, in particular an epitope of the diphtheria toxin, the tetanus toxin, a hepatitis B virus surface antigen (patent FR 79 21811), the poliomyelitis virus VP1 antigen or any other viral or bacterial toxin or antigen.

The methods of synthesizing the hybrid molecules include the methods used in genetic engineering to construct hybrid nucleotide sequences encoding the desired polypeptide sequences. Reference may be advantageously made, for example, to the technique for producing genes encoding fusion proteins described by Minton in 1984.

The polypeptides, the antibodies described below and the nucleotide sequences according to the invention may advantageously be used in *in vitro* and/or *in vivo* methods for the detection and/or the identification of bacteria belonging to the species *Chlamydia pneumoniae,* in a biological sample (biological tissue or fluid) which is likely to contain them. These methods, depending on the specificity of the polypeptides, of the antibodies and of the nucleotide sequences which will be used, may in particular detect and/or identify the bacterial variants belonging to the species *Chlamydia pneumoniae* as well as the associated microorganisms capable of being detected by the polypeptides, the antibodies and the nucleotide sequences which will be chosen. It may, for example, be advantageous to choose a polypeptide, an antibody or a nucleotide sequence, which is capable of detecting any bacterium of the Chlamydia family by choosing a polypeptide, an antibody and/or a nucleotide sequence which is specific to the family or, on the contrary, it will be most particularly advantageous to target a variant of the species *Chlamydia pneumoniae,* which is responsible, for example, for the induction or the worsening of pathologies specific to the targeted variant, by choosing a polypeptide, an antibody and/or a nucleotide sequence which is specific to the said variant.

The polypeptides as defined above may advantageously be used in a method for the detection and/or the identification of bacteria belonging to the species *Chlamydia pneumoniae* or to an associated microorganism, in a biological sample (biological tissue or fluid) which is likely to contain them, characterized in that it comprises the following steps:
a) bringing this biological sample into contact with such a polypeptide or one of its representative fragments (under conditions allowing an immunological reaction between the said polypeptide and the antibodies which may be present in the biological sample);
b) detecting the antigen-antibody complexes which may be formed.

Preferably, the biological sample consists of a fluid, for example a human or animal serum, blood or biopsies.

Any conventional procedure may be used to carry out such a detection of the antigen-antibody complexes which may be formed.

By way of example, a preferred method uses immunoenzymatic procedures based on the ELISA technique, immunofluorescence procedures or radioimmunological procedures (RIA), and the like.

Accordingly, the invention also cites polypeptides as defined above, labelled with the aid of a suitable label such as a label of the enzymatic, fluorescent or radioactive type.

Such methods comprise, for example, the following steps:
- deposition of defined quantities of such a polypeptide composition into the wells of a microtitre plate,
- introduction, into the said wells, of increasing dilutions of serum, or of a different biological sample as defined above, which has to be analysed,
- incubation of the microplate,
- introduction, into the wells of the microtitre plate, of labelled antibodies directed against human or animal immunoglobulins, these antibodies having been labelled with the aid of an enzyme selected from those which are capable of hydrolyzing a substrate, thereby modifying the absorption of the radiation of the latter, at least at a defined wavelength, for example at 550 nm.
- detection, by comparison with a control, of the quantity of substrate hydrolyzed.

The invention also discloses a kit or set for the detection and/or the identification of bacteria belonging to the species *Chlamydia pneumoniae* or to an associated microorganism, characterized in that it comprises the following components:
- a polypeptide as defined above,
- where appropriate, the reagents for constituting the medium appropriate for the immunological or specific reaction,
- the reagents allowing the detection of the antigen-antibody complexes produced by the immunological reaction between the polypeptide(s) of the invention and the antibodies which may be present in the biological sample, it being possible for these reagents also to carry a label, or to be capable of being recognized in turn by a labelled reagent, more particularly in the case where the polypeptide according to the invention is not labelled,
- where appropriate, a reference biological sample (negative control) free of antibodies recognized by a polypeptide according to the invention,
- where appropriate, a reference biological sample (positive control) containing a predetermined quantity of antibodies recognized by a polypeptide according to the invention.

The polypeptides, peptides, fusion proteins or other derivatives, or analogs thereof encoded by a polynucleotide sequence in SEQ ID No. 1, may be used as an immunogen to generate antibodies which immunospecifically bind such an immunogen. Such antibodies may include, but are not limited to, polyclonal and monoclonal antibodies, humanized or chimeric antibodies, single chain antibodies, Fab fragments, F(ab')₂ fragments, fragments produced by a Fab expression library, anti-idiotypic (anti-ld) antibodies, and epitope-binding fragments of any of the above. In a specific embodiment, the antibody to a polypeptide, peptide or other derivative, or analog thereof encoded by a polynucleotide sequence in SEQ ID No. 1 is a bispecific antibody (see generally, *e.g.* Fanger and Drakeman, 1995, *Drug News and Perspectives* 8: 133-137). Such a bispecific antibody is genetically engineered to recognize both (1) an epitope and (2) one of a variety of "trigger" molecules, *e.g.* Fc receptors on myeloid cells, and CD3 and CD2 on T cells, that have been identified as being able to cause a cytotoxic T-cell to destroy a particular target. Such bispecific antibodies can be prepared either by chemical conjugation, hybridoma, or recombinant molecular biology techniques known to the skilled artisan.

Various procedures known in the art may be used for the production of polyclonal antibodies to a polypeptide, peptide or other derivative, or analog thereof encoded by a polynucleotide sequence in SEQ ID No. 1. For the production of antibody, various host animals can be immunized by injection with a polypeptide, or peptide or other derivative, or analog thereof, including but not limited to rabbits, mice, rats, etc. Various adjuvants, depending on the host species, may be used to increase the immunological response, including but not limited to Stimulon^{™} QS-21 (Aquila Biopharmaceuticals, Inc., Framingham, MA), MPL^{™} (3-O-deacylated monophosphoryl lipid A; RIBI ImmunoChem Research, Inc., Hamilton, MT), aluminum phosphate, IL-12 (Genetics Institute, Cambridge, MA), Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, BCG (bacille Calmette-Guerin), and corynebacterium parvum. Alternatively, polyclonal antibodies may be prepared by purifying, on an affinity column onto which a polypeptide as defined above has been previously attached, the antibodies contained in the serum of patients infected with a bacterium belonging to the species *Chlamydia pneumoniae.*

For preparation of monoclonal antibodies directed toward a polypeptide, peptide or other derivative, or analog, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor *et al.*, 1983, Immunology Today 4:72), and the EBV-hybridoma technique to produce human monoclonal antibodies (Cole *et al.*, 1985, in Monoclonal Antibodies and Cancer Therapy, Alan R. Liss, Inc., pp. 77-96). In an additional embodiment, monoclonal antibodies can be produced in germ-free animals utilizing technology described in PCT/US90/02545. In another embodiment, transgenic non-human animals can be used for the production of human antibodies utilizing technology described in WO 98/24893 and WO 96/33735. Human antibodies may be used and can be obtained by using human hybridomas (Cote *et al.*, 1983, Proc. Natl. Acad. Sci. U.S.A. 80:2026-2030) or by transforming human B cells with EBV virus *in vitro* (Cole *et al.,* 1985, in Monoclonal Antibodies and Cancer Therapy, Alan R. Liss, pp. 77-96). In fact, techniques developed for the production of "chimeric antibodies" (Morrison *et al.,* 1984, PROC. NATL. ACAD. SCI. U.S.A. 81:6851-6855; Neuberger *et al.,* 1984, Nature 312:604-608; Takeda *et al.,* 1985, Nature 314:452-454) by splicing the genes from a mouse antibody molecule specific for a polypeptide, peptide or other derivative, or analog together with genes from a human antibody molecule of appropriate biological activity can be used.

Techniques described for the production of single chain antibodies (U.S. Patent 4,946,778) can be adapted to produce polypeptide or peptide-specific single chain antibodies. An additional embodiment utilizes the techniques described for the construction of Fab expression libraries (Huse *et al.,* 1989, Science 246:1275-1281) to allow rapid and easy identification of monoclonal Fab fragments with the desired specificity for polypeptides, derivatives, or analogs.

Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, the Fab fragments which can be generated by treating the antibody molecule with papain and a reducing agent, and Fv fragments.

In addition, techniques have been developed for the production of chimerized (*See,* e.g.**,** Boss, M. et al., U.S. Patent No. 4,816,397; and Cabilly, S. et al., U.S. Patent No. 5,585,089) humanized antibodies (*See*, e.g., Queen, U.S. Patent No. 5,585.089). An immunoglobulin light or heavy chain variable region consists of a "framework" region interrupted by three hypervariable regions, referred to as complementarily determining regions (CDRs). The extent of the framework region and CDRs have been precisely defined ( See, "Sequences of Proteins of Immunological Interest", Kabat, E. et al., U.S. Department of Health and Human Services (1983). Briefly, humanized antibodies are antibody molecules from non-human species having one or more CDRs from the non-human species and a framework from a human immunoglobulin molecule.

The antibodies may also be labelled in the same manner as described above for the nucleic probes such as an enzymatic, fluorescent or radioactive type labelling.

The invention discloses, in addition, a method for the detection and/or the identification of bacteria belonging to the species *Chlamydia pneumoniae* or to an associated microorganism in a biological sample, characterized in that it comprises the following steps:
a) bringing the biological sample (biological tissue or fluid) into contact with a mono- or polyclonal antibody as defined above (under conditions allowing an immunological reaction between the said antibodies and the polypeptides of the bacterium belonging to the species *Chlamydia pneumoniae* or to an associated microorganism which may be present in the biological sample, that is, under conditions suitable for the formation of immune complexes);
b) detecting the antigen-antibody complex which may be formed.

The invention also discloses a kit or set for the detection and/or the identification of bacteria belonging to the species *Chlamydia pneumoniae* or to an associated microorganism, characterized in that it comprises the following components:
- a polyclonal or monoclonal antibody as defined above, labeled where appropriate;
- where appropriate, a reagent for constituting the medium appropriate for carrying out the immunological reaction;
- a reagent allowing the detection of the antigen-antibody complexes produced by the immunological reaction, it being possible for this reagent also to carry a label, or to be capable of being recognized in turn by a labelled reagent, more particularly in the case where the said monoclonal or polyclonal antibody is not labelled;
- where appropriate, reagents for carrying out the lysis of the cells in the sample tested.

The principle of the DNA chip which was explained above may also be used to produce protein "chips" on which the support has been coated with a polypeptide or an antibody, or arrays thereof, in place of the DNA. These protein "chips" make it possible, for example, to analyze the biomolecular interactions (BIA) induced by the affinity capture of target analytes onto a support coated, for example, with proteins, by surface plasma resonance (SPR). Reference may be made, for example, to the techniques for coupling proteins onto a solid support which are described in EP 524 800 or to the methods describing the use of biosensor-type protein chips such as the BIAcore-type technique (Pharmacia) (Arlinghaus et al., 1997, Krone et al., 1997, Chatelier et al., 1995). These polypeptides or antibodies, capable of specifically binding antibodies or polypeptides derived from the sample to be analysed, may thus be used in protein chips for the detection and/or the identification of proteins in samples. The said protein chips may in particular be used for infectious diagnosis and may preferably contain, per chip, several polypeptides and/or antibodies of the invention of different specificity, and/or polypeptides and/or antibodies capable of recognizing microorganisms different from *Chlamydia pneumoniae.*

The invention cites, in addition, a protein chip as defined above, containing in addition, at least one polypeptide of a microorganism different from *Chlamydia pneumoniae* or at least one antibody directed against a compound of a microorganism different from *Chlamydia pneumoniae,* immobilized on the support of the said chip.

The invention also cites a kit or set for the detection and/or the identification of bacteria belonging to the species *Chlamydia pneumoniae* or to an associated microorganism, or for the detection and/or the identification of a microorganism comprising a protein chip as defined above.

The present invention also discloses a method for the detection and/or the identification of bacteria belonging to the species *Chlamydia pneumoniae* or to an associated microorganism in a biological sample, characterized in that it uses a nucleotide sequence as defined above.

More particularly, the invention discloses a method for the detection and/or the identification of bacteria belonging to the species *Chlamydia pneumoniae* or to an associated microorganism in a biological sample, characterized in that it comprises the following steps:
a) where appropriate, isolation of the DNA from the biological sample to be analysed, or optionally production of a cDNA from the RNA in the biological sample;
b) specific amplification of the DNA of bacteria belonging to the species *Chlamydia pneumoniae* or to an associated microorganism with the aid of at least one primer as defined above;
c) detection of the amplification products.

These may be detected, for example, by the molecular hybridization technique using a nucleic probe according to the invention. This probe will be advantageously labelled with a nonradioactive (cold probe) or radioactive element.

For the purposes of the present invention, "DNA in the biological sample" or "DNA contained in the biological sample" will be understood to mean either the DNA present in the biological sample considered, or optionally the cDNA obtained after the action of a reverse transcriptase-type enzyme on the RNA present in the said biological sample.

Another aim of the present invention consists in such a method, characterized in that it comprises the following steps.
a) bringing a nucleotide probe as defined above into contact with a biological sample, the DNA contained in the biological sample having, where appropriate, been previously made accessible to hybridization, under conditions allowing the hybridization of the probe to complementary base pairs of the DNA of a bacterium belonging to the species *Chlamydia pneumoniae* or to an associated microorganism;
b) detecting the hybridization complex formed between the nucleotide probe and the DNA in the biological sample.

The present invention also discloses such a method, characterized in that it comprises the following steps:
a) bringing a nucleotide probe immobilized on a support as defined above into contact with a biological sample, the DNA in the sample having, where appropriate, been previously made accessible to hybridization, under conditions allowing the hybridization of the probe to the DNA of a bacterium belonging to the species *Chlamydia pneumoniae* or to an associated microorganism;
b) bringing the hybrid formed between the nucleotide probe immobilized on a support and the DNA contained in the biological sample, where appropriate after removal of the DNA in the biological sample which has not hybridized with the probe, into contact with a labelled nucleotide probe according to the invention;
c) detecting the new hybrid formed in step b).

According to an advantageous embodiment of the method for the detection and/or the identification defined above, it is characterized in that, prior to step a), the DNA in the biological sample is primer-extended and/or amplified beforehand with the aid of at least one primer according to the invention.

The invention discloses, in addition, a kit or set for the detection and/or the identification of bacteria belonging to the species *Chlamydia pneumoniae* or to an associated microorganism, characterized in that it comprises the following components:
a) a nucleotide probe as defined above;
b) where appropriate, the reagents necessary for carrying out a hybridization reaction;
c) where appropriate, at least one primer according to the invention as well as the reagents e.g., polymerase and/or deoxynucleotide triphosphates) necessary for a DNA amplification reaction.

The invention also discloses a kit or set for the detection and/or the identification of bacteria belonging to the species *Chlamydia pneumoniae* or to an associated microorganism, characterized in that it comprises the following components.
a) a nucleotide probe, called capture probe, as defined above;
b) an oligonucleotide probe, called detection probe, according to the invention;
c) where appropriate, at least one primer according to the invention as well as the reagents (e.g., polymerase and/or deoxynucleotide triphosphates) necessary for a DNA amplification reaction.

The invention also discloses a kit or set for the detection and/or the identification of bacteria belonging to the species *Chlamydia pneumoniae* or to an associated microorganism, characterized in that it comprises the following components:
a) at least one primer as described above ;
b) where appropriate, the reagents necessary for carrying out a DNA amplification reaction;
c) where appropriate, a component which makes it possible to check the sequence of the amplified fragment, more particularly an oligonucleotide probe according to the invention.

The invention discloses, in addition, a kit or set for the detection and/or the identification of bacteria belonging to the species *Chlamydia pneumoniae* or to an associated microorganism, or for the detection and/or the identification of a microorganism characterized in that it comprises a DNA chip as defined above.

The invention also discloses such a method or a kit or set for the detection and/or the identification of bacteria belonging to the species *Chlamydia pneumoniae,* characterized in that the said primer and/or the said probe are chosen from the nucleotide sequences specific to the species *Chlamydia pneumoniae,* in that the said polypeptides are chosen from the polypeptides specific to the species *Chlamydia pneumoniae* and in that the said antibodies are chosen from the antibodies directed against the polypeptides chosen from the polypeptides specific to the species *Chlamydia pneumoniae.*

Preferably, the said method or the said kit or set above, for the detection and/or the identification of bacteria belonging to the species *Chlamydia pneumoniae* is characterized in that the said primer and/or the said probe or the said polypeptides are chosen from the nucleotide sequences or polypeptides which have been identified as being specific to the species *Chlamydia pneumoniae* and in that the said antibodies invention are chosen from the antibodies directed against the polypeptides chosen from the polypeptides identified as being specific to the species *Chlamydia pneumoniae.*

The invention cites, in addition, such a method or a kit or set for the diagnosis of predispositions to, or of a condition caused by, cardiovascular diseases, preferably linked to the presence of atheroma, which are induced or worsened by a *Chlamydia pneumoniae* infection.

The invention also cites such a method or a kit or set for the diagnosis of predispositions to, or of conditions caused by, respiratory diseases induced or worsened by a *Chlamydia pneumoniae* infection; preferably, the said respiratory disease is asthma.

According to another aspect, the invention cites the use of such polypeptides, of cells transformed with a vector and/or of transformed animals as defined above, for the biosynthesis or the biodegradation of organic or inorganic compounds.

As has been mentioned above, the nucleotide sequences were identified by homology with sequences known to encode, for example, polypeptides or fragments of enzymatic polypeptides involved in the biosynthesis or the biodegradation of organic or inorganic molecules.

It is thus possible to use the said polypeptides in a similar manner for the biosynthesis or the biodegradation of organic or inorganic compounds of industrial or therapeutic interest (called compounds of interest).

Among these polypeptides, there may be mentioned in particular the enzymes involved in metabolism, such as the proteolytic enzymes, amino transferases, glucose metabolism, or the enzymes which may be used in the biosynthesis of sugars, amino acids, fatty acids, polypeptides, nucleotides, nucleic acids or any other organic or inorganic compound or in the biodegradation of organic or inorganic compounds.

Among these polypeptides, there may be mentioned, in addition, the mutated or modified enzymes corresponding to mutated or modified polypeptides which may also be used for the biosynthesis or the biodegradation of organic or inorganic compounds at the industrial level, such as, for example, the production of compounds of interest, the reprocessing of manufacturing residues applied to the food industries, to the papermaking industry or to the chemical and pharmaceutical industries.

The invention cites, in addition, the use of a nucleotide sequence, of a polypeptide, of an antibody, of a cell and/or of a transformed animal as defined above, for the selection of an organic or inorganic compound capable of modulating, regulating, inducing or inhibiting the expression of genes, and/or of modifying the cellular replication of eukaryotic or prokaryotic cells or capable of inducing, inhibiting or worsening the pathologies linked to an infection by *Chlamydia pneumoniae* or one of its associated microorganisms.

The invention also comprises screening assays that comprise methods of selecting compounds, capable of binding to a nucleotide sequence according to the invention, characterized in that it comprises the following steps:
a) bringing the said compound into contact with said nucleotide sequence;
b) determining the capacity of the said compound to bind with the said nucleotide sequence.

The transformed cells and/or animals as defined above may advantageously serve as a model and may be used in methods for studying, identifying and/or selecting compounds capable of being responsible for pathologies induced or worsened by *Chlamydia pneumoniae,* or capable of preventing and/or of treating these pathologies such as, for example, cardiovascular or respiratory diseases. In particular, the transformed host cells, in particular bacteria of the *Chlamydia* family whose transformation with a vector may, for example, increase or inhibit its infectivity, or modulate the pathologies usually induced or worsened by the infection, may be used to infect animals in which the onset of pathologies will be monitored. These nontransfonned animals, infected for example with transformed *Chlamydia* bacteria, may serve as a study model. In the same manner, the transformed animals may, for example, exhibit predispositions to cardiovascular and/or respiratory diseases and thus be used in methods for selecting compounds capable of preventing and/or of treating the said diseases.

The compounds capable of being selected may be organic compounds such as polypeptides or carbohydrates or any other organic or inorganic compounds already known, or new organic compounds produced using molecular modeling techniques and obtained by chemical or biochemical synthesis, these techniques being known to persons skilled in the art.

The said selected compounds may be used to modulate the growth and/or the cellular replication of *Chlamydia pneumoniae* or any other associated microorganism and thus to control infection by these microorganisms. The said compounds according to the invention may also be used to modulate the growth and/or the cellular replication of all eukaryotic or prokaryotic cells, in particular tumour cells and infectious microorganisms, for which the said compounds will prove active, the methods which make it possible to determine the said modulations being well known to persons skilled in the art.

Compound capable of modulating the growth of a microorganism is understood to designate any compound which makes it possible to act, to modify, to limit and/or to reduce the development, the growth, the rate of proliferation and/or the viability of the said microorganism.

This modulation may be achieved, for example, by an agent capable of binding to a protein and thus of inhibiting or of potentiating its biological activity, or capable of binding to a membrane protein of the outer surface of a microorganism and of blocking the penetration of the said microorganism into the host cell or of promoting the action of the immune system of the infected organism directed against the said microorganism. This modulation may also be achieved by an agent capable of binding to a nucleotide sequence of a DNA or RNA of a microorganism and of blocking, for example, the expression of a polypeptide whose biological or structural activity is necessary for the growth or for the reproduction of the said microorganism.

Associated microorganism is understood to designate in the present invention any microorganism whose gene expression may be modulated, regulated, induced or inhibited, or whose growth or cellular replication may also be modulated by such a compound. Associated microorganism is also understood to designate in the present invention any microorganism containing nucleotide sequences or polypeptides as defined above. These microorganisms may, in some cases, contain polypeptides or nucleotide sequences identical or homologous to those disclosed in the invention may also be detected and/or identified by the detection and/or identification methods or kit as defined above and may also serve as a target for these compounds.

The invention also discloses a pharmaceutical composition comprising a compound chosen from the following compounds:
a nucleotide sequence as defined above;
a polypeptide as defined above;
a vector as defined above;
an antibody as defined above; and
a compound capable of being selected by a method of selection as defined above, optionally in combination with a pharmaceutically acceptable vehicle.

An effective quantity is understood to designate a sufficient quantity of the said compound or antibody, or of a polypeptide, which makes it possible to modulate the growth of *Chlamydia pneumoniae* or of an associated microorganism.

The invention also cites a pharmaceutical composition comprising one or more polypeptides and/or one or more fusion polypeptides as defined above. Such compositions further comprise a pharmaceutically acceptable carrier or vehicle. Pharmaceutical compositions include compositions that comprise a polypeptide or fusion polypeptide that immunoreacts with seropositive serum of an individual infected with *Chlamydia pneumoniae.* In one embodiment, such a pharmaceutical composition can be utilized for the prevention or the treatment of an infection by a bacterium belonging to the species *Chlamydia pneumoniae* or by an associated microorganism.

The invention disclosed, in addition, an immunogenic composition or a vaccine composition, characterized in that it comprises one or more polypeptides and/or one or more hybrid (fusion) polypeptides as defined above. Such compositions further comprise a pharmaceutically acceptable carrier or vehicle. Immunogenic compositions or fusion polypeptide include compositions that comprise a polypeptide that immunoreacts with seropositive serum of an individual infected with *Chlamydia pneumoniae.*

Immunogenic or vaccine compositions can also comprise DNA immunogenic or vaccine compositions comprising polynucleotide sequences operatively associated with a regulatory sequence that controls gene expression. Such compositions can include compositions that direct expression of a neutralizing epitope of *Chlamydia pneumoniae.*

The invention also cites the use of a transformed cell as defined above, for the preparation of a vaccine composition.

The invention also cites a vaccine composition, characterized in that it contains a nucleotide sequence, a vector and/or a transformed cell as defined above.

The invention also cites the vaccine compositions as defined above, for the prevention or the treatment of an infection by a bacterium belonging to the species *Chlamydia pneumoniae* or by an associated microorganism.

The invention also disclosed the use of DNA encoding polypeptides of *Chlamydia pneumoniae,* in particular antigenic determinants, to be formulated as vaccine compositions. In accordance with this aspect, the DNA of interest is engineered into an expression vector under the control of regulatory elements, which will promote expression of the DNA, *i.e.*, promoter or enhancer elements. In one preferred embodiment, the promoter element may be cell-specific and permit substantial transcription of the DNA only in predetermined cells. The DNA may be introduced directly into the host either as naked DNA (U.S. Patent No. 5,679,647) or formulated in compositions with other agents which may facilitate uptake of the DNA including viral vectors, *i.e.,* adenovirus vectors, or agents which facilitate immunization, such as bupivicaine and other local anesthetics (U.S. Patent 5,593,972), saponins (U.S. Patent 5,739.118) and cationic polyamines (published international application WO 96/10038).

The DNA sequence encoding the antigenic polypeptide and regulatory element may be inserted into a stable cell line or cloned microorganism, using techniques, such as targeted homologous recombination, which are well known to those of skill in the art, and described *e.g.,* in Chappel, U.S. Patent No. 4,215,051; Skoultchi, WO 91/06667.

Such cell lines and microorganisms may be formulated for vaccine purposes. In yet another embodiment, the DNA sequence encoding the antigenic polypeptide and regulatory element may be delivered to a mammalian host and introduced into the host genome via homologous recombination (*See,* Chappel, U.S. Patent No. 4,215,051; Skoultchi, WO 91/06667.

Preferably, the immunogenic and/or vaccine compositions intended for the prevention and/or the treatment of an infection by *Chlamydia pneumoniae* or by an associated microorganism will be chosen from the immunogenic and/or vaccine compositions comprising a polypeptide or one of its representative fragments corresponding to a protein, or one of its representative fragments, of the cellular envelope of *Chlamydia pneumoniae.* The vaccine compositions comprising nucleotide sequences will also preferably comprise nucleotide sequences encoding a polypeptide or one of its representative fragments corresponding to a protein, or one of its representative fragments, of the cellular envelope of *Chlamydia pneumoniae.*

Among these preferred immunogenic and/or vaccine compositions, the most preferred are those comprising a polypeptide or one of its representative fragments, or a nucleotide sequence or one of its representative fragments whose sequences are chosen from the nucleotide or amino acid sequences identified in this functional group and listed above.

The polypeptides of the invention or their representative fragments entering into the immunogenic compositions may be selected by techniques known to persons skilled in the art, such as for example on the capacity of the said polypeptides to stimulate T cells, which results, for example, in their proliferation or the secretion of interleukins, and which leads to the production of antibodies directed against the said polypeptides.

In mice, in which a weight dose of the vaccine composition comparable to the dose used in humans is administered, the antibody reaction is tested by collecting serum followed by a study of the formation of a complex between the antibodies present in the serum and the antigen of the vaccine composition, according to the customary techniques.

The said vaccine compositions will be preferably in combination with a pharmaceutically acceptable vehicle and, where appropriate, with one or more appropriate immunity adjuvants.

Various types of vaccines are currently available for protecting humans against infectious diseases: attenuated live microorganisms (*M. bovis* - BCG for tuberculosis), inactivated microorganisms (influenza virus), acellular extracts (*Bordetella pertussis* for whooping cough), recombinant proteins (hepatitis B virus surface antigen), polysaccharides (pneumococci). Experiments are underway on vaccines prepared from synthetic peptides or from genetically modified microorganisms expressing heterologous antigens. Even more recently, recombinant plasmid DNAs carrying genes encoding protective antigens were proposed as an alternative vaccine strategy. This type of vaccination is carried out with a particular plasmid derived from an *E*. *coli* plasmid which does not replicate *in vivo* and which encodes only the vaccinal protein. Animals were immunized by simply injecting the naked plasmid DNA into the muscle. This technique leads to the expression of the vaccine protein *in situ* and to a cell-type (CTL) and a humoral type (antibody) immune response. This double induction of the immune response is one of the main advantages of the technique of vaccination with naked DNA.

The vaccine compositions can be evaluated in *in vitro* and *in vivo* animal models prior to host, e.g., human, administration. For example, *in vitro* neutralization assays such as those described by Peterson et al. (1988) can be utilized. The assay described by Peterson et al. (1988) is suitable for testing vaccine compositions directed toward either *Chlamydia pneumoniae* or *Chlamydia trachomatis.*

Briefly, hyper-immune antisera is diluted in PBS containing 5% guinea pig serum, as a complement source. *Chlamydiae* (10⁴ IFU; infectious units) are added to the antisera dilutions. The antigen-antibody mixtures are incubated at 37EC for 45 minutes and inoculated into duplicate confluent Hep-2 or HeLa cell monolayers contained in glass vials (e.g., 15 by 45 mm), which have been washed twice with PBS prior to inoculation. The monolayer cells are infected by centrifugation at 1000X g for I hour followed by stationary incubation at 37E for 1 hour. Infected monolayers are incubated for 48 or 72 hours, fixed and stained with a *Chlamydiae* specific antibody, such as anti-MOMP for *C.trachomatis, etc.* IFUs are counted in ten fields at a magnification of 200X. Neutralization titer is assigned based on the dilution that gives 50% inhibition as compared to control monolayers/IFU.

The efficacy of vaccine compositions can be determined *in vivo* by challenging animal models of *Chlamydia pneumoniae* infection, e.g., mice or rabbits, with the vaccine compositions. For example, *in vivo* vaccine composition challenge studies can be performed in the murine model of *Chlamydia pneumonia* infection described by Moazed et al. (1997). Briefly, male homozygous apoE deficient and/or C57 BL/6J mice are immunized with vaccine compositions. Post-vaccination, the mice are mildly sedated by subcutaneous injection of a mixture of ketamine and xylazine, and inoculated intranasally with a total volume of 0.03-0.05 ml of organisms suspended in SPG medium or with SPG alone. The inoculations of *Chlamydia pneumoniae* are approximately 3x10⁷ IFU/mouse. The mice are inoculated with *Chlamydia pneumoniae* at 8, 10, and 12 weeks of age. Tissues are then collected from the lung, spleen, heart, etc. at 1-20 weeks after the first inoculation. The presence of organisms is scored using PCR, histology and immunocytochemistry, or by quantitative culture/IFU after tissue homogenization.

Alternatively, *in vivo* vaccine composition challenge studies can be performed in the rabbit model of *Chlamydia pneumoniae* described by Laitinen et al. (1997). Briefly, New Zealand white rabbits (5 months old) are immunized with the vaccine compositions. Post-vaccination, the rabbits are sedated with Hypnorm, 0.3 ml/Kg of body weight, intramuscularly, and inoculated intranasally with a total of 0.5 ml of *Chlamydia pneumoniae* suspended in SPG medium or with SPG alone. The inoculations of *Chlamydia pneumoniae* are approximately 3x10⁷ IFU/rabbit. The rabbits are reinfected in the same manner and with the same dose 3 weeks after the primary inoculation. Tissues are then collected 2 weeks after the primary infection and 1, 2, and 4 weeks after the reinfection. The presence of *Chlamydia pneumoniae* is scored using PCR, histology and immunocytochemistry, or by quantitative culture/IFU after tissue homogenization.

The vaccine compositions comprising nucleotide sequences or vectors into which the said sequences are inserted are in particular described in International Application No. WO 90/11092 and also in International Application No. WO 95/11307.

The nucleotide sequence constituting the vaccine composition may be injected into the host after having been coupled to compounds which promote the penetration of this polynucleotide inside the cell or its transport up to the cell nucleus. The resulting conjugates may be encapsulated into polymeric microparticles, as described in International Application No. WO 94/27238 (Medisorb Technologies International).

According to another embodiment of the vaccine composition, the nucleotide sequence, preferably a DNA, is complexed with the DEAE-dextran (Pagano et al., 1967) or with nuclear proteins (Kaneda et al., 1989), with lipids (Felgner et al., 1987) or encapsulated into liposomes (Fraley et al., 1980) or alternatively introduced in the form of a gel facilitating its transfection into the cells (Midoux et al., 1993, Pastore et al., 1994). The polynucleotide or the vector may also be in suspension in a buffer solution or may be combined with liposomes.

Advantageously, such a vaccine will be prepared in accordance with the technique described by Tacson et al. or Huygen et al. in 1996 or alternatively in accordance with the technique described by Davis et al. in International Application No. WO 95/11307.

Such a vaccine may also be prepared in the form of a composition containing a vector, placed under the control of regulatory elements allowing its expression in humans or animals. It is possible, for example, to use, as vector for the *in vivo* expression of the polypeptide antigen of interest, the plasmid pcDNA3 or the plasmid pcDNAI/neo, both marketed by Invitrogen ® & D Systems, Abingdon, United Kingdom). It is also possible to use the plasmid V1Jns.tPA, described by Shiver et al. in 1995. Such a vaccine will advantageously comprise, in addition to the recombinant vector, a saline solution, for example a sodium chloride solution.

The immunogenic compositions can also be utilized as part of methods for immunization, wherein such methods comprise administering to a host, e.g., a human host, an immunizing amount of the immunogenic compositions of the invention. In a preferred embodiment, the method of immunizing is a method of immunizing against *Chlamydia pneumoniae.*

A pharmaceutically acceptable vehicle is understood to designate a compound or a combination of compounds entering into a pharmaceutical or vaccine composition which does not cause side effects and which makes it possible, for example, to facilitate the administration of the active compound, to increase its life and/or its efficacy in the body, to increase its solubility in solution or alternatively to enhance its preservation. These pharmaceutically acceptable vehicles are well known and will be adapted by persons skilled in the art according to the nature and the mode of administration of the active compound chosen.

As regards the vaccine formulations, these may comprise appropriate immunity adjuvants which are known to persons skilled in the art, such as, for example, aluminum hydroxide, a representative of the family of muramyl peptides such as one of the peptide derivatives of N-acetyl-muramyl, a bacterial lysate, or alternatively incomplete Freund's adjuvant, Stimulon^{™} QS-21 (Aquila Biopharmaceuticals, Inc., Framingham, MA), MPL^{™} (3-O-deacylated monophosphoryl lipid A; RIBI ImmunoChem Research, Inc., Hamilton, MT), aluminum phosphate, IL-12 (Genetics Institute, Cambridge, MA).

Preferably, these compounds will be administered by the systemic route, in particular by the intravenous route, by the intranasal, intramuscular, intradermal or subcutaneous route, or by the oral route. More preferably, the vaccine composition comprising polypeptides according to the invention will be administered several times, spread out over time, by the intradermal or subcutaneous route.

Their optimum modes of administration, dosages and galenic forms may be determined according to criteria which are generally taken into account in establishing a treatment adapted to a patient, such as for example the patient's age or body weight, the seriousness of his general condition, tolerance of the treatment and the side effects observed.

The invention cites the use of such a composition for the treatment or the prevention of cardiovascular diseases, preferably linked to the presence of atheroma, which are induced or worsened by *Chlamydia pneumoniae.*

Finally, the invention cites the use of such composition for the treatment or the prevention of respiratory diseases which are induced or worsened by the presence of *Chlamydia pneumoniae,* preferably asthma.

Other characteristics and advantages of the invention appear in the following examples and figures:

### Legend to the figures :

- Figure 1 :: Line for the production of *Chlamydia pneumoniae* sequences
- Figure 2 :: Analysis of the sequences and assembling
- Figure 3 :: Finishing techniques
Figure 3a) : Assembly map
Figure 3b) : Determination and use of the orphan ends of the contigs

### EXAMPLES

### Experimental procedures

### Cells

The *Chlamydia pneumoniae* strain (CM1) used by the inventors is obtained from ATCC (American Culture Type Collection) where it has the reference number ATCC 1360-VR.

It is cultured on HeLa 229 cells, obtained from the American Type Culture Collection, under the reference ATCC CCL-2.1.

### Culture of the cells

The HeLa ATCC CCL-2.1 cells are cultured in 75-ml cell culture flasks (Coming). The culture medium is Dulbecco's modified cell culture medium (Gibco BRL No. 04101965) supplemented with MEM amino acids (Gibco BRL - No. 04301140) L (5 ml per 500 ml of medium) and 5% foetal calf serum (Gibco BRL No. 10270 batch 40G8260K) without antibiotics or antifungals.

The cell culture stock is maintained in the following manner. The cell cultures are examined under an inverted microscope. 24 hours after confluence, each cellular lawn is washed with PBS (Gibco BRL No. 04114190), rinsed and then placed for 5 min in an oven in the presence of 3 ml of trypsine (Gibco BRL No. 25200056). The cellular lawn is then detached and then resuspended in 120 ml of culture medium, the whole is stirred in order to make the cellular suspension homogeneous. 30 ml of this suspension are then distributed per cell culture flask. The flasks are kept in a CO₂ oven (5%) for 48 hours at a temperature of 37°C. The cell stock is maintained so as to have available daily 16 flasks of subconfluent cells. It is these subconfluent cells which will be used so as to be infected with Chlamydia. 25-ml cell culture flasks are also used, these flasks are prepared in a similar manner but the volumes used for maintaining the cells are the following: 1 ml of trypsine, 28 ml of culture medium to resuspend the cells, 7 ml of culture medium are used per 25-ml flask.

### Infection of the cells with Chiamydia

Initially, the Chlamydiae are obtained frozen from ATCC (-70°C), in suspension in a volume of 1 ml. This preparation is slowly thawed, 500 µl are collected and brought into contact with subconfluent cells, which are obtained as indicated above, in a 25-ml cell culture flask, containing 1 ml of medium, so as to cover the cells. The flask is then centrifuged at 2000 rpm in a "swing" rotor for microtitre plates, the centrifuge being maintained at a temperature of 35°C. After centrifugation, the two flasks are placed in an oven at 35°C for three hours. 6 ml of culture medium containing cycloheximide (1 µg/ml) are then added and the flask is stored at 35°C. After 72 hours, the level of infection is evaluated by direct immunofluorescence and by the cytopathogenic effect caused to the cells.

### Direct immunofluorescence

Starting with infected cells, which were obtained as indicated above, a cellular smear is deposited with a Pasteur pipette on a microscope slide. The cellular smear is fixed with acetone for 10 minutes; after draining the acetone, the smear is covered with 30 µl of murine monoclonal antibodies directed against MOMP (major outer membrane protein) of Chlamydia (Syva, Biomérieux) labelled with fluorescein isothiocyanate. The whole is then incubated in a humid chamber at a temperature of 37°C. The slides are then rinsed with water, slightly dried, and then after depositing a drop of mounting medium, a coverslip is mounted before reading. The reading is carried out with the aid of a fluorescence microscope equipped with the required filters (excitation at 490 nm, emission at 520 nm).

### Harvesting of the Chlamydia pneumoniae

After checking the infection by direct immunofluorescence, carried out as indicated above, the culture flasks are opened under a sterile cabinet, sterile glass beads with a diameter of the order of a millimeter are placed in the flask. The flask is closed and then vigorously stirred while being maintained horizontally, the cellular lawn at the bottom, so that the glass beads can have a mechanical action on the cellular lawn. Most of the cells are thus detached or broken; the effect of the stirring is observed under an optical microscope so as to ensure proper release of Chlamydiae.

### Large-scale infection of the cell cultures

The product of the Chlamydiae harvest (culture medium and cellular debris) is collected with a pipette, and distributed into three cell culture flasks containing subconfluent HeLa ATCC CCL-2.1 cells, obtained as indicated above. The cells thus inoculated are placed under gentle stirring (swing) in an oven at 35°C. After one hour, the flasks are kept horizontally in an oven so that the culture medium covers the cells for 3 hours. 30 ml of culture medium containing actydione (1 µg/ml) are then added to each of the flasks. The culture flasks are then stored at 35°C for 72 hours. The cells thus infected are examined under an optical microscope after 24 hours, the cytopathogenic effect is evaluated by the appearance of cytoplasmic inclusions which are visible under an inverted optical microscope. After 72 hours, the vacuoles containing the Chlamydiae occupy the cytoplasm of the cell and push the cell nucleus sideways. At this stage, numerous cells are spontaneously destroyed and have left free elementary bodies in the culture medium. The Chlamydiae are harvested as described above and are either frozen at -80°C or used for another propagation.

### Purification of the Chlamydiae

The product of the Chlamydia harvests is stored at -80°C and thawed on a water bath at room temperature. After thawing, each tube is vigorously stirred for one minute and immersed for one minute in an ultrasound tank (BRANSON 1200); the tubes are then stirred by inverting before being centrifuged for 5 min at 2000 rpm. The supernatant is carefully removed and kept at cold temperature (ice). The supernatant is vigorously stirred and then filtered on nylon filters having pores of 5 microns in diameter on a support (Nalgene) allowing a delicate vacuum to be established under the nylon filter. For each filtration, three nylon filters are superposed; these filters are replaced after every 40 ml of filtrate. Two hundred milliliters of filtration product are kept at cold temperature, and then after stirring by inverting, are centrifuged at 10,000 rpm for 90 min, the supernatant is removed and the pellet is taken up in 10 ml of 10 mM Tris, vigorously vortexed and then centrifuged at 10,000 rpm for 90 min. The supernatant is removed and the pellet is taken up in a buffer (20 mM Tris pH 8.0, 50 mM KCI, 5 mM MgCl₂) to which 800 units of DNAse I (Boehringer) are added. The whole is kept at 37°C for one hour. One ml of 0.5 M EDTA is then added, the whole is vortexed and frozen at -20°C.

### Preparation of the DNA

The Chlamydiae purified above are thawed and subjected to a proteinase K (Boehringer) digestion in a final volume of 10 ml. The digestion conditions are the following: 0.1 mg/ml proteinase K, 0.1 × SDS at 55EC, stirring every 10 min. The product of digestion is then subjected to a double extraction with phenol-chloroform, two volumes of ethanol are added and the DNA is directly recovered with a Pasteur pipette having one end in the form of a hook. The DNA is dried on the edge of the tube and then resuspended in 500 µl of 2 mM Tris pH 7.5. The DNA is stored at 4°C for at least 24 hours before being used for the cloning.

### Cloning of the DNA

After precipitation, the DNA is quantified by measuring the optical density at 260 nm. Thirty µg of Chlamydia DNA are distributed into 10 tubes of 1.5 ml and diluted in 300 µl of water. Each of the tubes is subjected to 10 applications of ultrasound lasting for 0.5 sec in a sonicator (unisonix XL2020). The contents of the 10 tubes are then grouped and concentrated by successive extractions with butanol (Sigma B1888) in the following manner: two volumes of butanol are added to the dilute DNA mixture. After stirring, the whole is centrifuged for five minutes at 2500 rpm and the butanol is removed. This operation is repeated until the volume of the aqueous phase is less than 1 ml. The DNA is then precipitated in the presence of ethanol and of 0.5 M sodium acetate pH 5.4, and then centrifuged for thirty minutes at 15,000 rpm at cold temperature (4°C). The pellet is washed with 75% ethanol, centrifuged for five minutes at 15,000 rpm and dried at room temperature. A tenth of the preparation is analysed on a 0.8% agarose gel. Typically, the size of the DNA fragments thus prepared is between 200 and 8000 base pairs.

To allow the cloning of the DNA obtained, the ends are repaired. The DNA is distributed in an amount of 10 µg/tube, in the following reaction medium: 100 µl final volume, 1 × buffer (Biolabs 201L), 0.5 µl BSA 0.05 mg/ml, 0.1 mM dATP, 0.1 mM each of dGTP, dCTP or dTTP, 60,000 IU T4 DNA polymerase. The reaction is incubated for thirty minutes at 16°C. The contents of each of the tubes are then grouped before carrying out an extraction with phenol-chloroform and then precipitating the aqueous phase as described above. After this step, the DNA thus prepared is phosphorylated. For that, the DNA is distributed into tubes in an amount of 10 µg per tube, and then in a final volume of 50 µl, the reaction is prepared in the following manner: 1 mM ATP, 1 × kinase buffer, 10 IU T4 polynucleotide kinase (Biolabs 201L). The preparation is incubated for thirty minutes at 37°C. The contents of the tubes are combined and a phenol-chloroform extraction and then a precipitation are carried out in order to precipitate the DNA. The latter is then suspended in 1 µl of water and then the DNA fragments are separated according to their size on a 0.8% agarose gel (1 × TAE). The DNA is subjected to an electric field of 5 V/cm and then visualized on a LTV table. The fragments whose size varies between 1200 and 2000 base pairs are selected by cutting out the gel. The gel fragment thus isolated is placed in a tube and then the DNA is purified with the Qiaex kit (20021 Qiagen), according to the procedure provided by the manufacturer.

### Preparation of the vector

14 µg of the cloning vector pGEM-5Zf (Proméga P2241) are diluted in a final volume of 150 µl and are subjected to digestion with the restriction enzyme EcoRV 300 IU (Biolabs 195S) according to the protocol and with the reagents provided by the manufacturer. The whole is placed at 37°C for 150 min and then distributed in the wells of a 0.8% agarose gel subjected to an electric field of 5 V/cm. The linearized vector is visualized on a UV table, isolated by cutting out the gel and then purified by the Qiaex kit (Qiagen 20021) according to the manufacturer's recommendations. The purification products are grouped in a tube, the volume is measured and then half the volume of phenol is added and the whole is vigorously stirred for 1 min. Half the volume of chloroform-isoamyl alcohol 24:1 is added and vigorously stirred for 1 min. The whole is centrifuged at 15,000 rpm for 5 min at 4°C, the aqueous phase is recovered and transferred into a tube. The DNA is precipitated in the presence of 0.3 M sodium acetate, pH 5.4 and 3 volumes of ethanol and placed at -20°C for 1 hour. The DNA is then centrifuged at 15,000 rpm for 30 min at 4°C, the supernatant is removed while preserving the pellet, washed twice with 70% ethanol. After drying at room temperature, the DNA is suspended in 25 µl of water.

### Phosphorylation of the vector

25 µl of the vector prepared in the preceding step are diluted in a final volume of 500 µl of the following reaction mixture:

After repair, the DNA is subjected to a phenol-chloroform extraction and a precipitation, the pellet is then taken up in 10 µl of water, the DNA is quantified by measuring the optical density at 260 nm. The quantified DNA is ligated into the vector PGEm-5Zf(+) prepared by the restriction enzyme EcoRV and dephosphorylated (see preparation of the vector). The ligation is carried out under three conditions which vary in the ratio between the number of vector molecules and the number of insert molecules. Typically, an equimolar ratio, a ratio of 1:3 and a ratio of 3:1 are used for the ligations which are, moreover, carried out under the following conditions: vector PGEm-5Zf(+) 25 ng, cut DNA, ligation buffer in a final volume of 20 µl with T4 DNA ligase (Amersham E70042X); the whole is then placed in a refrigerator overnight and then a phenol-chloroform extraction and a precipitation are carried out in a conventional manner. The pellet is taken up in 5 µl of water.

### Transformation of the bacteria

### Plating of the bacteria

Petri dishes containing LB Agar medium containing ampicillin (50 µg/ml), Xgal (280 µg/ml) [5-bromo-4-chloro-indolyl-beta-D-galactopyranoside (Sigma B-4252)], IPTG (140 µg/ml) [isopropyl-beta-D-thiogalactoside (Sigma I-6758)] are used, 50 and 100 µl of bacteria are plated for each of the ligations. The Petri dishes are placed upside down at 37°C for 15 to 16 hours in an oven. The number of "recombinant" positive clones is evaluated by counting the white colonies and the blue colonies which are thought to contain the vector alone.

### Evaluation of the "recombinant" positive clones

Ninety-four white colonies and two blue colonies are collected with the aid of sterile cones and are deposited at the bottom of the wells of plates designed for carrying out the amplification techniques. 30 µl of the following reaction mixture are added to each well: 1.7 mM MgCl₂, 0.2 mM each of dATP, dCTP, dGTP and dTTP, two synthetic oligonucleotides corresponding to sequences flanking the cloning site on either side and orienting the synthesis of the DNA in a convergent manner (0.5 µM RP and PU primers, 1 U TAQ polymerase (GibcoBRL 18038-026)).

The colonies thus prepared are subjected to a temperature of 94°C for 5 min and then to 30 thermal cycles composed of the following steps: 94°C for 40 s, 50°C for 30 s, 72°C for 180 s. The reaction is then kept for 7 min at 72°C and then kept at 4°C.

The amplification products are deposited on an agarose gel (0.8%), stained with ethidium bromide, subjected to electrophoresis, and then analysed on an ultraviolet table. The presence of an amplification fragment having a size greater than 500 base pairs indicates the presence of an insert. The bacterial clones are then prepared so as to study the sequence of their insert.

### Sequencing

To sequence the inserts of the clones obtained as above, these were amplified by PCR on bacteria cultures carried out overnight using the primers for the vectors flanking the inserts. The sequence of the ends of these inserts (on average 500 bases on each side) was determined by automated fluorescent sequencing on an ABI 377 sequencer, equipped with the ABI Prism DNA Sequencing Analysis software (version 2.1.2).

### Analysis of the sequences

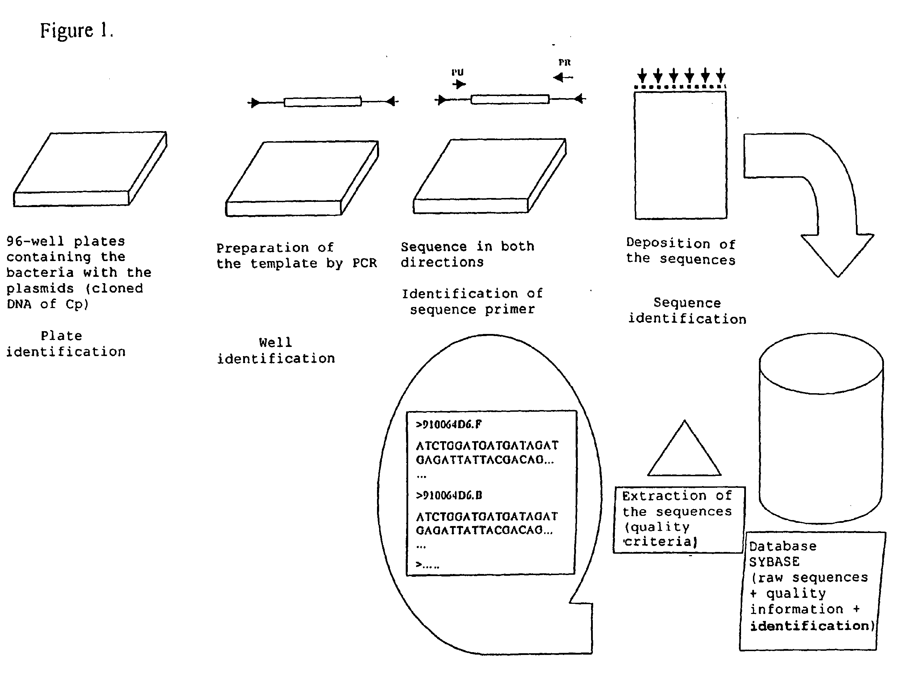

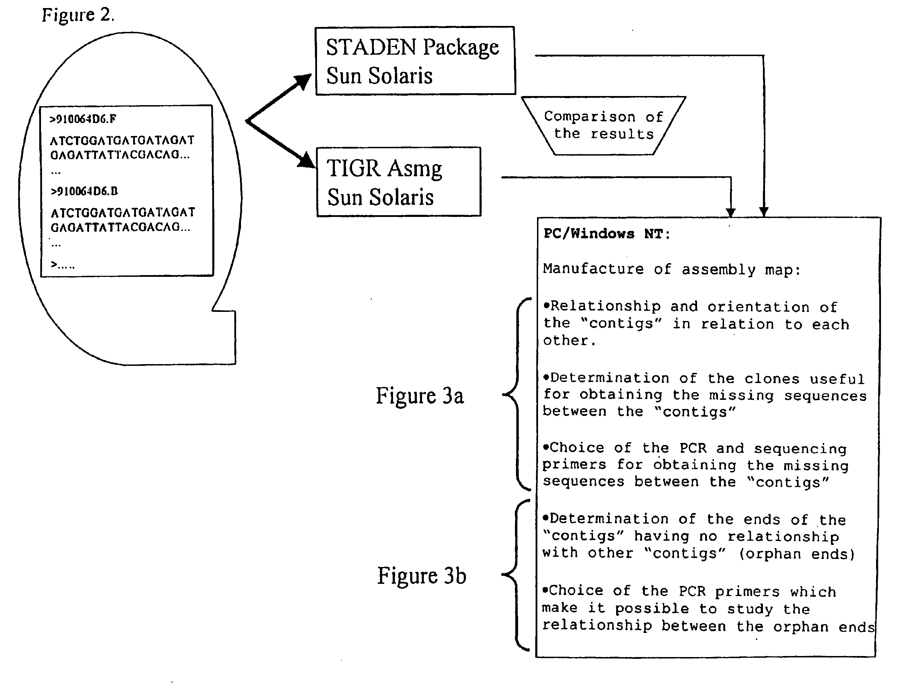

The sequences obtained by sequencing in a high-yield line (Figure 1) are stored in a database; this part of the production is independent of any treatment of the sequences. The sequences are extracted from the database, avoiding all the regions of inadequate quality, that is to say the regions for which uncertainties are observed on the sequence at more than 95%. After extraction, the sequences are introduced into a processing line, the diagram of which is described in Figure 2. In a first path of this processing line, the sequences are assembled by the Gap4 software from R. Staden (Bonfield et al., 1995) (OS UNIX/SUN Solaris); the results obtained by this software are kept in the form of two files which will be used for a subsequent processing. The first of these files provides information on the sequence of each of the contigs obtained. The second file represents all the clones participating in the composition of all the contigs as well as their positions on the respective contigs.

The second processing path uses a sequence assembler (TIGR-Asmg assembler UNIX/SUN Solaris); the results of this second processing path are kept in the form of a file in the TIGR-Asmg format which provides information on the relationship existing between the sequences selected for the assembly. This assembler is sometimes incapable of linking contigs whose ends overlap over several hundreds of base pairs.

The results obtained from these two assemblers are compared with the aid of the BLAST program, each of the contigs derived from one assembly path being compared with the contigs derived from the other path.

For the two processing paths, the strict assembly parameters are fixed (95% homology, 30 superposition nucleotides). These parameters avoid 3 to 5% of the clones derived from eukaryotic cells being confused with sequences obtained from the clones derived from *Chlamydia pneumoniae.* The eukaryotic sequences are however preserved during the course of this project; the strategy introduced, which is described below, will be designed, inter alia, not to be impeded by these sequences derived from contaminating clones.

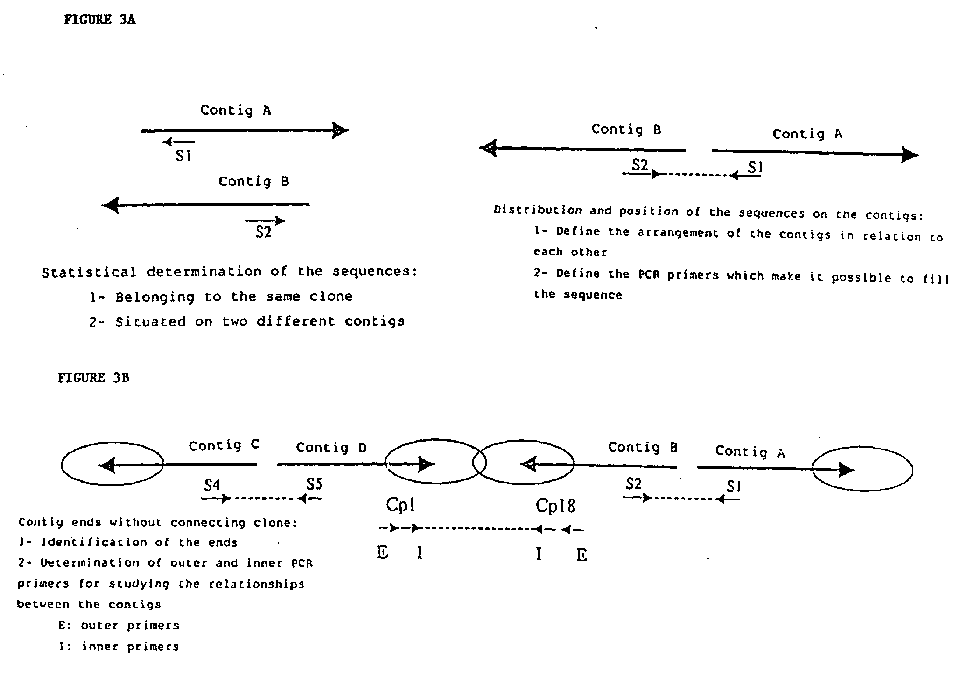

The results of these two assemblers are processed in a software developed for this project. This software operates on a Windows NT platform and receives, as data, the results derived from the STADEN software and/or the results derived from the TIGR-Asmg assembler, the software, results, after processing of the data, in the determination of an assembly map which gives the proximity relationship and the orientation of the contigs in relation to one another (Figure 3a). Using this assembly map, the software determines all the primers necessary for finishing the project. This treatment, which will be detailed below, has the advantage of distinguishing the isolated sequences derived from the contaminations, by the DNA eukaryotic cells, of the small-sized sequences clearly integrated into the project by the relationships which they establish with contigs. In order to allow, without any risk of error, the arrangement and the orientation of the contigs in relation to one another, a statistical evaluation of the accuracy of the names (naming) "naming" of sequence is made from the results of "contigation". This evaluation makes it possible to give each of the clone plates, as well as each of the subsets of plates, a weight which is inversely proportional to probable error rate existing in the "naming" of the sequences obtained from this plate or from a subset of this plate. In spite of a low error rate, errors may occur throughout the steps of production of the clones and of the sequences. These steps are numerous, repetitive and although most of them are automated, others, like the deposition in the sequencers, are manual; it is then possible for the operator to make mistakes such as the inversion of two sequences. This type of error has a repercussion on the subsequent processing of the data, by resulting in relationships (between the contigs) which do not exist in reality, then in attempts at directed sequencing between the contigs which will end in failure. It is because of this that the evaluation of the naming errors is of particular importance since it allows the establishment of a probabilistic assembly map from which it becomes possible to determine all the clones which will serve as template to obtain sequences separating two adjacent contigs. Table 2 of parent U.S. application serial No. 60/107078 filed November 4, 1998 and French application 97-14673 filed November 21, 1997 gives the clones and the sequences of the primers initially used during the initial operations.

To avoid the step which consists in ordering and then preparing the clones by conventional microbiological means, outer and inner primers oriented towards the regions not yet sequenced are defined by the software. The primers thus determined make it possible to prepare, by PCR, a template covering the nonsequenced region. It is the so-called outer primers (the ones most distant from the region to be sequenced) which are used to prepare this template. The template is then purified and a sequence is obtained on each of the two strands during 2 sequencing reactions which each use one of the 2 inner primers. In order to facilitate the use of this approach, the two outer primers and the two inner primers are prepared and then stored on the same position of 4 different 96-well plates. The two plates containing the outer primers are used to perform the PCRs which will serve to prepare the templates. These templates will be purified on purification columns preserving the topography of the plates. Each of the sequences will be obtained using primers situated on one and then on the other of the plates containing the inner primers. This distribution allows a very extensive automation of the process and results in a method which is simple to use for finishing the regions not yet sequenced. Table 3 of parent U.S. application serial No. 60/107078 filed November 4, 1998 and French application 97-14673 filed November 21, 1997 gives the names and the sequences of the primers used for finishing *Chlamydia pneumoniae.*

Finally, a number of contigs exist in a configuration where one of their ends is not linked to any other contig end (Figure 3b) by a connecting clone relationship (a connecting clone is defined as a clone having one sequence end on a contig and the other end of its sequence on another contig; furthermore, this clone must be derived from a plate or a subset of plates with adequate naming quality). For the *Chlamydia pneumoniae* project, this particular case occurred 24 times. Two adjacent PCR primers orienting the synthesis of the DNA towards the end of the consensus sequence are defined for each of the orphan ends of the consensus sequence. The primer which is closest to the end of the sequence is called the inner primer whereas the primer which is more distant from the end of the sequence is called the outer primer. The outer primers are used to explore the mutual relationship between the orphan ends of the different contigs. The presence of a single PCR product and the possibility of amplifying this product unambiguously using the inner primers evokes the probable relationship between the contigs on which the primers which allowed the amplification are situated. This relationship will be confirmed by sequencing and will allow the connection between the orphan ends of the consensus sequences. This strategy has made it possible to obtain a complete map of the *Chlamydia pneumoniae* chromosome and then to finish the project.

### Quality control

All the bases not determined with certainty in the chromosomal sequence were noted and the density of uncertainties was measured on the entire chromosome. The regions with a high density of uncertainties were noted and the PCR primers spanning these regions were drawn and are represented in Table 4 of parent U.S. application serial No. 60/107078 filed November 4, 1998 and French application 97-14673 filed November 21, 1997.

The sequence of each of the PCR products was obtained with two operational primers different from the amplification primers. The sequences were obtained in both directions for all the PCRs (100% success).

### Data banks

Local reorganizations of major public banks were used. The protein bank used consists of the nonredundant fusion of the Genpept bank (automated translation of GenBank, NCBI; Benson et al., 1996).

The entire BLAST software (public domain, Altschul et al., 1990) for searching for homologies between a sequence and protein or nucleic data banks was used. The significance levels used depend on the length and the complexity of the region tested as well as the size of the reference bank. They were adjusted and adapted to each analysis.

The results of the search for homologies between a sequence according to the invention and protein or nucleic data banks are presented and summarized in Table 1 below.

### Table 1: List of coding chromosome regions and homologies between these regions and the sequence banks.

Legend to Table 1: Open reading frames are identified with the GenMark software version 2.3A (GenePro), the template used is *Chlamydia pneumoniae* of order 4 on a length of 196 nucleotides with a window of 12 nucleotides and a minimum signal of 0.5. The reading frames ORF2 to ORF 1137 are numbered in order of appearance on the chromosome, starting with ORF2 (ORF column). The positions of the beginning and of the end are then given in column 2 (position). When the position of the beginning is greater than the position of the end, this means that the region is encoded by the strand complementary to the sequence which was given in the sequence SEQ ID No. 1.

All the putative products were subjected to a search for homology on GENPEPT (release 102 for ORF No. 2 to ORF No. 1137, and release 108 for ORF No. 1138 to ORF No. 1291 and ORF No. 6844 to ORF No. 6849) with the BLASTP software (Altschul et al. 1990). With as parameters, the default parameters with the exception of the expected value E set at 10⁻⁵ (for ORF No. 2 to ORF No. 1137) and P value set at e⁻¹⁰ (for ORF No. 1138 to ORF No. 1291 and ORF No. 6844 to ORF No. 6849). Subsequently, only the identities greater than 30% (1% column) were taken into account. The description of the most homologous sequence is given in the Homology column; the identifier for the latter sequence is given in the ID column and the animal species to which this sequence belongs is given in the Species column. The Homology score is evaluated by the sum of the blast scores for each region of homology and reported in the Score column.

### Materials and Methods for transmembrane domains:

The DAS software was used as recommended by the authors (Cserzo et al., 1997).

This method uses, to predict the transmembrane domains, templates derived from a sampling of selected proteins. All the regions for which a "Cutoff" greater than 1.5 was found by the program were taken into account.

### Additional ORF Finder Programs

For this analysis, two additional ORF finder programs were used to predict potential open reading frames of a minimum length of 74 amino acids; Glimmer (Salzberg, S.L., Delcher, A., Kasif, S., and W. White. 1998. Microbial gene identification using interpolated Markov models. Nucleic Acids Res. 26:544-548.), and an in-house written program. The in-house program used a very simple search algorithm. The analysis required the that the genomic DNA sequence text be in the 5' to 3' direction, the genome is circular, and that TAA, TAG, and TGA are stop codons. The search parameters were as follows:
(1) A search for an ORF that started with a GTG codon was performed. If no GTG codons were found, then a search for an ATG codon was performed. However, if a GTG codon was found, then a search downstream for a ATG codon was performed. All start and stop nucleotide positions were recorded.
(2) A search for an ORF that started with a TTG codon was performed. If no TTG codons were found, then a search for a ATG codon was performed. However, if a TTG codon was found, then a search downstream for a ATG codon was performed. All start and stop nucleotide positions were recorded.
(3) The analysis described in steps 1 and 2 were repeated for the opposite strand of DNA sequence.
(4) A search for ORFs that determined all ORF lengths using start and stop positions in the same reading frames was performed.
(5) All ORFs whose DNA length was less than 225 nucleotides were eliminated from the search.

### Surface Exposed Protein Search Criteria

Potential cell surface vaccine targets are outer membrane proteins such as porins, lipoproteins, adhesions and other non-integral proteins. In *Chlamydia psittaci,* the major immunogens is a group of putative outer membrane proteins (POMPs) and no homologs have been found in *Chlamydia pneumoniae* and *Chlamydia trachomatis* by traditional analysis (Longbottom, D., Russell, M., Dunbar, S.M., Jones, G.E., and A.J. Herring. 1998. Molecular Cloning and Characterization of the Genes Coding for the Highly Immunogenic Cluster of 90-Kilodalton Envelope Proteins from *Chlamydia psittaci* Subtype That Causes Abortion in Sheep. Infect Immun 66:1317-1324.) Several putative outer membrane proteins have been identified in *Chlamydia pneumoniae,* all of which may represent vaccine candidates. The major outer membrane protein (MOMP) gene (omp1) has been found in various isolates of *Chlamydia pneumoniae* (Jantos, CA., Heck, S., Roggendorf, R., Sen-Gupta, M., and Hegemann, JH. 1997. Antigenic and molecular analyses of different chlamydia pneumoniae strains. J. Clin Microbiology 35(3):620-623.) Various criteria, as listed below, were used to identify putative surface exposed ORFs from the genomic DNA sequence of *Chlamydia pneumoniae* (French application 97-14673 filed 21 November 1997). Any ORF which met any one or more of the individual criteria were listed in this category.

Protein homology searches were done using the Blastp 2.0 tool (Altschul, S.F., Madden, T.L., Schaffer, A.A., Zhang, J., Zhang, Z., Miller, W., and D.J. Lipman. 1997. Gapped BLAST and PSI-BLAST: a new generation of protein database search programs. Nucleic Acids Res. 25:3389-3402. ) An ORF product was labeled surface exposed if there was homology to a known, or hypothetical, or putative surface exposed protein with a P score better than e⁻¹⁰.

Most, if not all, proteins that are localized to the membrane of bacteria, via a secretory pathway, contain a signal peptide. A software program, SignalP, analyzes the amino acid sequence of an ORF for such a signal peptide (Nielsen, H., Engelbrecht. J., Brunak, S., and G. von Heijne. 1997. Identification of prokaryotic and eukaryotic signal peptides and prediction of their cleavage sites. Protein Engineering 10:1-6.) The first 60 N-terminal amino acids of each ORF were analyzed by SignalP using the Gram-Negative software database. The output generates four separate values, maximum C, maximum Y, maximum S, and mean S. The S-score, or signal region, is the probability of the position belonging to the signal peptide. The C-score, or cleavage site, is the probability of the position being the first in the mature protein. The Y-score is the geometric average of the C-score and a smoothed derivative of the S-score. A conclusion of either a Yes or No is given next to each score. If all four conclusions are Yes and the C-terminal amino acid is either a phenylalanine (F) or a tyrosine (Y), the ORF product was labelled outer membrane (Struyve, M., Moons, M., and J. Tommassen. 1991. Carboxy-terminal Phenylalanine is Essential for the Correct Assembly of a Bacterial Outer Membrane Protein. J. Mol. Biol. 218:141-148.)

The program called Psort, determines the localization of a protein based on its signal sequence, recognition of transmembrane segments, and analysis of its amino acid composition (Nakai, K., and M. Kanehisa. 1991. Expert system for predicting protein localization sites in gram-negative bacteria. Proteins 11:95-110.) An ORF product is considered to be an outer membrane protein if the output data predicts the protein as outer membrane with a certainty value of 0.5 or better and whose value is at least twice as large as the next predicted localized certainty value.

Finally, ORF products that were not predicted to be outer membrane or surface exposed, based on the above criteria, were further analyzed. The blastp output data for these ORFs were searched using various general and specific keywords, suggestive of known cell surface exposed proteins. An ORF was labeled surface exposed if the keywords matched had a Blastp hit, had a P score better than e⁻¹⁰, and that there was no better data indicating otherwise. The following is a list of the searched keywords:

| | | | | | |
|---|---|---|---|---|---|
| Adhesion | Adhesin | Invasin | Invasion | Extensin | |
| Omp | Outer Surface | Porin | Outer Membrane | | |
| Cell Surface | Cell Wall | Pilus | Pilin | Flagellar sheath | BtuB |
| Cir | ChuA | CopB | ExeD | FadL | FecA |
| FepA | FhuA | FmdC | FomA | FrpB | GspD |
| HemR | HgbA | Hgp | HmbR | HmuR | HMW |
| HrcC | Hrp | InvG | LamB | LbpA | LcrQ |
| Lmp1 | MxiD | MOMP | PilE | HpaA | NoIW |
| NspA | OpcP | OpnP | Opr | OspA | PhoE |
| PIdA | Por | PscC | PulD | PupA | QuiX |
| RafY | ScrY | SepC | ShuA | SomA | SpiA |
| Tbp1 | Yop | YscC | mip | Tol | |

Those ORFs that did not meet the minimum requirement for being an outer membrane protein based on the above search criteria but which were homologous to identified outer membrane ORFs in *Chlamydia trachomatis* were included. The *Chlamydia trachomatis* genome (French patent applications FR97-15041, filed 28 November 1997 and 97-16034 filed 17 December 1997) was analyzed using the above search criteria and a number of outer membrane ORFs were identified. These *Chlamydia trachomatis* ORFs were then tested against the *Chlamydia pneumoniae* genome using Blastp. Any *Chlamydia pneumoniae* ORF with a Blastp P value better than e⁻¹⁰ against a *Chlamydia trachomatis* outer membrane was included in this section, if there was no better data indicating otherwise. A list of ORFs in the *Chlamydia pneumoniae* genome encoding putative surface exposed proteins is set forth above in the specification.

### Identification of Putative Lipoproteins in the Genome of Chlamydia pneumoniae

Lipoproteins are the most abundant post-translationally modified bacterial secretory proteins (Pugsley, A. P.. 1993. The complete general secretory pathway in Gram-negative bacteria. Microbiol. Rev. 57:50-108). The characteristic features of lipoproteins are a thiol-linked diacylglyceride and an amine-linked monoacyl group on the cysteine that becomes the amino-terminal residue after signal peptide cleavage by Signal Peptidase II. (Pugsley, A. P.. 1993. The complete general secretory pathway in Gram-negative bacteria. Microbiol. Rev. 57:50-108). The identification of putative lipoproteins from the genomic sequencing of *Chlamydia pneumoniae* was done by examining the deduced amino acid sequence of identified ORFs for the presence of a signal peptide with a Signal Peptidase II cleavage site analogous to the consensus sequence for prolipoprotein modification and processing reactions (Hayashi, S., and H. C. Wu. 1992. Identification and characterization of lipid-modified proteins in bacteria, p. 261-285. *In* N. M. Hooper and A. J. Turner (ed.) Lipid modification of proteins: A practical approach. Oxford University Press, New York; Sutcliffe, I. C. and R. R. B. Russell. 1995. Lipoproteins of Gram-positive bacteria. J. Bacteriol. 177:1123-1128.).

*Chlamydia pneumoniae* ORFs were initially screened for the most basic of lipoprotein characteristics, a cysteine in the first 30 amino acids of the deduced protein. ORFs with a standard start codon (ATG, GTG, or TTG) and having one or more of the following characteristics were selected for direct analysis of their first 30 amino acids:
(a) Significant Signal P value (at least two out of the four values are Yes)
(b) PSORT value indicating membrane passage (IM-inner membrane, Peri-periplasm, or OM-outer membrane)
(c) Identification of the word lipoprotein among the ORF blastp data set.
(d) A Blastp value of <e⁻¹⁰ with a putative lipoprotein from *Chlamydia trachomatis* (French applications 97-15041 filed 28 November 1997 and 97-16034 filed 17 December 1997).

The first 30 amino acids of each ORF in this set were analyzed for the characteristics commonly found in lipoprotein signal peptides (Pugsley, A. P.. 1993. The complete general secretory pathway in Gram-negative bacteria. Microbiol. Rev. 57:50-108; Hayashi, S., and H. C. Wu. 1992. Identification and characterization of lipid- modified proteins in bacteria, p. 261-285. *In* N. M. Hooper and A. J. Turner (ed.) Lipid modification of proteins: A practical approach. Oxford University Press, New York; Sutcliffe, I. C. and R. R. B. Russell. 1995. Lipoproteins of Gram-positive bacteria. J. Bacteriol. 177:1123-1128.) Putative lipoprotein signal peptides were required to have a cysteine between amino acid 10 and 30 and reach a minimum score of three based on the following criteria for lipoprotein signal peptides:
(a) Identification of specific amino acids in specific positions around the cysteine which are part of the consensus Signal Peptidase II cleavage site (Hayashi, S., and H. C. Wu. 1992. Identification and characterization of lipid-modified proteins in bacteria, p. 261-285. *In* N. M. Hooper and A. J. Turner (ed.) Lipid modification of proteins: A practical approach. Oxford University Press, New York); Sutcliffe, I. C. and R. R. B. Russell. 1995. Lipoproteins of Gram-positive bacteria. J. Bacteriol. 177:1123-1128). Since the identification of the cleavage site is the most important factor in identifying putative lipoproteins, each correctly positioned amino acid contributed toward reaching the minimum score of three. (b) A hydrophobic region rich in alanine and leucine prior to the cleavage site (Pugsley, A. P.. 1993. The complete general secretory pathway in Gram-negative bacteria. Microbiol. Rev. 57:50-108) contributed toward reaching the minimum score of three.
(c) A short stretch of hydrophilic amino acids greater than or equal to 1 usually lysine or arginine following the N-terminal methionine (Pugsley, A. P.. 1993. The complete general secretory pathway in Gram-negative bacteria. Microbiol. Rev. 57:50-108) contributed toward reaching the minimum score of three.
A list of ORFs in the *Chlamydia pneumoniae* genome encoding putative lipoproteins is set forth above in the specification.

### LPS-Related ORFs of Chlamydia pneumoniae

Lipopolysaccharide (LPS) is an important major surface antigen of Chlamydia cells. Monoclonal antibodies (Mab) directed against LPS of *Chlamydia pneumoniae* have been identified that can neutralize the infectivity of *Chlamydia pneumoniae* both in vitro and in vivo (Peterson, E.M., de la Maza, L.M., Brade, L., Brade, H. 1998. Characterization of a Neutralizing Monoclonal Antibody Directed at the Lipopolysaccharide of *Chlamydia pneumonia.* Infect. Immun. Aug. 66(8):3848-3855.) Chlamydial LPS is composed of lipid A and a core oligosaccharide portion and is phenotypically of the rough type (R-LPS) (Lukacova, M., Baumann, M., Brade, L., Mamat, U., Brade, H. 1994. Lipopolysaccharide Smooth-Rough Phase Variation in Bacteria of the Genus *Chlamydia.* Infect. Immun. June 62(6):2270-2276.) The lipid A component is composed of fatty acids which serve to anchor LPS in the outer membrane. The core component contains sugars and sugar derivatives such as a trisaccharide of 3-deoxy-D-manno-octulosonic acid (KDO) (Reeves, P.R., Hobbs, M., Valvano, M.A., Skumik, M., Whitfield, C., Coplin, D., Kido, N., Klena, J., Maskell, D., Raetz, C.R.H., Rick, P.D. 1996. *Bacterial Polysaccharide Synthesis and Gene Nomenclature* pp. 10071-10078, Elsevier Science Ltd.). The KDO gene product is a multifunctional glycosyltransferase and represents a shared epitope among the Chlamydia. For a review of LPS biosynthesis see, e.g., Schnaitman, C.A., Klena, J.D. 1993. Genetics of Lipopolysaccharide Biosynthesis in Enteric Bacteria. Microbiol. Rev. 57:655-682.

A text search of the ORF blastp results identified several genes that are involved in Chlamydial LPS production with a P score better than e⁻¹⁰. The following key-terms were used in the text search: KDO, CPS (Capsular Polysaccharide Biosynthesis), capsule, LPS, rfa, rfb, rfc, rfe, rha, rhl, core, epimerase, isomerase, transferase, pyrophosphorylase, phosphatase, aldolase, heptose, manno, glucose, 1pxB, fibronectin, fibrinogen, fucosyltransferase , lic, lgt, pgm, tolC, rol, ChoP, phosphorylcholine, waaF, PGL-Tb1. A list of ORFs in the *Chlamydia pneumoniae* genome encoding putative polypeptides involved in LPS biosynthesis is set forth above in the specification.

### Type III And Other Secreted Products

Type III secretion enables gram-negative bacteria to secrete and inject pathogenicity proteins into the cytosol of eukaryotic host cells (Hueck, C. J., 1998. Type III Protein Secretion Systems in Bacterial Pathogens of Animals and Plants. In Microbiology and Molecular Biology Reviews. 62:379-433.) These secreted factors often resemble eukaryotic signal transduction factors, thus enabling the bacterium to redirect host cell functions (Lee, C.A., 1997. Type III secretion systems: machines to deliver bacterial proteins into eukaryotic cells? Trends Microbiol. 5:148-156.) In an attempt to corrupt normal cellular functions, Chlamydial pathogenicity factors injected into the host cytosol will nonetheless, as cytoplasmic constituents be processed and presented in the context of the Major Histocompatibility Complex (MHC class I). As such, these pathogenicity proteins represent MHC class I antigens and will play an important role in cellular immunity. Also included in this set are secreted non-type III products that may play a role as vaccine components.

A text search of the ORF blastp results identified genes that are involved in *Chlamydia pneumoniae* protein secretion with a P score better than e⁻¹⁰. The following key-terms were used in the text search in an effort to identify surface localized or secreted products: Yop, Lcr, Ypk, Exo, Pcr, Pop, Ipa, Vir, Ssp, Spt, Esp, Tir, Hrp, Mxi, hemolysin, toxin, IgA protease, cytolysin, tox, hap, secreted and Mip.

*Chlamydia pneumoniae* ORFs that did not meet the above keyword search criteria, but have homologs *in Chlamydia trachomatis* that do meet the search criteria are included herein. The *Chlamydia trachomatis* genome (French patent applications FR97-15041, filed 28 November 1997 and 97-16034 filed 17 December 1997) was analyzed using the above search criteria and a number of ORFs were identified. These *Chlamydia trachomatis* ORFs were tested against the *Chlamydia pneumoniae* genome using Blastp. Any *Chlamydia pneumoniae* ORF with a Blastp P value < e⁻¹⁰ against a *Chlamydia trachomatis* homolog, identified using the above search criteria, was included. A list of ORFs in the *Chlamydia pneumoniae* genome encoding putative secreted proteins is in the specification.

### Chlamydia pneumoniae: RGD Recognition Sequence

Proteins that contain Arg-Gly-Asp (RGD) attachment site, together with integrins that serve as their receptor constitute a major recognition system for cell adhesion. The RGD sequence is the cell attachment site of a large number of adhesive extracellular matrix, blood, and cell surface proteins and nearly half of the known integrins recognize this sequence in their adhesion protein ligands. There are many RGD containing microbial proteins such as the penton protein of adenovirus, the coxsackie virus, the foot and mouth virus and pertactin, a 69 kDa (kilodalton) surface protein of *Bordetella pertussis,* that serve as ligands through which these microbes bind to integrins on the cell surfaces and gain entry into the cell. The following provides evidence supporting the importance of RGD in microbial adhesion:
a) The adenovirus penton base protein has a cell rounding activity and when penton base was expressed in E. coli, it caused cell rounding and cells adhered to polystyrene wells coated with the protein. Mutant analysis showed that both these properties required an RGD sequence. Virus mutants with amino acid substitutions in the RGD sequence, showed much less adherence to HeLa S3 cells, and also were delayed in virus reproduction (Bai, M., Harfe, B., and Freimuth, P. 1993. Mutations That Alter an RGD Sequence in the Adenovirus Type 2 Penton Base Protein Abolish Its Cell-Rounding Activity and Delay Virus Reproduction in Flat Cells. J. Virol. 67:5198-5205).
b) It has been shown that attachment and entry of coxsackie virus A9 to GMK cells were dependent on an RGD motif in the capsid protein VP1. VP1 has also been shown to bind αᵥβ₃ integrin, which is a vitronectin receptor (Roivainen, M., Piirainen, L., Hovi, T., Virtanen, I., Riikonen, T., Heino, J., and Hyypia, T. 1994. Entry of Coxsackievirus A9 into Host Cells: Specific Interactions with aᵥb₃ Integrin, the Vitronectin Receptor Virology, 203:357-65).
c) During the course of whooping cough, *Bordetella pertussis* interacts with alveolar macrophages and other leukocytes on the respiratory epithelium. Whole bacteria adheres by means of two proteins, filamentous hemagglutinin (FHA) and pertussis toxin. FHA interacts with two classes of molecules on macrophages, galactose containing glycoconjugates and the integrin CR3. The interaction between CR3 and FHA involves recognition of RGD sequence at the positions 1097-1099 in FHA (Relman, D., Tuomanen, E., Falkow, S., Golenbock, D. T., Saukkonen, K., and Wright, S. D. "Recognitition of a Bacterial Adhesin by an Integrin: Macrophage CR3 Binds Filamentous Hemagglutinin of Bordetella Pertussis." Cell, 61:1375-1382 (1990)).
d) Pertactin, a 69 kDa outer membrane protein of *Bordetella pertussis,* has been shown to promote attachment of Chinese hamster ovary cells (CHO). This attachment is mediated by recognition of RGD sequence in pertactin by integrins on CHO cells and can be inhibited by synthetic RGD containing peptide homologous to the one present in pertactin (Leininger, E., Roberts, M., Kenimer, J. G., Charles, I. G., Fairweather, N., Novotny, P., and Brennan, M. J. 1991. Pertactin, an Arg-Gly-Asp containing *Bordetella pertussis* surface protein that promotes adherence of mammalian cells Proc. Natl. Acad. Sci. USA, 88:345-349).
e) The RGD sequence is highly conserved in the VP1 protein of foot and mouth disease virus (FMDV). Attachment of FMDV to baby hamster kidney cells (BHK) has been shown to be mediated by VP1 protein via the RGD sequence. Antibodies against the RGD sequence of VP1 blocked attachment of virus to BHK cells (Fox, G., Parry, N. R., Barnett, P. V., McGinn, B., Rowland, D. J., and Brown, F. 1989. The Cell Attachment Site on Foot-and-Mouth Disease Virus Includes the Amino Acid Sequence RGD (Arginine-Glycine-Aspartic Acid) J. Gen. Virol., 70:625-637).

It has been demonstrated that bacterial adherence can be based on interaction of a bacterial adhesin RGD sequence with an integrin and that bacterial adhesins can have multiple binding site characteristic of eukaryotic extracellular matrix proteins. RGD recognition is one of the important mechanisms used by microbes to gain entry into eukaryotic cells.

The complete deduced protein sequence of the *Chlamydia pneumoniae* genome was searched for the presence of RGD sequence. There were a total of 54 ORFs that had one or more RGD sequences. Not all RGD containing proteins mediate cell attachment. It has been shown that RGD containing peptides that have proline immediately following the RGD sequence are inactive in cell attachment assays (Pierschbacher & Ruoslahti. 1987. Influence of stereochemistry of the sequence Arg-Gly-Asp-Xaa on binding specificity in cell adhesion. J. Biol. Chem. *262*:17294-98). ORFs that had RGD, with proline as the amino acid following the RGD sequence were excluded from the list. Also, RGD sequence may not be available at the surface of the protein or may be present in a context that is not compatible with integrin binding. Since not all RGD- containing proteins are involved in cell attachment, several other criteria were used to refine the list of RGD- containing proteins. A list of ORFs in the *Chlamydia pneumoniae* genome encoding polypeptides with RGD recognition sequence(s) is in the specification.

### Non-Chlamydia trachomatis ORFs

*Chlamydia pneumoniae* ORFs were compared to the ORFs in the *Chlamydia trachomatis* genome (French patent applications FR97-15041, filed 28 November 1997 and 97-16034 filed 17 December 1997) using Blastp. Any *Chlamydia pneumoniae* ORF with a Blastp P value worse than e ¹⁰ (i.e. >e⁻¹⁰) against *Chlamydia trachomatis* ORFs are included in this section. A list of ORFs in the *Chlamydia pneumoniae* genome which are not found in *Chlamydia trachomatis* is set forth above in the specification.

### Cell Wall Anchor Surface ORFs

Many surface proteins are anchored to the cell wall of Gram-positive bacteria via the conserved LPXTG motif (Schneewind, O., Fowler, A., and Faull, K.F. 1995. Structure of the Cell Wall Anchor of Surface Proteins in *Staphylococcus aureus.* Science 268:103-106). A search of the *Chlamydia pneumoniae* ORFs was done using the motif LPXTG. A list of ORFs in the *Chlamydia pneumoniae* genome encoding polypeptides anchored to the cell wall is in the specification.

### ATCC Deposits

Samples of *Chlamydia pneumoniae* were deposited with the American Type Culture Collection (ATCC), Rockville, Maryland, on November 19, 1998 and assigned the accession number 1360-VR. Cells can be grown, harvested and purified, and DNA can be prepared as discussed above. In order to enable recovery of specific fragments of the chromosome, one can run targeted PCR reactions, whose amplification products can then be sequenced and/or cloned into any suitable vector, according to standard procedures known to those skilled in the art.

In addition, a sample of three pools of clones covering chromosomal regions of interest were deposited with the American Type Culture Collection (ATCC), Rockville, Maryland, on November 19, 1998. Each pool of clones contains a series of clones. When taken together, the three pools in the sample cover a portion of the chromosome, with a redundancy of slightly more than two. The total number of clones in the sample is 196.

The clones cover the following three regions of interest:
(i) position 30,000 to 40,000 of SEQ ID No. 1, referred to as region A;
(ii) position 501,500 to 557,000 of SEQ ID No.1, referred to as region B; and
(iii) position 815,000 to 830,000 of SEQ ID No. 1, referred to as region C.

Table 4 lists groups of oligonucleotides to be used to amplify each of ORFs 2-1291 according to standard procedures known to those skilled in the art. Such oligonucleotides are referenced as Nos. 1292 to 6451. For each ORF, the following is referenced: one forward primer positioned 2,000 bp upstream of the beginning of the ORF; one forward primer positioned 200 bp upstream of the beginning of the ORF; one reverse primer positioned 2,000 bp downstream at the end of ORF, which is 2,000 bp upstream of the end site of the ORF on the complementary strand; and one reverse primer 200 bp downstream at the end of ORF, which is 200 bp upstream of the end site of the ORF on the complementary strand. The corresponding Nos. for the primers are listed in Table 4, where Fp is the proximal forward primer, Fd is the distal forward primer, Bp is the proximal reverse primer; and Bd is the distal reverse primer. The positions of the 5' ends of each of these primers on the nucleotide sequence of SEQ ID No. I are shown in Table 5.

Table 6 lists oligonucleotides (Nos. 6452-6843) to be used to amplify the inserts of each of the 196 clones present in the pooled sample according to standard procedures well known to those of skill in the art. These primers can also be utilized to amplify the chromosomal region corresponding to the region A, B or C within which the particular insert lies. Their positions are indicated in Table 7.

All publications and patent applications mentioned in this specification are herein incorporated by reference to the same extent as if each individual publication or patent application was specifically and individually indicated to be incorporated by reference.

| | | | **TABLE 1** | | | | |
|---|---|---|---|---|---|---|---|
| **ORF** | **Begin** | **End** | **Homology** | **ID** | **Species** | **Score** | **I%** |
| ORF2 | 42 | 794 | triosephosphate isomerase | L27492 | *Thermotoga maritima* | 567 | 54 |
| ORF3 | 1258 | 1614 | putative | | | | |
| ORF4 | 1807 | 2418 | polypeptide deformylase | D90906 | *Synechocystis sp.* | 316 | 40 |
| ORF5 | 3393 | 2491 | hypothetical protein | Z75208 | *Bacillus subtilis* | 338 | 42 |
| ORF6 | 3639 | 4067 | unknown | U87792 | *Bacillus subtilis* | 117 | 38 |
| ORF7 | 5649 | 4270 | putative | | | | |
| ORF8 | 7463 | 6012 | putative | | | | |
| ORF9 | 8051 | 8962 | putative | | | | |
| ORF10 | 9129 | 9959 | putative | | | | |
| ORF11 | 10687 | 10361 | putative | | | | |
| ORF12 | 10927 | 11232 | putative | | | | |
| ORF13 | 11246 | 12727 | amidase | U49269 | *Moraxella catarrhalis* | 1108 | 42 |
| ORF14 | 12691 | 14190 | PET112 | D90913 | *Synechocystis sp.* | 1044 | 46 |
| ORF15 | 14484 | 17249 | POMP91A | U65942 | *Chlamydia psittaci* | 1074 | 43 |
| ORF16 | 16039 | 15770 | putative | | | | |
| ORF17 | 17845 | 20853 | putative | | | | |
| ORF18 | 21137 | 22042 | putative | | | | |
| ORF19 | 22046 | 23476 | putative | | | | |
| ORF20 | 23681 | 26110 | putative | | | | |
| ORF21 | 26109 | 25861 | putative | | | | |
| ORF22 | 26241 | 26978 | putative | | | | |
| ORF23 | 26960 | 27754 | putative | | | | |
| ORF24 | 27747 | 28577 | putative | | | | |
| ORF25 | 28887 | 29492 | POMP91A | U65942 | *Chlamydia psittaci* | 180 | 39 |
| ORF26 | 29432 | 30028 | POMP91A | U65942 | *Chlamydia psittaci* | 361 | 51 |
| ORF27 | 30024 | 31472 | POMP91A | U65942 | *Chlamydia psittaci* | 879 | 54 |
| ORF28 | 31758 | 32288 | putative 98 kDa outer membrane protein | U72499 | *Chlamydia psittaci* | 144 | 43 |
| ORF29 | 32201 | 33991 | putative 98 kDa outer membrane protein | U72499 | *Chlamydia psittaci* | 1126 | 48 |
| ORF30 | 33852 | 34541 | putative 98 kDa outer membrane protein | U72499 | *Chlamydia psittaci* | 589 | 62 |
| ORF31 | 34783 | 36063 | POMP91B precursor | U65943 | *Chlamydia psittaci* | 469 | 46 |
| ORF32 | 36009 | 37529 | putative 98 kDa outer membrane protein | U72499 | *Chlamydia psittaci* | 1338 | 51 |
| ORF33 | 37881 | 39362 | putative 98 kDa outer membrane protein | U72499 | *Chlamydia psittaci* | 671 | 40 |
| ORF34 | 39418 | 39161 | putative | | | | |
| ORF35 | 39366 | 40715 | POMP90A precursor | U65942 | *Chlamydia psittaci* | 904 | 47 |
| ORF36 | 43076 | 41094 | putative | | | | |
| ORF37 | 43800 | 43066 | putative | | | | |
| ORF38 | 44828 | 43785 | putative | | | | |
| ORF39 | 45340 | 44753 | homologous to unidentified E. coli protein | M96343 | *Bacillus subtilis* | 136 | 44 |
| ORF40 | 45752 | 45372 | o530; This 530 aa orf is 33 pct identical (14 gaps) to 525 residues of an approx. 640 aa protein YHES HAEIN SW: P44808 | AE000184 | *Escherichia coli* | 269 | 43 |
| ORF41 | 46996 | 45701 | ABC transporter, ATP-binding protein (yheS) | AE000596 | *Helicobacter pylori* | 878 | 39 |
| ORF42 | 47961 | 47569 | putative | | | | |
| ORF43 | 48960 | 48040 | hypothetical protein | D64001 | *Synechocystis sp.* | 404 | 37 |
| ORF44 | 51452 | 50133 | Lon protease-like protein | X74215 | *Homo sapiens* | 1232 | 54 |
| ORF45 | 52606 | 51335 | unknown | Z54285 | *Schizosaccharomyces pombe* | 781 | 47 |
| ORF46 | 53684 | 53319 | putative | | | | |
| ORF47 | 54195 | 53746 | putative | | | | |
| ORF48 | 55278 | 56453 | heat-shock protein | U15010 | *Legionella pneumophila* | 975 | 45 |
| ORF49 | 56493 | 57266 | branched chain alpha-keto acid dehydrogenase E1-alpha | M97391 | *Bacillus subtilis* | 329 | 36 |
| ORF50 | 57297 | 58526 | branched chain alpha-keto acid dehydrogenase E1-beta | M97391 | *Bacillus subtilis* | 707 | 50 |
| ORF51 | 59851 | 58565 | putative | | | | |
| ORF52 | 61495 | 59924 | ComE | D90903 | *Synechocystis sp.* | 134 | 55 |
| ORF53 | 61324 | 62151 | putative | | | | |
| ORF54 | 62132 | 62470 | Hpr protein | X12832 | *Bacillus subtilis* | 136 | 36 |
| ORF55 | 62474 | 63733 | enzyme I (ptsI) | U32844 | *Haemophilus influenzae* | 381 | 35 |
| ORF56 | 63881 | 64186 | f831; This 831 aa orf is 46 pct identical (11 gaps) to 709 residues of an approx. 712 aa protein PT1A ECOLI SW: P32670 | AE000326 | *Escherichia coli* | 123 | 34 |
| ORF57 | 64611 | 64318 | ORF107 | X17014 | *Bacillus subtilis* | 128 | 33 |
| ORF58 | 65485 | 64673 | putative | | | | |
| ORF59 | 65999 | 65301 | dnaZX-like ORF put. DNA polymerase III | X06803 | *Bacillus subtilis* | 596 | 52 |
| ORF60 | 66244 | 67281 | putative | | | | |
| ORF61 | 67265 | 67699 | putative | | | | |
| ORF62 | 67703 | 68539 | putative | | | | |
| ORF63 | 68805 | 70736 | putative | | | | |
| ORF64 | 69172 | 68831 | putative | | | | |
| ORF65 | 70642 | 71142 | putative | | | | |
| ORF66 | 71325 | 72029 | putative | | | | |
| ORF67 | 72060 | 73637 | putative | | | | |
| ORF68 | 74061 | 76175 | YqfF | D84432 | *Bacillus subtilis* | 542 | 44 |
| ORF69 | 78351 | 77680 | porphobilinogen deaminase | D28503 | *Clostridium josui* | 262 | 42 |
| ORF70 | 79356 | 78355 | sms protein | D90914 | *Synechocystis sp.* | 736 | 52 |
| ORF71 | 79983 | 79693 | ribonuclease III (mc) | AE000579 | *Helicobacter pylori* | 98 | 33 |
| ORF72 | 80441 | 79938 | ORF3 | D64116 | *Bacillus subtilis* | 268 | 44 |
| ORF73 | 80475 | 80969 | putative | | | | |
| ORF74 | 81296 | 83080 | hypothetical protein | Y14079 | *Bacillus subtilis* | 893 | 38 |
| ORF75 | 83291 | 83932 | manganese superoxide dismutase | X77021 | *Caenorhabditis elegans* | 622 | 58 |
| ORF76 | 84005 | 84769 | acetyl-CoA carboxylase beta subunit (accD) | AE000604 | *Helicobacter pylori* | 602 | 50 |
| ORF77 | 84975 | 85244 | deoxyuridinetriphosphatase (dut) | U32776 | *Haemophilus influenzae* | 110 | 41 |
| ORF78 | 85123 | 85425 | deoxyuridine 5'-triphosphate nucleotidohydrolase (dut) | AE000596 | *Helicobacter pylori* | 265 | 68 |
| ORF79 | 85397 | 85903 | ORF2 | L26916 | *Pseudomonas aeruginosa* | 173 | 34 |
| ORF80 | 85909 | 86583 | enzyme IIANtr | U18997 | *Escherichia coli* | 170 | 42 |
| ORF81 | 86626 | 88065 | putative | | | | |
| ORF82 | 89257 | 91026 | putative | | | | |
| ORF83 | 91291 | 93030 | putative | | | | |
| ORF84 | 93295 | 94086 | putative | | | | |
| ORF85 | 95285 | 94707 | putative | | | | |
| ORF86 | 95667 | 96557 | putative | | | | |
| ORF87 | 96317 | 97456 | putative | | | | |
| ORF88 | 98435 | 97968 | putative | | | | |
| ORF89 | 99460 | 98426 | putative | | | | |
| ORF90 | 100144 | 101325 | elongation factor Tu | L22216 | *Chlamydia trachomatis* | 1917 | 95 |
| ORF91 | 101457 | 101720 | putative | | | | |
| ORF92 | 101704 | 102273 | transcription factor | L10348 | *Thermus aquaticus thermophilus* | 376 | 49 |
| ORF93 | 102356 | 102805 | ribosomal protein L11 | D13303 | *Bacillus subtilis* | 458 | 63 |
| ORF94 | 102835 | 103530 | ribosomal protein L1 | Z11839 | *Thermotoga maritima* | 642 | 51 |
| ORF95 | 103549 | 104058 | ribosomal protein L10 | M89911 | *Streptomyces antibioticus* | 82 | 31 |
| ORF96 | 104096 | 104491 | rpl12 (AA 1-128) | X53178 | *Synechocystis PCC6803* | 325 | 47 |
| ORF97 | 104601 | 108386 | DNA-directed RNA polymerase beta chain | X64172 | *Staphylococcus aureus* | 2740 | 52 |
| ORF98 | 108401 | 112054 | rpoC | V00339 | *Escherichia coli* | 2947 | 54 |
| ORF99 | 112033 | 112590 | acetylomithine deacetylase (EC 5.1.1.16) | M22622 | *Leptospira biflexa* | 514 | 62 |
| ORF100 | 112672 | 113682 | transaldolase | L19437 | *Homo sapiens* | 755 | 49 |
| ORF101 | 113726 | 114121 | putative | | | | |
| ORF102 | 114711 | 114136 | putative | | | | |
| ORF103 | 115267 | 115755 | putative | | | | |
| ORF104 | 115911 | 116543 | putative | | | | |
| ORF105 | 116736 | 118055 | ATPase alpha-subunit | X63855 | *Thermus aquaticus thermophilus* | 934 | 50 |
| ORF106 | 117968 | 118522 | adenosine triphosphatase A subunit | D50528 | *Acetabularia acetabulum* | 147 | 32 |
| ORF107 | 118530 | 119843 | V-ATPase B subunit | U96487 | *Desulfurococcus sp. SY* | 751 | 48 |
| ORF108 | 119816 | 120457 | putative | | | | |
| ORF109 | 120451 | 122430 | v-type Na-ATPase | X76913 | *Enterococcus hirae* | 264 | 35 |
| ORF110 | 122504 | 122950 | ATP synthase, subunit K | U67478 | *Methanococcus jannaschii* | 184 | 31 |
| ORF111 | 123528 | 126347 | valyl-tRNA synthetase | X05891 | *Escherichia coli* | 1679 | 49 |
| ORF112 | 126332 | 129166 | protein kinase-like protein | U19250 | *Streptomyces coelicolor* | 427 | 37 |
| ORF113 | 134690 | 129213 | UvrA | D49911 | *Thermus thermophilus* | 3107 | 41 |
| ORF114 | 134925 | 136382 | pyruvate kinase | U83196 | *Chlamydia trachomatis* | 1748 | 71 |
| ORF115 | 137870 | 136482 | HtrB protein | X61000 | *Escherichia coli* | 147 | 38 |
| ORF116 | 137899 | 138240 | putative | | | | |
| ORF117 | 138239 | 137928 | putative | | | | |
| ORF118 | 139558 | 138257 | putative | | | | |
| ORF119 | 140352 | 139516 | YbbP | AB002150 | *Bacillus subtilis* | 231 | 46 |
| ORF120 | 140498 | 141841 | cyanide insensitive terminal oxidase | Y10528 | *Pseudomonas aeruginosa* | 538 | 50 |
| ORF121 | 141855 | 142658 | cyanide insensitive terminal oxidase | Y10528 | *Pseudomonas aeruginosa* | 310 | 40 |
| ORF122 | 144258 | 143050 | putative | | | | |
| ORF123 | 145258 | 144494 | putative | | | | |
| ORF124 | 145454 | 146749 | product similar to E. coli PhoH protein | Z97025 | *Bacillus subtilis* | 836 | 47 |
| ORF125 | 147318 | 146767 | putative | | | | |
| ORF126 | 148261 | 147677 | putative | | | | |
| ORF127 | 149029 | 152157 | isoleucyl-tRNA synthetase | U04953 | *Homo sapiens* | 2361 | 52 |
| ORF128 | 154108 | 152201 | leader peptidase I | D90904 | *Synechocystis sp.* | 225 | 47 |
| ORF129 | 155135 | 154308 | putative | | | | |
| ORF130 | 155141 | 155467 | YtiA | AF008220 | *Bacillus subtilis* | 201 | 43 |
| ORF131 | 155703 | 156779 | orf 361; ranslated orf similarity to SW: RF1_SALTY peptide chain release factor of Salmonella typhimurium | X78969 | *Coxiella burnetii* | 863 | 59 |
| ORF132 | 156748 | 157635 | product similar to E.coli PRFA2 protein | Z49782 | *Bacillus subtilis* | 144 | 37 |
| ORF133 | 157653 | 158996 | Ffh | U82109 | *Thermus aquaticus* | 797 | 45 |
| ORF134 | 159363 | 159986 | tRNA (guanine-N1)-methyltransferase (trmD) | U32705 | *Haemophilus influenzae* | 545 | 49 |
| ORF135 | 159880 | 160446 | putative | | | | |
| ORF136 | 160477 | 160839 | ribosomal protein L19 | X72627 | *Synechocystis sp.* | 319 | 50 |
| ORF137 | 160898 | 161539 | putative protein highly homologous to E. coli RNase HII . | D32253 | *Magnetospirillum sp.* | 427 | 49 |
| ORF138 | 161527 | 162153 | 5'guanylate kinase (gmk) | U32848 | *Haemophilus influenzae* | 385 | 43 |
| ORF139 | 162144 | 162443 | putative | | | | |
| ORF140 | 162437 | 164098 | methionyl-tRNA synthetase | AB004537 | *Schizosaccharomyces pombe* | 861 | 54 |
| ORF141 | 165451 | 164228 | exodeoxyribonuclease V (recD) | U32811 | *Haemophilus influenzae* | 432 | 32 |
| ORF142 | 166349 | 165411 | putative | | | | |
| ORF143 | 166949 | 168442 | putative | | | | |
| ORF144 | 169416 | 171029 | putative | | | | |
| ORF145 | 170857 | 171459 | putative | | | | |
| ORF146 | 172652 | 173428 | putative biotin-protein ligase | Z97992 | *Schizosaccharomyces pombe* | 292 | 44 |
| ORF147 | 174626 | 173439 | putative | | | | |
| ORF148 | 174816 | 175613 | putative | | | | |
| ORF149 | 175598 | 175954 | putative | | | | |
| ORF150 | 175958 | 176935 | putative | | | | |
| ORF151 | 177708 | 176938 | orf 3'of chaperonin homolog hypB [Chlamydia psittaci, pigeon strain P-1041, Peptide Partial. 98 aa] | S40172 | *Chlamydia psittaci* | 376 | 74 |
| ORF152 | 177128 | 177376 | putative | | | | |
| ORF153 | 179472 | 177841 | putative | M69217 | *Chlamydia pneumoniae* | 2678 | 100 |
| ORF154 | 179822 | 179517 | putative | M69217 | *Chlamydia pneumoniae* | 498 | 99 |
| ORF155 | 181793 | 179943 | Pz-peptidase | D88209 | *Bacillus licheniformis* | 1088 | 38 |
| ORF156 | 182628 | 181876 | o247; This 247 aa orf is 51 pct identical (0 gaps) to 117 residues of an approx. 160 aa protein YPH7 CHRVI SW: P45371 | AE000174 | *Escherichia coli* | 401 | 42 |
| ORF157 | 184420 | 183074 | glutamate-1-semialdehyde 2,1-aminomutase | X53696 | *Escherichia coli* | 823 | 41 |
| ORF158 | 184988 | 184467 | ORF o211 | U28377 | *Escherichia coli* | 87 | 54 |
| ORF159 | 185483 | 185112 | hypothetical protein | D90906 | *Synechocystis sp.* | 91 | 33 |
| ORF160 | 185902 | 185483 | ribose 5-phosphate isomerase | U28377 | *Escherichia coli* | 111 | 41 |
| ORF161 | 186174 | 185839 | ribose 5-phosphate isomerase A (SP:P27252) | U32729 | *Haemophilus influenzae* | 190 | 46 |
| ORF162 | 187720 | 186587 | hypothetical | D83026 | *Bacillus subtilis* | 536 | 42 |
| ORF163 | 188318 | 190933 | ATP-dependent protease binding subunit | M29364 | *Escherichia coli* | 2010 | 53 |
| ORF164 | 191090 | 191635 | putative | | | | |
| ORF 165 | 191547 | 192743 | putative | | | | |
| ORF166 | 192969 | 193469 | putative | | | | |
| ORF167 | 194044 | 193610 | putative | | | | |
| ORF168 | 194196 | 195809 | unknown | Z84395 | *Mycobacterium tuberculosis* | 242 | 52 |
| ORF169 | 196088 | 198073 | DNA ligase (EC 6.5.1.2) | M24278 | *Escherichia coli* | 1317 | 46 |
| ORF170 | 198132 | 199454 | putative | | | | |
| ORF171 | 199351 | 202818 | putative | | | | |
| ORF172 | 204552 | 202999 | PcpB | U60175 | *Sphingomonas chlorophenolica* | 80 | 41 |
| ORF173 | 205648 | 204692 | putative | | | | |
| ORF174 | 205807 | 207327 | leucine tRNA synthetase | AF008220 | *Bacillus subtilis* | 1595 | 57 |
| ORF175 | 207182 | 207775 | leucyl-tRNA synthetase | X06331 | *Escherichia coli* | 363 | 51 |
| ORF176 | 207779 | 208267 | transfer RNA-Leu synthetase | M88581 | *Bacillus subtilis* | 285 | 43 |
| ORF177 | 208267 | 209577 | KDO transferase | Z31593 | *Chlamydia pneumoniae* | 2262 | 100 |
| ORF178 | 211807 | 211271 | KDO-transferase | X80061 | *Chlamydia psittaci* | 105 | 38 |
| ORF179 | 212188 | 211844 | putative | | | | |
| ORF180 | 214079 | 212448 | pyrophosphate-dependent phosphofructokinase beta subunit | Z32850 | *Ricinus communis* | 1003 | 45 |
| ORF181 | 214907 | 214083 | CinI | U44893 | *Butyrivibrio fibrisolvens* | 111 | 41 |
| ORF182 | 216154 | 215429 | putative | | | | |
| ORF183 | 216115 | 216678 | putative | | | | |
| ORF184 | 216728 | 217282 | putative | | | | |
| ORF185 | 217267 | 217866 | putative | | | | |
| ORF186 | 218593 | 218261 | putative | | | | |
| ORF187 | 219821 | 218994 | putative | | | | |
| ORF188 | 221382 | 220309 | putative | | | | |
| ORF189 | 222719 | 221433 | GMP synthetase | M10101 | *Escherichia coli* | 1151 | 48 |
| ORF190 | 223521 | 222724 | IMP dehydrogenase | X66859 | *Acinetobacter calcoaceticus* | 778 | 58 |
| ORF191 | 224499 | 225008 | putative | | | | |
| ORF192 | 225140 | 225559 | putative | | | | |
| ORF193 | 225555 | 226802 | putative | | | | |
| ORF194 | 227800 | 226892 | putative | | | | |
| ORF195 | 228335 | 228072 | putative | | | | |
| ORF196 | 229251 | 228643 | putative | | | | |
| ORF197 | 230983 | 229622 | YqhX | D84432 | *Bacillus subtilis* | 1386 | 56 |
| ORF198 | 231483 | 230983 | acetyl-CoA carboxylase biotin carboxyl carrier protein | U38804 | *Porphyra purpurea* | 199 | 52 |
| ORF199 | 232063 | 231509 | elongation factor P | D64001 | *Synechocystis sp.* | 282 | 32 |
| ORF200 | 232739 | 232053 | pentose-5-phosphate-3-epimerase | D90911 | *Synechocystis sp.* | 463 | 43 |
| ORF201 | 233166 | 234356 | putative | | | | |
| ORF202 | 233518 | 233165 | putative | | | | |
| ORF203 | 234536 | 235186 | ORF2 | L35036 | *Chlamydia psittaci* | 570 | 60 |
| ORF204 | 235379 | 236689 | putative | | | | |
| ORF205 | 236680 | 237618 | putative | | | | |
| ORF206 | 237521 | 238345 | putative | | | | |
| ORF207 | 238281 | 238973 | putative | | | | |
| ORF208 | 238871 | 240115 | putative | | | | |
| ORF209 | 240191 | 241564 | putative | | | | |
| ORF210 | 242281 | 241604 | YqiZ | D84432 | *Bacillus subtilis* | 379 | 39 |
| ORF211 | 242933 | 242274 | f222; This 222 aa orf is 48 pet identical (0 gaps) to 208 residues of an approx. 232 aa protein YCKA BACSU SW: P42399 | AE000284 | *Escherichia coli* | 382 | 45 |
| ORF212 | 243416 | 242976 | arginine repressor protein (argR) | U32800 | *Haemophilus influenzae* | 229 | 46 |
| ORF213 | 243500 | 244531 | sialoglycoprotease | U15958 | *Pasteurella haemolytica* | 565 | 53 |
| ORF214 | 244480 | 246021 | oligopeptide permease homolog AII | AF000366 | *Borrelia burgdorferi* | 457 | 34 |
| ORF215 | 246330 | 247811 | OppAIV | AF000948 | *Borrelia burgdoferi* | 453 | 35 |
| ORF216 | 247831 | 249174 | OppA gene product | X56347 | *Bacillus subtilis* | 255 | 37 |
| ORF217 | 249437 | 251038 | dciAE | X56678 | *Bacillus subtilis* | 469 | 37 |
| ORF218 | 251325 | 252212 | OppB gene product | X56347 | *Bacillus subtilis* | 652 | 42 |
| ORF219 | 253156 | 254007 | oligopeptidepermease | X89237 | *Streptococcus pyogenes* | 574 | 48 |
| ORF220 | 253974 | 254852 | ATP binding protein | L18760 | *Lactococcus lactis* | 433 | 40 |
| ORF221 | 255258 | 256094 | KDO-transferase | X80061 | *Chlamydia psittaci* | 106 | 46 |
| ORF222 | 256640 | 257455 | putative | | | | |
| ORF223 | 257502 | 258239 | 2-OXOGLUTARAT | A47930 | *Spinacia oleracea* | 636 | 52 |
| ORF224 | 257869 | 257501 | putative | | | | |
| ORF225 | 259248 | 260897 | pyrophosphate-fructose 6-phosphate 1-phosphotransferase beta-subunit | M55191 | *Solanum tuberosum* | 1055 | 44 |
| ORF226 | 262753 | 261788 | putative | | | | |
| ORF227 | 263059 | 262757 | putative | | | | |
| ORF228 | 264375 | 263182 | putative | | | | |
| ORF229 | 265985 | 264747 | putative | | | | |
| ORF230 | 266637 | 266059 | putative | | | | |
| ORF231 | 267338 | 266538 | putative | | | | |
| ORF232 | 267922 | 267473 | putative | | | | |
| ORF233 | 269647 | 270771 | tRNA guanine transglycosylase | L33777 | *Zymomonas mobilis* | 628 | 44 |
| ORF234 | 272777 | 273145 | ORF 4 | D00624 | *Bacteriophage chp1* | 100 | 41 |
| ORF235 | 273253 | 273636 | putative | | | | |
| ORF236 | 273705 | 273977 | putative | | | | |
| ORF237 | 276016 | 275717 | putative | | | | |
| ORF238 | 276439 | 276020 | putative | | | | |
| ORF239 | 276792 | 277253 | putative | | | | |
| ORF240 | 277318 | 277599 | putative | | | | |
| ORF241 | 278578 | 277877 | putative | | | | |
| ORF242 | 279258 | 278554 | FbpC | U33937 | *Neisseria gonorrhoeae* | 312 | 39 |
| ORF243 | 280435 | 279533 | putative | | | | |
| ORF244 | 281547 | 280849 | putative | | | | |
| ORF245 | 281696 | 282325 | CMP-2-keto-3-deoxyoctulosonic acid synthetase | U15192 | *Chlamydia trachomatis* | 637 | 63 |
| ORF246 | 282459 | 284069 | CTP synthetase | U15192 | *Chlamydia trachomatis* | 2000 | 68 |
| ORF247 | 284056 | 284517 | ORF3 | U15192 | *Chlamydia trachomatis* | 453 | 65 |
| ORF248 | 284606 | 285775 | glucose 6-phosphate dehydrogenase | U83195 | *Chlamydia trachomatis* | 1263 | 77 |
| ORF249 | 285592 | 285987 | glucose 6-phosphate dehydrogenase | U83195 | *Chlamydia trachomatis* | 519 | 79 |
| ORF250 | 286179 | 286976 | glucose-6-phosphate dehydrogenase isozyme | D88189 | *Actinobacillus actinomycetemcomitans* | 216 | 40 |
| ORF251 | 287583 | 287002 | putative | | | | |
| ORF252 | 287951 | 287451 | putative | | | | |
| ORF253 | 288499 | 288816 | putative | | | | |
| ORF254 | 289674 | 288505 | putative | | | | |
| ORF255 | 288839 | 289213 | putative | | | | |
| ORF256 | 289970 | 290254 | putative | | | | |
| ORF257 | 291931 | 292803 | gamma-D-glutamyl-L-diamino acid endopeptidase II | X64809 | *Bacillus sphaericus* | 95 | 39 |
| ORF258 | 293258 | 292755 | ScoS9 | U43429 | *Streptomyces coelicolor* | 233 | 45 |
| ORF259 | 293718 | 293272 | ribosomal protein L13 (rpL13) | U32823 | *Haemophilus influenzae* | 364 | 47 |
| ORF260 | 294630 | 293953 | glutamine transport ATP-binding protein Q | U67524 | *Methanococcus jannaschii* | 387 | 46 |
| ORF261 | 296153 | 294636 | putative | | | | |
| ORF262 | 294817 | 295068 | putative | | | | |
| ORF263 | 296354 | 297862 | conserved hypothetical protein | AE000586 | *Helicobacter pylori* | 641 | 46 |
| ORF264 | 298415 | 297879 | putative | | | | |
| ORF265 | 298777 | 298253 | putative | | | | |
| ORF266 | 299572 | 298781 | putative | | | | |
| ORF267 | 300487 | 299633 | putative | | | | |
| ORF268 | 301586 | 300702 | putative | | | | |
| ORF269 | 302440 | 301571 | putative | | | | |
| ORF270 | 302838 | 302437 | putative | | | | |
| ORF271 | 303335 | 302745 | putative | | | | |
| ORF272 | 304394 | 303852 | putative | | | | |
| ORF273 | 304606 | 305223 | f311; This 311 aa orf is 22 pct identical (13 gaps) to 186 residues of an approx. 488 aa protein YACA_BACSU SW: P37563; pyu1 ofD21139 | AE000232 | *Escherichia coli* | 250 | 38 |
| ORF274 | 305394 | 306236 | survival protein surE | U81296 | *Sinorhizobium meliloti* | 156 | 42 |
| ORF275 | 306501 | 307439 | YqfU | D84432 | *Bacillus subtilis* | 547 | 42 |
| ORF276 | 308033 | 307458 | 3-octaprenyl-4-hydroxybenzoate carboxylyase | U61168 | *Bacillus firmus* | 403 | 42 |
| ORF277 | 308924 | 308037 | 4-hydroxybenzoate octaprenyltransferase | U61168 | *Bacillus firmus* | 152 | 40 |
| ORF278 | 309485 | 310180 | putative | | | | |
| ORF279 | 310426 | 311214 | putative | | | | |
| ORF280 | 311597 | 311253 | putative | | | | |
| ORF281 | 312772 | 311780 | putative | | | | |
| ORF282 | 313425 | 312772 | putative | | | | |
| ORF283 | 313646 | 313377 | putative | | | | |
| ORF284 | 313937 | 314665 | lysophospholipase homolog | AF006678 | *Schistosoma mansoni* | 141 | 44 |
| ORF285 | 315576 | 314755 | dnaZX | X17014 | *Bacillus subtilis* | 154 | 39 |
| ORF286 | 316157 | 315531 | unknown | D26185 | *Bacillus subtilis* | 284 | 31 |
| ORF287 | 318657 | 316156 | DNA gyrase | L47978 | *Aeromonas salmonicida* | 1785 | 48 |
| ORF288 | 321042 | 318676 | DNA gyrase subunit B | U35453 | *Clostridium acetobutylicum* | 1838 | 59 |
| ORF289 | 321445 | 321098 | putative | | | | |
| ORF290 | 322309 | 321710 | putative | | | | |
| ORF291 | 323190 | 322366 | outer membrane protein | AE000654 | *Helicobacter pylori* | 376 | 43 |
| ORF292 | 323843 | 323181 | hypothetical | U70214 | *Escherichia coli* | 356 | 37 |
| ORF293 | 324878 | 323856 | ATP-binding protein (abc) | U32744 | *Haemophilus influenzae* | 545 | 44 |
| ORF294 | 325340 | 326410 | f374; This 374 aa orf is 30 pct identical (9 gaps) to 102 residues of an approx. 512 aa protein FLIC SALMU SW: P06177 | AE000299 | *Escherichia coli* | 1194 | 62 |
| ORF295 | 326433 | 327836 | Xas A | AE000246 | *Escherichia coli* | 479 | 33 |
| ORF296 | 328465 | 327839 | putative | | | | |
| ORF297 | 329360 | 328857 | putative | | | | |
| ORF298 | 330907 | 329357 | putative | | | | |
| ORF299 | 332455 | 330956 | MgtE | U18744 | *Bacillus firmus* | 203 | 36 |
| ORF300 | 334536 | 332395 | putative | | | | |
| ORF301 | 336091 | 334877 | putative | | | | |
| ORF302 | 336103 | 337302 | putative | | | | |
| ORF303 | 338129 | 338830 | putative | | | | |
| ORF304 | 338965 | 339501 | putative | | | | |
| ORF305 | . 339508 | 340143 | putative | | | | |
| ORF306 | 340247 | 342967 | putative | | | | |
| ORF307 | 343385 | 343810 | cAMP-dependent protein kinase type I regulatory subunit | U75932 | *Rattus norvegicus* | 102 | 37 |
| ORF308 | 344171 | 343935 | acyl carrier protein (acpP) | AE000570 | *Helicobacter pylori* | 198 | 55 |
| ORF309 | 345082 | 344330 | 3-ketoacyl-ACP reductase | U39441 | *Vibrio harveyi* | 598 | 48 |
| ORF310 | 346005 | 345082 | malonyl-CoA:Acyl carrier protein transacylase | U59433 | *Bacillus subtilis* | 538 | 45 |
| ORF311 | 346784 | 346437 | beta-ketoacyl-acyl carrier protein synthase III (fabH) | AE000540 | *Helicobacter pylori* | 273 | 50 |
| ORF312 | 347029 | 346715 | beta-ketoacyl-acyl carrier protein synthase III | M77744 | *Escherichia coli* | 265 | 63 |
| ORF313 | 347034 | 347723 | recombination protein | D90916 | *Synechocystis sp.* | 363 | 42 |
| ORF314 | 348075 | 350459 | putative | | | | |
| ORF315 | 350598 | 351071 | putative | | | | |
| ORF316 | 351075 | 352175 | rifampicin resistance protein | L22690 | *Rickettsia rickettsii* | 495 | 46 |
| ORF317 | 353291 | 352230 | putative | | | | |
| ORF318 | 353442 | 354467 | pyruvate dehydrogenase E1 component, alpha subunit | D90915 | *Synechocystis sp.* | 571 | 44 |
| ORF319 | 354451 | 354933 | pyruvate dehydrogenase E1 beta subunit | U09137 | *Arabidopsis thaliana* | 495 | 59 |
| ORF320 | 355000 | 355449 | pyruvate dehydrogenase E1 component, beta subunit | U38804 | *Porphyra purpurea* | 336 | 47 |
| ORF321 | 355448 | 356743 | F23B12.5 | Z77659 | *Caenorhabditis elegans* | 759 | 46 |
| ORF322 | 355953 | 355642 | putative | | | | |
| ORF323 | 359310 | 356827 | glycogen phosphorylase B | U47025 | *Homo sapiens* | 2193 | 57 |
| ORF324 | 359120 | 359377 | putative | | | | |
| ORF325 | 359525 | 359908 | putative | | | | |
| ORF326 | 361290 | 359947 | DnaA | D89066 | *Staphylococcus aureus* | 375 | 46 |
| ORF327 | 363785 | 361362 | hypothetical | U32781 | *Haemophilus influenzae* | 394 | 44 |
| ORF328 | 364496 | 363888 | putative | | | | |
| ORF329 | 364832 | 365290 | putative | | | | |
| ORF330 | 365304 | 365669 | dpj | M76470 | *Escherichia coli* | 160 | 45 |
| ORF331 | 366599 | 365667 | NADPH thioredoxin reductase | AC002329 | *Arabidopsis thaliana* | 975 | 60 |
| ORF332 | 367291 | 369030 | ribosomal protein S1 (rpS1) | U32801 | *Haemophilus influenzae* | 1209 | 41 |
| ORF333 | 369134 | 369808 | NusA | U74759 | *Chlamydia trachomatis* | 995 | 87 |
| ORF334 | 369917 | 370438 | NusA | U74759 | *Chlamydia trachomatis* | 760 | 87 |
| ORF335 | 370365 | 372647 | | U74759 | *Chlamydia trachomatis* | 2173 | 61 |
| ORF336 | 372557 | 373066 | initiation factor IF2-beta (infB; gtg start codon) | X00513 | *Escherichia coli* | 333 | 39 |
| ORF337 | 373020 | 373442 | ORF6 gene product | Z18631 | *Bacillus subtilis* | 192 | 34 |
| ORF338 | 373467 | 374195 | tRNA pseudouridine 55 synthase | D90917 | *Synechocystis sp.* | 358 | 47 |
| ORF339 | 374176 | 375099 | hypothetical 34.6 kD protein in rpsT-ileS intergenic region | AE004113 | *Escherichia coli* | 395 | 39 |
| ORF340 | 375676 | 375083 | hypothetical GTP-binding protein in pth 3' region | AE000219 | *Escherichia coli* | 507 | 53 |
| ORF341 | 376173 | 375634 | hypothetical | U32723 | *Haemophilus influenzae* | 480 | 59 |
| ORF342 | 376564 | 377643 | YscU | U08019 | *Yersinia enterocolitica* | 538 | 37 |
| ORF343 | 377956 | 379773 | lcrD gene product | X67771 | *Yersinia enterocolitica* | 1302 | 47 |
| ORF344 | 379781 | 380425 | putative | | | | |
| ORF345 | 380281 | 381000 | putative | | | | |
| ORF346 | 381008 | 381460 | putative | | | | |
| ORF347 | 381460 | 383037 | 4-alpha-glucanotransferase | L37874 | *Clostridium butyricum* | 302 | 38 |
| ORF348 | 383257 | 383523 | ribosomal protein L28 (rpL28) | U32776 | *Haemophilus influenzae* | 175 | 55 |
| ORF349 | 383553 | 385304 | hypothetical protein | D90901 | *Synechocystis sp.* | 565 | 38 |
| ORF350 | 385397 | 386458 | comE ORF1 | D64002 | *Synechocystis sp.* | 187 | 10 |
| ORF351 | 387242 | 386514 | putative | | | | |
| ORF352 | 388764 | 387013 | putative | | | | |
| ORF353 | 390120 | 390932 | methylenetetrahydrofolate dehydrogenase | D64000 | *Synechocystis sp.* | 588 | 53 |
| ORF354 | 390919 | 391818 | f351; Residues 1-121 are 100 pct identical to YOJL_ECOLI SW: P33944 (122 aa) and aa 152-351 are 100 pct identical to YOJK ECOLI SW: P33943 | AE000310 | *Escherichia coli* | 186 | 39 |
| ORF355 | 392379 | 391885 | small protein | D90914 | *Synechocystis sp.* | 387 | 46 |
| ORF356 | 392582 | 392986 | putative | | | | |
| ORF357 | 392776 | 393684 | putative | | | | |
| ORF358 | 394151 | 394804 | RecF protein | D90907 | *Synechocystis sp.* | 232 | 34 |
| ORF359 | 394928 | 395308 | putative | | | | |
| ORF360 | 395259 | 395990 | putative | | | | |
| ORF361 | 397815 | 395953 | hypothetical | U32773 | *Haemophilus influenzae* | 391 | 36 |
| ORF362 | 398850 | 397831 | H. influenzae predicted coding region HI0807 | U32763 | *Haemophilus influenzae* | 580 | 39 |
| ORF363 | 400085 | 399099 | putative | | | | |
| ORF364 | 401245 | 400073 | YtgC | AF008220 | *Bacillus subtilis* | 244 | 30 |
| ORF365 | 401474 | 401136 | putative | | | | |
| ORF366 | 402199 | 401423 | unknown | U52850 | *Erysipelothrix rhusiopathiae* | 534 | 46 |
| ORF367 | 403193 | 402186 | putative | | | | |
| ORF368 | 403650 | 404165 | putative | | | | |
| ORF369 | 404343 | 405914 | adenine nucleotide translocase | Z49227 | *Arabidopsis thaliana* | 1280 | 55 |
| ORF370 | 405984 | 407327 | putative | | | | |
| ORF371 | 407712 | 408806 | putative | | | | |
| ORF372 | 410439 | 409075 | putative | | | | |
| ORF373 | 411826 | 410954 | putative | | | | |
| ORF374 | 412482 | 414302 | lepA gene product | X91655 | *Bacillus subtilis* | 1827 | 59 |
| ORF375 | 415402 | 414407 | 6-phosphogluconate dehydrogenase, decarboxylating (gnd) | U32737 | *Haemophilus influenzae* | 687 | 51 |
| ORF376 | 415848 | 415237 | 6-phosphogluconate dehydrogenase, 6PGD [Ceratitis capitata=medflies, Peptide, 481 aa] | S67873 | *Ceratitis capitata* | 695 | 64 |
| ORF377 | 417131 | 415866 | tyrosyl-tRNA synthetase (tyrS) | J01719 | *Escherichia coli* | 821 | 45 |
| ORF378 | 417258 | 417566 | putative | | | | |
| ORF379 | 418326 | 417454 | whiG-Stv gene product | X68709 | *Streptoverticillium griseocarneum* | 464 | 41 |
| ORF380 | 420057 | 418426 | FLHA gene product | X63698 | *Bacillus subtilis* | 455 | 49 |
| ORF381 | 420448 | 420720 | ferredoxin IV | M59855 | *Rhodobacter capsulatus* | 174 | 63 |
| ORF382 | 420980 | 421552 | putative | | | | |
| ORF383 | 421556 | 422029 | putative | | | | |
| ORF384 | 422461 | 422925 | putative | | | | |
| ORF385 | 423562 | 424320 | putative | | | | |
| ORF386 | 424250 | 424591 | putative | | | | |
| ORF387 | . 424830 | 426047 | putative | | | | |
| ORF388 | 426240 | 427397 | putative | | | | |
| ORF389 | 428841 | 430703 | GcpE | D90908 | *Synechocystis sp.* | 877 | 47 |
| ORF390 | 430694 | 431446 | YfiH | U50134 | *Escherichia coli* | 136 | 35 |
| ORF391 | 431597 | 432100 | putative | | | | |
| ORF392 | 432165 | 432779 | putative | | | | |
| ORF393 | 433272 | 432832 | dihydrolipoamide succinyltransferase (sucB) | U32839 | *Haemophilus influenzae* | 475 | 64 |
| ORF394 | 433925 | 433227 | dihydrolipoamide succinyltransferase (sucB) | U32839 | *Haemophilus influenzae* | 332 | 45 |
| ORF395 | 436678 | 433934 | alpha-ketoglutarate dehydrogenase | U41762 | *Rhodobacter capsulatus* | 1530 | 44 |
| ORF396 | 437176 | 438357 | oxygen-independent coproporphyrinogen III oxidase (hemN) | AE000628 | *Helicobacter pylori* | 442 | 42 |
| ORF397 | 440317 | 438518 | putative | | | | |
| ORF398 | 440001 | 440345 | putative | | | | |
| ORF399 | 441233 | 444517 | ORF f286 | U18997 | *Escherichia coli* | 168 | 45 |
| ORF400 | 440719 | 441012 | putative | | | | |
| ORF401 | 442192 | 441230 | putative | | | | |
| ORF402 | 442888 | 442343 | putative | | | | |
| ORF403 | 442371 | 442961 | putative | | | | |
| ORF404 | 443578 | 443003 | [karp] gene products | M86605 | *Chlamydia trachomatis* | 505 | 78 |
| ORF405 | 444500 | 443526 | aminopeptidase | D17450 | *Mycoplasma salivarium* | 273 | 39 |
| ORF406 | 444842 | 444528 | putative | | | | |
| ORF407 | 445009 | 444743 | putative | L39923 | *Mycobacterium leprae* | 133 | 33 |
| ORF408 | 445718 | 445182 | putative | | | | |
| ORF409 | 445807 | 447804 | Sulp | U18908 | *Zea mays* | 1307 | 52 |
| ORF410 | 448738 | 447803 | putative | | | | |
| ORF411 | 449628 | 448618 | RuvB protein | U38840 | *Thermologa maritima* | 845 | 53 |
| ORF412 | 450298 | 450867 | deoxycytidine triphosphate deaminase (dcd) | AE000554 | *Helicobacter pylori* | 573 | 58 |
| ORF413 | 450713 | 451207 | putative | | | | |
| ORF414 | 451211 | 452452 | hemolysin | D90914 | *Synechocystis sp.* | 227 | 39 |
| ORF415 | 452448 | 453659 | similar to [SwissProt Accession Number P379081 | D90888 | *Escherichia coli* | 96 | 33 |
| ORF416 | 454843 | 453725 | NifS gene product | L34879 | *Anabaena azollae* | 533 | 38 |
| ORF417 | 455608 | 454865 | hypothetical protein | D90908 | *Synechocystis sp.* | 371 | 36 |
| ORF418 | 456243 | 457007 | putative | | | | |
| ORF419 | 457016 | 457708 | putative | | | | |
| ORF420 | 458368 | 457979 | unknown | D26185 | *Bacillus subtilis* | 152 | 36 |
| ORF421 | 459496 | 458372 | mutY homolog | U63329 | *Homo sapiens* | 466 | 46 |
| ORF422 | 459493 | 460194 | hypothetical protein | D90914 | *Synechocystis sp.* | 98 | 38 |
| ORF423 | 461446 | 460355 | putative | | | | |
| ORF424 | 462298 | 461450 | putative | | | | |
| ORF425 | 462444 | 463349 | enoyl-ACP reductase | Y13861 | *Nicotiana tabacum* | 1008 | 69 |
| ORF426 | 464241 | 463342 | putative | | | | |
| ORF427 | 464574 | 465065 | putative | | | | |
| ORF428 | 465129 | 465611 | putative | | | | |
| ORF429 | 465571 | 466317 | putative | | | | |
| ORF430 | 466317 | 467093 | H. pylori predicted coding region HP0152 | AE000536 | *Helicobacter pylori* | 246 | 36 |
| ORF431 | 466999 | 467502 | putative | | | | |
| ORF432 | 469691 | 467715 | unidentified transporter-ATP binding | Z82044 | *Bacillus subtilis* | 496 | 45 |
| ORF433 | 470691 | 469660 | acetyl-CoA carboxylase subunit | AF008220 | *Bacillus subtilis* | 781 | 52 |
| ORF434 | 472010 | 470709 | putative | | | | |
| ORF435 | 471545 | 471799 | putative | | | | |
| ORF436 | 472359 | 472045 | putative | | | | |
| ORF437 | 473523 | 472732 | orf1 | X75413 | *Escherichia coli* | 313 | 42 |
| ORF438 | 474889 | 473441 | murE gene product | Z15056 | *Bacillus subtilis* | 679 | 37 |
| ORF439 | 477323 | 475365 | penicillin-binding protein 2 | X59630 | *Neisseria meningitidis* | 451 | 42 |
| ORF440 | 478496 | 477597 | hypothetical protein | D90906 | *Synechocystis sp.* | 534 | 52 |
| ORF441 | 478722 | 479273 | putative | | | | |
| ORF442 | 479277 | 479705 | putative | | | | |
| ORF443 | 480050 | 481450 | chromosomal replication initiator protein DnaA | D90909 | *Synechocystis sp.* | 793 | 40 |
| ORF444 | 481469 | 482053 | OrfH | U35673 | *Borrelia burgdorferi* | 157 | 37 |
| ORF445 | 482600 | 482025 | putative | | | | |
| ORF446 | 482654 | 484204 | NADH:ubiquinone oxidoreductase subunit B | Z37111 | *Vibrio alginolyticus* | 801 | 49 |
| ORF447 | 484211 | 485170 | NADH:ubiquinone oxidoreductase (GP:Z37111 4) | U32702 | *Haemophilus influenzae* | 258 | 48 |
| ORF448 | 485170 | 485838 | NADH:uniquinone oxidoreductase | Z37111 | *Vibrio alginolyticus* | 543 | 55 |
| ORF449 | 485813 | 486580 | unidentified protein of Na+-translocating NADH-quinone reductase | D49364 | *Vibrio alginolyticus* | 488 | 48 |
| ORF450 | 486976 | 486638 | putative | | | | |
| ORF451 | 489071 | 487764 | putative | | | | |
| ORF452 | 489341 | 489090 | putative | | | | |
| ORF453 | 489958 | 489152 | putative | | | | |
| ORF454 | 490549 | 489962 | putative | | | | |
| ORF455 | 491163 | 490522 | putative | | | | |
| ORF456 | 491396 | 491112 | putative | | | | |
| ORF457 | 492121 | 491390 | putative | | | | |
| ORF458 | 492304 | 494838 | ClpC adenosine triphosphatase | U02604 | *Bacillus subtilis* | 2370 | 46 |
| ORF459 | 495943 | 494822 | hypothetical protein in purB 5' region | AE000213 | *Escherichia coli* | 927 | 53 |
| ORF460 | 496011 | 496565 | putative | | | | |
| ORF461 | 496569 | 497228 | putative | | | | |
| ORF462 | 497358 | 497834 | putative | | | | |
| ORF463 | 497770 | 498327 | putative | | | | |
| ORF464 | 499209 | 499589 | putative | | | | |
| ORF465 | 499520 | 499792 | putative | | | | |
| ORF466 | 500774 | 504169 | putative 98 kDa outer membrane protein | U72499 | *Chlamydia psittaci* | 1215 | 45 |
| ORF467 | 504139 | 504600 | putative 98 kDa outer membrane protein | U72499 | *Chlamydia psittaci* | 319 | 47 |
| ORF468 | 504865 | 506877 | putative 98 kDa outer membrane protein | U72499 | *Chlamydia psittaci* | 992 | 42 |
| ORF469 | 506790 | 507671 | putative 98 kDa outer membrane protein | U72499 | *Chlamydia psittaci* | 739 | 46 |
| ORF470 | 507718 | 510507 | putative 98 kDa outer membrane protein | U72499 | *Chlamydia psittaci* | 1813 | 42 |
| ORF471 | 508325 | 507912 | putative | | | | |
| ORF472 | 510660 | 513440 | POMP90A precursor | U65942 | *Chlamydia psittaci* | 1830 | 46 |
| ORF473 | 514965 | 513787 | hypothetical | D83026 | *Bacillus subtilis* | 482 | 48 |
| ORF474 | 517347 | 515419 | putative 98 kDa outer membrane protein | U72499 | *Chlamydia psittaci* | 1554 | 51 |
| ORF475 | 517058 | 517363 | putative | | | | |
| ORF476 | 517798 | 517277 | putative 98 kDa outer membrane protein | U72499 | *Chlamydia psittaci* | 222 | 41 |
| ORF477 | 518200 | 517847 | POMP91B precursor | U65943 | *Chlamydia psittaci* | 162 | 42 |
| ORF478 | 518300 | 521146 | putative 98 kDa outer membrane protein | U72499 | *Chlamydia psittaci* | 1900 | 45 |
| ORF479 | 521392 | 522948 | POMP91A | U65942 | *Chlamydia psittaci* | 490 | 39 |
| ORF480 | 523244 | 524809 | putative 98 kDa outer membrane protein | U72499 | *Chlamydia psittaci* | 507 | 35 |
| ORF481 | 524379 | 524125 | putative | | | | |
| ORF482 | 524649 | 526238 | putative 98 kDa outer membrane protein | U72499 | *Chlamydia psittaci* | 969 | 41 |
| ORF483 | 526265 | 527104 | putative | | | | |
| ORF484 | 526947 | 526702 | putative | | | | |
| ORF485 | 526975 | 528450 | putative 98 kDa outer membrane protein | U72499 | *Chlamydia psittaci* | 197 | 48 |
| ORF486 | 528408 | 529199 | putative outer membrane protein | U72499 | *Chlamydia psittaci* | 154 | 37 |
| ORF487 | 530612 | 529542 | putative | | | | |
| ORF488 | 531656 | 530616 | putative | | | | |
| ORF489 | 533974 | 532067 | putative | | | | |
| ORF490 | 536432 | 534324 | putative | | | | |
| ORF491 | 537150 | 536707 | putative | | | | |
| ORF492 | 537928 | 537080 | putative | | | | |
| ORF493 | 538438 | 537932 | putative | | | | |
| ORF494 | 538737 | 538333 | putative | | | | |
| ORF495 | 539594 | 539127 | putative | | | | |
| ORF496 | 541215 | 539590 | putative | | | | |
| ORF497 | 542571 | 541282 | putative | | | | |
| ORF498 | 543014 | 542457 | putative | | | | |
| ORF499 | 543369 | 542962 | putative | | | | |
| ORF500 | 543809 | 546628 | putative 98 kDa outer membrane protein | U72499 | *Chlamydia psittaci* | 506 | 89 |
| ORF501 | 546619 | 549525 | POMP91A | U65942 | *Chlamydia psittaci* | 128 | 50 |
| ORF502 | 547293 | 546994 | putative | | | | |
| ORF503 | 549699 | 550523 | putative 98 kDa outer membrane protein | U72499 | *Chlamydia psittaci* | 96 | 32 |
| ORF504 | 550490 | 551551 | putative 98 kDa outer membrane protein | U72499 | *Chlamydia psittaci* | 223 | 33 |
| ORF505 | 551448 | 552623 | putative 98 kDa outer membrane protein | U72499 | *Chlamydia psittaci* | 139 | 46 |
| ORF506 | 552652 | 555117 | putative 98 kDa outer membrane protein | U72499 | *Chlamydia psittaci* | 487 | 48 |
| ORF507 | 555029 | 555493 | putative | | | | |
| ORF508 | 558006 | 555673 | putative | | | | |
| ORF509 | 559694 | 558162 | putative | | | | |
| ORF510 | 558208 | 558573 | putative | | | | |
| ORF511 | 561692 | 559899 | putative | | | | |
| ORF512 | 561412 | 561708 | putative | | | | |
| ORF513 | 563942 | 561777 | 1,4-alpha-glucan branching enzyme | X73903 | *Streptomyces coelicolor* | 1743 | 45 |
| ORF514 | 564969 | 563950 | putative | | | | |
| ORF515 | 566204 | 564936 | YqeV | D84432 | *Bacillus subtilis* | 639 | 38 |
| ORF516 | 567717 | 566302 | putative GTPase required for high frequency lysogenization by bacteriophage lambda | U00005 | *Escherichia coli* | 686 | 41 |
| ORF517 | 568526 | 567708 | putative | | | | |
| ORF518 | 569467 | 568742 | putative | | | | |
| ORF519 | 571065 | 569431 | putative | | | | |
| ORF520 | 571828 | 571118 | arginine-binding periplasmic protein 1 precursor | AE000188 | *Escherichia coli* | 197 | 45 |
| ORF521 | 572202 | 573308 | putative | | | | |
| ORF522 | 573146 | 575056 | putative | | | | |
| ORF523 | 575023 | 575916 | carboxysome formation protein | D90901 | *Synechocystis sp.* | 557 | 59 |
| ORF524 | 577891 | 576497 | putative | | | | |
| ORF525 | 578914 | 578204 | putative | | | | |
| ORF526 | 579924 | 578857 | putative | | | | |
| ORF527 | 580187 | 579858 | protein kinase C inhibitor | D90906 | *Synechocystis sp.* | 260 | 49 |
| ORF528 | 580017 | 580406 | putative | | | | |
| ORF529 | 581086 | 580187 | Yer156cp | U18917 | *Saccharomyces cerevisiae* | 176 | 34 |
| ORF530 | 581367 | 581828 | putative | | | | |
| ORF531 | 581678 | 582367 | putative | | | | |
| ORF532 | 582361 | 583428 | putative | | | | |
| ORF533 | 584690 | 583431 | putative | | | | |
| ORF534 | 585237 | 584950 | putative | | | | |
| ORF535 | 585626 | 586888 | hypothetical protein | D64004 | *Synechocystis sp.* | 805 | 45 |
| ORF536 | 586846 | 587907 | putative | | | | |
| ORF537 | 589049 | 588180 | putative | | | | |
| ORF538 | 590500 | 589301 | putative | | | | |
| ORF539 | 590755 | 592458 | aminoacyl-tRNA synthetase | L25105 | *Chlamydia trachomatis* | 2125 | 71 |
| ORF540 | 592526 | 592903 | has homology to putative heat shock proteins of Bacillus subtilis and Clostridium acetobutylicum; ORFA; putative | L25105 | *Chlamydia trachomatis* | 324 | 59 |
| ORF541 | 592836 | 593747 | Possible negative regulator of CIRCE element; Homologs in B. subtilis and Clostridia spp. referred to as hrcA or orfA | U52216 | *Chlamydia trachomatis* | 960 | 65 |
| ORF542 | 593747 | 594298 | grpE | M62819 | *Chlamydia trachomatis* | 661 | 71 |
| ORF543 | 594331 | 595947 | DnaK protein homolog; 71,550 Da; putative | M69227 | *Chlamydia pneumoniae* | 2619 | 100 |
| ORF544 | 595905 | 596309 | DnaK protein homolog; 71,550 Da; putative | M69227 | *Chlamydia pneumoniae* | 674 | 100 |
| ORF545 | 596514 | 597215 | putative | | | | |
| ORF546 | 597184 | 597957 | vacB gene product | U14003 | *Escherichia coli* | 306 | 48 |
| ORF547 | 597755 | 598612 | ORF-2 | D11024 | *Shigella flexneri* | 168 | 46 |
| ORF548 | 598602 | 599204 | homologous to DNA glycosylases; hypothetical | D83026 | *Bacillus subtilis* | 374 | 47 |
| ORF549 | 599373 | 599939 | putative | | | | |
| ORF550 | 600903 | 602072 | hemolysin | X73141 | *Serpulina hyodysenteriae* | 362 | 36 |
| ORF551 | 602240 | 602587 | hypothetical protein | D90908 | *Synechocystis sp*. | 182 | 35 |
| ORF552 | 602637 | 603272 | putative | | | | |
| ORF553 | 603142 | 604512 | putative | | | | |
| ORF554 | 604627 | 605853 | conserved hypothetical protein | AE000579 | *Helicobacter pylori* | 423 | 40 |
| ORF555 | 605790 | 606620 | putative | | | | |
| ORF556 | 606571 | 607281 | putative | L14679 | *Lactococcus lactis* | 384 | 45 |
| ORF557 | 609004 | 607355 | putative | | | | |
| ORF558 | 610906 | 609932 | putative | | | | |
| ORF559 | 611786 | 611004 | diaminopimelate epimerase | D90917 | *Synechocystis sp.* | 207 | 55 |
| ORF560 | 612333 | 611746 | ATP-dependent Clp protease proteolytic subunit | D90915 | *Synechocystis sp.* | 389 | 44 |
| ORF561 | 613897 | 612341 | serine hydroxymethyltransferase | D90903 | *Synechocystis sp.* | 909 | 52 |
| ORF562 | 615179 | 616279 | putative | | | | |
| ORF563 | 616610 | 617383 | putative | | | | |
| ORF564 | 618796 | 617810 | ORF o328 | U18997 | *Escherichia coli* | 413 | 45 |
| ORF565 | 620004 | 618826 | branched chain alpha-keto acid dehydrogenase E2 | M97391 | *Bacillus subtilis* | 688 | 41 |
| ORF566 | 619649 | 619918 | putative | | | | |
| ORF567 | 621265 | 620021 | Hypothetical protein | Y14083 | *Bacillus subtilis* | 727 | 37 |
| ORF568 | 622359 | 621265 | hypothetical | U32691 | *Haemophilus influenzae* | 294 | 52 |
| ORF569 | 623420 | 622560 | rRNA methylase | D90913 | *Synechocystis sp.* | 244 | 38 |
| ORF570 | 624297 | 623335 | hypothetical protein (SP:P39587) | U67605 | *Methanococcus jannaschii* | 147 | 35 |
| ORF571 | 624773 | 624174 | riboflavin synthase alpha chain | AE000261 | *Escherichia coli* | 424 | 50 |
| ORF572 | 625029 | 625484 | ORF 168 | D28752 | *Synechococcus sp.* | 323 | 43 |
| ORF573 | 625488 | 625883 | YteA | AF008220 | *Bacillus subtilis* | 172 | 35 |
| ORF574 | 625892 | 626395 | signalpeptidase II | X78084 | *Staphylococcus carnosus* | 204 | 38 |
| ORF575 | 626444 | 627790 | D-alanine permease (dagA) | U32770 | *Haemophilus influenzae* | 566 | 33 |
| ORF576 | 627912 | 628607 | putative | | | | |
| ORF577 | 628774 | 629697 | putative | | | | |
| ORF578 | 629660 | 631639 | POMP91A | U65942 | *Chlamydia psittaci* | 579 | 44 |
| ORF579 | 631725 | 633551 | putative | | | | |
| ORF580 | 633520 | 636957 | putative 98 kDa outer membrane protein | U72499 | *Chlamydia psittaci* | 266 | 45 |
| ORF581 | 637232 | 638098 | adhesion protein | D90903 | *Synechocystis sp.* | 267 | 38 |
| ORF582 | 640648 | 639593 | GTP-binding protein | D90901 | *Synechocystis sp.* | 759 | 45 |
| ORF583 | 640979 | 640728 | 50S ribosomal protein L27 | U38804 | *Porphyra purpurea* | 265 | 65 |
| ORF584 | 641327 | 641007 | 50S ribosomal subunit protein L21 | U18997 | *Escherichia coli* | 210 | 41 |
| ORF585 | 641687 | 642283 | hypothetical protein | D90906 | *Synechocystis sp.* | 76 | 39 |
| ORF586 | 643023 | 642286 | assimilatory sulfite reductase | L26503 | *Saccharomyces cerevisiae* | 284 | 42 |
| ORF587 | 643330 | 643076 | putative | | | | |
| ORF588 | 643704 | 643351 | ribosomal protein S10 (rpS10) | U32761 | *Haemophilus influenzae* | 349 | 69 |
| ORF589 | 645628 | 643676 | translation elongation factor EF-G (fusA) | AE000625 | *Helicobacter pylori* | 1991 | 58 |
| ORF590 | 645783 | 645538 | elongation factor G (AA 1-691) | X16278 | *Thermus aquaticus thermophilus* | 170 | 80 |
| ORF591 | 646269 | 645793 | ribosomal protein S7 | Z11567 | *Chlamydia trachomatis* | 730 | 88 |
| ORF592 | 646751 | 646314 | ribosomal protein S 12 (AA 1-123) | X52912 | *Cryptomonas phi* | 485 | 67 |
| ORF593 | 647848 | 647045 | putative | | | | |
| ORF594 | 648393 | 650336 | ORF of prc gene (alt.) | D00674 | *Escherichia coli* | 554 | 42 |
| ORF595 | 651016 | 650420 | hypothetical sulfur-rich protein | U41759 | *Chlamydia psittaci* | 301 | 50 |
| ORF596 | 652956 | 651289 | 60kDa CrP | X53511 | *Chlamydia pneumoniae* | 2951 | 100 |
| ORF597 | 653395 | 653126 | 9kDa CrP | X53511 | *Chlamydia pneumoniae* | 502 | 99 |
| ORF598 | 655740 | 654193 | glutamyl-tRNA synthetase homolog | U41759 | *Chlamydia psittaci* | 2259 | 82 |
| ORF599 | 656508 | 655966 | early stage-specific transcription experimentally demonstrated; early upstream open reading frame (EUO) | L13598 | *Chlamydia psittaci* | 666 | 62 |
| ORF600 | 658140 | 657022 | unknown | U41759 | *Chlamydia psittaci* | 950 | 44 |
| ORF601 | 660216 | 658525 | RecJ recombination protein | U41759 | *Chlamydia psittaci* | 807 | 73 |
| ORF602 | 663238 | 660248 | protein-export membrane protein SecD | D64000 | *Synechocystis sp.* | 413 | 41 |
| ORF603 | 664461 | 663157 | putative | | | | |
| ORF604 | 665735 | 664635 | putative | | | | |
| ORF605 | 666212 | 666994 | hypothetical protein | D64006 | *Synechocystis sp.* | 538 | 58 |
| ORF606 | 666998 | 667921 | o298; This 298 aa orf is 33 pct identical (24 gaps) to 248 residues of an approx. 256 aa protein CDSA ECOLI SW: P06466 | AE000238 | *Escherichia coli* | 253 | 45 |
| ORF607 | 667909 | 668568 | cytidylate kinase | AE000193 | *Escherichia coli* | 400 | 48 |
| ORF608 | 668502 | 669203 | hypothetical protein | D90915 | *Synechocystis sp.* | 225 | 33 |
| ORF609 | 669154 | 670893 | arginyl-tRNA-synthetase | D64006 | *Synechocystis sp.* | 1365 | 49 |
| ORF610 | 672226 | 670853 | UDP-N-acetylglucosamine enolpyruvyl transferase (murZ) | U32788 | *Haemophilus influenzae* | 642 | 40 |
| ORF611 | 671137 | 671424 | putative | | | | |
| ORF612 | 672453 | 673001 | putative | | | | |
| ORF613 | 673072 | 674721 | putative | | | | |
| ORF614 | 674549 | 674262 | putative | | | | |
| ORF615 | 675518 | 674796 | ORF246 gene product | X59551 | *Escherichia coli* | 520 | 43 |
| ORF616 | 676083 | 675499 | putative | | | | |
| ORF617 | 676630 | 676067 | putative | | | | |
| ORF618 | 677016 | 676600 | ORF3 | D10279 | *Bacillus subtilis* | 361 | 63 |
| ORF619 | 677647 | 677015 | peptide release factor 2 | X99401 | *Bacillus firmus* | 427 | 43 |
| ORF620 | 677990 | 678259 | unknown | Z49939 | *Saccharomyces cerevisiae* | 175 | 48 |
| ORF621 | 679444 | 680097 | unknown | D26185 | *Bacillus subtilis* | 263 | 38 |
| ORF622 | 680097 | 680897 | unknown | D64126 | *Bacillus subtilis* | 506 | 45 |
| ORF623 | 681637 | 680849 | putative | | | | |
| ORF624 | 681409 | 682281 | putative | | | | |
| ORF625 | 682453 | 682821 | putative | | | | |
| ORF626 | 682763 | 683902 | sensor protein | L39904 | *Myxococcus xanthus* | 190 | 48 |
| ORF627 | 684616 | 683969 | putative | | | | |
| ORF628 | 685169 | 684534 | putative | | | | |
| ORF629 | 685986 | 685117 | putative | | | | |
| ORF630 | 686278 | 687288 | NtrC/NifA-like protein regulator | U17902 | *Escherichia coli* | 820 | 45 |
| ORF631 | 687483 | 688151 | putative | | | | |
| ORF632 | 688740 | 689501 | putative | | | | |
| ORF633 | 690242 | 689622 | putative | | | | |
| ORF634 | 690470 | 691126 | unknown | Z48008 | *Saccharomyces cerevisiae* | 380 | 46 |
| ORF635 | 692600 | 691497 | putative | | | | |
| ORF636 | 692674 | 695064 | phenylalanyl-tRNA synthetase beta-subunit (pheT) | U32810 | *Haemophilus influenzae* | 593 | 45 |
| ORF637 | 695049 | 696032 | putative | | | | |
| ORF638 | 697964 | 696585 | OppC-like protein | D85103 | *Synechococcus sp.* | 371 | 37 |
| ORF639 | 699803 | 698274 | OppB gene product | X56347 | *Bacillus subtilis* | 197 | 40 |
| ORF640 | 701926 | 699788 | AppA | U20909 | *Bacillus subtilis* | 324 | 43 |
| ORF641 | 703196 | 702567 | putative | | | | |
| ORF642 | 704221 | 703208 | putative | | | | |
| ORF643 | 704240 | 705289 | ferrochelatase | X73417 | *Arabidopsis thaliana* | 266 | 42 |
| ORF644 | 706070 | 705300 | histidine periplasmic binding protein P29 | U58045 | *Campylobacter jejuni* | 128 | 31 |
| ORF645 | 706841 | 706254 | conserved hypothetical protein | AE000592 | *Helicobacter pylori* | 155 | 37 |
| ORF646 | 707596 | 706811 | putative | | | | |
| ORF647 | 708666 | 707677 | ADP-glucose pyrophosphorylase | X55650 | *Solanum tuberosum* | 595 | 43 |
| ORF648 | 709793 | 709119 | pyrE-F gene product | X71842 | *Arabidopsis thaliana* | 400 | 44 |
| ORF649 | 711523 | 710132 | transcription termination factor | J01673 | *Escherichia coli* | 1251 | 60 |
| ORF650 | 712236 | 711523 | putative | | | | |
| ORF651 | 714734 | 712125 | DNA polymerase I | J04479 | *Streptococcus pneumoniae* | 1334 | 43 |
| ORF652 | 715759 | 714761 | protease IV | U67512 | *Methanococcus jannaschii* | 101 | 55 |
| ORF653 | 717538 | 715886 | adenine nucleotide translocase | Z49227 | *Arabidopsis thaliana* | 832 | 39 |
| ORF654 | 719113 | 720243 | replicative DNA helicase | D26185 | *Bacillus subtilis* | 776 | 44 |
| ORF655 | 720590 | 722422 | homologous to E.coli gidA | X62540 | *Pseudomonas putida* | 1575 | 52 |
| ORF656 | 722406 | 723056 | putative | | | | |
| ORF657 | 723551 | 723120 | nucleoside 5'-diphosphate phosphotransferase (EC 2.7.4.6) | J05207 | *Myxococcus xanthus* | 451 | 62 |
| ORF658 | 724246 | 723626 | Holliday junction DNA helicase (ruvA) | U32716 | *Haemophilus influenzae* | 293 | 43 |
| ORF659 | 724754 | 724251 | crossover junction endodeoxyribonuclease (ruvC) | U32717 | *Haemophilus influenzae* | 296 | 53 |
| ORF660 | 725868 | 724900 | putative | | | | |
| ORF661 | 727115 | 726270 | putative | | | | |
| ORF662 | 728126 | 727119 | glyceraldehyde-3-phosphate dehydrogenase | U83198 | *Chlamydia trachomatis* | 1340 | 75 |
| ORF663 | 728594 | 728208 | ribosomal protein L17 | L33834 | *Chlamydia trachomatis* | 439 | 82 |
| ORF664 | 729614 | 728604 | RNA polymerase alpha-subunit | L33834 | *Chlamydia trachomatis* | 1356 | 89 |
| ORF665 | 729778 | 729533 | RNA polymerase alpha-subunit | L33834 | *Chlamydia trachomatis* | 273 | 82 |
| ORF666 | 730149 | 729751 | ribosomal protein S11 | L33834 | *Chlamydia trachomatis* | 562 | 90 |
| ORF667 | 730539 | 730174 | ribosomal protein S13 | L33834 | *Chlamydia trachomatis* | 544 | 89 |
| ORF668 | 731983 | 730598 | homolog | L25077 | *Chlamydia trachomatis* | 1956 | 83 |
| ORF669 | 732427 | 731996 | ribosomal protein CtrL15e | M80325 | *Chlamydia trachomatis* | 563 | 77 |
| ORF670 | 732917 | 732423 | ribosomal protein CtrS5e | M80325 | *Chlamydia trachomatis* | 702 | 84 |
| ORF671 | 733598 | 733320 | ribosomal protein L6 | M60652 | *Chlamydia trachomatis* | 316 | 87 |
| ORF672 | 733869 | 733492 | ribosomal protein L6 | M60652 | *Chlamydia trachomatis* | 469 | 77 |
| ORF673 | 734298 | 733900 | ribosomal protein CtrS8e | M80325 | *Chlamydia trachomatis* | 572 | 82 |
| ORF674 | 734858 | 734319 | ribosomal protein CtrL5e | M80325 | *Chlamydia trachomatis* | 730 | 90 |
| ORF675 | 735195 | 734863 | ribosomal protein CtrL24e | M80325 | *Chlamydia trachomatis* | 420 | 70 |
| ORF676 | 735578 | 735342 | ribosomal protein CtrL14e | M80325 | *Chlamydia trachomatis* | 270 | 95 |
| ORF677 | 735861 | 735604 | ribosomal protein S17e | M80325 | *Chlamydia trachomatis* | 322 | 77 |
| ORF678 | 736492 | 736079 | 50S ribosomal protein L16 | D90905 | *Synechocystis sp.* | 439 | 60 |
| ORF679 | 737192 | 736524 | ribosomal protein S3 | D64071 | *Actinobacillus actinomycetemcomitans* | 612 | 58 |
| ORF680 | 737555 | 737211 | ribosomal protein L22 | Z21677 | *Thermotoga maritima* | 228 | 48 |
| ORF681 | 738688 | 737837 | 50S ribosomal subunit protein L2 | U18997 | *Escherichia coli* | 769 | 62 |
| ORF682 | 739048 | 738713 | putative | | | | |
| ORF683 | 739736 | 739065 | ribosomal protein L4 | X67014 | *Bacillus stearothermophilus* | 308 | 46 |
| ORF684 | 740477 | 739773 | ribosomal protein L3 | Z46265 | *Thermus aquaticus thermophilus* | 463 | 50 |
| ORF685 | 740659 | 740958 | putative | | | | |
| ORF686 | 741722 | 740721 | putative | | | | |
| ORF687 | 742789 | 741827 | methionyl-tRNA formyltransferase | D64001 | *Synechocystis sp.* | 511 | 48 |
| ORF688 | 743618 | 742782 | UDP-N-acetylglucosamine acyltransferase | L22690 | *Rickettsia rickettsii* | 542 | 43 |
| ORF689 | 744092 | 743634 | (3R)-hydroxymyristol acyl carrier protein dehydrase | D90910 | *Synechocystis sp.* | 339 | 55 |
| ORF690 | 744604 | 744107 | UDP-3-0-acyl N-acetylglcosamine deacetylase | D90902 | *Synechocystis sp.* | 287 | 45 |
| ORF691 | 744953 | 744498 | UDP-3-O-acyl-GlcNAc deacetylase | U67855 | *Pseudomonas aeruginosa* | 262 | 51 |
| ORF692 | 746608 | 744986 | apolipoprotein N-acyltransferase (cute) | U32716 | *Haemophilus influenzae* | 194 | 50 |
| ORF693 | 747085 | 746621 | low homology to P14 protein of Heamophilus influenzar and 14.2 kDa protein of Escherichia coli | D78189 | *Bacillus subtilis* | 235 | 37 |
| ORF694 | 747974 | 747219 | polymerase III | M22996 | *Bacillus subtilis* | 180 | 34 |
| ORF695 | 748594 | 748169 | hypothetical protein | D90914 | *Synechocystis sp.* | 160 | 43 |
| ORF696 | 749145 | 748573 | putative | | | | |
| ORF697 | 749652 | 749957 | trxA | L39892 | *Chlamydia psittaci* | 393 | 72 |
| ORF698 | 750446 | 749979 | spoU | L39892 | *Chlamydia psittaci* | 559 | 72 |
| ORF699 | 751219 | 750446 | mip | L39892 | *Chlamydia psittaci* | 948 | 60 |
| ORF700 | 753042 | 751291 | aspartyl-tRNA synthetase | D90910 | *Synechocystis sp.* | 1347 | 47 |
| ORF701 | 754309 | 753020 | histidine--tRNA ligase | Z17214 | *Streptococcus equisimilis* | 757 | 44 |
| ORF702 | 755120 | 756175 | hexosephosphate transport protein | M89480 | *Salmonella typhimurium* | 870 | 49 |
| ORF703 | 756120 | 756485 | hexosephosphate transport protein | M89479 | *Escherichia coli* | 321 | 45 |
| ORF704 | 756499 | 760227 | DNA polymerase III alpha-subunit (dnaE) | AE000646 | *Helicobacter pylori* | 1977 | 42 |
| ORF705 | 761217 | 760297 | putative | | | | |
| ORF706 | 761297 | 761809 | putative | | | | |
| ORF707 | 761782 | 762282 | putative | | | | |
| ORF708 | 762260 | 762895 | putative | | | | |
| ORF709 | 762867 | 763316 | hypothetical protein | D90908 | *Synechocystis sp.* | 177 | 43 |
| ORF710 | 763780 | 763325 | putative | | | | |
| ORF711 | 763861 | 765168 | DD-carboxypeptidase | M85047 | *Bacillus subtilis* | 292 | 37 |
| ORF712 | 766809 | 765697 | fmu and fmv protein | D90902 | *Synechocystis sp.* | 130 | 36 |
| ORF713 | 768051 | 766888 | putative | | | | |
| ORF714 | 768566 | 768321 | putative | | | | |
| ORF715 | 769342 | 768551 | putative | | | | |
| ORF716 | 770532 | 769378 | putative | | | | |
| ORF717 | 771451 | 770804 | putative | | | | |
| ORF718 | 773058 | 771847 | 3-phosphoglycerate kinase | U83197 | *Chlamydia trachomatis* | 1540 | 72 |
| ORF719 | 773094 | 773456 | putative | | | | |
| ORF720 | 774376 | 773093 | putative phosphate permease | U84890 | *Mesembryanthemum crystallinum* | 870 | 45 |
| ORF721 | 775123 | 774380 | putative | | | | |
| ORF722 | 775398 | 774916 | putative | | | | |
| ORF723 | 775046 | 776077 | sporulation protein | M57689 | *Bacillus subtilis* | 698 | 43 |
| ORF724 | 776070 | 777041 | was dppE | U00039 | *Escherichia coli* | 565 | 56 |
| ORF725 | 777964 | 777536 | orf288; translated orf similarity to SWISS-PROT: YGI2_PSEPU hypothetical 32.4 kDa protein of Pseudomomas putida | Y10436 | *Coxiella burnetii* | 256 | 46 |
| ORF726 | 778176 | 777904 | B.subtilis genes rpmH, mpA, 50kd, gidA and gidB | X62539 | *Bacillus subtilis* | 112 | 37 |
| ORF727 | 778621 | 779334 | putative | | | | |
| ORF728 | 781173 | 780307 | f406; This 406 aa orf is 28 pct identical (12 gaps) to 264 residues of an approx. 440 aa protein YAOA SCHPO SW: 010089 | AE000263 | *Escherichia coli* | 603 | 40 |
| ORF729 | 781526 | 781116 | f406; This 406 aa orf is 28 pct identical (12 gaps) to 264 residues of an approx. 440 aa protein YAOA SCHPO SW: 010089 | AE000263 | *Escherichia coli* | 258 | 45 |
| ORF730 | 782784 | 781555 | f423; This 423 aa orf is 29 pct identical (1 gaps) to 172 residues of an approx. 488 aa protein YC24 CYAPA SW: P48260 | AE000263 | *Escherichia coli* | 197 | 44 |
| ORF731 | 783572 | 782805 | hypothetical chloroplast ORF 16 | U38804 | *Porphyra purpurea* | 597 | 52 |
| ORF732 | 785032 | 783581 | ABC transporter subunit | D64004 | *Synechocystis sp.* | 1720 | 62 |
| ORF733 | 786412 | 785360 | putative | | | | |
| ORF734 | 788429 | 786450 | pbp | Y14206 | *Streptomyces coelicolor* | 148 | 55 |
| ORF735 | 788944 | 788528 | penicillin-binding protein 3 | X84053 | *Pseudomonas aeruginosa* | 148 | 38 |
| ORF736 | 789758 | 788901 | putative | | | | |
| ORF737 | 790332 | 791504 | major outer membrane protein | M64064 | *Chlamydia pneumoniae* | 2028 | 99 |
| ORF738 | 791846 | 792721 | ribosomal protein S2 | U60196 | *Chlamydia trachomatis* | 904 | 70 |
| ORF739 | 792724 | 793569 | elongation factor Ts | U60196 | *Chlamydia trachomatis* | 1023 | 71 |
| ORF740 | 793580 | 794323 | UMP kinase | U60196 | *Chlamydia trachomatis* | 891 | 72 |
| ORF741 | 794304 | 794843 | ribosome-releasing factor | U60196 | *Chlamydia trachomatis* | 673 | 73 |
| ORF742 | 795217 | 795732 | unknown | D26185 | *Bacillus subtilis* | 105 | 42 |
| ORF743 | 795722 | 796795 | unknown | D26185 | *Bacillus subtilis* | 208 | 33 |
| ORF744 | 798735 | 797053 | putative | L33796 | *Vibrio cholerae* | 386 | 34 |
| ORF745 | 799823 | 798681 | putative | | | | |
| ORF746 | 799297 | 799578 | putative | | | | |
| ORF747 | 801313 | 799808 | Pkn5 | U40656 | *Myxococcus xanthus* | 345 | 33 |
| ORF748 | 802453 | 801332 | putative | | | | |
| ORF749 | 803299 | 802457 | putative | | | | |
| ORF750 | 803811 | 803290 | putative | | | | |
| ORF751 | 805151 | 803826 | YscN | U02499 | *Yersinia enterocolitica* | 1185 | 53 |
| ORF752 | 805860 | 805156 | putative | | | | |
| ORF753 | 806604 | 806332 | putative | | | | |
| ORF754 | 806913 | 806608 | putative | | | | |
| ORF755 | 808222 | 806903 | putative | | | | |
| ORF756 | 808751 | 808146 | putative | | | | |
| ORF757 | 809437 | 808673 | putative | | | | |
| ORF758 | 809939 | 809454 | putative | | | | |
| ORF759 | 811235 | 810213 | delta-aminolevulinate synthase (EC 2.3.1.37) | M30785 | *Escherichia coli* | 172 | 40 |
| ORF760 | 811779 | 813056 | DNA gyrase subunit B | U35453 | *Clostridium acetobutylicum* | 584 | 38 |
| ORF761 | 812890 | 812516 | putative | | | | |
| ORF762 | 812954 | 813583 | DNA gyrase subunit B | Z19108 | *Spiroplasma citri* | 371 | 39 |
| ORF763 | 813587 | 815023 | gyrA | X92503 | *Mycobacterium smegmatis* | 414 | 55 |
| ORF764 | 815420 | 815746 | putative | | | | |
| ORF765 | 816036 | 817010 | orf-X; hypothetical protein; Method: conceptual translation supplied by author | U48870 | *Bacillus subtilis* | 569 | 47 |
| ORF766 | 817111 | 817356 | unknown | Z74024 | *Mycobacterium tuberculosis* | 114 | 34 |
| ORF767 | 817791 | 818609 | 3-deoxy-d-manno-octulosonic acid 8-phosphate synthetase | Z50747 | *Chlamydia psittaci* | 1112 | 78 |
| ORF768 | 818609 | 819094 | protein of unknown function | Z50747 | *Chlamydia psittaci* | 545 | 65 |
| ORF769 | 819104 | 819823 | ATP binding protein | U72493 | *Chlamydia trachomatis* | 1099 | 88 |
| ORF770 | 820722 | 819826 | putative | | | | |
| ORF771 | 822313 | 821000 | putative | | | | |
| ORF772 | 823503 | 822238 | putative | | | | |
| ORF773 | 823678 | 825612 | putative | | | | |
| ORF774 | 825461 | 826312 | putative | | | | |
| ORF775 | 827280 | 826645 | putative | | | | |
| ORF776 | 828604 | 827171 | 76 kDa protein | L23921 | *Chlamydia pneumoniae* | 2179 | 100 |
| ORF777 | 830026 | 828713 | 76 kDa protein | L23921 | *Chlamydia pneumoniae* | 1162 | 100 |
| ORF778 | 831047 | 830085 | mviB homolog | U50732 | *Chlamydia trachomatis* | 982 | 58 |
| ORF779 | 831725 | 831051 | mviB homolog | U50732 | *Chlamydia trachomatis* | 740 | 65 |
| ORF780 | 832220 | 833098 | T05H10.2 | Z47812 | *Caenorhabditis elegans* | 407 | 34 |
| ORF781 | 833851 | 833396 | ribosomal protein S4 (rps4) | AE000633 | *Helicobacter pylori* | 372 | 53 |
| ORF782 | 834068 | 835039 | This ORF is homologous to a 40.0 kd hypothetical protein in the htrB 3' region from E. coli. Accession Number X61000 | L22217 | *Mycoplasma-like organism* | 377 | 49 |
| ORF783 | 835792 | 835127 | uridine kinase | L31783 | *Mus musculus* | 436 | 43 |
| ORF784 | 837624 | 836116 | ORF f397 | U29581 | *Escherichia coli* | 92 | 38 |
| ORF785 | 838951 | 840882 | putative | | | | |
| ORF786 | 840869 | 842185 | exodeoxyribonuclease V (recB) | U32811 | *Haemophilus influenzae* | 409 | 40 |
| ORF787 | 841989 | 843455 | DNA helicase II | U39703 | *Mycoplasma genitalium* | 110 | 46 |
| ORF788 | 843242 | 844021 | exodeoxyribonuclease V (recB) | U32811 | *Haemophilus influenzae* | 196 | 40 |
| ORF789 | 845018 | 843987 | MreC protein | M31792 | *Escherichia coli* | 76 | 53 |
| ORF790 | 846174 | 844990 | aspartate aminotransferase (aspC) | X03629 | *Escherichia coli* | 754 | 40 |
| ORF791 | 848509 | 846311 | GreA | U02878 | *Rickettsia prowazekii* | 190 | 35 |
| ORF792 | 848568 | 849014 | putative | | | | |
| ORF793 | 849082 | 850488 | NADH:ubiquinone oxidoreducatase subunit A (GP:Z37111 2) | U32702 | *Haemophilus influenzae* | 445 | 37 |
| ORF794 | 851512 | 850574 | porphobilinogen synthase | U38348 | *Chlorobium vibrioforme* | 769 | 45 |
| ORF795 | 852064 | 852447 | putative | | | | |
| ORF796 | 852398 | 853690 | putative | | | | |
| ORF797 | 855118 | 854243 | geranylgeranyl pyrophosphate synthase | D85029 | *Arabidopsis thaliana* | 408 | 41 |
| ORF798 | 855751 | 855128 | f147; This 147 aa orf is 26 pct identical (1 gaps) to 99 residues of an approx. 728 aa protein E2BE RABIT SW: P47823 | AE000143 | *Escherichia coli* | 187 | 36 |
| ORF799 | 856551 | 855829 | membrane associated regulatory protein | M28368 | *Salmonella typhimurium* | 172 | 36 |
| ORF800 | 856730 | 858556 | unknown function | Z32530 | *Chlamydia trachomatis* | 842 | 35 |
| ORF801 | 858717 | 859601 | exodeoxyribonuclease V (recD) | U32811 | *Haemophilus influenzae* | 182 | 51 |
| ORF802 | 859591 | 860205 | exonuclease V alpha subunit (AA 1-608) | X04582 | *Escherichia coli* | 235 | 45 |
| ORF803 | 861132 | 860284 | putative | | | | |
| ORF804 | 861426 | 861163 | 30S ribosomal protein S20 | Z67753 | *Odontella sinensis* | 153 | 41 |
| ORF805 | 861701 | 862921 | putative | | | | |
| ORF806 | 863026 | 864798 | major sigma factor | U04442 | *Chlamydia psittaci* | 2661 | 94 |
| ORF807 | 864831 | 865256 | putative | | | | |
| ORF808 | 865226 | 866581 | dihydropterin pyrophosphokinase /dihydropteroate synthase | Y08611 | *Pisum sativum* | 455 | 48 |
| ORF809 | 866562 | 867119 | dehydrofolate reductase, type I (folA) | U32772 | *Haemophilus influenzae* | 213 | 49 |
| ORF810 | 867025 | 867816 | M. jannaschii predicted coding region MJ0768 | U67522 | *Methanococcus jannaschii* | 207 | 36 |
| ORF811 | 867820 | 868497 | putative | | | | |
| ORF812 | 869743 | 868661 | RecA | U16739 | *Chlamydia trachomatis* | 1512 | 87 |
| ORF813 | 870633 | 870094 | unknown function | Z32530 | *Chlamydia trachomatis* | 308 | 45 |
| ORF814 | 871929 | 870646 | unknown function | Z32530 | *Chlamydia trachomatis* | 1410 | 63 |
| ORF815 | 872538 | 872086 | putative | | | | |
| ORF816 | 873908 | 872517 | putative | | | | |
| ORF817 | 874281 | 874670 | nifR3-like gene product | Z37984 | *Azospirillum brasilense* | 181 | 32 |
| ORF818 | 874582 | 875286 | ORF1 gene product | X62399 | *Escherichia coli* | 307 | 42 |
| ORF819 | 877857 | 875377 | DNA topoisomerase I | L27797 | *Bacillus subtilis* | 1488 | 50 |
| ORF820 | 878446 | 879255 | putative | | | | |
| ORF821 | 880635 | 879268 | sigma factor (ntrA) (AA 1-502) | X05888 | *Azotobacter vinelandii* | 257 | 47 |
| ORF822 | 882524 | 880593 | DNA helicase II | D90906 | *Synechocystis sp.* | 1140 | 50 |
| ORF823 | 882612 | 883319 | ipa-57d gene product | X73124 | *Bacillus subtilis* | 601 | 51 |
| ORF824 | 884155 | 883538 | hypothetical protein | D90915 | *Synechocystis sp.* | 344 | 39 |
| ORF825 | 884340 | 885611 | 19/20 residue stretch (32-51) identical to N-terminal putative signal sequence of unknown, partly cloned B. subtilis gene.; putative | L19954 | *Bacillus subtilis* | 456 | 37 |
| ORF826 . | 885722 | 887302 | heat shock protein | L12004 | *Chlamydia trachomatis* | 915 | 39 |
| ORF827 | 887587 | 888153 | bas1 protein | Z34917 | *Hordeum vulgare* | 474 | 50 |
| ORF828 | 888627 | 888220 | putative | | | | |
| ORF829 | 889330 | 888716 | hypothetical protein | Y14079 | *Bacillus subtilis* | 223 | 55 |
| ORF830 | 889898 | 889323 | peptidoglycan-associated lipoprotein | X65796 | *Escherichia coli* | 222 | 50 |
| ORF831 | 891190 | 889898 | TolB | U32470 | *Haemophilus influenzae* | 280 | 35 |
| ORF832 | 891828 | 891247 | putative | | | | |
| ORF833 | 892421 | 892017 | exbD peptide | M28819 | *Escherichia coli* | 77 | 48 |
| ORF834 | 893116 | 892421 | inner membrane protein (tolQ) | U32722 | *Haemophilus influenzae* | 157 | 54 |
| ORF835 | 892521 | 892925 | putative | | | | |
| ORF836 | 893392 | 895419 | inner membrane copper tolerance protein | Z36905 | *Escherichia coli* | 120 | 35 |
| ORF837 | 895745 | 896527 | unknown | D26185 | *Bacillus subtilis* | 381 | 41 |
| ORF838 | 896668 | 897558 | succinate dehydrogenase subunit C | Y08563 | *Paenibacillus macerans* | 253 | 40 |
| ORF839 | 897565 | 899442 | succinate dehydrogenase subunit A | Y08563 | *Paenibacillus macerans* | 1667 | 57 |
| ORF840 | 899420 | 900229 | succinate dehydrogenase subunit B | Y08563 | *Paenibacillus macerans* | 656 | 54 |
| ORF841 | 903230 | 900237 | putative | | | | |
| ORF842 | 905081 | 903234 | putative | | | | |
| ORF843 | 906931 | 905045 | sigma factor SibG regulation protein RsbU | D90905 | *Synechocystis sp.* | 117 | 35 |
| ORF844 | 907248 | 907832 | putative | | | | |
| ORF845 | 907784 | 908128 | putative | | | | |
| ORF846 | 908132 | 908677 | putative | | | | |
| ORF847 | 908589 | 909320 | putative | | | | |
| ORF848 | 909405 | 911465 | putative | | | | |
| ORF849 | 911677 | 912360 | putative | | | | |
| ORF850 | 912303 | 912821 | putative | | | | |
| ORF851 | 912937 | 913983 | putative | | | | |
| ORF852 | 915128 | 914067 | putative | | | | |
| ORF853 | 916658 | 915303 | enolase | L29475 | *Bacillus subtilis* | 1036 | 60 |
| ORF854 | 915627 | 915376 | enolase | U43738 | *Mycoplasma pneumoniae* | 226 | 65 |
| ORF855 | 917707 | 916853 | excinuclease ABC subunit B (uvrB) | U32804 | *Haemophilus influenzae* | 724 | 46 |
| ORF856 | 918837 | 917722 | excinuclease ABC subunit B (uvrB) | U32804 | *Haemophilus influenzae* | 1029 | 54 |
| ORF857 | 919868 | 918837 | tryptophanyl-tRNA synthetase (trpS) | U32746 | *Haemophilus influenzae* | 376 | 40 |
| ORF858 | 920434 | 919880 | putative | | | | |
| ORF859 | 921187 | 920438 | ORF8 | X82078 | *Chlamydia sp.* | 164 | 50 |
| ORF860 | 921959 | 921195 | hypothetical protein | X62475 | *Chlamydia psittaci* | 511 | 44 |
| ORF861 | 923773 | 921995 | Threonyl tRNA Synthetase | Z80360 | *Bacillus subtilis* | 1476 | 44 |
| ORF862 | 922146 | 922415 | putative | | | | |
| ORF863 | 923943 | 923674 | putative | | | | |
| ORF864 | 924077 | 925006 | putative | | | | |
| ORF865 | 925436 | 925083 | putative | | | | |
| ORF866 | 926524 | 925349 | putative | | | | |
| ORF867 | 927920 | 926433 | putative | | | | |
| ORF868 | 928319 | 927951 | putative | | | | |
| ORF869 | 928963 | 928334 | putative | | | | |
| ORF870 | 929248 | 930987 | DNA mismatch repair protein (mutL) | U32692 | *Haemophilus influenzae* | 585 | 40 |
| ORF871 | 930995 | 932059 | YqhT | D84432 | *Bacillus subtilis* | 445 | 39 |
| ORF872 | 932121 | 933515 | putative | | | | |
| ORF873 | 932881 | 932513 | putative | | | | |
| ORF874 | 933485 | 935746 | pulD (ttg start codon) | M32613 | *Klebsiella pneumoniae* | 210 | 33 |
| ORF875 | 935724 | 937082 | epsE | M96172 | *Vibrio cholerae* | 890 | 55 |
| ORF876 | 937229 | 938410 | PilG | U32588 | *Neisseria gonorrhoeae* | 280 | 38 |
| ORF877 | 938281 | 938805 | putative | | | | |
| ORF878 | 938809 | 939255 | putative | | | | |
| ORF879 | 939165 | 939782 | putative | | | | |
| ORF880 | 939760 | 940791 | putative | | | | |
| ORF881 | 940822 | 941106 | putative | | | | |
| ORF882 | 940977 | 941351 | putative | | | | |
| ORF883 | 942537 | 941623 | yscT | L25667 | *Yersinia pseudotuberculosis* | 169 | 44 |
| ORF884 | 942784 | 942500 | yscS | L25667 | *Yersinia pseudotuberculosis* | 173 | 42 |
| ORF885 | 943149 | 942799 | HrcR | AE000107 | *Rhizobium sp. NGR234* | 265 | 52 |
| ORF886 | 943799 | 943029 | pathogenicity protein | M64094 | *Xanthomonas campestris* | 252 | 41 |
| ORF887 | 944055 | 943732 | putative | M74011 | *Yersinia enterocolitica* | 112 | 33 |
| ORF888 | 944413 | 943994 | putative | | | | |
| ORF889 | 945395 | 944556 | putative | | | | |
| ORF890 | 945853 | 945389 | putative | | | | |
| ORF891 | 946392 | 945751 | HrcJ | U56662 | *Erwinia amylovora* | 229 | 44 |
| ORF892 . | 947410 | 948081 | putative | | | | |
| ORF893 | 949871 | 948915 | ORF YOR196c | Z75104 | *Saccharomyces cerevisiae* | 702 | 44 |
| ORF894 | 951058 | 949868 | dihydrolipoamide dehydrogenase E3 subunit | M57435 | *Bacillus subtilis* | 745 | 39 |
| ORF895 | 951249 | 950959 | dihydrolipoamide acetyltransferase E3 subunit | M73535 | *Staphylococcus aureus* | 166 | 49 |
| ORF896 | 951664 | 952134 | putative | | | | |
| ORF897 | 952674 | 952165 | SNF | X98455 | *Bacillus cereus* | 229 | 47 |
| ORF898 | 953491 | 952589 | helicase | U39680 | *Mycoplasma genitalium* | 307 | 42 |
| ORF899 | 955324 | 953495 | FO1G4.1 | Z68341 | *Caenorhabditis elegans* | 133 | 57 |
| ORF900 | 955823 | 955281 | putative | | | | |
| ORF901 | 957082 | 955847 | branched-chain amino acid carrier | Z48676 | *Lactobacillus delbrueckii* | 297 | 40 |
| ORF902 | 957902 | 957270 | endonuclease III | U11289 | *Bacillus subtilis* | 317 | 37 |
| ORF903 | 959231 | 957906 | homologous to E.coli 50K | X62539 | *Bacillus subtilis* | 805 | 45 |
| ORF904 | 959376 | 960284 | phosphatidylserine decarboxylase | U72715 | *Chlamydia trachomatis* | 776 | 51 |
| ORF905 | 960266 | 961669 | putative | | | | |
| ORF906 | 961856 | 964765 | secretory component | U06928 | *Caulobacter crescentus* | 1812 | 55 |
| ORF907 | 966855 | 965395 | 28.2% of identity to the Escherichia coli GTP-binding protein Era; putative | L47648 | *Bacillus subtilis* - | 778 | 41 |
| ORF908 | 968204 | 966975 | poly(A) polymerase | L47709 | *Bacillus subtilis* | 383 | 41 |
| ORF909 | 968791 | 968237 | ClpX-like protein | U18229 | *Bacillus subtilis* | 340 | 39 |
| ORF910 | 969498 | 968731 | ATP-dependent protease ATPase subunit | D64006 | *Synechocystis sp.* | 846 | 66 |
| ORF911 | 969858 | 969511 | ClpP | U16135 | *Synechococcus sp.* | 257 | 54 |
| ORF912 | 970118 | 969762 | ATP-dependent clp protease proteolytic component (clpP) | AE000591 | *Helicobacter pylori* | 362 | 63 |
| ORF913 | 970593 | 970300 | putative | | | | |
| ORF914 | 971261 | 970542 | putative | | | | |
| ORF915 | 971680 | 971123 | putative | | | | |
| ORF916 | 971876 | 975100 | SNF | X98455 | *Bacillus cereus* | 778 | 49 |
| ORF917 | 975419 | 976516 | MreB protein | M96343 | *Bacillus subtilis* | 960 | 55 |
| ORF918 | 976584 | 978320 | phospho enol pyruvate carboxykinase | S56812 | *Chlorobium limicola* | 1667 | 64 |
| ORF919 | 977680 | 977231 | putative | | | | |
| ORF920 | 978399 | 980738 | putative | | | | |
| ORF921 | 980756 | 981928 | putative | | | | |
| ORF922 | 982974 | 981931 | precursor protein (AA -22 to 371) | X52557 | *Chlamydia trachomatis* | 97 | 50 |
| ORF923 | 984120 | 983119 | NAD+ dependent glycerol-3-phosphate dehydrogenase | L47648 | *Bacillus subtilis* | 618 | 43 |
| ORF924 | 985502 | 984120 | AgX-1 antigen [human, infertile patient, testis, Peptide, 505 aa] | S73498 | *Homo sapiens* | 254 | 34 |
| ORF925 | 987180 | 985882 | ORF 4 | M72718 | *Bacillus subtilis* | 697 | 38 |
| ORF926 | 987172 | 987444 | putative | | | | |
| ORF927 | 989846 | 989049 | nifU-like protein | AE000542 | *Helicobacter pylori* | 302 | 31 |
| ORF928 | 991048 | 989846 | putative | | | | |
| ORF929 | 991638 | 990955 | phosphoglyceromutase | L09651 | *Zymomonas mobilis* | 471 | 53 |
| ORF930 | 991794 | 992498 | ORFX13 | L09228 | *Bacillus subtilis* | 403 | 39 |
| ORF931 | 993619 | 993041 | biotin [acetyl-CoA-carboxylase] ligase | L47709 | *Bacillus subtilis* | 136 | 38 |
| ORF932 | 993530 | 994792 | rod-shape-determining protein | M22857 | *Escherichia coli* | 312 | 44 |
| ORF933 | 995970 | 994795 | cadmium-transporting ATPase | D64005 | *Synechocystis sp.* | 358 | 47 |
| ORF934 | 996857 | 995739 | ATPase | L28104 | *Transposon Tn5422* | 449 | 39 |
| ORF935 | 997603 | 996782 | putative | | | | |
| ORF936 | 998969 | 997572 | seryl-trna synthetase | Y09924 | *Staphylococcus aureus* | 851 | 42 |
| ORF937 | 998896 | 1000023 | orf2, homologue to B.subtilis ribG | X64395 | *Escherichia coli* | 596 | 40 |
| ORF938 | 1000087 | 1001340 | GTP cyclohydrolase II | D90912 | *Synechocystis sp.* | 1078 | 52 |
| ORF939 | 1001357 | 1001818 | riboflavin synthase beta subunit | U27202 | *Actinobacillus pleuropneumoniae* | 278 | 36 |
| ORF940 | 1003288 | 1001873 | putative | | | | |
| ORF941 | 1003487 | 1004146 | putative | | | | |
| ORF942 | 1004485 | 1005639 | D-alanine glycine permease (dagA) | AE000603 | *Helicobacter pylori* | 394 | 33 |
| ORF943 | 1005643 | 1005972 | hypothetical protein MTCY180.08 | Z97193 | *Mycobacterium tuberculosis* | 274 | 58 |
| ORF944 | 1006784 | 1006116 | similar to trithorax protein in final three exons | U13875 | *Caenorhabditis elegans* | 155 | 46 |
| ORF945 | 1007563 | 1006769 | yycJ | D78193 | *Bacillus subtilis* | 406 | 38 |
| ORF946 | 1009226 | 1007568 | YtpT | AF008220 | *Bacillus subtilis* | 992 | 47 |
| ORF947 | 1009989 | 1009336 | putative | | | | |
| ORF948 | 1015852 | 1016337 | putative | | | | |
| ORF949 | 1016561 | 1016181 | putative | | | | |
| ORF950 | 1016297 | 1017532 | putative | | | | |
| ORF951 | 1016802 | 1016452 | putative | | | | |
| ORF952 | 1018993 | 1017701 | phenolhydroxylase component | U32702 | *Haemophilus influenzae* | 909 | 47 |
| ORF953 | 1019454 | 1019137 | ORF | M63939 | *Escherichia coli* | 96 | 45 |
| ORF954 | 1020764 | 1019562 | pCTHom1 gene product | M94254 | *Chlamydia trachomatis* | 1185 | 65 |
| ORF955 | 1021405 | 1021037 | histone H1-like protein | M80324 | *Chlamydia psittaci* | 319 | 62 |
| ORF956 | 1021821 | 1024286 | phosphoprotein | L25078 | *Chlamydia trachomatis* | 739 | 41 |
| ORF957 | 1024697 | 1024248 | putative | | | | |
| ORF958 | 1025569 | 1024508 | protoporphyrinogen oxidase | U25114 | *Mus musculus* | 86 | 38 |
| ORF959 | 1026969 | 1025590 | oxygen independent coprophorphyrinogen III oxidase | D90912 | *Synechocystis sp.* | 880 | 42 |
| ORF960 | 1027789 | 1026947 | uroporphyrinogen decarboxylase | M97208 | *Bacillus subtilis* | 372 | 38 |
| ORF961 | 1031199 | 1027945 | transcription-repair coupling factor (trcF) (mfd) | U32805 | *Haemophilus influenzae* | 1584 | 42 |
| ORF962 | 1031717 | 1031172 | alanyl-tRNA synthetase | X95571 | *Thiobacillus ferrooxidans* | 76 | 31 |
| ORF963 | 1033057 | 1031612 | alanyl-tRNA synthetase | AE000353 | *Escherichia coli* | 889 | 40 |
| ORF964 | 1033425 | 1033039 | alanyl-tRNA synthetase (alaS) | AE000629 | *Helicobacter pylori* | 327 | 51 |
| ORF965 | 1033784 | 1033200 | alanyl-tRNA synthetase | X59956 | *Rhizobium leguminosarum* | 416 | 47 |
| ORF966 | 1033963 | 1036038 | transketolase | Z73234 | *Bacillus subtilis* | 1398 | 44 |
| ORF967 | 1036945 | 1036010 | AMP nucleosidase | AE000290 | *Escherichia coli* | 265 | 42 |
| ORF968 | 1037110 | 1037679 | elongation factor P | U14003 | *Escherichia coli* | 458 | 51 |
| ORF969 | 1037696 | 1037944 | putative | | | | |
| ORF970 | 1038916 | 1037975 | putative | | | | |
| ORF971 | 1040582 | 1039026 | HSP60 chaperonin | X62914 | *Clostridium perfringens* | 284 | 31 |
| ORF972 | 1040997 | 1042337 | PROBABLE UDP-N-ACETYLMURAMOYLALANYL-D-GLUTAMYL-2, 6-DIAMINOLIGASE (EC 6.3.2.15). | AB001488 | *Bacillus subtilis* | 446 | 39 |
| ORF973 | 1042357 | 1043403 | ORF-Y (AA 1-360) | X51584 | *Escherichia coli* | 582 | 45 |
| ORF974 | 1043367 | 1044623 | UDP-N-acetylmuramoylalanine-D-glutamate ligase (murD) | U32793 | *Haemophilus influenzae* | 348 | 42 |
| ORF975 | 1044607 | 1045362 | hypothetical protein | Y14079 | *Bacillus subtilis* | 115 | 38 |
| ORF976 | 1045384 | 1046538 | spoVE gene product (AA 1-366) | X51419 | *Bacillus subtilis* | 479 | 35 |
| ORF977 | 1046447 | 1047517 | mur | Y13922 | *Enterococcus hirae* | 256 | 45 |
| ORF978 | 1047521 | 1049956 | UDP-N-acetylmuramate-alanine ligase (murC) | U32794 | *Haemophilus influenzae* | 756 | 38 |
| ORF979 | 1050611 | 1050036 | unknown | Z74024 | *Mycobacterium tuberculosis* | 78 | 44 |
| ORF980 | 1050925 | 1050566 | cycY gene product | U14003 | *Escherichia coli* | 179 | 34 |
| ORF981 | 1051728 | 1051090 | putative | | | | |
| ORF982 | 1051743 | 1052063 | hypothetical protein | D90908 | *Synechocystis sp.* | 135 | 33 |
| ORF983 | 1052101 | 1053126 | tma delta(2)-isopentenylpyrophosphate transferase | Z98209 | *Mycobacterium tuberculosis* | 441 | 37 |
| ORF984 | 1054201 | 1053107 | conserved hypothetical protein | AE000579 | *Helicobacter pylori* | 826 | 44 |
| ORF985 | 1054242 | 1055555 | putative | | | | |
| ORF986 | 1055483 | 1055908 | putative | | | | |
| ORF987 | 1056609 | 1056965 | YqeL | D84432 | *Bacillus subtilis* | 202 | 38 |
| ORF988 | 1056961 | 1058232 | beta-ketoacyl-ACP synthase | L13242 | *Ricinus communis* | 1266 | 55 |
| ORF989 | 1058238 | 1058687 | diadenosine tetraphosphatase | U30313 | *Homo sapiens* | 122 | 42 |
| ORF990 | 1059371 | 1058727 | inorganic pyrophosphatase (ppa) | AE000576 | *Helicobacter pylori* | 209 | 39 |
| ORF991 | 1059526 | 1060578 | leucine dehydrogenase LeuDH | U51099 | *Bacillus cereus* | 680 | 45 |
| ORF992 | 1061553 | 1060579 | 3'(2'),5'-bisphosphate nucleotidase | U40433 | *Arabidopsis thaliana* | 335 | 43 |
| ORF993 | 1061674 | 1062411 | putative | | | | |
| ORF994 | 1062377 | 1064077 | 2-acylglycerophosphoethanolamine acyl transferase/acyl carrier protein synthetase | U29581 | *Escherichia coli* | 383 | 44 |
| ORF995 | 1064116 | 1065243 | 7-keto-8-aminopelargonic acid synthetase (bioF) | M29291 | *Bacillus sphaericus* | 200 | 35 |
| ORF996 | 1067451 | 1065178 | priA | Y10304 | *Bacillus subtilis* | 1009 | 43 |
| ORF997 | 1068065 | 1067376 | putative | | | | |
| ORF998 | 1068209 | 1068706 | putative | | | | |
| ORF999 | 1069958 | 1068819 | unknown | U41759 | *Chlamydia psittaci* | 777 | 41 |
| ORF1000 | 1071163 | 1070033 | unknown | U41759 | *Chlamydia psittaci* | 381 | 36 |
| ORF1001 | 1072438 | 1071332 | unknown | U41759 | *Chlamydia psittaci* | 254 | 37 |
| ORF1002 | 1072997 | 1073476 | putative | | | | |
| ORF1003 | 1074239 | 1075864 | lysyl-tRNA synthetase | D90906 | *Synechocystis sp.* | 1007 | 48 |
| ORF1004 | 1076790 | 1075867 | cysteinyl-tRNA synthetase | L14580 | *Bacillus subtilis* | 395 | 52 |
| ORF1005 | 1077268 | 1076573 | cys-tRNA synthetase (cysS) | U32693 | *Haemophilus influenzae* | 431 | 56 |
| ORF1006 | 1077999 | 1078724 | putative | | | | |
| ORF1007 | 1079088 | 1078672 | ribonuclease P protein component (gtg start codon) | M11056 | *Escherichia coli* | 78 | 46 |
| ORF1008 | 1079642 | 1079944 | 30S ribosomal subunit protein S14 | U18997 | *Escherichia coli* | 260 | 50 |
| ORF1009 | 1080501 | 1079995 | F18C12.2 | Z75536 | *Caenorhabditis elegans* | 118 | 38 |
| ORF1010 | 1080775 | 1081341 | putative | | | | |
| ORF1011 | 1083158 | 1081350 | deoxyribodipyrimidine photolyase | J03294 | *Bacillus subtilis* | 687 | 44 |
| ORF1012 | 1084677 | 1083235 | DNA mismatch repair protein | U71154 | *Aquifex pyrophilus* | 735 | 48 |
| ORF1013 | 1085648 | 1084632 | DNA mismatch repair protein | D90909 | *Synechocystis sp.* | 565 | 39 |
| ORF1014 | 1086117 | 1086737 | DNA primase (dnaG) | U32735 | *Haemophilus influenzae* | 303 | 40 |
| ORF1015 | 1086692 | 1087897 | DnaG | Z83860 | *Mycobacterium tuberculosis* | 222 | 37 |
| ORF1016 | 1088646 | 1089005 | putative | | | | |
| ORF1017 | 1089146 | 1089805 | putative | | | | |
| ORF1018 | 1092931 | 1089890 | glycyl-tRNA synthetase | U20547 | *Chlamydia trachomatis* | 2569 | 48 |
| ORF1019 | 1093179 | 1092889 | putative | | | | |
| ORF1020 | 1093584 | 1094204 | phosphatidylglycerophosphate synthase | U87792 | *Bacillus subtilis* | 163 | 55 |
| ORF1021 | 1095619 | 1094192 | glycogen (starch) synthase | D90899 | *Synechocystis sp.* | 574 | 40 |
| ORF1022 | 1096074 | 1096628 | partial ctc gene product (AA 1-186) | X16518 | *Bacillus subtilis* | 86 | 37 |
| ORF1023 | 1096633 | 1097082 | peptidyl-tRNA hydrolase | U31570 | *Chlamydia trachomatis* | 378 | 53 |
| ORF1024 | 1097266 | 1097601 | ribosomal protein S6 (rps6) | AE000630 | *Helicobacter pylori* | 179 | 39 |
| ORF1025 | 1097622 | 1097867 | ribosomal protein S18 homolog; putative | M62820 | *Chlamydia trachomatis* | 324 | 86 |
| ORF1026 | 1097886 | 1098392 | putative heat shock protein ORF; putative | M62820 | *Chlamydia trachomatis* | 190 | 79 |
| ORF1027 | 1099521 | 1099279 | putative | | | | |
| ORF1028 | 1099689 | 1101053 | putative | | | | |
| ORF1029 | 1102192 | 1101107 | putative | | | | |
| ORF1030 | 1104950 | 1102116 | glycerol-3-phosphate acyltransferase | M80571 | *Cucumis sativus* | 574 | 43 |
| ORF1031 | 1106508 | 1104946 | ORF_f495; orfF of ECMRED, uses 2nd start | U18997 | *Escherichia coli* | 855 | 38 |
| ORF1032 | 1106722 | 1107249 | putative | | | | |
| ORF1033 | 1107463 | 1108101 | PlsX | U59433 | *Bacillus subtilis* | 282 | 45 |
| ORF1034 | 1108041 | 1108421 | fatty acid/phospholipid synthesis protein (plsX) | AE000540 | *Helicobacter pylori* | 205 | 35 |
| ORF1035 | 1108520 | 1113370 | putative 98 kDa outer membrane protein | U72499 | *Chlamydia psittaci* | 352 | 44 |
| ORF1036 | 1114958 | 1113447 | putative | | | | |
| ORF1037 | 1116915 | 1115071 | lipid A disaccharide synthetase (lpxB) | U32786 | *Haemophilus influenzae* | 477 | 42 |
| ORF1038 | 1118183 | 1116894 | poly(A) polymerase | AE000123 | *Escherichia coli* | 555 | 46 |
| ORF1039 | 1118846 | 1120030 | putative | L12968 | *Escherichia coli* | 880 | 50 |
| ORF1040 | 1120040 | 1120522 | glucosamine fructose-6-phosphate aminotransferase (isomerizing) (glmS) | AE000651 | *Helicobacter pylori* | 396 | 52 |
| ORF1041 | 1120510 | 1121430 | glutamine amidotransferase; glucosamine--fructose-6-phosphate aminotransferase | AE000450 | *Escherichia coli* | 494 | 44 |
| ORF1042 | 1121321 | 1121866 | L-glutamine:D-fructose-6-P amidotransferase precursor | U17352 | *Thermus aquaticus thermophilus* | 374 | 50 |
| ORF1043 | 1122123 | 1122899 | tyrosine-specific transport protein | AE000284 | *Escherichia coli* | 281 | 41 |
| ORF1044 | 1124842 | 1125564 | putative | | | | |
| ORF1045 | 1126526 | 1125579 | cell division protein (ftsY) | U32760 | *Haemophilus influenzae* | 497 | 41 |
| ORF1046 | 1126519 | 1127676 | succinyl-CoA synthetase beta-subunit | J01619 | *Escherichia coli* | 784 | 43 |
| ORF1047 | 1127672 | 1128571 | succinyl coenzyme A synthetase alpha subunit | U23408 | *Dictyostelium discoideum* | 978 | 63 |
| ORF1048 | 1130230 | 1131336 | putative | | | | |
| ORF1049 | 1131480 | 1132553 | putative | | | | |
| ORF1050 | 1132830 | 1133843 | putative | | | | |
| ORF1051 | 1134121 | 1134855 | serine protease HtrA | D90905 | *Synechocystis sp.* | 307 | 51 |
| ORF1052 | 1134642 | 1135592 | GsrA protein | D78376 | *Yersinia enterocolitica* | 497 | 41 |
| ORF1053 | 1135964 | 1135653 | putative | | | | |
| ORF1054 | 1137132 | 1135954 | R11H6.1 | Z93386 | *Caenorhabditis elegans* | 445 | 37 |
| ORFI055 | 1137169 | 1140102 | Ydr430cp; CAI: 0.15 | U33007 | *Saccharomyces cerevisiae* | 559 | 40 |
| ORF1056 | 1141365 | 1140112 | hypothetical 54.7 kD protein in udp 3' region precursor (o475) | AE000459 | *Escherichia coli* | 222 | 34 |
| ORF1057 | 1142150 | 1141356 | phosphatidylserine synthase (pssA) | AE000614 | *Helicobacter pylori* | 307 | 41 |
| ORF1058 | 1142520 | 1145660 | ribonucleotide reductase subunit M1 | K02927 | *Mus musculus* | 1433 | 45 |
| ORF1059 | 1145627 | 1146721 | ribonucleoside diphosphate reductase, beta subunit (nrdB) | AE000553 | *Helicobacter pylori* | 443 | 32 |
| ORF1060 | 1146862 | 1147545 | unknown | Z95398 | *Mycobacterium leprae* | 191 | 35 |
| ORF1061 | 1147666 | 1148190 | YtqB | AF008220 | *Bacillus subtilis* | 262 | 44 |
| ORF1062 | 1148514 | 1148224 | ORF2 | U01958 | *Bacillus licheniformis* | 135 | 54 |
| ORF1063 | 1149136 | 1148348 | ORF2 | M31827 | *Bacillus subtilis* | 268 | 40 |
| ORF1064 | 1149702 | 1149166 | putative | | | | |
| ORF1065 | 1150031 | 1150591 | unknown | Z85982 | *Mycobacterium tuberculosis* | 445 | 49 |
| ORF1066 | 1150785 | 1151147 | ribosomal protein L20 (AA 1-119) | X16188 | *Bacillus stearothermophilus* | 273 | 44 |
| ORF1067 | 1151165 | 1152181 | phenylalany-tRNA synthetase beta subunit | Z75208 | *Bacillus subtilis* | 777 | 40 |
| ORF1068 | 1152522 | 1154591 | putative | | | | |
| ORF1069 | 1155666 | 1154566 | putative | | | | |
| ORF1070 | 1156743 | 1155670 | putative | | | | |
| ORF1071 | 1156859 | 1157815 | hypothetical | U32723 | *Haemophilus influenzae* | 252 | 42 |
| ORF1072 | 1157982 | 1160735 | ATP-binding protein | U01376 | *Escherichia coli* | 1314 | 56 |
| ORF1073 | 1162620 | 1160917 | polynucleotide phosphorylase | AF010578 | *Pisum sativum* | 1416 | 52 |
| ORF1074 | 1162970 | 1162590 | polyribonucleotide phophorylase | U52048 | *Spinacia oleracea* | 312 | 53 |
| ORF1075 | 1163532 | 1164020 | orf150 gene product | X95938 | *Porphyromonas gingivalis* | 335 | 43 |
| ORF1076 | 1163995 | 1164294 | putative | | | | |
| ORF1077 | 1165569 | 1165030 | putative | | | | |
| ORF1078 | 1166108 | 1165566 | putative | | | | |
| ORF1079 | 1166644 | 1166141 | putative | | | | |
| ORF1080 | 1167055 | 1168374 | putative | | | | |
| ORF1081 | 1169218 | 1168337 | methionine aminopeptidase | D64003 | *Synechocystis sp.* | 488 | 54 |
| ORF1082 | 1169823 | 1169218 | ORF o197 | U18997 | *Escherichia coli* | 281 | 30 |
| ORF1083 | 1171324 | 1170572 | putative | | | | |
| ORF1084 | 1172085 | 1171177 | hypothetical | U32720 | *Haemophilus influenzae* | 162 | 44 |
| ORF1085 | 1172394 | 1173773 | fumarase | D64000 | *Synechocystis sp.* | 1292 | 57 |
| ORF1086 | 1175209 | 1173881 | prs-associated putative membrane protein | U02424 | *Escherichia coli* | 570 | 39 |
| ORF1087 | 1175555 | 1175127 | hypothetical protein in pth-prs intergenic region | AE000219 | *Escherichia coli* | 278 | 46 |
| ORF1088 | 1175778 | 1177043 | hypothetical protein | Z96072 | *Mycobacterium tuberculosis* | 109 | 43 |
| ORF1089 | 1177177 | 1179048 | putative | | | | |
| ORF1090 | 1179156 | 1180085 | penicillin tolerance protein (lytB) | U32781 | *Haemophilus influenzae* | 731 | 54 |
| ORF1091 | 1180045 | 1180779 | putative | | | | |
| ORF1092 | 1181942 | 1180788 | putative | | | | |
| ORF1093 | 1182296 | 1181961 | putative | | | | |
| ORF1094 | 1183844 | 1182300 | putative | | | | |
| ORF1095 | 1184420 | 1183848 | putative | | | | |
| ORF1096 | 1185382 | 1184366 | putative | | | | |
| ORF1097 | 1185858 | 1185226 | putative | | | | |
| ORF 1098 | 1186164 | 1186481 | putative | | | | |
| ORF1099 | 1187386 | 1186484 | site-specific recombinase | U92524 | *Salmonella typhimurium* | 401 | 48 |
| ORF1100 | 1187370 | 1189028 | phophoglucoisomerase-like protein | L40822 | *Chlamydia trachomatis* | 1154 | 63 |
| ORF1101 | 1189321 | 1190889 | putative | | | | |
| ORF1102 | 1191142 | 1192146 | NADP-malate dehydrogenase | L40958 | *Flaveria bidentis* | 775 | 46 |
| ORF1103 | 1191974 | 1191729 | putative | | | | |
| ORF1104 | 1193815 | 1192991 | putative | | | | |
| ORF1105 | 1195702 | 1194248 | o460; This 460 aa orf is 46 pct identical (26 gaps) to 458 residues of an approx. 488 aa protein ARCD PSEAE SW: P18275 | AE000256 | *Escherichia coli* | 1022 | 44 |
| ORF1106 | 1196303 | 1195716 | putative | | | | |
| ORF1107 | 1196831 | 1196337 | putative | | | | |
| ORF1108 | 1197807 | 1196746 | putative | | | | |
| ORF1109 | 1198740 | 1197883 | putative | | | | |
| ORF1110 | 1200232 | 1198721 | shikimate 5-dehydrogenase | U67551 | *Methanococcus jannaschii* | 245 | 37 |
| ORF1111 | 1201286 | 1200135 | 3-dehydroquinate synthase (aroB) | U32705 | *Haemophilus influenzae* | 478 | 45 |
| ORF1112 | 1202386 | 1201259 | 2,3-dihydroxybenzoic acid | L29562 | *Vibrio anguillarum* | 780 | 50 |
| ORF1113 | 1202901 | 1202350 | putative | | | | |
| ORF1114 | 1204162 | 1202816 | 5-enolpyruvylshikimate 3-phosphate synthase | U67500 | *Methanococcus jannaschii* | 520 | 40 |
| ORF1115 | 1203177 | 1203464 | putative | | | | |
| ORF1116 | 1205028 | 1204180 | putative | | | | |
| ORF1117 | 1206392 | 1204878 | bioA gene product | A02587 | *unidentified* | 834 | 48 |
| ORF1118 | 1206742 | 1206086 | dethiobiotin synthase (bioD) | U32830 | *Haemophilus influenzae* | 243 | 37 |
| ORF1119 | 1207872 | 1206724 | L-alanine - pimelyl CoA ligase | U51868 | *Bacillus subtilis* | 601 | 41 |
| ORF1120 | 1208852 | 1207851 | biotin sythase | U24147 | *Arabidopsis thaliana* | 892 | 52 |
| ORF1121 | 1210518 | 1209742 | tryptophan hydroxylase | U26428 | *Gallus gallus* | 237 | 34 |
| ORF 1122 | 1210703 | 1211494 | dihydrodipicolinate reductase | U47017 | *Pseudomonas syringae pv. tabaci* | 345 | 37 |
| ORF1123 | 1211870 | 1212754 | aspartate-semialdehyde dehydrogenase | U67476 | *Methanococcus jannaschii* | 444 | 43 |
| ORF1124 | 1212742 | 1214064 | aspartokinase III | U00006 | *Escherichia coli* | 473 | 47 |
| ORF1125 | 1214046 | 1214858 | dihydrodipicolinate synthase | D64006 | *Synechocystis sp.* | 238 | 40 |
| ORF1126 | 1215551 | 1216318 | putative | | | | |
| ORF1127 | 1216493 | 1216849 | putative | | | | |
| ORF1128 | 1217183 | 1219612 | putative | | | | |
| ORF1129 | 1220068 | 1219673 | putative | | | | |
| ORF1130 | 1219710 | 1220669 | putative | | | | |
| ORF1131 | 1220630 | 1221376 | putative | | | | |
| ORF1132 | 1221645 | 1223681 | unknown | D26185 | *Bacillus subtilis* | 621 | 43 |
| ORF1133 | 1223894 | 1224988 | putative | | | | |
| ORF1134 | 1225000 | 1225830 | high level kasgamycin resistance | D26185 | *Bacillus subtilis* | 422 | 41 |
| ORF1135 | 1227810 | 1225879 | hypothetical protein | D90903 | *Synechocystis sp.* | 1129 | 43 |
| ORF1136 | 1226528 | 1226908 | putative | | | | |
| ORF1137 | 1229972 | 1228311 | exonuclease VII, large subunit (xseA) | U32723 | *Haemophilus influenzae* | 666 | 46 |
| ORF1138 | 47569 | 47018 | Integrase/recombinase | AE001308 | *Chlamydia trachomatis* | 716 | 72 |
| ORF1139 | 49980 | 49117 | putative | | | | |
| ORF1140 | 53356 | 52898 | putative | | | | |
| ORF1141 | 54477 | 54884 | O-Sialoglycoprotein Endopeptidase | AE001307 | *Chlamydia trachomatis* | 311 | 51 |
| ORF1142 | 63753 | 63998 | PTS PEP Phosphotransferase | AE001306 | *Chlamydia trachomatis* | 198 | 61 |
| ORF1143 | 77164 | 77487 | putative | | | | |
| ORF1144 | 79724 | 79302 | Sms Protein | AE001302 | *Chlamydia trachomatis* | 458 | 57 |
| ORF1145 | 88721 | 88951 | putative | | | | |
| ORF1146 | 94067 | 94429 | putative | | | | |
| ORF1147 | 122832 | 123341 | hypothetical protein | AE001303 | *Chlamydia trachomatis* | 398 | 61 |
| ORF1148 | 147536 | 147234 | putative | | | | |
| ORF1149 | 158990 | 159346 | S16 Ribosomal Protein | AE001277 | *Chlamydia trachomatis* | 467 | 78 |
| ORF1150 | 168470 | 168979 | putative | | | | |
| ORF1151 | 169183 | 169452 | putative | | | | |
| ORF1152 | 171785 | 171504 | Cationic Amino Acid Transporter | AE001278 | *Chlamydia trachomatis* | 262 | 68 |
| ORF1153 | 172518 8 | 171775 | Cationic Amino Acid Transporter | AE001278 | *Chlamydia trachomatis* | 533 | 48 |
| ORF1154 | 193599 | 194045 | putative | | | | |
| ORF1155 | 195704 | 196075 | S/T Protein Kinase | AE001288 | *Chlamydia trachomatis* | 536 | 82 |
| ORF1156 | 210687 | 210145 | KDO-transferase | X80061 | *Chlamydia pneumoniae* | 856 | 96 |
| ORF1157 | 211100 | 210708 | putative | | | | |
| ORF1158 | 215420 | 215088 | putative | | | | |
| ORF1159 | 217914 | 218246 | putative | | | | |
| ORF1160 | 218925 | 218701 | putative | | | | |
| ORF1161 | 223785 | 223525 | IMP dehydrogenase | U13372 | *Borrelia burgdorferi* | 270 | 63 |
| ORF1162 | 224271 | 223999 | putative | | | | |
| ORF1163 | 228691 | 228407 | putative | | | | |
| ORF1164 | 235050 | 235334 | (Methylase) | AE001287 | *Chlamydia trachomatis* | 331 | 66 |
| ORF1165 | 252308 | 253021 | Oligopeptide Permease | AE001293 | *Chlamydia trachomatis* | 838 | 72 |
| ORF1166 | 258280 | 258912 | Dicarboxylate Translocator | AE001294 | *Chlamydia trachomatis* | 909 | 80 |
| ORF1167 | 261325 | 261567 | putative | | | | |
| ORF1168 | 268195 | 268878 | hypothetical protein | AE001287 | *Chlamydia trachomatis* | 556 | 52 |
| ORF1169 | 269447 | 268881 | putative | | | | |
| ORF1170 | 271263 | 271538 | putative | | | | |
| ORF1171 | 271957 | 272346 | putative | | | | |
| ORF1172 | 274176 | 274550 | putative | | | | |
| ORF1173 | 275736 | 275314 | Disulfide bond Oxidoreductase | AE001291 | *Chlamydia trachomatis* | 519 | 73 |
| ORF1174 | 276490 | 276927 | hypothetical protein | AE001291 | *Chlamydia trachomatis* | 249 | 53 |
| ORF1175 | 277577 | 277861 | hypothetical protein | AE001291 | *Chlamydia trachomatis* | 256 | 52 |
| ORF1176 | 288163 | 287909 | putative | | | | |
| ORF1177 | 290130 | 289789 | putative | | | | |
| ORF1178 | 290989 | 291225 | putative | | | | |
| ORF1179 | 291372 | 291860 | adenylate cyclase | AE001286 | *Chlamydia trachomatis* | 388 | 48 |
| ORF1180 | 311239 | 311622 | putative | | | | |
| ORF1181 | 328665 | 328384 | putative | | | | |
| ORF1182 | 337348 | 338289 | sodium-dependent transporter | AF017105 | *Chlamydia psittaci* | 1112 | 72 |
| ORF1183 | 364764 | 364369 | Prolipoprotein Diacylglycerol Transferase | AE001298 | *Chlamydia trachomatis* | 300 | 54 |
| ORF1184 | 389623 | 390135 | hypothetical protein | AE001282 | *Chlamydia trachomatis* | 75 | 33 |
| ORF1185 | 393729 | 394343 | ABC superfamily ATPase | AE001282 | *Chlamydia trachomatis* | 473 | 52 |
| ORF1186 | 407379 | 407621 | putative | | | | |
| ORF1187 | 410944 | 410708 | putative | | | | |
| ORF1188 | 427632 | 427988 | putative | | | | |
| ORF1189 | 428172 | 428486 | putative | | | | |
| ORF1190 | 436761 | 437246 | hypothetical protein | AE001279 | *Chlamydia trachomatis* | 661 | 81 |
| ORF1191 | 460911 | 461159 | putative | | | | |
| ORF1192 | 477597 | 477313 | hypothetical protein | AE001300 | *Chlamydia trachomatis* | 309 | 62 |
| ORF1193 | 487303 | 487001 | putative | | | | |
| ORF1194 | 487764 | 487534 | Glycine Cleavage System H Protein | AE001300 | *Chlamydia trachomatis* | 221 | 67 |
| ORF1195 | 498502 | 499017 | hypothetical protein | AE001275 | *Chlamydia trachomatis* | 206 | 32 |
| ORF1196 | 499795 | 500466 | putative | | | | |
| ORF1197 | 571928 | 572344 | putative | | | | |
| ORF1198 | 572367 | 572131 | putative | | | | |
| ORF1199 | 588184 | 587915 | hypothetical protein | AE001312 | *Chlamydia trachomatis* | 256 | 62 |
| ORF1200 | 600587 | 600907 | (Metalloenzyme) | AE001316 | *Chlamydia trachomatis* | 314 | 61 |
| ORF1201 | 609731 | 608895 | putative | | | | |
| ORF1202 | 614039 | 614755 | hypothetical protein | AE001317 | *Chlamydia trachomatis* | 475 | 46 |
| ORF1203 | 614823 | 615152 | putative | | | | |
| ORF1204 | 638244 | 638831 | ABC Transporter ATPase | AE001315 | *Chlamydia trachomatis* | 614 | 61 |
| ORF1205 | 638819 | 639094 | (Metal Transport Protein) | AE001315 | *Chlamydia trachomatis* | 265 | 63 |
| ORF1206 | 639073 | 639636 | (Metal Transport Protein) | AE001315 | *Chlamydia trachomatis* | 687 | 69 |
| ORF1207 | 647901 | 648236 | hypothetical protein | AE001317 | *Chlamydia trachomatis* | 139 | 38 |
| ORF1208 | 678510 | 679469 | phosphohydrolase | AE001320 | *Chlamydia trachomatis* | 995 | 63 |
| ORF1209 | 688178 | 688732 | hypothetical protein | AE001320 | *Chlamydia trachomatis* | 366 | 43 |
| ORF1210 | 696045 | 696563 | methyltransferase | AE001321 | *Chlamydia trachomatis* | 369 | 49 |
| ORF1211 | 708998 | 708588 | Glucose-1-P Adenyltransferase | AE001322 | *Chlamydia trachomatis* | 507 | 83 |
| ORF1212 | 709808 | 710089 | putative | | | | |
| ORF1213 | 718240 | 717737 | Glycerol-3-P Phosphatidyltransferase | AE001323 | *Chlamydia trachomatis* | 573 | 66 |
| ORF1214 | 737828 | 737565 | S19 Ribosomal Protein | AE001323 | *Chlamydia trachomatis* | 439 | 94 |
| ORF1215 | 779502 | 780257 | hypothetical protein | AE001322 | *Chlamydia trachomatis* | 476 | 48 |
| ORF1216 | 806310 | 805864 | hypothetical protein | AE001337 | *Chlamydia trachomatis* | 512 | 67 |
| ORF1217 | 820931 | 820707 | putative | | | | |
| ORF1218 | 837696 | 839096 | Exodeoxyribonuclease V, Gamma | AE001334 | *Chlamydia trachomatis* | 967 | 49 |
| ORF1219 | 883307 | 883549 | putative | | | | |
| ORF1220 | 892010 | 891726 | putative | | | | |
| ORF1221 | 893277 | 893564 | putative | | | | |
| ORF1222 | 936998 | 937225 | Gen. Secretion Protein E | AE001327 | *Chlamydia trachomatis* | 256 | 67 |
| ORF1223 | 946865 | 947419 | putative | | | | |
| ORF1224 | 975187 | 975411 | SWF/SNF family helicase | AE001341 | *Chlamydia trachomatis* | 363 | 96 |
| ORF1225 | 985882 | 985517 | hypothetical protein | AE001342 | *Chlamydia trachomatis* | 166 | 33 |
| ORF1226 | 987713 | 987180 | hypothetical protein | AE001342 | *Chlamydia trachomatis* | 447 | 59 |
| ORF1227 | 988215 | 987733 | Flagellar M-Ring Protein | AE001342 | *Chlamydia trachomatis* | 304 | 44 |
| ORF1228 | 988754 | 988530 | Flagellar M-Ring Protein | AE001342 | *Chlamydia trachomatis* | 92 | 36 |
| ORF1229 | 992542 | 992841 | hypothetical protein | AE001343 | *Chlamydia trachomatis* | 112 | 39 |
| ORF1230 | 992759 | 993067 | hypothetical protein | AE001343 | *Chlamydia trachomatis* | 100 | 32 |
| ORF1231 | 1004247 | 1004528 | D-Ala/Gly Permease | AE001344 | *Chlamydia trachomatis* | 283 | 64 |
| ORF1232 | 1015013 | 1014294 | 235aa long hypothetical protein | AB009472 | *Pyrococcus horikoshii* | 104 | 54 |
| ORF1233 | 1056147 | 1056545 | putative | | | | |
| ORF1234 | 1077682 | 1078035 | predicted disulfide bond isomerase | AE001351 | *Chlamydia trachomatis* | 233 | 46 |
| ORF1235 | 1088121 | 1088381 | putative | | | | |
| ORF1236 | 1098430 | 1098852 | Predicted Kinase | AE001352 | *Chlamydia trachomatis* | 384 | 59 |
| ORF1237 | 1098798 | 1099319 | Predicted Kinase | AE001352 | *Chlamydia trachomatis* | 322 | 45 |
| ORF1238 | 1123198 | 1123515 | Transport Permease | AE001354 | *Chlamydia trachomatis* | 313 | 72 |
| ORF1239 | 1123606 | 1124256 | Tyrosine Transport | AE001354 | *Chlamydia trachomatis* | 577 | 58 |
| ORF1240 | 1124453 | 1124797 | Tyrosine Transport | AE001354 | *Chlamydia trachomatis* | 323 | 50 |
| ORF1241 | 1129253 | 1129567 | putative | | | | |
| ORF1242 | 1164947 | 1164474 | hypothetical protein | AE001357 | *Chlamydia trachomatis* | 412 | 56 |
| ORF1243 | 1170457 | 1170053 | hypothetical protein | AE001358 | *Chlamydia trachomatis* | 283 | 59 |
| ORF1244 | 1172342 | 1171863 | ABC transporter permease | AE001358 | *Chlamydia trachomatis* | 457 | 55 |
| ORF1245 | 1192155 | 1192835 | putative | | | | |
| ORF1246 | 1192759 | 1192992 | putative | | | | |
| ORF1247 | 1193861 | 1194142 | putative | | | | |
| ORF1248 | 1194036 | 1193779 | (D-Amino Acid Dehydrogenase) | AE001311 | *Chlamydia trachomatis* | 269 | 79 |
| ORF1249 | 1209748 | 1209053 | conserved hypothetical protein | AE000958 | *Archaeoglobus fulgidus* | 121 | 38 |
| ORF1250 | 1215111 | 1215419 | putative | | | | |
| ORF12S1 | 1216302 | 1216538 | putative | | | | |
| ORF1252 | 1228072 | 1227818 | hypothetical protein | AE001306 | *Chlamydia trachomatis* | 134 | 39 |
| ORF1253 | 1228304 | 1228080 | xseB | AL021897 | *Mycobacterium tuberculosis* | 89 | 33 |
| ORF1254 | 26599 | 26222 | putative | | | | |
| ORF1255 | 27609 | 27367 | putative | | | | |
| ORF1256 | 67206 | 66967 | putative | | | | |
| ORF1257 | 70612 | 70352 | putative | | | | |
| ORF1258 | 132703 | 132945 | putative | | | | |
| ORF1259 | 178073 | 178393 | putative | | | | |
| ORF1260 | 208576 | 208349 | putative | | | | |
| ORF1261 | 209156 | 208929 | putative | | | | |
| ORF1262 | 209263 | 209024 | putative | | | | |
| ORF1263 | 210304 | 210639 | putative | | | | |
| ORF1264 | 299009 | 299452 | putative | | | | |
| ORF1265 | 352106 | 351717 | putative | | | | |
| ORF1266 | 420182 | 419949 | Flagellar Secretion Protein | AE001280 | *Chlamydia trachomatis* | 115 | 43 |
| ORF1267 | 553602 | 553381 | putative | | | | |
| ORF1268 | 556538 | 556807 | putative | | | | |
| ORF1269 | 594348 | 593797 | putative | | | | |
| ORF1270 | 595169 | 594876 | putative | | | | |
| ORF1271 | 662148 | 662381 | putative | | | | |
| ORF1272 | 706528 | 706893 | putative | | | | |
| ORF1273 | 803315 | 803650 | putative | | | | |
| ORF1274 | 849551 | 849306 | putative | | | | |
| ORF1275 | 913676 | 913275 | putative | | | | |
| ORF1276 | 927087 | 926836 | putative | | | | |
| ORF1277 | 930587 | 930360 | putative | | | | |
| ORF1278 | 986531 | 986764 | ORF 12 | M72718 | *Bacillus subtilis* | 106 | 48 |
| ORF1279 | 996229 | 996486 | putative | | | | |
| ORF1280 | 1000373 | 1000002 | putative | | | | |
| ORF1281 | 1010291 | 1010037 | putative | | | | |
| ORF1282 | 1011128 | 1010793 | 106aa long hypothetical protein | AB009472 | *Pyrococcus horikoshii* | 159 | 50 |
| ORF1283 | 1012924 | 1012694 | putative | | | | |
| ORF1284 | 1028659 | 1028913 | putative | | | | |
| ORF1285 | 1086481 | 1086762 | putative | | | | |
| ORF1286 | 1118658 | 1118879 | Phosphoglucomutase | AE001354 | *Chlamydia trachomatis* | 291 | 84 |
| ORF1287 | 1170098 | 1169835 | hypothetical protein | AE001358 | *Chlamydia trachomatis* | 187 | 53 |
| ORF1288 | 1180828 | 1181184 | putative | | | | |
| ORF1289 | 1182658 | 1183035 | putative | | | | |
| ORF1290 . | 1195076 | 1194795 | putative | | | | |
| ORF1291 | 1195890 | 1196183 | putative | | | | |

**Table 2**

| ORF Nos | begin | end | potential start |
|---|---|---|---|
| 2 | 42 | 794 | 42 |
| 3 | 1258 | 1614 | 1261 |
| 4 | 1807 | 2418 | 1807 |
| 5 | 3393 | 2491 | 3393 |
| 6 | 3639 | 4067 | 3639 |
| 7 | 5649 | 4270 | 5649 |
| 8 | 7463 | 6012 | 7463 |
| 9 | 8051 | 8962 | 8051 |
| 10 | 9129 | 9959 | 9138 |
| 11 | 10687 | 10361 | 10639 |
| 12 | 10927 | 11232 | 10927 |
| 13 | 11246 | 12727 | 11246 |
| 14 | 12691 | 14190 | 12691 |
| 15 | 14484 | 17249 | 14484 |
| 16 | 16039 | 15770 | 16036 |
| 17 | 17845 | 20853 | 17845 |
| 18 | 21137 | 22042 | 21137 |
| 19 | 22046 | 23476 | 22046 |
| 20 | 23681 | 26110 | 23681 |
| 21 | 26109 | 25861 | 26109 |
| 22 | 26241 | 26978 | 26241 |
| 23 | 26960 | 27754 | 26960 |
| 24 | 27747 | 28577 | 27747 |
| 25 | 28887 | 29492 | 28950 |
| 26 | 29432 | 30028 | 29432 |
| 27 | 30024 | 31472 | 30024 |
| 28 | 31758 | 32288 | 31758 |
| 29 | 32201 | 33991 | 32201 |
| 30 | 33852 | 34541 | 33852 |
| 31 | 34783 | 36063 | 34783 |
| 32 | 36009 | 37529 | 36009 |
| 33 | 37881 | 39362 | 37881 |
| 34 | 39418 | 39161 | 39418 |
| 35 | 39366 | 40715 | 39366 |
| 36 | 43076 | 41094 | 43076 |
| 37 | 43800 | 43066 | 43800 |
| 38 | 44828 | 43785 | 44768 |
| 39 | 45340 | 44753 | 45340 |
| 40 | 45752 | 45372 | 45752 |
| 41 | 46996 | 45701 | 46996 |
| 42 | 47961 | 47569 | 47961 |
| 43 | 48960 | 48040 | 48960 |
| 44 | 51452 | 50133 | 51452 |
| 45 | 52606 | 51335 | 52606 |
| 46 | 53684 | 53319 | 53684 |
| 47 | 54195 | 53746 | 54195 |
| 48 | 55278 | 56453 | 55278 |
| 49 | 56493 | 57266 | 56493 |
| 50 | 57297 | 58526 | 57297 |
| 51 | 59851 | 58565 | 59851 |
| 52 | 61495 | 59924 | 61495 |
| 53 | 61324 | 62151 | 61324 |
| 54 | 62132 | 62470 | 62132 |
| 55 | 62474 | 63733 | 62474 |
| 56 | 63881 | 64186 | 63881 |
| 57 | 64611 | 64318 | 64611 |
| 58 | 65485 | 64673 | 65485 |
| 59 | 65999 | 65301 | 65999 |
| 60 | 66244 | 67281 | 66244 |
| 61 | 67265 | 67699 | 67265 |
| 62 | 67703 | 68539 | 67760 |
| 63 | 68805 | 70736 | 68805 |
| 64 | 69172 | 68831 | 69172 |
| 65 | 70642 | 71142 | 70642 |
| 66 | 71325 | 72029 | 71325 |
| 67 | 72060 | 73637 | 72060 |
| 68 | 74061 | 76175 | 74061 |
| 69 | 78351 | 77680 | 78351 |
| 70 | 79356 | 78355 | 79356 |
| 71 | 79983 | 79693 | 79983 |
| 72 | 80441 | 79938 | 80441 |
| 73 | 80475 | 80969 | 80475 |
| 74 | 81296 | 83080 | 81332 |
| 75 | 83291 | 83932 | 83291 |
| 76 | 84005 | 84769 | 84005 |
| 77 | 84975 | 85244 | 84975 |
| 78 | 85123 | 85425 | 85123 |
| 79 | 85397 | 85903 | 85397 |
| 80 | 85909 | 86583 | 85909 |
| 81 | 86626 | 88065 | 86626 |
| 82 | 89257 | 91026 | 89257 |
| 83 | 91291 | 93030 | 91291 |
| 84 | 93295 | 94086 | 93295 |
| 85 | 95285 | 94707 | 95279 |
| 86 | 95667 | 96557 | 95667 |
| 87 | 96317 | 97456 | 96317 |
| 88 | 98435 | 97968 | 98435 |
| 89 | 99460 | 98426 | 99460 |
| 90 | 100144 | 101325 | 100144 |
| 91 | 101457 | 101720 | 101457 |
| 92 | 101704 | 102273 | 101704 |
| 93 | 102356 | 102805 | 102356 |
| 94 | 102835 | 103530 | 102835 |
| 95 | 103549 | 104058 | 103549 |
| 96 | 104096 | 104491 | 104096 |
| 97 | 104601 | 108386 | 104601 |
| 98 | 108401 | 112054 | 108401 |
| 99 | 112033 | 112590 | 112033 |
| 100 | 112672 | 113682 | 112672 |
| 101 | 113726 | 114121 | 113726 |
| 102 | 114711 | 114136 | 114711 |
| 103 | 115267 | 115755 | 115267 |
| 104 | 115911 | 116543 | 115911 |
| 105 | 116736 | 118055 | 116778 |
| 106 | 117968 | 118522 | 117968 |
| 107 | 118530 | 119843 | 118530 |
| 108 | 119816 | 120457 | 119816 |
| 109 | 120451 | 122430 | 120451 |
| 110 | 122504 | 122950 | 122504 |
| 111 | 123528 | 126347 | 123528 |
| 112 | 126332 | 129166 | 126332 |
| 113 | 134690 | 129213 | 134690 |
| 114 | 134925 | 136382 | 134931 |
| 115 | 137870 | 136482 | 137867 |
| 116 | 137899 | 138240 | 137899 |
| 117 | 138239 | 137928 | 138239 |
| 118 | 139558 | 138257 | 139558 |
| 119 | 140352 | 139516 | 140352 |
| 120 | 140498 | 141841 | 140498 |
| 121 | 141855 | 142658 | 141855 |
| 122 | 144258 | 143050 | 144258 |
| 123 | 145258 | 144494 | 145258 |
| 124 | 145454 | 146749 | 145454 |
| 125 | 147318 | 146767 | 147318 |
| 126 | 148261 | 147677 | 148261 |
| 127 | 149029 | 152157 | 149029 |
| 128 | 154108 | 152201 | 154108 |
| 129 | 155135 | 154308 | 155135 |
| 130 | 155141 | 155467 | 155141 |
| 131 | 155703 | 156779 | 155703 |
| 132 | 156748 | 157635 | 156748 |
| 133 | 157653 | 158996 | 157653 |
| 134 | 159363 | 159986 | 159363 |
| 135 | 159880 | 160446 | 159880 |
| 136 | 160477 | 160839 | 160477 |
| 137 | 160898 | 161539 | 160898 |
| 138 | 161527 | 162153 | 161527 |
| 139 | 162144 | 162443 | 162144 |
| 140 | 162437 | 164098 | 162437 |
| 141 | 165451 | 164228 | 165451 |
| 142 | 166349 | 165411 | 166349 |
| 143 | 166949 | 168442 | 166949 |
| 144 | 169416 | 171029 | 169416 |
| 145 | 170857 | 171459 | 170857 |
| 146 | 172652 | 173428 | 172652 |
| 147 | 174626 | 173439 | 174626 |
| 148 | 174816 | 175613 | 174816 |
| 149 | 175598 | 175954 | 175598 |
| 150 | 175958 | 176935 | 175958 |
| 151 | 177708 | 176938 | 177708 |
| 152 | 177128 | 177376 | 177128 |
| 153 | 179472 | 177841 | 179472 |
| 154 | 179822 | 179517 | 179822 |
| 155 | 181793 | 179943 | 181793 |
| 156 | 182628 | 181876 | 182628 |
| 157 | 184420 | 183074 | 184420 |
| 158 | 184988 | 184467 | 184988 |
| 159 | 185483 | 185112 | 185483 |
| 160 | 185902 | 185483 | 185902 |
| 161 | 186174 | 185839 | 186174 |
| 162 | 187720 | 186587 | 187720 |
| 163 | 188318 | 190933 | 188318 |
| 164 | 191090 | 191635 | 191090 |
| 165 | 191547 | 192743 | 191547 |
| 166 | 192969 | 193469 | 192969 |
| 167 | 194044 | 193610 | 194044 |
| 168 | 194196 | 195809 | 194196 |
| 169 | 196088 | 198073 | 196088 |
| 170 | 198132 | 199454 | 198132 |
| 171 | 199351 | 202818 | 199351 |
| 172 | 204552 | 202999 | 204552 |
| 173 | 205648 | 204692 | 205639 |
| 174 | 205807 | 207327 | 205807 |
| 175 | 207182 | 207775 | 207182 |
| 176 | 207779 | 208267 | 207779 |
| 177 | 208267 | 209577 | 208267 |
| 178 | 211807 | 211271 | 211807 |
| 179 | 212188 | 211844 | 212188 |
| 180 | 214079 | 212448 | 214079 |
| 181 | 214907 | 214083 | 214907 |
| 182 | 216154 | 215429 | 216154 |
| 183 | 216115 | 216678 | 216115 |
| 184 | 216728 | 217282 | 216728 |
| 185 | 217267 | 217866 | 217267 |
| 186 | 218593 | 218261 | 218590 |
| 187 | 219821 | 218994 | 219821 |
| 188 | 221382 | 220309 | 221382 |
| 189 | 222719 | 221433 | 222719 |
| 190 | 223521 | 222724 | 223521 |
| 191 | 224499 | 225008 | 224499 |
| 192 | 225140 | 225559 | 225140 |
| 193 | 225555 | 226802 | 225555 |
| 194 | 227800 | 226892 | 227743 |
| 195 | 228335 | 228072 | 228335 |
| 196 | 229251 | 228643 | 229251 |
| 197 | 230983 | 229622 | 230983 |
| 198 | 231483 | 230983 | 231483 |
| 199 | 232063 | 231509 | 232063 |
| 200 | 232739 | 232053 | 232739 |
| 201 | 233166 | 234356 | 233166 |
| 202 | 233518 | 233165 | 233518 |
| 203 | 234536 | 235186 | 234536 |
| 204 | 235379 | 236689 | 235379 |
| 205 | 236680 | 237618 | 236689 |
| 206 | 237521 | 238345 | 237521 |
| 207 | 238281 | 238973 | 238281 |
| 208 | 238871 | 240115 | 238871 |
| 209 | 240191 | 241564 | 240191 |
| 210 | 242281 | 241604 | 242281 |
| 211 | 242933 | 242274 | 242933 |
| 212 | 243416 | 242976 | 243416 |
| 213 | 243500 | 244531 | 243500 |
| 214 | 244480 | 246021 | 244480 |
| 215 | 246330 | 247811 | 246330 |
| 216 | 247831 | 249174 | 247870 |
| 217 | 249437 | 251038 | 249455 |
| 218 | 251325 | 252212 | 251325 |
| 219 | 253156 | 254007 | 253156 |
| 220 | 253974 | 254852 | 253974 |
| 221 | 255258 | 256094 | 255258 |
| 222 | 256640 | 257455 | 256640 |
| 223 | 257502 | 258239 | 257502 |
| 224 | 257869 | 257501 | 257869 |
| 225 | 259248 | 260897 | 259248 |
| 226 | 262753 | 261788 | 262753 |
| 227 | 263059 | 262757 | 263059 |
| 228 | 264375 | 263182 | 264375 |
| 229 | 265985 | 264747 | 265985 |
| 230 | 266637 | 266059 | 266637 |
| 231 | 267338 | 266538 | 267338 |
| 232 | 267922 | 267473 | 267922 |
| 233 | 269647 | 270771 | 269647 |
| 234 | 272777 | 273145 | 272777 |
| 235 | 273253 | 273636 | 273253 |
| 236 | 273705 | 273977 | 273705 |
| 237 | 276016 | 275717 | 276016 |
| 238 | 276439 | 276020 | 276418 |
| 239 | 276792 | 277253 | 276792 |
| 240 | 277318 | 277599 | 277318 |
| 241 | 278578 | 277877 | 278578 |
| 242 | 279258 | 278554 | 279258 |
| 243 | 280435 | 279533 | 280435 |
| 244 | 281547 | 280849 | 281547 |
| 245 | 281696 | 282325 | 281717 |
| 246 | 282459 | 284069 | 282459 |
| 247 | 284056 | 284517 | 284056 |
| 248 | 284606 | 285775 | 284606 |
| 249 | 285592 | 285987 | 285592 |
| 250 | 286179 | 286976 | 286179 |
| 251 | 287583 | 287002 | 287583 |
| 252 | 287951 | 287451 | 287951 |
| 253 | 288499 | 288816 | 288499 |
| 254 | 289674 | 288505 | 289674 |
| 255 | 288839 | 289213 | 288839 |
| 256 | 289970 | 290254 | 289970 |
| 257 | 291931 | 292803 | 291931 |
| 258 | 293258 | 292755 | 293258 |
| 259 | 293718 | 293272 | 293718 |
| 260 | 294630 | 293953 | 294630 |
| 261 | 296153 | 294636 | 296153 |
| 262 | 294817 | 295068 | 294817 |
| 263 | 296354 | 297862 | 296354 |
| 264 | 298415 | 297879 | 298415 |
| 265 | 298777 | 298253 | 298777 |
| 266 | 299572 | 298781 | 299572 |
| 267 | 300487 | 299633 | 300487 |
| 268 | 301586 | 300702 | 301568 |
| 269 | 302440 | 301571 | 302440 |
| 270 | 302838 | 302437 | 302838 |
| 271 | 303335 | 302745 | 303335 |
| 272 | 304394 | 303852 | 304394 |
| 273 | 304606 | 305223 | 304606 |
| 274 | 305394 | 306236 | 305394 |
| 275 | 306501 | 307439 | 306501 |
| 276 | 308033 | 307458 | 308033 |
| 277 | 308924 | 308037 | 308924 |
| 278 | 309485 | 310180 | 309485 |
| 279 | 310426 | 311214 | 310426 |
| 280 | 311597 | 311253 | 311504 |
| 281 | 312772 | 311780 | 312772 |
| 282 | 313425 | 312772 | 313425 |
| 283 | 313646 | 313377 | 313646 |
| 284 | 313937 | 314665 | 313937 |
| 285 | 315576 | 314755 | 315576 |
| 286 | 316157 | 315531 | 316157 |
| 287 | 318657 | 316156 | 318657 |
| 288 | 321042 | 318676 | 321042 |
| 289 | 321445 | 321098 | 321445 |
| 290 | 322309 | 321710 | 322309 |
| 291 | 323190 | 322366 | 323181 |
| 292 | 323843 | 323181 | 323843 |
| 293 | 324878 | 323856 | 324878 |
| 294 | 325340 | 326410 | 325340 |
| 295 | 326433 | 327836 | 326433 |
| 296 | 328465 | 327839 | 328465 |
| 297 | 329360 | 328857 | 329360 |
| 298 | 330907 | 329357 | 330907 |
| 299 | 332455 | 330956 | 332455 |
| 300 | 334536 | 332395 | 334536 |
| 301 | 336091 | 334877 | 336091 |
| 302 | 336103 | 337302 | 336103 |
| 303 | 338129 | 338830 | 338129 |
| 304 | 338965 | 339501 | 338965 |
| 305 | 339508 | 340143 | 339508 |
| 306 | 340247 | 342967 | 340247 |
| 307 | 343385 | 343810 | 343385 |
| 308 | 344171 | 343935 | 344171 |
| 309 | 345082 | 344330 | 345073 |
| 310 | 346005 | 345082 | 346005 |
| 311 | 346784 | 346437 | 346784 |
| 312 | 347029 | 346715 | 347029 |
| 313 | 347034 | 347723 | 347034 |
| 314 | 348075 | 350459 | 348075 |
| 315 | 350598 | 351071 | 350598 |
| 316 | 351075 | 352175 | 351096 |
| 317 | 353291 | 352230 | 353267 |
| 318 | 353442 | 354467 | 353442 |
| 319 | 354451 | 354933 | 354451 |
| 320 | 355000 | 355449 | 355000 |
| 321 | 355448 | 356743 | 355448 |
| 322 | 355953 | 355642 | 355953 |
| 323 | 359310 | 356827 | 359310 |
| 324 | 359120 | 359377 | 359120 |
| 325 | 359525 | 359908 | 359525 |
| 326 | 361290 | 359947 | 361290 |
| 327 | 363785 | 361362 | 363746 |
| 328 | 364496 | 363888 | 364496 |
| 329 | 364832 | 365290 | 364832 |
| 330 | 365304 | 365669 | 365304 |
| 331 | 366599 | 365667 | 366599 |
| 332 | 367291 | 369030 | 367291 |
| 333 | 369134 | 369808 | 369134 |
| 334 | 369917 | 370438 | 369917 |
| 335 | 370365 | 372647 | 370365 |
| 336 | 372557 | 373066 | 372557 |
| 337 | 373020 | 373442 | 373020 |
| 338 | 373467 | 374195 | 373467 |
| 339 | 374176 | 375099 | 374176 |
| 340 | 375676 | 375083 | 375676 |
| 341 | 376173 | 375634 | 376173 |
| 342 | 376564 | 377643 | 376564 |
| 343 | 377956 | 379773 | 377956 |
| 344 | 379781 | 380425 | 379805 |
| 345 | 380281 | 381000 | 380281 |
| 346 | 381008 | 381460 | 381008 |
| 347 | 381460 | 383037 | 381460 |
| 348 | 383257 | 383523 | 383257 |
| 349 | 383553 | 385304 | 383553 |
| 350 | 385397 | 386458 | 385400 |
| 351 | 387242 | 386514 | 387242 |
| 352 | 388764 | 387013 | 388764 |
| 353 | 390120 | 390932 | 390120 |
| 354 | 390919 | 391818 | 390961 |
| 355 | 392379 | 391885 | 392379 |
| 356 | 392582 | 392986 | 392582 |
| 357 | 392776 | 393684 | 392776 |
| 358 | 394151 | 394804 | 394151 |
| 359 | 394928 | 395308 | 394928 |
| 360 | 395259 | 395990 | 395259 |
| 361 | 397815 | 395953 | 397815 |
| 362 | 398850 | 397831 | 398850 |
| 363 | 400085 | 399099 | 400085 |
| 364 | 401245 | 400073 | 401236 |
| 365 | 401474 | 401136 | 401474 |
| 366 | 402199 | 401423 | 402199 |
| 367 | 403193 | 402186 | 403166 |
| 368 | 403650 | 404165 | 403650 |
| 369 | 404343 | 405914 | 404343 |
| 370 | 405984 | 407327 | 405984 |
| 371 | 407712 | 408806 | 407712 |
| 372 | 410439 | 409075 | 410439 |
| 373 | 411826 | 410954 | 411826 |
| 374 | 412482 | 414302 | 412482 |
| 375 | 415402 | 414407 | 415402 |
| 376 | 415848 | 415237 | 415848 |
| 377 | 417131 | 415866 | 417131 |
| 378 | 417258 | 417566 | 417258 |
| 379 | 418326 | 417454 | 418326 |
| 380 | 420057 | 418426 | 420057 |
| 381 | 420448 | 420720 | 420448 |
| 382 | 420980 | 421552 | 420980 |
| 383 | 421556 | 422029 | 421556 |
| 384 | 422461 | 422925 | 422461 |
| 385 | 423562 | 424320 | 423562 |
| 386 | 424250 | 424591 | 424250 |
| 387 | 424830 | 426047 | 424830 |
| 388 | 426240 | 427397 | 426240 |
| 389 | 428841 | 430703 | 428841 |
| 390 | 430694 | 431446 | 430694 |
| 391 | 431597 | 432100 | 431597 |
| 392 | 432165 | 432779 | 432165 |
| 393 | 433272 | 432832 | 433272 |
| 394 | 433925 | 433227 | 433922 |
| 395 | 436678 | 433934 | 436678 |
| 396 | 437176 | 438357 | 437176 |
| 397 | 440317 | 438518 | 440317 |
| 398 | 440001 | 440345 | 440001 |
| 399 | 441233 | 440517 | 441233 |
| 400 | 440719 | 441012 | 440719 |
| 401 | 442192 | 441230 | 442192 |
| 402 | 442888 | 442343 | 442888 |
| 403 | 442371 | 442961 | 442371 |
| 404 | 443578 | 443003 | 443578 |
| 405 | 444500 | 443526 | 444500 |
| 406 | 444842 | 444528 | 444842 |
| 407 | 445009 | 444743 | 445009 |
| 408 | 445718 | 445182 | 445718 |
| 409 | 445807 | 447804 | 445807 |
| 410 | 448738 | 447803 | 448738 |
| 411 | 449628 | 448618 | 449628 |
| 412 | 450298 | 450867 | 450298 |
| 413 | 450713 | 451207 | 450713 |
| 414 | 451211 | 452452 | 451211 |
| 415 | 452448 | 453659 | 452448 |
| 416 | 454843 | 453725 | 454843 |
| 417 | 455608 | 454865 | 455608 |
| 418 | 456243 | 457007 | 456243 |
| 419 | 457016 | 457708 | 457016 |
| 420 | 458368 | 457979 | 458368 |
| 421 | 459496 | 458372 | 459496 |
| 422 | 459493 | 460194 | 459493 |
| 423 | 461446 | 460355 | 461446 |
| 424 | 462298 | 461450 | 462298 |
| 425 | 462444 | 463349 | 462444 |
| 426 | 464241 | 463342 | 464241 |
| 427 | 464574 | 465065 | 464574 |
| 428 | 465129 | 465611 | 465129 |
| 429 | 465571 | 466317 | 465571 |
| 430 | 466317 | 467093 | 466317 |
| 431 | 466999 | 467502 | 466999 |
| 432 | 469691 | 467715 | 469691 |
| 433 | 470691 | 469660 | 470691 |
| 434 | 472010 | 470709 | 472010 |
| 435 | 471545 | 471799 | 471545 |
| 436 | 472359 | 472045 | 472359 |
| 437 | 473523 | 472732 | 473523 |
| 438 | 474889 | 473441 | 474889 |
| 439 | 477323 | 475365 | 477323 |
| 440 | 478496 | 477597 | 478496 |
| 441 | 478722 | 479273 | 478722 |
| 442 | 479277 | 479705 | 479277 |
| 443 | 480050 | 481450 | 480050 |
| 444 | 481469 | 482053 | 481469 |
| 445 | 482600 | 482025 | 482600 |
| 446 | 482654 | 484204 | 482654 |
| 447 | 484211 | 485170 | 484211 |
| 448 | 485170 | 485838 | 485170 |
| 449 | 485813 | 486580 | 485813 |
| 450 | 486976 | 486638 | 486976 |
| 451 | 489071 | 487764 | 489071 |
| 452 | 489341 | 489090 | 489341 |
| 453 | 489958 | 489152 | 489958 |
| 454 | 490549 | 489962 | 490549 |
| 455 | 491163 | 490522 | 491163 |
| 456 | 491396 | 491112 | 491396 |
| 457 | 492121 | 491390 | 492121 |
| 458 | 492304 | 494838 | 492304 |
| 459 | 495943 | 494822 | 495943 |
| 460 | 496011 | 496565 | 496170 |
| 461 | 496569 | 497228 | 496569 |
| 462 | 497358 | 497834 | 497358 |
| 463 | 497770 | 498327 | 497770 |
| 464 | 499209 | 499589 | 499209 |
| 465 | 499520 | 499792 | 499520 |
| 466 | 500774 | 504169 | 500774 |
| 467 | 504139 | 504600 | 504139 |
| 468 | 504865 | 506877 | 504865 |
| 469 | 506790 | 507671 | 506790 |
| 470 | 507718 | 510507 | 507718 |
| 471 | 508325 | 507912 | 508325 |
| 472 | 510660 | 513440 | 510660 |
| 473 | 514965 | 513787 | 514920 |
| 474 | 517347 | 515419 | 517347 |
| 475 | 517058 | 517363 | 517058 |
| 476 | 517798 | 517277 | 517798 |
| 477 | 518200 | 517847 | 518200 |
| 478 | 518300 | 521146 | 518363 |
| 479 | 521392 | 522948 | 521407 |
| 480 | 523244 | 524809 | 523322 |
| 481 | 524379 | 524125 | 524379 |
| 482 | 524649 | 526238 | 524649 |
| 483 | 526265 | 527104 | 526268 |
| 484 | 526947 | 526702 | 526947 |
| 485 | 526975 | 528450 | 526975 |
| 486 | 528408 | 529199 | 528408 |
| 487 | 530612 | 529542 | 530612 |
| 488 | 531656 | 530616 | 531656 |
| 489 | 533974 | 532067 | 533974 |
| 490 | 536432 | 534324 | 536432 |
| 491 | 537150 | 536707 | 537150 |
| 492 | 537928 | 537080 | 537928 |
| 493 | 538438 | 537932 | 538438 |
| 494 | 538737 | 538333 | 538737 |
| 495 | 539594 | 539127 | 539594 |
| 496 | 541215 | 539590 | 541215 |
| 497 | 542571 | 541282 | 542571 |
| 498 | 543014 | 542457 | 543014 |
| 499 | 543369 | 542962 | 543369 |
| 500 | 543809 | 546628 | 543815 |
| 501 | 546619 | 549525 | 546619 |
| 502 | 547293 | 546994 | 547293 |
| 503 | 549699 | 550523 | 549699 |
| 504 | 550490 | 551551 | 550490 |
| 505 | 551448 | 552623 | 551448 |
| 506 | 552652 | 555117 | 552652 |
| 507 | 555029 | 555493 | 555029 |
| 508 | 558006 | 555673 | 558006 |
| 509 | 559694 . | 558162 | 559694 |
| 510 | 558208 | 558573 | 558208 |
| 511 | 561692 | 559899 | 561692 |
| 512 | 561412 | 561708 | 561412 |
| 513 | 563942 | 561777 | 563942 |
| 514 | 564969 | 563950 | 564969 |
| 515 | 566204 | 564936 | 566198 |
| 516 | 567717 | 566302 | 567717 |
| 517 | 568526 | 567708 | 568526 |
| 518 | 569467 | 568742 | 569467 |
| 519 | 571065 | 569431 | 571065 |
| 520 | 571828 | 571118 | 571783 |
| 521 | 572202 | 573308 | 572202 |
| 522 | 573146 | 575056 | 573146 |
| 523 | 575023 | 575916 | 575023 |
| 524 | 577891 | 576497 | 577891 |
| 525 | 578914 | 578204 | 578914 |
| 526 | 579924 | 578857 | 579924 |
| 527 | 580187 | 579858 | 580187 |
| 528 | 580017 | 580406 | 580017 |
| 529 | 581086 | 580187 | 581086 |
| 530 | 581367 | 581828 | 581367 |
| 531 | 581678 | 582367 | 581678 |
| 532 | 582361 | 583428 | 582361 |
| 533 | 584690 | 583431 | 584690 |
| 534 | 585237 | 584950 | 585237 |
| 535 | 585626 | 586888 | 585626 |
| 536 | 586846 | 587907 | 586888 |
| 537 | 589049 | 588180 | 589049 |
| 538 | 590500 | 589301 | 590455 |
| 539 | 590755 | 592458 | 590755 |
| 540 | 592526 | 592903 | 592526 |
| 541 | 592836 | 593747 | 592836 |
| 542 | 593747 | 594298 | 593747 |
| 543 | 594331 | 595947 | 594331 |
| 544 | 595905 | 596309 | 595905 |
| 545 | 596514 | 597215 | 596514 |
| 546 | 597184 | 597957 | 597184 |
| 547 | 597755 | 598612 | 597755 |
| 548 | 598602 | 599204 | 598602 |
| 549 | 599373 | 599939 | 599373 |
| 550 | 600903 | 602072 | 600903 |
| 551 | 602240 | 602587 | 602240 |
| 552 | 602637 | 603272 | 602637 |
| 553 | 603142 | 604512 | 603142 |
| 554 | 604627 | 605853 | 604627 |
| 555 | 605790 | 606620 | 605790 |
| 556 | 606571 | 607281 | 606571 |
| 557 | 609004 | 607355 | 609004 |
| 558 | 610906 | 609932 | 610906 |
| 559 | 611786 | 611004 | 611786 |
| 560 | 612333 | 611746 | 612333 |
| 561 | 613897 | 612341 | 613897 |
| 562 | 615179 | 616279 | 615179 |
| 563 | 616610 | 617383 | 616610 |
| 564 | 618796 | 617810 | 618796 |
| 565 | 620004 | 618826 | 620004 |
| 566 | 619649 | 619918 | 619649 |
| 567 | 621265 | 620021 | 621265 |
| 568 | 622359 | 621265 | 622359 |
| 569 | 623420 | 622560 | 623420 |
| 570 | 624297 | 623335 | 624297 |
| 571 | 624773 | 624174 | 624773 |
| 572 | 625029 | 625484 | 625029 |
| 573 | 625488 | 625883 | 625488 |
| 574 | 625892 | 626395 | 625892 |
| 575 | 626444 | 627790 | 626444 |
| 576 | 627912 | 628607 | 627930 |
| 577 | 628774 | 629697 | 628774 |
| 578 | 629660 | 631639 | 629660 |
| 579 | 631725 | 633551 | 631725 |
| 580 | 633520 | 636957 | 633520 |
| 581 | 637232 | 638098 | 637232 |
| 582 | 640648 | 639593 | 640648 |
| 583 | 640979 | 640728 | 640979 |
| 584 | 641327 | 641007 | 641327 |
| 585 | 641687 | 642283 | 641687 |
| 586 | 643023 | 642286 | 643023 |
| 587 | 643330 | 643076 | 643330 |
| 588 | 643704 | 643351 | 643704 |
| 589 | 645628 | 643676 | 645628 |
| 590 | 645783 | 645538 | 645756 |
| 591 | 646269 | 645793 | 646269 |
| 592 | 646751 | 646314 | 646751 |
| 593 | 647848 | 647045 | 647848 |
| 594 | 648393 | 650336 | 648393 |
| 595 | 651016 | 650420 | 651007 |
| 596 | 652956 | 651289 | 652956 |
| 597 | 653395 | 653126 | 653395 |
| 598 | 655740 | 654193 | 655740 |
| 599 | 656508 | 655966 | 656508 |
| 600 | 658140 | 657022 | 658140 |
| 601 | 660216 | 658525 | 660216 |
| 602 | 663238 | 660248 | 663238 |
| 603 | 664461 | 663157 | 664452 |
| 604 | 665735 | 664635 | 665735 |
| 605 | 666212 | 666994 | 666212 |
| 606 | 666998 | 667921 | 666998 |
| 607 | 667909 | 668568 | 667909 |
| 608 | 668502 | 669203 | 668502 |
| 609 | 669154 | 670893 | 669175 |
| 610 | 672226 | 670853 | 672226 |
| 611 | 671137 | 671424 | 671137 |
| 612 | 672453 | 673001 | 672453 |
| 613 | 673072 | 674721 | 673072 |
| 614 | 674549 | 674262 | 674549 |
| 615 | 675518 | 674796 | 675518 |
| 616 | 676083 | 675499 | 676083 |
| 617 | 676630 | 676067 | 676630 |
| 618 | 677016 | 676600 | 677016 |
| 619 | 677647 | 677015 | 677647 |
| 620 | 677990 | 678259 | 677990 |
| 621 | 679444 | 680097 | 679444 |
| 622 | 680097 | 680897 | 680097 |
| 623 | 681637 | 680849 | 681637 |
| 624 | 681409 | 682281 | 681409 |
| 625 | 682453 | 682821 | 682453 |
| 626 | 682763 | 683902 | 682763 |
| 627 | 684616 | 683969 | 684616 |
| 628 | 685169 | 684534 | 685169 |
| 629 | 685986 | 685117 | 685986 |
| 630 | 686278 | 687288 | 686278 |
| 631 | 687483 | 688151 | 687483 |
| 632 | 688740 | 689501 | 688740 |
| 633 | 690242 | 689622 | 690242 |
| 634 | 690470 | 691126 | 690470 |
| 635 | 692600 | 691497 | 692600 |
| 636 | 692674 | 695064 | 692674 |
| 637 | 695049 | 696032 | 695064 |
| 638 | 697964 | 696585 | 697964 |
| 639 | 699803 | 698274 | 699803 |
| 640 | 701926 | 699788 | 701926 |
| 641 | 703196 | 702567 | 703196 |
| 642 | 704221 | 703208 | 704221 |
| 643 | 704240 | 705289 | 704240 |
| 644 | 706070 | 705300 | 706070 |
| 645 | 706841 | 706254 | 706838 |
| 646 | 707596 | 706811 | 707596 |
| 647 | 708666 | 707677 | 708666 |
| 648 | 709793 | 709119 | 709793 |
| 649 | 711523 | 710132 | 711523 |
| 650 | 712236 | 711523 | 712236 |
| 651 | 714734 | 712125 | 714734 |
| 652 | 715759 | 714761 | 715759 |
| 653 | 717538 | 715886 | 717538 |
| 654 | 719113 | 720243 | 719113 |
| 655 | 720590 | 722422 | 720590 |
| 656 | 722406 | 723056 | 722406 |
| 657 | 723551 | 723120 | 723551 |
| 658 | 724246 | 723626 | 724246 |
| 659 | 724754 | 724251 | 724754 |
| 660 | 725868 | 724900 | 725868 |
| 661 | 727115 | 726270 | 727115 |
| 662 | 728126 | 727119 | 728126 |
| 663 | 728594 | 728208 | 728594 |
| 664 | 729614 | 728604 | 729614 |
| 665 | 729778 | 729533 | 729778 |
| 666 | 730149 | 729751 | 730149 |
| 667 | 730539 | 730174 | 730539 |
| 668 | 731983 | 730598 | 731983 |
| 669 | 732427 | 731996 | 732427 |
| 670 | 732917 | 732423 | 732917 |
| 671 | 733598 | 733320 | 733598 |
| 672 | 733869 | 733492 | 733869 |
| 673 | 734298 | 733900 | 734298 |
| 674 | 734858 | 734319 | 734858 |
| 675 | 735195 | 734863 | 735195 |
| 676 | 735578 | 735342 | 735578 |
| 677 | 735861 | 735604 | 735861 |
| 678 | 736492 | 736079 | 736492 |
| 679 | 737192 | 736524 | 737192 |
| 680 | 737555 | 737211 | 737555 |
| 681 | 738688 | 737837 | 738688 |
| 682 | 739048 | 738713 | 739048 |
| 683 | 739736 | 739065 | 739736 |
| 684 | 740477 | 739773 | 740477 |
| 685 | 740659 | 740958 | 740659 |
| 686 | 741722 | 740721 | 741722 |
| 687 | 742789 | 741827 | 742789 |
| 688 | 743618 | 742782 | 743618 |
| 689 | 744092 | 743634 | 744092 |
| 690 | 744604 | 744107 | 744604 |
| 691 | 744953 | 744498 | 744953 |
| 692 | 746608 | 744986 | 746608 |
| 693 | 747085 | 746621 | 747085 |
| 694 | 747974 | 747219 | 747974 |
| 695 | 748594 | 748169 | 748594 |
| 696 | 749145 | 748573 | 749145 |
| 697 | 749652 | 749957 | 749652 |
| 698 | 750446 | 749979 | 750446 |
| 699 | 751219 | 750446 | 751219 |
| 700 | 753042 | 751291 | 753042 |
| 701 | 754309 | 753020 | 754309 |
| 702 | 755120 | 756175 | 755120 |
| 703 | 756120 | 756485 | 756120 |
| 704 | 756499 | 760227 | 756499 |
| 705 | 761217 | 760297 | 761178 |
| 706 | 761297 | 761809 | 761330 |
| 707 | 761782 | 762282 | 761782 |
| 708 | 762260 | 762895 | 762299 |
| 709 | 762867 | 763316 | 762867 |
| 710 | 763780 | 763325 | 763780 |
| 711 | 763861 | 765168 | 763861 |
| 712 | 766809 | 765697 | 766809 |
| 713 | 768051 | 766888 | 768051 |
| 714 | 768566 | 768321 | 768566 |
| 715 | 769342 | 768551 | 769342 |
| 716 | 770532 | 769378 | 770532 |
| 717 | 771451 | 770804 | 771451 |
| 718 | 773058 | 771847 | 773058 |
| 719 | 773094 | 773456 | 773094 |
| 720 | 774376 | 773093 | 774376 |
| 721 | 775123 | 774380 | 775123 |
| 722 | 775398 | 774916 | 775398 |
| 723 | 775046 | 776077 | 775046 |
| 724 | 776070 | 777041 | 776070 |
| 725 | 777964 | 777536 | 777964 |
| 726 | 778176 | 777904 | 778176 |
| 727 | 778621 | 779334 | 778684 |
| 728 | 781173 | 780307 | 781173 |
| 729 | 781526 | 781116 | 781526 |
| 730 | 782784 | 781555 | 782784 |
| 731 | 783572 | 782805 | 783572 |
| 732 | 785032 | 783581 | 785032 |
| 733 | 786412 | 785360 | 786412 |
| 734 | 788429 | 786450 | 788429 |
| 735 | 788944 | 788528 | 788944 |
| 736 | 789758 | 788901 | 789758 |
| 737 | 790332 | 791504 | 790338 |
| 738 | 791846 | 792721 | 791846 |
| 739 | 792724 | 793569 | 792724 |
| 740 | 793580 | 794323 | 793580 |
| 741 | 794304 | 794843 | 794304 |
| 742 | 795217 | 795732 | 795217 |
| 743 | 795722 | 796795 | 795722 |
| 744 | 798735 | 797053 | 798735 |
| 745 | 799823 | 798681 | 799823 |
| 746 | 799297 | 799578 | 799297 |
| 747 | 801313 | 799808 | 801313 |
| 748 | 802453 | 801332 | 802453 |
| 749 | 803299 | 802457 | 803299 |
| 750 | 803811 | 803290 | 803811 |
| 751 | 805151 | 803826 | 805151 |
| 752 | 805860 | 805156 | 805860 |
| 753 | 806604 | 806332 | 806604 |
| 754 | 806913 | 806608 | 806913 |
| 755 | 808222 | 806903 | 808222 |
| 756 | 808751 | 808146 | 808751 |
| 757 | 809437 | 808673 | 809437 |
| 758 | 809939 | 809454 | 809939 |
| 759 | 811235 | 810213 | 811235 |
| 760 | 811779 | 813056 | 811779 |
| 761 | 812890 | 812516 | 812890 |
| 762 | 812954 | 813583 | 812954 |
| 763 | 813587 | 815023 | 813587 |
| 764 | 815420 | 815746 | 815420 |
| 765 | 816036 | 817010 | 816036 |
| 766 | 817111 | 817356 | 817111 |
| 767 | 817791 | 818609 | 817797 |
| 768 | 818609 | 819094 | 818609 |
| 769 | 819104 | 819823 | 819104 |
| 770 | 820722 | 819826 | 820722 |
| 771 | 822313 | 821000 | 822313 |
| 772 | 823503 | 822238 | 823503 |
| 773 | 823678 | 825612 | 823678 |
| 774 | 825461 | 826312 | 825461 |
| 775 | 827280 | 826645 | 827280 |
| 776 | 828604 | 827171 | 828604 |
| 777 | 830026 | 828713 | 830026 |
| 778 | 831047 | 830085 | 831047 |
| 779 | 831725 | 831051 | 831725 |
| 780 | 832220 | 833098 | 832220 |
| 781 | 833851 | 833396 | 833851 |
| 782 | 834068 | 835039 | 834068 |
| 783 | 835792 | 835127 | 835792 |
| 784 | 837624 | 836116 | 837624 |
| 785 | 838951 | 840882 | 838951 |
| 786 | 840869 | 842185 | 840869 |
| 787 | 841989 | 843455 | 841989 |
| 788 | 843242 | 844021 | 843242 |
| 789 | 845018 | 843987 | 844997 |
| 790 | 846174 | 844990 | 846174 |
| 791 | 848509 | 846311 | 848509 |
| 792 | 848568 | 849014 | 848568 |
| 793 | 849082 | 850488 | 849088 |
| 794 | 851512 | 850574 | 851512 |
| 795 | 852064 | 852447 | 852064 |
| 796 | 852398 | 853690 | 852398 |
| 797 | 855118 | 854243 | 855118 |
| 798 | 855751 | 855128 | 855751 |
| 799 | 856551 | 855829 | 856551 |
| 800 | 856730 | 858556 | 856730 |
| 801 | 858717 | 859601 | 858717 |
| 802 | 859591 | 860205 | 859591 |
| 803 | 861132 | 860284 | 861132 |
| 804 | 861426 | 861163 | 861426 |
| 805 | 861701 | 862921 | 861701 |
| 806 | 863026 | 864798 | 863026 |
| 807 | 864831 | 865256 | 864831 |
| 808 | 865226 | 866581 | 865226 |
| 809 | 866562 | 867119 | 866562 |
| 810 | 867025 | 867816 | 867025 |
| 811 | 867820 | 868497 | 867820 |
| 812 | 869743 | 868661 | 869743 |
| 813 | 870633 | 870094 | 870633 |
| 814 | 871929 | 870646 | 871929 |
| 815 | 872538 | 872086 | 872538 |
| 816 | 873908 | 872517 | 873908 |
| 817 | 874281 | 874670 | 874281 |
| 818 | 874582 | 875286 | 874582 |
| 819 | 877857 | 875377 | 877857 |
| 820 | 878446 | 879255 | 878446 |
| 821 | 880635 | 879268 | 880635 |
| 822 | 882524 | 880593 | . 882524 |
| 823 | 882612 | 883319 | 882612 |
| 824 | 884155 | 883538 | 884155 |
| 825 | 884340 | 885611 | 884343 |
| 826 | 885722 | 887302 | 885722 |
| 827 | 887587 | 888153 | 887587 |
| 828 | 888627 | 888220 | 888627 |
| 829 | 889330 | 888716 | 889330 |
| 830 | 889898 | 889323 | 889898 |
| 831 | 891190 | 889898 | 891190 |
| 832 | 891828 | 891247 | 891828 |
| 833 | 892421 | 892017 | 892421 |
| 834 | 893116 | 892421 | 893116 |
| 835 | 892521 | 892925 | 892521 |
| 836 | 893392 | 895419 | 893392 |
| 837 | 895745 | 896527 | 895745 |
| 838 | 896668 | 897558 | 896668 |
| 839 | 897565 | 899442 | 897565 |
| 840 | 899420 | 900229 | 899420 |
| 841 | 903230 | 900237 | 903230 |
| 842 | 905081 | 903234 | 905081 |
| 843 | 906931 | 905045 | 906931 |
| 844 | 907248 | 907832 | 907299 |
| 845 | 907784 | 908128 | 907784 |
| 846 | 908132 | 908677 | 908132 |
| 847 | 908589 | 909320 | 908589 |
| 848 | 909405 | 911465 | 909405 |
| 849 | 911677 | 912360 | 911725 |
| 850 | 912303 | 912821 | 912303 |
| 851 | 912937 | 913983 | 912937 |
| 852 | 915128 | 914067 | 915128 |
| 853 | 916658 | 915303 | 916658 |
| 854 | 915627 | 915376 | 915627 |
| 855 | 917707 | 916853 | 917707 |
| 856 | 918837 | 917722 | 918837 |
| 857 | 919868 | 918837 | 919868 |
| 858 | 920434 | 919880 | 920434 |
| 859 | 921187 | 920438 | 921187 |
| 860 | 921959 | 921195 | 921959 |
| 861 | 923773 | 921995 | 923773 |
| 862 | 922146 | 922415 | 922146 |
| 863 | 923943 | 923674 | 923943 |
| 864 | 924077 | 925006 | 924077 |
| 865 | 925436 | 925083 | 925436 |
| 866 | 926524 | 925349 | 926524 |
| 867 | 927920 | 926433 | 927920 |
| 868 | 928319 | 927951 | 928319 |
| 869 | 928963 | 928334 | 928963 |
| 870 | 929248 | 930987 | 929248 |
| 871 | 930995 | 932059 | 930995 |
| 872 | 932121 | 933515 | 932175 |
| 873 | 932881 | 932513 | 932881 |
| 874 | 933485 | 935746 | 933485 |
| 875 | 935724 | 937082 | 935724 |
| 876 | 937229 | 938410 | 937229 |
| 877 | 938281 | 938805 | 938281 |
| 878 | 938809 | 939255 | 938824 |
| 879 | 939165 | 939782 | 939165 |
| 880 | 939760 | 940791 | 939790 |
| 881 | 940822 | 941106 | 940822 |
| 882 | 940977 | 941351 | 940977 |
| 883 | 942537 | 941623 | 942429 |
| 884 | 942784 | 942500 | 942763 |
| 885 | 943149 | 942799 | 943149 |
| 886 | 943799 | 943029 | 943799 |
| 887 | 944055 | 943732 | 944055 |
| 888 | 944413 | 943994 | 944404 |
| 889 | 945395 | 944556 | 945395 |
| 890 | 945853 | 945389 | 945853 |
| 891 | 946392 | 945751 | 946392 |
| 892 | 947410 | 948081 | 947431 |
| 893 | 949871 | 948915 | 949871 |
| 894 | 951058 | 949868 | 951058 |
| 895 | 951249 | 950959 | 951249 |
| 896 | 951664 | 952134 | 951664 |
| 897 | 952674 | 952165 | 952674 |
| 898 | 953491 | 952589 | 953491 |
| 899 | 955324 | 953495 | 955324 |
| 900 | 955823 | 955281 | 955823 |
| 901 | 957082 | 955847 | 957082 |
| 902 | 957902 | 957270 | 957902 |
| 903 | 959231 | 957906 | 959231 |
| 904 | 959376 | 960284 | 959376 |
| 905 | 960266 | 961669 | 960347 |
| 906 | 961856 | 964765 | 961856 |
| 907 | 966855 | 965395 | 966855 |
| 908 | 968204 | 966975 | 968204 |
| 909 | 968791 | 968237 | 968791 |
| 910 | 969498 | 968731 | 969498 |
| 911 | 969858 | 969511 | 969858 |
| 912 | 970118 | 969762 | 970118 |
| 913 | 970593 | 970300 | 970593 |
| 914 | 971261 | 970542 | 971261 |
| 915 | 971680 | 971123 | 971680 |
| 916 | 971876 | 975100 | 971876 |
| 917 | 975419 | 976516 | 975419 |
| 918 | 976584 | 978320 | 976584 |
| 919 | 977680 | 977231 | 977680 |
| 920 | 978399 | 980738 | 978399 |
| 921 | 980756 | 981928 | 980756 |
| 922 | 982974 | 981931 | 982962 |
| 923 | 984120 | 983119 | 984120 |
| 924 | 985502 | 984120 | 985502 |
| 925 | 987180 | 985882 | 987180 |
| 926 | 987172 | 987444 | 987172 |
| 927 | 989846 | 989049 | 989846 |
| 928 | 991048 | 989846 | 991048 |
| 929 | 991638 | 990955 | 991638 |
| 930 | 991794 | 992498 | 991794 |
| 931 | 993619 | 993041 | 993619 |
| 932 | 993530 | 994792 | 993548 |
| 933 | 995970 | 994795 | 995970 |
| 934 | 996857 | 995739 | 996857 |
| 935 | 997603 | 996782 | 997603 |
| 936 | 998969 | 997572 | 998969 |
| 937 | 998896 | 1000023 | 998896 |
| 938 | 1000087 | 1001340 | 1000087 |
| 939 | 1001357 | 1001818 | 1001357 |
| 940 | 1003288 | 1001873 | 1003288 |
| 941 | 1003487 | 1004146 | 1003496 |
| 942 | 1004485 | 1005639 | 1004689 |
| 943 | 1005643 | 1005972 | 1005643 |
| 944 | 1006784 | 1006116 | 1006784 |
| 945 | 1007563 | 1006769 | 1007563 |
| 946 | 1009226 | 1007568 | 1009226 |
| 947 | 1009989 | 1009336 | 1009989 |
| 948 | 1015852 | 1016337 | 1015852 |
| 949 | 1016561 | 1016181 | 1016561 |
| 950 | 1016297 | 1017532 | 1016297 |
| 951 | 1016802 | 1016452 | 1016802 |
| 952 | 1018993 | 1017701 | 1018993 |
| 953 | 1019454 | 1019137 | 1019454 |
| 954 | 1020764 | 1019562 | 1020764 |
| 955 | 1021405 | 1021037 | 1021405 |
| 956 | 1021821 | 1024286 | 1021821 |
| 957 | 1024697 | 1024248 | 1024697 |
| 958 | 1025569 | 1024508 | 1025551 |
| 959 | 1026969 | 1025590 | 1026969 |
| 960 | 1027789 | 1026947 | 1027789 |
| 961 | 1031199 | 1027945 | 1031199 |
| 962 | 1031717 | 1031172 | 1031717 |
| 963 | 1033057 | 1031612 | 1033057 |
| 964 | 1033425 | 1033039 | 1033425 |
| 965 | 1033784 | 1033200 | 1033784 |
| 966 | 1033963 | 1036038 | 1033963 |
| 967 | 1036945 | 1036010 | 1036945 |
| 968 | 1037110 | 1037679 | 1037110 |
| 969 | 1037696 | 1037944 | 1037696 |
| 970 | 1038916 | 1037975 | 1038916 |
| 971 | 1040582 | 1039026 | 1040582 |
| 972 | 1040997 | 1042337 | 1040997 |
| 973 | 1042357 | 1043403 | 1042357 |
| 974 | 1043367 | 1044623 | 1043367 |
| 975 | 1044607 | 1045362 | 1044607 |
| 976 | 1045384 | 1046538 | 1045384 |
| 977 | 1046447 | 1047517 | 1046447 |
| 978 | 1047521 | 1049956 | 1047521 |
| 979 | 1050611 | 1050036 | 1050611 |
| 980 | 1050925 | 1050566 | 1050925 |
| 981 | 1051728 | 1051090 | 1051728 |
| 982 | 1051743 | 1052063 | 1051743 |
| 983 | 1052101 | 1053126 | 1052101 |
| 984 | 1054201 | 1053107 | 1054201 |
| 985 | 1054242 | 1055555 | 1054242 |
| 986 | 1055483 | 1055908 | 1055483 |
| 987 | 1056609 | 1056965 | 1056609 |
| 988 | 1056961 | 1058232 | 1056985 |
| 989 | 1058238 | 1058687 | 1058238 |
| 990 | 1059371 | 1058727 | 1059371 |
| 991 | 1059526 | 1060578 | 1059526 |
| 992 | 1061553 | 1060579 | 1061553 |
| 993 | 1061674 | 1062411 | 1061674 |
| 994 | 1062377 | 1064077 | 1062377 |
| 995 | 1064116 | 1065243 | 1064116 |
| 996 | 1067451 | 1065178 | 1067451 |
| 997 | 1068065 | 1067376 | 1068065 |
| 998 | 1068209 | 1068706 | 1068230 |
| 999 | 1069958 | 1068819 | 1069958 |
| 1000 | 1071163 | 1070033 | 1071163 |
| 1001 | 1072438 | 1071332 | 1072438 |
| 1002 | 1072997 | 1073476 | 1072997 |
| 1003 | 1074239 | 1075864 | 1074239 |
| 1004 | 1076790 | 1075867 | 1076790 |
| 1005 | 1077268 | 1076573 | 1077268 |
| 1006 | 1077999 | 1078724 | 1077999 |
| 1007 | 1079088 | 1078672 | 1079088 |
| 1008 | 1079642 | 1079944 | 1079642 |
| 1009 | 1080501 | 1079995 | 1080468 |
| 1010 | 1080775 | 1081341 | 1080775 |
| 1011 | 1083158 | 1081350 | 1083158 |
| 1012 | 1084677 | 1083235 | 1084677 |
| 1013 | 1085648 | 1084632 | 1085648 |
| 1014 | 1086117 | 1086737 | 1086117 |
| 1015 | 1086692 | 1087897 | 1086692 |
| 1016 | 1088646 | 1089005 | 1088646 |
| 1017 | 1089146 | 1089805 | 1089146 |
| 1018 | 1092931 | 1089890 | 1092931 |
| 1019 | 1093179 | 1092889 | 1093179 |
| 1020 | 1093584 | 1094204 | 1093584 |
| 1021 | 1095619 | 1094192 | 1095619 |
| 1022 | 1096074 | 1096628 | 1096074 |
| 1023 | 1096633 | 1097082 | 1096633 |
| 1024 | 1097266 | 1097601 | 1097266 |
| 1025 | 1097622 | 1097867 | 1097622 |
| 1026 | 1097886 | 1098392 | 1097886 |
| 1027 | 1099521 | 1099279 | 1099521 |
| 1028 | 1099689 | 1101053 | 1099704 |
| 1029 | 1102192 | 1101107 | 1102192 |
| 1030 | 1104950 | 1102116 | 1104950 |
| 1031 | 1106508 | 1104946 | 1106508 |
| 1032 | 1106722 | 1107249 | 1106722 |
| 1033 | 1107463 | 1108101 | 1107463 |
| 1034 | 1108041 | 1108421 | 1108041 |
| 1035 | 1108520 | 1113370 | 1108520 |
| 1036 | 1114958 | 1113447 | 1114958 |
| 1037 | 1116915 | 1115071 | 1116915 |
| 1038 | 1118183 | 1116894 | 1118183 |
| 1039 | 1118846 | 1120030 | 1118846 |
| 1040 | 1120040 | 1120522 | 1120040 |
| 1041 | 1120510 | 1121430 | 1120510 |
| 1042 | 1121321 | 1121866 | 1121321 |
| 1043 | 1122123 | 1122899 | 1122123 |
| 1044 | 1124842 | 1125564 | 1124842 |
| 1045 | 1126526 | 1125579 | 1126526 |
| 1046 | 1126519 | 1127676 | 1126519 |
| 1047 | 1127672 | 1128571 | 1127672 |
| 1048 | 1130230 | 1131336 | 1130230 |
| 1049 | 1131480 | 1132553 | 1131480 |
| 1050 | 1132830 | 1133843 | 1132830 |
| 1051 | 1134121 | 1134855 | 1134121 |
| 1052 | 1134642 | 1135592 | 1134642 |
| 1053 | 1135964 | 1135653 | 1135964 |
| 1054 | 1137132 | 1135954 | 1137132 |
| 1055 | 1137169 | 1140102 | 1137169 |
| 1056 | 1141365 | 1140112 | 1141344 |
| 1057 | 1142150 | 1141356 | 1142150 |
| 1058 | 1142520 | 1145660 | 1142520 |
| 1059 | 1145627 | 1146721 | 1145627 |
| 1060 | 1146862 | 1147545 | 1146862 |
| 1061 | 1147666 | 1148190 | 1147666 |
| 1062 | 1148514 | 1148224 | 1148514 |
| 1063 | 1149136 | 1148348 | 1149136 |
| 1064 | 1149702 | 1149166 | 1149702 |
| 1065 | 1150031 | 1150591 | 1150031 |
| 1066 | 1150785 | 1151147 | 1150785 |
| 1067 | 1151165 | 1152181 | 1151165 |
| 1068 | 1152522 | 1154591 | 1152522 |
| 1069 | 1155666 | 1154566 | 1155666 |
| 1070 | 1156743 | 1155670 | 1156740 |
| 1071 | 1156859 | 1157815 | 1156859 |
| 1072 | 1157982 | 1160735 | 1157982 |
| 1073 | 1162620 | 1160917 | 1162620 |
| 1074 | 1162970 | 1162590 | 1162970 |
| 1075 | 1163532 | 1164020 | 1163532 |
| 1076 | 1163995 | 1164294 | 1163995 |
| 1077 | 1165569 | 1165030 | 1165569 |
| 1078 | 1166108 | 1165566 | 1166108 |
| 1079 | 1166644 | 1166141 | 1166644 |
| 1080 | 1167055 | 1168374 | 1167055 |
| 1081 | 1169218 | 1168337 | 1169218 |
| 1082 | 1169823 | 1169218 | 1169823 |
| 1083 | 1171324 | 1170572 | 1171324 |
| 1084 | 1172085 | 1171177 | 1172085 |
| 1085 | 1172394 | 1173773 | 1172394 |
| 1086 | 1175209 | 1173881 | 1175209 |
| 1087 | 1175555 | 1175127 | 1175360 |
| 1088 | 1175778 | 1177043 | 1175778 |
| 1089 | 1177177 | 1179048 | 1177177 |
| 1090 | 1179156 | 1180085 | 1179156 |
| 1091 | 1180045 | 1180779 | 1180045 |
| 1092 | 1181942 | 1180788 | 1181942 |
| 1093 | 1182296 | 1181961 | 1182296 |
| 1094 | 1183844 | 1182300 | 1183844 |
| 1095 | 1184420 | 1183848 | 1184420 |
| 1096 | 1185382 | 1184366 | 1185382 |
| 1097 | 1185858 | 1185226 | 1185858 |
| 1098 | 1186164 | 1186481 | 1186185 |
| 1099 | 1187386 | 1186484 | 1187386 |
| 1100 | 1187370 | 1189028 | 1187370 |
| 1101 | 1189321 | 1190889 | 1189321 |
| 1102 | 1191142 | 1192146 | 1191142 |
| 1103 | 1191974 | 1191729 | 1191974 |
| 1104 | 1193815 | 1192991 | 1193815 |
| 1105 | 1195702 | 1194248 | 1195702 |
| 1106 | 1196303 | 1195716 | 1196303 |
| 1107 | 1196831 | 1196337 | 1196831 |
| 1108 | 1197807 | 1196746 | 1197651 |
| 1109 | 1198740 | 1197883 | 1198668 |
| 1110 | 1200232 | 1198721 | 1200232 |
| 1111 | 1201286 | 1200135 | 1201286 |
| 1112 | 1202386 | 1201259 | 1202350 |
| 1113 | 1202901 | 1202350 | 1202901 |
| 1114 | 1204162 | 1202816 | 1204162 |
| 1115 | 1203177 | 1203464 | 1203177 |
| 1116 | 1205028 | 1204180 | 1205028 |
| 1117 | 1206392 | 1204878 | 1206392 |
| 1118 | 1206742 | 1206086 | 1206742 |
| 1119 | 1207872 | 1206724 | 1207872 |
| 1120 | 1208852 | 1207851 | 1208852 |
| 1121 | 1210518 | 1209742 | 1210518 |
| 1122 | 1210703 | 1211494 | 1210703 |
| 1123 | 1211870 | 1212754 | 1211870 |
| 1124 | 1212742 | 1214064 | 1212742 |
| 1125 | 1214046 | 1214858 | 1214046 |
| 1126 | 1215551 | 1216318 | 1215551 |
| 1127 | 1216493 | 1216849 | 1216493 |
| 1128 | 1217183 | 1219612 | 1217183 |
| 1129 | 1220068 | 1219673 | 1220068 |
| 1130 | 1219710 | 1220669 | 1219710 |
| 1131 | 1220630 | 1221376 | 1220630 |
| 1132 | 1221645 | 1223681 | 1221645 |
| 1133 | 1223894 | 1224988 | 1223900 |
| 1134 | 1225000 | 1225830 | 1225000 |
| 1135 | 1227810 | 1225879 | 1227810 |
| 1136 | 1226528 | 1226908 | 1226528 |
| 1137 | 1229972 | 1228311 | 1229972 |
| 1138 | 47569 | 47018 | 47569 |
| 1139 | 49980 | 49117 | 49980 |
| 1140 | 53356 | 52898 | 53356 |
| 1141 | 54477 | 54884 | 54477 |
| 1142 | 63753 | 63998 | 63753 |
| 1143 | 77164 | 77487 | 77164 |
| 1144 | 79724 | 79302 | 79724 |
| 1145 | 88721 | 88951 | 88721 |
| 1146 | 94067 | 94429 | 94067 |
| 1147 | 122832 | 123341 | 122832 |
| 1148 | 147536 | 147234 | 147536 |
| 1149 | 158990 | 159346 | 158990 |
| 1150 | 168470 | 168979 | 168470 |
| 1151 | 169183 | 169452 | 169204 |
| 1152 | 171785 | 171504 | 171785 |
| 1153 | 172518 | 171775 | 172518 |
| 1154 | 193599 | 194045 | 193599 |
| 1155 | 195704 | 196075 | 195704 |
| 1156 | 210687 | 210145 | 210684 |
| 1157 | 211100 | 210708 | 211100 |
| 1158 | 215420 | 215088 | 215420 |
| 1159 | 217914 | 218246 | 217914 |
| 1160 | 218925 | 218701 | 218925 |
| 1161 | 223785 | 223525 | 223785 |
| 1162 | 224271 | 223999 | 224271 |
| 1163 | 228691 | 228407 | 228691 |
| 1164 | 235050 | 235334 | 235050 |
| 1165 | 252308 | 253021 | 252308 |
| 1166 | 258280 | 258912 | 258280 |
| 1167 | 261325 | 261567 | 261325 |
| 1168 | 268195 | 268878 | 268195 |
| 1169 | 269447 | 268881 | 269447 |
| 1170 | 271263 | 271538 | 271263 |
| 1171 | 271957 | 272346 | 271957 |
| 1172 | 274176 | 274550 | 274176 |
| 1173 | 275736 | 275314 | 275736 |
| 1174 | 276490 | 276927 | 276490 |
| 1175 | 277577 | 277861 | 277577 |
| 1176 | 288163 | 287909 | 288163 |
| 1177 | 290130 | 289789 | 290130 |
| 1178 | 290989 | 291225 | 290989 |
| 1179 | 291372 | 291860 | 291372 |
| 1180 | 311239 | 311622 | 311239 |
| 1181 | 328665 | 328384 | 328665 |
| 1182 | 337348 | 338289 | 337348 |
| 1183 | 364764 | 364369 | 364764 |
| 1184 | 389623 | 390135 | 389623 |
| 1185 | 393729 | 394343 | 393729 |
| 1186 | 407379 | 407621 | 407379 |
| 1187 | 410944 | 410708 | 410944 |
| 1188 | 427632 | 427988 | 427632 |
| 1189 | 428172 | 428486 | 428172 |
| 1190 | 436761 | 437246 | 436761 |
| 1191 | 460911 | 461159 | 460911 |
| 1192 | 477597 | 477313 | 477597 |
| 1193 | 487303 | 487001 | 487303 |
| 1194 | 487764 | 487534 | 487764 |
| 1195 | 498502 | 499017 | 498502 |
| 1196 | 499795 | 500466 | 499795 |
| 1197 | 571928 | 572344 | 571928 |
| 1198 | 572367 | 572131 | 572367 |
| 1199 | 588184 | 587915 | 588184 |
| 1200 | 600587 | 600907 | 600587 |
| 1201 | 609731 | 608895 | 609731 |
| 1202 | 614039 | 614755 | 614039 |
| 1203 | 614823 | 615152 | 614823 |
| 1204 | 638244 | 638831 | 638244 |
| 1205 | 638819 | 639094 | 638819 |
| 1206 | 639073 | 639636 | 639073 |
| 1207 | 647901 | 648236 | 647901 |
| 1208 | 678510 | 679469 | 678510 |
| 1209 | 688178 | 688732 | 688178 |
| 1210 | 696045 | 696563 | 696045 |
| 1211 | 708998 | 708588 | 708998 |
| 1212 | 709808 | 710089 | 709808 |
| 1213 | 718240 | 717737 | 718240 |
| 1214 | 737828 | 737565 | 737828 |
| 1215 | 779502 | 780257 | 779502 |
| 1216 | 806310 | 805864 | 806310 |
| 1217 | 820931 | 820707 | 820931 |
| 1218 | 837696 | 839096 | 837696 |
| 1219 | 883307 | 883549 | 883307 |
| 1220 | 892010 | 891726 | 892010 |
| 1221 | 893277 | 893564 | 893277 |
| 1222 | 936998 | 937225 | 936998 |
| 1223 | 946865 | 947419 | 946865 |
| 1224 | 975187 | 975411 | 975187 |
| 1225 | 985882 | 985517 | 985882 |
| 1226 | 987713 | 987180 | 987713 |
| 1227 | 988215 | 987733 | 988215 |
| 1228 | 988754 | 988530 | 988754 |
| 1229 | 992542 | 992841 | 992542 |
| 1230 | 992759 | 993067 | 992759 |
| 1231 | 1004247 | 1004528 | 1004268 |
| 1232 | 1015013 | 1014294 | 1015013 |
| 1233 | 1056147 | 1056545 | 1056147 |
| 1234 | 1077682 | 1078035 | 1077682 |
| 1235 | 1088121 | 1088381 | 1088121 |
| 1236 | 1098430 | 1098852 | 1098430 |
| 1237 | 1098798 | 1099319 | 1098798 |
| 1238 | 1123198 | 1123515 | 1123198 |
| 1239 | 1123606 | 1124256 | 1123606 |
| 1240 | 1124453 | 1124797 | 1124453 |
| 1241 | 1129253 | 1129567 | 1129253 |
| 1242 | 1164947 | 1164474 | 1164947 |
| 1243 | 1170457 | 1170053 | 1170457 |
| 1244 | 1172342 | 1171863 | 1172342 |
| 1245 | 1192155 | 1192835 | 1192155 |
| 1246 | 1192759 | 1192992 | 1192759 |
| 1247 | 1193861 | 1194142 | 1193861 |
| 1248 | 1194036 | 1193779 | 1194036 |
| 1249 | 1209748 | 1209053 | 1209748 |
| 1250 | 1215111 | 1215419 | 1215111 |
| 1251 | 1216302 | 1216538 | 1216302 |
| 1252 | 1228072 | 1227818 | 1228072 |
| 1253 | 1228304 | 1228080 | 1228304 |
| 1254 | 26599 | 26222 | 26599 |
| 1255 | 27609 | 27367 | 27609 |
| 1256 | 67206 | 66967 | 67197 |
| 1257 | 70612 | 70352 | 70588 |
| 1258 | 132703 | 132945 | 132703 |
| 1259 | 178073 | 178393 | 178073 |
| 1260 | 208576 | 208349 | 208576 |
| 1261 | 209156 | 208929 | 209156 |
| 1262 | 209263 | 209024 | 209263 |
| 1263 | 210304 | 210639 | 210304 |
| 1264 | 299009 | 299452 | 299030 |
| 1265 | 352106 | 351717 | 352061 |
| 1266 | 420182 | 419949 | 420170 |
| 1267 | 553602 | 553381 | 553602 |
| 1268 | 556538 | 556807 | 556538 |
| 1269 | 594348 | 593797 | 594342 |
| 1270 | 595169 | 594876 | 595160 |
| 1271 | 662148 | 662381 | 662160 |
| 1272 | 706528 | 706893 | 706528 |
| 1273 | 803315 | 803650 | 803339 |
| 1274 | 849551 | 849306 | 849551 |
| 1275 | 913676 | 913275 | 913676 |
| 1276 | 927087 | 926836 | 927087 |
| 1277 | 930587 | 930360 | 930587 |
| 1278 | 986531 | 986764 | 986531 |
| 1279 | 996229 | 996486 | 996229 |
| 1280 | 1000373 | 1000002 | 1000334 |
| 1281 | 1010291 | 1010037 | 1010273 |
| 1282 | 1011128 | 1010793 | 1011128 |
| 1283 | 1012924 | 1012694 | 1012924 |
| 1284 | 1028659 | 1028913 | 1028659 |
| 1285 | 1086481 | 1086762 | 1086481 |
| 1286 | 1118658 | 1118879 | 1118658 |
| 1287 | 1170098 | 1169835 | 1170098 |
| 1288 | 1180828 | 1181184 | 1180828 |
| 1289 | 1182658 | 1183035 | 1182658 |
| 1290 | 1195076 | 1194795 | 1195055 |
| 1291 | 1195890 | 1196183 | 1195890 |
| 1292 | 189042 | 188809 | 189030 |
| 1293 | 691250 | 691567 | 691250 |
| 1294 | 914544 | 914780 | 914556 |
| 1295 | 928525 | 928833 | 928579 |
| 1296 | 1040685 | 1040948 | 1040712 |
| 1297 | 377646 | 378068 | 377646 |

**Table 4**

| *NO (ORF)* | *Fp* | *Fd* | *Bp* | *Bd* |
|---|---|---|---|---|
| 2 | 1292 | 1293 | 3796 | 3797 |
| 3 | 1294 | 1295 | 3798 | 3799 |
| 4 | 1296 | 1297 | 3800 | 3801 |
| 5 | 1298 | 1299 | 3802 | 3803 |
| 6 | 1300 | 1301 | 3804 | 3805 |
| 7 | 1302 | 1303 | 3806 | 3807 |
| 8 | 1304 | 1305 | 3808 | 3809 |
| 9 | 1306 | 1307 | 3810 | 3811 |
| 10 | 1308 | 1309 | 3812 | 3813 |
| 11 | 1310 | 1311 | 3814 | 3815 |
| 12 | 1312 | 1313 | 3816 | 3817 |
| 13 | 1314 | 1315 | 3818 | 3819 |
| 14 | 1316 | 1317 | 3820 | 3821 |
| 15 | 1318 | 1319 | 3822 | 3823 |
| 16 | 1320 | 1321 | 3824 | 3825 |
| 17 | 1322 | 1323 | 3826 | 3827 |
| 18 | 1324 | 1325 | 3828 | 3829 |
| 19 | 1326 | 1327 | 3830 | 3831 |
| 20 | 1328 | 1329 | 3832 | 3833 |
| 21 | 1330 | 1331 | 3834 | 3835 |
| 22 | 1332 | 1333 | 3836 | 3837 |
| 23 | 1334 | 1335 | 3838 | 3839 |
| 24 | 1336 | 1337 | 3840 | 3841 |
| 25 | 1338 | 1339 | 3842 | 3843 |
| 26 | 1340 | 1341 | 3844 | 3845 |
| 27 | 1342 | 1343 | 3846 | 3847 |
| 28 | 1344 | 1345 | 3848 | 3849 |
| 29 | 1346 | 1347 | 3850 | 3851 |
| 30 | 1348 | 1349 | 3852 | 3853 |
| 31 | 1350 | 1351 | 3854 | 3855 |
| 32 | 1352 | 1353 | 3856 | 3857 |
| 33 | 1354 | 1355 | 3858 | 3859 |
| 34 | 1358 | 1359 | 3862 | 3863 |
| 35 | 1356 | 1357 | 3860 | 3861 |
| 36 | 1360 | 1361 | 3864 | 3865 |
| 37 | 1362 | 1363 | 3866 | 3867 |
| 38 | 1364 | 1365 | 3868 | 3869 |
| 39 | 1366 | 1367 | 3870 | 3871 |
| 40 | 1368 | 1369 | 3872 | 3873 |
| 41 | 1370 | 1371 | 3874 | 3875 |
| 42 | 1374 | 1375 | 3878 | 3879 |
| 43 | 1376 | 1377 | 3880 | 3881 |
| 44 | 1380 | 1381 | 3884 | 3885 |
| 45 | 1382 | 1383 | 3886 | 3887 |
| 46 | 1386 | 1387 | 3890 | 3891 |
| 47 | 1388 | 1389 | 3892 | 3893 |
| 48 | 1392 | 1393 | 3896 | 3897 |
| 49 | 1394 | 1395 | 3898 | 3899 |
| 50 | 1396 | 1397 | 3900 | 3901 |
| 51 | 1398 | 1399 | 3902 | 3903 |
| 52 | 1402 | 1403 | 3906 | 3907 |
| 53 | 1400 | 1401 | 3904 | 3905 |
| 54 | 1404 | 1405 | 3908 | 3909 |
| 55 | 1406 | 1407 | 3910 | 3911 |
| 56 | 1410 | 1411 | 3914 | 3915 |
| 57 | 1412 | 1413 | 3916 | 3917 |
| 58 | 1414 | 1415 | 3918 | 3919 |
| 59 | 1416 | 1417 | 3920 | 3921 |
| 60 | 1418 | 1419 | 3922 | 3923 |
| 61 | 1420 | 1421 | 3924 | 3925 |
| 62 | 1422 | 1423 | 3926 | 3927 |
| 63 | 1424 | 1425 | 3928 | 3929 |
| 64 | 1426 | 1427 | 3930 | 3931 |
| 65 | 1428 | 1429 | 3932 | 3933 |
| 66 | 1430 | 1431 | 3934 | 3935 |
| 67 | 1432 | 1433 | 3936 | 3937 |
| 68 | 1434 | 1435 | 3938 | 3939 |
| 69 | 1438 | 1439 | 3942 | 3943 |
| 70 | 1440 | 1441 | 3944 | 3945 |
| 71 | 1444 | 1445 | 3948 | 3949 |
| 72 | 1446 | 1447 | 3950 | 3951 |
| 73 | 1448 | 1449 | 3952 | 3953 |
| 74 | 1450 | 1451 | 3954 | 3955 |
| 75 | 1452 | 1453 | 3956 | 3957 |
| 76 | 1454 | 1455 | 3958 | 3959 |
| 77 | 1456 | 1457 | 3960 | 3961 |
| 78 | 1458 | 1459 | 3962 | 3963 |
| 79 | 1460 | 1461 | 3964 | 3965 |
| 80 | 1462 | 1463 | 3966 | 3967 |
| 81 | 1464 | 1465 | 3968 | 3969 |
| 82 | 1468 | 1469 | 3972 | 3973 |
| 83 | 1470 | 1471 | 3974 | 3975 |
| 84 | 1472 | 1473 | 3976 | 3977 |
| 85 | 1476 | 1477 | 3980 | 3981 |
| 86 | 1478 | 1479 | 3982 | 3983 |
| 87 | 1480 | 1481 | 3984 | 3985 |
| 88 | 1482 | 1483 | 3986 | 3987 |
| 89 | 1484 | 1485 | 3988 | 3989 |
| 90 | 1486 | 1487 | 3990 | 3991 |
| 91 | 1488 | 1489 | 3992 | 3993 |
| 92 | 1490 | 1491 | 3994 | 3995 |
| 93 | 1492 | 1493 | 3996 | 3997 |
| 94 | 1494 | 1495 | 3998 | 3999 |
| 95 | 1496 | 1497 | 4000 | 4001 |
| 96 | 1498 | 1499 | 4002 | 4003 |
| 97 | 1500 | 1501 | 4004 | 4005 |
| 98 | 1502 | 1503 | 4006 | 4007 |
| 99 | 1504 | 1505 | 4008 | 4009 |
| 100 | 1506 | 1507 | 4010 | 4011 |
| 101 | 1508 | 1509 | 4012 | 4013 |
| 102 | 1510 | 1511 | 4014 | 4015 |
| 103 | 1512 | 1513 | 4016 | 4017 |
| 104 | 1514 | 1515 | 4018 | 4019 |
| 105 | 1516 | 1517 | 4020 | 4021 |
| 106 | 1518 | 1519 | 4022 | 4023 |
| 107 | 1520 | 1521 | 4024 | 4025 |
| 108 | 1522 | 1523 | 4026 | 4027 |
| 109 | 1524 | 1525 | 4028 | 4029 |
| 110 | 1526 | 1527 | 4030 | 4031 |
| 111 | 1530 | 1531 | 4034 | 4035 |
| 112 | 1532 | 1533 | 4036 | 4037 |
| 113 | 1534 | 1535 | 4038 | 4039 |
| 114 | 1536 | 1537 | 4040 | 4041 |
| 115 | 1538 | 1539 | 4042 | 4043 |
| 116 | 1540 | 1541 | 4044 | 4045 |
| 117 | 1542 | 1543 | 4046 | 4047 |
| 118 | 1544 | 1545 | 4048 | 4049 |
| 119 | 1546 | 1547 | 4050 | 4051 |
| 120 | 1548 | 1549 | 4052 | 4053 |
| 121 | 1550 | 1551 | 4054 | 4055 |
| 122 | 1552 | 1553 | 4056 | 4057 |
| 123 | 1554 | 1555 | 4058 | 4059 |
| 124 | 1556 | 1557 | 4060 | 4061 |
| 125 | 1558 | 1559 | 4062 | 4063 |
| 126 | 1562 | 1563 | 4066 | 4067 |
| 127 | 1564 | 1565 | 4068 | 4069 |
| 128 | 1566 | 1567 | 4070 | 4071 |
| 129 | 1568 | 1569 | 4072 | 4073 |
| 130 | 1570 | 1571 | 4074 | 4075 |
| 131 | 1572 | 1573 | 4076 | 4077 |
| 132 | 1574 | 1575 | 4078 | 4079 |
| 133 | 1576 | 1577 | 4080 | 4081 |
| 134 | 1580 | 1581 | 4084 | 4085 |
| 135 | 1582 | 1583 | 4086 | 4087 |
| 136 | 1584 | 1585 | 4088 | 4089 |
| 137 | 1586 | 1587 | 4090 | 4091 |
| 138 | 1588 | 1589 | 4092 | 4093 |
| 139 | 1590 | 1591 | 4094 | 4095 |
| 140 | 1592 | 1593 | 4096 | 4097 |
| 141 | 1594 | 1595 | 4098 | 4099 |
| 142 | 1596 | 1597 | 4100 | 4101 |
| 143 | 1598 | 1599 | 4102 | 4103 |
| 144 | 1604 | 1605 | 4108 | 4109 |
| 145 | 1606 | 1607 | 4110 | 4111 |
| 146 | 1612 | 1613 | 4116 | 4117 |
| 147 | 1614 | 1615 | 4118 | 4119 |
| 148 | 1616 | 1617 | 4120 | 4121 |
| 149 | 1618 | 1619 | 4122 | 4123 |
| 150 | 1620 | 1621 | 4124 | 4125 |
| 151 | 1624 | 1625 | 4128 | 4129 |
| 152 | 1622 | 1623 | 4126 | 4127 |
| 153 | 1626 | 1627 | 4130 | 4131 |
| 154 | 1628 | 1629 | 4132 | 4133 |
| 155 | 1630 | 1631 | 4134 | 4135 |
| 156 | 1632 | 1633 | 4136 | 4137 |
| 157 | 1634 | 1635 | 4138 | 4139 |
| 158 | 1636 | 1637 | 4140 | 4141 |
| 159 | 1638 | 1639 | 4142 | 4143 |
| 160 | 1640 | 1641 | 4144 | 4145 |
| 161 | 1642 | 1643 | 4146 | 4147 |
| 162 | 1644 | 1645 | 4148 | 4149 |
| 163 | 1646 | 1647 | 4150 | 4151 |
| 164 | 1648 | 1649 | 4152 | 4153 |
| 165 | 1650 | 1651 | 4154 | 4155 |
| 166 | 1652 | 1653 | 4156 | 4157 |
| 167 | 1656 | 1657 | 4160 | 4161 |
| 168 | 1658 | 1659 | 4162 | 4163 |
| 169 | 1662 | 1663 | 4166 | 4167 |
| 170 | 1664 | 1665 | 4168 | 4169 |
| 171 | 1666 | 1667 | 4170 | 4171 |
| 172 | 1668 | 1669 | 4172 | 4173 |
| 173 | 1670 | 1671 | 4174 | 4175 |
| 174 | 1672 | 1673 | 4176 | 4177 |
| 175 | 1674 | 1675 | 4178 | 4179 |
| 176 | 1676 | 1677 | 4180 | 4181 |
| 177 | 1678 | 1679 | 4182 | 4183 |
| 178 | 1684 | 1685 | 4188 | 4189 |
| 179 | 1686 | 1687 | 4190 | 4191 |
| 180 | 1688 | 1689 | 4192 | 4193 |
| 181 | 1690 | 1691 | 4194 | 4195 |
| 182 | 1694 | 1695 | 4198 | 4199 |
| 183 | 1696 | 1697 | 4200 | 4201 |
| 184 | 1698 | 1699 | 4202 | 4203 |
| 185 | 1700 | 1701 | 4204 | 4205 |
| 186 | 1704 | 1705 | 4208 | 4209 |
| 187 | 1708 | 1709 | 4212 | 4213 |
| 188 | 1710 | 1711 | 4214 | 4215 |
| 189 | 1712 | 1713 | 4216 | 4217 |
| 190 | 1714 | 1715 | 4218 | 4219 |
| 191 | 1720 | 1721 | 4224 | 4225 |
| 192 | 1722 | 1723 | 4226 | 4227 |
| 193 | 1724 | 1725 | 4228 | 4229 |
| 194 | 1726 | 1727 | 4230 | 4231 |
| 195 | 1728 | 1729 | 4232 | 4233 |
| 196 | 1732 | 1733 | 4236 | 4237 |
| 197 | 1734 | 1735 | 4238 | 4239 |
| 198 | 1736 | 1737 | 4240 | 4241 |
| 199 | 1738 | 1739 | 4242 | 4243 |
| 200 | 1740 | 1741 | 4244 | 4245 |
| 201 | 1742 | 1743 | 4246 | 4247 |
| 202 | 1744 | 1745 | 4248 | 4249 |
| 203 | 1746 | 1747 | 4250 | 4251 |
| 204 | 1750 | 1751 | 4254 | 4255 |
| 205 | 1752 | 1753 | 4256 | 4257 |
| 206 | 1754 | 1755 | 4258 | 4259 |
| 207 | 1756 | 1757 | 4260 | 4261 |
| 208 | 1758 | 1759 | 4262 | 4263 |
| 209 | 1760 | 1761 | 4264 | 4265 |
| 210 | 1762 | 1763 | 4266 | 4267 |
| 211 | 1764 | 1765 | 4268 | 4269 |
| 212 | 1766 | 1767 | 4270 | 4271 |
| 213 | 1768 | 1769 | 4272 | 4273 |
| 214 | 1770 | 1771 | 4274 | 4275 |
| 215 | 1772 | 1773 | 4276 | 4277 |
| 216 | 1774 | 1775 | 4278 | 4279 |
| 217 | 1776 | 1777 | 4280 | 4281 |
| 218 | 1778 | 1779 | 4282 | 4283 |
| 219 | 1782 | 1783 | 4286 | 4287 |
| 220 | 1784 | 1785 | 4288 | 4289 |
| 221 | 1786 | 1787 | 4290 | 4291 |
| 222 | 1788 | 1789 | 4292 | 4293 |
| 223 | 1790 | 1791 | 4294 | 4295 |
| 224 | 1792 | 1793 | 4296 | 4297 |
| 225 | 1796 | 1797 | 4300 | 4301 |
| 226 | 1800 | 1801 | 4304 | 4305 |
| 227 | 1802 | 1803 | 4306 | 4307 |
| 228 | 1804 | 1805 | 4308 | 4309 |
| 229 | 1806 | 1807 | 4310 | 4311 |
| 230 | 1808 | 1809 | 4312 | 4313 |
| 231 | 1810 | 1811 | 4314 | 4315 |
| 232 | 1812 | 1813 | 4316 | 4317 |
| 233 | 1818 | 1819 | 4322 | 4323 |
| 234 | 1824 | 1825 | 4328 | 4329 |
| 235 | 1826 | 1827 | 4330 | 4331 |
| 236 | 1828 | 1829 | 4332 | 4333 |
| 237 | 1834 | 1835 | 4338 | 4339 |
| 238 | 1836 | 1837 | 4340 | 4341 |
| 239 | 1840 | 1841 | 4344 | 4345 |
| 240 | 1842 | 1843 | 4346 | 4347 |
| 241 | 1846 | 1847 | 4350 | 4351 |
| 242 | 1848 | 1849 | 4352 | 4353 |
| 243 | 1850 | 1851 | 4354 | 4355 |
| 244 | 1852 | 1853 | 4356 | 4357 |
| 245 | 1854 | 1855 | 4358 | 4359 |
| 246 | 1856 | 1857 | 4360 | 4361 |
| 247 | 1858 | 1859 | 4362 | 4363 |
| 248 | 1860 | 1861 | 4364 | 4365 |
| 249 | 1862 | 1863 | 4366 | 4367 |
| 250 | 1864 | 1865 | 4368 | 4369 |
| 251 | 1866 | 1867 | 4370 | 4371 |
| 252 | 1868 | 1869 | 4372 | 4373 |
| 253 | 1872 | 1873 | 4376 | 4377 |
| 254 | 1876 | 1877 | 4380 | 4381 |
| 255 | 1874 | 1875 | 4378 | 4379 |
| 256 | 1878 | 1879 | 4382 | 4383 |
| 257 | 1886 | 1887 | 4390 | 4391 |
| 258 | 1888 | 1889 | 4392 | 4393 |
| 259 | 1890 | 1891 | 4394 | 4395 |
| 260 | 1892 | 1893 | 4396 | 4397 |
| 261 | 1896 | 1897 | 4400 | 4401 |
| 262 | 1894 | 1895 | 4398 | 4399 |
| 263 | 1898 | 1899 | 4402 | 4403 |
| 264 | 1900 | 1901 | 4404 | 4405 |
| 265 | 1902 | 1903 | 4406 | 4407 |
| 266 | 1904 | 1905 | 4408 | 4409 |
| 267 | 1906 | 1907 | 4410 | 4411 |
| 268 | 1908 | 1909 | 4412 | 4413 |
| 269 | 1910 | 1911 | 4414 | 4415 |
| 270 | 1912 | 1913 | 4416 | 4417 |
| 271 | 1914 | 1915 | 4418 | 4419 |
| 272 | 1916 | 1917 | 4420 | 4421 |
| 273 | 1918 | 1919 | 4422 | 4423 |
| 274 | 1920 | 1921 | 4424 | 4425 |
| 275 | 1922 | 1923 | 4426 | 4427 |
| 276 | 1924 | 1925 | 4428 | 4429 |
| 277 | 1926 | 1927 | 4430 | 4431 |
| 278 | 1928 | 1929 | 4432 | 4433 |
| 279 | 1930 | 1931 | 4434 | 4435 |
| 280 | 1934 | 1935 | 4438 | 4439 |
| 281 | 1936 | 1937 | 4440 | 4441 |
| 282 | 1938 | 1939 | 4442 | 4443 |
| 283 | 1940 | 1941 | 4444 | 4445 |
| 284 | 1942 | 1943 | 4446 | 4447 |
| 285 | 1944 | 1945 | 4448 | 4449 |
| 286 | 1946 | 1947 | 4450 | 4451 |
| 287 | 1948 | 1949 | 4452 | 4453 |
| 288 | 1950 | 1951 | 4454 | 4455 |
| 289 | 1952 | 1953 | 4456 | 4457 |
| 290 | 1954 | 1955 | 4458 | 4459 |
| 291 | 1956 | 1957 | 4460 | 4461 |
| 292 | 1958 | 1959 | 4462 | 4463 |
| 293 | 1960 | 1961 | 4464 | 4465 |
| 294 | 1962 | 1963 | 4466 | 4467 |
| 295 | 1964 | 1965 | 4468 | 4469 |
| 296 | 1966 | 1967 | 4470 | 4471 |
| 297 | 1970 | 1971 | 4474 | 4475 |
| 298 | 1972 | 1973 | 4476 | 4477 |
| 299 | 1974 | 1975 | 4478 | 4479 |
| 300 | 1976 | 1977 | 4480 | 4481 |
| 301 | 1978 | 1979 | 4482 | 4483 |
| 302 | 1980 | 1981 | 4484 | 4485 |
| 303 | 1984 | 1985 | 4488 | 4489 |
| 304 | 1986 | 1987 | 4490 | 4491 |
| 305 | 1988 | 1989 | 4492 | 4493 |
| 306 | 1990 | 1991 | 4494 | 4495 |
| 307 | 1992 | 1993 | 4496 | 4497 |
| 308 | 1994 | 1995 | 4498 | 4499 |
| 309 | 1996 | 1997 | 4500 | 4501 |
| 310 | 1998 | 1999 | 4502 | 4503 |
| 311 | 2000 | 2001 | 4504 | 4505 |
| 312 | 2002 | 2003 | 4506 | 4507 |
| 313 | 2004 | 2005 | 4508 | 4509 |
| 314 | 2006 | 2007 | 4510 | 4511 |
| 315 | 2008 | 2009 | 4512 | 4513 |
| 316 | 2010 | 2011 | 4514 | 4515 |
| 317 | 2012 | 2013 | 4516 | 4517 |
| 318 | 2014 | 2015 | 4518 | 4519 |
| 319 | 2016 | 2017 | 4520 | 4521 |
| 320 | 2018 | 2019 | 4522 | 4523 |
| 321 | 2020 | 2021 | 4524 | 4525 |
| 322 | 2022 | 2023 | 4526 | 4527 |
| 323 | 2026 | 2027 | 4530 | 4531 |
| 324 | 2024 | 2025 | 4528 | 4529 |
| 325 | 2028 | 2029 | 4532 | 4533 |
| 326 | 2030 | 2031 | 4534 | 4535 |
| 327 | 2032 | 2033 | 4536 | 4537 |
| 328 | 2034 | 2035 | 4538 | 4539 |
| 329 | 2038 | 2039 | 4542 | 4543 |
| 330 | 2040 | 2041 | 4544 | 4545 |
| 331 | 2042 | 2043 | 4546 | 4547 |
| 332 | 2044 | 2045 | 4548 | 4549 |
| 333 | 2046 | 2047 | 4550 | 4551 |
| 334 | 2048 | 2049 | 4552 | 4553 |
| 335 | 2050 | 2051 | 4554 | 4555 |
| 336 | 2052 | 2053 | 4556 | 4557 |
| 337 | 2054 | 2055 | 4558 | 4559 |
| 338 | 2056 | 2057 | 4560 | 4561 |
| 339 | 2058 | 2059 | 4562 | 4563 |
| 340 | 2060 | 2061 | 4564 | 4565 |
| 341 | 2062 | 2063 | 4566 | 4567 |
| 342 | 2064 | 2065 | 4568 | 4569 |
| 343 | 2066 | 2067 | 4570 | 4571 |
| 344 | 2068 | 2069 | 4572 | 4573 |
| 345 | 2070 | 2071 | 4574 | 4575 |
| 346 | 2072 | 2073 | 4576 | 4577 |
| 347 | 2074 | 2075 | 4578 | 4579 |
| 348 | 2076 | 2077 | 4580 | 4581 |
| 349 | 2078 | 2079 | 4582 | 4583 |
| 350 | 2080 | 2081 | 4584 | 4585 |
| 351 | 2082 | 2083 | 4586 | 4587 |
| 352 | 2084 | 2085 | 4588 | 4589 |
| 353 | 2088 | 2089 | 4592 | 4593 |
| 354 | 2090 | 2091 | 4594 | 4595 |
| 355 | 2092 | 2093 | 4596 | 4597 |
| 356 | 2094 | 2095 | 4598 | 4599 |
| 357 | 2096 | 2097 | 4600 | 4601 |
| 358 | 2100 | 2101 | 4604 | 4605 |
| 359 | 2102 | 2103 | 4606 | 4607 |
| 360 | 2104 | 2105 | 4608 | 4609 |
| 361 | 2106 | 2107 | 4610 | 4611 |
| 362 | 2108 | 2109 | 4612 | 4613 |
| 363 | 2110 | 2111 | 4614 | 4615 |
| 364 | 2112 | 2113 | 4616 | 4617 |
| 365 | 2114 | 2115 | 4618 | 4619 |
| 366 | 2116 | 2117 | 4620 | 4621 |
| 367 | 2118 | 2119 | 4622 | 4623 |
| 368 | 2120 | 2121 | 4624 | 4625 |
| 369 | 2122 | 2123 | 4626 | 4627 |
| 370 | 2124 | 2125 | 4628 | 4629 |
| 371 | 2128 | 2129 | 4632 | 4633 |
| 372 | 2130 | 2131 | 4634 | 4635 |
| 373 | 2134 | 2135 | 4638 | 4639 |
| 374 | 2136 | 2137 | 4640 | 4641 |
| 375 | 2138 | 2139 | 4642 | 4643 |
| 376 | 2140 | 2141 | 4644 | 4645 |
| 377 | 2142 | 2143 | 4646 | 4647 |
| 378 | 2144 | 2145 | 4648 | 4649 |
| 379 | 2146 | 2147 | 4650 | 4651 |
| 380 | 2148 | 2149 | 4652 | 4653 |
| 381 | 2150 | 2151 | 4654 | 4655 |
| 382 | 2152 | 2153 | 4656 | 4657 |
| 383 | 2154 | 2155 | 4658 | 4659 |
| 384 | 2156 | 2157 | 4660 | 4661 |
| 385 | 2158 | 2159 | 4662 | 4663 |
| 386 | 2160 | 2161 | 4664 | 4665 |
| 387 | 2162 | 2163 | 4666 | 4667 |
| 388 | 2164 | 2165 | 4668 | 4669 |
| 389 | 2170 | 2171 | 4674 | 4675 |
| 390 | 2172 | 2173 | 4676 | 4677 |
| 391 | 2174 | 2175 | 4678 | 4679 |
| 392 | 2176 | 2177 | 4680 | 4681 |
| 393 | 2178 | 2179 | 4682 | 4683 |
| 394 | 2180 | 2181 | 4684 | 4685 |
| 395 | 2182 | 2183 | 4686 | 4687 |
| 396 | 2186 | 2187 | 4690 | 4691 |
| 397 | 2190 | 2191 | 4694 | 4695 |
| 398 | 2188 | 2189 | 4692 | 4693 |
| 399 | 2194 | 2195 | 4698 | 4699 |
| 400 | 2192 | 2193 | 4696 | 4697 |
| 401 | 2196 | 2197 | 4700 | 4701 |
| 402 | 2200 | 2201 | 4704 | 4705 |
| 403 | 2198 | 2199 | 4702 | 4703 |
| 404 | 2202 | 2203 | 4706 | 4707 |
| 405 | 2204 | 2205 | 4708 | 4709 |
| 406 | 2206 | 2207 | 4710 | 4711 |
| 407 | 2208 | 2209 | 4712 | 4713 |
| 408 | 2210 | 2211 | 4714 | 4715 |
| 409 | 2212 | 2213 | 4716 | 4717 |
| 410 | 2214 | 2215 | 4718 | 4719 |
| 411 | 2216 | 2217 | 4720 | 4721 |
| 412 | 2218 | 2219 | 4722 | 4723 |
| 413 | 2220 | 2221 | 4724 | 4725 |
| 414 | 2222 | 2223 | 4726 | 4727 |
| 415 | 2224 | 2225 | 4728 | 4729 |
| 416 | 2226 | 2227 | 4730 | 4731 |
| 417 | 2228 | 2229 | 4732 | 4733 |
| 418 | 2230 | 2231 | 4734 | 4735 |
| 419 | 2232 | 2233 | 4736 | 4737 |
| 420 | 2234 | 2235 | 4738 | 4739 |
| 421 | 2236 | 2237 | 4740 | 4741 |
| 422 | 2238 | 2239 | 4742 | 4743 |
| 423 | 2242 | 2243 | 4746 | 4747 |
| 424 , | 2244 | 2245 | 4748 | 4749 |
| 425 | 2246 | 2247 | 4750 | 4751 |
| 426 | 2248 | 2249 | 4752 | 4753 |
| 427 | 2250 | 2251 | 4754 | 4755 |
| 428 | 2252 | 2253 | 4756 | 4757 |
| 429 | 2254 | 2255 | 4758 | 4759 |
| 430 | 2256 | 2257 | 4760 | 4761 |
| 431 | 2258 | 2259 | 4762 | 4763 |
| 432 | 2260 | 2261 | 4764 | 4765 |
| 433 | 2262 | 2263 | 4766 | 4767 |
| 434 | 2266 | 2267 | 4770 | 4771 |
| 435 | 2264 | 2265 | 4768 | 4769 |
| 436 | 2268 | 2269 | 4772 | 4773 |
| 437 | 2270 | 2271 | 4774 | 4775 |
| 438 | 2272 | 2273 | 4776 | 4777 |
| 439 | 2274 | 2275 | 4778 | 4779 |
| 440 | 2278 | 2279 | 4782 | 4783 |
| 441 | 2280 | 2281 | 4784 | 4785 |
| 442 | 2282 | 2283 | 4786 | 4787 |
| 443 | 2284 | 2285 | 4788 | 4789 |
| 444 | 2286 | 2287 | 4790 | 4791 |
| 445 | 2288 | 2289 | 4792 | 4793 |
| 446 | 2290 | 2291 | 4794 | 4795 |
| 447 | 2292 | 2293 | 4796 | 4797 |
| 448 | 2294 | 2295 | 4798 | 4799 |
| 449 | 2296 | 2297 | 4800 | 4801 |
| 450 | 2298 | 2299 | 4802 | 4803 |
| 451 | 2304 | 2305 | 4808 | 4809 |
| 452 | 2306 | 2307 | 4810 | 4811 |
| 453 | 2308 | 2309 | 4812 | 4813 |
| 454 | 2310 | 2311 | 4814 | 4815 |
| 455 | 2312 | 2313 | 4816 | 4817 |
| 456 | 2314 | 2315 | 4818 | 4819 |
| 457 | 2316 | 2317 | 4820 | 4821 |
| 458 | 2318 | 2319 | 4822 | 4823 |
| 459 | 2320 | 2321 | 4824 | 4825 |
| 460 | 2322 | 2323 | 4826 | 4827 |
| 461 | 2324 | 2325 | 4828 | 4829 |
| 462 | 2326 | 2327 | 4830 | 4831 |
| 463 | 2328 | 2329 | 4832 | 4833 |
| 464 | 2332 | 2333 | 4836 | 4837 |
| 465 | 2334 | 2335 | 4838 | 4839 |
| 466 | 2338 | 2339 | 4842 | 4843 |
| 467 | 2340 | 2341 | 4844 | 4845 |
| 468 | 2342 | 2343 | 4846 | 4847 |
| 469 | 2344 | 2345 | 4848 | 4849 |
| 470 | 2346 | 2347 | 4850 | 4851 |
| 471 | 2348 | 2349 | 4852 | 4853 |
| 472 | 2350 | 2351 | 4854 | 4855 |
| 473 | 2352 | 2353 | 4856 | 4857 |
| 474 | 2356 | 2357 | 4860 | 4861 |
| 475 | 2354 | 2355 | 4858 | 4859 |
| 476 | 2358 | 2359 | 4862 | 4863 |
| 477 | 2360 | 2361 | 4864 | 4865 |
| 478 | 2362 | 2363 | 4866 | 4867 |
| 479 | 2364 | 2365 | 4868 | 4869 |
| 480 | 2366 | 2367 | 4870 | 4871 |
| 481 | 2368 | 2369 | 4872 | 4873 |
| 482 | 2370 | 2371 | 4874 | 4875 |
| 483 | 2372 | 2373 | 4876 | 4877 |
| 484 | 2374 | 2375 | 4878 | 4879 |
| 485 | 2376 | 2377 | 4880 | 4881 |
| 486 | 2378 | 2379 | 4882 | 4883 |
| 487 | 2380 | 2381 | 4884 | 4885 |
| 488 | 2382 | 2383 | 4886 | 4887 |
| 489 | 2384 | 2385 | 4888 | 4889 |
| 490 | 2386 | 2387 | 4890 | 4891 |
| 491 | 2388 | 2389 | 4892 | 4893 |
| 492 | 2390 | 2391 | 4894 | 4895 |
| 493 | 2392 | 2393 | 4896 | 4897 |
| 494 | 2394 | 2395 | 4898 | 4899 |
| 495 | 2396 | 2397 | 4900 | 4901 |
| 496 | 2398 | 2399 | 4902 | 4903 |
| 497 | 2400 | 2401 | 4904 | 4905 |
| 498 | 2402 | 2403 | 4906 | 4907 |
| 499 | 2404 | 2405 | 4908 | 4909 |
| 500 | 2406 | 2407 | 4910 | 4911 |
| 501 | 2408 | 2409 | 4912 | 4913 |
| 502 | 2410 | 2411 | 4914 | 4915 |
| 503 | 2412 | 2413 | 4916 | 4917 |
| 504 | 2414 | 2415 | 4918 | 4919 |
| 505 | 2416 | 2417 | 4920 | 4921 |
| 506 | 2418 | 2419 | 4922 | 4923 |
| 507 | 2420 | 2421 | 4924 | 4925 |
| 508 | 2422 | 2423 | 4926 | 4927 |
| 509 | 2426 | 2427 | 4930 | 4931 |
| 510 | 2424 | 2425 | 4928 | 4929 |
| 511 | 2430 | 2431 | 4934 | 4935 |
| 512 | 2428 | 2429 | 4932 | 4933 |
| 513 | 2432 | 2433 | 4936 | 4937 |
| 514 | 2434 | 2435 | 4938 | 4939 |
| 515 | 2436 | 2437 | 4940 | 4941 |
| 516 | 2438 | 2439 | 4942 | 4943 |
| 517 | 2440 | 2441 | 4944 | 4945 |
| 518 | 2442 | 2443 | 4946 | 4947 |
| 519 | 2444 | 2445 | 4948 | 4949 |
| 520 | 2446 | 2447 | 4950 | 4951 |
| 521 | 2450 | 2451 | 4954 | 4955 |
| 522 | 2454 | 2455 | 4958 | 4959 |
| 523 | 2456 | 2457 | 4960 | 4961 |
| 524 | 2458 | 2459 | 4962 | 4963 |
| 525 | 2460 | 2461 | 4964 | 4965 |
| 526 | 2462 | 2463 | 4966 | 4967 |
| 527 | 2466 | 2467 | 4970 | 4971 |
| 528 | 2464 | 2465 | 4968 | 4969 |
| 529 | 2468 | 2469 | 4972 | 4973 |
| 530 | 2470 | 2471 | 4974 | 4975 |
| 531 | 2472 | 2473 | 4976 | 4977 |
| 532 | 2474 | 2475 | 4978 | 4979 |
| 533 | 2476 | 2477 | 4980 | 4981 |
| 534 | 2478 | 2479 | 4982 | 4983 |
| 535 | 2480 | 2481 | 4984 | 4985 |
| 536 | 2482 | 2483 | 4986 | 4987 |
| 537 | 2486 | 2487 | 4990 | 4991 |
| 538 | 2488 | 2489 | 4992 | 4993 |
| 539 | 2490 | 2491 | 4994 | 4995 |
| 540 | 2492 | 2493 | 4996 | 4997 |
| 541 | 2494 | 2495 | 4998 | 4999 |
| 542 | 2496 | 2497 | 5000 | 5001 |
| 543 | 2498 | 2499 | 5002 | 5003 |
| 544 | 2500 | 2501 | 5004 | 5005 |
| 545 | 2502 | 2503 | 5006 | 5007 |
| 546 | 2504 | 2505 | 5008 | 5009 |
| 547 | 2506 | 2507 | 5010 | 5011 |
| 548 | 2508 | 2509 | 5012 | 5013 |
| 549 | 2510 | 2511 | 5014 | 5015 |
| 550 | 2514 | 2515 | 5018 | 5019 |
| 551 | 2516 | 2517 | 5020 | 5021 |
| 552 | 2518 | 2519 | 5022 | 5023 |
| 553 | 2520 | 2521 | 5024 | 5025 |
| 554 | 2522 | 2523 | 5026 | 5027 |
| 555 | 2524 | 2525 | 5028 | 5029 |
| 556 | 2526 | 2527 | 5030 | 5031 |
| 557 | 2528 | 2529 | 5032 | 5033 |
| 558 | 2532 | 2533 | 5036 | 5037 |
| 559 | 2534 | 2535 | 5038 | 5039 |
| 560 | 2536 | 2537 | 5040 | 5041 |
| 561 | 2538 | 2539 | 5042 | 5043 |
| 562 | 2544 | 2545 | 5048 | 5049 |
| 563 | 2546 | 2547 | 5050 | 5051 |
| 564 | 2548 | 2549 | 5052 | 5053 |
| 565 | 2552 | 2553 | 5056 | 5057 |
| 566 | 2550 | 2551 | 5054 | 5055 |
| 567 | 2554 | 2555 | 5058 | 5059 |
| 568 | 2556 | 2557 | 5060 | 5061 |
| 569 | 2558 | 2559 | 5062 | 5063 |
| 570 | 2560 | 2561 | 5064 | 5065 |
| 571 | 2562 | 2563 | 5066 | 5067 |
| 572 | 2564 | 2565 | 5068 | 5069 |
| 573 | 2566 | 2567 | 5070 | 5071 |
| 574 | 2568 | 2569 | 5072 | 5073 |
| 575 | 2570 | 2571 | 5074 | 5075 |
| 576 | 2572 | 2573 | 5076 | 5077 |
| 577 | 2574 | 2575 | 5078 | 5079 |
| 578 | 2576 | 2577 | 5080 | 5081 |
| 579 | 2578 | 2579 | 5082 | 5083 |
| 580 | 2580 | 2581 | 5084 | 5085 |
| 581 | 2582 | 2583 | 5086 | 5087 |
| 582 | 2590 | 2591 | 5094 | 5095 |
| 583 | 2592 | 2593 | 5096 | 5097 |
| 584 | 2594 | 2595 | 5098 | 5099 |
| 585 | 2596 | 2597 | 5100 | 5101 |
| 586 | 2598 | 2599 | 5102 | 5103 |
| 587 | 2600 | 2601 | 5104 | 5105 |
| 588 | 2602 | 2603 | 5106 | 5107 |
| 589 | 2604 | 2605 | 5108 | 5109 |
| 590 | 2606 | 2607 | 5110 | 5111 |
| 591 | 2608 | 2609 | 5112 | 5113 |
| 592 | 2610 | 2611 | 5114 | 5115 |
| 593 | 2612 | 2613 | 5116 | 5117 |
| 594 | 2616 | 2617 | 5120 | 5121 |
| 595 | 2618 | 2619 | 5122 | 5123 |
| 596 | 2620 | 2621 | 5124 | 5125 |
| 597 | 2622 | 2623 | 5126 | 5127 |
| 598 | 2624 | 2625 | 5128 | 5129 |
| 599 | 2626 | 2627 | 5130 | 5131 |
| 600 | 2628 | 2629 | 5132 | 5133 |
| 601 | 2630 | 2631 | 5134 | 5135 |
| 602 | 2632 | 2633 | 5136 | 5137 |
| 603 | 2634 | 2635 | 5138 | 5139 |
| 604 | 2636 | 2637 | 5140 | 5141 |
| 605 | 2638 | 2639 | 5142 | 5143 |
| 606 | 2640 | 2641 | 5144 | 5145 |
| 607 | 2642 | 2643 | 5146 | 5147 |
| 608 | 2644 | 2645 | 5148 | 5149 |
| 609 | 2646 | 2647 | 5150 | 5151 |
| 610 | 2650 | 2651 | 5154 | 5155 |
| 611 | 2648 | 2649 | 5152 | 5153 |
| 612 | 2652 | 2653 | 5156 | 5157 |
| 613 | 2654 | 2655 | 5158 | 5159 |
| 614 | 2656 | 2657 | 5160 | 5161 |
| 615 | 2658 | 2659 | 5162 | 5163 |
| 616 | 2660 | 2661 | 5164 | 5165 |
| 617 | 2662 | 2663 | 5166 | 5167 |
| 618 | 2664 | 2665 | 5168 | 5169 |
| 619 | 2666 | 2667 | 5170 | 5171 |
| 620 | 2668 | 2669 | 5172 | 5173 |
| 621 | 2672 | 2673 | 5176 | 5177 |
| 622 | 2674 | 2675 | 5178 | 5179 |
| 623 | 2678 | 2679 | 5182 | 5183 |
| 624 | 2676 | 2677 | 5180 | 5181 |
| 625 | 2680 | 2681 | 5184 | 5185 |
| 626 | 2682 | 2683 | 5186 | 5187 |
| 627 | 2684 | 2685 | 5188 | 5189 |
| 628 | 2686 | 2687 | 5190 | 5191 |
| 629 | 2688 | 2689 | 5192 | 5193 |
| 630 | 2690 | 2691 | 5194 | 5195 |
| 631 | 2692 | 2693 | 5196 | 5197 |
| 632 | 2696 | 2697 | 5200 | 5201 |
| 633 | 2698 | 2699 | 5202 | 5203 |
| 634 | 2700 | 2701 | 5204 | 5205 |
| 635 | 2702 | 2703 | 5206 | 5207 |
| 636 | 2704 | 2705 | 5208 | 5209 |
| 637 | 2706 | 2707 | 5210 | 5211 |
| 638 | 2710 | 2711 | 5214 | 5215 |
| 639 | 2712 | 2713 | 5216 | 5217 |
| 640 | 2714 | 2715 | 5218 | 5219 |
| 641 | 2716 | 2717 | 5220 | 5221 |
| 642 | 2718 | 2719 | 5222 | 5223 |
| 643 | 2720 | 2721 | 5224 | 5225 |
| 644 | 2722 | 2723 | 5226 | 5227 |
| 645 | 2724 | 2725 | 5228 | 5229 |
| 646 | 2726 | 2727 | 5230 | 5231 |
| 647 | 2728 | 2729 | 5232 | 5233 |
| 648 | 2732 | 2733 | 5236 | 5237 |
| 649 | 2736 | 2737 | 5240 | 5241 |
| 650 | 2738 | 2739 | 5242 | 5243 |
| 651 | 2740 | 2741 | 5244 | 5245 |
| 652 | 2742 | 2743 | 5246 | 5247 |
| 653 | 2744 | 2745 | 5248 | 5249 |
| 654 | 2748 | 2749 | 5252 | 5253 |
| 655 | 2750 | 2751 | 5254 | 5255 |
| 656 | 2752 | 2753 | 5256 | 5257 |
| 657 | 2754 | 2755 | 5258 | 5259 |
| 658 | 2756 | 2757 | 5260 | 5261 |
| 659 | 2758 | 2759 | 5262 | 5263 |
| 660 | 2760 | 2761 | 5264 | 5265 |
| 661 | 2762 | 2763 | 5266 | 5267 |
| 662 | 2764 | 2765 | 5268 | 5269 |
| 663 | 2766 | 2767 | 5270 | 5271 |
| 664 | 2768 | 2769 | 5272 | 5273 |
| 665 | 2770 | 2771 | 5274 | 5275 |
| 666 | 2772 | 2773 | 5276 | 5277 |
| 667 | 2774 | 2775 | 5278 | 5279 |
| 668 | 2776 | 2777 | 5280 | 5281 |
| 669 | 2778 | 2779 | 5282 | 5283 |
| 670 | 2780 | 2781 | 5284 | 5285 |
| 671 | 2782 | 2783 | 5286 | 5287 |
| 672 | 2784 | 2785 | 5288 | 5289 |
| 673 | 2786 | 2787 | 5290 | 5291 |
| 674 | 2788 | 2789 | 5292 | 5293 |
| 675 | 2790 | 2791 | 5294 | 5295 |
| 676 | 2792 | 2793 | 5296 | 5297 |
| 677 | 2794 | 2795 | 5298 | 5299 |
| 678 | 2796 | 2797 | 5300 | 5301 |
| 679 | 2798 | 2799 | 5302 | 5303 |
| 680 | 2800 | 2801 | 5304 | 5305 |
| 681 | 2804 | 2805 | 5308 | 5309 |
| 682 | 2806 | 2807 | 5310 | 5311 |
| 683 | 2808 | 2809 | 5312 | 5313 |
| 684 | 2810 | 2811 | 5314 | 5315 |
| 685 | 2812 | 2813 | 5316 | 5317 |
| 686 | 2814 | 2815 | 5318 | 5319 |
| 687 | 2816 | 2817 | 5320 | 5321 |
| 688 | 2818 | 2819 | 5322 | 5323 |
| 689 | 2820 | 2821 | 5324 | 5325 |
| 690 | 2822 | 2823 | 5326 | 5327 |
| 691 | 2824 | 2825 | 5328 | 5329 |
| 692 | 2826 | 2827 | 5330 | 5331 |
| 693 | 2828 | 2829 | 5332 | 5333 |
| 694 | 2830 | 2831 | 5334 | 5335 |
| 695 | 2832 | 2833 | 5336 | 5337 |
| 696 | 2834 | 2835 | 5338 | 5339 |
| 697 | 2836 | 2837 | 5340 | 5341 |
| 698 | 2838 | 2839 | 5342 | 5343 |
| 699 | 2840 | 2841 | 5344 | 5345 |
| 700 | 2842 | 2843 | 5346 | 5347 |
| 701 | 2844 | 2845 | 5348 | 5349 |
| 702 | 2846 | 2847 | 5350 | 5351 |
| 703 | 2848 | 2849 | 5352 | 5353 |
| 704 | 2850 | 2851 | 5354 | 5355 |
| 705 | 2852 | 2853 | 5356 | 5357 |
| 706 | 2854 | 2855 | 5358 | 5359 |
| 707 | 2856 | 2857 | 5360 | 5361 |
| 708 | 2858 | 2859 | 5362 | 5363 |
| 709 | 2860 | 2861 | 5364 | 5365 |
| 710 | 2862 | 2863 | 5366 | 5367 |
| 711 | 2864 | 2865 | 5368 | 5369 |
| 712 | 2866 | 2867 | 5370 | 5371 |
| 713 | 2868 | 2869 | 5372 | 5373 |
| 714 | 2870 | 2871 | 5374 | 5375 |
| 715 | 2872 | 2873 | 5376 | 5377 |
| 716 | 2874 | 2875 | 5378 | 5379 |
| 717 | 2876 | 2877 | 5380 | 5381 |
| 718 | 2878 | 2879 | 5382 | 5383 |
| 719 | 2880 | 2881 | 5384 | 5385 |
| 720 | 2882 | 2883 | 5386 | 5387 |
| 721 | 2886 | 2887 | 5390 | 5391 |
| 722 | 2888 | 2889 | 5392 | 5393 |
| 723 | 2884 | 2885 | 5388 | 5389 |
| 724 | 2890 | 2891 | 5394 | 5395 |
| 725 | 2892 | 2893 | 5396 | 5397 |
| 726 | 2894 | 2895 | 5398 | 5399 |
| 727 | 2896 | 2897 | 5400 | 5401 |
| 728 | 2900 | 2901 | 5404 | 5405 |
| 729 | 2902 | 2903 | 5406 | 5407 |
| 730 | 2904 | 2905 | 5408 | 5409 |
| 731 | 2906 | 2907 | 5410 | 5411 |
| 732 | 2908 | 2909 | 5412 | 5413 |
| 733 | 2910 | 2911 | 5414 | 5415 |
| 734 | 2912 | 2913 | 5416 | 5417 |
| 735 | 2914 | 2915 | 5418 | 5419 |
| 736 | 2916 | 2917 | 5420 | 5421 |
| 737 | 2918 | 2919 | 5422 | 5423 |
| 738 | 2920 | 2921 | 5424 | 5425 |
| 739 | 2922 | 2923 | 5426 | 5427 |
| 740 | 2924 | 2925 | 5428 | 5429 |
| 741 | 2926 | 2927 | 5430 | 5431 |
| 742 | 2928 | 2929 | 5432 | 5433 |
| 743 | 2930 | 2931 | 5434 | 5435 |
| 744 | 2932 | 2933 | 5436 | 5437 |
| 745 | 2934 | 2935 | 5438 | 5439 |
| 746 | 2936 | 2937 | 5440 | 5441 |
| 747 | 2938 | 2939 | 5442 | 5443 |
| 748 | 2940 | 2941 | 5444 | 5445 |
| 749 | 2942 | 2943 | 5446 | 5447 |
| 750 | 2944 | 2945 | 5448 | 5449 |
| 751 | 2946 | 2947 | 5450 | 5451 |
| 752 | 2948 | 2949 | 5452 | 5453 |
| 753 | 2952 | 2953 | 5456 | 5457 |
| 754 | 2954 | 2955 | 5458 | 5459 |
| 755 | 2956 | 2957 | 5460 | 5461 |
| 756 | 2958 | 2959 | 5462 | 5463 |
| 757 | 2960 | 2961 | 5464 | 5465 |
| 758 | 2962 | 2963 | 5466 | 5467 |
| 759 | 2964 | 2965 | 5468 | 5469 |
| 760 | 2966 | 2967 | 5470 | 5471 |
| 761 | 2968 | 2969 | 5472 | 5473 |
| 762 | 2970 | 2971 | 5474 | 5475 |
| 763 | 2972 | 2973 | 5476 | 5477 |
| 764 | 2974 | 2975 | 5478 | 5479 |
| 765 | 2976 | 2977 | 5480 | 5481 |
| 766 | 2978 | 2979 | 5482 | 5483 |
| 767 | 2980 | 2981 | 5484 | 5485 |
| 768 | 2982 | 2983 | 5486 | 5487 |
| 769 | 2984 | 2985 | 5488 | 5489 |
| 770 | 2986 | 2987 | 5490 | 5491 |
| 771 | 2990 | 2991 | 5494 | 5495 |
| 772 | 2992 | 2993 | 5496 | 5497 |
| 773 | 2994 | 2995 | 5498 | 5499 |
| 774 | 2996 | 2997 | 5500 | 5501 |
| 775 | 2998 | 2999 | 5502 | 5503 |
| 776 | 3000 | 3001 | 5504 | 5505 |
| 777 | 3002 | 3003 | 5506 | 5507 |
| 778 | 3004 | 3005 | 5508 | 5509 |
| 779 | 3006 | 3007 | 5510 | 5511 |
| 780 | 3008 | 3009 | 5512 | 5513 |
| 781 | 3010 | 3011 | 5514 | 5515 |
| 782 | 3012 | 3013 | 5516 | 5517 |
| 783 | 3014 | 3015 | 5518 | 5519 |
| 784 | 3016 | 3017 | 5520 | 5521 |
| 785 | 3020 | 3021 | 5524 | 5525 |
| 786 | 3022 | 3023 | 5526 | 5527 |
| 787 | 3024 | 3025 | 5528 | 5529 |
| 788 | 3026 | 3027 | 5530 | 5531 |
| 789 | 3028 | 3029 | 5532 | 5533 |
| 790 | 3030 | 3031 | 5534 | 5535 |
| 791 | 3032 | 3033 | 5536 | 5537 |
| 792 | 3034 | 3035 | 5538 | 5539 |
| 793 | 3036 | 3037 | 5540 | 5541 |
| 794 | 3038 | 3039 | 5542 | 5543 |
| 795 | 3040 | 3041 | 5544 | 5545 |
| 796 | 3042 | 3043 | 5546 | 5547 |
| 797 | 3044 | 3045 | 5548 | 5549 |
| 798 | 3046 | 3047 | 5550 | 5551 |
| 799 | 3048 | 3049 | 5552 | 5553 |
| 800 | 3050 | 3051 | 5554 | 5555 |
| 801 | 3052 | 3053 | 5556 | 5557 |
| 802 | 3054 | 3055 | 5558 | 5559 |
| 803 | 3056 | 3057 | 5560 | 5561 |
| 804 | 3058 | 3059 | 5562 | 5563 |
| 805 | 3060 | 3061 | 5564 | 5565 |
| 806 | 3062 | 3063 | 5566 | 5567 |
| 807 | 3064 | 3065 | 5568 | 5569 |
| 808 | 3066 | 3067 | 5570 | 5571 |
| 809 | 3068 | 3069 | 5572 | 5573 |
| 810 | 3070 | 3071 | 5574 | 5575 |
| 811 | 3072 | 3073 | 5576 | 5577 |
| 812 | 3074 | 3075 | 5578 | 5579 |
| 813 | 3076 | 3077 | 5580 | 5581 |
| 814 | 3078 | 3079 | 5582 | 5583 |
| 815 | 3080 | 3081 | 5584 | 5585 |
| 816 | 3082 | 3083 | 5586 | 5587 |
| 817 | 3084 | 3085 | 5588 | 5589 |
| 818 | 3086 | 3087 | 5590 | 5591 |
| 819 | 3088 | 3089 | 5592 | 5593 |
| 820 | 3090 | 3091 | 5594 | 5595 |
| 821 | 3092 | 3093 | 5596 | 5597 |
| 822 | 3094 | 3095 | 5598 | 5599 |
| 823 | 3096 | 3097 | 5600 | 5601 |
| 824 | 3100 | 3101 | 5604 | 5605 |
| 825 | 3102 | 3103 | 5606 | 5607 |
| 826 | 3104 | 3105 | 5608 | 5609 |
| 827 | 3106 | 3107 | 5610 | 5611 |
| 828 | 3108 | 3109 | 5612 | 5613 |
| 829 | 3110 | 3111 | 5614 | 5615 |
| 830 | 3112 | 3113 | 5616 | 5617 |
| 831 | 3114 | 3115 | 5618 | 5619 |
| 832 | 3116 | 3117 | 5620 | 5621 |
| 833 | 3120 | 3121 | 5624 | 5625 |
| 834 | 3124 | 3125 | 5628 | 5629 |
| 835 | 3122 | 3123 | 5626 | 5627 |
| 836 | 3128 | 3129 | 5632 | 5633 |
| 837 | 3130 | 3131 | 5634 | 5635 |
| 838 | 3132 | 3133 | 5636 | 5637 |
| 839 | 3134 | 3135 | 5638 | 5639 |
| 840 | 3136 | 3137 | 5640 | 5641 |
| 841 | 3138 | 3139 | 5642 | 5643 |
| 842 | 3140 | 3141 | 5644 | 5645 |
| 843 | 3142 | 3143 | 5646 | 5647 |
| 844 | 3144 | 3145 | 5648 | 5649 |
| 845 | 3146 | 3147 | 5650 | 5651 |
| 846 | 3148 | 3149 | 5652 | 5653 |
| 847 | 3150 | 3151 | 5654 | 5655 |
| 848 | 3152 | 3153 | 5656 | 5657 |
| 849 | 3154 | 3155 | 5658 | 5659 |
| 850 | 3156 | 3157 | 5660 | 5661 |
| 851 | 3158 | 3159 | 5662 | 5663 |
| 852 | 3160 | 3161 | 5664 | 5665 |
| 853 | 3164 | 3165 | 5668 | 5669 |
| 854 | 3162 | 3163 | 5666 | 5667 |
| 855 | 3166 | 3167 | 5670 | 5671 |
| 856 | 3168 | 3169 | 5672 | 5673 |
| 857 | 3170 | 3171 | 5674 | 5675 |
| 858 | 3172 | 3173 | 5676 | 5677 |
| 859 | 3174 | 3175 | 5678 | 5679 |
| 860 | 3176 | 3177 | 5680 | 5681 |
| 861 | 3180 | 3181 | 5684 | 5685 |
| 862 | 3178 | 3179 | 5682 | 5683 |
| 863 | 3182 | 3183 | 5686 | 5687 |
| 864 | 3184 | 3185 | 5688 | 5689 |
| 865 | 3186 | 3187 | 5690 | 5691 |
| 866 | 3188 | 3189 | 5692 | 5693 |
| 867 | 3190 | 3191 | 5694 | 5695 |
| 868 | 3192 | 3193 | 5696 | 5697 |
| 869 | 3194 | 3195 | 5698 | 5699 |
| 870 | 3196 | 3197 | 5700 | 5701 |
| 871 | 3198 | 3199 | 5702 | 5703 |
| 872 | 3200 | 3201 | 5704 | 5705 |
| 873 | 3202 | 3203 | 5706 | 5707 |
| 874 | 3204 | 3205 | 5708 | 5709 |
| 875 | 3206 | 3207 | 5710 | 5711 |
| 876 | 3210 | 3211 | 5714 | 5715 |
| 877 | 3212 | 3213 | 5716 | 5717 |
| 878 | 3214 | 3215 | 5718 | 5719 |
| 879 | 3216 | 3217 | 5720 | 5721 |
| 880 | 3218 | 3219 | 5722 | 5723 |
| 881 | 3220 | 3221 | 5724 | 5725 |
| 882 | 3222 | 3223 | 5726 | 5727 |
| 883 | 3224 | 3225 | 5728 | 5729 |
| 884 | 3226 | 3227 | 5730 | 5731 |
| 885 | 3228 | 3229 | 5732 | 5733 |
| 886 | 3230 | 3231 | 5734 | 5735 |
| 887 | 3232 | 3233 | 5736 | 5737 |
| 888 | 3234 | 3235 | 5738 | 5739 |
| 889 | 3236 | 3237 | 5740 | 5741 |
| 890 | 3238 | 3239 | 5742 | 5743 |
| 891 | 3240 | 3241 | 5744 | 5745 |
| 892 | 3244 | 3245 | 5748 | 5749 |
| 893 | 3246 | 3247 | 5750 | 5751 |
| 894 | 3248 | 3249 | 5752 | 5753 |
| 895 | 3250 | 3251 | 5754 | 5755 |
| 896 | 3252 | 3253 | 5756 | 5757 |
| 897 | 3254 | 3255 | 5758 | 5759 |
| 898 | 3256 | 3257 | 5760 | 5761 |
| 899 | 3258 | 3259 | 5762 | 5763 |
| 900 | 3260 | 3261 | 5764 | 5765 |
| 901 | 3262 | 3263 | 5766 | 5767 |
| 902 | 3264 | 3265 | 5768 | 5769 |
| 903 | 3266 | 3267 | 5770 | 5771 |
| 904 | 3268 | 3269 | 5772 | 5773 |
| 905 | 3270 | 3271 | 5774 | 5775 |
| 906 | 3272 | 3273 | 5776 | 5777 |
| 907 | 3274 | 3275 | 5778 | 5779 |
| 908 | 3276 | 3277 | 5780 | 5781 |
| 909 | 3278 | 3279 | 5782 | 5783 |
| 910 | 3280 | 3281 | 5784 | 5785 |
| 911 | 3282 | 3283 | 5786 | 5787 |
| 912 | 3284 | 3285 | 5788 | 5789 |
| 913 | 3286 | 3287 | 5790 | 5791 |
| 914 | 3288 | 3289 | 5792 | 5793 |
| 915 | 3290 | 3291 | 5794 | 5795 |
| 916 | 3292 | 3293 | 5796 | 5797 |
| 917 | 3296 | 3297 | 5800 | 5801 |
| 918 | 3298 | 3299 | 5802 | 5803 |
| 919 | 3300 | 3301 | 5804 | 5805 |
| 920 | 3302 | 3303 | 5806 | 5807 |
| 921 | 3304 | 3305 | 5808 | 5809 |
| 922 | 3306 | 3307 | 5810 | 5811 |
| 923 | 3308 | 3309 | 5812 | 5813 |
| 924 | 3310 | 3311 | 5814 | 5815 |
| 925 | 3316 | 3317 | 5820 | 5821 |
| 926 | 3314 | 3315 | 5818 | 5819 |
| 927 | 3324 | 3325 | 5828 | 5829 |
| 928 | 3326 | 3327 | 5830 | 5831 |
| 929 | 3328 | 3329 | 5832 | 5833 |
| 930 | 3330 | 3331 | 5834 | 5835 |
| 931 | 3338 | 3339 | 5842 | 5843 |
| 932 | 3336 | 3337 | 5840 | 5841 |
| 933 | 3340 | 3341 | 5844 | 5845 |
| 934 | 3342 | 3343 | 5846 | 5847 |
| 935 | 3344 | 3345 | 5848 | 5849 |
| 936 | 3346 | 3347 | 5850 | 5851 |
| 937 | 3348 | 3349 | 5852 | 5853 |
| 938 | 3350 | 3351 | 5854 | 5855 |
| 939 | 3352 | 3353 | 5856 | 5857 |
| 940 | 3354 | 3355 | 5858 | 5859 |
| 941 | 3356 | 3357 | 5860 | 5861 |
| 942 | 3360 | 3361 | 5864 | 5865 |
| 943 | 3362 | 3363 | 5866 | 5867 |
| 944 | 3364 | 3365 | 5868 | 5869 |
| 945 | 3366 | 3367 | 5870 | 5871 |
| 946 | 3368 | 3369 | 5872 | 5873 |
| 947 | 3370 | 3371 | 5874 | 5875 |
| 948 | 3374 | 3375 | 5878 | 5879 |
| 949 | 3378 | 3379 | 5882 | 5883 |
| 950 | 3376 | 3377 | 5880 | 5881 |
| 951 | 3380 | 3381 | 5884 | 5885 |
| 952 | 3382 | 3383 | 5886 | 5887 |
| 953 | 3384 | 3385 | 5888 | 5889 |
| 954 | 3386 | 3387 | 5890 | 5891 |
| 955 | 3388 | 3389 | 5892 | 5893 |
| 956 | 3390 | 3391 | 5894 | 5895 |
| 957 | 3392 | 3393 | 5896 | 5897 |
| 958 | 3394 | 3395 | 5898 | 5899 |
| 959 | 3396 | 3397 | 5900 | 5901 |
| 960 | 3398 | 3399 | 5902 | 5903 |
| 961 | 3400 | 3401 | 5904 | 5905 |
| 962 | 3402 | 3403 | 5906 | 5907 |
| 963 | 3404 | 3405 | 5908 | 5909 |
| 964 | 3406 | 3407 | 5910 | 5911 |
| 965 | 3408 | 3409 | 5912 | 5913 |
| 966 | 3410 | 3411 | 5914 | 5915 |
| 967 | 3412 | 3413 | 5916 | 5917 |
| 968 | 3414 | 3415 | 5918 | 5919 |
| 969 | 3416 | 3417 | 5920 | 5921 |
| 970 | 3418 | 3419 | 5922 | 5923 |
| 971 | 3420 | 3421 | 5924 | 5925 |
| 972 | 3422 | 3423 | 5926 | 5927 |
| 973 | 3424 | 3425 | 5928 | 5929 |
| 974 | 3426 | 3427 | 5930 | 5931 |
| 975 | 3428 | 3429 | 5932 | 5933 |
| 976 | 3430 | 3431 | 5934 | 5935 |
| 977 | 3432 | 3433 | 5936 | 5937 |
| 978 | 3434 | 3435 | 5938 | 5939 |
| 979 | 3436 | 3437 | 5940 | 5941 |
| 980 | 3438 | 3439 | 5942 | 5943 |
| 981 | 3440 | 3441 | 5944 | 5945 |
| 982 | 3442 | 3443 | 5946 | 5947 |
| 983 | 3444 | 3445 | 5948 | 5949 |
| 984 | 3446 | 3447 | 5950 | 5951 |
| 985 | 3448 | 3449 | 5952 | 5953 |
| 986 | 3450 | 3451 | 5954 | 5955 |
| 987 | 3454 | 3455 | 5958 | 5959 |
| 988 | 3456 | 3457 | 5960 | 5961 |
| 989 | 3458 | 3459 | 5962 | 5963 |
| 990 | 3460 | 3461 | 5964 | 5965 |
| 991 | 3462 | 3463 | 5966 | 5967 |
| 992 | 3464 | 3465 | 5968 | 5969 |
| 993 | 3466 | 3467 | 5970 | 5971 |
| 994 | 3468 | 3469 | 5972 | 5973 |
| 995 | 3470 | 3471 | 5974 | 5975 |
| 996 | 3472 | 3473 | 5976 | 5977 |
| 997 | 3474 | 3475 | 5978 | 5979 |
| 998 | 3476 | 3477 | 5980 | 5981 |
| 999 | 3478 | 3479 | 5982 | 5983 |
| 1000 | 3480 | 3481 | 5984 | 5985 |
| 1001 | 3482 | 3483 | 5986 | 5987 |
| 1002 | 3484 | 3485 | 5988 | 5989 |
| 1003 | 3486 | 3487 | 5990 | 5991 |
| 1004 | 3488 | 3489 | 5992 | 5993 |
| 1005 | 3490 | 3491 | 5994 | 5995 |
| 1006 | 3494 | 3495 | 5998 | 5999 |
| 1007 | 3496 | 3497 | 6000 | 6001 |
| 1008 | 3498 | 3499 | 6002 | 6003 |
| 1009 | 3500 | 3501 | 6004 | 6005 |
| 1010 | 3502 | 3503 | 6006 | 6007 |
| 1011 | 3504 | 3505 | 6008 | 6009 |
| 1012 | 3506 | 3507 | 6010 | 6011 |
| 1013 | 3508 | 3509 | 6012 | 6013 |
| 1014 | 3510 | 3511 | 6014 | 6015 |
| 1015 | 3512 | 3513 | 6016 | 6017 |
| 1016 | 3516 | 3517 | 6020 | 6021 |
| 1017 | 3518 | 3519 | 6022 | 6023 |
| 1018 | 3520 | 3521 | 6024 | 6025 |
| 1019 | 3522 | 3523 | 6026 | 6027 |
| 1020 | 3524 | 3525 | 6028 | 6029 |
| 1021 | 3526 | 3527 | 6030 | 6031 |
| 1022 | 3528 | 3529 | 6032 | 6033 |
| 1023 | 3530 | 3531 | 6034 | 6035 |
| 1024 | 3532 | 3533 | 6036 | 6037 |
| 1025 | 3534 | 3535 | 6038 | 6039 |
| 1026 | 3536 | 3537 | 6040 | 6041 |
| 1027 | 3542 | 3543 | 6046 | 6047 |
| 1028 | 3544 | 3545 | 6048 | 6049 |
| 1029 | 3546 | 3547 | 6050 | 6051 |
| 1030 | 3548 | 3549 | 6052 | 6053 |
| 1031 | 3550 | 3551 | 6054 | 6055 |
| 1032 | 3552 | 3553 | 6056 | 6057 |
| 1033 | 3554 | 3555 | 6058 | 6059 |
| 1034 | 3556 | 3557 | 6060 | 6061 |
| 1035 | 3558 | 3559 | 6062 | 6063 |
| 1036 | 3560 | 3561 | 6064 | 6065 |
| 1037 | 3562 | 3563 | 6066 | 6067 |
| 1038 | 3564 | 3565 | 6068 | 6069 |
| 1039 | 3566 | 3567 | 6070 | 6071 |
| 1040 | 3568 | 3569 | 6072 | 6073 |
| 1041 | 3570 | 3571 | 6074 | 6075 |
| 1042 | 3572 | 3573 | 6076 | 6077 |
| 1043 | 3574 | 3575 | 6078 | 6079 |
| 1044 | 3582 | 3583 | 6086 | 6087 |
| 1045 | 3584 | 3585 | 6088 | 6089 |
| 1046 | 3586 | 3587 | 6090 | 6091 |
| 1047 | 3588 | 3589 | 6092 | 6093 |
| 1048 | 3592 | 3593 | 6096 | 6097 |
| 1049 | 3594 | 3595 | 6098 | 6099 |
| 1050 | 3596 | 3597 | 6100 | 6101 |
| 1051 | 3598 | 3599 | 6102 | 6103 |
| 1052 | 3600 | 3601 | 6104 | 6105 |
| 1053 | 3602 | 3603 | 6106 | 6107 |
| 1054 | 3604 | 3605 | 6108 | 6109 |
| 1055 | 3606 | 3607 | 6110 | 6111 |
| 1056 | 3608 | 3609 | 6112 | 6113 |
| 1057 | 3610 | 3611 | 6114 | 6115 |
| 1058 | 3612 | 3613 | 6116 | 6117 |
| 1059 | 3614 | 3615 | 6118 | 6119 |
| 1060 | 3616 | 3617 | 6120 | 6121 |
| 1061 | 3618 | 3619 | 6122 | 6123 |
| 1062 | 3620 | 3621 | 6124 | 6125 |
| 1063 | 3622 | 3623 | 6126 | 6127 |
| 1064 | 3624 | 3625 | 6128 | 6129 |
| 1065 | 3626 | 3627 | 6130 | 6131 |
| 1066 | 3628 | 3629 | 6132 | 6133 |
| 1067 | 3630 | 3631 | 6134 | 6135 |
| 1068 | 3632 | 3633 | 6136 | 6137 |
| 1069 | 3634 | 3635 | 6138 | 6139 |
| 1070 | 3636 | 3637 | 6140 | 6141 |
| 1071 | 3638 | 3639 | 6142 | 6143 |
| 1072 | 3640 | 3641 | 6144 | 6145 |
| 1073 | 3642 | 3643 | 6146 | 6147 |
| 1074 | 3644 | 3645 | 6148 | 6149 |
| 1075 | 3646 | 3647 | 6150 | 6151 |
| 1076 | 3648 | 3649 | 6152 | 6153 |
| 1077 | 3652 | 3653 | 6156 | 6157 |
| 1078 | 3654 | 3655 | 6158 | 6159 |
| 1079 | 3656 | 3657 | 6160 | 6161 |
| 1080 | 3658 | 3659 | 6162 | 6163 |
| 1081 | 3660 | 3661 | 6164 | 6165 |
| 1082 | 3662 | 3663 | 6166 | 6167 |
| 1083 | 3666 | 3667 | 6170 | 6171 |
| 1084 | 3668 | 3669 | 6172 | 6173 |
| 1085 | 3672 | 3673 | 6176 | 6177 |
| 1086 | 3674 | 3675 | 6178 | 6179 |
| 1087 | 3676 | 3677 | 6180 | 6181 |
| 1088 | 3678 | 3679 | 6182 | 6183 |
| 1089 | 3680 | 3681 | 6184 | 6185 |
| 1090 | 3682 | 3683 | 6186 | 6187 |
| 1091 | 3684 | 3685 | 6188 | 6189 |
| 1092 | 3686 | 3687 | 6190 | 6191 |
| 1093 | 3688 | 3689 | 6192 | 6193 |
| 1094 | 3690 | 3691 | 6194 | 6195 |
| 1095 | 3692 | 3693 | 6196 | 6197 |
| 1096 | 3694 | 3695 | 6198 | 6199 |
| 1097 | 3696 | 3697 | 6200 | 6201 |
| 1098 | 3698 | 3699 | 6202 | 6203 |
| 1099 | 3702 | 3703 | 6206 | 6207 |
| 1100 | 3700 | 3701 | 6204 | 6205 |
| 1101 | 3704 | 3705 | 6208 | 6209 |
| 1102 | 3706 | 3707 | 6210 | 6211 |
| 1103 | 3708 | 3709 | 6212 | 6213 |
| 1104 | 3714 | 3715 | 6218 | 6219 |
| 1105 | 3720 | 3721 | 6224 | 6225 |
| 1106 | 3722 | 3723 | 6226 | 6227 |
| 1107 | 3724 | 3725 | 6228 | 6229 |
| 1108 | 3726 | 3727 | 6230 | 6231 |
| 1109 | 3728 | 3729 | 6232 | 6233 |
| 1110 | 3730 | 3731 | 6234 | 6235 |
| 1111 | 3732 | 3733 | 6236 | 6237 |
| 1112 | 3734 | 3735 | 6238 | 6239 |
| 1113 | 3736 | 3737 | 6240 | 6241 |
| 1114 | 3740 | 3741 | 6244 | 6245 |
| 1115 | 3738 | 3739 | 6242 | 6243 |
| 1116 | 3742 | 3743 | 6246 | 6247 |
| 1117 | 3744 | 3745 | 6248 | 6249 |
| 1118 | 3746 | 3747 | 6250 | 6251 |
| 1119 | 3748 | 3749 | 6252 | 6253 |
| 1120 | 3750 | 3751 | 6254 | 6255 |
| 1121 | 3754 | 3755 | 6258 | 6259 |
| 1122 | 3756 | 3757 | 6260 | 6261 |
| 1123 | 3758 | 3759 | 6262 | 6263 |
| 1124 | 3760 | 3761 | 6264 | 6265 |
| 1125 | 3762 | 3763 | 6266 | 6267 |
| 1126 | 3766 | 3767 | 6270 | 6271 |
| 1127 | 3770 | 3771 | 6274 | 6275 |
| 1128 | 3772 | 3773 | 6276 | 6277 |
| 1129 | 3776 | 3777 | 6280 | 6281 |
| 1130 | 3774 | 3775 | 6278 | 6279 |
| 1131 | 3778 | 3779 | 6282 | 6283 |
| 1132 | 3780 | 3781 | 6284 | 6285 |
| 1133 | 3782 | 3783 | 6286 | 6287 |
| 1134 | 3784 | 3785 | 6288 | 6289 |
| 1135 | 3788 | 3789 | 6292 | 6293 |
| 1136 | 3786 | 3787 | 6290 | 6291 |
| 1137 | 3794 | 3795 | 6298 | 6299 |
| 1138 | 1372 | 1373 | 3876 | 3877 |
| 1139 | 1378 | 1379 | 3882 | 3883 |
| 1140 | 1384 | 1385 | 3888 | 3889 |
| 1141 | 1390 | 1391 | 3894 | 3895 |
| 1142 | 1408 | 1409 | 3912 | 3913 |
| 1143 | 1436 | 1437 | 3940 | 3941 |
| 1144 | 1442 | 1443 | 3946 | 3947 |
| 1145 | 1466 | 1467 | 3970 | 3971 |
| 1146 | 1474 | 1475 | 3978 | 3979 |
| 1147 | 1528 | 1529 | 4032 | 4033 |
| 1148 | 1560 | 1561 | 4064 | 4065 |
| 1149 | 1578 | 1579 | 4082 | 4083 |
| 1150 | 1600 | 1601 | 4104 | 4105 |
| 1151 | 1602 | 1603 | 4106 | 4107 |
| 1152 | 1608 | 1609 | 4112 | 4113 |
| 1153 | 1610 | 1611 | 4114 | 4115 |
| 1154 | 1654 | 1655 | 4158 | 4159 |
| 1155 | 1660 | 1661 | 4164 | 4165 |
| 1156 | 1680 | 1681 | 4184 | 4185 |
| 1157 | 1682 | 1683 | 4186 | 4187 |
| 1158 | 1692 | 1693 | 4196 | 4197 |
| 1159 | 1702 | 1703 | 4206 | 4207 |
| 1160 | 1706 | 1707 | 4210 | 4211 |
| 1161 | 1716 | 1717 | 4220 | 4221 |
| 1162 | 1718 | 1719 | 4222 | 4223 |
| 1163 | 1730 | 1731 | 4234 | 4235 |
| 1164 | 1748 | 1749 | 4252 | 4253 |
| 1165 | 1780 | 1781 | 4284 | 4285 |
| 1166 | 1794 | 1795 | 4298 | 4299 |
| 1167 | 1798 | 1799 | 4302 | 4303 |
| 1168 | 1814 | 1815 | 4318 | 4319 |
| 1169 | 1816 | 1817 | 4320 | 4321 |
| 1170 | 1820 | 1821 | 4324 | 4325 |
| 1171 | 1822 | 1823 | 4326 | 4327 |
| 1172 | 1830 | 1831 | 4334 | 4335 |
| 1173 | 1832 | 1833 | 4336 | 4337 |
| 1174 | 1838 | 1839 | 4342 | 4343 |
| 1175 | 1844 | 1845 | 4348 | 4349 |
| 1176 | 1870 | 1871 | 4374 | 4375 |
| 1177 | 1880 | 1881 | 4384 | 4385 |
| 1178 | 1882 | 1883 | 4386 | 4387 |
| 1179 | 1884 | 1885 | 4388 | 4389 |
| 1180 | 1932 | 1933 | 4436 | 4437 |
| 1181 | 1968 | 1969 | 4472 | 4473 |
| 1182 | 1982 | 1983 | 4486 | 4487 |
| 1183 | 2036 | 2037 | 4540 | 4541 |
| 1184 | 2086 | 2087 | 4590 | 4591 |
| 1185 | 2098 | 2099 | 4602 | 4603 |
| 1186 | 2126 | 2127 | 4630 | 4631 |
| 1187 | 2132 | 2133 | 4636 | 4637 |
| 1188 | 2166 | 2167 | 4670 | 4671 |
| 1189 | 2168 | 2169 | 4672 | 4673 |
| 1190 | 2184 | 2185 | 4688 | 4689 |
| 1191 | 2240 | 2241 | 4744 | 4745 |
| 1192 | 2276 | 2277 | 4780 | 4781 |
| 1193 | 2300 | 2301 | 4804 | 4805 |
| 1194 | 2302 | 2303 | 4806 | 4807 |
| 1195 | 2330 | 2331 | 4834 | 4835 |
| 1196 | 2336 | 2337 | 4840 | 4841 |
| 1197 | 2448 | 2449 | 4952 | 4953 |
| 1198 | 2452 | 2453 | 4956 | 4957 |
| 1199 | 2484 | 2485 | 4988 | 4989 |
| 1200 | 2512 | 2513 | 5016 | 5017 |
| 1201 | 2530 | 2531 | 5034 | 5035 |
| 1202 | 2540 | 2541 | 5044 | 5045 |
| 1203 | 2542 | 2543 | 5046 | 5047 |
| 1204 | 2584 | 2585 | 5088 | 5089 |
| 1205 | 2586 | 2587 | 5090 | 5091 |
| 1206 | 2588 | 2589 | 5092 | 5093 |
| 1207 | 2614 | 2615 | 5118 | 5119 |
| 1208 | 2670 | 2671 | 5174 | 5175 |
| 1209 | 2694 | 2695 | 5198 | 5199 |
| 1210 | 2708 | 2709 | 5212 | 5213 |
| 1211 | 2730 | 2731 | 5234 | 5235 |
| 1212 | 2734 | 2735 | 5238 | 5239 |
| 1213 | 2746 | 2747 | 5250 | 5251 |
| 1214 | 2802 | 2803 | 5306 | 5307 |
| 1215 | 2898 | 2899 | 5402 | 5403 |
| 1216 | 2950 | 2951 | 5454 | 5455 |
| 1217 | 2988 | 2989 | 5492 | 5493 |
| 1218 | 3018 | 3019 | 5522 | 5523 |
| 1219 | 3098 | 3099 | 5602 | 5603 |
| 1220 | 3118 | 3119 | 5622 | 5623 |
| 1221 | 3126 | 3127 | 5630 | 5631 |
| 1222 | 3208 | 3209 | 5712 | 5713 |
| 1223 | 3242 | 3243 | 5746 | 5747 |
| 1224 | 3294 | 3295 | 5798 | 5799 |
| 1225 | 3312 | 3313 | 5816 | 5817 |
| 1226 | 3318 | 3319 | 5822 | 5823 |
| 1227 | 3320 | 3321 | 5824 | 5825 |
| 1228 | 3322 | 3323 | 5826 | 5827 |
| 1229 | 3332 | 3333 | 5836 | 5837 |
| 1230 | 3334 | 3335 | 5838 | 5839 |
| 1231 | 3358 | 3359 | 5862 | 5863 |
| 1232 | 3372 | 3373 | 5876 | 5877 |
| 1233 | 3452 | 3453 | 5956 | 5957 |
| 1234 | 3492 | 3493 | 5996 | 5997 |
| 1235 | 3514 | 3515 | 6018 | 6019 |
| 1236 | 3538 | 3539 | 6042 | 6043 |
| 1237 | 3540 | 3541 | 6044 | 6045 |
| 1238 | 3576 | 3577 | 6080 | 6081 |
| 1239 | 3578 | 3579 | 6082 | 6083 |
| 1240 | 3580 | 3581 | 6084 | 6085 |
| 1241 | 3590 | 3591 | 6094 | 6095 |
| 1242 | 3650 | 3651 | 6154 | 6155 |
| 1243 | 3664 | 3665 | 6168 | 6169 |
| 1244 | 3670 | 3671 | 6174 | 6175 |
| 1245 | 3710 | 3711 | 6214 | 6215 |
| 1246 | 3712 | 3713 | 6216 | 6217 |
| 1247 | 3716 | 3717 | 6220 | 6221 |
| 1248 | 3718 | 3719 | 6222 | 6223 |
| 1249 | 3752 | 3753 | 6256 | 6257 |
| 1250 | 3764 | 3765 | 6268 | 6269 |
| 1251 | 3768 | 3769 | 6272 | 6273 |
| 1252 | 3790 | 3791 | 6294 | 6295 |
| 1253 | 3792 | 3793 | 6296 | 6297 |
| 1254 | 6300 | 6301 | 6376 | 6377 |
| 1255 | 6302 | 6303 | 6378 | 6379 |
| 1256 | 6304 | 6305 | 6380 | 6381 |
| 1257 | 6306 | 6307 | 6382 | 6383 |
| 1258 | 6308 | 6309 | 6384 | 6385 |
| 1259 | 6310 | 6311 | 6386 | 6387 |
| 1260 | 6312 | 6313 | 6388 | 6389 |
| 1261 | 6314 | 6315 | 6390 | 6391 |
| 1262 | 6316 | 6317 | 6392 | 6393 |
| 1263 | 6318 | 6319 | 6394 | 6395 |
| 1264 | 6320 | 6321 | 6396 | 6397 |
| 1265 | 6322 | 6323 | 6398 | 6399 |
| 1266 | 6324 | 6325 | 6400 | 6401 |
| 1267 | 6326 | 6327 | 6402 | 6403 |
| 1268 | 6328 | 6329 | 6404 | 6405 |
| 1269 | 6330 | 6331 | 6406 | 6407 |
| 1270 | 6332 | 6333 | 6408 | 6409 |
| 1271 | 6334 | 6335 | 6410 | 6411 |
| 1272 | 6336 | 6337 | 6412 | 6413 |
| 1273 | 6338 | 6339 | 6414 | 6415 |
| 1274 | 6340 | 6341 | 6416 | 6417 |
| 1275 | 6342 | 6343 | 6418 | 6419 |
| 1276 | 6344 | 6345 | 6420 | 6421 |
| 1277 | 6346 | 6347 | 6422 | 6423 |
| 1278 | 6348 | 6349 | 6424 | 6425 |
| 1279 | 6350 | 6351 | 6426 | 6427 |
| 1280 | 6352 | 6353 | 6428 | 6429 |
| 1281 | 6354 | 6355 | 6430 | 6431 |
| 1282 | 6356 | 6357 | 6432 | 6433 |
| 1283 | 6358 | 6359 | 6434 | 6435 |
| 1284 | 6360 | 6361 | 6436 | 6437 |
| 1285 | 6362 | 6363 | 6438 | 6439 |
| 1286 | 6364 | 6365 | 6440 | 6441 |
| 1287 | 6366 | 6367 | 6442 | 6443 |
| 1288 | 6368 | 6369 | 6444 | 6445 |
| 1289 | 6370 | 6371 | 6446 | 6447 |
| 1290 | 6372 | 6373 | 6448 | 6449 |
| 1291 | 6374 | 6375 | 6450 | 6451 |

**TABLE 5**

| *No* | *or.* | *5'position* | | *No* | *or.* | *5' position* | | *No* | *or.* | *5'position* |
|---|---|---|---|---|---|---|---|---|---|---|
| 1292 | F | 1229848 | | 3012 | F | 833844 | | 4732 | B | 455875 |
| 1293 | F | 1227874 | | 3013 | F | 831936 | | 4733 | B | 457736 |
| 1294 | F | 1018 | | 3014 | F | 834905 | | 4734 | B | 457231 |
| 1295 | F | 1229162 | | 3015 | F | 832943 | | 4735 | B | 459146 |
| 1296 | F | 1588 | | 3016 | F | 835834 | | 4736 | B | 458008 |
| 1297 | F | 1229711 | | 3017 | F | 833938 | | 4737 | B | 459836 |
| 1298 | F | 2253 | | 3018 | F | 837457 | | 4738 | B | 458598 |
| 1299 | F | 369 | | 3019 | F | 835536 | | 4739 | B | 460488 |
| 1300 | F | 3381 | | 3020 | F | 838723 | | 4740 | B | 459717 |
| 1301 | F | 1508 | | 3021 | F | 836826 | | 4741 | B | 461652 |
| 1302 | F | 4042 | | 3022 | F | 840649 | | 4742 | B | 460417 |
| 1303 | F | 2126 | | 3023 | F | 838723 | | 4743 | B | 462365 |
| 1304 | F | 5735 | | 3024 | F | 841751 | | 4744 | B | 461391 |
| 1305 | F | 3843 | | 3025 | F | 839825 | | 4745 | B | 463286 |
| 1306 | F | 7832 | | 3026 | F | 842960 | | 4746 | B | 461680 |
| 1307 | F | 5909 | | 3027 | F | 841123 | | 4747 | B | 463584 |
| 1308 | F | 8887 | | 3028 | F | 843765 | | 4748 | B | 462520 |
| 1309 | F | 7010 | | 3029 | F | 841844 | | 4749 | B | 464418 |
| 1310 | F | 10139 | | 3030 | F | 844768 | | 4750 | B | 463584 |
| 1311 | F | 8175 | | 3031 | F | 842852 | | 4751 | B | 465539 |
| 1312 | F | 10640 | | 3032 | F | 846089 | | 4752 | B | 464547 |
| 1313 | F | 8799 | | 3033 | F | 844175 | | 4753 | B | 466398 |
| 1314 | F | 10997 | | 3034 | F | 848293 | | 4754 | B | 465288 |
| 1315 | F | 9037 | | 3035 | F | 846449 | | 4755 | B | 467243 |
| 1316 | F | 12458 | | 3036 | F | 848867 | | 4756 | B | 465835 |
| 1317 | F | 10572 | | 3037 | F | 846964 | | 4757 | B | 467738 |
| 1318 | F | 14187 | | 3038 | F | 850351 | | 4758 | B | 466558 |
| 1319 | F | 12365 | | 3039 | F | 848426 | | 4759 | B | 468474 |
| 1320 | F | 15529 | | 3040 | F | 851788 | | 4760 | B | 467322 |
| 1321 | F | 13629 | | 3041 | F | 849899 | | 4761 | B | 469217 |
| 1322 | F | 17626 | | 3042 | F | 852166 | | 4762 | B | 467738 |
| 1323 | F | 15699 | | 3043 | F | 850278 | | 4763 | B | 469637 |
| 1324 | F | 20909 | | 3044 | F | 853976 | | 4764 | B | 469912 |
| 1325 | F | 19006 | | 3045 | F | 852069 | | 4765 | B | 471814 |
| 1326 | F | 21800 | | 3046 | F | 854899 | | 4766 | B | 470920 |
| 1327 | F | 19927 | | 3047 | F | 853006 | | 4767 | B | 472826 |
| 1328 | F | 23462 | | 3048 | F | 855595 | | 4768 | B | 472075 |
| 1329 | F | 21557 | | 3049 | F | 853679 | | 4769 | B | 473922 |
| 1330 | F | 25637 | | 3050 | F | 856479 | | 4770 | B | 472231 |
| 1331 | F | 23729 | | 3051 | F | 854582 | | 4771 | B | 474144 |
| 1332 | F | 25997 | | 3052 | F | 858498 | | 4772 | B | 472579 |
| 1333 | F | 24071 | | 3053 | F | 856492 | | 4773 | B | 474501 |
| 1334 | F | 26727 | | 3054 | F | 859372 | | 4774 | B | 473751 |
| 1335 | F | 24828 | | 3055 | F | 857424 | | 4775 | B | 475664 |
| 1336 | F | 27528 | | 3056 | F | 860050 | | 4776 | B | 475116 |
| 1337 | F | 25628 | | 3057 | F | 858116 | | 4777 | B | 477009 |
| 1338 | F | 28643 | | 3058 | F | 860941 | | 4778 | B | 477566 |
| 1339 | F | 26765 | | 3059 | F | 859023 | | 4779 | B | 479490 |
| 1340 | F | 29202 | | 3060 | F | 861464 | | 4780 | B | 477851 |
| 1341 | F | 27313 | | 3061 | F | 859572 | | 4781 | B | 479753 |
| 1342 | F | 29793 | | 3062 | F | 862749 | | 4782 | B | 478728 |
| 1343 | F | 27835 | | 3063 | F | 860895 | | 4783 | B | 480616 |
| 1344 | F | 31488 | | 3064 | F | 864599 | | 4784 | B | 479496 |
| 1345 | F | 29639 | | 3065 | F | 862683 | | 4785 | B | 481418 |
| 1346 | F | 31957 | | 3066 | F | 865003 | | 4786 | B | 479928 |
| 1347 | F | 30050 | | 3067 | F | 863040 | | 4787 | B | 481844 |
| 1348 | F | 33570 | | 3068 | F | 866331 | | 4788 | B | 481674 |
| 1349 | F | 31666 | | 3069 | F | 864443 | | 4789 | B | 483578 |
| 1350 | F | 34564 | | 3070 | F | 866799 | | 4790 | B | 482281 |
| 1351 | F | 32664 | | 3071 | F | 864889 | | 4791 | B | 484243 |
| 1352 | F | 35783 | | 3072 | F | 867574 | | 4792 | B | 482820 |
| 1353 | F | 33875 | | 3073 | F | 865664 | | 4793 | B | 484721 |
| 1354 | F | 37597 | | 3074 | F | 868402 | | 4794 | B | 484449 |
| 1355 | F | 35741 | | 3075 | F | 866513 | | 4795 | B | 486360 |
| 1356 | F | 39135 | | 3076 | F | 869823 | | 4796 | B | 485499 |
| 1357 | F | 37236 | | 3077 | F | 867898 | | 4797 | B | 487293 |
| 1358 | F | 38939 | | 3078 | F | 870414 | | 4798 | B | 486116 |
| 1359 | F | 37038 | | 3079 | F | 868478 | | 4799 | B | 487980 |
| 1360 | F | 40872 | | 3080 | F | 871862 | | 4800 | B | 486811 |
| 1361 | F | 38972 | | 3081 | F | 869956 | | 4801 | B | 488721 |
| 1362 | F | 42825 | | 3082 | F | 872261 | | 4802 | B | 487217 |
| 1363 | F | 40923 | | 3083 | F | 870367 | | 4803 | B | 489101 |
| 1364 | F | 43563 | | 3084 | F | 874062 | | 4804 | B | 487567 |
| 1365 | F | 41652 | | 3085 | F | 872141 | | 4805 | B | 489423 |
| 1366 | F | 44531 | | 3086 | F | 874363 | | 4806 | B | 487984 |
| 1367 | F | 42623 | | 3087 | F | 872439 | | 4807 | B | 489909 |
| 1368 | F | 45150 | | 3088 | F | 875155 | | 4808 | B | 489291 |
| 1369 | F | 43250 | | 3089 | F | 873244 | | 4809 | B | 491191 |
| 1370 | F | 45478 | | 3090 | F | 878156 | | 4810 | B | 489561 |
| 1371 | F | 43579 | | 3091 | F | 876291 | | 4811 | B | 491461 |
| 1372 | F | 46755 | | 3092 | F | 879046 | | 4812 | B | 490221 |
| 1373 | F | 44874 | | 3093 | F | 877133 | | 4813 | B | 492078 |
| 1374 | F | 47347 | | 3094 | F | 880361 | | 4814 | B | 490773 |
| 1375 | F | 45386 | | 3095 | F | 878450 | | 4815 | B | 492672 |
| 1376 | F | 47818 | | 3096 | F | 882361 | | 4816 | B | 491383 |
| 1377 | F | 45897 | | 3097 | F | 880493 | | 4817 | B | 493293 |
| 1378 | F | 48893 | | 3098 | F | 883067 | | 4818 | B | 491616 |
| 1379 | F | 46995 | | 3099 | F | 881185 | | 4819 | B | 493537 |
| 1380 | F | 49907 | | 3100 | F | 883310 | | 4820 | B | 492362 |
| 1381 | F | 48000 | | 3101 | F | 881416 | | 4821 | B | 494246 |
| 1382 | F | 51088 | | 3102 | F | 884035 | | 4822 | B | 495083 |
| 1383 | F | 49169 | | 3103 | F | 882152 | | 4823 | B | 497027 |
| 1384 | F | 52651 | | 3104 | F | 885495 | | 4824 | B | 496168 |
| 1385 | F | 50721 | | 3105 | F | 883599 | | 4825 | B | 498063 |
| 1386 | F | 53065 | | 3106 | F | 887340 | | 4826 | B | 496789 |
| 1387 | F | 51176 | | 3107 | F | 885448 | | 4827 | B | 498688 |
| 1388 | F | 53516 | | 3108 | F | 887996 | | 4828 | B | 497500 |
| 1389 | F | 51611 | | 3109 | F | 886093 | | 4829 | B | 499390 |
| 1390 | F | 54242 | | 3110 | F | 888494 | | 4830 | B | 498057 |
| 1391 | F | 52351 | | 3111 | F | 886570 | | 4831 | B | 499966 |
| 1392 | F | 55058 | | 3112 | F | 889100 | | 4832 | B | 498552 |
| 1393 | F | 53159 | | 3113 | F | 887201 | | 4833 | B | 500508 |
| 1394 | F | 56274 | | 3114 | F | 889655 | | 4834 | B | 499240 |
| 1395 | F | 54348 | | 3115 | F | 887776 | | 4835 | B | 501145 |
| 1396 | F | 57078 | | 3116 | F | 891025 | | 4836 | B | 499812 |
| 1397 | F | 55156 | | 3117 | F | 889105 | | 4837 | B | 501762 |
| 1398 | F | 58343 | | 3118 | F | 891504 | | 4838 | B | 500020 |
| 1399 | F | 56392 | | 3119 | F | 889593 | | 4839 | B | 501915 |
| 1400 | F | 61103 | | 3120 | F | 891795 | | 4840 | B | 500716 |
| 1401 | F | 59177 | | 3121 | F | 889841 | | 4841 | B | 502628 |
| 1402 | F | 59701 | | 3122 | F | 892279 | | 4842 | B | 504395 |
| 1403 | F | 57802 | | 3123 | F | 890400 | | 4843 | B | 506292 |
| 1404 | F | 61887 | | 3124 | F | 892182 | | 4844 | B | 504885 |
| 1405 | F | 59971 | | 3125 | F | 890288 | | 4845 | B | 506772 |
| 1406 | F | 62255 | | 3126 | F | 893010 | | 4846 | B | 507107 |
| 1407 | F | 60348 | | 3127 | F | 891139 | | 4847 | B | 509003 |
| 1408 | F | 63515 | | 3128 | F | 893101 | | 4848 | B | 507933 |
| 1409 | F | 61557 | | 3129 | F | 891211 | | 4849 | B | 509795 |
| 1410 | F | 63657 | | 3130 | F | 895494 | | 4850 | B | 510741 |
| 1411 | F | 61761 | | 3131 | F | 893599 | | 4851 | B | 512656 |
| 1412 | F | 64088 | | 3132 | F | 896448 | | 4852 | B | 508573 |
| 1413 | F | 62196 | | 3133 | F | 894511 | | 4853 | B | 510445 |
| 1414 | F | 64422 | | 3134 | F | 897341 | | 4854 | B | 513663 |
| 1415 | F | 62537 | | 3135 | F | 895442 | | 4855 | B | 515585 |
| 1416 | F | 65072 | | 3136 | F | 899197 | | 4856 | B | 515276 |
| 1417 | F | 63140 | | 3137 | F | 897279 | | 4857 | B | 517040 |
| 1418 | F | 65978 | | 3138 | F | 899999 | | 4858 | B | 517602 |
| 1419 | F | 64088 | | 3139 | F | 898075 | | 4859 | B | 519510 |
| 1420 | F | 67046 | | 3140 | F | 903008 | | 4860 | B | 517602 |
| 1421 | F | 65146 | | 3141 | F | 901103 | | 4861 | B | 519510 |
| 1422 | F | 67466 | | 3142 | F | 904798 | | 4862 | B | 518075 |
| 1423 | F | 65580 | | 3143 | F | 902923 | | 4863 | B | 519947 |
| 1424 | F | 68569 | | 3144 | F | 906993 | | 4864 | B | 518429 |
| 1425 | F | 66686 | | 3145 | F | 905129 | | 4865 | B | 520326 |
| 1426 | F | 68609 | | 3146 | F | 907564 | | 4866 | B | 521416 |
| 1427 | F | 66688 | | 3147 | F | 905665 | | 4867 | B | 523319 |
| 1428 | F | 70423 | | 3148 | F | 907913 | | 4868 | B | 523196 |
| 1429 | F | 68479 | | 3149 | F | 905998 | | 4869 | B | 525096 |
| 1430 | F | 71099 | | 3150 | F | 908349 | | 4870 | B | 525033 |
| 1431 | F | 69206 | | 3151 | F | 906425 | | 4871 | B | 526939 |
| 1432 | F | 71829 | | 3152 | F | 909186 | | 4872 | B | 524599 |
| 1433 | F | 69935 | | 3153 | F | 907286 | | 4873 | B | 526501 |
| 1434 | F | 73745 | | 3154 | F | 911413 | | 4874 | B | 526494 |
| 1435 | F | 71931 | | 3155 | F | 909481 | | 4875 | B | 528361 |
| 1436 | F | 76942 | | 3156 | F | 912084 | | 4876 | B | 527330 |
| 1437 | F | 75022 | | 3157 | F | 910176 | | 4877 | B | 529238 |
| 1438 | F | 77404 | | 3158 | F | 912718 | | 4878 | B | 527167 |
| 1439 | F | 75556 | | 3159 | F | 910814 | | 4879 | B | 529067 |
| 1440 | F | 78133 | | 3160 | F | 913813 | | 4880 | B | 528673 |
| 1441 | F | 76192 | | 3161 | F | 911941 | | 4881 | B | 530573 |
| 1442 | F | 79079 | | 3162 | F | 915106 | | 4882 | B | 529456 |
| 1443 | F | 77122 | | 3163 | F | 913211 | | 4883 | B | 531376 |
| 1444 | F | 79471 | | 3164 | F | 915053 | | 4884 | B | 530864 |
| 1445 | F | 77481 | | 3165 | F | 913141 | | 4885 | B | 532745 |
| 1446 | F | 79670 | | 3166 | F | 916630 | | 4886 | B | 531906 |
| 1447 | F | 77816 | | 3167 | F | 914731 | | 4887 | B | 533776 |
| 1448 | F | 80236 | | 3168 | F | 917500 | | 4888 | B | 534199 |
| 1449 | F | 78356 | | 3169 | F | 915594 | | 4889 | B | 536103 |
| 1450 | F | 81108 | | 3170 | F | 918615 | | 4890 | B | 536674 |
| 1451 | F | 79182 | | 3171 | F | 916715 | | 4891 | B | 538552 |
| 1452 | F | 83024 | | 3172 | F | 919639 | | 4892 | B | 537422 |
| 1453 | F | 81158 | | 3173 | F | 917732 | | 4893 | B | 539270 |
| 1454 | F | 83786 | | 3174 | F | 920216 | | 4894 | B | 538165 |
| 1455 | F | 81886 | | 3175 | F | 918312 | | 4895 | B | 540048 |
| 1456 | F | 84739 | | 3176 | F | 920971 | | 4896 | B | 538658 |
| 1457 | F | 82821 | | 3177 | F | 919057 | | 4897 | B | 540578 |
| 1458 | F | 84866 | | 3178 | F | 921889 | | 4898 | B | 538970 |
| 1459 | F | 82967 | | 3179 | F | 920015 | | 4899 | B | 540857 |
| 1460 | F | 85175 | | 3180 | F | 921773 | | 4900 | B | 539859 |
| 1461 | F | 83240 | | 3181 | F | 919871 | | 4901 | B | 541736 |
| 1462 | F | 85690 | | 3182 | F | 923428 | | 4902 | B | 541474 |
| 1463 | F | 83790 | | 3183 | F | 921546 | | 4903 | B | 543411 |
| 1464 | F | 86397 | | 3184 | F | 923841 | | 4904 | B | 542791 |
| 1465 | F | 84507 | | 3185 | F | 921936 | | 4905 | B | 544691 |
| 1466 | F | 88470 | | 3186 | F | 924795 | | 4906 | B | 543234 |
| 1467 | F | 86563 | | 3187 | F | 922945 | | 4907 | B | 545134 |
| 1468 | F | 89038 | | 3188 | F | 925102 | | 4908 | B | 543608 |
| 1469 | F | 87121 | | 3189 | F | 923188 | | 4909 | B | 545513 |
| 1470 | F | 91017 | | 3190 | F | 926130 | | 4910 | B | 546851 |
| 1471 | F | 89146 | | 3191 | F | 924248 | | 4911 | B | 548762 |
| 1472 | F | 93075 | | 3192 | F | 927729 | | 4912 | B | 549793 |
| 1473 | F | 91147 | | 3193 | F | 925829 | | 4913 | B | 551652 |
| 1474 | F | 93846 | | 3194 | F | 928112 | | 4914 | B | 547523 |
| 1475 | F | 91948 | | 3195 | F | 926130 | | 4915 | B | 549430 |
| 1476 | F | 94410 | | 3196 | F | 929014 | | 4916 | B | 550754 |
| 1477 | F | 92561 | | 3197 | F | 927129 | | 4917 | B | 552702 |
| 1478 | F | 95447 | | 3198 | F | 930776 | | 4918 | B | 551775 |
| 1479 | F | 93541 | | 3199 | F | 928876 | | 4919 | B | 553674 |
| 1480 | F | 96074 | | 3200 | F | 931898 | | 4920 | B | 552876 |
| 1481 | F | 94197 | | 3201 | F | 929987 | | 4921 | B | 554756 |
| 1482 | F | 97706 | | 3202 | F | 932291 | | 4922 | B | 555340 |
| 1483 | F | 95841 | | 3203 | F | 930323 | | 4923 | B | 557240 |
| 1484 | F | 98142 | | 3204 | F | 933264 | | 4924 | B | 555736 |
| 1485 | F | 96292 | | 3205 | F | 931339 | | 4925 | B | 557619 |
| 1486 | F | 99925 | | 3206 | F | 935505 | | 4926 | B | 558229 |
| 1487 | F | 98011 | | 3207 | F | 933605 | | 4927 | B | 560135 |
| 1488 | F | 101229 | | 3208 | F | 936779 | | 4928 | B | 558821 |
| 1489 | F | 99338 | | 3209 | F | 934873 | | 4929 | B | 560696 |
| 1490 | F | 101429 | | 3210 | F | 937000 | | 4930 | B | 559955 |
| 1491 | F | 99552 | | 3211 | F | 935108 | | 4931 | B | 561816 |
| 1492 | F | 102137 | | 3212 | F | 938062 | | 4932 | B | 561979 |
| 1493 | F | 100237 | | 3213 | F | 936162 | | 4933 | B | 563858 |
| 1494 | F | 102600 | | 3214 | F | 938536 | | 4934 | B | 561979 |
| 1495 | F | 100657 | | 3215 | F | 936689 | | 4935 | B | 563812 |
| 1496 | F | 103330 | | 3216 | F | 938934 | | 4936 | B | 564167 |
| 1497 | F | 101429 | | 3217 | F | 937000 | | 4937 | B | 566081 |
| 1498 | F | 103877 | | 3218 | F | 939541 | | 4938 | B | 565229 |
| 1499 | F | 101966 | | 3219 | F | 937640 | | 4939 | B | 567096 |
| 1500 | F | 104336 | | 3220 | F | 940603 | | 4940 | B | 566419 |
| 1501 | F | 102469 | | 3221 | F | 938681 | | 4941 | B | 568318 |
| 1502 | F | 108182 | | 3222 | F | 940758 | | 4942 | B | 567974 |
| 1503 | F | 106280 | | 3223 | F | 938826 | | 4943 | B | 569872 |
| 1504 | F | 111814 | | 3224 | F | 941387 | | 4944 | B | 568753 |
| 1505 | F | 109911 | | 3225 | F | 939470 | | 4945 | B | 570655 |
| 1506 | F | 112412 | | 3226 | F | 942261 | | 4946 | B | 569707 |
| 1507 | F | 110553 | | 3227 | F | 940373 | | 4947 | B | 571605 |
| 1508 | F | 113442 | | 3228 | F | 942563 | | 4948 | B | 571285 |
| 1509 | F | 111571 | | 3229 | F | 940654 | | 4949 | B | 573207 |
| 1510 | F | 113891 | | 3230 | F | 942807 | | 4950 | B | 572080 |
| 1511 | F | 112010 | | 3231 | F | 940907 | | 4951 | B | 573948 |
| 1512 | F | 114990 | | 3232 | F | 943510 | | 4952 | B | 572628 |
| 1513 | F | 113112 | | 3233 | F | 941608 | | 4953 | B | 574524 |
| 1514 | F | 115684 | | 3234 | F | 943771 | | 4954 | B | 573563 |
| 1515 | F | 113776 | | 3235 | F | 941872 | | 4955 | B | 575436 |
| 1516 | F | 116526 | | 3236 | F | 944330 | | 4956 | B | 572628 |
| 1517 | F | 114656 | | 3237 | F | 942413 | | 4957 | B | 574524 |
| 1518 | F | 117731 | | 3238 | F | 945147 | | 4958 | B | 575279 |
| 1519 | F | 115825 | | 3239 | F | 943262 | | 4959 | B | 577202 |
| 1520 | F | 118292 | | 3240 | F | 945527 | | 4960 | B | 576190 |
| 1521 | F | 116389 | | 3241 | F | 943620 | | 4961 | B | 578039 |
| 1522 | F | 119593 | | 3242 | F | 946627 | | 4962 | B | 578174 |
| 1523 | F | 117685 | | 3243 | F | 944741 | | 4963 | B | 580011 |
| 1524 | F | 120231 | | 3244 | F | 947165 | | 4964 | B | 579148 |
| 1525 | F | 118292 | | 3245 | F | 945278 | | 4965 | B | 581040 |
| 1526 | F | 122278 | | 3246 | F | 948674 | | 4966 | B | 580227 |
| 1527 | F | 120382 | | 3247 | F | 946774 | | 4967 | B | 582047 |
| 1528 | F | 122610 | | 3248 | F | 949646 | | 4968 | B | 580656 |
| 1529 | F | 120682 | | 3249 | F | 947716 | | 4969 | B | 582542 |
| 1530 | F | 123309 | | 3250 | F | 950731 | | 4970 | B | 580420 |
| 1531 | F | 121390 | | 3251 | F | 948837 | | 4971 | B | 582322 |
| 1532 | F | 126113 | | 3252 | F | 951418 | | 4972 | B | 581322 |
| 1533 | F | 124213 | | 3253 | F | 949545 | | 4973 | B | 583212 |
| 1534 | F | 128975 | | 3254 | F | 951940 | | 4974 | B | 582051 |
| 1535 | F | 127091 | | 3255 | F | 950034 | | 4975 | B | 583973 |
| 1536 | F | 134603 | | 3256 | F | 952365 | | 4976 | B | 582592 |
| 1537 | F | 132806 | | 3257 | F | 950461 | | 4977 | B | 584513 |
| 1538 | F | 136249 | | 3258 | F | 953230 | | 4978 | B | 583651 |
| 1539 | F | 134352 | | 3259 | F | 951316 | | 4979 | B | 585588 |
| 1540 | F | 137680 | | 3260 | F | 954978 | | 4980 | B | 584932 |
| 1541 | F | 135756 | | 3261 | F | 953125 | | 4981 | B | 586813 |
| 1542 | F | 137680 | | 3262 | F | 955613 | | 4982 | B | 585457 |
| 1543 | F | 135799 | | 3263 | F | 953697 | | 4983 | B | 587360 |
| 1544 | F | 138035 | | 3264 | F | 956989 | | 4984 | B | 587145 |
| 1545 | F | 136135 | | 3265 | F | 955136 | | 4985 | B | 589063 |
| 1546 | F | 139266 | | 3266 | F | 957684 | | 4986 | B | 588150 |
| 1547 | F | 137363 | | 3267 | F | 955778 | | 4987 | B | 590044 |
| 1548 | F | 140208 | | 3268 | F | 959156 | | 4988 | B | 588404 |
| 1549 | F | 138351 | | 3269 | F | 957187 | | 4989 | B | 590304 |
| 1550 | F | 141636 | | 3270 | F | 960035 | | 4990 | B | 589320 |
| 1551 | F | 139735 | | 3271 | F | 958117 | | 4991 | B | 591193 |
| 1552 | F | 142808 | | 3272 | F | 961584 | | 4992 | B | 590733 |
| 1553 | F | 140900 | | 3273 | F | 959727 | | 4993 | B | 592677 |
| 1554 | F | 144272 | | 3274 | F | 965172 | | 4994 | B | 592682 |
| 1555 | F | 142372 | | 3275 | F | 963269 | | 4995 | B | 594583 |
| 1556 | F | 145217 | | 3276 | F | 966747 | | 4996 | B | 593126 |
| 1557 | F | 143335 | | 3277 | F | 964843 | | 4997 | B | 595026 |
| 1558 | F | 146527 | | 3278 | F | 968015 | | 4998 | B | 594005 |
| 1559 | F | 144645 | | 3279 | F | 966111 | | 4999 | B | 595882 |
| 1560 | F | 146965 | | 3280 | F | 968508 | | 5000 | B | 594521 |
| 1561 | F | 145086 | | 3281 | F | 966609 | | 5001 | B | 596421 |
| 1562 | F | 147455 | | 3282 | F | 969289 | | 5002 | B | 596170 |
| 1563 | F | 145501 | | 3283 | F | 967389 | | 5003 | B | 598096 |
| 1564 | F | 148810 | | 3284 | F | 969537 | | 5004 | B | 596532 |
| 1565 | F | 146904 | | 3285 | F | 967640 | | 5005 | B | 598451 |
| 1566 | F | 151964 | | 3286 | F | 970078 | | 5006 | B | 597438 |
| 1567 | F | 150062 | | 3287 | F | 968137 | | 5007 | B | 599365 |
| 1568 | F | 154064 | | 3288 | F | 970317 | | 5008 | B | 598191 |
| 1569 | F | 152113 | | 3289 | F | 968394 | | 5009 | B | 600088 |
| 1570 | F | 154888 | | 3290 | F | 970857 | | 5010 | B | 598836 |
| 1571 | F | 152963 | | 3291 | F | 968969 | | 5011 | B | 600749 |
| 1572 | F | 155418 | | 3292 | F | 971657 | | 5012 | B | 599476 |
| 1573 | F | 153558 | | 3293 | F | 969757 | | 5013 | B | 601327 |
| 1574 | F | 156528 | | 3294 | F | 974954 | | 5014 | B | 600192 |
| 1575 | F | 154606 | | 3295 | F | 973067 | | 5015 | B | 602103 |
| 1576 | F | 157433 | | 3296 | F | 975200 | | 5016 | B | 601131 |
| 1577 | F | 155516 | | 3297 | F | 973300 | | 5017 | B | 603030 |
| 1578 | F | 158771 | | 3298 | F | 976362 | | 5018 | B | 602307 |
| 1579 | F | 156842 | | 3299 | F | 974418 | | 5019 | B | 604209 |
| 1580 | F | 159105 | | 3300 | F | 977009 | | 5020 | B | 602810 |
| 1581 | F | 157219 | | 3301 | F | 975050 | | 5021 | B | 604759 |
| 1582 | F | 159657 | | 3302 | F | 978153 | | 5022 | B | 603529 |
| 1583 | F | 157761 | | 3303 | F | 976255 | | 5023 | B | 605402 |
| 1584 | F | 160240 | | 3304 | F | 980532 | | 5024 | B | 604759 |
| 1585 | F | 158316 | | 3305 | F | 978632 | | 5025 | B | 606662 |
| 1586 | F | 160675 | | 3306 | F | 981701 | | 5026 | B | 606076 |
| 1587 | F | 158778 | | 3307 | F | 979785 | | 5027 | B | 608046 |
| 1588 | F | 161289 | | 3308 | F | 982885 | | 5028 | B | 606843 |
| 1589 | F | 159402 | | 3309 | F | 980983 | | 5029 | B | 608746 |
| 1590 | F | 161918 | | 3310 | F | 983878 | | 5030 | B | 607504 |
| 1591 | F | 159979 | | 3311 | F | 981973 | | 5031 | B | 609404 |
| 1592 | F | 162214 | | 3312 | F | 985264 | | 5032 | B | 609224 |
| 1593 | F | 160297 | | 3313 | F | 983395 | | 5033 | B | 611138 |
| 1594 | F | 163996 | | 3314 | F | 986953 | | 5034 | B | 609952 |
| 1595 | F | 162045 | | 3315 | F | 985049 | | 5035 | B | 611865 |
| 1596 | F | 165189 | | 3316 | F | 985623 | | 5036 | B | 611138 |
| 1597 | F | 163288 | | 3317 | F | 983760 | | 5037 | B | 613033 |
| 1598 | F | 166730 | | 3318 | F | 986956 | | 5038 | B | 612012 |
| 1599 | F | 164828 | | 3319 | F | 985049 | | 5039 | B | 613917 |
| 1600 | F | 168243 | | 3320 | F | 987506 | | 5040 | B | 612554 |
| 1601 | F | 166327 | | 3321 | F | 985592 | | 5041 | B | 614453 |
| 1602 | F | 168907 | | 3322 | F | 988307 | | 5042 | B | 614136 |
| 1603 | F | 167064 | | 3323 | F | 986404 | | 5043 | B | 616017 |
| 1604 | F | 169129 | | 3324 | F | 988783 | | 5044 | B | 614978 |
| 1605 | F | 167294 | | 3325 | F | 986927 | | 5045 | B | 616936 |
| 1606 | F | 170632 | | 3326 | F | 989593 | | 5046 | B | 615399 |
| 1607 | F | 168692 | | 3327 | F | 987694 | | 5047 | B | 617342 |
| 1608 | F | 171229 | | 3328 | F | 990733 | | 5048 | B | 616565 |
| 1609 | F | 169381 | | 3329 | F | 988783 | | 5049 | B | 618402 |
| 1610 | F | 171553 | | 3330 | F | 991559 | | 5050 | B | 617618 |
| 1611 | F | 169614 | | 3331 | F | 989675 | | 5051 | B | 619515 |
| 1612 | F | 172433 | | 3332 | F | 992323 | | 5052 | B | 619027 |
| 1613 | F | 170533 | | 3333 | F | 990421 | | 5053 | B | 620937 |
| 1614 | F | 173217 | | 3334 | F | 992522 | | 5054 | B | 620142 |
| 1615 | F | 171316 | | 3335 | F | 990640 | | 5055 | B | 622052 |
| 1616 | F | 174567 | | 3336 | F | 993308 | | 5056 | B | 620230 |
| 1617 | F | 172680 | | 3337 | F | 991361 | | 5057 | B | 622124 |
| 1618 | F | 175342 | | 3338 | F | 992795 | | 5058 | B | 621498 |
| 1619 | F | 173479 | | 3339 | F | 990919 | | 5059 | B | 623385 |
| 1620 | F | 175709 | | 3340 | F | 994573 | | 5060 | B | 622583 |
| 1621 | F | 173752 | | 3341 | F | 992673 | | 5061 | B | 624479 |
| 1622 | F | 176909 | | 3342 | F | 995517 | | 5062 | B | 623718 |
| 1623 | F | 175009 | | 3343 | F | 993570 | | 5063 | B | 625598 |
| 1624 | F | 176704 | | 3344 | F | 996518 | | 5064 | B | 624533 |
| 1625 | F | 174761 | | 3345 | F | 994660 | | 5065 | B | 626462 |
| 1626 | F | 177608 | | 3346 | F | 997317 | | 5066 | B | 625020 |
| 1627 | F | 175709 | | 3347 | F | 995450 | | 5067 | B | 626893 |
| 1628 | F | 179259 | | 3348 | F | 998653 | | 5068 | B | 625774 |
| 1629 | F | 177384 | | 3349 | F | 996762 | | 5069 | B | 627660 |
| 1630 | F | 179719 | | 3350 | F | 999865 | | 5070 | B | 626146 |
| 1631 | F | 177800 | | 3351 | F | 997908 | | 5071 | B | 628010 |
| 1632 | F | 181629 | | 3352 | F | 1001112 | | 5072 | B | 626646 |
| 1633 | F | 179743 | | 3353 | F | 999238 | | 5073 | B | 628522 |
| 1634 | F | 182851 | | 3354 | F | 1001651 | | 5074 | B | 628020 |
| 1635 | F | 180952 | | 3355 | F | 999731 | | 5075 | B | 629982 |
| 1636 | F | 184230 | | 3356 | F | 1003237 | | 5076 | B | 628882 |
| 1637 | F | 182335 | | 3357 | F | 1001317 | | 5077 | B | 630730 |
| 1638 | F | 184870 | | 3358 | F | 1004049 | | 5078 | B | 629982 |
| 1639 | F | 182962 | | 3359 | F | 1002132 | | 5079 | B | 631822 |
| 1640 | F | 185241 | | 3360 | F | 1004252 | | 5080 | B | 631862 |
| 1641 | F | 183348 | | 3361 | F | 1002307 | | 5081 | B | 633762 |
| 1642 | F | 185611 | | 3362 | F | 1005400 | | 5082 | B | 633774 |
| 1643 | F | 183685 | | 3363 | F | 1003518 | | 5083 | B | 635675 |
| 1644 | F | 186336 | | 3364 | F | 1005892 | | 5084 | B | 637192 |
| 1645 | F | 184445 | | 3365 | F | 1003958 | | 5085 | B | 639082 |
| 1646 | F | 188059 | | 3366 | F | 1006516 | | 5086 | B | 638321 |
| 1647 | F | 186171 | | 3367 | F | 1004599 | | 5087 | B | 640221 |
| 1648 | F | 190828 | | 3368 | F | 1007332 | | 5088 | B | 639082 |
| 1649 | F | 188956 | | 3369 | F | 1005446 | | 5089 | B | 640954 |
| 1650 | F | 191294 | | 3370 | F | 1009066 | | 5090 | B | 639317 |
| 1651 | F | 189428 | | 3371 | F | 1007190 | | 5091 | B | 641243 |
| 1652 | F | 192686 | | 3372 | F | 1014072 | | 5092 | B | 639860 |
| 1653 | F | 190788 | | 3373 | F | 1012172 | | 5093 | B | 641780 |
| 1654 | F | 193380 | | 3374 | F | 1015614 | | 5094 | B | 640868 |
| 1655 | F | 191474 | | 3375 | F | 1013733 | | 5095 | B | 642770 |
| 1656 | F | 193388 | | 3376 | F | 1016078 | | 5096 | B | 641243 |
| 1657 | F | 191474 | | 3377 | F | 1014172 | | 5097 | B | 643106 |
| 1658 | F | 193977 | | 3378 | F | 1015924 | | 5098 | B | 641605 |
| 1659 | F | 192059 | | 3379 | F | 1014059 | | 5099 | B | 643503 |
| 1660 | F | 195480 | | 3380 | F | 1016230 | | 5100 | B | 642538 |
| 1661 | F | 193585 | | 3381 | F | 1014330 | | 5101 | B | 644407 |
| 1662 | F | 195868 | | 3382 | F | 1017479 | | 5102 | B | 643243 |
| 1663 | F | 193969 | | 3383 | F | 1015558 | | 5103 | B | 645145 |
| 1664 | F | 197913 | | 3384 | F | 1018915 | | 5104 | B | 643550 |
| 1665 | F | 196013 | | 3385 | F | 1017003 | | 5105 | B | 645450 |
| 1666 | F | 199088 | | 3386 | F | 1019328 | | 5106 | B | 643925 |
| 1667 | F | 197213 | | 3387 | F | 1017440 | | 5107 | B | 645837 |
| 1668 | F | 202776 | | 3388 | F | 1020813 | | 5108 | B | 645848 |
| 1669 | F | 200876 | | 3389 | F | 1018915 | | 5109 | B | 647759 |
| 1670 | F | 204467 | | 3390 | F | 1021621 | | 5110 | B | 645987 |
| 1671 | F | 202497 | | 3391 | F | 1019671 | | 5111 | B | 647969 |
| 1672 | F | 205584 | | 3392 | F | 1023996 | | 5112 | B | 646490 |
| 1673 | F | 203664 | | 3393 | F | 1022107 | | 5113 | B | 648429 |
| 1674 | F | 206940 | | 3394 | F | 1024277 | | 5114 | B | 646973 |
| 1675 | F | 205063 | | 3395 | F | 1022385 | | 5115 | B | 648871 |
| 1676 | F | 207560 | | 3396 | F | 1025368 | | 5116 | B | 648115 |
| 1677 | F | 205587 | | 3397 | F | 1023468 | | 5117 | B | 650007 |
| 1678 | F | 208048 | | 3398 | F | 1026671 | | 5118 | B | 648516 |
| 1679 | F | 206139 | | 3399 | F | 1024821 | | 5119 | B | 650374 |
| 1680 | F | 209923 | | 3400 | F | 1027688 | | 5120 | B | 650567 |
| 1681 | F | 208023 | | 3401 | F | 1025823 | | 5121 | B | 652472 |
| 1682 | F | 210455 | | 3402 | F | 1030916 | | 5122 | B | 651251 |
| 1683 | F | 208569 | | 3403 | F | 1029047 | | 5123 | B | 653140 |
| 1684 | F | 211049 | | 3404 | F | 1031342 | | 5124 | B | 653186 |
| 1685 | F | 209147 | | 3405 | F | 1029430 | | 5125 | B | 655076 |
| 1686 | F | 211596 | | 3406 | F | 1032795 | | 5126 | B | 653628 |
| 1687 | F | 209705 | | 3407 | F | 1030916 | | 5127 | B | 655515 |
| 1688 | F | 212226 | | 3408 | F | 1032978 | | 5128 | B | 656010 |
| 1689 | F | 210311 | | 3409 | F | 1031078 | | 5129 | B | 657870 |
| 1690 | F | 213832 | | 3410 | F | 1033730 | | 5130 | B | 656761 |
| 1691 | F | 211960 | | 3411 | F | 1031839 | | 5131 | B | 658636 |
| 1692 | F | 214866 | | 3412 | F | 1035774 | | 5132 | B | 658389 |
| 1693 | F | 212921 | | 3413 | F | 1033821 | | 5133 | B | 660295 |
| 1694 | F | 215173 | | 3414 | F | 1036884 | | 5134 | B | 660436 |
| 1695 | F | 213307 | | 3415 | F | 1034954 | | 5135 | B | 662352 |
| 1696 | F | 215800 | | 3416 | F | 1037476 | | 5136 | B | 663483 |
| 1697 | F | 213957 | | 3417 | F | 1035577 | | 5137 | B | 665358 |
| 1698 | F | 216489 | | 3418 | F | 1037714 | | 5138 | B | 664701 |
| 1699 | F | 214549 | | 3419 | F | 1035847 | | 5139 | B | 666607 |
| 1700 | F | 216980 | | 3420 | F | 1038782 | | 5140 | B | 665978 |
| 1701 | F | 215100 | | 3421 | F | 1036884 | | 5141 | B | 667856 |
| 1702 | F | 217665 | | 3422 | F | 1040777 | | 5142 | B | 667238 |
| 1703 | F | 215793 | | 3423 | F | 1038856 | | 5143 | B | 669172 |
| 1704 | F | 218039 | | 3424 | F | 1042132 | | 5144 | B | 668195 |
| 1705 | F | 216071 | | 3425 | F | 1040216 | | 5145 | B | 670046 |
| 1706 | F | 218476 | | 3426 | F | 1043148 | | 5146 | B | 668791 |
| 1707 | F | 216560 | | 3427 | F | 1041215 | | 5147 | B | 670691 |
| 1708 | F | 218769 | | 3428 | F | 1044388 | | 5148 | B | 669426 |
| 1709 | F | 216809 | | 3429 | F | 1042445 | | 5149 | B | 671326 |
| 1710 | F | 220020 | | 3430 | F | 1045164 | | 5150 | B | 671116 |
| 1711 | F | 218128 | | 3431 | F | 1043224 | | 5151 | B | 673055 |
| 1712 | F | 221210 | | 3432 | F | 1046223 | | 5152 | B | 671659 |
| 1713 | F | 219275 | | 3433 | F | 1044324 | | 5153 | B | 673547 |
| 1714 | F | 222497 | | 3434 | F | 1047299 | | 5154 | B | 672474 |
| 1715 | F | 220601 | | 3435 | F | 1045364 | | 5155 | B | 674347 |
| 1716 | F | 223292 | | 3436 | F | 1049803 | | 5156 | B | 673238 |
| 1717 | F | 221403 | | 3437 | F | 1047914 | | 5157 | B | 675140 |
| 1718 | F | 223775 | | 3438 | F | 1050341 | | 5158 | B | 674944 |
| 1719 | F | 221877 | | 3439 | F | 1048431 | | 5159 | B | 676911 |
| 1720 | F | 224250 | | 3440 | F | 1050862 | | 5160 | B | 674797 |
| 1721 | F | 222377 | | 3441 | F | 1048907 | | 5161 | B | 676669 |
| 1722 | F | 224906 | | 3442 | F | 1051515 | | 5162 | B | 675741 |
| 1723 | F | 223008 | | 3443 | F | 1049572 | | 5163 | B | 677643 |
| 1724 | F | 225283 | | 3444 | F | 1051828 | | 5164 | B | 676340 |
| 1725 | F | 223418 | | 3445 | F | 1049917 | | 5165 | B | 678204 |
| 1726 | F | 226670 | | 3446 | F | 1052885 | | 5166 | B | 676911 |
| 1727 | F | 224770 | | 3447 | F | 1050957 | | 5167 | B | 678770 |
| 1728 | F | 227849 | | 3448 | F | 1053963 | | 5168 | B | 677240 |
| 1729 | F | 225937 | | 3449 | F | 1052057 | | 5169 | B | 679136 |
| 1730 | F | 228185 | | 3450 | F | 1055238 | | 5170 | B | 677873 |
| 1731 | F | 226269 | | 3451 | F | 1053362 | | 5171 | B | 679767 |
| 1732 | F | 228393 | | 3452 | F | 1055849 | | 5172 | B | 678549 |
| 1733 | F | 226512 | | 3453 | F | 1053963 | | 5173 | B | 680420 |
| 1734 | F | 229334 | | 3454 | F | 1056332 | | 5174 | B | 679692 |
| 1735 | F | 227499 | | 3455 | F | 1054465 | | 5175 | B | 681628 |
| 1736 | F | 230761 | | 3456 | F | 1056738 | | 5176 | B | 680320 |
| 1737 | F | 228846 | | 3457 | F | 1054830 | | 5177 | B | 682220 |
| 1738 | F | 231287 | | 3458 | F | 1058019 | | 5178 | B | 681126 |
| 1739 | F | 229334 | | 3459 | F | 1056110 | | 5179 | B | 683046 |
| 1740 | F | 231731 | | 3460 | F | 1058504 | | 5180 | B | 682558 |
| 1741 | F | 229927 | | 3461 | F | 1056587 | | 5181 | B | 684404 |
| 1742 | F | 232865 | | 3462 | F | 1059300 | | 5182 | B | 681857 |
| 1743 | F | 231027 | | 3463 | F | 1057406 | | 5183 | B | 683768 |
| 1744 | F | 232865 | | 3464 | F | 1060356 | | 5184 | B | 683046 |
| 1745 | F | 231027 | | 3465 | F | 1058400 | | 5185 | B | 684944 |
| 1746 | F | 234315 | | 3466 | F | 1061455 | | 5186 | B | 684128 |
| 1747 | F | 232394 | | 3467 | F | 1059456 | | 5187 | B | 686124 |
| 1748 | F | 234823 | | 3468 | F | 1062092 | | 5188 | B | 684893 |
| 1749 | F | 232865 | | 3469 | F | 1060243 | | 5189 | B | 686740 |
| 1750 | F | 235154 | | 3470 | F | 1063884 | | 5190 | B | 685389 |
| 1751 | F | 233245 | | 3471 | F | 1061983 | | 5191 | B | 687290 |
| 1752 | F | 236429 | | 3472 | F | 1064928 | | 5192 | B | 686207 |
| 1753 | F | 234520 | | 3473 | F | 1063056 | | 5193 | B | 688106 |
| 1754 | F | 237268 | | 3474 | F | 1067125 | | 5194 | B | 687534 |
| 1755 | F | 235271 | | 3475 | F | 1065240 | | 5195 | B | 689424 |
| 1756 | F | 238047 | | 3476 | F | 1067963 | | 5196 | B | 688416 |
| 1757 | F | 236162 | | 3477 | F | 1066075 | | 5197 | B | 690275 |
| 1758 | F | 238636 | | 3478 | F | 1068596 | | 5198 | B | 688955 |
| 1759 | F | 236736 | | 3479 | F | 1066668 | | 5199 | B | 690855 |
| 1760 | F | 239957 | | 3480 | F | 1069752 | | 5200 | B | 689727 |
| 1761 | F | 238047 | | 3481 | F | 1067890 | | 5201 | B | 691626 |
| 1762 | F | 241373 | | 3482 | F | 1071068 | | 5202 | B | 690496 |
| 1763 | F | 239482 | | 3483 | F | 1069210 | | 5203 | B | 692386 |
| 1764 | F | 242017 | | 3484 | F | 1072701 | | 5204 | B | 691349 |
| 1765 | F | 240072 | | 3485 | F | 1070806 | | 5205 | B | 693249 |
| 1766 | F | 242740 | | 3486 | F | 1073987 | | 5206 | B | 692864 |
| 1767 | F | 240829 | | 3487 | F | 1072090 | | 5207 | B | 694724 |
| 1768 | F | 243281 | | 3488 | F | 1075643 | | 5208 | B | 695287 |
| 1769 | F | 241373 | | 3489 | F | 1073742 | | 5209 | B | 697187 |
| 1770 | F | 244244 | | 3490 | F | 1076350 | | 5210 | B | 696275 |
| 1771 | F | 242345 | | 3491 | F | 1074450 | | 5211 | B | 698172 |
| 1772 | F | 246052 | | 3492 | F | 1077354 | | 5212 | B | 696786 |
| 1773 | F | 244179 | | 3493 | F | 1075555 | | 5213 | B | 698696 |
| 1774 | F | 247581 | | 3494 | F | 1077778 | | 5214 | B | 698185 |
| 1775 | F | 245697 | | 3495 | F | 1075880 | | 5215 | B | 700090 |
| 1776 | F | 249216 | | 3496 | F | 1078445 | | 5216 | B | 700037 |
| 1777 | F | 247244 | | 3497 | F | 1076529 | | 5217 | B | 701923 |
| 1778 | F | 251003 | | 3498 | F | 1079373 | | 5218 | B | 702172 |
| 1779 | F | 249137 | | 3499 | F | 1077523 | | 5219 | B | 704050 |
| 1780 | F | 252064 | | 3500 | F | 1079715 | | 5220 | B | 703443 |
| 1781 | F | 250189 | | 3501 | F | 1077850 | | 5221 | B | 705316 |
| 1782 | F | 252900 | | 3502 | F | 1080538 | | 5222 | B | 704441 |
| 1783 | F | 251000 | | 3503 | F | 1078655 | | 5223 | B | 706351 |
| 1784 | F | 253718 | | 3504 | F | 1081108 | | 5224 | B | 705516 |
| 1785 | F | 251855 | | 3505 | F | 1079228 | | 5225 | B | 707413 |
| 1786 | F | 254993 | | 3506 | F | 1083006 | | 5226 | B | 706312 |
| 1787 | F | 253138 | | 3507 | F | 1081108 | | 5227 | B | 708190 |
| 1788 | F | 256414 | | 3508 | F | 1084404 | | 5228 | B | 707058 |
| 1789 | F | 254509 | | 3509 | F | 1082465 | | 5229 | B | 708979 |
| 1790 | F | 257283 | | 3510 | F | 1085896 | | 5230 | B | 707856 |
| 1791 | F | 255383 | | 3511 | F | 1083990 | | 5231 | B | 709719 |
| 1792 | F | 257279 | | 3512 | F | 1086468 | | 5232 | B | 708906 |
| 1793 | F | 255379 | | 3513 | F | 1084563 | | 5233 | B | 710811 |
| 1794 | F | 258061 | | 3514 | F | 1087889 | | 5234 | B | 709258 |
| 1795 | F | 256107 | | 3515 | F | 1085985 | | 5235 | B | 711132 |
| 1796 | F | 259005 | | 3516 | F | 1088427 | | 5236 | B | 710074 |
| 1797 | F | 257128 | | 3517 | F | 1086527 | | 5237 | B | 711924 |
| 1798 | F | 261075 | | 3518 | F | 1088927 | | 5238 | B | 710328 |
| 1799 | F | 259195 | | 3519 | F | 1087027 | | 5239 | B | 712212 |
| 1800 | F | 261551 | | 3520 | F | 1089668 | | 5240 | B | 711748 |
| 1801 | F | 259650 | | 3521 | F | 1087768 | | 5241 | B | 713690 |
| 1802 | F | 262535 | | 3522 | F | 1092655 | | 5242 | B | 712456 |
| 1803 | F | 260611 | | 3523 | F | 1090767 | | 5243 | B | 714407 |
| 1804 | F | 262960 | | 3524 | F | 1093357 | | 5244 | B | 715001 |
| 1805 | F | 261060 | | 3525 | F | 1091465 | | 5245 | B | 716854 |
| 1806 | F | 264509 | | 3526 | F | 1093957 | | 5246 | B | 715983 |
| 1807 | F | 262614 | | 3527 | F | 1092070 | | 5247 | B | 717887 |
| 1808 | F | 265837 | | 3528 | F | 1095818 | | 5248 | B | 717800 |
| 1809 | F | 263925 | | 3529 | F | 1093955 | | 5249 | B | 719668 |
| 1810 | F | 266239 | | 3530 | F | 1096359 | | 5250 | B | 718468 |
| 1811 | F | 264367 | | 3531 | F | 1094509 | | 5251 | B | 720383 |
| 1812 | F | 267185 | | 3532 | F | 1097047 | | 5252 | B | 720469 |
| 1813 | F | 265286 | | 3533 | F | 1095114 | | 5253 | B | 722367 |
| 1814 | F | 267909 | | 3534 | F | 1097365 | | 5254 | B | 722645 |
| 1815 | F | 266037 | | 3535 | F | 1095498 | | 5255 | B | 724559 |
| 1816 | F | 268594 | | 3536 | F | 1097646 | | 5256 | B | 723280 |
| 1817 | F | 266756 | | 3537 | F | 1095767 | | 5257 | B | 725273 |
| 1818 | F | 269299 | | 3538 | F | 1098161 | | 5258 | B | 723775 |
| 1819 | F | 267505 | | 3539 | F | 1096242 | | 5259 | B | 725691 |
| 1820 | F | 271044 | | 3540 | F | 1098560 | | 5260 | B | 724469 |
| 1821 | F | 269121 | | 3541 | F | 1096663 | | 5261 | B | 726387 |
| 1822 | F | 271737 | | 3542 | F | 1099044 | | 5262 | B | 725016 |
| 1823 | F | 269838 | | 3543 | F | 1097150 | | 5263 | B | 726902 |
| 1824 | F | 272558 | | 3544 | F | 1099454 | | 5264 | B | 726088 |
| 1825 | F | 270645 | | 3545 | F | 1097547 | | 5265 | B | 727988 |
| 1826 | F | 273007 | | 3546 | F | 1100878 | | 5266 | B | 727397 |
| 1827 | F | 271098 | | 3547 | F | 1098942 | | 5267 | B | 729236 |
| 1828 | F | 273463 | | 3548 | F | 1101839 | | 5268 | B | 728347 |
| 1829 | F | 271500 | | 3549 | F | 1099956 | | 5269 | B | 730278 |
| 1830 | F | 273922 | | 3550 | F | 1104621 | | 5270 | B | 728816 |
| 1831 | F | 272057 | | 3551 | F | 1102789 | | 5271 | B | 730718 |
| 1832 | F | 275083 | | 3552 | F | 1106487 | | 5272 | B | 729846 |
| 1833 | F | 273094 | | 3553 | F | 1104562 | | 5273 | B | 731740 |
| 1834 | F | 275495 | | 3554 | F | 1107225 | | 5274 | B | 730005 |
| 1835 | F | 273554 | | 3555 | F | 1105318 | | 5275 | B | 731898 |
| 1836 | F | 275739 | | 3556 | F | 1107814 | | 5276 | B | 730377 |
| 1837 | F | 273878 | | 3557 | F | 1105922 | | 5277 | B | 732272 |
| 1838 | F | 276229 | | 3558 | F | 1108282 | | 5278 | B | 730759 |
| 1839 | F | 274371 | | 3559 | F | 1106374 | | 5279 | B | 732659 |
| 1840 | F | 276548 | | 3560 | F | 1113162 | | 5280 | B | 732249 |
| 1841 | F | 274638 | | 3561 | F | 1111308 | | 5281 | B | 734124 |
| 1842 | F | 277098 | | 3562 | F | 1114813 | | 5282 | B | 732647 |
| 1843 | F | 275178 | | 3563 | F | 1112949 | | 5283 | B | 734590 |
| 1844 | F | 277358 | | 3564 | F | 1116611 | | 5284 | B | 733144 |
| 1845 | F | 275448 | | 3565 | F | 1114766 | | 5285 | B | 735088 |
| 1846 | F | 277609 | | 3566 | F | 1118605 | | 5286 | B | 733858 |
| 1847 | F | 275739 | | 3567 | F | 1116725 | | 5287 | B | 735787 |
| 1848 | F | 278314 | | 3568 | F | 1119754 | | 5288 | B | 734124 |
| 1849 | F | 276386 | | 3569 | F | 1117874 | | 5289 | B | 736028 |
| 1850 | F | 279310 | | 3570 | F | 1120291 | | 5290 | B | 734523 |
| 1851 | F | 277385 | | 3571 | F | 1118385 | | 5291 | B | 736441 |
| 1852 | F | 280627 | | 3572 | F | 1121099 | | 5292 | B | 735088 |
| 1853 | F | 278702 | | 3573 | F | 1119202 | | 5293 | B | 736978 |
| 1854 | F | 281471 | | 3574 | F | 1121886 | | 5294 | B | 735416 |
| 1855 | F | 279559 | | 3575 | F | 1119982 | | 5295 | B | 737315 |
| 1856 | F | 282239 | | 3576 | F | 1122979 | | 5296 | B | 735822 |
| 1857 | F | 280288 | | 3577 | F | 1121038 | | 5297 | B | 737700 |
| 1858 | F | 283832 | | 3578 | F | 1123376 | | 5298 | B | 736099 |
| 1859 | F | 281933 | | 3579 | F | 1121486 | | 5299 | B | 737981 |
| 1860 | F | 284384 | | 3580 | F | 1124136 | | 5300 | B | 736714 |
| 1861 | F | 282486 | | 3581 | F | 1122333 | | 5301 | B | 738612 |
| 1862 | F | 285373 | | 3582 | F | 1124623 | | 5302 | B | 737448 |
| 1863 | F | 283473 | | 3583 | F | 1122723 | | 5303 | B | 739321 |
| 1864 | F | 285919 | | 3584 | F | 1125306 | | 5304 | B | 737802 |
| 1865 | F | 284059 | | 3585 | F | 1123423 | | 5305 | B | 739693 |
| 1866 | F | 286742 | | 3586 | F | 1126300 | | 5306 | B | 738048 |
| 1867 | F | 284879 | | 3587 | F | 1124399 | | 5307 | B | 739948 |
| 1868 | F | 287216 | | 3588 | F | 1127440 | | 5308 | B | 738964 |
| 1869 | F | 285329 | | 3589 | F | 1125545 | | 5309 | B | 740808 |
| 1870 | F | 287671 | | 3590 | F | 1128968 | | 5310 | B | 739282 |
| 1871 | F | 285751 | | 3591 | F | 1127134 | | 5311 | B | 741190 |
| 1872 | F | 288273 | | 3592 | F | 1129916 | | 5312 | B | 739956 |
| 1873 | F | 286323 | | 3593 | F | 1128111 | | 5313 | B | 741906 |
| 1874 | F | 288618 | | 3594 | F | 1131255 | | 5314 | B | 740743 |
| 1875 | F | 286685 | | 3595 | F | 1129330 | | 5315 | B | 742597 |
| 1876 | F | 288273 | | 3596 | F | 1132598 | | 5316 | B | 741190 |
| 1877 | F | 286323 | | 3597 | F | 1130684 | | 5317 | B | 743081 |
| 1878 | F | 289723 | | 3598 | F | 1133896 | | 5318 | B | 741942 |
| 1879 | F | 287836 | | 3599 | F | 1132002 | | 5319 | B | 743875 |
| 1880 | F | 289508 | | 3600 | F | 1134373 | | 5320 | B | 743009 |
| 1881 | F | 287667 | | 3601 | F | 1132510 | | 5321 | B | 744914 |
| 1882 | F | 290750 | | 3602 | F | 1135431 | | 5322 | B | 743875 |
| 1883 | F | 288858 | | 3603 | F | 1133531 | | 5323 | B | 745738 |
| 1884 | F | 291142 | | 3604 | F | 1135730 | | 5324 | B | 744325 |
| 1885 | F | 289253 | | 3605 | F | 1133823 | | 5325 | B | 746234 |
| 1886 | F | 291702 | | 3606 | F | 1136932 | | 5326 | B | 744824 |
| 1887 | F | 289812 | | 3607 | F | 1135040 | | 5327 | B | 746724 |
| 1888 | F | 292522 | | 3608 | F | 1139875 | | 5328 | B | 745207 |
| 1889 | F | 290633 | | 3609 | F | 1137942 | | 5329 | B | 747073 |
| 1890 | F | 293035 | | 3610 | F | 1141133 | | 5330 | B | 746828 |
| 1891 | F | 291142 | | 3611 | F | 1139231 | | 5331 | B | 748738 |
| 1892 | F | 293731 | | 3612 | F | 1142301 | | 5332 | B | 747344 |
| 1893 | F | 291786 | | 3613 | F | 1140366 | | 5333 | B | 749206 |
| 1894 | F | 294530 | | 3614 | F | 1145346 | | 5334 | B | 748253 |
| 1895 | F | 292670 | | 3615 | F | 1143505 | | 5335 | B | 750094 |
| 1896 | F | 294367 | | 3616 | F | 1146637 | | 5336 | B | 748856 |
| 1897 | F | 292513 | | 3617 | F | 1144743 | | 5337 | B | 750717 |
| 1898 | F | 296092 | | 3618 | F | 1147417 | | 5338 | B | 749376 |
| 1899 | F | 294209 | | 3619 | F | 1145547 | | 5339 | B | 751265 |
| 1900 | F | 297611 | | 3620 | F | 1147981 | | 5340 | B | 750180 |
| 1901 | F | 295757 | | 3621 | F | 1146086 | | 5341 | B | 752086 |
| 1902 | F | 298027 | | 3622 | F | 1148126 | | 5342 | B | 750667 |
| 1903 | F | 296092 | | 3623 | F | 1146211 | | 5343 | B | 752569 |
| 1904 | F | 298555 | | 3624 | F | 1148913 | | 5344 | B | 751458 |
| 1905 | F | 296582 | | 3625 | F | 1147044 | | 5345 | B | 753343 |
| 1906 | F | 299403 | | 3626 | F | 1149702 | | 5346 | B | 753262 |
| 1907 | F | 297511 | | 3627 | F | 1147890 | | 5347 | B | 755162 |
| 1908 | F | 300409 | | 3628 | F | 1150561 | | 5348 | B | 754535 |
| 1909 | F | 298579 | | 3629 | F | 1148660 | | 5349 | B | 756429 |
| 1910 | F | 301332 | | 3630 | F | 1150946 | | 5350 | B | 756398 |
| 1911 | F | 299433 | | 3631 | F | 1149046 | | 5351 | B | 758298 |
| 1912 | F | 302215 | | 3632 | F | 1152302 | | 5352 | B | 756708 |
| 1913 | F | 300282 | | 3633 | F | 1150392 | | 5353 | B | 758611 |
| 1914 | F | 302492 | | 3634 | F | 1154344 | | 5354 | B | 760465 |
| 1915 | F | 300618 | | 3635 | F | 1152371 | | 5355 | B | 762358 |
| 1916 | F | 303627 | | 3636 | F | 1155448 | | 5356 | B | 761441 |
| 1917 | F | 301730 | | 3637 | F | 1153548 | | 5357 | B | 763356 |
| 1918 | F | 304350 | | 3638 | F | 1156630 | | 5358 | B | 762077 |
| 1919 | F | 302487 | | 3639 | F | 1154729 | | 5359 | B | 763945 |
| 1920 | F | 305173 | | 3640 | F | 1157756 | | 5360 | B | 762528 |
| 1921 | F | 303226 | | 3641 | F | 1155862 | | 5361 | B | 764410 |
| 1922 | F | 306244 | | 3642 | F | 1160695 | | 5362 | B | 763118 |
| 1923 | F | 304350 | | 3643 | F | 1158788 | | 5363 | B | 765018 |
| 1924 | F | 307232 | | 3644 | F | 1162326 | | 5364 | B | 763539 |
| 1925 | F | 305310 | | 3645 | F | 1160468 | | 5365 | B | 765504 |
| 1926 | F | 307799 | | 3646 | F | 1163300 | | 5366 | B | 764000 |
| 1927 | F | 305877 | | 3647 | F | 1161413 | | 5367 | B | 765907 |
| 1928 | F | 309173 | | 3648 | F | 1163763 | | 5368 | B | 765391 |
| 1929 | F | 307301 | | 3649 | F | 1161842 | | 5369 | B | 767328 |
| 1930 | F | 310158 | | 3650 | F | 1164224 | | 5370 | B | 767041 |
| 1931 | F | 308306 | | 3651 | F | 1162283 | | 5371 | B | 768951 |
| 1932 | F | 311020 | | 3652 | F | 1164800 | | 5372 | B | 768271 |
| 1933 | F | 309118 | | 3653 | F | 1162908 | | 5373 | B | 770171 |
| 1934 | F | 311031 | | 3654 | F | 1165312 | | 5374 | B | 768799 |
| 1935 | F | 309126 | | 3655 | F | 1163427 | | 5375 | B | 770686 |
| 1936 | F | 311552 | | 3656 | F | 1165877 | | 5376 | B | 769562 |
| 1937 | F | 309658 | | 3657 | F | 1163960 | | 5377 | B | 771608 |
| 1938 | F | 312510 | | 3658 | F | 1166827 | | 5378 | B | 770752 |
| 1939 | F | 310614 | | 3659 | F | 1164936 | | 5379 | B | 772652 |
| 1940 | F | 313134 | | 3660 | F | 1168099 | | 5380 | B | 771701 |
| 1941 | F | 311255 | | 3661 | F | 1166212 | | 5381 | B | 773620 |
| 1942 | F | 313674 | | 3662 | F | 1168991 | | 5382 | B | 773316 |
| 1943 | F | 311717 | | 3663 | F | 1167093 | | 5383 | B | 775178 |
| 1944 | F | 314490 | | 3664 | F | 1169769 | | 5384 | B | 773690 |
| 1945 | F | 312633 | | 3665 | F | 1167907 | | 5385 | B | 775579 |
| 1946 | F | 315306 | | 3666 | F | 1170349 | | 5386 | B | 774596 |
| 1947 | F | 313355 | | 3667 | F | 1168446 | | 5387 | B | 776522 |
| 1948 | F | 315932 | | 3668 | F | 1170953 | | 5388 | B | 776300 |
| 1949 | F | 314033 | | 3669 | F | 1169031 | | 5389 | B | 778224 |
| 1950 | F | 318434 | | 3670 | F | 1171641 | | 5390 | B | 775346 |
| 1951 | F | 316516 | | 3671 | F | 1169703 | | 5391 | B | 777266 |
| 1952 | F | 320876 | | 3672 | F | 1172172 | | 5392 | B | 775618 |
| 1953 | F | 318949 | | 3673 | F | 1170256 | | 5393 | B | 777518 |
| 1954 | F | 321403 | | 3674 | F | 1173649 | | 5394 | B | 777266 |
| 1955 | F | 319547 | | 3675 | F | 1171759 | | 5395 | B | 779200 |
| 1956 | F | 322084 | | 3676 | F | 1174885 | | 5396 | B | 778224 |
| 1957 | F | 320217 | | 3677 | F | 1172999 | | 5397 | B | 780087 |
| 1958 | F | 322911 | | 3678 | F | 1175559 | | 5398 | B | 778396 |
| 1959 | F | 321049 | | 3679 | F | 1173649 | | 5399 | B | 780301 |
| 1960 | F | 323634 | | 3680 | F | 1176927 | | 5400 | B | 779557 |
| 1961 | F | 321726 | | 3681 | F | 1175025 | | 5401 | B | 781481 |
| 1962 | F | 325117 | | 3682 | F | 1178912 | | 5402 | B | 780503 |
| 1963 | F | 323211 | | 3683 | F | 1176985 | | 5403 | B | 782380 |
| 1964 | F | 326213 | | 3684 | F | 1179826 | | 5404 | B | 781419 |
| 1965 | F | 324254 | | 3685 | F | 1177910 | | 5405 | B | 783311 |
| 1966 | F | 327607 | | 3686 | F | 1180498 | | 5406 | B | 781747 |
| 1967 | F | 325695 | | 3687 | F | 1178666 | | 5407 | B | 783680 |
| 1968 | F | 328162 | | 3688 | F | 1181716 | | 5408 | B | 783004 |
| 1969 | F | 326262 | | 3689 | F | 1179839 | | 5409 | B | 784912 |
| 1970 | F | 328630 | | 3690 | F | 1182069 | | 5410 | B | 783820 |
| 1971 | F | 326723 | | 3691 | F | 1180140 | | 5411 | B | 785752 |
| 1972 | F | 329134 | | 3692 | F | 1183626 | | 5412 | B | 785255 |
| 1973 | F | 327178 | | 3693 | F | 1181716 | | 5413 | B | 787155 |
| 1974 | F | 330734 | | 3694 | F | 1184128 | | 5414 | B | 786655 |
| 1975 | F | 328810 | | 3695 | F | 1182244 | | 5415 | B | 788572 |
| 1976 | F | 332123 | | 3696 | F | 1185004 | | 5416 | B | 788671 |
| 1977 | F | 330252 | | 3697 | F | 1183084 | | 5417 | B | 790554 |
| 1978 | F | 334575 | | 3698 | F | 1185897 | | 5418 | B | 789164 |
| 1979 | F | 332660 | | 3699 | F | 1184029 | | 5419 | B | 791064 |
| 1980 | F | 335884 | | 3700 | F | 1187151 | | 5420 | B | 790001 |
| 1981 | F | 333980 | | 3701 | F | 1185251 | | 5421 | B | 791900 |
| 1982 | F | 337129 | | 3702 | F | 1186262 | | 5422 | B | 791734 |
| 1983 | F | 335202 | | 3703 | F | 1184361 | | 5423 | B | 793679 |
| 1984 | F | 337910 | | 3704 | F | 1189054 | | 5424 | B | 792944 |
| 1985 | F | 335955 | | 3705 | F | 1187160 | | 5425 | B | 794875 |
| 1986 | F | 338746 | | 3706 | F | 1190885 | | 5426 | B | 793809 |
| 1987 | F | 336795 | | 3707 | F | 1188990 | | 5427 | B | 795692 |
| 1988 | F | 339217 | | 3708 | F | 1191507 | | 5428 | B | 794580 |
| 1989 | F | 337362 | | 3709 | F | 1189579 | | 5429 | B | 796450 |
| 1990 | F | 339999 | | 3710 | F | 1191932 | | 5430 | B | 795066 |
| 1991 | F | 338083 | | 3711 | F | 1190008 | | 5431 | B | 796966 |
| 1992 | F | 343144 | | 3712 | F | 1192524 | | 5432 | B | 795956 |
| 1993 | F | 341266 | | 3713 | F | 1190640 | | 5433 | B | 797855 |
| 1994 | F | 343699 | | 3714 | F | 1192759 | | 5434 | B | 797018 |
| 1995 | F | 341813 | | 3715 | F | 1190869 | | 5435 | B | 798918 |
| 1996 | F | 344108 | | 3716 | F | 1193642 | | 5436 | B | 798989 |
| 1997 | F | 342204 | | 3717 | F | 1191742 | | 5437 | B | 800875 |
| 1998 | F | 344851 | | 3718 | F | 1193557 | | 5438 | B | 800069 |
| 1999 | F | 342933 | | 3719 | F | 1191657 | | 5439 | B | 801944 |
| 2000 | F | 346148 | | 3720 | F | 1194015 | | 5440 | B | 799840 |
| 2001 | F | 344219 | | 3721 | F | 1192120 | | 5441 | B | 801701 |
| 2002 | F | 346493 | | 3722 | F | 1195490 | | 5442 | B | 801533 |
| 2003 | F | 344590 | | 3723 | F | 1193560 | | 5443 | B | 803445 |
| 2004 | F | 346815 | | 3724 | F | 1196093 | | 5444 | B | 802717 |
| 2005 | F | 344907 | | 3725 | F | 1194215 | | 5445 | B | 804581 |
| 2006 | F | 347836 | | 3726 | F | 1196474 | | 5446 | B | 803559 |
| 2007 | F | 345956 | | 3727 | F | 1194592 | | 5447 | B | 805419 |
| 2008 | F | 350379 | | 3728 | F | 1197659 | | 5448 | B | 804032 |
| 2009 | F | 348432 | | 3729 | F | 1195724 | | 5449 | B | 805931 |
| 2010 | F | 350856 | | 3730 | F | 1198499 | | 5450 | B | 805383 |
| 2011 | F | 348951 | | 3731 | F | 1196578 | | 5451 | B | 807291 |
| 2012 | F | 352008 | | 3732 | F | 1199912 | | 5452 | B | 806107 |
| 2013 | F | 350106 | | 3733 | F | 1197986 | | 5453 | B | 807988 |
| 2014 | F | 353209 | | 3734 | F | 1200969 | | 5454 | B | 806533 |
| 2015 | F | 351305 | | 3735 | F | 1199133 | | 5455 | B | 808430 |
| 2016 | F | 354224 | | 3736 | F | 1202121 | | 5456 | B | 806954 |
| 2017 | F | 352312 | | 3737 | F | 1200227 | | 5457 | B | 808724 |
| 2018 | F | 354781 | | 3738 | F | 1202957 | | 5458 | B | 807133 |
| 2019 | F | 352871 | | 3739 | F | 1201058 | | 5459 | B | 809033 |
| 2020 | F | 355223 | | 3740 | F | 1202590 | | 5460 | B | 808442 |
| 2021 | F | 353261 | | 3741 | F | 1200694 | | 5461 | B | 810357 |
| 2022 | F | 355393 | | 3742 | F | 1203923 | | 5462 | B | 808972 |
| 2023 | F | 353519 | | 3743 | F | 1202049 | | 5463 | B | 810896 |
| 2024 | F | 358901 | | 3744 | F | 1204631 | | 5464 | B | 809674 |
| 2025 | F | 357001 | | 3745 | F | 1202753 | | 5465 | B | 811557 |
| 2026 | F | 356594 | | 3746 | F | 1205864 | | 5466 | B | 810192 |
| 2027 | F | 354692 | | 3747 | F | 1203964 | | 5467 | B | 812105 |
| 2028 | F | 359240 | | 3748 | F | 1206483 | | 5468 | B | 811472 |
| 2029 | F | 357374 | | 3749 | F | 1204592 | | 5469 | B | 813357 |
| 2030 | F | 359721 | | 3750 | F | 1207629 | | 5470 | B | 813325 |
| 2031 | F | 357763 | | 3751 | F | 1205727 | | 5471 | B | 815179 |
| 2032 | F | 361071 | | 3752 | F | 1208802 | | 5472 | B | 813133 |
| 2033 | F | 359240 | | 3753 | F | 1206909 | | 5473 | B | 815134 |
| 2034 | F | 363605 | | 3754 | F | 1209500 | | 5474 | B | 813808 |
| 2035 | F | 361731 | | 3755 | F | 1207557 | | 5475 | B | 815737 |
| 2036 | F | 364142 | | 3756 | F | 1210483 | | 5476 | B | 815246 |
| 2037 | F | 362246 | | 3757 | F | 1208584 | | 5477 | B | 817168 |
| 2038 | F | 364567 | | 3758 | F | 1211618 | | 5478 | B | 815995 |
| 2039 | F | 362708 | | 3759 | F | 1209745 | | 5479 | B | 817892 |
| 2040 | F | 365039 | | 3760 | F | 1212523 | | 5480 | B | 817264 |
| 2041 | F | 363184 | | 3761 | F | 1210554 | | 5481 | B | 819164 |
| 2042 | F | 365445 | | 3762 | F | 1213827 | | 5482 | B | 817579 |
| 2043 | F | 363517 | | 3763 | F | 1211927 | | 5483 | B | 819491 |
| 2044 | F | 367040 | | 3764 | F | 1214875 | | 5484 | B | 818890 |
| 2045 | F | 365144 | | 3765 | F | 1212992 | | 5485 | B | 820733 |
| 2046 | F | 368825 | | 3766 | F | 1215293 | | 5486 | B | 819332 |
| 2047 | F | 366993 | | 3767 | F | 1213430 | | 5487 | B | 821217 |
| 2048 | F | 369698 | | 3768 | F | 1216043 | | 5488 | B | 820096 |
| 2049 | F | 367760 | | 3769 | F | 1214183 | | 5489 | B | 821951 |
| 2050 | F | 370141 | | 3770 | F | 1216226 | | 5490 | B | 820945 |
| 2051 | F | 368239 | | 3771 | F | 1214374 | | 5491 | B | 822870 |
| 2052 | F | 372329 | | 3772 | F | 1216927 | | 5492 | B | 821151 |
| 2053 | F | 370375 | | 3773 | F | 1215064 | | 5493 | B | 823079 |
| 2054 | F | 372779 | | 3774 | F | 1219490 | | 5494 | B | 822558 |
| 2055 | F | 370881 | | 3775 | F | 1217534 | | 5495 | B | 824449 |
| 2056 | F | 373223 | | 3776 | F | 1219431 | | 5496 | B | 823767 |
| 2057 | F | 371342 | | 3777 | F | 1217534 | | 5497 | B | 825634 |
| 2058 | F | 373939 | | 3778 | F | 1220403 | | 5498 | B | 825876 |
| 2059 | F | 372017 | | 3779 | F | 1218475 | | 5499 | B | 827737 |
| 2060 | F | 374849 | | 3780 | F | 1221383 | | 5500 | B | 826583 |
| 2061 | F | 372953 | | 3781 | F | 1219499 | | 5501 | B | 828435 |
| 2062 | F | 375351 | | 3782 | F | 1223653 | | 5502 | B | 827511 |
| 2063 | F | 373487 | | 3783 | F | 1221767 | | 5503 | B | 829428 |
| 2064 | F | 376316 | | 3784 | F | 1224758 | | 5504 | B | 828829 |
| 2065 | F | 374416 | | 3785 | F | 1222881 | | 5505 | B | 830729 |
| 2066 | F | 377737 | | 3786 | F | 1226308 | | 5506 | B | 830262 |
| 2067 | F | 375828 | | 3787 | F | 1224409 | | 5507 | B | 832158 |
| 2068 | F | 379537 | | 3788 | F | 1225625 | | 5508 | B | 831286 |
| 2069 | F | 377660 | | 3789 | F | 1223654 | | 5509 | B | 833182 |
| 2070 | F | 380033 | | 3790 | F | 1227566 | | 5510 | B | 831946 |
| 2071 | F | 378160 | | 3791 | F | 1225677 | | 5511 | B | 833848 |
| 2072 | F | 380789 | | 3792 | F | 1227858 | | 5512 | B | 833372 |
| 2073 | F | 378889 | | 3793 | F | 1225937 | | 5513 | B | 835267 |
| 2074 | F | 381238 | | 3794 | F | 1228081 | | 5514 | B | 834125 |
| 2075 | F | 379279 | | 3795 | F | 1226189 | | 5515 | B | 835992 |
| 2076 | F | 382969 | | 3796 | B | 1019 | | 5516 | B | 835267 |
| 2077 | F | 381124 | | 3797 | B | 2954 | | 5517 | B | 837193 |
| 2078 | F | 383293 | | 3798 | B | 1843 | | 5518 | B | 836111 |
| 2079 | F | 381425 | | 3799 | B | 3739 | | 5519 | B | 837952 |
| 2080 | F | 385178 | | 3800 | B | 2694 | | 5520 | B | 837844 |
| 2081 | F | 383278 | | 3801 | B | 4545 | | 5521 | B | 839751 |
| 2082 | F | 386271 | | 3802 | B | 3694 | | 5522 | B | 839381 |
| 2083 | F | 384392 | | 3803 | B | 5513 | | 5523 | B | 841221 |
| 2084 | F | 386780 | | 3804 | B | 4290 | | 5524 | B | 841127 |
| 2085 | F | 384891 | | 3805 | B | 6238 | | 5525 | B | 843073 |
| 2086 | F | 389383 | | 3806 | B | 5924 | | 5526 | B | 842409 |
| 2087 | F | 387504 | | 3807 | B | 7846 | | 5527 | B | 844323 |
| 2088 | F | 389901 | | 3808 | B | 7687 | | 5528 | B | 843691 |
| 2089 | F | 388001 | | 3809 | B | 9583 | | 5529 | B | 845602 |
| 2090 | F | 390700 | | 3810 | B | 9189 | | 5530 | B | 844244 |
| 2091 | F | 388732 | | 3811 | B | 11095 | | 5531 | B | 846153 |
| 2092 | F | 391612 | | 3812 | B | 10261 | | 5532 | B | 845319 |
| 2093 | F | 389763 | | 3813 | B | 12119 | | 5533 | B | 847139 |
| 2094 | F | 392346 | | 3814 | B | 10982 | | 5534 | B | 846411 |
| 2095 | F | 390463 | | 3815 | B | 12839 | | 5535 | B | 848300 |
| 2096 | F | 392540 | | 3816 | B | 11463 | | 5536 | B | 848760 |
| 2097 | F | 390639 | | 3817 | B | 13355 | | 5537 | B | 850653 |
| 2098 | F | 393487 | | 3818 | B | 12950 | | 5538 | B | 849242 |
| 2099 | F | 391609 | | 3819 | B | 14850 | | 5539 | B | 851174 |
| 2100 | F | 393904 | | 3820 | B | 14425 | | 5540 | B | 850753 |
| 2101 | F | 392025 | | 3821 | B | 16332 | | 5541 | B | 852649 |
| 2102 | F | 394703 | | 3822 | B | 17477 | | 5542 | B | 851795 |
| 2103 | F | 392782 | | 3823 | B | 19400 | | 5543 | B | 853690 |
| 2104 | F | 395024 | | 3824 | B | 16296 | | 5544 | B | 852696 |
| 2105 | F | 393098 | | 3825 | B | 18161 | | 5545 | B | 854596 |
| 2106 | F | 395705 | | 3826 | B | 21128 | | 5546 | B | 853938 |
| 2107 | F | 393791 | | 3827 | B | 22976 | | 5547 | B | 855846 |
| 2108 | F | 397607 | | 3828 | B | 22265 | | 5548 | B | 855338 |
| 2109 | F | 395705 | | 3829 | B | 24185 | | 5549 | B | 857240 |
| 2110 | F | 398807 | | 3830 | B | 23701 | | 5550 | B | 855982 |
| 2111 | F | 396957 | | 3831 | B | 25599 | | 5551 | B | 857873 |
| 2112 | F | 399848 | | 3832 | B | 26350 | | 5552 | B | 856786 |
| 2113 | F | 397886 | | 3833 | B | 28258 | | 5553 | B | 858722 |
| 2114 | F | 400914 | | 3834 | B | 26350 | | 5554 | B | 858783 |
| 2115 | F | 399008 | | 3835 | B | 28258 | | 5555 | B | 860735 |
| 2116 | F | 401183 | | 3836 | B | 27241 | | 5556 | B | 859824 |
| 2117 | F | 399301 | | 3837 | B | 29113 | | 5557 | B | 861787 |
| 2118 | F | 401964 | | 3838 | B | 27977 | | 5558 | B | 860442 |
| 2119 | F | 400060 | | 3839 | B | 29896 | | 5559 | B | 862329 |
| 2120 | F | 403450 | | 3840 | B | 28804 | | 5560 | B | 861415 |
| 2121 | F | 401527 | | 3841 | B | 30700 | | 5561 | B | 863252 |
| 2122 | F | 404124 | | 3842 | B | 29727 | | 5562 | B | 861677 |
| 2123 | F | 402206 | | 3843 | B | 31642 | | 5563 | B | 863558 |
| 2124 | F | 405765 | | 3844 | B | 30253 | | 5564 | B | 863171 |
| 2125 | F | 403865 | | 3845 | B | 32158 | | 5565 | B | 865099 |
| 2126 | F | 407131 | | 3846 | B | 31775 | | 5566 | B | 865021 |
| 2127 | F | 405243 | | 3847 | B | 33657 | | 5567 | B | 866922 |
| 2128 | F | 407456 | | 3848 | B | 32511 | | 5568 | B | 865497 |
| 2129 | F | 405563 | | 3849 | B | 34422 | | 5569 | B | 867408 |
| 2130 | F | 408841 | | 3850 | B | 34214 | | 5570 | B | 866808 |
| 2131 | F | 406901 | | 3851 | B | 36114 | | 5571 | B | 868732 |
| 2132 | F | 410478 | | 3852 | B | 34765 | | 5572 | B | 867342 |
| 2133 | F | 408573 | | 3853 | B | 36664 | | 5573 | B | 869242 |
| 2134 | F | 410725 | | 3854 | B | 36289 | | 5574 | B | 868064 |
| 2135 | F | 408832 | | 3855 | B | 38186 | | 5575 | B | 869974 |
| 2136 | F | 412263 | | 3856 | B | 37759 | | 5576 | B | 868732 |
| 2137 | F | 410363 | | 3857 | B | 39682 | | 5577 | B | 870664 |
| 2138 | F | 414168 | | 3858 | B | 39585 | | 5578 | B | 869974 |
| 2139 | F | 412268 | | 3859 | B | 41496 | | 5579 | B | 871880 |
| 2140 | F | 415013 | | 3860 | B | 40942 | | 5580 | B | 870857 |
| 2141 | F | 413111 | | 3861 | B | 42840 | | 5581 | B | 872753 |
| 2142 | F | 415636 | | 3862 | B | 39640 | | 5582 | B | 872149 |
| 2143 | F | 413743 | | 3863 | B | 41543 | | 5583 | B | 874087 |
| 2144 | F | 417033 | | 3864 | B | 43329 | | 5584 | B | 872758 |
| 2145 | F | 415114 | | 3865 | B | 45196 | | 5585 | B | 874658 |
| 2146 | F | 417163 | | 3866 | B | 44025 | | 5586 | B | 874131 |
| 2147 | F | 415332 | | 3867 | B | 45979 | | 5587 | B | 876122 |
| 2148 | F | 418166 | | 3868 | B | 45048 | | 5588 | B | 874903 |
| 2149 | F | 416265 | | 3869 | B | 46970 | | 5589 | B | 876793 |
| 2150 | F | 420186 | | 3870 | B | 45582 | | 5590 | B | 875548 |
| 2151 | F | 418259 | | 3871 | B | 47472 | | 5591 | B | 877437 |
| 2152 | F | 420697 | | 3872 | B | 45979 | | 5592 | B | 878078 |
| 2153 | F | 418861 | | 3873 | B | 47901 | | 5593 | B | 880011 |
| 2154 | F | 421313 | | 3874 | B | 47216 | | 5594 | B | 879478 |
| 2155 | F | 419437 | | 3875 | B | 49128 | | 5595 | B | 881385 |
| 2156 | F | 422172 | | 3876 | B | 47791 | | 5596 | B | 880874 |
| 2157 | F | 420342 | | 3877 | B | 49689 | | 5597 | B | 882771 |
| 2158 | F | 423342 | | 3878 | B | 48196 | | 5598 | B | 882771 |
| 2159 | F | 421412 | | 3879 | B | 50126 | | 5599 | B | 884644 |
| 2160 | F | 424008 | | 3880 | B | 49180 | | 5600 | B | 883542 |
| 2161 | F | 422073 | | 3881 | B | 51105 | | 5601 | B | 885447 |
| 2162 | F | 424585 | | 3882 | B | 50231 | | 5602 | B | 883777 |
| 2163 | F | 422711 | | 3883 | B | 52149 | | 5603 | B | 885689 |
| 2164 | F | 426021 | | 3884 | B | 51697 | | 5604 | B | 884430 |
| 2165 | F | 424107 | | 3885 | B | 53619 | | 5605 | B | 886335 |
| 2166 | F | 427407 | | 3886 | B | 52917 | | 5606 | B | 885834 |
| 2167 | F | 425513 | | 3887 | B | 54735 | | 5607 | B | 887782 |
| 2168 | F | 427936 | | 3888 | B | 53619 | | 5608 | B | 887528 |
| 2169 | F | 426053 | | 3889 | B | 55476 | | 5609 | B | 889442 |
| 2170 | F | 428592 | | 3890 | B | 53910 | | 5610 | B | 888432 |
| 2171 | F | 426717 | | 3891 | B | 55816 | | 5611 | B | 890292 |
| 2172 | F | 430475 | | 3892 | B | 54416 | | 5612 | B | 888879 |
| 2173 | F | 428558 | | 3893 | B | 56326 | | 5613 | B | 890775 |
| 2174 | F | 431378 | | 3894 | B | 55107 | | 5614 | B | 889595 |
| 2175 | F | 429417 | | 3895 | B | 57009 | | 5615 | B | 891481 |
| 2176 | F | 431927 | | 3896 | B | 56693 | | 5616 | B | 890119 |
| 2177 | F | 430046 | | 3897 | B | 58586 | | 5617 | B | 892034 |
| 2178 | F | 432609 | | 3898 | B | 57489 | | 5618 | B | 891428 |
| 2179 | F | 430710 | | 3899 | B | 59394 | | 5619 | B | 893320 |
| 2180 | F | 433005 | | 3900 | B | 58749 | | 5620 | B | 892050 |
| 2181 | F | 431082 | | 3901 | B | 60649 | | 5621 | B | 893950 |
| 2182 | F | 433712 | | 3902 | B | 60086 | | 5622 | B | 892259 |
| 2183 | F | 431812 | | 3903 | B | 62002 | | 5623 | B | 894158 |
| 2184 | F | 436521 | | 3904 | B | 62375 | | 5624 | B | 892701 |
| 2185 | F | 434640 | | 3905 | B | 64275 | | 5625 | B | 894611 |
| 2186 | F | 436897 | | 3906 | B | 61715 | | 5626 | B | 893194 |
| 2187 | F | 435057 | | 3907 | B | 63633 | | 5627 | B | 895056 |
| 2188 | F | 439741 | | 3908 | B | 62699 | | 5628 | B | 893347 |
| 2189 | F | 437882 | | 3909 | B | 64601 | | 5629 | B | 895263 |
| 2190 | F | 438296 | | 3910 | B | 63981 | | 5630 | B | 893787 |
| 2191 | F | 436377 | | 3911 | B | 65858 | | 5631 | B | 895711 |
| 2192 | F | 440475 | | 3912 | B | 64268 | | 5632 | B | 895642 |
| 2193 | F | 438538 | | 3913 | B | 66227 | | 5633 | B | 897542 |
| 2194 | F | 440281 | | 3914 | B | 64423 | | 5634 | B | 896759 |
| 2195 | F | 438394 | | 3915 | B | 66309 | | 5635 | B | 898650 |
| 2196 | F | 440989 | | 3916 | B | 64834 | | 5636 | B | 897802 |
| 2197 | F | 439080 | | 3917 | B | 66756 | | 5637 | B | 899694 |
| 2198 | F | 442121 | | 3918 | B | 65705 | | 5638 | B | 899665 |
| 2199 | F | 440252 | | 3919 | B | 67611 | | 5639 | B | 901565 |
| 2200 | F | 442121 | | 3920 | B | 66228 | | 5640 | B | 900460 |
| 2201 | F | 440221 | | 3921 | B | 68163 | | 5641 | B | 902360 |
| 2202 | F | 442780 | | 3922 | B | 67538 | | 5642 | B | 903450 |
| 2203 | F | 440879 | | 3923 | B | 69404 | | 5643 | B | 905354 |
| 2204 | F | 443285 | | 3924 | B | 67961 | | 5644 | B | 905307 |
| 2205 | F | 441384 | | 3925 | B | 69841 | | 5645 | B | 907291 |
| 2206 | F | 444276 | | 3926 | B | 68796 | | 5646 | B | 907290 |
| 2207 | F | 442406 | | 3927 | B | 70662 | | 5647 | B | 909083 |
| 2208 | F | 444472 | | 3928 | B | 70984 | | 5648 | B ' | 908055 |
| 2209 | F | 442568 | | 3929 | B | 72885 | | 5649 | B | 909955 |
| 2210 | F | 444960 | | 3930 | B | 69392 | | 5650 | B | 908358 |
| 2211 | F | 443040 | | 3931 | B | 71314 | | 5651 | B | 910273 |
| 2212 | F | 445556 | | 3932 | B | 71365 | | 5652 | B | 908900 |
| 2213 | F | 443681 | | 3933 | B | 73287 | | 5653 | B | 910831 |
| 2214 | F | 447565 | | 3934 | B | 72253 | | 5654 | B | 909607 |
| 2215 | F | 445676 | | 3935 | B | 74167 | | 5655 | B | 911450 |
| 2216 | F | 448396 | | 3936 | B | 73916 | | 5656 | B | 911760 |
| 2217 | F | 446496 | | 3937 | B | 75760 | | 5657 | B | 913589 |
| 2218 | F | 450057 | | 3938 | B | 76398 | | 5658 | B | 912584 |
| 2219 | F | 448133 | | 3939 | B | 78328 | | 5659 | B | 914529 |
| 2220 | F | 450444 | | 3940 | B | 77734 | | 5660 | B | 913054 |
| 2221 | F | 448555 | | 3941 | B | 79610 | | 5661 | B | 914956 |
| 2222 | F | 450988 | | 3942 | B | 78592 | | 5662 | B | 914208 |
| 2223 | F | 449054 | | 3943 | B | 80517 | | 5663 | B | 916113 |
| 2224 | F | 452212 | | 3944 | B | 79577 | | 5664 | B | 915388 |
| 2225 | F | 450329 | | 3945 | B | 81476 | | 5665 | B | 917272 |
| 2226 | F | 453450 | | 3946 | B | 79968 | | 5666 | B | 915880 |
| 2227 | F | 451581 | | 3947 | B | 81861 | | 5667 | B | 917747 |
| 2228 | F | 454643 | | 3948 | B | 80203 | | 5668 | B | 916886 |
| 2229 | F | 452718 | | 3949 | B | 82108 | | 5669 | B | 918778 |
| 2230 | F | 456004 | | 3950 | B | 80665 | | 5670 | B | 917940 |
| 2231 | F | 454124 | | 3951 | B | 82565 | | 5671 | B | 919827 |
| 2232 | F | 456785 | | 3952 | B | 81257 | | 5672 | B | 919070 |
| 2233 | F | 454897 | | 3953 | B | 83184 | | 5673 | B | 920972 |
| 2234 | F | 457749 | | 3954 | B | 83370 | | 5674 | B | 920107 |
| 2235 | F | 455856 | | 3955 | B | 85203 | | 5675 | B | 922088 |
| 2236 | F | 458132 | | 3956 | B | 84202 | | 5676 | B | 920666 |
| 2237 | F | 456205 | | 3957 | B | 86080 | | 5677 | B | 922554 |
| 2238 | F | 459216 | | 3958 | B | 85032 | | 5678 | B | 921412 |
| 2239 | F | 457348 | | 3959 | B | 86902 | | 5679 | B | 923307 |
| 2240 | F | 460692 | | 3960 | B | 85520 | | 5680 | B | 922216 |
| 2241 | F | 458792 | | 3961 | B | 87367 | | 5681 | B | 924104 |
| 2242 | F | 460133 | | 3962 | B | 85648 | | 5682 | B | 922661 |
| 2243 | F | 458230 | | 3963 | B | 87548 | | 5683 | B | 924538 |
| 2244 | F | 461228 | | 3964 | B | 86155 | | 5684 | B | 924024 |
| 2245 | F | 459327 | | 3965 | B | 88052 | | 5685 | B | 925893 |
| 2246 | F | 462183 | | 3966 | B | 86806 | | 5686 | B | 924192 |
| 2247 | F | 460269 | | 3967 | B | 88768 | | 5687 | B | 926063 |
| 2248 | F | 463120 | | 3968 | B | 88389 | | 5688 | B | 925245 |
| 2249 | F | 461220 | | 3969 | B | 90207 | | 5689 | B | 927137 |
| 2250 | F | 464355 | | 3970 | B | 89174 | | 5690 | B | 925672 |
| 2251 | F | 462444 | | 3971 | B | 91107 | | 5691 | B | 927558 |
| 2252 | F | 464842 | | 3972 | B | 91319 | | 5692 | B | 926744 |
| 2253 | F | 463010 | | 3973 | B | 93151 | | 5693 | B | 928659 |
| 2254 | F | 465346 | | 3974 | B | 93306 | | 5694 | B | 928169 |
| 2255 | F | 463451 | | 3975 | B | 95184 | | 5695 | B | 930064 |
| 2256 | F | 466061 | | 3976 | B | 94311 | | 5696 | B | 928543 |
| 2257 | F | 464143 | | 3977 | B | 96210 | | 5697 | B | 930439 |
| 2258 | F | 466780 | | 3978 | B | 94761 | | 5698 | B | 929238 |
| 2259 | F | 464842 | | 3979 | B | 96578 | | 5699 | B | 931109 |
| 2260 | F | 467462 | | 3980 | B | 95640 | | 5700 | B | 931227 |
| 2261 | F | 465578 | | 3981 | B | 97452 | | 5701 | B | 933127 |
| 2262 | F | 469419 | | 3982 | B | 96835 | | 5702 | B | 932291 |
| 2263 | F | 467538 | | 3983 | B | 98743 | | 5703 | B | 934184 |
| 2264 | F | 471324 | | 3984 | B | 97685 | | 5704 | B | 933738 |
| 2265 | F | 469419 | | 3985 | B | 99639 | | 5705 | B | 935651 |
| 2266 | F | 470463 | | 3986 | B | 98655 | | 5706 | B | 933127 |
| 2267 | F | 468587 | | 3987 | B | 100585 | | 5707 | B | 935001 |
| 2268 | F | 471822 | | 3988 | B | 99680 | | 5708 | B | 935969 |
| 2269 | F | 469897 | | 3989 | B | 101592 | | 5709 | B | 937869 |
| 2270 | F | 472471 | | 3990 | B | 101592 | | 5710 | B | 937305 |
| 2271 | F | 470610 | | 3991 | B | 103448 | | 5711 | B | 939223 |
| 2272 | F | 473208 | | 3992 | B | 101950 | | 5712 | B | 937448 |
| 2273 | F | 471319 | | 3993 | B | 103878 | | 5713 | B | 939423 |
| 2274 | F | 475143 | | 3994 | B | 102534 | | 5714 | B | 938633 |
| 2275 | F | 473243 | | 3995 | B | 104467 | | 5715 | B | 940533 |
| 2276 | F | 477091 | | 3996 | B | 103031 | | 5716 | B | 939032 |
| 2277 | F | 475181 | | 3997 | B | 104947 | | 5717 | B | 940928 |
| 2278 | F | 477375 | | 3998 | B | 103754 | | 5718 | B | 939478 |
| 2279 | F | 475475 | | 3999 | B | 105653 | | 5719 | B | 941392 |
| 2280 | F | 478473 | | 4000 | B | 104281 | | 5720 | B | 940021 |
| 2281 | F | 476586 | | 4001 | B | 106192 | | 5721 | B | 941918 |
| 2282 | F | 479058 | | 4002 | B | 104786 | | 5722 | B | 941017 |
| 2283 | F | 477158 | | 4003 | B | 106618 | | 5723 | B | 942925 |
| 2284 | F | 479829 | | 4004 | B | 108635 | | 5724 | B | 941392 |
| 2285 | F | 477916 | | 4005 | B | 110512 | | 5725 | B | 943238 |
| 2286 | F | 481237 | | 4006 | B | 112299 | | 5726 | B | 941586 |
| 2287 | F | 479312 | | 4007 | B | 114196 | | 5727 | B | 943496 |
| 2288 | F | 481769 | | 4008 | B | 112839 | | 5728 | B | 942787 |
| 2289 | F | 479903 | | 4009 | B | 114713 | | 5729 | B | 944657 |
| 2290 | F | 482435 | | 4010 | B | 113960 | | 5730 | B | 943043 |
| 2291 | F | 480535 | | 4011 | B | 115829 | | 5731 | B | 944971 |
| 2292 | F | 483976 | | 4012 | B | 114352 | | 5732 | B | 943404 |
| 2293 | F | 482075 | | 4013 | B | 116272 | | 5733 | B | 945286 |
| 2294 | F | 484899 | | 4014 | B | 114932 | | 5734 | B | 944025 |
| 2295 | F | 483029 | | 4015 | B | 116831 | | 5735 | B | 945981 |
| 2296 | F | 485593 | | 4016 | B | 116002 | | 5736 | B | 944302 |
| 2297 | F | 483674 | | 4017 | B | 117886 | | 5737 | B | 946175 |
| 2298 | F | 486401 | | 4018 | B | 116781 | | 5738 | B | 944654 |
| 2299 | F | 484498 | | 4019 | B | 118702 | | 5739 | B | 946533 |
| 2300 | F | 486762 | | 4020 | B | 118284 | | 5740 | B | 945633 |
| 2301 | F | 484859 | | 4021 | B | 120181 | | 5741 | B | 947515 |
| 2302 | F | 487287 | | 4022 | B | 118749 | | 5742 | B | 946073 |
| 2303 | F | 485366 | | 4023 | B | 120691 | | 5743 | B | 947974 |
| 2304 | F | 487487 | | 4024 | B | 120124 | | 5744 | B | 946645 |
| 2305 | F | 485642 | | 4025 | B | 122009 | | 5745 | B | 948517 |
| 2306 | F | 488811 | | 4026 | B | 120691 | | 5746 | B | 947646 |
| 2307 | F | 486942 | | 4027 | B | 122601 | | 5747 | B | 949545 |
| 2308 | F | 488918 | | 4028 | B | 122655 | | 5748 | B | 948344 |
| 2309 | F | 487001 | | 4029 | B | 124563 | | 5749 | B | 950219 |
| 2310 | F | 489740 | | 4030 | B | 123173 | | 5750 | B | 950104 |
| 2311 | F | 487772 | | 4031 | B | 125141 | | 5751 | B | 952004 |
| 2312 | F | 490300 | | 4032 | B | 123579 | | 5752 | B | 951301 |
| 2313 | F | 488400 | | 4033 | B | 125526 | | 5753 | B | 953207 |
| 2314 | F | 490880 | | 4034 | B | 126570 | | 5754 | B | 951505 |
| 2315 | F | 488969 | | 4035 | B | 128539 | | 5755 | B | 953387 |
| 2316 | F | 491167 | | 4036 | B | 129398 | | 5756 | B | 952382 |
| 2317 | F | 489268 | | 4037 | B | 131325 | | 5757 | B | 954257 |
| 2318 | F | 492066 | | 4038 | B | 134942 | | 5758 | B | 952927 |
| 2319 | F | 490096 | | 4039 | B | 136814 | | 5759 | B | 954794 |
| 2320 | F | 494600 | | 4040 | B | 136628 | | 5760 | B | 953711 |
| 2321 | F | 492697 | | 4041 | B | 138531 | | 5761 | B | 955611 |
| 2322 | F | 495778 | | 4042 | B | 138117 | | 5762 | B | 955556 |
| 2323 | F | 493845 | | 4043 | B | 139995 | | 5763 | B | 957444 |
| 2324 | F | 496350 | | 4044 | B | 138531 | | 5764 | B | 956049 |
| 2325 | F | 494396 | | 4045 | B | 140363 | | 5765 | B | 957977 |
| 2326 | F | 497139 | | 4046 | B | 138525 | | 5766 | B | 957358 |
| 2327 | F | 495210 | | 4047 | B | 140361 | | 5767 | B | 959202 |
| 2328 | F | 497504 | | 4048 | B | 139778 | | 5768 | B | 958136 |
| 2329 | F | 495651 | | 4049 | B | 141692 | | 5769 | B | 960022 |
| 2330 | F | 498216 | | 4050 | B | 140577 | | 5770 | B | 959490 |
| 2331 | F | 496381 | | 4051 | B | 142487 | | 5771 | B | 961374 |
| 2332 | F | 498990 | | 4052 | B | 142067 | | 5772 | B | 960507 |
| 2333 | F | 497076 | | 4053 | B | 143981 | | 5773 | B | 962439 |
| 2334 | F | 499284 | | 4054 | B | 142919 | | 5774 | B | 961892 |
| 2335 | F | 497401 | | 4055 | B | 144787 | | 5775 | B | 963792 |
| 2336 | F | 499563 | | 4056 | B | 144478 | | 5776 | B | 965000 |
| 2337 | F | 497644 | | 4057 | B | 146417 | | 5777 | B | 966954 |
| 2338 | F | 500555 | | 4058 | B | 145520 | | 5778 | B | 967076 |
| 2339 | F | 498645 | | 4059 | B | 147378 | | 5779 | B | 968975 |
| 2340 | F | 503868 | | 4060 | B | 146972 | | 5780 | B | 968474 |
| 2341 | F | 502008 | | 4061 | B | 148872 | | 5781 | B | 970326 |
| 2342 | F | 504574 | | 4062 | B | 147545 | | 5782 | B | 969039 |
| 2343 | F | 502741 | | 4063 | B | 149452 | | 5783 | B | 970930 |
| 2344 | F | 506571 | | 4064 | B | 147756 | | 5784 | B | 969718 |
| 2345 | F | 504671 | | 4065 | B | 149677 | | 5785 | B | 971619 |
| 2346 | F | 507498 | | 4066 | B | 148484 | | 5786 | B | 970080 |
| 2347 | F | 505565 | | 4067 | B | 150382 | | 5787 | B | 971991 |
| 2348 | F | 507615 | | 4068 | B | 152436 | | 5788 | B | 970371 |
| 2349 | F | 505777 | | 4069 | B | 154325 | | 5789 | B | 972257 |
| 2350 | F | 510441 | | 4070 | B | 154353 | | 5790 | B | 970832 |
| 2351 | F | 508522 | | 4071 | B | 156228 | | 5791 | B | 972738 |
| 2352 | F | 513523 | | 4072 | B | 155395 | | 5792 | B | 971481 |
| 2353 | F | 511660 | | 4073 | B | 157286 | | 5793 | B | 973403 |
| 2354 | F | 516834 | | 4074 | B | 155740 | | 5794 | B | 971909 |
| 2355 | F | 514938 | | 4075 | B | 157613 | | 5795 | B | 973810 |
| 2356 | F | 515101 | | 4076 | B | 157002 | | 5796 | B | 975372 |
| 2357 | F | 513277 | | 4077 | B | 158902 | | 5797 | B | 977234 |
| 2358 | F | 517031 | | 4078 | B | 157861 | | 5798 | B | 975634 |
| 2359 | F | 515093 | | 4079 | B | 159764 | | 5799 | B | 977548 |
| 2360 | F | 517620 | | 4080 | B | 159219 | | 5800 | B | 976739 |
| 2361 | F | 515698 | | 4081 | B | 161121 | | 5801 | B | 978639 |
| 2362 | F | 518070 | | 4082 | B | 159569 | | 5802 | B | 978543 |
| 2363 | F | 516181 | | 4083 | B | 161484 | | 5803 | B | 980448 |
| 2364 | F | 521162 | | 4084 | B | 160221 | | 5804 | B | 977907 |
| 2365 | F | 519241 | | 4085 | B | 162109 | | 5805 | B | 979832 |
| 2366 | F | 523023 | | 4086 | B | 160670 | | 5806 | B | 980997 |
| 2367 | F | 521123 | | 4087 | B | 162572 | | 5807 | B | 982862 |
| 2368 | F | 523865 | | 4088 | B | 161075 | | 5808 | B | 982167 |
| 2369 | F | 522003 | | 4089 | B | 162983 | | 5809 | B | 984051 |
| 2370 | F | 524373 | | 4090 | B | 161789 | | 5810 | B | 983206 |
| 2371 | F | 522530 | | 4091 | B | 163728 | | 5811 | B | 985082 |
| 2372 | F | 526029 | | 4092 | B | 162380 | | 5812 | B | 984344 |
| 2373 | F | 524115 | | 4093 | B | 164291 | | 5813 | B | 986279 |
| 2374 | F | 526479 | | 4094 | B | 162671 | | 5814 | B | 985741 |
| 2375 | F | 524580 | | 4095 | B | 164573 | | 5815 | B | 987653 |
| 2376 | F | 526756 | | 4096 | B | 164340 | | 5816 | B | 986106 |
| 2377 | F | 524823 | | 4097 | B | 166222 | | 5817 | B | 988045 |
| 2378 | F | 528167 | | 4098 | B | 165693 | | 5818 | B | 987667 |
| 2379 | F | 526263 | | 4099 | B | 167632 | | 5819 | B | 989585 |
| 2380 | F | 529315 | | 4100 | B | 166627 | | 5820 | B | 987418 |
| 2381 | F | 527408 | | 4101 | B | 168472 | | 5821 | B | 989315 |
| 2382 | F | 530372 | | 4102 | B | 168668 | | 5822 | B | 987936 |
| 2383 | F | 528484 | | 4103 | B | 170565 | | 5823 | B | 989842 |
| 2384 | F | 531842 | | 4104 | B | 169244 | | 5824 | B | 988447 |
| 2385 | F | 529945 | | 4105 | B | 171102 | | 5825 | B | 990355 |
| 2386 | F | 534077 | | 4106 | B | 169734 | | 5826 | B | 988979 |
| 2387 | F | 532190 | | 4107 | B | 171575 | | 5827 | B | 990875 |
| 2388 | F | 536335 | | 4108 | B | 171259 | | 5828 | B | 990066 |
| 2389 | F | 534585 | | 4109 | B | 173158 | | 5829 | B | 991966 |
| 2390 | F | 536858 | | 4110 | B | 171701 | | 5830 | B | 991268 |
| 2391 | F | 534931 | | 4111 | B | 173585 | | 5831 | B | 993171 |
| 2392 | F | 537710 | | 4112 | B | 172018 | | 5832 | B | 991858 |
| 2393 | F | 535810 | | 4113 | B | 173925 | | 5833 | B | 993763 |
| 2394 | F | 538105 | | 4114 | B | 172759 | | 5834 | B | 992722 |
| 2395 | F | 536211 | | 4115 | B | 174706 | | 5835 | B | 994621 |
| 2396 | F | 538901 | | 4116 | B | 173718 | | 5836 | B | 993082 |
| 2397 | F | 536979 | | 4117 | B | 175602 | | 5837 | B | 994988 |
| 2398 | F | 539360 | | 4118 | B | 174902 | | 5838 | B | 993290 |
| 2399 | F | 537421 | | 4119 | B | 176765 | | 5839 | B | 995230 |
| 2400 | F | 541059 | | 4120 | B | 175869 | | 5840 | B | 995015 |
| 2401 | F | 539160 | | 4121 | B | 177781 | | 5841 | B | 996927 |
| 2402 | F | 542198 | | 4122 | B | 176181 | | 5842 | B | 993839 |
| 2403 | F | 540335 | | 4123 | B | 178083 | | 5843 | B | 995750 |
| 2404 | F | 542650 | | 4124 | B | 177158 | | 5844 | B | 996203 |
| 2405 | F | 540840 | | 4125 | B | 179120 | | 5845 | B | 998090 |
| 2406 | F | 543589 | | 4126 | B | 177599 | | 5846 | B | 997094 |
| 2407 | F | 541677 | | 4127 | B | 179539 | | 5847 | B | 998977 |
| 2408 | F | 546376 | | 4128 | B | 177928 | | 5848 | B | 997835 |
| 2409 | F | 544486 | | 4129 | B | 179888 | | 5849 | B | 999728 |
| 2410 | F | 546731 | | 4130 | B | 179693 | | 5850 | B | 999224 |
| 2411 | F | 544872 | | 4131 | B | 181621 | | 5851 | B | 1001101 |
| 2412 | F | 549480 | | 4132 | B | 180070 | | 5852 | B | 1000267 |
| 2413 | F | 547547 | | 4133 | B | 181968 | | 5853 | B | 1002146 |
| 2414 | F | 550245 | | 4134 | B | 182017 | | 5854 | B | 1001594 |
| 2415 | F | 548328 | | 4135 | B | 183925 | | 5855 | B | 1003567 |
| 2416 | F | 551224 | | 4136 | B | 182865 | | 5856 | B | 1002100 |
| 2417 | F | 549328 | | 4137 | B | 184809 | | 5857 | B | 1003941 |
| 2418 | F | 552433 | | 4138 | B | 184640 | | 5858 | B | 1003571 |
| 2419 | F | 550520 | | 4139 | B | 186551 | | 5859 | B | 1005412 |
| 2420 | F | 554767 | | 4140 | B | 185253 | | 5860 | B | 1004381 |
| 2421 | F | 552882 | | 4141 | B | 187108 | | 5861 | B | 1006269 |
| 2422 | F | 555444 | | 4142 | B | 185703 | | 5862 | B | 1004753 |
| 2423 | F | 553541 | | 4143 | B | 187661 | | 5863 | B | 1006691 |
| 2424 | F | 557979 | | 4144 | B | 186129 | | 5864 | B | 1005890 |
| 2425 | F | 556089 | | 4145 | B | 188059 | | 5865 | B | 1007762 |
| 2426 | F | 557923 | | 4146 | B | 186395 | | 5866 | B | 1006199 |
| 2427 | F | 555988 | | 4147 | B | 188339 | | 5867 | B | 1008109 |
| 2428 | F | 561193 | | 4148 | B | 188056 | | 5868 | B | 1007050 |
| 2429 | F | 559292 | | 4149 | B | 189840 | | 5869 | B | 1008929 |
| 2430 | F | 559671 | | 4150 | B | 191218 | | 5870 | B | 1007819 |
| 2431 | F | 557777 | | 4151 | B | 193089 | | 5871 | B | 1009683 |
| 2432 | F | 561555 | | 4152 | B | 191880 | | 5872 | B | 1009446 |
| 2433 | F | 559655 | | 4153 | B | 193768 | | 5873 | B | 1011365 |
| 2434 | F | 563727 | | 4154 | B | 193026 | | 5874 | B | 1010314 |
| 2435 | F | 561828 | | 4155 | B | 194899 | | 5875 | B | 1012109 |
| 2436 | F | 564714 | | 4156 | B | 193709 | | 5876 | B | 1015234 |
| 2437 | F | 562803 | | 4157 | B | 195592 | | 5877 | B | 1017133 |
| 2438 | F | 566079 | | 4158 | B | 194284 | | 5878 | B | 1016571 |
| 2439 | F | 564180 | | 4159 | B | 196187 | | 5879 | B | 1018486 |
| 2440 | F | 567470 | | 4160 | B | 194284 | | 5880 | B | 1017755 |
| 2441 | F | 565569 | | 4161 | B | 196187 | | 5881 | B | 1019661 |
| 2442 | F | 568454 | | 4162 | B | 196032 | | 5882 | B | 1016781 |
| 2443 | F | 566609 | | 4163 | B | 197932 | | 5883 | B | 1018708 |
| 2444 | F | 569194 | | 4164 | B | 196298 | | 5884 | B | 1017022 |
| 2445 | F | 567291 | | 4165 | B | 198245 | | 5885 | B | 1018924 |
| 2446 | F | 570873 | | 4166 | B | 198296 | | 5886 | B | 1019233 |
| 2447 | F | 568996 | | 4167 | B | 200200 | | 5887 | B | 1021143 |
| 2448 | F | 571678 | | 4168 | B | 199677 | | 5888 | B | 1019674 |
| 2449 | F | 569809 | | 4169 | B | 201577 | | 5889 | B | 1021630 |
| 2450 | F | 571983 | | 4170 | B | 203050 | | 5890 | B | 1021020 |
| 2451 | F | 570083 | | 4171 | B | 204943 | | 5891 | B | 1022923 |
| 2452 | F | 571837 | | 4172 | B | 204776 | | 5892 | B | 1021630 |
| 2453 | F | 569998 | | 4173 | B | 206682 | | 5893 | B | 1023525 |
| 2454 | F | 572927 | | 4174 | B | 205877 | | 5894 | B | 1024510 |
| 2455 | F | 571022 | | 4175 | B | 207768 | | 5895 | B | 1026410 |
| 2456 | F | 574804 | | 4176 | B | 207568 | | 5896 | B | 1024936 |
| 2457 | F | 572868 | | 4177 | B | 209477 | | 5897 | B | 1026858 |
| 2458 | F | 576267 | | 4178 | B | 208009 | | 5898 | B | 1025836 |
| 2459 | F | 574354 | | 4179 | B | 209935 | | 5899 | B | 1027677 |
| 2460 | F | 577925 | | 4180 | B | 208490 | | 5900 | B | 1027197 |
| 2461 | F | 576082 | | 4181 | B | 210396 | | 5901 | B | 1029089 |
| 2462 | F | 578598 | | 4182 | B | 209832 | | 5902 | B | 1028022 |
| 2463 | F | 576721 | | 4183 | B | 211779 | | 5903 | B | 1029936 |
| 2464 | F | 579758 | | 4184 | B | 210948 | | 5904 | B | 1031445 |
| 2465 | F | 577878 | | 4185 | B | 212834 | | 5905 | B | 1033319 |
| 2466 | F | 579620 | | 4186 | B | 211360 | | 5906 | B | 1031943 |
| 2467 | F | 577731 | | 4187 | B | 213221 | | 5907 | B | 1033839 |
| 2468 | F | 579950 | | 4188 | B | 212036 | | 5908 | B | 1033277 |
| 2469 | F | 578022 | | 4189 | B | 213948 | | 5909 | B | 1035186 |
| 2470 | F | 581080 | | 4190 | B | 212409 | | 5910 | B | 1033697 |
| 2471 | F | 579248 | | 4191 | B | 214308 | | 5911 | B | 1035554 |
| 2472 | F | 581459 | | 4192 | B | 214299 | | 5912 | B | 1034009 |
| 2473 | F | 579555 | | 4193 | B | 216199 | | 5913 | B | 1035943 |
| 2474 | F | 582128 | | 4194 | B | 215173 | | 5914 | B | 1036282 |
| 2475 | F | 580221 | | 4195 | B | 217077 | | 5915 | B | 1038161 |
| 2476 | F | 583209 | | 4196 | B | 215689 | | 5916 | B | 1037178 |
| 2477 | F | 581305 | | 4197 | B | 217544 | | 5917 | B | 1039088 |
| 2478 | F | 584650 | | 4198 | B | 216374 | | 5918 | B | 1037902 |
| 2479 | F | 582828 | | 4199 | B | 218284 | | 5919 | B | 1039802 |
| 2480 | F | 585407 | | 4200 | B | 216932 | | 5920 | B | 1038167 |
| 2481 | F | 583467 | | 4201 | B | 218839 | | 5921 | B | 1040079 |
| 2482 | F | 586579 | | 4202 | B | 217507 | | 5922 | B | 1039198 |
| 2483 | F | 584650 | | 4203 | B | 219410 | | 5923 | B | 1041036 |
| 2484 | F | 587655 | | 4204 | B | 218089 | | 5924 | B | 1040803 |
| 2485 | F | 585772 | | 4205 | B | 220031 | | 5925 | B | 1042721 |
| 2486 | F | 587899 | | 4206 | B | 218491 | | 5926 | B | 1042560 |
| 2487 | F | 586058 | | 4207 | B | 220380 | | 5927 | B | 1044460 |
| 2488 | F | 589079 | | 4208 | B | 218839 | | 5928 | B | 1043630 |
| 2489 | F | 587173 | | 4209 | B | 220716 | | 5929 | B | 1045526 |
| 2490 | F | 590446 | | 4210 | B | 219152 | | 5930 | B | 1044850 |
| 2491 | F | 588616 | | 4211 | B | 221152 | | 5931 | B | 1046748 |
| 2492 | F | 592279 | | 4212 | B | 220125 | | 5932 | B | 1045609 |
| 2493 | F | 590407 | | 4213 | B | 221963 | | 5933 | B | 1047551 |
| 2494 | F | 592585 | | 4214 | B | 221602 | | 5934 | B | 1046761 |
| 2495 | F | 590716 | | 4215 | B | 223507 | | 5935 | B | 1048677 |
| 2496 | F | 593527 | | 4216 | B | 222939 | | 5936 | B | 1047741 |
| 2497 | F | 591593 | | 4217 | B | 224878 | | 5937 | B | 1049700 |
| 2498 | F | 594047 | | 4218 | B | 223791 | | 5938 | B | 1050218 |
| 2499 | F | 592210 | | 4219 | B | 225688 | | 5939 | B | 1052151 |
| 2500 | F | 595658 | | 4220 | B | 224019 | | 5940 | B | 1050831 |
| 2501 | F | 593758 | | 4221 | B | 225909 | | 5941 | B | 1052744 |
| 2502 | F | 596225 | | 4222 | B | 224491 | | 5942 | B | 1051223 |
| 2503 | F | 594387 | | 4223 | B | 226407 | | 5943 | B | 1053071 |
| 2504 | F | 596964 | | 4224 | B | 225279 | | 5944 | B | 1051974 |
| 2505 | F | 595006 | | 4225 | B | 227131 | | 5945 | B | 1053854 |
| 2506 | F | 597536 | | 4226 | B | 225798 | | 5946 | B | 1052287 |
| 2507 | F | 595635 | | 4227 | B | 227692 | | 5947 | B | 1054238 |
| 2508 | F | 598383 | | 4228 | B | 227030 | | 5948 | B | 1053379 |
| 2509 | F | 596448 | | 4229 | B | 228925 | | 5949 | B | 1055253 |
| 2510 | F | 599154 | | 4230 | B | 228032 | | 5950 | B | 1054458 |
| 2511 | F | 597254 | | 4231 | B | 229939 | | 5951 | B | 1056325 |
| 2512 | F | 600368 | | 4232 | B | 228555 | | 5952 | B | 1055816 |
| 2513 | F | 598433 | | 4233 | B | 230455 | | 5953 | B | 1057680 |
| 2514 | F | 600665 | | 4234 | B | 228925 | | 5954 | B | 1056172 |
| 2515 | F | 598769 | | 4235 | B | 230828 | | 5955 | B | 1058031 |
| 2516 | F | 602011 | | 4236 | B | 229587 | | 5956 | B | 1056825 |
| 2517 | F | 600087 | | 4237 | B | 231371 | | 5957 | B | 1058710 |
| 2518 | F | 602418 | | 4238 | B | 231239 | | 5958 | B | 1057197 |
| 2519 | F | 600513 | | 4239 | B | 233111 | | 5959 | B | 1059089 |
| 2520 | F | 602921 | | 4240 | B | 231737 | | 5960 | B | 1058522 |
| 2521 | F | 601009 | | 4241 | B | 233660 | | 5961 | B | 1060355 |
| 2522 | F | 604391 | | 4242 | B | 232306 | | 5962 | B | 1058919 |
| 2523 | F | 602468 | | 4243 | B | 234186 | | 5963 | B | 1060810 |
| 2524 | F | 605571 | | 4244 | B | 233044 | | 5964 | B | 1059646 |
| 2525 | F | 603671 | | 4245 | B | 234873 | | 5965 | B | 1061521 |
| 2526 | F | 606334 | | 4246 | B | 234599 | | 5966 | B | 1060801 |
| 2527 | F | 604452 | | 4247 | B | 236504 | | 5967 | B | 1062701 |
| 2528 | F | 607133 | | 4248 | B | 233738 | | 5968 | B | 1061774 |
| 2529 | F | 605167 | | 4249 | B | 235682 | | 5969 | B | 1063687 |
| 2530 | F | 608673 | | 4250 | B | 235454 | | 5970 | B | 1062682 |
| 2531 | F | 606773 | | 4251 | B | 237347 | | 5971 | B | 1064555 |
| 2532 | F | 609710 | | 4252 | B | 235569 | | 5972 | B | 1064300 |
| 2533 | F | 607794 | | 4253 | B | 237469 | | 5973 | B | 1066236 |
| 2534 | F | 610711 | | 4254 | B | 236954 | | 5974 | B | 1065489 |
| 2535 | F | 608882 | | 4255 | B | 238812 | | 5975 | B | 1067386 |
| 2536 | F | 611524 | | 4256 | B | 237891 | | 5976 | B | 1067725 |
| 2537 | F | 609623 | | 4257 | B | 239761 | | 5977 | B | 1069601 |
| 2538 | F | 612119 | | 4258 | B | 238568 | | 5978 | B | 1068285 |
| 2539 | F | 610213 | | 4259 | B | 240472 | | 5979 | B | 1070188 |
| 2540 | F | 613820 | | 4260 | B | 239227 | | 5980 | B | 1068930 |
| 2541 | F | 611861 | | 4261 | B | 241122 | | 5981 | B | 1070898 |
| 2542 | F | 614604 | | 4262 | B | 240341 | | 5982 | B | 1070188 |
| 2543 | F | 612704 | | 4263 | B | 242266 | | 5983 | B | 1072078 |
| 2544 | F | 614960 | | 4264 | B | 241805 | | 5984 | B | 1071383 |
| 2545 | F | 613056 | | 4265 | B | 243697 | | 5985 | B | 1073283 |
| 2546 | F | 616387 | | 4266 | B | 242570 | | 5986 | B | 1072658 |
| 2547 | F | 614471 | | 4267 | B | 244401 | | 5987 | B | 1074584 |
| 2548 | F | 617574 | | 4268 | B | 243155 | | 5988 | B | 1073699 |
| 2549 | F | 615586 | | 4269 | B | 245067 | | 5989 | B | 1075652 |
| 2550 | F | 619430 | | 4270 | B | 243636 | | 5990 | B | 1076111 |
| 2551 | F | 617510 | | 4271 | B | 245538 | | 5991 | B | 1077988 |
| 2552 | F | 618561 | | 4272 | B | 244754 | | 5992 | B | 1077010 |
| 2553 | F | 616679 | | 4273 | B | 246679 | | 5993 | B | 1078959 |
| 2554 | F | 619799 | | 4274 | B | 246248 | | 5994 | B | 1077598 |
| 2555 | F | 617886 | | 4275 | B | 248169 | | 5995 | B | 1079390 |
| 2556 | F | 621043 | | 4276 | B | 248035 | | 5996 | B | 1078260 |
| 2557 | F | 619133 | | 4277 | B | 249968 | | 5997 | B | 1080217 |
| 2558 | F | 622333 | | 4278 | B | 249397 | | 5998 | B | 1078959 |
| 2559 | F | 620411 | | 4279 | B | 251305 | | 5999 | B | 1080869 |
| 2560 | F | 623110 | | 4280 | B | 251305 | | 6000 | B | 1079354 |
| 2561 | F | 621211 | | 4281 | B | 253161 | | 6001 | B | 1081215 |
| 2562 | F | 623952 | | 4282 | B | 252487 | | 6002 | B | 1080217 |
| 2563 | F | 622052 | | 4283 | B | 254380 | | 6003 | B | 1082067 |
| 2564 | F | 624774 | | 4284 | B | 253274 | | 6004 | B | 1080742 |
| 2565 | F | 622872 | | 4285 | B | 255156 | | 6005 | B | 1082621 |
| 2566 | F | 625263 | | 4286 | B | 254230 | | 6006 | B | 1081580 |
| 2567 | F | 623369 | | 4287 | B | 256130 | | 6007 | B | 1083489 |
| 2568 | F | 625664 | | 4288 | B | 255120 | | 6008 | B | 1083400 |
| 2569 | F | 623773 | | 4289 | B | 256980 | | 6009 | B | 1085290 |
| 2570 | F | 626220 | | 4290 | B | 256331 | | 6010 | B | 1084927 |
| 2571 | F | 624297 | | 4291 | B | 258223 | | 6011 | B | 1086797 |
| 2572 | F | 627684 | | 4292 | B | 257706 | | 6012 | B | 1085868 |
| 2573 | F | 625785 | | 4293 | B | 259578 | | 6013 | B | 1087768 |
| 2574 | F | 628536 | | 4294 | B | 258488 | | 6014 | B | 1086965 |
| 2575 | F | 626655 | | 4295 | B | 260396 | | 6015 | B | 1088872 |
| 2576 | F | 629438 | | 4296 | B | 258089 | | 6016 | B | 1088185 |
| 2577 | F | 627541 | | 4297 | B | 260005 | | 6017 | B | 1090076 |
| 2578 | F | 631496 | | 4298 | B | 259202 | | 6018 | B | 1088704 |
| 2579 | F | 629606 | | 4299 | B | 261035 | | 6019 | B | 1090504 |
| 2580 | F | 633301 | | 4300 | B | 261140 | | 6020 | B | 1089236 |
| 2581 | F | 631397 | | 4301 | B | 263031 | | 6021 | B | 1091181 |
| 2582 | F | 637012 | | 4302 | B | 261834 | | 6022 | B | 1090076 |
| 2583 | F | 635112 | | 4303 | B | 263716 | | 6023 | B | 1091944 |
| 2584 | F | 638002 | | 4304 | B | 263031 | | 6024 | B | 1093259 |
| 2585 | F | 636114 | | 4305 | B | 264890 | | 6025 | B | 1095056 |
| 2586 | F | 638598 | | 4306 | B | 263293 | | 6026 | B | 1093403 |
| 2587 | F | 636682 | | 4307 | B | 265179 | | 6027 | B | 1095301 |
| 2588 | F | 638836 | | 4308 | B | 264599 | | 6028 | B | 1094437 |
| 2589 | F | 636938 | | 4309 | B | 266560 | | 6029 | B | 1096375 |
| 2590 | F | 639333 | | 4310 | B | 266208 | | 6030 | B | 1095839 |
| 2591 | F | 637471 | | 4311 | B | 268109 | | 6031 | B | 1097798 |
| 2592 | F | 640506 | | 4312 | B | 266867 | | 6032 | B | 1096858 |
| 2593 | F | 638598 | | 4313 | B | 268783 | | 6033 | B | 1098751 |
| 2594 | F | 640730 | | 4314 | B. | 267558 | | 6034 | B | 1097305 |
| 2595 | F | 638885 | | 4315 | B | 269472 | | 6035 | B | 1099205 |
| 2596 | F | 641468 | | 4316 | B | 268249 | | 6036 | B | 1097835 |
| 2597 | F | 639550 | | 4317 | B | 270042 | | 6037 | B | 1099724 |
| 2598 | F | 642029 | | 4318 | B | 269121 | | 6038 | B | 1098097 |
| 2599 | F | 640162 | | 4319 | B | 271051 | | 6039 | B | 1100046 |
| 2600 | F | 642785 | | 4320 | B | 269709 | | 6040 | B | 1098615 |
| 2601 | F | 640954 | | 4321 | B | 271643 | | 6041 | B | 1100561 |
| 2602 | F | 643129 | | 4322 | B | 271051 | | 6042 | B | 1099098 |
| 2603 | F | 641229 | | 4323 | B | 272920 | | 6043 | B | 1100975 |
| 2604 | F | 643440 | | 4324 | B | 271761 | | 6044 | B | 1099614 |
| 2605 | F | 641522 | | 4325 | B | 273662 | | 6045 | B | 1101442 |
| 2606 | F | 645316 | | 4326 | B | 272570 | | 6046 | B | 1099747 |
| 2607 | F | 643376 | | 4327 | B | 274469 | | 6047 | B | 1101651 |
| 2608 | F | 645552 | | 4328 | B | 273370 | | 6048 | B | 1101298 |
| 2609 | F | 643613 | | 4329 | B | 275313 | | 6049 | B | 1103227 |
| 2610 | F | 646025 | | 4330 | B | 273884 | | 6050 | B | 1102435 |
| 2611 | F | 644186 | | 4331 | B | 275821 | | 6051 | B | 1104381 |
| 2612 | F | 646773 | | 4332 | B | 274219 | | 6052 | B | 1105179 |
| 2613 | F | 644904 | | 4333 | B | 276115 | | 6053 | B | 1107090 |
| 2614 | F | 647678 | | 4334 | B | 274796 | | 6054 | B | 1106770 |
| 2615 | F | 645712 | | 4335 | B | 276716 | | 6055 | B | 1108631 |
| 2616 | F | 648128 | | 4336 | B | 275980 | | 6056 | B | 1107502 |
| 2617 | F | 646249 | | 4337 | B | 277886 | | 6057 | B | 1109392 |
| 2618 | F | 650179 | | 4338 | B | 276241 | | 6058 | B | 1108337 |
| 2619 | F | 648244 | | 4339 | B | 278138 | | 6059 | B | 1110240 |
| 2620 | F | 651010 | | 4340 | B | 276716 | | 6060 | B | 1108653 |
| 2621 | F | 649149 | | 4341 | B | 278625 | | 6061 | B | 1110570 |
| 2622 | F | 652904 | | 4342 | B | 277185 | | 6062 | B | 1113632 |
| 2623 | F | 651003 | | 4343 | B | 279054 | | 6063 | B | 1115499 |
| 2624 | F | 653946 | | 4344 | B | 277489 | | 6064 | B | 1115225 |
| 2625 | F | 652070 | | 4345 | B | 279380 | | 6065 | B | 1117081 |
| 2626 | F | 655735 | | 4346 | B | 277886 | | 6066 | B | 1117154 |
| 2627 | F | 653827 | | 4347 | B | 279722 | | 6067 | B | 1119051 |
| 2628 | F | 656759 | | 4348 | B | 278125 | | 6068 | B | 1118403 |
| 2629 | F | 654894 | | 4349 | B | 280012 | | 6069 | B | 1120310 |
| 2630 | F | 658287 | | 4350 | B | 278841 | | 6070 | B | 1120257 |
| 2631 | F | 656399 | | 4351 | B | 280733 | | 6071 | B | 1122178 |
| 2632 | F | 659973 | | 4352 | B | 279577 | | 6072 | B | 1120776 |
| 2633 | F | 658109 | | 4353 | B | 281466 | | 6073 | B | 1122682 |
| 2634 | F | 662935 | | 4354 | B | 280672 | | 6074 | B | 1121660 |
| 2635 | F | 661035 | | 4355 | B | 282564 | | 6075 | B | 1123554 |
| 2636 | F | 664393 | | 4356 | B | 281767 | | 6076 | B | 1122120 |
| 2637 | F | 662513 | | 4357 | B | 283676 | | 6077 | B | 1123999 |
| 2638 | F | 665972 | | 4358 | B | 282564 | | 6078 | B | 1123243 |
| 2639 | F | 664090 | | 4359 | B | 284462 | | 6079 | B | 1125024 |
| 2640 | F | 666765 | | 4360 | B | 284311 | | 6080 | B | 1123752 |
| 2641 | F | 664879 | | 4361 | B | 286210 | | 6081 | B | 1125688 |
| 2642 | F | 667690 | | 4362 | B | 284740 | | 6082 | B | 1124484 |
| 2643 | F | 665707 | | 4363 | B | 286647 | | 6083 | B | 1126360 |
| 2644 | F | 668261 | | 4364 | B | 285998 | | 6084 | B | 1125020 |
| 2645 | F | 666370 | | 4365 | B | 287975 | | 6085 | B | 1126928 |
| 2646 | F | 668934 | | 4366 | B | 286210 | | 6086 | B | 1125790 |
| 2647 | F | 667029 | | 4367 | B | 288110 | | 6087 | B | 1127735 |
| 2648 | F | 670871 | | 4368 | B | 287201 | | 6088 | B | 1126747 |
| 2649 | F | 668964 | | 4369 | B | 289106 | | 6089 | B | 1128662 |
| 2650 | F | 670629 | | 4370 | B | 287803 | | 6090 | B | 1127899 |
| 2651 | F | 668715 | | 4371 | B | 289737 | | 6091 | B | 1129808 |
| 2652 | F | 672231 | | 4372 | B | 288217 | | 6092 | B | 1128819 |
| 2653 | F | 670334 | | 4373 | B | 290112 | | 6093 | B | 1130695 |
| 2654 | F | 672846 | | 4374 | B | 288417 | | 6094 | B | 1129798 |
| 2655 | F | 670946 | | 4375 | B | 290319 | | 6095 | B | 1131693 |
| 2656 | F | 674040 | | 4376 | B | 289106 | | 6096 | B | 1131563 |
| 2657 | F | 672139 | | 4377 | B | 290961 | | 6097 | B | 1133490 |
| 2658 | F | 674573 | | 4378 | B | 289459 | | 6098 | B | 1132846 |
| 2659 | F | 672674 | | 4379 | B | 291358 | | 6099 | B | 1134684 |
| 2660 | F | 675234 | | 4380 | B | 289914 | | 6100 | B | 1134070 |
| 2661 | F | 673377 | | 4381 | B | 291796 | | 6101 | B | 1136016 |
| 2662 | F | 675834 | | 4382 | B | 290477 | | 6102 | B | 1135089 |
| 2663 | F | 673906 | | 4383 | B | 292423 | | 6103 | B | 1137037 |
| 2664 | F | 676378 | | 4384 | B | 290381 | | 6104 | B | 1135815 |
| 2665 | F | 674477 | | 4385 | B | 292309 | | 6105 | B | 1137715 |
| 2666 | F | 676746 | | 4386 | B | 291463 | | 6106 | B | 1136186 |
| 2667 | F | 674888 | | 4387 | B | 293372 | | 6107 | B | 1138084 |
| 2668 | F | 677769 | | 4388 | B | 292104 | | 6108 | B | 1137365 |
| 2669 | F | 675834 | | 4389 | B | 293999 | | 6109 | B | 1139255 |
| 2670 | F | 678270 | | 4390 | B | 293027 | | 6110 | B | 1140364 |
| 2671 | F | 676378 | | 4391 | B | 294951 | | 6111 | B | 1142228 |
| 2672 | F | 679221 | | 4392 | B | 293507 | | 6112 | B | 1141611 |
| 2673 | F | 677325 | | 4393 | B | 295409 | | 6113 | B | 1143485 |
| 2674 | F | 679874 | | 4394 | B | 293999 | | 6114 | B | 1142478 |
| 2675 | F | 677978 | | 4395 | B | 295838 | | 6115 | B | 1144291 |
| 2676 | F | 681173 | | 4396 | B | 294889 | | 6116 | B | 1145907 |
| 2677 | F | 679288 | | 4397 | B | 296750 | | 6117 | B | 1147783 |
| 2678 | F | 680607 | | 4398 | B | 295312 | | 6118 | B | 1146953 |
| 2679 | F | 678674 | | 4399 | B | 297219 | | 6119 | B | 1148846 |
| 2680 | F | 682210 | | 4400 | B | 296373 | | 6120 | B | 1147769 |
| 2681 | F | 680303 | | 4401 | B | 298305 | | 6121 | B | 1149703 |
| 2682 | F | 682542 | | 4402 | B | 298114 | | 6122 | B | 1148415 |
| 2683 | F | 680607 | | 4403 | B | 299985 | | 6123 | B | 1150357 |
| 2684 | F | 683716 | | 4404 | B | 298656 | | 6124 | B | 1148758 |
| 2685 | F | 681842 | | 4405 | B | 300623 | | 6125 | B | 1150658 |
| 2686 | F | 684312 | | 4406 | B | 299027 | | 6126 | B | 1149462 |
| 2687 | F | 682410 | | 4407 | B | 300899 | | 6127 | B | 1151258 |
| 2688 | F | 684880 | | 4408 | B | 299805 | | 6128 | B | 1149932 |
| 2689 | F | 682916 | | 4409 | B | 301692 | | 6129 | B | 1151845 |
| 2690 | F | 685958 | | 4410 | B | 300722 | | 6130 | B | 1150814 |
| 2691 | F | 684143 | | 4411 | B | 302621 | | 6131 | B | 1152747 |
| 2692 | F | 687264 | | 4412 | B | 301846 | | 6132 | B | 1151409 |
| 2693 | F | 685363 | | 4413 | B | 303706 | | 6133 | B | 1153285 |
| 2694 | F | 687959 | | 4414 | B | 302660 | | 6134 | B | 1152540 |
| 2695 | F | 685958 | | 4415 | B | 304642 | | 6135 | B | 1154341 |
| 2696 | F | 688514 | | 4416 | B | 303066 | | 6136 | B | 1154863 |
| 2697 | F | 686605 | | 4417 | B | 304962 | | 6137 | B | 1156751 |
| 2698 | F | 689372 | | 4418 | B | 303626 | | 6138 | B | 1155886 |
| 2699 | F | 687431 | | 4419 | B | 305479 | | 6139 | B | 1157813 |
| 2700 | F | 690201 | | 4420 | B | 304643 | | 6140 | B | 1156963 |
| 2701 | F | 688318 | | 4421 | B | 306514 | | 6141 | B | 1158871 |
| 2702 | F | 691271 | | 4422 | B | 305479 | | 6142 | B | 1158093 |
| 2703 | F | 689372 | | 4423 | B | 307390 | | 6143 | B | 1159947 |
| 2704 | F | 692436 | | 4424 | B | 306459 | | 6144 | B | 1160998 |
| 2705 | F | 690546 | | 4425 | B | 308393 | | 6145 | B | 1162864 |
| 2706 | F | 694813 | | 4426 | B | 307662 | | 6146 | B | 1162864 |
| 2707 | F | 692930 | | 4427 | B | 309601 | | 6147 | B | 1164740 |
| 2708 | F | 695787 | | 4428 | B | 308298 | | 6148 | B | 1163244 |
| 2709 | F | 693920 | | 4429 | B | 310153 | | 6149 | B | 1165090 |
| 2710 | F | 696363 | | 4430 | B | 309145 | | 6150 | B | 1164244 |
| 2711 | F | 694463 | | 4431 | B | 311044 | | 6151 | B | 1166175 |
| 2712 | F | 698029 | | 4432 | B | 310468 | | 6152 | B | 1164517 |
| 2713 | F | 696133 | | 4433 | B | 312338 | | 6153 | B | 1166482 |
| 2714 | F | 699556 | | 4434 | B | 311437 | | 6154 | B | 1165167 |
| 2715 | F | 697631 | | 4435 | B | 313337 | | 6155 | B | 1167100 |
| 2716 | F | 702303 | | 4436 | B | 311857 | | 6156 | B | 1165789 |
| 2717 | F | 700432 | | 4437 | B | 313860 | | 6157 | B | 1167710 |
| 2718 | F | 702964 | | 4438 | B | 311857 | | 6158 | B | 1166376 |
| 2719 | F | 701079 | | 4439 | B | 313860 | | 6159 | B | 1168228 |
| 2720 | F | 704018 | | 4440 | B | 313015 | | 6160 | B | 1166872 |
| 2721 | F | 702120 | | 4441 | B | 314911 | | 6161 | B | 1168764 |
| 2722 | F | 705018 | | 4442 | B | 313687 | | 6162 | B | 1168598 |
| 2723 | F | 703172 | | 4443 | B | 315549 | | 6163 | B | 1170498 |
| 2724 | F | 705992 | | 4444 | B | 313866 | | 6164 | B | 1169447 |
| 2725 | F | 704105 | | 4445 | B | 315784 | | 6165 | B | 1171347 |
| 2726 | F | 706535 | | 4446 | B | 314911 | | 6166 | B | 1170043 |
| 2727 | F | 704685 | | 4447 | B | 316804 | | 6167 | B | 1171947 |
| 2728 | F | 707455 | | 4448 | B | 315809 | | 6168 | B | 1170689 |
| 2729 | F | 705553 | | 4449 | B | 317701 | | 6169 | B | 1172616 |
| 2730 | F | 708360 | | 4450 | B | 316382 | | 6170 | B | 1171556 |
| 2731 | F | 706385 | | 4451 | B | 318284 | | 6171 | B | 1173507 |
| 2732 | F | 708897 | | 4452 | B | 318881 | | 6172 | B | 1172305 |
| 2733 | F | 706997 | | 4453 | B | 320778 | | 6173 | B | 1174210 |
| 2734 | F | 709589 | | 4454 | B | 321262 | | 6174 | B | 1172562 |
| 2735 | F | 707689 | | 4455 | B | 323214 | | 6175 | B | 1174508 |
| 2736 | F | 709907 | | 4456 | B | 321665 | | 6176 | B | 1174018 |
| 2737 | F | 707963 | | 4457 | B | 323565 | | 6177 | B | 1175899 |
| 2738 | F | 711269 | | 4458 | B | 322571 | | 6178 | B | 1175429 |
| 2739 | F | 709396 | | 4459 | B | 324461 | | 6179 | B | 1177348 |
| 2740 | F | 711864 | | 4460 | B | 323425 | | 6180 | B | 1175793 |
| 2741 | F | 709985 | | 4461 | B | 325316 | | 6181 | B | 1177675 |
| 2742 | F | 714531 | | 4462 | B | 324095 | | 6182 | B | 1177347 |
| 2743 | F | 712594 | | 4463 | B | 325977 | | 6183 | B | 1179199 |
| 2744 | F | 715653 | | 4464 | B | 325135 | | 6184 | B | 1179316 |
| 2745 | F | 713725 | | 4465 | B | 327001 | | 6185 | B | 1181171 |
| 2746 | F | 717511 | | 4466 | B | 326634 | | 6186 | B | 1180309 |
| 2747 | F | 715615 | | 4467 | B | 328557 | | 6187 | B | 1182212 |
| 2748 | F | 718865 | | 4468 | B | 328081 | | 6188 | B | 1181048 |
| 2749 | F | 716993 | | 4469 | B | 329959 | | 6189 | B | 1182918 |
| 2750 | F | 720365 | | 4470 | B | 328719 | | 6190 | B | 1182162 |
| 2751 | F | 718471 | | 4471 | B | 330596 | | 6191 | B | 1184078 |
| 2752 | F | 722155 | | 4472 | B | 328893 | | 6192 | B | 1182528 |
| 2753 | F | 720253 | | 4473 | B | 330825 | | 6193 | B | 1184437 |
| 2754 | F | 722897 | | 4474 | B | 329590 | | 6194 | B | 1184078 |
| 2755 | F | 720989 | | 4475 | B | 331485 | | 6195 | B | 1186015 |
| 2756 | F | 723385 | | 4476 | B | 331127 | | 6196 | B | 1184698 |
| 2757 | F | 721493 | | 4477 | B | 333069 | | 6197 | B | 1186540 |
| 2758 | F | 724029 | | 4478 | B | 332679 | | 6198 | B | 1185631 |
| 2759 | F | 722081 | | 4479 | B | 334592 | | 6199 | B | 1187530 |
| 2760 | F | 724678 | | 4480 | B | 334790 | | 6200 | B | 1186079 |
| 2761 | F | 722749 | | 4481 | B | 336673 | | 6201 | B | 1188004 |
| 2762 | F | 726048 | | 4482 | B | 336311 | | 6202 | B | 1186704 |
| 2763 | F | 724143 | | 4483 | B | 338267 | | 6203 | B | 1188610 |
| 2764 | F | 726897 | | 4484 | B | 337572 | | 6204 | B | 1189251 |
| 2765 | F | 724997 | | 4485 | B | 339431 | | 6205 | B | 1191165 |
| 2766 | F | 727969 | | 4486 | B | 338545 | | 6206 | B | 1187609 |
| 2767 | F | 726086 | | 4487 | B | 340463 | | 6207 | B | 1189506 |
| 2768 | F | 728380 | | 4488 | B | 339058 | | 6208 | B | 1191165 |
| 2769 | F | 726446 | | 4489 | B | 341011 | | 6209 | B | 1193050 |
| 2770 | F | 729281 | | 4490 | B | 339740 | | 6210 | B | 1192378 |
| 2771 | F | 727410 | | 4491 | B | 341628 | | 6211 | B | 1194291 |
| 2772 | F | 729510 | | 4492 | B | 340366 | | 6212 | B | 1192265 |
| 2773 | F | 727579 | | 4493 | B | 342354 | | 6213 | B | 1194114 |
| 2774 | F | 729949 | | 4494 | B | 343265 | | 6214 | B | 1193058 |
| 2775 | F | 728036 | | 4495 | B | 345125 | | 6215 | B | 1194987 |
| 2776 | F | 730367 | | 4496 | B | 344126 | | 6216 | B | 1193224 |
| 2777 | F | 728455 | | 4497 | B | 345957 | | 6217 | B | 1195115 |
| 2778 | F | 731760 | | 4498 | B | 344391 | | 6218 | B | 1194035 |
| 2779 | F | 729866 | | 4499 | B | 346291 | | 6219 | B | 1195955 |
| 2780 | F | 732172 | | 4500 | B | 345324 | | 6220 | B | 1194384 |
| 2781 | F | 730275 | | 4501 | B | 347236 | | 6221 | B | 1196265 |
| 2782 | F | 733018 | | 4502 | B | 346289 | | 6222 | B | 1194291 |
| 2783 | F | 731197 | | 4503 | B | 348198 | | 6223 | B | 1196205 |
| 2784 | F | 733252 | | 4504 | B | 347090 | | 6224 | B | 1195955 |
| 2785 | F | 731354 | | 4505 | B | 348914 | | 6225 | B | 1197863 |
| 2786 | F | 733674 | | 4506 | B | 347292 | | 6226 | B | 1196570 |
| 2787 | F | 731760 | | 4507 | B | 349158 | | 6227 | B | 1198423 |
| 2788 | F | 734054 | | 4508 | B | 347946 | | 6228 | B | 1197051 |
| 2789 | F | 732172 | | 4509 | B | 349851 | | 6229 | B | 1198951 |
| 2790 | F | 734632 | | 4510 | B | 350799 | | 6230 | B | 1198058 |
| 2791 | F | 732736 | | 4511 | B | 352598 | | 6231 | B | 1199931 |
| 2792 | F | 735071 | | 4512 | B | 351313 | | 6232 | B | 1198960 |
| 2793 | F | 733219 | | 4513 | B | 353223 | | 6233 | B | 1200867 |
| 2794 | F | 735381 | | 4514 | B | 352400 | | 6234 | B | 1200490 |
| 2795 | F | 733445 | | 4515 | B | 354357 | | 6235 | B | 1202395 |
| 2796 | F | 735852 | | 4516 | B | 353522 | | 6236 | B | 1201512 |
| 2797 | F | 733957 | | 4517 | B | 355411 | | 6237 | B | 1203426 |
| 2798 | F | 736244 | | 4518 | B | 354690 | | 6238 | B | 1202606 |
| 2799 | F | 734401 | | 4519 | B | 356610 | | 6239 | B | 1204532 |
| 2800 | F | 736982 | | 4520 | B | 355158 | | 6240 | B | 1203139 |
| 2801 | F | 735071 | | 4521 | B | 357057 | | 6241 | B | 1205063 |
| 2802 | F | 737321 | | 4522 | B | 355676 | | 6242 | B | 1203691 |
| 2803 | F | 735397 | | 4523 | B | 357681 | | 6243 | B | 1205597 |
| 2804 | F | 737566 | | 4524 | B | 356995 | | 6244 | B | 1204382 |
| 2805 | F | 735696 | | 4525 | B | 358866 | | 6245 | B | 1206284 |
| 2806 | F | 738491 | | 4526 | B | 356173 | | 6246 | B | 1205249 |
| 2807 | F | 736564 | | 4527 | B | 358074 | | 6247 | B | 1207170 |
| 2808 | F | 738797 | | 4528 | B | 359607 | | 6248 | B | 1206651 |
| 2809 | F | 736935 | | 4529 | B | 361536 | | 6249 | B | 1208536 |
| 2810 | F | 739513 | | 4530 | B | 359550 | | 6250 | B | 1206976 |
| 2811 | F | 737626 | | 4531 | B | 361442 | | 6251 | B | 1208862 |
| 2812 | F | 740420 | | 4532 | B | 360135 | | 6252 | B | 1208092 |
| 2813 | F | 738526 | | 4533 | B | 362033 | | 6253 | B | 1210002 |
| 2814 | F | 740457 | | 4534 | B | 361536 | | 6254 | B | 1209115 |
| 2815 | F | 738599 | | 4535 | B | 363461 | | 6255 | B | 1210973 |
| 2816 | F | 741553 | | 4536 | B | 364013 | | 6256 | B | 1209979 |
| 2817 | F | 739676 | | 4537 | B | 365905 | | 6257 | B | 1211892 |
| 2818 | F | 742518 | | 4538 | B | 364716 | | 6258 | B | 1210739 |
| 2819 | F | 740565 | | 4539 | B | 366707 | | 6259 | B | 1212639 |
| 2820 | F | 743344 | | 4540 | B | 365000 | | 6260 | B | 1211761 |
| 2821 | F | 741509 | | 4541 | B | 366941 | | 6261 | B | 1213680 |
| 2822 | F | 743875 | | 4542 | B | 365513 | | 6262 | B | 1212985 |
| 2823 | F | 741984 | | 4543 | B | 367447 | | 6263 | B | 1214894 |
| 2824 | F | 744240 | | 4544 | B | 365892 | | 6264 | B | 1214299 |
| 2825 | F | 742365 | | 4545 | B | 367873 | | 6265 | B | 1216189 |
| 2826 | F | 744725 | | 4546 | B | 366877 | | 6266 | B | 1215132 |
| 2827 | F | 742858 | | 4547 | B | 368725 | | 6267 | B | 1217036 |
| 2828 | F | 746380 | | 4548 | B | 369265 | | 6268 | B | 1215714 |
| 2829 | F | 744493 | | 4549 | B | 371167 | | 6269 | B | 1217542 |
| 2830 | F | 746957 | | 4550 | B | 370088 | | 6270 | B | 1216541 |
| 2831 | F | 745071 | | 4551 | B | 371988 | | 6271 | B | 1218462 |
| 2832 | F | 747868 | | 4552 | B | 370669 | | 6272 | B | 1216828 |
| 2833 | F | 746023 | | 4553 | B | 372611 | | 6273 | B | 1218677 |
| 2834 | F | 748351 | | 4554 | B | 372871 | | 6274 | B | 1217166 |
| 2835 | F | 746451 | | 4555 | B | 374773 | | 6275 | B | 1218973 |
| 2836 | F | 749395 | | 4556 | B | 373315 | | 6276 | B | 1219876 |
| 2837 | F | 747505 | | 4557 | B | 375227 | | 6277 | B | 1221743 |
| 2838 | F | 749745 | | 4558 | B | 373665 | | 6278 | B | 1220892 |
| 2839 | F | 747857 | | 4559 | B | 375592 | | 6279 | B | 1222895 |
| 2840 | F | 750165 | | 4560 | B | 374428 | | 6280 | B | 1220288 |
| 2841 | F | 748278 | | 4561 | B | 376335 | | 6281 | B | 1222189 |
| 2842 | F | 751013 | | 4562 | B | 375355 | | 6282 | B | 1221657 |
| 2843 | F | 749169 | | 4563 | B | 377248 | | 6283 | B | 1223517 |
| 2844 | F | 752798 | | 4564 | B | 375913 | | 6284 | B | 1223930 |
| 2845 | F | 750889 | | 4565 | B | 377796 | | 6285 | B | 1225828 |
| 2846 | F | 754878 | | 4566 | B | 376483 | | 6286 | B | 1225211 |
| 2847 | F | 752967 | | 4567 | B | 378318 | | 6287 | B | 1227132 |
| 2848 | F | 755856 | | 4568 | B | 377873 | | 6288 | B | 1226090 |
| 2849 | F | 754001 | | 4569 | B | 379798 | | 6289 | B | 1227979 |
| 2850 | F | 756262 | | 4570 | B | 380040 | | 6290 | B | 1227132 |
| 2851 | F | 754372 | | 4571 | B | 381898 | | 6291 | B | 1229039 |
| 2852 | F | 760075 | | 4572 | B | 380699 | | 6292 | B | 1228061 |
| 2853 | F | 758175 | | 4573 | B | 382561 | | 6293 | B | 1229948 |
| 2854 | F | 761069 | | 4574 | B | 381249 | | 6294 | B | 1228293 |
| 2855 | F | 759172 | | 4575 | B | 383174 | | 6295 | B | 267 |
| 2856 | F | 761549 | | 4576 | B | 381689 | | 6296 | B | 1228524 |
| 2857 | F | 759660 | | 4577 | B | 383629 | | 6297 | B | 444 |
| 2858 | F | 761988 | | 4578 | B | 383282 | | 6298 | B | 267 |
| 2859 | F | 760141 | | 4579 | B | 385161 | | 6299 | B | 2068 |
| 2860 | F | 762611 | | 4580 | B | 383789 | | 6300 | F | 25997 |
| 2861 | F | 760747 | | 4581 | B | 385647 | | 6301 | F | 24032 |
| 2862 | F | 763097 | | 4582 | B | 385560 | | 6302 | F | 27128 |
| 2863 | F | 761136 | | 4583 | B | 387427 | | 6303 | F | 25189 |
| 2864 | F | 763622 | | 4584 | B | 386760 | | 6304 | F | 66744 |
| 2865 | F | 761742 | | 4585 | B | 388588 | | 6305 | F | 64845 |
| 2866 | F | 765438 | | 4586 | B | 387508 | | 6306 | F | 70130 |
| 2867 | F | 763525 | | 4587 | B | 389369 | | 6307 | F | 68200 |
| 2868 | F | 766664 | | 4588 | B | 388984 | | 6308 | F | 132477 |
| 2869 | F | 764747 | | 4589 | B | 390900 | | 6309 | F | 130559 |
| 2870 | F | 768045 | | 4590 | B | 390387 | | 6310 | F | 177854 |
| 2871 | F | 766196 | | 4591 | B | 392260 | | 6311 | F | 175906 |
| 2872 | F | 768329 | | 4592 | B | 391202 | | 6312 | F | 208127 |
| 2873 | F | 766429 | | 4593 | B | 393055 | | 6313 | F | 206180 |
| 2874 | F | 769107 | | 4594 | B | 392044 | | 6314 | F | 208688 |
| 2875 | F | 767244 | | 4595 | B | 393959 | | 6315 | F | 206807 |
| 2876 | F | 770507 | | 4596 | B | 392615 | | 6316 | F | 208732 |
| 2877 | F | 768633 | | 4597 | B | 394499 | | 6317 | F | 206877 |
| 2878 | F | 771618 | | 4598 | B | 393218 | | 6318 | F | 210051 |
| 2879 | F | 769725 | | 4599 | B | 395123 | | 6319 | F | 208141 |
| 2880 | F | 772865 | | 4600 | B | 393909 | | 6320 | F | 298801 |
| 2881 | F | 770975 | | 4601 | B | 395807 | | 6321 | F | 296907 |
| 2882 | F | 772865 | | 4602 | B | 394566 | | 6322 | F | 351495 |
| 2883 | F | 770970 | | 4603 | B | 396498 | | 6323 | F | 349572 |
| 2884 | F | 774810 | | 4604 | B | 395027 | | 6324 | F | 419727 |
| 2885 | F | 772927 | | 4605 | B | 396931 | | 6325 | F | 417822 |
| 2886 | F | 774131 | | 4606 | B | 395531 | | 6326 | F | 553133 |
| 2887 | F | 772232 | | 4607 | B | 397467 | | 6327 | F | 551247 |
| 2888 | F | 774604 | | 4608 | B | 396227 | | 6328 | F | 556301 |
| 2889 | F | 772782 | | 4609 | B | 398132 | | 6329 | F | 554410 |
| 2890 | F | 775851 | | 4610 | B | 398070 | | 6330 | F | 593567 |
| 2891 | F | 773934 | | 4611 | B | 399935 | | 6331 | F | 591675 |
| 2892 | F | 777314 | | 4612 | B | 399189 | | 6332 | F | 594641 |
| 2893 | F | 775412 | | 4613 | B | 400970 | | 6333 | F | 592748 |
| 2894 | F | 777677 | | 4614 | B | 400351 | | 6334 | F | 661934 |
| 2895 | F | 775781 | | 4615 | B | 402208 | | 6335 | F | 660041 |
| 2896 | F | 778400 | | 4616 | B | 401465 | | 6336 | F | 706309 |
| 2897 | F | 776472 | | 4617 | B | 403507 | | 6337 | F | 704409 |
| 2898 | F | 779281 | | 4618 | B | 401705 | | 6338 | F | 803092 |
| 2899 | F | 777333 | | 4619 | B | 403666 | | 6339 | F | 801192 |
| 2900 | F | 780063 | | 4620 | B | 402461 | | 6340 | F | 849060 |
| 2901 | F | 778150 | | 4621 | B | 404410 | | 6341 | F | 847142 |
| 2902 | F | 780885 | | 4622 | B | 403507 | | 6342 | F | 913050 |
| 2903 | F | 778994 | | 4623 | B | 405356 | | 6343 | F | 911152 |
| 2904 | F | 781333 | | 4624 | B | 404421 | | 6344 | F | 926614 |
| 2905 | F | 779431 | | 4625 | B | 406295 | | 6345 | F | 924714 |
| 2906 | F | 782524 | | 4626 | B | 406160 | | 6346 | F | 930121 |
| 2907 | F | 780674 | | 4627 | B | 408052 | | 6347 | F | 928238 |
| 2908 | F | 783349 | | 4628 | B | 407645 | | 6348 | F | 986297 |
| 2909 | F | 781433 | | 4629 | B | 409450 | | 6349 | F | 984362 |
| 2910 | F | 785138 | | 4630 | B | 407922 | | 6350 | F | 996001 |
| 2911 | F | 783238 | | 4631 | B | 409744 | | 6351 | F | 994109 |
| 2912 | F | 786197 | | 4632 | B | 409039 | | 6352 | F | 999731 |
| 2913 | F | 784328 | | 4633 | B | 410960 | | 6353 | F | 997877 |
| 2914 | F | 788274 | | 4634 | B | 410673 | | 6354 | F | 1009782 |
| 2915 | F | 786387 | | 4635 | B | 412559 | | 6355 | F | 1007891 |
| 2916 | F | 788679 | | 4636 | B | 411193 | | 6356 | F | 1010540 |
| 2917 | F | 786778 | | 4637 | B | 413064 | | 6357 | F | 1008671 |
| 2918 | F | 790090 | | 4638 | B | 412049 | | 6358 | F | 1012465 |
| 2919 | F | 788213 | | 4639 | B | 413946 | | 6359 | F | 1010540 |
| 2920 | F | 791608 | | 4640 | B | 414525 | | 6360 | F | 1028431 |
| 2921 | F | 789711 | | 4641 | B | 416425 | | 6361 | F | 1026524 |
| 2922 | F | 792499 | | 4642 | B | 415622 | | 6362 | F | 1086215 |
| 2923 | F | 790605 | | 4643 | B | 417559 | | 6363 | F | 1084362 |
| 2924 | F | 793324 | | 4644 | B | 416072 | | 6364 | F | 1118417 |
| 2925 | F | 791440 | | 4645 | B | 417968 | | 6365 | F | 1116527 |
| 2926 | F | 794068 | | 4646 | B | 417351 | | 6366 | F | 1169595 |
| 2927 | F | 792185 | | 4647 | B | 419259 | | 6367 | F | 1167713 |
| 2928 | F | 794998 | | 4648 | B | 417789 | | 6368 | F | 1180592 |
| 2929 | F | 793098 | | 4649 | B | 419748 | | 6369 | F | 1178709 |
| 2930 | F | 795457 | | 4650 | B | 418569 | | 6370 | F | 1182406 |
| 2931 | F | 793582 | | 4651 | B | 420453 | | 6371 | F | 1180498 |
| 2932 | F | 796831 | | 4652 | B | 420345 | | 6372 | F | 1194573 |
| 2933 | F | 794931 | | 4653 | B | 422177 | | 6373 | F | 1192667 |
| 2934 | F | 798455 | | 4654 | B | 421003 | | 6374 | F | 1195654 |
| 2935 | F | 796551 | | 4655 | B | 422873 | | 6375 | F | 1193753 |
| 2936 | F | 799056 | | 4656 | B | 421819 | | 6376 | B | 26870 |
| 2937 | F | 797147 | | 4657 | B | 423675 | | 6377 | B | 28721 |
| 2938 | F | 799558 | | 4658 | B | 422291 | | 6378 | B | 27835 |
| 2939 | F | 797649 | | 4659 | B | 424158 | | 6379 | B | 29730 |
| 2940 | F | 801106 | | 4660 | B | 423186 | | 6380 | B | 67456 |
| 2941 | F | 799204 | | 4661 | B | 425075 | | 6381 | B | 69351 |
| 2942 | F | 802227 | | 4662 | B | 424544 | | 6382 | B | 70820 |
| 2943 | F | 800325 | | 4663 | B | 426443 | | 6383 | B | 72708 |
| 2944 | F | 803050 | | 4664 | B | 424859 | | 6384 | B | 133173 |
| 2945 | F | 801153 | | 4665 | B | 426714 | | 6385 | B | 135068 |
| 2946 | F | 803599 | | 4666 | B | 426302 | | 6386 | B | 178637 |
| 2947 | F | 801682 | | 4667 | B | 428193 | | 6387 | B | 180518 |
| 2948 | F | 804925 | | 4668 | B | 427640 | | 6388 | B | 208864 |
| 2949 | F | 803016 | | 4669 | B | 429523 | | 6389 | B | 210727 |
| 2950 | F | 805633 | | 4670 | B | 428212 | | 6390 | B | 209376 |
| 2951 | F | 803672 | | 4671 | B | 430111 | | 6391 | B | 211305 |
| 2952 | F | 806109 | | 4672 | B | 428709 | | 6392 | B | 209483 |
| 2953 | F | 804192 | | 4673 | B | 430627 | | 6393 | B | 211383 |
| 2954 | F | 806386 | | 4674 | B | 430926 | | 6394 | B | 210875 |
| 2955 | F | 804453 | | 4675 | B | 432851 | | 6395 | B | 212766 |
| 2956 | F | 806668 | | 4676 | B | 431681 | | 6396 | B | 299694 |
| 2957 | F | 804746 | | 4677 | B | 433569 | | 6397 | B | 301582 |
| 2958 | F | 807924 | | 4678 | B | 432324 | | 6398 | B | 352312 |
| 2959 | F | 806022 | | 4679 | B | 434223 | | 6399 | B | 354200 |
| 2960 | F | 808445 | | 4680 | B | 433015 | | 6400 | B | 420390 |
| 2961 | F | 806525 | | 4681 | B | 434902 | | 6401 | B | 422291 |
| 2962 | F | 809212 | | 4682 | B | 433504 | | 6402 | B | 553822 |
| 2963 | F | 807283 | | 4683 | B | 435426 | | 6403 | B | 555736 |
| 2964 | F | 809982 | | 4684 | B | 434196 | | 6404 | B | 557050 |
| 2965 | F | 808079 | | 4685 | B | 436042 | | 6405 | B | 558930 |
| 2966 | F | 811554 | | 4686 | B | 436913 | | 6406 | B | 594583 |
| 2967 | F | 809659 | | 4687 | B | 438807 | | 6407 | B | 596527 |
| 2968 | F | 812268 | | 4688 | B | 437475 | | 6408 | B | 595405 |
| 2969 | F | 810340 | | 4689 | B | 439423 | | 6409 | B | 597289 |
| 2970 | F | 812712 | | 4690 | B | 438591 | | 6410 | B | 662614 |
| 2971 | F | 810799 | | 4691 | B | 440490 | | 6411 | B | 664530 |
| 2972 | F | 813355 | | 4692 | B | 440583 | | 6412 | B | 707138 |
| 2973 | F | 811466 | | 4693 | B | 442491 | | 6413 | B | 709063 |
| 2974 | F | 815198 | | 4694 | B | 440583 | | 6414 | B | 803951 |
| 2975 | F | 813243 | | 4695 | B | 442441 | | 6415 | B | 805790 |
| 2976 | F | 815798 | | 4696 | B | 441274 | | 6416 | B | 849771 |
| 2977 | F | 813917 | | 4697 | B | 443135 | | 6417 | B | 851730 |
| 2978 | F | 816879 | | 4698 | B | 441459 | | 6418 | B | 913917 |
| 2979 | F | 814940 | | 4699 | B | 443353 | | 6419 | B | 915796 |
| 2980 | F | 817571 | | 4700 | B | 442412 | | 6420 | B | 927331 |
| 2981 | F | 815676 | | 4701 | B | 444339 | | 6421 | B | 929238 |
| 2982 | F | 818388 | | 4702 | B | 443184 | | 6422 | B | 930857 |
| 2983 | F | 816489 | | 4703 | B | 445100 | | 6423 | B | 932735 |
| 2984 | F | 818884 | | 4704 | B | 443131 | | 6424 | B | 986987 |
| 2985 | F | 816921 | | 4705 | B | 445100 | | 6425 | B | 988912 |
| 2986 | F | 819597 | | 4706 | B | 443800 | | 6426 | B | 996771 |
| 2987 | F | 817680 | | 4707 | B | 445789 | | 6427 | B | 998623 |
| 2988 | F | 820485 | | 4708 | B | 444771 | | 6428 | B | 1000593 |
| 2989 | F | 818555 | | 4709 | B | 446620 | | 6429 | B | 1002496 |
| 2990 | F | 820764 | | 4710 | B | 445100 | | 6430 | B | 1010541 |
| 2991 | F | 818878 | | 4711 | B | 446962 | | 6431 | B | 1012452 |
| 2992 | F | 821982 | | 4712 | B | 445229 | | 6432 | B | 1011365 |
| 2993 | F | 820080 | | 4713 | B | 447187 | | 6433 | B | 1013249 |
| 2994 | F | 823403 | | 4714 | B | 445974 | | 6434 | B | 1013146 |
| 2995 | F | 821559 | | 4715 | B | 447872 | | 6435 | B | 1015044 |
| 2996 | F | 825235 | | 4716 | B | 448028 | | 6436 | B | 1029168 |
| 2997 | F | 823320 | | 4717 | B | 449927 | | 6437 | B | 1031036 |
| 2998 | F | 826405 | | 4718 | B | 448958 | | 6438 | B | 1087041 |
| 2999 | F | 824501 | | 4719 | B | 450858 | | 6439 | B | 1088885 |
| 3000 | F | 826945 | | 4720 | B | 449850 | | 6440 | B | 1119102 |
| 3001 | F | 825046 | | 4721 | B | 451753 | | 6441 | B | 1121033 |
| 3002 | F | 828489 | | 4722 | B | 451103 | | 6442 | B | 1170355 |
| 3003 | F | 826588 | | 4723 | B | 453045 | | 6443 | B | 1172218 |
| 3004 | F | 829813 | | 4724 | B | 451482 | | 6444 | B | 1181427 |
| 3005 | F | 827917 | | 4725 | B | 453330 | | 6445 | B | 1183338 |
| 3006 | F | 830824 | | 4726 | B | 452676 | | 6446 | B | 1183263 |
| 3007 | F | 828906 | | 4727 | B | 454575 | | 6447 | B | 1185158 |
| 3008 | F | 831936 | | 4728 | B | 453884 | | 6448 | B | 1195296 |
| 3009 | F | 830099 | | 4729 | B | 455783 | | 6449 | B | 1197175 |
| 3010 | F | 833126 | | 4730 | B | 455068 | | 6450 | B | 1196406 |
| 3011 | F | 831274 | | 4731 | B | 456963 | | 6451 | B | 1198306 |

**TABLE 6**

| *clone Name* | *NO (B)* | *NO (F)* | *Chromosomal region* |
|---|---|---|---|
| 790313H3# | 6452 | 6648 | A |
| 790331B1# | 6453 | 6649 | A |
| 790233A9# | 6454 | 6650 | A |
| 790031G7# | 6455 | 6651 | A |
| 890021E4# | 6456 | 6652 | A |
| 790021E11# | 6457 | 6653 | A |
| 790332G10# | 6458 | 6654 | A |
| 790271B6# | 6459 | 6655 | A |
| 790253H6# | 6460 | 6656 | A |
| 790214E8# | 6461 | 6657 | A |
| 790352D2# | 6462 | 6658 | A |
| 790373F2# | 6463 | 6659 | A |
| 790424A7# | 6464 | 6660 | A |
| 790282F3# | 6465 | 6661 | A |
| 790272F5# | 6466 | 6662 | A |
| 790424F6# | 6467 | 6663 | A |
| 890033H11# | 6468 | 6664 | A |
| 790264H10# | 6469 | 6665 | A |
| 790293A5# | 6470 | 6666 | A |
| 790391E8# | 6471 | 6667 | A |
| 890022B8# | 6472 | 6668 | A |
| 790332B9# | 6473 | 6669 | A |
| 790251B9# | 6474 | 6670 | A |
| 790344E8# | 6475 | 6671 | B |
| 790323F3# | 6476 | 6672 | B |
| 790231G2# | 6477 | 6673 | B |
| 790341C5# | 6478 | 6674 | B |
| 790332H9# | 6479 | 6675 | B |
| 890013A8# | 6480 | 6676 | B |
| 790394F2# | 6481 | 6677 | B |
| 790222G5# | 6482 | 6678 | B |
| 790402A10# | 6483 | 6679 | B |
| 790283F6# | 6484 | 6680 | B |
| 790041H11# | 6485 | 6681 | B |
| 790381C7# | 6486 | 6682 | B |
| 790213E1# | 6487 | 6683 | B |
| 790211C4# | 6488 | 6684 | B |
| 790251B5# | 6489 | 6685 | B |
| 790043H9# | 6490 | 6686 | B |
| 790303F7# | 6491 | 6687 | B |
| 790251G5# | 6492 | 6688 | B |
| 790044H7# | 6493 | 6689 | B |
| 790022E4# | 6494 | 6690 | B |
| 790252A8# | 6495 | 6691 | B |
| 790313E9# | 6496 | 6692 | B |
| 790264G2# | 6497 | 6693 | B |
| 790372A4# | 6498 | 6694 | B |
| 790411C2# | 6499 | 6695 | B |
| 790322B7# | 6500 | 6696 | B |
| 790254F7# | 6501 | 6697 | B |
| 790323B12# | 6502 | 6698 | B |
| 790263E5# | 6503 | 6699 | B |
| 790223C8# | 6504 | 6700 | B |
| 790231H2# | 6505 | 6701 | B |
| 790324E12# | 6506 | 6702 | B |
| 790271D7# | 6507 | 6703 | B |
| 790222E8# | 6508 | 6704 | B |
| 790083G7# | 6509 | 6705 | B |
| 790241D3# | 6510 | 6706 | B |
| 790303C8# | 6511 | 6707 | B |
| 790283F10# | 6512 | 6708 | B |
| 790241B7# | 6513 | 6709 | B |
| 790373F10# | 6514 | 6710 | B |
| 790362F9# | 6515 | 6711 | B |
| 790263H8# | 6516 | 6712 | B |
| 790393D10# | 6517 | 6713 | B |
| 790313D12# | 6518 | 6714 | B |
| 890024C6# | 6519 | 6715 | B |
| 890024B10# | 6520 | 6716 | B |
| 790212E2# | 6521 | 6717 | B |
| 790362E10# | 6522 | 6718 | B |
| 790344G11# | 6523 | 6719 | B |
| 890011D2# | 6524 | 6720 | B |
| 790341B11# | 6525 | 6721 | B |
| 790064E10# | 6526 | 6722 | B |
| 790212E1# | 6527 | 6723 | B |
| 790213G5# | 6528 | 6724 | B |
| 790331F2# | 6529 | 6725 | B |
| 890024B9# | 6530 | 6726 | B |
| 790421F5# | 6531 | 6727 | B |
| 890014D11# | 6532 | 6728 | B |
| 790373F3# | 6533 | 6729 | B |
| 790293D4# | 6534 | 6730 | B |
| 790211A3# | 6535 | 6731 | B |
| 790211H8# | 6536 | 6732 | B |
| 790264E7# | 6537 | 6733 | B |
| 790292B11# | 6538 | 6734 | B |
| 790312A2# | 6539 | 6735 | B |
| 890012D5# | 6540 | 6736 | B |
| 790012D12# | 6541 | 6737 | B |
| 790291E10# | 6542 | 6738 | B |
| 790241 C9# | 6543 | 6739 | B |
| 790343F1# | 6544 | 6740 | B |
| 790241D7# | 6545 | 6741 | B |
| 790031H7# | 6546 | 6742 | B |
| 790081C4# | 6547 | 6743 | B |
| 790013B7# | 6548 | 6744 | B |
| 790213F3# | 6549 | 6745 | B |
| 790292F9# | 6550 | 6746 | B |
| 790423F4# | 6551 | 6747 | B |
| 790331F3# | 6552 | 6748 | B |
| 790222B10# | 6553 | 6749 | B |
| 790261G12# | 6554 | 6750 | B |
| 790423G10# | 6555 | 6751 | B |
| 790392A9# | 6556 | 6752 | B |
| 790331B5# | 6557 | 6753 | B |
| 790323H3# | 6558 | 6754 | B |
| 890014H8# | 6559 | 6755 | B |
| 790231B6# | 6560 | 6756 | B |
| 790252F7# | 6561 | 6757 | B |
| 790392C10# | 6562 | 6758 | B |
| 790021D4# | 6563 | 6759 | B |
| 790052D10# | 6564 | 6760 | B |
| 790261E3# | 6565 | 6761 | B |
| 890023E10# | 6566 | 6762 | B |
| 790244B7# | 6567 | 6763 | B |
| 790383E1# | 6568 | 6764 | B |
| 790401B11# | 6569 | 6765 | B |
| 790411B5# | 6570 | 6766 | B |
| 790423A11# | 6571 | 6767 | B |
| 790031A4# | 6572 | 6768 | B |
| 790241G3# | 6573 | 6769 | B |
| 790044F7# | 6574 | 6770 | B |
| 790252B10# | 6575 | 6771 | B |
| 790293F9# | 6576 | 6772 | B |
| 790282H3# | 6577 | 6773 | B |
| 790381C10# | 6578 | 6774 | B |
| 790024H5# | 6579 | 6775 | B |
| 790354H7# | 6580 | 6776 | B |
| 790411F9# | 6581 | 6777 | B |
| 790324G10# | 6582 | 6778 | B |
| 790014A5# | 6583 | 6779 | B |
| 790381F3# | 6584 | 6780 | B |
| 790424D3# | 6585 | 6781 | B |
| 790394A10# | 6586 | 6782 | B |
| 790423C10# | 6587 | 6783 | B |
| 790214D6# | 6588 | 6784 | B |
| 790214C4# | 6589 | 6785 | B |
| 790014F11# | 6590 | 6786 | B |
| 790352F10# | 6591 | 6787 | B |
| 790381H6# | 6592 | 6788 | B |
| 790282G5# | 6593 | 6789 | B |
| 790263C8# | 6594 | 6790 | B |
| 890022B4# | 6595 | 6791 | B |
| 790283C6# | 6596 | 6792 | B |
| 790293B2# | 6597 | 6793 | B |
| 790073A3# | 6598 | 6794 | B |
| 790313E10# | 6599 | 6795 | B |
| 790361D3# | 6600 | 6796 | B |
| 790014A11# | 6601 | 6797 | B |
| 790254G2# | 6602 | 6798 | B |
| 790381C6# | 6603 | 6799 | B |
| 790424E3# | 6604 | 6800 | B |
| 790421G8# | 6605 | 6801 | B |
| 790013C3# | 6606 | 6802 | B |
| 790263E8# | 6607 | 6803 | B |
| 790373C1# | 6608 | 6804 | B |
| 790041C1# | 6609 | 6805 | B |
| 790344A7# | 6610 | 6806 | B |
| 790271D6# | 6611 | 6807 | B |
| 790342H2# | 6612 | 6808 | B |
| 890021A6# | 6613 | 6809 | B |
| 790381E7# | 6614 | 6810 | C |
| 790013G10# | 6615 | 6811 | C |
| 790254A4# | 6616 | 6812 | C |
| 790213D8# | 6617 | 6813 | C |
| 790052A4# | 6618 | 6814 | C |
| 790213D3# | 6619 | 6815 | C |
| 790394D2# | 6620 | 6816 | C |
| 790214D2# | 6621 | 6817 | C |
| 790014A4# | 6622 | 6818 | C |
| 790324H4# | 6623 | 6819 | C |
| 790082B4# | 6624 | 6820 | C |
| 790324A6# | 6625 | 6821 | C |
| 790424A12# | 6626 | 6822 | C |
| 790044G8# | 6627 | 6823 | C |
| 790323C6# | 6628 | 6824 | C |
| 790312G4# | 6629 | 6825 | C |
| 790053C11# | 6630 | 6826 | C |
| 890022B7# | 6631 | 6827 | C |
| 790392A2# | 6632 | 6828 | C |
| 890023D8# | 6633 | 6829 | C |
| 790301F1# | 6634 | 6830 | C |
| 790343A11# | 6635 | 6831 | C |
| 790421A2# | 6636 | 6832 | C |
| 790271G2# | 6637 | 6833 | C |
| 790302G12# | 6638 | 6834 | C |
| 790341E5# | 6639 | 6835 | C |
| 790283B6# | 6640 | 6836 | C |
| 790222A4# | 6641 | 6837 | C |
| 790241B8# | 6642 | 6838 | C |
| 790014C2# | 6643 | 6839 | C |
| 790402C1# | 6644 | 6840 | C |
| 790264E9# | 6645 | 6841 | C |
| 790242G4# | 6646 | 6842 | C |
| 790422F3# | 6647 | 6843 | C |

**TABLE 7**

| *NO* | *or.* | *5'position* | | *NO* | *or.* | *5'position* | | *NO* | *or.* | *5'position* |
|---|---|---|---|---|---|---|---|---|---|---|
| 6452 | B | 29372 | | 6583 | B | 547718 | | 6714 | F | 519646 |
| 6453 | B | 30198 | | 6584 | B | 547184 | | 6715 | F | 520201 |
| 6454 | B | 31007 | | 6585 | B | 547684 | | 6716 | F | 520563 |
| 6455 | B | 31126 | | 6586 | B | 547342 | | 6717 | F | 521015 |
| 6456 | B | 32735 | | 6587 | B | 548946 | | 6718 | F | 521162 |
| 6457 | B | 32264 | | 6588 | B | 549071 | | 6719 | F | 521543 |
| 6458 | B | 32898 | | 6589 | B | 550054 | | 6720 | F | 521739 |
| 6459 | B | 33582 | | 6590 | B | 549989 | | 6721 | F | 522328 |
| 6460 | B | 33519 | | 6591 | B | 550426 | | 6722 | F | 522567 |
| 6461 | B | 34836 | | 6592 | B | 550055 | | 6723 | F | 522915 |
| 6462 | B | 35795 | | 6593 | B | 550132 | | 6724 | F | 523300 |
| 6463 | B | 35548 | | 6594 | B | 550132 | | 6725 | F | 523791 |
| 6464 | B | 35825 | | 6595 | B | 551400 | | 6726 | F | 523959 |
| 6465 | B | 37239 | | 6596 | B | 551572 | | 6727 | F | 524369 |
| 6466 | B | 36761 | | 6597 | B | 551468 | | 6728 | F | 524801 |
| 6467 | B | 37045 | | 6598 | B | 550849 | | 6729 | F | 525085 |
| 6468 | B | 36761 | | 6599 | B | 552137 | | 6730 | F | 525241 |
| 6469 | B | 37958 | | 6600 | B | 552325 | | 6731 | F | 525738 |
| 6470 | B | 38636 | | 6601 | B | 552583 | | 6732 | F | 526263 |
| 6471 | B | 39813 | | 6602 | B | 553033 | | 6733 | F | 526628 |
| 6472 | B | 41140 | | 6603 | B | 553629 | | 6734 | F | 526779 |
| 6473 | B | 40575 | | 6604 | B | 553960 | | 6735 | F | 527004 |
| 6474 | B | 40526 | | 6605 | B | 553914 | | 6736 | F | 527230 |
| 6475 | B | 501495 | | 6606 | B | 554354 | | 6737 | F | 527381 |
| 6476 | B | 502410 | | 6607 | B | 555783 | | 6738 | F | 527545 |
| 6477 | B | 502586 | | 6608 | B | 555687 | | 6739 | F | 527691 |
| 6478 | B | 503233 | | 6609 | B | 556441 | | 6740 | F | 527932 |
| 6479 | B | 503749 | | 6610 | B | 557054 | | 6741 | F | 527995 |
| 6480 | B | 504488 | | 6611 | B | 556627 | | 6742 | F | 528167 |
| 6481 | B | 504206 | | 6612 | B | 557292 | | 6743 | F | 528610 |
| 6482 | B | 504310 | | 6613 | B | 557050 | | 6744 | F | 529063 |
| 6483 | B | 505455 | | 6614 | B | 815995 | | 6745 | F | 529710 |
| 6484 | B | 505877 | | 6615 | B | 817104 | | 6746 | F | 531140 |
| 6485 | B | 506655 | | 6616 | B | 817104 | | 6747 | F | 531488 |
| 6486 | B | 506513 | | 6617 | B | 816920 | | 6748 | F | 531842 |
| 6487 | B | 507532 | | 6618 | B | 820464 | | 6749 | F | 532064 |
| 6488 | B | 507742 | | 6619 | B | 821017 | | 6750 | F | 532350 |
| 6489 | B | 508050 | | 6620 | B | 821379 | | 6751 | F | 532794 |
| 6490 | B | 507771 | | 6621 | B | 821504 | | 6752 | F | 533117 |
| 6491 | B | 509120 | | 6622 | B | 822723 | | 6753 | F | 533536 |
| 6492 | B | 509646 | | 6623 | B | 823298 | | 6754 | F | 533868 |
| 6493 | B | 510137 | | 6624 | B | 823380 | | 6755 | F | 534200 |
| 6494 | B | 510953 | | 6625 | B | 824414 | | 6756 | F | 534844 |
| 6495 | B | 511165 | | 6626 | B | 824204 | | 6757 | F | 535213 |
| 6496 | B | 511526 | | 6627 | B | 825288 | | 6758 | F | 535678 |
| 6497 | B | 511993 | | 6628 | B | 825346 | | 6759 | F | 535970 |
| 6498 | B | 513012 | | 6629 | B | 825403 | | 6760 | F | 536504 |
| 6499 | B | 512983 | | 6630 | B | 826237 | | 6761 | F | 537013 |
| 6500 | B | 512781 | | 6631 | B | 824995 | | 6762 | F | 537710 |
| 6501 | B | 514155 | | 6632 | B | 826838 | | 6763 | F | 538047 |
| 6502 | B | 515036 | | 6633 | B | 828146 | | 6764 | F | 538353 |
| 6503 | B | 515287 | | 6634 | B | 827878 | | 6765 | F | 538718 |
| 6504 | B | 516292 | | 6635 | B | 827571 | | 6766 | F | 539188 |
| 6505 | B | 516234 | | 6636 | B | 828472 | | 6767 | F | 539471 |
| 6506 | B | 516337 | | 6637 | B | 828484 | | 6768 | F | 539910 |
| 6507 | B | 517347 | | 6638 | B | 828691 | | 6769 | F | 540774 |
| 6508 | B | 517005 | | 6639 | B | 829507 | | 6770 | F | 540962 |
| 6509 | B | 516888 | | 6640 | B | 829169 | | 6771 | F | 541721 |
| 6510 | B | 516234 | | 6641 | B | 828763 | | 6772 | F | 542198 |
| 6511 | B | 517560 | | 6642 | B | 829769 | | 6773 | F | 542644 |
| 6512 | B | 517337 | | 6643 | B | 831582 | | 6774 | F | 543180 |
| 6513 | B | 518756 | | 6644 | B | 830481 | | 6775 | F | 543877 |
| 6514 | B | 518943 | | 6645 | B | 831468 | | 6776 | F | 544601 |
| 6515 | B | 519833 | | 6646 | B | 831670 | | 6777 | F | 544866 |
| 6516 | B | 520123 | | 6647 | B | 832293 | | 6778 | F | 545442 |
| 6517 | B | 520574 | | 6648 | F | 28484 | | 6779 | F | 545948 |
| 6518 | B | 520888 | | 6649 | F | 29043 | | 6780 | F | 546209 |
| 6519 | B | 522154 | | 6650 | F | 29656 | | 6781 | F | 546585 |
| 6520 | B | 523041 | | 6651 | F | 30157 | | 6782 | F | 546960 |
| 6521 | B | 522052 | | 6652 | F | 30712 | | 6783 | F | 547114 |
| 6522 | B | 522217 | | 6653 | F | 31175 | | 6784 | F | 547726 |
| 6523 | B | 523035 | | 6654 | F | 31658 | | 6785 | F | 548045 |
| 6524 | B | 524995 | | 6655 | F | 31902 | | 6786 | F | 548480 |
| 6525 | B | 523567 | | 6656 | F | 32638 | | 6787 | F | 548561 |
| 6526 | B | 523477 | | 6657 | F | 33203 | | 6788 | F | 548775 |
| 6527 | B | 523967 | | 6658 | F | 33804 | | 6789 | F | 549037 |
| 6528 | B | 525211 | | 6659 | F | 34164 | | 6790 | F | 549153 |
| 6529 | B | 525215 | | 6660 | F | 34426 | | 6791 | F | 549597 |
| 6530 | B | 526133 | | 6661 | F | 35131 | | 6792 | F | 550049 |
| 6531 | B | 525674 | | 6662 | F | 35675 | | 6793 | F | 550520 |
| 6532 | B | 526561 | | 6663 | F | 36097 | | 6794 | F | 550890 |
| 6533 | B | 526697 | | 6664 | F | 36641 | | 6795 | F | 550997 |
| 6534 | B | 526715 | | 6665 | F | 36835 | | 6796 | F | 551040 |
| 6535 | B | 526844 | | 6666 | F | 37236 | | 6797 | F | 551247 |
| 6536 | B | 527261 | | 6667 | F | 38287 | | 6798 | F | 551854 |
| 6537 | B | 527503 | | 6668 | F | 38711. | | 6799 | F | 552333 |
| 6538 | B | 528775 | | 6669 | F | 39117 | | 6800 | F | 552603 |
| 6539 | B | 528249 | | 6670 | F | 39798 | | 6801 | F | 552823 |
| 6540 | B | 530307 | | 6671 | F | 500539 | | 6802 | F | 553207 |
| 6541 | B | 527772 | | 6672 | F | 501016 | | 6803 | F | 553898 |
| 6542 | B | 529406 | | 6673 | F | 501319 | | 6804 | F | 554298 |
| 6543 | B | 527752 | | 6674 | F | 501632 | | 6805 | F | 554767 |
| 6544 | B | 529829 | | 6675 | F | 502155 | | 6806 | F | 555323 |
| 6545 | B | 529907 | | 6676 | F | 502623 | | 6807 | F | 555595 |
| 6546 | B | 529574 | | 6677 | F | 503025 | | 6808 | F | 555965 |
| 6547 | B | 529635 | | 6678 | F | 503681 | | 6809 | F | 556248 |
| 6548 | B | 530391 | | 6679 | F | 504389 | | 6810 | F | 815116 |
| 6549 | B | 531516 | | 6680 | F | 504744 | | 6811 | F | 815376 |
| 6550 | B | 532154 | | 6681 | F | 505468 | | 6812 | F | 815849 |
| 6551 | B | 532606 | | 6682 | F | 505652 | | 6813 | F | 816098 |
| 6552 | B | 533407 | | 6683 | F | 505822 | | 6814 | F | 818726 |
| 6553 | B | 533664 | | 6684 | F | 505833 | | 6815 | F | 819337 |
| 6554 | B | 533916 | | 6685 | F | 506933 | | 6816 | F | 820080 |
| 6555 | B | 534707 | | 6686 | F | 507220 | | 6817 | F | 820750 |
| 6556 | B | 533482 | | 6687 | F | 507559 | | 6818 | F | 821170 |
| 6557 | B | 534614 | | 6688 | F | 508216 | | 6819 | F | 821815 |
| 6558 | B | 534935 | | 6689 | F | 508619 | | 6820 | F | 822490 |
| 6559 | B | 536823 | | 6690 | F | 509329 | | 6821 | F | 822789 |
| 6560 | B | 535986 | | 6691 | F | 509783 | | 6822 | F | 823244 |
| 6561 | B | 536143 | | 6692 | F | 510383 | | 6823 | F | 823762 |
| 6562 | B | 537505 | | 6693 | F | 510729 | | 6824 | F | 823964 |
| 6563 | B | 537618 | | 6694 | F | 511188 | | 6825 | F | 824245 |
| 6564 | B | 538165 | | 6695 | F | 511773 | | 6826 | F | 824609 |
| 6565 | B | 538702 | | 6696 | F | 511869 | | 6827 | F | 824948 |
| 6566 | B | 540278 | | 6697 | F | 512946 | | 6828 | F | 825490 |
| 6567 | B | 539156 | | 6698 | F | 513202 | | 6829 | F | 826064 |
| 6568 | B | 539619 | | 6699 | F | 513821 | | 6830 | F | 826405 |
| 6569 | B | 540115 | | 6700 | F | 514322 | | 6831 | F | 826480 |
| 6570 | B | 540724 | | 6701 | F | 514811 | | 6832 | F | 827089 |
| 6571 | B | 541484 | | 6702 | F | 515101 | | 6833 | F | 827418 |
| 6572 | B | 540968 | | 6703 | F | 515611 | | 6834 | F | 827496 |
| 6573 | B | 542062 | | 6704 | F | 515911 | | 6835 | F | 827730 |
| 6574 | B | 541898 | | 6705 | F | 516123 | | 6836 | F | 828180 |
| 6575 | B | 543100 | | 6706 | F | 516169 | | 6837 | F | 828348 |
| 6576 | B | 543846 | | 6707 | F | 516215 | | 6838 | F | 828729 |
| 6577 | B | 543820 | | 6708 | F | 516305 | | 6839 | F | 830099 |
| 6578 | B | 544382 | | 6709 | F | 517240 | | 6840 | F | 830281 |
| 6579 | B | 545158 | | 6710 | F | 517993 | | 6841 | F | 830491 |
| 6580 | B | 545678 | | 6711 | F | 518174 | | 6842 | F | 830550 |
| 6581 | B | 545905 | | 6712 | F | 518756 | | 6843 | F | 830576 |
| 6582 | B | 546683 | | 6713 | F | 519133 | | | | |

### Publications Cited in the Specification

Adames et al., 1985, Nature, 318 : 533-538.
Aldous, M.B. et al., 1992, J. Infect. Dis., 166 : 646-649.
Alexander et al., 1987, Mol. Cell. Biol., 7 : 1436-1444.
Allan, G. M. et al., 1995, Vet. Microbiol., 44 : 49-64.
Altschul, S.F. et al., 1990, J. Mol. Biol., 215 : 403-410.
Altschul et al., 1993, Nature Genetics, 3 : 266-272.
Altschul et al., 1997, Nucl. Acids Res., 25 : 3389-3402.
Ansubel et al., 1989, Current Protocols in Molecular Biology,
Arlinghaus, H.F. et al., 1997, Anal. Biochem., 69, 18, 3747-53.
Bai, M. Et al., 1993, J. Virol., 67 : 5198-5205.
Barany, F., 1911, PNAS. USA, 88 : 189-193.
Beattie, K. et al., 1993, Clin. Chem., 39(4) : 719-721.
Bernoist and Chambon, 1981, Nature, 290 : 304-310.
Borman, S., 1996, Chem. Eng. News, 74(50) : 42-43.
Braun, J. et al., 1994 Ann., Rheum Dis 53 : 100-105.
Brinster et al., 1982, Nature, 296 : 39-42.
Buckholz, R.G., 1993, Yeast systems for the expression of heterologous gene products. Curr. Op. Biotechnology 4 : 538-542.
Burg, J.L. et al., 1996, Mol. and Cell. Probes, 10 :257-271.
Campbell, LA. et al., 1992 J. Clin. Microbiol. 30 : 434-439.
Casas-Ciria J. et al., 1996
Chatelier, R.C. et al., 1995, Anal. Biochem., 229, 1, 112-118.
Chee, M. et al., 1996, Science, 274 : 610-613.
Chu, B.C.F. et al., 1986, NAR, 14 : 5591-5603..
Chu, P.W.G. et al., 1993, Virus Research, 27 : 161-171.
Clark, E.G., 1997, American Association of Swine Practitioners, 499-501.
Cole et al., 1985, in Monoclonal Antibodies and Cancer Therapy, Alan R. Liss, Inc., pp. 77-96.
Cote et al., 1983, PNAS USA, 80 : 2026-2030.
Cserzo, M., Wallin, E., Simon, I. von Heijne G and Elofsson, A., 1997, Prot. Eng., 10: 673-676.
DeBoer et al., 1980, Scientific American, 242 : 74-94.
DeBoer et al., 1983, PNAS USA, 80 : 21-25.
Derisi, J. et al., 1996, Nature Genet, 14 : 457-460.
Distance Relationships: Atlas of Protein Sequence and Structure, Washington : National Biomedical Reserach Foundation.
Duck, P. et al., 1990, Biotechniques, 9 : 142-147.
Dulac, G.C. et al., 1989, Can. J. Vet. Res., 53 : 431-433.
Edwards, C.P., and Aruffo, A., 1993, Current applications of COS cell based transient expression systems. Curr. Op. Biotechnology 4 : 558-563.
Edwards, S. et al., 1994, Vet. Rec., 134 : 680-681.
Erlich, H.A., 1989, In PCR Technology. Principles and Applications for DNA Amplification. New York : Stockton Press.
Falsey, et al., J. Infect. Dis. 168 :493-496.
Fanger and Drakeman, 1995, Drug News and Perspectives, 8 : 133-137.
Felgner, et al., 1987, Proc. Natl. Acad. Sci., 84 : 7413.
Fodor, S.P.A. et al., 1991, Science, 251 : 767-771.
Fontes, E.P.B. et al., 1994, J. Biol. Chem., Vol. 269, N° 11 : 8459-8465.
Fox, G. Et al., 1989, J. Gen. Virol., 70 : 625-637.
Fraley et al., 1980, J. Biol. Chem., 255 : 10431.
Gardner et al., 1981, Nucl. Acids Res. 9 : 2871
Gaydos, C.A. et al., 1994 J. Clin. Microbiol. 32 : 903-905.
Grayston, J.T. et al., 1986 N. Engl. J. Med., 315 : 161-168.
Grayston, JT. et al., 1996 Rev., Med Interne 17, 45S-47S.
Gonnet et al., Science, 256 : 1443-1445.
Green Publishing Associates and Wiley Interscience, N.Y.
   Pearson and Lipman, 1988, PNAS USA, 85(8) : 2444-2448.
Grosschedl et al., 1984, Cell, 38 : 647-658.
Guateli, J.C. et al., 1990, PNAS. USA, 87 : 1874-1878.
Hackland, A.F. et al., 1994, Arch. Virol., 139 : 1-22.
Hahn, DL. Et al., 1991 JAMA. 266
Haidl, et al., 1992 N. Engl. J. Med. 326 :576-577.
Haidl, et al., Chlamydial infections 1994
Hammer et al., 1987, Science, 235 : 53-58.
Hanahan, 1985, Nature, 315 : 115-122.
Hanson, S.F. et al., 1995, Virology, 211 : 1-9.
Harding, J.C., 1997, American Association of Swine Practitioners, 503.
Harding, R.M. et al., 1993, Journal of General Virology, 74 : 323-328.
Hashiguchi, K. et al., 1992 J. Laryngol. Otol. 106 : 208-210.
Hayashi, S. and Wu, H.C., 1992, in N.M. Hooper and A.J. Turner (ed.) Lipid Modification of Proteins: A Practical Approach. Oxford University Press, New York, pp. 261-285.
Heinkoff and Heinkoff, 1993, Proteins, 17 : 49-61.
Herrera-Estrella et al., 1983, Nature, 303 : 209-213.
Herrera-Estrella, 1984, Nature, 310 : 115-120.
Heyraud-Nitschke, F. et al., 1995, Nucleic Acids Research, Vol. 23, N° 6.
Higgins et al., 1996, Meth. Enzymol., 266 : 383-402.
Homer, G.W., 1991, Surveillance 18(5) : 23.
Houbenweyl, 1974, in Meuthode der Organischen Chemie, E. Wunsch Ed., Volume 15-1 et 15-II, Thieme, Stuttgart.
Hueck, C.J., 1998, Molec. Biology Rev., 62 : 379-433.
Huovinen, P. et al., 1989 Ann., Intern Med 110 : 612-616.
Huse et al., 1989, Science, 246 : 1275-1281.
Huygen, K. et al., 1996, Nature Medicine, 2(8) : 893-898.
Innis, M.A. et al. 1990. in PCR Protocols. A guide to Methods and Applications. San Diego : Academic Press.
Inoue et al., 1987, Nucl. Acids Res., 15 : 6131-6148.
Inoue et al., 1987, FEBS Lett. 215 : 327-330.
Jackson, LA. et al., 1997 Am., J. Pathol. 150. : 1785-1790.
Jantos et al., 1997, J. Clin. Microbiol., 35(3) : 620-623.
Kabat E. Et al., 1983, Sequences of Proteins of Immunological Interest, U.S. Dept. Of Health and Human Services.
Kaneda, et al., 1989, Science, 243 : 375.
Kelsey et al., 1987, Genes and Devel., 1 : 161-171.
Kievitis, T. et al., 1991, J. Virol. Methods, 35 : 273-286.
Kleemola, M. et al., 1988, J. Infect. Dis. 157 : 230-236.
Kohler, G. et al., 1975, Nature, 256(5517) : 495-497.
Kollias et al., 1986, Cell, 46 : 89-94.
Kozbor et al., 1983, Immunol. Today, 4 : 72.
Krone, J.R. et al., 1997, Anal. Biochem., 244, 1, 124-132.
Krumlauf et al., 1985, Mol. Cell. Biol., 5 : 1639-1648.
Kuo, CC. et al., 1988, J. Clin. Microbiol. 26 : 812-815.
Kuo, CC. et al., 1993, J. Infect. Dis. 167 : 841-849.
Kwoh, D.Y. et al., 1989, PNAS. USA, 86 : 1173-1177.
Ladany, S. et. al., 1989, J. Clin. Microbiol. 27 : 2778-2783.
Laitinen, K. et al., 1997. Chlamydia pneumoniae Infection Induces Inflammatory Changes in the Aortas of Rabbits. Infect. Immun. 65:4832-4835.
Lazarowitz, S. G. et al., 1989, The EMBO Journal, Vol. 8 N° 4 : 1023-1032.
Leder et al., 1986, Cell, 45 : 485-495.
Lee, C.A., 1997, Trends Microbiol., 5 : 148-156.
Leininger, E. et al., 1991, PNAS USA, 88 : 345-349.
Lipshutz, R.J. et al., 1995, Biotechniques, 19(3) : 442-447.
Liu, H. et al., 1997, J. Gen. Virol. 78(Pt6) : 1265-1270.
Livache, T. et al., 1994, NAR, 22(15) : 2915-2921.
Lockhart, D.J. et al., 1996, Nature Biotechnol., 14 : 1675-1680.
Longbottom et al., 1998, Infect Immunol., 66 : 1317-1324.
Luckow, V.A., 1993, Baculovirus systems for the expression of human gene products. Curr. Op. Biotechnology 4 : 564-572.
Lukacova, M. Et al., 1994, Infect. Immunol. June, 62(6): 2270-2276.
MacDonald, 1987, Hepatology, 7 : 425-515.
Mankertz, A. et al., 1997, J. Virol., 71 : 2562-2566.
Mason et al., 1986, Science, 234: 1372-1378.
Matson, R.S. et al., 1994, Analytical Biochemistry, 217: 306-310.
Matthews, J.A. et al., 1988, Anal. Biochem., 169 : 1-25.
McNeilly, F. et al., 1996, Vet. Immunol. Immunopathol., 49 : 295-306.
Meehan, B.M. et al., 1997, J. Gen. Virol., 78 : 221-227.
Mérel, P., 1994, De la PCR aux puces à ADN, Biofutur, 139 : 58.
Merrifield, R.D., 1966, J. Am. Chem. Soc., 88(21) : 5051-5052.
Midoux, 1993, Nucleic Acids Research, 21 : 871-878.
Miele, E.A. et al., 1983, J. Mol. Biol., 171 : 281-295.
Moazed, T.C. et al., 1997. Murine Model of Chlamydia pneumoniae Infection and Atherosclerosis. J. Infect. Dis. 175:883-890.
Mogram et al., 1985, Nature, 315 : 338-340.
Mordhorst, C.H. et al., 1992 Eur., J. Clin. Microbiol. Infect Dis 11 : 617-620.
Morrison et al., 1984, PNAS USA, 81 : 6851-6855.
Morrison, R.P. et al., 1995. Gene Knockout Mice Establish a Primary Protective Role for Major Histocompatibility Complex Class II-Restricted Responses in Chlamydia trachomatis. Infect. Immun. 63:4661-4668.
Murphy, F.A. et al., 1995, Sixth Report of the International Committee on Taxonomy of Viruses. Springer-Verlag Wien New York.
Nakai, K. and Kanehisa, M., 1991, Proteins, 11 : 95-110.
Nielsen, H. et al., 1997, Protein Engin., 10: 1-6.
Neuberger et al., 1984, Nature, 312: 604-608.
O'Donell-Maloney, M.J., 1996, Trends Biotechnol., 14: 401-407.
Ogawa, H. et al., 1992 J. Laryngol. Oto 106 : 490-492.
Olins, P.O., and Lee, S.C., 1993, Recent advances in heterologous gene expression in E. coli. Curr. Op. Biotechnology 4 : 520-525.
Omitz et al., 1986, Cold Spring Harbor Symp. Quant. Biol., 50: 399-409.
Pagano et al., 1967, J. Virol., 1: 891.
Peterson, E.M. et al., 1998, Infect. Immunol. Aug., 66(8): 3848-3855.
Peterson, E. et al., 1988. Protective Role of Magnesium in the Neutralization by Antibodies of *Chlamydia trachomatis* Infectivity.
Pierschbacher and Ruoslahti, 1987, J. Biol. Chem., 262 : 17294-17298.
Pinkert et al., 1987, Genes and Devel., 1 : 268-276.
Pugsley, A.P., 1993, Microbiol. Rev., 57 : 50-108.
Puolakkainen, M. et al., 1993 J. Clin. Microbiol. 31 : 2212-2214.
Rank, R.G. et al., 1988. Susceptibility to reinfection after a primary chlamydial genital infection. Infect. Immun. 56:2243-2249.
Readhead et al., 1987, Cell, 48 : 703-712.
Reeves, P.R. et al., 1996, in Bacterial Polysaccharide Synthesis and Gene Nomenclature, Elsevier Science Ltd., pp. 10071-10078.
Roivainen, M. Et al., 1994, Virology, 203 : 357-365.
Rolfs, A. et al., 1991, In PCR Topics. Usage of Polymerase Chain reaction in Genetic and Infectious Disease. Berlin : Springer-Verlag.
Salzberg et al., 1998, Nucl. Acids Res., 26 : 544-548.
Sambrook, J. et al., 1989, In Molecular cloning : A Laboratory Manual. Cold Spring Harbor, NY : Cold Spring Harbor Laboratory Press.
Sanchez-Pescador, R, 1988, J. Clin. Microbiol., 26(10) : 1934-1938.
Sani, 1985, Nature, 314 : 283-286.
Sarver et al., 1990, Science, 247 : 1222-1225.
Schachter, J. 1980. Chlamydiae, p.357-365. In E.H. Lennette (ed.), Manual of clinical microbiology, 3^{rd} ed. American Society for Microbiology, Washington, D.C.
Schneewind, O. Et al., 1995, Science, 268 : 103-106.
Schwartz and Dayhoff, eds., 1978, Matrices for Detecting Karlin and Altschul, 1990, PNAS USA, 87 : 2267-2268.
Segev D., 1992, in « Non-radioactive Labeling and Detection of Biomolecules ». Kessler C. Springer Verlag, Berlin, New-York : 197-205.
Sheldon, E.L., 1993, Clin. Chem., 39(4) : 718-719.
Shiver, J.W., 1995, in Vaccines 1995, eds Chanock, RM. Brown, F. Ginsberg, H.S. & Norrby, E.), pp.95-98, Cold Spring Harbor Laboratory Press, Cold Spring Harbor, New York.
Shoemaker, D.D. et al., 1996, Nature Genet, 14 : 450-456.
Shor, A. et. al., 1992 S. Afr. Med. J. 82 : 158-161.
Sosnowsky et al., 1997, PNAS., 94, 1119-1123.
Struyve, M. et al., 1991, J. Mol. Biol., 218 : 141-148.
Sundelof, et al., 1993 Scand. J. Infec. Dis. 25 :259-261.
Sutcliffe, I.C. and Russell, R.R.B., 1995, J. Bacteriol. 177 : 1123-1128.
Swift et al., 1984, Cell, 38 : 639-646.
Takeda et al., 1985, Nature, 314 : 452-454.
Tascon, R.E et al., 1996, Nature Medicine, 2(8) : 888-892.
Thom, D.H. et al., 1990 Am. J. Epidemiol 132 : 248-256.
Thomas, GN. et al., 1997 Scand., J. Infect. Dis. Suppl 104, 30-33.
Tischer, I. et al., 1982, Nature, 295 : 64-66.
Tischer, I. et al., 1986, Arch. Virol., 91 : 271-276.
Tischer, I. et al., 1988, Zentralbl Bakteriol Mikrobiol Hyg [A] 270 : 280-287.
Tischer, I. et al., 1995, Arch. Virol., 140 : 737-743.
Tompson et al., 1994, Nucl. Acids Res., 22(2) : 4673-4680.
Urdea, M.S., 1988, Nucleic Acids Research, 11 : 4937-4957.
Villa-Kamaroff et al., 1978, PNAS USA, 75 : 3727-3731.
Wagner et al., 1981, PNAS USA, 78 : 1441-1445.
Walker, G.T. et al., 1992, NAR 20 : 1691-1696.
Walker, G.T. et al., 1992, PNAS. USA, 89 : 392-396.
White, B.A. et al., 1997, Methods in Molecular Biology, 67, Humana Press, Towota.
Yamamoto et al., 1980, Cell, 22 : 787-797.
Yershov, G. et al., 1996, PNAS., USA, 93 : 4913-4918.

### SEQUENCE LISTING

<110>Genset SA
<120>Chlamydia pneumoniae genomic sequence and polypeptides, fragments thereof and uses thereof, in particular for the diagnosis, prevention and treatment of infection
<151>1997-11-21
<160> 1
<210>1
<211>1230025
<212>DNA
<213>Chlamydia pneumoniae
<400>1

atagaaaact attaaaaaat cattgattct gtcgggaaag tatgcggata aaattcagag 60
agaataagga gaggaagatg acaaggcaga gttatgtttt gggcaattgg aaaatgcaca 120
aaacaatcca agaagctaaa gagtatgttc aaacattagc ttctntacta caaggagaac 180
ctctttcctg cactataggc atagcttctc catttacctc tttgagagcg attcatgaga 240
tgataaacac tacgggagct tttctctggt tgggagcaca aaatgtccat cccgagcttt 300
cgggtgcttt tactggagaa atttccttac ctatgcttaa ggaggtagga gtggaatttg 360
ttttagtagg tcactccgag cgtcgtcata tttttggaga gagtgatgcc tttattgctt 420
caaaggtaaa gtctgtagct caggcgggac tcgtgcctgt tctttgtgtt ggagagagct 480
tagaagttcg tgaagaggga aaggcgcatc aggtaatcaa aaaacagttg cttttgggat 540
tggaacagat ggataatggt tccgaatttt tgatcgccta tgaaccagta tgggctatcg 600
gcacagggaa ggtggcagaa gcttcggatg tgcaagatat tcatatgttt tgtcgtgagg 660
tagtggcaga gaggttctca gaagctacag ctgaagagat ttcgattttg tacggaggat 720
ctgtgaaggt cgataatgct cagcgatttg ggcaatgtag cgacgtcgat ggtcttttag 780
ttggcggant tctttagang ggcaaagttt ttttgaagtc gctaaaaatt ttaatgtata 840
atttgtgaga gttatgagat ttttttgtct attttttctt gggttcctag gatcttttca 900
ttgtgttgct gaagacaagg gcgtggattt atttggagtc tgggacgata accaaattac 960
agagtgtgac gatagttaca tgacagaggg tcgtgaagag gttgaaaagg tagtggacgc 1020
ttagtccatc ggcttttatt tatattctcc ctaaggaagt cctgtattga agatcgcttt 1080
ctcatagata gaagtaattt tcagatagtc aataattggt ttttttaaga gaatgctagg 1140
caggtgctcg tgtttgggca tttgattaag tctacatgaa tctggaggga gagattcttc 1200
tggtattgag aagtagaaca aaaaacaagg atcagacgtt ctccgatgtc ttcctaatcg 1260
atgtctttaa ataaggagat tggcatgaca gtgttgtttt acgcattttt attcattttc 1320
ctttttctat gtgtaattct ttgtggctta atcctggttc aagagagtaa gagcatgggg 1380
ttaggttctt cgttcggcgt ggattctgga gattctgtct ttggtgtctc tactccagat 1440
attttgaaaa aagtgacttc atngtgtgct gttgctttct gcataggttg tttactactt 1500
tcattttcca cgaatctctt ggggaaaaag ttagatgcta aagaatttct attgcctgct 1560
gctgaggaga gcgacactca agcttcttct gagagcgttg aagcagatga atcctagcct 1620
atttgcggaa ttaggtgttg tctagattga agtgcaataa agctagcaag tttttatctt 1680
catacgagat atgagtgtac ggtcggataa gagtagaaat ctttcttttg ttcctatggt 1740
taagaagtcc tttggcttcc ttaaagagta tgactcttat caacccaaga aatgttttag 1800
atccaagtgc ttgtcgtacg agatttcttc acagagctct gccaagccca tgttcagact 1860
atgattagac gtttagaata ttacggcagt cctattttaa ggaaaaagtc ttccccaatt 1920
gcagagatca cagatgagat tcgtaatctc gtgagtgata tgtgtgatac tatggaagca 1980
catcgtggtg tcggtttagc cgctcctcag gtagggaaaa acgtcagttt atttgtcatg 2040
tgtgtagata gagagactga ggatggagag ttgattttct ctgagtctcc gagggtattt 2100
atcaatcctg ttctatcaga tccttctgaa accccgatca taggtaaaga aggatgtctt 2160
tctattcctg gattgcgagg agaagtattc cgccctcaga aaatcacagt gaccgctatg 2220
gatctcaatg gtaaaatatt tactgagcac ttggaaggat tcactgcacg tatcattatg 2280
cacgagactg accatctgaa tggagttctc tatattgatc ttatggaaga acccaaagat 2340
cctaaaaaat ttaaagcctc tttagagaag atcaaacgtc gctacaatac acacttgagt 2400
aaagaagaac tagtttctta attgctcttc agtctgatgt aggtgatatt ttcttgtctc 2460
ttgcgtcaca tttgttgtca gctttgctta tttccccgaa caaatttcgt caaaggtttt 2520
aaaatgtgtc ttgctgattt ttgctaagag ctctttccct cgttgcttag cgatctctct 2580
tcctgctgct ttgacattga atccagcacc tttaggaagc tgtacttgat attgttcttc 2640
caacttctgt atcgactgta caaatgcatc tctagccaat atagaagctg ctgctacgac 2700
tacatcttgt tctgcacgtg gcttttgtat taaagtaata tcggtttctt ttttttgaag 2760
tgctttgagt agggtgtatt ctgaagctgc aaactgatct gaaatagcaa agacatctcc 2820
tgcaggtttg ggtgctaagt tgttgataac agtagcgtgg gcccaagcaa gaagtgtatt 2880
taaattctgg aatttcccat atagctcgtt atatttttct gggtatagaa tgatgacatc 2940
gcagacacat agtgagcgta tgatacgtgc taaagaagcg attttcgtgt ctttgagatt 3000
tttagagtct tggactttat tctcatagag tttttttaag atctctgcat tcgatgcata 3060
gactgccgca atacataaag ggccaaaaaa atcacctttc cctgattcat cgactcccaa 3120

ccttggacga aggtcttgct ctacccttgc atgggtgaag gtatgaagga tttctggttc 3180
taagaaaaat tctatgaatt cctcacttcc tttaccttgg attacgagtt tcccggaggg 3240
gtatagagtg caggtaacag tgttagagcg agcttgaaat acggtattct gtggctgaga 3300
aaagataaaa tttttttctt ttagctgatc tcttaaattg ttttgagcag aagttgttaa 3360
agtaacaaca aatggtggcg gcatgcagga catctaccta taaattatag aaaaaattta 3420
gaacgattga attcctagtc ctaggaatcc aggatgcaaa gtgctcatac tttttatttt 3480
tgcctactat atcgtatagt tatgacaact tccaagtaca aattaaacaa caattttgta 3540
tatttctgca aataactgcg gggagccaaa gatacaagag tgtaagatgt ctagtatttt 3600
acattcttaa ggttttgaga aacactatat aggtaatcat gcaagaacac atacataaag 3660
aattgctaca tctaggtgaa atctttcgct catcacgaga gtctcaatcc ctatcgttaa 3720
aggatgtaga ggctgcaacc tcgatacgat atagttgttt agaagctatt gaacaggggt 3780
gtttaggaaa attgatttct ccagtttatg ctcagggatt tattaagaaa tacgctacgt 3840
atcttgggtt ggatggagat agtatcttac aagaacatcc ttatgtcatg aaaattttta 3900
aagagttttc agatcataat atggagatgc ttttagacct tgaatcgatg ggaggaagga 3960
attctccgga aagagcaatt cattcttggt cgaatctttg gtgggcaggg ctgatcatta 4020
taggtggcat catggtgtgg tggctcggat cgttgttttc tattttttaa cttaaggtct 4080
gttggtctct atttagacca acagatagga tggagtactt cttaccctaa tttgaatagt 4140
tgtgagactt tctgagcccc ttgcaaaagt tgtctttgat tttcaggaga ccagggatct 4200
tcatccctac cctcatcatc ctcaggacct aagggctctc ctaaaccatc acttcctgtt 4260
gtactatcac agccatcggt tcggtctctt ctctgttggg agcttcctcc aacaccgaag 4320
gcactggcat ggaatgccaa atcctccgcc tgtcacaata ccatctcttt ctatttcgtc 4380
gtcatcccac gggtcaggag gtggtggagc ttgtctagga gcttcgcctt gggtttgtct 4440
ccaaatgttc tctccagctg tggtggtgac taataccttt ctaagaattc tttgccaagg 4500
accttggact ctagggtcaa gaacagtttg ctctggatgt agaggatcta aaggaagata 4560
tccttttgaa agaaggcata gaaggacaag gacttcaaat acctgagatt ctaaactcaa 4620
accaatacca ggacctaaga aagtcgcaag atcactggca acaagtcttt ttagtgattt 4680
ttttattaag tccacaattt gtttagcagc ttgtcctaaa ggacccttgt aaccggattc 4740
taagctcaaa acctctaacc tggataattt ttgtaataaa gtcctaaaat tgagtttgca 4800
tgtaacacgg cattcttgag cggggtgctt cttttgttga ggagctggcg cgcctcctac 4860
agcaccttgt agctgttctt cgtctgttgg aggtggggga cagggttgct gttctggttg 4920
tagaatccaa caagtaggag gattgtcaga tgaagttaca aaggaaagag ccccctctga 4980
aacagcttga agtatgagac tccatggtgg tgtctgttgc atgtcttcgc ctaagccgtt 5040
gttttgtaaa agcttgacta gagattcacc catgcatctt ggttgagctg cttttagtag 5100
ttcccgatgt agcttgcaac cattttcaac ctcctgttta tccccgtcac ccaacggttc 5160
accttgctca atcttctgtg tacagtgcgg tagctgttgg aaagacatac caacacaaat 5220
aggtccgtat tgctgtataa gttgttgtag aaattgtgct gagggattat caggatcatc 5280
cacggtctct aagcagcatg cgcaacatcc tccccatatc cattgtaaga aaccaggaca 5340
atgctctctg caccagggag tcccggttct ataacaataa tgtcctagac gttgcattct 5400
agcactatct aagagattat taagtaacga gcctgcttgt tctaccatac ctagcacatt 5460
gtctgatccg agcgagatac ccatttcacg acctccagtc ccaggctgtt gtgtaattac 5520
acgtccacct tgagagccag attggcctcc ttcttgcagt ggaatatctt ctcctccggg 5580
atttggtggg ggcgtttcgc gataataaca attacaattt ccacatggaa atgtcatgag 5640
gcttctcctt tagtgaggtt gagttgtatg gatttttata attacgagtt cttctagaaa 5700
ggaattatag aatgttcaga tagagtattt taaagcgggt tatttcttat tggatgatta 5760
agaaaaatag gaaatcttga ttatgttttt ttgtcgagat tacttttaga gaaagagttt 5820
aaagtgatcg cgggttatta gtcaatttat tttttaattt aacatagatc ctctcttaag 5880
tttccttgtt gtgatgggat aaatatttgg ggaagaaatg ccgcaaaatt ttttctaagt 5940
tctcaaactt ttcaagaaaa catagattct tggatagaac aaaagcttcg cggcaaaaaa 6000
taggaatctt attctttgtt tatttttatc cctaaaaata gagtttgtaa atgacccgag 6060
cattcaagga tacgctgttg atacgccaaa ttcccatcct catcatcatc ttcattttca 6120
tcttcttctt cgagacttga catgagaggg acagattctg caggggtagg acaatcaaga 6180
ctttcctgtc ttactaatga gggtttcttt tttaaaaacg atgctgaagt tttccctgtt 6240
ctatagctgt attgtagtcg atagaagatg gtcataatcg gatcatctat catgtgtata 6300
tctacgacag atgtctcatc ggtatttgca gggcaatagc caatcgctaa catcaataaa 6360
acgacgatat cacgaaaatt tgggcacgta taacatattt ctccacctgt gtgctttaga 6420
atagaagatg tgctttgtga tgcagataag agaaagctat tcagcatttt cacaatcagg 6480
agaccaaaag gacctaaagc tgaaaaatct cctgttttta gattggtgac ttggattttt 6540
cctaattgtc tggccatgcc attgtcattg tatttttttg ctacgtactg gggctctgca 6600
ccgactgaag atgggcgtcg cgcatctctt gatccacagg ataggcgtga cgtcgttaag 6660
gggctcgtat ttaagatata gcacgcagga atgcgtgtag aattagaagc agaagatgtt 6720
cctgaggctt cctctgattc tatagttttt tcccaagacc ttgtagcaat aaagcttagc 6780
acggcttgga ctagtgaatc gtctagaccg cgttctctaa tgctgttgac tccttcaagg 6840
aaaaagtttt gagaaaggct ctgcatcgtt ccatagagct gggttttagc ttccgagcaa 6900
tgctgtctaa attcattttt ctgttcttca gacaaaatag tgtttttttc tacaagttcc 6960

attaagttga ggcccatagt tttagcagct aaagcaacgg caataggacc atgggtctgt 7020
tcgcattcat ggacaagtcc agcaacctga ttatcctctc tgtctaagca acacgcttta 7080
tacgttccac aaatgagtct tccaaaagat ccgcaacgct gaccacatcc agttgcgtaa 7140
ctgtcataac aatactctgt acatccttgc gttcgtctat gtgatagaat ggagtctacc 7200
aattctcctg cttcttggat catgtgtatt gtactttcat caccttgagc agtattaaat 7260
tggacacgtt tctctacttt tgtaatttta gtaggttgcg tagttatagg agcagctcct 7320
ccactcgaac cagcttcttc gcttatatct gaagccgatt gccgcgttgt ttcttctgtt 7380
gttgttactt caacccattc gcagttgaaa tttccacaca ttctgaacca agaattattt 7440
gggcaaacca tagtcgtacc tttttaatat ttagatttat ttaggtttta gagagttgaa 7500
aagaactctc taagaacagg aaaagagtcg gtatgctctg cttgcgactg tgctgtttgg 7560
aatgggaaac gaagggatcc cagtgtctat agcacagtag agtgaggcta aacgtcaaaa 7620
ttgatttgaa gaaatttgaa ttgctgacaa gagggggaga ggaattaaaa acgtcggacg 7680
gaagaagatg tttagagcat gtataaagaa aattttaaat tagatgcatt atttcatggg 7740
tcttacctta tttagactta agatggtaag gaagtctata aaatcatttt ttttaaaatc 7800
tacggaattt aaaaaattac ttgataatga tgtggagtcg tagaagtgca aactcactag 7860
aaatttcctg aatttgagaa ggagtttaaa ataggtattc ttgaaaagat caatgcacgt 7920
gtagtggaaa gaaaggcgta acctacactg gaaggagttt cttttagaag aaggtttctt 7980
ttttcgttgt taatgacaaa aactctctaa gaacatcgtg tcaaccgttt ttattgttgt 8040
gtttatttaa aaaaaagatt atatccttca tgcaaattgg tgctgttgga aacaaatgtt 8100
gaaaatccag aaaaaaagaa tgtgtgtcag cgtagtcatc acggtaggcg ccatagtggg 8160
gtttttcaat tctgcagacg cagcaccaaa gaaaaagaag atccctatac agattctcta 8220
ctcctttact aaagtctctt cctatttaaa aaacgaagac gcaagtacta tattttgcgt 8280
cgatgtggat cgtggacttc tccagcatcg gtatttaggt agtccaggat ggcaggaaac 8340
cagacgtcgg cagttattta aatccttaga aaatcaatca tacggcaacg aacgtttagg 8400
agaagaaact cttgctattg atattttcag gaacaaagag tgcttggaga gcgagatccc 8460
agagcagatg gaagctatcc ttgcaaattc ctcggccttg gtcttaggca tctcttcttt 8520
tgggatcaca ggaattcctg cgactttgca tagtttgctt cgacagaatc tatctttcca 8580
aaaacgctct atagcatcgg agagcttcct tttaaagatc gatagtgccc cctcagatgc 8640
ctctgttttt tataaaggcg tgcttttccg cggagagact gcgatcgtgg atgcgttaag 8700
ccaattattt gcccagctcg atctttctcc taaaaaaatt atctttctag gagaagaccc 8760
tgaggtcgtt caagctgttg ggtctgcttg tataggttgg ggcatgaact ttttaggcct 8820
ggtatactat cctgctcaag aaagcctttt ttcttatgtt catccttact ctacagcaac 8880
ggagctccaa gaagcacagg gtttacaagt aatttcagat gaagtcgcac agcttacttt 8940
aaacgctctt ccgaaaatga attaacatga tgttagaaca acaagcccta cgtattctct 9000
ttccccctga gggtgcttag ggcccttttt atctagcatg agcggagatt taggttttag 9060
tgagattttt ggtaaatatg aaaatatttt cttaaaaaga tcatgaaaca gtcatacttt 9120
tgcattgaag gatctttatg agacgttatc ttttcatggt tctagcttta tgcctatata 9180
gagcagcccc tctcgaagca gtggtcatta aaattactga tgcgcaggca gttcttaagt 9240
ttgctagaga gaaaacttta gtatgtttca atattgaaga tactgtggta tttcctaaac 9300
agatggtcgg ccagtctgca tggctctaca atagagagct cgatcttaag actacactct 9360
ccgaagaaca agccagagag caggcgtttc tggagtggat ggggatttct tttcttgtcg 9420
attatgaatt ggtgagcgcg aacttaagga atgtactcac aggcttatct ttgaaacgat 9480
cttgggtact cgggatttct caaagacctg tgcatttgat aaaaaatact cttcgcattc 9540
tgcgttcttt caacatagac ttcacctcgt gtccagctat ttgtgaagat ggttggctct 9600
ctcatcctac aaaggacaca acattcgatc aggccatggc tatagaaaaa aacattttgt 9660
ttgttggctc tctcaaaaac ggtcagccca tggacgcagc ccttgaagtt cttctctctg 9720
ggatctcttc tcctcctagc caaatcatct atgtggatca ggatgcagaa cgtttgcgtt 9780
ctatcggtgc tttttgtaaa aaagcaaaca tttattttat agggatgctc tacaccccag 9840
ccaagcagcg tgtagaaagt tacaatccta aacttactgc gatccaatgg tcgcagattc 9900
gtaagaatct ttccgatgaa tattatgaat ccctcttgag ctatgtaaag agcaaagggt 9960
agtgtcagca aatcgatgtg ggaatggtat cctgtggatg gcatagacac gcaaggcgtt 10020
ttttatgccg tttttagatc cttgtaggtg cagaaaaact aaaaagatcg gaagagaagg 10080
gattcgaacc cctggttcct gtaagagaac ttctgatttc gaatcagacg cattcgacca 10140
ctctgccact cttccgtaat gcttcaatag tttagagaat ctacactcag attgtctaca 10200
tgtattgcac aatgtttttc ataaatttat gaaatcataa tgaagattag agaagatggc 10260
cgagtagtga agacgaattc ttagagaaaa attagagtgt tttcttgcgt atctaggcag 10320
aaaactaccc tcaagaagag gcttgagggt gaaacatcta atttttgtca gaatgatgag 10380
agtggcagcc tcctgaagag cagctgtctc cgtgtgaatg acgggacttc ttgctgcaga 10440
aaggacaaaa gcttagcaag caaatgacac cagcaattcc aatgataata taggtaattt 10500
gggtggcagg agtagaaacg cctccacaca acttagcaat aatatttaat ttattgtgag 10560
tgattcctat aagtcccacg ttcaatgcac aaagaataac aataagagag gataatcctc 10620
gaataatttt gcctagcatc gcttttctcc taagtgatgt atacttaatt tcatctttta 10680
atctttttta taatttaata aacagttttt ttcaaaagac ttctttgcta aagtctctca 10740
gtctcgttga ctttttgaat acaaaggttt aagttgttca agaagttttt gaactgtaaa 10800

caacttccag tttttatgat tagagagaca gttcagaata aaaacttcaa ttttttaagt 10860
ttagataatc tttctttact tgcattgaga aaagagcata tttttataag ccgcaattta 10920
ggataacata tggagcaatt tcatttggat agggaagaaa ttttactttt ggcaaaggcc 10980
tcagcactac aactttctga agagcttatc caggagtatc aaacttcttt aagcgctgtg 11040
attacttcta tgaaagaagc tcttgccata gagattgatg atgcggattc atgcgaatct 11100
ctttttatgc atgtagtaaa tgttgaagat ttgagagaag attcggtgac ctctgatttc 11160
aatcgagagg aatttttgcg taatgttcca gagtctttag ggggattagt gaaagttcct 11220
gcggtaatca agtaagggag tctgaaaaat tatgtatcga tatagtgctt tagaattagc 11280
aaaagctgtg actttagggg aactgacagc cacaggggtg actcaacatt tttttcatag 11340
aatagaagaa gctgaggggc aggtaggtgc ctttatttcc ttgtgtaagg aacaagcttt 11400
agaacaggca gagctcatag ataaaaagcg ttcgcgtgga gaacctttag gaaaactcgc 11460
aggtgttcct gtaggaatta aagataatat tcacgttaca ggcctgaaga caacatgcgc 11520
ctctcgtgtg ctcgagaatt atcaaccacc gtttgatgct actgttgtag aaagaatcaa 11580
aaaagaagat gggattatct taggcaaact caatatggat gagtttgcta tgggatcaac 11640
aacgctatat tctgcttttc atcctaccca caacccctgg gatttatctc gtgttcctgg 11700
aggttcttca gggggatctg cggccgcagt ttctgctaga ttttgtcccg tagccctagg 11760
atcagatacc ggaggatcca tccgtcagcc cgcagcattt tgtggtgttg taggttttaa 11820
gccttcctac ggagccgttt cgcgttacgg gcttgtagcc tttgcctctt cgctagatca 11880
aatcggtcct ttagccaata ctgtagaaga cgtcgcccta atgatggatg tgttttctgg 11940
tagagatcct aaagatgcaa cctcaagaga gtttttccgt gattctttta tgagcaagtt 12000
gtctacggag gttcctaaag tgattggggt gcctagaaca tttttagagg gactccgtga 12060
tgatattagg gagaatttct tctcttcatt agccattttt gaaggagaag gaacccatct 12120
tgtggatgtg gagttggata ttctcagcca cgctgtatct atatattaca ttttagcatc 12180
tgctgaagct gccacgaatt tagcaaggtt cgatggggtg cgttatggat atcgttctcc 12240
tcaagcgcat accatcagcc aactctacga tctctcacgt ggagaaggat ttggcaaaga 12300
ggtcatgcgc agaatcctct tagggaacta tgtcttgtct gcggagagac agaatgttta 12360
ttataagaaa gctacggcag tgcgtgctaa gattgtaaaa gcatttagaa ctgcatttga 12420
aaagtgtgaa atcttagcca tgcccgtctg ttctagcccc gcgtttgaaa taggagaaat 12480
tctagatcct gtgactttat atctacagga tatctatact gtagctatga atttagcgta 12540
tcttcctgcc attgccgtac cctctggatt ttctaaggag ggcctgccct taggcctaca 12600
gattatcgga cagcaaggac aagaccaaca agtgtgccaa gtgggttaca gtttccaaga 12660
gcatgcgcaa attaagcaat tgttttctaa gagatatgcc aaaagtgttg ttctaggagg 12720
tcaatcatga gtgctgttta tgcagattgg gaatcagtca taggacttga agttcacgta 12780
gaattgaaca cagcatccaa gttatttagc tctgctttaa atcgctttgg agatgaacca 12840
aacactaata tttctacagt atgtacagga ttgccaggat cattgcctgt attgaatcag 12900
agtgccgtgg agaaagctgt gctttttggc tgcgctgtcg aaggtgaaat ctctctattg 12960
agtcgtttcg ataggaagtc ttacttctat cccgatagtc ccaggaattt tcaaattaca 13020
caattcgaac atcctattat ccgaggagga cgcattaagg cgattgtcca aggggaagag 13080
cgttattttg aattagccca aacccatata gaagatgatg ccggaatgct gaagcacttc 13140
ggagagtttg ccggtgtaga ctacaatcgt gccggagtcc ccctaatcga aattgtttca 13200
aaaccctgta tgttttgtcc tgaagatggc tgttgttacg caacttcttt ggtttccttg 13260
ttagactata ttggaatttc cgattgcaat atggaagaag gctccatccg ttttgatgtc 13320
aatgtctccg tacgccctaa gggatcccca gaacttcgca ataaggtaga aatcaagaat 13380
atgaactcct tcgcttttat ggcacaagct ttagaagctg aaaaacaacg tcagatcgat 13440
gagtatctta atcagccaaa taaagatccc aagctggtga ttccagccgc tacctaccgc 13500
tgggatcccg aaaagaaaaa aactgtgctg atgcgtctca aagagagtgc cgaagattat 13560
aaatattttc ccgagcctga tctgccgaca ctacaattga cagagtccta tatagaaagg 13620
attcgcaaga ccttgccaga acttccttat gacaagtacc atcgctatat tcaggagtac 13680
ggtctatccg aagatatcgc aagtattctg atcagcgata agaatatcgc aacgtttttt 13740
gaagtcgctt gtaaagattg taaaaacttt aggtctttat ctaactgggt aaccgttgaa 13800
tttggaggcc gctgcaaaac cctaggagtg aagttgccat cttcaggaat tttccccgag 13860
ggagtcgctc agctggtcaa cgcaatcgac caaggtgtga tcacagggaa aattgctaag 13920
gaaatcgcag atcttatgat ggaatcccca ggaaagaatc ctgaggagat tttaaaagag 13980
aagccagagc tgcttcccat gtcagatgaa ggggaattgc agaaaattat cgcagaggtg 14040
gttcttgcaa atcctgaatc tatcgtagac tataaaaatg gaaagactaa ggctctagga 14100
ttcctagtcg ggcagattat gaagcgtaca gcaggaaaag ctcctcccaa gcgagtgaac 14160
gaacttttac ttttagaatt agataagggc tagaattttc tgtcacagtc tgtcttaaaa 14220
aatcataaaa gcatgttaga gatctttcta acatgctttt tttatctaaa tacacatttc 14280
taagttgcaa aaacgaaagc agaaaatttc gatttgcctt tctacctaca ctttatggat 14340
ccatcagaat taaattttgc aaagccatgt ctacgcggga gataattctt tttaagtgac 14400
aagaaattct tgtgctcggc ttgctttctt attcttattg acgtattgct tgatcagata 14460
ttcattttga tttaggtact aaaatgcgat tttcgctctg cggatttcct ctagtttttt 14520
cttttacatt gctctcagtc ttcgacactt ctttgagtgc tactacgatt tctttaaccc 14580
cagaagatag ttttcatgga gatagtcaga atgcagaacg ttcttataat gttcaagctg 14640

gggatgtcta tagccttact ggtgatgtct caatatctaa cgtcgataac tctgcattaa 14700
ataaagcctg cttcnatgtg acctcaggaa gtgtgacgtt cgcaggaaat catcatgggt 14760
natattttaa taatatttcc tcaggaacta caaaggaagg ngctgtactt tgttgccaag 14820
atcctcaagc aacggcacgt ttttctgggt tctccacgct ctcttttaat cagagccccg 14880
gagatattaa agaacaggga tgtctctatt caaaaaatgc acttatgctc ttaaacaatt 14940
atgtagtgcg ttttgaacaa aaccaaagta agactaaagg cggagctatt agtggggcga 15000
atgttactat agtaggcaac tacgattccg tctctttcta tcagaatgca gccacttttg 15060
gaggtgctat ccattcttca ggtcccctac agattgcagt aaatcaggca gagataagat 15120
ttgcacaaaa tactgccaag aatggttctg gaggggcttt gtactccgat ggtgatattg 15180
atattgatca gaatgcttat gttctatttc gagaaaatga ggcattgact actgctatag 15240
gtaagggagg ggctgtctgt tgtcttccca cttcaggaag tagtactcca gttcctattg 15300
tgactttctc tgacaataaa cagttagtct ttgaaagaaa ccattccata atgggtggcg 15360
gagccattta tgctaggaaa cttagcatct cttcaggagg tcctactcta tttatcaata 15420
atatatcata tgcaaattcg caaaatttag gtggagctat tgccattgat actggagggg 15480
agatcagttt atcagcagag aaaggaacaa ttacattcca aggaaaccgg acgagcttac 15540
cgtttttgaa tggcatccat cttttacaaa atgctaaatt cctgaaatta caggcgagaa 15600
atggatactc tatagaattt tatgatccta ttacttctga agcagatggg tctacccaat 15660
tgaatatcaa cggagatcct aaaaataaag agtacacagg gaccatactc ttttctggag 15720
aaaagagtct agcaaacgat cctagggatt ttaaatctac aatccctcag aacgtcaacc 15780
tgtctgcagg atacttagtt attaaagagg gggccgaagt cacagtttca aaattcacgc 15840
agtctccagg atcgcattta gttttagatt taggaaccaa actgatagcc tctaaggaag 15900
acattgccat cacaggcctc gcgatagata tagatagctt aagctcatcc tcaacagcag 15960
ctgttattaa agcaaacacc gcaaataaac agatatccgt gacggactct atagaactta 16020
tctcgcctac tggcaatgcc tatgaagatc tcagaatgag aaattcacag acgttccctc 16080
tgctctcttt agagcctgga gccgggggta gtgtgactgt aactgctgga gatttcctac 16140
cggtaagtcc ccattatggt tttcaaggca attggaaatt agcttggaca ggaactggaa 16200
acaaagttgg agaattcttc tgggataaaa taaattataa gcctagacct gaaaaagaag 16260
gaaatttagt tcctaatatc ttgtggggga atgctgtaga tgtcagatcc ttaatgcagg 16320
ttcaagagac ccatgcatcg agcttacaga cagatcgagg gctgtggatc gatggaattg 16380
ggaatctctt ccatgtatct gcctccgaag acaatataag gtaccgtcat aacagcggtg 16440
gatatgttct atctgtaaat aatgagatca cacctaagca ctatacttcg atggcatttt 16500
cccaactctt tagtagagac aaggactatg cggtttccaa caacgaatac agaatgtatt 16560
taggatcgta tctctatcaa tatacaacct ccctagggaa tattttccgt tatgcttcgc 16620
gtaaccctaa tgtaaacgtc gggattctct caagaaggtt tcttcaaaat cctcttatga 16680
tttttcattt tttgtgtgct tatggtcatg ccaccaatga tatgaaaaca gactacgcaa 16740
atttccctat ggtgaaaaac agctggagaa acaattgttg ggctatagag tgcggaggga 16800
gcatgcctct attggtattt gagaacggaa gacttttcca aggtgccatc ccatttatga 16860
aactacaatt agtttatgct tatcagggag atttcaaaga gacgactgca gatggccgta 16920
gatttagtaa tgggagttta acatcgattt ctgtacctct aggcatacgc tttgagaagc 16980
tggcactttc tcaggatgta ctctatgact ttagtttctc ctatattcct gatattttcc 17040
gtaaggatcc ctcatgtgaa gctgctctgg tgattagcgg agactcttgg cttgttccgg 17100
cagcacacgt atcaagacat gcttttgtag ggagtggaac gggtcggtat cactttaacg 17160
actatactga gctcttatgt cgaggaagta tagaatgccg cccccatgct aggaattata 17220
atataaactg tggaagcaaa tttcgttttt agaaggtttc cattgcctgt gtggttccgg 17280
atcttaacta taaatcctgg actatggatc ataggcattg ggtctctcga acttgtgtgg 17340
agaataacga cattttatat gcataacgga atactcgtat cacctcagcc cctagagaca 17400
ttctttaggg gttctttatt tgtctaaact tcgtatttta tcgagaatcc tttacgttct 17460
tggtttgctt gtctccgagg agttctctaa cgaatcatag ggattccagg gttctgttcc 17520
ttgagtcctt tggcagctga tttgcgtctt tctcaggagt agagcgtgtg gtttcagggc 17580
ttgggactcc tagtaagacg agccattctg atagcctctc taggagagcg cttcgaagag 17640
gttcctgcaa ctgcttctaa gtaaaaattt aaagagaagt ctagtttttt cgatacattg 17700
acttcttttt tgaaaccaga cactcattgc tagccgaatt ttaagagtcc atgagcatga 17760
tttctgtaaa atcctcgcaa tgagcttttt gtcttctcac cttatgatgt gctttctata 17820
agtatttaaa tgaactttta ttaaaagagt tttcgctata acctttcgct cattttttcc 17880
tttcttgtcg tgataccgct aactgatagc actacaagct ctctttcaac gtctctctta 17940
gatgaaggaa acccacaatc catgaggaaa cttcgtattc ttgcgatcgt tctcatagct 18000
ttgagcatta ttttgattgc aggtggtgtg gtattgctta ctgtagcgat ccctggatta 18060
agttcagtca tttcttcccc ggcagggatg ggtgcctgtg ctttgggatg tgtgatgctt 18120
gctttaggga tcgatgttct tctgaagaaa cgagaagtcc ctatagttct cgcatctgta 18180
actacgacac caggaactgg cagccctaga agtggtattt ctatttcagg agctgatagc 18240
accatacgtt ctcttcctac gtatctcttg gacgagggac atccacaatc catgaggaaa 18300
cttcgtattc ttgcgatcgt tctcatagtt tttagcatta ttttgattgc aagtggtgtg 18360
gtattgctta ctgtagcgat ccctggatta agttcagtca tttcttcccc ggcagggatg 18420
ggtgcctgtg ctttgggatg tgtgatgctt gctttaggga tcgatgttct tctgaagaaa 18480

cgagaagtcc ctatagttct cgcatctgta actacgacac caggaactgg cagccctaga 18540
agtggtattt ctatttcagg agctgatagc accatacgtt ctcttcctac gtatcccttg 18600
gacgagggac atccacaatc catgaggaaa cttcgtattc ttgcgatcgt tctcatagtt 18660
tttagcatta ttttgattgc aagtggtgtg gtattgctta ctgtagcgat ccctggatta 18720
agctcgatca tttcttcccc agcggagatg ggtgcttgtg ctttgggatg tgtgatgctt 18780
gctttgggga tcgacgttct tctgaagaaa cgagaagtcc ctatagtagt tcccgcacct 18840
attcctgaag aagtcgtcat agatgatata gatgaagaga gtatacggct gcagcaggaa 18900
gctgaagccg ctttagcaag acttcctgag gagatgagtg catttgaagg ttacataaaa 18960
gttgtcgaga gtcatttgga gaacatgaaa agcctgcctt atgatggtca tgggctagaa 19020
gagaaaacga aacatcagat aagagtcgtc agatcttctt tgaaggctat ggttccagaa 19080
tttttagata tcagaagaat ttttgaagaa gaagagttct tttttctctc agctcgcaaa 19140
cgacttatag atttagctac tactttagta gagagaaaaa ttttaacaga gcaacttgag 19200
cgcaataatt taaggaaagc gttttcttat ttatatcagg actcaatttt taaaaaaatt 19260
attgataact tcgagaagtt agcatggaaa tttatgattt tgagtaaatc aatttgtcga 19320
tttacaatta tttttgaaaa tcatgaacat ggtgtagcaa agagcctgtt acacaagaat 19380
gcagtgttac tggagaaggt aatctatagg agtttgcaaa aaagctatag agatataggc 19440
atgtcatctg caaagatgaa aatcttgcac ggcaaccctt ttttctcttt ggaagataat 19500
aaaaagacga taatgaaaga acacgcagag atgcttgaaa gtctcagtag ctataggaag 19560
gtatttttag ctctatctga tgagaacgtt gtagatacac ctagcgatcc aaagaaatgg 19620
gatttgtcag gaatcccctg tagggacgcg ttgtctgaga tttctcgtga tgaacagtgg 19680
cagaagaaag cacatctaaa gcatcaagag tccctctata cgcaagctag ggatcgttta 19740
acagaccaga gctctaaaga aaatcagaaa gagttagaga aagctgaaca agagtacata 19800
tcttcttggg aacgggttaa aaaatttgag attgagagag tacaggagag gatacaggca 19860
attcaaaagc tttatcctaa tatcctcgag agagaagaag aaaccacagg tcaggagact 19920
gtgactccaa ctgttcaagg gacgacggct tcatccgatt taacagatat tttaggaaga 19980
atagaggtct ccagtaggga ggataatcag aatcaagagt cttgtgtaaa agtcttaaga 20040
agtcatgagg tagaaatgag ctgggaagtc aaacaagagt atggccctaa gaaaaaagaa 20100
tttcaggatc aaatgggttc tttagagagg ttttttacag agcatattga agagttagaa 20160
gtattacaga aggactactc taaacacttg tcttatttta aaaaagtaaa caataagaaa 20220
gaggttcaat atgcgaagtt taggttgaag gttttagagt cagatttaga agggattcta 20280
gctcagactg agagtgctga gagtctgtta actcaagaag aacttccgat tcttgcaact 20340
cggggagcct tagagaaagc tgttttcaaa gggagtctat gttgcgcgct agcaagcaaa 20400
gcaaaaccct attttgaaga ggatcccaga ttccaagatt ctgatacgca attgcgagct 20460
ctgactctaa ggttacagga ggctaaggca agcctggaag aagagataaa gagattttca 20520
aatcttgaga acgatattgc agaggaaaga cgccttctta aagagagcaa gcagacgttc 20580
gaaagagcag gtttaggggt tctccgagaa attgcagtcg agtctactta tgatttgcgt 20640
tccttaacaa atacatggga agggacccca gagagtgaga aggtctattt tagcatgtat 20700
cttaattatt acaacgaaga gaaacgtagg gnnaaaacaa gattggttga aatgacacag 20760
aggtatagag attttaaaat ggccttggaa gctatgcagt ttaatgaaga agcccttttg 20820
caagaggaac tctctattca agctcccagt gaataacaag aatccgaggc aagagctcgg 20880
catgtcgctc gctataggct acgagttgcc caatctcgtt tggctgattt agaagctgga 20940
gtcatccaga aacgtaaaac aatcaaagat ttgttcaaga aacgacagtc tcaaccttct 21000
tctaagaaat agaaaatttc gtccatgctt ccagggtttt gaaatgagaa aaagaccttg 21060
gaataaaata attgatatga gtagcttagt tgctttaact tatgtttttg agcatgatga 21120
catcacagat tcataatgca agtattctta tctcctcaac tacccccccc ccccccgacg 21180
ctctctaggg gtttcttgtt ctaaacttcg tagtttatcg atcactcttt tagttcttgg 21240
tgtgcttttg ttgaccttgg ggattccggg actcactgca gggatctctt ttggagccgg 21300
tttgggattt tctgctttag gaggagtgct cgtgatttcg ggacttctat tccttctagt 21360
aagacgagag gttccgacag tacgttcaga ggaaattccc agaggggttt ctgtgacccc 21420
ttctgaagag cctgctctag agaaggctca aaaagaaccg gagacaaaga aaattttaga 21480
tcggttgccg aaggaattgg atcagttaga tacgtatatt caggaagtgt ttgcatgttt 21540
agagaggctg aaggatccta agtacgaaga tcgaggtctt ttaacagagg cgaaggagaa 21600
acttcgagtt tttgacgttg ttgagaaaga tatgatgtca gagtttttag acatacaacg 21660
agtgttgaat gaggaagcat attatgtaga acattgtcaa gatcccctag agaatatagc 21720
ctacgagatt ttctcttccc aagagcttcg tgactactac tgtgcagggg tgtgtgggta 21780
tttgccttct ggggatgctc gagcggatcg attaaagaga tcagttaagg aggtaatgga 21840
tcgctttatg agggtgacct ggaaatcttg ggaggcatca gtcatgttgg atcatagcta 21900
tggggtagcg cgagagttat tcaagaaggc agtaggagta ctagaggaga gtgtctataa 21960
aattctgttt aagagctata gagatgcgtt ttatgaatgt gagaaggcaa agatccagag 22020
ggatgggcgt ttcaaatggt tataggatac gagtgctcat gcggaacaaa ggtttaggga 22080
tatcaatggt tgttgggagg acttaaagca aacgatcttt tgggtaggag aacatgattg 22140
tacggacata gagaccgtac gtaaaagctg tatgtggctg gatcgttatg cagataaatt 22200
tattttgagg gaaaaagagg aaaagatgga gcgtcatgag ctctttcatg cgactatggt 22260
ccgaaaagca tctgggcacg cgtatgctaa agctaaagca gcctttgaaa aggagagatc 22320

taatgagaat cagaggaaag tcaaggatgt tgaaaaatgg ttatctaaag gtttagcgga 22380
gtttcgtaat caagagtctc gcagagctcg ggagaggctg agagagctgc aaactttgta 22440
tcctgaggtt tctgtagaag agagagtgtt agagagacaa aggactaaaa aagttaatct 22500
ggagaacttg tatgcagata tagaaaagaa gtatcaccac tgtgttcgag agcaagagca 22560
ttactggaaa gaggtagaga acaaggaagc agagtatagg gagaacggag aaaaggttct 22620
ctctgccgag gaggtgtcag agtgtcttca gaggttggaa gattgtttag agacgtggtc 22680
taagaaatta acaaaagcgg aagagagtgt ctttgagatg aagtttgatg cgacagaaaa 22740
actagggaat aaagtacttt ctgatgtaac gaaccgtctt gagattttat gtgaagatgc 22800
tgaggagatg atttttcgaa tcgaagagat agagatgact ctgcgtatgg tagagcttcc 22860
actacttttt atgaaaaata cttttgagaa agcctctcta caatacaaca gctgcaaaga 22920
gatgttagcc aaagtagagc cccaatgtaa ggaaagccca acctatagaa gtagccaaga 22980
gcgcttagaa aggttgaatc aggatttaca aacagcatat acaaattgcc aggagagact 23040
ccagggtttt tcagatttgg aatcaaaagt acgtacatgt agagatcatc ttagagagca 23100
gatgaaacat ttcgaagttc aaggactgaa ttttataaac gaagagcttt tatgggtcgg 23160
ggcagagctc tttacacaag ccagattgga tctagtagca acagttccgt atatggagtt 23220
ctatttgcag taccataata ttaaaagaga aaaagttcga tcccaatgga tggcgaagac 23280
cgagaggtat agagagattc ggcaggcatt tcaaggggtg atgaaggaag atttgttagc 23340
agaagatacg atcttgaaag aagaagatta ttggctgctt cgcgatgatt ggttgctgcg 23400
tgatgagagg aagaatagac aaagacgttt aatctgtaat aagatagcag cagcgcaaca 23460
gcgagtcaaa ggcttctaac ctgaagagat ttaaaagatc ctgaaagaga aaacacctct 23520
tgtggctaga gtgcgttctt tatttactcg cgaggaccat acctagcata aaactccagg 23580
agctgtgttt ctttaaaatt ctttgaataa aatactatat attagtagct tagtgggttt 23640
aatttatgtg tttgatcgcg atggcaccac agattcataa tgcaagtact tctatctccg 23700
cagctacccc cccccccaac actctgtagg gtcgatttct tctccatcta aacttcgcgt 23760
tttagcgatt acttttttag tttttggtat gctcttactg atttcaggag ctctctttct 23820
gacgttaggg attccaggat tgagtgcagc aatttctttt ggattaggca tcggtctctc 23880
cgcattagga ggagtgctga tgatttcggg actactatgt cttttagtaa aacgagagat 23940
tccgacagta cgaccagaag aaattcctga aggggtttcg ctggctcctt ctgaggagcc 24000
agctctacag gcagctcaga agactttagc tcagctgcct aaggaattgg atcagttaga 24060
tacagatatt caggaagtgt tcgcatgttt aagaaagctg aaagattcta agtatgaaag 24120
tcgaagtttt ttaaacgatg ctaagaagga gcttcgagtt tttgactttg tggttgagga 24180
taccctctcg gagattttcg agttgcggca gattgtggct caagagggat gggatttaaa 24240
ctttttgatc aatgggggac gaagcctcat gatgactgca gaatctgaat cgcttgattt 24300
gtttcatgta tcgaagcggc tagggtattt accttctggg gatgttcgag gggaggggtt 24360
aaagaaatct gcgaaggaga tagtcgctcg tttgatgagc ttgcattgcg agattcacaa 24420
ggtggcggta gcgtttgata ggaattccta tgcgatggca gaaaaggcgt ttgcgaaagc 24480
gttgggagct ttagaagaga gtgtgtatcg gagtctgacg cagagttata gagataaatt 24540
tttggagagt gagagggcga agatcccatg gaatgggcat ataacctggt taagagatga 24600
tgcgaagagt gggtgtgctg aaaagaagct tcgggatgcc gaggaacgtt ggaagaaatt 24660
taggaaagca gtcttttggg tagaagaaga cgggggcttt gacatcaata atctccttgg 24720
agactggggg acagtgcttg atccttatag acaagagaga atggacgaga taacgttcca 24780
tgagttgtat gaaaaaacta cgtttttgaa aagactgcac agaaagtgtg cgttagcgaa 24840
aacaaccttt gaaaagaaga gatctaaaaa gaatttgcag gcagtcgagg aggcgaatgc 24900
acgtaggttg aaatatgtaa gggattggta tgatcaggag tttcagaaag caggggagag 24960
attagagaaa ctgcatgctt tgtatcctga ggtttcagtc tctataagag agaacaaaat 25020
acaagagacg cgctctaatt tagagaaagc ctatgaggct atcgaagaga actatcgttg 25080
ctgtgtccga gagcaagagg actactggaa agaagaagag aaaagggaag cggagtttag 25140
ggagagggga aacaagattc tttctcctga ggagctggaa agttctttgg agcaattcga 25200
ccatggtttg aaaaattttt ctgagaaatt aatggaattg gaagggcata tcttaaaact 25260
tcagaaagaa gccacagcag aggtggagaa taaaatactt tcagatgcag agagccgcct 25320
tgagattgta tttgaagatg tcaaggagat gccctgtcga attgaggaga tagagaagac 25380
gctgcgtatg gcggagctgc ccctacttcc tacgaagaag gcgtttgaga aggcctgctc 25440
acaatataat agctgcgcag agatgttgga gaaggtgaag ccttactgca aggagagcct 25500
cgcctatgtg actagcaaag agcgtttagt gagcttggat gaagatttac gacgagccta 25560
cacagagtgt cagaagagat tccaggggga ttcgggtttg gagtcggaag taagagcctg 25620
tcgagagcaa ctgcgagagc ggatccaaga gtttgaaact caagggctgg acttggtgga 25680
aaaagagttg ctttgtgtga gtagtagatt aagaaataca gagtgcgatt gtgtatctgg 25740
tgttaagaaa gaagcacctc ctggtaagaa gttttatgcc cagtattatg atgagattta 25800
tcgagttaga gttcaatccc gatggatgac gatgtctgag agattgagag agggagttca 25860
agcatgcaac aagatgttga aggcaggcct aagcgaagaa gataaggttc ttaaagaaga 25920
agagtattgg ttgtatcgag aggagagaaa gaataaagag aaacgtttgg ttggtactaa 25980
gatagtagca acgcagcagc gagttgcagc atttgaatcc atagaagttc ctgagattcc 26040
tgaggcccca gaggagaaac cgagtttgct ggataaagcg cgttctttat ttactcgcga 26100
ggaccattcc tagaactatt ctaggagtct atgtgcacta ttttttaaga ggtttattat 26160

tccgtgggta tgtctgagag actttgatta aatgggatat tattatatcc tagttgctct 26220
aatcaatatt cctcttatga ttttggtatt cgatcatgac tgcagcacca gctattctac 26280
acgtatcccc gacgccccct gaagaaacaa aattcgttat tcctaaagat agtaaatctc 26340
gcgctcttgg gattacttta ttagtcgtag gcatccttct ggtagtttgt ggtgcgattg 26400
tactcagtgg agtgatttct ggattgagtg cactcattgt ttgtggattg ggtattagta 26460
cgatttctct aggagttgtc ctatttgttt taggattgat attattactt agaaagcggg 26520
aacttacctt agaacagatc gaggctaagc aaattgcgga gacctttgct gatgaactga 26580
aagaactaga aatgtacatt cagtcgacag agaaaagctt agagaagata gaagggtccc 26640
gttatagtga ccaaggtttt ctgaatcgtg ccacccaaaa aatcttagat ttagaatctt 26700
cattgagctc tattacttct gagtttcgtg atcttaggca actctttgat gaagaaaaaa 26760
tagagttact ttctggagaa aggcttttag aatttattgc agcgaattta tttaaacaag 26820
gaagagatgt ctatttaaat ttagggaatt tagcagacat tcgtgcgtac atggggccca 26880
acaattataa agttgcgatg gtcatagaaa aagctaaagc agttgtgcat gagtttatag 26940
ttctgactac aatggctagg gaattagagt tttttttcta aaaaataaat atggtttatt 27000
aaaggggatg taccaggaga atctaagatt gttggaaagg cttctttata atagtgttca 27060
aaagagctat gcggatcggc tgttttccta tgaaaagaca aagatggtgc acgatactcc 27120
gctgattcct tgggaagagg ataaggaaaa atgtgctgaa gctgagaaag ctttcttaga 27180
gcaacagaag attctcctag attatggaaa atctatcttt tggctgaatg agaacgatga 27240
gatcaattta aacgatcctt ggagttgggg tcttaatacg gtgaggacta ggaaagtatt 27300
ccaagaggtt gacgacagtg aacgttggaa tcataaggta ctcattcaaa aactcgagga 27360
cgattatgag aaacttctag aggaaagttc aaaagagtct actgaagcaa ataagaagct 27420
tttatctgac ttagtagatc gtcttgaaga tgctaagaca aaatttttcc tgaagaaaca 27480
ggaggaggtg gagactcgcg ttaaggatct tagagctcga tatggaggca cagtagatcc 27540
taagcaggat acggaagcta agaagaaagt cgaattggag gctagcttag aaaccttttt 27600
agattccatc gaatcagagc tagtacagtg tttagaagat caagatatat attggaaaga 27660
acaggatgtc aaagatctag cacgtacgca agagctcgag gaacaagata ttgaagcgaa 27720
gagggaagaa gctgccgaag acctaagaaa gtcttaatga gcgtttaaag aagtcaaaaa 27780
ctatgttaga tagggctaaa tggcatattg aaaatgctga ggacagtatt acctggtgga 27840
ctagtcagat agaaatgaag gatatgaaag caagactgaa gatcttaaaa gaagatataa 27900
caagtgttct acctgaaata gatgagattg aaacgtgttt aagcttagag gagcttcctt 27960
tgcttacgac cagggaactc ttaactaagt cctacctaaa gtttaagatt tgttcggaaa 28020
cactattaaa aatgacttct gtgtttgaga acaatatcta tgttcaggag tacgaggttc 28080
agctgcaaaa tctagggttt aagttacaag gtatatctca gagattcgga aagaaacaag 28140
acgattttgc gaatctagag gaacaggttg ctttgcaaaa gaaacgactc agagagctca 28200
ctcagaattt tgaaatacaa ggattcaatt tcatgaaaga agattttaag gcagccgcta 28260
aagatcttta tataagaagt acagctgaac aaaagatgaa ctttgatgtg ccttgcatgg 28320
agctcttccg taggtatcat gaggaggtca acaagccgct tcttgagttg atgtacaatt 28380
gtgcagacag ttatagagat gctaagaaaa agctttgctc tctacgtctt gatgaaaaag 28440
agttattaca aaaagaaatc aagaaagagg aattttatca aaagaaacaa caaaggcatg 28500
cagatagatc acgtcatact aggtatcaaa agctacgaat tgctgaagag cttgctcttg 28560
agctgaagaa gaaaatctaa tcactaaagc ctagttaagg gttcttttga attgggccct 28620
gtgtttccat ttctctaggg atcctcttag ctgaggatct tctttgtacc tcttttgatt 28680
cgaaaagagg tgctattgtg agttgccaat agcaacgatc caccttcgtt tttgaaaaga 28740
tacatcagtt gctaagggaa cttttcagaa aatatcataa aaaactccca agattttggc 28800
ttggaaagcg agcctttctt gctacttttg ttacaacaaa agtgttctat tttaacgtgc 28860
gtatcatttg tgactaagag atagacttgc tttctttatc taatcttctg tattggaaag 28920
aaagcccctt gagggaaaaa aaggttgtta tgaagattcc actccgcttt ttattgatat 28980
cattagtacc tacgctttct atgtcgaatt tattaggagc tgctactacc gaagagttat 29040
cggctagcaa tagcttcgat ggaactacat caacaacaag cttttctagt aaaacatcat 29100
cggctacaga tggcaccaat tatgttttta aagattctgt agttatagaa aatgtaccca 29160
aaacagggga aactcagtct actagttgtt ttaaaaatga cgctgcagct ggagatctaa 29220
atttcttagg agggggattt tctttcacat ttagcaatat cgatgcaacc acggcttctg 29280
gagctgctat tggaagtgaa gcagctaata agacagtcac gttatcagga ttttcggcac 29340
tttcttttct taaatcccca gcaagtacag tgactaatgg attgggagct atcaatgtta 29400
aagggaattt aagcctattg gataatgata aggtattgat tcaggacaat ttctcaacag 29460
gagatggcgg acaattaatt gtgcaggctc cttgaagatc gcaaacaata agtccctttc 29520
ttttattgga aatagttctt caacacgtgg cggagcgatt cataccaaaa acctcacact 29580
atcttctggt ggggaaactc tatttcaggg gaatacagcg cctacggctg ctggtaaagg 29640
aggtgctatc gcgattgcag actctggcac cctatccatt tctggagaca gtggcgacat 29700
tatctttgaa ggcaatacga taggagctac aggaaccgtc tctcatagtg ctattgattt 29760
aggaactagc gctaagataa ctgcgttacg tgctgcgcaa ggacatacga tatactttta 29820
tgatccgatt actgtaacag gatcgacatc tgttgctgat gctctcaata ttaatagccc 29880
tgatactgga gataacaaag agtatacggg aaccatagtc ttttctggag agaagctcac 29940
ggaggcagaa gctaaagatg agaagaaccg cacttctaaa ttacttcaaa atgttgcttt 30000

ctactttgta gctaagaatc aaaggtacag tctacggagg aactctctat taccagcaca 33900
acgaaaccta tatctctctt ccttgcaaac tacggccttg ttcgttgtct tatgttccta 33960
cagagattcc tgttctcttt tcaggaaacc ttagctacac ccatacggat aacgatctga 34020
aaaccaagta tacaacatat cctactgtta aaggaagctg ggggaatgat agtttcgctt 34080
tagaattcgg tggaagagct ccgatttgct tagatgaaag tgctctattt gagcagtaca 34140
tgcccttcat gaaattgcag tttgtctatg cacatcagga aggttttaaa gaacagggaa 34200
cagaagctcg tgaatttgga agtagccgtc ttgtgaatct tgccttacct atcgggatcc 34260
gatttgataa ggaatcagac tgccaagatg caacgtacaa tctaactctt ggttatactg 34320
tggatcttgt tcgtagtaac cccgactgta cgacaacact gcgaattagc ggtgattctt 34380
ggaaaacttt cggtacgaat ttggcaagac aagctttagt ccttcgtgca gggaaccatt 34440
tttgctttaa ctcaaatttt gaagccttta gccaattttc ttttgaattg cgtgggtcat 34500
ctcgcaatta caatgtagac ttaggagcaa aataccaatt ctaatgcgtt agctttggta 34560
aagagctcca tacatcgaag ggaaaagagc ttttaagatt tcttgaaggc tcttttcgat 34620
ttcgatttcc attttagtgt tttgctaaaa cactttctaa tttttttctt ttgttttcta 34680
cattgaaaaa aagagagtta cggcagctgt aaagttttta atattgctcc ctttgttcca 34740
tttatgtagc gttcagactt tgcactaaaa cgagggtgtc atatgagatc gtctttttcc 34800
ttgttattaa tatcttcatc tctagccttt cctctcttaa tgagtgtttc tgcagatgct 34860
gccgatctca cattagggag tcgtgacagt tataatggtg atacaagcac cacagaattt 34920
actcctaaag cggcaacttc tgatgctagt ggcacgacct atattctcga tggggatgtc 34980
tcgataagcc aagcagggaa acaaacgagc ttaaccacaa gttgtttttc taacaccgca 35040
ggaaatctta ccttcttagg gaacggattt tctcttcatt ttgacaatat tatttcgtct 35100
actgttgcag gtgttgttgt tagcaataca gcagcttctg ggattacgaa attctcagga 35160
ttttcaactc ttcggatgct tgcagctcct aggaccacag gtaaaggagc cattaaaatt 35220
accgatggtc tggtgtttga gagtataggg aatcttgatc ttaatgaaaa tgcctctagt 35280
gaaaatgggg gagccatcaa tacgaagact ttgtctttga ctgggagtac gcggtttgta 35340
gcgttccttg gcaatagctc gtcgcaacaa gggggagcga tctatgcttc tggtgactct 35400
gtgatttctg agaatgcagg aatcttgagc ttcggaaaca acagtgcgac aacatcagga 35460
ggcgcgatct ctgctgaagg gaaccttgtg atctccaata accaaaatat ctttttcgat 35520
ggctgcaaag caactacaaa tggcggagct attgattgta acaaagcagg ggcgaaccca 35580
gaccctatct tgactctttc aggaaatgag agcctgcatt ttctgaataa cacagcagga 35640
aatagtggag gtgcgattta taccaaaaaa ttggtgttat cctcaggacg aggaggagtg 35700
ttattttcta acaacaaagc tgcgaatgct actcctaaag gaggggcaat tgcgattcta 35760
gattctggag agattagcat ttctgcagat ctcggcaata tcattttcga gggcaatact 35820
acgagcacta caggaagtcc tgcgagtgtg accagaaatg ctatagatct tgcatcgaat 35880
gcaaaatttt taaatctccg agcgactcgg ggaaataaag ttattttcta tgatcctatc 35940
acgagctcag gagctactga taagctctct ttgaataaag ctgacgcagg atctggaaat 36000
acctatgaag gctacatcgt tttctctgga gagaaactct cagaagtaag aaacctgaca 36060
atctgaagtc tacatttaca caggctgtag agcttgctgc aggtgcctta gtattgaaag 36120
atggagtgac tgtagttgca aatactataa cgcaggtcga gggatcgaaa gtcgttatgg 36180
atggagggac tacttttgag gcaagcgctg agggggtcac tctcaatggc ctagccatta 36240
atatagattc cttagatggg acaaataaag ctatcattaa ggcgacggca gcaagtaagg 36300
atgttgcctt atcagggcct atcatgcttg tagatgctca ggggaactat tatgagcatc 36360
ataatctcag tcaacagcag gtctttgcct taatagagct ttctgcacaa ggaacgatga 36420
ctactacaga tatccccgat accccaattc taaatactac gaatcactat ggtatcaagg 36480
gaactggaat aattgtttgg gtcgacgatg caactgcaaa aacaaaaaat gctaccttaa 36540
cttggactaa aacaggatac aagccgaatc cagaacgtca gggacctttg gttcctaata 36600
gcctgtgggg ttcttttgtc gatgtccgct ccattcagag cctcatggac cggagcacaa 36660
gttcgttatc ttcgtcaaca aatttgtggg tatcaggaat cgcggacttt ttgcatgaag 36720
atcagaaagg aaaccaacgt agttatcgtc attctagcgc gggttatgca ttaggaggag 36780
gattcttcac ggcttctgaa aatttcttta attttgcttt ttgtcagctt tttggctacg 36840
acaaggacca tcttgtggct aagaaccata cccatgtata tgcaggggca atgagttacc 36900
gacacctcgg agagtctaag accctcgcta agattttgtc aggaaattct gactccctac 36960
cttttgtctt caatgctcgg tttgcttatg gccataccga caataacatg accacaaagt 37020
acactggcta ttctcctgtt aagggaagct ggggaaatga tgccttcggt atagaatgtg 37080
gaggagctat cccggtagtt gcttcaggac gtcggtcttg ggtggatacc cacacgccat 37140
ttctaaacct agagatgatc tatgcacatc agaatgactt taaggaaaac ggcacagaag 37200
gccgttcttt ccaaagtgaa gacctcttca atctagcggt tcctgtaggg ataaaatttg 37260
agaaattctc cgataagtct acgtatgatc tctccatagc ttacgttccc gatgtgattc 37320
gtaatgatcc aggctgcacg acaactctta tggtttctgg ggattcttgg tcgacatgtg 37380
gtacaagctt gtctagacaa gctcttcttg tacgtgctgg aaatcatcat gcctttgctt 37440
caaactttga agttttcagt cagtttgaag tcgagttgcg aggttcttct cgtagctatg 37500
ctatcgatct tggaggaaga ttcggatttt aatcctaagt tttccaacga gatagcatca 37560
gggtaagcca gggctctatg taagagattt catagagccc tctctttgtc ttgctttttg 37620
tattttattt ttatatttcg tgaatccgtt gttccaatgt tcgaaaggta tcctagatga 37680

gattgctgtt gtcttgtcct atgcttttta tagctgcatg tgcctcattt tttggctttc 37740
aggaagaaat gcaaggcaga aatatacaat ctcttgatgc aaatgcttct agtctagggg 37800
aacttttttc tatttctacg aagggagtgt cttgtctaga actccatagg gagatcgcac 37860
gctaaatatg agaatataat atgaagactt cagtttctat gttgttggcc ctgctttgct 37920
cgggggctag ctctattgta ctccatgccg caaccactcc actaaatcct gaagatgggt 37980
ttattgggga gggcaataca aatacttttt ctccgaaatc tacaacggat gctgcaggaa 38040
ctacctactc tctcacagga gaggttctgt atatagatcc ggggaaaggt ggttcaatta 38100
caggaacttg ctttgtagaa actgctggcg atcttacatt tttaggtaat ggaaataccc 38160
taaagttcct gtcggtagat gcaggtgcta atatcgcggt tgctcatgta caaggaagta 38220
agaatttaag cttcacagat ttcctttctc tggtgatcac agaatctcca aaatccgctg 38280
ttactacagg aaaaggtagc ctagtcagtt taggtgcagt ccaactgcaa gatataaaca 38340
ctctagttct tacaagcaat gcctctgtcg aagatggtgg cgtgattaaa ggaaactcct 38400
gcttgattca gggaatcaaa aatagtgcga tttttggaca aaatacatct tcgaaaaaag 38460
gaggggcgat ctccacgact caaggactta ccatagagaa taacttaggg acgctaaagt 38520
tcaatgaaaa caaagcagtg acctcaggag gcgccttaga tttaggagcc gcgtctacat 38580
tcactgcgaa ccatgagttg atattttcac aaaataagac ttctgggaat gctgcaaatg 38640
gcggagccat aaattgctca ggggacctta catttactga taacacttct ttgttacttc 38700
aagaaaatag cacaatgcag gatggtggag ctttgtgtag cacaggaacc ataagcatta 38760
ccggtagtga ttctatcaat gtgataggaa atacttcagg acaaaaagga ggagcgattt 38820
ctgcagcttc tctcaagatt ttgggagggc agggaggcgc tctcttttct aataacgtag 38880
tgactcatgc cacccctcta ggaggtgcca tttttatcaa cacaggagga tccttgcagc 38940
tcttcactca aggaggggat atcgtattcg aggggaatca ggtcactaca acagctccaa 39000
atgctaccac taagagaaat gtaattcacc tcgagagcac cgcgaagtgg acgggacttg 39060
ctgcaagtca aggtaacgct atctatttct atgatcccat taccaccaac gatacgggag 39120
caagcgataa cttacgtatc aatgaggtca gtgcaaatca aaagctctcg ggatctatag 39180
tattttctgg agagagattg tcgacagcag aagctatagc tgaaaatctt acttcgagga 39240
tcaaccagcc tgtcacttta gtagagggga gcttagtact taaacaggga gtgaccttga 39300
tcacacaggg attctcgcag gagccagaat ccacgcttct tttggatctg gggacctcat 39360
tataagcttc tacagaagat attgtcatca caaatttatc tataaatgcc gataccattt 39420
acggaaagaa tcctatcaat attgtagctt cagcagcgaa taagaacatt accctaacag 39480
gaaccttagc acttgtaaat gcagatggag ctttctatga gaaccatacc ttgcaagact 39540
ctcaagacta tagctttgta aagttatctc caggagcggg agggactata attactcaag 39600
atgcttctca gaagcctctt gaagtagctc cttctagacc acattatggc tatcaaggac 39660
attggaatgt gcaagtcatc ccaggaacgg gaactcaacc gagccaggca aatttagaat 39720
gggtgcggac aggatacctt ccgaatcccg aacggcaagg atctttagtt cccaatagcc 39780
tgtggggttc ttttgttgat cagcgtgcta tccaagaaat catggtaaat agtagccaaa 39840
tcttatgtca ggaacgggga gtctggggag ctggaattgc taatttccta catagagata 39900
aaattaatga gcaccgctat cgccatagcg gtgtcggtta tcttgtggga gttggcactc 39960
atgctttttc tgatgctacg ataaatgcgg ctttttgcca gctcttcagt agagataaag 40020
actacgtagt atccaaaaat catggaacta gctactcagg ggtcgtattt cttgaggata 40080
ccctagagtt tagaagtcca cagggattct atactgatag ctcctcagaa gcttgctgta 40140
accaagtcgt cactatagat atgcagttgt cttacagcca tagaaataat gatatgaaaa 40200
ccaaatacac gacatatcca gaagctcagg gatcttgggc aaatgatgtt tttggtcttg 40260
agtttggagc gactacatac tactacccta acagtacttt tttatttgat tactactctc 40320
cgtttctcag gctgcagtgc acctatgctc accaggaaga cttcaaagag acaggaggtg 40380
aggttcgtca ctttactagc ggagatcttt tcaatttagc agttcctatt ggcgtgaagt 40440
ttgagagatt ttcagactgt aaaaggggat cttatgaact tacctttgct tatgttcctg 40500
atgtgattcg caaagatccc aagagcacgg caacattggc tagtggagct acgtggagca 40560
cccacggaaa caatctctcc agacaaggat tacaactgcg tttagggaac cactgtctca 40620
taaatcctgg aattgaggtg ttcagtcacg gagctattga attgcgggga tcctctcgta 40680
attataacat caatctcggg ggtaaatacc gattttaata gggaactgag agttccttat 40740
tgtagagagt atttacaagg attttgagac gaaagagatt tcttgttgta ggtatctctc 40800
cacagggtct agttcaattt ctatagttca tggggtttct ttttttataa agaagaaacc 40860
ccatgtctga cgagacctca cctggatcta ttgcattctc tatggtaagg gatccatgac 40920
ttgacacagg aggttcattc tcatgtctaa gtcagaggtt agaagggagg ttttttaagg 40980
tttgtcgagc tttaagaaga agaaaaatcg actatctgct tctcggcgtt catacacccc 41040
atagagctgc caatgttcga agatcttcgt ccctagaatc atctggtatt ctaggtagtt 41100
cggagtgtcc tgcgatgcca gccatagcgt aaggataagc ggtaattcca acagggatga 41160
ggtcgtacaa ataatttccc taaaatgaga ttcctatgat cagagagagg ggagtctaaa 41220
agctggtcaa tgggacggct gacatctaaa atgaagttct ccctgtcaca cttaatcagg 41280
ctgtatttgc ttctatgcag ggattctaga gtcatagcca cattgtcatt tccgatccac 41340
tcccaacgta tgttcatgtg atcccaacag tgctttttcc aaatccattc agcatctaag 41400
gagactgtat ttttctttcc aaaaggtaga gatagctcgc atgcagtttt gggaaacgtg 41460
ggtttgcttt ctgtattgct caagatgtgg gtagtccaca gcttcgcatg gattctcggg 41520

aatcgagggt tagtcttact cagtaccgag gtatctatac ccgctttcag aaggtttaag 41580
gagtgaaagg catcttgaat ggaaaagata taatgatctt cattcttagc tagaggacga 41640
gtctctgtaa tgaaggtaac gaacggctct ataatatggc gtctttgaat gtaggactta 41700
tgtaatagaa agcgataatc aagttgtagc ttcgcggaaa gctgactatg gcgcgaggag 41760
atctcaggaa catcgctata gtaaatcaga gaactcccta gggtggagga gagcgttcct 41820
ataggtagag gcacagtttt atggagctta gggcgcgcag caagacgtag tgaagagaaa 41880
ttctcgccaa cgatatgatc gctaaaagca aagtttaaat acccacattc tacgatgttt 41940
tcaaggtaca ctcccgtatt ataaatagaa atcgggtact gccttaatgt taaataaggg 42000
agctcttggt tggcattttg gaaagagttt accttaacag aagaggtgag atacccttca 42060
aaatagttgt cattccaagt gcaatcgaca cgtgtggggc ctgtattttt caacatgaag 42120
ttgttgggga aaatgtcagc aacagtttcc caactatcgc tgagatggta ttctccagaa 42180
aaatttacat gcttatgcgt gaagcagaaa tctccgtgta ggcgatagcg atcatgagct 42240
tctgccatat cgatagcaag gcggtgggca taatagcttt tcatattgaa gacattctca 42300
ggaacctgct tctgagaaca atggaggttg aatcccatgc cgacgccatg cttgaaaaag 42360
ctatccaaga aaaatgtcga ggagaaatgc ttcctagaaa tcggcgagta gctcatcccc 42420
aaataggatc ccagaaatcc tcctgttcct cctcgaaagt ttatcggagg cttagggatc 42480
tccataggca tgatagaaaa tggaggtaag aaaagtatcg gaatgcgaca cacccttaat 42540
gttgtcttcc ctatagaaag aagactatct gaagaatatt ccaggtaatc tccggagagg 42600
cacaggtctt ttttgggacc ctcggaggta gagatatatc ccttccgaat gactatggtt 42660
tctggggtta gagtgatcat agacccccct agaaaccaag gatacatcgc gaatcttcca 42720
ttagtaagaa gacaagagtc tgtatcttcg taatactcta ggtaatcaca aactagggtt 42780
tttgccctat agttcaccat aacattgcca tgtgcgacaa gcttcaggct ttgacccaca 42840
gtattttcta catacacttt attggcttgt atccgcaggt tgttatggat attcaatacc 42900
ccatcttcga tggtcacaat cccagaaaca ctcttaaagt gactaagata ggagtttttc 42960
tttttcacag cctcttgatg agtcaaagca tcagctgagg aacccataag aacaaaggaa 43020
gctagaaata agaagcaacg tttcataaga cgtaggttgg ggttactcca caatttttat 43080
gagtaagcct gcaaggactg ggaaattttt tgcatctcct tccgtcatca gttgtatcaa 43140
taaacggata tcttcggaag acttcgaggt ggctagtgtc tctagaatat ccaacatgag 43200
cttcgtacgg ctttctgggg tgacctgata acgtagatag ggcatgctgg gatggggtct 43260
ttggttttcc gtgtccacaa ataacaaggt ttcctgaatt agcttttttg cataatcatg 43320
gagagaacgt tttttttcag gatctttggt gagattataa atagcaagat ctgcataggc 43380
gcggatgata ggttctccag gaagcttcgc agcttggaaa agtagatcta aggcttcctg 43440
atgtgaggtg tgacttaaaa aagaaatcgc agtagtggca agctgagttt tctgactcgc 43500
caaaagctta taaatacagg ggaggtaagc ttctttaggt aggcggaaga gaaacgtaag 43560
gatctgctct tcaagacctc gggttgtgga gagcaacctt tccctctcct ggggatcttg 43620
agggactatg atgttcaccc gcttccaatt ttgtaaagta cgccccttag agaaactcaa 43680
ggctagagtc tcattataat gtggttggac aagcctttcg gtaatgtatt ccagtagttt 43740
aggggtgtca caccctaact ctaagagagc taaagctaca ttcaacttgc ttcgctgttc 43800
ttagttttta ggaatatcgg cagggcaatc ggaatcccta tctcagaggg tagatgccgt 43860
aaggcataca gggctcgagg ccgctcctca agtgcttgct tttttatcac gggaagagcg 43920
tcctcttctt tccccaaagc aattaaagct tgagctgctg ctaaagtgac atccacatca 43980
ggcttctgca attgcttttt tatattgtag tagctctgac catccttaag cttccctaaa 44040
gcataaagaa tagcttcttg atcttgagga gacgcactcg ttagcaaatt cctaagtgtc 44100
ggaagaaagc gtttttgttg gtattctccg atctgcaaag ctgtggcact ccgaatcgcg 44160
cttttcttgg cagctaagag atcccgaata taagcatcag attcttcagt ctccaagcgt 44220
aggaatattg ccgcagatag gcattggatt tcttcgggaa gcttatgaat gaaagaatgt 44280
agatgatcaa tgactttagt gttcttcaaa ttagcaagtc tataggcggc ttctaagcgg 44340
atgacaggat agggagatgc taaagcttta aacagtaagt cgtcagaagt tttcccaaga 44400
tgtcctgaga ctgccgataa aaccagtagc tgctgcaggg ggtctgcagt ttccatagct 44460
tgggagagca cgtccaaggc ttctgaagat cccgccaggc ctgctccaat gatggtgctt 44520
tttctagttt gcggatctga ggagtggatg ctttgcttga gatagtcttc tccgattttt 44580
cttaaaacga agaagtcatg atcaccgtag gcatctagag cttccagata tgttgctaag 44640
gcctgctgtg tagattgcgt acttatataa aggatcttat gacctacaga ctcagggaat 44700
ttagcaacaa gagaaatggg aagactacac aataaaagtc caaagagagt tagatggaat 44760
agtcccatag atcgatgttc aactcctcta gcaagtattt caatgtttgg atagggagac 44820
cttgaacatt ataaacacat ccatggacct ttttcaaaat caagccacca tggcaaacgt 44880
cataagcgcc acaattattt aatgttccta cagtgtcgat ataagactct atgcggtgat 44940
caggaatcat agtaagtgaa atctgagaag tctctgaccc tgtgaggagt tttcccttat 45000
gtaatacggc aatactcgtg accacatcat gcgtctgatt cctcaaggtc tttaacatct 45060
ggatagcgnn tgctttgnct tgaggttttg tgaaaatacg gccatcgtaa gaaacaatag 45120
tatcgccagt gaggataata cagtcacaag gcgaatgtag ctcagaaact gcataggcct 45180
tttgtgcggc aagttcttgt gtataggcta taggatcccc agagtagctg actttactct 45240
catcaaaatt tgaaggaatg acagtaaagg gaacacgaaa tttttctaaa ataaattttc 45300
ttcttggcga agaagaccct aaaactaaag gaagggacat agaataaaag ctcctcgtgt 45360

tagatacatt acaacaattg cttatgacct gcagtatagt ctaccatagt tccatcgaag 45420
aatgtaatct tatctttatc aaagatcaat aacttcgtag cacaatcttg aatcagtcct 45480
ctgtcgtgag agacaaaaat cgctgtgccc ttatagtcat tgatagccca agaaagagca 45540
gacacagact ctaagtctaa gtggttgttc gcttcatcaa ggataaggac attatggttt 45600
tctagcatca tccccgccat gagaagacga gctgtttctc ctccagataa tgcttggatt 45660
tgcttgaaag catcatcgcc tccaaaaagc atttccccta acacactgcg gatttcttga 45720
tcgttaattc cggttttgcg attgcgtanc actcaaatag cgtttcttga ccacaatcag 45780
ctagaacatc gctgtggttt tgaggaaaat atgaacagat agcttgatgg cctaacttga 45840
tacttcctga agaaggagcc tcaacacctg caagtaactt cattaatgta gttttaccca 45900
atccattgtt cccgataata ccaagcttgt ctccttgata aatctctaaa gaaaaaggat 45960
ggattacctg atgatcccca taatcctttg taatcgcttc taaagacaaa acgactttcc 46020
ctgaggattt gtcagatagg gggaaacgaa tgtaaggacg ttggatattg gattttttta 46080
attcttgtgg ctgaagcttc ttaatctctc ttaatcgaga ctgcacttga ctcgctcgtg 46140
atcccgcacc aaatttagca acaaattcct taagctgaga aattttcttt tccttagatt 46200
tgatgtcggc cttttcttgc tcgcgagacg cggttttcat ctctaccata tcgtcatagt 46260
ttccagggta gataatgatc gtgtcgtaat caatgtcagc aatgtgagtc gtaattgtat 46320
ttaagaaatg acggtcgtga ctgactacaa tgacagtgcc ttcataatct tttaaaaagt 46380
ttcccagcca attaatggaa tagaggtcca agtggtttgt tggctcatca agaagaagtg 46440
cttcggggtg accaaagaga gcctggcaaa gaagaactcg aaattgtagg tctatgggaa 46500
tcatagccat tttcttatca aacatctcat tggggatgcc aatgcctgtg aggagttctt 46560
cggcttctga atctgctcgg tacccatttt cctcgccgat gatctcttca atttcaccaa 46620
gctccattcc aatggcgtca gtaaattctt gtagatagag attatcacgg cgttgtaaag 46680
cttcccaaag acgagtattt cccataatga cacaatctaa gactgttgtg tcgtgaaagc 46740
tatcgatatt ttgacgcagg atccccactt ttttaggtaa ggaaatcgaa cctcttgtag 46800
gttctatcat tcccataatg atttttaaaa gggtggactt tcctgcgccg ttaggaccag 46860
taagaccgta gcagttcccg gggttgaaaa cgacggaaac atcatcgaat aaaattcgag 46920
tgcctaaaga tttgccaatt ttatctaata ctatgctcat agcagacagc ataacaaagt 46980
gctgcttaga gtacaagagc tttgccctgt tctcttaggc atggggatgg gcttcttggt 47040
gggtctgttt ttttaacttt acagagactt gcgtatagac cgttgttgtc tctaaagaac 47100
tatgaccaag aagtgcttgg attgttttta aatccatacc actctctagc caatgggtgg 47160
ctatagtgtg acggattgta tggggggtga tgtgccctga aagtccagaa cgtcgaagat 47220
attcttgaaa acttctgtca atagatcgtg ttgaaatgcg cctcccaaag cgattgagaa 47280
agatggcttg aggatccttt tccaaacgct ttctatccgg atggttcagg tagatttgga 47340
tccattgtat ggcattcgat gtcacgggaa taatcctttc ttttttccct ttcccacgga 47400
tgcgaatcag atgagtactc aaatcaaagt cctgtttatt aacagcgaca atctcactaa 47460
tcctcaaacc cgaactatag aacagctcca tgagacagcg atcacgaagt ccgtgatatt 47520
tagaaatgtc aggagtcgcc atcagcactt caacttgcgc ataggtcatc ggggaaggca 47580
gctccttagg aagacgaggt ccgtggatag tttccgcaga ttttccaaaa gaatcttttg 47640
aataacacag taatgggcaa agcttttaat ggaagagagg caacgcttaa ttgttctctt 47700
agctttgcca ttttctatca gtttcgcgat gtacatgcgt acatgctctt tggtgaataa 47760
ggaaaaagga agttcagaga ctttgcgctt ctctgtggct aactgtaaag gagaagaagg 47820
agcgaggtta ccacgttccn ctaaaaaaat tttcaatcca ttaagatcta gacaataatt 47880
tcttaatgta tgcggagagg cactttttac cattttcaaa tagtctaaga acgaatagat 47940
agaggcaatc ataacttctc tttaccttac aataaagatc gtaaaaagac ttcctcttag 48000
ctttaggaag gctagcttct tactattctt tccgagctat ttatttagaa gaggattttt 48060
cggaaagggg tagctgcgat attcttgtgc tacggacaca ttaggaaata ctgccgaggc 48120
ttctttataa aaatcatcca gatttaagta acgtgccgag aagtgtgtga gaataagctt 48180
ttgtgttgcg gcacgtttcg caagagtcgc cgcttgtttt gcagtcatat gaaaatggct 48240
ctcagcaaga tggcggtgct gttcgaggta ggtgctttca cacaacatca tgcagctatt 48300
ttttgcgaga tctatagcag cttggcaagg caaagtatcc gcaataatcg caatgctatc 48360
ccccttgcgg acgtagctaa catcactaag atataccgta gaaccgccta tcgaaatttc 48420
ttggtcacga ataagatctt ggataatcag accacgaatg cctcgagatt ccagctcttt 48480
aggaagaaat tttatagtgt ctggttccgt gattctccat cctaaagtgt ctacttggtg 48540
ttggagtcgc tgtgcttcaa tacgaaaact accaaaatct tcgacaattc cttcctcaga 48600
gattggatgc tccaccacct ggatggtttc gtgataaatt gttccataac gtaagcggtc 48660
aaagtatttt ttccctgaag caggatagta gcaatggata gggtgcgaaa ccttgtccaa 48720
gttaagacgc atcaacatgg agcctagacc caaacaatga tctccatgga aatgactcac 48780
aaaaattcta ttgactgttg taggagcaat atttgcaaag atgaattgcc tttgagtgcc 48840
ttcaccagga tcaaaaagta gaccctcacc attccagcga aataggtagg ctccttgatt 48900
gcgtgttcgt gtaggctgtt ggctcgagca ccctaaaata attaactctc tagaactcat 48960
aaggatttac tttaagaaaa agtcacagaa taaactaagt gaaattatat acgccactac 49020
aacttttgaa tgacaacttt tttatcattt aaaattttgt tttcttataa ctcctgcatt 49080
gttgtcttca tatttatagg aatcgcaaga tagttatggg tctgtcggtt gcttgcagaa 49140
ccaagggagt atcgctttcc gttgtacggg tggaggagat gaagaatcag aactctcatt 49200

gcctgatgat tgatgttcta gatcttctat agggaggggg acggtttttt tcaatagcac 49260
aggttttctt tttttcgacg gaaccatggg ttttggtgct gatctcttat gtatgctact 49320
tgtcgttcta tgtaaggagt gtttagtttt cttcttcttc tttgagcttt ctgttgttga 49380
agttttagac ccttttgtag ttgccgtatg aggtgctttt cccttggctt tgcgatggag 49440
ttcaggggaa gattcatcag aggattcaat ttcagaagaa cgcttgcttt gcctaggtga 49500
atggtgttca tcagagatgt gaggactctc ttcaccactt ttgaatttgt ttgttacata 49560
gttgacgcct ttttctaatt gcgttcttat gaagtttttt ggttcgtggt catgatgtgg 49620
ggagagttgc gggctccctt ttctagacgc agattttgta ggttgtgatt cagaggcatc 49680
ttttggggca atttcttttg ttttttttgt ggatatccaa ttaatgagac acggattgaa 49740
aatactcaag agcactagaa gtgccaatga gcttaaacaa agaggaattc ctacagaggg 49800
aataaacatg cacagcatag ctccaacaag aactacgacg acgagaacgc atataatgat 49860
taggtaagca atgttgcaaa tgtctcgaat cgatttggaa ggagccgctt gtttgatcgg 49920
ttgaggttta tggaaatacg aagtcactga gttgcgcagg ctaggaataa gatgggacat 49980
agcaaagtta gattaaagct ctctgaaaag aacagcttat gtattcaagg tctgtttgta 50040
aatatacaga cttttttata cgcattaaat ctaccttaat gcgtataaaa atagtgattc 50100
tcaagagaac aagggttcaa cttggctttc tattttagct tgggaaaagc aactttgaga 50160
acatcatcat agtgtgaaac aaaatgtatt ttcaaccctg ttttcagata tgcaggaagc 50220
tcttcatagt ctctacggtt gtcttcaggg aaaatcaaga tgttcaatcg tgatcttcga 50280
gctgcaatca gtttctctcg aatgcctcct acacctaaaa cacgacctgt aagagtaatt 50340
tctccagtca ttcccaaatt attcactacc ggagtttcca acagcaacga aaggagagag 50400
gtcaccatag taattcctgc agaaggaccg tctttagggg tggctccctc aggaatgtgg 50460
atatgtactt gagactttgg aaagaacgta tagcctgggg cataccgatg gagagcactg 50520
tgaaggtagg tccaagcaat ctgagaagac tccttcatta cttccccagc ctgacctgta 50580
aggtgcatgt ctgttttgag tgaggacacc tgtacacttt ctatatataa cgttgctcca 50640
cctaaagagg tccaggcaag tcctgtggcc actcctacag gagtggattc atagaagcga 50700
tcgctagaaa atatcggttt ccctaagtag gtctgcagat ttttcgaaga gattttaaaa 50760
gtaatcttct tagatttggg tttttcttga ttttgaacaa tctttaaagc aactttcctc 50820
aatacttttt tgatattccc attaagagta cgtaccccag cttctcgtgc atagttattg 50880
atcatgtact ttaaagcttc aggttggaaa ttcacttcgc ttgctgttaa accgatttct 50940
ttgcgagctt tgggaactag atacttttta gcaatttgaa gtttctcttc taaaatgtag 51000
ccagaaagtc ggagaatctc catgcgatct aaaagaggat ccggaatggt atctagaaca 51060
ttggcagtta aaatgaatag tacattggat agatcaacac gtacgtctaa ataatgatca 51120
agaaaatctt tgttttgctc aggatctaaa acctctaata aggcagaggc aggatctcca 51180
tgataactcg caccaatttt atctacctca tcaatcataa tcacaggatt catagcttgg 51240
ctttgcttga gcgcctggac catttttcct ggcattgccc caatataggt gcggcgatgc 51300
cccttgatct cggcctcatc acgcatgcct cctactgaga aacggaagaa ctttctatgc 51360
aggactttag caatgtgcgc cctatgcttg ttttcccaac tcctggaggg cctacaagac 51420
agatgatact tccttttaat cctttggaaa gcttacctac actgattaac tcgagaatgc 51480
gttgtttaat ctcatcaagg ccatagtgat ccttgttcag gacgatttct gctttcttta 51540
agtcatggta ttctttactt tgtatgcccc aaggaatgat tgtcagccaa tccaggtaat 51600
tgcggcatac ggtatattcc gccgaagaag tttctaaagt ctgaagtttt tcaatttcat 51660
cttgaatcac ttccatagca taatcaggaa catggcgttt tcttagcctt tccgaaaact 51720
tctctatatc aatagcgcgg tcttctttct ctaaccccag ttctttttta ntcgttttta 51780
actgctcttt taagaagaat tccttttggc ttttngtaat cgtagcttca attttttgat 51840
taatgctgct ctggaggcgg cttaagtcta attccttttt tagtaagatc agtgccttat 51900
caatgcgatc atgcatgttg gtggtctcta agacctcttg gagctcttcc cgagttgctg 51960
ttgttaaagc aacagagaaa tccgcaagct tgcctggttc agtaaaatcc gaatgaccaa 52020
gaaaaatttg tagttcttct ttaaagagag gatttagttt taaaaggtct ttgatgacag 52080
agacaatact aatagagtac gcttttagct cttctgtaag ctctttattg tccgcatgat 52140
aggaaactcg agctttaaga tatttgtctt taatgggttc tataatccga atacgctctt 52200
caatgcttaa aagaacttga gcactgccac cctcgattgg catgatacgg aggattcttg 52260
cggcaactcc agttttatgc agctggttga agctcacttt taaaatatcg gcgttctctt 52320
ttttggttaa gacaagacca atatattttt gagacgactt cgctaaaacc tttaatactt 52380
cataataagg acccgactca ataagaatgg gagccgccat tccggggaaa aaaggtcgtt 52440
tatttaatgg caggataaat aactcagaag gcagcaaacg ctccgtagac tgatcttcgg 52500
attcctcttc agattcatct aaaagctttt caacatcttc tggatttgga tctaagatgg 52560
gggagtcgct attggttgta gagtccacaa ttgtccttat gctcggctat atttgtctgt 52620
cgctccagta aatacggctc tttttctcta gggtaaaaag agggagcgat tttaaaagat 52680
tgcatatttc gctatactac aaaatagatg caattaggcg catatataca acttctaaaa 52740
aatatctaga agggattaaa gattaggaaa gactcgtgtt taaggcaaat cgtctttttt 52800
tattttttca aaaaaaatag tttgtatcta ctgtttaaaa ttgctcagag ataagggatt 52860
aagaaaaact ccattgtttt tgagtttttt acttttatag ggggtagatt ttccaaggga 52920
aaaacgtcag tagtccaaat ccagcctttt tgtccttcgt ttgcattgaa ggtaaatgca 52980
gggatcggaa gacaaaagaa cacgcttgca ttgtttttaa tttaacagcc atttcctctt 53040

gttgttcttt atgataatta agagcccgtt cctcgcctga aatatttgga acagatacca 53100
aggaatgaaa atagaagtta gggagaaggg aattataaga gaagccaata tcttgctttg 53160
gtgaaggagc ttgaccaaga tctgtgattt tagtgaaaga aaacccttct tgtataagct 53220
cgttataggt ttttccagaa cgcactaaaa tgtgtagcgc atgaattttc tttaatgttg 53280
taggagatgg taaaggaaga ctgaggtttt cttggtcagg tgtaagtgtt tttttccaaa 53340
tcctccttct taacatctng gattagacca cgatacttga ngaataagat aatncgtaaa 53400
acgatcttaa taaagagagc tagaatcaga ggaagaatta aaatatagga gagaattttt 53460
attatttttt cagatttcga gattgggagg gcatgatcaa cagatagaaa ggtagctcca 53520
gaaactcctg tgatgataac agaagtgtct cctcccaaat gtaaatagtg ttcaacgata 53580
ttggaacatt tttcaataat gccagagctt tttcctaagt aaggaagaaa agataaggac 53640
tgatccgaga agactatagg atgaaaaaac tgaagaaaca tacgttagaa tttttatttt 53700
ttttatatta taacgtacta gccttcaaaa agtaactatt ctttatggct tagtcttgtt 53760
tctctcagaa tttagataaa cataaaattt gatcttcagt tccataacag aacgactcca 53820
aataatctct tgtaattgtg cggccatagt tggggggatt cttggatctt caatctcaaa 53880
tgaattcaga tattgaaaac gggacgtccc atagagaata tcggattggg tagcttctcg 53940
tttgcctagt tgttggcgta agcttattaa actactctta atagaagtag ttctcaattt 54000
gatttgaaat tttttcaaga gttcctgacc ccctttccta agcaaggaag aatcgagtag 54060
cattgggcgt aaaagtatat aggagtaaca aaatcgcaac ccatcgttcg tccttatatt 54120
tagagcggcg aagaatctaa tgtatctgaa taaattaaaa caataaatgt tttcatttct 54180
ttcattttta tttgattaaa aggggtttgt tggtatagtt tattttttta tatcaaaaat 54240
cgacgcatgt acttctataa gtatgttatc atagatactt ctggatacta tccattctta 54300
gcttgtgtgg ataatcaaca agtgctggag cattggtctt tgccagtcgg tccagatctt 54360
ggaattgttt tagagtttct ttttaaaagt aaaaatctct cttttcaggg agttgcggtg 54420
gctctaggtc caggaacttt tctgcaacac ggatagggat ttctttcgct caaggattgg 54480
caatggcaaa gaatgtgcct ttgctaggat atagctcttt ggaaggatac ttattatcta 54540
aagatgaaaa aaaagcttta atgcttcctt tggggaaacg tggaggcgtc ctgactttaa 54600
gctctgagat tcctgaagag ggcttgaatg aaaaaaggag aggggtgggg ccgggagctt 54660
tgctctctta tgaagaggcc tctgattact gcgttgctca tggatattat catgtgattt 54720
ctcctaatcc gcagctcttt gcgagcagtt tttctgataa gatcaccgta gaagaagttg 54780
ctccttcggt agaacagatc cgcaggcacg tgatttctca attcatgttt gtagaatatg 54840
acaagcagct ctctcctgat taccgtagct attcatgtat tttttgattt gtatttctta 54900
aatatttttt acattgttgc ttccgaatcg attcaactat cccttgaagc ctaatagaag 54960
tagtggtaca atcgaggctc ttctaaaaga catagtaaaa agattattat tgtgtttata 55020
gaaggccaga gatttgttat ttattgcgta ataataaggt aatgcatgcc cagtgttaaa 55080
gttcgagttg gagagcctgt agatcgtgct ctgcgcatct taaaaaagaa aatagataaa 55140
gaagggattt taaaagctgc taaatcccat cgcttttatg acaagccttc tgtcaagaaa 55200
cgagctaaat ctaaggctgc ggctaagtat cgtagtcgtt aattggcaat gtcgtattcg 55260
ttggtagtgt ttcaggtatg gattattatt caatttaggg catttctaaa actgcttccg 55320
cagaagaaat taaaaaagcc tatcgcaaat tagctgttaa atatcatccg gataaaaatc 55380
ctggggatgc tgcagcggaa aaacgcttca aagaagtttc cgaagcttat gaagttctca 55440
gtgatcctca gaagcgcgac tcttacgatc gtttcggtaa ggacggtcct tttgctggag 55500
ccggtggctt tggtggcgct ggaggcatgg ggaacatgga agatgccttg cgcactttca 55560
tgggagcctt tggcggagag ttcggaggtg gaagcttctt tgatggtctt tttggtgggc 55620
ttggtgaagc ttttggaatg cgctcagatc ctgcaggcgc tcgtcaagga gccagtaaga 55680
aagttcatat taatttgact tttgaagaag cagctcatgg tgttgagaag gaacttgtag 55740
tttctggata taaatcttgt gaaacctgtt ctggtcaagg agctgtaaac cctcaaggga 55800
ttaaatcctg cgaacgttgc aaaggttcgg gacaagtggt acagagtcgt ggatttttct 55860
ccatggcctc tacatgtcca gaatgcggtg gcgaaggccg tattatcaca gacccttgtt 55920
cttcatgtcg cggccaagga agagttaaag ataaacgtag tgtccatgtg catatccccg 55980
caggtgtgga ttctggaatg cgcttgaaga tggaaggcta tggagatgca ggccaaaatg 56040
gagctccctc cggagatctc tatgtcttta ttgatgtaga gtctcatccc gtatttgagc 56100
gtcgtggaga tgacttgatc ctagagcttc ccattggttt tgtagatgct gctctcggta 56160
tgaagaaaga aattcctacg ttattgaaga cagaaggatc gtgtcgtctt acggttcctg 56220
aaggaattca aagtggaacc attttaaaag taagaaatca gggctttcct aatgttcatg 56280
ggaaaggtcg tggagatctt ttagttcgca tttctgtaga aactcctcaa aatttatcag 56340
aagagcaaaa agaactttta cgtacttttg cttctacaga aaaagcagag aactttccta 56400
agaagcgtag ctttttagat aaaatcaaag gttttttttc tgacttcaca gtataagaag 56460
gagaaaagac cgactttagc tgagagagat ccatgggagt agtacaaaat caagttattt 56520
cttctataag agatgtttta aagctagtct gggaattgcg gttcgcagag cataagatgc 56580
ttctcctctc taggcagagc ggctcgggcg gcacatttca gttgtcttgt gcaggtcatg 56640
agcttgccgg cgttcttgct ggtaaaagtc tcattcctgg taaagactgg tccttccctt 56700
attatagaga tcaagggttc cctataggct tgggttgtga tctctctgag atctttgctt 56760
cgtttctagc tcgtacaact ccaaatcatt cctctgcgag gatgatgcct tatcactatt 56820
ctcataaaaa attgcgtatt tgctgtcagt ccagtgttgt aggaacacag tttttacaag 56880

ccgcaggtcg tgcttgggct gtcaagcact cgtcagctga tgaagttgtc tatgtttctg 56940
gaggcgatgg agctacatct cagggtgaat tccatgaaat gttgaacttt gtagcactac 57000
accaactgcc tttaatcact gtaatccaaa ataatcattg ggcaatttct gttccttttg 57060
aagaccaatg tggagccgac cttgccagct tgggtcgttg ccatcaagga ttagctgtct 57120
atgaggtaga tggaggcaac tatacttctc ttacagaaac tttttctcat gccgtagatc 57180
aagcgcgtca acattcggtg cctgcattga ttttaatcga tgtggttcgc ttgagctctc 57240
atagcaattc cgataatcag gaaaaataac cctccgcttt agacctgaaa ctatccatgg 57300
ataaggatcc cttaatcctt ctagagaaag aggctatcaa tgtttttggt ctgtctccct 57360
ttgaaatcga ggagatcaag gctgaagctc aagaagaagt tcgaaaatct tgtgagattg 57420
ctgaagctct tccttttccc tctaagggat ctacaagcca tgaagtcttc tctccttata 57480
ccgagactct cattgattat gagaattctg aaagcgctca gaatttgcgt aactctgaac 57540
ctaaagtgat gcgtgatgct atctccgaag cccttgtaga agagatgact cgagattctg 57600
gagtcattgt ctttggtgag gatgtcgctg gagataaagg aggagtcttc ggtgtcacca 57660
ggaatttgac agaaaaattc ggaccacaac ggtgtttcaa ttctccctta gctgaagcaa 57720
ccattatagg aaccgccata ggcatggcct tagacgggat tcataagcct gtcgttgaga 57780
ttcagttcgc agattatatt tggccgggga tcaatcagct attttctgag gcctctagca 57840
tctactatcg ttcagctggc gaatgggaag ttcctctggt aatacgagcc ccttcaggag 57900
gctatatcca gggaggaccg taccattcgc aaagtataga agggttccta gcacactgtc 57960
ctggaattaa agttgcctat ccttctaatg ctgctgatgc taaagctttg ctaaaggcag 58020
cgattcgaga cccgaatcca gtagtgtttt tggagcataa ggccctctat caaaggcgta 58080
tttttagtgc ctgcccagtt ttttctcatg actatgttct gcctttccgc aaggccgcta 58140
ttgttcatcc cgggaaagat ctcacgatag tttcttgggg aatgcctctg gtattgagtt 58200
tagaggttgc tcaggaatta gcctctcggg ggatttccat agaagttata gatttgcgta 58260
ctatggtgcc ttgtgacttc gctacggttc taaaatcctt agagaaaacc ggaaggttgt 58320
tggtgattca cgaggcttca gagttttgtg gctttggcag tgagcttgtc gctactatgt 58380
cggaacaagg atacgcttat ttagatgctc ctatccgtcg tcttggtggg cttcatgctc 58440
ccgttcccta ctctaaggtt cttgaaaacg aagtgcttcc tcataaggag tctattttac 58500
aagccgcgaa aagtctcgca gaattctagg cgattctcca gcttctttct aggccgggat 58560
tttaattcaa aaagagagcc cgacacgctg tttgtagagg tacttgggag atgctattta 58620
ctgaaacatt agagagtgcg tactcttctt tggatgctaa taaaatgcgc acggattcta 58680
aaagagcaag atggcaacaa ctctcccatg tagtaaattc ctcttgatct gcgctgaggt 58740
tatttaatcc aaaaacttct aactgtacac tttcaagatt gtgctcaata cagatatcaa 58800
agcattgcaa ataagccagt actaaattag taaatgcctc tttcatacta aaatctttca 58860
atgtaattgc gcggcgcatt ttttccttaa tgagagtttc taatgtaggg ggattgataa 58920
caatcagatg agaggtgtac agttgcgcgc cctcttctac atcccaaggg ccagaaacgc 58980
atgaacctat aggcaagtgt ttttctggag tgggaatgtt gtttttgatc ttttcccagc 59040
agctcctttg taaacatgtt gtaacagcaa acgctcttcc taatgttgtg gagaaatcca 59100
tgctagaatg tgaagagata acaacagccc ctgatttttt atttagaatt ttatcatgag 59160
aaaaatgtcc tttaaagcag tgaaaacgga atccagattt ttctttagat ctcaggagaa 59220
tacttgctag cttgttggaa tacttacgat cttcgggtct ggtttctgga tctagagaga 59280
atagctgctt gagtaacgaa aaacaagcct taggatcata cgtagaggga taatgtatat 59340
caggattgca ggtcagtttt gttctatcga ctttaggtag agttggaggt aagggtattc 59400
ctggagctgg cgcgggtatt tctggagtta gtgtgggtgc tcttagagtt ggtgagggtg 59460
gaggagtggg gagtggctca ggaatcttag gtttttctag gtagtgtata aaatacaaca 59520
acgtaaaagc aataaagact gcggtaagta tgaatagagg catggtgatc tctagggaac 59580
accctaggca aatggcaaaa actcccccta ggcaagatag gaatgctaaa atggcaaaag 59640
caattgcgtt gactattgaa gatggagttt ctttcttgcg ttgaggttta aaaggtgcct 59700
gcgtggggat tgtagggata tggtcaggag aagaatctgg atccggaaca ggcggtgtct 59760
tctgggatac aatagaggag tcgggtaaag atggagtcga tatttccgcc ataagaattc 59820
ccctacaagt tgttggcaat aaaaaattta cttaatttta atataaaaac aaatcagaaa 59880
aacaaagatt attttgattt gtcattagaa tattgttgtt ttatagggtt tcgatgaagc 59940
gataagaagt cgcagaagtt gatgcatagg gggagacgtt tgtgcgtaaa attccaatga 60000
agaataaaat aagagaagct aaggtaagca tccaaggagg gacaggagca aaggaaagag 60060
tctttaggat attggggttg tgtagccaag gatgtgagag gaatccctgg ataagcgctt 60120
cggtgatagg ggagcaacat ggcaagatga ttgttgcaat gaggaaaaca atgatgggga 60180
gaatggtaaa aggaacaatg agattataga ggagaccctc aaggggtaag ctcccaaagt 60240
actgcatgat tggcagaaca ataaagagtt gtgccgatag tgaaattgcc aaagtcatgg 60300
caagatagcg gataggatac aaccaaaatg gagagagaaa ctgagtccaa ggggtgtaga 60360
gaaaggaaaa gatcttcggg aaaaagagta gaatccccaa cgtcgctaag aagcttaaaa 60420
caaaggtagg agagaagcgc gaaaaaaaga tagaacataa aataaagcct gcgcctagac 60480
gatttagccc cgaacaggac ccagaaaaac accatgaaaa acaaagtagg gttacggaga 60540
tccaagagcg ccataccgag agagacatag ggaaaataca ggatagagaa gtcagaacaa 60600
tgaagcttaa gatttttttg atttttaaag gaagaagagc acagagcatc cagagagtag 60660
tagcacacag agagaaatgc cagcccgaga tagcaaagag atgagataac cctttttgtc 60720

tgaagaggtc tctgagattt tgaggaaggg gagttcctag cagaagactc gaagcaaagg 60780
ggcctacctc agaagaggga aacctatggt tcaggaaatg gcagctagat tctcggcatt 60840
tctctttcat gatatagaac ctagacctag gaatttcttt gtagcaagca ttagacttaa 60900
aaacaatttg agatgtatga tgtagtgtcc cttcgagctc gtaaaccttt ttaagctcta 60960
aacgggattc tgatagaatt tgacacnaga ggtggtgggc tcttttnccc acagggcgtc 61020
tgaatacaaa gagcttctcc gtagtagntc cttgcccccc tgcgtggtga atcacaaaag 61080
tccctgatat agggccgtca tggaggaatg gcgcaggagt taacatcaaa gaaatgatcc 61140
aagcacttgc taaagggagc cattgtttag ggttgcgagg aaggaacatt cctaagaaaa 61200
taagaatcag agcactacat tctgggtagc cacgggatgt gatgccagca agccaataga 61260
gcccacataa gaaaatagga tgtctttgtt ggaagcactc acaagaggcg cgtaatcgaa 61320
tgagccaaga gcttatggga tagcgaaaga aaaaagatga aaaaacctga caacgattcc 61380
acatttgatg ttcgctcttt ctttcccttc gatgtgttat gtatagagca gttgcgcaag 61440
gaaatgtctt gggaagtggt ttcagcgaag atcccgcgtc ttcctcgagg gtggtacgag 61500
ctcatgggac tatcaaaaga agatcgtata gatttttgct tagacttctg gtgttccgta 61560
ctagggattg agcataaaga atctccaagt atttgtcgtt tttttctttt actagagacc 61620
attgaagttt acatctatcg cttggaaaaa gaaccttatc aactaaagat gttttatgta 61680
ttccgtgatg gtcgttgtgg gtttcaagga gagcctcctc ttctagattt tttagggcat 61740
cataggctgc ctcctttagg ggaccgccat tacgagaaat ttttctctat tcataatgga 61800
ttcgggaaat gggaggatga ggggattttc cccatgaggt ctttagcaaa ggtacaacaa 61860
aaattacgtc agcagctcgt tgtaatgaat aagatgcagg cggaagataa ttgttattct 61920
ttaggtatct ttccttttta tggctatgaa gagccttttg cttatcagag tttctttttt 61980
gatcctgaaa tacgcagaga ccttccttct ccgaatgtgt tgttaaatga agagagcttg 62040
gagcatcgaa gcttagaaac tattgagttg ttgcatctgt ctaagagcta ttatccttct 62100
ttcctctcgt ggttggagaa ctatctacat agtgaggagg tgtataatga atgagcctac 62160
tcgcacttat ctagaaagtg agaaagatac acaagatcag atcgaagagc tccaggcaac 62220
ttgtatagtt aagaatgcag caggaatcca tgtgcgtcct gcaggtgtta ttgttcgact 62280
ctttgatgga gagccttgtg atgtgcattt cacctacgca ggtaaaacga taaatgcaaa 62340
gagtatcatg agtattctta tgttgggagc tccacaagga ggagagattc ttgtgactat 62400
tagaagcaaa gaagctcatc gtatcttaca aaagatacaa gatgcgttta gttccggttt 62460
tggagaacta taaatggata cacagtcctc tataggtaac gaagaatggc gtattgcagg 62520
aacctctgta gtttctggga tggccttagg taaagtattt tttttgggaa catccccctt 62580
gcatgttcgt gagctgactc tacctcaaga agaagtcgaa catgaaatac atcgttatta 62640
taaagctttg aatcgctcga agtctgatat cgtagcttta gaacaggaag ttacgggaca 62700
gcaaggcctt caagaggttt cctctatcct acaagcacac ttggagatta tgaaagaccc 62760
tctccttacg gaggaggtgg tcaatactat ccgtaaggat cgtaaaaatg cagaatatgt 62820
cttttcttca gtcatgggta aaatagaaga gtcgttaaca gcagtccgcg ggatgccttc 62880
tgttgtagat cgtgttcaag atatccatga tatctccaat agagttatcg gccatctgtg 62940
ttgccaacat aagagttctt taggagaatc tgatcagaat ttgatcatat tctctgagga 63000
attgaccccc tcagaagtcg ccagtgctaa ctctgcctat atccgagggt ttgtctcatt 63060
agtgggagca gccacatcac atacagctat cgtctcgcga gcaaagagca ttccctatct 63120
tgctaatatc tccgaggagc tttggaacat cgcaaagcga tataatggca agttagtctt 63180
aatcgacggt tatcgtggag agctaatctt taatcctaaa ccagcgactc tacaaagctg 63240
ctataaaaaa gagctttccg tggttgccca tacctctcag agattagtaa gaaagtccct 63300
acacccgatt gtttcttcgc atgcaggcag tgataaggac gtagaagatc tattagagaa 63360
cttccctcaa acctccatag gcctctttcg ttctgagttt ttagctgtaa ttttaggacg 63420
cctacctaca ctaagagagc aagtagatct ttacgagaag ctcgcacgtt ttcctggaga 63480
ttcgccctca gtactgcgcc tctttgattt tggtgaagac aaaccttgtc ctggaataaa 63540
aaataagaaa gaacgttcta tacgatggtt gctagactat agtgtgattc ttgaggatca 63600
gctccaagca attgctaaag cctctttgca aggctccata aaggttctca ttccaggagt 63660
gtctgacgtt tctgagatta tagaagtcaa aaagaaatgg gagaccatcc agacgaggtt 63720
ccctaaaagg ccataaggtt tcttggggga ctatgataga atttccttct gcagtttgga 63780
tgattgaaga gatccttcct gaatgtgatt ttctctctat agggacgaat gaccttgtcc 63840
aatatacttt gggaatttcc agggaatccg ctcttcctaa acatctaaat gtaactttgc 63900
ccccagcagt gatccgcatg attcaccatg tacttcaagc tgcgaacaaa atcaggttcc 63960
tgttagcatt tgtggagagg ccgcagggca gctcagtctg actcctttat ttataggcct 64020
gggagttcaa gagctctcag tagctatgcc tgtaatcaat agacttcgca atcatatcgc 64080
cctgctagag ttgaactcct gccttgaaat tacagaagcc cttttacaag ctaaaacatg 64140
ctctgaagtt gaagaacttt taaatagaaa caacaaaatc acatcataaa aattccatta 64200
tacttttttt atataaagat cttttatgat gtataaagtt tgtaaaaact gtgttttcta 64260
gtcgtgccaa tgtgcaatag gaaaaaatac caactccacg gcggatctct agttctagaa 64320
aggcattgtg gaacgcataa gagacatttc ttgatccatc tgctcttttg caagtttgaa 64380
tgcagcacgg aacaagtctt caatgacttc tggatcttca gggtctaagc acgtaggttg 64440
tacttttact gaaattaagt cacactttcc gttgatgaca acagagacaa gaccgttgcc 64500
agcttgccct tcgtaacgct tttctaatag tgaggcctcc atttctagga attgctgttc 64560

cataatttta gcttcttttt ttttcttagc gtatccgctg cccatgctta tctatcctta 64620
ggtgaaatat tgatctttcc tctcatgaaa ttttctaaga ggttcttgct tattgtctta 64680
aaattcctga aaattctaca acagcaaact gtaataatgt gtctacagct gcagatttta 64740
ttgaagctga gcttttaact tctacagata taattttacc ttccgcagca ggttgatttt 64800
ttttctctaa aaaactctgt tctttgtagg tgggctgagg ttgaggagct gatacttgct 64860
gcgtcaaggt gggctcctta atattgcgaa gcccttcaaa ctgccgactc ttaatagaag 64920
agatcaactc tgataaaaca ggcctttgat aaatgcgaat gatatgaatg atgacggttt 64980
ctaaaaatgt ctgttcgaag atggtatttt gtaggtgctt agcagattct ccaaggaaat 65040
ctattatttc tagaagctgc tccgtcttat actgagagct gaacttgctt gttgtagaat 65100
tcgtaagaag aagattacga taaaataatg taaggtcatg gagaaatgtg acaggtgcta 65160
ccccagaatt taagaagtcc gttacgatcc ctaaggctgt cgcatagtcc ctttgaagaa 65220
tcgcattgtc taaagtccgg agagaatctt gggaagcaaa gcctaaagct tgggcaaccg 65280
tgtcgggaga gagagattta ggaaataaag atattacgta ggtcataaag agattctgca 65340
tcacgcaagc ttccttgtgc tgcacgggcg atcggcgcca atgcttcttg cgacgcctca 65400
atatggtcat cttgagccat aagcgatagc ttctccagga tcgttttttc aggaatcctt 65460
tgaagatgca ttttttgaca acgacttaaa atagttccgg gaattttatg gatttctgta 65520
gttgcaaaga aaaattttac atgttgtgga ggctcttcta aagtcttcaa taaagcattg 65580
aaggcttcct tagtgagcat atgaacttca tctatgatat aaattttaaa ctttgctttt 65640
acaggagtga ataatacagt ttcattaatt tgacggatat cttcgatacc acggtgggag 65700
gctccgtcaa tttctaaaac gtctaaagag gatcctgaag caatctcttt acaagaaaaa 65760
cactggttgc agggctcgcc atcctcgcta agatgcacgc agttcagagc ttttgctaaa 65820
atgcgagcta gtgtggtttt ccctgtacca cgaattccag aaaatagata ggcgtgggcg 65880
gctcggttga agaccaaggc attttttaat acagcgacaa cagagctctg acctagaatt 65940
tctcgaaaga tttgtggacg gtactttcta gaggatgctt ggtagggttg tagagtcatt 66000
gtataaccaa gagaatgtgt atagaaagct cattttctca tttaagagat tttttcttga 66060
agaccttttc tgattttcat aagaaaattc ttttcgcaga gatggaatga ttttccttct 66120
aaaatagaat ttgtgaattc ttctttagaa ggaaaatgaa tctctttgaa taaaatacta 66180
tatattagta gcttagtggg tttaacttat gtgtttgatc gcgatggcac cacagattca 66240
taatgcaagt acctctatca ccacagctac ccccctcccc aacactctgt agggtcgatt 66300
tcttctcgat ataaacttcg cgttttagcg attacttttt tagttcttgg tgtgctttta 66360
ctgatttcag gagctctctt tttgacgttg gggataccag gactcactgc aggggtctct 66420
tttggattag gtataggtct ctctgcgtta ggaggagtgc ttgttgtctc aggactacta 66480
tgccttctag taaaacgaga ggtttcgaaa gtatgtcccg aggagattcc ggcagtacaa 66540
ccagaagaga ctcctgaagg ggttcctgtg actccatttg agaagccagc tctagatgaa 66600
gcccagaagg agcagaagac tcagaaaatt ttagatcagc tgcctcaaga attggatcag 66660
ttagataggt atattcagga agtgttcgca tgtttaggac cgctgaaaga tcttaagtac 66720
gaagatcaag gttttttaca agacgtcaag gaggagtttc aagtttttga ctttgttcaa 66780
aaagatatga ttgcggagtt tgtagagcta cagcagattc tatgtcaaga agggaggttg 66840
ctagagttcg taatcaatca gacacgatat ataggaagag atctttttaa aagagaggat 66900
agtttatata aattatggga atggcttggg tatttacctt ctggggatgt tcgaggggag 66960
cggttaaaga aatctgctcg tgaggttgtg gatcgcttta tgagaacgac ttgtaacata 67020
cggaagatag ccatgacttt tgataggcat gtttatagtg tggcgaagac ggcctttgaa 67080
aaggcatttg gagccttgga gacgtgtgtg tatgagagta tgagagagag ttatagagag 67140
gcattttgtg agtatgagaa ggcgaagctg cttggggatg aggagaagag tgcacatgcc 67200
gagcaaaggt ttcaggatat aaagaaccgt tgggaggatg taaaggatgc attcttttgg 67260
gtaaaagaag atggggaaga ttgaaattga tgatgcaatt ggaaacagtt gtaaatggag 67320
tgagcgttat gaagagcaca ggattactcg agcaagatgg tataaggtcg cggagcatca 67380
gttgtttaat gcgactatga gagtgaaaga ttcgttacga gagcataatg aagcaagagt 67440
cgcttttgag aaggagagat ctaaggagaa tcagaggcaa gtccaaaaaa agaaagaaaa 67500
gaggttgcga gatttaaagg aattgcatga tcaggagctt ccgagagcac aggagaggtt 67560
gagagagctg caagctttgt atcctgaaat tgcagtctct gttgtagagg ccaggagaga 67620
ggtagcctct gatttagaga aagctcatga gagtattgac aagcactatc aaagctgtgt 67680
tcgagagcaa gagctctact gagaagaaga agagaaacag gaagcggagt ttagggagaa 67740
cggcacaaag attcgctcta tggaggaggt gtctgagtat cttcagcaag tagaaaatca 67800
gttggaatcc tgttccaagc gattaaccaa gatggaaact tttgccttag gtgtgaggtt 67860
ggaagctaaa gaagagatag agtctatcat actttctgat gtagtgaacc gttttgaggt 67920
tttatgtaga gatattgaag atatgctatc tcgagtcgag gagatagagc ggatgttacg 67980
tatggcggag cttcctgtac ttcctataaa agaagcgctt accaaggctt ttgtacaaca 68040
taacagctgt aaagagaagt taaccaaggt agagccttac tttaaagaga gccctgcata 68100
tctaactagt gaaaaccgat tgcagagttt gaatcagact ttacaacgtg cgtacaaaga 68160
gtcccaaaag gtttcaggtt tagaatcgga agtgagagcc tgtcgagagc agcttaaaga 68220
tcaagtaaga cagtttgaaa ctcaaggagt gagcttgata aaagaagaga ttctctttgt 68280
gactagtacc tttagaacta aatttagcta tcattcattt cgattacatg ttccttgcat 68340
gaggttgtat gaggagtatt atgatgacat tgatctagag agaactcgag ctcgatggat 68400

ggcgatgtct gagaggtata gagatgcttt tcaggcattc caggagatgt tgaaggaagg 68460
cctagttgaa gaagctcagg ctcttagaga aaccgagtac tggttatatc gagaggagag 68520
aaagagtaaa aagaaacatt gatttgcgct aagctaacag cagcgcagca gcgagttgca 68580
gcatttgaat ccatagaagt tcctgagatt cctgaggccc cagaagagaa accgagtttg 68640
ctggataaag cgcgttcttt atttacccga gaagatcgtt cttagaacca ctctaggagt 68700
ctctaggccc tgttttttta aattctttga ataaaatact atatattagt ggctgagtta 68760
gtttaactta tgtgtttgat tccgatagca ccacagattc ataatgcaag tacctctatc 68820
accacagcta cccccccccc cccccccccc cagaccaatc tgtaggggct tctttttgtc 68880
tgtctaaatt tcgtgtttta gcaatcactt ttttagttct tggtgtgctc ttactgattt 68940
caggagctct ctttctaacg ttagggattt caggagtctc tcttggagtt ggtttggggc 69000
tctctgcatt aggaagtgta ctcgttattt cgggatttct attgctttta gaaagacgag 69060
aggtttcggg agtgggttta gaggggattc cgacaggtat tcctgtgggt ccttctgcag 69120
aaccttcttc agaggaaata cagaagaagc aaaaagcaaa gcaaatttta gatcaactgc 69180
ctcaggaact agatcagtta gatacggata tccagcacgt gctctcatgt ttagggaaac 69240
tgaaagatct taagtgcaaa gatcgaggtc ttttaaaaga tgccaaggag aaactgcaag 69300
tttttgactt tgtttggaaa gacatgatga tggagtttgt agagctacag caggtcatgg 69360
atcaagagag ccggtatcta gagggcctga tccatgaggt acaaagtata gcacacaaac 69420
tttttgtaga tgatgtaaat attagatccc atttagggga gtcgtgcggg tatttacctt 69480
ccgaggatgt tcgaggggaa ctgttaaaga gattcgctaa agaggtcgta gctcgcttta 69540
tgaaagtgac tcgcgacata cggaagatag caatggcttt taacaaaaat gcctatgggg 69600
cagcaaaaaa tgcctttgat aaggcttttg gaagcttgga aacgtgtctg tataagagtc 69660
tgactaagag ttatagagat accttttgtg actataagag agcaaagatc cttccggatg 69720
agaataatag cgctcgtgcc gagcaaaggt ttagggaagt caaggatcat tgggaggact 69780
taaacgaaac ggtcttttgg gtaaaagaag acggtcgtat tgacatagaa gtgctcactg 69840
cagtcggtgg gtggccagat cgttatccag agcatcttat tcttgaaaaa agaaaggata 69900
aggtaatgag ccatcagttg tgggaggcga ctatgcgtgt gaaagaagct gaagtaacgt 69960
atagtgtagc aagagtcgcc tttgaaaagg atggatctca gcagaatcag aagaaattcc 70020
aagaaaagac aaaagagagg ctgcgatgtt taaaggattt gcgtgatcag gagtgtcatc 70080
gtgcacaaga gagattagaa aaactgacgg ctttgtatcc tgaggtttca gtctctgtag 70140
tagagacgga gagagagagg aaatttaatt tagagaaagc ctatgggaat ctcgaagagc 70200
gctatcagag cgttgtgcaa gatcaagagg actactggac agaacaaaag aacagggaag 70260
cagaatttag ggcgaaagga actaaggttc gctctatgga ggaggtggca gagcatcttc 70320
agatcttaga aaatctattg gaagactgtt ataagagatt atcaaaagca gaaacttttg 70380
ccttaggggt ggagagggaa gctacagaag agatagagta taccatactc tctgatgcag 70440
cgaaccgtct taaggtttta tgtgaagata ttgaggacac cctgcctcga gtcgaagaaa 70500
tagagatgat gctgcgtatg gcagagcgtc cactccatcc tataaagcaa gcatttacca 70560
aggcttttgt acaatataac aggtgcaaag agaggttagc aaaggtagag ccctattata 70620
aagagagccc tgcatatgta aatagcgaag agcgattgca gagtttggat caggcttcac 70680
agtgcataca aagagtccca aaaggtttca agtttagaaa cggaagcatg tacatataga 70740
gagtatctta gagaacaagt acaacagttt gaaactcaag gagtgagctt gataaaagaa 70800
gagcttctct ttttaagcag tactctcaaa agtaaattga gctatgatcc attaatagca 70860
aacattccct gtatgaagtt ttattaccag tattatgatg acattgataa agcgagagct 70920
caatcccgat ggctggagaa gtctgagagg tatagaaatg ctaagaggag attccaagag 70980
atcgtgaaga aaggcctatt caaagaagct aagcccttga aaaaagagga gtataggtta 71040
cttcaagagg agagaagcaa taaggagaag cgtttgattt acaataagat ggcagtagct 71100
cggcaacgag ttcaagaatt tgaatcgatg gagattccag aatagaaagt aagtcttatg 71160
gataaagcgc gttctttatt tactcgagaa gatcgttcct agcacaactc tagaaaccta 71220
tgagttgtgt tgctttaaaa atttttttga ataaaatgct atgtattagt agcttagttg 71280
gttaaactta tgtatttggt cacgatggca tcacagattc ataatgcaag tacacgtatc 71340
acccaccaca gctacccccc gatcactcgg taggggctac ttcttggcaa cctaagcttc 71400
gtattttaac cattactttt ttagttcttg gtgtgctttt actgatttca ggagctctct 71460
ttctaacgtt gggagttcca ggacttgctg cagggctctc ttttggatta ggcatcggtc 71520
tctccgcatt aggaggcgta ctggttgttt caggacttct attctttctc ataagacgag 71580
gggtttcgaa agttcgtcca gaagagattc ctgtgactcc ttcccatgaa gcccagaaga 71640
ttttatgtca gctacctcag gaactggatc agttagatac gtctattcag gaagtagtct 71700
catgtttagg gaaactgaaa gatcttaagt acgaagatca agggctttta acagaggtac 71760
aggagaaact tcgagttttt gactttgtca ggaaagacat ggtgacagag tttttagagc 71820
tacagcaggt tgtggctcaa gaaggacaat ttctagatta cctaatcaat caggtgcaaa 71880
gcatatcaca caaacttttt gtacctgatg taaatattgg agctcattta gcggagttgt 71940
gtgggtattt accttctggt gatgttcgag tggagcgttt aaagagatct gctcgtcagg 72000
ttgtagatcg ctttcatgag ggtgacttgt gacacgcgga aggtggcaat ggcttttgac 72060
gagaatgctg tggagtggca aaaaatgcct ttgataaggc ttttggggca ttagaagagt 72120
gtgtgtataa gagtctgaca gagagttata gagaggcatt ttatgaatat gagaaggcga 72180
agatccttag gaatgaagat gtagaatggc tgcaggataa gaataagagc gcacgtgctg 72240

agcagagatt tagggaagtc aaggatcgtt gggaggactt aaaggaaacg gtcttttggg 72300
taaaagaaaa cggttgtatt gacctagaag tgctcactgc agtgggtggg tggccggatc 72360
gtggtccaga gcatcttatt cctgaaaaaa gaaggaataa ggtaatgagc cataaattat 72420
gggaggcgac tatgcgaatg aagggagcag aaggaacgta tagtgtagca agagtcgcct 72480
ttgaaaagga tggatctaga aagaatcaga agaaattcca agaaaagaca aaagagtggt 72540
tgcgatgttt aaaggatttg catgatcagg agtgtcatcg tgcacgggag agattggcag 72600
aacttgaagc tttgtatcct gaggtttcag tctctgtagt agagacggag agagagacaa 72660
aatttaaatt agagactgct tatgggaatc tcgaagagcg ctatcagagc gttgtgcgag 72720
atcaggagga ctactggaaa gaagaagaaa acaaggaagc agagtttagg gaaaaaggaa 72780
caaaggttcg ctctccagag gaggtggtag agtatcttca gatcttagaa aatctgttgg 72840
aagactgttc taagcaatta actatagcgg aagtggttgt cttaggtgta gagctggaag 72900
ctacagcaga gttcgagtat accatactct ctgatgcagc gaatcgtctt aaggttttat 72960
gtgaagatat tgaggacatc ctgcctcgag tcgaagaaat agaaatcatg ctacgtatag 73020
cagagcttcc attccttcct ataaagcaag catttactaa ggccttttta caatataaca 73080
gctgtaaaga taagttagca aaggtggagc cctactgtca ggagagcgtg gactatagaa 73140
gaaacaaaga gcggtttcag agtttgaatc aggatttaca aaatgtatac caagagtgcc 73200
agaaggctac aggtttagaa tcggaagtga gtgcatatag agatcatctt agagagcaga 73260
tcacagagtt tgaaactcaa gggctggacg tgataaaaga agaacttctt tttgtgagta 73320
gtactctcaa aagtaaattg agctatgatc cattaatagc agacattccc tgtatgaagt 73380
tttatgagga gtattatgat ggcattgata aagcgagagt tcaatcccga tggctggaga 73440
agtctgagag gtatagaaag gcgaagaagg gattccaaga gatgctgaag gaaggcctat 73500
tcaaagaaga tcaggctttg aaaaaagcag agtatagatt acttcgagag aagagaatga 73560
ataaggagaa gcttttgatt tgcaataaga tagaagcagc tcagcagcga gtccaagaat 73620
ttggaccctc ggattcataa tgaaaaatga catatcggct cttctccctc tgatatttca 73680
ggagtctcaa gcaatgtttt tcgcctagtg ctctattttg tctaaatttt agagaggaag 73740
gtcgcagctg aaggctttca tttgtagaca ttttatagaa aaatcagaaa aataccttta 73800
gaaaaaataa gaacactttc atcttaaagt gactatagtg ggatttttta gatttgtttt 73860
agaaggcatt ttaacgatgt atcattttca aaagattcgc atgacactta caactcaggg 73920
atttgttctt aataaatctt taaggaagga ttatgaactg tggtttgtct atggatcatg 73980
tccagaatct aaggtaaaac tacagacttc ctctcataaa tggttgtgag acgaagtttt 74040
ctaagtgtgt ggttagatag atgaaagaac ttagacatga atcctataac cgtgcattac 74100
ataagctaag ccatcaatgg gttcgctact ttctctatac tttcgtatcg tgttccttca 74160
tagtcgccat atttactttt gcatggttaa aggtcctcta tgttcccgaa tgnaaggctg 74220
gtgagatctc acgtatttct ctgacagctc ctatggattt tnctttaagt tggagcgctc 74280
ataaatttta taaacgtact gcccacattt cagaagcctt tgggaaggtc tatcatctta 74340
cactctctcc cggtagtctc ctcagcaaag aggggaacgc cgatgaaaac actgactatt 74400
ggtttaaaaa agcagctgat tttttgttgt ctaccaactt tgtcgatagt tcaactcaaa 74460
aatgtcttaa ggacttgtgt atatatcctc ctttattggg gaaagaaaag aaaaccttag 74520
aaatcaatat caactcgaat aaagggaatg ttattgctca gtgcttctgc cacttaaaaa 74580
tttttcttat acaagaaaat tgtccccagc cctgttttga tgcaatcatg gatattttga 74640
agatcgccaa cttcgaagtg gccgtggata aggaaatgtc aggttgtgtg aaaggagagc 74700
ttctcggaaa acgttgcatt gagaaaatta ccaagggcac acctatatta gaaaagtatc 74760
agagaatcga tgatcgggat gctaaaattc taaagcagct tcgagcgcaa ctcctttcag 74820
tgcatacctt attttcctgt agatccttat ggggggctat ttttgtagtt ttactcatac 74880
ttctatgggg ctacggtgct ttgaaagccc tgtgtcctga gatgttgaaa tctccccagc 74940
gctttatgct ctatattgcg attctaactc tttcgttgct gtggtgcaga gggacagaaa 75000
tcttttgcgc ctattgggtt tcctatctat cttacccacc gatttaccca tttacagctg 75060
tactcctagg atattttcta ggtcttccca tagcaggatt ttcctgtacg tttcttgccc 75120
ttctctacac cttgggatcc gatctttgga ataatagttg gtttctatct ataaacctac 75180
tttgttcttg gagaatctta gtgagcttac atcgcgtcag tcgcctttct tcggtgtttt 75240
gggcgtgtat gaaacttgga ggcgtagcta tgggaagcct gctcatgttt cggatattta 75300
caaatacaat atcaagagaa gccctatatg ctgatgggat cgaaagcttc gtttatagtc 75360
tgatcaccgc aatcagcgtt gttgctttga tccctgtctt cgaggcttct ttcggagctt 75420
ctacaaactt ttcgctcctc acctatttat ctcccgaaaa cgcattgctg aagcgtcttt 75480
tcaaagaagc tccaggtacc taccagcatt ccgtattagt tggaagctta gcagaagctg 75540
cagctcaagc tataggtgca gatagcctct attgcttggt tgcagctcat taccatgata 75600
tcgggaaact gattaatcca ggatttttca gtgaaaatca aaaaatctta caacaatctg 75660
gtcattcgct atccccatta gagtgcgcta agatgattat gcgccatatt cctgaagggg 75720
tgaatcttgc taggcaggna gggcttcctg agtctgatat ccaggtgata gaagagcatc 75780
acggaacctc tgtgatccgc tcagcatact acagccatat ggtagagaac ccttctacag 75840
ggagctttga tgaggaatta ttccgatatt ctggaaataa accctcctct aaagaaacta 75900
caatcattat gatcgcggat tcttttgaag cagcctcgcg atctctaaaa aatgccagtc 75960
ttccagatct ccaaagactc atcgatcaga ttatccaagg gaagttacaa gacggtcagt 76020
tttcttgttc tccaattacg ttagatgaac tcgctttgat tagcaagagc atggtgcaaa 76080

ccctctacgg agctctacat tctcggatga aataccctga aatatcgtat caaatttcta 76140
tggattcctg ccccaaaccc tctataggag gaacttagtt ctctagatta cgtggcgttt 76200
gatttccagc aaatgtgctt tgaaatataa ttttttatgc tctcatatgt agaaagagaa 76260
aaaagcatta gaatgccaag aagcagtagc gacaaaacga ccaaaatgag aatagggact 76320
aaaattccca gtcctcctag gatggaaatg caaattaggc cgtatccgtt tacgaaggcc 76380
tcgacacact ctttctgttc gcaagaaagc gaaggcgagt acccgatctg aactgtagtc 76440
cgatctaggg atttttttat ttgataagca cggaccaaac ccgcaattgg tactagactc 76500
acaaatatca aaaacacgtt cgaaaagcag tctaaactaa agggtttttc atccacccaa 76560
atagcacggc tttttttctg gaatagtttg tttgagtcct tgtgagtgtg acctgggata 76620
tatatcgtgt ttcctgtcaa tattttgtca tagtaaggtt gatgtagcat agtaagccaa 76680
aaaatttctt gatagataac tagcaggtaa acgatattcc ttcttttctc tttcagtcaa 76740
tggtattttt tataagtcac taagaataaa aactcataaa gatggtctag cttcgctttg 76800
cttattttta acttctacag tgtctaaagg gaaagaaatg tagtaaaaga ctacaaatga 76860
gacatgcaat tacgccaagg tgttatttag caacaataaa tttgtaacag tatcagagga 76920
cactgattga cgcaaagcaa agcaacaaag gcgaaatggc aacatgatta actgaaaaag 76980
cacagaaata agtaggatag ctaggagcag aagaaatata gggagaagga ttccacatcc 77040
accaagaatt ccagcggtct ttatagagct tgtcagtcta gatgacatcc tacatagcgc 77100
atttttgcag ttagtatgag gggagatagc ctaagttagc cttgacattc tcgtcattcc 77160
agtttgtttg atgctttgca tatctcattg catagaaata ttccgagaat gggaaaccat 77220
gagacctata tacatccagg agtgctcccg agtagtcatg ctcaggatgt tagcagatct 77280
acagtttacc ccagtcgaag ttttatcatg agacgtatgc tcatgggctg gaatttcaat 77340
cgtgttccct cgaagagctc cgagcagtta atggatggtc atcgcatacc tcttatattt 77400
tttgggaagc atcatcctac tatatctatt ttaaatgtca atagattttc ttggctctcc 77460
attttttaca atggagaaag gggtttttga gaagaaaacc aaactcacca tacagattaa 77520
agtacaacaa aatctaagga agaccagttt gtattttagc agcatcatag aaatacttga 77580
tggtttatga gagctatata ggaggaccct ctatccacat agtgtctcca gattcaggta 77640
gtctctcctg cgattctatg aaaagcgagg ggagagttat gaggagtggc agtgtatagg 77700
ggtaaataac tgtttccatt tacctgcgtg atccttggct gtaattgcta aggatccttg 77760
tagggcatgg tatggaggag gaagttctat actataagca tgatgcaggt gaagtcttaa 77820
actcgcagct ttcgcaagaa caatagcatc ataatgacca cggtggagct gatccagcct 77880
ctcttcaata gtgcctcgaa tatccaagat ctgtccttga ggaaacagtt gttttagtac 77940
tgcacttcga cgtagagaag aactccctag ccggggactc aagggtaggg gctcatgaac 78000
atagtggtca gcatacacta acaggtctgc aggatgtaga catcgtgtta tggcaactac 78060
aggaagagag ggagtctcag gaagatcctt agcagagtgt atcgccagat cgcagactcc 78120
tttatggact aaggcatcga cgccatcagt gaaaaaataa gagttttcta caagatgtaa 78180
ggggattttc ttctcacgat ccccagtagt ctctgtagtg cttaactgga accaaagttt 78240
gggataccac gagcgcanaa aagaaataca ctcatgtact tgagctttcg ctaaaatttg 78300
aatttctaga aagcaataac gaagggggcg cttgccctga caggaaatca cttaaacagg 78360
ggtcagagta acagacggat agcatctttt tattgttttc actccttgta aacgaaagtt 78420
ttctcgaatt tccttgggaa gagatgaaat ttgaccctca ggaagaattg caccttcaaa 78480
tcccatgagt tttccctctt taatgcgtct ctctaaatga gccacatgac ggatttctcc 78540
tccaagacct acctctccaa ttacaatgga attgttaggt aacaggcggt tgtatagcga 78600
ggaagcaacg gcaagtagag cccccaagtc cgcagcaggc tctataatct ttaaaccccc 78660
cgtaatggat aggaagacat ccatggtaaa tagtttgact tgagcccttt tttctaatac 78720
agctaaaagt aaagaaaagc gattcggatc gaatcccgca gtcttcctta ctggattagc 78780
aaagggagac gaagagacca aagcctgcag ctcgataaga agagccccag agccttctat 78840
aataggaatg atcatagacc ctgtcgttgg ccccgtcttt tcctgaagga aaagtcctga 78900
agggttgcta acctctttga gaccatctgc atgcatcgag agaatcaata gttcatttgt 78960
agggccaaag cgatttttca cagagcgaat catacggtaa ttcgcatggg aattcccctc 79020
aaagtaaagt acagtatcta caagatgttc caatacccta ggacctgcga tctctccaga 79080
ttttgtcacg tggccgataa taaatgtcgt gatctgcgca cttttagcaa tctgcattag 79140
ttcataagta acttctcgga cctgagctac cgatcctggt gcagagttta gcgtggggtt 79200
aaatataatc tgaatggaat caataattaa aatatcaggt tccaaagtcg ctatttgctg 79260
cttgatattg tccaagtttg tttcaggaaa taaataaatc aaaggtgatg agatattgag 79320
cgcttcgctc tcaaagacgt ctgcgttaca gattcttcac cacaaacata aagaacttta 79380
tacttttgcg atgccaatct ctccgcagtt tgaagaagga gtgtcgactt tccaatgccg 79440
ggatccccac caagaagagt gaggcttcca cgaacaaccc ctcctccaag gatgcgatcc 79500
caccccgcat gatcaataaa tatccgagat tcattctcta attcgataga gcttaatgca 79560
atcgcactcg tagaagatcg cgacgaagtc ccagatcgag cctgggggac atattcttca 79620
accaaagagt tccagttgtg gcagcctgga cattgcccta accatttagg agcagtagct 79680
ccacattgat tacacgtcca ttgtgttttg gtttttgttg ccatacgtat ctagtgcctg 79740
ttgtgctgca attttttctg cttccttttt ggatgacgca tttccctctc cccaaacctc 79800
ttgattcaca agaacctgga tctggtaact gacatttcct tgagcatccg taactgccgt 79860
ggattgatat accggaagaa cgcgaaactg cttttgtgtg aactgctgaa gaaggttctt 79920

aggattgcca gacattaaag gaagaatttc ttctctagga ggaaggaggg gaacgtaagt 79980
ttcctagctg gagaaagacc cccatccaaa tacacagcac ctaaaataga ttcaaataga 80040
ttggcatagg cagaaagacg tcctcgctca ctctggattt tttccccttt tcctataaga 80100
agataatccc caatccctag catggttgta taacgacagc acgcttttgc attcactaaa 80160
gaagcccgtg ccgtggatag agttccctca tccatcgaag gaaagagaag aaaaagatgc 80220
tcagtaacaa tgagaccaag gacagcatct cctaaaaatt ctaaacgctc actatcttca 80280
atttgcaccg ccgactcgtt tttatatgag gggtgagtca gcgctatttc taagagctta 80340
ggttgtgtaa atgtaaaatt taacttagct tcaatagccg tgatgtctat agggggatgc 80400
atagatagga gggcgccgtc cttaaactta gagttgggaa tttttatagg cggaaaaaag 80460
cttaaagtct atagtgcttt tggatttgtt atcttattcg gatacgtatg aggtctgctc 80520
tacacctcca acacttgcgc catttccata accacggctc tattttattt gaaaatcttc 80580
tgactataaa agattgtttt ttattagaaa caaaattaca aaatttattt gccaaagcat 80640
caaaaactat agacactgtg cggtggagag agaatatatt tcgctcaatg ccagagattt 80700
atacagtcgt tcgtaaacgg cgtttggatt tctttgcagc ggaattggtg caccgcccca 80760
agctttccct cgttcgagat ctctgggtct tcccaggaga agagatcctt gaaggagaag 80820
aagattgcat gcttttcctt ttactttcag gagatcgtgc aggaagcggt atattcttta 80880
caggacccta tccttcagat ctttatgaat tggagaaggg aactacgggg ttgcttttag 80940
ctttctcttc tgtagggatt ccagtaattt aatctttctt ctctcgctct agaaacagaa 81000
ataagagaca gggacttaca gttcttattg cctccatgtt ttgattcagc atctttctga 81060
aggaaaaatc accatattct ttacaaagag cttctcatgt tttagttccc cgcttgcttt 81120
caatacttaa ttacaaaaac cacaggccga agtataacgg ctttgagtga tcaagtgtat 81180
tctataggat ttattttgaa gggtggattc ttaattaaga aaacctttct caaacaatgg 81240
attataccag accaagcttg aagaatccct ggactttatg caaaaagggt gttaagagtt 81300
cctgaaacta tccttacata ggatttcttt aatgaaagaa gtagaacaac gtatccggtc 81360
attatacgat gcagtaacag ctgaaaatat ttgtagatgg ttgtccaatg attgtaccca 81420
acaagatgca aagactatcc taggatggtt agatacagat cctgcacagc ttgaagatct 81480
attcggagcg actcttacct ttggtaccgg aggactccgt agtcttatgg gtatcggaac 81540
aaataggatc aacctgttta ctatacgtcg aacgacgcaa gggctggttc aggtgctccg 81600
cgctcatctt ccccatcccg gagatcctat gcgtgtagtt gtcggttgtg atacccgcca 81660
taactctata gaatttgctc aagaaactgc aaaagtcctc gcaggtaatg gctgcgaagt 81720
tttcttgttt cagtatcccg aacctttggc tttagtctcc tttacggtga gatacgaaag 81780
ggccatcggc ggagtgatga tcaccgcctc tcataatcct cccaattaca atgggtataa 81840
agtttatatg gcttcgggag gccaagttct ccctccctta gatcaagaga ttgttgccgc 81900
ctgtagtgca gtgaacgaaa ttttatcagt gccctcgata gatcatccca atattcacct 81960
cattggaaaa gaatacgaag ccctttacag agacactttg aagcaactgc aactctatcc 82020
cgaagcaaac cggatttcag gaaggtcttt atctatttcc tattcgccat tgcatggaac 82080
aggaatttct ctcgttcctc atgttctcaa agactgggga tttttatccg tacatcttgt 82140
ggaaaaacag gccataggtg acggcgattt cccaaccgtg cagctgccaa atcctgagga 82200
tccagaggct ctgactctgg gcactgagca aatgctcgct aatgacgatg atctttttat 82260
agctaccgac ccagatgccg atcgcgtggg cgtggtttgt ctagaagacg gccaacccta 82320
ccgatttaac ggaaatcaaa tggcgagcct tttagcagac cacatcttag gagcttggag 82380
caaaacaaga cacttaggag aacatgataa attggtcaag agcttggtga ctacagaaat 82440
gctctctgct atcgcaaagc actatcatgt ggatcttatt aatgtcggaa caggatttaa 82500
atacatcgga gagaaaattg aatcctggcg caattccaca aacaaattcg tatttggagc 82560
cgaggaatct tacggttgtc tctacggcac tcacgtagaa gataaagacg ctattattgc 82620
gtcagcattg attgcagaag ccgcactaca acaaaaatta caaggaaaaa ctctatgcga 82680
cgcactcctt tctctttacg aaacatacgg atactttgct aacaaaacgg agtctgtggt 82740
tttttccgca aaaactgacg aacaagaaat aagaaaaaaa ctttcacacc ttgaggaaat 82800
cagttctgcg aattttttct cagggaaata ccaagtagag aaatttgaaa actataagca 82860
agggataggt ttcaatcttc tatcgaagga ttcctacgcc ctcaccctgc ctaaaacatc 82920
tatgctctgt tattatttta gtgggggagg tcgggtaatc atacgaccct caggaacaga 82980
acctaaaatc aagttctact tcgaaatgtc aactcattat ccagagcgcg ttaccgataa 83040
agaaatacaa aaacacgtga agcagagagt tttcaacatt tagacgattt tatttttgat 83100
tttaaagaga aattttccaa tttgtgagtg gaaaaatcat cttggagaat atcctaaagc 83160
tatttacact tgggctaaat ctccttcaga ataaggcctt ctttcaaggc cattgttgta 83220
tccgaaacaa ggttgagtag agtacttgct ttcctagcaa aactttctga acttaaatca 83280
aggaggttaa atactaaaaa ggtatgttgt tatgagtttt gttccttatt ctttaccaga 83340
gttaccctat gattatgacg ctttggagcc tgtaatttct tctgaaatta tgattttaca 83400
ccaccaaaag catcatcaga tctacattaa taatcttaac gcggctttga agagattaga 83460
tgctgcagaa acacaacaaa accttaatga actcattgct ttagaacccg ctctccgctt 83520
taacggggga ggacacatca accactctct attctgggaa actcttgctc ctatcgatca 83580
agggggagga cagcctccaa atcatgagct cctttctctt attgaaagat tttggggtac 83640
gatggacaac tttttaaaaa aattaatcga agttgctgca ggagttcaag gctccggttg 83700
ggcctggcta ggattttgtc ccgcaaaaca agaacttgtc ttacaagcaa cagcaaatca 83760

ggatcctcta gagcctctca cagggaaact ccctctgctt ggcgtggatg tttgggagca 83820
cgcctattac ctgcaatata aaaatgttcg tatggattat ttaaaagcct ttcctcaaat 83880
aattaattgg ggacatatag aaaatagatt ttctgaaata atatcatcta aataatttga 83940
atttggtgat tttaattgca gtgttaataa cattaattta aaattgcttc ctaacagaac 84000
ctagattagg tggcttgtgc gtctattttc ttacgacaaa cccaagatta aagtgcaaaa 84060
aatcaaggca gatggtttta gtggttggct caagtgtaat cattgtcacg agatgattca 84120
cgcaaatgag ctaggacaaa attataattg ttgtcctaag tgctcctatc attaccgtat 84180
tactgcgatc gaaagagtca agctgcttgc agacaaagat tcttggcgtc ctctttatac 84240
ggatctgaaa tcccaagatc ccttggaatt tatagatacc gatacctacg caaatcgcct 84300
agaaaaagct cgaaagaata ctacagaaag cgaaggcgtc attgtaggta tatgtactat 84360
aggcctccac cccgtagccc tcgccgttat ggatttcaat tttatggcag gatctatggg 84420
tgctgttgta gggganaaac tgaccagact tatagaggaa gccattgaaa ccaggctccc 84480
tgtaattatt gtcagcgctt ctggaggcgc acgtatgcag gaatctgtat tttctttaat 84540
gcagatggtg aagacctcag cagctcttgc taagcttcat gaagcaggtc taccctatat 84600
ttcagtcctc accaacccaa cttcaggtgg agtgacagcc tctttcgctg ccctcggtga 84660
tattataata gcagaaccta aagcactgat ttgtttcgca ggacctcgag tcgtcgctca 84720
ggtgatagga gaagatctcc ccgaaggctt caaaaatctg aattcctact agaacatggc 84780
atgattgata aaatcgttga gcgtaaagaa ttgaaaacca cccttcagac tttacttgat 84840
tactttttag cccaagaata cactggcggg aaaagtaaag ctcctagaga tctttcgaaa 84900
aggcttaaag agattttttt gttgacagat gacagtgaat aaaacatcat accgcatctt 84960
gcaatgataa cattatctgt aacgctatcc ttatgactgt attttgtgaa ttggattcag 85020
gaggagaact tcctgaatat actacgccag gagccgctgg tgcggatctt agggcaaaca 85080
tcgaagaacc catcgctctg ctgcctggac aacgtgcttt gatccctacc ggaatcaaag 85140
cagaaattcc cgaagtacga gctacaggtc cgtcctcgga gcggtttggc tttaaagcac 85200
ggcattactg ttttaaattc cccagggact atcgattcag attatagggg agagattcgt 85260
gtaatcttaa tcaacttcgg tgatagtaca ttcattattg aacctaagat gcggatagct 85320
caagttgttt tatctcctgt agtacaggca acgtttgttg ttaagcaaga nagtttagcg 85380
gaaactgccc gaggaagtgg aggttttggt catactggag caagctaaga tgccatccta 85440
ttgtcaaaat caacaagatt tttctttatt ctctcttttg tctcctagac ttgtaatgtt 85500
tttaggcaaa cactcccgag atgaaatcct ccaagatctt acagatcttg tggatgctgc 85560
aggcctactt gaagacaaac aagccttttt tgatgctctt gtccgtcgtg aaaacatcat 85620
gtccacagga atcggaatgg gcgtggctat tcctcacgga aaactcgaaa gctgctctaa 85680
tttttttatt gctataggca tccatacgca aggcatttta tgggacgcta ttgacggagc 85740
cctcgtacgc ctcgtcttct tgatcggagg tccagaaaat gctcaagccg aatatctcaa 85800
gttattatct actttgactt tatctttgag agaagagtct cgtcgtcaac agttgttaca 85860
ggtgaatacg attgaagaag tcatgaatgt atttgtgggg atgtaaaaat ggatttaaag 85920
ttagatgaag tcgcctcttt gttagatgtt tccgaacata cagttctgca atggcttaaa 85980
gaaggagcca ttccaagcta tagtatgaat aatgaatacc gctttagtcg tgaagaaatc 86040
gaagactggc tattgcataa ccaagcactc atgatccaag aacgcggcga agataaagaa 86100
gcacttaaag atctttcttt gaaatatagt ctctacaaag caattcatcg tggcggcgtg 86160
ctttgcgatg ttgtggttca tagtaaagaa gaagctctcc aatacgcctc taaatacatc 86220
gcccaaaagt ttcaattaga cgaaagcgta ctttttgaaa tgctctccca cagagaaaat 86280
cttatgtcca caggtatagg agaaggaatt gccctgcccc atgccaaaga ctttttaatt 86340
aatgcctact atgacattgt ggttcctatg tttcttgcag agcccataga atacggggct 86400
ctagatggaa aacctgtagg cattcttttc ttcctttttg cttgccagga taaaagtcac 86460
ttaaacttag taaataaaat agtccatctc gggatgtctt taaatgcccg aagctttttt 86520
aaaaattatc ctaacaaaga tcaactttta gcgtacgtta aggaatggga gtcccaaact 86580
cattaatagc tagagtttaa aaagattttt aagtccaagt tgtgaaaaaa atccttttgt 86640
tgctatggtg atcctcatag gcttccagga gattgtagtc gcatgatgag ctctaagcgt 86700
acctcgaaaa tagcggtgct ttcaatttta ttaacattta ctcactctat agggttcgca 86760
aatgcgaatt cgtccgtagg tcttggcacg gtctacatta catccgaggt tgtaaagaag 86820
cctcagaaag gatcagaaag gaaacaagcc aaaaaagaac ctcgtgctcg taaaggatac 86880
ttagtccctt cttcaaggac tctttcagct cgagcccaaa agatgaaaaa ctcctctcgt 86940
aaagagtctt caggtggttg taacgaaatt tctgcaaatt ctacacccag atctgtaaaa 87000
ttacgaagaa acaaacgtgc agaacaaaag gcagctaaac aaggattttc agctttttct 87060
aacctaactt tgaaaagcct acttcctaaa cttccttcaa aacaaaaaac ttcaattcac 87120
gagagagaaa aagcaacctc aagatttgtt aatgagtctc agcttagttc cgcacgaaaa 87180
cgctactgca caccatcttc agccgctcct tccctatttt tagaaacaga aatcgttcga 87240
gctcctgtag aaagaactaa agaacttcaa gataatgaaa ttcatattcc tgtagtgcaa 87300
gtccaaacga accccaaaga acaaaataca aagacaacta aacagttggc atcccaagcc 87360
tcgattcaac aatctgaagg aaccgagcaa tcattgcgag agctcgccca aggtgctagc 87420
ctacctgtct tagtgcgctc taatcctgaa gtgtctgtac aaagacaaaa agaagagtta 87480
ttaaaagaac tcgtagctga acgtagacaa tgtaaaagaa agtctgtaag acaagctctt 87540
gaagctcgtt ctttaactaa gaaagttgct agaggcggtt ctgtgacctc gactttacga 87600

tacgatccag aaaaagcggc ggaaatcaaa agtagacgca attgcaaagt aagtcctgaa 87660
gcacgtgaac aaaaatattc atcttgcaaa agagatgctc gcgctaatgg gaaacaagac 87720
aagacaactc ctagtgaaga tgcttctcaa gaagaacaac aaactggggc aggactcgta 87780
cgcaagactc ctaaatctca ggttgcaagt aatgctcaga acttctaccg aaattctaaa 87840
aatacaaaca tagatagcta tcttacagct aaccaataca gctgtagttc tgaagaaaca 87900
gattggccat gttcttcctg cgtctctaaa cgcagaactc acaacagtat atctgtatgt 87960
accatggtag ttactgtcat tgcgatgatc gtaggggctt tgattatagc taatgctaca 88020
gaatctcaaa caacatcaga tccaactcct ccaactccta ctccatagtt gtatagccct 88080
tgctggacgt gtagctctac ccaaaatctt agatagcctt cttatctatg attttagtgg 88140
gtagagcttt ccctcccgac tcgtcttctt ttcaattttc tctttgtaat tacactttat 88200
ctctctttct atctttttcg ggagtacctt cttattttag atagagaaag cttagttttt 88260
cttttgttta agaaataatt ataagctcgt taatataatc aatttgcctt taagtaaaat 88320
gataaaacta tctaatctat agtgatttgt gttgtatagc attattatat tgcatcgtgt 88380
cagatttgcg tgttcattct aggggaacac cgacccagac tcatagtgag atctgtgatg 88440
cccatcctac caacagattt ttgaaaaaac accctacact tgacctatgt atgcgaattg 88500
taagcacaat tgtctctgtc tttatgattt tagcagacat cgttctcctc ctgggctccc 88560
tcttactttt acccctcctt atagttttac tttgggaatc ttcttaggaa gatgcctttc 88620
tacgtctaat attgttttat tataatttta aaaatgatta taatttctta ttaacttcat 88680
gaatatttac atttaataaa agtataatgt ataattagtt atgactaaaa ttcaatgtag 88740
tgctcagtat tatagatctc gaccggccga gagggcccaa actcctccgc aacctttcct 88800
tgctagggat cgcgcggatt tttgggagag acatcctaga ttcagtgcat gttgtcgtgt 88860
cttattactc gttgcttggg tggttctcgc tctactgttt ctctttgtta tgcttcttcc 88920
tctagccgct gggtcgtatt tacttgcttt ttaaggtatt cttcacctaa aaacttgtgt 88980
tttgggtgcc tgtagctatt tcaaaggcat gtttttatcg atcggagctt ctctttgttt 89040
gggcgagtcc gttcgtatct ttgtaattct tctacgattt agttttatcc ttatcttaga 89100
ccaacctctt cataggtgga tcctgtagag gaactattcg actcaggagt cgtagaccta 89160
aggtattcgt gtttaaagat aaaagtttta ttttctaaga gttttttaat tattaagatt 89220
tttatttaaa aatatatctt ttgattagat ctctaatacg attattataa atataatatg 89280
tttttcaaaa aaaattatat gacagatttt cctactcact tcaaaggacc caaacttaac 89340
cccattaaag taaatccaaa cttttttgag aggaatccta aagtcgcaag ggtactgcaa 89400
attacagccg tagtcttagg aatcattgcc ctcttatccg gtatagtact cattataggc 89460
acccctctcg gagctcctat aagtatgatc ctcggcggat gtcttttagc ttctggaggc 89520
gccttatttg ttggtggtac gattgctacg atattgcaag ctagaaatag ttataagaag 89580
gccgtgaacc aaaagaaact ctcagagcct ttgatggaac gccccgaatt gaaagcctta 89640
gattattccc tagatctgaa agaggtatgg gacctacatc attcttgttg tcaacatctt 89700
aaaaaaatag acctgaatct ttccgaaacc caaagggaag ttctaaatca aatcaaaatt 89760
gatgatgagg gaccctccct aggggaatgc gccgctatga tttcagaaaa ctacgacgca 89820
tgcttaaaga tgctcgcgta tcgtgaggag ctcctgaaag aacaaaccca ataccaagag 89880
acacgattca atcagaacct cactcataga aataaagttt tgctctccat cctctcaagg 89940
atcacggaca atatttctaa agcgggcggg gtcttttctt tgaaattttc cacgctaagc 90000
tcgcggatgt cacgaattca taccaccacc actgtgattc tggctttaag tgccgttgtt 90060
tctgtcatgg tcgtagcagc tctaattcca ggtggcattt tagcactacc tatacttttg 90120
gctgttgcta tttctgcagg agtgattgtc accggacttt cctatctagt tcgtcagatt 90180
ttaagtaaca ccaagcgtaa tcgtcaggat ttttataaag attttgtaaa aaatgtagat 90240
atagagcttc ttaaccaaac ggtaacttta cagcgattcc tctttgaaat gctcaaaggt 90300
gttctgaaag aagaagaaga agtctcctta gaaggtcaag attggtatac acaatacata 90360
accaatgcac ccatagaaaa aagattgatc gaagagatca gagttaccta caaagagatc 90420
gatgctcaga ccaaaaaaat gaagacagac ttggagttct tagaaaatga ggtgcgttcc 90480
gggagactgt ctgtagcgtc cccgtcggaa gatccaagtg aaactcctat ttttactcaa 90540
ggtaaggagt ttgcaaagtt acgtcgccaa acctctcaga atatatccac gatttatggt 90600
ccggacaatg aaaatattga tcccgaattt tccttaccct ggatgcctaa aaaagaagaa 90660
gaaatagacc atagcttaga acctgttaca aagttggaac ccggttcaag agaagagttg 90720
ttgttggtag agggggtcaa cccaacctta agagaactca atatgagaat tgcacttcta 90780
caacaacaac tatcaagtgt ccgaaaatgg agacaccctc gaggggaaca ttacgggaat 90840
gttatctatt cagatacaga actcgatcgt attcagatgc tagaaggcgc attttataat 90900
cacctcaggg aagctcaaga ggaaatcacc cagtctctcg gagaccttgt tgacattcaa 90960
aaccgtattt tagggatcat agttgaaggg gactcagatt caagaacaga agaagagcct 91020
caggaatagg attctcatat aaataacaac aaactaagag ctgttttttt ctcttacagc 91080
tctttctaga caggatctct aaggtcctaa attcatgttt tccattatca tccttgccta 91140
cgacctaggc tgcatccgga ttctataagt acgtactctc agttgctttt tctacaagtt 91200
gggctagccc acaggctgca aacagaaatt ttatttcttg aaatctattt tcttatttac 91260
aaatttattt ccctaaaatg aataaactaa ggagtttata tggcaaatcc cacacaatcg 91320
cgaccaccga gtccggagat aagtatagaa gaactagagc ttcaagaact tgcaggatcc 91380
tcgaatactg agactatttc taatacacct cccccgtcat gcgctgctac tgccgaagaa 91440

gtatctcttt ttattgaggg aggccgtaga aactcagaag atgaggaggg acctctagga 91500
tcttgtgagg tgtacgatgt tgtctgtata acaaaccaag gagatcctga ggttagagat 91560
cacgaagtca gagttatgta cattaacggc agcggtcgaa cacaacatga gggtattctt 91620
gatgctatga acatctgtga tctcagagga gaacccgtca ggttcataca caatagtggg 91680
tatggtttag ggagctgctt cttagggatt cgaaatcgta ttcctcctag agataatgtt 91740
attagccaag caatacaagc acgatggaat gagtttttta ttttcgcaga aaatgcaaat 91800
cgagattaca tcgttctttt ctctggtaat ggaggtctct atcttcaagt cgctttagat 91860
aactccatat actcacatca tattctttgt gttggcattg gaagcagtta ttatatccaa 91920
ggaaattatc gtgttcacaa ctaccgtgtg acaggggatt ggacgaccct cctggatcgt 91980
cggggggcaa cagcagtaaa tactacaacg ttgccttatg cagattctgc tgaaggactc 92040
tttttaccct cagtacgctg tccctcatac caatgggcat tgcgttgtgg agaacagtgc 92100
ctgatcatgg ataacaacca acaagttggg tttcgccccc aagattcctc ttcagaaatc 92160
gccttagtag taaatttaaa tcaggaccac agcacctgga ctcgtctgat tgaatggata 92220
gatcgggggg attctcaggc tgttctagaa ttgaatcctc aaccgagtca ttgtcgtgat 92280
attgcattga ctgcactata cgctacaaca aggatttctt ctttacttca agagtgccta 92340
atgatttctg tgacttatgc tccagaggtt ttcgtcacct atgctatcgt tacaggatac 92400
tctataatga ccttgcgcta ttttattcta ttattaacaa atcgtccagg ctgccggcgg 92460
cattttcgtg ttttaagatt agcggcttta gggttgcagt ccttaggatt tttgactgta 92520
ttgcttgatc atatcaatgt aacacggaga gtcaatcgcc gccccccctt aatatcagta 92580
atcttctgta ctgctagttt tgccacagga agtttcattt atgtagactt aacacgcatg 92640
tttttcacga gcttacgttc gcgcttgcaa ttgtttgttc aaagaagatt aacaggaaga 92700
ggtctaccac tgagaagggt ttttgtaaat cacctagact ctttgagatt ttctcaaaat 92760
gctttgataa cctttcatgg gggacttttt atgcctctca taataggttt ttttaatcag 92820
ctggtcattc aggttcctcg agttgtcatc agaccaaata ccactgccgt ttatgatctc 92880
aaccagacct cacaggaagc gtgggactct ggagacgtat tagctatagg acagaccata 92940
aacttcttgc tttgcatgat tctattggtc atcaatacct ttttcttcgt gagatccgta 93000
cgaaggaatt tgcatcgtag acctcatcga tagcaactgt gcagaaccct actctttaga 93060
tttcaaaaat aactgatacc gaaatgcccc tgtactatag gtgccattgt tccttagaaa 93120
tctaaagaga tcggcctctt tcctttatat ctgaaatcat gactaggaat aaggagttta 93180
ctgtctttaa acgcatctcg ttgacattaa aaccaaataa aaacatgttt ttatttggtc 93240
tattctgtta aaatagatag gtttttttta actctgatcc taaagtgtca ttgaaaaggt 93300
tcagggtatt cttatagagg tccccctatg gcagtagaag gaagagtaaa tagttctcaa 93360
gccttaaatc aagattgtca agaagtctta gcaaataaac aatcgaaagg cctcctaagg 93420
tgcagaattc tatctatagt agtagctgtt atcaccttta tcgccggggt tgtgttgata 93480
gctttaacat tagcctctat tttaacttct gttccctact tagcgttagg agtgttttta 93540
ctgattgtca ctctgggatg tataatattt gctctttgct ctgagaaaat aaaaaaggtt 93600
cccccgactc ctatttcaca taaagaggag atcattgcct ggttcgaaga aagaaaaaat 93660
attgatatgg aaaaggaaaa agaagatccg gagcattttg gaagaaccgc tacggatatc 93720
ccaatgagat ctgcattaga tcagtttaac cactcttgtc accatattca cgagagcccc 93780
gcgttaacag aaacttatag aagccatcaa gatgttctcc tctttaagga ctggtgtcct 93840
gttacgttgc ctgatgtaac ctcagaagaa gaagtcttaa tacgcagtgt ggttggtagc 93900
tatttattaa tggaggcgtg cgttccaaaa gtatccatgc ttatcgacga actccataat 93960
aagcttatnt ctccttccga aagagagtgc ctctttatag ataaaaaaac attgcagcga 94020
aaagctagtt ttcttttcac tcagaaagat ctcgcaacat tctttcttga cctatacgcg 94080
ggtgaatgat ggtcatttag caccgtttcg agcaggagca aaatggatct taatacatta 94140
cgttaggtta agacgtcaac acaatcagaa cgactttttt actccaggac attcttgtta 94200
ctatgctcgt ctagccttta accaaaccca acgactctat catcaattat tcaatgtaga 94260
aaagcttcgt agtatctatg cgaacatgga taaagaccct ctatgtcacc catgggctnt 94320
cattcctatc tatgatttat tgaaaacaga ggaccatgga gatggttttc tagaacaaca 94380
agaagatcgg gaatatccaa gtagagctgc tcaagatcaa ttttggggct aatgtttaaa 94440
ggatcagttt tttaaaacac ggattctaaa ttgtaattca ggattactat ttttcttcag 94500
aaagcttaga cctactgctt gtgaggcagg ggagtagtct tacaccccaa aggaaatata 94560
ccgaataaaa atatctagaa gaggctctag atacatcttt ggaaatagac tctccgttct 94620
tagatactct agattatcta aatttcatga ggagagatca aaagaaaatg ttccaagatg 94680
ttatggaaca ttttctctct agtttatttt tttaatagca taagagtttg tttaatatct 94740
tgttcacgtt gaaatgctag ctcgcggctt actttttctt tattccaatc taaaatggac 94800
tttttaaact ctaaatcctt ctctcggagc tctaattcgc gatttttcaa ctccaattcg 94860
cggttccgaa tgtcaaattc acgattaaaa caatcgataa tttgggcaac ttgtccagat 94920
tgacacatct tagaaatgcc ctctaatctt tgaaccatgc tatcgaaaaa atctaagaca 94980
tattcattag aagactcttt atcagtcttt tcagaaagac cttctgagtc agaagtacca 95040
caactttcct caatctcttc cgaacttttt tgcccgtcgg aaattgcttg tagttcatta 95100
cgtatctctt tgccctcttc gattggggtt tcaatcagct cattgagttg tgttattaaa 95160
acttcaggat cctcctcaat tctggactga gaagagacat cctcattgca aatgcctttt 95220
aggggagcgg agaaactgca ggagaaaatt aaactataaa taaaaaattt cttaatcata 95280

aaactttaaa gtaattaatt taataaatcg attttaatgg tattatagaa atataaaaat 95340
caaaatacaa aagaaatttg tgaataaaaa aaagaatagc aaattatttt ttgttcatga 95400
tttgtgaatt ttaaaaaata ttatttatta aatacaaacc tgttaacaat aggttgtgaa 95460
aggagattac gatttttttg agatatcctt attaactaca aaatggtctt acctatatta 95520
ggttaattaa aataatataa attatttatg tgttattgat ttataattaa attttcttta 95580
tgtttatcta ctcatattta atcttggtat aatgacaann cccccaccat cccgatcctc 95640
ctctcctcct ccctatgatt ggatagaact tcaagatctg gggaatacga ataacaatag 95700
cagtcgagct accccccccc gaagtaggcg gtgagctgcc cccgtatttt tcagctagca 95760
actttgttgt aatagagcgg ggcgctccta gtctgccttc tccacagcaa cttttatctc 95820
ttccagaata ttctaggcag ccgccaccag gatattttga tgaaacagca agcataacta 95880
gcagaacgag tgaagagatg tttggtacct tggtctctac cttgtgttgt cctgccaact 95940
cggaaaggga ttgggaagat cacgaggtaa attgtattta tattgctagt accagtgaca 96000
ctcaacttga agctgttcaa ggtgggatgc atatcactga gttacgtggt gaacccgtaa 96060
gagttcttta tgagacgggt cacttatacg catttgctag agaaaataca tgtcattccc 96120
gtttagaagt tagccataca gttagagcta tgacgtactt ttgggaccga ttttttagtc 96180
gccactggaa cgtggggcga cgtttcctag tattttacca gggaaacgga ggcgcctatg 96240
ttcaggcagc cctcgattca tccatgcata ctcaggatat ctatgttcta gggctctctc 96300
cgactgtcta tattagaggg aactatcacg tacagcacta ccgtgttcga ggattttggc 96360
cctcttgcct ggattctcta gcggcctgtg cggaaaatac atcagtactt cctacgggga 96420
atcgagtgac ggaatctttt acccctctct attcagccac acatttgata acgcgatacg 96480
gtatggtgag agatgcctgt tggtttgttc tgagggcatg ggaatgcttc cagaaacgca 96540
acaacaaaca tctcctttaa cttcactaga agggggacat gaggtagctc tagttctcaa 96600
tccccagcag aacccagagg ctctaagtat tgcctctaga ttgatgcatg aagaaagagg 96660
tgggagatta gaatctaact atatgcctgg acgttctagt aatcctttca tgacaagtat 96720
gtatgttctc gtacggctga atacacttgc tcagatctac ctgatgtctc cttattattc 96780
tttccaaagc aacgacattg tatgccttat ctttataagc agtgctgctg tagagacagt 96840
aagctacata ttcctgactg taactgactc aacttgtggg cgtcggtacc tgcgtgtccc 96900
acggctagtt tgtacagggt tacgtaacct ggcgttaccc acaactctac tagagctact 96960
tattttgtca taccctcgat cagtagaggg ggtacctttc aatgttagat tcattcttgg 97020
atatatgtgc actactagag ttgtattttt tgcatggaac ttgatcctcc actggccttt 97080
ccgatgtcta cgccatggaa tccaattgtt tgttcataga agtataatag gacatacgtt 97140
gggagcaaga attactgatt taaccctagc aagtatgcga tacgcaatag tgtttccatc 97200
tatagtaagt tcatgcttgt taactgctct tgctcatgca aatactaaca tacttgcctt 97260
ggacccttat agattgatcg aatctggaga tttaagacgt cccgcattta atgatgatga 97320
aatgcaacaa gcagataatc cttgggatgc ttactctatc ggcttagtta taaacacgtg 97380
tatctacatg ttaattttat tcgcaaacct aattttcatg gtgtactctg tacgaagata 97440
ccatagatcc cgccgctaag agtagcttgc cttaagtttc gtactatcta ttcttcggca 97500
atgcaagata agaaaacatt gattaagagc gaatagaacc ctaaaaaact gtgtttattt 97560
tttattacag ttttttagat ataaagatct ctttttagtt ccgtatctct aaatgaaatc 97620
aaagggttcc ttgtgaagaa aaccctacag attgcaaccg aagaatagac tcgaaatgag 97680
gggacacctt tgggaatctc tgttcgtagg tgcgtgactg attagaaaaa aagttttgat 97740
ttttcaaatg aaataagcct actttgtatt agaggctgat tcataaactt tcctaagaaa 97800
tatatgataa gaaaaacttg gggcggacag gatttgaacc tgtgacctac gggttatgag 97860
tccgcagctc taaccactga gctaccgccc cccaaggtaa gagagcaatg ctaacacata 97920
attttctaat gatcaaggga tatccacgcc tgaacggaat gtagttatgt ttgaggcggc 97980
tttaagtatt gtaccgctat ttgcttcata cgcatgtaca gagattctcc accattcaac 98040
ctaccaaaag aaagataggg agtcaggcgc tgaaaaaaaa ttggcttgca tgtcaaaatt 98100
gtagaaggag tttctccaga atatacctca gtaaataaag aggcgattcc tgaagacact 98160
aaagctttcg tataagtgaa aaaaaataaa attccatctt gatatacctc gtaaagatat 98220
aaatcactct gacaacctac aaccaagttc tccttcaaca tgcgcttctt gtcaaaagca 98280
tcccttgaag aactattttc catcaactta agatacagat gatctttttg aaaaggttct 98340
ggaaatagct cctcaataat tttatgctgt ttttttaaac acctagcgtg ttgcagagga 98400
cagataaatt ccaaaatact tctcaatgat ttgattcaat gatacgatca ggctcttttt 98460
tgatttgttg caaagtctga gataaggtca taatacattg gtcagctctt gcaataatat 98520
catcaggact gttgttattc aagtcaaggt atgagctagt tacaccacat tctggcatcg 98580
aaaaacaata gggcataggc tgatttaagt ttagtttcaa agaagctaaa agattcgctg 98640
tggcaggatc cgtttggttg ttgcgattta tctctataag cgcattcaac tgcttttgat 98700
tgatcatttg gtgcttgatg atttgatgct ggttgatcgc ttgctcaatg ttctctattc 98760
gttttttgtg ggccttgcga tcaatagcat aagtagcctc atctcctaag cgcgccccat 98820
aaacagtatt aatacttaat tgatttccaa tggaaaaacc tataacaaga ccaacagcct 98880
gaggaaccgc ataaatcaac gctgaaatag aggctatcat cgtggctaaa agaatttgtc 98940
gtgttccgtt cggatctaat tgcaaaattc catggtttat caaattccat agactgttta 99000
catgcctatg cttgttctct ttatctaaac atgtcgctgt aaaaatgcca aaaacaacgc 99060
caatccctag acccacccct aaccaaattg ttagagttaa agcaaccgta tgatgacata 99120

ctaaaattaa tgcccaagct aaaatataaa gtaatatata tttccaattc ctttggagga 99180
ataaaccaat tttttcaacc gcgtttttta aacgagttgt ccaagaaaat gaattttgat 99240
tataaggaac taatttcaga tcagttaatg caactaacgc tgaaatagga gggctgacta 99300
taggagagtc ccctgaagca tttgaatcag gagagttcga tctagaagag ctcagtctgt 99360
cacaaaatag ttcttgagga acgtattggt cagaaggcga aattcctcca gaagatagtg 99420
gagtagacat tcgatagtga aaaaaataaa acaataagca ctatattaaa ataaggactt 99480
tttctttgta aacagaggtt tttgtgcttc tatagcccct ctagtttact caatatttta 99540
ttggtagtat tcgcaaggtt ttgtgaaagg gatttgactt tttctaaata acaaatataa 99600
tcctaatgcg tttatagaat aggggaaatg aagagcaccg ttctattttt agggctgaat 99660
ctacccacta ggccagccag tcacaggctt aggcttaaaa gatttgttta aggataattg 99720
atcaatggcg aaaaaagaag atactcttgt actcgaaggt aaggtagaag agctccttcc 99780
aggaatgcat tttcgtgtaa tactagaaaa cggtatgcca gttaccgccc atttgtgcgg 99840
aaaaatgcgt atgagtaata ttcggattgc ttgttggaga ccgcgttact gtcgagatgt 99900
ccgcctatga cttaacaaaa gctagggttg tctacagaca tcgttaatta tattttctat 99960
tgatgtttta aaataagtga catagactag gcggtttttc aagaccggga agcaatgcat 100020
aagtaagccc agatagctca gtggtagagc acttgcatgg taagcaagcg gtcgtaggtt 100080
caattcctat tctgggcaga aagaatggtt ggagtaatca ataattttta agaggatttt 100140
gagatgtcaa aagaaacttt tcaacgtaat aagccccata tcaatattgg gacgatcggg 100200
cacgttgacc atggtaaaac tacgctaaca gcggcaatta cacgcgcgct atcaggggat 100260
ggattggcgt ctttccgtga ctatagttca attgacaata ctccagaaga aaaggctcgt 100320
ggaattacta tcaacgcttc tcacgttgaa tacgaaaccc caaatcgtca ctacgctcac 100380
gtagactgcc ctggtcacgc tgactatgtt aaaaatatga ttacaggcgc cgctcaaatg 100440
gacggagcta tcctagtcgt ttcagctaca gacggagcta tgccacaaac taaagaacat 100500
atcttgctag ctcgccaggt tggagttcct tatatcgttg ttttcttgaa taaagtagat 100560
atgatctctc aagaagatgc tgaacttatt gaccttgttg agatggaact tagtgagctt 100620
cttgaagaaa aaggctacaa aggatgccct attatccgtg gttctgcttt gaaagctctt 100680
gaaggtgatg caaattatat cgaaaaagtt cgagaactta tgcaagctgt ggatgacanc 100740
atccctacac cagaaagaga aattgataag cctttcttaa tgcctatcga agacgtattc 100800
tcaatctctg gtcgtggtac tgtggttaca ggaagaatcg agcgtggaat cgttaaagtt 100860
tctgataaag ttcagctcgt gggattagga gagactaaag aaacaatcgt tactggagtc 100920
gaaatgttca ggaaagaact tcctgaaggt cgtgcaggag aaaacgttgg tttactcctc 100980
agaggtattg gaaagaacga tgttgaaaga ggtatggtgg tttgtcagcc taacagcgtg 101040
aagcctcata cgaaatttaa gtcagctgtt tacgttcttc agaaagaaga aggcggacgt 101100
cataagcctt tcttcagcgg atacagacct cagttcttct tccgtactac agacgtgaca 101160
ggagtcgtaa ctcttcctga aggaactgaa atggtaatgc ctggagataa cgttgagctt 101220
gatgttgagc tcattggaac agttgctctt gaagaaggaa tgagatttgc aattcgtgaa 101280
ggtggtcgta ctatcggcgc tggaacgatt tcaaagatca atgcttaaaa atgaatttcg 101340
cgatgatttt catcatcgcg attttctggg tgtgtagctt agctggtaga gcagtggcct 101400
ccaaagccgc cggtcggggg ttcgattccc ttcgcacccg tagatttaat ttttaatcta 101460
gaagttggtt tatgaaacaa caacacaatc gtaaggcttt atctcgcaag attggcacag 101520
tgaaaaaaca agccaaattt gcaggaagct tttagatgga gattaaaaaa attgaatggg 101580
taagcaagca cgatcttaag aaatacataa aagtagttct tatcagtatt tttggttttg 101640
gatttgctat ttatttcgta gatcttgtgt tgcgtaagtc aatcacatgt ttagatggta 101700
taacaacctt tttgttcggt taattgcatg tataaatggt atgtcgttca agtttttaca 101760
gctcaagaaa agaaagtaaa aaaggcttta gaagatttta aagagtcttc aggaatgact 101820
gattttatac aggaaattat cttgcctatt gaaaatgtca tggaagtgaa aaaaggagaa 101880
cataaggtcg ttgaaaaata catctggcct ggatacctct tagttaaaat gcatctgact 101940
gacgagtctt ggctctatgt taaaagtaca gcaggtatag tcgagtttct tggaggcgga 102000
gtccctgtag ctctttctga agatgaagta agaagtatct taacagatat agaagagaag 102060
aaatcgggag tggtgcaaaa acatcagttc gaggttggtt ctagagtgaa aattaatgac 102120
ggagtctttg tcaattttat cggcacggtt tccgaagttt tccatgataa aggacgcctg 102180
agtgttatgg tttctatctt tggaagagaa actagggtag atgatttaga attttggcaa 102240
gtggaagagg tagccccagg gcaagaaagt gagtagatag gttaaaatca gtgtattctt 102300
attccttatc ttcttataat tttagttttt cgtttcttac cctctgtttg tagaggtgtc 102360
tcagtgcaaa gtaaggttta gtatgtcggt aaaaaaggta atcaaaataa ttaagttgca 102420
aatccctggg ggtaaagcaa atcctgcgcc acccatagga ccagctttag gtgctgctgg 102480
agtcaatatt atgggcttct gtaaggagtt taatgctgca actcaagata agcctggaga 102540
cttacttcca gtagtcatca ctgtttatgc tgataaaact tttactttta taaccaaaca 102600
gcctccagtc tcctctttaa taaagaaaac tttgaatctg gaatcaggat ctaaaattcc 102660
taatcgtaat aaagtaggaa aacttactca ggctcaagtt gaagcaattg ctgaacaaaa 102720
aatgaaagat atggatattg tccttctaga atctgcgaaa cgtatggtgg aaggaactgc 102780
ccgtagtatg ggtatagacg tagaataaat tgttacttgt agagctgtag aattatgaca 102840
aaacatggaa aacgtatacg aggcatctta aagaactatg atttctcaaa atcatattct 102900
ttgcgggagg ctatagatat tttaaaacaa tgtcctccag tacgcttcga tcaaactgta 102960

gatgtatcta tcaagttagg gatagatcct aaaaagagcg accaacaaat tcgtggagcc 103020
gtttttttac ctaatggtac aggaaaaact ttaagaattt tggtttttgc ttcagggaac 103080
aaagtcaaag aagctgttga agcgggcgca gactttatgg gaagcgacga tcttgttgaa 103140
aaaattaaat ccgggtggct ggaattcgat gttgctgtcg ctaccccaga tatgatgcgt 103200
gaagtaggaa aattaggaaa agtcttagga cctagaaatc taatgcctac acctaaaaca 103260
ggaacggtaa ccacagacgt tgctaaagca atctccgaat tgcgtaaagg aaaaattgaa 103320
tttaaagcag accgcgcagg cgtatgtaat gtaggcgtag gtaagttgtc ttttgaaagc 103380
agtcaaatca aagaaaatat tgaagctcta agttctgctt taattaaggc caaacctcct 103440
gcagctaaag gtcaatattt agtctcattc actatttctt ccactatggg gcctggtatt 103500
tctatagata ctagagaatt aatggcatct taatcttaaa gagggaaaat gaaacaagaa 103560
aaaacattac ttcttcaaga ggtagaagac aaaatttccg cagcacaggg attcatttta 103620
ttaagatacc ttagatttac cgccgcgtat tctagagaat tcagaaactc actttctgga 103680
gtttctgcag aatttgaagt tttaaagaag agaatcttct ttaaagctat agaagctgca 103740
ggtttagagg tagattgtag tgatacagat gggcatctcg gtgtagtctt ttcctgtgga 103800
gatcctgttt ctgccgcaaa gcaggtactg gactttaata aacaacataa agactcttta 103860
gttttcctcg ctggaaggat ggacaatgcg tctctgtctg gtgcagaggt agaagctgtc 103920
gccaaattgc catctcttaa agaacttaga cagcaggttg ttggtttatt cgctgctcca 103980
atgtcccaag ttgtaggaat tatgaattct gtcctttctg gagtgatctc ctgtgtggat 104040
caaaaggcag gaaagaacta aagaattaaa attaaaactc tcaaaataag taagggtgac 104100
aaaagtgaca acagaaagtt tggaaacttt agtagagaag ttaagtaatt taactgtact 104160
agaactctct caattgaaaa aattattaga agagaagtgg gatgttactg cttctgctcc 104220
cgtagttgct gttgctgctg gtggtggcgg agaagctcct gttgctgccg aacctacaga 104280
atttgcagta accctcgaag atgttcctgc agataaaaaa atcggcgtct taaaagtcgt 104340
tagggaagta actggattag ctttaaaaga agctaaagaa atgacagaag gtttacctaa 104400
aactgttaaa gaaaaaactt ctaaaagtga tgctgaagat actgttaaga agttacaaga 104460
tgctggcgca aaagcctcat ttaagggact gtaatttgta gaaaagaaaa atcgaaagat 104520
ttttcttttc tttttctttt catgtataaa aaaccgaatg ctccctttag aagcatacgt 104580
aggcttaatt tagggaaatt ttgtcgcatc aaaatagcag gagaactcgc acgttgaagt 104640
gccctgaacg ggtcagtgtt aaaaaaaagg aagatatccc agaccttcca aatcttatcg 104700
aaatccaaat taagtcttat aagcagtttc ttcaaattgg aaaattagca gaagaaagag 104760
aaaatatcgg tttagaagag gttttcaggg aaatttttcc cattaaatcc tataacgaag 104820
ctaccgttct tgagtacctt tcatataatt tgggtgtgcc aaaatattct ccagaagaat 104880
gtatccgtag aggaattacc tatagcgtca ctttgaaagt ccgttttcgt ttaaccgatg 104940
aaacgggaat caaagaagaa gaagtctata tgggaacgat ccctctaatg actgataaag 105000
ggacatttat cattaatgga gctgaaagag tcgttgtttc ccaagttcat cgttctccag 105060
gaattaactt tgaacaagaa aaacattcca aaggtaatat tttattctcc ttcagaatca 105120
ttccttatcg tggaagttgg ctcgaagcta ttttcgatat taatgactta atttatatcc 105180
atattgatag aaaaaaacgt agaagaaaaa ttctagcaat cacctttatc cgagctcttg 105240
gatactcttc agatgcagat atcatcgaag aattcttcac aataggagaa agttctctta 105300
gaagtgagaa agactttgct cttcttgttg gaaggatttt agcagacaat attattgatg 105360
aagcctcctc tctagtttat ggaaaagccg gagaaaagtt aagtacagca atgttaaaac 105420
ggatgctcga tgctggaatc gcttctgtta agattgctgt agatgctgat gaaaatcatc 105480
ctattatcaa aatgctcgct aaggatccta cagattcata cgaagccgct ttaaaagatt 105540
tttatcgtag actacgtcca ggagaacctg caactctagc taatgcacgt tctactatca 105600
tgaggctctt ctttgacccc aaacgttata atctaggacg tgtagggcgt tataagctca 105660
atcgcaaact aggcttctct atagatgatg aagctctgtc tcaagttact ttgagaaaag 105720
aagatgtgat cggagcctta aagtatctga ttcgtttgaa aatgggagat gaaaaagctt 105780
gtgtagacga tattgatcat cttgctaatc gacgtgtccg ctctgtcgga gaactcattc 105840
aaaatcaatg tcgttcagga cttgctagaa tggagaaaat tgttagagag agaatgaatt 105900
tattcgattt ctcctcagat acgttgactc caggaaaagt tgtctctgct aaaggtctcg 105960
ctagcgtgtt aaaagatttc tttggccgct cccagctttc gcagtttatg gaccaaacca 106020
accctgtagc tgagttaact cacaaacgac gtctttctgc attaggtcca ggaggactaa 106080
atagagaacg cgcaggattt gaagttcgtg acgtgcacgc aagtcattat ggacgtattt 106140
gtcctattga aactcctgaa ggtccaaata ttggtctgat cacctctctt tcctcttttg 106200
ctaaaattaa cgaatttgga ttcattgaaa ctccttatag aattgtaaga gatggaatcg 106260
taacagatga aatcgaatac atgacagccg atgttgaaga agaatgtgtg attgcacagg 106320
cttcagcaag cctagatgag tacaatatgt ttacggaacc cgtctgttgg gtacgttatg 106380
ctggagaagc tttcgaagca gatacaagca ccgtaaccca tatggatgtt tctccgaaac 106440
agctcgtttc tattgttaca ggattgattc ctttcttaga gcacgacgat gcgaaccgcg 106500
ccttgatggg ctccaatatg caacgtcaag cggttccctt acttaaaacc gaagctcctg 106560
ttgttggcac tggattagaa tgtcgtgctg ctaaagattc tggagctatt gttgttgcag 106620
aagaagatgg tgttgttgat tttgttgatg gttacaaagt agttgttgct gcaaaacata 106680
atcctacaat taaacgtacc tatcatctga aaaagttcct tagatctaat tcaggaactt 106740
gcattaacca acagcccttg tgtgcagtcg gtgatgtcat aactaagggt gatgtgattg 106800

ctgatggacc cgcaactgat cgtggagaac ttgctttagg taaaaatgta ctcgttgcct 106860
ttatgccttg gtatggatac aactttgagg atgcgatcat tatctctgaa aaattgatca 106920
gagaagatgc ctatacctct atttatattg aggaattcga actaacagcc cgagatacaa 106980
aattaggaaa agaagagatc actcgtgaca ttcctaacgt atctgatgaa gtattggcca 107040
atctcggtga ggatgggatc attcgtatcg gtgctgaggt taaacctggg gatattcttg 107100
ttggtaagat cacaccaaaa tcagaaacag aattagctcc agaagagcgt ctgctccgtg 107160
ctatttttgg tgaaaaagct gctgacgtta aagatgcatc tttaacagtg cctccaggaa 107220
ctgaaggcgt cgttatggat gttaaagtct tcagtagaaa ggatagattg tcaaagagtg 107280
atgacgaact tgtagaagaa gctgttcatc ttaaagattt gcaaaaagga tataaaaacc 107340
aagttgcaac tttaaaaaca gaatatcgtg agaaattagg agctctctta ttaaatgaga 107400
aagcacctgc agccattatt caccgtcgta cagcagaaat cgttgttcat gaaggcctac 107460
tctttgatca agagacaata gaacggatag aacaagaaga tttagtggat cttttaatgc 107520
ctaactgtga aatgtatgaa gtgttgaaag gacttctatc agattacgaa acggcattac 107580
aacggctaga aatcaattat aagactgaag ttgagcatat tcgtgaggga gatgcagatt 107640
tagatcatgg tgtcattcgc caagttaaag tctacgttgc ctctaagaga aaacttcaag 107700
ttggagataa aatggctgga cgacacggaa ataaaggtgt tgtttccaaa atcgttcccg 107760
aagcggatat gccatatctc tctaacggag aaactgtaca aatgatcctg aaccccctcg 107820
gggtgccttc aaggatgaac cttggacagg tattagaaac acacctaggt tatgcagcaa 107880
aaactgcagg catttacgtg aaaacccctg tttttgaagg attccctgaa caacgtatct 107940
gggatatgat gatagaacag ggattaccag aagatgggaa gtccttctta tatgatggga 108000
agacaggtga acgctttgat aacaaggtag tgataggcta tatctatatg ctaaagctca 108060
gtcacttgat cgctgataag attcacgcaa gatctatagg gccatattct ttagtcacgc 108120
aacaacctct cggtggtaaa gctcagatgg gaggacaaag attcggggaa atggaagttt 108180
gggctctaga agcatatggg gttgctcata tgctccaaga aattctaacc gtgaaatctg 108240
atgatgtctc aggaagaaca aggatttacg aatctatcgt taagggggaa aacctcttgc 108300
gatcaggaac gcctgagtcg ttcaatgtgc taattaaaga gatgcagggt ctaggacttg 108360
atgttcgtcc tatggtcgta gacgcttaaa aaatgacgtt ttggagaaaa taatgttcgg 108420
agaaaattct cgagacattg gagttctttc taaagaagga ctatttgata aattagagat 108480
aggcatagct tcagatatta caattcgtga taaatggtct tgtggagaaa tcaaaaagcc 108540
agaaactata aattaccgta cgtttaaacc tgaaaagggc ggtctatttt gtgaaaaaat 108600
ccttggtcct actaaagatt gggaatgttg ctgcggaaaa tataaaaaaa taaaacataa 108660
aggaattgtc tgcgatcgat gcggagttga agttactctt tcaaaagtcc gtcgtgaacg 108720
tatggctcat atcgagttag cagttcctat tgtccatatt tggtttttca aaacaactcc 108780
atcacgcatt ggtaatgttc ttggaatgac agcttcggat ctggaacgtg tcatttatta 108840
tgaagaatat gtagttattg acccaggtaa gacagaccta actaaaaaac aacttcttaa 108900
tgatgcgcaa tatcgtgaag ttgttgagaa gtggggtaag gacgctttcg ttgctaaaat 108960
gggtggcgaa gctatctatg atttgcttaa atccgaagat ctccaaagct tgcttaaaga 109020
tcttaaagag cgtttacgca aaacaaaatc tcagcaagcg agaatgaagt tagccaaacg 109080
tcttaaaatc attgagggat ttgtttcttc atccaaccac ccggagtgga tggtattaaa 109140
aaatatccca gtagttccac ctgatctccg tcctcttgtt cctttagatg gcggtcgttt 109200
tgcgacttct gatttaaacg atctctaccg ccgtgtaatt aatcgtaaca atcgtcttaa 109260
agcgatctta cgtttaaaaa caccagaggt tattgttcgt aatgaaaagc gtatgcttca 109320
agaagctgtt gatgctcttt ttgataacgg tcgacatggt catccggtca tgggagctgg 109380
aaaccgacca ttgaaatcct tgtcagaaat gttaaaggga aaaaatggac gcttccgtca 109440
aaatctttta ggaaaacgtg ttgactactc tggacgttct gtaattattg ttggtcctga 109500
attgaagttt aatcaatgcg gattgcctaa ggaaatggct ttagagctat tcgaaccctt 109560
tattattaan agactaaaag atcaaggcag cgtttatacc attcgttctg ctaagaaaat 109620
gattcaacga ggagccccag aagtttggga cgttctcgaa gagatcatta agggacatcc 109680
agtacttctt aaccgagcac ctacattgca ccgtttagga attcaagctt tcgaacctgt 109740
attgatagaa ggtaaagcga ttcgtataca ccccctagtt tgcgcagcgt ttaacgctga 109800
cttcgacgga gaccaaatgg ccgtgcacgt tcctctatct gtagaggcac aactggaagc 109860
taaagtttta atgatggctc cagacaacat cttccttcct tcctcaggaa agcctgtggc 109920
tattccttcg aaagatatga ctttaggatt atattatctg atggcagatc ctacctattt 109980
tcctgaagaa catggaggaa aaactaagat atttaaagat gaaatcgaag tattgcgtgc 110040
tttaaataac ggtggattca ttgatgatgt tttcggagat cgtcgtgatg aaacaggacg 110100
cggtatccat attcatgaaa agattaaagt gcgtattgat ggacaaatta ttgagacaac 110160
cccaggaagg gtattgttca acagaattgt tcctaaagaa ctcggcttcc aaaattacag 110220
catgccaagt aagcgtataa gtgagcttat tttacagtgc tataagaaag tcggtttaga 110280
agctactgta cgtttcttag atgaccttaa agatcttgga tttattcaag ctacaaaagc 110340
cgcaatctct atgggattga aggatgttcg tattcctgat atcaagagtc atatcctcaa 110400
agatgcctac gataaggttg ctatcgtcaa aaaacaatat gatgatggga tcattactga 110460
aggggagcgt cattccaaaa ctattagtat ttggactgaa gtttccgaac agctttcaga 110520
tgccctctat gttgaaatta gcaaacaaac acgtagcaag cataacccct tgttcctgat 110580
gattgattct ggagcccgag gtaataaatc ccagttgaaa cagttgggag cgttacgagg 110640

attaatggcg aagccaaacg gagcaattat tgaatctcca attacttcga actttagaga 110700
aggattgaca gttttagagt actccatctc ctcacacggt gcgagaaaag gtttagccga 110760
tacagctcta aaaactgccg actccggata cttaacacgt agacttgtag acgtagccca 110820
agacgtgatc attaccgaaa aagattgcgg tacgttaaat cacattgaga tttctgcaat 110880
aggtcaaggt tctgaagaac tcttgcctct taaagatcgt atctatggac gtactgtagc 110940
tgaagatgtc tatcaaccag gtgataaaag tcgactactt gctcaatcgg gtgatgtact 111000
caactccgta caagcagaag caattgatga tgccggtatt gagacaatta agattcgttc 111060
tacattaacg tgcgaaagtc ctcgcggagt ttgtgcaaag tgttacggcc tcaatttagc 111120
taatggtaga ctcattggca tgggtgaagc tgttggtatt attgctgctc agtcgattgg 111180
ggaacctgga actcagttaa caatgagaac gttccaccta gggggtattg ctgctacgtc 111240
ttcaactcct gagattatta cgaatagtga tggtatctta gtctacatgg atctccgtgt 111300
tgttctgggg caagaaggtc acaatcttgt cttgaataag aagggagctt tacatgttgt 111360
aggtgatgaa ggtcgtactc tcaatgagta taaaaagctg ctttcaacca agtctataga 111420
aagcctagag gtatttcctg tagaactagg agtgaaaatt cttgttgctg acggaactcc 111480
tgtttctcaa ggacaaagaa tcgcagaagt tgaactacac aatattccta tcatttgcga 111540
taagcctggc tttattaaat atgaagattt ggttgaggga atctctacag agaaagttgt 111600
gaacaagaac acaggacttg ttgaacttat tgtgaaacag caccgagggg agttacatcc 111660
tcagattgct atctatgatg atgctgactt gtcagaactt gtcggaacct atgcgattcc 111720
ttcaggagcg attatctctg tagaagaagg acaacgggtt gatccaggta tgttgttagc 111780
tagacttcct cgcggagcta tcaaaacaaa agatattact ggcggtttgc ctcgtgttgc 111840
tgaattagta gaagctcgta aacctgaaga tgctgctgac atcgccaaaa ttgatggtgt 111900
tgttgacttc aaaggaattc aaaagaacaa acgtattctt gttgtctgtg atgaaatgac 111960
aggtatggaa gaagaacatc tgattccatt aaccaaacat ttgattgtac aacgtggaga 112020
tagtgtgatt aagggcagca gcttaccgat ggtttagttg ttcctcatga aatcctagaa 112080
atttgcggag ttcgtgaact tcagaagtac ctggtaaatg aggtgcagga agtttaccgt 112140
ctgcagggcg ttgacattaa cgataagcat attgaaatta ttgttcgtca gatgttacaa 112200
aaagtacgaa ttactgaccc aggtgatacg actctgctct ttggcgaaga cgtgaataag 112260
aaagagtttt atgaagaaaa tcgtcgtacc gaagaagacg gtggtaagcc agctcaagct 112320
gttcccgtct tattgggaat tacgaaagct tctttgggta cggaatcgtt tatatcagca 112380
gcttctttcc aagacacaac tcgagtctta acagatgcag cttgttgtag caaaaccgac 112440
taccttcttg gatttaagga aaatgtgatc atgggtcata tgattcctgg tggtacaggc 112500
tttgaaacgc ataagcgtat taagcagtat ctagaaaaag aacaagaaga tctcgttttt 112560
gattttgtta gtgaaacnga gtgtgtttnn taactaggtg acacagtctt ttatcaagga 112620
ggttatgttt acaacctcct tgataggaat gttttttttt gttaacgttg cctagagatc 112680
aacagtgatg ccaaggtgcc tatgtctaac caatttgatc aattaaagaa gttgagcact 112740
atcgtttgtg atagcggaga cccagagcta gttaaagcct cgggatctca agacgctaca 112800
acaaaccctt ctttgatctt aaaagtggcc caagaaccca aatttcaaga gctattaaac 112860
gaagctgtag tttggggaat ccgacagaac ggtgatgatc ttcagactct ttcttttatt 112920
ttagacaaaa ttcaggttaa ctttgctcta gaaattatca aaaatatccc tggtagaatt 112980
tctcttgaaa ttgacgctag gctttctttc aacgttgaag ctatggtaca gcgtgccgta 113040
ttcctttcgc agcttttcga agctatggga ggagataaaa agcgcctgtt agtaaagatt 113100
cctggaactt gggaaggtat tcgagctgtt gaatttttag aagcaaaggg catagcatgt 113160
aatgtcactt tgatttttaa tttagttcaa gcgattgcag ctgctaaagc taaagcaact 113220
ttaatttctc cttttgttgg ccgtatttat gattggtgga tcgcggctta tggtgatgaa 113280
ggttactcta tagatgcaga tccaggtgtc gcttcagtat caaatattta cgcgtattac 113340
aaaaaattcg gtattcctac gcaaattatg gcagcatctt ttcgtacaaa agagcaggta 113400
ctagcattag ctggttgcga tcttttaacg atatctccaa agctgctgga tgagctaaag 113460
aaatctcaac acccagtaaa aaaagaatta gatcctgcag aagctaaaaa gttagatgtg 113520
cagccaatag aactcacaga aagctttttt cgctttttaa tgaatgagga tgctatggct 113580
acaganaaac ttgctgaagg aattcggata tttgcaggag atactcaaat tcttgagact 113640
gcaattacag agtttataaa gcaaattgct gcagaaggtg cgtaattgct tactaaatta 113700
agccgatttg gggataccac cttaaagcga aatgaaaaat aagatggact ataaatcgca 113760
actagtattt tcttgccctt gttgttgcaa aggcaatgtt tgtttctcag tttttaactt 113820
agacgttatt ttaacatgta acgtttgctc atctacttat acattcgatt ctgtcatacg 113880
taatgagatt cgtcagtttg tagcactatg taaaaggata catgatgcta attctatact 113940
tggaaatgct actgtgtcgg tatcggtaga agacaaccaa atggatattc cctttcaatt 114000
gctgttttct cgtttccctg tagtattaaa tctctcttta gatggaaaga aaatagctat 114060
tcgtttcctc tttgatgctt taaatacaag tatcttacac caagaaagcg atcttatttc 114120
ttaatcctaa gtttatttgt tttcgttttg cagagcttcc aaagcttttt caaggattgc 114180
atctcctgaa gtagcctctt taccaccgcg aatttggaaa gaagagacta cttttaaaaa 114240
ggtaaatgaa agttctgcat agtcatccaa agttgcagtg cttgtaaaaa tataagcagt 114300
atggtcaatg actgttgtcg cttgtaaaca aaatacacgt ccccatgaag agtttttctc 114360
tgttttgata atagtaaact ctccgctagg agattgaatt tgggtaaata ttccagattc 114420
taaagtcatc tcattggctt tatgataggc taagatttcc tcaatatact cttttgaaga 114480

tttggaagtg atttcctgag caatgttgat ggtaggagtg agatttcctt tccccttgcc 114540
tataaggaga acatctaatt tttctgggag ctgtgtttta tcgtcaatac actgccaaag 114600
agagggtgtc tgtatgctat agtttttccc tgaatagcga acccattgga cgggggagag 114660
cgagggtttt ttctcaaatt ttcctatttt tttccgcgca gtgctcttgt tttatagcga 114720
gaaacactcg cattcgttgc tgccgaagat cgtatcctag aggtccttct tgcttttgct 114780
tgctttgagt gagatgcaga gacttgagtt cttggctgag cagagaaagc agggaattga 114840
gctacaatca aaataaaaag aataaaaaat ttcatagaaa attatgtagt tagttgtaca 114900
ttaagataga ttcaaagcac atagcatagc tctctacctt atacaagatt gtatcgtttt 114960
cagaaaaata ttcccaatag ggaatctttg gatctagaag aaggcctact ttcaaacaaa 115020
catagaaagg caattcttct tttttgaatt ctatgtttat aggaattttt tcttagagag 115080
ttagaggaga gcatgggatt tatcttgaag atatcaaagc aattttcttc tagaacatct 115140
ctgcaacccg tttcttattt gtggatcagt tgaattaaat gctagaatga cataaactgt 115200
tagattagtg gaatcataat aacatctttt ttaaagaaaa ttcttttcta aaagaataag 115260
cggtaaaata ttatggcgaa tcttaatgcc gatggtaagc ttaagcaaat ctgcgatgct 115320
ttgcgtttag acactctaaa gcctgcagaa gacgaggctg cggcgttatt gcataatgct 115380
aaagaacaag cgaaaagaat tattcaagaa gctcaagaag aagccagaaa aatcttagag 115440
acggcagaag agagagctca tcaaaagata aaacaaggcg aagttgctct aagccaagca 115500
gggaagcgcg ctttggaagc cttaaaacag gctgtagaaa acaaaatatt tagagagtct 115560
ttagtagagt ggctggagca tgtaaccacc gatcctgagg tttctacaaa gttaattcaa 115620
gctttagtgc aggctttgga agctcaaggg gtttcaggaa atctgaccgc ctatatagga 115680
aaacatgtga gtcctagagc tgttaatgag ctcctaagga aaggctgtaa caacaaaaac 115740
tacgaaagaa aagtgtagtt gttggaagtt ttgttggtgg tgttcaatta aaagttgaag 115800
aaaagaactg ggttctggat cttagttcct cagctcttct tgagattttc acacgttatt 115860
tgcagaaaga ttttcgtgaa atgatttttc aaggatcttg actttaataa agtcatgaaa 115920
agatcttctc aatatttaaa gttgtcgtca tgactcaata ttatttttta tcttcatttt 115980
tacctactca gctaccagaa tccgtacctc tattttctat ttcggactta gacgatctac 116040
tttatttaaa cctatcagaa aacgatcttt gcaattacgg acttcttaaa cgtttttttg 116100
atttcgaaaa tttcgctttc ttttgggctg gtaaaccgat tcccttctct tttggggagg 116160
tgactcagga aaatgtagaa agaatgcttt cctctcagca gtggtctgat gacaatgatt 116220
ttgaagattt ctttaaggat tttttaatga atcataagtc ttctcaagat cgtttgaatc 116280
acttttcaga tttatttaga gagtttcttt cctatcatca aacgaattct tcaaagtttc 116340
ttcaagatta tttcagattt caacaacaac ttcgtgttgt actcgcggga ttccgtgcaa 116400
gagtcttgaa tatggatgtt tcctatgttt tgcgcgacga agatagttcc gatccagttg 116460
tgctcgaggt gctcatgcag aaagattctc ctaattatga gcntcctgaa gagtttncgg 116520
atttacaggg cgttttggat gactaanggc cttctngcct nanacactga atnggngcgc 116580
ntngccnnta taccaatttc ataaactcga gggattttgt tccngactcc tactttgatg 116640
ggaatgtcat tttagcaaga tgtgctacat atatgtttgc tattcgtaca gcttagcaag 116700
tgttgaaaaa ggaagagaaa ttattaatca tatagaaaag gcaatcaaat ggtaacagtt 116760
tcagaaccaa acttgctcag ggacatgtta tagaagctta tggaaacttg ttacgtgtac 116820
gctttgacgg atatgttaga caaggtgaag ttgcatatgt caacgtagat aatacctggt 116880
taaaagcaga agtgattgaa gttgctgatc aagaagtcaa ggttcaggta tttgaagata 116940
cacaaggcgc gtgtcgagga gctcttgtta cgttttcagg acatctttta gaagccgagt 117000
tagggcctgg cttgcttcag ggcattttcg atggacttca aaatcgtctt gaggtgctag 117060
ctgaagatag ttctttcttg cagagaggca agcatgttaa tgctatttct gatcataatt 117120
tatggaatta tactcccgta gcttctgttg ggatactttt aagacgagga gatcttctag 117180
gaacagtacc tgaaggacga tttactcata agattatggt tcctttttct tgctttcaag 117240
aggttaccct gacttgggta atttctgaag gaacctataa tgctcatact gtggtcgcaa 117300
aagctcgaga tgctcagggt aaagaatgtg cctttactat ggtgcaaaga tggccgatca 117360
aacaagcttt tattgaagga gagaagatcc ctgcgcataa gattatggat gtgggtttgc 117420
gaatcttaga tacgcaaatt ccagtattga aggggggaac tttctgtacc ccaggacctt 117480
ttggtgcagg gaaaacagtc ttacaacacc atctttctaa gtacgctgct gtagatattg 117540
tgattttgtg tgcgtgcgga gagcgtgctg gtgaagttgt tgaggtatta caagagttcc 117600
ctcatcttat cgacccccat accggaaagt ctttaatgca cagaacatgt attatttgta 117660
acacatcatc catgcctgtg gctgcccgag agtcttcgat ctatttagga gtgacgattg 117720
cagaatacta tcgccagatg ggactagata ttctgctttt agctgattct acatcccgat 117780
gggcacaagc ccttagagag atttcgggac gtcttgaaga aatccctgga gaggaagcat 117840
ttcctgcata cctgtcttct agaatagctg ctttttatga gcgaggagga gctatcacca 117900
cgaaagatgg ttctgaagga tctttaacta tatgtggtgc ggtgtctcct gcaggaggaa 117960
actttgaaga accagtcact caatctacat tagctgtagt cggagcgttc tgtggtcttt 118020
caaaagcacg actgacgcac gtaggtatcc ttcaatagac cctttgattt cttggtcaaa 118080
atatttgaac caggtaggac aaattttaga agagaaggtt tcaggctggg gtggtgctgt 118140
gaaaaaagca gcacagtttc tagagaaagg ttcagaaatc ggcaagcgta tggaagttgt 118200
cggtgaagaa ggggtttcta tggaagacat ggaaatctac ttaaaggcag aactttatga 118260
tttttgttat ctccagcaga acgcattcga tcctgtggac tgttattgtc cttttgagag 118320

acagatagag ttattttcat taatcagtcg tatttttgat gctaaatttg tttttgatag 118380
tcctgatgat gcaagaagct ttttccttga gctgcagagc aagattaaga cattaaatgg 118440
cctgaaattt ctttcagagg aatatcatga gagtaaagag gtcatagtta gactgttgga 118500
aaaaacaatg gtacaaatgg cgtaaggata tgcaaacaat ctacacaaaa ataactgata 118560
ttaaaggcaa tttaatcact gtagaagcag agggagctcg tttaggggag cttgctacaa 118620
tcacaagatc cgacggaaga tcttcgtatg cttcggtatt gcgttttgac cttaagaaag 118680
taactctcca ggtttttggt ggcacatcgg gcttatccac tggagatcat gtcacgttct 118740
tagggagacc catggaggtc acatttggga gctcattatt aggcagacga ttgaatggta 118800
tagggaaacc cattgataat gagggggagt gttttggaga acctatagag attgctactc 118860
caacatttaa ccctgtctgt cgtattgttc ctaggagtat ggtacggaca aatattccta 118920
tgattgatgt tttcaactgt ttagtgaaat ctcagaaaat tcctattttt tcttcttctg 118980
gagaacatca taatgctttg ttaatgcgga ttgctgcaca gacagacgcg gatatagttg 119040
tgattggtgg gatggggctt acattcgtag attacagctt ttttgttgaa gagtctaaga 119100
agctaggatt tgcagataag tgtgtgatgt ttattcataa agctgtagat gctcctgtag 119160
aatgtgtttt ggttcctgat atggccctag cttgtgctga aaaatttgct gtagaagaga 119220
aaaagaacgt cttggttttg cttacagaca tgacagcgtt tgctgatgct cttaaggaaa 119280
tttctatcac tatggatcaa attcctgcca atcgtgggta ccccggttcc ctatattctg 119340
atctagcttt acgctatgaa aaagctgtag aaattgccga tggggggtcg atcaccttaa 119400
ttactgtaac tacgatgcct agtgacgaca ttacacatcc tgttcctgat aacacaggat 119460
acattacaga gggacaattc tacttgagga ataatcgtat agatccgttt ggttctcttt 119520
caagattgaa gcagctggtc attggtaagg tgactcgaga ggatcatgga gatcttgcga 119580
atgctttaat tcgtctttat gcggattccc gtaaagctac agaaagaatg gctatgggat 119640
tcaagttatc gaattgggat aagaaattac ttgcgttttc cgagcttttt gaaactcgtt 119700
tgatgagttt agaggtaaat attcctttag aagaagcttt agatattggt tggaaaattc 119760
tagctcaaag tttcacttct gaagaagtgg gaattaaagc ccagttaata aataagtatt 119820
ggccaaaagc atgtctgtcc aagtaaagct aacaaagaac tcctttcgac tagaaaaaca 119880
aaaactagca cgattacaaa cgtaccttcc gacattaaaa cttaagaaag ctttattgca 119940
ggctgaggta caaaacgctg ttaaagatgc tgcagagtgt gacaaggact atgtacaggc 120000
ttatgagcgg atttatgctt ttgcggaatt gtttagtatt cctctctgta cagattgtgt 120060
agagaagagt tttgagattc agagtataga taacgacttt gaaaacatag ctggtgttga 120120
ggtccctata gtccgtgagg taacactatt tccagcttcg tattctcttt tagggacccc 120180
gatatggtta gatacgatgc tctcagcatc aaaagaactt gtggtcaaaa aagtcatggc 120240
cgaagtctcg aaagaacgtc taaagatctt agaagaagaa ttacgagccg tttcaattcg 120300
agtcaattta tttgagaaga agctcattcc tgaaactacg aagatactca agaagattgc 120360
ggttttctta agtgatcgta gcatcaccga tgtaggtcaa gttaaaatgg caaaaaagaa 120420
gatagaactc cggaaagcaa ggggggatga gtgcgtttaa atatacataa gtatctcttt 120480
ataggacgca ataaggcgga ttttttttct gcaagtagag agcttggtgt tgtagagttt 120540
atttctaaaa agtgtttcat taccacagaa cagggccatc gttttgtaga atgcttaaaa 120600
gtttttgatc atttagaagc cgaatactcc ttagaagctt tagagtttgt taaagatgag 120660
agtgtttcag tcgaagatat tgtctccgag gtccttactt taaataagga aatcaaggga 120720
cttttagaaa ctgtaaaggc attaaggaaa gagattgtta gagtcaagcc cctaggggca 120780
ttttcttctt cagagattgc agagctgtct agaaagacag gaatatctct acgatttttc 120840
tataggacgc ataaagataa tgaggattta gaggaggact ctcctaacgt tttttatctt 120900
tctacagcgt ataattttga ttattatcta gttcttggag ttgtggatct tcctagagat 120960
cgctacacag agattgaagc tccacgttct gtaaatgagt tgcaagtaga ccttgcaaat 121020
cttcagcgcg agattagaaa cagatccgac cgtctttgtg atctctatgc ctatcgtaga 121080
gaagtcctgc gagggctttg taattatgac aatgaacaaa ggcttcatca agcaaaagag 121140
tgttgcgagg acttgttcga tgggaaagtc tttgctgttg cgggttgggt catcgtcgat 121200
agaatcaaag aattacaaag tctttgcaat cgttatcaaa tttatatgga aagggttcct 121260
gttgatcctg atgagacgat ccctacctac cttgagaata aaggtgtagg tgtgatggga 121320
gaggatcttg tacagattta tgatactcca gcatattccg ataaagatcc ttccacttgg 121380
gtattttttg cttttgtgct cttcttctct atgattgtca atgatgctgg ctacggcctg 121440
ctatttctaa tgtcttcgct tctattctct tggaaattcc gtcgtaagat gaagttctct 121500
aaacatctct cacgcatgct gaagatgacc gctattttag gtcttggttg tatatgttgg 121560
ggaacgacaa caacttcatt ttttggaatg agttttagta aaacgagtgt gtttagagaa 121620
tactctatga cgcatgtctt ggctttgaaa aaggccgaat actacctgca aatgcgtcct 121680
aaagcctata aggaactcac gaatgagtac ccctcgttaa aagcgattcg tgatcccaag 121740
gccttcttgc tagcaactga aataggaagt gcaggtatag aatctcgtta tgtagtctac 121800
gataagttta tcgataatat ccttatggaa ttagcgctgt ttattggagt cgtacacctt 121860
tccttaggta tgttgcgcta tcttcgttat cgttattctg gcattgggtg gattctcttt 121920
atggttagcg cctatcttta tgtgcctatt tatcttggta ctgtatcttt gattcattat 121980
cttttccatg ttccctatga attaggagga caaataggat attatggcat gtttggtgga 122040
attgggcttg ctgttgtact ggcaatgata cagaggagtt ggcgtggagt tgaggaaatc 122100
atttctgtga tccaagtgtt ctctgatgtt ctctcgtatc tccgtatata tgctttagga 122160

cttgctggtg ctatgatggg agccacgttt aatcaaatgg gagcaagatt gcctatgctt 122220
cttggttcta tagttattct tcttggtcac tccgtgaata tcattctttc tattatggga 122280
ggagtgattc atggacttag gttaaatttt atagagtggt accactacag ttttgatggg 122340
ggaggtcgtc ccttacgtcc tctgagaaag attgtctgta gcgaagatgc tgaggcttcg 122400
gggattcact tagataataa ttcaatagtt tgataaactt cccttgcctt taagagagga 122460
acatgaaaga aatcttgtca agttcgtaat tatttaaagg tatttgaagg gagcacatga 122520
ggtaagtatg attgatatgt ctgttgttgg gcctgctttg gttttaggct tagctatgat 122580
tggaagtgct ataggatgtg gcatggctgg agtcgcttca catgcagtaa tgtctcggat 122640
agatgaagga catgggaagt tgataggaat gtcagcgatg ccctcatctc agtctatcta 122700
tgggtttatt ttgatgttgc tgatgcaagc agcaataaaa aatggaaccc tatcgccagt 122760
aggagggatc gctataggtt tatctgtggg agccgccctt ttagtatctt ccgtgatgca 122820
aggcaagtgt tgtgtcagcg gaattcaagc ttatgctcga tcttcgtcaa tatatgggaa 122880
gtgttatgca gcgattggga ttgtcgaatc tttttcattg tttgctgttg tttttgcgct 122940
actactactc taaacttgta tttgggctta cagttctgtt agccgcaata agtgtgattt 123000
gcttattggg ttgttcagaa ccttcattat cttcttttac agaatacgta ggtccagagt 123060
atagtgcagc agcccaactc agtatcgagc agagttgtca tgatgaggtg tatggacagc 123120
aggttgtagt gacctggagt cttccctcac gtatgaggaa atgccttccc gtgactttgt 123180
atctctgggt atattatggt aatggcaagg tagagaaatt gacctatgag gtcaatcaaa 123240
gtgcggggta tcgagtgtat tgcctcaagg gactagaata caaagaactc cagggcatta 123300
tctcctatcc gttgcgttat gtagcgggaa tcaagagatt gtgagtaggc gtcaccatct 123360
ttggatggag gttatctctc tggattctcc ttaataaaaa atatcaaatc ataaacatgc 123420
cctattttta gaaaaagcag cataaagata ataaaataga actatgctac ttgctctaag 123480
tttacagggt atttcttagt agagatcaca aatttggatt aagaattatg acaacagaag 123540
attttccaaa agcatataac tttcaggata cagaacccga gttgtatgtg ttttgggaaa 123600
agaatgggat gtttaaggct gaagcttcga gtgataagcc tccatattct gtaatcatgc 123660
cgcccccaaa tgttactggg gttttgcata tgggccatgc tttggtcaat acccttcaag 123720
atgttcttgt tcgttacaaa cgcatgtcag gatttgaagt ttgttggatt ccaggaactg 123780
accatgcagg aattgctacc caggctgtag tggaaaggca tctccaagct tctgaaggca 123840
agcgtcgtac ggactatagc cgagaagact ttttgaagca tatttgggca tggaaagaaa 123900
agagcgaaaa agtcgttctc tcccaactgc gacagctggg gtgttcctgt gattgggata 123960
ggaaacgctt tactatggag ccgcttgcga atcgtgcggt caaaaaagct ttcaaaaccc 124020
tatttgaaaa tgggtatatt tatcgtgggt actaccttgt aaactgggat cctgttctcc 124080
aaaccgccct ggcggatgat gaggtggaat acgaagagaa agatggatgg ctctattata 124140
ttcgctatcg tatggtaggt tctcaagagt ctattgttgt agcaacaaca agacccgaaa 124200
cttcattagg agacactggg atcgcagtgt ctcctaacga cgagcgctat gcatcatgga 124260
ttggtgcgag cgttgaagtg ccttttgtaa atcgtcagat tcctatcatt ggagatgctt 124320
ctgtagatcc tactttcgga acaggagctg taaaagtgac tcctgctcat gataaggacg 124380
attatcttat ggggaccaac catcatcttc ctatgattaa cattctcacc ccctcaggag 124440
gaatcaatga gaatggtgga ccttttgctg ggatggctaa agagaaagca cgcgaggaga 124500
tcctcattgc actagaagaa caggggttat ttgtaaggaa agagccttat aagcttcgtg 124560
tcggtgtttc ttatcgatct ggagctgtaa ttgagcctta tctttctaaa cagtggtttg 124620
tctctgtgtc agagttccgt ggagctttgc gagagtttgt agaaagtcaa gatattaaga 124680
ttttccctaa agactttgtc aaaaattact tgtcctgggt caaccacctt agagattggt 124740
gtattagtag gcagctgtgg tggggacatc gtattcctgt ttggtatcat aaaaatcatg 124800
acgaacgggt cctttgttat gatggagagg gcattcctga agaagtcgct caagatcctg 124860
attcttggta ccaggatccc gatgttctag atacctggtt ctcttcaggc ttatggccac 124920
tgacctgctt ggggtggcct gatgaaaatt ctccagattt gaagaaattt taccccaccg 124980
ctctattagt tacagggcac gacatcttgt ttttctgggt aactcggatg gtgttactat 125040
gttcttcaat gtcaggggaa aagccttttt cagaagtttt ccttcatgga ttgatatttg 125100
ggaagtctta taagcgttat aacgactttg gtgaatggtc ctatatttct gggaaagaga 125160
agctagctta tgatatggga gaagcgcttc ccgatggtgt tgttgccaaa tgggaaaagc 125220
tctctaaatc caaagggaac gttatcgatc ctttagagat gatcgctact tatggtaccg 125280
atgcggtacg cttgactttg tgttcttgtg caaatcgcgg agagcagata gatcttgatt 125340
acaggctatt tgaagaatac aagcactttg caaataaggt ttggaacgga gctaggttta 125400
tctttggtca tatctcagat cttcagggca aggatttgct tgcaggtatt gatgaagact 125460
ctttagggct tgaagatttt tatattttag atggttttaa ccaactgatt catcagcttg 125520
aggaggctta tgctacctat gcttttgata aagtggcaac tttagcttat gaatttttcc 125580
gtaatgatct ctgttccacg tatattgaga ttattaaacc cacactcttt ggtaagcagg 125640
gaaacgaggc ttcgcaatct acgaagcgga ccttacttgc tgttcttctt attaatgtat 125700
taggagttct tcatcctgta gctcctttca ttacagaatc tttattttta agaattcagg 125760
ataccttagg agcccttcct gaaggagatg gggatgcatt tacaggtcat gctttacgta 125820
tgctacgttc tcgtgcttgt atggaagctc cctatccaaa agcttttgat gttaagatac 125880
cccaagatct tagagaatct tttactttag ctcaaaggct cgtttatact attaggaata 125940
tccgtgggga gatgcaactg gatccgcgtt tacatctgaa agcttttgtt gtttgttctg 126000

atactaccga gattcagagc tgtatcccca tacttcaggc attaggaggg ttagaatcta 126060
tacagctcct agataaagag cctgaaaggg gcctctatag ctttggtgtt gttgatacta 126120
tacgcctggg gatttttgtc cctgaagagc atcttcttaa agagaaaggg cgtttagaaa 126180
aagaaagagt taggttagaa cgagctgtgg agaacttaga gcgcttatta ggagatgaga 126240
gtttttgcca aaaggcaaac ccgaatcttg tagttgcgaa gcaagaagct ttaaagaata 126300
atcgtataga attacaaggc attcttgata agcttgcatc gtttgcttag acagagagga 126360
ccaacgatct ttggagcgct atgatattgt tagaattatt ggaaagggag gcatgggtga 126420
agtctatctt gcctacgatc ctgtatgttc tcgtaaagta gctcttaaaa aaattcgtga 126480
agatcttgca gaaaatcctc ttttgaaaag gaggttttta cgagaggcaa gaattgccgc 126540
tgaccttatt catcctggtg ttgttcctgt ctatactatt tacagcgaga aagatcctgt 126600
atactacacg atgccctaca tagagggata tacactaaaa accttactga agagtgtatg 126660
gcaaaaggaa tccctgtcta aggaattagc agagaaaact tctgtagggg catttctttc 126720
tatctttcat aagatctgct gcactataga atatgtccat tctcggggca ttcttcatcg 126780
cgaccttaaa cccgataaca tcttattagg tctttttagt gaggctgtaa tcttagattg 126840
gggagcagca gttgcctgtg gagaagaaga ggatcttctt gatatagatg tcagcaaaga 126900
ggaggtgctc tcttcaagaa tgacaattcc aggaagaata gtagggactc cagattatat 126960
ggctcctgag aggctcctgg gccatccagc ttctaaaagt acagacattt atgctttagg 127020
agtggttctt tatcagatgc tcactctctc ttttccttat agaagaaaaa aaggaaagaa 127080
aatagttctt gacggtcaga gaattccaag tcctcaagag gtagctcctt atcgagaaat 127140
ccctccgttt ctttccgctg tagtgatgag aatgttggct gtagatcctc aagagcgcta 127200
ttcttcggta acagagctta aggaagatat cgagagtcat ctgaaaggga gtcctaaatg 127260
gactttaacc acagccctgc cacctaaaaa atcttctagt tggaagctaa acgaacctat 127320
tttactttct aagtattttc caatgttgga ggtctctcca gcgtcatggt acagtttagc 127380
aatctctaat attgagagtt tttctgagat gcgcttggag tatactcttt ctaaaaaagg 127440
cttgaacgaa ggctttggta ttttacttcc cacgtcagaa aatgctttag gggagatttt 127500
ttaccagggg tatggctttt ggctgcatat taaggagaga accttatccg tgtctctggt 127560
gaaaaatagc ctagaaatcc agaggtgctc tcaagatttg gaatctgata aagagacctt 127620
cttgatagct ttagagcagc ataatcatag tttatctttg tttgtcgatg gtacgacttg 127680
gcttatccat atgaattatc tgccaagtcg tagtgggcga gtcgctatca tagttcgcga 127740
tatggaagat atcctggaag atataggcat ttttgaaagt agtggctctt tgagggtcag 127800
ttgtcttgct gttcctgacg cttttcttgc tgagaagtta tatgatcgcg ctttagtgct 127860
ttaccgaagg atcgcagaat ctttcccagg acgtaaagaa ggttatgaag caaggttcag 127920
agcaggaatt acagttttag agaaggcctc tacagataat aatgaacagg aatttgctct 127980
agccattgaa gaattctcaa aattacatga cggggttgct gctcccttag aataccttgg 128040
taaggcttta gtatatcaga gactccaaga gtataatgaa gaaattaaga gtttgctatt 128100
agcattgaaa cgttattcgc agcatcctga aatctttagg cttaaagacc atgtggttta 128160
ccgactccat gagagctttt ataaacggga tcgccttgct ctggtgttca tgattttagt 128220
attggaaata gctccccagg caatcactcc agggcaggaa gaaaaaatcc tggtttggtt 128280
aaaggacaaa tctcgggcta ccttattttg cctcctggat cccacggtct tagagctgcg 128340
ctcttctaaa atggaattat ttttaagtta ttggtctggg tttattcccc atctcaatag 128400
tctatttcat agagcttggg atcaaagcga tgtgcgagct ttgatcgaga ttttctatgt 128460
tgcttgtgat cttcataaat ggcagtttct ctcttcttgt atcgacatat ttaaagagtc 128520
tcttgaggat cagaaagcca cagaagagat tgttgagttc tctttcgagg atttaggggc 128580
atttcttttt gctattcaga gcatctttaa caaggaagat gcagagaaga tctttgtttc 128640
taatgatcaa ttatcgccaa tccttcttgt ttatatattc gatctttttg caaatcgtgc 128700
tcttctggaa tctcaaggag aggctatttt tcaggctttg gatctcatcc gaagtaaagt 128760
tcctgaaaat ttttatcatg attacttgcg gaatcatgaa atccgagcgc atctttggtg 128820
ccgcaatgag aaggctctaa gcacgatttt tgaaaactat acagagaaac agctaaagga 128880
tgagcaacat gaactgttcg ttctctatgg atgttacctt gctcttatac aaggtgctga 128940
ggcggcaaag cagcattttg atgtatgtcg tgaagatcgc attttccctg cttcattatt 129000
agctagaaat tacaatcgtt taggtcttcc caaagatgct cttagctatc aagagcggcg 129060
tttgttattg cgacaaaagt ttctctattt ccattgtctt ggtaaccacg acgagcgtga 129120
cttatgccag actatgtatc acctcttaac cgaagaattt cagctttaaa tgatttgtgt 129180
atggtctcag gatcttagat tctaagttct taagaatcga gttcttcatt gcagatatac 129240
tttttcagca acgagtcctt acttgcatag atatctttgg gagatcctga aaaaagaagc 129300
tttcctcctt gtttcccaga tcccgggcct atctctatga ggtagtccgc agattttaac 129360
aacttcacat cgtgatctat gtagattacc gagtggccgc tatttatgag ggagcgaagt 129420
ttttctggaa gatgttgttt tttgattgga tctagagaag aaaagagctc atcaatgaga 129480
aatagggtgg gagtctctgg agtttgatag agaaaataag cagttttcag tgctgttttt 129540
tcacttacag ataaggaaga gagtttttgg ccgataggaa gatagccgag tcctatgtca 129600
agaagtgctt ttagaggttt ttgtatcttt ttaataaagg gaaatcggag ggctacagtt 129660
tcaatcggag tatgcaaaag ttctccgaaa tgcttgcctt cataaaggac ttcctgagca 129720
agaggttgga tacgaaatcc tgagcaggta gggcagggac gcttttctaa agcgtaaaaa 129780
gcccgatcta tccattggta cccaagtcct tggcaatccg agcattgtcc ttgttttgta 129840

tttgtactga acatcgtaga tgaaatattc agggctttgg cttgtgttag cgaagcataa 129900
aaagctctca aggaaggagc aatatcgaaa taggtgctga tatcagagcg ttgcgatgaa 129960
gctattggat gagagtcgat cactacaagg tcggaaaatg ttgtagttcc ttttgctatc 130020
aggagctcag cttgtttttt aaatccttct aaaagtagag aggttttccc agatcctgaa 130080
actcctccaa tggcaaccaa agcatggaga ggagccgata ctttcaaatt ctgaatatgg 130140
tgtatcgata gatttacctt taatgtacga tcggatcctc gggtatggtt tgccttactt 130200
atagaaagtg gagccttggg acaaacctca gtttggggaa cgttagcgtg taggtctaca 130260
gatgggcaaa cctcagtatc agaatccatg agaaatcctc cttgaggtcc agatcctgga 130320
cccaagaaga tcgcgtgatc ggcatggggg attaaagaac aggaccgatc ggtagcaatt 130380
actgtgttgt tatttgctac gagctcttta agtagctgaa ctatggtagg aagatcctgg 130440
gggtgcagtc cagaaagagg ctcttcgaat aagtagacga tgttggtaag atttatagag 130500
atctttttgg caaggtgtaa acggtagttc tcaccatcac tgagagtgtc ttgcctttgc 130560
ccaagggtaa tatagctcag gcctacttta ctaatgaagg taaggcgatt catcaaatct 130620
tggattatgc ttcttgtatc atcggttcct atagtgttta ggaaagattc taagaatgtg 130680
gcatcttcct gataaatatc caaaagcgag gtattgttga ttcgcacata gttggcataa 130740
tcgtttagtc ctgagccttt gcaggcagga caggaggtaa gagcaagcag aggttttatc 130800
aggggagagt cagattcagt atctagctgc tccataagta gtgcgttcat tcctggaaac 130860
tctgaggaac ctcggcaaaa atttaggaac tctttcgtag ttagcaactt tagtggttga 130920
gaggcattct catctttaag cagcttttgt acaggcttca tatagctctt tggaaagaat 130980
aaactaaaaa actctaaagg agtgtagtga gcaatttttt ctttatgctc ttctagggag 131040
atttttagga tttcgccacg acctccacat gtaaggcaac gaccctcggg atggtcagaa 131100
gnaagtagtt gatgtgtaat ctcgggatag agtctccctt tcttatcttt ccaccctaag 131160
gaatacgaga gttttcgttg ctttttctga gagatataga tccagatttc tgaggataga 131220
gaaaaagcta ccgagattgc ggataataaa gaggaactat tttcggggga tacttttgta 131280
tgttgtatga ctatagcagg ttctatgaga tttagaggca gcctctcatc taaatcatag 131340
aggttcccct cagaatataa ctttatgaat ccctcttttt gtttttcttg aaggaagatt 131400
tccagatcag aaccaagagg aatcggagag gttatagtga cgtaatcgtc tttatagctt 131460
ttgagtaggc tatcgatgat ggtctgaggt gtagttttag agagtttttc ttctgtaagg 131520
ggagaaaaag gttctcctaa gatggcaaag agcttttcca gaccattgct taatccgaga 131580
gccgaggcta ttgtatggta cgagcgattg gaggagctac attttcttac cgaaatgact 131640
ggagagagtc cttttacctc tcctacagag ggtagaggag tctccttaag cagaccctgt 131700
ctaatgtagg gagggaagag ctcggcataa gcgatatttc ctgatgcata gagtatatca 131760
aagactaaag aatgtttccc tgaggctcca ggacccgcaa ttgctattag ggagtttctc 131820
ggaagagcaa gatctatgtg tttgagatta ttttgatagg catccttgat aaggatatca 131880
caagatttag gagaggatgg cggttcagat tttactaccg gtatgtctag agaaccttca 131940
atataggggg ccaacgcttt tgctgttgga gtatttagtt ggataagatc cttaggggtg 132000
caggatgcca ggaggtatcc tccgagatct cctccttcag gacctaattc caaaacataa 132060
tcacaaactt tgacaacgtg catgttatgt tcgataacaa ggactgtgtg ccctagatat 132120
gtgagggata gaaggacctc gatcagtgct tggatatcat gagtatgaag gcctgtcgta 132180
ggttcatcta ggacatagag tgtttgctta ggagaagcaa agagaagctc gtgagcgagt 132240
tttagccttt gaatttcccc tccggataat gtggaaagag gtcttcctaa gggcaggtaa 132300
tctaggcgta gggaacatag agcatggatt ttttcatgaa ttttaggatg tgaaatgaaa 132360
aatttttctg cttcgtacgc tgtcatatct aaaatatcag cgatgttctt cccttcatag 132420
aggatttcca atacttctga gtgataacgc ttcccttggc attcagaaca ggggatgggt 132480
gtatcatcat cggagatggt catcgttcct aacccctgac actgaataca agctccttga 132540
ggttggttga agctaaaatg agcttttgtc agtccttgac gtaggctgcg aggttgagag 132600
gcgaagagtt cccggatatc atcaaaggct ttaatatagg tcaaaggtat cgaacgctgt 132660
gagcgtcctg gaagatctcg ggtaatgtga atcaagcggc ctatgcatcc ccactcaaaa 132720
tgcagatttt tagggttctc ttgtttcaag aagctttcta tagcaggcac taacgtatta 132780
ttaattaaag aggatttccc cgatccagag actcctgtaa ctccgattag ccgagctaac 132840
ggcagacgaa tagaaagatt tttaaggttg tggatcgttg cttctgttag caagagccaa 132900
gatgtgggag cttcccggga ttctggaatg ggaatggtaa gctcttgacg caagtatttt 132960
gctgtcagag atgaggagtt catgaggaag tcctcaggct ttccattaaa gaggacctcg 133020
cctccgaaaa ttcctgctcc agggccgatg tcaataatcc tatccgcaag agaaatcatc 133080
cgttcttcat gctcaacaag aatcaccgta ttgccttgat ctcgtagctt tttaatgaca 133140
ccgatgagct tttcagtgtc ttgtggatgc aagcctatgg agggctcatc taggatatag 133200
gtaattccaa aaagttctcc tcctaggtgt tttgctattg ctgtacgttc ttgttctcct 133260
ccagaaaggg tagctaatgc gcgatttgga gtgaggtagc ctagccctaa gtcaataaga 133320
aaagagagcc tttgctttag tccctgcagg atttcttgaa tagagagaga aggagatttt 133380
accttagaaa aaaatacgtg ccagttattt agagacatct gttggaattc agtgaatgtt 133440
tttccttccc aagtagctac ggaagcgtag tctcctaggc ctgtaccttt acatagggaa 133500
caggaatgtg ctgacatgcc tttggagaga taacgtgagg gcttcgtggt ataacgaact 133560
ttatctccta tatcgttaag tacacctctc catactttat aggtgagatt cttttttcct 133620
aaagtttgat cgaagagtcg tacaggaaga accaagttat tttttcccct aagaaaaata 133680

ttttggattt ctggggaaag atctttccat ggagtttcta gattgaaatt taaggcatca 133740
gcaagagctt ggtatatagt atgatagaga taggaagaac aatttccagc aaagctacag 133800
caattctctt taatcgaaag attttcatcg ataagaaggg gattatctat ggaaataaag 133860
atcccggatc cttgacaaag agagcaacgg ctctccaggg catgaggaga aaataattgt 133920
tgagttagag gggtataggt gacgtcatcg atctgttgct ttgtggagaa tgtcatgagc 133980
tcttcgtcac taagaactga gcaatgaccc tctccgaatt ccaaagctgt gaataggcta 134040
actttgagcc ttgcaatatt attttcactt ttgattagag tatcaataac aatatcaaca 134100
gagcagtctt caggaatccc tgaagttagg aaggagtaaa tggggtggat cgtgccgtta 134160
caacgtactt ttgtaaatcc ctgttgtgca tactcatgga ttgcagcaat atctttacgt 134220
agcagaggag ctaaaataga gatctgtaca ccttcagaga gctccataat ggtactaaga 134280
actttctcct tgctgtagag atctaagact tctttagtct taggatctcg agcctgtcct 134340
tctagcgtga agagaagagc aaggtgcgaa aaaagttcag tagtgctccc gacagtcgca 134400
tgactatagt gtgaaaagtg gttctgtttt attgctattg ttggtgagag accatgaatt 134460
tcttctactt taggattggg cagcgtcgtt atcgtagtag cgaagaatgt cggaagtgtt 134520
gaaatatagc gttttcttcc agcagcatat agggtatcaa aggctatcga ggattttcct 134580
gatcccgaaa ctcctgtgag tagaacgatt tcctcggagt taaaatggat agaaacgttt 134640
tttagatttc taactttgat cccagaaaca tatacaggaa gtgatttcat aaagaattct 134700
cgtaatatac ttagaaaggt ctcttaccta accttgagaa aagagtcata tccgcactga 134760
tatcttggga tttcaagtac aaattaaacc gcaatattgt atattcctgc aagtatcctc 134820
cctctcaaga gtttgagtta ataaagagaa ttttttaata ttttttcaaa aaagaatata 134880
aaatatttca ttataccatg agtttttcat tgaatagaca ataggacagt atgatcacac 134940
gcactaaaat tatttgcact atagggccag caacgaatag tccagagatg ttagcaaaac 135000
ttctagatgc tgggatgaac gtagcaagat taaatttcag tcatgggagt cacgaaactc 135060
atggacaggc tattggattt ctcaaggagt taagggagca gaagcgggtt cctttagcaa 135120
ttatgctaga tactaagggg cctgaaattc gtttagggaa tattcctcag ccaatttcgg 135180
tttctcaggg acaaaagctt cgtctggtaa gtagtgatat cgatgggagt gctgaagggg 135240
gagtgtctct ctatcctaag gggatatttc cctttgttcc tgagggtgct gatgttttaa 135300
tagatgatgg ctacattcat gctgttgttg tctcttcaga ggctgattct ttagaattag 135360
agtttatgaa cagtggcctt ctcaagtctc ataaatcttt gagtatccga ggtgttgatg 135420
ttgctcttcc ctttatgaca gagaaagata ttgcggatct taagtttggg gtagagcaga 135480
atatggatgt ggttgctgca tcttttgtgc gctacggtga agatattgaa actatgcgca 135540
agtgtttagc agacttaggc aatcctaaga tgcccatcat tgcaaaaata gaaaatcgtt 135600
taggggtaga aaatttctct aagattgcca agcttgcgga tggaattatg attgctagag 135660
gagatttagg aatcgagctt tctgtcgttg aagtcccaaa tttgcaaaag atgatggcta 135720
aggtttctag agaaacaggt cacttctgtg tgactgcaac gcagatgcta gaatctatga 135780
ttcgcaatgt cttacctaca cgagctgaag tctctgatat tgccaatgca atttatgatg 135840
gttcttcagc agtgatgttg tcaggggaaa ctgcatctgg agcccatccc gtggctgccg 135900
tgaaaatcat gcgttctgtg attttagaaa cagaaaagaa tctctcccat gattcattct 135960
taaaattaga cgaaagcaat agcgctcttc aggtgtcccc ctatctctca gccattggat 136020
tggcaggcat tcagattgca gaaagggcag acgccaaagc tcttattgtt tatacagaat 136080
caggaagttc tccgatgttt ctctctaaat atcgtccgaa attccctatc attgccgtga 136140
ctccaagcac ttctgtttac tatcgcctag ctttggaatg gggggtctat cctatgctta 136200
cccaggaaag tgatcgcgct gtatggagac atcaggcctg tatttatggc atagaacagg 136260
gcattctctc taattatgat cggattcttg tgcttagcag aggagcctgt atggaagaaa 136320
caaataatct taccctgaca atagtgaatg atattttgac tgggtcggaa tttcctgaaa 136380
cctagaattt ttgctttaaa atccaggact tcgcaaattt ttcgagaata tacagatgtt 136440
ttcgtaaata tgaattaggg cttttactcc actgtaagtc aggccccttt cgacttcacc 136500
ttgaagtttt ttctctcttt atctttaaga tttttagaat agaagatcct caaagagttt 136560
tttagaggag cttgggggtg gtctagagat ttttctagga atcttttaga gtacacagca 136620
caagaacctg tttttctaaa atgcttgtat aaatgctgta gattgttagt gagatcaaaa 136680
atagcaggat agcaattcgg tagagctaga atgtcttgat catttcggag ttggatcaag 136740
gagtactctg ggaattgttc ttgaagttct tcaagatgat ccgcatttcc tagtgttaga 136800
tgaagagtgg ttcctgaaaa gcattctgct agagctttca gaaaagaaaa gtgtgaggag 136860
acttgatcta caaaaactag aatatggcta tagcggtact tcttttttat tacgttagag 136920
atctttctct tccacctttt atgaatccac atccattgtt caggctgact ggcgatccct 136980
ttttctaaaa atcccatcat ctgatccata aggatagcca cggattcttt catagggagg 137040
cttttattag catacagctt ggcactcgga atcacttcga agcctttagc ttggcgagaa 137100
acattaacag caatcacagg aaaacctgtt ttataagcta atagtgctgg agatgtcgtt 137160
gtgaatgctg gagagccaaa gagaggatac gtgtatgaag acatcaacaa ggcttgatct 137220
ccaacaatcc ccacgagttt cccttgattc agagcttcta tgccctgttg gattccgttt 137280
tttgggggta caatcttacc tttgaaaact tctctaagag caaagatttt cttgctgagc 137340
ctttgatttt ttatagcctt agcaaaggcg attccaggat agtttttagt gatataaaga 137400
aaaggaagtt cccagtttgc ctggtggcca caaaataaaa taaggccctg cttctcttgt 137460
agattcttaa aagtttcctc taaatcttca ttggaaatga cctcttcaga agaaaaacct 137520

ttggggtttc gtgaggatgt cacgattgta atgagtttgt ctatatttcc gacaagttgc 137580
tcgattgcga gtaattctaa gagtgtaatt ataagatgct gcaaagattg acgagctatt 137640
ttataacgct catcaaatgt tttttctgga aacgctaatg ctaagtttgt gagggctgtt 137700
tttcgataat cgctgatgat ataaaaggct agaaatccaa aaccttttcc taatcctgtt 137760
aaaaaagatc ttggggtatg cctgcataaa gcaataatac cagagactag gtaatacaga 137820
ggggcttcta ggattgttct cttgatctga tggaattttt tgcccacaag ctaatttgac 137880
tttcgcaaat cactttaaat aatactattg tgcttcttac tatctcaaga tttctcgttt 137940
tgcagcgaag acgctccaga aaggaacatg cttaacagta tcgtaactaa gagaactagg 138000
acagcagcaa cattactcat tccgaaagtc atcccagaag ctccaagcac tccagtacaa 138060
atcaaaatga tcagtataaa agaaacaata gcagtaagag ctaaaagtcc tgcagacact 138120
gtcgctacat ttgctttgga ttctgagcta tcagaacaac aacaaactgt gttgattgca 138180
gcttcaaagc cctggcccaa gcaatctatt aagcacataa agtttcccct aactaaattt 138240
tagtgagtct gcactaccac tcttttttat ataatttagt tgtttccttt gtttttgtct 138300
ggatcacaac tttattattt ttgatttctg caataagatt taaaggttca ccgtctgctg 138360
taaaaagtgc aagctttctt aaatattctc ggaatctatg acgaattcca gcctcatcaa 138420
taagggctat atcgtgtaaa attccatgtt cttgggctat aatagtctga agaaaagtat 138480
tttctaaagt tttttcatct aaaaattcta tagcccagtt gatcgaagaa gggtcttgtg 138540
gtgagggcat cacaattgtt aagaccatgt tgttctttac gacatctaaa aactgtctac 138600
ttacatcctt aacatataaa ggaatattta tttgatgaat tccatgattc agaataatgg 138660
gagggacagg atctaagctg tactctagag gattcatcgt ttgaatgaag gttacaggaa 138720
agaatagaaa gaccggtaaa tttaaattta ggggaatgca ttcccttttt aaaaataaaa 138780
ggcgaagaaa atctgcttga gggtcattga gatccatgaa ggtattttca aagggaatca 138840
aaatttcttt ccattcttta ggaatgggga aaataatttc gtcatgactt ccctgagcaa 138900
tgcgatttct ttctaactct tcgaacgaaa ttttattcaa attaaaggtt agctcaagac 138960
cttgctcttt caaagcattg atatattctt tggggccact gactttttga ttcaagtact 139020
tcggccagac atccaaatac tcatatcctt tcgggggact ccctataggt ttcgttatag 139080
ttaataaaat gtcttcggtt acatattgtg taagtcggac aaaaatatcg ttagcgtcaa 139140
cgctatggat gtctttgcgt atgttgatct catgatctac agagacaagg ttatgcttat 139200
ctatagtggc aatccaactt tccgtatggt ttgctgcact gatcactacc tctaaatttg 139260
aaggacggag gtcttgaacg gtatttttat tcccagtgat tgttaaagag acttttttat 139320
tcaggaatcc gcttttttgt agtccaagaa cggtttggtc tggatgtagg tctacgatgc 139380
gcacagggac atttgtgagt gttcgcgtga tggtaacact ttgtcctacg aggatccaaa 139440
tgatgatggc aaaacctaag gaaacaactt ttctaggcca atgccgaata aagagttgag 139500
ataaaaattt tatcatcgtt tccaaatcca agagaataaa ggttttcttt tgtgttcttt 139560
aggggaaaga atactgcgga gtaccgcttt gaatctatct atttttactc cgcgtgttag 139620
aaggccgtct ctagacaaag agacacttcc attttcttca gatactgtga taattagagc 139680
atcagatcgt tggctagctc ctagagctgc gcgatgcctt gtccccatgg atcgggaaag 139740
ctgcgtcgta tcatgagcta gtgggagaac gacgcgagca taggctagaa tgtctcctct 139800
tagaatgacg gcaccatcgt gcaatggaga tgaaggttcg aaaatcgtct ctaaaagttc 139860
ttcagagaaa gttgcattga ttttcaccga agaaaaactt aggtattcat cgaaagaatc 139920
tttgttttct aaaacaacaa gagccccgat ttggcgttct gatagctgat aaatactggc 139980
agctaattgc tctacgaact gctcttgagt atctatgaag aattttttcc catgaaatcg 140040
tatacgagag agagccaaac gaatttctgg ttggaaaata ataaagacca cgatggcagc 140100
gatattgact acgtggagca tcaatctacg gatgataggg aggtggagtt tatcggctag 140160
gacaaataga aagagaaacg caagcaagcc aaagacaaca tccatagctc gggtgcccca 140220
gaaaaatttt aataggtagt ttaacattac ccaaattaaa atgatttcta gcaaaggtgt 140280
tgtataataa gtaatatcaa agggcatagt tttactaggt ccttgggtag tatactgaga 140340
aaagttgcac aaagtgctcg gctattgtcc taggaaaggt aacctattat cttagcatac 140400
gaacaaaaag gttaacagca aagtattttg tagttgcaat actttgtttc atcgttcaca 140460
ttacagtttc taattttata tacaattctg gaagtttatg gatgcgctta tcttatctag 140520
aatacaattt ggattgttta taacttttca ttaccttttt gtgcctctga gtatgggttt 140580
gagcatgatg cttgtgatca tggaaggcct ctacttggtt acaaaaaagc aaatttataa 140640
gcaaatgaca tggttttggg ttgggatttt tgccctaaca tttgttcttg gagtcgttac 140700
tggaatcatg cagatatttt ctttcggttc taactgggca aatttctcag aatatacagg 140760
aaatattttc ggcaccttat taggtagtga aggtgttttt gcttttttct tggaatcagg 140820
atttttagga attttgttat ttggtcgcca caaggtctct aagaaaatgc atttcttttc 140880
tacgtgcatg gtagctttag gagctcatat gagtgccttt tggattattt gtgcgaattc 140940
ttggatgcag actccttcag gttacgagat ggtgatgcat aaaggaaaac tcatccctgc 141000
tttaacctcc ttctggggag tggtcttctc tccaacaact atagatcgct ttattcatgc 141060
agtcttagga acttggctgt caggagtttt tcttgttata agtgtatcag catattattt 141120
atggaaaaaa cgtcatcatg agtttgctaa acaaggaatg aagataggga cgatttgtgc 141180
agttatagtc ttagttttac aattgtggtc tgcagatgta acggctaggg gagttgctaa 141240
aaatcagcct gcgaagttag cagcttttga aggtatcttc aaaaccgaag aatatactcc 141300
tatatgggct tttggttatg tagacatgga aaaagaacgg gttatagggc tgcctattcc 141360

aggagcactt tcttttcttg ttcatagaaa tataaaaacc ccagtcactg gtttagatca 141420
aattcctaga gatgaatggc ctaatgtaca ggctgtcttt cagctgtatc acctgatgat 141480
catgttgtgg ggggttatgg tcgctttaac tttgatttcc tggtctgcat ataagggatg 141540
gcgatgggcg ttaaaaccct ttttcttagt cattttaact ttttctgtct tattaccaga 141600
aatttgtaac gagtgtggtt ggtgcgctgc tgaaatggga agacaacctt gggtagttca 141660
aggattatta aaaaccaaag atgcggtgtc tcctatagtg caggcgaata aaattgtaca 141720
atctttggta atatttagct tagtattcat tgctcttctg actctcttta ttactgtact 141780
ttgtaaaaaa ataaagcatg gtcctgaaga ggaaaatgat cttacagaat ttgaagtgaa 141840
atagaggtat ttttatggaa ctttctctaa caagcctttt accacttgcg tggtatgtaa 141900
ttcttggagt tgctgtcttt gcgtattctt ttggcgacgg ttttgatctt gggctcggag 141960
ctgtttatct taaagctaag gaggataaag aacgtcggat tcttcttaat tccataggac 142020
ctgtatggga cggcaatgag gtctggttag tgatcattgt cggtgggtta tttgcaggat 142080
ttcctgcatg ctatgccaca cttctctcga ttttctatat gcctatctgg actttggtac 142140
tcctttatat ttttagggga tgttctttag aattccgaag taaatcggaa tcagtgtctt 142200
ggaaaatatt ttgggatatt atctttattt gttctgggac tgccatcagc tttttcttag 142260
gcacgattgt tgggaatctg atccttggat tgcctttgtc tccagacacc tcttatgctt 142320
ctttatcctg gattttattt ttccgtcctt atgcagcctt atgtggcgct gtagttgcca 142380
gtgcgtttgc tactcacggt tccttcttcg cattaatgaa gacttcggat tctttaaatg 142440
ctaggattgc tcagcaattt ccttatattc tttcgtcctt ccttgtcttc tatgttctct 142500
tcttaggagc aagtttaatc tctattccca agcgttttga tgctttccct acgtatccac 142560
tcttgatttt gctcattgct ttaacgagct gctgctgtgt tgctgctaag acgagcgtgt 142620
ctaagaaaca ttatggtacg catttattta ttctacactg aacttgttgt ctctcattct 142680
gtcggcagct accctaacgt tccctaatat tcttctctct actgtagatc cacagtatag 142740
ttatactatc tacaatagcg ctgttgaaac taaaacgtta aaaagccttt tgattatagt 142800
gcttataggc cttcctttca tcattactta tacgtgttat atttatcgtg tgtttagagg 142860
aaaaactaat tttccctcta tatattgagt ttcatttaaa gagtccgaag ccttcattcc 142920
atgagggtcg gatttagatt aaagagactg tgattgcgta tagatccaag aatgaatcga 142980
agaacagagg atctagaaaa tgaagagctt ccattggaat cctttggaag ctatcattat 143040
acttcgttat gaagcttctt taattccaac tacataataa gcaggaaaag caaaatggtt 143100
gttaggacgt gggagacatt ccacagtgac acgctttcct aaccattgct ctaagttgat 143160
acttgtaccg taaagaaaag caatcgtgtt ttcctgagct tttagtaggt aatccccggg 143220
attgttcttt actacatgag gatacacttc taatacaccc gcaagcactt gttttttctt 143280
ctgttcagcg cgataaaacg cttcttgtgt tagtgcttca gagtggtcat tgccttgctg 143340
catactggcc cagattctga agagagaata ctctaggttt tctcttcctt gggtaagagg 143400
agctgttttt aatgcagttt gcttacgaat atgacgtgaa agtaatgaag ttgtaacttc 143460
agaagaagaa accttaggag tggaacattg tgagcttgca atcgaagtat tttgagattc 143520
tagagattta gaaagatagg catcttggat ttcttctaaa gctttttgta taagcccttg 143580
aattcctgga acatctttaa actcttcgga ttgtacaagg ttgatctttt tataaattgc 143640
ttccagatca atctcattga ggcttttctc aagctctata tgagcaaagt ttaaagcaga 143700
attgataagg tccatggcaa tctttttttg tccctcgcgt tgcgtataca gctcgatggg 143760
tcctttgtta gcaacaaagt tttttgcaac atagaatacg cattgtgagg gcaagaccac 143820
ctctaaccat ttcccatgtg gctcttgaga agctggctgt atttgtgtgc ctcgggagag 143880
tctcacaagt actggagctg atgttgaggg ttctaaacga acattgactt gttcaccttc 143940
aacgacatta tctaaaacaa atgagcggaa cacataacct gtaattcctg gaggcgcaga 144000
aattacgtag tagtctttgc tttctccgat aacagcaaca agatctcctt tagaaaattc 144060
cctaatgatg gtcccatcag tatgaggtgc tagacgcatc cgtacgtggt ttcccttaat 144120
ttctccagta aatgaagagg gaagttgttc tggaaaggat acggattggg aatcggcagc 144180
atagattgct ggtgagttga ttgcagttcc taaagctaaa agaagcatag aaatctggag 144240
cattctcatg cttttctcct acaaatatat ttcgtctaac cgttggttga aaacatcgct 144300
tacagcaaaa aggagattaa cccctagaaa aagaattata ttgacttcaa ggaaaaagtc 144360
aatcggctta ccaaaagctt aattatgaat gcagttctac taagtatata aaacagcttt 144420
tatatgttca cagttgggtt cgccacaagt acagcctatg gggggtccca agtagacgct 144480
aaactgatca ctaggattta agggattcgt tacaatatac aactttatcc ccactttgca 144540
taatgtccca agtgcggaaa gtcagatctt tatcagaaac cgccagggta tcttcttcat 144600
tcatgaccct tccaatttgg cagtgcatac aattgcaatg gggttctggt ctgggtaaaa 144660
gcgttgcatt attccctgaa agtacccgaa tgacatcagc cattttctct aagacgtcgg 144720
taggagcatc aggatgatct ttatgttctg gagtatgttg tagtattgct tcgatgggat 144780
ttgttccaga aaagagaggg gaaattagat ttttaggtaa tacttgaatg tcgtttccct 144840
tagttatctg ctgcaataca ttcataagga cccctacacc tagtttatca tcatcacgag 144900
aatcctcttt ccccgattga gaagtttcta ggtagagaag gtgctcttga aaagcaatgt 144960
caataatcga ctgatctaaa ttaggaatag aaatgatttt gccatcaata aggtggagcc 145020
ttagtgttcc ttggtctttg ttttctcctt cctgggattc aataaatgca atttggctcc 145080
atcttgcaga gatgaatggg ggaatacaaa tcagttgatc attaattttg actttcatag 145140
cgatcttggg ggtgaatcta atcttagcag tttgaggcca ttgtaaatgt atctcttttt 145200

ttttacaata aattagcagc atagggtttt gattgctaaa ttttctaaaa ggcacgtatc 145260
atgtaaatgg atttttattt atgaatatat tttttgcaat agttcctgat agaaaaacga 145320
aagtatggta gttggacctt aacaacacaa tacgtctttt aatgaaaaga agggttttct 145380
ttggaagtta tgttagagtt gttttaggca aaaatttgta tctgataaag aataaaaggt 145440
tcagtaagag aaggtgagga cgcaaatgaa gaaaacaatg gtcattgata caagtgtgtt 145500
catctatgat ccagaagccc ttttttcttt tgaaaatact cgaattatca ttcctttccc 145560
agtcattgaa gagctagaag ccttcggaaa atttagagat gagtctgcta aaaacgcgtc 145620
tcgagcatta agtaatattc gtttgctttt agagaatgca aaaactaaag ttacagatgg 145680
tgtgctctta cctagtggta gtgagttgcg tatcgaggtg gcgccccttt ctaatgatga 145740
taggcgaggg aaacttctta ccttggagtt gctcaagatt attgctaaac gagaacccat 145800
ggtttttgtg actaagagct tgggacgcag ggtgcgtgct gaagcactac aaattgagtc 145860
tcgagactat gaaagtaaac gcttttcttt tcgttcctta taccgtggat ttagagaact 145920
gcaagtttct caggaggata ttgaaaactt ctataagaat ggctacttag atcttcctct 145980
agacgtggtc tcttcgccaa acgagtattt tttcatgtcc gcaggagaaa accattttgc 146040
tttgggtaga tactacgtaa gcgaaggaaa gattatcgca ttaaaggcaa tggataagag 146100
tgtttgggga atcaagcctt taaatacaga acagcgatgt gccttggatt tgttgcttag 146160
ggatgatgtc aagttagtca ccctaatcgg gcaagcagga tctggaaaga ccattttggc 146220
tttagcagct gctatgcata aagtttttga taaggaaacc tataataaag ttttggtaag 146280
ccgtcccata gtccctatgg gaagagatat agggtttctt ccaggattaa aggaagataa 146340
actgatgcat tggatgcaac ctatatatga taatatggaa gtgttattta gcattaacca 146400
gatggggaat tcttcagagg ctctccaagc tcttatggat gctaaaaaat tggaaatgga 146460
agctcttacc tatatccgag ggcgctctct acccaaagct tttattatta ttgatgaagc 146520
tcaaaacctc actccccatg aaatcaagac aattatctca agagctggga aaggaacgaa 146580
aattgttctt acaggagatc ctacacaaat cgatagtttg tattttgatg aaaattctaa 146640
cggactcacc tatctagttg ggaagttcca tcacttggcc ttatatggac acatgtttat 146700
gacacgtaca gaacgttccg aacttgcagc tgcggccgca actatcctat agaaccttcg 146760
atgttataag tgacttttct tcttcggatc tgtaatagca ggagttttca agtctgtaag 146820
gacaatcaac attcttctgt ctgtatttcc gggttcattt ccaaattctt gcatagcggc 146880
aacaagagcg cattttacag cgtgtatcca tctcatgcgg actttattct cttcactatt 146940
aggctttttt ctattttcag gaggcgcgta gatcccagaa gaaattagag ggatttgtag 147000
gaaggttaca cctaaagttt gagctaagct gaagcagttt aggtaggcgt ttttgcagcg 147060
atcaaaggct gcttgagact tgttatgatg atccactgct ttaggcccaa gaagttgtgc 147120
taggtagtgt ggtttacctt gttttcctgt gtggttggat ccatcggcat ttctccacat 147180
gcctgcccga cattcaccct cattaagagg agtcccctgg cgttcaggat ttagaggtcg 147240
ttgcgacagc cccaagaatc gacactgact gctgccgata gaacttgatt tgtacccgcc 147300
ccatctcgac tcattgttct attggctgcg tttacaatca tcatagacat actatcagtg 147360
acaagacgag gttgtgcaat atttcctgac gtcgagacaa aaatcagctt tgattgattt 147420
agtgcccagg caaagatttt ctggtttttt tctgctggtg gccatgggaa cggctcctct 147480
acaggagtcc aattctccaa gagcatctta tgaaacagag atagaggaga aaccatagag 147540
tctggtgacg gtttcttgcc taggatgggg gaagcttcta tacttccagg tacaatgtga 147600
tctgtaggtg taggggagac ctctggtctc tgaggtatgg gtggcttagg ttgtgtcggt 147660
atttctttag gtgttacagg tgggggcttt tttttttatc ttcttgtaac agatcatcca 147720
ggaacggatc tttgaatcct gttggagagg taggagtaga aaatatggcg tctatttgct 147780
gccccatgtc aaggggagta ggtgttaagg ggttttgagg ttctcttggc tctggaggac 147840
ggcagcaagg acaaagatac cgaacgatca actttataat cttaatgact cctaagacca 147900
ggtaatacag agctttaatg ccgaaaattt ctagccaagc ttcttttcta actttaggaa 147960
gttcctcata gatctcagga cagggtaatg ttttacacac tgaagagaag ttccctgtca 148020
ttagaaccat agaatattga agagaactaa ttccttttaa ggttttgata cctataaatg 148080
tactaaatat cggaatatag cttgcaatac gttgtaaatt tgtgcggtgt aagggattca 148140
ctaaaggagt atttcttagt gcggttgagg tttgacccag aggcgtgtgc cgatggctaa 148200
ttataaagac ttcttcatta aaaatgctca taagaatcgc cttataatga aataaatttt 148260
ttcattaaga attataaaac aacttatctt tcttttctat ttctatgaaa tctagaaggg 148320
ttttttaatg gatatttgat ttctttaaag actttaggaa gacattgtct aaatttggta 148380
aaaaagacag attctcctcg ttaagagggg atgagtagca ttttaaaata atttttttaa 148440
ataatggagg tggagagact cgaactctcg tccttgacaa actccctgct aacctctaca 148500
tgcttatctt ctagaattgt ttacattgga ctcccttagc tagaagcctc tatagcagcc 148560
aatgactctc aaaaatctcg aacgaacttc cttgagaatt agagaaagta agttccaacc 148620
agataaatga cggtattttg caagcctctg gtggagctcg cagataccgg gttacctaga 148680
gattatctag ctaacaactt ttgctaatta agcagctagt ctttcctcta ctgagtcaaa 148740
caagctaata atttcgcatt cagccttagg ttcggcattt attgttttgt tggcttttta 148800
ggaggccagc caacgccctc cgcatgcaat taacacttca ttttcaagtc gaaacctata 148860
cacccccaca acaattttag aagctggtta gcttttattc aatcatcgta ttttggtcaa 148920
gttctaagaa cttcttagag atctctagag aggtgggcta tggacgaaaa cctaaaaaac 148980
ctctattgta aatatgtttt catatcaaaa tgttcctaaa ggcaaaagat gacagcagat 149040

gaggtaggga aaaatagctt tgcaaaaaaa gaagaacagg ttttgaagtt ttggaaagac 149100
aatcaaattt ttgaaaagtc tttgcaaaat cgtcagggaa aaaccctata ttctttctat 149160
gacggccctc cttttgctac aggtcttcca cattacggtc acttattagc aagtaccatt 149220
aaggatgttg ttggacgcta tgctaccatg gacgggtact atgtgccgcg acgttttggc 149280
tgggattgcc atggggttcc tgtggaatat gaggtggaaa agtctctgag tttaacagca 149340
cccggaccca tcgaagattt tggtatagca tcctttaacg aagagtgtcg taaaatcgta 149400
tttagatacg ttcacgagtg ggaatactat atcaatcgta taggacgttg ggtagatttt 149460
tcttctactt ggaaaactat ggacgcttct tttatggaaa gtgtctggtg ggttttccaa 149520
tctctatata accaaggatt agtgtacgaa ggtacaaaag ttgtcccttt ttcaacagca 149580
ttaggaacac ctctctctaa ttttgaagca agccaaaatt ataaagaagt cgatgacccg 149640
tctcttgttg taagaatgcc tcttcagaat gattccgcat ccttgcttgt atggacaacg 149700
actccatgga cattgccttc taatatggct atagctgtag gggaaactct ggtttatgtc 149760
cgtattcaag ataaaaaaag tggagagcag tggatcctaa gtcagggatg tgtttctcgt 149820
tggttttcaa atccagaaga atttgtaatt ttagagagtt tttctgggaa agatcttgtt 149880
ggtaggactt atgagccccc ttttactttt ttccaatcta agcgagagga aggagctttt 149940
cgtgtcattg cagcttcgtt tgttgaggaa agtgaaggaa caggagtcgt acatatggct 150000
ccagcgtttg gtgaaggaga ctttttagtt tgtaaggaga accatgttcc tttagtctgt 150060
cctgtagatg ctcacggaag ttttacagaa gaaatacctc aatatcaagg gcaatacatt 150120
aaacatgctg acaaggaaat catcaagttc ttgaagaaag aaggaaggat tttttaccac 150180
ggaacagtaa aacaccggta tcctttctgt tggagaacgg atactccttt gatttataaa 150240
gccgtgaatt cttggttcgt cgctgtagaa aagattaaag ataagatgct tcgtgctaac 150300
agctcgatcc attgggttcc tgaacatatc caagaagggc gttttggaaa atggttggaa 150360
ggcgctcgtg attgggctat cagtagaaat cgttattggg gaacgccaat tccgatttgg 150420
aaaagtgctg atggcgagat tcttgttgta ggatctatcc gagagctaga agaacttaca 150480
ggaactcaga tcacagatat tcataggcat tttattgatg atttgaacat tgtcaaagat 150540
ggcaagccct ttcatcgaat tccctacgtt tttgattgct ggttcgactc tggagcgatg 150600
ccttatgccc aaaatcatta tccttttgaa aatcaaaagg aaaccgaaga ggcatttcct 150660
gcagacttta ttgctgaagg gttggatcag acgcgaggat ggttttatac tctcacagtg 150720
atttctgcaa ttttatttga tcgtcctgca tttcgtaatg ccattgtgaa tgggattatt 150780
cttgcagaag acggcaataa aatgtcaaaa cgtctaaata attaccctag tcctaaatac 150840
gttttagata cttatggagc tgacgcgctt cgtctatatt tgcttcatag tgttgtcgta 150900
aaggctgaag atcttcgctt ttctgataaa ggaatcgagg gtgttttgaa gcaaatcctt 150960
cttcctctaa cgaacgtact ttcctttttt aatacctatg ccgagctgta tggttttgat 151020
ccgaaatcac aagatataga accagcttat acagagattg atcaatggat tttatccaat 151080
ttgtatagtg ttgtaggtaa agttcgtgag agcatgagtc agtatcattt aaactttgct 151140
gtagaaccct ttgtgacctt tattgatgat ctgactaact ggtatatacg tcgctgtcgt 151200
agacgttttt gggaagctga agatactcct gaccgtagag ctgcattttc tactttatat 151260
gaagttctca cagttttttg taaggtaatt gctcccttcg ttccttttct tgccgaagat 151320
atctatcaga agttgaagtt agaaaaggaa cctgaatctg ttcatctctg tgattttcct 151380
caagtcgaga tggataaaat tctccctgat ctagaaaagc gtatgcacga tattcgggaa 151440
atcgtaggtt taggccattc tttaagaaaa gaacacaagt taaaagttcg tcagccttta 151500
gcaaactttt atgttgtcgg gtctaaagat agattgtcgc ttctaaaaac atttgaaggg 151560
ttgattgctg aagagctgaa tgtgaaaaat gtgattttct atgaagaagc tccgagtttc 151620
atttatacta ccgtcaaacc taattttcgt atgcttggga aaaaagttgg atctaagatg 151680
aaagaggtcc aaaaagctct cagtgaactg ccaaacaatg ctatagataa gctgattcag 151740
gaagaaacat gggttttaac cattgatgat agagaaatag ctttggatgg tgatgacgtc 151800
gtgatttgtc gtcacacaga tcctggatat attgcccgta gttccgctct atttagtgtg 151860
attttagatt gccagttaag agaacctctt atagtcgaag gtatagcaag agagctagtc 151920
aataagatta atactatgcg tcgaaatcaa caacttcatg tttctgaccg catcgcatta 151980
agaataaaaa ccacagaggc tgttcatcgc gctttcttgg attatgaaaa ctatatttgc 152040
gaagaaacgt taattatagc ctatgatttt actcaggatt ctgatttcca aggggaaaac 152100
tgggatatta atggacatgc aacgcaaatt gaaattacag ttagttctat agattcttag 152160
agattttcta gaaacaactg aaaaaccata agaatggtta tttcttgtgg tttttctcct 152220
ctttcttagg aaagagtctg cgtcgttttt gatagtagac atatccaatg agagagagac 152280
ccgtcgctaa tgctatccca ctaacaagat aacctgagag tgttgttgga gcagaaactc 152340
cagttaaccg tcccatgcgt ccaataggcc agaatgtaca tagaggagat cctaagagat 152400
tttccatagg aacaaagcca aattctcgac tatccgcact catagggtag ttatctccca 152460
agacgagaac atgaccttta ggaacttgaa taccaaaatt atgtataaac tccacgaatt 152520
ctttaaaatc ttctggaggg agtcccttgt caacaaaagc tatatagggt tgtgtctctg 152580
aagacccctc ttgcttttcc gtttcagaag tcacaaattt ttgcagagtt ggatcattct 152640
ttataaatac aggagaatcc atgatataaa gattcccttg gttaaagaat gcataacggt 152700
taggtaaagg tgcttgcagc ggattcacag gattataaat agaactaaag ttgatcccgc 152760
agttaaaaag ttcaatcact tgcttatcat tgagctgagt aagggggtga gaagatttta 152820
gcttataacg aatctctcca aagccaattt gatacgcttc gcctttagaa tattcataac 152880

aaccatcagg gaccttgggc aggagaattg cataggcttt ggcaattcct gaagtgttaa 152940
tcttgaattg atggtattta tacgcacatc cttgagcaac aataaaacga gaggtagtaa 153000
gattgttccg aattaagtgc aaatgttcct tacgcaaagg aagtaaagtc ttcataggtt 153060
gaatcgcagg cgagagctga tgctcatagt gacgcaacag aggctttggg taggaaaggt 153120
tcgctgtatg gcaaatttct aagtagactt tagttggact tcctggattc ggaagtagat 153180
gggatgttcg tgcctgatgt tctgttaaga tgcgcaccat agcatagtta cccataccaa 153240
aaagatcggc atagctgact ggcgaaagat gaggatcttt taatttatta ggctcgtctt 153300
gatgccattc tttatggtca aagaattgtc catacatgga ggtttgaggg aaaatcagcc 153360
gaccataact ttgattgaac tgcttaaaat ctataattgt tttctgccct tctgtatggc 153420
tgctggtagt gccatcaaag gatatatagg ggacgtgata taagttttct aaaccatgga 153480
cagaaggaaa ctctatgcgt ttacctgcat catcaagacc ataaattttt cctccataga 153540
aatataagaa gtccccaggt cttcccatgc aacgtttaat gtaacgcttt tttcctggaa 153600
tcaatccgaa gtactttgta tcagcatctg ggatagggag gtcgcctaca gtgaaaacaa 153660
caagaccccc gcgagttacg gattcaggat tgaaggcaag tggtttctta gcaaaagggc 153720
aatggagacc aaatgttgtt ttggatacaa gaatccgatc ctgttctaaa attgtaggcc 153780
tcatggatcc tgtaggcact tcataaagtt caaaccaaaa ttgccgaact aagaaggcta 153840
caacaccagc aaaaagaagg gccttgataa gctcataggt tttgcgtccg aaggaattag 153900
gataacggtt ggaaaatgct aatgcttgct gagctaagtc gcttgcagtt tcttgatcat 153960
gttcaaagat agcctcttct agttgttcta gtagttcttg cagttgcttt ttatctgcag 154020
gggaatgggc gagtttttta ctttttaaaa gcttataagt actgcggagg atatgacgac 154080
ttttatttag agaatagtgt tgtttcataa agatactggg gtaagaaagt cagattctat 154140
ctctttagat tgattataga acggctgcat tggatacttt agagctaatg gagactgaag 154200
acctaaatga aaaatcaaaa caatcctcga gctaaattcg taagtacatt ctagcgtatt 154260
agatttttcg atccaaaatt attttgctat attgttagat cttttcattg tgtcttagag 154320
tcaggaagag aatagacata gggagaagtt gccccgtgtc ctatatccac agctcttact 154380
atagaagtcc ctttaggagt ctcgatactc acccataaag gaaacacaga aatgtcaggt 154440
tgggtgaaat acataaggat tcctgtttct gataaggggc ctcgatcttt aggccagagc 154500
ccctgccatg cattcacaga gattgacgtg agtggagccc cttcaaaaga aactttggga 154560
gaccagggct tgttttcttt tcctttagtc ttgattacgt ttgcgggagc tggagataaa 154620
ggaagatcaa agatttgcaa gaaggcagga actgtagtgg attttcctac aggctctagg 154680
ctcttggttc gagtgtttaa agagaaaaac tgagaacctt cagaagagag cgaaacaaca 154740
aagacttgtg aaggtgattc cagttgatga atgaccgttt tccaagaagc ttgttctaaa 154800
gaagggcgtt ctttatgggc gatacaggga aaatggatga tctctatcca aacggtttta 154860
ggggttgttg atttgactaa aacaaacgtt ctttgagacc ctcgacttaa aacagtatag 154920
tctccagtct tagcaagaaa aatattttct tgaattgttc gtaaagaact agtttgagag 154980
gcaaaaagac cagaaaaagc cgtcgagaag aggaaagata gaaagaggaa taagaagagt 155040
gtttttcctt gctttttcat aagatatttc tgtagaatcc tctcgaagta tgtcgagcta 155100
tcatttggca taataggata gcgattttgg agttgtcaat atgaaaaaaa atacccaccc 155160
tgaatataga caggttttat ttgtagattn ttcaacaggg tataaatttg tttgtggatn 155220
tacctatcaa agtgaaaaaa ctgaagtttt tgaaggtaaa gagtatcctg tatgttatgt 155280
cagcgtatcc tcttcttctc atcctttttt cactggaagt aagaagtttg ttgatgctga 155340
aggtagggta gataagttct taaaacgtta tagtaatgta agacagcctg cacagcaacc 155400
tcagcctgaa gaagacgcac tacctgctgc taaaggaaag aaaaaagttg taactaagaa 155460
aaagaaataa aacttctttt agatttccca tttataaaac ccattctcag gctctcaagc 155520
ccgagaatgg gtttttttgt gagggctttc tctcttgcaa tagcttgtat aatacgttat 155580
gctttcctag gtcacgataa ttttgaaata gataacgtta gatgcctgta tcagtatacc 155640
aaggtgtttt agagaatttt ttatctgatt ccatttgata ctgatttttc accacttttt 155700
agggattcat gaagaaaaaa gttgccgagt atttaaaccg tttagcagaa gtcgaaataa 155760
aaatttcaaa tcctgaaatt ttttctaatt ctaaagaata tagcgctctt agcaaggaac 155820
attcttatct tctagaattg aaaaacgcct acgataaaat cttaaattta gaaaaagtcc 155880
ttgctgatga taagcaagct ttagctattg agaaagatcc agagatggtc gttatgcttg 155940
aagaggggat taacgaaaat aaagtagagc tagagaaatt aaataaaata ttagaaagct 156000
tattagtccc cccagatcct gatgatgatc taaatgtcat tatggaacta cgagccggta 156060
caggaggcga ggaagccgct ctctttgttg gagattgtgt ccgcatgtat cacctgtacg 156120
cctcctctaa gggatggaaa tacgaggtac tctctgcgtc agaatccgat cttaagggat 156180
ataaggaata cgtcatgggg atctcaggaa ctggggtgaa gcgtttactt cagtatgagg 156240
ctggtacaca tcgagttcag agagttcctg aaacagaaac tcaaggacgt gtacatacat 156300
ctgcaattac aatcgctgtc cttccagaac cttcagaaga agatacagag cttcttatta 156360
atgagaagga tttaaaaatt gatacattca gagcctctgg tgctggagga cagcacgtaa 156420
acgttactga ttctgcggtg agaatcacac acctgcctac aggtgttgta gttacatgcc 156480
aggatgagcg cagtcaacat aaaaataaag ataaggccat gcggattctt aaagcccgga 156540
ttcgtgatgc agaaatgcaa aaacgccata acgaggcgtc tgctatgcgt tctgctcagg 156600
taggaagtgg ggatcgttcc gagagaattc gcacctataa tttttctcaa aatcgcgtga 156660
ctgatcatag aatcggatta actttatata acttagataa agttatggaa ggagacctag 156720

atccaattac gactgcaatg gtgagtcatg cctaccacca gttactcgaa catggaaatt 156780
aaaaaggcga ttcaagaggg aaccgcttac ctagattatt atggggtgcc tctttctgat 156840
tgcgaagccc tgtatattct catggattta ttagaagtca gttcaagggc aaagttattc 156900
gatcttgttg gaattagcga aacgatgctt atggagtatc gaaagaggct agctttaagg 156960
gggcaacggt gtcctactgc atatctcaat ggtgccgtga gtttttgggg attaagattg 157020
agagtggatt ctagggtttt aattcccagg acagagactg agctgcttgc tgagtatatt 157080
atcaactatc ttttatctca ttctgagatt caaacttttt atgatatttg ttgtggtagc 157140
gggtgtttag ggctagctat caagaaatcc tgtcctcatg tggaagtggt gctttcagat 157200
gtttgtccgc aagcagttgc cgtcgcaaat gaaaatgcta aaagtaatgg tttggatgta 157260
aagattcttc taggcgattt gtcagccccc tacactcgtc ctgcagatgc ttttgtttgt 157320
aatcccccct atttgtcttt taatgaaatt attcatatag atcccgaagt gcgttgttac 157380
gagccttgga aggctcttgt tggaggttct acgggtttgg agttttatca gcgtatcgcc 157440
caagaattgc ctaagattgt aacttctaca ggagtcggtt ggttggagat tggatccagt 157500
caaggagaaa gtataaagaa tattttttcg aagcacggaa tttatggccg tctccatcaa 157560
gatttgtctg gacgcgatag aatttttttt cttgaaatgg atgggagaga tcctgtatcc 157620
tcgggggctt attcttgatt ttttctggat aaatgattaa ttctttatcg caaaagctat 157680
cttctatttt ttcttttttg gtttcttctc gtagaattaa tgaagaaaat atttccgaat 157740
ctattagaga agttcgtctg gctctcttgg atgccgatgt aaattatcat gtagttaagg 157800
attttatttc taaagttaaa ganaaaatcc ttggagaaga gatctggaag catgtttccc 157860
cagggaaaca gtttatacgt tgtttgcatg aggaattagt agcattttta agcgatggaa 157920
gagaagagtt tactattcag aagacgcctt cgatcatcct tctttgcgga ctccaggggg 157980
caggaaaaac aacaacagct gctaagcttg ctgattatgt aattaagaat aagaaagcaa 158040
aaaaagtcct tgtggttcct tgtgatctca aaagattcgc tgctgtagat caattaaaaa 158100
ttttggttgc tcaaacgaaa gctgaatttt accaaagtca agagaacaag cctattgatg 158160
ttgttgttaa agcgcttgca tatgctaaag aaaatggtca tgattttgtg attctggata 158220
ctgcagggcg tctcaatata gataacgagc ttatggaaga gctgacggcg atacaaaaag 158280
tttctcaagc taatgagcgt ctttttgtga tgaatgtagc tatggggcaa gatgttttag 158340
caacagtgca agcttttgat cagtctttag atcttacagg cgtgattctt tccatgactg 158400
atggagatgc tcgagcaggc gctgttttct caattaagca cgtccttggt aagcccatta 158460
aatttgaagg atgcggagaa cgcattcaag atcttcgttc attcgatcct caatctatgg 158520
cggaacgcat tcttggaatg ggggatacca taaattttgt taaagaaatg cgcgagtata 158580
tttctgagga agaagacgct gagctaggta aaaaactagt tactgcggct tttacttatg 158640
aagactatta taaacagatg aaagcatttc gtcgcatggg acctctaaga aaacttttgg 158700
gaatgatgcc tggttttaat aatgcgaaac ctagccaaaa ggaaatcgag gattctgaac 158760
aacagatgaa aagaacggag gcgattatcc tgtccatgac tcctgaagag agaaaggagt 158820
tggtggaatt ggatatgagc cgtatgaaga ggattgcttc tggttgtggt ttaactttag 158880
gcgacgtgaa ccagtttcga aaacagatgt cgcaatcgaa aaaatttttt aaaggaatgt 158940
ctaaaggcaa gatggaacaa gttaggaaaa aaatgtcagg aggaaatcag tggcgttaaa 159000
aattcgttta agacagcaag ggcgtagaaa tcatgttgtt tatagattag tgctcgcaga 159060
tgtcgagtct cctcgtgatg gtaaatacat agaattatta ggttggtacg atccacatag 159120
ctctataaat tatcagctga aaagtgaacg aattttttat tggttagaga ggggagccca 159180
actttcttcg aaagctgaag ctttagtaaa gcagggagct ccaggagtgt atagtgcgct 159240
attgtctaaa caagaagctc gtaagttagt tgttcgtaag aagcgacgtg cttatagaca 159300
gcgtcggtct acacaaagag aagaggctgc aaaagatgca actaagtagg tagtgaactg 159360
ggatgaagat cgatatactt tctttatccc caggttattt tgatggtcca ttgcaaacga 159420
gtattcttgg tagggccata aagcagagac tcttagatgt ccagcttaca aatcttcgtg 159480
actttggact cggaaagtgg aaacaagttg atgatactcc gtttagtggt ggtgggatgc 159540
ttttaatggc agagcctgtc acttcagcta ttaggagtgt aagaaaggag aattctaagg 159600
taatttacct ctctcctcaa ggagctttgt tgacagctga aaagagtcga gaattggctg 159660
ctgcttcgca tttgatatta ctttgcggtc actacgaagg tattgatgag cgtgctatag 159720
agagcgaagt ggatgaagag attagtatag gggactatgt cctgactaat ggtggaattg 159780
ctgctctggt ccttatcgat gcagtttctc gttttattcc cggtgtattg gggaatcaag 159840
agagtgctga gagagattct ttagaaaatg gtttgctaga aggacctcag tatacacgcc 159900
ctagagagtt tgaagggaaa gaagttccag aagtattgtt gcaaggggat cacaaagcca 159960
tttcatcagt ggagattgga gcaaagtgag cgtagaactt atgagagacg tcctgatttg 160020
tatctgaact atctctataa acgctcgatt gatcacaaat ttgatgagga gactacaaca 160080
aatagggatc atttcaagtg tgacaagatc tctgtagtac tagaggtaaa taagttaaag 160140
cgcgcaaaaa atttttactg taaggtattc ggtctggatg ccatgagctg cgagaataaa 160200
ttttgtcttc ctcatgaagg caaaaccata ttctggttac gagaagttca agctgagaaa 160260
aaaaacatag tgactctctc cctttcctta gattgtgcat gcgaagagga cttttgttat 160320
cttcttagaa gatgggagtt atttggtgga aagttgttag aaaagcaagc tgatgagcat 160380
gctgtatggg ccctagcaca agatttagat gggcatgcat ggatattctc gtggcatagg 160440
atgaaataga agaaagagaa ttttaggtgg tatattatgg tgaatttact caaagaatta 160500
gaacaagaac agtgtaggaa tgatcttccc gagtttcatg ttggcgatac aattcggtta 160560

gctacaaaga tttcagaagg cggtaaagaa cgagttcagg tatttcaagg tactgtgatg 160620
gctcgtcgag gcggcggctc tggagagact gtatccttgc atcgtgttgc ttatggtgaa 160680
ggcatggaaa agagtttctt gcttaatagt cctaggattg taagtattga aattgttaag 160740
cgcggtaaag ttgctcgagc tcgtctgtat tatctgagag gaaaaactgg taaggctgct 160800
aaagttaaag agtttgtagg acctagatct tcaaagaaat agtctgtagc aagacttcat 160860
attgtcttat tttgattttt ataatctata gtagcttatg aatacttcta tttctgaaat 160920
tcagcgtttt ctttctatga ttgcttttga gaaagagctc gtctcagaag attttagtgt 160980
cgtcgctgga atagatgaag ctggaagagg gccactggca ggtcccgtag ttgctagtgc 161040
ctgtatttta cctaagggaa aggtatttcc tggagtaaat gatagtaaga agctatctcc 161100
taaacaacga gcccaagttc gggatgcttt gatgcaagat cctgaggtct gttttggtat 161160
aggcgtaatt tctgtagaga ggatagatca agttaacatt ttagaagcca ctaaagaggc 161220
tatgcttcaa gcaatatctt ctttaccgat atctccagat attcttcttg tggatggtct 161280
ttatttaccc catgacattc cttgtaagaa aatcattcaa ggagatgcta aatctgcatc 161340
catagcggcg gcttctattt tagcaaaaga acatcgtgat gatttgatgt tacaactaca 161400
caggctctat cctgaatatg gatttgatag acataaggga tacggaactt ccttgcatgt 161460
agaagcaata cgacgttatg gtcccagtcc ctgccatagg aagagctttt ctccaataaa 161520
gcaaatgtgt gctattgtat gaataagatc ctagttgact ctcctttttc tccagatcac 161580
cagaagtgct gtcctaagct ttttacaatt agtgctcctg ctggagttgg aaagacaaca 161640
cttgtccgta tgttagagca agagttttct tctgcttttg ctgagactat atcggtaaca 161700
acaaggaaac ctcgagaggg tgaagtccca ggtaaagatt atcattttgt ttcccacgaa 161760
gaatttcaaa gacttttgga tcgtcaggct ctcttagaat gggtgttctt attcggagag 161820
tgttacggaa caagtatgtt agagattgaa agaatttgga gcctagggaa gcacgctgtt 161880
gctgttattg atatccaagg agccttgttt attcgctctc ggatgcctag tgtatctatt 161940
tttattgctc caccttcaca ggaggagtta gaaagaaggt tagcttcacg gggatctgaa 162000
gagggctctc aaagaaaaga acggctggag cacagtctta ttgagctagc agctgcaaat 162060
cagtttgatt atgtcattat taacgacgac ttaaatcaag cgtacagggt tttaaaaagc 162120
atttttatag ctgaagaaca taggaacata ttatgattaa aaaagatcgt ttcactaatg 162180
aaaagttaaa taagcttttc gatagtcctt ttagcctagt gaactacgcg attaaacaag 162240
caaagatcaa aattgccaaa ggcgatgttc gctcctctaa tgttgcgatc gaaacactcg 162300
tcttgttaga tagagaaggg atacagcctg agtttactga agagattgta gtaactgcta 162360
gccctactgt ggaaagaaag agatcagaac atacaaattc tagaaaaaaa gatccctcag 162420
catatacttg gagtgatgta aagtaatgcc acaaaaagtc ctgattactt cagctttacc 162480
ctatgctaat ggtccgctac attttggaca tattgcagga gtctatcttc ctgcagatgt 162540
gtatgcaaga ttccgtagat tgttaggaga cgatgtcctt tatatttgtg gttccgatga 162600
atttggcata gcgatcacct taaatgcgga tcgtgagggg ttggggtatc aagagtacgt 162660
ggatatgtac cataagttac ataaagatac ttttgagaag ttagggtttg ctttggattt 162720
cttttctagg acgacgaacc cttttcatgc tgagcttgtc caagattttt attcccaact 162780
taaagcgtct ggattgattg aaaatcgcat atctgaacaa ctgtattcag aacaagaaca 162840
acgttttctt gcggatcgtt atgtagaagg gacgtgtcct cggtgcggtt ttgatcatgc 162900
tcgaggagac gagtgtcaga gctgtggtgc ggattatgag gctatagatt taatcgaccc 162960
taagtctaag atttctgggg ttgagttagt aaaaaaagag actgagcact catattttct 163020
tttggaccgt atgaaagacg ctctactttc ttttattcag ggatgctatt tacctgatca 163080
tgtccgtaaa tttgttgttg attacataga acatgtcagg tctcgagcca ttactcgaga 163140
tttatcttgg gggattcctg ttccagactt tcctggaaag gtgttttatg tatggtttga 163200
cgctcctata ggatatatca gtggaactat ggaatgggca gcttctcaag gaaaccctga 163260
cgaatggaag cgtttctggc ttgaagacgg tgtagagtat gtccagttta taggtaaaga 163320
taatcttcct ttccattctg tagttttccc agctatggaa ttgggtcaga aacttgacta 163380
taaaaaagtt gatgccctcg tagtttcaga gttttatctt ttagaaggac ggcaattcag 163440
taaatccgag ggcaattatg tggatatgga caagtttttg agttcctatt ccttagacaa 163500
attgcgctat gtattggcgg ctacagctcc tgaaacttcg gatagtgagt ttactttcct 163560
tgattttaag actcgttgta attctgagtt ggtaggaaag tttgggaatt ttataaaccg 163620
agttcttgct tttgcagaaa agaatcacta tgacaagctt tcttatcatt ctgtggtttt 163680
agaagatagt gacagggcat ttcttgaaga agtgcgtcaa cttgttcgag atgctgagaa 163740
gtgctacaga gagtatagtt tacgtaaggc tacgagtgtg attatgtcac tggcagcttt 163800
agggaatgtc tattttaacc aacaagcacc ttggaagcta ttgaaagaag ggactcgtga 163860
gcgtgttgag gccattttat tctgcgcatg ttattgtcag aagttgttag ctttaatttc 163920
ttatcctatt attcccgaaa gcgctgtagc tatttgggag atgatctcac caaaatcttt 163980
agaaaattgc aatttggata cgatgtatgc tagggatcta tggaaagaag aaattcttga 164040
tgttataaac gaagaatttc atttgaagtc ccccaggtta ttatttacta ctgtagagta 164100
gagctcgagg tcttttcttt ttagaatcct gatctgtagg tgtaattaca gatctgcata 164160
atttttctta gtatcgagct ctttaagaac ttctgctagt cctgtacatc ggtgttgcac 164220
tcgattattt cttgttgcaa tagcaaatgg ctttttttgt cccaactaga atcaccaatt 164280
ttttcccccg cgtaatagca gtatagagaa gatttctata gagcatcata aagtgcgagg 164340
tatgaatagg aatgataatg caggggcttt cacttccctg gtacttatgt actgaggtag 164400

cataggctaa gactagatcg tcaagctctg agaaagaata gccgacatgc tttccttcca 164460
tacggacaac aacagcttta tcttcgaagt tgattgtaga gacatagcct atatcgccgt 164520
taaagacttc tttattatag ttgttgcgga tttgcattac cttatcgcca acggcatagg 164580
attgaaatct accgtgaaga tttgcttttt tagggtttaa tgcatgtttg agtgctttat 164640
ttagattata gattcctagg gttccttttt tcatgggagc tagtacttgg atatcttgag 164700
gatagatatg gtatttttgt gggacgaatt ttgtcacaag atgaatgata tgattgagag 164760
cctcttcttg atcatccttt tggaaaaata agaaatcacg acgccctgtt tctgaatata 164820
atatggggag ttccccttca tttaccctat gggcattcgt aacgattccc gaatcatgaa 164880
cttggcggaa gatcttattt aatctgatga ctgtcatttt attcgaagta atcaagtctt 164940
taaggatatt tcctggcccc acgctgggta gctggtgaat gtctccaata aagacaagag 165000
ttgtgtagtc aggaagtgct ttcaggaagt ggtgcagcaa gtgcgtgtcc atcattccgg 165060
attcgtcaac aatgatcaga tcacagtcta taggattgtc atggttcttg cggaaagatt 165120
tcgttttaaa atcatactgt agcagagcat gaatggtgac ggagtgtttt tgtgtaattt 165180
cggtcatccg tttagcggct tttcctgtag gagctgcgag gatgatttta tgagtcacct 165240
gttcaaaaat tttcagtatt gcttgggtaa tggtactttt tccagttcca gggcccccag 165300
taatgatgag aagtttttca gaaaaacagg ctttaattgc ttctcgttgt tgttctgcga 165360
gatctatact tagttttcct ctacccaagc aattgctttt tctccgtcta tagaacggat 165420
tctcctcgaa gaaaataaaa tgcgcttgag atcagaaaca atagttttct ctgcgagatg 165480
gagataacgt gtccagacat ggagtgtccc agaaatgtct tgaatatgta aaagttacgc 165540
ttttgcatat tgaggatttg cgtatcgatt tcttcgagag taataggagt atcaaagaca 165600
tcttgattta atagtttggc gacgacatct atcaggagct ctatcggata gcaagtatga 165660
ccttcttctt gaagttcttc taaggagtgc tggataccag cacataggcg actttcagaa 165720
tttctgggga cgcctagttt catagctatg aaatcagcag ttttgaatcc gatgccctcc 165780
atttctctgg ctagaaggaa gggatcttcg caaatttttt ctatggattt ctcttggtat 165840
tttttaaaaa ttctcactcc ataatgaatc gggatattgt attcttggag aaagagaaga 165900
gttttcctta acattttttg ctcgcagagt tgtttgcaaa tagagacaca tcgtgtttcg 165960
ctaattccag aaacctcact taagcgttct ggagtgatat cgaggacata acatgttttc 166020
tcttgaaatt tctcgatgat cttttctgcg attttagggc cgattccttt gatgagtttt 166080
gaggtgaggt aatggaatac gccacgatat tcataaagaa gaggagagtc gtaactatgg 166140
atttggaaat acttagtatt tgaaggggaa tggctccaga caccatagat ttggatcggg 166200
gatcctagtt ccaaaggttg gggaagtttg cctttaatta ggataggagt cgttttattg 166260
ggtattttga tataagcagt aatgtcccca gagtctttgt tttcaacaag tatttgctct 166320
aagtatccgc agattttctc catagaattc taaaatcttg ttttcctgga agatgaagta 166380
gcttaaggaa tctttataaa aagcttctaa aaagtatgga ttataaactt tttgagataa 166440
aaacgagaca tcccaaggct tttaattttg ggataaactt atcaaagaat tattttatat 166500
agtaattatt tcctcgtata ttgttttttg ccataggaca caaaatctat cctaccgaag 166560
aacctttgtt attacaacaa tttttataac aatagattga ttaggaaaga tcgatgttcc 166620
cttgtgcaat aggttcatgt tttatcagaa cacgagtttc ttttttaatt cttttttctt 166680
ttgctattgc attcagctcc atgaggagag cttgagtacg atttatacaa aactgggata 166740
gggattgcct ctatccgtgt tggacaaata cctaagaaga ttttgattcc ggctttccga 166800
ctatatagag aagaaattcc taaggcactt taattaaaag gtttattgcc atattcttgc 166860
cttgaaaatt tttgattata gattttggta ttgaacaatg actagtgcag taaaaacctc 166920
atcactcatc caagtgactc aaccttaaat cagaagtaaa caacgtacag ttgcaattac 166980
gctccttgtg cttggcattc ttttgattgc ttctgggatt atttttctag ctgtcgctat 167040
tcctggattg agttcagcag ttgccttagg attgggctgt ggtatgactg ctttaggaac 167100
tgttttgttg attacaggac ttgtcttgct gatcaggagt gagaagctcg ctctagaaca 167160
agtagaaata aagcaagcta ggaccagggt gaataatgag ttagatcaac tcagtcagta 167220
tgttttctac acagaaaatg ttttagataa tttgaagcgt tggtcgtatc gagatttagg 167280
ttttgtgaga caggcgcaag aggaggttac aaatttagag caagacattg aagaaatttt 167340
cttgacgttg cgagatatta gaaatgctct tgataacgaa gagtttttta tgactcatgc 167400
gaaacagtgt ttagcccaag tcggagaaag cttatttcag gatgctagta tagatgagtt 167460
tattaatttg gctcatctat ccgaaatacg tcagcatttg gatatcaatg atccgagatg 167520
gtctatgatt acaaagaaag ttaaaggcac tgtggttcgg tttatctatg tctctacaat 167580
gtataaacaa ataaaatcta attttgaaaa aagtgacttc ggacaactta ggaagatgct 167640
actgaacaat tacaaaacaa tagaagaggt cttgtatcag agttttcaaa ggggctacaa 167700
tagagccgct ttgttgagtg aaaagacaag aattattcat acgagttctc ttttgcattg 167760
ggaaaaggac gaagataagc atcttaatat taagaacgag tgtgcaagtc gtcttgagaa 167820
tttcaagaag tttagaacac tatttcttgg attatcagag gaagacgtta ttgactttac 167880
tggagcgtct ggttgggatt gttccaaact gcctcggaaa gaggtcccgc ttgatggtgg 167940
caagaagaaa ctgaggttta aagaaccttt tgcagatgaa caagtcggag attgggatcg 168000
cactacgtct cttgagcata tgacacctca agaggaagat cctttagaca ggttaatgga 168060
tcaggttgaa caagaggcta cttcagtctt aaaagatcag gatcgttatt ggaaagagat 168120
cgagacaagc gaagcaaagt ttaggtccct gccacgggaa gatgattttg aaaagcagtc 168180
acagattgat agttatattc gggatttgga cgaccattta tcggtttggg cgaatcagtt 168240

atctgctgca gaagatgctt tgatagaggt tacagatgtg caggaacatg gaaatagaga 168300
aatgcttaag aatatacaac agggactgga gcttattgaa gatgctgtaa aagctactct 168360
acctagagtt gactttatac aagagctttt agagaaggaa gagcttccgt tggttgctgc 168420
taggatgagt ttagagaata gttagaagat aagcagctgt gcagagatta tgtcagaagt 168480
gaagcctttg tttttaaaga atgactcttt tgatttggca actcagagat tccagaatct 168540
aattaacatg ctacaagagc aagccgagat atataacgag tatgaagaaa agaatgctag 168600
ggttcagaat gagattaagg agcaaaagga ctttgtgaaa agatgcatag aggactttga 168660
agccagagga ctgggggtgc taaaagaaga gcttgcatct ttgacgcgtg atttccatga 168720
taaagcaaaa gcagagactt ctatgctcat tgaatgtcct tgtattggtt tttattatag 168780
tattcatcag gaggaacaaa ggcaaaggca agaaaggctt caaaagatgg ctgagcgcta 168840
tagggactgt aaacaagtct tggaggctgt ccaggtggag caaaaagata tgatatcttc 168900
tagagtcgtt gtcgatgaca gctactttga agaagaaaaa gaagaacaaa aggtggataa 168960
cagaaagaaa gaacaggact aggtctattt ttctacagct tttcccgtag ggggagggga 169020
gttctagaat tttttctgtc gcctatncaa gataacactg ttgttttgtg gtatcaaatt 169080
tagacattac actagcttta aagtttgagc cctcccccgc tttaagatcg ctttctgaag 169140
ctgagtctaa ggtacaaact tttgctatag gattattagt tattggtatt ctcatactat 169200
tgcatgggat tatttttttt ctctggagct atttctagtt gtggtctttt agtgtctcta 169260
ggagttggtt taggacttag tgttttagga gtacttttac ttctcttagc aggtcttttg 169320
ctttttaaga tccaaagtat gcttcgagag gtgcctaagg ctcctgatct attagattta 169380
gaagatgcaa gtgaacggct tagagtaaag gctagccgtt ctttagcaag cctcccgaag 169440
aaatcagtca gctagagagc tacattcgtt ctgcagctaa tgatctaaat acaattaaga 169500
cttggccgca taaagatcaa agactcgtcg agaccgtgtc acgaaaatta gagcgtctgg 169560
cagctgctca aaactatatg atttctgaac tctgcgagat tagtgagatt cttgaggaag 169620
aggagcatca tctaattttg gctcaggaat ctctagaatg gataggtaag agtctatttt 169680
ctacctttct ggacatggaa tcttttttaa atttgagcca tctatctgaa gtgcgtccgt 169740
acttagctgt aaatgatcct agattattag aaattaccga agaatcttgg gaagtagtga 169800
gtcatttcat aaatgtaacg tctgctttta agaaagctca gattcttttt aagaacaacg 169860
aacattctcg gatgaagaag aagttagaaa gtgttcaaga gttactggaa acatttattt 169920
ataagagttt aaagagaagt tatcgagaat taggatgctt aagtgaaaag atgagaatca 169980
ttcacgacaa tcctctcttc ccttgggtgc aagatcagca gaagtatgct catgctaaga 170040
atgaatttgg agagattgcg cggtgtttag aggagtttga aaagacgttc ttctggttgg 170100
atgaggagtg tgctatttct tacatggact gttgggattt tctaaatgag tctattcaga 170160
ataagaagtc cagagtagat cgagattata tatccacgaa gaaaattgca ttaaaggata 170220
gagcccgcac ttatgctaag gttcttttag aagagaatcc gactacagag ggtaaaatag 170280
atttgcaaga cgctcaaaga gcctttgagc gtcaaagtca ggagttttat acactagagc 170340
atacggaaac aaaggtgaga ctagaagcac ttcaacagtg cttctcggat cttagggagg 170400
cgacgaacgt aaggcaagtt aggtttacaa attctgaaaa tgcgaatgat ttaaaggaga 170460
gtttcgagaa gatagataaa gagcgtgtgc gatatcaaaa agagcaaagg ctctattggg 170520
aaacaataga tcgcaatgag caagagctta gggaagagat tggggagtcg cttcgtttac 170580
aaaatcggag aaaagggtat agggctggat atgatgctgg gcgtttaaaa ggtttgttgc 170640
gtcagtggaa gaaaaatctc cgcgatgtgg aagcccacct tgaagatgca actatggatt 170700
ttgagcatga agtaagcaag agcgaattgt gcagtgttcg ggcgaggctc gaggttctag 170760
aagaagagct gatggatatg tctcctaaag ttgcggatat agaagagttg ttgtcctatg 170820
aagagcgttg tattcttcct attagggaaa atttagaaag ggcatacctc caatataata 170880
agtgttctga aattttatcc aaggcaaagt ttcttctttc cggaagacga gcaattgcta 170940
gtttcggaag cgaatctaag agaggtgggt gcccagttaa aacaagtaca gggaaaatgt 171000
caagagaggg cccaaaagtt cgcaatattt gaaaagcata ttcaggagca gaaaagcctt 171060
attaaagagc aagtgcggag ttttgatcta gcgggagttg ggtttttaaa gagtgagctt 171120
cttagtattg cttgtaacct ttatataaag gcggttgtta aggagtctat accagttgat 171180
gtgccttgta tgcagttata ttatagttat tacgaagata atgaagctgt agtgcgaaac 171240
cgccttttaa atatgacgga gaggtatcaa aattttaaaa ggagtttgaa ttccatacaa 171300
tttaatggtg acgttctttt acgggatccg gtctatcaac ctgaaggtca tgagaccagg 171360
ctaaaggaac gggagctaca agaaacaact ttgtcttgta agaaattaaa agtggctcaa 171420
gatcgtcttt ctgaattaga gtcaaggctg tctaggagat agtaaaaaag ttgagttctt 171480
tgcgcagtgt tttgatggat tattcgttta gaagacgata cttgagagtt ttccaaactt 171540
ttctgtactt ttcttccgaa gagaactagg cagaggaggc ttcctcctaa cattagaaag 171600
attcctatac cttcttgtag agaaggaaga ctttgtgagt agagataggt aagcacaagg 171660
ccaaaaattg gttcaaagat taatatcgcc cctagcaatg ctggtgagag attcaaacta 171720
gctttattcc aggctattaa agcttttgct gaggaaaata ttcccatagc actacatagc 171780
aacaagaaga gcagtcgctc ggatcccggt gtatgcgaga taagattgtg tgttacgtgg 171840
gtaattccac agagatcgag aataataatc atagggaggc agatgatcaa agcgctgatt 171900
ccgatgaggt agctccaggt aatctggagt taggtngggg tgtttttcga gtagcgattg 171960
attgcggatc acatagatta cccaaagact tgttgagagt atgactgcaa tgactcccaa 172020
gatagagtag agaggagagg cggctgttgg caagttgagt gcggagaggt gtgtcagaat 172080

cactcctgtg atgatcacac tgctgatagc aaagagtaga gaatagggga gttctttttg 172140
ctttgtattg gagtggtaga gtacggcggt tggagccagg ctggcgatga ctacagtgat 172200
tgcggatcca acatagcgga tgccaagggt gattccgaag taatacacgg ggttaatcag 172260
cagtgtccag aggaggcttt ttctccaaat atataaagga gtttttttaa ttacggaggg 172320
attttttata gcgcaagcaa tcagggagaa aataccaaaa atggtataac gggtaagtac 172380
aatatcaaga tcgccaaacg aaccgaggaa gtttggtatg acaaagacga ttccccagta 172440
taggcaggca acgagcccat ggaagatgcc taggggtaca ttacgggttc tggattcttg 172500
atttgcgctg gggaacataa agctctctta agggtatgag caatagaatt gcctacaatt 172560
ttaacatggt agggattttt gttggtattg agactttaaa taatacgaat tgcttttctg 172620
ttcgagagga aaataagaag ataggggaag aatgttaagg aatcaggtac ttgtttactg 172680
tagtgagggt gtttctcctt attatttacg gcatacgata cgttttctca agtactatag 172740
cactcaagaa ggtgctttcg atattcttag ggtcnacggg aattttttga ttaagaatcc 172800
tttttgggaa gaaacgacgc gcttattggt attcccaggg ggtgcggacc gcccctatca 172860
tcgtgtactt catggtttag gcactgcccg tattttccaa tatgtttctg agggagggaa 172920
ttttctaggg atttgtgctg gggcatattt tggttctaag atgatttatt tttatgagcc 172980
tgagggagcg ccgttgcaag gggctcgaga tctagggttt ttcccgggga ctgccaaagg 173040
tcctgcttat agggggaatt tttcttatgt gagtccttct ggtgtaaggg tttcacctca 173100
gttattttca gattttggct tggggtatgc gatgtttaat ggggggtgtt ttttcgaggg 173160
ctcggaagga tatcctgggg tgaatatcga atctcggtat gacgatcttc cagggaagcc 173220
tgcgagcata gtgtctagga ttgtcagtaa gggcttagcg gttctttcag gacctcatat 173280
agagtatctt cctcattact gtcgtatggt taaggagaac gtccagaaaa cacgtgaatt 173340
cctccaaagg gagcgtacaa ctttggaccg ctattgtcag aatcttgtac agcgtttgcg 173400
tcagcctgca ttttcgaaag cggactgctg aagatctaaa ttaggcaatc gaaatcatca 173460
gcgtgtattt ttgtcgtatc gaactcggtg ataagctgat ataggattgg gctgcaaaga 173520
actatggagt gcgttaggac atttcggatg ttattggagg cataagacat ctcttcatag 173580
ccgtggagta gtccaaacga ttgaatttgt ggagctgtnt ggctgntntt atatacaaag 173640
cgattgaaat agtctctagg ttctatagtg tactcatctt cattatagtc taattgctcg 173700
aatacacaca gaaaagcgag tagttccatt tgtcttgcta tgggatttga gcttcctgtg 173760
ttaaggctat ctatgagtag tatttgattt ctttctaatg ctgttgaaaa taggttatgg 173820
tagtggataa agaatttttt gaaaattaga tagagttggt tcgagatagg atcccattca 173880
gatcttagaa tgtcaggatt atgtactaca gggaagtagt aggtgatttc atttgttgtg 173940
gtttcgatga ggttattttc atactgcgtt ttgttctgca agtcggtcaa tgaaacttga 174000
gaggctatcc ctaagttgtt tacatgtttt tcttgtaatt tttgataggt tttttgtaaa 174060
ggttcggagg tgacaaagat tctcggatcc atatggatat gagaaaggat atactgttca 174120
tcttgttcta atattttcca attggcgaga gcacttttaa ttaggaaatc tggatcttta 174180
agaaagattt ttttccattc ttcgaaggat ggaaaaaaga gctcattttt taggtttatg 174240
gatgttggat ctaggagtag tgtttcggag gattgtattt tatttttgag aggatttagg 174300
agccaggggt ctaaagaagg tgtttcttta ggaaaaatag atttggtttt ttgttcgtcc 174360
ttttgaacac ttttcgagca ctgtgttcca atgagcaggg agattcctgt acttaggacg 174420
attatactga ttagggacaa tgctattatc aaaagagtat ttgggagaag tccggagagg 174480
gctatgagta tgcatgctgc aatccccaag aatagggaaa gtatggcgag agaaagtttc 174540
gttatagcag cgaatttgga ttctggcagc agttgagcgg gttttttatt ttcgtgtata 174600
attgaagaac acttgaccat agaagactat caaagaaaat gcaaataata aattgaaaaa 174660
tcgttgaaag aaggagtcga agagaaaaaa tactccggag gatgggttcg aaccaacgac 174720
caatggatta acagtccact gctctaccgc tgagctactc cggaacagct tttactccct 174780
acatattcta tgaccaaagg gtaaaaagtc aatgattttc gtctctgaaa aaagaaagat 174840
tttctttaag tcttgcaatt ttccttatat ttttcttcac tagtgcgtat gtttttccat 174900
cgatatgctt tctggagttg tttatggaga atgctatgtc atcatcgttt gtgtataatg 174960
ggccttcgtg gattttaaaa acgtcagtag ctcaggaggt atttaaaaag cacggtaagg 175020
ggattcaggt tctcttaagt acttcagtga tgctttttat aggtcttgga gtctgtgcct 175080
ttatatntcc tcaatnnctg attgtntttg ttttgactat agatttgctt atgctcgcta 175140
taagcttggt attgtttctc ttaaaagttc tgtacgctcc ttcaatggta gatcgtttgt 175200
ggtgttctga aaaaggatat gctcttcatc aacatgagaa cgggcctttt ttggatgtga 175260
agcgtgtaca gcaaattctt ctaagatcac cctatattaa agttcgggct ttatggccgt 175320
ctggagatat ccctgaggat ccttcacaag ctgcggttct attactttct ccttggactt 175380
tcttttcatc cgtggatgta gaggctttat taccgagtcc tcaagaaaag gagggtaagt 175440
atatagatcc tgtgctgcct aagttgtcta ggatagagag agtctcactt ttagtgtttt 175500
tgagtgcatt tactttggat gacttaaacg aacagggagt caatcctttg atgaataatg 175560
aggaattttt attttttata aataagaaag cgcgtgacat gggattcagg atttaaaaca 175620
cgagattatg tcttcgttag agaaaacagg agtgccatta gacccctcaa tgagttttca 175680
agtttcacaa gcgatgtttt ctgtatatcg ctacttgaga caaagggatt taacgacttc 175740
agaattaaga tgttttcacc tcttaagttg ttttaaaggg gatgtggttc attgtttagc 175800
ttcatttgaa aaccctaaag atttagcaga ttctgacttt ttagaagctt gtaagaacgt 175860
ggaatggggt gagtttattt cggcatgtga gaaggctctt ttaaagaatc cgcaaggaat 175920

ttccattaag gatctaaaac aatttttagt gaggtaatct atgatcgagt ttgcttttgt 175980
tcctcatacc tccgtgacag cggatcggat tgaggatcgc atggcctgtc gcatgaacaa 176040
gttgtctact ttagcaatta caagtctttg tgtattgatc agttcagttt gtattatgat 176100
tgggatttta tgcatttctg gaacggttgg gacctatgca tttgttgtag gaattatttt 176160
ttctgtgctt gctttggtag catgtgtttt ctttctttat ttcttttatt tttcttctga 176220
ggaatttaag tgtgcttctt cgcaggagtt tcgttttttg cctataccag ctgtggtttc 176280
tgcattgcgt tcctatgaat acatttctca ggacgctatc aatgacgtta taaaagatac 176340
gatgcagttg tctacccttt cttctctttt agatcccgaa gcttttttct tagaatttcc 176400
ttattttaac tctttgatag tgaatcattc gatgaaggaa gcggatcgtt tgtctcgaga 176460
ggcttttttg attttattag gtgagattac ttggaaggat tgtgaaacaa aaattttgcc 176520
atggttgaaa gatcctaata tcactcctga tgatttctgg aagctattaa aagaccattt 176580
cgatttaaag gactttaaga agaggatcgc cacttggata cggaaggcct atccagaaat 176640
tagattaccg aagaagcatt gtttagataa gtctatctat aaggggtgtt gtaagttttt 176700
attacttgct gagaatgatg tgcaatatca gaggttatta cataaggtct gttatttctc 176760
tggggagttt cctgccatgg ttttaggttt gggaagtgaa gtgcctatgg tgttaggact 176820
ccctaaggtt cccaaggatc ttacctggga gatgtttatg gaaaatatgc ctgttcttct 176880
gcaaagcaaa agagaggggc attggaaaat ctccttggaa gacgtagcct ctctttaatg 176940
aaagaagagt cctcggatga agcctatgat ttcttctgta ggtgttccag gggtattgat 177000
atcgttaaga cggtcttgga attgtggata cgatgagtag aagtgtttat gagcttgttg 177060
ggataggggt ttaataatat ctccagggag gttcaggtgg tagaggcttt cttctagagc 177120
taggtagagc aaggaaggga gagcctctga gagttttggt aattctttag ctgagctagt 177180
atggctatgg aattccaagg atggcacaga aagatgggga atgtctttat ggttagctct 177240
gagtccgtac aaccgtgtgt ttagaaatag gcatcttcgg atgagcacgg gagcacggtc 177300
tatccataag tttgaggggg tttctttttg tggatggaag tttttccata ctaaagacca 177360
gtttgttttt gtgccgtgac tgtcgtaggg gaatatggtg aagtttgccc catggataat 177420
gagctcggag atttccaact gtttcgttag tagcatagat atggaagaaa atcgtacatc 177480
tgcgtactcg atttcggcgg cgtaaggaaa gcgttcagaa gctagagggt tatgaatgca 177540
gatatgacgg attttaattc cagaagttct tatggagact cttcctacag tgacttgggt 177600
gtggaggcgg ttagatagcc attgctcaac gatactttct ttgcgcatcc agaaatatcc 177660
aacgatgcag caccctataa gaaatagatt ttttagcagt ttaaacatat aggcagagga 177720
gttttatatc ttaataatta agatagatta agaaatgaat attcccaaag aaaaaggtct 177780
cttttcttta gttagaaaag agacctcatg gaaaattgtt aatactagct taagagacta 177840
gtagtccatt cctgcgcttg gcatcgctgg agctgaagaa gatttctctt ctgggatatc 177900
agcgattaag gcttctgttg tgaggagtaa tcctgcgata gaagctgcgc tttctagagc 177960
tgagcgagtc actttagttg gatctaaaat tcctgcgtca atcatatctg tataagcgtc 178020
acgtaaagca tcatagcctt catttgcaga tcttgctaga acttgctgac aaatgatagc 178080
gccttcttta cctgcgttac ttgcaatttg ctttaatgga gctgttaatg cttttagaat 178140
aatacgagta ccaatagctt cgtcttcgtt tgctagcata ggaaggaaag cttctagtgt 178200
agggatacag cgaactaagg cagttccacc accagggagg attccttctt cgacagctgc 178260
aatggttgcg tgttgtgcat catctactct gtcttttttc tctttcatct ctatttcggt 178320
agcagctcct acgcggatta cggcgacacc accggagagt ttagctaaac gctcttggag 178380
tttttctttg tcgtaatctg aagtgctatc ttcgatttgt tttttaatat tgtcgcatcg 178440
agcttggata tcaggtttgt ttcctaagcc ttcgacgatt gtggtatctt ctttagttac 178500
gataactttc ttagcttttc ctaacattgc tagagttgta ttctctagtt tcatgccaag 178560
ttcttcgcta actagttggc caccagtaag gatagcgatg tcttctaaca tagcttttct 178620
tctgtcaccg aaaccaggag ctttcactgc acagactctg aatcctgcac ggagtctatt 178680
gactactaga gttgctaaag cttctccttc aatttcttct gcaatgatta aaagagggcg 178740
tccagattct gctacttgtt gtaaaactgg aaggaagtct ttaattccag agattttttt 178800
atcgtagatt agaatcagag cgtcttctaa aacgcattct tgagtttctg gatttgtgga 178860
gaagtagctg gagaggtatc cacggttgaa gttcattcct tctacaacgt cgagaacagt 178920
ttcgaagcct ttagcttctt caacagtaat ggatccgttt ttaccaactt tttccatagc 178980
ttctgcaata agatttccga tttcggaatc attatttgct gagatagtag ctacttgagc 179040
gatttctttg tgatgttgta caggtttact aatttttttg agttcatcaa caacaacttt 179100
tacggctttg tcgatacctc tttttaggtc cataggattg gcaccggcag tgacatttct 179160
tagaccttcg ctatagattg cttctgcaag aacagttgct gttgtagttc cgtcgcctgc 179220
tttgtcagca gttttgctgg cgacttcttt taccatctga gcgcccatgt tttcatgttt 179280
gtcttcgagc tcgatttctt tagctacagt aacaccatct ttagtcactt ggggagagcc 179340
aaagctctta tctataacta cgtgacgtcc tttaggacct agagtaactt ttactgcttc 179400
tgcaagagtt tttacccctt tatgtatttt ttntctggct tcttcattat atttaatatt 179460
tttcgctgcc atcgttgttc tccttaactt tctataatct gcaaactagt attttatttt 179520
aggacggcca tgatttcact ggactgtaga atgacatact cttcgtcatc gattgtgatt 179580
tcttgacctg catacttatc cattaaaatg atatcgccaa cttgaacttc gaaaggaagt 179640
agagtaccgt catcagttcg tttgcctgtg cctaaaacaa ggacctcagc acgatcttgc 179700
ttcctttttg ctgtatcggg taagatgatt cctccacgag cagtggcttc ttcttcttcc 179760

ctttttacca agattctatc gcccaaaggt ttaattcgga gggtcgttgc ttgatcagac 179820
atttatatgc tccttatgtt ttacgttcta agagactttg ctatttctgc aatcacgata 179880
acaaagcgtt tttttttgtg caataatttt agcactcaat aattttaagt gctaaaaact 179940
caatcttctg aaagcaagga agagagtagg tctatctttt tcgtaatgaa tgcaaaggct 180000
ttatctaatg gagcagaggt ggtcatatcc aatcccgatt tctttaatat attgagtggg 180060
aagtcagacc ttccgctttt caaaaatttt aaataaagtt cgagagcccc tggttcttgt 180120
gtaaganttt tttcagcaaa ggagagggca gctatgatac ctgtcgcata ttgataaaca 180180
tagaaattat agtagaagtg agggattcta gcccattcta atgcagatag ggaatccgag 180240
gttacaacac ctccgtagaa ttctttttgt aaattaccgt aagttgcgga gaggaactct 180300
tcagtaagag gagttccttg ttctgctgca gaatgaattt cgtattcgaa ggcagcgaaa 180360
aaggtttgac ggaacagagt cgcaaatatg gtgtctagag ttttagtgat aattacgatt 180420
ttgtcttctt tactttgatc tgatttgctg agagcttcca tgaggagcat ctcattgaat 180480
gttgaggcaa tttcagcaag aaacagaggg tattgggcat catgataggg ctgtgcttct 180540
ctactgaagt aggagtgcat gctatgtcca gcttcatggg caatgacgga aacgtcatag 180600
agtgtattcg tgtagtttag aagaatataa ggagcgctat catagcatcc tgaggagtag 180660
gctcccgaac gcttgtgctt attttcgtat ctgtctaccc aacgatttga aagaagacca 180720
tttcttagaa tttcaacata atgagtccct aagggaagga ggcttttgca aactaggtca 180780
acgccttctt cataactata attcttactt gtagtttggg aaataggagc ataaacatca 180840
taaaagtgga attcttttag atttagagct tcttttttta ggttaaaata ccgattgata 180900
agagaagtgt gtttctttgt ttcgtttata agattgatat aaacagttgt agggatgtta 180960
tgttggaata gggatgcctc taggcacgaa gggtaatttc ttgctttggc ttcaaagaga 181020
tgcgcttgga ctttcccatt gagaagattc gcgaaggtgt tacggtaatc atagtaacgt 181080
tggaattgag ctaagtaggc agtacggcgc aattcctgat ctggggattg catatacagc 181140
gaagccaggg catgggatag cggatgttct tctccgttcg aatctttagc tataccaaaa 181200
ggaatttctg catcgcttaa ggaagagaaa gctttatttg agacattaag ggctgcaaac 181260
gaggaggcta agatcttttc ttcgtttgct gttcctgtgt ggggagaaag acggaaaatt 181320
ttttctaggt aaaatctata gggagcgagc actgagctgg atagcaaggc agcgactttt 181380
tcttcagaaa gagcaattan agctggttgg atccaagaaa tttcctgaga aaagagagtg 181440
tagagataga caatggattg gtagtcgctt tccccttcgg gatttgtaat atcttgatcg 181500
tggatgaggt gagcgtatat gtagagttgg tctaattttc gttctacaga gaattttttt 181560
gataaaagtt cgagtaaaga ttcggggtta tcgatttgat aatgagaggg agagaattcg 181620
ggccatatgg gagaacgatc ttttccagag ctacaaagat cgaaatcttt tttccactcc 181680
tctctatttg cgtacatgag agttgtgtcc caacaatgct ttggatctac ttgagttctt 181740
gttgggagtg cttcggtttt cagttcagta gtcatgaggg aaggaactcc tttttaagag 181800
tcaggatgaa tgagaaagct cattataaga caatctttaa gaaaaagcca agaacaaatc 181860
ttggtttaaa gattagaagg ggttggccgc atctataaag gttgtggaaa taggaaattc 181920
gctttttaga tgctctgcaa gagattttgg acctactttt tctgtggctg tatgtccaaa 181980
tgctaggaag ttgatattgc tttctagagc tgtcgaccat gcaggttcat caaaatttcc 182040
tgtgatgaag caatcgactt gggacgtggc tgccgaagag agttctctat aagctcctcc 182100
tgagatcaga gctgctgagg agactctaga ggggccgccc aaggcagatc cttttagggg 182160
agcttggtaa tatcgagata acaggtcaat gaaagaatct atatcgatag gagagaaaga 182220
gccttgcact cctaaataag ggagggaaga accaaagggc ttcaagtcat gccaatttag 182280
atccagggca actctccagt tatttcctaa ggtagggtga gcatccaaag gaaggtggta 182340
ggcaatgagt tggatattgt gttctattag taattggatg cgcttatgga tcatgccggt 182400
aataggatag ggcatacctt tccaaaaaat tccgtggtgt acaatgagaa cgtttgcttc 182460
ggccgcaaca gcttgtttta tggtttctag atctgcggta actgcaacag cgattttctt 182520
taccggagtt tggggatctc caacttgaag tccgttgggt ccataatcct gaaatatttt 182580
tgatgagaga agagtctcaa gatgagaaag gagatccgca acattcatag aacagactta 182640
tttaaaaaat agcatttagt acacaagtgc ccattattgt aaaggttttg ttataaaaac 182700
aagtaagtat tcttagctca tcaaactatt ctatatgtaa gatattagct agttccatgt 182760
ttagtaatta taaatgcatt tagagtcttt ctgtactttt agaattttac atggcttgat 182820
gttttccttt agaaatnncc aaaaaaatgg gggttgtaat tcggcgtatc tatctgtggt 182880
agagaggatt acatttcata ttctataaac ttcggcagta ttttagagtg ttagtatttg 182940
gaatgtttta gtttcttagg ggaaaccatc cgcagatgag attttttcca acgtattaag 183000
ttttatcatc tctatctaat tttcagaaag agtatgaaga tgagccctgg catgtgaaaa 183060
gcagagacta ttaganaaat ctttgagccg aagagtcaaa gattttaatc aggctatcga 183120
taatgatgtt ctgtgcgtaa gttaggtttt cttcggtatg tgcggagcta atgaagtttg 183180
cttcgagggg agatggagaa aggtagactc cattatcaaa tacttcagag tagaaggttt 183240
gaaatttctc tacatcggaa ttttttgctt catcaaaatt tgtgggtgca gattctgtaa 183300
aaaagaggct gaacatcgtt ccctgatgta ctagagatac tgggaatcct tgagatcgga 183360
tttcctcttc aattggagaa taaaagagag cctctaattg gctgagatgg tcatagaatc 183420
cttcggattg gcataattga atggcggcgt gtcctgtagc catagcgagg aagttcccag 183480
acatggtacc tgcttggaat atggtgcctt cgggcatgag gtgatcgaga attgagcggt 183540
gtcctacaag ggctgcagca ggtaggcctc ctcctaagat ttttccatag atggtaatat 183600

cgggggagag attgaaaata tcttgagctc cttggaatgc cactcgaaat cctgtgacga 183660
cttcatccat aatagaaaga cttccgaagc gtttgcagag ttctatgata tcgtctagaa 183720
attcggcttt gggaaggacg atacccatat ttgcacatat aggttcaaag ataattcctg 183780
ctacttgagg tcctagagct tccatgacat ggtgtaggat ttggctattg ttataaggca 183840
gggatatcaa taaagaatgt ggagagggcg tgtgtatcaa tgaagttagg ttgtctatag 183900
tttcttcagt tgttgagatg cctccaagaa gagtatctgc atgaccgtga tatcccccta 183960
taaatttgat aataatagag cggtttgtga ttcctcgagc gagacgtact gcagtcattg 184020
ttgcttccgt tcccgaggat acaaaacgga ttttatgttc tttgagtttg agcgaggaga 184080
ggagcattgt tgcaaataga atttcctctt cagaggttaa gccataggag gttcctttta 184140
gagctgtttt ttggatagcc ttgacaattt tgggatgact gtggccgtga attaaagctc 184200
cccagcctcc acaaaaatca ataaactctc gtccgtgagt atctaggaaa atatctcctt 184260
gtgctgagct cactataggg ggtgtgactc ctacagaacg gcaggcccga acgggagagt 184320
tcacgcctcc tgggaagact tggcatgcct cttcaaaagt aacggtatgc ttttgatttg 184380
agcagttcaa catggaattt cttttagtga gtttccacat attttggaga agctgatctt 184440
taatggctag aaaaagccgt gagttagttt agcaatagat tgtcagggac tgtagaaagc 184500
gaggcatatt ttcctccaag atctttaaga actaaagccc agagatcttc tggttctgag 184560
taaaagacgt agtctttgtt ccctggagct agaaaccagt cattgcttag gaattctttt 184620
tcaagttgtc ctgcttgcca tccgctatag ccaaaacata ggttgatttc tggcccagat 184680
tcgctagagg cgatttcttg gaggaaggga agatctcctc ctaagtagac tgatggacaa 184740
atttctaacg tttgttcagg aatttcggag catgaatgaa gtaacatcat ttggtttgct 184800
tgtagggggc ctcccntaca aaagcggata ttatgattgg agactttttc aaaggtaaag 184860
atgtcatctg agatttcaaa tcccagggtt ttatttaaga tgagaccgaa agaaccattg 184920
aggctatgtt cacaaagtag gatgacacta cgagcaaaga ctccttggtt tatattcagg 184980
agaagcgact aacaaagatc ctttttctag gcgtgcataa ggaattttca taatatctct 185040
gagtttattc ttcgtagggg actacagcat caaatactga tttaacacac aagatgggat 185100
tttatgagtt aaggcgagag ggatctatcg ctaggacggg catcgacatc aacgacgtat 185160
agaaattcgc gatctttttg ttctaaaaag agtcttgtgt agaaaacgtt atctttatag 185220
tcattaatta ctacacgtaa cacttggata tcgaaacctg aaaagacaaa gttcagtaga 185280
tcatgagcaa aaggtctttg aggagagtgt ccttcagtat cagcaccttg aaatgcttgc 185340
cccatggaaa cgtgcccata tatagcgaac tttttttctt cagtacctaa gatcatgcct 185400
gcataattac aaaaactgac aagtttgtaa aaattgagaa gtactaaggg ggtttcttct 185460
aggagttctt tttctaagct catacagaat attttttgct gattagacct tggctattgc 185520
tagaccacac ttcgactttc tctataacaa acccgacttc aataacacca tgaatttgta 185580
ttaactttaa taaatccttt tcaggatttg ggtaggagtt gggggaaaaa atatcgtaaa 185640
tgtagttgct gctgtctgta ataaataggt caccagtatc ttgtaggcgc cattctcctt 185700
catatccaag atggcggatt tcttcaataa ttgctgagcg accgaatcga ctgatttcta 185760
aaggaacacg aaattttcct aggactggaa ccagtttact ttcatcaaca aggataatgc 185820
tgcgttttgc tgctcttaaa agaatctttt ctctgaaaaa ggtccgccac cacctttgat 185880
catccgcaat tgagggtcga cttcatcagc accgtctacc gtaagatcta gggaagagaa 185940
tttttctggg tttaagaggg ggatggcaag ctgctttgct agagcataag aattttgaga 186000
agaagctata gcatgaactg ctaaggactc tgtttgaatt ctatgggcga gtgcaaagat 186060
aaattcctta gctgtagatc cactgcctaa gccaagaatc atgcctgaag ttacttgtgt 186120
agcagcctca tgggccaggc attttttctc atgaagatga agatcttttt ccacagcgct 186180
actactatgt tacaaaatat tcccacagct tatgcaaagg gattcttcca atactataca 186240
aaaatcgaaa ccttttggac gagtctttag ggtaatcgat tttcagatct ttaaatgaaa 186300
gcgtattgat cctattttct gctataggtt ttagtgggta ggaacggatc ttttgtattg 186360
cattgtcatg ggatactcta gaaatctcga agtgcgaaca aaaattaaga atggaattct 186420
tagtagacaa tagttggagt attttgaagt gaaatttttc caaagtcccc ttcgatttag 186480
aataggagtg actttaaaat taagatagaa aacgaaccta gataacgtac ataaagggaa 186540
gagctggttg gatctataga ggttccaacc agctctggtt tcattatttc caaacttctt 186600
cggaaatttc ttttactaga gcaactttag cccattgctc ttcttctgtt aatttatttc 186660
ctatttcaca tgaagcaaaa ccacactgtg gacttagaga gagtctttcc aagggcaggt 186720
agtctgctgc ttgatgtatg cgagcaatga cctcatcctt attttcaagt gtaggggttt 186780
tgctggtaac aagacctaag cagacagttt tttctccaga aatgaaggtg agaggagaga 186840
agtctccaga acgctcatga tcaaactcta aatagtagcc gtctacattt gtttgttcga 186900
ataggggctt tgcaataaag tcataactac cactagcaaa gaattttgag tggtagttcc 186960
cacggcatac atgtaaatta acgactagat catcgggacg atctgcaatt acaagattat 187020
taatcagaag atattgttga atcagatctt gaagaccttt ttcatcgata ccataccacg 187080
aacagactcg agggtctact aaacctcccc gagtacagtc atctaattgg agatagcggc 187140
agccagcatc ataaagatcg cgaatgactt tacgataacc tgcaacaata tcttcaatta 187200
gctcctgatt tgtaggatag aatttacgtg tgacctctat attattaggg aagatcatct 187260
gctttaaaaa ctgtgccggt gcaggaagag tttgctttgc agtcgtaaat tcatcttcta 187320
gagcttttac aaatttaaag tgatccacaa atgggtggtg agatacagag atcttgtctg 187380
tcagataggt atcatcgatc atagcgcgtt ctccatcaaa gaaaactcct tctgtagctc 187440

tgtggtgacc tacgccatga aaaccccaca tgaagtcgta atgccacgta gctctgcgga 187500
attctccatc agtaataaaa gaaagacctg ctgctttttg ttttttgatc aaatcttgga 187560
tagcgatatc ctcaatttgc atgagttgat ctagagaaat agagccttct ttaaggcttt 187620
ctctagtttt ttttaaatgc tcaggacgca aaaaactacc gacaacatca aaatgagatt 187680
tcagaggtct ttttagtgaa gtattcatga ttgtcctcct tcagactagt gaaatggaaa 187740
gagctaagta gaaaaacgtc gaattttcta taggattaaa aataagaata aacaagttat 187800
aggcgtttct gaaagacaga atcgttactt aatactttcc acaatgttct agcatttaag 187860
aacataggat atattttgtc caactataac tagatcattt aaaaatattt gtaatttagt 187920
tggaatatat aaaatcatta gcattaagca aactgttttt cttttcttag tttttagatg 187980
cgaataattt gataatttct caggtaatta aaaatctttt aattgttttt tctgtttatg 188040
tttgacaagc acaaggtgga tgccactcta tagtcacgat ctttaagtcc ttgatttgtg 188100
aagaaattgg tccttaggag ttgccgcttt cttcagacag ttcaagagat cacttcctaa 188160
tgtattttta gaaaggggaa agagatcctc ttaggttaga cgtttaggtt tggtagtatt 188220
tcgatagggt ttgataaagt tgcctcttct acattctcag aggaaagttt atagattttt 188280
tattattcct atgtaataag aaaaaccttt ttaaaaagtg cttggggtga attttatgga 188340
gaaattttcc gatgctgtct ctgaagcttt agagaaggct ttcgaacttg ctaaatcttc 188400
gaaacatacc tatgtcacag aaaatcacct attactggct ttattagaaa atacagagtc 188460
tctcttttat ttggtaatta aggacattca tgggaaccct ggtttgctca atacggcagt 188520
taaagatgcg ctctcacgag agccgactgt agttgaagga gaggtggatc ctaaaccttc 188580
tccgggttta caaacccttc ttagggatgc caaacaagag gcaaagacat taggagatga 188640
atacatttct ggagatcatc tgctgcttgc tttttggagt tcaaacaaag agccttttaa 188700
ttcttggaag caaacaacaa aagttagttt taaagatctt aagaatctga ttactaaaat 188760
acgacgagga aatcgtatgg attcgccaag cgctgaaagt aattttcagg gtttagaaaa 188820
gtattgtaaa aatttaacag cattagctcg tgaaggtaaa ctggatcctg tgatcggtag 188880
agatgaagaa attcgtagaa ccatccaagt gctttcccgt agaactaaaa ataaccctat 188940
gcttattggt gagccgggtg tagggaaaac tgctatagca gaaggattag ctcttaggct 189000
tatccagggt gatgttcctg aatctctcaa aggtaaacag ctttatgtct tagatatggg 189060
agctttgatt gcaggagcta agtatcgagg tgagtttgaa gaaagactaa agagtgtttt 189120
aaaagatgta gaatctggag atggcgagca cattatcttt attgatgagg tgcatactct 189180
tgttggagca ggagctactg atggagctat ggatgctgcg aatcttttaa agcctgcatt 189240
agcaagaggg acgctacact gtattggcgc gacgactttg aatgagtatc agaagtatat 189300
tgaaaaagat gctgctttgg aacgtcgatt tcagcctatt tttgtgacag agccttcttt 189360
ggaggatgct gtctttattc ttcgtggact aagagaaaaa tatgaaattt tccatggagt 189420
caggattaca gagggggctt tgaatgccgc agtcctactt tcctatcgtt atatcccaga 189480
tcgctttctt ccagataagg ctatcgattt gatagatgaa gcggcaagtt taattcgcat 189540
gcaaattggt agtcttcctc ttcctattga tgaaaaggag agagagcttg ctgctttgat 189600
cgttaagcaa gaggctataa aacgcgagca atctccttcc tatcaagaag aggcggatgc 189660
tatgcagaag tctatagatg ctttgagaga ggaattagca tctctacgtt tgggttggga 189720
tgaagagaag aagttgattt cggggctcaa ggaaaaaaag aattccttgg aaagtatgaa 189780
attttctgaa gaggaggcgg agcgtgttgc agactataat cgtgtagctg agcttcggta 189840
tagtttaatt ccccaacttg aagaagaaat caaacaggat gaagcctctt taaatcaaag 189900
agataaccgt ctccttcaag aagaagttga cgagcgattg attgcgcaag tggtagctaa 189960
ttggacaggg attcctgtgc aaaaaatgct agaaggggaa gctgagaaac tgttaattct 190020
tgaagaatcc ttagaagaac gtgtggtagg acagcctttt gcagtctctg cggttagtga 190080
ttctattcgt gctgcacgtg taggtttaaa tgatcctcaa cgtcccttag gagtcttttt 190140
atttttaggg ccaacagggg taggaaaaac cgagcttgca aaagctcttg cagatcttct 190200
tttcaataaa gaggaagcta tggtccgctt cgatatgtca gagtatatgg aaaagcattc 190260
catttccaag cttataggat cttctccagg gtatgtgggt tatgaggaag gtgggagtct 190320
ttctgaggct cttcgacgac gtccctattc agtagttctc tttgatgaga tagagaaagc 190380
agataaggaa gttctaaata tccttttaca ggtttttgat gatgggattc ttacggatgg 190440
gaaaaaacgc aaagtaaatt gtaaaaatgc cttgtttatc atgacatcaa atataggttc 190500
tccagaactt gcagattatt gttcaaaaaa aggaagtgag cttacgaaag aagcgattct 190560
ttctgtagtc tctccagtat tgaaaagata cttgagccct gaatttatga accgaattga 190620
tgagatactt ccttttgttc cattaacgaa agaagatatc gtgaaaatag ttggcattca 190680
aatgcgaagg attgcccaga gattaaaggc acggcggatc aatttatctt gggatgattc 190740
tgtaatatta tttcttagtg aacagggtta tgacagtgct ttcggagccc gccctttaaa 190800
acgtttgatc caacaaaaag ttgtgatctt gctttctaag gctttgctta aaggagatat 190860
taaacctgat acatcgattg agttgacgat ggcaaaagag gtgctcgtat ttaaaaaagt 190920
ggaaactcct tcttagagag ttttctatgg gtgcggaatt ttagatacta ggaaaagccc 190980
tctttgttaa gagaatggat aggtttttag attctatgtc ttcttgctac gctttcttgt 191040
ttcccgggat cataaaaaaa ctaggacgat aaagtgtgtg taggattaga attgtgcggc 191100
aagcttcatt tggctaaaca agagcagtca cagaaattta agaagtccta tgtttaagag 191160
ctttatagta aggtatatgt ttgtaggtgg ccttgtttca ttcttgcttc ctatccccga 191220
cttggaatgt gcgaataatg taacaaaaac ttatgataag aaagcttctg ttatatccag 191280

agatcttaag ctacaggaag actgccagaa gttttggaat cttgatccgt ataaactaga 191340
aagtctttgt gcttatcaag tgctttacca tgatgactat agttccaaga gaatacgaga 191400
gctttttcct caaatccaaa aagacgaagt ccccatattt gcaacaatga ttcttacttt 191460
agggaaagta gaccgtggct tttctcctga agaaatttca ttgatccaaa aactttctta 191520
cccaggcctc tcattggctt ctttgagagg gtctacagaa attagacccg aatacagatt 191580
tggctcgtgc tttagtagtg tcggagtttt ctggagattt agggaagaac cgagctgact 191640
actatagcaa ttgccttgat attttggcgt tgcgtattca tgcagaacgt caaaggtatt 191700
tagatcagtc tccttgtgtt cctggaacct ccgagtttca taaggcaact atagaagcta 191760
ttaatacgat actcttctat gaagaagcag ttcgttatcc ttcgaagaaa gaaatgtttt 191820
ctgatgaatt ttcttttctt tcttcagtta cagatagaaa attcggcgta tgtttagggg 191880
tctcttctct ttatttctct ttgtcacagc gcttagattt acctttagag gctgtgacgc 191940
ctcctgggca tatctactta cgttatcagg gtggtgaggt gaacattgag actacagctg 192000
gaggcgccca tcttcctaca gcaagttact gtgattgtct agatttagaa gaccttcagg 192060
tgcgtactcc tgaagaaatg atagggctta cttttatgaa ccagggctct tttgctctgc 192120
agaagaaaaa gtataaggaa gcggaagagg cttataaaaa ggctcaagag tatttgggag 192180
acgaggaact acaagagctt ttggggtttg ttcaaatcct aggaggaaag aaaaaagagg 192240
ggaaatcttt gattggtaaa agtcctcgcg cttcccagaa aggatcggtg gcttatgact 192300
accttaaagg tagaatcaac attccaacac tagctctttt attttcttat ccaggatcca 192360
attatgaaga gatagcttct tatgaagaag aactcaaaaa ggctatgaaa agctcgatgc 192420
catgttgtga aggacagcgt cgtcttgctt cagtagcatt tcatttgggg aagacagcgg 192480
aggcggttgc tcttttagaa aaatgcgttg aggatatccc taatgatctt tctcttcatt 192540
taaggttatg taaaatccta tgtgatcgac atgagtatac aaaggctttg aaatacttca 192600
taattgcgga aagacttatg gaggatcagg gatttcttaa aaaagacaat cgttcgttcg 192660
ctttatttta tgaggtgaaa aaaatcatat ccaaagtggc tcctcaaaaa gctaacacct 192720
tgcttttaat ggagtctgaa agataacttg atcagttctt tctgtattgc tcttatttat 192780
aacatgttat aacattgcaa gtgttaattt ttaacagatc tttatttgtt gcaatatttt 192840
tttaaataag aattgagcta ttttttagcc tcatattgca gatgtcatga aagatttgaa 192900
tcaaagtaag tcttgctttc caagctttgg taggattaaa gtgtttgaga tgaattcgca 192960
ttttttaaat tattgttgga attagtatga gcagttcgga agttgttttc cagacagttc 193020
atggccttgg ctttggtgga ttgtcttcaa aaagtgttgt cccttttaag aaaagtcttt 193080
cggatgcgcc ccgtgttgtg tgctcgattt tagttttgac tctggggttg ggagcgcttg 193140
tttgtggtat tgccattact tgttggtgtg tcccgggagt tattttaatg gggggaattt 193200
gcgctatagt tttaggtgca atttctttag ctttaagtct attttggttg tggggtttat 193260
tttctaattg ttgtggttct aagagagttt taccgggtga gggattgcta cgggataagc 193320
ttttagatgg tggattttca agagcggcac cttcaggaat gggacttccg ggtgatggat 193380
ctccaagagc gtcaacgcca tcttgcctag aggaacttca agcagagata caggcagtta 193440
ctcaagctat cgatcagatg tcagatgatt gactctaaag cgtagaggta cttaagggag 193500
aggctttgct aatcagtaaa gaaactttaa tacaagtaag atctaagttg aataacttaa 193560
aaagataatg aataaaaaca aaatagcatg ggggaccaat ggctgttcaa tctataaaag 193620
aagccgtaac atcagccgca acatcagtag gatgtgtaaa ctgttctaga gaggctatac 193680
cagcatttaa tacagaggag agagcaacga gtattgctag atctgttata gcagctatca 193740
ttgctgttgt agctatctcc ttactcggac taggtcttgt agttcttgct ggttgctgtc 193800
ctttaggaat ggctgcgggt gctataacaa tgctgctggg tgtagcatta ttagcttggg 193860
caatactgat tactttgaga ctgcttaata tacctaaggc tgaaataccg agtccaggga 193920
acaacggtga gcctaatgaa agaaattcag caactcctcc tctagagggt ggtgttgcag 193980
gagaagccgg tcgcggcggg gggtcacctt taacccaact tgatctcaat tcaggggcgg 194040
gaagttagat tttttatcta acctactaag ttagtatttt aactgtaggt ttttccttcc 194100
gttgttttaa aagaacctca agaataacta gaggttcttg tttgtttatt gcaatcttcg 194160
tttttgctat ctatagttaa cttatataaa tataaggcaa atggtggaga gttagctcta 194220
tggaaagtga gaaagatata ggagctaagt ttttaggtga ctataggatt ctctatcgca 194280
aggggcagag cctatggagc gaagatcttt tagccgaaca tcgatttata aaaaaacgtt 194340
accttattcg attacttctt cctgatctag gaagttctca accattcatg gaagcttttc 194400
atgatgttgt tgttaaacta gcaaaattaa accatccagg catcctcagt atagaaaatg 194460
tttctgaatc tgagggaaga tgtttcttgg taacacaaga gcaagacatc cccatccttt 194520
cactaacgca atatttaaaa agtattcccc gcaaacttac agagctagaa attgtagata 194580
ttgtaagcca actcgcttct cttttagatt atgtgcattc agaaggactg gctcaagaag 194640
agtggaatct tgattctgtc tatattcata ttttgaatgg tgttcctaaa gtcatactcc 194700
ctgatctggg gtttgcttca ttgataaaag aacgtatttt ggacgggttt atttcagatg 194760
aggagaatcg agaatctaaa ataaaagaaa gggtactact tcacacttca gaaggaaaac 194820
aaggtagaga agatacgtat gctttggtgg ctatcaccta ttatttactt tttggttttc 194880
ttcctcaagg cattttccct atgccttcga aagttttttc tgattttatc tatgattggg 194940
attttttaat tagctcttgt ttaagttgtt ttatggaaga aagggcaaaa gaacttttcc 195000
ccttaataag aaaaaaaact ttaggagaag agctgcaaaa tgttgtcact aactgtatag 195060
aaagctcttt aagggaagtg ccagatcctt tggaatcttc tcagaatctt cctcaagcgg 195120

tccttaaagt aggggaaacg aaggtaagtc accagcagaa ggaatctgcg gaacatttag 195180
aatttgtgtt agtggaagca tgctccatag atgaagccat ggataccgct atagaatccg 195240
aaagtagttc tggagttgag gaggaagggt attccctagc tctacagtct ttattagttc 195300
gggaaccagt agtgagtcgt tatgtagaag ctgagaaaga agaacccaaa ccgcaaccca 195360
tacttacaga aatggtttta atagagggag gagaattctc ccgaggaagt gtcgaagggc 195420
aacgtgatga gcttcctgta cataaggtaa ttttacatag ctttttctta gatgttcatc 195480
ctgtgacgaa cgaacagttt aatcgttatt tagaatgttg tggtagtgaa caggataagt 195540
attataatga gttaatccga ttgcgagatt ctcgtataca gcgtcgttcg ggtaggcttg 195600
ttatagagcc aggttatgct aagcaccctg tcgttggggt tacttggtat ggagcctcag 195660
ggtatgcaga atggatagga aaacgcctgc ctacagaagc tgaatgggaa atagctgctt 195720
ctggcggggt ggcttgctac gctatccctg tggggaggaa atcgaaaaaa gccgggcaaa 195780
ttttttcact gcggatacga caacagtcat gagttatcca cccaatcctt atggcctcta 195840
tgatatggca gggaatgtct acgagtggtg ccaagattgg tatgggtatg atttttatga 195900
aatttctgct caagagccag agagtcctca aggtcctgct caaggagtct atcgggtgct 195960
aagaggggga tgttggaaga gcttaaaaga tgatcttcgc tgtgctcatc gccatcgtaa 196020
taatcctggg gctgtaaata gtacgtatgg ttttaggtgc gctaaaaata tcaattaaga 196080
gaggttcatg aaggaagaga attcacaagc acactactta gctttatgtc gtgaattaga 196140
agaccatgat tattcttatt atgtgttgca tcgtcctaga atctctgatt atgaatatga 196200
catgaaatta cggaagcttc ttgaaataga gagaagtcat cccgaatgga aagtcttatg 196260
gtctccctca acacgtctcg gagatcgtcc ctctggaact ttttctgtgg tttcccataa 196320
ggaaccgatg ctttccattg ccaatagcta ttctaaagaa gaactaagtg agtttttttc 196380
tagggtagaa aaatccctag gtacaagtcc acgttataca gtagaactta aaatcgatgg 196440
gattgcagta gcaatacgtt atgaagatcg tgtgttggtt caagcactca gccgaggaaa 196500
tggaaagcag ggagaggata tcacatcgaa tattcgaaca atacgctcct tgcctttaag 196560
acttccagaa gatgctccag agtttattga agtacgtggc gaggtcttct tctcttattc 196620
tacgtttcaa attatcaatg agaagcagca acaattagag aaaactattt ttgccaaccc 196680
gagaaatgct gcaggaggta ccttaaagtt actttctcct caagaaagtc gcaaacgtaa 196740
attagaaatt tctatctata atctcattgc tccaggagat aacgattctc attatgaaaa 196800
tcttcagcgc tgccttgaat ggggatttcc tgtatctggt aaaccaagat tgtgctctac 196860
cccagaggaa gtgatctcag ttttaaagac tatagaaact gagagagctt ccttgcctat 196920
ggaaatcgat ggtgctgtca tcaaggtaga cagtttggca agtcagagag ttcttggagc 196980
cacagggaaa cactatagat gggccttagc ttataaatat gccccagagg aagcagagac 197040
ccttcttgag gatattctag ttcaagtagg aagaacggga gttctgactc ctgtagctaa 197100
actcactcct gtactgttgt cagggtcttt agtatctaga gcgtctctat acaatgaaga 197160
tgagattcat agaaaagaca tccgtattgg tgataccgtt tgtgttgcta aaggtggaga 197220
ggtgattcca aaagtagttc gggtatgcag agaaaaacgt cctgaaggtt ctgaagtttg 197280
gaatatgcct gaattctgcc ctgtctgcca tagtcacgta gttcgggaag aagatagagt 197340
ttctgtgcgt tgtgtcaatc ctgagtgtgt tgcaggagct attgaaaaaa ttcgtttttt 197400
tgttggtcgg ggagctttaa atatcgatca tttaggggtg aaggtaatca caaagctgtt 197460
tgaattaggg ttagtgcaca cgtgtgcgga cctatttcag ctgactactg aagatttaat 197520
gcaaattccc gggatacggg aacgctctgc aagaaatatt ctagagagta tcgagcaagc 197580
taaacatgtg gatctagatc gttttcttgt tgctctgggg attcctctca ttggaattgg 197640
tgttgctact gtactagctg gccacttcga gactttagat cgggtaattt ccgcgacttt 197700
tgaagaactt ctttcactag agggtattgg agagaaggtg gctcatgcta ttgctgagta 197760
tttttcagac tctacgcatc ttaacgaaat caagaaaatg caggatttag gagtgtgtat 197820
atctccttat cataaatcag gatctacgtg ttttggcaag gcttttgtga tcacagggac 197880
gttagaggga atgtctcggt tagatgcaga aactgctatc cggaattgtg ggggtaaggt 197940
aggctcctct gtctcgaaac agaccgatta cgtagttatg ggaataaccc aggatctaa 198000
attagagaag gctaggaaat tgggagtctc tatcttagat caagaagcct ttacaaatct 198060
aattcattta gaataattta ttttaaaatt ttcttaatac attaattctt atttgtaaaa 198120
gttttattta aattatttat tataaattct tttacagcta taattgtccg tattttataa 198180
gtttttttgt tcttttggga gtaaacatgg cttcttcttc aaacaattcg actaaacagg 198240
acggcatacc atcttgggta aacccaaatg tccagtggaa tcgagcgtcc caggtgggtg 198300
atcaagaagc gaattctcta actccagagg ctcaaacttc acgtagctgg ttttccgatc 198360
gcaagcattt tcttgaagtc ttagacgtta gtctagagga gatggagaac aatgacctta 198420
agaaatactc tagatataag acgattatcc tgattgccac gctggtcact gttgcgatta 198480
cctgtatcgt tcctatctct atggtgtttg gtatcccgat gtgggtgccc tgtcttattt 198540
tatttggagc gggtctttct tcggcttttc tttctcatcg tcttcaatct aagtgcaagg 198600
agatccattt aagataccga gcgtaccaga tttatcgcca gcagctgttg agtcagtacc 198660
ctgacttgag aaagtctact ctctataaat atagtattac ccatgtcaaa ccgaaaaagg 198720
gatttgttgg taaactcgta gaaaatttgc gccctgattt gcataaaaat aaggacgatg 198780
ggggtgctgc tgcagactcc agattagatt ttgcgggata tggagtaaag cattatcaga 198840
cggatgctct acttggagtt tcaggtgtta atagtgtaga atggcaacgt cttgcctctc 198900
tgattatgag tgttaagaac gacattttaa atgatgtggg aagcagagag cccattgata 198960

aagcgcaaag gtctgcttta gtagtcagtg gtaaggatat tggaggggag attcagcctg 199020
gaggtatttt agatatttcc agagatattc tagcgatctg tggctacggt atgaatgtag 199080
gtgttgaggc gaagaaagct atagaccagt ataagaagtg gtatctcaat agtagtacat 199140
ttattgcttg gaatccgcag cttcctgcta ttgcccagtc ctatttacta gaacaacaac 199200
gacatctaga ttatgctgct aagattttcc aagatctttc cgcattgacg acagcccatg 199260
gtacagggca ggctcttgaa gatttagata gtttgctttg ttattatgat cagttaattg 199320
aatctaaagg tgtcggtgaa aagataatag catcgattca ccagaaagca tctcgactta 199380
gcaatgcaag attcctgcga tcaggaacat ttaaagaaat ggtcgaatct ataccacgtg 199440
ttttcaatta ctattaaaga attcactgaa ggtaagctag aacaaaatga ggtagtatct 199500
agaatacaaa ggcttcgagg taagttagaa aaaagtaaat gcagcattct tggaaattgt 199560
cgaaccaacg cagaatatgc aacaaagtct gaaaaaaaac tcgcagatta tttgctgcag 199620
attggggata gagaaccttt ccttactgga atgcataagg cgatagccac cggaaaagct 199680
attcaaggaa aagtggaagg agtcatttca caacatcctg aaaagcaaat tatgatgctt 199740
cggtgttcta tagagagact cgaagggatg ttgcgtcgag aggattgggg agcaatccta 199800
caaaaaaacg aagacgaagt ccttgcattg aagagtacaa tggaagctca gcttcaagga 199860
tttaaggacc ttgtaggtac ctgggaagga aaatatcagg aatttaagaa aaacaagctt 199920
tccaaagttt tagtttacga cttcacaaaa tcctattcta accttctaaa tcgtttggag 199980
gtactccatg ccgagagctc cacggatgat ttggtattac atgtcgatag aatgtcggaa 200040
gatctgaaga aaacaatcga ggagattgac ggcaatttat ttcaggtaac tcctgaagag 200100
ctctctttgt tagctcggga atatcaggga ctcatgaatg aacttcctct gatcgttcaa 200160
gaggggaatc ggctccaaga agcaatctct agtgaagggg tttctcaagg attgatgttg 200220
ttgaactctt tattgaatag agatgaaaaa ataaataaaa acatagaaag cagtaggaaa 200280
aacttagtag ctatcgcgaa acaagcacgt agcgatgcga gaaatataga cagtcaggga 200340
ttggctcctt tgatccaaag gaatagagct agcctggaca acattctcca gaatatgtat 200400
ttgtttaacg gcagtatacg taatatccat gctctagata cggaaacgtt agtggcaact 200460
tcctctaata tgttttctgc gatgcatacc ttcgactgga atatctatac gaatttgctt 200520
gatgttttag aaatccaaag caaaccagct cctgccccta tggagaatcc tgaccttcct 200580
ggagctcttc ctgaagaggt ccaggatgcg gttgctgaag atgtttctgg gactcacagg 200640
ctacatcacc aggtgttaaa gagacgctgt gctgacttaa aaaatatgat cagtcaattg 200700
cagaagtcga taaacaaatg gggaatggct aaggccattg tcctgggaat tgttgcggtg 200760
ctcttctgtg ttcttagtgc tatttttatt ggtcagaaca ttttatcctt actcattctc 200820
tcttgtgtag ggttactttt gactcaggta tgtcctttaa tctttgatcg tatatctaag 200880
agcaaggagt ttgagaagca agtgcttgag acagcgcagt ccttgattcc tgccactaag 200940
attcttccct cagaattcaa taataaggat cttaatcgtt tagctaagct ccaggataat 201000
ttaaatcttg agggttttgg tcctacatgg gcgcgcaata ttgtgagtga tctagagggc 201060
attccgacta aagaaaagag cttgaaggat cttactaaag agttccgtaa ggattctaaa 201120
aacttaaata agcgtataaa aagacgtttc aaggaggggt taggacaaga agcgcctgtg 201180
gttcgtccta ctatccccca agatattcgt ggagctgagg tttttgcaga gttacatcgc 201240
gagttagagc accttcaaaa gcaaaaagaa gagattagta ttcggggaga tgctctggtt 201300
caagagcgca tgggtctgtg cttagaaaag tctaagtacg acaatgaaaa ggctcatgct 201360
gccgctatga ctaagaaggt tggaaaatta caaaacatag ataggcttca aaaaaataat 201420
gaaacgtatg taaggattca gaattttttt agaactttga ttcaagagaa attagggcgt 201480
gacacagtcc aagagataga cgtagtcaaa gaggctaagg aattacacga attagcagca 201540
atcatttacg gcaataccag tgggaaatct cagaagcaaa gagcaaaaaa gcagtttaaa 201600
gagaatgttt tacacatagc agggaagggt caattagaac ttttagaggc ttacttgaat 201660
gtgacagctt ctcaagggct ctgtcgccat caaatgcagg cttcatttag agaaagaatc 201720
ttgctaaatc ccgatggagc aaaacatgga gaagccgaga ggacgcttgc ttctagggaa 201780
gaaatgttga aaactctagg gctttcttat ttgacgcctt ttgtaagatt ttcttctcca 201840
gaaagtacgc agtctggata taaccaaatt ctgaaagtcc gtgagcagct cttcgatatt 201900
gagcagaggc ttcagaatca ggagactgtg agtcccgagg actatgcggc tgtacaagct 201960
gctttagcag cttatgtccg caagcatgaa tctcttatag tttctactta tggattgggt 202020
gctcaagaag gacaaacgag ttctaaagtg accactttaa tgcgagattt gcatgctgta 202080
gaagagcttg ttgagatggg tgtcgaaacg tatcgattga atcgcagcga tcagattctg 202140
catcgcgtgc attctgtttt acacagccat ctgcgagata gcgattcttc aggaaatgga 202200
attattgatg tagttaagaa attgtttgag cttctgaaca ataatgggaa caatcctaat 202260
gatcccgaat gccaaaagta tatgcagata cttttagatg caccagtcag tctattgtat 202320
ggtgcattta aaagtttcaa aaacgaattt ttacttaatt tcacggaatt gaatattgct 202380
aattcaacaa aagctgctga ggaagaagct aaaaggtatg ttgaagagaa aggtagaggt 202440
tttgagactt attgggagga ggctaagcaa cgattggaag caattgctgc tgagttggac 202500
gacttaagga atcaagagac tctattggaa caagaaattc gtttggcgaa tttaaagata 202560
agtatcttta gtgatttaaa tttaagagag aaggtttcag tagaaaaagc agctttagaa 202620
gaagaaatcc aaggaataca agagcaatat gcagagatgc aggggattga agatctagag 202680
ttaaaacaaa aattcgaaga tttgcaaaag aaacttgaag ctctagaaga aagattgttg 202740
caaataggtc gaaggataga ttcctctgta gacaagcaga aagaactgtt gggtctcttg 202800

ggtagagaag aggctgctta gagaaatcat tgcgtttgca gatctcattg atggtcagag 202860
attctttttc tcatcaactg caatgaaaac catagaggac taggaaaggc tctctatggt 202920
tttttttcac aatttccact aaatgcccac aaacgatagg tagaccttcc aataggatag 202980
cgtcctaaaa aggagttagc tggtcttttc gctggcaaaa attctaagta gatatgagat 203040
cagctcgtgc agcttaaagg tatgtgttct atatccgata taacgatcgg ggcggataat 203100
aaatagcgaa tttggatttg cgtgatagag gttaaggatt cgaggttcct taacattgca 203160
aatctctatc cattcgccat attcttcttg tagagcttcc tttaagtcgg ggatatcttt 203220
aaaaaagata agaaggtgct tactactttt taaaggatct aagaggaaag aaccgttttc 203280
taggcgagca tctatagctc tcattcctgg accaggacca tggatttcct tatcttgagg 203340
agacattttg ataatatcgc tagaacggta tttcagtgct tggtgagggg ggtagtaata 203400
ctcttctcct gtagtattaa actttcgaca tccctttaaa aagtagtaca tcaaagcagg 203460
tgtatagaag cgagaaaatg ggagtttctt cgcgcgcttt tccgtagtag gactaatata 203520
aggtaggata ttgccatctt cctgttcttt tgtaatcacc aaatgtttta atgcagcttt 203580
tttcaataca ggaagtagct tccaagcgag attaaaggct gcgtgaatat tggtattaat 203640
accgttgaga taagaaagaa gcagagtatt agagaggcta cctaaaaata atacatttcc 203700
atgttcagga gggaatgcgt ggtgacttgt ttttatatgg aaattttcat cagagatgac 203760
gagattgtaa gtataaagta gcttctgttt aagtttcggt gatatggaat gcgttccttg 203820
gggtagacag agctgtttcg ttttttcctg gggattatag aaaacgaaat ttaagaagtt 203880
ctttgtgatg ggaagaagat ggatatgatc ttcttcaaag ggctcgccct catcgcaatt 203940
gataaaaata acttctcgat ttattctacg tgctctcagc tggcttttga caagatccct 204000
gatgtctagg ttgttgtcag cctcacaggc tataatccac tttggattgt agatctcacg 204060
attttcaaaa ttttgtgata ctttagtact ttcaataaag atactgttat caactagagt 204120
tacggggcgt gtcgaccaat ctatgacgcc tccgcgtttt agaaactcgt caattaggtg 204180
ctgttctaaa ctttgatatg ttgttgatag agaaaaagga actggagagt ccgttgcttg 204240
gctgaactta aataataagg ttctcttttt ccaatggtaa cgcgcaccaa agatcttatg 204300
gttggcttgg ataaaatcgc ctagcatttc actattgtga agaagctcca aggaagagca 204360
agacaagatt acagggagct tacgacaatc taagaagcta ggatcctcag gagaagctct 204420
gtggtcgata acttttacag agatcccatg ttgtattagc atatttgcca aaatgagacc 204480
tgtaggatta gcacctatga ctaaaatgtc tgccatactt gccctcggat agtgaaaaag 204540
attttcataa cattatagga taaaatccta ggaggaatca agaaacgaat tccaagagct 204600
atgaatatag gtgcttccat ctagaagttg tatttaaaat tgtagtgttc tagaaccgtt 204660
gtaccttgaa ttaaggtaca actgaaattt aaagtgttcg ttggaagtgg ctcacaattt 204720
gatctaagat catatctaaa tcaggagctg tggctaagtt gtgtcctgtg ttagggtaag 204780
agatgaaagt cattcttccg ggagctgtgt ttttaaatag tgtctgttgt gtcctagaaa 204840
caagagtgtc atcaatgcct tgttgatgca aaatataggg ttttgtgggt agggaatttg 204900
ctgtaacgtg atcttggatg cgtatgagga gatcaacatc gccagagcaa acaattatag 204960
gaggaggacc aaatccaaag tccttcccaa cagagataat atctccttcg ccgtgtttag 205020
agaaattctc atagagctct tttaataaga tgcccccatc tgcaattgga gcccatacac 205080
tcagggcttt gatatttaag tctcttgggt tatagatttt agccaactcg aaagctatgt 205140
ggcatcctaa agaaaaacct gaaattccta aacgatatgc attgagatct gggtgttctt 205200
ggacagtttc aagtatggtt tgtgcatcac gtaaataggt ctctatagga acttcttcag 205260
caactccttc actatctcca catccggcca tgtcgacacg taaagtggca attccagctg 205320
cagcgaattt tcttcctaat tttcgatagg ctccagttaa acctccgaat tttgttcctc 205380
ggaagccgtg aaacaacacg actgtaggga accctccttc cggtgtggga gtgttaggaa 205440
gatgtaaaac accaataaga ttgtgatcgt cacatttgat agtaactgct aaacatactt 205500
cttgcttcgg acatacttct gtcttgattt gaacganatc ttctggaatt tgaggaaatc 205560
ccggaacacg gactggagct gccgaagccc ctatagctac tgaaaatagg caagaaacta 205620
aaaaagcaac tttacgcatc ttgattaact aattaaaaaa ggaacacata tagagtaggg 205680
gggcgctcct gtttttgtca atgtcatgga agtttttgaa ggaaaaacgg acaagactct 205740
tgttttttcc tctggggaga cgtacactaa gcctttttaa tttttatata tataaaagtt 205800
tagaatatgc gatatgaccc caacttaata gaaaaaaaat ggcaacaatt ttggaaagaa 205860
catcgaagct ttcaagcaaa tgaagacgag gataaagtaa aatattatgt tttagacatg 205920
ttcccttatc cttcaggagc aggtctacat gtaggccacc ttattggcta tacagcgaca 205980
gatattgttg cgagatataa aagagcacgg ggattctcag ttcttcatcc tatgggctgg 206040
gatagctttg gtttgcccgc agaacaatat gcgattcgga caggaaccca tcctaaagtc 206100
acgacccaga agaatatcgc taattttaaa aaacagctct ccgctatggg attttcgtat 206160
gatgaaggac gagaatttgc tacgagtgat cccgactatt atcattggac tcagaaactt 206220
ttcctttttc tttatgatca aggactcgcc tatatggccg acatggcagt gaactactgt 206280
ccagaacttg gtaccgtatt atcgaatgaa gaagttgaaa atggattctc aatagaaggg 206340
ggatatcctg tagagcggaa aatgcttcgt cagtggattc tcaaaatcac agcatatgcc 206400
gataagttat tagaaggtct cgatgcccta gattggcccg aaaatgtaaa gcagttacag 206460
aaaaattgga tagggaaatc tgaaggggct ctcgtaacan ttcatttgac gcaagagggc 206520
agtctagaag ccttcactac ccgcctagac actttattag ggtgagtttt cttagtgatt 206580
gctcctgagc acccagattt agattctata gtgagtgaag agcaaagaga cgaagtcaca 206640

gcctatgtac aagagagtct caggaaaagt gaacgagatc gcattagctc tgttaagaca 206700
aaaacagggg tctttacagg aaactatgcc aagcacccca ttacagggaa ccttttacct 206760
gtttggattt cagattatgt cgtcttaggc tatggcacag gcgtagttat gggagtccca 206820
gcgcatgacg agagagatcg agagtttgct gaaatgtttt ctcttccgat tcatgaggtg 206880
attgatgata acggggtttg tattcatagc aattacaacg acttttgtct taatggcttg 206940
tctgggcaag aagctaaaga ttatgtaatc aactacctgg agatgcgttc tctcggaaga 207000
gctaagacta tgtacaggct gcgagactgg ctcttctcta gacagagata ttggggagag 207060
cctatcccca tcattcattt tgaagatgga acgcaccgtc ctttagaaga tgatgagctg 207120
cctcttctcc ctccgaatat tgatgactat cgtcccgaag gattcggtca gggtccttta 207180
gcgaaggctc aagattgggt gcatatctac gacgagaaga caggtagacc aggatgtaga 207240
gagacttata ctatgccaca gtgggcaggc tcttgctggt attatcttcg tttctgtgat 207300
gcacacaact actcagttgc cttggagtaa agaaaaagaa agctattgga tgcctgtaga 207360
tctttacatt ggaggtgcag aacacgctgt tcttcatctt ctttactcga gattttggca 207420
tcgagtcttc tatgacgcgg gtcttgtctc aacaccagaa ccttttaaga aactgatcaa 207480
ccagggactt gtgttagcct cttcataccg aattcctggt aagggatacg taagcataga 207540
agacgttagg gaagaaaatg gaacgtggat ctcaacttgt ggagagattg tggaagttag 207600
acaagagaaa atgtctaaat cgaaactcaa tggtgtggat cctcaggttt tgattgaaga 207660
gtatggtgca gatgccttac gtatgtacgc tatgttttcg ggacccttgg ataaaaataa 207720
aacctggtcc aatgaaggtg tttggggggt gccgtcgttt cctaaatcgt ttttatgatt 207780
tggtncttcg tcagaggttc aagatataga agaccgtgac gggctggttc tcgctcacaa 207840
attggtgttt aggattacag aacatattga aaaaatgtct ttgaatacca taccgtcttc 207900
atttatggaa tttctgaacg atttttcaaa gcttccagtc tattctaaac gtgccttgtc 207960
tatggctgtt cgtgtattgg agcctatanc tccgcatatc agcgaagagt tatgggttat 208020
attgggaaac ccaccaggga ttgatcaagc agcatggcct caaatagacg agagttacct 208080
agttgctcaa actgtgactt ttgttgttca ggttaatggg aagttacgag gacgtctcga 208140
ggtagccaaa gaagctccta aagaagaagt tttatctttg tctcgaagtg tagttgcaaa 208200
gtatctagag aacgctcaaa tacgaaaaga aatttatgtt cctaataaac tagtgaattt 208260
tgtcctatga tgctacgagg tgtccatcgt atttttaagt gtttctacga tgttgtttta 208320
gtttgtgcat ttgtaattgc cttacctaag cttctttata agatgttagt ttatggtaag 208380
tataagaaat ctctagcagt tcgttttggt ctgaaaaagc cgcatgtccc tggagaaggg 208440
cctttggtgt ggtttcatgg agcatctgta ggggaagttc gtttgcttct acctgtactt 208500
gaaaaatttt gtgaagaatt tccaggttgg cgttgtctag tgacttcatg tacagaactt 208560
ggagtgcagg tggcaagcca agtgtttatt cctatgggag ccactgtttc aatactgcct 208620
ttggatttta gcataattat caaatcggta gtcgctaaac tgcgtccctc ccttgcagtc 208680
ttttctgaag gggactgctg gctaaatttt attgaggaag caaaacgtat aggagcaact 208740
actctcgtca ttaatggtag aatttccata gattcttcaa agcgttttaa atttttaaag 208800
cgcctaggta aaaactattt ctctccagta gatggatttt tattacagga cgaagtccaa 208860
aaacagcgtt ttctttcttt agggatacct gaacataaat tgcaggttac agggaatatt 208920
aagacctatg tagcagcaca gacagcactg cacttagaaa gggaaacttg gagagatcgt 208980
ttgagattgc caacggactc gaaattagta atcctaggtt ctatgcatag aagtgatgca 209040
ggaaaatggc ttcctgtagt gcagaaatta ataaaagagg gggtctcagt tttatgggtg 209100
ccaagacacg ttgaaaagac caaggatgtt gaagaatctt tgcatcggtt gcacattcct 209160
tatgggttgt ggagccgcgg cgccaatttt tcttatgtac cagttgtcgt tgttgatgaa 209220
attggcttat tgaaacaact ttatgttgct ggtgatttag catttgttgg aggtactttc 209280
gatcctaaga tcggaggaca taatttatta gaacctctcc aatgtgaagt ccctttaatt 209340
tttggtccac atattacatc gcaatcagag cttgcgcaac gcctgttgct ttctggtgca 209400
ggactttgtt tagacgaaat agagcctata atcgatacag tttctttctt actaaataat 209460
caagaagtgc gtgaggctta tgtacagaag ggaaaagtgt tcgtaaaagc agaaacagct 209520
tcctttgacc gtacatggag agcattaaaa agttatattc ccttgtacaa aaatagttaa 209580
gtttgataaa ttcatcggca tatcgcggga tagagtagtg gtcatctcgt tgggctcata 209640
acccaaaggt cggaggttcg aatccttctc ccgctaattn ccattttgca tggcggtata 209700
gctcaggtgg ttagagcagc agaancataa tctgcgtgtc gttggttcaa atccgactac 209760
cgctatccat gctagaagac attcattttt taataaaatt taaaaaaatc ttcttagcca 209820
aaatttccta cctatatttt ttacataaca ctttgttttt caatttggat ttatgttttc 209880
tcgaagaaaa taaagatctt cataaccaag taaatcagac ttaaagtacg tcctgatgcc 209940
tttttcttga ttagagaatc cgttagaact cgaattgtgt gttttcataa acaattcatg 210000
ccgcatttct tcaataggaa ttagagaaat ttttnatttt tctaagaaag gcactccaaa 210060
actcaaggag ttttaactaa ctaaaaagga taaggaaaat aaaatatagc agataatact 210120
gtatttttga atagctcttg cttactcatc tctaataaac aagtgattct ttaacacacc 210180
tttattggag ccttcaaaaa aaagagtaaa gccagtggta ttggactcac gaagatcaca 210240
ataaaagaac cctgcaccga gctccttaag cctttctagt agaataaagc cacatccgtc 210300
aggatgatca gattgtggag ttgcagctct ataaaaatag aaacagtacc atatagtaaa 210360
gagattgcct attgtagatg gtattccaac accaccctga gctattggat acttattagg 210420
gaaaaatgcc gtcctaccaa gaaaagcgct tcgtccaggt tcctcactaa gaacaacaat 210480

attgtgcatc atatgtctag ccagaagctt tttgctttcc aagctaaaat atttgttgcc 210540
ttcttctttc agaagaatat cttttatcag tttttcttct tcaggatgac ctgtaaagtc 210600
aaaaggtcga tttttcaaga ggagttctgt aggccaatct aacgttaaaa gtaattttat 210660
aaaacttgcc ttaaggagtt tcaactttca taagttgata tttcttagga agagaataga 210720
agagcggaga aacagttaga gccgcttctt tcactagcgc aggaattttt ggcaatgatc 210780
agttcttcgg ctttagtaat ttctgtgggg ataaaaaata ctgctcgatc caaatcttta 210840
ttaaacttgt tatataagag gtagcgtatc gctagagcta tcaagactag aggaaacagg 210900
ataaaggaaa ggatttttaa aattttttct gctgtagaaa caacagcctt ctctttttta 210960
gccaagataa gtccggaggg tcgaagagaa ataatgcgcg tcacagtctc tcctcctaaa 211020
caaaaatagg agttcagctg agccatcaac gaagcttgcc aattagggga agctccagga 211080
gaaaattggt atatgttcat aagggcttct ttataagtga caaagctaaa ttataatcat 211140
ttttgtatta agattaaaaa cagaaatctc aagggaaaac agtacaattt agattagatt 211200
tatccgcctg agcaccatcc gaactctaaa ctttttagga agtctagaat agagagccat 211260
gcccttttta attgtctttg ataaatccat gatccttcaa cacagatcca tagttttctt 211320
cccaaactat aaggaatcct ataggtccat atggacctcc attacgcccg tcattttgag 211380
cgcctgcttg tggtctatat tttaatccaa ggtttaagag cctattatgg acttcgcagc 211440
ctgcttgttt aaaatccttt tctagtttgg ggccgttaga atggtagtaa taaaaataaa 211500
tgaaggccca tacagtgggt tttttagttt catgactgaa ctcgattaag aaagattctc 211560
tacctccatc aatagtggat gtaggaaagt tcaatccggt ttcgagctta aagagttgtt 211620
ctaagagata acgtgccaag attttcttgc tttctaaaca aaactcctcg ccaggattat 211680
tcttttcaag tttagcaact gtatcaatta tagccttgct ttcaggataa taggcaaaat 211740
caaaatcctc actcaaatag agtttaggcc atggtataga atctgtatct aagtcctcta 211800
agagcaaatc tatatttata gagagggtaa ttttaggagg ctacccttta gaagtttgga 211860
tgtacatact ttggtatttt gtaggtaggg caaagaaacc tgggtggacc tctcgagctg 211920
ctttttcaac taattgagga tttgctgcaa gaattagctc cagttcttta ggcgtgtctt 211980
gagggataac gaagcatttt ctatcgaatt ttctatgtaa gaagtagcgg atagccagag 212040
cgattaaaat gatcgggaag aagatcagag caaggatctt tataactttt tctgctgttg 212100
agacctgtac tttttcttca gtagctaaag aaaacccaga gggagtcatg gagaaaatcc 212160
gagttgcagt ttctcctcca aaacaacact aagagtcgag tttactcata agcccagctt 212220
gccagttcgg gcagatgcct ggagaaaaag agtatatgtt gctcataaac aatccctttt 212280
aaagaattag ccatcttatt ttataaattt tctgattcta taagtcttag ttgagaattt 212340
taatttttaa tatagatgtt tttttaaagc ttaagaaatc ttaacacaaa agagccgttc 212400
ctttgcatct agcttagaat attcttatag gaatggacag gttgtcagag tagaggcgaa 212460
tgcgtttgat tttcccaaag cagggtcaaa ggtcgttgat ctataagctc ctctttcccg 212520
aagtactgta aagggcctgg aaaacggtat aaatcttcaa ccaaacaaga atcactttgt 212580
tgaagtaaat gttgaacagc aggggatttt gggtctacag aatctgtttt aataacagga 212640
gtctctgtcc cacaacgatt ttctaagtgc atcatcttat ataatggagt ggctccccct 212700
tgccattcag tataagattg agcaagatta ttgattgtaa tcatataacc agtcttttgt 212760
cggaccagaa ataacgcaga aatgatccct aaagcgattc cataattaca atcaaagttg 212820
gaagggaatc ctgctcgtgc ttcataacca aaaaaatgcg atacagaatg gaattccata 212880
tggggtttta tcttttcgat ttccttcttt accattactg caagcagctc ttctgtagca 212940
atttttgana ctctaacatt tccatgagaa tctcgagcta gaagaagttg gttcgcaata 213000
tccttaggaa acaagtgaaa tgtttttatg tctctggaga gagcttggaa agaatttttc 213060
atagaagaat ctccattggc gagtaaaaca ttgagttcat ctataagctt gcgtgtatcg 213120
aaaatatgct caatcagtcc ctctgggatt aacacagtac tatagttttt tccagattta 213180
tagcggcgta ccaaacctaa agcaagctgt tcactcagtt gctttaaaga gattttccta 213240
gtggcaataa gttcgctaat taaagctata ttagggaggg tctgcaatcc gcattctaaa 213300
gtagtataag aggcctgctg ccccataagg cggatgaaat ggtggtattt ttttgcagaa 213360
agagcatctt tagcaagatt cccaatcatt tctgagtacg tgcgacaaga agtatgaaaa 213420
cctaacgagg tttcaatcca acagttctta agatcaccat ctatagtttt agggactcca 213480
atcacggatg ttttgcagtt gtgagcaagg aaatattctg caagcatcgc agtgtccgta 213540
ttggaattat ttcctcctat aatgagtagt ccgtctaact ttagttgctt gactgtgttg 213600
aggatgtttt ttttctgctc ttcagtttta attttttctc ggcttgagga gagcatgtcg 213660
aaccctccca tgttgtaata atcatagatt acggagatat ccagatcttt atagagccca 213720
cgagtaagcc ctaaaggtcc tttgatgaat ccaaataagc gagtcttggg attgaatact 213780
cgtaaagcat caaaaagacc aataacgaca ttatgccccc caggagcttg tcctcctgat 213840
agtaaaaccc caatctttaa tggttttgat gacgtttcct gttcagtata aatagaaact 213900
tcagggatcc gacatagatt gggaatgtgt ttttgcaact ctggaggggg tgaaggggga 213960
gacgaagttt cttggatatg tttcgaacgt atcgtttcta ataaagtaag aatctcaggg 214020
cggtagcgaa ggcgttggat ttcaaaataa cttttattta acgagagaag ttccacagtt 214080
tattccccag aagttaattc tctttttaac cattgcgtga gatccgaaag agccgaagat 214140
tcggcaaaag gaaacgcgtg atcgacatcg ggataagtaa gaatagtaat tggtttgtct 214200
tgatttgcaa atgcttcggt aaacagtgtt ctgtggttga tagaaaccag aagatcttgt 214260
tcaccttgca tataaagaat tggagggaga ttgcgagcac taggcataag ctctttcaca 214320

atatctattt ttaagaattg cgtataaaaa tcaggattta aagtcatccc agcataagta 214380
atggcgcctt tttgactcat tgtgatcact tcaggagcat ttttttgtgc ttccgcagcc 214440
attaattctc ctgaaattgt aggcgcccat acagctaagg ctttgatttt attaaaaaaa 214500
ggaagagtct gaagagcaag agttcctccg agtgatgaac caaaaatagc gagcctttct 214560
tggtctatat gaagtaggga atgcgtatat tcaatgattt cacgaatgtt ttgcttataa 214620
ttttcaagag aaaaatccat aagctcaccc tcacagtctc catgaccaag gagatctact 214680
cttagagctg cgatacctaa ccgagtgagt tcctgagcta gcctgacatg agagcgtttc 214740
gaaccggttt tatctgaagc taaaccatgg agaaggatca ctataggata gggaggatta 214800
taatggagag gagtatgtag aaggccaaaa gttgtaaaat tattcagtag ggtcagtgaa 214860
aacatggtgc gctgctcatg cttttccaat accacgcctg taatcaaaag aggctcctgc 214920
tttctggatg tgcttgacct atgtcaatgt agtttctaaa gaaaaattag tcgagaaaat 214980
tcgttgcatg gaatgcctct aactaaagtt ctcaaaaaca gccatcaaaa atcgtgtaaa 215040
atagtatcag aacaatccct ggatgaatag tttacaggaa tgatttaaac ctccttattg 215100
ggttgatagg aatcttccca atagatacga atcccttgaa aaccttgaaa attaggattg 215160
tctggtcctt gagaggggca aattggaaac ctaactccta actcttgtaa ttgctctaag 215220
atctcaaatc ctcctcctcg atcttcatct agaggacctt gtctgaaaaa gacttgcggc 215280
catattgtag gattttgtgg agtaaaatga gaatagatat aacgaacact agggaataga 215340
gtcagtcttc cagatgtatt gttaggaagg gcttcagggt tttcttgtgt gatcccatca 215400
ataaataggt gctgtaacat gaattttaaa agctacgctt tcctacagaa tttatgtaag 215460
ttcctgtccg cagattttgt acattctgta taacacgttt atctgccttg ctgcaaaaat 215520
ccatagtaga gttaagatac tccgtaggcc aggaaaccga atctaaatta atgtcatcag 215580
caagttgttc aagatctaaa gaaaagcgaa tgccgtgact tgaaattata atctgacggt 215640
attttgcagg tacacagaaa aaagagggtt ttgtagttcg aagagccgct tcaacttnta 215700
gaggatttga tcccatagct tgtagatttt tttgggatac tggaaccaat gttgttaatt 215760
tataatgaga cgccgtgtnt cctgaacgac caggaatagg cacatactga ggagctcttt 215820
ctaaaatntt agatacaagt aagtgattag catatttagc atgtagaaaa tagtgtaggg 215880
ctaaagcgat taaaatcaga gggaagatta agaaggataa aatctttaat attttcacta 215940
ttgtggagat ccgaacgttt tctacttttc caataattaa gttaggttct tgcataacta 216000
tagatacaac cttgattcta ttcccgccta agaaaagata agaatctacc tttgacataa 216060
gctgaaatat ccaaggtgta tctacatctg aagagatgct ataaagtttc ataattcgaa 216120
aaggcgattg tatattgtat aaagtgtaaa caaattataa aatgtatatc gatcattcat 216180
acccctacac cagcgacacc tctttgcacg gagggggaaa tttttccagg tcttgtagat 216240
tctgcaattc agaatgatct cgagagatta ctcacagtaa agaaaagacc tgatatcatc 216300
cgggagtatt tgcgagcagg aggtagtctt gttacaacat accctaagga aggtcagaga 216360
ttgcgctccc cagaacagtt aagagttctg gatgatttag tgcaaagcta tccaaatcac 216420
ctacatgcga ttgaacttga ttgtggtgca atccctcaag atttgatcgg agccacctat 216480
atcatcacgt tcgccgattt ttccacctat attctctctt taagaagcta ccaagccaat 216540
tctccctccg atgatacatg ggggatttgg tttggatcta ttgacgatcc tgttcaagca 216600
gtcatatcat ttttaaaaga tcatggattt gctcttccct cgaccttagc tcaagatcct 216660
ttgctttgta ctaacaagta atttattaag tgaaataaat aatataaatt aaaaatttat 216720
cgcttaattg tgttttaaat gtatttatat taaaataatt ttttcttttt taaaacagct 216780
tatgactaga agtactattg aaagcagtga ttcgctatgc tcaaggtctt tttctcaaaa 216840
attaagtgtc cagacattaa aaaatctctg tgaaagtaga ttaatgaaga tcacttctct 216900
tgtgattgct ttcctaactc taattgtggg gggtgctctt atagctttag caggaggggg 216960
ggttctttct ttccctcttg ggctaatctt aggaagcgta ctcgttttgt tttcttctat 217020
ctatttagtc tcttgttgta aattttttac tttaaaagag atgacaatga cctgtagtgt 217080
caaatctaaa atcaatatat ggtttgaaaa gcaacgaaac aaagacatcg aaaaggcatt 217140
agagaatcca gatctctnng gagaaaataa gagaaatgtt ggaaatcgtt cggcaagaaa 217200
tcaactagaa atgatcttac acgagactga cggaattatt ttgaaaagat atatgaaagg 217260
agctaaaatg tacttttatt tatgaattgg gttccaaaaa caatagacca tgtagatcca 217320
gaatcagaga tagatatacg taaagtcgtc tcctgctata agttgataaa agaatgtcaa 217380
cctgaatttc gatctcttat aagtgaatta ctaggagtga ttcggtgtgg cttaagacta 217440
ttaaaacgtt ctaagtatca agaacaggct agaactgtat ctgatgaaga tgcacctctt 217500
ttctgcctga ctcgttctta ttatcaagat ggttatctca cgccattaag agcaggacct 217560
cgtgatctta taaatcacta tatacacttg cgtcgccgag agaatcctaa gcattttttc 217620
agtcctaagc atccatgtta ttatgctcga ttggctttta atgagtcagt gtgtgtctat 217680
agagaactct ttgatataga gcgacttaca aaaatgtatg tcgagggtga ttattctaaa 217740
gaacaagaga aaaacctaca ggctattctt agttttgtga aaactctaga tgaaggaaag 217800
gactttctta ttgaacataa agataccgat ctcattggga gaggttttac tgatgtgttc 217860
tgcacttaaa tcaatgaaat tgtctcaaac aatataataa aagcgattct attatgtctg 217920
aaagtattaa cagaagcatt catttagaag cctctacacc attttttata aaattaacga 217980
atctctgtga aagtagatta gttaagatca cttctcttgt tatttctcta ttagctttag 218040
tgggtgcggg agtcactctt gtggttttat ttgtagctgg gatccttcct ttacttcctg 218100
tactcatctt agaaattatt ttaataaccg tccttgtctt gcttttttgt ttggtattgg 218160

aaccttattt aatagaaaaa cctagtaaaa taaaggaact acctaaagta gacgagctat 218220
ctgtagtaga aacggacagt actctttaaa attatattta atgtataggg ctaatcggag 218280
tacgcatttg aatatcagag caattactaa aggaattatg agcaggtaag ataggatctt 218340
cagtgtacag tgtacagttc atagagcctg gcttgaattt cctatcttta gcactcaatt 218400
cttccacagt gattccttga tcctgtttgc ttaggaagac tatttgagac tgtcccagat 218460
caaaataaga atctacggtt gcagctaaac gttctaagcc gtaattctcg ctatttaaaa 218520
aaggtttagc aagaaggatg gtagggcaaa aagggagcat aaaaaatcct cttgggaaat 218580
gcgaccacaa attttacatc atctgttcta ttaatgctat ttctatttta cgaattcatg 218640
caacataata acaattatca gactttatat ggagatccta gtagactatc gtaaggctta 218700
agaacaaaat atatcggcgc cacctctccc aataaaagtg gtgtctttat gtgtagcaag 218760
aaagtcgtca aagccgtcct tctcatctaa aatttgcaca aaactaagaa tcgactttag 218820
gttgtcttcg ttttgctgct cataatcacc cgcgccatac atttgtttaa gttccgctgt 218880
gttaaagagt tttcgataga tacgcactga ctcattaaag gccaatcgag catagtaaca 218940
tggatgccca ggattaaaaa aatgctcagg attctcacgc cgacgtaagt ttatatagct 219000
ctatttataa tataacgcgg tcctgctcgt agtggtgtaa gatagccatc tcgataataa 219060
gaatgagtcg ggcagaaaag aggtgcatct ttatgggata cagttttagc tcgctcttga 219120
tacctagaac aacggcttaa taaccctaag ccactctgca tcgctcttag taattcactt 219180
ataagagatc gaaattcagg tttacatgct ttaattaact tataacagga gatgactgta 219240
cgtatagagt cttctgatac tggatctaca tcgtccattg tttttggaac ccagttcata 219300
aataaaagta catctagatt cctctcatag atctttttca aaacatttcc atcacaatcg 219360
cgtaaagtct cttttacttg acttcttgca gagcgattat tgtcttccgc cctattttct 219420
ccaaagagct caggattttc cgtagccttt tcaagatctt tatttcgttg cttttgaaac 219480
cattggtgga tttgagagtc ggtattgtag acaaccccta gctcttttat agggcagaat 219540
ttatatgaaa aaagtaagaa taaagcacaa agcacaacga gcacaattcc caagactaac 219600
caaggaagaa aagaaagaat gcctgctcca gctaaagccg tgagagtaat tcccccaact 219660
aaagctaaca aagcgataac aaaagaagtt atcttcacta atctactttc acacagattt 219720
cttaatggct gggtatttaa ttccataact aatggttgtg aacactctga accaacattt 219780
ttcatgttat tttctccgat agtgaattct atatgaaaag attaacactg tactttcaaa 219840
taatttttaa aagattaagt ttaatattta tacaaatata aactaattat ttcgtttcgt 219900
gaatcttgta aaataactaa gaaaaagtat cggtatgagt cacagatcaa aaagagatat 219960
ttgtcatagc atgttttata agaaattctt ttctgtgatt ttcttatcta agctagacag 220020
gtctttcctc ttgtttccaa caaaaagtcg agttatttta tatttaagct acgttaaaag 220080
gcaatcttaa atcatcatat caaaaattta atgagacacc ctccccttcg agctctcttt 220140
tcttcccatc gactctcttg aaagcttctt ctctggagac gctttttttt aacattactg 220200
ctttagaata gaaataaagt aagaagaaaa gctataagtc ttaggaactt ataaaaaata 220260
aaaaggaaat ttagtgggtc gtatctatag tttttctcct ggaacttatc ctaactggca 220320
agtaactctt atgggtaaac tagatggctg ttttcgttta agagacgaga aagtcactcg 220380
ggttatctcg atcaatccct ctggatttac cttagctgac gaaaaaatag tccgggtctc 220440
tatgccttct ccagcatgag ctgcacaacc aaaccctgag tcataagcat ttctatatcc 220500
agaagctaat cgcttaggac aggctccggg cgcagattct gatcctgcgg attgtatccc 220560
tacaaataga ccaggaaatg tagaatcaac ctcttggagc cattgaacag cttcttgggc 220620
aggttcttgt gtgtttattt gtggagcaaa tgttttattg aagcagttta aaaatcgtaa 220680
agtaatcccg tgttttgaaa acgtttgcga agcacgatac aagatttgat aaaacttcat 220740
acgcgcgtgc ttttcaggta atgaaggata caaaacatgg gcaaggcgga tattttgttg 220800
tacttcagta tacacgatag tatcgtccag acattgctgg agatagttat tgaaaatgag 220860
aagaaggtct tcttcatttt ggattcctcc aggaggaaag cgatgtcctt gtactgtagc 220920
catcactcta tcaaagctat taaaatcgta atttaagata ttatattgta atacacttaa 220980
atccggatcc ttttcgtgac agatatcttg aaagtttcgg aaaatattgg agtattgttt 221040
atgaggattc ttaggagaaa gtaatcgatg attggtccaa gaattataag accattttaa 221100
gaacccattt ttcacaccta aaatccaagc taattgaggt gttatggtcc caggaaggtg 221160
tacatggata tctgctttgg ggaggttttt gatgatagct tcggtgcaag cgattgtatt 221220
gagtcgtggt actaatgcaa agatctcatt gcatatctta tgtacagaat cttccttttc 221280
aagattcttg aataacgtat cataagacat agaaagaacg aacgctataa ctgtgcaagt 221340
gttatacttt tctcctctaa tgatatgatt tttacgaaca ttctaatgat gttactaaat 221400
aacgagtctt tgatttaccc ctatggtggc tattcccatt ctatagttgc tggtggcttg 221460
tcagaaatat catagaccac tcggcttacc tcgggtattt cattaataat tcgcgatgag 221520
caagaactga gaacatcgca tggaaggtag gcccatcgtc ctgtcatgaa atctgtagat 221580
tctacagcac gtaatgctat ggtataacca tagcttctac aatctccttt tacagatact 221640
gattttatag gaagaaatag agcaaaggct tggcttattt tatcgtagag ttttgctttc 221700
ctaagctctt ctataaagat gaggtccgcc cgtcgtaaaa tggctagata ttcaggaagg 221760
atctctccaa tcacacgaat tgtcaagcca ggtccaggaa aaggatgcct gtccaagaga 221820
tagctagaaa gtcctagggc ttctcctaaa attcgaactt catctttaaa taaataacgt 221880
aagggctcga ctaacttcag cttaagattt tttggaagcc cccctacatt atgatgtgat 221940
tttattactt cggaggcatg tccagagcgt gaggactcaa taacatctga gtagatggtt 222000

ccttgagcta accattgtac gtctaatgac tgagcgactt catcaaatac ttcaataacg 222060
gtatcttgaa ttttacttac aaggtcttgc tgaatataca agggattcca tagtgtggga 222120
gcagaacaga tctcttgaac aaaagtttct agaatcttat ttcccgttgg agtggagtca 222180
gaaacctcgg gatgaaattg cagcccgtac aaccgttgtt tggtattttc tattcctgag 222240
atcgagcatt gtgaggtgga tgcgattaca ttaaatcctt caggaattgt cgtaacatga 222300
tcccgatggc tcatccgaat ctctgtgtct agagattcgc agtcgacgat gtgtttgaag 222360
agctcacaag gatacagatg gatgggcgta tatccaaatt ctcctacacc agggcttaca 222420
gtccctccaa aatctctagc cataagctgc atgccatagc aaatagctag aattggaatg 222480
ccaagtttat agatttcagg atctaaatgt ggagccttgt tttcatagac agagtgagga 222540
cctcctgaga gaatgatccc caaaggcgct ctttctttta aacattgcac agagatattc 222600
cagggaagaa cttcgcaata tacaaataac ttccgcactt gctttgctaa tacataagta 222660
tattgagatc caaaatctag aataaatatg gtgttcaaat gtctccttgc actctgcaac 222720
ttaataattt aaggttggtt gaactttgta aatattatga atatgacttt cagctcttcc 222780
agattcagta attcgaacaa aggaagcctt agtttttaaa tctttgagag tttcagctcc 222840
aacatacccc atacctgagc gtattcctcc taaaatttga tagaggacat cgtggacaga 222900
gcctttataa gcgactagtc cttcaactcc cccaggaacc agctttttct gtccctgtgt 222960
ttgaaaatac cggtcagcac ttccttgttt catagcgcct aaagatccca tgccgcggta 223020
ccttttaaaa agcttctcat cgatagaaac gatatcccca ggagcttcat cagtccctgc 223080
aagcaaactt cctagcatga cacagtctgc tcctgctgct aatgctttta ccacatctcc 223140
agaatagcgg attctcccat cagcaattac agtcacggca gagtttttaa gagcttttgc 223200
tacgtttgta atggcagtaa tttgtggata accgacccct gaaacgattc tagttgtaca 223260
gatagatcct gggccaatac ctacctttac agcgtcaact ccaatctcag ctaaggaaac 223320
tgcggcttca gctgtaacaa gattccctac aactaaagaa atttgtggga actgggattt 223380
tatttctaaa actgtttgga atactccttt agagtgtgca tgagctgtgt caatgactag 223440
aacatttgct ccagcttcca caagatgatg cgctcttgaa attcctaaag gaccaatgcc 223500
tatagcggct cctatgggag cttaaggata ggcttcttta atttttctca cagatgagct 223560
ttgttctact tcgctcatat tttatgtaag attcccaaa ccaccttctt gagctaaagc 223620
tagtgccatg gctgtttctg tgacagaatc catagcagct gaaagaatcg gtatatttag 223680
agagagggtt tttgaaatgg ccgttttcaa ggacacttca gaaggaagta tttcagaata 223740
ttgggggatt aaaagaacat catcaaaagt taaggcttct tccatgtatg ctacaatagg 223800
acactttaaa ggatagtcaa tgatcccctg ctatactgtc aatgcttttt tcatgattcg 223860
tgacattcct tcgaatctta agaagaaatt tctttggaac agaagtttct tcttccgtgt 223920
ttaggcttct tattgaactt atagcttaga aaagattgaa ggaaggaaag ataagactat 223980
attgattctc ttgtcttaag atatacgacg acagcactcc ctattcctac actcgataat 224040
aagtagaacc aatactataa gagataagat cccaaataca agtgaagctg tttctccaga 224100
agttccataa acagaaaaac aaatgatgat gctaagtagg gataaaaaga taaagagaca 224160
tcgggaacag taatgacaac aattcgatac caaagttttt gcatctatat cctcgattgg 224220
ggaggggctc gttgattcaa cgaatctatc cggaaatacg ctaaagtcca taaaaccttg 224280
ttgatgtttg taagaatcta ttttgtagat gagatcgaaa atcgtctaac gatctgattc 224340
ttctcaatac tttatttgcc tggaatcata aaaccctcaa cctcttcgat gtcgttgtgt 224400
ggcgcaatct attgcaaatc acgaaaatac ttgattgagg gcttcggaga aatttgaagg 224460
ggtgatgcac aataggtgcc agttcttgcg ttttttaaaa aatttttata tggtttgtgg 224520
agaaaattgt tatactatca atgattatga ctactatatc taactcaccc tcccctgcat 224580
tgaatcccga actttccctt attcctccac caacacttgt atcttcaggt acgcaaacat 224640
ctctagctta tacgatcccc gcacaaggac gaagatccac cctacgtatt atattagata 224700
tattcattat cattcttggt ttagctacga tcatttctac ctttattgtt attttctttt 224760
taaatgggct gaacttgctc tcgaccccat ctattatctc ttcgtcatgt ttaatcattg 224820
ttggattgct ttttttgatt atggggttat atttcatgat ctcgagtttg gatcaggggc 224880
ttgtaggcct tctgcaaaag gaactctctc aagccgaaga aagagaagaa gagtatatcc 224940
aggaaatcga agctttaaga ggagctccta gagcagaatc tcccacagag tctcctagta 225000
cctggttatg atttacaggt atgaagttct tattttctaa aatttgtcag cagtttttct 225060
tttataagaa tactttttaa ttcttgtatt taaattcctt acgacaaaag ggtgaaaaac 225120
gtcttgtaga acattctagc ttctattagc ctgtttccaa tttttattaa ggagacgcga 225180
tatggagcaa cccaattgtg tgattcagga tactacaact gttttgtatg ccttaaatag 225240
ctttgatcct agacttagtg atgacactca cagacttggg aagcaatcac ctcttgaagc 225300
agaaaatgct cttggagaat ttattgaagg tttggataca aatagctttc ctttagagga 225360
agttgccatt cccatcctgc caggttatca ccctaagttt tatttatctt tcatagatag 225420
ggacgatcaa ggtgtccact atgaagtttt agatggcgta tttttaaaga cagtcgctgc 225480
ttgtattata gagaactcct tcttaactga ttctatgagc ccggagcttc tcagcgaagt 225540
taaggaagct ctgaaacgat gatgatccta tggatgaatc cgatggagaa gaagcttcaa 225600
aagattctgc attttcagct agtttttcct atgagtttgt aaaatcaagt actcgagaat 225660
ctaaaaatac agtcacacac tcaacagcgt ctcgtacatt atatatttta aggcaggatt 225720
gttcttatga tccaagagct ctcaaagtag atgatgaatt tcgttattgg gtagaaaaaa 225780
ggttggacgc caagaatcca gattcattaa atgcgttcgt taaagaggta ggaactcatt 225840

atgtcgcgtc agtgacttac ggtggcattg gttttcaagt gctaaagatg tcttatctcc 225900
aagtcgagga gttagagaaa gaaaaaatct cgatatctgt agctgcagca agttctttat 225960
taaaaagtaa aacatcgaac gcgacagaga aaggttattc ttcgtatcag tcggaatcat 226020
cagctcaaac agtatttctt ggtggaacag tattacctga tctccagcaa gacaagttgg 226080
atttcaaaga ttggtctgaa agcattccta atgagcccat tcctctagct attagtgtat 226140
cttcaattac agatctcata attccagaac ttttcccttc tgaagatgct caagtcttat 226200
cccagaagaa atcagctcta ggacaagtta ttcttaatta tctagagagt cacaagccta 226260
aagaagaagg cccaaaacca gtccaaatta cttctggatt caattcatcg tcttcggtat 226320
ttacgcttca agcagcaaaa gctcctaaga ctgtgtcttt cccctatata gattattggt 226380
ctacaattcc ctatcttttc cccactctta aagaaacttc aggtgctcaa cctctctcgt 226440
tctacttgag gtttgatgac atctttgagc aacaaaattt agtccataat acttcatata 226500
ttttagcttc aacctcggtg aggttaggat atttcggaga ttcatataga gattatgatg 226560
ctctatcttt ctatggtagt tggcctcaag catattttga ctgggcaggc tataaagata 226620
ggtgtacttg gaccttagaa aaactcaata caactggaga tcttttcatc cgttctggag 226680
acgagatacg tttaaaacac aatacctctg ggaaatatct tgctacaacg agcatgtctg 226740
atggctatca gacattaact tgtacgacac agacgagtga ttctgtcttt ataattactg 226800
tataaataga gttaagacat ccccttaagt tttaagggga tgtcttattt gctttctaga 226860
gaactcatct ctaagtaaac ttcttttctt agggctgagg aggaactttc ttatagcttt 226920
catgaacaca gagtatagaa gttccgtttg ctaaaaacgt gcttctgagg tagtcctctg 226980
caaaggaatc tccaaaagca ttcagagaat cagaggaaga aacgctttcc cagactaaag 227040
aatagtaccc ttttacaggt aggacctctt ttgagcaagc actctcatct gcaatccagg 227100
cataagcaat gacaaaagga ttcccctgag gatacgagca cacttcaacg ggatacaggc 227160
tataaacacc ttctcgcctt ctagctatta caaagtcttc ttctgtcatc tcaaggccta 227220
tagcattaca gcgtcgtccc gaagatttaa tgagattcag tacagctcga tttttcggac 227280
catcgtgaga agttctacgg cgacttccgt tttttaagta ctttcctgtg tctatattaa 227340
aagaacgctc gtatccattt atccaaatag gatgtggacg atacgttttt aatgtaagag 227400
ctgctgattt tgggtgcatc agacttccat aagtcgatat gggaatgtaa gaacctttga 227460
ttttgagatt tttatggatt tctctccaaa ttttacgaga ctcttcatca gtcttagcta 227520
cgacataagg atcattgtgc tcttcagaaa agatttgagt cgtacgaaat gcagaaggat 227580
aatgaggcag ttgcgaaaca ctcttatttc catgaacaaa gaattcttcg ctcgttgaag 227640
tgttgtgata attccaagag ggcttttgat tacaggagga aagagatcca agacctaatc 227700
ctaaacatac acttgaaaag ataagagaca tgggctgttt catggattcc tcggagatta 227760
agaaaatgaa ttttgatnng tttgctctct tttgtcctac ttaaaggagc ttttcaacaa 227820
cgtttcggtc tgaaattttg ctaaacaaga ccctgccgtt accctagctt gtcccagctt 227880
ctctagcata atttgaaaat tgtaaaggaa atatatgaaa aaaggaatac gaaacccacc 227940
ctatctaagg nattcttaat tctgaacgta tagtatccag aactaagaat tctctatctt 228000
aggaagtcta taatgaaagc accccgagat tcgggagaga acttttgtta gtgcatgcct 228060
tgaacagttt agtccaagta cacccacact tctctttctc tgttttcata acctgtgata 228120
atgcctagcg cgagcagttg ttttaaaatg ctctcaccta aacaaagctt accatgccca 228180
ggttcggtct taccagctat atggaaaagc tgacctgtag ttgttttagc atctatagat 228240
tctcctccaa atttctgaga gaccgtacag tgacgttttg tttgaggatc tctttcaaca 228300
ttccgttgca ctagaaaatc taagtattct ttcactcaaa tctaacttct cctcataaga 228360
aaggttaggt ttgccttcaa agatgcatag gagccggaga gatctaaaag caataactca 228420
acttagtgaa aattaaggag gacatgaact ttctgtcttt cttaagagat cttggcaact 228480
tagcgatcaa tccctttaag aagtgatgag gtactacctt taaaatcttt cgcactaacg 228540
cggcttgctg gttgtgttgt gcttcaaggt cttgttcatt aaagaaccct atagattcaa 228600
aaatacagga atagttagga gtttttgcgt catttacatt taagaggatc gctttttttc 228660
ctggaatgat tccagagcgg cctgcttgca aagtaaaatt aaagaaatta gcaacacatg 228720
cttttgatgg acgcgagctt cttcctgatt tgtggaatga tttacgaagt cttctgttaa 228780
actttggaac tgaggttgct tcaattctag tttcgaaatg agtatcccag atcttaggtc 228840
aagcccaaca acccttggaa cctcaatatt tcgaatttta cacgctgcaa gtaggtttct 228900
tgcatgtgtt ctacgtacca tagcctctac aagaacatta tctagaggat tagataagcc 228960
attcatctca ttgttttttg aagatacaaa tgtaaagcgg agattcggat aattatctac 229020
acaaaaagta aatacggaat aatgattagt atgataaaca tctaataaac agattttctt 229080
agcctcgaga atctgattag cccgagcagg atggcaatcc gcttgattaa aataggcatc 229140
acgtacttcc ctctgagcgt gcaatatctg agaaggatac gtttcagacg ccacggtatt 229200
ctggataaag actggcttat acttacaatc cagaatctta tgtaaagacg tctaacacaa 229260
taaatctccc tagaaatatc gtctctagct agaacatctg ttgtatcccc tccccaatag 229320
aaataagagt cgagggcttg cacagctttt tctaaactgc taggtttttc atgatgagaa 229380
tttaaagaat aattattata aaatagcttc ataaattttt taaaaaaata ataaacgccg 229440
tctattttaa acaattcatg cacttattaa gaagctatta attcaacaac agcttaataa 229500
atttaaaaat catcttttta taaagaaata tttatataaa aataattctt agaccattgt 229560
aaatttaatt agaaaagcct ccgagcttca agagccctaa ggaagattct taaattattt 229620
agaattcttt aaaaaaagaa tttccttgag caagtaggtt atcgatatag ttaatatcgt 229680

adttagattc tagaaacttc ggattatcca acataaactg gtggaaaggt attgtagact 229740
gcacaccacc gatatgaaac tctttcaaag ctcgtttcat gatagctata gcttcttctc 229800
ggtttttgcc cttagcaatt acctttgcta tcatagaatc ataataagga gggattgcat 229860
agccgctata acaagctcca tctacacgaa ttgaaggacc tgcaggagga agataataat 229920
ctaaacgacc tggagatggt gagaaattat tggtaggatc ctcagcgtta atgcgacatt 229980
ggatgatatg acccgagaac tcaatgttct tttgtttcca aggcagctta tttcccatgg 230040
ctacatgaat ctgttctttt acaagatcta tacctgtgac ttcttcagta atggtatgct 230100
ctacctgaat tcgggtattc atttccataa agtagaattt tttgtcttta tctaataaga 230160
attcgactgt tccaacagaa aaatatccgg cgcttcttgc tagatctaca gcaacttttc 230220
ctactttgac tcggatttcc gcattgagaa tgggactagg agtctcttca atcaactttt 230280
gacgtcgccg ttgaatggtg cagtctcttt ctcctaaatg cacataattt ccatgggtat 230340
ccccaatgac ttggatttct aaatgccttg gattttctat aaacttttca atataaacat 230400
tggggttatt aaaaccggct tcggcttctg cacgtgcggc agaaaacgct ctatagaatt 230460
cgtccttttc tttaacaata cgaattcctc ttcccccacc tccagcaacg gctttaataa 230520
caatagggaa acctattttt tcagctattt ttaaaccttc gctctcgtct tcaataatgc 230580
cttcagaacc tggaataaca ggacatttga ttttctttgc cagggacttc gcagcaatct 230640
tatcccccat catagcaata gactctgaac tcggccctat aaaggttaag ccacagctct 230700
cgcatattga agcaaagttt gcgttttcac ttaaaaaccc atatccagga tgcacagcat 230760
cagctcctgt gatctcacag gcagccaaga tattggatat ctttaaataa gactttgctg 230820
cttgaggctc tccaatacaa atagcctcgt cagcaagaag tacgtggaga gcctcttgat 230880
ctgctaaaga atatacagcc actgtcgaca atcctaaatc atgacaggca cgtataattc 230940
taacagcaat ttcccctcta ttagcgatta agactttttt catgatgcat ctttagctat 231000
acgaaacaac ttagacccaa attggacagg atccccattg gtaatcaata cttcaagaac 231060
acgcccactc attcctgctt tcacttcatt cattactttc atagcctcaa cgatacaaac 231120
aatagtatct tccgaaacaa tatcgccagg ttttacaaaa gaaggagaat ctggggctgg 231180
agaaccatag aaagttccca ctaaaggaga acttataaag tctccggaac ttgttgtagt 231240
agaagtttct gaattttctg tagtagtctc tttaattgta tcttttttag ggtccgttgg 231300
gataggtcgt tcttgagaaa atccactaaa taacctgctg tcataaaaca caggctcttg 231360
tctattcccc tccctagtat ctctttccaa ctccaattca agcccttcac gttttatagc 231420
aaaacgcttc ataccattgc gtcccatagc aatcatgagc ttttctattt gttttaagtc 231480
cataccaagt cttctttaat tgaaattaga cgcgttgaat atactcacaa gtccgcgtat 231540
ctatttttat aacatcacca atttctacaa aaggtgggac cataacttca attcctgttt 231600
ccagcaaagc tttcttaact cctccggaaa gagagagaga atctccagga aagtctgttt 231660
ttgataccat aagctctaga aaatgaggca gctctacaga aaaaaccaca ttgtcataga 231720
ccattgcaga gacagtcaca cctgccttta aaaacaaaaa gttatccttc atgatttctt 231780
gtggaatgaa taacttttca taatttccta aatctaaaaa aagataactt tcatcttcaa 231840
gatataaata ttctaaagtg cgggtttcaa attgagcctc ttttacctct tgagttgctt 231900
tgaaatttct ctcaataaca acatcagaat ccgcagcctg caaagcgact ttaatgaagg 231960
attcgccttt gggccctgcc accttagaca ctgaggttac tttataaaga ccgtcctttg 232020
tagaaataaa catccctacg gataattggc tacttaacac cataattttc tcctcgcagc 232080
aacaaaattt tatcttccat tgctaatgaa tctgcttcga ataagtaaga agctgtgact 232140
aaaatatctg ctcctgcatc tcgacataac ggcgcagatt gctgatctat gcctccatct 232200
acttctatta aacaagaatc ctttaaacct aaagtcttta tcgcatgacg tgcaaaagca 232260
atcttttcta ttgtatttgg taaaaagctc tgtcctgtaa atcccggata aactgacatt 232320
agcacaacga catcacaaaa cggaaggaaa gaaggaagaa attcgattga agtatcggga 232380
gaaaaagcta gacccgcttg aaccccacat ttttttatat aagatagaag ctcttttata 232440
tcctctgaag cttcaaagtg tactataatt ctatccgcac cagaacgaac gaaactttct 232500
ataaattcaa aaggattgta aatcatagcg tggacttcta aaaatagatc cgtagatcta 232560
ttaatggcag caatgatccc tggaccaaaa gtaaggttcg gaacaaagtg gccatccatg 232620
atatctatgt gtataaaatc acttcccgcc tgctctagtt tttttgcttc tacacccaaa 232680
caggtaagat ctgcccccat aatcgaaggg ccaactaata cggattcctg tttcttcacc 232740
tcagcctatc tcaacttcac tacgctaaat ttaaagtaga gtgagcttta actttctact 232800
atttatattt actggttgat gtattcatga actcctactc attgtatgaa tagaaaatga 232860
atacggacta ctaccataat acagaacgaa tatatttcaa agaaccattg aacatttctt 232920
attaaaaatt cttttctttt tatacaaaaa tcccaagatt aatctttctg tttcactaat 232980
gaaaatgcat ttggagataa ggattatgat atagcaaata tattgtgatt ctaacctcct 233040
ataccttcat caaacatcaa attagtgaaa taaagatgat taaatatatt ttggatcctc 233100
agcaagaaag gtataaattt cttgattttt agatcgagaa aaacaacaat tcttatccaa 233160
gttaacctat taaggataaa attcttatgt catctcctgt aaataacaca ccctcagcac 233220
caaacattcc aataccagcg cccacgactc caggtattcc tacaacaaaa cctcgttcta 233280
gtttcattga aaaggttatc attgtagcta agtacatact atttgcaatt gcagccacat 233340
caggagcact cggaacaatt ctaggtctat ctggagcgct aaccccagga ataggtattg 233400
cccttcttgt tatcttcttt gtttctatgg tgcttttagg tttaatcctt aaagattcta 233460
taagtggagg agaagaacgc aggctcagag aagaggtctc tcgatttaca agtgagaatc 233520

aacggttgac agtcataacc acaacacttg agactgaagt aaaggattta aaagcagcta 233580
aagatcaact tacacttgaa atcgaagcat ttagaaatga aaacggtaat ttaaaaacaa 233640
ctgctgagga cttagaagag caggtttcta aacttagcga acaattagaa gcactagagc 233700
gaattaatca acttatccaa gcaaacgctg gagatgctca agaaatttcg tctgaactaa 233760
agaaattaat aagcggttgg gattccaaag ttgttgaaca gataaatact tctattcaag 233820
cattgaaagt gttattgggt caagagtggg tgcaagaggc tcaaacacac gttaaagcaa 233880
tgcaagagca aattcaagca ttgcaagctg aaattctagg aatgcacaat caatctacag 233940
cattgcaaaa gtcagttgag aatctattag tacaagatca agctctaaca agagtagtag 234000
gtgagttgtt agagtctgag aacaagctaa gccaagcttg ttctgcgcta cgtcaagaaa 234060
tagaaaagtt ggcccaacat gaaacatctt tgcaacaacg tattgatgcg atgctagccc 234120
aagagcaaaa tttggcagag caggtcacag cccttgaaaa aatgaaacaa gaagctcaga 234180
aggctgagtc cgagttcatt gcttgtgtac gtgatcgaac tttcggacgt cgtgaaacac 234240
ctccaccaac aacacctgta gttgaaggtg atgaaagtca agaagaagac gaaggaggta 234300
ctcccccagt atcacaacca tcttcacccg tagatagagc aacaggagat ggtcagtaat 234360
ctgccgtaaa gtcttcaaag acttccttag aaaataagca gtaaagtttt aactttactg 234420
cttattnttt tttgaaatga actcactcat taaagatatt tgcaacaaat tttcctgcgt 234480
cttatgcttg ttcttttagg attgagtttt tctcacttac actagaaccc tacctatgca 234540
ttcaaaattt ctttctcgaa gaaaaaaaaa tagttctcat aaggaggaaa cctcttggga 234600
ttgtatagcc tcaagttaca ataagatagt ccaagataaa gggcactact atcatagaga 234660
aactatcctt ccccaactcc tgccttcact caccttaggt tcaaaaagtt ctgtattgga 234720
tattggctgc ggtcaaggtt ttttagaaag ggcccttcct aaggaatgtc gttatctagg 234780
catagatatc tcttctagat tgattgctct agcaaagaaa atgcgatcgg taaactctca 234840
tcagtttaag gttgcagatc ttagcaaacg cctagagttc gtagaaccga cattattctc 234900
tcatgcagta gcaatcctct cccttcaaaa tatggaattc cccggagagg ctatacgtaa 234960
tacagctacg ctcctcgaac cactcgggca attttttata gttttaaacc atccttgttt 235020
tcgtattcct agggcatcat cctggcacta tgatgaaaat aaaaaaagct atctctcgtc 235080
atatagatcg ttatctctcc ccaatgaaaa tcccaatcat ggctcaccca ggacaaaaag 235140
attcgccttc taccctctcc tttcactttc ctctaagcta ttggtttaaa gaactgtctt 235200
ctcatggatt cttagtttca ggtcttgagg aatggacatc ttcaaaaacc tcaacaggaa 235260
aacgagctaa ggcagaaaac ctttgtcgaa aggaatttcc attattcctt atgatttcat 235320
gcattaagat aaaataaatt tttaattatt taattaggtt ttttattaga ataaataaaa 235380
aacacnaaat tcttgcatta tgtttcgaaa acttttcccg ttttctaaaa aaaaaacagg 235440
tcagaagcaa cgtcttcgaa acaatggact tctgcaagcg atcattcaat caataaaagt 235500
cttactacat aacgaagctt ccaaggaagc ctgcgtgtta agctactatg gtttgcttac 235560
ttgtgttcct attttagtat tctttctaag gctttcccaa cacttattca ctaatctgaa 235620
ttggaaagaa tggttgatta tcaaattccc agattataaa aagccaatcg tagctattgt 235680
ggaagccgca tatcatgcta cagaaagcaa tataggatta gtcctagttg gaagcttttt 235740
tgttttctgt tgggctggca ttttaatgct cttatctcta gaagatggcc taaataagat 235800
cttccgcacc agctggactc caatatcttt aaagaggtta gtctcttatt ttgtgattac 235860
cttagtgagt cccatgattt ttattatcgt ctgtggttcc tggatttata ttacacagat 235920
catgcctatc caatacgcta agttgttttc tctcagccat tccatgacag cattgtactt 235980
tatttctagg tttgtcccct acctgctgct ctacctagct ctattttgct gttatgcttt 236040
tcttcctcgc gttgcaatcc aaaaaacatc agctcttatc tctacgctaa tcataggatc 236100
tgtatggata gtctttcaaa aggcattctt tagccttcaa gtctctattt ttaactatag 236160
cttcacttat ggcgccctcg tagccctgcc ttcattcctt ctcctgctat atatctatac 236220
aatgatctac ctattcggag gagcactgac atttattatc cagaatcgag ggtgcacttt 236280
catatttctt ggggacaaaa tcctgcccag ctgttattta caactcatta cctcaacata 236340
tattctagct ttgacaacac gtcagttcaa tgaaggcctc tcccctttaa ctgctcaatt 236400
catcgccaaa caatcgaaag tacctattgg tgaggtctct caatgtctag atgtattaga 236460
aaaagaaggt tttctttttc cttataacaa tgggtaccag cctgtcttca atttctctga 236520
acttacaatc aaagatattg ctgacaaact cctgcatcgg gaaattttca agaaattcaa 236580
tcccgacctt gggattactt tcatagaaaa cagcttccag aacatattta accaagcttc 236640
taaaaataaa gagaatctta ctcttagcga gattgctagg cgaatcaaat gaaacgaaga 236700
tcatggctta aaattttggg aatctgttta ggcagcagca ttgtcttggg attccttata 236760
ttcttgcccc aactactttc aacagaatca gggaaatacc ttgtgttttc cctgattcat 236820
aaagaatccg gactctcgtg ttctgctgaa gaacttaaga tttcatggtt tggacggcaa 236880
acagctagaa aaataaaact cactggagaa gctaaagatg aggtctnttc tgctgagaaa 236940
ttcgaactcg acggatctct attacgtctt ctgatttata aaaagcctaa agggattact 237000
ctatcaggat ggtctttaaa aattaatgag cctgcctcta tagaccatcc ttctgtgagt 237060
cacttagatc caggatcttt acttacctac ctaaatgact gcaagattat ttctgagcac 237120
ggatttatca ctatgaagac agtatcagga tcttcattat ctgtatcagg gtnttatcta 237180
gaganatctt cagaaaagtt catgacgaaa tgcgtggtct ctgaagatca gcaatccggg 237240
aacatcttta tagagagtgt actttctcct gatgtcagta tttccgctca gttttcttca 237300
gttcccgttg catttttttaa aatttttata gcttcccctt tctggaccca tcttctctct 237360

tatgaagaca taatcaatct atcagcagag gcaacacata ccaatgatgg taagatttct 237420
atgacagcct ctggcgaggg aaatcaaatt caaatgaagc ttcaaggcca tattcataaa 237480
tccacatttt atattgtaga agggagttct tcgttcatag aacttaaacc tgagctcgcc 237540
tcagctcttt gcaaccagat cattccgctg tccacaccca ttactagtaa gcaaatccan 237600
tgctacggtc tcttatgcta aaattccctt ggatattacg aaatggaaac atattgaaat 237660
tacctctcaa gcacagctcc ctgaagtcgc aatacatccg aaagacccta atcttgcatt 237720
acagctgcgc gacacaaagc taggaattaa aaagacggag aaattntcag acatccgtta 237780
ctcctcatct acagtcttag gaggagcttc tccctctcac cttaatggtt taatcagtat 237840
agataacaaa aaacatctta ctaaatttcg tctacaacaa gcacaactcc cccacaccta 237900
tctaagagcc attttccctc aacctttcgt gatcaatgtt cccctggatg ttgcttatta 237960
ttcattaaat atcgaaggga cgtacaaaaa tgctcactta gaggcagatg ctatcctaga 238020
taacccgcta ttgaaattgt catgctccat gtctggagca tggaaaaatt ttctttttaa 238080
agggcaagga acgtaccact ttaataaaaa atggcaggag attctctctc cccacttctc 238140
ttacgctgaa gctagattct caggaaaagc acaaattacc gatacgaatc tctttttccc 238200
taaattttct ggaaaaatta ctgcaagaga aaatgagctg ctcatccatg caaaatttgg 238260
ttcccctaat gaacctataa aacctgaaac tacctctata ctcatccacg gacaattttg 238320
ttctctgcca actcagccta gtttctaatc acctagcccc cttccatttg aagaaattga 238380
cattttcctt ccatacggat ggaggtaagt ttgtaaccaa aggaaacctc caagctctta 238440
ttgagaatcc agactatccc gacctaaata atacgcgtat cctaatccct gatcttcttc 238500
tttctcttga tgaatcctca acttcacctt cttcaaaaga cttgaaaatc cagggttctg 238560
gagagatatt ttctttgcct ctggattcta ttactaagac ctatgggaaa caagtgcgtc 238620
tctctcctta ttttggttcc tctggagact tgaactttgt agtaaactac aatcctaaag 238680
atcagaataa gctcacacta ctatctanct ttaagtcaga agctctccta ggagaactga 238740
agttagtcat ggacttttct atgaagctat cttcaggaac tcagggaact ctccagtggg 238800
aagtgagccc agaacgttat gcaagtttct ttaaaaacgc atcatgctct cccacctgtt 238860
tgcttcatag aactgcaaat gtacgcttag acatctcaaa actctcttgt ccagaggaaa 238920
ccaaaggttt atcttgtctc acgcttcttg ccgcagagga cttgaaggtt cattagaagc 238980
aacaccgttg atcttctatg ataatgtgtc taaagagact tttattatta atgactttan 239040
aggttctttg cgagccaaca atttagacgc taaaatagaa tatgatctta aaggctcgtg 239100
tctagctcct aggcaagact ctaaaactct tgcagaattt tcattagaag gacaggtaga 239160
tcatctgttc tctccagagt ctcgagaatt taaacaaact gcaaattgga ttcacatacc 239220
ctcttcgttc attgctggaa tcattcccat gtctccagga ttgaaagctc agatatcctc 239280
gcttgcaggc cctagaatca acgtatcaat taaaaatgcg ttccgatttg gagaaggccc 239340
tgtcgacatt atggtcgact ctgaaaacct tcaagctcag attccactga tcttaaacga 239400
aaagtccatc ttactgagag agaatctaac agcgcacctt agtataaatg aagatgtaaa 239460
taaggctttc ctacaagagt ttaatcccct cttagcaggg ggagcctact cacaataccc 239520
agtaacctta gagatcgata aacaaaactt ctatctccct atacgcccgt attcttttga 239580
agaattccgc atccaatccg caacattgga tatggggaaa atctcaatag caaatacagg 239640
aactatgtat gctcttttcc aattccttga tattacggat caaaagcaat ttgtagaatc 239700
ttggttcact ccaattttct tttctgtaca aaaaggctct atcatttgta agcgcctcga 239760
cgcccttatc gatcgtagaa tccgccttgc tctatggggg aaaactgata tcgctcatga 239820
tcgtctgttt atgaccttgg gtatcgatcc tgaagttatt aagaaatact ttcataacac 239880
ctctttaaaa actaaaaact tcttccttat aaaaatccga ggatccatct cgtctcctga 239940
agtggactgg tcttcagctt acgctagaat cgctctatta aaaagctaca gtcttgggaa 240000
cccgtttagt agtcttgccg ataagctatt ctcttctctt ggcgactcta cccccccacc 240060
aacagtacac cccttccctt gggaaaaatc taattttgat tctatagaaa ataaatagaa 240120
tcaatataaa aacttaacag ttctttttaa ttaattattt ttataaatct taaaaataaa 240180
attaaattaa ttattaggaa taaaactcat gagaaaacgt cactctttcg actcaacctc 240240
tacaaaaaaa gaagccgtca gtaaagctat ccaaaaaatc atcaagatta tggaaacaac 240300
agacccttct ttaaatgtag aaacccccaa tgcagaaatc gaaagcattc tccaagaaat 240360
caaagaaatt aaacaaaagt tatcgaaaca agcagaagac ctcggtcttt tagaaaaata 240420
ctgttctcaa gaaaccctct ctaatcttga aaacactaac gcatcgctca agctttccat 240480
aggcagtgtc atagaagaac tcgcttctct caaacaactt gtagaggaaa gtattgaaga 240540
atccttaggg caacaagacc aactcatcca atctgtactt attgagatct ctgataagtt 240600
tctttcctct ataggggaga ctctttctgg aaatcttgat atgaaccaga acgtaattca 240660
aggactccta atcaaagaaa accctgaaaa atctgaagca gcttctgtag gatatgtaca 240720
gactctacta gagcctctaa gtaaaaggat cggcgagact cataagaagg tcgctactca 240780
tgatgtgaat atctcctcct tgcaatttca tatgatgtca gtggcaggag gtaggttccg 240840
tggtcatatt gatatgaatg gctatcgcgt tttaggattg ggagagccta aaaatggaga 240900
agatgctgtc tctaaggatt atttagaacg ttacgtaagc tcgcagctca ctatagacaa 240960
agttgaagac aagcccatta caaagccaaa taagggaaaa ttactctatt cccaagggac 241020
gagtcctaaa cttgaaggtc ccttaccttt agggcttctg acatctggaa tctcaggatt 241080
tacttggaaa agtgcaagca aatctaacga tggaagcttc ccttttagtg ctttaagaca 241140
caaggaaaca gagtcggata cagattgctt ccagattact tccacaacgc tctcaggaaa 241200

tcaagcagga acctacacct ggtccttatc tttaaaagtg ttggtgcctt caatcttcca 241260
aatcgaaaaa ccagaagtcc agctctctct tgtctactct tatgaagact ggcttcctat 241320
cgataatatc ttcaatatgt ctcagcctag gaccatacca ctagctctcc taggacaaac 241380
aatgcttgca ggacaaaaat atgatatcct agagctcgcc gcacatcaaa caaaccaaac 241440
tctaatgatt agcccgaact gttctcgatt ctctctacaa ttaaaacaaa ctaatcagtt 241500
tgaaaactcc cctgtcgatt tctatattgt ccatgccgct cattcctgcc actggtcagg 241560
attctaaagt atcactacag ctgctctagc agctgtagtc ctattgagca gagtggatat 241620
atttcgataa tggatgacca gaatcgagct ctccgtcacg cttgtcatag acccccgcaa 241680
cagttccttg atctataaga tagatacgat ccaaacaact atgaacaaat tgcatgtcat 241740
gagtagttaa ccctacagtc agttcctggt ctcgaagtgt ttctaaaaga tgtcggaacg 241800
atgccgtagc aaaaggatct aaagccgatg taggttcatc aaaaagtaat gtatgtttat 241860
ccatacataa agaacgtaca atagccacac gttgtttttg tcccccagag agctggtcag 241920
gataattctt agcaacctct tcaatatcca acaaatgtaa aagctcgaac gccttttctc 241980
gagcttcttc ggtactacga cccttgatat ggatttgtgg atgggtgcaa tttcctaata 242040
ctgtcatatg ggaaaataac tcgggttgtt ggaaaactag agctggagcc tccccttcaa 242100
tccaaatatc tccttgagtg ggctggacta agcccgccaa agcacgtaaa atcattgttt 242160
ttcctgaacc actcttccca acaaacagtg taatgtgccc tcgctctaaa gaaaaagtta 242220
caccatctag aatctttttc ttatttacag agtaggcaag gtttcggact ctaattgtca 242280
taactccttc tcttttctga taacctagaa atacaggaaa atgatgttgt cattaagaaa 242340
tatagcccag cacaaataag atacatttcc atagggttca actctctcga gacaatatcc 242400
ttagttactt tagttaactc ggggacaccg acaaccatta aaatactact ttccttgatc 242460
aaagaaacaa attcattggt taaggatggt aaaatatttt taaaaacttg aggataaata 242520
atataaacga aaatttgata cttcttatat cccaaaacca ttgcggattc ccactgccct 242580
atagaaagag aattgatacc tccacggata ttttctgcaa gatatgctgc agaattcata 242640
cttaaagcaa taattccagc aactagaggt gtgggttcta tggggagaac ttcaggcaat 242700
ccaaaatata taatcaaaat ttgaataaat aaaggagtcc cgcggataac agtcacataa 242760
ctattagcta aaagtttcgt taacttagaa gggaagtata gagaagtcac cgttccaatc 242820
aaaagaccta aaatagaacc acatagaatg ccgattccgc tcacacataa ggtgtatccg 242880
catcctctta acaacagtct tgctatagct agccaatgat ccactccgac ctcttatgca 242940
tatttattta taatgaaatc atatttatgc aattaatcca agaaaacttg cagtaaattt 243000
gcaatcgaaa ccatcaacaa tgggagcctc ccttcatcta taggagtgac aaaaatcgtg 243060
tcatctcctg ccaaagttcc aagaatttca tctttgagcc cttgatctaa caaagcagcg 243120
atccaagaag ctgaaccagg aaccgtacga attacaataa gagaggcgtt atggcgaata 243180
gagagcacca aatgacgggt cgtggtcttc tctgttgaag agggtaaaga ataacgagca 243240
ccacgctctc cagcaacctt tacagcctga atctttcgta gccaacgaga tacagacgac 243300
tgggttgtag caaaaccttg agctaagagt tttgcacata attcctcctg agttgccgct 243360
ccttcaagac gtaaaatttc ttttaaagcc tcatctatag ttactttttt tttcataaaa 243420
accccatgta acttttactt gctcatattg agaagtcccc catactataa aaggcaacgt 243480
tttcttttct tggtttttta tgctcaccct aggcttggaa agttcttgcg atgagactgc 243540
ctgcgctata gttaatgagg ataagcagat attagcaaat attattgcct ctcaagatat 243600
ccatgcatcc tatggcggag tcgttcctga acttgcttca agagcacatc tccatatctt 243660
cccacaagtg ataaataaag ctctacaaca ggccaactta ttgatcgaag atatggatct 243720
gattgcagta acgcaaactc cagggttgat aggttctcta tcagtaggag tgcattttgg 243780
taaaggcatt gccataggag caaaaaaatc cttgattgga gtcaatcacg tcgaagctca 243840
tctctatgct gcctatatgg cagcgcaaaa cgtgcaattc cctgctttag gtcttgtggt 243900
ctctggagct cataccgcag cgttttttat agaaaatcct acatcctata aactcatagg 243960
aaaaactcga gatgatgcta taggagaaac ttttgataaa gtaggacgct ttctaggatt 244020
accataccct gcaggcccat taattgaaaa actcgcttta gaaggctctg aggacagtta 244080
tccttttagt ccagctaaag tcccaaacta tgacttttca ttcagcggtc ttaaaacagc 244140
tgttctctac gcaatcaaag gaaataatag tagcccccgc tctcctgctc cagagatatc 244200
tttagaaaaa caaagagata tcgctgcttc atttcaaaaa gcggcctgca ctactattgc 244260
acaaaaactt cccactatta taaaagaatt ttcgtgccga tctatactta ttggaggtgg 244320
cgtagccatt aatgaatact ttagatccgc aatacaaact gcgtgtaatc tacctgtata 244380
cttcccccct gctaaactat gctcagataa tgctgctatg attgcaggtc tagggggaga 244440
aaattttcaa aaaaactcta gtattccgga aattcgtata tgcgcaagat atcagtggga 244500
atctgtatca ccattctcct tagcctctcc gtagtcctcc aaggctgcaa ggagtccagt 244560
cactcctcta catctcgggg agaactcgct attaatataa gagatgaacc ccgttcttta 244620
gatccaagac aagtgcgact tctttcagaa atcagccttg tcaaacatat ctatgaggga 244680
ttagttcaag aaaataatct ttcaggaaat atagagcctg ctcttgcaga agactactct 244740
ctttcctcgg acggactcac ttatactttt aaactgaaat cagctttttg gagtaatggc 244800
gaccccttaa cagctgaaga ctttatagaa tcttggaaac aagtagctac tcaagaagtc 244860
tcaggaatct atgcttttgc cttgaatcca attaaaaatg tacgaaagat ccaagaggga 244920
cacctctcca tagaccattt tggagtgcac tctcctaatg aatctacact tgttgttacc 244980
ctggaatccc caacctcgca tttcttaaaa cttttagctc ttccagtctt tttccccgtt 245040

cataaatctc aaagaaccct gcaatccaaa tctctaccta tagcaagcgg agctttctat 245100
cctaaaaata tcaaacaaaa acaatggata aaactctcaa aaaaccctca ctactataat 245160
caaagtcagg tggaaactaa aacgattacg attcacttca ttcccgatgc aaacacagca 245220
gcaaaactat ttaatcaggg aaaactcaat tggcaaggac ctccttgggg agaacgcatt 245280
cctcaagaaa ccctatccaa tttacagtct aaggggcact tacactcttt tgatgtcgca 245340
ggaacctcat ggctcacctt caatatcaat aaattccccc tcaacaatat gaagcttaga 245400
gaagccttag catcagcctt agataaggaa gctcttgtct caactatatt cttaggccgt 245460
gcaaaaactg ccgatcatct cctacctaca aatattcata gctatcccga acatcaaaaa 245520
caagagatgg cacaacgcca agcttacgct aaaaaactct ttaaagaagc tttagaagaa 245580
ctccaaatca ccgctaaaga tctcgaacat cttaatctta tctttcccgt ttcctcgtca 245640
gcaagttctt tactagtcca acttatacga gaacagtgga aagaaagttt agggttcgct 245700
atccctattg tcggaaagga atttgctctt ctccaagcag acctatcttc agggaacttc 245760
tctttagcta caggaggatg gttcgcagac tttgctgatc ctatggcatt tctaacgatc 245820
tttgcttatc catcaggagt tcctccttat gcaatcaacc ataaggactt cctagaaatt 245880
ctacaaaaca tagaacaaga gcaagatcac caaaaacgct cggaattagt gtcgcaagct 245940
tctctttacc tagagacctt tcatattatt gagccgatct accacgacgc atttcaattt 246000
gctatgaata aaaaactttc ataatctagg agtctcacca acaggagttg tggacttccg 246060
ttatgctaag gaaaattagc acctctttta atctcgcaaa cttgtcaaga actgaatctt 246120
atactaaact gggtgccttt gtggcacctc gtttccttct gactgctctt ctctctctac 246180
tcaaccgcat ccctaaaagt tgaaatctta ttctaaagaa aggtctttta tgctccgttt 246240
cttcgctgta tttatatcaa ctctttggct cattacctca ggatgttccc catcccaatc 246300
ctctaaagga atttttgtgg taaatatgaa aggaaatgcc acgctccttg gatcctggaa 246360
aaactcgtct cattgcagac caaactctaa tgcgtcatct atatgaagga ctcgtcgaag 246420
aacattccca aaatggagag attaaaccag cccttgcaga aagctacacc atctccgaag 246480
acgggactcg gtacacattt aaaatcaaaa acatcctttg gagtaacgga gaccctctga 246540
cagctcaaga ctttgtctcc tcttggaagg aaatcctaaa ggaagatgcg tcctccgtat 246600
atctctatgc gtttttacct atcaaaaatg ctcgggcaat ctttgatgat actgagtctc 246660
cagaaaatct aggagtccga gctttagata agcgtcatct cgaaattcag ttagaaactc 246720
cctgcgcgca tttcctacat ttcttgactc ttcctatttt tttccctgtt catgaaactc 246780
tgcgaaacta tagcacctct tttgaagaga tgcccattac ctgcggtgct ttccgccctg 246840
tgtctctaga aaaaggcctg agactccatc tagagaaaaa ccctatgtac cataataaaa 246900
gccgtgtgaa actacataaa attattgtac agtttatctc aaacgctaac actgcagcca 246960
ttctattcaa acataagaaa ttagattggc aaggacctcc ttggggagaa cctatccctc 247020
cagaaatctc agcttctcta catcaagatg accagctctt ttctcttccg ggcgcttcga 247080
ctacatggtt actctttaat atacaaaaaa aaccttggaa caatgctaaa ttacgcaagg 247140
cattgagcct tgcaatagac aaagatatgt taaccaaagt ggtataccaa ggtcttgcag 247200
aacctacaga tcatatccta catccaagac tttatccagg gacctatccc gaacggaaaa 247260
gacaaaacga aagaattctt gaggctcaac aactctttga agaagctcta gacgaacttc 247320
aaatgacacg cgaagatcta gaaaaggaaa ctttgacttt ctcaaccttt tctttttctt 247380
acggaaggat ttgccaaatg ctaagagaac aatggaagaa agtcttaaaa tttactatcc 247440
ctatagtagg ccaagagttt ttcacaatac aaaaaaactt cctagagggg aactattccc 247500
taaccgtgaa ccaatggacc gcagcattta ttgatccgat gtcttatctc atgatctttg 247560
ccaatcctgg aggaatttcc ccctatcacc tccaagattc acactttcaa actcttctca 247620
taaagatcac tcaagaacat aaaaaacacc tacgaaatca gcttattatt gaagcccttg 247680
actatttaga acactgtcac attctcgaac cactatgtca tccaaatctt cgaattgctt 247740
tgaacaaaaa cattaaaaac tttaatcttt ttgttcgacg aacttcagac tttcgtttta 247800
tagaaaaact ataggagaaa aagtttagac ttaaaattcg atagtaaatt tattaaagta 247860
atttttaaaa tgttttcacg atggatcacc ctctttttat tattcattag ccttactgga 247920
tgctcctcct actcttcaaa acataaacaa tctttaatta ttcccataca tgacgaccct 247980
gtagcttttt ctcctgaaca agcaaaacgg gccatggacc tttctattgc ccaacttctt 248040
tttgatggtc tgactagaga aactcatcgc gaatccaatg atttggaatt agcgattgcc 248100
agtcgctata cagtctctga agacttttgc tcttatacgt tctttatcaa agacagcgct 248160
ttatggagcg acggaacacc aatcacctcc gaagatatcc gtaacgcttg ggagtatgca 248220
caggagaact ctccccacat acagatcttc caaggactta acttctcaac tccttcatca 248280
aatgcaatta cgattcatct cgactcgccc aaccccgatt ttcctaagct tcttgccttt 248340
cctgcatttg ctatctttaa accagaaaac ccgaagctct ttagcggtcc gtatactctt 248400
gtagagtatt tcccagggca taacattcat ttaaagaaaa accctaacta ttacgactac 248460
cactgcgtct ccatcaactc catcaaactg ctcattattc ctgatatata tacagccatc 248520
cacctcctaa acagaggcaa ggtggactgg gtaggacaac cctggcatca agggattcct 248580
tgggagctcc ataaacaatc gcaatatcac tactacacct atcctgtaga aggtgccttc 248640
tggctttgtc taaatacaaa atccccacac ttaaatgatc ttcaaaacag acatagactc 248700
gctacttgta ttgataaacg ttctatcatt gaagaagctc ttcaaggaac ccaacaacca 248760
gcggaaacac tgtcccgagg agctccacaa ccaaatcaat ataaaaaaca aaagcctcta 248820
actccacaag aaaaactcgt gcttacctat ccctcagata ttctaagatg ccaacgcata 248880

gcagaaatct taaaggaaca atggaaagct gctggaatag atttaatcct tgaaggactc 248940
gaataccatc tgtttgttaa caaacgaaaa gtccaagact acgccatagc aacacagact 249000
ggagttgctt attacccagg agcaaatcta atttctgaag aagacaagct cctgcaaaac 249060
tttgagatta tcccgatcta ctatctgagc tatgactatc tcactcaaga ttttatagag 249120
ggagtaatct ataatgcttc tggagctgta gatctcaaat atacctattt cccctagaca 249180
aaagaagtct ttggtaaggg gtttttttta ttgaagagac ctttcttcac cagtatacta 249240
ttgtatcttt ataagaagtt tcttctgtat ataaattgct atatgaagaa acagtaagta 249300
ataggagcat taggatacgc ctccttaagg tatttctatc ctgaaagata caataatctc 249360
attcccccat cgactaaatc caccacggac tccgacctcc catgtcttca atccatatga 249420
acgtaatatt aagtagcaaa ttgagtacta tataatgaag atgcataggc ttaaacctac 249480
cttaaaaagt ctgatcccta atcttctttt cttattgctc actctttcaa gctgctcaaa 249540
gcaaaaacaa gaacccttag gaaaacatct cgttattgcg atgagccatg atctcgccga 249600
cctagatcct cgcaatgcct atttaagcag agatgcttcc ctagcaaaag ccctctatga 249660
aggactgaca agagaaactg atcaaggaat cgcactggct cttgcagaaa gttataccct 249720
gtcaaaagat cataaggtct atacctttaa actcagacct tctgtgtgga gcgatggcac 249780
tccactcact gcttatgact ttgaaaaatc tataaaacaa ctgtacttcg aagaattttc 249840
accttccata catactttac tcggcgtgat taaaaattct tcggcaatcc acaatgctca 249900
aaaatctctg gaaactcttg ggatacaggc aaaagatgat cttactttgg tgattaccct 249960
agagcaacct ttcccatact ttctcacact tatcgctcgc cccgtattct cccctgttca 250020
tcacaccctt agggaatcct ataagaaagg aacaccccca tccacataca tctccaatgg 250080
gccctttgtc ttaaaaaaac atgnacacca aaactactta attttagaaa aaaatcctca 250140
ctactatgat catgaatcag taaagttaga ccgagtcacc ttaaaaatta tcccagacgc 250200
ctccacagcc acgaaacttt tcaaaagtaa atctatagat tggattggct caccttggag 250260
cgctccgata tctaacgaag accaaaaagt tctctcccaa gaaaagattc ttacctattc 250320
tgtttcaagc accacccttc ttatctataa cctgcaaaaa cctctaatac aaaataaagc 250380
cctcaggaaa gccattgctc atgctattga tagaaaatct atcttaagac tcgtgccttc 250440
aggacaagaa gctgtaactc tagttccccc aaatctttca caactcaatc ttcaaaaaga 250500
gatctcaaca gaagaacgac aaacaaaagc cagagcatat tttcaagaag ctaaagaaac 250560
actttctgaa aaagaactcg cagaactcag catcctctat cctatagatt cctcgaattc 250620
ctccatcata gctcaagaaa tccaaagaca acttaaagat accttaggat tgaaaatcaa 250680
aatccaaggc atggagtacc actgcttttt aaagaaacgt cgtcaaggag atttcttcat 250740
agcgacagga ggatggattg cggaatacgt aagccccgta gccttcctat ctattctagg 250800
caaccccaga gacctcacac aatggagaaa cagtgattac gaaaagactt tagagaaact 250860
ctatctccct catgcctaca aagagaattt aaaacgcgca gaaatgataa tagaagaaga 250920
aaccccgatt atccccctgt atcacggcaa atatatttac gctatacatc ctaaaatcca 250980
gaatacattc ggatctcttc taggccacac agatctcaaa aatatcgata tcttaagtta 251040
gatccgaaat ggaaaaatta aaaattttat agacaatctt gaaaagagaa ttaaaaattt 251100
ttaatttaaa ttatagttgc aattgaaaac gcccctaaga atcggggccc ctaactactg 251160
aatctacgtg aaatgcaatt gttaaaaaaa taagagattt atagaaaaat aaaagtcttc 251220
cttcccaacg cattttttgt attgaagatg actaanantg naagtataat gacttaaaca 251280
ttttagagct gaggtctact tcaaggtaga aatgcttaat agggttgtct ctcgtgttct 251340
catacataaa aaaccgaatt ctttttaatt tgctttctct atggattgtt ttgacactca 251400
cgttcctagt tatgaaaacc atcccaggag atcctttcaa tgacgaaggc tgcaatgttc 251460
tttccgaaga ggtcttacaa accctaaagt ctcgatacgg tttagataaa cctctctatc 251520
aacaatacac acaatacctc cactccatcg caaaactaga ttttgggaac tcgttagttt 251580
ataaagatcg caaagtaacg aacatcattt cgactgcctt tcctatatca gcaatcctag 251640
gattgcaaag tctttttctc tccataggag gggggatcgc tctcggcacc atagcagcat 251700
taaaaaaaaa gaaacaaaga cgctatattc taggcgcctc tatactccaa atctcgattc 251760
ctgcttttat attcgcaaca ctcttacaat atgtctttgc tgtaaaaatt cctcttcttc 251820
ctatcgcctg ttggggaagc tttactcata ctatactccc gactctcgca cttgctgtaa 251880
ctcccatggc cttcatcata cagcttacct actcttcagt atccgcagca ttaaacaaag 251940
actatgtcct actagcctat gcaaaaggac tctccccact taaagtcgtt ataaaacata 252000
ttttacccta cgccatattc ccaaccattt cttattccgc attcctaact actacagtga 252060
ttacaggaac ctttgctatc gaaaatatct tctgtattcc tggattaggt aaatggttta 252120
tttgtagtat caaacaacga gactacccag tagcccttgg cttatccgta ttttatggaa 252180
cttatttatg ctctcttctt tactttctga cctgattcaa tccattatag atccgcaaat 252240
ccgttatgcg cacggaaagg aaaaaaaaag aaaataaaag tcataagaaa aaaagaagca 252300
taaacttatg gaaaacctat cctcagctcc atcacgtagc atttggaaat ctataatcca 252360
gaataaaatg cttgttctag gcctcacgac cctcataatt ctaatgcttg gagccctcct 252420
tttgccatgg ttctatcaag attatgaaca gacttcatta aaagacattc ttgtctctcc 252480
atgctcgcgc tttccctttg gcacagacac tctaggcagg tgcatgtttg cccgaactct 252540
acgaggtcta cgactctcct tactcatagc gacgatcgct acacttattg atgtgtgtgt 252600
gggactttta tgggccactg ttgctatatc tggagggaaa aaaatagatt tcttaatgat 252660
gcgaaccaca gagattctct tttctctacc gagaatcccc atcattattc ttcttttagt 252720

catcttccat cacggactgc tcccgctaat ccttgcaatg acaattacag gatggattcc 252780
tatatctcga attatctacg gtcagtttct actcctgaaa aataagccct ttgtcctttc 252840
tgcaaaagcc atgcatgcct ccacgtttca tattctaaag aagcatcttc ttcccaatac 252900
cctagctccc atcatatcta cattgatttt tactattcct aacgctatct ataccgaagc 252960
cttcattagc ttcctgggtc taggaataca gcctcctcaa gcaaagcctc ggcaccttag 253020
ttaaagaggg aatcaatgct atagattact acccatggct atttttcttc ccctctctaa 253080
ttatgattgc cctctctata agcttcaatc tcatcggcga gggggctaaa acactatgtc 253140
tcgaagaggg atctcatgga taactactta ctaaatatca aggatctcac aataacctct 253200
acaaacccta agagaactct aattgaaaat ttatcactac agctcaaaga aaatcgaaat 253260
cttgctctag tcggagagag cggctcaggg aaaactacaa ttaccaaagc catcctaggc 253320
ttcctccccg aaaattgtct gatcaaaacc ggaagtattt tatttgaaga tatagatatt 253380
accaagctct caccaaaaga gctccataag atccgcggtc aaaagatcgc cacaatacta 253440
caaaatgcta tgggttctct aaccccatcc atgcgcatag gaatgcaaat catagaaacc 253500
ttaagacaac accacaaaat gaataaagag gaagcctata ataaagctat gcaactcctt 253560
accgatgttt gcattcctaa tccaaaatat agcttctcac aatacccctt tgaattgagt 253620
ggtggcatgc gccaacgtgt tgtaatcgcc atagcactcg caagccaacc taagctcatt 253680
cttgccgatg aacctacaac agccctagac tctatgtcac aagctcaagt ccttaggatt 253740
cttcgtaata tccaacaaca gaaacaagct acaatccttc ttgttaccca taacctctct 253800
ctagtcaaag agctctgtaa tgatatctgt attatcaaag acggcaaact catagaaaca 253860
ggaaccgttg aagagatttt cctctctccg aaacacccct atactctcaa gctcctcaat 253920
gctgtctcta aaatccctat taaaaaaacc agctctccca tccttaaaaa taagttccaa 253980
cctctaatga gtatgcaagg tggtttatga caactctact aagtataaag gacctttccc 254040
taaccatcag aggaaagaaa attcttaatc atattaacct caacctaatc aaaggaagct 254100
acttaacaat cgtaggaccc agtggctcag gaaaatcttc cttagcactt actattctgg 254160
atctcctaaa acctaccaca ggaacaatca cgtttcatat ggaccccaag atccccagag 254220
cacgtaaggt ccaagtgatc tggcaggata tcgactcgag tctaaatccc tgcatgtcta 254280
taaaaggaat tatttccgaa cccctaaata tcatcggaac ctattctaaa gccgaacaaa 254340
ataaagagat ttataacgtt cttgatcttg tgaacctccc caagtctgtt ctccacctta 254400
agccttataa actcagtgga ggacaaaaac aacgcatagc cattgcaaaa gctctagtct 254460
caaaacccga gctccttatc tgtgatgaac ccctctcctc actagacacg ctcaaccaat 254520
ccctaatctt agaccttttt caaacaataa aaaaagaata ccaaaatacc cttctcttta 254580
tcacccacga tatgtccgca gcgtattata ttgcagacac tatcgccgtc atggatcaag 254640
gaagtcttgt cgaacatgct tgtagagaaa aaattttctc aactcccaag catacaacta 254700
cacaagatct tctcgacgcc atccccatat tttccttgat ctccacagaa atggaaccct 254760
cggaagaata cgaattacaa gtcgcctcaa agtanataga tttagaaata acgaattctt 254820
atagaaaaat taggattttt gacgtctctc aatagaaaaa tgaaagtctg atatgatgaa 254880
tcaaaaaaac caatagttaa tttgattttt gaattgtttt ttctcagaaa gttccttgac 254940
tccactaaaa actcgctatc tagttataaa atagagtcaa cgcctcctac ggccgtccaa 255000
tctagggtta gatcaaaaaa acttaacact agaatcagac gccaattatt tctaaactat 255060
tgtattacaa tcattcctat ttgaattaaa cactttgaat ccccataatg ttcttgcttc 255120
aacaaacgca accattctaa aagaaccctc gccttctcta ttgcgacgtg taccttaatt 255180
tattgaaata taaagcatcc gaaaactgta gttctattaa gagctgggcg cttaggaacc 255240
ttaagttatt cctataaatc gtccccctcc cccaaaaaaa caataaagag accagttgta 255300
tgaataccta taccttctct cctacacttc agaaaagctt cagcctattt cttttagaaa 255360
aattagactc ttactttttc tttggaggga ctcgtacaca aatcttagtc atcacaccaa 255420
ccaatattag attagcagct aaaaaaagag ggtgtaaggt ttctactata gaaaagataa 255480
tcaagatcct ctcttttatc ctgctgcccc tagttatcat tgcctttata cttcgctatt 255540
tcttacataa gaaattcgat aaacagttct tgtgtatccc aaaagtcatt tctaacgaag 255600
acgaagctct tcttggatct agaccacaag cagttgaaaa agcagttcga gaaatatctc 255660
cagccttctt ctctatacca agaaaatacc aacttattag aatcgacact cctaaagatg 255720
acgctccctc aatccttttc cctataggca tagagatcat tctcaaagat ttatgtattg 255780
atacactcaa gcaatctaat cttttcctta aaagagaaat ggatttctta ggtcatccag 255840
aagaaaaagc attattcgac tcgatatgtt ctatagaaaa agatcaagaa tggatgagct 255900
tggaaagtaa aaaactttta atcacgcact tcctaaagta tctctttgtc tctggaatcg 255960
aacaactaaa tccaggcttt aacccagaga atgggcgtgg gtatttttca gaaataagta 256020
cagcaaagat ccattttcat cagcacggtc gatatgggcc aatccgttct tcgggaccca 256080
tcatgaagga aatataaaaa taaaaggcat gggctaccaa atcttcacaa gacttaaaaa 256140
acttggaatc tcattctcct cttataattc cattaatcct aatccttact tcttcgatga 256200
aggctgtttt gtctactggg aatcccaatt taaatccgca ctgcaagatc acgggattct 256260
ccagaaacag acagaaacat tctatagaaa tacttaaata catttctaag aagtctgtct 256320
aagagaactc caaacctata aaaaaaccct taaatatttc ctaaatagaa gactctataa 256380
aaggctatgg ttccgctagt gcctttagaa atccccctca gctattgcta aagttctttc 256440
tagtatgtga agagttatgt atacttactg ttgctacaca tagagctctc ttagaaactc 256500
ctttagctct atcatttttt aaagaactta aagacaaaat atgtctacag ggcgaaagac 256560

atactacaac tacataacct ataaaggatt tactatcctt aatacatcac cgttatgttc 256620
ttaatttctc tatctgtaaa tcgttgacag aagaagccct gcatgctacg actcaattaa 256680
cagtgatgca attggagtat ctttgctaat ggatatttcc catatcctgg aagatcttgc 256740
ctatgacgaa gggatccttc caagggaagc tatagaagcg gctattgtta aacaaatgca 256800
aattacgcct tatttactgc atattttaca cgacgctact cagcgcgtcc ctgagattgt 256860
aaatgatggg agttatcaag gtcacctcta tgccatgtat ctcctcgcac aattcagaga 256920
aagtcgcgca ctccctctca tcattaaact ctttgcattt gaagatgata ctccacacgc 256980
aatagcaggt gatgtcctaa ccgaagatct gcctaggatc ctagctagcg tctgcaatga 257040
tgactcgcta attaaagagc tcatagaaac tccaaaaatc aatccttatg tgaaggcagc 257100
ngcaatctct ggtcttgtaa ctcttgtagg agccgggaaa attcctaggg ataaagttat 257160
ccgttanttt gcagaacttc taaactatag attagaaaaa cagccctcgt tcgcttggga 257220
taacctaatc gcagggatct gtactcttta ccccggagag ctcttctatc caataagcaa 257280
agcctttgac ggaggacttg ttgatacatc tttcatcagc atggaagatg tcgaaaatat 257340
tatccacgaa gaaaccgtgg aatcttgtat ccataccctc tgttcttcta cagaactcat 257400
taatgacact ctagaagaaa tggaaaaatg gttagaagac ttccccatag aaccgtgaca 257460
tcgatcaatc aatatttcta accactacac cgaggtccta agtgaacaaa aaaaaacgtt 257520
tcttatctct cctctttctg actgcagtac ttttaggcat ttggttctct ccccatcccg 257580
catctataaa ttctaatgct tggcaactct tcgctatatt cacaactact atcatgggaa 257640
tcattttcca gcccgtcccc atgggagcta ttgccattat tggaatctcc acactactac 257700
tcacacaaac gttaactcta gaacaaggat tgtcaggatt ccataatcct atagcgtggt 257760
tagtcttcct ctccttctca atagcaaaag gaatcataaa aacaggactc ggtgaacgaa 257820
tcgcatactt ctttgtcagt gctttgggga aaagtcctct aggactcagc tatggactgg 257880
taatcacaga ttttttcctt gcacctgcaa tccccagcgt gactgctcga gctggaggga 257940
ttctctatcc cgtagtcacg agcttatcag attcctttgg aagttccgca gaaaaaggaa 258000
ctcaagatct tatcggatcc ttcctcatta aagttgccta tcaaagctca gtgatcacca 258060
gcgctatgtt cctcactgct atggcaggaa accctctggt ggcagctcta gcaggccacg 258120
tcggggtttc tttatcttgg gttctatggg caaaagccgc aatcattcca ggactcctca 258180
gtctattcct catgccgatc atactctaca aactctaccc accaaaaaat cacatcttgt 258240
gaagaggcta tccgatcggc aaaacttcga ctcaaagaaa tggggccgct aaaaaaagaa 258300
gaaaaaacaa tattgatgat ctttttcctc cttgtagtcc tctggacttt tggagatctc 258360
ttaggaatct cagcaacaac agcagccctt ataggactgt ctctcctcat cctcactaat 258420
attctagatt ggcaaaaaga tgtcatagca aatacaacag catgggaaac attcatctgg 258480
ttcggagcct taatcatgat ggcttccttc ttaaaccaac tcgggtttat cccactcgta 258540
ggagactcag cagcagcact ggtcagtggc ctctcttgga aaattggctt ccctctactc 258600
ttcctgatct acttctactc ccactacctt tttgctagca ataccgcgca tatcggagcc 258660
atgtatccca tattcctcgc agtctccata tcgttaggga ctaatcctat attcgcagca 258720
ctcaccctag cattcgcaag caaccttttt ggaggactca ctcattacgg atccggaccc 258780
gcacccctct acttcgggtc acatctcgtt actgtccaag agtggtggcg gtcaggattc 258840
gctcttagca ttgtcaatat tgttatctgg ataggaatcg gaagcttgtg gtggaaagcc 258900
ctcggactca tttagaaacc aagtctttta tatagaaaat attctttagc aagtttatat 258960
tttgtgaaat atganagctn aatcatagga ccacctgtga tttagccttc tgtcataagc 259020
tctcttttta tagcctaaag ttgaagaaag cttagccgta aaaggtcttg ttgtattctt 259080
ccaacgagtt acaacgaaat acactaaagt tcattctcaa agtagaatca ttccttaaaa 259140
taagaccttt ttaaaaaaac ctgatgttct tatgcacaaa aagaatccca ttgactttag 259200
ccttgggatg atgcaaaaag caaaattaga gtgtagccgt tatccttatg catcctttat 259260
acgttgatct tgatactatt atcagctcct actctcctcc cttacctaaa gaatttcaag 259320
aagcagcctc tttaattgct gttccagata cttcacattc taagcctgtc gttccaggag 259380
tgaaaaccct ctttccacaa acctaccacc ttccctatct aaagtttgtc caaggagaaa 259440
atgtcgttca cactcctcta aaagtaggcg taatgttctc aggaggacct gctccaggag 259500
gacataatgt catccaagga ctcttcaata gtctaaaaga tttccatccc gattcctccc 259560
tcgtggggtt cgtaaataat ggagacggtc ttacaaacaa taaaagcata gacattactg 259620
aagagtttct ctccaaattc cgaaattccg gaggcttcaa ctgtatagga acaggaagga 259680
aaaaaattgt aactccagaa gctaaagagg cttgtctaaa gaccgcagag gctctggatc 259740
tcgacggact agtcattatt ggcggtgatg gctccaatac agcaaccgct attcttgcag 259800
agtattttgc aaaacgacgc ccaaaaacct ctattgtcgg agttcctaaa actatagatg 259860
ggatctacaa acacaccttc ttggatctgg ccttcggatt tgatactgca acaaaattct 259920
actcttcaat cattagcaat atttcaagag atgctctttc ctgtaaagct cattaccact 259980
tcattaaact tatgggacgc tcagcatccc atattgcttt ggaatgtgct ctccaaactc 260040
atccaaatat tgcccttatc ggcgaagaaa ttgccgaaaa aaatctacca ctaaaaacca 260100
tcatccataa aatctgctcc gtaattgcag atagagccgc tatggaaaaa tactatggcg 260160
tcatcctcat cccagaaggc attatcgagt tcatcccaga aatcatcaac ttaattacag 260220
aaatcgaaag cctatcagaa tacgaagata aaatctccag gctctctcca gaatcccaac 260280
gcctactgaa aagcttccca gcacctatca tcgagcaaat cctcaatgac cgcgatgctc 260340
acggtaatgt ctatgtttct aaaattagtg tcgataaact actcatccac ctggtcagca 260400

atcatctcca acaatatttc cctaacgtcc ctttcaatgc gatctcacat tttctaggat 260460
atgaaggacg ctcgggattg cctacaaaat tcgataatac ctacggctat agcctcggat 260520
acggcgccgg tattctcgtc cgcaatcact gcaacggcta tctctctact atagaatccc 260580
tagcatgccc tttcatgaaa tggaaattac gggcaattcc cgtagtgaaa atgttcacag 260640
taaaacaaca ggcagatgga actctacaac ctaaaattaa aaaatacctc gtagatatag 260700
gaagcacggc atttcgtaaa tttaagctct ataggaaaat ttgggccctc gaagactcct 260760
accgattcct agggcctcta caaatagaaa ctcctccaga aatgcactct gataatttcc 260820
ctcctcttac ccttttgctt aatcataact tttggcaacg tcaccagggt tgcatagaaa 260880
tccctgatac tacgtattaa ttacgttcta atacgttctt aattcctgaa aatctaagat 260940
gcttccacgc aagcttatcc gcatactaaa cttagcagaa gtaatggatc tgattcggat 261000
acagtaagac tgttacaagc cacaactggt gcactgaatc ctgtacatgc catgcaactc 261060
ggtacgaatg ccacgccatg acgaatcaaa gggaaactag atacaaacgt ggcaataaga 261120
aaagagagcg tcgatcaacc aaggactttt gataagctac ctgcctatag aatcctctcc 261180
ccacttaaca aagtttttta taaaganact ttcattctta ttaataagag ataattcaat 261240
cgttactatt taaaaataag caacttagaa tcaactatag agagaaaaca attattatat 261300
taaaattcat cgaacaacat taggttgaag atggaaactt atagcttttc tacagaacta 261360
cagaaaaata cttctctcta tatcatggaa aagttagatt cctatttttc ctttcaaggc 261420
aaacgcacac gggtaattgc aataacccct gcaggtttag ccatcgccta cgagcagaat 261480
atccacctct ctatgaccgt gaaaatatta aaagtcctct cctttccacg gtctctcctc 261540
aggacaacta gtttgtggta tcgcccttga taatacgaat atcgataaac aaaaacaact 261600
atccgaagaa ctcaaagact ctcccaacca acattttgtc tatatagaac tccaaaatgc 261660
cttcttctcc tataccgaga tctaataaag tttcatagac atcgtatcga agattttgta 261720
atcattcctg cttattctgt gtctttccgc ggcgacttca cagtttcttc ttcgaacata 261780
gaatctaaac tatgtttagc ggcanttcat acttcctcat aactaaacag ccccgttcta 261840
acttactttt agcaattgca aactcaagct ctttagtttg ttttattccc ttttcgagct 261900
ctagaaggct gaatttctcc ctaatgaact cctctttagc cctagcctgc tcttgaaaat 261960
ctgtggatgt cttacataac aatgtatact catacagcaa caataagtac ctcaatcgtg 262020
attgatacaa acaatccttt tgatactcct cttgactggg agtcttagaa aaacaaaaaa 262080
gagaaacatc atgcaattca ataataatgt cgctaagttt cctttgcaga tcttgaatcg 262140
agcatttctc catcgcatgc tcctctctaa gaaggctctc attgagctcc ttggtttctt 262200
tttcaaaagt gcatagcttc ctctttgcta tttcaacaca atcattcccc caatccacaa 262260
actcttctat cttgcaaaga ggccatctaa aatctaacag gtaatatgca ttcaaatctc 262320
ggattgctgt ttttagaaaa tgtatctccg aagcaataca aaaagccctc cgattcactt 262380
ttgataaata ggcacccaag aattctatag tctgatagat agcacctatg tccccaagac 262440
ctaaaacatc tctggctttt gtcagtaaag cattccacgc atcgattgct ttttgagact 262500
caaaaagcac atcttcatct tccgtagcct ttaaaaattt gtcctggccc agcccgagta 262560
aaacgaattc aagatgatcc aaacattgat aaagtgaagt gatctcttgg tcacttgtcg 262620
gcttctggtc ttttaaatgc gcaaactctc ttttaagagc ctctaactct actagcatag 262680
tgctaatagt agcatgctct ttctctctaa ttgtcttgaa atactctctt tctctttccc 262740
aaagtttctg gtactataat aaagattttg cctcctcaac taaaagtccg actcctccag 262800
ctaataaaag aagtcccaga acaatcccaa gaaccccaaa aactaatgaa aggactccat 262860
gagaaaatac tgtgagtatg gcaaccccag caagaagaaa aagagaacct ataataacta 262920
agctcacagc taagatcaca aaagtacttt gtttaaagca cctcttctgt tgtatctggt 262980
cagaagtctt atgaagcaaa gcagattgaa tagaagaggc ctgtacattg gaaatatcag 263040
gataagacat aacacattct caacaaaact tatgggaaaa gaataaaatc ttctttaagg 263100
cattttattt ttaagcaatg ccttataaaa agaaatgtta taactttgaa tggcttaaaa 263160
aataaaatat ttatttgttt atgcctctag aaggatatcc acataaagtt gagcaatctc 263220
gagttctaaa gtctttgtat gtactacgca catttgtagc tcttcaaagg tctcaataca 263280
atcctccaca gccatccgag cccttaccct gtcaagagta tctattggtt tcctagatac 263340
tacctcatcg ttgtatatcg cccataaagc atgtaacata gtcacacgta tacagtggat 263400
ttggtattct tccaaaacat cctcagaatc actcgtaaaa agattcgttt cattttctaa 263460
gtctgtttca atctcagtac aacaacacag aagttctgaa accgatagct gttttgctct 263520
tttcttttct tcattcactc tcatatcttc cacacagact tttacataat tacgcaaatt 263580
ttttgctgtg cggcacatag catatgcttc cataagaaaa tggggagcag ttccctcaaa 263640
aacatcacca atataaagat tactcagtgc tttctttgaa acatgaattt ctgaatagca 263700
ccaaaggtct tctctgtagg atctccaaaa agcagctttc ctatcataga gcttcttgaa 263760
gatctcctga gcagattttc ttcgaaataa tagtgcctct ttacactgct ctaaaataga 263820
gttttgctct tgggctctct cctgaatttc acttagattt cgaggaacac ctatctttag 263880
aatctgtttt ttctctgttt tagaaccttc caaaacatcg agtcgtgcag aaaacccacg 263940
aatcaatgta tcctttttct gctctaagtc caactccctt gctaaatcag caatctgatc 264000
ctgcatctgt ataatctcac taggaagctt cttatcgaac cctccctcaa caccaaatag 264060
ctctttaggc tctaatccat agcgctcctt aaatttaaga agcgccaatc ctatacttat 264120
taagagcagc cctaaaatca tgctgctcat ccctaaaagg tacgtagaga aaactcccaa 264180
gaataccatc cccaggcata cgaaaagagt ccctaaaata ataagacaaa cgatcacgac 264240

attaaagacg tggtgaatgc gaatactcgc cgtttctata ggcccaaaag ggaaaagact 264300
gtgtttacaa gaagtaagtt ctgaaaaact taatatctcc gaataaatag acacaaccac 264360
tccatacgac agctctcaga gagattttat aaaataattt ctcantttta atattttttt 264420
acaaaccgtt cttctaattc aataattctt gaaattcgcc tcttgaagta tgttgtaata 264480
ctatttgata ttaaggatga taaacgtacc ctcaactctc gaaaacagaa aacagaaaac 264540
agaaaggatc gttctctttc ttccacaaag gaaggataag ctctaatctg ggaattccta 264600
tctgattggt attagaaaaa attaccctag agaaaactac caaaatctat aaaccttgaa 264660
tgtgtagaac tgctaagaga ggcaactatg gtcatcatag tttagaaacg ctctcttctg 264720
tgctttagta ggagatctgt aaactaagtt tcttctgtca ctatatcttt tatcttatgg 264780
atcagttcta gttccaatga cnntattacg tcttgaagtg atcgtaactc tttttcaatc 264840
tctgtgagtt tttcaaaata cgcagatttt atagcacctc tgaactggag cttacgcacc 264900
agaagggcgt tcaacccagt agaagccgcc ctcattcgat tttcacattc ttcaagagtt 264960
aggccagaag ttactgttcc agaaataagg aactcataat gatgcagaac tatttcgcta 265020
tttaaataat tcaaaattaa aagctgttga tacggactaa aaagttcagg atgggaaaga 265080
ttttcaggat ctagcccctg atttttaagg gcttcttcat tttttttcat ttctaagaga 265140
gactcagttc tacccatgaa aagacgtgcc ggactttctt tcttggctct atccataagc 265200
ttttttgttg ccctatcgac aatatcaaaa gctttctcta cttctttaaa ttccctactc 265260
atttcattaa tgaacttctt acgttcctga gctttcacat caagcttatc taatctagag 265320
gttaatctta taaacagacc tttgatagcc tcaatttcat cataatctac cttgagtata 265380
cctgggggga gcagaaaaaa atttaataga acttggaggt ctttcctaag cctatacaat 265440
tctaaatata atgaagactt ttcctcatgc aaatcaaaaa tattgctttg gatcccttta 265500
acctgttctc caaaagtggt aaaatttctt ctggtttcta aaaatctatc tcgaacaata 265560
agaaatttag tcacttcgtc ttctaaagtt agccatttct ccatgcaatt gataaattta 265620
ccctgaagct cttggcttag atcagagaaa tctttcgtat ggaactgaaa cagattttgc 265680
aacttttcaa atgttacccg caactcagag atgtcattgg ggatctcttt cttatgaatc 265740
accaaagaat ctaaatcttg atcataataa tctaaatctt gacgagtctt gtgtttgtga 265800
cataagaaaa caataagagc tacagccaat aaaacaagac ctatgcaaag tatgggaagc 265860
gccgctccta aaagaactcc gcaagacgta tgaacaagga atgcaagaga accagataaa 265920
aagaaaatcc caaggataaa taaagcgaca accaccacag tagaaacgtg cgaaaaatac 265980
atcctttaga atctacacgt tgaggatgtg gagcaggagg tatgttacaa taggtgggac 266040
gctcatagtc ttcctttaat ccaagaaatc atcgtgagca tcttcaaaca cactatcatt 266100
accatctaat gattcgctct cactcaacaa atccgtcgta gatgcaacaa atgccacatt 266160
tgctctgact tgatccaatt taactctaag agtcttgcga atacgtgctt ttaaggcctc 266220
atactcctct aagactttct cttgttccga agagaaagtt tgagttaaaa actgctcaac 266280
aatacgacat tgaacttcat aaaggcgcaa taaatgatac tgaataagag ttggttcaca 266340
actcagatct tctactaccg atttcctgac ttgaatatca agaagttctg ctttagcttt 266400
atctagtgct tcttgcagct cttcttcaga aaagacggtt ctgtgtaaag atgctaattt 266460
ttcttcaaat gcactcacaa cttcttcgaa aagttgaatc tctttttcaa cggaaagcct 266520
tgtttttagg acttctaata gttccctcct ataacttcgg gaaacacctc cagtttctct 266580
actttccttt tgtctcgata acattcctgt tctatcttta cgtcttttaa agcagattta 266640
aacttagtca actcacaagc aagcttcaat ccagatttcc ctactttcat gacttctttt 266700
cgaagcttat ctatttcaca ttgaaggcga tctccttttt tccttaaaaa ttcaagaggc 266760
ccccagatat tctttaactc agtaagtaac gctacatccc ttccgtctag ttccttccaa 266820
atcgtctcgt atttttcgac ttcacgtcca aacagaagaa gatccccctc aaatgtagcg 266880
aactgactct tatgttgagc gacaacctct ttaaagtcct cccactcatc taatagaaaa 266940
ttgctccatt cctgaacttc attaagctct tttctaagat ctgcccacgg ttgatattct 267000
aaagtaattt tttgttctaa tgatctcgtg gcttctgaaa gtttccggtg ataagagaca 267060
aagaaaatca gccccaaaat caagagtaaa cttcctaaga aaacacctgc gcccccgagt 267120
cctacagtca agacagaaaa actaaaaata gcatttacaa gcaagagcat ccccacacat 267180
aacaggagaa cacccaagac aatcaaagat acagaaagga tcagagactt atatttaggt 267240
ggatgaactc ctaaagttga agagggttgg ggggacggag acgggaaaca atcacgggca 267300
atagcgctag acataacttg ctttttactt gaagttaact acttaatttc catacactta 267360
tatgaatgaa gtttcttttt gtcaacaaac cagtaataca aacattttaa taaaaattta 267420
taatatttgt attaaaacca aataaatcaa taaacaatag ccccgtttat taaacaaaga 267480
cactatcgct ataacgaaat ttctccgcta tcctcagctt ttcttcaata attcctagac 267540
gctcagaaat cgcactcatg tctctctcag attcaagatc ctctttatat aagggagttt 267600
catattgagg aacctctttt tcaagataga gagaaagtac atcaggaaca cgtttctctt 267660
gagataagag caaaatcagg ctaaaagaga aaagaaaaag agcaaaaatc aagcagccga 267720
gtcctagagt aaaagcagcc cccgcagaaa gtccaaccag acctatatcc aaacaaatga 267780
cagataaaat ggataagaca actccaagaa taagtaagcc ccccgccact atataggagt 267840
tgattctcaa agaagtgtct tcaggaataa aatcaacttg aatacgatca aatgtcacag 267900
gattcatggt gcgtgccttg ttctagattt ttcgcaccaa agatgttatc aattatttat 267960
tttaaatagc atgcaaaata atacaatttt gatttgatgc tatcccctaa gacatatcca 268020
cgaaactctt taaccatgca cgcacaagct tcatatagcg ttgtgcgatt tacacctcga 268080

gacatctatg actgggaagg atttccaagc atcatctaca aataaaatcc tacatatctt 268140
aaactaaaac gaaatgtatc tgatctatga tttcactaac caaaatgctg caaattgaaa 268200
ggatttttat ctgtgaatga attaattttt ggattccaga ctttctctgt tgtagtttta 268260
ggagttttct ttgcctctag aggaaaggct tggcttacag gatggctatc gctgctctca 268320
agcatcatga atgtctttgt tctaaaacaa atccatctct ggggttttga agttacgtct 268380
gctgatgtct atgtgattgg tttgcttact tgtctaaatt atgcccgaga gcactacgaa 268440
aaaaacgata tcaatgatgc tatgctatgc tcctgggtca tctccatagc gtttttggtt 268500
ctcacccagc tacacctatt tttaatcccc tcacctaacg actcttctca agagcatttc 268560
ttagctcttt tttcttctac tccaagaatc gtagtagcct ctctggtcac tttaattttc 268620
gttcagatcg tggatataaa actctttacc ttccttcaac gagttttttc aaagaaatat 268680
tttgcaatgc gctcaacaat ttccctgctc ttttctcaac tcattgatac cataatattt 268740
tcatttttag gattgtatgg attggtcagc aatctttgtg acgttatgat ctttgcaatg 268800
ctagtcaaag gcattgtaat tacactagct ataccgactc taacagtaac taaagccgtt 268860
ttagatcgtc gttcctctta agctaagaaa ttaacagctt cacctatacc ctctaacatc 268920
ctttcggtaa aggagaggtt ctgcacacaa gtaaaggttg aattacaata gagaagtagg 268980
cttagaatta ttgcgacaag gaaagcaagg aagcacccta taatatcaaa gacaagaatc 269040
agaaccccta aacctaaaat ctcaataaca gcttgaagca cctcgaatct gtacttcatt 269100
ttatctctag gatggatcgg ccctgaatgc gcttctatca atcgaatgat tccaccaatg 269160
gtagaaatga taggaaggct acgatatagc ctatggtgta ttggagtctt cttaaagtca 269220
tgaatacgat aaggagtctc tttaagttct gaatcaacaa aataccttcc ctggaccaaa 269280
gctcttaaag acttaggcca ggaggaagct acatttgcaa aatcttcata agattcgaaa 269340
aacacttttt tagacaaaaa aattaaaaaa caaaacaaat atacattatt attcattaaa 269400
ataaaagttt taaaaagatt aatttcttta aaacaaaata tccccaataa aatattgtac 269460
ctaccagcgc ctatccgtat taagacccag gaattcagaa agctcctttg gagtaaaaga 269520
ccctacttta aatctaagaa atcccaagct agggtcagat aaaacaaacc ctatttactt 269580
aaaaattttg tcattgtaaa cttcctcttc cctaaaagac acatccacct tgttctttat 269640
agttaaggat ctagtttggc actcaaattc catctcatcc atcaatctaa gaaatcccaa 269700
gctagggtcg gacaaataga gaccagccat ggagtgatcg atacacccgc atttgtcccc 269760
gtagcaactc acggagcttt aaaaggagtg attgatcaca gcgatattcc tctgctcttc 269820
tgtaatacct accaccttct tcttcatcca ggcccagaag cagtagctaa acttgggggg 269880
ctgcaccagt ttatgggacg tcaagcacca atcattacag attccggggg atttcaaatt 269940
tttagcctag cctatggttc tgtagctgaa gaaatcaaaa gttgtggcaa aaaaaaaggc 270000
atgtcctctc tagttaaaat tactgatgaa ggcgcatggt tcaaatccta tagagacggg 270060
agaaagctat tcctctctcc agaactctca gtacaagccc aaaaagatct cggagctgat 270120
attattatcc ctctagacga gcttctcccc ttccatacag accaagaata cttcttaact 270180
tcgtgttccc gtacgtatgt ctgggaaaaa cgttctttag aatatcatcg aaaggatcct 270240
agacaccaat ccatgtatgg ggtaatccac ggaggcctcg atccagaaca acgtcgtatt 270300
ggcgttcgtt ttgttgagga tgagccattc gatggctctg ctatcggagg cagcctagga 270360
agaaaccttc aagaaatgtc tgaagtggtt aaaatcacca cttcatttct atcaaaagaa 270420
cgtcccgtac acctattagg aatcggcgat cttccctcca tatacgctat ggtcggcttt 270480
ggcatagact ctttcgacag ttcttacccg actaaagctg cccgtcatgg tcttatctta 270540
tcaaaagcag gacccatcaa aatcggtcag caaaaatata gtcaggactc ttccactata 270600
gacccctcgt gctcttgttt gacctgcttg tcaggaatct ctagggcata cctgagacac 270660
ctttttaaag taagagaacc taacgctgct atctgggctt ctatacataa tctacatcac 270720
atgcaacaag tgatgaaaga gattcgtgaa gccatcttaa aagatgaaat ctaagtctcc 270780
tttctaaaga ttccacatct aaaaaactct aatttttcca tttattttca aaaaaatctg 270840
taaagatttc tttatttaga tcacaaaata ctctcttatt tataagaata aaaaaaatta 270900
atttttatta aaaaataatt aatttatcgg attttaaacc aactttttat aaaattgatt 270960
ctatagtttt ttaaaaaaaa agggaatttt ttatatgtcg aaggaaagca ttagaagtta 271020
ttctgaaatt tctactccaa cgccgatatt cagagaaacg ccctcgaaag aaggcgtggc 271080
atataaactt cagcttagat caccagctaa agactgcata ctcaggaata gagtatctct 271140
aaaaggagct ctattaagat ccattccatt ttacggatca ttcttaggtg ctaaaagaat 271200
ccatagtgcc tggtctgcaa aagatgcccc ctgcacaact agagtgtatc actacctagt 271260
cggtgggctt gagttattgg gactcggggt ttgttgttct agcgtgtaaa gtactcgcca 271320
ccgctctaaa gtttttattc tctaaagcct cctcgaagat aaaacaaatg aaatggcgag 271380
agaaagcgcg caacctagca gccaaagata cggtacaatc aataaaagag ttctgttccg 271440
ttgatcttac atcttgcttt acaagatgtt tcaggcttcg aaatagagtg gtagaggaag 271500
gtgcatctga aaaccaaaca gtaagagaga tcattgtata atctcatagt tttctatgta 271560
ttcttaaaca aggatatacg cctataaaat cgcaagaaaa ctttctcata cttcaataaa 271620
gaaacgaagt acctatacgt agcagctact aagaaaaaag gaatccccac ttctcttagt 271680
agctgctatt attataaaac acgtaattta ataacgtaga ccgcctaacc ccatagcatt 271740
gtcttccctc ccacaatagc gcacagaatc cgaaaattat aaaagtctta attatgaaga 271800
attttaaata aatgattgaa aaactcgtac tatttcaaag atcttagcat tttataaaac 271860
aagcgctatc ttcataatcc gctttatata aacattttat tttatctaaa attagattag 271920

gatgcactac gttacaagta tgaaataggc atccaaatgg taaacagata caagagctct 271980
gcagaatttt ccgctgatca ttactatgat gacaacctgg ttcggatggg gtataaaaga 272040
aacttaagag gactagctcc tgtggagaat gaagtctgtc tttttgagga gaataaccta 272100
ctcgaatctg tcatggcgtc tataccaatt atgggatcga tacttggctt aggcagactt 272160
catagtgttt ggtctacaca ggaccctaaa gatagtaaaa tctctataat tttccatact 272220
gcacttggaa ttctagaaac cctaggtcta ggaatcattg ttctccttat taaaataacg 272280
attactattc tccttattct atttactcca tgtcttctct gttatttcat gtattcctgc 272340
tgcttatagt gattttcatc ctatttagtt aggttctaac attctctatt taaaaaaagc 272400
tttgaatgtt cctttgacaa gtagacgagc aacctaaagt ttccttcgga gaatcacgag 272460
ttttttcttc aggttacatc tcagttttag aggaaactaa gacgtagaac gtttgtgttg 272520
cgaatccatc ttaatcatga atgattctca tatggcacaa gcagttctcc aagctctcta 272580
ccagtaagaa gttgggtata ggagctttgg ctaccgtagg ctccaatctc acacacgatc 272640
cttcggacaa cttgtaaaac tcggcatact agcattaggg tctgaaacat cctggcatag 272700
aaagcttctc tctcgcatgg aaacaagtcg tttcagaatt catgattcta gctgttttat 272760
cgagccaatt ctgtaaggtg aaaagttttt aaaaccttgg gagaagttgc gagaattaaa 272820
tgcttttgaa ttaactcaac ctgaagagta tcgaaaccgt tgggttttga tgccttgtct 272880
taagtgtcgt ttttgtagaa cgcaacatgc aaaagtctgg tcttatcgtt gtgtccatga 272940
agcttctttg tatgagaaaa attgttttct tactttgact tatgatgata agcatttacc 273000
tcagtatggt tcgttggtaa agctgcattt acagctgttt cttaagagat taagaaagat 273060
gatttctcct cataaaattc gttattttga atgtggtgcg tatggaacca aattacaaag 273120
acctcattat catctacttt tatcatgaca taaagatttt tttaaaaatc ttaaaaagaa 273180
atgacctaag gactgttaat tatatggaat ttttattacg agtttcatta aaatgttcgg 273240
ctaaaaagtt aaagtacgat gctaataggc agatacagta gtgatgacca attcactgaa 273300
gcaacaaaaa acaccccaac cataattaag ctaggttttg ttagagataa tctcgaggga 273360
ttaacgaacc ctatctctga aatcgtctcg gaaacctcct cttctattaa agattccgtt 273420
cttcgctctc ttcctatttt agggtccatt ttaggatgcg cccgacttta cagcacactc 273480
tctacaaatg atcctcttga cgaaactcaa gaaaagattt ggcacactat atttggagcc 273540
ttagaaacct taggcttagg gattctcatc ctcttattta aaattatttt tgttatatta 273600
cactgcatat ttcatctagt tattgggttc tgcaaataac aacaattaat ccacgcctac 273660
ggcgtaatta aattgtatct ttttagaaaa agacaagcgt ataatataca tttaaaaatc 273720
ctaaaaaaaa taagaaaatg aagccaaata gtattatttt tttagaaaat actaagcatt 273780
atcccgacat ctttcgagaa ggatttgttc gtgatcgtca tggactaatg gaagcctcgg 273840
attggttact ttctacggaa attacgatca ttcgctccat tctgggagct atccctattt 273900
taggaaatat tcttggagcc ggacgactct atagcgtttg gtatacaagt gacgaagatt 273960
ggaaaaaaca agtggtttga cacacgatat ttggaatcct agaagttctt ggccttggga 274020
ttcttgcttt agcattaaag attctcctaa ccaccattta ttacttgcta cgaggcctct 274080
ggaacgtttc ctttatgctt atagagatct tttccgcact ggtccctaat tatccagtac 274140
ttgtttaaaa ctctttcaca ataaaatttt tacttatgac taaaaatgct ataaattcac 274200
aaacaacaac cccacaaccc aatttaacag acgcagaacc tatcgctagc cgtgcgcaat 274260
gtaaatcaat agcggtaatc attagtttgt ttgctctggg aatgctccta ctctgtctgg 274320
ggataatcct tatttccata cctattcctg gacttgctgc acaagttgct ctcggcctcg 274380
gaatagtaag tttaatctta ggaattgctt tagccaacat aggtttccta tgtttattac 274440
ttagatgcaa gcagttcccc aaaaacccga tacattgccc tctgaaagct ctaaacagcc 274500
ttccgaggga agcactccca ccgcactccc atggcaagct ggagaatttt tagaaaaagt 274560
acaagtatct gcaaccccta tactccttcc caagaacaaa gatgaagagt tatcagcaaa 274620
agttatgaaa gaaggagccg aagcagcctt cttcaattaa acaagctgtt ctagaatcta 274680
cagagaaatt aatcgatgct agaaaacaag aggagagccg acgagaggct aggaaaaaaa 274740
tcgtggcgga ggaggctgaa gcatctagaa aacgtattca acagcaaatg gcagccgacc 274800
aagaagcgtt aagaaaacga aaagaagaag tagctaaaag aaagtaagct atttttaata 274860
gaaaaagaat gccatactat gcaaacaccc tggagttcat ccagggaact caaagtctat 274920
gtcctttaat tcaaatatgg gtttgtaaga caccattata aaggacaact agaaatcgaa 274980
gatgcttctc acgactggga tttcttagaa cccccttcta catggaaacg cactctcctt 275040
gctgcaattc ctattctagg atccgtcata ggtctaggaa gaccctttag caatctggtc 275100
cattagagaa ccccaggact ctcaagaata caagtctata ttctggcaca ctctatgtgc 275160
tgtcctagaa attttaggac tcgggattgt agctcttatt ctaaagatct tagcaacctt 275220
tattatggca atgccaggtt taaagagagt tgcaactttc ctattttatt cttaagagtt 275280
acaaattctt taggcctaga atcgtctacc ctatcctcta taattttttg taagaactaa 275340
gagacataca atggcaccga aagcaacaac agacgccata gggatcgtca cgtagctaaa 275400
aagaaagatc tttgttgagc aggagactct accgcagata tccaattgca tccctggaat 275460
ttcttgcaag aaaacttggt agatagaaat ccctaagcct aggactgcct gaggaaggat 275520
atacagtttg attgaagagt cctcgcgata agctgaaatt cctaaaatta cagtgagtgg 275580
gaacagacag attctctgat agtagcaaag aatacaaggc tctacgttaa gaatatagct 275640
ataaaaaatg ctaatcaaag tgccagcaca agaaatagcc caagcaaaat ataaagcata 275700
gctacggata aaattaatca tcatgatctc cttcaacagc ttgcagctga cgaatatgtt 275760

gaatcgctct ttctatttca tgaaacgtag gatcttcgat taagtagtct ccgactacag 275820
ccgttggtgt tgctaactgt cctcctaaaa cctgagaccc gtatagatta ttcttcttaa 275880
tctgctcgtt atactgtcct gaagcgatac actgttccaa gcctttagga ttaacactac 275940
gtccagaatt tatttttaaa ccctcagcca actttgtaag aaacccagga gtcacccagg 276000
ccgggagcat tcctctttag gataagtcaa aatacgatgg aaatattcca tataagcgtc 276060
tatatctgcc tgacgtggaa tcgtgatgat aaatacatag caatgcttga gctgcaggtt 276120
tagacccgcg aataaagcag acaggaatca aagtaaaaga aatctctcca gtatcaatat 276180
agtgcttctt taacaagggg aacacttcag tagtgaattc tgcacaagca gaacaagaag 276240
gctcctcaaa tactgttatg tttataggag cataaggatt ccctatggta ggaaagtgtt 276300
ttgcatttgt aggaatatga gctttagggg gtagaatcgt atgtttttta tgtattagaa 276360
agccaaagca aacgataaaa aacatcgcag tgcacagaac taggancttt ttattcaaag 276420
gactcgtaaa gagaatttgt taattgctta atagaacaaa aaacataaaa ttcaaaagcg 276480
ttttcttttt tganaattga acaggaaaac ttttccttta agtttaagaa atccgctcta 276540
tcttttacct ataacaccgc aaatctcact aaaagtactt tcacttttat tctactgctt 276600
ttattaagga aaaaagatca gggcctaaga tttatggata aagaaacact agaaaatatc 276660
tatcgacatt ttcgataccg ttttttaaaa ctcaatatcc tccctgcatt tcttggtctc 276720
cttcttctat gttctccaaa taccctaaat tatacacaag tcgatgtcat cttctctgat 276780
cgtctttgta gttgtttact tattttctta gctattgctt ccctaaccaa acgttctctt 276840
ctctggttag gagccccact aggcatctgg gttacccttt tcgcctgcgt tgcagacgat 276900
ctcctactat ttttgcaaat gatactctaa ttggattcgc aattcttgcc gtagtgtgta 276960
tttcccctac acgacccgaa gcccttgaag taggcccgac attacctgaa ggtttttctt 277020
acaatccttc tgcaggagga cgcagagctg cagtactatt cctaagctta ctggggtggc 277080
tagaagctcg gtatcttact gcttccagct tgggaattac atcgagtcag tcttcgaact 277140
tcttactatt gtactcatct ataatgactg tatactctct gctcgtggtt ctctctctag 277200
caggaagtga gcgccgctgg cacacaagac caaaaatcgt aatagcgaca gcttagcttt 277260
aacaggcgtc attattttaa ctcttctccc tatcatccta caccaactgc gctatgattg 277320
ctggctatgc tttgcctaac tatagaacct gctcttgccg tggtctttgc ttacgatgaa 277380
accagggcca ctttgcgcta tatttctcaa tttttaggag ataaacgagc tcttactaga 277440
gcctcgttct ttggatcaga atactataaa cacactctgt cttgggaaga aagaacagta 277500
cgtcctctac gaaaggcata taaacaggca tttgagggga tctccttccc aatcaaccag 277560
ttattggcta tcctagttgc tagtttttgt aaaagtcaat agcagtatgg gccttcctac 277620
ctttcctagn natttcctca atatatgttg ttggtttatt atcgtcctgt tcatcttagc 277680
ttttgcagaa agccttcgtc atttgcgttg gatgaatctg atcttctctg cagcgatttt 277740
attctctcca gtactctttc atattcccgt agaatctccc atgttcttgc cgatcatcgt 277800
tacaggactc attctaatta ttctatctat aggaaagaga cgaagaacta aacgcaaact 277860
ctaaaagaga agcttacgtg cctaacccct cccataaggg attggttttt gaggcgtttt 277920
ttgcttcttt cttagcttcg cgttcgtcac gcttttctct ttggttcttg ttcatgtaga 277980
tattctcctt agcacgctct aaagcttttt gtatcagctc atcatgtttt aaagattcag 278040
gagaatcctt ttttggaatg attttaagcg tcttacctaa gatcatagaa gagaatcttt 278100
tgctatactc aggtactagc acctgagatt ctatgtagag agaaagagct ttcttattag 278160
gagttttctt ttctttttca cagtggtcat agatttcttt gtaattgctc tgtgtaatcc 278220
tctcatggag agccaacact tgcttatggt gggaataagc atagatattc aaaggcaaaa 278280
caagaagaac aaaaacgaga atcgagggta atagccaggg aagaatcaca tcataaagaa 278340
tagatgcacc ttccagagga gcacccaaaa gtatagatcc taaaacaaaa ccaaaaatac 278400
aaaacatgag aagtgctgct gctaaaatta tagcaggagc cccatgccaa atatgattaa 278460
aggctttctt gtagcaagct actttctcct ccccagtaag tacaacataa cggcttaaag 278520
aagaggaagt ggtgaccaga ggctgtgaac tcatgtatat aagtgctttt ttaaatcttt 278580
aatgaaacat agcccattat ttaaaggtct cattgaagga tccagaggaa caggagtgag 278640
tgttttgttc ttaataacgt acaagacatc ccctaaacag gaaacgtcat gaaagtcgtg 278700
agtgacaaga agcaccgtct tattttcctt ctttgctaaa gcaacaatat cttggtagag 278760
ctgttcttta agcaatacgt ccaaagacga aaaaggttca tctaaaagga gaataggctt 278820
taaagacaag cactgagctg caagagcgat gcgctgcctt tgccctccag aaagttcgtc 278880
tggataacga tcaagaagct gtccgagatc aaaattgtgt ataatctctt caaggcgttc 278940
attggataag gcgttgtgac ttgtattgat gccaagctcc gttgacaacg tcatgttttt 279000
taaagccgta cgccaaggaa gcagggcttc tttttgctgc atataggcaa cgtctttgcg 279060
atttagaggg ctcccattcc atagaagttc gccttcttgc aaaggtagga aacccgcaag 279120
caaacgaaac aaagttgtct ttccaactcc agaacttcct aaaataatcg taattgtccc 279180
tggagacgct tggaaagaag catcctttaa aatgacttga ttgtcacaag aatagcatag 279240
acgatgagct tgtaacatgg aaaagcctct atcttgaata gaaacgaagg cttgcggtta 279300
ccaagacttg aacttgggac ctcgacatta tcagtgtcgc gctctaacca actgagctat 279360
aaccgcgatt tggagactag gagattcgaa ctcctgacct tctgaatgca aatcagacgc 279420
tctaccaact aagctaagtc cccggccatc ccaataaggg aaaagtaaag aatcatctta 279480
cctatcaaga gatttaagct caacaaagaa agtataggaa aatctcacct taatgagaag 279540
aacagagttc ttttaataca ctcttgagca aggtatccat ataaatttta cttgcctcac 279600

tttgaagatg cttccaatcc tcgttactcc gtgattctaa aggatgaggg agaaggatat 279660
tcacacctaa acaaggaatg ctatattcat agcatacctg agaaacagcg ccgctgacac 279720
tatcaaaacc atgaatctct ggatacaatt tttgtaagga aagaaaatag tttcgcgaca 279780
tcgcgaaaga ctcgcctgta gcaaccaaac cttccattaa ggtgtgctcc gttttggttg 279840
ttgatttcaa atacccatga gtcttcaaaa gctcttcgat ttcttgttta tgggtagaaa 279900
taaactcttc gcctccacga agaattgcct cccgatgaac ctcactggtt gcaaaaacac 279960
tctttttaat gtctggaatc tcaaatcttt caaagaaagg cctcacatct gcatcataat 280020
taatgtagcc tttagaaact aagacgctgc caaaacggct atcttgagac ctagagtaac 280080
acgagcctat aattagaata agatccactc gatgtttaag aatcatatta caagccacaa 280140
cagctgaaga aactttatta ggccaaagag cagaaactac aaagtatttc ccaaaggagt 280200
cgccagagta ataaattctc tgtccctcta gagtcttttt actatgagaa aaccaaggaa 280260
tagaacaatt accatcaaaa gaaacgggag taaccccagg taaagcaaaa ataatactta 280320
cacgacttaa aggactctgt ttttcttcta gaatagtgaa attatcagca gagaatgcga 280380
ccaaaggaag agagctaaga ataagaaaca gaaaacgacg cataagaaat ttcttctaag 280440
ttaaagatac tgttttcgca ttgtataaga aaaagatctg ttaaatagaa ttaaaaaacg 280500
aaagtacgct tctttttaag ctctagatac tcccttaaac tcttcttaac tctgtccttt 280560
gttctttaat gacacatcca tttctactac tataaaagta ctgacaaaag cacagagtac 280620
gattttcaaa tcgggagtct caagatgaaa gaagatccca atctcttgaa cgtaagacct 280680
tcgagaaatt cttagcaaaa agagaagaac tttgcccaaa aacatagtcc ttgctttttc 280740
tcaagaatta gagctttcag atgcctagta tattgagaat ataaataaaa aaaccattgc 280800
caacccggca atggttctta aaacacaaat ccaaaatata gaatcttaca gactcacttt 280860
ctttttcact gaagaaggaa tcgttcttgg ttctcgtatt ttagtaattt taaaagactg 280920
agaaaacgcc tcgtattctt tatccaaggc ctgaggattc ttattcttat aaaccataaa 280980
gacttgataa agagtgtgat ttacggaaat caacatccct ctgaaataaa catcttcgca 281040
aacaatccaa aattccaaag ccttatggcc ttgaatctgc cttgcttgca tgaaaagaac 281100
ctgggattca gggagagcct gcatcatgcc tgaaaacccc tcttgcagat tgagctctgg 281160
acgacttata tctacttttt caggatactc ccaaacagag actacataca cagtgttgtc 281220
tggatgagtc tctgttacat aggtatcata acgtatggta atctctgatt gagggacttc 281280
tacaatttgc cccgaatgat caggctcccc aggaaattcc acagaaaacc cagaacttga 281340
tgtatagtca tagcgtttcc atgaaagact gtctttaact ggtaaaattc tagcctcttc 281400
ctgaatctct ttttttgaga accatccttt gactttccct aaaaaaccag atttagcttc 281460
gactcccata ccagggatag ggtgaaaagc taaaatcgat actactatgg ataataaggc 281520
ctttttgcaa ctctgcaaca taataaacta aaaaacaaaa caagataact aacttaataa 281580
tatcccttcg agatttttat tttgtaaaat aaaatacttt tttttcagaa aaataaaaaa 281640
aatattgcgt tttataaaat gcatcacaat aatcctggta gtcttaaaca cataagtttt 281700
tgttaggtat ctccttatga agccggaaga gtctgagtgt ctgtgtattg gagttttgcc 281760
cgcacgctgg aatagcagtc gctatccagg aaagcctttg gctaaaattc atggaaaaag 281820
cttaatacaa agaacttatg agaatgcttc ccaaagttct ctattagata aaattgttgt 281880
tgctactgac gatcagcata ttatcgacca cgtgactgat tttggtggtt atgcagtgat 281940
gacttctcct acatgttcca atggtacaga acgcacaggt gaagtagcta gaaagtactt 282000
ccctaaagct gagattattg taaatattca aggtgatgag ccttgtctaa attctgaggt 282060
tgtcgacgct ttggttcaga agttgagaag ttctcctgaa gcagaactgg tgactcctgt 282120
ggcactcacg acagatcgtg aagagatctt aacagaaaaa aaagtaaaat gtgtttttga 282180
ctctgaggga agggctctgt attttagtcg cagtcctatt ccttttattc ttaaaaaagc 282240
aaccccagta tatctccata ttggagtata tgcttttaaa agagaggctc ttttccgcta 282300
ccttacagca tanctcannt cctcgtaagc gatgccgaag atcttgagca attacgtttc 282360
ctagaacatg gaggcaagat ccatgtgtgt atcgtagatg caaaaagtcc ctctgttgat 282420
tatccagaag acatagctaa agtagaacaa tatatcacat gcctttcaaa tgcatatttt 282480
taacaggagg agttgtctcc tctttaggaa aagggttaac agcagcatcc ctagccctaa 282540
ttttagaacg tcaacggctt aacgttgcta tgttaaaatt ggatccatat ctaaatgtag 282600
atccaggaac tatgaatccc tttgagcatg gagaaatcta tgttacagat gatggggttg 282660
agacagatct tgatctcggt cactatcata gattctcttc tgctgcactt tctagacatt 282720
caagtgccac ttcaggtcaa atttatgctc gtgtcattaa aagagagcgt gagggtgatt 282780
atctaggaag cacggtacaa gtcatcccac acattaccaa tgaaatcatt caagtcattt 282840
tagacgcagc taaagagcac tctccagatg ttcttat[?]gt cgagattgga gggaccatag 282900
gagatattga atctcttccc ttcctagaag caattcgaca atttcggtat gaccattccg 282960
aagattgtct aaatattcat atgacttatg tcccctattt acaggctgct gacgaagtta 283020
aaagtaagcc aacgcaacac tccgtacaaa ctctacgtgg tattggcatc attcccgacg 283080
cgattctatg tcgttctgaa aaacctttaa ctcaagaagt taaatctaaa atcagtctct 283140
tttgcaatgt tcccaaccgg gcagtgttta acgttataga tgtaaaacat accatttatg 283200
aaatgccttt gatgcttgct caagagaaaa ttgccaattt cataggggaa aagttaaagt 283260
tagctacggt tccagaaaat cttgatgact ggagggtact ggtaaatcag ctatctcaag 283320
atcttccgaa ggtaaaaatt ggagtcgttg ggaagtatgt tcaacaccga gatgcctata 283380
agtccatatt cgaagcactc actcatgcag ctttaagatt aggtcatgct gctgaaatta 283440

tccctattga tgctgaagat gaaaatctta ctatggaact ctctcaatgc gacgcatgtt 283500
tagttcctgg aggcttcggc gttcgtggtt gggaaggaaa aatcgctgca gctaaattct 283560
gtcgagaaca aggcattcct tattttggta tttgcctagg aatgcaagtg cttgttgtag 283620
agtatgctcg caatgtctta aatctggatc aggcaaattc cctagaaatg gaccccaaca 283680
cccctcatcc tattgtatat gtcatggagg ggcaagatcc cttagtagct acgggaggca 283740
ccatgcgctt aggagcgtat ccttgtctat taaagccagg gagcaaagcc cataaagcat 283800
ataacgaatc ttctctgatt caggagcgcc accgccatcg ctatgaagta aatccggatt 283860
acatacagag tttagaagac cacggcttac ggatcgttgg gacttgtcct ccacaagggc 283920
tttgtgaaat tattgaagtt tcggatcatc cttggatgat tggtgtgcaa ttccatccag 283980
aatttgtatc taaactcatc tctccccatc ctctatttat cgcatttata gaagcagctc 284040
tagtctattc taaggatgca agccatgtct aagccatcta gttgcaaagc ataccttggc 284100
atagactacg ggaaaaaacg gatcggcctt gcctatgcag ccgaacccct cctattgaca 284160
ctaccgattg gaaatataga agcaggtaaa aatcttaagt tgtcagcaga agctcttcat 284220
aagattattt taagtagaaa tataacttgt gtagttctag ggaatcccct tcctatgcaa 284280
aaaggtcttt actcatctct gcaagaggaa gtttccttac ttgctgagga gcttaagaag 284340
ctttctacgg tagaaatcat cctatgggat gaacggcttt cttcagtaca agcggaacgt 284400
atgttaaagc aagattgtgg actaagcaga aaagatcgga aaggaaaaac agattccctg 284460
gctgcaacat taatcttaac aagttttcta gatagcttac ctaaaaaact aaccttgtaa 284520
tcatcaaaaa ctacgggatt ttaattttag aaacttttta ctttttgtta ttttcgcaag 284580
tgcggggaca aaaaaggaga taaaaatgac gaatgttgtt caggaaacta taggtggatt 284640
gaattcccca cgaacgtgcc ctccttgtat tttagttatc tttggagcga ctggagatct 284700
gacggcaagg aaacttttac ccgctctata tcacctcact aaagaaggac gcctttcaga 284760
ccagtttgtt tgcgtaggat ttgcacgtcg agagaaatcg aatgaactgt tccgccaaga 284820
gatgaaacaa gctgtcatac aattttctcc ttccgaatta gatattaagg tatgggaaga 284880
tttccaacag cgcctctttt atcatcgctc agaattcgat aacaatatgg gatatacatc 284940
tctcaaggac tccttagaag atttagataa aacgtacgga acacgtggaa atcgtctttt 285000
ttatctttct actccccccc aatatttttc tagaatcatt gaaaatttaa ataaacataa 285060
gcttttctat aaaaatcaag accaagggaa accctggtcc cgtgtcatta tagaaaaacc 285120
ttttggaaga gacttagata gtgctaagca acttcagcaa tgtatcaatg agaatcttaa 285180
tgaaaattcg gtctatcata tagatcacta tttagggaag gaaacggttc aaaacatctt 285240
aacaacacgt ttcgccaata cgattttcga atcgtgttgg aattcacaat atatcgatca 285300
tgtccaaatc agtttgagtg aaacgattgg cataggatct cgcggcaatt tctttgagaa 285360
atctgggatg cttcgggata tggtacagaa ccatatgatg cagctactct gtttactcac 285420
tatggagcct cctacaactt ttgatgctga tgaaatcaga aaaganaaaa tcaaaattct 285480
tcaacgtatc tcaccatttt cagaaggttc ttcgattgtc cgaggacaat atggtccagg 285540
aacggttcaa ggagtctcgg tccttggcta tcgtgaagaa gagaatgttg acaaagattc 285600
ccgagtagag acctacgtag ctttaaaaca gtcattaata atccccgttg gcttggagtt 285660
cctttctatt tacgtgcagg aaaacgactc gccaaaaaat ctacagacat ttctattatt 285720
tttaaaaaat caccctacaa tttatttgca gccgaagaat gttcacgttg tccgatagaa 285780
aatgatttgc taatcatcag aattcaaccg gacgaaggtg tcgctttgaa attcaactgt 285840
aaggttccag gaactaataa tattgtccgt cctgttaaga tggacttccg ttacgacagc 285900
tatttccaaa ctacaactcc agaagcatac gagcgtttat tatgtgattg cattataggg 285960
gatcgtacgt ttatttacgg ggggggatag aagttatggc ttcttggaag ctttttactc 286020
ctgtattaga ggagtgggac caagattcct caccctcgtt tccaaactat cctgcaggat 286080
cttcaggtcc taaagaagct gatgctctca ttgaaagaga cggaagaagc tggagacctt 286140
tatagacaat cttatacagc atctagaaat cgataagcat gacaaacata gggattgaga 286200
ctatggcaac actgataaat ttcaatgata cgaacaaact tttgcttaca aagcaacctt 286260
ctctatttat agatctagct agtaaagatt ggatagcttc tgcgaaccag gcaattaagc 286320
aacggggagc attttatgta gcattatctg gaggcaaaac tcctttagaa atctataaag 286380
atatcgttat caataaagac aaacttatag atcctagtaa gatttttcta ttttggggag 286440
atgaaagact agctccgata acatcgtcag aaagtaatta cggccaggct atgagcattc 286500
tccgtgattt gaatattcct gatgagcaga tctttcgaat ggaaacagaa aatcccgatg 286560
gagcgaaaaa ataccaagaa cttatagaaa ataaaattcc tgatgctagc tttgatatga 286620
ttatgttagg actaggagaa gatggtcaca ccctttctct tttttccaat acctcggctt 286680
tggaggagga aaatgacctt gtggtcttta attctgttcc acatctagaa acagaaagaa 286740
tgaccttaac ctttccttgc gtacataaag gcaagcatgt tgttgtttat gttcaggggg 286800
aaaataaaaa gcctatcctt aaaagtgtct tcttttctga aggtagagaa gaaaaactct 286860
atcctataga gcgtgtaggt agggaccgct cacctctatt ttggattatt tctccagaat 286920
cttatgatat agcagacttc gataatatct cttcgatata taaaatggac atcctctaaa 286980
aaagataggc gttgcgattt agccgtagat agtatcgtaa gccggcggtg gaaaaggagg 287040
aggagcacta ggcatggtag gagccgtagg gtatcctgct ccaaaatggg atcctgcaaa 287100
gccccctccc ccaccaaagt gacttcctcc aaaattaccg tttcctcctc ttctattctg 287160
ataatcagag gagcctccat aatgctgaga tggtcgagaa cctccaccag gagctcctcc 287220
ttgaactaac tgagcaactt cttcgttaga gaagaggtgt ccataatgag acagacagtc 287280

tgtgagaaat aggtctcttg ccaatgcatt tgtttggaaa tgagagggat catgtagcag 287340
agcctcacta acggcagtat gcaaaacaac attcgttagg tgacccacgc atgcctcttg 287400
gtgctttgcc ttacgaacta tagcaatcca tagtcctagc agagaaatta acagagcgcc 287460
tccacctgca gcgcagaccc catcattgcc gcagtaatta cgggagcagc tccaggagct 287520
acaaaaaata atactaagac aatgccagct gttaaggcta ttaaagctaa agaagctatt 287580
aagctaacga ctaaggaccc tatcatagca ctcttataca acttacacgc ttttacagcc 287640
tcttctgatg accttatggt tgcaacacct gcagctcgat ggaagacatt ttgagcagga 287700
tgacgtaata cttttgcctt cgccttattc agagagtaaa tcatcttccc aagcgtataa 287760
actttataag cacaataaac cagggcacct aatgtcggtc ctaggataag agcaattacg 287820
aagaaaagta gtgtagctcc taaaacgatc gctgatgtct ctttttctgg agtttgacag 287880
ctcttacatt gcgaaaagca cataatcaat attccgaagc tattgaacaa attccccaaa 287940
ttctgcgcan tttaaaccct gtcatcatca tgtcggttgg tctatcaaat agagattcaa 288000
tatcaggaac tcgtcccttc atgatctcac taaacattac ttctgtactc tcggaaggag 288060
atacaaattt gttttctaga aaataacaga agttactgcg caccactgaa ttagctttaa 288120
caacagattc tgaggaaggg gaaaacacca taaaataaac caattactaa caatacatta 288180
cttactaacg ataagtattt agctaagggc tctcagcctt aagtaagttc tttgtttatt 288240
aacagaagta ttttatctta atgatttaaa aacttcaact ctcgagactg attttagaat 288300
cgtatttaaa ataatttaag aattttaata accgattgtt tgaacgaaaa acttaaaaac 288360
ttaaaaattt aaatttataa tttagataaa aattaaaaac tacagcttgc ctattgctaa 288420
aataaataat tcataaacac aatgcgaaac ttcgttataa aaagcacatg caatctagtc 288480
ttaaaacggt gattataaaa attagcgata ataaggagga ggagaagaag agggaaaagg 288540
agaattcgac gagtctatcg tagaataggc cggtggagat tctctaggaa tcacgttgct 288600
gctaccatcg ctcccctcct catgaaacag agtctcgtaa cttggaggtg gcgtcgtctg 288660
acggttcaag gggacttcgc tctcgatatc tctatactcg tcatcactga agaactgttg 288720
gtatcttcgt atagactgag taagcacttt tttagttcct ggagtgatag gaaggtaggg 288780
catctgaata attgtgttac tcactataca acgcaatagt gcggtatgca tatgatgaac 288840
accttcttgg cttttctgca cgctatacac agaagcaagt aaaaagccta ttacagagag 288900
gaggattcct gttcctcctg cagcacagca tcctatcatt gctgcggtca ttacagcagg 288960
agctccagga tctaaaaaaa atagagcaaa aacaaggcct acaattaaag ctgctaaaga 289020
tatcgtaata attaagccaa gcacaaagaa aattaatgtg gattgtctaa atactttaca 289080
agccttgatt gtagattgtg aggcggcagc ggcggctaca gcccctgaac gatgtataaa 289140
aggatctgat ttctgtacgg aatttatgac ctcagtgtgg gttcttgata aagaggatat 289200
tttcttggta agttgataaa ttttatatgc agagtagcac aaaatgccta gtgtaggccc 289260
gaacacaata gctaagactg taaagaggac agctgcgaaa ataccggtta tgagtgctcc 289320
acagggatca gtgtgatgag aatctgtcac taacaacagg ggacaacacc ctattccaaa 289380
tattttttca aaaaatctct gataaatcat tttcaagctg atattagaat acacttgaaa 289440
gtgatgttcg ccttctgtct ctactacatc tttcaaatca tctataatag cagttgacga 289500
aagtgttcct cgggctagga atactttaat cggaagaatc tctgtgttat gttcaacaaa 289560
ttcgtccata gctaaagata ctaggacgtc gcggacctgt gtattagaag caatgcttgc 289620
ctcatgtgaa gaagaaagag aaatcatggc agacataaat tttaccagaa aaatttatca 289680
cgcctctttg tttaaaaata gtcatttttt atttaatgca aggaagaaga gtttccttgg 289740
tctatttaaa aagacttctc tttctaacga agcgagctca tttccttaag gtttgattaa 289800
tccagacatt cctgccaatt gaagaatcgg ggcttgcata aagactattg cggatgagag 289860
tctagcgtcc tcactagtcc tatgtgaaat tataaatccg tgaattgcgg ttcctatgac 289920
tgggacgatt aacaagcacg caagtagata aggaaggcca ccaccataga ggcatttaaa 289980
atgtgatccc agacttactc tttccccagg taaagcactc gatgcactac ataacttaaa 290040
cggaaatgag agaagtagaa atagaacctt taaaatcaac aaaactacac ttacaactgc 290100
acaaactaca gcaattacag gatacaacat agtatctaca actaaacaag ccgtttttct 290160
tactcaaggg ttcatcatca taatctcctt gagacattct aaaaaaaata gaacaagcca 290220
taagaataac cgttggtttc tgagaaaatt gatataaaga acaatatttt aaagattttt 290280
agttttaatt ggaataaaaa ttctaaccct tccccctcta attacgatgt agttgtttaa 290340
aatattttta attattagaa actacttgtt attggtccat gaatataaaa atagaggtct 290400
ttctagctta gaaaaacccc tatctatagt tattaccaat ttataatagc agttgatccc 290460
acttgataag caggagatat aataaagtat cttaaacgat tagtatccga ttgtactgta 290520
ataatcgtgc tataaattag ggtccctatg atcggaataa gtactaggca tcctaaccaa 290580
tctgatggac ctggtttatc ctttggtcca aacaagcatt gaaaattctc tttacacgag 290640
ggcagaggcc ttgatttaca agctgcaata caggtattaa caaggaattt tatagctaaa 290700
aacagtagtt ttactaccat caagacggca aaaacaactg cacaaatgac cgccataaaa 290760
gggtagagaa gaatatctgc aactagagct actttcgacg caacgaatct gtcgggctcg 290820
tttgcattac acacatgata accaatagaa caagccatta ttatcctccc tttaacaaat 290880
tttaataaaa aaactaaata attatagttt atttattaaa aaaaataaag ttatattctt 290940
tactatttcc ccgttcaagc caaagagacc tcttttcttt taattttctt gtttttctat 291000
atttacagta ttttgaaaag atatatagta gtcctaggga aaatcttggg actcataacg 291060
atccaattct atcagaatct aggtggaatg tcttcggagc gctatagtgc tttgcattct 291120

aggaaaagtc tctctgtttt accccatgtc gtacgaaaag tattgttaag tttccctgat 291180
tttaggggta atggtgacgt caatttaagg aacattcgaa gtgactaagg gctctgtttt 291240
tattattatg gggcctccag gctcaggcaa aggaacccaa tctcaatatc ttgccaatag 291300
aataggctta ccccacatca gtactgggga tttattaaga gcgattattc cgagaaggaa 291360
ctcctaatgg attgaaggct aaagcctacc tagataaggg tgcttttgtt cctagtgatt 291420
ttgtatggga aatactgaaa gaaaaactgc aaagccaagc ctgctctaaa ggatgcatta 291480
tcgatgggtt cccgagaacc ttagatcagg cgcatcttct ggatagtttt cttatggacg 291540
tccattctaa ctacacggtg attttcctag agatttctga agacgagatc ttaaaaagag 291600
tgtgttcaag atttctttgc ccctcctgtt cgcgtatcta caacacaagt cagggacata 291660
ccgaatgtcc agactgtcat gtgcctttga tacggcgttc tgacgatacc ccggaaatca 291720
ttaaagaaag attaacaaaa tatcaagaac gcacagctcc tgttattgcc tattatgaca 291780
gcttagggaa gctatgtagg gtttcttctg aaaacaaaga ggatcttgtt tttgaagaca 291840
ttttgaaatg catttataaa tagtttttct tccttccaaa gaaaagtacc gaattcaccc 291900
gaaaaaaatt cactcagaac cttgtcttaa acttgtcaga aggaaattat gaaacactac 291960
ctatcatttt ctccttctgc tgattttttc tctaaacaag gtgctattga aactcaagtc 292020
ctttttggag agcgcgtctt agtcaaaggg agcacctgct atgcatattc ccaattattc 292080
cacaatgagc tgttatggaa gccctatcca ggtcatagct ttcgttctac cctagtcccc 292140
tgcactcctg aatttcatat ccatccaaat gtttctgtgg tttctgtgga tgcattttta 292200
gatccttggg ggatccctct tccttttgga actttactcc atgtgaattc tcaaaatacc 292260
gttattttcc ctaaggatat tctcaatcat atgaacacca tctggggctc cggcacacct 292320
caatgcgatc ctagacatct acgtcgtcta aattataact tctttgctga acttttaatt 292380
aaagacgcag accttttact gaactttccc tatgtatggg gaggacggtc tgtacacgaa 292440
agtctggaaa agccgggtgt tgattgttcg ggatttatca atatccttta ccaggcacag 292500
ggatacaacg tccctagaaa cgctgcagat caatatgcgg attgtcattg gatctctagc 292560
tttgagaacc ttccttctgg tgggttaata tttctttacc ctaaagaaga aaagcgtatt 292620
tctcatgtta tgttgaaaca ggatagttcc accctcattc atgcttctgg tggagggaaa 292680
aaagtggagt atttcatttt agaacaagat gggaagtttt tagattcgac ttatctattt 292740
tttagaaata atcagagggg acgggcattt tttgggatcc ctagaaaaag aaaagccttt 292800
ctgtaataag aaaggctttt tccaaaaacg attcgaaaaa cggtaaatat cttaacgctt 292860
agagaattgg aagcttttac gagctttttt atgtccgtat tttttacgtt ctttccttct 292920
aggatctcta gtaagaaccc gcaactcttt aggtcttgtc tattctcttc attttctttt 292980
aagagagctc gtgcaaggcc taatcttgta gcaattacct gcccttgaat ccctccaccg 293040
ctcacacgaa taattaaatc gtattgactt tggtcttctg taattttttt caaaggagaa 293100
agaattgtag ttctttgaat ttccaaagga aaataatctt caaaagactt accgtttaca 293160
tcaattttac cacttccagg tcgtaaacgg acgctagaga cagcctgttt tcttctacct 293220
gtagctacag attcttgtat tgtacttttt gccacaactc atcctaaatt aaatatctaa 293280
taaaattggc ttttgagatt caaaagtttc gtatgaatcc ccttttacaa tccttaagga 293340
cttcaattgt ttctttccta agcgagttct gggcatcatt cccttgatcg catgctcaat 293400
aatgtaatta ggttttctcg ccatcatatt ttcaaaagga atttctcgca ttccagagat 293460
atatcctgtg tagtagcgat agattttttg gcctttctta gctccagtta ggcgaacctt 293520
ctctgcatta ataacaataa caccgtctcc catagccaca tgaggagtat aggtgacttt 293580
atgcttgcct cttaaaattt ttgccacttc tgaagaaagc cttcctaagg ttttcccagc 293640
agcatcaaca acataccatg acttcgtagt ttcactggac tttactatag ttgtttttgt 293700
gtcttttctt ttttccataa taatgtaact gtcttactag agagggcgat tataagtctg 293760
ttcaaatttt tttccaaaca aaaacagccg aaaataacaa gtctcttagt ttaaaaatat 293820
ttccataaat ttcttttcgt acaattagca gttttttctt aacggctgct gatataaaag 293880
cattcagaca ccgtctaaag ctagatctca aaaatatgga tttatcttga aatcagaggg 293940
agtgttttct taagagttat gaaaaaatag cttgccattg gataagactc cttccctaga 294000
acacctagag gcgaggtgct tattatgtgt cacaataaga attccacata atgcggatgc 294060
ttgttctaac agaaggttat gaatctgttc tgaagtctct tcatcgagat tccccgaagg 294120
ctcgtctgcc aaaaggatgg ccggttcgtt gattaacgct ctagcaatag cgactcgctg 294180
tttttcccct cctgataatt tagagcagcg agtccgtact ttgtcttcaa gattcactaa 294240
atccaagagc tctagagccc tggtatatac aggagatcct ttagatatgt tttttcgagc 294300
aattagagct ggcattaaga cattttttaa tactgtgtcg tcttctagca aataaaaatt 294360
ttggaagaca aagccgatat gctggtttct aaaattcgca agatcctggt tttttagatc 294420
cttatcaaaa aagcgtaagc ttccagaaga aggaacatcc aaagttccta agagatgcaa 294480
tagcgtggtt ttaccattac ctgaggctcc tgtaatcgat atagtttctc ctgcatgcag 294540
tgataacgat acatcggtca aaatagaaat attttggttc tgttgctgga tagttttaga 294600
aaggttttta gcttctataa gtaaggacat agctaatctg cttttaaaat ttctgagaca 294660
tgcatttttg cgacttttct tgcaggcagg gctcctgaaa ctgcggctaa aagtagcgta 294720
cctaacccta gaaaataaat agcctggggg tgaacgctat tgggaagatt ctggccaaag 294780
aaagcagtat taaatgtttc tcttccctgt aaatagttca gtgcttttac aatgaattgt 294840
aaatttttta atgtaattat agcgaatatc gttcctataa ccactccaca agctcctgaa 294900
aatgctccac aacaagcaaa gatgatcttt aaacttcgtg atgatgtccc catagcttta 294960

agaatgccta tttccttttt cttattattt acaaggagca tcgacatagt cacgatgttg 295020
gagcaagcaa caataagaat aagtatgcac acaaaaagaa agagaacttg atcactttga 295080
agttgatcta ggataggctg gaaataatcg taatcgtgta gggaagaaat ctcccaatag 295140
tcatcgacac ctaaggaagt tagaatattt tctatttgtt tttttacaaa gacaatgcgt 295200
ttggtattgg ggaaaaatag atggaagccg ttactcatcc ccaagccctc ggattgagaa 295260
cgaatggatc tagcaagatc tgggtctata aatacagttc ttccccctaa cggagagagc 295320
ccgggattat aaaatccgat gacatgaact gtatattgag tttctttttc attctctatg 295380
gagtaggtac taaaaactcc tgtatcccct actttataac cagagtcttt ataagtacta 295440
gggagtatga tcgaagctcc tcggtagagt tcttctaaat gatggaaatc ttgttgccat 295500
cccgaggggc ttctattaaa tggattcaat tccgcagagg tatagtctgt ttcatcgtag 295560
ggtaaaacct tatcttcata ggaaagctta gagggatacg ttaaaaaatg cgtgagattt 295620
ctaggctgag gtttttgtaa ttttagagag gttttgatat cgaggtaacc cactccctgc 295680
tcaaattcga tcactttccc gtgttgggat tgaaggtagg gtcctaaaga ttctagagtc 295740
atctttacag gatctttttg ctgccctcct agatcacagt cttttaatgg aaatgtttcc 295800
ggaagaaggt agtctgattc aggatcataa gggtcgactt gtggagaagc tattttttct 295860
cctaaagttt tcgtagtgta gttagaaaga ctggagtgtt tgtctatttg atagtagtac 295920
gaagaataat atgtgtcgga gggaagaata gtaattggag aatggagttg ggaaagatcc 295980
tctatccatc tttgttctaa accgtgaatg actgaaataa aaactataga aagccagaca 296040
acaagagaaa tgatacccac agaaaatagg gagactatag ctgaatatag ccttcccctt 296100
cctggaatca aatactttaa agctactgaa aattcgaact tcatgacttc taactaagaa 296160
tacgatttta gaagcgagta gttaagtacg atgaaatcct tttacctagc ttctttgaaa 296220
attacgtgcc tacgcagttt tctatcatat tttttgagtt ctagtcgacc tgttgttttt 296280
cttttgtttt ttacagtcca gtacatatca gaactttccg agctttttaa tttaataatc 296340
tcccgattct tgcttgccat ggatgagtcc ttttaagaaa atagtaaatc gcttactatg 296400
ctatatttct tttcaaaaag aatcaagaac tctcccaatc attattagag aacctaggat 296460
gacaacaaaa agtttaggat ctttcaattc agttatttcc aaaaataaaa ttcattttat 296520
tagtttggga tgctctcgga accttgtaga tagcgaagtc atgctaggca ttcttcttaa 296580
ggcaggttac gagtctacta atgaaattga agatgctgac tatttaattt taaatacctg 296640
tgcgttttta aaaagtgcta gagatgaagc taaagattat ctagaccatc taattgatgt 296700
aaaaaaagag aacgctaaaa ttattgtaac tggatgcatg acttccaacc acaaagatga 296760
gcttaaaccc tggatgtcac acatccatta cctactaggt tctggggatg ttgagaatat 296820
tctttctgct attgagtctc gtgaatctgg agaaaaaatc tctgcaaaga gttacattga 296880
gatgggagaa gttccaagac agctttccac accaaaacac tatgcctatt taaaagttgc 296940
tgagggctgt agaaaacgtt gtgctttttg tattattcct tccattaaag gaaagctccg 297000
cagcaaacct ctggatcaaa ttcttaaaga attccgcatc cttgtaaaca agagtgtgaa 297060
agagattata ttgatagctc aagacctagg agattatgga aaggatctct ctacagaccg 297120
cagttcgcag ctagaatcac tattacatga gttactgaaa gagcctggtg attattggct 297180
gcggatgttg tatttatatc ctgatgaagt gagtgatggc attatagatc ttatgcaatc 297240
taatcccaaa cttcttccct atgtagatat tcccttacag cacattaacg accgtatttt 297300
aaagcaaatg cgaagaacga cttctaggga gcaaatccta ggattcctag aaaaattacg 297360
tgccaaggtt cctcaggtct atatccgttc ttctgttatt gtgggtttcc ctggtgaaac 297420
tcaggaagaa ttccaggagt tagctgattt tattggtgag ggttggattg ataatctcgg 297480
aattttcttg tactctcaag aagcgaatac cccggcagca gaactccctg accagatacc 297540
agaaaaagtt aaagaatcga ggttgaaaat tctatctcaa attcagaaac gcaatgtgga 297600
taaacataat cagaagctca ttggggaaaa aatagaagca gttattgata actatcatcc 297660
tgaaacgaat cttttactca ctgcaaggtt ctatggacaa gctcctgaag tggacccttg 297720
tattattgta aatgaggcga agcttgtttc tcattttgga gaaagatgct ttatagaaat 297780
cacagggact gctggttacg accttgtagg gcgtgttgta aaaaaatctc agaaccaagc 297840
tttgctaaaa actagcaaag cttagggtcc tgtggttagt taacaaaagg caaagtcgct 297900
atattccaag aaaaactttg cactctaaag ctataacttc gattttccaa aacaaagtat 297960
ccaggtctca cagcataata tgttgagcaa agccactgca ttggtcgtgc acggtaataa 298020
cttgttccag aacacgtcga catagctaat tcaattctta tgtcaggaaa taacgggttt 298080
tgatttgctt catatgctac caaaagaagc tcccctcctg taccattatc aatccaatct 298140
ccatgcttgg tcataaaaat tgaaatacga aagagcctag gaatagaagc acttgcttga 298200
aatcgcatca tccaagtctg acttgagtcg atataaatag cggaattgat cactgtactt 298260
tgtgttgacg tttgattgaa cttattccaa ggtacattga ctccccccaa ccaatatctc 298320
ccgaacctac atgccctcct gaaagagcag ctcgctgcat catcatgact ctggtaagtt 298380
tgtagtatcc ataaggcaca tactgttgag gttgctcagt attaatattt gcatagtctt 298440
gacgcttatt ccccaaggag tcttgataag aaaaaggaga attcgataga ttcacccgac 298500
cgttgaaaac catagtattg aaggtttgtg gagtgatatt gatatttcct ccaacggaaa 298560
gatcgttctg gcatatcagt cctccatctg catcaacaaa atttcctaaa acttgagtat 298620
tctctgtagc aatattctga ccaatagtaa gtgctgtttt cttttgctca tcagtaaggt 298680
tgattgattc gatgttaaat cgaacaaaag taggaatcga tattgttatt ttttgtgttg 298740
tgggtatcga actcgacatt aatctacctc cacaaaatta gatggaaatg gaaaccaaat 298800

gaccacatgt ggatagagac ataaggttca tccatatcgt gctctctaag ttaatccaag 298860
gatagtcata aggaggacta gaaaccagct ctgttacact gaagattgca gttacagcga 298920
cactcgtacg gtcactcgaa taacctcctc tagtatctga agtacaaagc aacgttttat 298980
tatttccaga gacaaaatta agaattagag tggcagaatt atctccgtca tgttgtccgc 299040
tccaccgtgt gagctgcata gtgacttgat agatcccgac ttgtttgagt ttaaccacag 299100
gctcaatatc tgcatatctg ctgaagtcag gggtcgcgtt acgactcaca cttttagagg 299160
tgatagctgc aacatgagtt tcaattgtag tgttaggggc aacgtaataa ccattcccag 299220
gataatacgt atagagattc gcagcctgac atccagtttt tctataataa ctaaatgtaa 299280
gcgcatctcg agcagactga ggatcactaa tattattgca tactggagtt tgcgatacgt 299340
ctgtcttaga atagatatta aaaccgtcag agagtgtagt agcattaagt tgagaacctt 299400
ccgcagacac attacccttc acaataaatt cacatggaga agtcgtggta tctaagactt 299460
tgagatcccg agtctctaaa ttattttcta ttcttacatt cctttcgaca ctgagagttg 299520
cttgatctat aaataaattc tgatctttga cccccagagt tttcattggg atttataggt 299580
ggagtcgtcg gtccaaatat gtttccctgt tgttttatnt ttttcataac taccctctat 299640
cgtccggata atagagaaga gaaaaggagt aattccctat acctgcacta gaaccaccgt 299700
tactgagata gaaaataccc gctctatagc gatccgaagc attcggatca ttaggattag 299760
gagctacatc tacagaggct ctatatattg aggtccccag tgtacctatt gttgcatacc 299820
ctccaccact ataaatcgtg ctttcacatt ggacttgtcc taaaccatta ttgatataaa 299880
tggctcctcc ccaaccatta ttccatcccc accgtttacc aacagtaaat gaaacgatat 299940
aggttcctgc tgcaagaaat tgtatcgcag tatttccccc tacagttaat tctcgtgtac 300000
tttgcttcca tcctataaaa cgaaatatac tatttacatt acgtgactga tatagagtat 300060
attccgaacc agtcagaggc aaataagatt cccctgagtt aaaatcaaaa tctccgatca 300120
gaggcttgga acagaaaaaa tattcgggag aacgaacata attcgctggc accggatcgc 300180
agtcttcttt tggcaatgca caattactca gacgattatt tttaaaatct atagagattt 300240
gggaggaagt aaagttttgt gcatttaaat tataagtagt tcctgaagtt aatccagttg 300300
ctgaaacttg atctttaaat gttgcattcc cctgaacaga aaatgtttca gtcgatgaga 300360
ttccttgggc ctgtgtatca aaggtcttga gattggctgc taacaaatca ccatctacag 300420
ttgagtctaa tttaaaatag cactcattat tattaatatt gtttttcatg ataactttta 300480
ataaagttta atttatcaac tcaaaatcaa attaataata ttaagtcaaa ttattaaatt 300540
taaaatatag aaaagataag aatcagaaaa tattcaaact taagaaaagg gattcaataa 300600
aaatgagtgt gaataaccgt aagaaataga agccaagtga tataaggaaa tcgaactcca 300660
agcatcgtta gtttctaaca atacttggag ttcgatgatt attcaacgta aggcaaggca 300720
actatgttcc aagaaaagaa agagactctt aaccctccac cagctctatt ctggaaagta 300780
aagtaaccat tattttgagc atagtatgta acgcaaacta cctgtaaagg acgtgtttca 300840
taataactgg accctctaga ggtcgtcatt gcaagatctg tgacatttat cctccctccg 300900
ccttgctcat attcattcgc cgctaagaga atatctgctc ctgttccgtt atccaaccaa 300960
gaaccatgtt tagccataat aacactaata cgaaaaagtt ttggcacctt attatttact 301020
tcgaaaacca atttagttga gtcatttggg tcaatataaa tctctgttcc tgatgttttt 301080
tgagttgacg tttgatcgaa cttattccaa gggacataac ttccagatgg aacacttcca 301140
ccacctacat accctgatga atgagcagct cgctgtgcca tcataatttg cgtacgttta 301200
taatacccga aaggaacata ttcttgaggt ttccctgagc tcattttttc atagtcggta 301260
atgtcttgtc cttgagaatt tttataagac aaaggtgaat tagataaatt taagcgacca 301320
tcaaaaacca tgctattggt gcttgtaggt ctgatattta tatctttttg tattgtaaga 301380
tccgattgac aagtcagtcc gccgtctgta caggtgagat ctcctctaac tacagtattt 301440
tctgtagtta cttttccccc aacagtaaat gtcgtttttt tttgatcttc agtcaaattg 301500
atagattgaa tattaaatct aacaaaagtc ggaattgaaa tttttgtttt cggtgtagga 301560
tttgacatta atctacctcc gcaaaattag cttgaaatgg aaaccaaata acacatgttg 301620
acatggattt aatatctaaa ccaatagtag tttctaagaa taaccaaggg taatcatgag 301680
ggggggtagc aacaatttct gttaaagtaa acgtgcctgt tacagcaata ctagtcctat 301740
gtcctcctga gtaacctctc gtatctgaag cacaaagcag cgtcttatta ttcccaatca 301800
ttaagttcag atataaatta ggattatcaa gtccactatg ttgcccgctc tcgcgacgta 301860
tttgtattgt gacttgatag attccaggtg ccttgaactg aatatagggg aatctcgtta 301920
cagcatcata aattttagct gaagcgctaa tgcgatacgt ctctggattc gggttttgac 301980
acaccgtttc gattggttta cctacagtag taggctgaga tgaactatag taggtataca 302040
gattaagggc ctggcatcca gtgttcctat aatagttata tgttaaagca tctcttggag 302100
attggggatc gctaacattt gtaaacttag gaactcggcc atcctgatct tcagaagtga 302160
tctccaaacc tttagaaagt gttgtcgctt taaattgaga actttctgct gataatccgc 302220
ccccgacagt aaattcacat ggagaagtga ttgtatctgc aacttttaaa tctcgagtct 302280
ctaaaaagtt ttctatatcg acattgccat ccacattaag agtcgcttga tcaagaaata 302340
agttctgatc ttttattcca gtattttcgg ctacatttgt agacggagta gaagcattag 302400
gtagaatttt ttgtaggttt cttcttttca taactacacc ttatcccccg caaaataaag 302460
aagtgtaaag gaatagttcc ctattaccgc gctgtgatct ccattgcgaa caaacaaaaa 302520
attattcata tatttgtcac ttggattgtt cgggtcagga tctatatcac tatgatctct 302580
atatactgcg gtacttaaat accctatcgt tgaataccct ccgccactat acactgtgct 302640

cccgcacaac attgttccat cgccagtgta tttcccttca aatagacgaa tcgatcctcc 302700
ccaaccattg ttccatcccc agcgcttacc aatagtaaaa gttaggatat aggttcctgc 302760
agcaagaagt tgtatggctg tgttcccccc aagtgtaatt ttttcgaatt ttgatcccaa 302820
tctacaaaac gaaaaacatc accagccttg cttgactgat acaaagtata gtttgatcca 302880
tctccagtta taggtaagta tcgactactt ccatcaaaca taaaagtccc ttctattggc 302940
ttggcacaga aaaaatattg gggagaacgg acatagttcg cgggaacagg atcgcatggg 303000
tttttcggta atgcgggatt actaagacga ttattcttca tatcgattgt tattgaggaa 303060
ggcgccgggc ctgtagcatt taacttatag gtagatgccg aagtcaaacc tgttgctgaa 303120
actttctctt taaatgtcgc gttcccctga acagaaaatg tttcagtcga tgagattcct 303180
ttagcttgct tatcaaaagt ctgaatattc gaagctaata agtctccatc tactgttgaa 303240
tcgagtttaa aataacactc attgttatct tgattatttg ttgttgtcat ttcaataaaa 303300
tagttataag tttttctttt aaaaacaaaa tagatctatt tagtcaaata attaactaat 303360
aataattctc ttacgaaaca agataataaa taaaattcac aaaaaatagg aactgttcgc 303420
aatcaaaaac ctttgattat cgattgagat ttggaacttt taattcaaaa gttctctccc 303480
cgaatccaaa taatttttta cattttctga gcgaattgat tagatagaaa ctatgagatg 303540
acaattcatg aaatgcattc ccaatagtta taagttttat ataagatcct tgagaattca 303600
agatattgaa gaaaaagaag tgggggtggc tggatttgaa ccaacgtatc cgtaaggagc 303660
cggatttaca gtccgatgca attgaccact atgcgacacc cccaaaaatg ctggagaaag 303720
gaattgaacc ctcaaccgtt cgattacaaa tcgaatgctc tgccaattga gctactccag 303780
cagaaataga agtgcgatta tctcaaaaag aaagttttat tctcaatcag aaagtaactt 303840
atctgaaatt aatcttcctc aactggaggt ttttgagagt tttcagatat gcgaaaatct 303900
tcttgtgtag ggataaaatc ctcacaaaca aaattagtgt cttgattcaa agtagctgta 303960
ctctccatag gtggagctag attctctcca gtatttagaa tctcaaaatt atgctgctga 304020
agtttctctg taaccaattt ctcttctttt gcctgagctt tcttaagagc ttttttactc 304080
attacttctt tgttcttagt aatctgatga aacttaccaa cagccgcttt ctgttctgaa 304140
gttaaagatt gagataaacg ctggaacaac ttcgtacaat tatctcctgc ttctccctgc 304200
tcggatgcag agagaacttc agcagaaaat ttaaattttc cttcagttgt ataccgagct 304260
gtttgttctt ctttttttaa acaacattgc ttgtactttt tattagaacc acaagggcat 304320
aaatcatttc tattaatttt ttttgacacg tgatcctctt ccacacaaga tcttttagaa 304380
aagtctaaat aagctcaaaa taaaatccaa agtcaaatgc aaaaacctat tgcttttttc 304440
tttaaccttg gaacctagta ctcgttaata aaaagaagaa aaacttaatg aataattaaa 304500
ggtacaaact ttatgccagc ctattcacct gaactataag tttctggatt tttaacagcg 304560
atttcttaag aataatattc ctttaaccat ccattcattg ttttcatgtc cactttactt 304620
ttaaatcctc cgtggatgaa agcgggaaaa cgtatagaaa gcttggtccg aaaagcactc 304680
tatacccata ccatgttagc aaatcatcgt aaaattgtag ttgctctcag tggtgggaaa 304740
gatagtctta cacttctttt aatgctcaaa gcaatttctg gaagaggatt cccagattta 304800
gatctccacg cggtcaatat cggaggaaaa tactcttgtg gagcagaagt taataaacct 304860
tatttaaccc gcatctgtga tcaactgtgc attccgttta gaacaattcc ctctccttat 304920
gcacccgaaa ccccagaatg ttatccatgc tctcaagcaa ggagacgttt acttttccaa 304980
gccgctaaag aaataggagc ttcggctatc gcttttggtc atcaccgaga tgacctcgtc 305040
caaaccgcct tattaaatct tctacataaa gcagagtttg ctggaatgct tcccgttttg 305100
gatatggtcc atttcggagt taccattttg cgccccttaa ttttcactcc cgaattctgg 305160
attcgcaagt tcgctaagga aaacgcttcg caagagtcac ttgccgttgt cccgtggttt 305220
cattaagaag caaagcggaa caaagtttaa agttattaga agaggtattc cctttagcac 305280
gtcataatat cgctttggca attcaagaac atgggtcatc caaatcacaa aaaatttgag 305340
agacactttg ttattaatta atttttaata taaataaggn agtaaaagtt tacttggttc 305400
tcatgaataa aagattaaag ataattctaa ctaatgatga tggaatcaca gctaaaggna 305460
tgagttgcct agtctctgct ctattggaag caaatattgg tgatatttat attgcagctc 305520
cccaagccga acagtcgggg aaaagcatgg caatctctct gaaccaagta gtctgcgcct 305580
ctccgtatgc atacccgcaa cccgttaaag aagcatgggc agtaggaggc tctcctacag 305640
attgcgttag actcggcctt agaacacttt ttgaatcggt ttcccctgat ttagtgattt 305700
caggaattaa ctgtgggaac aacatatgca agaatgcttg gtattcagga accataggcg 305760
ctgcaaaaca agccttggtc gatggcattc catcgatggc actttctcag gataaccata 305820
tttctttctt tcaacaagac aaggctcctg aaattttaaa agcattagtg atttatctct 305880
tgtctcaacc ctttccttgt ttaactgggt taaatattaa ctttcctacc agtcctgggg 305940
gttcctcttg ggaaggtatg cgacttgtgc ctccaggaga tgaatttttt tacgaggaac 306000
ctcaatacct aggctctgta aacaaaaatc aatattatgt agggaaaatt tctggagtac 306060
ggattggaga gcatccatca gaagaactcg cttgcatgct agaaaaccat atcagcgtgt 306120
ctcctatatt ttcacaaaac tctcctatcg gcttaatgac tctagaggaa tttcaaaaga 306180
cacaagaaaa tttcaatgca tcgcttttaa gctctgagct gaccactaaa attttctaaa 306240
agccccctag tctttggtat cagaggtctt tttttacaaa agatctcttg tttaatcgag 306300
atttaatcac ttaaattgaa agaaaaataa ttgtgattaa actatttctt ctggtaagtt 306360
tatgctccta tcttattgga tgcgtagctc agcggttaga gcacctgtct tacacacagg 306420
gggtcatagg ttcaaatcct gtcgtgtcca tttgcgggag tagttcaatt ggttagagca 306480

ccgccctgtc aaggcggaag ttgcgggttc gacccccgtc tctcgcgaag tatgctttca 306540
gaggtttcag gatgtctcac ggtccacgtc caacaaaatt cagtttccct ctgtatttct 306600
ccaaaacatt aagttggttt attttaggtg gctttcttgc tgcttgcgga gttcagatgg 306660
tattagtgcc taatgaactt atcgatggtg gtattgttgg cctctccatt atagcctcgc 306720
attttttagg tcacaaagcc cttccttttt gcttagttct cttcaatctt ccttttgtat 306780
tcctagcatt taagcaaatt gggaaatact ttgtgattca aatgttgaca gccgtgatta 306840
ttttttcgtg ttctctctgg cttattgatc aactcccctc ttggctcggc atgagcccct 306900
ttgtttttaa aggatcagaa atggaaaccg ttgtgctagg cggtgctatt attggcgtgg 306960
gttgtggatt gattatccgc cacggagggt ctacagatgg cacagagatt ctaggaatca 307020
ttatcaataa aaagaaaggc tacactgttg gccaaatcat tttatttgta aacttcttta 307080
tctttgcttt atctggtatt gtctacaaaa attggcacac tgctttcgtt tcatttctaa 307140
cctatggaat tgcaacaaaa gtcatggata tggtgatttt aggcctcgaa gatacgaagt 307200
ctgtcactat tattacctct tctccaagaa agctaggcca tattctcatg gaaaccttag 307260
ggattggctt aacctatatt catgcagaag gaggatactc cggagaacct agaaaccttc 307320
tttatgttgt tgtcgaacgt cttcaacttt cacaactaaa agaaatcgtt catagagaag 307380
atccctcagc atttatcgcc attgagaacc tccatgaggt aatcaatggt agacgaactt 307440
aatccttgaa agaatcactc tggattcgac cattgtttcg ttaggtcgct agggatattt' 307500
agataagcaa ggatttttcc tactaaagca ttttcaagat cttcaaccga ttgaggctta 307560
aagtaccaca taggcatcgg cggaaaaatc gttgccccac tcttgcttaa ttttaagaga 307620
ttttctaaat ggatcgtatg caaaggggtc tctctaggga caagaatcaa gggtcgcctt 307680
tctttaagag ccacatcagc aacacgtcgc agtaaattat ctgctaaacc tatggaaata 307740
gctgcaactg tagtcatact gcaaggaata atgatcgtag cttctacagg acaagatcct 307800
gacgctaaag agctctctat agcttgaatg ctatgagtat gaatatactc taagttttct 307860
tctgaaaata atgcgtcaaa agactgacac cctagttcat aatacaacgt ctttcttcct 307920
gaaggagaaa taattacttc aacttgatgt tttgcattta cgagttcctt aataagctta 307980
acagctagta ttaccccaga ggcacctgaa atgcccacaa cataacgctt catctactca 308040
aactccaaaa caaagtcata cttacaagaa acgatagagc aatcgcaata ttcgctaaaa 308100
agaatttggc tctctccttc ttgatctttt ttactgtagt tagaatacat tctaactacc 308160
ttaagaatca caactagagg aatgatagct gtgaaataga actctttatc taaagaacca 308220
acaaaacctg aaaaaatata agcaagataa cttacccata agtttacttt tgcaatttca 308280
acggctttct tttcaccgta atgtgcagga acactacgca atccttcttc cctatcaaat 308340
tcggtatctt caattgcata tatgatatca tttgcagcta ttaccatacc cacactccct 308400
ccccataaaa gagcaagaaa gcataaacgc atggaaagcc ccgactcagc aaaagcacaa 308460
aaattcataa gaatcgctac agtgtacacc aaccctaatc cccaatggca gaaaaaagtc 308520
acccttttca tatagggata gacgatcata agagtcagtg aagcgatgcc caaactaaaa 308580
atacggagaa tcttgcagag aaaaagaaaa agaaaactac aaaatagaga gagtacccaa 308640
gcaaaattta aagaaacaag attcgcagga aggactcttt tcgatgttct tgtatttttt 308700
ttatcaatga accggtcgat acactgattc actacaattc ccgtagtcct tgcaaaaaca 308760
aaggctatgg ctccaaaaac tgaaatcttg aacccttctt taaatgataa attttgggaa 308820
atctcattta tagaaagagc aaagactgtg gatgctgata aaaaaagtat tgaaaatatt 308880
gaatacttaa aattaactaa atttaaaaaa taatttaatc tcacaattat tatcttctat 308940
ataattattt ttttatttat aaaaaaaata tactcctctc tattcatcgg gggtgatccc 309000
cccaatcttt ttagaacccc ctatgttagg gggttcgtct ttttatgcct ctactgtgta 309060
catttcaggc attagtgcga tacatttttc ttaaatccaa aaagttccat gttctttcgt 309120
atagagggta gttcttgtgc atcagaaatg gtccttaaac aaaactccta acgccaagac 309180
aacaagctga ttatgaagtg cttgtcaaaa caaaaatcct tttaaatata ttgctcttta 309240
aagaaaaata atgacaagaa attcgtaaaa aagcgctgtt ttattaacaa aaaggaagag 309300
actttatact tatgattcta gtacaaaatt gcagaatcag atgaaaagaa ctgtactagg 309360
gtttttaaaa tttagttatt atatcaagaa ataaaattaa aaataccttt aacctaaaaa 309420
gattaatctt gatttaattg atttttctaa taaaattatt cgatctcaaa aataaaacga 309480
gtagataatg gcgttagatg aaattaataa tcagaacaac ccatcgcaac agatagcctc 309540
ctcgacttct caaacttcta aaatcaacca agatcgaaaa actttcgcat gcactgttac 309600
cctacttgtt gtagcaactt tgatgatcct atccgggatt gttttgctat ttactatagg 309660
ttccctagga ctcagcgttc ctttatctgg aattctaggg acttttgcag tgacagtagg 309720
agctgttctc tttatcacag gtctaactat tctagttaga aaatccctag gaatcgaaca 309780
gaaaaacgaa gacttgaatt ttttaaagat taagacccca actcccccag cacgccccct 309840
aatgtcaaag tttagtgtta cctgctccac tacaagtatt gttttaggaa tggctcttct 309900
tatcggtgct gtcgtctccg tattttttct cacaggatat ctacaactag gcttgtgtgc 309960
aggacttgta ggtcttggaa ccgccctatt tgttgcagga ttagcaagga tgtcccctcg 310020
tagcctagca gaccaagaag gctccggctc cgccgattct caatcaaata ttgttggaat 310080
aggtgagcca aaagcagctc aggaacaaaa atggtataaa atggcagtgg taaggggaga 310140
agatggtata ccaacagcaa ttcgcctaac accagagaaa taaactatta ttttaagaac 310200
aaaaaagggt tttaaagtag gtaaatgaga aaacttactc actataaaac tcttaaaact 310260
ataaaaattc cgaactttta tactatatcg aaagcaattc atccaggaat tacagaaaat 310320

tgaaaaacta tttaataatc tacctaaatt ttctgattta aagaactata agatgtaact 310380
ttaacgattt ttttagtaaa ctaattcgga ttaaaaatga aatgagtgtc aataatgtca 310440
ttgaataaaa ccaatgccct tctcaatcaa ccagagcctg ctgtttgcct taatgcttgg 310500
gaccctaaat atatcaacca agatcgaaaa actttcgcat gcactgttac cctacttgtt 310560
atagcaactc tgatgatcct aacaacagga gttatcgtat tacttgctat gggctctcct 310620
ggattaagcg ttcttgtatc aacaattata ggaacctctg taacaacttt agggactgct 310680
ctcttcataa ttggtttagt taaactaatt aaaaaatcat tagcatggat acagtatcag 310740
aaatactttc aagaagtcgt aaaacagaaa tatgaaccct ttagcattcc taaaaatgat 310800
aacgtacaca aactcacttc gtgcttacca tcacctttag atattgagag tccgtctcca 310860
gaagcaagta ccccagtctc taagttacgc attgcatgtt caggagtcgc tattgtttta 310920
ggagtgactc ttcttattgg tgctgtcgtc tccgtatttt tctgcactgg atacctacaa 310980
ctagctctat gtgtaggatt cgcttgtctt ggaactgccc tctttgttgg gggattggca 311040
gggttgcgga cccacagctt aatcgctcag ggcatcatgt atctttacct gacttactat 311100
ctatcatcgg ctctggaaga aagaaacgaa acagtcaagg atcagcgtaa cgaaatcaat 311160
acatatttaa ccgaggaatg caggcagcaa aaaagggaaa aggcactgtt ggaatagaaa 311220
caatgggcac tatctctcat gtctcaatgt cagagtagca gtacatctac ctgggaatgg 311280
atgaaatctt ttgtgccaaa ctggaagaat ccaactcccc ccttatctcc tataccttct 311340
gaggacgaat ttatattagc atacgagcca tttgttctac cgaaaacaga tccagaaaac 311400
gcacaagcta atcctccagg cacatctaca ccgaatgtag aaaacgggat cgatgatctc 311460
aaccctcttc tggggcaacc caacgaacaa aacaatgcca acaatccagg aacttctgga 311520
tctaatccta catctctacc cgcccccgaa cgactccctg aaactgaaga gaacagccaa 311580
gaagaagaac aaggatctca aaataatgag gatcttatag gataaaaaca gtgcgaatga 311640
gttccgccct gtttaatact gtgttaagaa agagcagcca ttcagataaa tgtccagcta 311700
aagcaagaag gacaaagact cttgcttctc ctttgataca agaaatgact cctatctttc 311760
atagtttata gcaatactat aatttcacgt ctgttatctt tggattcact tgttctacaa 311820
tgacttgctt taaaaatttc tcagcccgag aagaaagtat ggactctgct ttccctttag 311880
aaaaaatacg atgaatcaac tgatttagaa cgggatgggc ttccgcagaa ctacactcat 311940
taaaaaagaa attttcgtct tcatttgcat aaaagaaatc cgacccaaga acaatacttg 312000
ataaaattcc gagattctcc gcatggagga catgcttctc caaatcaccc aaggaatctc 312060
ctacataaga cctcactaga ttcaaaccaa taacaccttt tcttctcaca atctccttgg 312120
catgggcatc tacaaggttc cttcgatgat caagaacaga tcgaaaatta gaatgactgg 312180
caatcactgc gagattgggc aatttatcgg cagtataatc taaaatatct tcagctaact 312240
tatcactaca atggctaaga tctattggaa ctccaagttc gtacatgata tctaaaagaa 312300
ccttaccatc attagaaagt ctcttaggag cttctgtgcc gcctccaaaa cggttatccc 312360
ctttccatac gattcctaga taggcaagag gcccctgttt agttaaatgt ataagctttg 312420
ctaatagagt cccaaggggg gcggtatcat ctcccaaagc tgaggcattt tctatactac 312480
gaataagact tagtgatttt ttttgagaag aggatccgtt ctcttcttct tcataagata 312540
ataacccaat atccggatat tggttaggaa gagaaaaaaa cagagagttt tgtttatcac 312600
aattaggttc cccccgactg tgggggacaa aaatagcaca cacctgctga cgtacgcctc 312660
cagataggag ttgttctgga gaacacctca ccgcaggatc tttccgacaa aaatgcggat 312720
gcgaaagtaa gtcacaatgc atatcgatag tcataacaca cctaaatgct actcaatata 312780
ataaggggaa agaaagggga aaagttcggg ataatccgat gtatttatat cataaataaa 312840
gactatagaa ggagttttct tttttaaagc ttcccgtaat ctggtataaa aattcttagg 312900
tttaggatgt tcacaaacca cacctttaaa atctaaagat ccgctcaaaa aagatactaa 312960
ttgctcttga tttttcactg tatagatctt gatatgcttc gaaaaggcat gtgacgttct 313020
atgaaaaatc ttagcttcag tccctgcaat gttttctata acactgcgag aaatcagccc 313080
ctcacgataa atataaagag gatctgaggc aactactgtg gactctaaac catgggaaca 313140
agggccatca aaaatacaga gatcatgatc agcaaagtct gcgaatatct cttgagctgt 313200
aagagctgaa ggaaactctg aaagattcgc agatgttccg attaaggttc cacagtgatc 313260
cacaatttcc cgaactacag agtgatctac aatgcgaaac gctagtgttt ctttagggaa 313320
tctaggattg cggtgcttaa ctactaaagt aattgctcct gggaaaagtt gagctaattt 313380
cttagctgta ggagataagg gataaccaga aatgttttcg attcttcaat agaatttaca 313440
taaagggcaa aagctttact aggttctcta tcttttaaag catagagtct ttcttcagct 313500
tcagaggcat acaagctaag aacaaaccca tagacagtgt cagtaggaag agcgacaatt 313560
ttcccttgat gtatagctga cataacctct ggaagcgaaa aagtaatctg tgctttttta 313620
tcaggcacaa taactccaaa aatagattaa agaaggtcta tgttttttag aatccgcaca 313680
aaaccgctta tacatataat ttgtacgctc tttatcaaaa agagaattcc ttagattaac 313740
gcaaaaagtt aatctaaatc cataattttt attcagaaaa tagatttttt attaacttat 313800
acaaactaaa cattcaaaaa tccaaaacta catcttaaaa tattgactac gccacttgaa 313860
gacaaaaaag aaattattga tccctaattc ccttcgatat aacgtggtaa cttcttatta 313920
ttaaaaataa aaacttatga cagactactc tttctttcgt cgcaaaattg gcaatattga 313980
agccatagag tgccctggaa atcctcaaga tcctatcatt attctgtgtc atggttacgg 314040
atcacttgct gataatctca ccttctttcc ttcgatatgt tccttttcaa aattacgccc 314100
cacatggatt tttccaaatg gaatccttcc cttggagaat gacttccgag gctctcgtgc 314160

atgctttcct cttaatgttc ttttattaca agaactctct aggctctatg ccaatggagt 314220
agggaacctt caagaaaaat atgatgaact atttgatgta gatctagaga caccgaaaga 314280
agctttggaa gaacttatcc tcaatctcaa tcgaccctat aatgaaatta tcattggtgg 314340
atttagtcaa ggagccatcc tggctaccca ccttgtctta acttctcaga atccttatgc 314400
tggagcctta atctttgctg gcgcaagact gttcaatcaa ggctgggaag aaggacttaa 314460
acaatgtgct caagtgccat ttttacaaag ccacggttat gaagacgaaa ttcttcctta 314520
tcacttagga gcacacctta atgatcttct attaacaaag ttgaacgggc aatttgtttc 314580
tttccatgga ggacatgaaa ttccctctgt agtattccaa aaaatgcaag ttacagttcc 314640
taattggata gatcctgccc ggggctgaca gaaacgagtt ccttttcttt atactggaga 314700
gataccaact gaatagctac ccattctaaa acagatgctg ccgatgagga gttattcaat 314760
gaccgaccag gcagattcca caattaaaag caccttatct aaaggcagta atggcaattg 314820
taggatctcc ttaacatgct caggatagtt taaagctgaa gcctttaatc ctaaattcag 314880
agtataacga tctcgatata gctctaggag cacttccatg aatcgttgca ccttgtctct 314940
taacacctgt ttatctgtct ctgaagattc ttttataatt tgagacactt cagtaacggg 315000
aatttcacat tgggcatagc gaaataaata agaaaacgtc tctttggaac ataagatttt 315060
ttctcccctc tcaataaaaa tagagagact ccgagaaatg atagtctttg gcaatctctg 315120
aacttttgct gttgtcaata tgataacagc atgtttcgga ggttcttcga agacttttaa 315180
aaatgcggaa atcgcagcta aagtcattcg atctgcttcg tgaataatgt agatcttata 315240
gtttgcttca aaaggagata tataaatctg cttcttaatt cccctaggaa gatctataga 315300
gtgcagtctt ccctttcctt ctggaaagaa ttgataaata tcgggatgaa tcttttgaga 315360
aactttatgt tcggaacctg gagatgacga tagcaaaatc tccgatgcta actgttctgc 315420
tttatcctgt aaaacaggaa gggtaaatcc atgcagcagt atagctggag gcacttcctg 315480
atgataaact ttcctaagta aagcttccca gccttgattc tcttcttcta ggtgcataac 315540
ccaagttgcg tatgtagcat cactttgtct attaaactag ctagtgattc acgagcatca 315600
agaaccaagt aacgactagg atcggcacta gcaagagata aaaatccttc tcggatcctg 315660
ttatgatagc tcaaaggctt tttttcaaac ttgtcaaaaa ccttttgtcg gtgcttcctt 315720
tgtaagccaa tgtctgcagg aatatccaag agtaaaacaa aattaggaag gaaaggtgta 315780
ggtcctacaa ccttagaaca aaggtcggca acaaaatcag cacccaaacc ttcggctatt 315840
ccctgataca caatcgtgga atcgtggaat ctctcacaaa tgacaatata gccatcacgc 315900
aatgcgggaa tgatcacttc ctgaatatgt tgagcacgtg atccaagaaa caaaaagagt 315960
tcacaacagc gagagagttc taagtgagga ggttccaaaa ttaaatctcg gagtctttcg 316020
cctataaggc accctccagg ttcccttgtt aacaaaacct tccgatcttg agctactagc 316080
tgatctccta aagcctttgc taaagaactt ttgccagacc cttcgccccc ctcaatcacg 316140
ataaacacaa tactacctta ggcttactgt atcagaacat tcttcttcgg accctgatag 316200
tacttcatca tcattttcat tagaagagag tttttccata gaaacaagag catccccttc 316260
tttcaagtga actaaacgca ccccttgagt cgatcttccc atcactctaa catcttgcat 316320
gttaatgcga attgcctgtc cctgactcga cattaataaa atactgtcgt gatccgtaac 316380
aggaatagct cctaggacat tgccgtttct ttcattgata aggatagaac gtacgcccac 316440
gcctccacga ttggtttcgc ggaaatcttc aactaaagat ctcttaccaa aaccttgatc 316500
acatacaatt aaaaccgact ggttctcagt cacaatttga caactgacaa ctttatcttc 316560
ttcatttttc aaagagacac cgcgaacccc acgtgcagtt ctacccatag gacgaacttt 316620
ttcatggggg aagcgaactg ccataccaag atgagtaaat aacattacct tctcttcatc 316680
actaacaatg tgacaagctg ctatgagttc gtctccctca tctatctcta aagcacgtat 316740
tccctttttc ctagggttgc taaaggcgtc taaggaaact tttttcacaa caccacgttt 316800
ggtggctaaa aataagaagc cggcattatc aaaattctta atattcaata tagctgcgag 316860
ctcctcacca ggacgaattc cctctaggaa gttgatgatg ggcttacctt tcgccctccg 316920
ctccccttct gggagctgcc ataccttcaa ccaataacat tggccaaaat tggtgaagat 316980
taggagatag tctttagtaa aggcagagta tacagcttta aggaaccctg ctcccttctt 317040
catatcaaat cccgtaactc catgcccgcc acgacgttgt tctttgaaca cttttacagg 317100
cattctcttt acgtaatcat ctccggatat cgtgataata acagactcat tggtaatgat 317160
gtcttcaata tcacgaatat catcagcatc gaattctata gtagtgcgtc gagctacctt 317220
atgatgcttc aaaagatctt gtaactcatt tctgatgata tcttttacta aaccttcatc 317280
cgataaaact tgtttataat aagctatctt atttaataac tcttcgtact ctttttgaat 317340
cttttcagct tctaaaccag taagttgata taaacgcagt tctaaaatcg caagagcttg 317400
aggttcggta aaaccaaaag attcaataat ccgttcttta gcatgctctt tattcccact 317460
ctcgcggatc gtttttacta atgcatctaa gcaagataaa gccttaaggt atccttctaa 317520
aacatgagct cgtgtttctg ctttatttaa ttcataacga gtcctacgac ggatgacttc 317580
tttacgatgg cgtatccaag cggaaatcat cctatgaata ctcatcgttc taggtaagtt 317640
cttatccagg gcaagcatat ttgccccaaa agttacctga acatcggtga acttgtagag 317700
cctattgata atgatttccg aagattctcc tttttgattt tcaaggacaa cacggattcc 317760
atctttatca gactcatcgc gaacatctga aatgcctgcg agagtctttt cattcacaag 317820
atttgcaatt tgctcaatca aacgtgattt attcacatta taaggcatct ctgtgatgat 317880
gatgctctca cgatgtttat cttcattctc ttctacatgc agacgagctc gaactttaat 317940
ctttcctcgt cctgtagtat acgtggagcg aattccttca gaaccgcaga taattcctcc 318000

tgtagggaaa tcagggcctg gcatgacttg taaaatctca tccacagaag cctgtggatt 318060
tgcaagcaga agaagtgtag cttctataag ttcccctaaa ttgtgtgggg ggatattcgt 318120
tgccatccca acagcaattc ctgaagaacc attgcaaaga agattcggaa atttagaagg 318180
aaaaactaca ggctcgtgtt ttgtttcatc atagtttgga acaatatcca cagtatcttt 318240
gtccaagtct tccataagat acatagcact atgggtaaga cgagcttctg tgtatcgcat 318300
agcagcaggt ggatctccgt ctatggagcc gaagtttcct tgtccgtcca ccaaaggata 318360
acgcatcgcc cagttctgag ccatacggac aagcgtagga taaatgacgc tctcaccatg 318420
tggatggtag tctccagagg tatccccaca aatttttgca catttacgat gcttagctcc 318480
tggagaaaga cttagctgct tcatggcata aagaactctt ctttgtgaag gcttgagtcc 318540
atcccggata tctggaagag cccgagatat aatcacagac atcgaataac gaaggtaact 318600
ttccttcatc tcttcttcaa gatttttagg gactataatt tcatctttat tgaacatagg 318660
gattgtggac tcctaaatat ctaaattatt tatcctaatg gacaaagcat gactttctat 318720
gaattctctt cttggaggga cttcttcccc catcaacata gtgaaaatat ggtctgcttc 318780
tacggcatcc ttcaatgaca catgaatgag tgttctctgc tcaggattca tagtagtatc 318840
ccaaagctgg tcggcattca tctctccaag acctttatac ctctgaattt ctatgccttt 318900
tcttccaaga tttttaagat agttaatgac ttcttccaag gtatagcagc tatagttaca 318960
gcttggggaa tcttcatttc caatcacaat ctcgtttttc tgagggataa gatagctaga 319020
aatatctaaa ccatattctt tgagttgatt ttgaatatct acgaacacag caactttata 319080
aagctctatg attttaaact tatgagtttc ttcttgagct aaagcttctt ctttttcctc 319140
atcagaataa agatagcgcc ctccctgcat tccagttgcc ggagcaagat agtacaaagg 319200
atagcctatc ccctctttat acatctctaa aaattcagag aagggaatcg cttttttctc 319260
aagagtgttt ataaagctct ctacatctaa aatgacgttg ataaaactct ctaaagcctc 319320
tccacgtaat tctctttccg tagatttaaa gagaatggag ctctcattcg tgcctaacat 319380
gagcaaatag ctgtccattt ctttctctga aagaatataa cggaagtctt ttttcttact 319440
caccttgtat aaaggaggtt gagcaatata aacacattca ttttcaataa gcgctgtcat 319500
atgacgatag aagaatgtga gaagtagggt acgaatatga gaaccgtcca cgtcagcatc 319560
tgtcatgata atgatacgtc tatagcgtaa tttactgaga ttaaaattat cagcacctat 319620
gccacagcct aaagctgcta tgatggttcc tatctcttgg ttttggaaaa ttttctgtag 319680
acgagctttt tctacgttca gaattttacc tcgaataggc agaattgctt gaaatcttcg 319740
atctctacct tgtttcgcag atcctccagc agaatccccc tccacaatgt acatctcaca 319800
cttttcggga tctttttcta aacaatcaat tagttttcca ggtaagcgtg cgctatctaa 319860
agcacttttc cttaaagtca attctcgagc tttttttgca gcttctctag cttgcgctgc 319920
aacaaaaacc ttatcaacaa tcatcctagc aatttgagga ttctcttcaa aaaagattgt 319980
cagagcttcc cctacaacct gttgagccac tgagctaaca tcactgtttc ctaatttctg 320040
ttttgtttgc ccttcaaatt gtggatttgg gacctttaca gaaatcacag ctgtcagacc 320100
ttctcgaata tcttctccgg ttaatgcaag cttattattc ttcgcaaggt tatgagcttt 320160
aatatacgta ttgattaccc tagtaagcgc ggtagaaaac cctgtaagat gcgttcctcc 320220
ttggcgtgta ggaatattat tggcatagga ataaacaagt tcagaatacc ctgaattcca 320280
ttgtaaggct gcttcaaact cgatttctcc atcatctcct actcgagttc cacaaatata 320340
aatcggttca gagaaaaggc tttctttatt ttgattcagg taacttacaa aagattgaat 320400
ccctccctca taaaagaagg taaccttgtc aaagctaaca tctcgatcat cttcaaagac 320460
tattgtgatc ccacgattta agaaagcaag ctctcgcaag cgtttcatta aaatagagcg 320520
atcaaaagta caagtcgaaa atattttagg atcagggtag aaaacgattt ctgttccctg 320580
ccgatcacta acacttacat actgcaatgg agttacagga attcccctag agaactccat 320640
ttgataacac ttcttatctt taaagaccgt ggcaactaat ttctccgaaa gagcattaac 320700
gcaagaaacc ccaactccgt gcaagcctcc ggatacttta tagctatcct tatcgaattt 320760
tcctccagca tgaaggactg ttaaaaccac ttctaaagca gagacctctc taccttgttt 320820
tgcagactct ctttcgtgaa cttctatagg gattcctcgg ccattatcta cgatgacaat 320880
acccccgtcc tctaaaatgc gaacatcaat cctagagcaa taacctgcca tggcttcgtc 320940
aatgctgttg tctacaacct catagactag atggatgaag acccgtgatt cccgtatctc 321000
caatgtacat cccggggcgc tcacgaacag cttgtagccc ttctaaaaca gtaatagcgg 321060
atgcatcgta atttttttct tttgggtcca taaactatcc taacaaaaac tgtatttccc 321120
taatctgcac gtgagaagcc acctgataca aactcataat taaatcattt tgaggcgtct 321180
gttttaacaa agcatataac gaggaattgt aaacttttac taacaaaata tgatctctga 321240
accctatagc ttgagacatc cctttatatt tgtctctaaa aacctgattc caagcgtcaa 321300
tagcgtcatg aggcctagct gccataattt tttgaagttc ctgcaaataa ttatggagat 321360
agtgtttagc gtgttttata ggggaagctg ttcctttatt ttgaacttga gatcctcccc 321420
ttttttttcg ctttaagaac atatcctacc tgaagaataa cacagcgcat atcacaaaaa 321480
cacagattta tcaaattgag agacctttat gtctcgtctt ttctctgttt ttctttatga 321540
tcagttttta aataaagttt taataacatg ctgcatcaaa agaaacttct tatatccaca 321600
gaaacgaaaa aagtcaataa gacgaagagg aattcttatg ataaaaatcc cttttagggg 321660
gctgtaaaga tgttaagaga gcagtaaaca cacatgtcgt tacatgctaa acatatacaa 321720
cggtaggata aggcttatct ggagaaataa gagtcttcac ctctataggc tctagctctt 321780
caccttgaga gcaaaaacct aggtacttta caaccaccac cacgcagaat gcaatcgctc 321840

ctataatagc gactacacaa actccaaggc caaacaagca aacgcataga acatctatga 321900
ggcattttat taatttaatg attacttgga gcactaaggc tgtgatttct aacgctagta 321960
taacgatccc tccgccgaga atctctacaa ctccacgaac ccgatgtgcc caaagattta 322020
cagaagttga ggatttacta tgtgttgatt tctctaattt ttcctgcgca acttcctcta 322080
tatcctcagc ataagtgcta ctaattctag aacaacctat aactatattc aaaataggaa 322140
tagcagcttg gaaattagtt gaaaactgct gaacagaagt aataccaaaa gctggggctc 322200
taacattaat tatcattaga attacaccgt agaaaattag ggggggtaga ttctttatga 322260
gaacttcttt tccttcaata aaatatttcg ggtaaaagat cgaaatattt tattgaagga 322320
aataagatca aaaagagcgc acaagtacaa cgacttattg ctctaaccat tgtcttgagt 322380
cattgtcaaa atattcccat gatattttgt atcaaaaaaa tgttgtacag aaggagattg 322440
aaacagcttc tgtaatttta tcattttagg agaacctacg tcttcagaac gaatgacaac 322500
aaggtttgta tacttagata ccgaaagatc ctctaaacaa agactatctt tctttggaga 322560
aaggtttgct gctatagcaa aatttccagg aatgacagca gcatcaacgt caggaagaga 322620
tccgacaaga agaggagctg acacctctaa tatgttgata cttctatttt ctttcccaca 322680
gacatcttta gctgtcatat ttaaattagc aggccctttg caaacaatga gtccgcactc 322740
ttctaacaag tgtagagcac gctgagcatt cgtacgatcc acaggaatcg ctatagtcag 322800
tttcttctgg ctttttaagc gctctaaaga agaatgtttc ttagaataaa ttgcttgagg 322860
ttccaaatga actttagcga taacaactaa ttcaccctta caatcataac gctcgcattc 322920
gtcatcaaga aaagcttgat gttgaaagta atttgcatct acttgtttat ccaaaagcaa 322980
acgattagga atacgataat catctactgg aagtattttc agcttgattc caagatcttt 323040
agcctcttcc tgtaaactct ccaataattc cgcatgaggt gtcggactgg ctacaatacg 323100
tattttattc tgagcatctt ccttatggca agaactcaaa acaaaaatta aacctacaag 323160
taatgataat ttttttttca tagaatccct ctatacttta atacacgacg cccccagaaa 323220
tctcctagaa tacgcactga ctcgataaga actaaagtga tgacaagtac tgatgttgtg 323280
acagaccatt caaaacggta gtaaccatac tgcaataata actgtcctag ccctccgcct 323340
ccaacaaatc ctgcaagagt cgaacatgaa atgagatgaa caacaagaga cttaagagaa 323400
aatatgagct gtggatagct ttcaggtaga agtataccaa aaagaatatt ccttttagga 323460
attcccaaag caacggcaga ttcaagatag ttaagggctg aattacgaaa agcatcaacc 323520
actatagtaa caacaaaagg aatggctccg atagtgagag gaacaataga agccgtaggg 323580
cctaaagaag tccctacaat ccatcgcgtg atcggaaaaa gaataacaat aagaatcgca 323640
aatggaatcg cggtaagaaa acttagaatc attgagattg ttgcgtaaag gcttttctta 323700
ggattaagac tcttaggcga cgtgcagaaa agccccaaac ctagcatccc tccaatagca 323760
caagagaaga aaaaagcagt cgacaccata taaagtgtat tgactgtttc ttttaataaa 323820
atctgaataa gatcggattg cataatcttt ttctaataaa attctttaat aacgacgcct 323880
agctcaatta aaagctcttt tgctttttta cgttgctcaa cttccccttc caaaacaata 323940
attagaaatc ccattgggga ttttctaaat aaattaatgt tgccggaaag aatattgata 324000
gagaccagtc cagtctgaat caccttacta atgatccctt gtatagcgag ttctttggaa 324060
aaatttaatc taagaacttc ttccctgtct tctgcaaaat aacacgaact caaagcagca 324120
atatttatat cttcatggaa caattcattt gtaattgagt tctcagaatt taaaaaaagt 324180
tcttctgtag ttcctagttc ttcgacagca ccttgatgca ttaccaaaac atgagagcat 324240
atttttttta ctacatcgat ctcatgcgac actaaaacta aagtaattcc cctttcctga 324300
ttcaattgaa gcagcctttc aataatattt tcagtagatt taggatccaa agcagaggta 324360
atctcatcac aaagcacaac ttcaggttga cacacaattg ccctggcaat ggcaacttct 324420
tgtttctgac caccacttaa atttcctgga taggcgtcat gtctatgata gaggtttaaa 324480
aaattaagag tatcgtatac ctgttcctca acttcactct tagacatttc tgaatgatga 324540
atacgcaaag gataggctat gttttcaaat acggttttcg aagaaaacaa cccataattt 324600
tgagaaatat aggcaacttt tttagaaaaa ttacgacgtg aaaacttctg tgttggcaaa 324660
gagttatcaa acccagctac acttatagag ccagatgtcg gcatgtcaag aaagtctaaa 324720
caacgcaaaa gcgttgtttt cccagaaccg ctatgaccaa ctataccaaa aacctctcca 324780
ggatagacag aaaaggagac tttggagaga agaatatggt cgcctaactt cttactaaca 324840
tcttgaacgg aaatgatagg agaatgttgt tctgacacaa gccacgcctc tccttttatt 324900
tatgaatata aaatagaata caaagaatct tgaagattcg ttaaccatta aactataata 324960
ctattatatt tctgcaagca agtttactgc tcttaaaatt ttgttatcaa actatctant 325020
ataaacaaaa agagggttga atagatgtaa gttctatttc aaacgacaca acatttgatc 325080
tactatacaa caagatttca tcgaaagcat tttcaagacg aacttcttgg tgacgataca 325140
tcacaaaaaa taaatagctg cgattttaaa cagacgcatc aaaaatatat agaactaaga 325200
acaacttgaa attttaacat aataaaaaaa atactcctta agtcttttat aatacattaa 325260
aaaactaata cttaacttaa taaactcgtt gataaagttc cttgacaaat ccctgggtta 325320
accccaaact gagctctgaa taagcttaag gagacacacc ctcatgttga atattcatga 325380
tattctagga aatgatgacg aaaacctatt gtcatatcaa tgtaaacaca ttacaaaaga 325440
caaactaact cttccctctc atgattttgt agacaaggta tttggactct ctgataggaa 325500
taatcgtgtt ctcagatccc tacaaactat gttttctcat ggaaggttag caaattcagg 325560
ttatctatct atacttcctg tagaccaagg catagagcac tcggcaggag cctcttttgc 325620
tattaatcct atatattttg atccagaaaa cattgtgaag cttgccatag aatctggatg 325680

tagtgctgtg gcctctacct atggaacact gagcttactt tctaggaaat atgctcataa 325740
gattcccttt atgctaaagc taaaccacaa cgaactcctc tcctatccaa caaaatatca 325800
tcaaattttc tttactcaag tagaagcagc ttattcaatg ggcgccgttg ccgtaggagc 325860
tactgtttat ttcggttctg agacttctaa tgaagaaatt gtagcagttt ctaatgcatt 325920
tgctaaagct cgttccctag gtcttgcaac agtactttgg tgctatctac gcaatccagc 325980
ttttgttgct aatggagtag attatcatac ggcagcagat ctaacaggac aggctgatca 326040
tttaggcgct accctaggag ccgatattgt gaaacaaaag ctccccacat gccagggagg 326100
atttaaggcc atcaattttg ggaaaacaga cgaaagagtg tattctgaac tctcttcaaa 326160
tcatcccatt gatctttgcc gttatcaagt cttaaatagc tactgtggca aggtaggcct 326220
aattaactcg ggaggacctt caggaaaaaa tgatttacaa gaagcggcta gaacagcagt 326280
gatcaacaaa agagcagggg gaatgggtct cattcttgga agaaaagctt tccaacgtcc 326340
cctatccgaa ggcatccaat tattaaacct ggttcaagat atctatttag atcctaatat 326400
tacaatcgct taacttttca aagaaggtct ttatgcactc ccactcaaaa ccaacgaaac 326460
cgttgggaac attcacggtc ggcatgttat cacttgctgt agtgattagt ttgcgtaatc 326520
tcccgttaac agcaaaacat ggtctttcca ctctgttttt ttatggacta gcagtcatat 326580
gttttatgat tccgtatgct ctgatttctg ctgagcttgc ttctttcaag cctcagggaa 326640
tttatatttg ggcacgtgac gctctaggca aatggtgggg attctttgct atatggatgc 326700
aatggtttca caacatgacg tggtatcctg ccgtgttagc ttttatagcg agtaccattg 326760
tttataaaat caatccagaa ctcgctcaca acaaagtgta cattgcaacc gtgatccttg 326820
ctggtttttg gatacttaca ttttttaatt ttttaggaat tacttcttcc gcattattca 326880
gctctatttg tgtaatcata ggaacattaa ttccaggagt catcttagtt agtttggctc 326940
tcttttggat tttttctggc aatcccattg ctatttctct ttcttgggga aatcttcttc 327000
ctaatttcag taacgtatct tcacttgtac tactagctgg aatgttactt gcgttatgtg 327060
gtctagaggc taatgcgaac cttgcttctg atatggtaaa tcctagaaaa aattatccaa 327120
aggcagtctt cattggtgca atagcaacac tcactatttt agttctgggt tctttatcca 327180
tagcaatagt gattccgaaa gaagaaatta gtttagtctc tggactagta aaaacgttta 327240
ccttgttctt tgataaatat aacctctcct ggatgactgg aatcgttgta gtcatgacca 327300
ttgcaggatc gctaggcgaa cttaatgctt ggatgtttgc aggaacaaag gggcttttta 327360
tttccactca gaatgactgt cttccccgac tctttaagaa agtaaatagc aaaaatgttc 327420
caacgaactt aatgttattc caaggtattg ttgtgacaat attcacactt ttatttctat 327480
gccttgattc agcagacctg gtgtattgga ttttaactgc actgagcgtg cagatgtatc 327540
ttgcgatgta catctgtctg tttcttgcag gaccgatctt acgtatcaaa gaaccaaggg 327600
ctcaacgcct ctattctgta ccaggaaagt ttttgggaat ctgtacgatg tctatcttag 327660
gaattctctc ctgtgcgttt gctctttggg tgagcttcct gcctcctaga gaacttgctc 327720
agatatctga aggcagcaaa ataggatata ctacattcct gcttttagca tttagcttga 327780
attgcttaat tcctttcgga atctatttca cgcataaacg cttatctaaa aagagctaat 327840
ctaaaagcat ttttgggaaa agaaaagaaa gaagccttcc ttgttgtatg gcagcctgga 327900
aagcttctgg attcaaattt gctctcctgc aaaaagtttc aatctctcct tgacaaataa 327960
gtcctgtttg aaaatatcct aaatatatcg cgtgtaaaag cttatcccat aaatgtatag 328020
gcattaataa ctttaggtat ttacctacag atatgctgaa ttcctctgat tccagtagag 328080
cctctaagaa aatgaatggg ttttgtttca tcttctttct atcgatagcc attagaatct 328140
ctgccccctg tttgaaccgt cgagctcgca aaagctgtgg tatcgatgta atagacaacg 328200
caggaagaac aagttttttc aatccttcct tacaagcttc cttggtctta ttgaagtctt 328260
cttgctgatc ctcggcatct ttggcacaat cctcactatt aatactttgc caacagctgg 328320
gatctggctc agggtataga gcattcggta ctattaaatc caaccttaat gacagttggt 328380
ttagcaaggc gacttcgtcc ccttcaaaca gatgatgtaa aaatggagta atatatttct 328440
ttgttctaca aaattttgca cctcttcaga agaaaattgg ggacaaaatt gctgaataat 328500
cttaactgcc tgagacctat catgactaga tcgtaaacac aaccctgaaa gtaggagatt 328560
aatgcttatc aggcccaaag aacaagattc tatggtttca gctgtttcac tggaaatttc 328620
gatcccaagg gtcgagtgtt tcttcggatc tagcatactt ttcaatcaca taggatctaa 328680
agaaaataat acttctaata tctcttctcc tggagcttct aaaatttcta gtaaaacttc 328740
ataaagagga gcaattgagg aattctttaa atctttcttc gcaaatttta taagaagctt 328800
cactgggtta gggggtgatt ttcgaaagct aataatctta gcaacagcat tttttataag 328860
atcttggaag tatgagagtc ttcttcccta ctctcattag gccttccctg ctcatctaga 328920
aaatttgaat aaggaagcat cttcggccta cggaaatcat cagctggaaa gttcttaaga 328980
agttcatcaa ttgcagacac tgaatcttct tctcgaccta agagtaaatt aagatcctca 329040
tcagagaaaa atactgaagg actggttata gaaaagacct tagtcttttc tatagaagat 329100
tgctgataga ataaataggc aacgcaaaga acagagcaga ccgatagagg gatcagcatg 329160
cccaaaacaa acgtcccaac actaatgatg ccacaaaagt agagaacaga catcagtgtg 329220
gctatagcta agatagctaa aatcaaataa gtaatcggtc ttaaactact tttcaatgaa 329280
agaccatagc tacttgtgtt gttcactaca ggctcatcta tgactggtac ttggggagcg 329340
ggtatgttca tatctaaaaa tcaaataagt gttgtaataa aagatccaga atttttccag 329400
aacctatcgc ttgcgttatc tctttgaaag gaactcctaa atgttctgct ataatcttaa 329460
gttccttttt tctagctatg ccagaacaaa gcattcccat tagcatagga cgaaaaagcg 329520

cagcccaatt actcatcggc attgctttct ctaataaacc ttttaagaaa gtctgacact 329580
tgggattctc gtgtagcaat tctattaata aaaatgggtt ttgtaaaata acagattgtt 329640
gatttgagaa gaatgtaaat aaaagggaga agtccttgta gtggctagct acagaactag 329700
taaaattcca actggagggt gacggtatct tctcacaaaa tgctaggcgc tcactcaaag 329760
actttttaag aacgcttaga tctttaaagt catacatata ctcagaagag ttgattgccg 329820
gaacgtagtt gctcccatct ttagctgaag gaagttcctg agcatcgctt gggaaaagag 329880
ctcgaaactc cctttgcatc tcagaagact ggaaagataa aagaaactgc actatatttc 329940
ctgcttctaa atgcgccttt aactcgtcag taagaagatt agggtcgtat gcaacgattt 330000
tttcaataca cttttcctct aatgaccatc catagaacca aaaattaata gaaagtacca 330060
acttctgcat atgaggagtg agctttcgtt tttccgtttt taaagaattc aggtgcgcaa 330120
tcaactgttg ggtcaccata tttaaaggct ctggttttgt taaaagaaga atgagtaggt 330180
cagagcttgc atattcttct actatagatc ccagacgcct taaacaagcg gtcaaagagt 330240
actttacctt gcgactcaag aaacgaggaa gaaaaagatt cttaatttca gagatgactc 330300
ccctgagcaa gctaccagaa ctcttagagc tttcctccag agcgcgaggg aagtcaacaa 330360
ttaacgttgt gatctctttt tctagagact gcattcgctc tgctatcggc ttagaataga 330420
ctttattctg atgataaaat gtactttgga taacataggg cttaaaatca tcttctacta 330480
ttgatgggat tttagcctta aaagaaacgg tcttacgctc tttgggaaga ctttgacctt 330540
caaccggaac aggcttaaga ctttcagggt cttttatttc aaggtctttg gtggggtcta 330600
tcttttcttc gttagagggc tcaattaagg gttttcctcc cttatacaaa cacattaggg 330660
aaacaatggc catcatggag catactgaag cggcacaccc ccaaacaaga agacctagag 330720
gagcggcaag tacatgacac cctaccaaag cgaaaacaac acccagcaat aaaagaaccg 330780
aaatggctaa aagacagaga atagtcacag gagacaatga gaaggactga ttcttcctgt 330840
aaattaactc gtgacttgct gtggtctctt tatttgcagt gaaaaaaaat aaattaaaca 330900
attcttttta aaaaagttag ttaagcaaaa tcgacgaaaa tattatacaa aactaattaa 330960
aaaagagaaa gtttatacct ccggctatca gaaaaaagat tatcattgac ataatatcat 331020
ttagggctgt gacaatgggc ccggaggcta aagccgggtc tactcctagc ttagcaaaga 331080
aaaatgggga taaaacccct agagtcgtag ctgtgagaga agctcccaga accccagtag 331140
ccaccgtaac gcctagttga atacctcctc cggagaagat atttaagcct agaaagccca 331200
ttaaatagac aacaagacca catagaatcc ctaaaacgac tcctgtaagc agcccgatac 331260
tcatttcttt aaaaatggtc tctcgacgcc gtccgaaaga aagcgtccct gtagccatac 331320
tccgcactaa aatagtgcta cattgaacac ctacatttcc tgacattccg ttaatcaaag 331380
gaataaagaa gataatcaga gctaaaaggg cgggggaaat tttttggaaa tatgccatga 331440
cggaggcgct aatcaaacct gcaaatagag tcaccaaaag ccagggagct cttagtaaaa 331500
atctttgcac aacatgacag gtctgataac ccacatcttc cgtagtacct gccatcctag 331560
ctatagtctc atcagcgatg tcctcgatag cttcaacaac atcttcataa gtaatagccc 331620
caatcaagaa attttcctca tcaacaaccg gaagagcggc aattttatac ctctctacaa 331680
gatcgacgac ctcttcacgg gtagcatcag gaagcacctt atgttcaatc tgattcataa 331740
tttgctttaa agacatctcg ggagggttaa tgatcaaact tctatcggta acgacacctt 331800
gcaactcgcc tttaaaatcc aaaacaaaga ccaaacgagt taaatcaatc ccaggattac 331860
ttcgaataca agcagaaacg tctttcaccg ttgtttccat caaaaaggca aaaaactcat 331920
tggtcatcag ccttcctgca gtatttcttc cgtgtttctg caaatcacga atttttagtg 331980
cttttttaga atctataagc tcaagaattc tccgatacct acgatcgggg atatcatcta 332040
aaacccatac cgcttcatct ggaggcatct gttcaattaa agcgcagacc tcagaatccg 332100
acaacctacg aaaaatcgcc caccgcgaag cagaatctgt attgatgata aacgcaactt 332160
tagcggtaat acaagagagg ttcttatata aaatagcacg cgattccgag ggaagacagg 332220
aaacagcata agcaaggtct atcgggttat actcaatcac aattttagaa agatcatgag 332280
aatgtatatc tgtagaaaga caagtaaatg ctttttctaa cttaaaactc agctcgtcat 332340
ctagatgact cgtcctggaa tccatcaagt tcccagaact aaaagctgta tctagtttct 332400
cttcatttcg gttttgttcc ccaaccataa tcttcccttt tgattctcta caacttcacg 332460
aaatgcatgg atttccctga ggttagctaa ataaggatcg tttaaccact tggcttctgt 332520
aatacgaacg ccatactgcg cagagcgaat gaccattccc cacgccttgt tcacatcttt 332580
gatcactgca taaaactttc ccaaagtata atacgcctct ggacacccca aagaagttgc 332640
cttctctaaa tatgttctag caagcttacc taaacacaca ttcttatttt tccaaaatag 332700
gaataagtat actttcccta acagtagtaa gaaattctca tcttcaagat aaacttcaac 332760
tagaggttct aaaatctccc gtgcttcttc agccgggtct gtatcttgta gtaatcttcc 332820
tttccccaac aaaaccgcag caagaatcaa tttaacccta gaggaggata aagtaagatc 332880
taccttagtt aaaagtgtgt atgcctcatc ataatgaaac aaactttgtt gcagttcggc 332940
aagcaaataa tgagattgtc cccaaagttc taggatttcc agtcgaccac ttaaatccca 333000
cgctctctga taatgtaaca gtgactctga taagaaaatt tctttataag cttcatcgat 333060
tgtggcttct gccaagcatt ttaatgctaa tcctcgatca ctccaaaata aaaaagcctc 333120
tggacgcaaa gaacataacc tacttgctac atctacagct tttcgtagca atctagcact 333180
ttttttcttg atcccccaag aaaaatatgc atcaaataat ttttgccaca ttcctgtcgc 333240
atccaaatcc cattctaaac aagactgaaa acaagaaata gcagaagcaa aatgcgagtc 333300
ctcattgaaa taaagtgcgg agcaaagctg cacaacacca agagcatgga ctaacgcact 333360

attgccagga aacatcctca tagcagaaat cagcctgtgc cgactatctt taaataaatt 333420
tggttcttct aagtataacc ctaaaatagc aatccctgta gctagtaaac ctgaaagcgc 333480
aatgggatca ttcgtctttt tttgtaaaga agccagtttt tctaaaccaa cttcaatata 333540
cttcatatta ctattcaacc atcccgaacg aatgaggagc tcaccccaaa ccatccatag 333600
ccctgaaaga ttaggaaacg cctgaacggt ttggtaaagg atgttcatag cctgatcaaa 333660
atgctctttt ttataagtca gatcgaataa cttcacagaa gcaagagcat agctataacg 333720
ataattttga taggcaaggg tgtcaccatc cctgctgaaa ctcagaaaaa tggctttaga 333780
aaaatgctcc atccctcttt ctatgagcaa agactttccc tgtctagttc ctaaaactac 333840
caggccctta gcatagtcag catggatatg agcattatta ggtgataaat ctagagctaa 333900
ggccagacat ttcatgccgc aggccaaatc actcaattct aagtgcttgt ggtataattc 333960
tataaaacta cggcccacag cctgatacac agaagcactt gctactaaag aaacctcttc 334020
ttgcttagct cctatacgga agtgctccca tccaaaacga ttcttcagtt gaggatgcat 334080
tttcaaaagc tgttctatta aacggaaatt atctacaact tcagagagaa atttcatctc 334140
tcccgtctga gaataaagtt caaaattgat acaggttctt gcagagcaca aggaaagcct 334200
atcagaagca ctaattccta atttttcaaa aagaaaatct atataggtca aaacatcaat 334260
gaaaatcgac atacttttat gttttaaacc ataagagacc ccatagaaaa tcactctctt 334320
tacaacttta gggtgattgt gagtcatcat taatagagac cgagacaaag ctaagtagcc 334380
ctcgctcggt tcgtcttgtt gtgccaataa ttcaatttca gagaaaagga aatcaaccac 334440
atcttctgga caactgaact catcctgcaa gcccaacata tttaaggctc ttttagagga 334500
aataaaaggt aatggtgatc taatcataca ccttctttaa ttatcatagc taaatcacct 334560
tgaaattcat attagaaggt gttcttaccc actaataaaa actagttttt gaaaagaatt 334620
ctaaaaacct tcttttaatc ttttcaaaaa caagaacatc tatttcttat tgacaaagca 334680
cttcatcatt tcatagcttc aaaactgaga tcagtctatc ctatttcatt tttcaattaa 334740
aaatattttc tatctctata gtgacagtta tcccctttag aaaaggctgc ccatcgtctt 334800
taaaacaaag agcctgtcac tataaatctt gtttaaaact cgcaaaagac tctaggttaa 334860
attcttttga tacttaaaca ataaaatttt tagcactgtc attccccgaa tcagggatca 334920
cacttatatg tggatatcta ccactagtgt cgagccctcg tctctcaggg agctggattg 334980
agggatgggc tccagaaaca atttctcctt tatcaaaaag gaagacagat tgtggctggc 335040
aacacaacat caaataaaaa gcatgctgtc ctaaaccttt acgcccccca cctccttcaa 335100
atcggaataa ggaatgagcc tcattggcta aagatacaga tcgcgcaagt atcacgttat 335160
cattccaatc tctttctaca ctccccatca atacacggcc tgcaagaggg tccaacaaac 335220
tataaaaagc tcctatcata catgcagaag cataaaaagg agctttcaaa aaagcagcaa 335280
gagaccgccc catctctcga gcaacatctt tataaataaa tctatcgtct tcgggaagat 335340
gtttacagaa aaaatgctcg taaatcattc ggaaaatgat ataaaatgga accactaaaa 335400
agcgtatcaa attgtaaatc attctaagca aagctacaaa aggcatcgca cacggaattc 335460
caaaacaagg gaatgcgcga aataataatg gtggacattg ataaaccgca gctattccag 335520
aaccgtcagc actcagcgtc gatatatcta gataagagca agcactcacg tgcttttcat 335580
cgaatttttc caaagtatgg ctggaggata taggaaccca acaccgtctg ctaaaaggtg 335640
gaatacacac ccctgaaatt acatctactt tcttccccag acgatgcaga atgtacatgc 335700
ctccagtaag caaaattgtt gggataagaa ttgctgcagt aaccaaaggc caggaagtaa 335760
ttacaagtgt accttgtatg ctcgctaaca ttatactgaa tagaatcaac acacccacga 335820
cagcaagagc gatcaccgtg agcctcacaa aaaggttcgc atcatattta ttcactttac 335880
aaacagaaac ggacagggta tcagatctat gctttcgcaa tgctgaacga aaatagcttt 335940
gccatccttg tttggggaaa gatgccttag gactaattgg tcttaaactt tcgtttgtag 336000
atgtaagatt aaaacaagac ataattacat tataaaattt tgctcggcat tcaaacccaa 336060
agaaaagaaa ttcacgaatt gttatactta tttaccaatt aaaattttaa tcgacttatg 336120
aaaaaacaac gctctcatta tacaaaaaat aatcttttat tacttctttc tatattggtt 336180
ggcctaggtt taggaagtgt gcaatcccca tggattgttt attctgccga atgcatagca 336240
aatacttttc taaaattctt acgtttactt agcattcctt tggtattctg cgctctcggc 336300
tccaccatta cttctataca aaatttcaat actatggtga ctctaggaaa aagaatttta 336360
tattataccc tgctgacaac agttatcgct gcttccattg gacttctgct gttcttttta 336420
ctccgtcccc aaatgataac tcaagatgcc ctagccacaa ctacaaagtg taatccccta 336480
ggatacttgg atgtccttag cgacaccctc ccagaaaata tctttaagcc attcctccaa 336540
ggaaatgtca tttcagccgc ttgcctagca gtcctgctag gaaccgcgtc cctatttctt 336600
caagaaaaag aaaaacattt cgtaaatcaa tttttaattc catttttttc tatctttctc 336660
aacctggcta gaggcggtct aaaacttctc ccaatagcaa tgctcgggtt ctctgtcatc 336720
ttgttcaaag aattgaaaga tcaaagcaac cttacaatgt ttgccgagta tctgctttgc 336780
gttataggag cgaacctcgc ccaaggtttt attgttctcc ccatactact taaaataaac 336840
aaagtctctc ctttaaaagt cgcaaaagca atgtctcctg cactagtgac agcttttttc 336900
tcaaaatcat cggcagcaac attacccctc accatggaac ttgctgaaga tgatttaaaa 336960
ataaataaga atctttctcg atttagcttc ccgctatgct ctgtcattaa catgaacggg 337020
tgcgctgctt ttattctaat tactgttttg tttgttgcga cttccaatgg tatgatcatt 337080
tcgcctctaa tgtctttagg atggattttt attgcgactc tcgcagctat aggaaatgcg 337140
ggcgtaccca tgggatgcta ctttcttact ctttctcttc tcacatctat gaatgttcct 337200

ttatctatat taggtctcat cttacctttt tatactgtaa tagatatgat agaaacttct 337260
cttaatgttt ggtctgattg ctgcgtagtc agtttagcaa actaacaact ctcaaaaaaa 337320
ctctcactat aaaggagtgc tttaaccatg aataaaaaac acgccagttt ttcatctcga 337380
ctaggattta tattctctat gatagggatt gccgttgggg caggaaacat ctggcgcttc 337440
ccgagagttg ctgctcagaa cggaggtggt gcattcctaa ttctctggct atgtttttta 337500
tttttatggt ccataccctt aattattata gaactctcta ttgggaaact aaccaagaaa 337560
gctcctatag gggctttaat taaaactgca gggaaaaaat ttgcttgggc tgggggcttc 337620
attacccttg ttaccacttg tatactcgcc tactactcta caattgtagg ttggggatta 337680
agctactttt attatgcagt ttcaggaaaa attcacctgg gaaatgactt tgcaaaatta 337740
tggacatccc actatcagag ttctatccct ctctgggcac acctcacctc attaggatta 337800
gcctatcttg tcattcgtaa aggcattgtc catgggattg aaaaatgtaa taaaatcctg 337860
atccccgcat tctttctatg taccatcgct ctacttttac gagcagtgac tcttccagga 337920
gccgttcaag gaatcaaaca actctttagt tgtgataaaa gttgcttttc aaactacaaa 337980
gtatggatag aagctcttac gcaaaatgct tgggatacgg gagccggatg gggcctactg 338040
cttgtgtatg cgggctttgc ctcaaaaaaa acgggagtag tgagcaatgg agctctaaca 338100
gctatatgta ataaccttgt ttccttaatc atgggggatc attatctttt ccacatgtgc 338160
ttctttagac attttaggaa ccacgcagct acaagatgga gcaggagcnt caagcatagg 338220
gattaccttt atctacctac cagagttatt tacccgtttg cctggaggaa tttatctaac 338280
caccctgttt agctctattt tcttcctagc attttctatg gcagcgcttt cttccatgat 338340
ttccatgctt ttccttctct cacagactct tgcagaattt ggaatcaagc cctacatttc 338400
tgaaaccctg gcaacaatca ttgcctttgt cctagggatc ccttctgcac ttagtctcac 338460
atttttctct aaccaagata ccgtttgggg agttgcactt attgtaaatg gcttgatctt 338520
tatttacgca gctttagtct atggcttccc taaactaaag aaagaagtca ttaacgctgc 338580
tcctggagat ctccgactca acaaagcctt tgattatata atcaaatatt tactcctaat 338640
tgagggaatt cttcttttag gatggtattt ctatgaagga ctcttccctg aaaatggtca 338700
gtggtggaat cctatttctc tctatagtct gggcagttta gtcctgcagt ggtctttagg 338760
actcataatc ttatggaagt tcaataaaca actttattta agatttcccc gttacaatca 338820
cgaaattcta taaatccaat cgataattct actctgaata ttcagggtag aatcacagca 338880
ttttggaaag aaaaaaatat ttaaagattg aaaataacgt ttttcctggc ctagtatgtt 338940
tggcattaat ctatttttag ataggaaaaa catatgtcag cacctatacc aaccccacaa 339000
gaattgtcag accaaatcac ctgcttaaat gtccaatacc aacaggtatc cgaactagca 339060
agagaaaaca aaggagatat tgaaggctta aaaacactga ctgcggctct aaccgctgat 339120
gctggcatac agccttcagc tgatgaaatt tactcattgc aaacagccgc agccctaatc 339180
ttatctgctt ccgaaaaacc tggaagcggt ccctctggaa gtactgaagg atctgttact 339240
gttcaatctc catgcaaatt caaaaaagta ctagcagtcg tattaacgat aattgcttta 339300
attgcaattg ctgtacttat agcttgtatt attgctgctt gcggaggttt ccctctactt 339360
ctatcagctc ttaacctata taccataggt gcttgcgtat cactaccaat tatagcttca 339420
acctcggttg cgcttatttg cttgtgcaca tttgtagcaa actctctaat taaacccgta 339480
attactgtcc gtacaacaag ataataagta aaaaacacaa aaaatagtga ttttatgacc 339540
tcaccgatcc cctttcagtc tagtggcgat gcctctttcc ttgccgagca gccacagcaa 339600
ctcccgtcta cttctgaatc tcagctagta actcaattgc taaccatgat gaagcatact 339660
caagcattat ccgaaacggt tcttcaacaa caacgcgatc gattacnaac cgcatctatt 339720
atccttcaag taggaggagc tcctacagga ggagcgggtg cgccttttca accaggaccg 339780
gcagatgatc atcatcatcc cataccgccg cctgttgtac cagctcaaat agaaacagaa 339840
atcaccacta taagatccga gttacagctc atgcgatcta ctctacaaca aagcacaaaa 339900
ggagctcgta caggagttct agtggttact gcaatcttaa tgacgatctc cttattggct 339960
attattatca taatactagc tgtgcttgga tttacgggcg tcttgcctca agtagcttta 340020
ttgatgcagg gtgaaacaaa tctgatttgg gctatggtga gcggttctat tatttgcttt 340080
attgcgctaa ttggaactct aggattaatt ttaacaaata agaacacgcc tctaccggct 340140
tcttaaaaaa ataaattgaa ttagaataag taatagtaat tttcttcata cctcccttgc 340200
aattaatcac tttgttctta taaaatgtct tcttttgctt gggtaggtat ggagtatgca 340260
gagagttttg cgactgctgt ttaacctaca tcatggggaa gaaaaaaggg ctttcctttt 340320
ttttctcttg ggattggtct gggggatagg ttgttacggc actctctctc tagctgaagg 340380
cttattcatt gaaaaattag gatcggcaga attaccaaaa atttatttag gttcttctct 340440
gatcctttgc gttctttcat ccctaattct ttacaatctt tttaaaaaac acatctcagc 340500
aacagctctt ttcttaattc ctgtttcttt atctatcctt tgtaattttt atcttattct 340560
ctcttctatc tttgctatcg atcccccccg gtctcctctg tttttctatc ggattgtaat 340620
ttggagttta acgattctct cttacacgag tttttgggga tttgtagatc aattttttaa 340680
tttacaagat ggaaaacgac acttctgtat ttttaatgct atcatcttct taggagatgc 340740
tatcggcagc ggaatcatag ctagcctggt acacacaata ggaatccagg gcatcctgat 340800
tttatttaca gcagccctgg tcctgacatt ccctatcgta ttctatgttt caaaatctct 340860
aaagtcgctt tccgatgacc atgacctttt catagataca ggccacccac cacccttatc 340920
aaaagcattg aaactctgtt tttatgataa atatactttt tatctgcttt gcttttattt 340980
tctcatgcaa ttgctagcga ttgctacaga gtttaactat ttaaaaatct ttgaaattca 341040

atttgcctct aaggaagaat tcgaactcgt cgcacacata ggaaagtgtt ccctgtggat 341100
ttctttagga aatatgtgct ttgctctttt cgcctacagt agaatagtaa agcgtcttgg 341160
agtcaataat atcattttat ttgctccgct atgtttctta agcctctttc tattttggac 341220
ctttaaaaca accctaagca ttgctgtcct tgctatggta gtacgtgaag gcgttaccta 341280
cgctcttgat gacaacaacc tccaactact catctatgga gtccccaaca aaatccgaaa 341340
ccaaattcgc atcgtagtgg aatcctttat agaacctatc gggatgttgg tctggtcctt 341400
agtctgtttc ttgtcttctc aacaatatgt gttctgccta atcatctcac taatcgccac 341460
tattctggtt tgcctcgtac gctcttatta tgcgaaggcg attctcaaaa atctatctgc 341520
acaagcccta caacttaccc gctctatgca agattggatc aaatctatga cagttaaaca 341580
aaagagacaa gtcgaactct tcttacttgc tcatcttaaa caccccagtg agcgtcatca 341640
aacctttgct tttcaacatc tcttaaatct agcaagccgc agtgtccttc caagcctcct 341700
tgcccatatg aacaagctca gcctccctaa taaactcaag actatagaaa tggtaaaatc 341760
tagcttatgg gccaaagatt ttctaacctt agagctcctg aaacgttgga caagtatttt 341820
cccccatcct gccatcgcat cagcaataca tctttatttt gcagaacacg atctcctaca 341880
tatcactcat attgctgaag acctctatga tactgttggt gatagacttc ttgccgcaat 341940
tcttacagta agaagacagg aagcttatgg gccctatcga gaccttgcag acaagcgcct 342000
gaaagaacta ctcaactcgg atcaacctga agatatagtc atggggttga ccatactgaa 342060
attagaaaag aatccacaga acttcccaat tcttttagac ttcttgaaca ccaaaaacga 342120
agatatctta attgtcacct gcaaagccct acacacttct gttagagcta atcataaacc 342180
ttattgcccc gaacttctga aaagactacg acaatgctcg cataatgatg aagcaagtca 342240
atatctatta aaaacaatta gcattgcttt agatatctca ttcgtaaaag acttactgat 342300
gacaacatca caactaaaaa acacctctag aaaatatgct gaggctatga ttggagagtt 342360
ggataaagaa gtcgccccag catttctcca agtcctcacc gatgagggaa cacacaatcg 342420
ttgtcgtatc cttgccgcca aagccctctg taaaatcgat aattggctgc tgaaaaaaca 342480
cgcgtataaa attgtgaagt ctaaagcaag taaggctctg ttctattcct atcacggcca 342540
ttacattcaa aagaaatacc ccacatacaa cctcagcttg ctggcaaaca cattaaattc 342600
taattattat gcagaagtaa acttcatgct ctctctccta gggattcttg gttccatgga 342660
gcactctggt gtactgattc gagcattaac tagtaaaaac caaaaaatca aagcacaagc 342720
actagaatct ttagaaaaaa actgcgatag ccacttattc tctttactag aaccctttgt 342780
taatcaacca ggcatgtgct atagcgaaaa atactacttc aaatgtggtg tgattcctct 342840
aactcttaaa gaacttttaa atatgatgga aaactcccca tcatctttaa acaaactaac 342900
agcacagcaa ctcaaagaag aactttccta ttgcgatcca gactttccaa tctgtaaata 342960
caatctataa ccaagaacat gaagacttca ggacagagga atcagaaacc ctaatatctt 343020
tcttatctat ctaaacttca atgattccaa tttagatatc cacagaaaaa ccttgggaaa 343080
ctgaagattt aaaaaaacca agattgattt ataagtttcg actgcaagaa aaatcaattt 343140
caacggttcc ttcttgaaac tcttctatct taaaggtaag atctacgtat caagtattac 343200
atctgtggtg atattaaaat ctttgatttt aacaacactg attcttctta aaccattgtg 343260
gaggcttttc tttaaactac ctgcatcgta gttattgctt ctatccttcc caaaataata 343320
caatccatct caaggatacg tctctgtgcc tttacttttg tcaaaaacta aaactatggt 343380
gtgaataagg aactttttta tgaatttgat cgatcgcgcc tttctactaa aaaaaacgat 343440
tatattccaa tctttagaca tggaccttct tttaacaatt gccgataaaa ctgaaacgat 343500
aatatttaag cctggcagca atgtattctc tataggacaa cctggattca gcttttatat 343560
cattgtagaa ggatacatta cgatctctaa agaaaaacta gagtctcctc taaatttaaa 343620
acctttagat tgttttggag aggaaagctt attcaataat aagcccaggg aatacaatgc 343680
ttctgcaaat acacaagtcc gcatgctagt tcttagcaaa ggacaaattc taaacattgt 343740
ggaagagtgc ccatccgtag ctttatcttt tttagagctc tatgctaaac aaatcaagtt 343800
cagagaacct taaaaagaca aaataaatct ttctagatga gctactctat agaaaaaaag 343860
gcacgccgta atcactattg aaaaacaata gctttaaaaa agattacgac gcacccagaa 343920
tataagaaag tttattgttc agcttgacgt ttcttaatat aagtaaatac atccccgaca 343980
gtacgaagct tctcagcatc ttcttctgaa atttcaaaag caaatttttc ttctaaagtc 344040
ataatcaatt ctgttaaatc taaactatca gcattcaagt cttcaataaa agaagagttc 344100
tcattaactt cttttggatc cactcctaac tgctcaacaa taattgctat tacatcatct 344160
tctaaactca ttgcttatat ccttttacta tttaaactat acaatacttc actattccct 344220
agggacaaag agaaagagaa ttagtatcgc aaaaaatcat tttttattcg acggttactt 344280
aattagttct cgaagagaaa ttttcaaatc cctttcttct attgtcttag taagtcaatc 344340
ccccatcaac aaccagtgtc tgcgcggtca tatagctcga taactgcgag gctaaaaaca 344400
acgccacacg agcaacatct tctggagtgc cagccctacc taaagggatc gacttaagcc 344460
actcagcttt taaattgtca ttcaacacgc ttgtcatgtc tgtttcaata aagcctggag 344520
caaggcagtt gacacgaata tttcttgcag ctacttcctt agctaaagat tttgtgaaag 344580
caataatccc agctttagca gcagcatagt tggtctggcc cgcactaccg atcttagcaa 344640
caatagaagc cacatttata atagatcctg aacgcgcctt aatcatatgg cgaatcactg 344700
aggaacatgt ataatacaag gaagtcaagt tggtgctaat caccgattgc cagtcgtcct 344760
cagacatacg catcaacaaa ttatccctgg taatgcctgc attatttacc aaaatatcta 344820
ttttgttgtg cttatctaaa aatttctgca cgcaatcttt cactccacca ttatgactca 344880

catccacacg agcaaaagaa acttcgccac ccaagcctgt taaactttct ataacagcct 344940
gacctcgctc ctcattcaat ccccaaattt ctacatctgc tccgttctca agaaaaagct 345000
taactatccc gagtccaatt cctcgagatc ctccagttac tataactttt ttgcctacta 345060
atgttatatc catacaaatc atacctctga taggaatttt tcaatctgag caaaagtacc 345120
aagacttgta atcggtttag aaatccctat agagcgattt aaaccagcca aaacttttcc 345180
tggacctaat tctaaaaact catccacctc tgattcgata tggtaacaac tctgatacca 345240
taacgtaggt gatgtcattt gccgagctaa acactctcgc atttcttcag tatttactaa 345300
agattttcct accacgtgtg acactaaggg aaggctagaa tctttcatgc ataaagcata 345360
aatgtctgga gctaagccat cttgagcaac ttgcattaaa ggagtatgaa atgctccaga 345420
cacctttaaa cgaactgctt ttttacatcc taaatcacga aataactcaa tcgcttggtc 345480
tactttttct gctattccag ccactacaag ctgtttgggt gcattataat tagcaatcca 345540
aattccttga ccaagacttg ttatattttc ctctataact tcagagggaa gccctaataa 345600
agccgccata gcccctgggc tctgattaca agcttcattc attaactgac cacgctttct 345660
aacaagctca aggccgtcga gcacggagat tctatcggaa gcaactaaag cagtatactc 345720
ccctaaactt aatccagaga ctaaagaagg ctgaatagaa gaacgctggg atagaacctt 345780
taccacagcc atgctatgaa gataaatagc tagctgacta tgtactgttt ccatcaaaag 345840
atcctcagga ccttcaaaca taattgaagt cagagaaaat cctaaccttt cattagcaaa 345900
atcaaaaagc tctctaacct caggatactc catatatagg tcttgtccca tacctacata 345960
ttggctccct tgtcctggga acaaaaaagc ataacgtttt ttcatgaaat tatcgtcctt 346020
attagacctg ctttaaaact actgcgcccc aagacaaacc gcccccaaag gcaactaaaa 346080
gtaaataatc atcaagctta atggattctg tatgaactaa ttcatccaaa gcaatgccca 346140
cagacgaggc cgcagtattt ccatacttat gtacactctt aaacactcta gactcatcaa 346200
tctcaaaacg cttcgctaaa gcatctatta ttctttccat ttagcttgat gaggtacaaa 346260
ccaatctata tcctcttcct gaatgcctgc cagggctatc gaatgtttag ctgccgtttc 346320
catacgtctc acagcatgct taaaaacttc ttttccctcc agagcaataa aatgtttgcc 346380
tgattgtaaa gtctctttag aagcaggaca acgactacct ccagcaggaa ggcttaatac 346440
tctcctagct taccatctgc gcctaaagac aacctattaa tctctaaaga tcctggccga 346500
ctctccccta tgacacaagc agctcctcca tctccaaaca acacacaggt attccgatct 346560
gtataatcta caaaagaaga caacttatca gcagcaatta acaatacatg gttatatgta 346620
cctgattcta cataagcctt agctacagac aaaccataca aatacccagt acaagccgcc 346680
tggcaatcaa atgtagggac atcctcaatg cctaaatgtg cttgagcaag actccgcttg 346740
atgggaaaat ataatctggt gctgctgtcg agaaaatgat acagtcaatc tgatccttgc 346800
ttaaacccgc atttgctata gctttctctg cagcgatggc tcccataaga gaagtgtact 346860
cctgaggtcc agcaatacga cgctctttga tccccgttct ggtcacgatc cactcatcag 346920
aggtatctac cattttttct aaatctgcgt ttgaaagaac tttctcaggc aaataggaac 346980
ccgttgccca aattgctgct tttttgtttt tattcacaga gaaccacatg taaagaaaaa 347040
agctagtata ctattcagag agtttatact ctaaccttaa tttaggcccc agacccgaat 347100
gtaaaaataa aattcacatc actatgacaa gatatccaga ttacttatct aaattaattt 347160
tctttttacg aaaacttcca ggaattggat ttaaaacagc agaaaaactt gcttttgaac 347220
tcatctcttg ggacagcgaa caattaaaaa tattaggtaa cgcttttcat aatgttgcta 347280
gtgagcgtag tcactgtccc ctatgtttta ctctcaaaga atctaaagag gcagactgtc 347340
acttttgtag agaagaaaga gataaccaaa gtctatgtat tgtcgcttct ccaaaagatg 347400
ttttctttct agaacgttct aaagtattca agggacgtta tcatgttctt ggttcactct 347460
tatcgcccat tacagggaaa catatagaaa acgagcgtct ctccatttta aaatcacgca 347520
tagaaacgct atgcccaaaa gaaattatcc tagccattga tgcaacctta gaaggagatg 347580
ctactgccct ttttctaaaa caagaattac aacatttctc tgtaaatatt tcccgtctag 347640
ctttaggtct tcctataggc ttatcttttg attatgtaga ttcagggaca ctggcaagag 347700
ctttttctgg acgacactcc tattagcagc tctcttatcc taaaagtaaa caagttctct 347760
tctatgctag tctttcttac tttcccctag gacacagtga attatcgaat ttgccttaaa 347820
accatgtaat cagtagagtt atttgagcga caaggtttat taagcttttt aaggaagcaa 347880
aaaacccgcc gaaatattca ggctctagtc tgagcacagt aataatcccg gtgttaggac 347940
gggaaaaaat caaacacaat ttaaatttct ttagaaatac aaaaataatc taagtagtaa 348000
ggtcttgtta aaaaacttct ttgtccctat ttttgtgctt acctcattat ctgataaaca 348060
agctttctat ctagggaaga ctcttgggaa tgctcatcat gcgaaataaa gttatcttgc 348120
aaatatctat tctagcgtta atccaaaccc ctttaacttt attttctact gaaaaagtta 348180
aagaaggcca tgtggtggta gactctatca caatcataac ggaaggagaa aatgcttcaa 348240
ataaacatcc cttacccaaa ttaaagacca gaagtggggc tcttttttct caattagatt 348300
ttgatgaaga cttgagaatt ctagctaaag aatacgactc tgttgagcct aaagtagaat 348360
tttctgaagg gaaaactaac atagcccttc acctaatagc taaaccctca attcgaaata 348420
ttcatatctc aggaaatcaa gtcgttcctg aacataaaat tcttaaaacc ctacaaattt 348480
accgtaatga tctctttgaa cgagaaaaat ttcttaaggg tcttgatgat ctaagaacgt 348540
attatctcaa gcgaggatat ttcgcatcca gtgtagacta cagtctggaa cacaatcaag 348600
aaaaaggtca catcgatgtt ttaattaaaa tcaatgaagg tccttgcggg aaaattaaac 348660
agcttacgtt ctcaggaatc tctcgatcag aaaaatcaga tatccaagaa tttattcaaa 348720

ccaagcagca ctctacaact acaagttggt ttactggagc tggactctat cacccagata 348780
ttgttgaaca agatagcttg gcaattacga attacctaca taataacggg tacgctgatg 348840
ctatagtcaa ctctcactat gaccttgacg acaaagggaa tattcttctt tacatggata 348900
ttgatcgagg gtcgcgatat accttaggac acgtccatat ccaagggttt gaggttttgc 348960
caaaacgcct tatagaaaag caatcccaag tcggccccaa tgatctttat tgccccgata 349020
aaatatggga tggggctcat aagatcaaac aaacttatgc aaagtatggc tacatcaata 349080
ccaatgtaga cgttctcttc atccctcacg caacccgccc tatttatgat gtaacttatg 349140
aggtaagtga agggtctcct tataaagttg ggttaattaa aattactggg aatacccata 349200
caaaatctga cgttatttta cacgaaacca gtctcttccc aggagataca ttcaatcgct 349260
taaagctaga agatactgag caacgtttaa gaaatacagg ctacttccaa agcgttagtg 349320
tctatacagt tcgttctcaa cttgatccta tgggcaatgc ggatcaatac cgagatattt 349380
ttgtagaagt caaagaaaca acaacaggaa acttaggctt attcttagga tttagttctc 349440
ttgacaatct ttttggagga attgaactat ctgaaagtaa ttttgatcta tttggagcta 349500
gaaatatatt ttctaaaggt tttcgttgtc taagaggcgg tggagaacat ctattcttaa 349560
aagccaactt cggggacaaa gtcacagact atactttgaa gtggaccaaa cctcattttc 349620
taaacactcc ttggatttta ggaattgaat tagataaatc aattaacaga gcattatcta 349680
aagattatgc tgtccaaacc tatggcggga acgtcagcac aacgtatatc ttgaacgaac 349740
acctgaaata cggtctattt tatcgaggaa gtcaaacgag tttacatgaa aaacgtaagt 349800
tcctcctagg gccaaatata gacagcaata aaggatttgt ctctgctgca ggtgtcaact 349860
tgaattacga ttctgtagat agtcctagaa ctccaactac agggattcgc gggggggtga 349920
cttttgaggt ttctggtttg ggaggaactt atcattttac aaaactctct ttaaacagct 349980
ctatctatag aaaacttacg cgtaaaggta ttttgaaaat caaaggggaa gctcaattta 350040
ttaaacccta tagcaatact acagctgaag gagttcctgt cagtgagcgc ttcttcctag 350100
gtggagagac tacagttcgg ggatataaat cctttattat cggtccaaaa tactctgcta 350160
cagaacctca gggaggactc tcttcgctcc ttatttcaga agagtttcaa taccctctca 350220
tcagacaacc taatattagt gcctttgtat tcttagactc aggttttgtc ggtttacaag 350280
agtataagat ttcgttaaaa gatctacgta gtagtgctgg atttggtctg cgcttcgatg 350340
taatgaataa tgttcctgtt atgttaggat ttggttggcc cttccgtcca accgagactt 350400
tgaatggaga aaaaattgat gtatctcagc gattcttctt tgctttaggg ggcatgttct 350460
aagatataaa ttaaggactt atcgaaggaa atctttgttg ttttcagaaa aggcttttgg 350520
tacccttttt ctatacccaa gtttagtaca ggtaattatg aaaaaattat tattttctac 350580
atttcttctt gttttaggat caacaagcgc agctcaatgc aaatttaggc tatgttaatt 350640
taaagcgatg tcttgaagaa tccgatctag gtaaaaagga aactgaagaa ttggaagcta 350700
ngaaacagca gtttgtaaaa aatgctgaga aaatagaaga agaactcact tctatttata 350760
ataagttgca agatgaagat tacatggaaa gcctatcgga ttctgcctct gaagagttgc 350820
gaaagaaatt cgaagatctt tcaggagagt acaatgcgta ccagtctcag tactatcaat 350880
ctatcaatca aagtaatgta aaacgcattc aaaaactcat tcaagaagta aaaatagctg 350940
cagaatcagt gcggtccaaa gaaaaactag aagctatcct taatgaagaa gctgtcttag 351000
caatagcacc tgggactgat aaaacaaccg aaattattgc tattcttaac gaatctttca 351060
aaaaacaaaa ctagtccaag tttaaggagt tttctatgtc cgaagcacca gtctacactc 351120
ttaaacagtt agctgagcta ctacaagtcg aagttcaagg aaatatagaa actcctattt 351180
caggtgttga agatattagt caggcgcaac ctcaccatat tgctttttta gataatgaga 351240
aatactctag ctttctaaaa aacaccaaag ctggtgctat tattttatct agatctcagg 351300
caatgcaaca tgcccaccta aagaaaaact ttcttattac caatgaatcc ccttctctaa 351360
catttcaaaa gtgcatagag ttgtttattg aacccgtaac atcagggttt cctggtattc 351420
atcctactgc agtgattcat cctactgcac gtattgagaa aaatgtaacc atagaacctt 351480
acgttgtcat tagtcaacat gcccatatcg gctctgacac atacatcgga gctggaagtg 351540
tcattggagc tcacagcgtt ctaggtgcta actgtctgat tcaccctaag gtggtgattc 351600
gagaaagagt cctcatggga aaccgtgtag ttgttcaacc tggagctgtt ttaggatcct 351660
gtggttttgg ttatattaca aatgcttttg gtcatcacaa acctttaaag catctaggct 351720
atgtgattgt aggtgatgat gtagaaatcg gagccaacac tacgatagat cgtggtcgat 351780
tcaagaacac cgtgatccat gaaggaacta aaatagataa ccaagtacaa gtagctcatc 351840
acgtagaaat tggaaagcat agtattattg ttgcccaagc aggcattgca ggttctacaa 351900
aaattggtga acatgtcatc attggagggc aaaccggaat tactgggcat atttctattg 351960
cagaccatgt gatcatgatt gctcaaactg gagtcacaaa atctatcacc tctccaggca 352020
tttatggagg cgctccagca cgaccttatc aagaaacaca tcggttgatt gctaaaattc 352080
ggaaccttcc taaaactgaa gaaagactaa gtaagttaga aaaacaagta agagatctat 352140
cgactcccag ccttgctgag attccttcag agatctaaaa ttctatttat tttattagtt 352200
ttgaaatcaa aaaaaagacc aaataattaa atatataaag aacgtactct tctttttatc 352260
atgagaattt tcatgatatt tttatttaaa atttctgggc taatcttccc tgtccgcaat 352320
agttgcatga ctattttcat tccaagattg aattcatcca gattagaaaa gataaaacat 352380
tctccgccag agtttaaagc ttttgcagtg tgctctgtat tggaaagtat agacgggctt 352440
ataaacttca atgaagaaaa atctacatgt aaagcaaggc cccgaattgt attcgctaag 352500
ctatatctca acgcaatagg actcgatgaa actgaaggta aagtaggcac atcattatca 352560

taaatacgtg cttgaatatt tccactacgc ttgagtcctt gaattagttt cacatagttt 352620
aataaatcta tatccacaat ctctttaaaa attaaagtga aagcaactcc atgttgcttt 352680
aaaacatccc cgagctcctt gcctagctca ctcatgctct gacaattcaa aagagagata 352740
ttcctatagg ttaaataata acgtagacta tttacaacgg tagttcctaa aagaagcggt 352800
aggtctagct ttttattcac agtaacaaca tgctcacact gttgttcgaa taatccgcaa 352860
cacagtaaaa cccctcccaa acctatagaa tcatttaaat gacaaattac ttcggtaaat 352920
tctacccagt cgctactaca tccgggagaa taaaaaaata actgagctat ttttttatcc 352980
atcgaagtta acatggcaaa caattcctta gtttgctcca actcttcatt tttgaatggc 353040
aaccattgag ttttttttaa gtagtagttt ttagagtccg tcttacgatt ctgaaattct 353100
tgagcagaag caacaatacg ttttgttata gttaaaagct tttcctctaa ttcttcatga 353160
tctcgagatt tatcttcact aggcttatga tcaaagtttt tgatgacacc gtctatcgaa 353220
tccacgtaaa actgagtgtg tcgattaatt ttacgagaaa cgtccatgtg atgtaatcct 353280
ttttgttctc tttaaattgc cacgagaaac atattaaaat tttttcgatt ttttaaaaaa 353340
acttaattat tttttcttcg gatagccctt gtcttttgaa acctaggctc ctataatgag 353400
atcaaaaacc gctcccgaag cgtctccctt ataaaaaagt tatggatagt tcagcacctt 353460
ataatatagc ttctcagggc acagagaaat ccacagtaga aaggatctta gacctttacg 353520
ggcccgcttc ctgtattaaa tttttaaaac agatggttct gattcgtgaa ttcgaagccc 353580
gaggagaaga agcctatcta gaagggctag tgggtggatt ttaccactct tacgctggcc 353640
aagaagctgt agcaactgct gcaatcgcaa acacaggact agatccctgg gtgttctctt 353700
cataccgctg ccacgcactt gcgattcttc tcaacattcc ccttcaagaa attgctgctg 353760
aacttttagg gaaagaaact ggatgcgctt taggtcgtgg aggatccatg catatgtgtg 353820
ggcctaattt ccctggagga tttggtattg tcggaggaca aattcccctc gcagctggag 353880
ccgcatttac catcaaatat caagaacaaa aaaatagagt ttctctatgc tttatcggag 353940
atggtgcggt agctcaaggt gtattccatg aaactctgaa ctttgtttct cttcaccaac 354000
tccctctaat gcttattatt gaaaataacg gctggagtat gggaacgtca ttaaatcgtg 354060
ctgttgcaaa acagcccata gcagagtctc aaggaagttc ctacgatatc cgtgcagtca 354120
cagtcaatgg ttttgatcta tttaactctc ttttaggatt tagagaggct tatcgctata 354180
tggttgatac cgaatctccg gttttagttg agtgtctctg ctcccgattt cgagggcatt 354240
ctatatcaga tcctaattta tatagatcga aagaagaaat gcagtgttta tttaaaaaag 354300
atcctattgt cctagctaaa gattggctaa ttcgattaga ggttctgact gaagaggaat 354360
ttcaaaatat acgccaagaa tgcaaaactg ctgttttaga agcgttctct aacgcaaaac 354420
tctcatcaga tccatccgtc accacattag aggaaggagt ctatgcctaa acataaaaca 354480
ttagaaattc gagaagctct ccgagaagca attgacgaag agatgtctcg cgatcctaat 354540
gtctgtattc ttggtgaaga ggttggtgac tacaatggtg cttataaagt caccaaaggc 354600
ttattagata aatggggccc taagagagtc attgatgctc ctattagtga agcagccttc 354660
tctggaattg gaataggagc cgcattgtca ggcctgcgcc ctattataga atttatgagc 354720
tggaactttt cctttgtagc cttagaccaa atcatttctc atgcagctaa gatgcatttt 354780
atgactggag ggaagttttc cgttcctata gtttttcgtg gccctaatgg tgctgcagcc 354840
caggtatctt gccagcattc tcattgcgtt gagtcgttgt atgctaatat tccaggtctt 354900
aattattata gccccttcga acccttacga cgctaaaggc ttattaaaat cagcaatcag 354960
aaataaanaa ccccgttctt tttttagaaa acgagctaga atataacttt aaaaggggaa 355020
gtccccaccg aagaatatct cgttcctatt gggaaagcac atagagttca agaaggaaat 355080
gaccttacaa ttattactta tagccgtatg gtttccatta caaaagaagc gtgttctcta 355140
gccaaaaaac gttggggctt gtctatagaa attattgatc taagaacgat caaaccttta 355200
gacatatcaa caattttatc atcggtacga aaaacttcac gctgtattgt aattgaagag 355260
ggccactact tcgctgggat ttcttctgaa attattgccc tgattactga gcatgttttt 355320
gattctcttg atgctccccc cttaagggta tgccaaaaag aaacgcctat gccctatagt 355380
aaaatcttag aacaggccac tttgcctaat gttaaccgaa tcttagatac cattgaaaaa 355440
gtcatgaggt aagtttgtga tctccttatt gaaaatgcca aagctttctc caactatgga 355500
agtgggcact atagtgaaat ggcataaaaa aagtaatgat caggtcagtt ttggagacgt 355560
cattgtagag atctctacag acaaagctat tttagaacat acagcaaatg aagatggctg 355620
gattcgtgaa atcttacgtc atgaaggcga gaaaatcgtt ataggcaccc ctattgcggt 355680
actctctaca gaagccaacg agccctttaa tctagaagaa cttcttccta agacagaacc 355740
ttctaacctt gaagcatctc caaaaggttc ttctgaagag gtctcgcctg caacaactcc 355800
acaagctgcc tcagcaacat tcacagcagt aacttttaag ccagagccac ctctctcctc 355860
gcctttagtc ttcaaacacg taggcactac gaataatctc tctccattag ctagacaact 355920
agcaaaagag aaaaacatag atgtctcatc aattcaaggg agtggtcctg gaggacgtat 355980
agtaaaaaaa gatttagaga aagctcctcc taaaagcatt gctggttttg gctatcctga 356040
gtctcccgaa gtgcctccag gttcctatca tgaggagaat ctctctccga ttcgggaagt 356100
gattgctgca cgcctacaag ctgctaagat ctctattcct cacttctatg taaggcagca 356160
ggtctacgcc tcacctctcc ttaatctgct caaagaactt caagctcagg gaatcaaact 356220
ctctattaac gattgcattg tacgtgcctg tgctctggcg ctcaaagagt tcccttctat 356280
caattcagga tttaacagtg tcgataataa aatcgtccgt tttgatacta tcgatatctc 356340
gatagctgtg gccattccag atggaattat tacgccaatt atacgctgcg cagaccgtaa 356400

aaatctcggc atgatttcag cagaaattaa gagcttagcg ttaaaagcaa gaaatcaatc 356460
tcttcaagac actgaataca aaggagggtc cttctgtgtc tctaacttag gaatgacagg 356520
aatcactgaa tttacagcga ttgtcaatcc tcctcaagcg gcgattcttg ccgtaggaag 356580
tgttacagaa caagctcttg ttcttgacgg agaaattact ataggatcta cctgcaatct 356640
taccctatct gtagatcata gagtgattga tggttatcct gctgcgatgt ttatgaaacg 356700
attacaaaag atcttagaag ctccggctgt cctactatta aactagcaat ctttgaacaa 356760
aaaggactct ttctatagct ccttggctat ggaaagagtc cctgattcca tcctgtctat 356820
ttcttaattt ccttctccag agcaagattt tgtaggaaca tgccaaatat ctctggcata 356880
atcctgaatg gctctgtcac tagagaaaaa gcccattcct gcagtattat aaatagaaat 356940
cttagtccat gaatctggtt ccttaaagag tttgttcaca ttttcatggg cagcgatata 357000
agactccaag tcagccaaga caaaaaaggg atctccttca tgcagtaggc gatgtactat 357060
cggtttaaac agatctttat cattgctatt gaaaaatccc tgttctagca aatctaaaac 357120
ctgacggatc ttaggattct tatcacaaat tgtctgagga cagtattccc tccgcagttg 357180
tacaatttgc tcctccaaaa gaccaaaaat aaacatattc tccttaccaa tatgctctgc 357240
catttctata tttgcaccgt ccatagttcc tatagtcaga gctccattca aagcaaattt 357300
catatttcct gttccagaag cctccattcc agctgtagaa atctgttctg aaagatctgt 357360
accaggaatg atatgctcag ccatagaaac tcgatagtta ggtaaaaaaa gaaccttaag 357420
cttatcatta actcgagaat cttgatttac aacgtcagca acgctattga ttaacttgat 357480
aatgagtttg gccatgacat agccaggagc cgccttacca gaaaaaatta ctgttgtagg 357540
gacgacatct tgattagggt tttctttcaa gtcattataa acatagatga ctctaagaat 357600
attcattagt tgtcgtttat actcatgaat acgcttaata tgacagtcaa agagagaatt 357660
agggtctact atttctccaa cttcattata aattctactt gttagatcct gcttattttt 357720
taattttacc cctttccaat gatctcggaa accactatct tcggcaaagg aacggatcaa 357780
tgaaagatga gaaagatcaa tgatataacg atcccctata gtttcattga gaagcttact 357840
caaacgagga ttacagagag caatccatcg tcgtggagtc accccattgg tcacattgat 357900
aaacttctca gggaaaaact cataaaactc tttaaagaga gtatctttaa tcagctgaga 357960
gtggaatgac gaaactccat ttacttttgc agaacctact acggcaaggt ttgccatatt 358020
gatacgcttt tgataccctt cttcaacaat ggataaagac cggcgcttat catcattttt 358080
aggatagcga gagccaactt tttctaacca acgggaattt atttcataaa taatctctaa 358140
atgccgaggt aataacttag agaataaatc gagaggccat ctctctaaag cctctgggag 358200
gattgtatga ttggtatagt taaagatgac tgtagtcatc tcccaagcct tatcccaagg 358260
taattcttcc ctatcgacta aaatatgcat catttcagca atccctagag cgggatgggt 358320
atcgtttaat tgtactacga ctttatccgc aaggttatcc aaacaaatat gtgtctttgt 358380
atatctgcgg ataatatctt gaatggttgc tgaaactaaa aaatactctt gtttgagacg 358440
caattcctgc ccctcagtaa tagaatcatt aggatagagg acgcgagaga tgttttctat 358500
caaggcgata tcttctatag cctggatata gttcccgtgg ttaaaatagc tgaattcaaa 358560
gcctcgcgga gattgtgctt gccatagcct tagagaattt acagtatcat taccgtaccc 358620
aggaatcgga atatcataag ccatcgccaa tacctcttgg gtatcgacaa gatctgccac 358680
ctgtttccct cgagaatcgg tataatgaat gacccttcca taaaatcgta cgggatagag 358740
gtactctccc ctacagattt cccaaggatt tccataacgt agccactcgt caggagcttc 358800
ctcttgatac ccgttgacga tcctctgatc aaaaatacca taatcatagc gtataccgta 358860
gccgtaggct ggaactgcta atgtagccat agaatccaag taacaagctg ccagtctccc 358920
caaaccacca tttcctaatc ctgcatcgga ttccatttct acaaggtggt caaagtcata 358980
atttaaagtt tttagtgcct tccttactaa atctagaatt cctaaattca aaagattgct 359040
tgttagactt ctccctaaaa gaaattccat ggaaaggtaa taaactcttt ttacatcatt 359100
tttatagtag ccattttgag ttttcagcca ccccttggcc aaccattcca taacagtttt 359160
tgcaacagct gtgaagatat ctctaggaga tgcggactct ggtgattgta caacacttaa 359220
atacagacga tctaaaatcg cccgtttcat agagtcaaca ctgactttgt tcttatcaaa 359280
actcgaaaaa tcttccacaa tgcaaccatt tcaaatacca tctagaaccc tccatatgcg 359340
aaatttgaaa aaaaaaaaag aacttcgcct tggtaaataa aagagagcct attaagagag 359400
cttaatagtt ctgaactaga gaaagactat aaataaaatt agttgataaa ttttaataaa 359460
attaaaataa tatctataaa aatattattg acaacaatta tatagacaga ttaaaattat 359520
ttaatttttt actcaggaga acaacatggc tacagtagca caaacacctc agactacaca 359580
accacaacca tcagtatctc acaaggcaac acatcgttat tgttcctggg tattttttaa 359640
gcctatttta gttagtctag gtctcctcct tgcttcctta accaccctag gactggttat 359700
tgccagtgga gtcaccctat ccttaggaat cggccattgt tcttgctata cagatagtac 359760
tgctggtatt gctcttgtcc ttgctttcaa tcatattaga caatttaaac aagctagaac 359820
agcggagttg aactcaatga aaatgatatc tgcgccggct gctgcaactg tccagaagca 359880
aaaattagag gatcgttact cctctaaata atcatctctt cgctagggaa aaattccaaa 359940
ctcttatgca aggaatttaa attctnnaga aatatcttga atagccatgt gaatatcatg 360000
gctattttct tctatttttt gttcaatcaa tcgtatggat gagattaccg tcgaatgatc 360060
tcttgaaaag acatcgccta ttctcacgta tgatagtgaa agcttctgac gacaaaagta 360120
catggctacc tgacgtggca atacatattc tcgggactga gaacgtccta aaatactctc 360180
ctgagagacc ccataatatt gagcaacatt acgaataatc tttaaaggag ttaaacgaac 360240

gcttcctgct gcttctaaaa catcttttaa aagagttttc acatcatctt catatagtaa 360300
ttggtgagag agttttttat acattaccct ctttgctaaa agattcagtg catgcagtaa 360360
ggtctttacg ttggaagata gcgcataaat taaaaaatct aaggccgttt cttgaatgcg 360420
aatagataag cgctctacct gtctcattaa gaaactgcgc aatccttcct gaaccaaagg 360480
atgtatcgga attgcaactc cccattcaaa cctgctgatc aatctatctt caacagcaac 360540
gagatccaca ggcgcatagg atgaagacac tacaatcaac ttcccttcag aatgaagaga 360600
attaaacgta tggaagaact cttcttgagt tgccgacttt cctgaaaaaa cctcgatatc 360660
ctcaatgaat agagcatcaa tattgcggta aaaagaacgg aatttttgca tttctcctga 360720
acggatagca gagactaagt gctctgtaaa caaatccgaa gaaacataga gaatcttacc 360780
tccagattca cgaagaacac tgatagctga ctgcattaag tgagtttttc cagatccctc 360840
aggtccaaac agataaattg gattaaaagt aactcctccg ttttcatcag gactcttagt 360900
aaattcctgt aaaacacgaa aaggaagatc attttcaggg gtaactaaaa aattagagaa 360960
ggtcatctca ggattcacac ttccataatg catggtaaag tatgctgtct tctcttgctg 361020
catctgcttc tccttataaa aaggagctgc tttatctacc gaagtaacgt gaacacgaat 361080
gggcttattg ttattattta caagaccaga tttaacctta tgtcttatat gctcctcaaa 361140
ccaagtaatt tgaaaagaat cttgagcttc aagatacaaa ttacaagcat caaaacataa 361200
gacctttaaa gatcgcaacc acttgtctac agtatttgtg ccaatttctt tctcttgtag 361260
caaaagaaat tcttcccatg ctcgcataaa ctatgagctc atataaatcg cttgtttcaa 361320
taagcctgta ttaagacaat tcgacttttt tttcagtatt atcgatgttt tttattattt 361380
aaaaccactt catttttaag atgtttgcta tctaatatct tattagtgat ccactgctgg 361440
acgactccta aaatcataga cgaaagccaa tagatgttta atcctgaagg gaagttatag 361500
aacatagcgg taaataaaat cgccatcatg ttccccataa cttgttgctg tttctgctga 361560
tccgtaacag gtcctttctt atgcaaactc gtgaccttct gttgtaagaa catcactata 361620
cctaatagaa taggaagtaa gtggaactca tttccaataa accatatcga tgtctgccaa 361680
gaaaacaaca catcaggagc tgttaagtta tcaatccacc caggaataaa cgaggctcct 361740
cgtaataaga atgatgactt taataaatca aacatcgcaa ttaggaaagg aagctgtatc 361800
aataaaggta aacaacccgt gataggattc actttgtttg tcttatacaa gcccatgatt 361860
tccatctgag cacgcttagg ttcgttctta tacttttgct gaatttgctg aatataagga 361920
gataaaatcn gcatacgcct ccnagatcgt atggaccatg cagataaagg atagagaagc 361980
aatttcaaaa atacagtaag taaaataatg gaaattcccc aagaacccgt aaccaatttg 362040
aagaacttca taataataaa taggagtgct gcaaaaggag ctgtaataaa tgcaaaaaca 362100
ccacggaaag aaatgctatc aagatactca ggatttctcc ccttctcctg agtaattgtc 362160
ttatctaata ctttaagtgt aggctctgcc aagggacctg catacactaa aaatcgatgt 362220
gtccctgcat cttttggcaa aggaagcaag gtctcatatc caggatattt tgatactgga 362280
tacagttgat ttttaggaga aatagcagac aatcttgtcg gagccgtaga acccgaaatg 362340
tagagagatc catagccaga agcaatttca gacaacggag ttaaaataat accgaaatat 362400
ccattcgaat ttaaaatcca ttgaggataa acaccacgac gtacagctaa aggctctttt 362460
acttttggaa gcttaacttt atctaaagac cccttatttt ttttgataac cctgtattta 362520
atggttgggg ctgaagcatt tgacatgatc tccacttcag gaactcctga agttacccat 362580
acatcttcgg tttctttcgt taaagtaatt gcagtttcaa aaacataggg cttttcttcc 362640
ggattctctg gaagtttgta taccttctga accgatctat ctaagctttc caattgaatg 362700
gaatgggggg tataggaaag aactcggtat cttaaagcca caggagtcgc tagctctctt 362760
cctgaaacca catttaatgc gtgatactct agaggaagta atttcttaga atcacttaat 362820
aatcccctgc gcaataaagg gtagtaacct ccaatcgagt ttttggcttg ttggccatca 362880
ggaagttttg aagacagccc agggaaaaga gcttcaggag atttctctga agctaaatcc 362940
ctatcaaaac caatttcatt cacaatgctt ttattatttg ttgaagcaaa aggtaaattg 363000
ataccttcta tagaaccact ctcttcagaa acaataatct gcatgtaatc attaaataaa 363060
acatagtggt tcgcagtatc tgacgacttt gccgaatctt ggtcattacc aaaaattaca 363120
gctcgtgtaa taggaagatc caaagaaact aacttttctt ctcgagaatc atagagacct 363180
aaaggaatat agtcgcttcc tgatctccaa aagacaagcg ctgtaccaaa gatcgtgcta 363240
tctttattgg aaattcttcc ttgtgcgtac tcacctagaa atactaaagg ctctttattg 363300
ttacgaaact caacaactag tacaggtaaa ccttcatgat tcgtaggaag aaaaactttt 363360
cccgtattcg taggattgaa agaggaaccc tgctgacgat ataaagccaa gtgaatatta 363420
tcgaaaccac acttgtgatc tacaaagctc caagattccc cagaagaata aacagactga 363480
gcagcttctc cattatgtaa taaaaataac ttgtctccaa cacgaactgc gtagttattc 363540
ttatgttctt ctccgtttac atcggtgtcc catgaagcta cacttaaccc tacagattct 363600
actgcagcta gcgtctgttc tgaaatcttt ctttgtttct ctgctagatt tttgcaggaa 363660
cgaaattcat tataaccaaa aaatatttga catcctacaa aagcaatccc aattaaagaa 363720
acaaaaagca aagtgcgttt attcatttga taaactctaa aaattaaact taagaggtca 363780
aaatatcata aacccacatt atctcccaaa ggagaatctc tcaatacagg tctcttattg 363840
tgatagactg taaaattcta aagtctcatg ccaaaaagat gctgctatat gtgtgaacgg 363900
tgccttcgag ctttcaatga gcaaatgata agtaaggcca caccaaatag aaataaaggt 363960
atagataaaa tttgaccaat tgtaagtaga caatcctctg ctaaaacttt cccttgatgg 364020
ctttttacat actccgcaaa aaaacgaatg aaagcgacag aaatacaggc tatagaagtc 364080

acatatccct tacctaaatg caaataacgc ttataggaaa gaaaatataa aattccagag 364140
acgaccaagt aactgattcc ctcataaagc tgcacaggat gcacaggaac tccctggaca 364200
ccttgcatag gatcagaaaa aaccaccccc caaggcaaag aagtcggtgt tcctacaatt 364260
tcttgattcc aaaaattacc caaacgaata aaaaacgctg caattccgaa aactgatcca 364320
cacaagtctg taaggaagag aaaagtcaat tttgaaatct tttttttata tatccaagaa 364380
aaaatggccg cccacaaaag aaagccaaag aacgcctcca tgactcgaca agcctccgtg 364440
ccatatttga atgatctctt caggatgttg taagtaaaaa ctccatccat aaaaaatcac 364500
ataggcaagt ctagctccag ggacaataaa taaaatagag tatataaaaa agttttctaa 364560
agccacgcgt agctggcttt tggaaaaact taaatgatct ttcaaaccat aataggaaag 364620
agccaaatac cttgctgaga gacatgctag aaaaatccct acagtaaaaa agacgccata 364680
ccaagtaagt cttagagacc atggctcgaa agaccagaca atttttgagc gatcccaata 364740
gatcacagcc atctcagctc gcatatctta ataccagtta gcaaaaaatt catactccaa 364800
gtaaaataga gtatttagaa attaagtgta atgcactatg gcaagaaaca tcaaatattt 364860
cctgatacta tttcctggta ttttgtggat atcagcagga atgaaactct tgctaaaagc 364920
taccgcaata gccctagacc ccctttcttc tttttttacc tactgtcttc tatctatggt 364980
ttcttgggga ttagcatccc taaaacatcg ctacttgcta agcaaaacta taaggaaaca 365040
gctgagtcta tcttctgaat tcttttcaca aaaaattaca tggattgcct atataaagca 365100
gacctttatc tctagaaggt ttctcatcat ggtcattatg attgccttct ctttagtcct 365160
tcgtcgttat atcagcaatc ctcaagcctt attcgtgatt cgagctacag tgggctatgc 365220
tctcattaaa actgccatcg cttacttctc aaaattacag aatgccctaa tggaaaatcc 365280
tgaaggaaat tagtgcgaac tctatggaaa tcattcatat aggaaccgat attattgaaa 365340
ttagccgcat tcgcgaggca attgcaactc acggcaatcg actactcaat agaatcttta 365400
cagaagcaga acagaaatat tgcttagaaa agaccgatcc catcccttca tttgcaggtc 365460
gctttgctgg aaaagaagct gtagcaaaag ctttaggaac tggcataggg agcgttgttg 365520
cctggaaaga catcgaagtc tttaaagtat ctcacggacc cgaagttctc ctcccttcgc 365580
atgtctatgc aaaaattgga atttctaaag tcattctctc tataagccac tgcaaagagt 365640
atgccacagc aactgcaatc gcattagcct aagaatcttt cagcatctag tgctgctata 365700
caaccacctc ctgcagaagt aaccgcctga cgatagtact tatcctgaac atctccagca 365760
gcaaatactc cagggacaga agtcttggac gttcctttct cagtcacaat atagcccgac 365820
tcatctaacg tcagctgtcc tccgagaaaa tccgtatttg gcttatggcc tatagcaaag 365880
aacacccccg cagcttctct agttgtaatt tcttgagtct gaacattctt aatatctacg 365940
gaacggacaa tgctatctcc agaaattttt acaatctcgc tattccataa aaatgtaatt 366000
ttttcattgt tttgcgcccg agcttccata gctttagaag cccgcagttt atctctacga 366060
tgaactacat atacgtggct tccataacga gtcaggtaaa gagcttcttc taaagcagaa 366120
tccccctccc caatcacata aagatcttta tttttaaaaa taggagaagc cccatcgcaa 366180
acggcacaag cagtcactcc tttttgccaa aattcatcgt ttcctgctcc aggaatttct 366240
aaacgtttag cagaagctcc tgtagctatg atacaggcat cacaagaata ggtttcttct 366300
tttgatttca aaataaaagg gcgaacagaa aaatctacgg aaataatatc ttgagctagt 366360
gtcttggtcc caaaccgcac agcctgctcc ttcatattat tcataagttt tggcccaaga 366420
atcccttcag gaaaccctgg aaaattctca acttctgttg tagtcataag ctggccacca 366480
gagatcccag agaaaaaccc ctcaaataaa agaggatgca aaagcgctct tgatgcataa 366540
attgccgctg tatatccaga tggacctgaa ccaataataa ttaaccggga atgaatcatt 366600
tataatttcc ttatctaact ttagaaattg agaacactgt tcttgatcta ctagaaaaaa 366660
gacacttaat tctagcctat ctaataaaca acacttagcg tctatgcgtc agtttctcta 366720
tgcaaaaaac ttgcttttga atagcttttg tctagtttta atttatacgg atttaagact 366780
aaagtttcaa atctcaagat cttcatcttc cagtaataac acctacattt ctgtgattaa 366840
tctttagaat tttcttagag ctaagaaacc aagacccatc ctattattta accctcaata 366900
agtatggttc tacttctgaa tgcacttggt tcttgagcag caaccaaaaa gatctatcag 366960
gcttaagaac aaagctgatg tagcccatct gggacgagaa tttatggggt gcttatgaaa 367020
aaaacgatat gttttctatg cgccaaatcc tgtttctcta catatgacaa ggcacattta 367080
tgataattac cacacgtatt ttataaaatc tggtaatata gaaaagttaa aaaagaggcc 367140
ttatacatct ctagaacgga agtataggat tttacgatta attcgattat atagaactaa 367200
tcgtctcctg caagggaggt cttgcctttt ttaaggttta tatttacact gtcttttttg 367260
actttgtagt ttttaggaga ataacaataa atgccaaaac aagctgaata tacttgggga 367320
tctaaaaaaa ttctggacaa tatagaatgc ctcacagaag acgttgccga atttaaagat 367380
ttgctttata cggcacacag aattacttcg agcgaagaag aatctgataa cgaaatacag 367440
cctggcgcca tcctaaaagg taccgtagtt gatattaata aagactttgt cgtagttgat 367500
gttggtctga agtctgaggg agtgatccct atgtcagagt tcatagactc ttcagaaggt 367560
ttagtgcttg gagctgaagt agaagtctat ctcgaccaag ccgaagacga agagggcaaa 367620
gttgtccttt ctagagaaaa agccacacga caacgtcaat gggaatacat cttagctcat 367680
tgtgaagaag gttctattgt taaaggtcaa attacacgta aagtcaaagg cggccttatt 367740
gtagatattg gaatggaagc cttcctacct ggatcacaaa ttgacaacaa gaaaatcaaa 367800
aatttagatg attatgtcgg aaaagtttgt gaattcaaaa ttttaaaaat taacgttgaa 367860
cgtcgcaata ttgttgtctc aagaagagaa ctcttagaag ctgagagaat ctctaagaaa 367920

gccgaactta ttgaacaaat ttctatcgga gaataccgca aaggagttgt taaaaacatt 367980
actgactttg gtgtattctt agatctcgat ggtattgacg gtcttctcca cattaccgat 368040
atgacctgga agcgcatacg acatccttcc gaaatggtcg aattgaatca agagttggaa 368100
gtaattattt taagcgtaga taaagaaaaa ggacgagttg ctctaggtct caaacaaaaa 368160
gagcataatc cttgggaaga tattgagaag aaataccctc ctggaaaacg agttcttggt 368220
aaaattgtga agcttctccc ctacggagct ttcattgaaa ttgaagaggg cattgaaggt 368280
ctaattcaca tttctgaaat gtcttgggtg aaaaatattg tagatcctag tgaagtcgta 368340
aataaaggcg atgaagttga agccattgtt ctatctattc agaaggacga aggaaaaatt 368400
tctctaggat taaagcaaac agaacgtaat ccttgggaca atatcgaaga aaaatatcct 368460
ataggtctcc atgtcaatgc tgaaatcaag aacttaacca attacggtgc tttcgttgaa 368520
ttagaaccag gaattgaggg tctgattcat atttctgaca tgagttggat taaaaaagtc 368580
tctcaccctt cagaactatt caaaaaagga aattctgtag aggctgttat tttatcagta 368640
gacaaagaaa gtaaaaaaat tactttagga gttaagcaat taagttctaa tccttggaat 368700
gaaattgaag ctatgttccc tgctggcaca gtaatttcag gagttgtgac taaaatcact 368760
gcatttggag cctttgttga gctacaaaac gggattgaag gattgattca cgtttcagaa 368820
ctttctgaca agccctttgc aaaaattgaa gatattatct ccattggaga aaatgtttcc 368880
gcaaaagtaa ttaagctaga tccagatcat aaaaaagttt ctctttctgt aaaagaatac 368940
ttagctgaca atgcttatga tcaagactct aggactgaat tagatttcaa ggattctcaa 369000
ggccctaaag agagaaagaa aaaaggaaaa tagcatctaa tgctggtaat gcagaggatc 369060
gtattattta gttctaaata atacgtcctt aatttagcta tttactgatt tccttattta 369120
caagaggagt ataatgaata aaaatcttgt agctattttt gactacatgg agaaagaaaa 369180
agggattcag cgctctacta ttataggagc tatcgaatct gctttaaaaa ttgctgctaa 369240
aaaaacctta agagatgacg cgaacatatc tgtaaacatt aattctcgta ctggtgacat 369300
cgaagtcttt tgtgaaaagg aaatagtaga aatttgtcag aatcctagca aagaaattcc 369360
tttagataaa gccagagaat acgatccgga ctgtcagatt ggtcagtaca tggatgtccc 369420
ttttgtttct gataattttg gaagaatagc tgctcacgca gcacgacaaa ttatcggtca 369480
aaagctaaga catgctgaga gagacgttat ttatgaagaa tatcgccatc gcgtaaatga 369540
aactttatct ggtgttgtca aacgttttgc taaaggttct aatttaatta ttgacttagg 369600
aaaagttgaa gcaattcttc ctacccggtt ttatcctaaa acagaaaaac ataagatcgg 369660
tgataaaatt tacgccctac tctatgaagt tcaagagtct gaaaatggtg gagcggaagt 369720
tatcctcagt cgtagtcacg cagaatttgt taaacaatta tttatttcaa gaagtcccag 369780
aactagaaga aggttctgtg gagattgtta agatagctcg tgaagctggg taccgcacga 369840
aactagctgt aaagatcgtc agaccctaaa actgatcctg ttggagcttt tgtaggtatg 369900
cgaggttctc gagtaaaaaa tatcatttcg agaattgaac gatgagaaaa ttgacattgt 369960
caattactcc cccgtctcta cagagttatt acagaatctt ctttatccaa tagaaatcca 370020
aaagattgct attttagaag acgacaaagt gattgcaatt gtcgttaatg atgcagacta 370080
cgctactgtt attggtaaac gaggaattaa cgctcgttta attagccaca ttctagacta 370140
cgagctcgaa gtacaacgta tgagtgagta caataagttg ctagaaattc aacgccttca 370200
attagcagaa ttcgatagtc cgcacttaga tcaaccctta gaaatggaag ggattagtaa 370260
gctagtcatc caaaatttag aacatgctgg atatgacaca attagaagag tattattagc 370320
gagtgctaat gatctggcat ctgttcctgg gatcagttta gagcttgctt ataagatcct 370380
tgagcaagtc agcaaatatg gagaaagtaa agttgacgaa aaacctgaaa ttgaagatta 370440
agaatgctca attaacgaaa gccgcggggc tggataagct aaaacaaaaa cttgcccaag 370500
caggatcttc tgaagctaaa tcttcttcag aaaaaccttc tgcgaaagaa aagtctgtaa 370560
aagtagctct tgccgcaact tctaccccta cggcaagtgc ggaacaagct tcaccagagt 370620
ctacttcacg tcgcattcgt gctaaaaatc gttcgtcgtt ctcatcatcc gaagaagagt 370680
cttctgctca tattccagtg gatacatctg aacctgctcc agtctccata gcagatcctg 370740
agcctgagtt agaagtagtc gatgaggttt gtgacgaaag tcctgaggtt catccagttg 370800
ctgaagttct tcctgagcaa cccgtattgc ccgaaacccc acctcaagaa aaagaattag 370860
agcctaagcc tgtgaagcct gctgaaccta aaagcgtcgt aatgattaaa tctaagttcg 370920
gccctacagg aaagcatatc aatcatctcc tagcaaaaac attcaaggct cctgccaagg 370980
aagagaaagt cgtagctggc tcgaaaagca caaagcccgt tgcttcagat aaaacaggga 371040
aacctggaac atctgaaggt ggtgaacaga ataatcgaga aaaacaattc aatcctgcta 371100
accgtagtcc tgcttctggt ccaaaaagag atgctgggaa gaaaaatctt accgactttc 371160
gtgatcgttc taagaaatct gatgaaagcc taaaggcttt tacaggaaga gatcgttacg 371220
gattaaatga aggcggagaa gaagacagat ggcgaaaaaa acgtgtttat aagcctaaaa 371280
aacactatga cgaagcctct atccagcgac ctacgcatat caaaatttcc ttgccaatta 371340
ccgtcaaaga tctggcaaca gaaatgaagc tcaaggcttc agaagtcatt caaaagttat 371400
tcattcatgg aatgacctat gtagtcaatg atattctaga cagcgaaact gcagtacaat 371460
ttattggctt agagtttgga tgtacaattg acatcgacta ttctgagcaa gataagttgt 371520
gcctaagcaa tgacactgta agagacgaaa ttcaatctac agatcccagc aagcttgtga 371580
ttcgctcccc tattgttgcg tttatgggtc acgtcgacca cggaaaaaca acactcattg 371640
actccttaag gaaaagtaat gtcgctgcaa cagaagctgg agcgattacc caacacatgg 371700
gagccttctg ctgctccacc ccagtgggag acataacaat tttagatact cctggtcacg 371760

aagctttctc tgcaatgcga gcccgtggag ctgaagtttg tgatattgtt gtgcttgtag 371820
tcgctggaga cgaaggaatt aaagnacaaa ctttagaggc tattgaacat gcaaaagctg 371880
ctgatatcgc tattgttgta gctatcaaca agtgtgataa gcctaatttt aattccgaaa 371940
ccatctatag acaactttct gaaatcaatc tattgccaga agcttgggga ggctcgactg 372000
ttacagtaaa tacctccgca aaaacaggag aaggtctttc agaactttta gagatgttag 372060
ctttacaagc tgaagtcttg gagctaaaag ccgatccttc agcacgtgct cgaggacttg 372120
ttattgaatc agaactgcac aagggtctcg gacctgttgc gactgttttg attcaaaatg 372180
gaagcttaaa actgggcgaa gctctcgtct tcaatgattg ttatggcaaa gtgaaaacta 372240
tgcataacga acataatgaa ttgatgaaag aagctgggcc atctattcct gtgttgatca 372300
caggtctatc ggacattcct aaagctggcg atcctttctt cgtcgtgaaa aacgagaaaa 372360
cggctagaga cattattgaa gctagatccg caggacaaca gcgttttgct ttacagcaaa 372420
agaagcggcc taactttgat tctatgttac agaataaaaa gactcttaag cttatgatta 372480
aagctgatgt tcaaggttcc atagaagctt tggtcagttc aatatctaag attaaatcag 372540
aaaaagtaga tgttgaaatt ttaacaaaca gtgtaggaga aatttcagaa tcagacattc 372600
gtttactgcc gcctctaaag cagttctcat cggtttccat acaggaatag aaagtcatgc 372660
ggaaccttta attaagagct taggagtccg agttgaacta tttaccgtca tctatcatgc 372720
tattgatgca attaaagaaa ttatgacttc tctattagat cctattgctg aagaaaaaga 372780
tgaaggttct gctgagatta aagaaatctt taggtcttca caagtaggat ctatttacgg 372840
ttgcatagtt actgaaggaa ttatgactcg caatcataaa gtccgagtat tacgtaataa 372900
agagatcctt tggaaaggta cgttatcttc attaaaacgt gttaaagaag atgtcaaaga 372960
agttcgcaaa ggtttagagt gtggaatttt gttagaagga taccagcaag ctcaaatagg 373020
tgatgtccta caatgttatg aagttatcta tcatccacaa aaactataac ttgaagtact 373080
gtatgacaga aaatagacgt attaaacggg taaatgcttt attacaagaa gccattgcaa 373140
aggtaatttt aaaagatgtt aagcatccca agatttctaa tctttggatc acggtaactc 373200
gtgtttctct atctaaggat ttgcactctg cacgtgttta tgtatctgta atgcctcatg 373260
agaataccaa ggaagaggct ttagaagctt taaaagtctc tgctggtttt atcgctcata 373320
gagcttcgaa aaatgtcgtc cttaaatatt tcccagaact tcatttttat ctcgatgata 373380
ttttctcacc tcaagattat atagaaaacc tgctttggca gattcaagag aaagaaaaga 373440
gttaataaac tatatttttt gggaacttga atactattaa agacatgact atggatcttg 373500
cagtagaatt aaaagagggc attcttcttg tagacaagcc tcaaggagaa acttcgttta 373560
gccttatccg cgctctaacc aagttaatag gcgttaaaaa gattggtcat gcaggaactt 373620
tagatccctt cgctactggc gttatggtca tgttgattgg ccgtaaattt actagacttt 373680
ctgatatttt actttttgaa gacaaggaat acgaagcaat tgcccattta gggacaacta 373740
ccgattctta tgattgcgac ggcaaagttg taggaagatc taagaagatt cctagtctcg 373800
aagaagtatt atcagctgcc gagtatttcc aaggagagat ccagcaactt cctcccatgt 373860
tttccgctaa aaaagtccaa gggaaaaagc tgtatgaata tgctagaaaa ggtttatcta 373920
tagaacgtca ccattctaca gttcaagttc acttgcagat tacgaaatat gagtaccctt 373980
tattgcattt tgtagtctct tgtagcaaag gaacttatat tcgcagcatt gctcatgagc 374040
ttggcacgat gttaggctgt ggagcttatc ttgagcagct acgccgttta cgcagtggcc 374100
gtttttctat agatgaatgt attgatggga atctattaga ccaccccgat ttcgatattt 374160
ctccctacct acgagatgcc catggaaata gcctatagtt taacgtcttc gttttctgta 374220
gattctgtaa ctgtaggttt tttcgacgga tgtcatctag ggcatagcaa tcttttatct 374280
attcttactt cctattctgg atccagtgga gttattacct ttgattctca tcctcaaacg 374340
gtactttctt taaatcacac gaaactcatc aatacaaaag aagagcgcct ccaattattg 374400
caaacgtttc ccatagactg gttaggtgtc cttacttttg atttaaattt tgcgaatcaa 374460
tcggcagaag aatttcttac tttgttacat cgtaacttga aatgcaaacg cctcatctta 374520
ggttatgatt cttgcatagg gaaagaacag caaagcaata ccgaggctct cgatactata 374580
ggcaagccgt taggtataga ggtcatcaag attcctcctt accgtatgga taacatagtt 374640
gtctccagca aagcaatccg ccagtttctg tccgcaggga atcttgaatg tgctcatcgt 374700
tttttgggtc atccctatgc catttctgga aaaataaccg agggctccgg aataggaggt 374760
tctctaggat tcgccactat aaatcttcct agagaagaaa gtttaattcc cctaggagtt 374820
tatgcttgtg aaatacgtta tgatagcact acctgtcagg gtgttatgaa tttaggaact 374880
gcccctactt ttggaagaga gtctttatat gcagaggcgc atatcttttc ctttgcggaa 374940
aatctatacg gcaaagaagt gagcattatc ccgagaaaat ttcttagaga agaaaaaaag 375000
tttcaatcaa aagaaactct aatacgagca attgaaaaag acattttgga tgctcaagat 375060
tggtttgcaa agggttcctt taattatgaa ggaacagcat agtatcaccg tcctggacga 375120
tataatcacg tccttctata tgtaatttcc ctaattctcg agcagctgca cgaccttgac 375180
actctatcat atcttcaaaa gtaatcactt cagcacgaat aaagcccttt tgaatatccg 375240
tatggatttc tccagcagct tcccaagcag aagaccctcg aaccactgtc catgcacgag 375300
attcttgagg acctgtagta aaataagaaa tcagtcctaa agtgtcatac gcagcacgca 375360
ctaatctatg aagtcctgat ttttcaagac ctaagctcat aagaaattct aagcgctctt 375420
caataggtaa ggaaacgatt tcttcttcta tacgaacaca gataggaacc actttagaat 375480
tttcttttgc agcaacttcc cgaacagcgg caacataatc attatccata tctggtagag 375540
aactctcgtc aacattagct atataaaaca taggcttcat ggtcaaaaac ggatagggct 375600

ttaatgccac aatttgttct ggagttaatt ctaaagtacg tagcggcagc ccttttctaa 375660
gtgagcaata attgtatcaa atagaggcaa gagagctcct acttcacgct ttcctttggc 375720
tagcttttct aatttgctat ggatattttt tgctgaggag aagtcagaaa aaatgagctc 375780
taagttgata acttcaatat cctcaacagg gttgactttt cctgaaacgt gtgtaacgtc 375840
tggatcatca aaacaacgca ctacatgagc aatagcatga gtttctcgaa tatgagagag 375900
aaaccgattt cccagacccg cgccatcgga agctccctta actaaacctg caatatctac 375960
aaatttcata tccgcataga tgatcttctg actattgcta attttagcta aggcttccag 376020
tctttcatcg ataacaggaa caatacccac attaggatcg atagtacaaa acggatagtt 376080
acaggaggca acttgagctc ctgttaaagc attgaataag ccagactttc ctacattagg 376140
aagccctaca attccacatt cagtatgact cataagacat ctaaaaataa aaataatgac 376200
atgctttttc gaatattaga aagacaactc ttctgtttca aaaaagcaca atagctagaa 376260
aaatagtacc ttcaaaatca agagtgcttt gaaaatgata aaaaaccact ataatcgatt 376320
gaagttttcc ctacctaata ctttcttcat atagaaccat cttgaagtga cctaaagtat 376380
tgctaaattc aacaactttt cgttattgtt ctttttttct actattcttc tttatctcta 376440
agattcattt ataatcttac taagaacaaa acgtttgggc ttcactcttg agaaaaacaa 376500
gagcataagg taatttgcag ataattccga atatagtcct cttaatcgaa ccttggcaac 376560
agcatgggtg aaaaaacaga aaaggccacg ccgaagcgac ttagagatgc tcggaaaaaa 376620
ggtcaagtag caaaatctca ggattttcct tctgcggtta cctttatcgt ctctatgttt 376680
acggctttct ccctatcgac cttttttttc aagcatttag gtggctttct ggtttccatg 376740
ctctcacaag ctcccactcg ccatgatcct gtaattacct tattttatct taagaactgt 376800
cttatgctta ttttaacagc atcacttccc ttactgggag ctgttgctgt tgttggcgtc 376860
attgtaggtt ttcttatcgt tggtcctaca ttttctaccg aagtttttaa accagatatc 376920
aagaagttca accctattga gaacatcaaa caaaagttta aaataaagac tctcatagag 376980
ctaatcaaat cgattttaaa aatttttgga gcagccttaa ttttatacat aacgttaaaa 377040
agcaaagtct ctttaattat agaaactgca ggagtctctc ctataattac tgctcaaatc 377100
ttcaaagaaa ttttttataa agcagtaacc tcgataggaa ttttcttttt gattgttgcg 377160
attcttgacc ttgtctatca gcgccacaat ttcgctaaag aattaaagat ggagaagttt 377220
gaggttaagc aggagtttaa agacacggaa ggaaatcctg agattaaagg ccgtcgtcga 377280
caaattgctc aagaaattgc ctatgaagac tcgtcatcac aggtgaaaca tgcaagcacc 377340
gtagtctcta atcccaaaga tattgctgtt gctattggct acatgcctga aaaatataaa 377400
gcaccttgga tcattgccat gggcatcaac ttacgagcta aaggatactt tgatgaagct 377460
gaaaagtacg gaattcccat tatgcgaaac gtacctttag cacatcagct tttggatgaa 377520
gggaaggaat taaaatttat tccagaatct acttacgaag ctattggaga aattctactc 377580
tatatcactt cactgaatgc gcaaaatcct aataataaaa atactaacca acctgatcat 377640
ttataatgaa taagctactc aatttcgtca gcagaacact tggtggcgat accgccttaa 377700
acatgatcaa taagtccagc gacttaatcc ttgctctttg gatgatgggc gttgtcttaa 377760
tgatcattat tcctttgcct ccgcctatcg ttgacttgat gatcaccatc aacttatcga 377820
tctctgtatt cttattgatg gtggctcttt atattccaag tgctttgcag ctgtctgttt 377880
ttccctcgtt gctcctcatc actacgatgt tccgcttggg aataatattt cctcttctcg 377940
acagattctc cttaaagcgt atgcgggtca tgtcattcag gcttcggaga cttcgtggtt 378000
ggagggaact atgtggtcgg gttcattatc ttcctcatta ttacaatcat tcagtttatc 378060
gtagtaacta agggtgccga gcgtgttgcc gaagttgctg cccgattccg attggatgcg 378120
atgccaggta aacagatggc gattgatgcg gacttacgag ctggtatgat tgatgccaca 378180
caagctcgtg ataaaagggc tcaaatccaa aaggaaagtg aactctacgg agccatggac 378240
ggtgccatga agttcatcaa aggagacgtt atcgctggta tcgttatctc tttgattaac 378300
attgttggcg gtttgacgat tggggtggct atgcacggca tggacctcgc tcaagcagct 378360
cacgtctaca ctcttctctc cattggagat ggtttagtct ctcaaattcc ttctcttttg 378420
attgcgttga cagcgggtat tgtcacgact cgtgtatcga gtgacaaaaa tacgaacttg 378480
ggtaaagaga tttctactca gctcgttaaa gaaccacgag cactactcct tgcaggtgct 378540
gcaactttag ggttggtttt cttcaagggc ttccctctat ggtccttctc catttagca 378600
ttaattttcg ttgccttagg gattctccta ctgactaaga aatcagcggc aggaaaaaaa 378660
ggtggtggct caggagcttc aacaaccgta ggggctgctg gtgatggcgc tgctactgtt 378720
ggggataatc ccgatgacta ttctctaact cttcccgtaa ttctagaact tggaaaagat 378780
ctctctaagc ttatccaaca caagacaaaa tcaggacaaa gctttgttga tgatatgatt 378840
cctaaaatgc ggcaagctct ctatcaggat atcggaatcc gataccctgg cattcatgtt 378900
cgcacagatt ccccttcttt agaaggatac gattatatga ttctgcttaa tgaagtccct 378960
tatgtgcgag gaaaaattcc tccgcaccat gtgttaacca atgaggtgga ggacaatctc 379020
agccgttata atctaccttt cattacctat aagaatgctg cgggtcttcc ttcagcttgg 379080
gttagtgaag atgcaaaagc tattctagag aaggcagcaa ttaaatattg gacgccgctc 379140
gaagtgatca ttctccatct ttcgtacttt ttccataaaa gctctcaaga gtttttggga 379200
attcaagagg tacgttctat gatcgaattt atggaacgtt cattcccgga cttagtgaag 379260
gaagtcacaa ggcttattcc attgcaaaag cttacggaaa tctttaagag attggttcaa 379320
gagcaaatct caattaaaga cctacgtaca atcttagaat ctctgagcga gtgggcgcaa 379380
actgagaaag atacagtttt gcttacagaa tatgtacggt cttctttaaa gctttatatc 379440

agcttcaagt tctctcaagg acaatcagca atttctgttt atctcttaga tccagaaatt 379500
gaagagatga ttcgtggagc aattaaacag acatcggcag gttcttacct tgctctagat 379560
cctgattctg tgaacctaat tttaaaatct atgaggaata cgatcacgcc aacacctgca 379620
ggaggccaac caccagtatt attgacagca attgatgtaa gaagatatgt acgaaaatta 379680
atagaaacag aattccctga cattgctgtg atttcttatc aagaaatcct accagaaatc 379740
cgcatccagc ctttaggaag aattcagatt ttctaattga tacgttgtcg ctcataggag 379800
gcatatggca gcatcaggag gcacaggtgg tttaggaggc actcagggtg tcaaccttgc 379860
agctgtagaa gctgcagctg caaaagcaga tgcagcagaa gttgtagcca gccaagaagg 379920
ttctgagatg aacatgattc aacaatctca ggacctgaca aatcccgcag cagcaacacg 379980
cacgaaaaaa aaggaagaga agtttcaaac tctagaatct cggaaaaaag gagaagctgg 380040
aaaggctgag aaaaaatctg aatctacaga agagaagcct gacacagatc ttgctgataa 380100
gtatgcttct gggaattctg aaatctctgg tcaagaactt cgcggcctgc gtgatgcaat 380160
aggagacgat gcttctccag aagacattct tgctcttgta caagagaaaa ttaaagaccc 380220
agctctgcaa tccacagctt tggactacct ggttcaaacg actccaccct cccaaggtaa 380280
attaaaagaa gcgcttatcc aagcaaggaa tactcatacg gagcaattcg gacgaactgc 380340
tattggtgcg aaaaacatct tatttgcctc tcaagaatat gcagaccaac tgaatgtttc 380400
tccttcaggn ttcgctcttt gtacttagaa gtgactggag acacacatac ctgtgatcag 380460
ctactttcta tgcttcaaga ccgctatacc taccaagata tggctattgt cagctccttt 380520
ctaatgaaag gaatggcaac agaattaaaa aggcagggtc cctacgtacc cagtgcgcaa 380580
ctacaagttc tcatgacaga aactcgtaac ctgcaagcag ttcttacctc gtacgattac 380640
tttgaaagtc gcgttcctat tttactcgat agcttaaaag ctgagggaat ccaaactcct 380700
tctgatctaa actttgtgaa gatagctgag tcctaccata aaatcattaa cgataagttc 380760
ccaacagcat ctaaagtaga acgagaagtc cgcaatctca taggagacga tgttgattct 380820
gtgaccggtg tcttgaactt attcttttct gctttacgtc aaacgtcgtc acgccttttc 380880
tcttcagcag acaaacgtca gcaattagga gctatgattg ctaatgcttt agatgctgta 380940
aatataaaca atgaagatta tcccaaagca tcagacttcc ctaaacccta tccttggtca 381000
tgattaaaaa aggattgcca tgcaaaacca atacgagcaa ttactagaat ccttagcacc 381060
cctattaaat acgacacttg ctccagataa aaataactct tgtttaatcc gtttcagcga 381120
tacccatgtc cctgtgcaaa tagaagaaga tggaaattcc ggagatcttg cagtatcgac 381180
actactaggt actcttcctg aaaacgtatt tcgcgagcgt attttcaaag ctgctctctc 381240
tgtaaatggc tcgttccaat ccagcatcaa gggaattcta ggctacggtg aggtcactca 381300
acagctctat ctttcagata tcctgagtat gaactaccta aatggagaaa agttattcga 381360
gtatctcaag ctcttttctt tgcatgctaa gatttggatg gaatccctaa gaacagggaa 381420
tcttcctgac cttcatgttt tgggaatcta ctacgtcgcg tgaatgtttt aaaatacaca 381480
aaacactcac cctcagcaca tgcttggaaa cttataggaa cctctcctaa acacgggatt 381540
tatctcccac tattttcaat acacacaaaa aatagctgtg gaatcggtga atttttagat 381600
ctcattcctc tgatctcttg gtgccaaaaa cagggcttca gcgttattca gcttctccct 381660
ttaaatgata ctggtgaaga tacgagtccc tataacagca tctcttccgt agccctgaat 381720
cccctattcc tttccctatc ctctcttcca aatatcgata ccatccctga agttgccaag 381780
aaacttcaag atatgcatga gttatgctcg actccatcag tcagctatac tcaagttaaa 381840
gaaaaaaaat gggcattctt aagagagtac taccaaaaat gttgcaagtc ttccctcgaa 381900
ggaaactcaa attttctgga gtttctagaa agcgagcgct attggcttta tccctatggg 381960
acctttcgtg caatcaaaca tcatatgcac ggagaaccta ttaataactg gccgaagtcg 382020
ctcacagatc aggagaattt tccggactta actaaaaaat tccatgatga agtcctcttt 382080
ttttcctatc tacagtttct ctgttaccaa cagctctgcg aagtgaaagc ctatgcagat 382140
caacaccacg tcctgcttaa aggagacctc cctattctta ttagcaagga tagctgtgat 382200
gtttggtatt tccgagacta cttttcttca tcaaggtctg taggagctcc tcctgacctc 382260
tacaattctg aaggacaaaa ctggcatctg cctatttata atttttcaca acttgccaaa 382320
gacgactaca tttggtggaa agagcgtctg cgatatgctc aaaacttcta ttccgtctat 382380
cgcttagatc atattatagg atttttccgt ttgtggattt gggattcttc aggaagagga 382440
aggttcattc cagacaatcc taaagactat ataaagcagg gcacggagat cctttctact 382500
atgctcggag cctcttctat gttacctatc ggagaagatt tagggattat accccaagac 382560
gtcaaaacga cattaacaca cttaggaatc tgtggaaccc ggattccacg atgggaacgc 382620
aactgggaaa gcgacagtgc cttcattccc ctaaaagatt ataatccact ttctgtgacc 382680
actctctcta cccacgactc tgatacgttt gcccaatggt ggctcaattc acctaaggaa 382740
gctaagcaat ttgctaaatt tctacatctt ccttttcaaa aaaccctgac tacagaaact 382800
caaatagaca tcttaaaact ttctcatgaa tcagcatcta tctttcatat caacctcttt 382860
aacgattatc tcgccctctg ccctgattta gtatcaaaaa atctacaaag agaacgcatt 382920
aatacacctg ggacaatttc taaaaagaat tggtcgtatc gagttcggcc ttccttagaa 382980
gaactcgcta ttcataaaaa atttaatggt tacattgaga agatccttac aggactgtaa 383040
ggatagcaat aaaacattta agtcttttta tagtaagaac cttataataa tttcctggaa 383100
cgaccgtctt tcttaaaaga attccttcat tatcaacaac agacaatcac attttctaag 383160
aaaacctctt ccccatatat tcgaaaagct ctagtataga ttccttttca tagagttgct 383220
ttattgagcg gatgttagag aattcatagg aagaatatgt caagaaagtg cccacttaca 383280

ggaaagagac ctcgccgtgg ttatagctat acacttcgag gtattgctaa aaagaaaaaa 383340
ggaattggtt tgaaagtgac agggaagact aaaagaagat ttttccctaa tatgttgacc 383400
aagcgtctat ggtctacaga agaaaaccgt tttcttaagc ttaaaatttc tgctagcgct 383460
cttcgtcaca ttgataagct cggattagag aaagttctcg aaagagctaa aagtaaaaat 383520
ttttaattta acttaagtat agggaaatat ttatgtcttt cttaaggcgt catatttctc 383580
tttttcgttc acaaaaacaa cttattgatg tttttgctcc cgtaagtcct aacctcgagt 383640
tagctgagat tcatcgtcgt gttattgaag atcaaggccc tgcccttctt tttcataatg 383700
tcatcggatc gtcattccca gtcctgacca atctctttgg aacaaaacat cgtgtagacc 383760
aacttttttc tcaagctcct gataacctca tcgctcgagt tgcccacctt atttcttcta 383820
caccaaagct ttcttctcta tggaaatctc gggatctatt aaaaagaata agctctttag 383880
ggctcaaaaa agctcgattc cgtcgctttc cttttgtttc tatgtcctca gttaacttag 383940
atcaccttcc cttactcaca agctggcctg aagatggtgg agcctttctc acacttcctc 384000
ttgtctatac ggaatcgccg actcttacta cacccaatct tgggatgtat cgcgtgcaac 384060
ggttcaatca aaacaccatg ggcctccatt ttcaaatcca gaagggcgga gggatgcatc 384120
tgtatgaagc agagcaaaaa aagcaaaacc ttcctgtttc ggtatttttg tctggaaacc 384180
cctttttaac cctttctgcg attgcccccc tacctgagaa tgtctcggaa cttctctttg 384240
ctaccttcct ccaaggagcg aagctccttt ataaaaaaac aaacgaccat ccccaccctc 384300
tactctacga tgcggaattc atcctggtcg gagaatctcc ggccgggaaa cgtcgtcctg 384360
aaggtccttt tggcgatcat ttcggatact acagtctcca acatgacttc cctgaattcc 384420
actgtcataa aatctatcac agaaaagatg caatctatcc tgctacagta gtcggcaaac 384480
cctaccaaga agatttttat atagggaaca aactccaaga atacctctcc cctttatttc 384540
cgttagttat gcctggtgtg cgtagactta aaagttacgg agaatcaggg tttcatgcac 384600
tgactgcggc tgtcgttaaa gaacgctatt ggagagaatc tctaaccaca gctcttagaa 384660
ttcttggaga gggccaactt tccctaacga aattcctaat ggtcacagac caagaggtgc 384720
ctctcgacag gttctccgtg gttctagaaa ccattttaga gcgtctacag ccagaccgag 384780
atcttattat tttctcagaa actgcaaacg atacgttaga ctatacagga ccaagcttaa 384840
ataagggctc caagggaatc ttcatgggaa taggaaaagc catccgagac cttccccatg 384900
gatatcaggg aggaaaaatc catggagttc aagacatcgc tcccttttgt cgtggttgcc 384960
tagtgttgga aacatccctc gaggaccgat gtattaaatc tctccttcac catccagatc 385020
taaaatcatg gcctctgatt atccttgccg ataatctgag agaaaccatt caaagtgaaa 385080
aagattttct ctggaggacc ttcacacgat gtgccccagc aaatgatctt cacgcgctcc 385140
acagccattt tgctactcac cgtcctaatt acaactttcc cttcgttatc gatgccctga 385200
tgaagccttc ctatcctaaa gaagtagagg tcgacccatc tacaaaacaa aaggtttccg 385260
aacgatggca cgcatatttc cccaataaag aaacttttta tatttaataa gaatcttatt 385320
ctattaaacg tttaattaaa ttagttattt ttatttttaa aaatatataa aaacaaaaaa 385380
gctattttaa gagtaaaaaa tgaataaaag acaaaaagat aaattaaaaa tctgtgttat 385440
tattagcacg ttgattttag taggaatttt tgcaagagct cctcgtggtg acacttttaa 385500
gactttttta aagtctgaag aagctatcat ctactcaaat caatgcaatg aggacatgcg 385560
taaaattcta tgcgatgcta tagaacacgc tgatgaagag atcttcctac gtatttataa 385620
cctctcagaa cccaagatcc aacagagttt aactcgacaa gctcaagcaa aaaacaaagt 385680
tacgatctac tatcaaaaat ttaaaattcc ccaaatctta aagcaagcca gcaatgtaac 385740
tttagtcgag caacctccag cagggcgtaa actgatgcat caaaaagctc tttccataga 385800
taagaaagat gcttggctag gatctgcgaa ctacaccaat ctttctctac gtttagataa 385860
taatctcatt ctaggaatgc atagctcgga gctctgtgat ctcattatca caaatacctc 385920
tggagacttt tctataaagg atcaaacagg aaagtatttt gttcttcctc aagatcgtaa 385980
aattgcaata caagctgtac tcgaaaaaat ccagacagct cagaaaacca tccaagttgc 386040
tatgtttgct ctgacccact cggagattat tcaagcctta catcaagcaa aacaacgagg 386100
aatccatgta gatattatca ttgatagaag tcatagcaaa cttactttta agcaattacg 386160
acaattaaat atcaataaag actttgtttc tataaatacc gcaccctgta ctcttcacca 386220
taagtttgca gttatagata ataaaactct acttgcagga tctataaatt ggtctaaagg 386280
aagattctcc ttaaatgatg aaagcttgat catactggaa aacctgacca aacaacaaaa 386340
tcagaaactt cgaatgattt ggaaagatct agctaagcat tcagaacatc ctacagtaga 386400
cgatgaagaa aaagaaatta tagaaaaaag tcttccagta gaagagcaag aagcagcgtg 386460
atgatctaaa atagtcacag aagaaggcct agctcgtgat tcaagtatgt ctaggcctct 386520
tcatcctttt gataaaaaag agtggaggca gtttctaaga ctcctttatt tctagcaatt 386580
acccaagctt ctgctgctgc tgcaattgcc tgagaagcgg cagcccctaa agagatgccc 386640
ttttttttct ttgtaacttt aatagatcgc ttctgtttgt ttttttcgtc tttttcctga 386700
tcttcttttt tcttactatc gcaatatagt tttttaaaat ctaaatgcac atgacgcaac 386760
tgagaaagca agaccttggc ttgcttttgt ccgagtagct tataacgatg cctctgtttc 386820
ttctcctctt gttcgcgatc tccaatagct agcaaggcaa gccgaatcgc ttcattcgta 386880
gcaatttcct cagaaatttt ttctactaaa ggagagtttt gacgggaatc tttagctatg 386940
ctttgtgcag atagcgcacc ataaggatca aaacgaatta tacgcatcac atcaaaccga 387000
cttaaaaccc taaatatccg tagataattt ttttcccttc ttttcctgac gggaagcttt 387060
ctggcccgaa gatcctcttc tttgatctcc gctaaaacca ccactatttc cccccccacc 387120

atcgtcatga actgaagcag cttcaggctt tccttgaacc gatagacgtg aagatcctga 387180
aaacggctgg ggagaacgga gaacaattgg cggctcaaca gcaacatttc gatactgata 387240
aatcaaagga tctggaggac ctccaggacc tttatgagct tcgagcttct ctttatagtc 387300
atcagtagca cgaggtagag gagcgagtaa agacactcca cgttgcgcaa ggaaatgata 387360
tgaggaactc acagcatcac ttcctaaagc ccctcccgta ccactttcct tagggaaaac 387420
actctccaca gcaataggga gaaagttttg actgctattc tctggatttt tgaatgccgt 387480
agattcttta acaacggctt caggttcctc cctggaaacg atgctatctt taggaaaacg 387540
atacatactt tctggatgag gcaaactttt aacctcagca ctcttcacag taccttcccg 387600
aagaggagaa aagttcgtat ggttcgtact cttctctatt cctggagatt tatgctttga 387660
tttatctccc gatctctggg tgggtaaagt tcctgaaaac ttgggaggag gaaatactgt 387720
agaaggacca gaccaaacag aattactcaa tacctcttga ggcgctgccc ctgtaagaag 387780
cccagccata gcacgtgcaa gcatcatctg atcctcactc aactgctctc gactttcttg 387840
tttctctgct tccttttccg ccctttctaa agtattagaa tttggaattc ctgttggatt 387900
cgcttctgga gaatcagang aagatacctt tacttttcct tctactgctc tgggagatgt 387960
catggctgta ctganctctt cctgtcctaa atcaganaga tctatcatgc cttcgttgcc 388020
tgttgccatt gccctgcgca ctaatgatga ggtctcaaca ttgggatcga gaagagagct 388080
gaccatctcc cctcctaaac gagccactgt ccaagcattt accttctcct cactcatagg 388140
ctctatggcc ttttggaaag atccaaacga agccacggaa ctagattctg aagagcttaa 388200
acttaaactc ctggcacctg aaaatgctga agatttcaaa gcctctaaag aaagctgaga 388260
aatatcagag gaactccctc ccatacccga agcatctaag aaaaatccct gcatttcagg 388320
tgaaatacgg tttcccttct ttagagcaac atccatctct gtcggcaata aggatgttcc 388380
ctcaccacta tcggaagcac gctttgctga cactctcgaa gcccgagatc cgaatcctga 388440
catgaatcct tgcacactag cgcgcatttt agaaaatgta cctttcaatt tggattttgg 388500
agaggttgat gatcgatctt gagctttacg atacttagcc ttatttatct gagattctgt 388560
tgcatacatt cctgtagatc ctgaacgcac taagctttcc tgcttcgcag cagaggaggc 388620
ctgcttggtt tcagaaaata tagattcttt tagcggagac gggccttctg cctgctctcc 388680
ttgcagagca ggattccact ttcctggatc cgaagaaggc tgaacccctc cgccacctga 388740
aactgccata atgattctct gaatttaaaa aataaaactt ataactactt ataattttaa 388800
aacaaaaaat aattaattta aaaaaaagaa cttacaaaca aattttaaaa atctaaaaaa 388860
caaaaaaaaa cgcaaggtga aaacaccttg cgtaaaaaaa cttatgggag gacagagaac 388920
agcgagtaga tgcaacaaag tgcatcgctg tcgagctaga gggatcactc ggatccgact 388980
gtctaggtat taagattttg gagatcttaa taccgagaca gttcctaact ctaaattaat 389040
tacccaggct tcctgtcgtt ccatcacggg atagtcccgt tctgcgattt ttttttaatt 389100
taaccccgcc taaagacgga tagagtactt actaaaatgg tccttgtcag taaaaacaaa 389160
cccattctca taattgaaaa tcaaaacttt atcggtgtcc ttcttctgag aatctaagag 389220
agagcgaact aactgatcgt attcttgagc acggtgttta agagttttct gttccttagc 389280
aataagaaat ttcgacgatt tttagctttt atccgcgcag gcttcttcaa cacttccgtt 389340
gatttctttt gaatctcctg agccaaagcc ctatccgttc tgctcttctt aggcttcccc 389400
ataaaaaata caaaaacaaa aaaatcaact taataaacta attttaatta aaaaacttat 389460
taagaataaa ctaattttaa tctaaaaata aaatattata taaataattg tttaaaagca 389520
gtttagataa acataaaaaa tcaagctaag aatcctatgc tttgctatat acaagtctgc 389580
aaaacctttt gaaatcgata atcataattc aaagcgtaaa ccatgtctgt ccacataaca 389640
ccgcgcaaat gctttatttt gtgtatttta tccatgttca cccttccaac gctcttccct 389700
aaagcacatc tgatcctttt ttccccttat attgttcttt gtttctattg tttctcaaaa 389760
gataagggac tggtactcgc tctaggctgt ggtgtcttaa gtgatcttgc cttaggaagc 389820
cgcggtgtat ttctactgct ctaccctcta actgctctga tcacccataa ggcacacctc 389880
attttttcaa aagagagcaa agccgccttg gtcattgtga atatgatttt ctatggagtc 389940
tttttactcc taaccattcc tatgtgcgcc ttgttcggac atgaagtccg ttggtcaata 390000
gatgtgctaa tgatacctct aaaatgttct ttcttagata atctcatctt cacttctgta 390060
atctatatac ttccttgcgc aataaactca ggaatccata aaatgatatc tttttttagg 390120
agattggtat gttactgaga gggattcctg cagctgaaaa aatccttcag agactcaaag 390180
aggaaatctc acaaagtcct acctctccgg ggcttgctgt ggtcctgatt ggcaatgacc 390240
ccgcatctga ggtgtacgtt ggcatgaaag tcaaaaaagc tacagaaatc ggaattatct 390300
ccaaagcgca caagttaccc tctgactcta ccctctcctc agtccttaag ctcatagaac 390360
gattgaatca agatcctagc atccacggca tcctcgtgca acttcccttg cccaaacact 390420
tggacagcga agtgattctc caagcgatct ccccagacaa agatgtggac gggcttcacc 390480
ctgtgaacat gggaaagttg ctccttggaa attttgatgg acttctaccc tgcactcctg 390540
caggaattat tgaactcctg aactattatg aaattcctct tcgaggccgc catgccgcta 390600
ttgtagggag aagcaacatc gtggggaaac ccttagcggc cctcatgatg caaaagcatc 390660
ctcaaactaa ctgtacagtc acagttcttc atagccagtc ggaaaacctc ccagaaatct 390720
taaagacagc tgatatcatt attgctgctc taggagcacc gctttttata aaggaaacta 390780
tggtagcccc acatgctgtg atcgtagatg taggaacaac aagagtccct gcagacaatg 390840
cgaaaggcta tactcttctt ggagatgtag attttaataa cgttgtgaca aaatgcgcag 390900
aatcactcca gttcctggag gcgttggtcc catgactgtc gctatgctca tgagcaatac 390960

atggcgatgt taccaaaatt tttcttagtt cttttatgtc ttggactctg ttcatgctct 391020
caaaaaacga caacaatcga aggagagcag atgacaatct tctatcgcat tgttctggga 391080
acctctttat ccgcaaaaga aaaagcatct ttatcccaac aaattgatag atgctttcat 391140
aagatcgact cgatttataa caactggaat ccctattctg aactctcgat aatcaaccga 391200
gctccagcag atgtccccat aactttatct gtagaacttt ccgagtttct agatcaggta 391260
gatacacttt acaaactttc agaaggacgt tttgacccta ctgtaggacc tttaaaaacc 391320
ctatggcttc tacatctcaa aagtcaaacc ctccccccta aagacgtttg ggaacaacat 391380
tataaagaca tgggctggca acacttggag tttcagtcaa acacaaaaac tctaatcaaa 391440
aagaatcctc atgttcaaat cgacctctgt ggtgttgtca aaggttatgc cgtagattgt 391500
ctaaatgaaa tttgcaatac cttttgtccg aacaactatg tagagtgggg aggagagatc 391560
aaaacgtcag ggcatcatcc ctcgggaaga ccttggcgta ttttttctga agcagcaggt 391620
acgatcttag atatcgatga tatggcaatt gcaacaagtg gaaatcatat tcaaaaatgg 391680
tgtgttgaag gaaaaattta cacccatatt cttgatactc gtacagggaa acccctagag 391740
ctaagctcct atcctatcca aagtgtttca gtagtccatc cgactgcgca tacgccgacg 391800
ctattgccac agtcctcatg acttttgatt ctaaaataga agcaaaacag tgggctgaag 391860
aacaccatat cctaacctat atcaatgatg gcgcctcttc atagcagcgg caacttcacg 391920
ttccttttct ctttctatga tcgtacgacg cttatcataa gcttttttcc cacgacaaca 391980
acccaaacgt accttaacat agccgcgact cagaaacatt cccagaggaa tcaaagtcat 392040
gcccttttga gcaatcttac cctctaactt acgaagttca tatctatgaa gaaggagttt 392100
acgtttacga cgctcctcat ggttatagat atttccaaac cgatagggag caatactcgc 392160
gtttaataac cacccctcac ctttagaaac aatgacataa gcatcaccga ggtttccccc 392220
atgatcgcgc aacgacttaa tctcagtccc agtcaaaacg atgcctgctt ctaaagtctc 392280
tataacttca tagttacgca gagccttgcg attagaaaca atttcttttt gtgccataag 392340
atcctccccc ataattgggg acaaaaacca gtataacaaa aagcaatttt tccttcttaa 392400
gacaatgatt agaatcctct ttccttgatt tccagggaag atttcctcaa tctaaagcct 392460
tttttattag taaaatatct agaagaccag aaaatcactt tgtagtaata tcccgatcgt 392520
acctctaccg tctctcctag agaggcgtag cccttcatat aaagttagac tcaggttata 392580
ggaaaatatg aaattcgttg tatcccgaaa tgagctagga aaccttatca aaaaaattca 392640
aagtgtcgtc cctcaaaaca cacctattcc agtactcacc catgttttga ttgaaactta 392700
taatgatgaa ttagttttca ctgctacgga tctgacagtg agcacacgtt gcgtcaccaa 392760
agctaaagtc tatgagaaag gcgctatttc cattccctcc aagagatttt ttcaattagt 392820
aaaagaatta acagaggcaa atttagaaat ttcctcttca gcaggggaaa tggcacaaat 392880
cacctcggga tcttcatatt tcgcctactc agcatggaaa aagaagactt ccccatgctc 392940
cctgatatac aaaatgcttt gcgtttttcc ttgcctgcag agcagctaaa aaccatgcta 393000
cagagaactt cattcgctgt atctagagaa gaaagccgct atgttcttac tggagtcctg 393060
cttgctatcg ccaatggcgt ggctaccatc gtagggactg acggaaagcg tttagcaaaa 393120
atagatgctg aagttacttt agataaaagt ttttctgggg aatatattat tcctatcaaa 393180
gcagtagaag aaattataaa gatgtgctcc gatgaaggtg aagctacgat cttcttggat 393240
caagataaga ttgcggttga atgtgacaat actctcctga tcacaaaact tctttctgga 393300
gaatttccag atttctcccc cgtcatatct acagaaagca acgtaaaact cgatctgcat 393360
cgcgaagaac taattactct gctcaaacaa gtggctttat ttacaaatga gtcctctcac 393420
tccgtgaagt ttctttcttt acccggagag ctcactctaa cagccaactg tactaaggtg 393480
ggtgaaggaa aggtaagcat ggctgtaaat tattctggcg aactcctaga aattgccttt 393540
aatccctttt tctttttaga tatcctgaag catagtaagg atgaattagt cagcttaggg 393600
atctcggatt cctataatcc tggaatcatt accgattctg cctcaggatt atttgtcatc 393660
atgcctatga ggctacatga tgattaataa actccctaag gagaatcctt taggtcacta 393720
ccctgccgat gtttatgaaa atctgctctc tgaagctaaa aaattttcgt aaccacagtg 393780
atttagaaat ctcactggct cctaaactca attatgccca aggaaaaaca aacctcctag 393840
aagcgcttta tgttttgtcc ttgggaaggt cttttcgcac gcaacatctc acagatacca 393900
tcaccttcgg atcttcccat ttcttcttag aaacacagtt tgagaaagac caccttcccc 393960
aagctctctc catctataca gacaagcaag gaaaaaaaat ctgctataac caacttccta 394020
taaaaacctt atcgcagctg atagggaaag tacctattgt gcttttctct tcaaaagacc 394080
gccttctaat ttcaggagct cctgcggatc gtcgcctttt cctaaatctg cttttatctc 394140
aatgcgataa ccactatacc ctctgcttat cgtactatca tcgcgctctt cagcagagaa 394200
atgctctctt aaaaagcaag caaacctcaa ccgtggcctc tgggatgaac agttggtcaa 394260
acacggcacc tacctatcca tccaacggtt tctctgtagt cagaaacttt cagatttatc 394320
caaagaactt tggtctaaca acctaaaaga acaattggcc ttaaaattta aaagttcctt 394380
aattaaaaat tctgatattt ctgaaactgc tgttgccgaa gaatttcata aacagctctc 394440
tatatcactt cctagagatc tcgaatgggg aagcacttcc gttggccctc atcgcgaaga 394500
ctttctactc actatgaacc aaatgcctgt gtctcaattc tctagtgaag ggcagaaaca 394560
cagtcttttg gcaatcttaa ggcttgctga gtgcctatat ctaaagcaat ctcatcacgt 394620
ctcccctcta gtctgtctag atgatatcca tgctggatta gataatgaac gtgtcggtca 394680
actccttgac cctgccccaa ctctgggtca gactctgatt acttccaccc atatgcatgg 394740
ggaacttcca aaaacaagcc ttgttttaag tatcgagaac gctcaagttt ctgagcaaat 394800

tatctaaaac aataacatca tttttctttt gcgttaaaag taagcgaatt agttattttt 394860
ataaataagt tttaataaac atattctttt ttaataaaaa acttatttaa aataattata 394920
tcggtgacac atgaagaaat ttttattaac tatactcttt ttagctgtgg gtaatccttt 394980
attctcggaa acctcggtaa tccaaaccct tccatctgga attgggggat taaaggaaac 395040
ctcaaaacaa aaagaatccg tggtctgcgt gcatgcgttt ttaagatctt atacatcttt 395100
aaaacctatt gctcgcgttc tagaaaaaga acattacgat gtctttattt ggaattatga 395160
gacgcgcaag tttactctag aaaagcatgc tgaacatctc aatcgcttgc tgaaaaaaat 395220
agctgaactt aagcctggag tccctataaa cttcgtaact cattctattg gaggagtcat 395280
tgttcgtgcg cttgctgaaa aaaatagctg aacttaagcc tggagtccct ataaacttcg 395340
taactcattc tattggagga gtcattgttc gtgtagcact cgctcaccct gattgccccg 395400
aagaagccaa aaaaggaaaa gctattctca tggctcctcc gaacgcaggg tctacactag 395460
ctagacgcta ccgctgtgtg aaattcgtac agttcgtatt tggaggaaaa ttaggacgac 395520
agcttcttac ctactgcccc acaaagatgt taaatgtcgg gaaactccct tcgtctttag 395580
acgttctcat tcttagtggg aacagacata gcaaattcct tcctttccgc ctgccctatg 395640
aaaacgatgg taaggtatgc actatagaga caaagctaga tactccacat aaagcttacg 395700
tgatccacac gagtcatacc tacatcatta ctaatcggaa gtcgctctat cttatgaaag 395760
agtttttaaa agaaggaaat acaaccccga taatcgagca cgttcccgaa gcagctttag 395820
aacaaactgt tatggaagac aaacaaaaga actcaagact taagccttac cctaaccaag 395880
acatctacgt tatacactgc tttggttctc gtccttacaa cctttacgga tttccaaaaa 395940
aatggagcct taaccaaaaa aacgaaataa atcctgaaaa gttagaaaaa taaagaagat 396000
gatcaataaa aaagtgaacg gaaccaaaat cctttccaca atcttcatat tcaaacgtct 396060
tcttgtgact atctcccaca aacatagaag gatataacct ccatccaaaa caggaatagg 396120
aagcaaattc aagacagcca aattcatact aattagaccg atccaaaaga gcacttcaga 396180
aaaccctacc gaccatcctg tatgtaaaac ctgcacaata cccacaggtc ctgaaagcca 396240
ttgtggactc agatgtccag taactaaagc tttcaaggtg atcaaacttt ccttagtaat 396300
atttgataac ataaccacag gtgaaggatt atacctcacc ttaagatctt tcaaagaaat 396360
ccctaaagat ggtttttgct tctcagcatc aagacgctcc aaatagtatc tttgtttatc 396420
cttgttctta atcttcttag ctacttccaa ctgtttatcc aaactctccg aagaataaac 396480
atcaatccaa ggacgaggct gaacagggtc aagaagacga taaggacccg cgacttctac 396540
tgggtgagac tctcctaaat ggttcaaaat ttgtaacaga tcttcggaat gataagaggc 396600
gataaaccgc ttatcagcat ctcgagaatt cacctcttca agttcttgcg gactcatctg 396660
ctgaacaata atagagaccc gatggttctg aacaagacgt aaaatatcta cacttccaga 396720
aacaggagtt ccatcaatag ctagaatgcg atccccaagc tgtagcctct cttgaggttg 396780
tggcaaagga gactctggat ctatagcagt aagttcacct tctatgtatc cataactatt 396840
gattacataa ggcaatgtat ataacgaaga ccacttgcct ttaagtccag cctcatactg 396900
cgtatctata agctcattac gaaggtaggg agtgtaatgt aaaacggaag ccaataccct 396960
aggttgacga gaaaagaaga ttttgtcatt ccgtgctact ttcacaaaag cataagactc 397020
attgagtatc tgagatatct gagccattga gaaaagaagt gtgccatcca tccaaacgaa 397080
acgatcattc ggacgtagct ctgaattctc cataggagag ttcttcgtta ggggcacctg 397140
gttgccatac aaaagataac tcgctccaga acagggaacc ccgaattttg tgggatcaaa 397200
ctcaacatca atagcgaact ctttgctagg aactgtcaaa tagccaggac gtttgatttc 397260
tagattgaga tgcccctcta ataaagaggt tgttagcatg tccttatctc ccacataagg 397320
cttaccatta cacgtaagaa tctcgtctcc agggagcaat ccttctgcct gtaaaacagg 397380
atggacccaa cctaccactt tagaacagtc gctataattt ttacttcttc ccccattcat 397440
gtaaagaatg ctgaaagcca agacagctaa taaaatattg gcaagaggac cagcaacaag 397500
aaccagaatg cgtttccaag gagacttact aaaaaatccc tgaggaatat catagacaga 397560
gtctatcttc cccttctccc ctttttcttt ggtacgttcc atacctctga tacgaacata 397620
gcctccaaaa ggaatgcatc caatgcgata ttctatgccg cctatacgct ttttaaataa 397680
agcaggacca aagcctatgc taaaactctc tacagccatt cctacagctt ttgctactac 397740
cagatgacca agttcatgaa ttaacactaa aatccctaaa gctagggctg ctagaataaa 397800
atagattatt gtcatatacc tactcgatta tatttcttga gcaagagctc tagcctcacc 397860
atctacttct aaaatatctt ctaaagagtg gcaggcataa accttatgac attccataag 397920
agtcgttaat ttgcgtaaaa tgtcacacca agaaatctct tcgcaaagga acctccgcac 397980
taatacttca ttggctgcat taaaaaagct tccagaagac ccctgtttct ctaatacctg 398040
ttgtgctaaa cggatactag gaaatcgctc ctcatctacc ggaaaaaatt ctaaagtttg 398100
tttcttcgaa aaatccatac catccctagg agatgcaaaa cgctctggag ctgttaaagc 398160
gtattgtatt gggaagagca tatcaggcgg attcatgata gaaatcacac tcccatctaa 398220
aaactctacc ataccatgga ttaagctctg aggatgaatt acagccagga tttcaacatt 398280
ttctaaacca aacagccaat acgcctcgat aatttcgagt cccttattga ccaatgtgga 398340
tgagtccaca gtcacttttg aacccatatt ccatatagga tggttcaaaa catcttgttt 398400
tgttacacaa gaaagctctt ctaaagactt gttgagcaga ggccctccag aagctgtaag 398460
aatcagtttc ttgattccct caatcgtcct gccttctaaa cattgataca aagcattatg 398520
ctcgctatca ataggaagaa cttttatacc atttcctttt gcagtcttag aaaccaattc 398580
gccagcacaa actaaaattt ctttgtttgc taaagctagt gcttttcctt ttttcatcga 398640

ctctagaatc gcgggtagcg cctcgattcc tgaagaagca gcaacgacag tagtgactgt 398700
atccatgata caaagttggg ttaaaccctc ctggcctagg aaaaattgca tatgggggaa 398760
tcgctgacag gcctcgttat aaacctcttc gttatagacc gctgcggcta acggagcaaa 398820
ctcctgnagt tgctgaaaaa taaccttaga ttatttccat aagaagccat agaaataatt 398880
ttaaattctg aaggatagcg ccgcacaatc tctaatgttt gacggccaat actacctgtt 398940
gacccaagaa cggctaaatg tttcaagcat gctaccttta actaggtgaa aaagagaatc 399000
catactccgc aagtcgagga attgtcaatt ctctattgat actatttcta actaaagtgg 399060
actaaactct aaaatttcat tcttttaaat cactgctaat attgagaact tttctcactt 399120
attttttcaa aatttttaga aaaatagcct cgaaaatagc ttataaattt aaccacgatg 399180
tacatcatcg tcaagaacac tacagagatt cccgaagtcg ataaccccaa atcataagca 399240
ctaagaattg ctctcgaaga tgcaggagct aaaaatgctg tancaatact aaaaactaac 399300
gaccaagcca taagactcct tatcgatttt gcaataacct tagcaataag cgatggaatg 399360
atcagaaaag caagtgccat taatacacct acagccttaa aagctcctac aagacatgca 399420
gaaagttgaa aaataatcaa ataatcaacc aaccgaatag gaattcctaa agaagaggca 399480
aatacagaat cgaaagaaga acaaactaag ctacggaacg caaaaatagt aattacagca 399540
ttagccaaaa tcacaatagt gacagggaaa atatcctctt tcgttaaaga atctgcgttt 399600
cctaacacaa gctccgttcc tatatgagca ttctttgtca taaagactaa caaaacaagg 399660
ctcagagaga ataataaaga aaagactaga gcggtgctgc tctcttctga aactttaaaa 399720
gtattacgaa taaagtaaat aagaaaccct gtcagcatag ctgttgccat tgctgcaaga 399780
gtcaaggtac ccaaagagag ggtcgtcagt tgatgcgtaa acaaacaaac acagaccaaa 399840
ccaaaaagga cagtatgaga gacagcattc gcatacatag ccatctttcg caagactaaa 399900
aaagttcctg caaaagcacc tgaacaggaa atagcaagga atactataat ctgaatatca 399960
tcaatataga gagaacccgt gaaaagactt ccagaaaaca gtctcgaaaa aaatactgaa 400020
aaaaattgga aaaaagatac tccataataa ggagaaggtc ccaaagccat tagacttcct 400080
tttttttatt tgggataatt tgtcgatgag gatcataaca aggatcattg agaatctctg 400140
tcaaggtatg atccaattct tcagtaagaa catgctctat ttcctcagcc aactcatgaa 400200
cactttcctt gctaaaatct aaagaattca caagatacga ttcccataat ctgtgagcac 400260
gaactaatct taaggcctca cttcttcctt tttttgtgag tcgataataa tcttgttctt 400320
ttttaacata accccgccat tctaaaatct gaactctcca tctagggaaa ggcttaggcc 400380
caaaatactc ctgatactta taactacaga caaaatctcg aacactaatg ttctctaaac 400440
gattatgaga aatatgccaa aacaccttta aaaggtgttc ttgatccttt gaaaacgaaa 400500
agtgcttcct acggacaaaa cgaatgaccc acccagattt tggagaaaaa agcaaacata 400560
gaccggccaa taatccagca caaatgacaa ccaaaggtcc cgtaggcaag gttacaggca 400620
ccgcctgttg ccctataata gcacgacatg tgaatgctac agagatatag cttcctaaag 400680
ctccgctaat ccctccaaag aatgcagaaa ggataagaat tgtacttaga cgatcggaaa 400740
gctgacgagc acctaaagaa ggagccacaa acatagcaga aattaaaaca atccctacgc 400800
ttcgaactcc acttacgatc accaacgata taaaaattag actgagtgct tcataaagaa 400860
cagtctttaa gccacaagta acagcaaaat ctttatcaaa agtagtcaca acaatttgtc 400920
gataccacca ccataaagca aataacgaag cacaaaagac gatcgcagcc aacgtagctt 400980
caagaaaacc taaagtggct gcttgcccat atagataggc gttaatgcga ttgtatagcg 401040
tagggctact ttccttgaca taactggcta aaatcactcc gatagcaaag aataccacaa 401100
gaacaaaaca aagggcggag tctttatgta atttacatac tttccctaag aaaacaatga 401160
tccccataac caaataccga agcagcacac ccaaacaaca caatccaaaa aatagaagct 401220
tgcaatgaga aaacatattg cgccatcaaa gctccaacta gaagtcctgg atacgacgcg 401280
tgagataaac tttcgcttaa aagaggctgc ttgctaatca agagaattgt cccccacaaa 401340
gctgtggtca tacaaatcaa agtgacagct aaaaaactag ataagaaaat cgtatcagaa 401400
aaaacacaac tgagcataat cagcacgatc caaattgttt tcctcgagag agcttcaggg 401460
tttgttccaa aagttcaatt tcacaaccat acgtttggaa aatagtgtct ccattcagac 401520
attcatcagt agggccacaa caaatcaaac gcttattcaa taaaaccaca tgatcaaata 401580
gttgacgcac atgactcaag tcatgatgaa caacgacgat agtctttccc tgatctcgca 401640
gctcttgcaa aacccctaca gatgttttaa acgaagccat atcaatcgct gaaaacaact 401700
catccataag atatagatct gctttttgca tcaaagcacg tgctaaaaat gctctttgtt 401760
gctgtcctcc tgagagctgt cctatttgtc tatctgctac ggattccaaa ccaactcttt 401820
ctaaaatatg aaaggcctcc cttcgatcat ccgaagaaat tctcccccac attcctttat 401880
agctgtaaca ccccataagg gctaaatcta agacagtcat tggaaaatcc caatccacgc 401940
tagctctctg aggcatatag gctatgcgct gacgcacctt cttaaatttt tgattaaaaa 402000
aataaacagt ccccgaagag ggtttgatca ggcctaagga agcctttaag agagtgcttt 402060
taccagctcc attaggacct aaaatagcag ttaatgaccc ctttcccaag gaaaaggata 402120
tgtgataaag aacggctgca tgctcatagt ttacacaaag gttgtgtaca gaccaaaaag 402180
tctcatcttt gacattcaag agccacccct cctaattctt ctgtgataag gcagacatta 402240
tgtttaaagg tgctaaaata attgtcgtcc acattatcac tatacaatgg tttttgagct 402300
agacgaacta aatgactttt cttcagagaa gaaacaattt ttttcaacgc atcttggttc 402360
agagtatcct cagggaaaac cacactgaca tcatgctcat taatataatc tacaaccgcc 402420
ataatatcac gaacactgat ttgagcttct ggagatagac cctcaggaga aatacaacga 402480

gacctccatg ctccggaagc cacttcttca ggagtagcta aatagcgacg tgtaaagtaa 402540
ctgaacgcat tatgacctga gacaagatac cgtaaatttt caggaattgt gctcaagcat 402600
tgtttcgccc aagaatctaa aatagacatt tcacaaacaa gttcctcact atttgcttta 402660
aattcagcag accattcagg gaacttttca atgagaactt ctgtaatttc tatgacagct 402720
tccttccaaa tagaaagatc catccagata tgaggatcgc aaataccgtc ttcttctaga 402780
ggaacaaagg ccccacgcgc tatcaaccgc tcccctaact tgacactatt gggattattt 402840
tctaaatgct tccgcaaact taatgtatgc tcaagaccca ggccgttaca aaaaattacg 402900
gcacttccag caatcttgtc cttatcccct ttaaccatct catacgcatg agggtctaag 402960
gatcctttga tcaaaacagc ggtagcaagc ctattcccca cgactctttc aacacaatca 403020
tgaatcatgc gattcatgga tagtatacaa ggacgtgaat ttgcattctg aaacccagaa 403080
ttggtacatc caaaagttat accacatgcc acgaaacaga aaatccaacg catcacttta 403140
aatatatatc ccattttcgc atccatctct ctagaagcat tttttaagat ccattaaaaa 403200
ccacttaacg aaccaccaaa catcaaagct ataacccgat ctaaaagatt tattatttac 403260
atttttcgat cccagaaact aatgaaaaag caaagtacca gaaaaacaag ttctattcaa 403320
aatatatttt atccagcaag aaatacaact aatagaaatg aaagaaatat ctttgttcag 403380
gatagtttta aaaaaattag aaaacccgtt tgttttataa gaaaacaaaa tgttatagaa 403440
aaaatcttct ttactttacg atttttatgt tttgaatttt gttaagaaac aaaaagatcc 403500
tgagaggaaa aatattgcaa aaaggagaac ttgcaatata atttatgttc gctgattgat 403560
taaaacagcg ctttaatttt atttctctaa acattaaatt gaaagtatgc cacattcttt 403620
cttgtaaaat atgtaagcat caattataaa aggtgggttt catggccgta gaacaatcac 403680
atataaaaga agaaatagaa aaactgatcg gaaaagctat taaaagagtc tgcggaaaca 403740
aagaaaacga tttatgtcgc tatcttccag gccctagcgg cggttatatg catcatttca 403800
ctctaaaaaa gatgaaaagc gctgctcccg aacaactttt aaaaatgtta aaaacattta 403860
ttttagaatc ggaaacccca cgcacaatta atcctaagcc tagagctcct agaggctcta 403920
aaaaacgtcg tgactttatt aactttacta aaacagatat tgaacgcgtt ttagaactgg 403980
caagacaagt tggagacaaa gacctcctcg ctcgctttag ccctaaaaaa ccgttaactt 404040
ctttaaaaag ggagttaatt cgttcgattc gcaacggtat cgtgagcgta gagctatgga 404100
atgcctacgt cgaagctgtg aaggctgtaa gctctcccaa ccttgaagtt acctctcctt 404160
tcgtttaatt aaaaaataaa ttttacaggc gacttagcaa taaagtcgcc taagaactct 404220
taatccctta ggagtatccc tttcctcttg tcaatagaga gaaaagatgg tatattataa 404280
ggtctttcga aatggaaaca attcaagtta gtccacaacg aaataaaaaa ctatcagaat 404340
agaaaataaa agtatttcag agggtaaata tgacaaaaac cgaagaaaaa ccttttggaa 404400
aattgcgctc tttcttgtgg ccgatacata ctcacgagct aaagaaagtt ctgccaatgt 404460
tcctaatgtt cttctgtatt acatttaact atacggtgtt acgcgataca aaagacactc 404520
ttattgtggg agctcctggt tctggtgcag aggcaatacc tttcatcaag ttttggcttg 404580
ttgtcccctg tgctattatc tttatgctta tttatgcaaa gctaagtaat attttaagta 404640
agcaggcctt attttatgca gtgggaacgc cctttttaat tttctttgcc ctgttcccga 404700
ctgtaattta tccgctacgc gatgttttac atcctacaga atttgctgac cgtttacagg 404760
ccatcctacc tccaggattg ctaggactcg ttgccatctt aagaaactgg acatttgctg 404820
cattttatgt acttgctgaa ctatggggaa gcgtcatgct atctctaatg ttctggggat 404880
ttgctaatga aattacaaaa atccacgaag caaagcgttt ctacgctctt ttcggtatcg 404940
gagctaatat ttctttacta gcttctggtc gtgcaattgt ttgggcttca aagttgagag 405000
cttccgtttc tgaaggtgta gatccttggg gaatttcttt acgtcttttg atggctatga 405060
ctattgtatc tggacttgtt cttatggcca gttactggtg gatcaataag aacgtattga 405120
ccgatcctcg cttctataat ccagaagaaa tgcaaaaggg gaaaaaaggt gctaaaccta 405180
aaatgaatat gaaagatagc ttcctctatc ttgctagatc tccttatatt cttttattag 405240
ctctcttggt tattgcctat ggtatttgca ttaacttaat cgaagtgact tggaaaagtc 405300
agctgaaact gcaatatcct aatatgaatg actatagtga gttcatgggg aacttctcct 405360
tctggactgg cgtagtatcc gtacttatca tgctatttgt tggtggtaac gtcattcgta 405420
aatttggatg gttaactgga gccctagtca ctcctgtcat ggttctccta acaggtatcg 405480
ttttcttcgc tcttgttatc tttagaaacc aagcttctgg gctggtcgct atgttcggta 405540
caactcctct catgctagct gtggttgtcg gagctataca gaatattctt tcgaaatcca 405600
caaaatacgc tctctttgac tcaactaaag aaatggccta tatccctctt gaccaagagc 405660
aaaaagtcaa aggtaaggct gctattgatg tagttgccgc ccgcttcgga aaatcaggag 405720
gagctttaat ccaacaaggt ttgctcgtta tctgtggaag tattggagct atgacccctt 405780
atcttgcagt gattcttctt ttcatcattg ctatttggtt ggtttctgca actaagttaa 405840
acaaactatt cttagcgcag tctgctctta aagaacaaga agtggctcaa gaagattcag 405900
ctcctgcttc ttcatagagt tgcttctctt actcttgttg atccctacct gctttttagt 405960
ggggtaggga ttttttttat taactcccat ttcacgaatt cgtacgcttt tttcaatcaa 406020
aaaaggttat aataaccgtg agacattctg gttgtactat gaagtgtagt cctttaacac 406080
tagttcccca tatattttta aaaaatgact gcgaatgtca tagatcttgt tctttaaaaa 406140
ttaggacaat tgcccgactc attcttgggc ttgttctagc tcttgttagc gcactttctt 406200
ttgttttcct tgctgcgccg attagctatg ctattggagg aactttagct ttagccgcta 406260
tcgtaatctt gattataacg ctagtcgtag cactgctagc taaatcaaag gttctgccca 406320

tccccaacga acttcagaag attatttaca atcgctatcc taaagaagtc ttttatttcg 406380
tgaaaacaca ctccctgact gttaacgaat taaaaatatt tattaattgc tggaaaagcg 406440
gtacagacct gcctccgaat ttacataaaa aagcagaggc tttcgggatc gatattctaa 406500
aatctataga tttaaccctg tttccagagt tcgaagagat tcttcttcaa aactgcccgt 406560
tatactggct ctcccatttt atagacaaaa ctgaatctgt tgctggggaa atcggattaa 406620
ataaaacaca aaaagtttat ggtttacttg ggcccttagc gtttcataaa ggatatacaa 406680
ctattttcca ctcttataca cgccctctac taacattaat ctcagaatca cagtataagt 406740
tcctatatag taaagcgtct aagaatcaat gggattctcc ttctgtgaaa aaaacctgcg 406800
aagaaatatt caaggaactc ccccacaata tgattttccg gaaggatgtt caaggaatct 406860
cacaattctt atttcttttc ttttctcatg gtatcacttg ggaacaggct cagatgattc 406920
aacttataaa tcctgataat tggaaaatgt tgtgtcagtt tgataaagca ggaggccact 406980
gttccatggc aacatttgga ggctttttga atactgaaac aaatatgttc gatccagtat 407040
cctctaacta tgaacctaca gtgaacttca tgacgtggaa agaattgaag gttttactag 407100
agaaagtaaa agaaagtcct atgcacccag cgagtgctct tgttcagaag atatgcgtaa 407160
atacaacgca ccatcaaaat ctgttaaaac gatggcaatt tgttcgtaat acgagttcac 407220
aatggacatc aagcttacct cagtatgctt tccacgccca aacctacaaa ctagagaaaa 407280
agaatagaaa gcagtctccc tatacgatct tccctataag gggagtctga tcatttgcaa 407340
tacctacaag caaattgcgt ttgaaagact ttttataatt gagatttaga aatataaaaa 407400
aatctttaat ttttataaaa agaatacgtt attcccaatc gggaaaagaa caaaaagggg 407460
ctcgcccttt ttttaaaaaa agcataacaa gctctctcgt tattcttctt ctagaagcta 407520
tcttcaatga aaacttttca tcaataatac aaaacaattt caataaaaat tttaaaaata 407580
aaaacatttc tattaatagg atttttgtta aatttacgat ataatacgca aattgatgag 407640
cctaggaaat gtatgagtaa tataacctcg ccagttattc aaaataatcg ctcttgtaat 407700
tattattttg aattaaagaa ttcaaccact attcatattg ttatcagtgc catcttactc 407760
tgcggacttt gatagctttc ttgtgtgtag cagctcctgt ttcctatatt ctaagtggcg 407820
cattgttagg attaggatta ttaatagcct tgattggtgt gattttagga ataaaaaaaa 407880
tcacgcctat gatttcatca aaagaacaag tattccccca agaactcgta aatagaatca 407940
gggcgcacta tcctaaattt gtctctgatt ttgtttcaga agctaaacca aatcttaaag 408000
atctcataag ttttattgat cttctaaatc aattgcactc tgaagttgga tcatctacaa 408060
attacaacgt atctgaagaa ctacaacaga aaatagatac gttcgagggt atcgcacgct 408120
taaaaaatga agtccgtact gcttctctta aaagacttga aagcgctgct tcttcccgtc 408180
ccctcttccc ctctttacca aaaatcttac aaaaggtatt tccatttttc tggttaggag 408240
agtttatttc tgcaggcagc aaggttgtag agctccatcg agttaagaaa attggaggca 408300
gcctcgaaga agaccttagt gattatataa aaccagagat gcttcctacc tattggttga 408360
ttcctttaga ttttagacca acaaattcct ctattctaaa tctacacaca ttagttttag 408420
ctagagtctt aactcgtgat gtttttcaac atcttaagta tgcagcatta aatggcgagt 408480
ggaacctgaa tcatagtgat ctaaatacta tgaaacagca gctctttgct aaatatcatg 408540
cggcgtatca atcctataaa catctatctc aaccctctct tcaagaggat gaattctata 408600
acctgctctt gtgtattttt aagcataggt actcgtggaa gcagatgtcc ttaataaaaa 408660
cagtcccggc tgatttatgg gaaaacctct gttgcttgac tttagaccat acaggacgac 408720
cccaagacat ggaatttgcc tctctaattg gtactctcta cacacaaggc ctaattcata 408780
aagaaagcga acatttcttt cttcattgac actccttagt ttagatcagt ttaaaacgat 408840
ccgtcgtcag tcaaccaata tagcgatgtt ccttgagaat ttagcaactc ataattccac 408900
ctttagaagc ttaccaccta taacagtcca tccactcaag agaagcgtct tctcccaacc 408960
tgaagaagac gagtcctccc tgctgatagg ttagagattt ctatttggga ataagaaaaa 409020
ttctactccc tctcgtagcc cctttcgaga ctaaaaagct agttacctgg tttagtcttt 409080
aaagaagata ctcgtcttcc tatgaattct agctcctgtt acaaaattga cgtcataaac 409140
cctagctctg gcatttccat gctctagagc tattctgtga agaccctgtc ttaatacact 409200
ctctaatcgc ttagaaactg aactcaaatg ccttaacaag aatttaccta gttctttctt 409260
tggctcatca gaagcagaaa atgcttgaac agcttctttt agatcctgaa tcacatacag 409320
gcctaggttt acatcaaaat cgatagaaga ttcgtcaaga agattttggg atgacaaagc 409380
tcctacaaga gcacaaagct gaaggttgta tgcggtactg ttatctacaa aacacagcca 409440
atcccaagca tctctaggaa gttgagtgat cagctgtagc tgatcaaaag aatagccatg 409500
caagctcaac aatagcaatt ccttactgat tgtctctttt gtaagtcccc ctgcttcggt 409560
ctttagagtt cctcgtgccg tataggtagt gtagatacgc tctacaattg ctttgacttc 409620
atcagtatcc catttctctt gaagagcctt gttttttaat aaaagaacgt cctctttcgt 409680
taattgttga agaatatgat gcgtctcttt acaaaaaatt gtagccttag ctgtactgta 409740
tcccaaaggc cctagccaat agtacccata acattctcta gggacaccta ggtctctaca 409800
aacttgtgga tctcctgctg atataaattt ctgaagccaa tataacgggc aggtttggct 409860
taaaagatct tcaaatattg gccaattatt cttttctaaa tcacctgcga gcctgctaat 409920
accaaaactc tctactttac ttcgcaattc agaaggcaat tgtttgattt tctcttcagg 409980
agatatgtta cacaacaggg cagtagaaaa ttgtctaaac tcggctaatg ttacctgctg 410040
ttctcttaca aatgcagaga ttgaaaggcc ataagcttca tctatcacgt gagtaaatct 410100
atcaggaatg attcttggtg ttggtggaag cttcttctct ccaaaaatca aagctaaaat 410160

tactaaagaa agaatgacaa aggcaataaa agctaaaaca ctcccaacaa tataagaaac 410220
gggaggagcc aaacagccta aagctgctaa tgcacctata caaaagaggc tggcaatggc 410280
tattcttaca atagtccttg aactcaaagg aaaggtagaa tgacactcgc aatgattttt 410340
aaataaagca tgtggaacga tcgtcatgct catagccaac tccttagata tagatctatt 410400
gaaggcagta tagattatgt gtaaataaaa atctctaatt caagaatttc ctgtaataaa 410460
aaatcccatg aaaaatagct ctctcttacc cttcagatta ggcaaacttt ttacaatccc 410520
cgaaaaaaat gcagatcgaa cacttaagga tttcaaaaat ttctgccaag ctgacctagg 410580
atttttttct cactccctat ctaaatggta tttaggataa ttcaagaagg tattttcgaa 410640
cctttgattt agatctaagg aacctaggta ctgtctaaac ctttgaaagt gtttttcaaa 410700
tacattacaa agatcttttg caaccccgtg ttttgttggt ttgcttaaca aactgcattt 410760
ctatcagttc ttcccaggtc aatagagaaa cctcaggaag gtaaaattgc tcttttgctt 410820
cgtataatgc ccaggacgaa tgtaacagcc caaccagacg tgataactgg aatccctcgc 410880
cttgagaatc aaaatcacaa agccaatccc atccggactc attcatatat tgaaataatt 410940
gcacttgctt tcaagagagc ttgtgagaaa aaataaggtg aaggagggaa tcaaactgtt 411000
cgtaatcagc gtttaactct gcttcatcta cctcttcttt atcagcataa tatcttgtaa 411060
aaaggttttg tctgatttga acgagttccg aagaactcca cgtgtttttc ttgagaccat 411120
cttctaaaag agcaaactct ccaaagctaa ttttctttaa caatagaaga gatcgacgtt 411180
caaaaagaga gggagcgttc tcgtacagtc ctaaaggacc acaccaatag tatccatagg 411240
ttcctggagt gacatccgat accatgggat ataccagacg tcccaaccaa tgcaatgggc 411300
aatgctgtag aagaatttct tcaaaattag ggagcttact tggatgtaca tctttgaact 411360
tctcaatgcc aaactgtaag agtttttctt gtaaatatac tggtgctttg tcgaaaacat 411420
tcgtcttatt aagaatagag atcaagtgat gtatctcata aatggaaacc tcctgatctc 411480
taataaaatc aacaacaata cttggataat gttcttggat gagttccatg attttcttag 411540
ggatcagcaa ctccttaggt tcttgtctca tcttataaat cataaacata gcagaagcaa 411600
aaagaataag agaaactaga acaagagcag ttcctaactc tatggataaa agagattgac 411660
tgcataccaa agctacaata gcgagtacag cagcaacaac agaaacgata atcaggctaa 411720
gcgctggctg gcacaccgaa aattgggtag cctgactata gttgacttgg gtattcctat 411780
acacaggctg tatattaacc atagaagctc cattgtgact tgagtatcag cgtgttatat 411840
tattttccta atcattttag aattcatgga attattttac ttctataaat ttctttcaca 411900
tagactccag agcttgaaaa aatcgattgc gaagacctga actcctactc aaatagatct 411960
cctgactttc cagttcttca ctacattgaa gagcactatc tcggacataa gaacactcat 412020
tcctctctta tactcttgga tcgagtcgat ttaactatct caaaccccaa tgctttcatg 412080
aattttatta tgcttgtcta aatgactgaa aaaatgaccc gcaaggactt tagatgaaca 412140
tatgtaaatg gtaaatattt ttttatctct tttagaatcg gtttcccttt ctatagaatc 412200
ctgagtggga atcagtttca tactaacgga gagcacttct tctcacttag ttttttctac 412260
tgctagccac tctggaactc tacctcaaat cagattcgac tttaaaccct tgtggtttct 412320
acatctcatc tattatactt tcatctatcc aaagctaaag atgaccgcat gatcattttg 412380
ctaataaaga gatgcagtcc gcaaaagtat ttgagaagct ggtctttgtc tgaaggagcc 412440
ttctcaatgc agctttatga aaccaggaat aaacctagat aacactacag tacattttga 412500
aagaatataa gatagagaac attcgcaatt tttcaatcat agcgcatatt gatcacggga 412560
agtctacaat tgctgatcgc cttttagaaa gtacgagcac agtagaagaa cgggagatgc 412620
gtgagcagct cttagattcc atggatcttg aaagagagcg tggcattaca attaaagctc 412680
atcctgtcac catgacgtat ctatatgaag gagaggtgta tcaactgaac ctgattgata 412740
cccctggtca cgtggacttt tcgtatgaag tctctcgatc tctatctgca tgtgagggcg 412800
ccttacttat tgtagatgcc gcccaggggg tgcaggcaca aagtcttgct aatgtctacc 412860
tggcccttga aagagattta gagatcattc ctgtattaaa caagattgat ctacctgccg 412920
ctgatcccgt gagaattgct caacagattg aagattatat aggcctagac actacgaaca 412980
ttattgcctg ttctgcaaaa acaggtcagg ggatccctgc aatcctgaaa gcaattatcg 413040
atcttgttcc tcctccaaaa gcacctgcag aaacagagct taaagcttta gtctttgatt 413100
ctcattatga cccttacgtt ggcattatgg tctacgtacg cattattagc ggggaattaa 413160
aaaaaggaga ccgcattact tttatggcgg ctaaaggctc ctcgtttgaa gtcttaggta 413220
taggggcctt tctccctaaa gcaacattta tagaaggttc cttacgccct ggtcaggtgg 413280
gtttttttat tgccaatctc aaaaaagtga aggatgtgaa gatcggcgat acagtcacga 413340
aaacaaaaca tcctgcaaaa actcctttgg aaggcttcaa agagatcaat ccggtagttt 413400
ttgctggaat ttatcctata gattcttctg attttgatac tttgaaagat gctttaggaa 413460
gactacagct caatgattct gctttaacta tagaacaaga aagcagtcac tctttaggct 413520
ttggttttcg ttgtggcttc ttaggacttc ttcatcttga gattatcttt gaaagaatca 413580
ttcgagaatt tgacttagat attattgcaa cggctccaag tgtcatctat aaagtcgtct 413640
taaaaaacgg gaaagttcta gatattgata acccctcagg atatccggat cctgcgatca 413700
tcgagcatgt ggaagagcct tgggttcatg tgaatattat cacccctcaa gaatatctga 413760
gcaacattat gaacctctgt ttagataaac gtgggatctg cgtaaaaaca gaaatgctag 413820
atcagcaccg tctagttctt gcttacgaac tccctttaaa tgagattgtc tcggatttca 413880
atgacaagct gaagtcagta actaaaggtt atggatcctt tgactaccgt cttggggatt 413940
accgtaaggg atcgatcatc aaattagagg ttcttattaa cgaggagccc atagatgctt 414000

tttcttgttt agtccataga gataaagcag aatctcgtgg aagaagtatc tgcgaaaagc 414060
ttgtggacgt gattccacaa caactcttca agattcccat ccaagctgcc attaacaaaa 414120
aagtcattgc cagagaaacg attcgtgcgc tttctaagaa cgtgaccgca aagtgttatg 414180
gcggagatat tactaggaaa cgcaagctgt gggaaaagca aaagaaagga aaaaaacgta 414240
tgaaggaatt tggaaaagtt tccattccca atacagcttt cattgaagtt ctaaaattag 414300
attaacattg acgcttaaaa tcagcacact gcttacaatt gaaaattcgg tagtggtgaa 414360
ctaaatctcg agctacctag ggtcttctcg agatttttta tttttacttc actctttctg 414420
tagttttcgt gtgcacccaa tcggtatgat agaactctcc tcgagggcga tcgttacgct 414480
cgtaggtatg agctccaaaa taatctcgca gtccttgagc taacgacatt gaagagcttg 414540
ctgtacgata gccatcataa aacgtgattg ctgctgctaa acagggaata ggtagccctg 414600
caccaattgc agtcactact gttctacgcc atcccatctc cgcatggcgt aatgctccac 414660
ggaaatattc ttggaagatg agcgaggtat tctctgggtt ggcagcaaat cctttatgta 414720
taacatctaa aaatgcactt tgaataatgc atcccccgcg ccacatcaaa gcaatttctc 414780
ctaggtctaa tccccaatta tattcttttg aagcttctcc taaaagcatg aatccctgag 414840
catagctgat gatcttggaa gcgtataaag catgaaagac atcttgtatg aataccgagg 414900
gatcatgggg catttcaaat attaaggggg ttcctggata attacgggca gcttgctcgc 414960
gtatctcttt ccaagaagaa aggaaacgag caagaacagc tcctatgatt aaggaaaggg 415020
gaactccaga atttaaagca tcgattgcgg tccactttcc tgtacctttt tggcccacga 415080
catctaaaat cgtatcaata acagggattc cttccggatc tttcaatgct aggacttcag 415140
aagcaatacg aattagatag ctttccaact ctagagtatt ccactctttc aaaattgtag 415200
caacggcagt tgcggagagc tttaggaaat ctcttaagat accgtaagtt cgcatatcaa 415260
ctggatatcg ccgtattcta taccattgtg aacagccttt acatagtggc ctgcaccgcc 415320
agttcctacc caagaacagc agggacggcc ctgtactttt gctgctattg attgaaaaat 415380
aggagccact aatggccacg cctcaggatt tcctccaggc ataattgatg ggccgtgacg 415440
tgcaccttct tctcctccag aaatccccac gcctaagaag agaatcccct tttcttgcaa 415500
ctctttacat cgtcgttcgg aatctttaaa atagctattc cccccatcga taatcacatc 415560
gccgggttct agaaaaggca gtaacgcatg aatgctctga tccacaggtt tccctgcttg 415620
aatcatcaac atgatctttc gtggtctctc caatgaattc acaaagtctt ctaaagattc 415680
aaaccctaca agctctcggt ggttagggta ttctttcaag aagtcccgcg ttttctctgg 415740
ggtccgatta tagacagaga cagaaaaacc atgatctatc atgtttaaga caagattttt 415800
ccccatgaca gctaagccaa taagaccaat attcgtttgc aaagctacct agcctcctta 415860
aactaattta aatatagaac aagctttcgt tttttacctt gagccaacaa cacatagtga 415920
ccataacaga tgtcttgttc ttcacaaaca ctatgctcat tagcgatggg cacattatta 415980
atatataccc ctttttgttc aattagcctt cgaatttccc ctttagattt acatagtccc 416040
aaaacaagaa ataggtctaa ccaacgtttc cctaacacct cggatttatc caatgaggcc 416100
cccatccctc ctgcaaacaa ttcatgaaaa tctttttccg ataaggatga aagattccct 416160
ggatgcatgc tacgagttac agaaagagcc tcttcaagcc ctagatctcc atgaatagca 416220
cttaagatat cttgggctac aaattccttc actgcaactg gatccgtctg tacacgccta 416280
tcaatatctt gaatttcttc attgctcaat aaagttaacg tacgagcaat tttagggatg 416340
gtatcatcgg gcaaacggag taagtattgg tacagctcaa aaggagaggt taaatctgaa 416400
tcgagccata cagttcccga ctctgttttc cctatttttt tcccctgagc attcgttaat 416460
aaaggatagg taaggccgta ggcctgaccc aaccctttac ggcgaataaa atcgattcct 416520
gaagtaatat tcccccactg atcgctacca ccgcactgca agatcgtgcc ataattttta 416580
aataagtgat aaaaatcata ggattgcagg attaaatagc taaactcggt atagctaatt 416640
ccttcatcag aatgcacccg ctgctttatt gtatctttca ctagcatttg gcctaaacga 416700
aagtgttttc ctatatccct taagaaatca atcagggaga tctcctgcaa ccagtctgca 416760
ttatttacaa gagtcacccc gggaagatag cgctggagac acgccgtgat cttttgactg 416820
ttatcaaaaa cttcacttgt ctgaagtaac gatctctcgc tctgtttccc tgagggatct 416880
ccaaccatac ctgtggctcc cccgactaaa gctatggggg taatccccag agcagcgagt 416940
ctcttcaaga aacaaatccc aatccaatga ccaatatgta gagcaggtgc ggtaggatca 417000
aatcctaaat aagcggcgat aggtccctct acggattcca aacctgcggt aaaattctct 417060
aaaatatttc gctcttgtaa agattgtaac caggattgca tgagtgatcg tacataatta 417120
agaattgcca agctctatct tatcgatcct aaagcttata tgcaaggcac cacctatcta 417180
acaataaaga aaagattgta cgctgagaat aattcctttg ctgagatacc ataacttcta 417240
tcttctctaa agattaggtc gctatgtcta catctccaat tggggttccg tcgatgctaa 417300
acgccgcaac tagtctaaat gccacaacta gcaaggcacc ccttcctacc tctaccctag 417360
ccgaacgtat taaagaatgg ctgccccgca ttcttctctt gattgtagga gcaatcttca 417420
caattgctgg ctgcattgtt atggcgttga ctaaacaaat tctttacgga ttactctgtg 417480
tcgtaggagg gcttctccta gctctaggac tgctcttaaa acctgagaac tgtatttatc 417540
gaaatgcaga gagtctgcgc gaagcttaag caatgcttta gagtgaattt gagagacgcg 417600
agactcactt accccaagga ccttaccgat ttccttaagg acaagttctt catagtagta 417660
cagggccatg accttgcgtt ccttttcctc aagttcctga atcgcattgg ctaaacataa 417720
agaaaattct tgtttatcta caacatcgta ccctgtctcg gcacgttcat cggggattct 417780
ctcttcaaga gccattccgg ctccttcatc actttgtgaa ggccactctt cattcagaga 417840

cacgattaat gcaggacggg cagatacaaa ccatcccgaa agctcttgtt gcgaaatatt 417900
gagatactca cacagttcaa gatccgtggg ttccttgcct aaagactggc gaagagaatc 417960
catagctcct gacaatttat tcgctttttg atggacacta cgaggaaccc agtcttgctt 418020
acgcagatca tcaataatgg cagccttaat cagaaatacc gcataacctt caaaacgacg 418080
acttctctca ggattataac gttccaccgc acggacgaga ccttcaacac ccgaagcata 418140
caaatcctcg gtctttacat gggaaggcat ccctgaaatc aaacgatgaa ccacactttt 418200
tactaaaggc aaatagaact caattaagct atcgcgatac tctatttcct gagtctccca 418260
gtagaagttc caaacctcta tgatgttttg agtttgctgt gttttcacaa attttttttt 418320
atctagttat ttattagatt aattcaaaaa aaatttttaa acaatattta caccaacaaa 418380
aaaaatacga aagtaatagt gcttacagaa gattaaatta aattaaggaa ctaaaacctc 418440
atctgaaacg atccctaaga aggaaatagg gatttcttta ggaagctcat catgagataa 418500
aaccaaaaga tcagggaaat gtgggtcgag catttttttc atctcaaatc gtgtttcaca 418560
gctcgtaact atggctcgaa aatctttaaa taccgaccgt tctaaaagac tgtctactcg 418620
acggatcaca ttctcttgca ttacaggatt agactttgag tatgagctgt ttatcaattc 418680
ttcaacatga aaatctatgg taattacctc aagggtttgt ttctgatccc agagacttct 418740
cccaatccaa tatccgagag actttcgcac tttttccgca aggatctcca agctgtctcc 418800
agaattttgg tataccgcaa cggcctctag aatctttggg naaagcttaa gcgataccct 418860
ttctctaaca aggaggcgag aaagaactac aagagagctt aaagagattt tcttaggaac 418920
gatgtcttca acagcgatgc caaacactct ctctgattcc tcaaggtact tttgaactac 418980
ctcggcattg agagcctcat gagcgatgtt tctaaggaaa ggaagcacag cctctggagt 419040
catttcatct aagtatacat tctggccaaa tactcggagc caaggacgct cttcaatacg 419100
tagagaagta agcacaggca atctaactcc taaatcttca aatacttctt cggatgctgc 419160
acgatatact tgatagaact gtgattcttg ttccttaggg caggccccct caacataaga 419220
gaacgcacgt tctatacaag aatcttctga tgcaggctct tcttttcgat acgccaacca 419280
taaaagactc gcgagcaaaa cgatagggaa ttttggagaa ctgggaatgc agcacaaaga 419340
aaagatcaat aacgacacca ccctgaaatg ctgacgcaac tgtttgtagt attcgaacag 419400
gtaatttaaa aggctctctt ccttatcgat tttactaata agagtggctg cagcacacga 419460
agtaagtaaa gcaggtactt gactcactaa agcatctcct aaaactgtaa accacatctg 419520
ctcaagagca taacccgaag tataataaag acaagttaca gaaactacgt tcacgagtaa 419580
aaggatacaa ctaataattg catccccttt aacaaaacga aagaccccct ccatggcaga 419640
gaagaaatcc ccttcttcta taagggcatt tttttgtttt ttgacagcct tataagaagc 419700
tcttccagaa acaagatcag aatctaaagc catctgtttt gctggaagag cctctaagaa 419760
aaaccgcgaa cggacctctg cgattctttc cgaacccttt gaaaccatca aaaagttcac 419820
aaagaaaaga aggaggcacg caaacgttgc tgcccataga cttcctaaag agaagaaact 419880
gcctaaagaa acaatcagag aagaggcggt tcctgaagag acaatccatc gtgttgatgc 419940
aagattcaat cccaaccgca ataggcaaag atataagaaa aatggaggaa aaacttcgct 420000
gaattgcttg aatttaaggt aaagacccaa cagaccgtta gtaaagacaa tgcaaaacta 420060
atacacaatc caaaatcaag aaggatctga ggaagaggta aaaagattag tactaggatg 420120
ctaagaggga caaagatcat tccccttaca ccatctttct tcccagacac aaaaacagcc 420180
tcctagagaa tccctgcctt ttctcatact tccctcttcc atatatctgc aagtaaaact 420240
ctaaagagac gttatgaagc atctttgatc ttgtcgacta tttccttcta tgctaacgtt 420300
gatctctttt actcgaaaat tttattttac ggagtatagc gcagcttggt tagcgcggtt 420360
gctttgggag caataggtcg ggggttcgaa tccctctact ccgaacttca ttttaatcta 420420
tccgacaata cgtagaagga aaactccatg gccaagctag tcattacctc tgatgatgaa 420480
caacaagagt tcgagttaga agacaatagt gagatcgcag agccttgtga atccatgggc 420540
attccctttg cttgtacaga aggtgtctgt ggaacttgtg tgatagaggt cttagaagga 420600
cgtgagaatc tttctgagtt tacggaacca gaatacgatt ttctaggaga acccgaagac 420660
tctaacgaac gtcttgcttg tcagtgccgc atcaaaggtg gctgtgtcaa agttactttc 420720
taatcttaga aaataaaaat tttatattaa tacaacttct attctgacga actcttttct 420780
aagtagttat gcgactcttt gcattcagga aaggcttgat ttactcgcga gcctttcccc 420840
tatctttctt gttttcaaaa aaaatttaac taaataaact tattattttt tatatttact 420900
tagaaagatg acaaattaaa aaatattttt aaatgcagaa atgtttttaa ttatttaaat 420960
atcaataaaa gagatataat ttaaaggaac tcaggtgaat tccttaatta tggctacaat 421020
ctcacccata tctttaactg tagatcatcc cctagtagac actaaaaaaa aatcctgcag 421080
caactttgat aagattcagt ctcgaattct attgattact gcaatctttg ctgtcttagt 421140
tactataggg accctactta ttggtttgct tttaaatatt cctgttatct atttcctcac 421200
aggaatttca tttattgctg ttgttcttag caactttatc ctttataaac gagcaaccac 421260
cctcttaaaa ccgcgtgctt gtggcaaaca caaagaaata aaaccaaaaa gggtctccac 421320
caacctacag tattcttcta tctctatcgc aatcaatcgt tctaaagaaa actgggaaca 421380
ccaacccaag gacctacaga atctccccgc accctctgca ttactcacag ataaccctta 421440
cgagatatgg aaagctaaac attcactgtt ttccctagta tccctcctac cgggaggcaa 421500
tcccaaaaca tctcttaaat tcaagcttcc gaaaatttac gaaagactct gttaattgaa 421560
gaaacctcgc aaaatgcgcc tatatcctcc ctacgtagat accactccct ccccaaaatc 421620
cttgctcaat gaggcaattc aggaaaccag ggtagaaata aatacagaac tccctgcggg 421680

agattcagga gaacgtttat actggcaacc cgatttccga ggccgcgtct tcctcccaca 421740
aataccaaca actcctgaag ccatctacca atactactat gcactctatg tcacttatat 421800
ccagactgcg atcaatacga acacccaaat tatccaaatc cctttataca gcttgaggga 421860
gcatctctat tctagagaat tgcccccgca atcaagaatg caacaatctt tggctatgat 421920
tacagcagta aaatacatgg ccgagctgca cccagaatat ccgctaacta ttgcttgtgt 421980
tgaaagatcc ttagcccaac tacctcaaga aagtattgag gatctctctt aggatctcta 422040
tctcactcaa aacggctatc ctaaaatcaa gggtacagta gattcgcgaa cgaaaaaaca 422100
aactcttggt acgcatcttt tcagtttggt tatctcgaga tgatgaggga caaaaaggct 422160
cctaaagcct ccaagatctt tccatgacgc aattcaagaa aaatcaaaat agcccctcaa 422220
taatagaaaa aacactctca cgacttatac aaatcaaata cgtaatattt tacgcacgaa 422280
ttcttaggta ctcttgtttt aataaaagat attgcaacac ttctcttcta agttaaatta 422340
aaaactcatg tttttataaa aaaatattac acaaaagaaa atatagaatt ttattctcta 422400
ataaaaatta aattaaggta taatatctgc gcaaatttta attaaaggat attaaaataa 422460
atgggatatc ttccagtatc tgctacggac gttctttttg aaagtccagc cgctccctta 422520
atcaatagcg caaacacaca aaatcagaaa ctcatagaac tcaaggggaa gcagcaagct 422580
gagtcttctc cacggacaat cacttctgtc atattggaag ttctcctagt gatcggatgc 422640
tgcctcatag ttcttagttt attggcaatc cgccctgctc tgcaattcac tctagaaact 422700
ggacatccag ctgccattgc agtccttgct gtctcaggaa caattctatt ggtggctgtt 422760
atcatcttgt tttgctttct agcagctgtg ccattcgctg ctaagaaaac ttataaatat 422820
gttaagacgg ttgatgacta tgcttcttgg cattctcatc agcaaacacc gaccctaggc 422880
actatctttt caggtatcgt ctatgcagaa tcccaggcgc aattatagct ctcctaacct 422940
tacaaacttt cttattgaga cctctacaac tcacccagtt tgtgattgac ctcctttttg 423000
tttaagatag agattatgaa gacccaaaaa gcggcaattc ttataagtct gactgtaccg 423060
acttatagac aaaagataag ccactgcctg tgtcttcaaa ttaaaactca ttaaattgtt 423120
attaaatgaa aatatttcac aaatagttaa tttctacact gttgtaaaac atcgttctcc 423180
ctaccccccc cttttattga cctctgaaga tgatatatgg gccttcgaaa gtaaacctga 423240
tgtcctccgt tacccatgcc catgaaatac acatgtcttc accccccatt agaatctcaa 423300
gtagtattgt cgatctaaga agtctttgaa tcccatggct ggggtagaat cgttccccat 423360
aaaaccacaa acgccaccct tttgaaccaa gtggagaaaa aactctgtgt ggcacccact 423420
tagagttctc ttatgaacta aacaataagg tgcgcctcta cgccttaact cattaaaatt 423480
aatgaaatta aaaagaattt ataaaaatct ttattatcaa agaaaaagat tttttgtnaa 423540
aaacaacaaa ttaagatata atcttttcct ttaaatagat attttatgac taaaaccact 423600
tcaatcccag atgtacacga gaatcaatca catttgtctg tagatgagag attgatctca 423660
gaatcacccg tgcttactaa gaaagaagtg attgctaaaa taataaaact cacagctctt 423720
attcttgctt tagccatagc tgtagggact gcagttgttg ctggagttct tggtatgcct 423780
ctcatggcta tagccactgg tgctgctctc cttgcagcag tcgtactctc ttgtcttctt 423840
ttaagaagaa gagagccatc caaaccgaca gaagagctcc ttgggcccca aaaacatgtc 423900
cccaaggata ttgcagctca agtgcaaccc tcagtccctc tggattacca aaagctgctg 423960
agaaatgaat ggaccctagt caatactctc tcagaaatca atatatcctg gactctccaa 424020
gatcctaatc aaagatacta tgtctgggaa catcaaggag ccccaattac cttagtagcc 424080
actacaggag acatcgctaa accacgcctg aaaacctcag gaagagtcat gattgttaac 424140
gcagcgaatt cgaacatgca atctggtgga gccggaacca atgctgctct ctcagcagcc 424200
acacacccta cttgttggaa caatacgaga acatctgggg gaaaaataaa cactggcaaa 424260
ggattatctg tgggtgaatg ccgctcagca ccctggatca atagagactg gacgaataaa 424320
tgatacgaac ccaggagaag cacatttctt agcacaactt cttggtccta aatatgaagg 424380
agaattgaaa gcacatcctg agaaattaag caatgttatt aagaaagcct atttgaactg 424440
ttttgatgaa gctctcaata accaagccac tgtggtccaa gtgcctctga tctcttcctc 424500
tatatactca cctggaggaa agctggaact agaacccgta aaccaaacaa agcctaatag 424560
cagtgcatat aagctttacc acatccgtac gtagtgggta aatgatataa agaaaggtct 424620
tatggaagct cttcgctcct ttgctgcgca gcacccctca actcccatgg actataatcc 424680
ttacagacca caaacagcta cttatggtcc cttttaacta aaaagatcta tttcaatagg 424740
atgcattaat aaactaattt tgttttataa aaaaatctaa taaaaataca aattagtaat 424800
ataatagttg aaaattgatc tattaataaa atattatgac agattctaat cccctaccct 424860
cttatacaga cgccagtctc tacagaactc ctgcgaaaca ttcctatccg attagactcc 424920
ctctcaaccg tacagataga atcgagaaaa tactgaaaat tgtcaccctc acactagccc 424980
tagcgtgcgc tttgggcttt agcattgctg ctggcatttt ggctatgcct attttttctg 425040
ccgtagttgt cataacatta gcaattgctg cggtctcact ttactccctt ttaaagaaac 425100
ctaaattata cgagattctt cctcaaatcg aacccgaatc tgagcaaagt tctctgtctc 425160
cctctcccca gcctcctgag caacaggacc tccctttgca gatcgatcca cttcccgatc 425220
ccgaatcact ccccgaagtc tctcttgctg atctaaccac acccccagaa gaacttaccg 425280
ctatcacggt cactcctggc tatgaggctc ttcttgaaca aaactgggat cttcttccga 425340
gcttagccgc tgtagaccca tcgtttacta cagaaacacc tcagcagccc tgttttattt 425400
ggaagcttaa agactcgaag cttatcttta tatctacctc aggagatatt gcagttccaa 425460
gaatcaaaac tcaaggcagg gtgatgattg ttaacgcagc aaacgagaac atctcccgag 425520

aaggaggggg aacgaataaa gctctatccc tggctacaag tctacagtgt tggaacgcat 425580
ctaggctccc tagagcgcac tctcgttctg gatcccaact acagccagga gaatgccgct 425640
cagcaaaatg ggaaaatagt gatcacacct caaacgacca tgtcccaggc aaagcacact 425700
tcttagcaca actgcttggt cccgaagctg ctaagtgtaa caacgatcct aagcaagcat 425760
ttgaagtaag caagaaagcg tttcataacc tgttccaaga agctgaaatc ataggcgttg 425820
atgtgattca actccccctc attggatgta atctatttgc tccatcaaga cttctaaacc 425880
tcgggaaaac aagagcagaa tggatcgagg ctataaaatt ggcactcatc acatctcttc 425940
aagattttgg atgggaacaa gacaaccagg aagagcaaaa aattatcatc cttacagaca 426000
aggaccagcc tcccatcatt ccgccccgtt tcgatctaac gactccctag tctatgtcgg 426060
aaagcgtccg tgcttccaat cgaacagctt tctaaaaaca cagccaattt atcctgatca 426120
caagcacccc ctagattctt ggctctaagc cgcatccaat actttttaaa gtgaaaatgt 426180
tgtctattta ttattttaaa taaacaactt ctatttaaaa tataaataac ttcacataaa 426240
aaagaatctt ttttaaactt tttgtttttt atttaaaaag ttttatgtcc accacagaac 426300
ccaatttgac taacgtaaat ctaaccatgc tgatcagcag cgaaagcatg cccacgcaac 426360
tcgcatctca taagctcaaa ggtctggacc ttgtcgcttt tattctaatt ataggaattg 426420
ctgtaagttc tggaaccgct gctataattt taggcattcc tctattattt attcttaccg 426480
ctctagcagt cttggctttt agtattcttc tctattttct cttaagagaa cctaaaagtc 426540
ctataagcgt aacgcatcag ccgacgccca tcataaaaga tacagacctt cctcctgtcc 426600
cgcccctagc actcacccca gtgcctacgg aagctgtcct agaagagccc ccgcttcctt 426660
cccctagaac ccatcaaaca ctgttacaag aaaattggga ccgtatacct gatctacagg 426720
ctaacacaga tatgcctttc atcgctgctg acaatcaaac cggttatgct tggcatttga 426780
aaaactcaaa cctgactttg atctctacgt tagggcccat tgaaaagcct cgctataaaa 426840
ctcaaggcat cgtcatgatt gtgaatgcag ccaccccaaa catggcaaac aacgtaaaag 426900
gaacaagtct cgcacttgcg aaagcaacta gtgtacgctg ttgggaaaat tcgaaaaaat 426960
ctccggatcc tctccgttca aaacagcccc tacaattagg agaatgccgc tcagcaaaat 427020
gggaaaatct aaacggaacc acgaatgcag gtaaagcagg actaccgcaa ttcttaggac 427080
aacttctagg gccgaaagct tctgactata actacaatcc taatgatgcg tttacctttt 427140
gtaggcaagc ctaccttaac tgtttgaatg aggccaagcg ccgtaaaaca accgtagttc 427200
agctcccctt gctttcctcc catttccctg gctctccaaa agacgaagag actactagtc 427260
tacgtctgca atggattgat ggtgtgaagt tagccttgat agatgctctg cagacatttg 427320
gatcagaagc agaaaatcaa aatcaaccgt gggttatcat tttgacaact cttgctagac 427380
atcccctcat cacaccctaa tctctccccc tggttaagca aaagagagta gatgcctttt 427440
tgtgaaaagg aaatcctttc ttaaaaaatg gtaagacttc tctctttaaa acatccaaaa 427500
catagaagat tttttaaaaa atcttttcta actttctaaa ccacgctata ctttctcaag 427560
caaatagaaa cgcctctgct ctccataaga caaagctttt gcgaaataaa gaaaattcta 427620
actaaaagac gatgcccgtg tcctcagccc ccctacccac aagccaccgc ccttcctctg 427680
gaaatctagg cctcatggaa ccaaattcca aagctctaaa agcaaagcat caagataaaa 427740
cgacgaagac gattaaactt ttagttaaaa tccttgttgc cattctagta atagaagttt 427800
taggaataat tgcagctttc tttattcctg ggactcctcc catctgcttg attatcctag 427860
gaggccttat tcttacaaca gtactctgtg tgcttcttct tgttataaag cttgcccttg 427920
taaacaaaac cgaaggaaca actgctgaac agcagataaa acgtaaactc tcttctaaaa 427980
gtatttctta gacaaacagc ggtgtttttc actcattata aataaaatat tttattccct 428040
aggtcataga aaatatgaga gtgtctcctc ctattttagt aggagattaa tttgtaaaac 428100
actaattaca ttgcatttaa aatagaaaac tataaaatta tagggcttgt catagaaata 428160
taggaaatca catgtcttct ccagtagtca caggaacatc aagtgcatct ccagttgaac 428220
aaacaaagct tggagaattc ctagaaaggt tatcgggatc aggacgatgc ataaaaattg 428280
cctttgcggc ttcaactgct ttactcctcc tcaatacctt tgtttctgga atcgttgcta 428340
tagccatgat ctttgtagca acatctgtcg gagcctactt tacagttata gggcccttat 428400
tcttgctctc cctaatcctt ctggctatca tgttaatctc gatgtataaa atcacgcatc 428460
catcacaaaa tacaccgatt tcaaattaga aaaaagcgtt cccaaagtac gagccccaag 428520
cactcctctt gggaactcct tctctttgtc aaacttcggg attcgttcta cgaacacaaa 428580
aacagattcg agttccttac gaatcgtcct ctaaagcaaa atctttatcc ccttgataag 428640
tatgaaataa ttattttaat tcacaataat ctctccatat actctcttga tggcctgaaa 428700
cactcggcca tccaaggtcg tcgtttctct ctgaaaattc ctcttattga ggtgaagttc 428760
tgtaaaattg ataaatgtag gcatccttag agaagcttta tcagcttcaa caaaattctc 428820
tcggctttta taattgataa aactctgaaa tttttgagat ttttatgaca ctcattaccc 428880
ctgccatcaa ttcctcgcga cgcaaaaccc atacagtaag aataggcaac ttatacatag 428940
gcagtgacca ctcaataaaa acccaatcaa tgacaacgac attaaccaca gacattgaca 429000
gtacagtaga gcaaatctac gctctagcgg aacataattg tgatattgtc agagtgactg 429060
tacagggaat caaggaagca caagcctgtg aaaaaattaa agaacgtctg attgctctag 429120
ggttaaatat ccctttggtt gcagatatcc acttcttccc tcaagcagct atgttagttg 429180
ctgattttgc tgacaaggtt cgcatcaatc caggcaacta catagataag aggaacatgt 429240
tcaaggggac gaagatctat acagaggcaa gctatgccca aagtctcctg cgtcttgaag 429300
aaaagtttgc tcctttagta gagaaatgta agcgactagg caaggctatg cgcattggag 429360

tgaaccacgg gtcactttcc gaaagaatca tgcaaaaata tggcgacact atcgaaggaa 429420
tggtagcctc agcaattgaa tatatcgctg tatgtgaaaa gctgaattat agagatgttg 429480
tcttctcaat gaaatctagc aatccgaaga tcatggtaac tgcataccgc caacttgcta 429540
aagacttaga tgctagaggc tggctctatc cccttcacct tggagttact gaagctggaa 429600
tgggcgtgga cgggatcata aaatccgcag taggaatcgg aactcttctt gccgaaggac 429660
tcggggatac catacgctgc tctctcacag ggtgtcccac tacagaaatt cctgtctgtg 429720
atagcttgct acgccatacg aaaatctact tagaccttcc agaaaagaaa aatccctttt 429780
ccctacaaca ctccgaaaac tttgtttctg ctgcagagaa gcctgcgaaa acaacacttt 429840
ggggagacgt ctacggagtc tttttaaaac tctatcctca ccatcttacc gactttactc 429900
ctgaagaact cttagaacac ttgggggtaa atcccgtaac aaaagaaaaa gcattcacaa 429960
ctcctgaagg ggtcgtcgtt ccccctgagt taaaagatgc tcctattaca gatgtacttc 430020
gagaacactt tttagttttc caccaccatc aagtgccttg cctatatgaa cacaatgagg 430080
agatttggga tagccctgct gttcatcaag ctccatttgt gcattttcat gcttcagacc 430140
ccttcattca tacctcccga gatttctttg aaaaacaagg acaccaagga aaaccgacca 430200
agctagtatt ttcaagggac tttgacaata aagaagaagc tgctatttcc atagcaacag 430260
agtttggagc tctgcttctt gatggccttg gagaagctgt ggttcttgac ttaccgaacc 430320
ttcccctaca ggacgtgcta aaaattgcct ttggcactct acaaaatgca ggggtgcgcc 430380
ttgtaaaaac agagtacatc tcctgtccta tgtgtggtcg gaccctcttt gatcttgaag 430440
aagtcaccac acgtatccgc aagagaacgc agcacctacc aggacttaag atcgctatca 430500
tgggttgtat tgtgaatggc cctggagaaa tggcagatgc agatttggga tttgtaggtt 430560
ccaaaacagg gatgatcgat ctttatgtaa aacatacttg tgtaaaagct cacataccca 430620
tggaagatgc tgaagaagaa ttaattcgac ttttacaaga acatggggta tggaaagacc 430680
ctgaagaaac taagttgaca gtatgactct atccttccac actcaccctc tgaactattg 430740
gactttcgaa gaattcgatg gtttgcctat acgccacgga gtcttttcaa aacaaaagga 430800
tgccgagggc acggtcttcg cagccaagaa tcctgagatt gcttcagctc tccaatctcc 430860
gaagtattgc gaccttcatc aacgccacgg cacttccgta cgttgtgtta cacctacatc 430920
ccccacctac caacctgcag acggactgtg cacgcagtct ccgctcctct ctctccatat 430980
ccgccattcc gattgccaag cagctatctt ttatgatcga gaacaccacg caatcgcaaa 431040
tgtacacagc ggatggcgag gattgcttgg caatatctat gctgtcaccg taggtactat 431100
gaaaaaatta tttcatacaa aaccacaaga tctcttcgta gctatcggcc cttccatcgg 431160
tccagattat gctatctatc ccgattacgc tacgttattt cctcgtagct ttcttccctt 431220
tatgaatccc aaaaaccatt ttgacctgcg tgcgattgct cgcaagcaac ttacgaattt 431280
aggaatctct aaagaccgca tttttatctc agacctctgt acctacacgg aacacgacgc 431340
tttcttttct tcaaggtacc ttgctcacca tcccgatccc aatctcacag gccaacattc 431400
aaaaaataga aataatgtaa ccgccgtcct tctcctaccc agagattaaa aagatccagc 431460
caagcttctt ttcctttccc ctaaggacct tagcttctag aagaacatgc ttcgttccaa 431520
cccactcaga attcagtaat cttcttgatg aaaaacaaca tgtaaactac aatttttagg 431580
aaaaaattaa gcttaaagac tttctatgaa attaggcgca tcaactaatc ataaagttca 431640
cgaaccagtg aagccaaaaa aagccaaact cgctgagatt gaagctanca aaacccaagc 431700
tacagaaggc acactcagaa gtaaaagtct tgctcttcaa attgcgcgtg ctgttcttta 431760
catacttttc gctgcactaa tgttagcagc tggaatcacg ttcgttacct tcgaagcttt 431820
aggcttccct ctaatacagg cgtatagcat tgctggtatt atcacactcg tgggattagc 431880
catcgggctc gtgcttctca tcttgagctt gttgcctaaa gaagacgagg aagcagatgc 431940
actttctaga aacgctcttc ttccattaac catcattgta atcgagcaac aacccatcac 432000
tcctaaacct gagatcccct attcttattt aactaaacta gccctattaa catcattgtt 432060
ccttacctta cgacgctctt cctcccaaag aaaaactcac taaagaaatc aaaattacag 432120
aagctacgaa ttgtgaaatt caccatctaa agaagggtcc ttaatttaag gttgtcacag 432180
ctaaagctcc taatctcaca gaaattagag atcacggggc tcgcgtacct tcgctattcc 432240
tcctttcacc agaaacttcg cattggaaag gggataagga agtctcggct cccctaaagc 432300
aactgcaaga tctcttagga gaggaacagt gggaagctat gaaaactaaa atgaactcta 432360
gaaaaaaagc aggtcaatgg gcaattttca attctccaac tcctggtgtc agttcaactt 432420
tagttttagc atggactcct tggggttatt acgacaagga tgtacaagat atcttagaaa 432480
gaaaagatcc gatgagctct tcgctttctg aaaaagactc aaaggagttc ttgaaaaatc 432540
tgtttgtaga tctcttagaa aatggcttca catcagtaca tattcacgca gaagaagctt 432600
tcactcctct tgatcatacc gggaaacctc actttaaaag agacaatgtg tacttacccg 432660
gaaagttgtt aggcgccttg aatgaggctg cggtacaagc caatgtaagt gcggatactc 432720
aatttacatt gttccttact caagatgagt gcaatccttt tcatgataag aaaagaggtt 432780
aaaaaatctg aaaaggctct aaaagagcct ttatgagaat cagagaaatt acaagtcgag 432840
taatgaggca ggattctcta ggccttcttt cactttgact aaaaacccaa cagcctcttt 432900
cccatcaata agacgatgat catagcttaa agcgacatac atcatatctg caattacaat 432960
ttcattatca agaacaacgg ggcgcttttc tatcttatgc atccccaaaa tccccacttg 433020
cgggggattg ataatgggag tcgaaagtag cgatccatat acgcctccat tggtaattgt 433080
gaaacctcct ccctcaagct ccgctattgc aagtaggcct tcacgagccc gaagggaaag 433140
atctgcgagt ttctgctcaa tctccccgtt agaaagttta tcgcaatcgc gtatcacagg 433200

aaccacaagt cctcgatcga tacctacagc aatagaaatg tcataatagt gacggtaaca 433260
atctcctcgc catcaatata ggcgttcact cgtggatatg ccttcaaagc ctctaagaca 433320
gctttcacaa agaaagacat aaatcctaac ttcaccccat atcgagatag aaactcttct 433380
tgtttttcct ttcgcaaatg aaaaagaggt gtcatataga cctcattgaa tgtcgtgagc 433440
atcgcagact catgtaaagc agacaaaaga cgccgcgaaa ttgtcttacg aatcgaggtc 433500
atgcgttctc gagtttctcc tcgatctcct gcagaaagac cttgggatcc ttggtccatc 433560
tgatcacgaa gaggaataaa cgttttattc tctggaggag actgacgcac cccagactga 433620
ggaaagcaaa tgatctcagc ttctatagtc tctttagact gagaatctcc aagctcttcc 433680
ccttcacctg cgggctctat ttttcccact acccccccta caggaacaac atcgccttct 433740
gaaacctccc agaaaattct tcccgatact ggggcataaa tgagctgatt taccttatca 433800
ctttcaattt ctagtaagcc ctggttttct tgaatcagag caccctctgt aactaacaag 433860
gaagctacgg tcacctcgct aatcgactct gcaatattag gaatgcgtac ttctgtagtc 433920
ataatttacc ttaaagaaaa gagggtttcc atacacgtga ccagctcttg acgactgact 433980
tcgctgatcc agaagctgtg gaactactcc gaggacgtcc tatatatagc agtttctcag 434040
gaagaatgtc ttgcaacgcc ataaacatat agtcataggc ccccatattc ttggattctt 434100
cttgtagcca aacaaaatgt ttcaaatgag aatacttatc gataaggctc actaaatcct 434160
caagagctaa aggatacaag ctctctatac gcaagcaaga aaagtcctta cgccgatctt 434220
gaggaagcat ttctgcataa tcataataga tctttcccga acacaatacc aaaatagaag 434280
catcataatt aggatcggca tcttcgagaa tagcacggaa tcccccaggt tctgtgaact 434340
cctcgatact acttacacat tgtggatatc tcagcagcaa cttaggagta aagatcacca 434400
aaggcaaaga aagatctctc ttagcatgct ctctgagaat ccgaaaatat tgcacaggag 434460
tggaaggcaa gaccacttga aaattccagt tcgcggctaa ttgcaaataa cgttctatac 434520
gagatgaaga atgctcgggt ccttggccct catacccatg gggaagaagc agaacaatgt 434580
cagagtgtaa atcccacttc tgaattcccg aagagatata ctgatcgaaa atgatttgtg 434640
caccattagc aaaatcccca aactgcgctt cccataacac taaagtcttt aatgcctgtt 434700
gagcatagcc atactcaaac cctaaaattg catattcgga aagaggagaa ttatacattt 434760
ctacagagcc ctgctctgca gaaagatggt acaatggaga gtaggtatct ccagtcacag 434820
tatcactcca taccaaatgt cgttggctga atgtcccgcg aatagaatct tgacctgaga 434880
gtctcaggtt gtacccttcg attaatagcg aagcaaaggc taattcttcg gccatcgccc 434940
aatcataacc aaccccacct tctgccattt tcattctttt ttctaaaaga gtcttaattt 435000
taggatgggg atgaaaattg tcagggaaac cacaaagacg cgagctcata tgaaaaagag 435060
tctcgcgatc caaagaaaca tcacaatcat gcaaaataag ctcgccgtta tttaagcgat 435120
cgcagtgatg acattctttt ttaggaaagg gttctggatc cgtccctttc aatacttgaa 435180
actcacgatt cagactctct tgaatctctt tttcaataga tgccaaagtt tcttcagaaa 435240
tatctgcaaa ctgcccttcc aacagatatt gcctaaacag ctcgcgaata ctcttctttc 435300
tcttaatctg atcatagagt aagggagctg ttactgaggg atcgtcactt tcattatgtc 435360
catacttgcg ataacagcag agatctatga tcacatcaca actaaatctc tcacgaactt 435420
gcagagcgta ctctatagct tctatacagg caacgacgtc ctcgctattc actcgaaata 435480
caggaatccc tagcatttta gcaatatccg tacaataagg ggtggacctt gactcccgtg 435540
gcactgcggt aaaccctatg taattattca caacaatgtg aagcgtaccc tcagtagaat 435600
accctggaac acgactcagc tggagagttt cataaaccac tccctgacca gaaaatgctg 435660
catctccatg aactaaaatt gctaagctgc tttgctcttt acctgcgtga ccttggtgtt 435720
gcaaggcagc cacgaccccc tcgacaatag gatctacaga ttcgagatga ctagcgtttg 435780
gcaacatcac aaaggtagtt tccctatctt tctgatggga ctttagcaca taccctttat 435840
ggtactttac atccccaaca ctctctaaac cacgtgctgc aggatcgtct tcaaactcca 435900
taaagacata acggtaaggc tttcccaaaa cattcgttaa tacattcaaa cgacctcgat 435960
gggccattcc taaaacgtag ttagaaattc ctaatgccga tccataatga acaagatgct 436020
ccaacatggg gaccaaggtc tctccgccct ctaaagaaaa acgtttctga cctgtaaatt 436080
ttatctgtaa gaactcttca aaaaacgttg ctttacataa gtctttatag gagcgaagga 436140
gctgctctgc aaagcgctcc acttgtcgct tctccataag attccaaaca aactcctgca 436200
actcaggagt acatgttagg gtttctaaag taagacttcc gcaatagcat ttttttaaag 436260
cttcgatcag ctctcgtacc gaaacctgag ctttaggaag tagacccgca gaaggcacct 436320
gctcatccag atcaatctta gcgatctttt cctgaatgaa tcgagaatct gtagttgggg 436380
caagcgttga aatttgactt tgcaaatatc cataataacg ataaatcgta catagaaact 436440
gagatttttg ttcttgaagc atagcaatag tttcattccc agaaatctta gtactagctt 436500
ctgatggaga tgctgcttga ccgagctgat acccttcaaa aaaatacttc caagaaggat 436560
ccaaagtctc gtgattcata aatctctgat acatagactc gatccaatcc atatccgaag 436620
aatatacttg ccccacaaac tcggaatcca taaaataatt aaactcaata aatacgatct 436680
aaatgtctgc ttcatagcaa atttttattt ttcaaaaaaa aaaaattttt ttttttttct 436740
gcagttgaaa aaacaaaaaa atgctatgca cctgctctcg aatacaggat gggaaccctt 436800
ggatgaaaag cgaacgtctt aaaaaattag aatcagaact tcatgacctt acgcaatgga 436860
tgcagttggg tttagtgcca aaaaaagaaa ttagcaggca ccaagaagaa atccgaatct 436920
tagaacataa aatttacgaa gaaaaagaac gtctacaact cctcaaagaa aacggagaga 436980
ttgaagagta cgtcacacca cgacgcagtc ccgcaaagac tgtctaccct gatggtccta 437040

gtatgtctga tattgaattt gtggaaccca cagaaacaga aattgatatc gacccaggcg 437100
aaaccgtaga actggaactc accgatgaag gacgtgaaga tggggcagta gaagtcgact 437160
attcccacga agacgatgaa gaccctttca gcgatcgcaa tcgctggaga cgcggtggta 437220
tcattgatcc cgatgctaat gaatggtaag gctcccctag ctctttatat tcatattccc 437280
ttctgcacaa aaaaatgtcg ctattgcagt ttttatacaa tcccctacaa aagtgaatct 437340
gtatcgctct attgtaatgc tgtaattcaa gaggggctaa gaaagctagc ccccatccaa 437400
gagacgcatt tcatagagac tgtgtttttt ggagggggaa caccttcatt agtttctcct 437460
cttgatctta agcgcatcct caaagagcta gccccccatg cccgggaaat tactttagag 437520
gccaaccccg aaaatctcac cgtaagctat ctacgtcaac tacaagagac tccaataaat 437580
agaattagcg ttggcgtaca aaccttcgac gactctatcc tacagctcct cggaagaacg 437640
cattcttcat ctgcggcaat cacagcactg caagaatgcc agaatcacgg attctctaat 437700
ctttctatag acctaatcta cggactgccc acacagtctt tggagatatt cctaagcgac 437760
ctacatcaag ctctgactct ccctatcact cacatttctc tatacaacct cactatagat 437820
ccccacacct ccttctataa acaccgcaaa attctagtcc ccacaattgc ccaagaagaa 437880
attctagctg agatgagcct ccttgctgaa aatctcctac tctcccaagg gttccaacgc 437940
tatgaacttg cttcatatgc caagccagat taccccgcaa agcacaacct ctattactgg 438000
acagatcgcc ctttcttagg cttaggagtt tcagcttcgc aataccttca cggagagcgg 438060
tcaaaaaatt atagtcatat ttctcactat ctacgtgctg tacgtaagaa tctccctacc 438120
caagagacct cagaaattct cccaaaaaaa gaacgaatca aagaagcctt agccctgcga 438180
ctccgactcc ttgaaggagc agacctcgcg gagttcccct ccacacttat ctccatgctt 438240
acgcaagatg taaaattaca aaacctattc agtgtgcatg gacaatgtct tgccctaaat 438300
agacagggcc gtctcttcca cgatacaata gcggaagaga ttatgggata ttccttctaa 438360
tccctgcgga aggctaaaac ctagactctg cccttcggac tcctgatgct ccgggaaaca 438420
tcagagaact cttaaaggta aagcttcttt ctcggtcttt cttttttttg ccgtgctctt 438480
atgttaaaaa taccaagatg tctccaaaga tacattagcg acgacgacgc ctagaatggc 438540
gtaatacgga cttcacaaag aaccatagat ttacgaacaa gacgaaagca cataagatga 438600
agtttaatgt ttggccaaca gctaatacat caccactgct ccaattttct tcagcacttt 438660
tattatgtag atcataaaca gcagtgttat ttggacgtac taagatacgt ggaacttgta 438720
ttgcaatctg gttaagcacc ataatggata ccggcaaata aagtcctcca tgtacagttt 438780
gtaaaaagcc tatgataaag cctgcacgat cgcgataaga agctcgtact gttcctacag 438840
gcaggccact tcctataact cgttgctgca caaattgttg tacacgatgt ctaagacctc 438900
gccccatcca gcgagtgcac tctacaaaga caatactccc tgaaattgta gacgcagaaa 438960
taaaaattgc tcgtaagatt tcctgaggca tccatagttt acgcaggacg ttgccacaat 439020
caaaaacaag aacaaggatg cttggtaaat atagagattg tgctacgatt cgtgcagtac 439080
ggaatctctc tctagactga ggataatttg taaagagtaa gaaaaaatac cggaccgcaa 439140
acgtcgtgtg catcaaaatt aaagagtaac agatctgcaa gtctaagccc tcatgaacag 439200
aggctaggat ccattcctgc gcaagtcctg aaactctaga aattgcataa agcgcagaaa 439260
gagctacatc aggacaccgt tggggatagg gattgatttc catatctgat gaaggcgatc 439320
cttgattcaa ccattctaca agcctactca tagaactagg atccgtagaa tcaatgacaa 439380
cagctagatc tctttcactg tgtggtaact gtgatctttc ggcatcttga gaaagacctc 439440
ccctatcttc tacatcctca cccctattcg caaccaagca ttgctctcca aaacgcaccg 439500
cccaagtaaa agaaggacaa cggatagaag gcagaaaaac accctcaaga cccgaagaat 439560
acggcagtat cgtggtgttc tccgccctag ttccccgaca atccagagaa ctaaagaagt 439620
ctccagaaac acggtaatta tggaccctaa agtctccttg aataaagacc gtcggggaaa 439680
tccctacaac aacaatacgt ggactatgcc tagattgcgt taacgcctgt tgtatataag 439740
gcgatgcatc cccatagaaa atcactagaa actcacgatt ctgattttca ggagcagaga 439800
aaaactgttc ccaaaccgtc aaaagggcct gacataaagg atgatcgaga gtaggaagag 439860
tacgacaagg actgatgggc caaggagaca tgccgcttcc atcgttatag agaagacgaa 439920
cggcctctcc ccgtacttca ctaatataca gcaattcctg tacagcttct tgatgcgttt 439980
gccaacttcc atttacatag agagtacgta cctcagaatc tgggggctca ggatcctcat 440040
caccgccaca aaggcaaacc aactcataca catccgaagc cacagaagca gatgaagaac 440100
ttctagggcc tcctgcaatg aagaccgaga tatcttcagc cgaagatgca actgttgata 440160
cgtctagaga aggaggagat attgtaattc tcgggatttg gatatcggcg ggaataggat 440220
ttggggatag aggttccgtt tcttcgagta gtgttggtgg tgaagatcga gattctgtgg 440280
gtgctgtcat caacgctcta aacttatttg gcaaagatta taaaataagt attgataaca 440340
cgcaatagag gattacgatt attttaaaaa gacgcaaacc tatgattatg cgctatttag 440400
aatcatgcaa cctttaattt ttgcttatga tcacaagaaa aatatccccg aagagcttac 440460
atgctaagaa actcctgaca atgataccgg ggataaaaca agctaagaaa aaattaattc 440520
gggatgtgga aaagaaatat tgttgggact ttggttatac tttgcttcac agaacctaag 440580
tcctcagtag ttctccatga ttgcacttgg cgtgtgagaa caagttcact cgggcccgaa 440640
aggtcagagg caacacaaag ctccgcatag gaaggtaaag tatctagaag agactcaaaa 440700
gtatagacgt tacgataagg agtttctata catactgaag tagatacctc tttggaggtc 440760
gctgcctttt ttatcgactt tacacgttcc ttaggacttt gcgggaggta tcccaaaaac 440820
gtaaagctct gggaaggcaa gcctgaaagc atgagcgcta acgttatcga acagggacct 440880

gaaaaagcct gcacaggaat ccccaaagca cgtgcacgac gcactaaact cgctccagga 440940
tctgcaatac agggaagacc cgcatcagag atcagtcccc aattctcccc gtgttttacg 441000
ataggctcta gataaaaatc ccaagcctta gggaggcgcg catgtttact aagaatagca 441060
agaggaaatt tatgaacttc gggaattttc cataaactta gaaatgccct acccccacga 441120
tcactttcta caatcagccc atctagtcta tgaactaatt ctcctataac ggaggggaga 441180
gtctctacag cacgggtacc gagagtattg ggaagaagat ataaagtcac aggttaacca 441240
ttctaatcac taaagtttcc acagctacta taggatcttg gacattattt ttaattaagg 441300
tttctgcata aaataaagaa tttaaggctt ggtgtagtct ctcctttcca taaaggacga 441360
acattcggtg tttattttct ttcgatccct cttcaatact acgtaaacca tagagacatt 441420
gggtacgaag gaaagtaata atccccaagg gatcttcacc atcctcgagt agaaaatgca 441480
actgctggtg accttctacc ggatccctct tcaatagaga gtctcgaaat ttccataggg 441540
aagccttttc ttttttgaca acgagctctt taatatcaga gtgatccaag gacgttttct 441600
tgccaacaga gcacagtagc ttatcgaatt cactgagaat atcaggaaga gaggttgaag 441660
caagtgcacg caaaaacaaa gatgccaatg attgagagca agaaatcccc acacgctcag 441720
ctctttgcaa caggaggcgt atgatccttt tctgacgatc tgcgggccac tcaccaaata 441780
aactcaaaga aagagccgat ggcaaggctt ttgacagttc tcgaaagcat tcttgttttg 441840
tggtgaagat caaaatcgta aggtgaggtt gaggattccg agcatagcgg cttagaaatt 441900
ccttagttgc tagagggaat ttctctgcat gaataatccc caaagtttca tgctcttgaa 441960
agagtgcaaa cgtctcggtc caagacatga gggttgctgg catgagtccc tgaccaccga 442020
gctctttgaa gctctcagag actaataatt caatcagcgc atctttatcg tcttccaaag 442080
cactccctat aagagctata gcgggcactt tctctgcata cgcctgggaa aagtcatcaa 442140
aactcgttaa ggatttttgc ataggtagag tcaaaaaagt agtatcctga acctactata 442200
cgtattttcc taccaactgt acagcatgaa gcctgaagat ctcgctctat aaagcttttt 442260
aaccttaaaa agaaaaacgg gaaggaaacc caatgtttcc ctttcccgtt aacatgagca 442320
ataatgcgaa aatcacaaat tatcgagaca tcattttgat cagttggtga cgccagccat 442380
nagctgtacg aacacggctt ttagagccat gctttctcgt agctgttgaa gaacagacac 442440
gtttacaact agatgtatgc ttatggtttt ngtggcatgc taaagcacag gctgcagctt 442500
tcttaggaga acctttagcc actgtagttt ttctagctac aggcttctta gcaacagtct 442560
tgcgaaccgc tctcttagct gtagtcttct ttgctactgt cttttttact acacgtttag 442620
cagcaacttt cttaactgca ggcttcttag caacagtctt acgaactgtt ctcttagctg 442680
tagtcttctt tgctactgtc tttttagcag ccgtcttacg aactgcaggt ttttttacag 442740
cggttttgcg aacagtagct tttttaaccg tacgtttagc cgcaactttt ttagcaggct 442800
tccgtacagc tcttgaagct gtctttttac cgctttgctt tttttgcgct ccaatcatct 442860
ttattcccct aattagacag gtaattactt acctgatcta tcggcaggga cgattgaaaa 442920
ctttaataaa aaaaatgact tttattttaa aaaaactaaa ataaaagtca ttactaaaac 442980
ataaataaat agaaagcaac tacttagaaa gactattttc taagtaataa agaatagaac 443040
gaacaccaaa acctgaagca tattttggat aacggtcttc ttctttttca tgatatgcag 443100
tacctgcaat atcaagatgt gcccaagcta ccgaagattc ttccaaaaat ctctgcaaga 443160
ataatgctgc tgtaatagcc cctgcacggt tactgcctag atttttcata tcagcaatat 443220
cagaatgcaa tgttttatca tacttcttaa ctagaggaag tctccataac ggctcggagg 443280
tttcggctga cgcctctaaa agatcttcag ctaaaacatc gttattggaa aagaaacctg 443340
caacctcttc tcctagagag actaccatag ctcctgttag agttgcaaaa tctataatac 443400
gtgtcggttt acaatatttt aaagcatatg taatcgcatc agcgaggata agacgtccct 443460
cagcatcggt actacaaatc tcaacagaaa gccccgacat tcctacatag acatctccca 443520
ttttatagga ggcgccatcg atagcattct ctgtaagcag gaatgatccc cgtgacattt 443580
ataggaagct ctaaaantgc taacgccgag agaatcccga ggactgtagc cccacctgcc 443640
atgtcttctt tcatagtaag catggatttt ccaggcttga ggtctaaacc tccagagtca 443700
aaagtgaccc ctttccctat caagacggtg tgatctttag acttaggacg tccttgataa 443760
cggacaacga taaagtgtgg atccacacaa gaacccttgg aaacagccaa taggagtccc 443820
attttctctt tggcgatggc atcttttccc aagaccttag tatcaatact agggaactct 443880
tttcccagat tcagagcaac ctctgccaat ttcttagggg taatttcatc agcattcctg 443940
ttcacaagat ctcgagtgag atatacgcct tcgaaaatgg cggcttcttt cctaaagata 444000
gcatccgcca ttttgggaac gataccgata accgtgactt tagaaagagg agtttcaaga 444060
ttacgatcta ccttattata acgtgggtag tcatagttta atgacaaaat tcctgaggac 444120
aaccccacta agaattcttc ggcagaaagc cgcaattcag aaattgtagg taagatgata 444180
ttgactgtgg aacactttgc tttacgtaag acacgagtta gtgtcgcata ggtttggaaa 444240
acaacatcag aggtgagctc ttcatttttc cctaagccta agaggacaat gcgtttttcc 444300
ttagctttag gactactata aaggagttca atctccccgg tttttccttg aaagttttct 444360
aaagcgggga gatacgaggg ttcaaactcg gcttcaaaag aagctgcatt ttttgcatcc 444420
ttaaaatgcc aaaagggcag gactatagca tctgccttaa cacgattacg cccagaggct 444480
tgagcatgaa ataaaaccac aaactctcct ttaatgacta ggaattaaaa aggaacatct 444540
tcacagacat actgctgttc ttgaccataa ccagcataca tatctttatc tttaatagct 444600
tctgcgtcca gtgcttcacc ttcaaaccct acggatacag attcatatcc cacttgctga 444660
tgattgtctt ctaaagatgg agaacggctg ccttcattgc gaccgaaagg actgaatttc 444720

aaagaatcta cactaatcac taaagaagaa ttgcggtgaa accatctttg ctcatgtaac 444780
tctctacaga gatatcgcca gcaacaatga ctcctgagcc tttcttcaag taaggaagca 444840
tcttatcata gcgattgtgc caaatattgc atttgcacca aacagtttca tctttcattc 444900
caactcgagt cttcactccc agtctcagag tgatcacacg ttttcctttg gaagtcattc 444960
gctcttcagg atctgcccca aggtaaccag caaaatgccc aaacatcata agatagcctt 445020
tagatttcta ttttaatact tctttaatta atacatctta agaacttctc aaaacagatt 445080
caggacaaaa acgagaagtc aataaagtca tttcttctct aagaagagct ttatcaatct 445140
tttaaattag aaaaaagatg tagtacttat atgcttaatt aagcacgaat acctgtagga 445200
ggaggttgca ttccaccacc gccttgaggc atttgtggca ttgtatctac agaaggttct 445260
ctaccagcac aaatatcagc acacacggta cgccacttaa caacagtttc aataaacaat 445320
tgagcaaatg cttttagtaa attcgtttca gcatatttca tatccaaaac gcagtgcatg 445380
agaatgagct gttccttagt agcaacacct actccacctc cagccatctg accgccaagc 445440
atagatcctt ctaataattt ctcatataag gccaatttcc tctgagtatt atcagggagt 445500
ccatctaaca gaggagcgta gacataaaga cgatcagaat gttcttcata agtaaggtga 445560
agagagaact caccatcaac aaataaaatg cacgtgttat tctgatcaaa agctacatcc 445620
ggcagtttta attctttagc aaaatttttt agattttcct cagcattttg cctgggacat 445680
gagggaatct ccttgtaata gattgttgtt gtttcaagtt accataagag tgatttgtta 445740
gtaaacgatt tcagaggctt aaaatcaaaa actcccttgt atttctggcg ccgttatgat 445800
atctgatcga cgatggaaaa agtttcttct tatccctcag ttcctttacc tcttggggct 445860
tctaaaattt ccccaaaccg ctatcgattt gctttatatg cttcacaagc taccgaagtc 445920
atccttgctt taacagacga aaattcagaa gtcatagaag tccctcttta ccccgataca 445980
caccgcacgg gtgcgatttg gcatatagag atcgagggta tttctgatca atcgtcttat 446040
gcatttcgtg ttcatgggcc taaaaagcat ggaatgcaat actcttttaa agaatatctt 446100
gcagatccct atgcgaagaa tattcattcc ccacagagtt ttggttcgcg aaagaaacag 446160
ggggattatg cattttgtta tttaaaggaa gaaccatttc cttgggatgg tgatcagcct 446220
ctgcatttgc cgaaagaaga gatgatcatc tatgagatgc atgtacgttc cttcacgcaa 446280
tcttcttcat ctagggttca tgctccggga accttcctag gaatcattga aaagatcgac 446340
catctgcata agctgggaat caacgctgtt gaactcttac ctatctttga gttcgatgag 446400
actgcgcatc cttttagaaa ttcgaaattc ccttatctgt gcaattattg gggttatgct 446460
cccctaaatt tcttttctcc ttgccgacgt tatgcttatg cctctgatcc ttgcgctcca 446520
agtagagagt ttaaaacttt agtaaagacc ttgcatcaag aaggtattga ggtcattctt 446580
gatgttgttt ttaatcatac gggcttgcaa gggacgacct gctctttgcc ttggatagac 446640
actccgagct attatatttt agatgcacaa ggtcacttta caaattattc aggctgtgga 446700
aacactctca atacaaaccg cgcccccacg acccaatgga ttctcgacat cttacgttat 446760
tgggtagaag aaatgcatgt cgatgggttc cgatttgatc ttgcttctgt cttttctcgt 446820
ggtccttcgg gatctcccct acaattcgct cctgttttag aggcgatttc ttttgatcct 446880
ttacttgcga gcacaaagat tatagctgag ccttgggatg ctggcggttt gtatcaggtg 446940
ggctatttcc ccacactgtc tccaagatgg agtgaatgga acggtccgta tcgtgataac 447000
gtgaaagcat ttcttaatgg ggatcaaaat ctcataggaa cctttgcttc tagaatttca 447060
ggatctcaag acatctatcc tcacggctcg cctacaaatt cgattaacta tgtcagttgc 447120
catgatggtt ttacgttatg tgacactgtg acttataacc acaaacataa tgaggctaac 447180
ggagaggata atcgtgacgg cacagatgcg aactacagct acaatttcgg aacggaaggg 447240
aaaacagaag accctggcat tcttgaagtt cgtgaaagac agttacgaaa tttttccttt 447300
actttgatgg tctcgcaagg cattccgatg attcaatcag gagatgagta tgcccatacc 447360
gcggaaggca ataacaaccg ttgggctttg gattcgaatg cgaattactt cctttgggat 447420
cagcttaccg caaagcctac actgatgcac tttctctgtg atctcattgc gtttcgaaaa 447480
aaatataaaa cactttttaa tcgaggcttt ctttccaata aggaaatcag ttgggtagat 447540
gctatgggaa atcccatgac atggcgccct ggaaatttct tagcatttaa aataaaatcg 447600
ccaaaagcgc atgtatatgt tgcttttcac gtgggagctc aagaccaact tgcgacctta 447660
cctaaagcct ccagcaactt tcttccttat caaatagttg ccgagagtca gcaagggttt 447720
gtccctcaaa atgtagcaac gccgacagtg tcgctacagc cccataccac gctaattgcg 447780
atcagccatg cgaaagaggt tacctgatct ctccgtccag ttcttcattc caggattcta 447840
taactacaaa atccacatcc ttgtagaact tctcaagaat ctgacgtgca ttgaatcctt 447900
ctatagcaag acgatctacg ttggactgca agagcccttg tgcatctata attaatcctt 447960
gagggctatc cacaactaaa cgagctgtcc agtctatagc ctcttctaca ccatagaact 448020
gcttggtcac accaaatcct gcataccctt cttcttcagc tttaacatgc caaaagtttt 448080
gaggatttct tgcaaggagc ttttcgtata gagcggttct ttctagaatg atgcaagcag 448140
atagagcttc tttatctagc tcttcaaggt actttataga aagtacagat ttcagataat 448200
cttcgattgt aacttcgtta gaaaccatga tgcaatggtt gtctttacga tgaacgtata 448260
gggaaccttg atactgaatc ccgttaaaaa aaagagaagc agtgtcatct acaggctcga 448320
tctttaaaca ctggagtccg ggataaaatt ctccccaacg gatcccttcg tatagagcgt 448380
ggaccacgca acgctggcct tgaatcgctg tgtctaataa gacattatct ccataaatgc 448440
gataaggacc tttggcttct atgagagctg tggtgctttc attagataaa aggacacgaa 448500
ttttaggttc aacgacagta tcctgcttca caaaagtatc ggataccttt acttctgaga 448560

atcctgagat actcatactg aagaaaagac ctaaaagtac gtttttcaat agtttcactg 448620
tccttctcct aaactcaata agttctttgc atgtcttttt aaatggtcgt aagcaagttg 448680
tgttaccatt ctgcctctgg gagtcttttt gataaaacct tttaaattta aaaacggttc 448740
ataaacatct tcaagagttt tgatatcttc tcccacagct accgataagg ttttaattcc 448800
aacgggacca ccttggtagt agtcgatgat tgtagtgaga agtttgatat caatttcatt 448860
caatccccaa tcatctatta atagcatagc caaagctttt tctgctacgt ccccattgat 448920
acagtttcct tctcggatct gagcaaaatc tctgacccaa cgtagaagat gatttgccag 448980
tcgtggcgtc cctcgggatc tcttagcaat ttctagtaat gcggagctgt cagcttcgat 449040
tccgagtaaa tgtgaggagc ggactaaaat ctcttttaga tcttgatccg agtaatagga 449100
aagtctcgca ctaaaagcaa agcgtgttct taaaggttcg cttagcattc ctgatcgagt 449160
cgttgctccc actaaagtga aaggagcaag atcgacacgg accgagcgag ctccgggtcc 449220
tgaatctata gtaatatcga ctttgaaatc ttccattgca gaatacaggt attcctcagc 449280
aactttcccc atacgatgga tctcatcgat gaaaaacacg tccccttctt gcaaactagt 449340
taaaagtcct aacaggtccg agggtttgat taactgaggc cctgatgcca agaccagccc 449400
tttccccacg gtgtaggcaa cgatgtgagc aagtgaggtt ttccctaagc ctgggggtcc 449460
aaaaaacaag caatgtcctg gaacttctcc tcgttgcaat gctgcgcaaa gaaatagatc 449520
taggcgttct tttaaatgat gctgtccata aaattcttct aaccctttag gtcttaacga 449580
aacatcaaat tttttatcct gatgcaagac agctacttga tgcgtcatgt acgaaccttc 449640
tctatcgctt tttctttagg tggattttat atttttttga aactctcatc gataacgccg 449700
atcggttata ctcggcatag tacacgagat actatataga agtacactat ggacctcttc 449760
tttctatcaa ggagagcagg gccaaagtac gagcactaga acctctccat cattttcatt 449820
ttatgattta aacttcctgt ggtgatctag atttcttttg tcttaaaaca gataaattaa 449880
agatggcgaa ctatttttaa aaacattttc cactgagaag ttggtagtaa aaattctcta 449940
gcttatcaat aaggagatgt taatcttttt atgaatccaa gaagttctta tcaaggtcct 450000
cgcatgaatg cattcaagag aaccaagatt cttctactat aagagtaaag agcagaccta 450060
cctgaagttc tccctaaaaa taaggtttgc ctttttccct taggattatc taacattacc 450120
tctaggtttc gtgataaaat tgtagccttt gggataggct tttcttgttt atacaactgg 450180
tcttttcaag agataatcaa agtctatctt atacttagaa aagttccccc caaaaaccag 450240
taaattgaaa aacagaatat cattaagaaa gcaatttcaa actgttaagg tttctaaatg 450300
agcataaaag aagataagtg gatacgagag atggccctaa atgccgatat gatccatccc 450360
tttgttaatg gccaagtgaa cgtaaatgag gagacaggcg aaaaacttat aagttacggc 450420
ctatcgagtt atggttacga cctccgccta tctcgagaat tcaaagtgtt caccaatgtc 450480
tataactctg ttgttgatcc aaaatgcttt actgaggata tcttcatctc tattactgat 450540
gacgtctgta ttgttcctcc aaattctttt gctctagctc gtagcgttga gtatttccga 450600
attcctagaa atgtcttaac aatgtgtata ggaaagtcta catatgcacg ctgtggaatt 450660
atcgtaaatg tcacaccttt tgagcctgaa tgggaagggc atgtgactat agaaatttct 450720
aacactacgc cattgccagc gaaaatttac gctaatgaag ggattgccca ggtcttattc 450780
tttgagtcta gtacgacctg cgaggtttct tatgcagaca gaaaaggaaa gtatcaaaag 450840
caacaaggca tcaccgtacc ttgtgtctaa agtttcagta agaaaaaaaa actggggttt 450900
tagattacta gaagaagtga tgatcaaatc ctggtgggtg atctttagca tcttaattgg 450960
aggctttgtt tatgatcgtg ctatccagga gttacgtaca gaagagctac gcttacaaag 451020
caaggtctct tctttatgcc aagacattct ttctgctcaa gaaaagcagc gtcaactcca 451080
attacatctg caacactggc aagactccgc tgctatagaa gctgctttaa tccagcgtct 451140
gggtctcatt cctaaaggct ataagaaact ctgtgtctcc ccaaagcaac aatcagaaaa 451200
taaggactga aaagagacca tgattcctac catgttaatg ttcttcatta tctgttttac 451260
tttatgctcg ggattcattt cgttatctca aattgctttg ttttctttgc ctacgagttt 451320
gatctcgcac tataagcgct ctaaatctaa gaaacagcag cgagtagcta cccttcttct 451380
acatccccac cacctgctca tcaccttaat tttttgtgat atcggactga atattgctat 451440
tcaaaactgt tttgccattc tatttggaga tgcagcttcg tggtggttta ctgtaggtct 451500
tcctttagca attactttga tcttaggtga gattctccct aaagcagtag ctcttccttt 451560
taatacacag attgctagtt ccgtagcccc tcttattctt tgtgttacta aaatcttcaa 451620
acccctactc cactggggta tcgtaggaat taattatgtg gtccaatgga ttttatcgaa 451680
gcaacagatt gatatcatcc aaccccaaga gctgaaggaa gtattgcaaa gttgtaagga 451740
tttcggcgta gtcaatcaag aagaaagccg tttactctat ggttatcttt ctcttagtga 451800
ttgtagtgtt aaagagcgta tgcagccacg ccaggatatt ttattttatg atatccaaac 451860
ccctttagag aacctctatc ttttattttc taaacagcat tgctcacgag ttcctatatg 451920
taacgataac ctccaaaacc ttctgggcat ttgcacagcg cgctctcttc ttttacatga 451980
caagccactg caatcttcgg atgatctcct ccccttgctg aaaaaaccgt attatatgcc 452040
agaaaccatc tctgcaaaaa tggctttatg tcagatggca gctgaagacg aaaccctagg 452100
gatgatcatt gatgaatacg gatctattga aggattgatc actcaagaag acctctttga 452160
aattgttgct ggagaaattg tagaccagag agataataaa atactctata ccacctcagg 452220
agctgatgtt attattgcct caggaacttt agaactccgt gagtttagtg agatcttcga 452280
tatcaaccta ccgacgaaca ataatattgc gactatagga ggctggttaa tagagcaaat 452340
cggaacgatt ccgacaacag gaatgaaact ctcttggaat aacttgcttt tccaggtatt 452400

agacgctgct ccgaatcgca ttcgccgtgt gtatataagg aaattgtatg actaattctg 452460
ctctcttttg gataggagtc aacattatct gtattgtctt acaaggattc tattcgatga 452520
tggaaatggc ctgcgtgtca tttaaccgtg tacgattgca atactatctg actaaagatc 452580
ataagaaagc tcgctacatt aatttcctga ttcgccgccc ctatcgttta tttggaacgg 452640
tgatgttagg agtgaatatc gctctacaag tcgggtctga gtcctcaaga aattgctatc 452700
gagctttagg aatcactcca gattacgctc ctttcactca aatttttata gttgtgattt 452760
ttgcagaact tctacctcta acaatatcac ggaagattcc tgaaaaatta gcactttggg 452820
gagcaccgat tctctattat tcccactata ttttctatcc tctgattcag ctcataggaa 452880
gtctcactga gggtctttac tatcttctaa atattaggaa agaaaaattg aactctacat 452940
taagtagaga cgagttccaa aaagctttag agactcacca tgaagaacaa gatttcaata 453000
caattgctac aaatattttc tctttaagtg cgacttgtgc agatcaggta tgccaacctt 453060
tagaacaggt taccatgctt ccttcttctg caaatgttaa agatttttgc cggactataa 453120
aaaatacaga tatcaacttt attcctgtct atcacaaggc ccgaaaaaac gttattggga 453180
ttgcccatcc taaagacttt gtcaataaag ctcttgatga acccctaatc aataatctac 453240
actcgccttg gtttatcact gcaaaatcaa aacttattcg tatcctcaaa gagtttcgag 453300
acaaccgttc gagtgttgct gttgtcctca atgcttctgg tgaacctata ggtattctta 453360
gtttaaatgc aattttcaaa atcttattca acactacaaa cattgctcat ttaaaaccca 453420
agaccatctc tgttattgaa agaacgtttc ctggcaactc tcgcataaaa gatctgcaaa 453480
aagaactcga tattcaattt ccgcaatatc ctgtagaaac cctagcccaa ttggtattgc 453540
aactgctaga cagtcctgca gaagtaggaa cttctgtaat tatcaacaac ttgcttttag 453600
aagttaaaga gatgtcttta tctgggataa aaaccgtatc gattaaaaac ttactctcat 453660
agattctgca ataagagtca ggagtgttct ttcagcttag aaacatgttc tcttttaaga 453720
cttaggaatt tttcaaacgt tctacgactt tttctataat tccaacggct ctttcaacat 453780
cttcttgcaa gagaagatgg ctaaaagaaa acctgagtgt tgccagggtc aactcttcat 453840
caacacccat gctgacaaga gatttaaagg gtgcggtagc acctgaagag catgcggatc 453900
cataaccaca agccactcct tctatatcta aggcgatttg caatacctca ccttccaacg 453960
gagggaaagc aattgctgag acgttgtttg cccgtggttg atccgcacaa tgaatatgga 454020
catcaggaat gcgtgctttg attgcctttt caaaaccatt tctatgggta agaatttcct 454080
gagagatacg ctcttgatga agatctaggt atttgaaaat ataaagcaga gaggcgattc 454140
cccaaagatt ttctgtgcct gcgcgcagcc ctccttgctg acctcctccc cacagctgag 454200
gatgtagttt gactcctgga gagaccagaa gagctccgat tccagagagt gcatgaaatt 454260
tatgtccact gaatgctgcc atagtgacac cagagggaag aactatcctc tccttaccta 454320
catttgcagt cgcatccaca ataaattgca attgtcgttc ttgcgcgaag tgggctatag 454380
cagctatatc agctttggca ccagtctcac tattgaccca acctaagatg attgctgaag 454440
ttttaggagt cacagctctt tcaatctgct ctatagtaag aacacatctc ccttcttcgg 454500
gatttaaata agaaacggaa agcgaggaat gttttaaagg ctctaagatg gcggggtgtt 454560
cgctacctga ggtgataaca tgactgtctt tagggaggct tgctattgct aaatttaaac 454620
tctcagtagc ccctgaggta tagaggacac ggccctgaaa cgaaaggacc ttttgcatcc 454680
agtgtgaagc ttctagaacc agttgacgag attttttacc taattgatgg acgctcgaag 454740
gattcgcgta cgtcccttct ataaggaagg ttttttggag aaattccaaa agtcccctct 454800
ctgggggtgt catcgcattg ttatccaaat agatcatgga atactatcct tagcaaagga 454860
actattgtat tcggactaat acgacagtag cgttgtcatc gcctccacga gtattggcta 454920
gagaaattaa tgcattcccc cgttcttcta gggtggcggg ctggttcaag atatcacgaa 454980
tatcgatatc tggaaccatg tttgtcaatc catccgaaca gaggcagtac aaatcttcct 455040
tttcacaagg aagattccga atgtcaggca tgacataggg acgacttccc aaaacattag 455100
tcagaatatg gcgataagaa tacaccttat ctgattgttt aggaagccca taacgatttt 455160
ttaattgatt ttctaaagaa tggtcttcgg taaggcggcg cagttctccc tcacgaatac 455220
gataaattcg actatctccc acatgaaata gccatgccct atccttccgg aattggatga 455280
agctaagagt ggttcccata ccctggagat gctcttccat ttggccgtgt tcatagacca 455340
caccattgac ctctaaaagg atctttttta aagtctcctt atactggtca tcctcatacc 455400
ccatcaattt tgactgttgc tcatcaatca gctccataag gctagtcact gcctcttgag 455460
aagcaatgtc tccaccaaga cgccccccaa caccgtcagc aatagcaacc acttgagaca 455520
tgaggtttac ctgccaaaaa tcttcatttc tagcgcgcac cctaccaata tcactcagac 455580
caaaataatc aaaatccaca aagtgctcct aaagagatcc tggatcacaa atagagtccg 455640
cccctataga cattctctaa tttcatatca ttattataaa gaattgaatt ttcctgacga 455700
aatcataatt tatcgctaaa ttaatggtcg catgatcgtt aattacaaag aaaaatttga 455760
taaataatcg ccctagagct aggctggaaa ggtcaattaa cctaaggatg aataatgaaa 455820
aaagcattct ctttactatt atcttgttta gtattgttag ctcttagtgg ttgcgtccct 455880
cctcaataag ggttcaacta cgcaccatga gttttaatca caaactcatg gtgttcctta 455940
gaagttcact aagaaccttt tcatcatagt ttttatgatg ataaagtaaa aattgctata 456000
aatcgaagaa ctgcctatgc aaatagaaaa tagtagtatt ctttttgcag aggtagtcat 456060
gaagtggttt attttttctg tgatctcagc tccagttgta ttcctcccag ggtgcacatt 456120
gattcctaaa gaaaaagtta ccaaagttcc ctcacaactt tggtcagaat ccctttctca 456180
accttgagtt tctaaactaa aactgagacg gtctctttgc ttgtttttat ttttttcttt 456240

gatataagct aatgagagtt ttaaatggaa aatctctcaa ttgcgaaagt attgatctta 456300
aaagtaaaaa ctttcccagg gctagaatct tttgcaaaat aagcaattta aggacagtga 456360
ctatgcgaaa aatgttggta ttattggcat ctttaggact tctatcccca accctatcca 456420
gctgcactca cttaggctct tcaggaagtt atcatcctaa gctatacact tcagggagca 456480
aaactaaagg tgtgattgcg atgcttcctg tatttcatcg cccaggaaag agtcttgaac 456540
ctttaccttg gaacctccaa ggagaattta ctgaagagat cagcaaaagg ttttatgctt 456600
cggaaaaggt cttcctgatc aagcacaatg cttcacctca gacagtctct cagttctatg 456660
ctccgattgc gaatcgtcta cccgaaacaa ttattgagca atttcttcct gcagaattca 456720
ttgttgctac agaactgtta gaacaaaaga cagggaaaga agcaggtgtc gattctgtaa 456780
cagcgtctgt acgtgttcgc gtttttgata tccgtcatca taaaatagct ctcatttatc 456840
aagagattat cgaatgcagc cagcctttaa ctaccctagt caatgattat catcgctatg 456900
gctggaactc aaaacatttt gattcaacgc ccatgggctt aatgcatagc cgtcttttcc 456960
gcgaagttgt tgccagagtt gagggctatg tttgtgctaa ctactcgtag tctaaggaaa 457020
tgtccaagtt tattcttctc ttgtcccttg gcgtcgctgc tctagcttcc aaaaacttct 457080
ttatctggcc agcaccctct gggaaaacac ctttaaantc cgccaagttt tatttggtgg 457140
tgctcttctt gtttttcttc cccttgtagc tcttagcgtg agctcacaaa ctgcggaatt 457200
actttccacc atgacaggaa ttagccttgc ctttgcattt ctgttctacc tgcnttttct 457260
ccccaaggat atcacacgtg ctatactttt ctctggagaa agancggtta aaacttcatg 457320
gcgtgctcta ggatctgcca tcagaatgtg gatcatcatc atcccagtaa cacaactgat 457380
tgggattatg atgagtaaat ttataacttt ggttcttcct acgcaagaga ttcacacaca 457440
agaagtcact caagaagttc agaactctct gcctataaca ggacactaca ttagcatgat 457500
tctaaattta ggcgtcctca ctccatttgg agaagaggta ttttttagag gaattctaca 457560
gacattcttg aaaaacaaaa tgacgcgcat agctgcggta ctatgctctt ctattatttt 457620
ctctttcatt cacattgaac actctttagg aagttgggtc ttttgtcccc gtgctctttg 457680
tttttccctt atctgcaggg tttctatatg aaaaagatcg gcacattctt tctcccattg 457740
cactgcacgg gttgtttaac ctcacctcat tgctattttt gggaataaag taaaaagagt 457800
aagttggaga acattctttt agcaaaagaa aactctcctt agggctgcta ccttccagtc 457860
ctgacccgat tcgcaggttc cttctcttaa aaggccccca acttaccctt ctttgatagc 457920
atatattctg ttttttcaaa agcaccatat gcttaaaaat agaccaaaac ttcaactatg 457980
tagattcatt tttctctcgg tgcttagcaa gctcccaaag gcgatcggcc tcttcaatcg 458040
agaccggctt gtaatcttca gcgaatatat aaggagcacg gcgacgcaat ttttccatag 458100
cctcattggc aacgtcttcg gaagcaagta cgccctctcg ttctaacaga aaacataaaa 458160
ttagaactaa agttaagaca tccccggctt cggaaccaac ctcttgtacc gtcttacctt 458220
gtaagacggc ctcgtgaaat tcctgacact ctccaagaat atgctctacc atagagacta 458280
gggattgctg aagtgaccaa ggacaacgtc cttcaactac catggcacgg acagtcccta 458340
tcaatttaga aaaagcgtga tctcgcatct atactcctat tgattctcta gacctgacat 458400
ccccgaggta gattagcaaa gcatctttaa tctttttgtg tcctgaagaa aaagctaagt 458460
gatctatatc actcaaaaga tgcaattccc cgaactgagg cagagaagtg gctttaaaaa 458520
ttataggaca caaatgaacc ttatgattag taaacgcatg ccgctgttct ttaaggttac 458580
ctaagaattc caaagggctt tctaaagaaa gctccatctt cttagtaaat ccttctatat 458640
cttgaagacc ttcctctggt tcaacttcaa tataaggaaa ttcatataag cctgccatca 458700
tttctttagg acgtctcttc tcgacaacca aagagccatc gtacaataca atcgctacca 458760
aacgatgcaa aaagatgacc ttttttctgg catgacgtac cggcaatacg aactgtttgt 458820
tctccctcca agctccacat gcttgacgga caggacaacg atgacattga ggaacttttt 458880
tacagataca agctcccaac tctatcagag cctcagctat aacctcggga ctcttatgag 458940
gaagaagcgc ttgagcaatc ctagaaaccc aagtacgagt tgattctaag tctatagaag 459000
tttctatcaa aaatatccgg ctaagaacac gcaagacatt gccatccaca gcagcagcac 459060
gcctcttaaa agcaaaggct agaatagcat gaaccgtata aggaccaact ccacgaattt 459120
gagctaagga aatggcatca tcagggatct ttccatgaaa ctcctccata accatgcgag 459180
ctccctctaa aagatggcgc gctcgagaat aataacccaa tccctcccat aacttaatga 459240
catcttcttc ttttgctgca gctaaagact ctatggtagg aaatctctcc atccactgat 459300
taaaataatc tataacaact tcagctcgcg tttgctgtag cataacttcg gaaacccaca 459360
cactataggg agtcgggtta tctctccaag gaagagatcg tttatttttt tcaaaccatt 459420
tttttaatgc ctctacagga aaattctttg ccttttcaga aaaagctatc tttgtcatac 459480
aaaatctctt aaaattttat gcaattatca aatgataaaa gggctgcttt acaatatttt 459540
atggaaaatt tttcctggct tgccacacaa gtctcaagat tatcttcttt tcttagatct 459600
caactgccta atcatagcaa gcaagaaatc ttggcgtcta ttcgccaaca tcgatgtcga 459660
gtgaacgggt tcatagaaag atttgaatcc tacaaggtac aacctggcga ccgtgtttcc 459720
ctatctctga tcccctcaac aaaacaacaa cctagcatcc tctgggagga tgactatagc 459780
attatctacg aaaaacctcc ccatcttact actgaacaaa tggcacacat gacacggttt 459840
tttactgtgc ataggttaga caaaggcacc tctgggtgtc ttctcatggg aaagtctaaa 459900
caagcggcta ctgagctcat gaaattgttc aagcaaagaa aaatccataa acaatacata 459960
gctttcgttt ttggtcatcc taaaaaaaaa tttggaaccg taaaatctta tacggccccc 460020
gtataccgga ggtgtggagc tgtgattttt ggagccgcag gcccatcaca gggagaaccc 460080

atcaaatccg cttacaaatg ggattgctgg gtcatcctat tgtcggagat gtcgactacg 460140
gacctaaaga acagcctccc cagatcttcc gccctctcct ccatgctcac tccctagaat 460200
ttatctcccc attcacaaat cttcccctaa aaatttgtgc gtcatcaacc gaagatccta 460260
gagaatgtgc tcggcactta cttcaagaaa aacccctaga actttacaat tagagattca 460320
taaaactgta attggagtcg tttacactgc ttcaaaacat aacctcctct tcttcatctt 460380
ctaaatcccg cctttctacc cttccggggt gcgacctagg agaaatcaca cttaaaggta 460440
aaactacagg aaaacgccct ctagaagaca caggggaacg ctctctagca cctaataaag 460500
aaggttgaga cgtaaccaca ggatctggtt gaggttgcgg atgtgccgtg actccagaag 460560
gtgtgggtac aggggggcgt tgtaaattta gtcccgccgt atgcatcaat gaagttgaca 460620
tctgtagtag gcacttatct ctttcttgaa cattgttagc atcgcttaac aaacttgttt 460680
tcgtaagaga attctcaaaa catcgtgcag ctaattctaa atagcgtgac gaggaagatt 460740
ctccccctcg cttcctttcc agatcgacac gcatgcccat cccccgagcc aaaatagcat 460800
tgacttctgg attacctaaa atgttgccac gttggggatc tcctgtggct gggacaaaat 460860
aacattgata gtgagataaa accaaagcaa ctaacgccat cagattttct ttggtgatcc 460920
aaatcggagt gcttcctcca ttggtagcta caaagaaaat agacattaat agattcatgc 460980
aggatgcaga caactggatt cccatgttct cgcacccgtt ctttttaagg gagaagacct 461040
taagtgatgg gaaagacata catatcctct ctaggttaaa agggctacaa acctgtgctc 461100
cttgctctcc ccatgaagaa agaaccatca ctttgctttc acactcgaat tccgtttcct 461160
gaagagaaga cttagcaatc cctgaggcaa ttgctgtact ccaaaaagga tcctgtaata 461220
aaccatccac acaactcgcc gcatccgccc acagatccat gctttgagac ttcaagatct 461280
cacaaaagtc catagaacac cggtcacatg cagccatgac ctccgcgatt ggaggaaatc 461340
cctcgagtcg atgtcctgat aataagctca tggtatcaat gccaagctca cttaacgcaa 461400
ttaaaagaac agccttgcca tatttctgtt ctaaagcctc gagttcttat aaagggattg 461460
cgggttcttg ggatccctct cctatcccgc aacaattttt ccatacacca cagaaaaaac 461520
ggccaaatcc tccacaaccg cactgtccgt ctggacagcc acaggtagga caaccgcagt 461580
tgccttggca ggtctctcca caataattat aacaaaattc gctagttttc tttgctccag 461640
gacttgttac tgctgcataa atgatctgtg ccaacgatgc agctgtccct aaaggatttg 461700
ctgctgcaga tcctgttgcc gataacaaat tcaacagtac cgaagcggcc tgtcctgcct 461760
gagttccacc acttcctcct tgagacgtag tcatctggct caccaagcta aggagttgct 461820
gtaattgctc tgaactcacg gacgatccgc cacttcctgt tgtcttgtct aagataagtt 461880
gcaaaagctg ttgctgcata cttgcactgg aagaggaggc tcccgaagtt cctgctgaag 461940
ttgatggttg tcccactgat ggttgtccca catttaataa attagtaagc aacgcttgca 462000
cttgatcagg tgaaagtcct aatgcagagg ctccggatgc ggtagtagtt gtcgctgtag 462060
aggaggcagc tcctccggtt tgaagaaaac cttgaactgc agatgagact gcttgtggtg 462120
ttgtggaaga aggcgctatc gtaacaacct gtggattctg tggtgagggg aatttcccta 462180
aaggattcgt caaacaacct cctaagtaat cagtgaaacc caaactacga taagttcgga 462240
tcgtaaattc ttttgaaaat ttttttagca gtacgcggga gtgagaagtt tggatattct 462300
ataaaatatg taaagaaaaa aatttaaaga atttacgttt ctatttacat tggaggcagg 462360
atctcccctg ttgaggcacc aaaaggtttt cttaataatc gaaagatttg ttactctcct 462420
tccaataagc ttcaaaagta taaggcggat tcatgctaaa gattgatcta acaggaaagg 462480
tagcatttgt tgcgggcatt ggtgatgacc aaggatatgg ctggggtatt gctaaacttc 462540
ttgcagaagc aggagctacg attattgtag gaacatgggt accgatttac aaaatttctt 462600
ctcagtcttg ggaattagga aaattcaatg aatctagaaa attatcgaat ggcactctct 462660
tagagattgc taagatctat cccatggacg caagttttga tagccctgaa gatgttcctg 462720
aagatattgc tgaaaataaa cgttacaagg gcattacggg attcacgata tcagaagtcg 462780
cagaacaggt aaaaaaagat tttggtcata ttgacattct tgtccactcg ctggcaaata 462840
gtcctgaaat ttctaagtct ctattagaaa catcaagaaa aggttactta gcggctctca 462900
gtgcctctag ttattctttt gttagccttc tctctcactt tggaagtatc atgaaccgtg 462960
gtggatcgac aatatcgctc acctatttgg cttctatgcg cgctgttcct ggatacggag 463020
ggggcatgag ttcggcaaaa gcagctttgg aaagtgacac caaaactctt gcttgggaag 463080
cgggacgccg ttggggcata cgtgtcaata ccatctctgc aggaccttta gcaagccgag 463140
ctggaaaagc aattggtttt attgaaagaa tggtagacta ttaccaagag tgggcgccta 463200
ttcccgaggc tatgaatgcc gagcaggtgg gtgccgttgc agctttctta gcatcacctc 463260
tagcttcagc aattactggt gagaccttat acgtagatca cggagccaat gtgatgggaa 463320
ttggtcctga gatgttccct aaagactcat aaggtcgtca taatagcgga caccagcttc 463380
ccaagctgaa agaatgccat tcttatctgc tgggggagct agaaagtccg catgaacgtg 463440
catctcttca ggtgcggaac tcatcacaat tttaaaatct cctctctcaa taagatcgag 463500
atcattagca tcatctcctg aagccatgac aaagggtttc tttccatcat aaagtatatt 463560
gacaacacga tctaaggctt tgcctttaga gacgctttta tctgttaaaa acaagatggc 463620
atagcgaaag tcaaagggcc agcgcattaa cgtcatcgtc gcgactgaag tcagtgcttc 463680
ttggcgttcc agctcctttt gaattctgat gacctcatct cgcagtccaa agacttttgc 463740
tgcagcaaaa ctaggaaaag catagtcgtc ttttaaagag cgcgtttcaa ataggatctc 463800
tctttcctta gcattaggaa agtacctagg atctacatat tcgtgtaaat cttgagctat 463860
aggagtcggt gaaaagcgat agtagtgatc cccgtaagga gctcctgatt ccacggaaaa 463920

aagagccgtt gccccctcca tacaatcttg taaaatacat aataaatctg agggtaaact 463980
tttagaatag agaagatttg atgatgttga agaccataca gaagcgccgt tttggcatcc 464040
taataaatat ggagcatcaa aatcagaaaa caagcgtgca gcatatttat aataccttcc 464100
cgtcaagaaa aacaacttcc aaccagcttg gtgcagcgca tagagccgct catacacctt 464160
tttatctaaa tgatgagatt gatgggtaat tgtaccgtca atatcagtca ctagtaactt 464220
ttccatagca tcaccatagt tttattaagc gctgccaagg aatttctcag gcaacttgaa 464280
aaaattcttt gcatccaccg acagactgct gtcgttttgt ctatgaaaga caagagcttg 464340
ggctctgtgg aacgagcaca ccctagaagg gtataaagga gtctgtgaat tacccaaatc 464400
atctagggga gctctctaga cggtacttgc aggaatatac atgattacgg tttaatctgt 464460
acttggaaat cattgtacta taatttatat agcatcgact aaaactatac ttcattatta 464520
cttgtatgta gttggataca aatcgggcta ccactataat tttatagaga tgatcgcggg 464580
tcttacacat gtttttaat ctttttcttt tagtttttaa gctttctgat gagcttgctc 464640
ttgcagaaac gatccaagag cccatttctg tacatgaaat gttcccagga agcatgaaat 464700
tagaaatgtt taaaatgcta ggatctttga ttctactttt aacaattttt ggctttggag 464760
tttgggcgtt taaaaagttt gtgagatcaa gaagtcacgg ttttggaggc tcgtctcaaa 464820
tcaaaatcct agaacgacgt tccctaacgc cgaaaacttc tatttacctc attcgagttg 464880
tgaataaaac tcttgtgatt gcagaaacac cagaaaaaat tacgctactg acagagtttc 464940
ctcccgacac tgatatcaat catctacttc aagaaaataa taagcagtct tcttcctctg 465000
caacctctga tttcctcagt aaagcaatac aaaagataca aaaaaaacaa cagacgaatc 465060
aagattagat ctagtattat cagccagcaa tgatgcaggc tctaggaaca acttggagta 465120
tttcctaaat gacgacatgg acattaaatc aaaataatct cacaaaattt cttaaaagtt 465180
cggatgaaga acctttctta gaaagagaaa gcggtcttac ttacattaac attcaagcta 465240
atggcaatga actcccttta ttttttgtaa tccgcagtga gggagaaata ctgcagttga 465300
tttgttacct tccctaccaa ttgcatgaat ctcataaggc atcaacagct cgtttactcc 465360
atctcttaaa tagggacatt gatattcccg gctttggcat ggatgaagaa cagggattga 465420
tattttatcg gcttgtgttg ccctgcctaa acggagaaat tcatgacaca ctattacgga 465480
tatatatcga tacaataaag ctagtctgtg atagtttttc tcatgctatt gggttgatct 465540
cttctgggaa tatgaatttg gatgaactaa gacgtcaggc tcttcaagag caacaagaaa 465600
aacgtaatga gtagtcaaac tatggatgtt cttattttct atgatacgga gaccacagga 465660
acacaaatag aaagagatcg cattatagaa attgctgcct acaatagtgt cacagatgag 465720
tcctttctta cttatgtgaa tccggaaatt cccattcctg atgaggcatc caaaattcat 465780
ggaatcacta cggatgcggt actttctgct cccaaatttc ctgaagccta cgagggattt 465840
aggaaatttt gcggagagga cagcatctta gtggctcata ataatgacgg ttttgatttc 465900
cccctactcg gtaaggaatg tcgcagacat tccttagagc ctctgacaaa ccgtacaata 465960
gactctctaa aatgggcaca aaaatatcgc cccgatctac caaaacataa tttacaatac 466020
ctaagacaag tttacggttt tgctgaaaat caagcacacc gagctctaga tgacgtagtg 466080
atattgcaca aggtatttac ttctttaatc ggtgatttac cgccccagca agtcctcgac 466140
ttgctgcaac agagctatca cccgaaagtc ttcaaaatgc cttttggcaa atacaaaggg 466200
cagcctcttg tggatattcc taagtcttac ttcgaatggc tggaaaacca aggagctttg 466260
gataagcctg aaaataaaga catcaaagcc gctatagctc tattacatca accgacatga 466320
tactgactgc tgccttttct ccttgcccga atgatatttt cctttttcgt tctttcttaa 466380
aagaccccca attcaggcct cttcttaacc aggtaacaat tgcggatatt gaaactttga 466440
ataccctagc tctgcagcga cggctctccc taatgaaaat gtcagcagcg ctcttccctc 466500
tagtttctga ttattataat cttatggacg taggaaatac cttaggatac aacagcggtc 466560
ctatcgtcct ctccttagat cctgaatgtt ctctagatac cttggcaact cctggagaga 466620
tgacaaccgc tcatgctctc tgtaaacttt actatcccaa ggcaaaactc attcccatgc 466680
cttatgacaa aattctatcc gcgatactgc aagggaaagt cgatggaggc gctctgattc 466740
atgaagagcg cttcagctac gatctccaat tgacattgcg ggcagacttt ggagagctat 466800
ggcgccgtaa gaccatcttt ccccttcctt taggatgttt agccattgcg aaatatgttc 466860
ctatggctac agtggatgct ctaacagcag cattaagaaa gtctttaatt tgctccctga 466920
aagatcctat aactgcggga gcaaaagcag tagaatactc taaaaataaa aacgtgaccg 466980
tgattcatag attcatagga acctatatca acaaagaaac ctttcaacta tctaaaactg 467040
ggaaaaaagc tttacatatg ctctggaagg ccaatgaatg ctgtcaatac acctaaaaaa 467100
atcctttgca ttgttgcaga ctatagagaa atttctcctc taattgaaca acttgatttt 467160
acacagatca acgagcatct ctatagttat cgttgtactg actaccatct agatctctat 467220
attgtccatg tttggggaag tacagccgtt ttaaatgctc ttcaaagcta ttgccaagca 467280
tatacagatt acgatctgtg gatcaatcca ggttttgtgg gggcatgttc tcccgagatt 467340
cctttaggtc aatgttacac tattgagaaa attgcaaacc tcactacgga tacacctcct 467400
gttctctctg aagatccccc ttatatcttt gacgctctac cggattctct acctaaaagc 467460
tctctggtta cctctccagt attgtaccat tatgggtttc aataagacgt ttaaacttct 467520
agatatggaa aggctatgct aatagcctca caagcagcag aacatcacat cccctgttct 467580
tttctcaaga tcacttctga ttatactgtt ccaggagact gtcccttcag cagattggag 467640
gaggtatcac aaaagctaac tcagacactt gtagagttgt tggcctgagc tcatggagag 467700
agcgatccca cctaagttgt tattgccatg tccgtaggat ttgcgactaa tttgtgatca 467760

ggaacaaaga ccctgttata ttctttagtc cctgagagct cccacatttt taaaaattca 467820
ggacacgtct gtaagagttc ttcttttgtg ccttcggcaa ttttttgacc attttctatg 467880
tagagcacgc gatctacatg ttcaagagtg gtcagcttat gggcaataat gatttgtgtg 467940
cactgtcctt taagctctcc aatgatattc ttaatgtaat tttcactaat ggcatctaga 468000
gctgacgttg cctcatctaa aattaagatg gaggcgtttt tcaacagagc acgtgctatt 468060
gccaaacgtt gctgctgtcc tcctgagaga ttcttcccag attcttcgag cacgctatgg 468120
actcctttag ggagctttaa aataaactca tcagcgtagg cacgttttag agcttctaaa 468180
acagcctcct cctccatatc cttaccacag gtaaggttat tccatacagt atcatagaat 468240
aagaaaggat tctgtaatac acaggcgatg tgattcctta aggacccttt gttatattcc 468300
gtaataggaa gagagtcgat aagaatcttt ccttgggaga cttcgtagag cctaggaagt 468360
aatttaacaa gtgttgtttt tccagatcct gtaggtccta caatgcctag agcttcgcct 468420
ttatgtaagg taaagcttag atttttgagg atgtgcttat cttcctgata gccgaaggaa 468480
acattctcga atgtgattgt attagaaagt ccaaggaact cgatttctct ttctttttga 468540
ctatgaagat cggggtgatt caagacttca taaaatctct ccgcagcagc acatcccctc 468600
atgatggagg tatnttcatc cccgaacttc ttaatagggt cgtagattag gtagagcaaa 468660
ccacaaaata cgataagttc ttcgggagga atagcaaatt tataaattcc gataacgacg 468720
acaaaagcaa aaaataaaga agctatggta tgcaggaggg gtcgtggaag caaaccgtaa 468780
gcagcacttt tctcctctaa agcagaaatc ttattgttat gctcacaata ttttgtgaag 468840
gcaaattttt ctgtacgaaa gacttttact gtcataaccc cagcaagaaa atcataaaga 468900
acggaggaaa atgaatcctg actcttttga atacgttttg ctaaattttt gatctttcta 468960
gcgatcacga caatgggaag gataaagata ggaaaggcaa cacaaataag aattgaaaac 469020
ttccatgaaa tcgacagaca gactcccaat gtcaatatga aggtaattgg ggcttgaatg 469080
tagttaatca ttaaagagtt tactgctaag gcaatgcttg cagaatctgt catgacacga 469140
ttacttaaat taccgatatc atgatcatgg aagaaggtca tggggagttg ttgtagggcc 469200
ttaaagtagt cctgacgtaa gtctcggctt acccgtatag caacgacttg cccaaggaaa 469260
cgttggaaaa ataaggtgac tgctttaaaa atagcaacgc agattaagaa gattgccagt 469320
cctcgaaagc ggctcacatc gatgtagtta cggacaaact tagagagctt gctcgtcaga 469380
gaggctgtgc ttttcccatg ttcggcgatg tatgtcgtgg catcagagac tgtaagtgtc 469440
tctgaatcct tactaattgc ctgccaattc tctaaaatat ctttctgact tagttctgaa 469500
acctttacaa gttttccaga ttccttacgt ccaaaaagta aaaaggcgtc ggggcctgtt 469560
ttagcaatca tccctaaaga aaaaatctcc atctgagatg aaaaggtaag tcctaaaatt 469620
gcgagtagag aacagcctaa tataacgaga tgatttttat gcctcaggac cgctttcaga 469680
agtagtttca taaagaccta tagagcgaaa tttttcgtac cgtttctcca acagctcttc 469740
tatagctaga tcttttaatc gtaaccactc ttggatgata aactctcgaa cattgctata 469800
taccaatgca ggatcgtggt gagctccccc aatgggctct ttgataacag tatcgataat 469860
gccaaattgt tttaagtttt ctccatgcat tttcaacatg gaagctgctt cgctattttt 469920
cttaggatct ttccaaagaa tggaggcgca tccttctggg gaaattacag aataatagga 469980
atgctctaac atagctacag aatcacctac agccatgccc aaagctccac ctgaacatcc 470040
ctcaccgata acgacaataa tcacgggagt ggcaagtctt gagagctcaa aaagattttt 470100
ggcaattgcc catccttgtc ctctctcttc agcagtcaat ccaggatatg ctcctggggt 470160
atcgacaaga aagaccacag gcaagccaaa cttttcagcg agttttccta agcgaagggc 470220
ttttctgaaa ccctcgggac ataacatacc gaagttccta tgaaggcgtg acgctgtatc 470280
gcatcccttt tcttggccaa taaggacaaa acgctgaccc tggattttta caaagccacc 470340
aacaactgcg ggatcatctc ggaaggtgcg atctccacaa agctcgacaa actcctcaca 470400
catcccttca atatagttga cagtacgggg acgcgaaggg tggcgacata tttgtacacg 470460
ctcccaagga gtcaaatccg aatagatctt ttcttttaat ttatctaaac gcttttccaa 470520
tttctgaatc tctgaagaag ataagagaga atttttctta tttttttctt taaattcggc 470580
tatagccttt tcatattcaa ctacttgttt ttcgtgtgga agaagttcca ttagaatgtg 470640
ctctcctcgt ataaaaagaa tcattttaat acaaacaatt cttaaacaca attacagacc 470700
caaaattaat tcatcttcgt aaagaatctt cctttctcag aggtcacttt tttttcaaaa 470760
attaacatga cgccctcttc tttgtaagga atattaatca aaaagtctgc attccaaaat 470820
ttttctttgc cttgcaaagc aaaaatttcc atatagggac ttgcagaaac aatgttgatt 470880
gcgtcatttt ccccaagaat cataatgtcg tttccgggac ctttataaga atctagggaa 470940
caggagcgca agcgagggcc gtcaacaacc tgacaattct tccccttaca gaaaatagaa 471000
aacgtcatgg cagaggtggc ctcagccaac gcagcgtgca cgcgatattg cttgtcggaa 471060
atagtgatct tacagcggat cggcagatgt acataggaat ctcgaaggta ggaaaatccc 471120
gtatttctgg gatgaggctt tcctgtagag gtgagaaaag aaatctctgt agtttggtga 471180
ganttttggc acacgaactc tttagcgatt ccacaacatc caaaataata acaatcacta 471240
atgtctccag agcaaggtcc ataagcgata acaccgacat cccctgagga atacgctccc 471300
aagctacttt gacatcctga agcggctaca aaagcggatg aagaacgcgt tctttgcatc 471360
cagaacccta gtgcaggatc ctcaagactg tattctgggt gaatttgcat tcctaattgg 471420
aagagaaacg attggctaag agttgtcttt tcttttagga gtcgagagtt ctcctggctc 471480
cagagagagg gaaagacgct cccttggtga tcaaagagtg cctgttgaaa atgactcatt 471540
ttagagacgt atgcctgact ctcttcataa aactctgcga tctgtaacag gagtaggcca 471600

aattccgcat gttctgcagg ataaggtaat cctttccaag gaaatccccc acaagaaaac 471660
agttctacag cacctgaagg gtactgctgt acatttaaaa agcttaagcc taaatcctcg 471720
ccttcttcta aggccatcaa aagatagacg gcccgagcaa tatcatgaat ttttagtaaa 471780
ggatggtagc ggaaaatctt gagtacagac tgccttaatg tcttatcttg gcacgcatag 471840
ataaactgtt ttacttgtga atcaaagttc gaattctggg acacttctat acaatgttgt 471900
aacctggatt gcagccccat tatcacgcac aatttgatca gaaaagcatt gcaccaaacg 471960
ataataataa caaaaattag agtcaagccc ggtaaaaaac cggtctgact ctaacaactg 472020
agacaaagaa gactacaaga attaagaatg cttattcgga gtttctatca agcgcttcat 472080
tcttttccct ggagtaaact ttacagcgcg tctagcagga atatgaatgg ggactgctgc 472140
attcctagga ttacgtccta cctttggttt tctttctact acttgcaaca caccaaaatc 472200
tctaaactca agcctgtcac ctttaaccaa ggcgtcggtc attttatcta gaaaattctg 472260
aatcacggta cgtacgtgat taggatgaat tttgtgatct tgtgagatcg tgctgattag 472320
tttcttcttt gtcatggtag ccatattaga caatgcctcc tattaaagtg ccctaaagta 472380
gctcgttgag taaccgtaaa taataaactt ttgaagtcta agctcatcat atctattcat 472440
cctttggatt caagacattt tttaaaaaat gcgcaatcac tataaaccat atcgattaat 472500
gcgaataact atatttctag aacctagaaa aatcattccc acatcttgag aaaaacttcc 472560
cctaaattgc tagcgtgcat ctaacacgtg acttctttaa tctaacttgg taaagtgctg 472620
gtctttgcgt cctcgtagct cagcaggata gagcggttgc ctcctaagca gcaggccatg 472680
cgttcgaatc gcatcgagga cgattttttg cctttgactc ctaaagtact aatttgcttg 472740
tatctgtggt ttacgtattt tagcgatatt ctgttttggt ttctgaaaac taggtccaga 472800
aagaaaatta tgaactccct cggcgatgcc tttcgctaca tgcatacgat agcgagcatc 472860
ttgcagggcc gcacgttcac gactattgga taaaaacccg gtttccacca aaactgcagg 472920
catagaagta tctctaatca caacaaagtt cgcagttttc aaacctcgag acttcaaaat 472980
gccatttttt tccatagcag ctaaaatgtt ttttcccagt acttctgaca tgcgattcct 473040
agtcggagat ccgaccttac cattataaaa atatacttcg gtgccaaagg ctgctgcgtt 473100
tgaagaatga ttacagtgga tgctgataaa gacatccccc tgcccacggt tcgacaaagc 473160
aacgcgtttc cctaagtcaa cgtatacatc agaagatcgg gttagctgag gtttataacc 473220
catccgcttt aagtaacttt gaaccgtcaa agcaagagac agggtcaggg acttctcttc 473280
ataatgaagt tccttacttg ccgtgccttg atcttttccc ccgtgtccag gatctataaa 473340
tataacctca ctgcgtcgta cacgctgagg aggattcggt gtttgagcaa aaatcgggtg 473400
acttcctaac acacataaag caaaaaacga cagttgctta gacataggaa gcgagtacct 473460
cacaaacagt ctgcttatca tcgaacgcaa ctgtttggtg tttaaatatt tggtaagctt 473520
catgcccttt tcccgctatt aacactatat ctctatctga ggcaatagac agagcatatg 473580
taattgcttg ttttctgtcg atttcgatga aatagttttt tgaataaaac ccatcacaaa 473640
tttcattcac aatatcttca ggaggctcgc tcctagggtt atctgaagtt acaacagcaa 473700
aaccataacg ctctaccacc tgggccatca acttccgttt actgcgatct ctatctccac 473760
cgcaaccaaa aacaacaatc agtcttcccc cctcaggaag taactcatgc aatcctgtta 473820
agacattgtc taaagcatcg gggtgtgtgg cataatcaat atatacaggg caggacccca 473880
taagtacagg atccaaacga cctggaggag gttgacacaa gcctatcttt tctagcaaat 473940
cttcaagatc gcaacgcaaa cttgcatgta ctgtagagat cgcagcaagt aggttataga 474000
cgttgtactt tccaataaat gaggaagagc acgcaatttt ttggtccccg tacaccaagg 474060
tatactttgt tcccgaggaa gaaagttgga tatcggtggc tcggtagtca gcagcactct 474120
ctataccata agtgatgacc ggtgcctttg cactctcaat acactgagaa gcgtagggag 474180
agtctgtgtt gataacaacc attcccgaag ggggcacgag agagaaaagc ttggctttcg 474240
ccgcaacata ggtttcaaat gtgccatgaa aatcgagatg atctaaggta atattagtca 474300
gaactgctgt atcaaaattg gtataggcta ctcttccaga ggcaagtcct atagaagaga 474360
cttccataac aacagcgtct ctattttgac gtaccatagt ggctaaatac ttctgtaaaa 474420
gagcgggtgt aggtgtagta aacccatctt taatcacccc ctctcctaag atatgctcta 474480
tggttcctaa aagccctgaa ggtttttgat agctatccaa taaagcttta atcaaacatg 474540
taactgtagt tttcccattg gttccagtca ccccaatggt atggagctta cttgaagggt 474600
attcgtaata ctttgcagaa agctcagcct ctaattcttc gagattagga gtgatgatct 474660
gaacaacgga aagaaacgga ttgtatagtg aagaagcaat ggcaattgct ccattagcta 474720
aagcatcgac agcaaaatca tttccgtcgt agcgctgtcc cttatgggct ataaaaatgt 474780
cgccaacact cacacaacgg gaatcacgtg tcaagttgcg cacttcaaga gggcgaactt 474840
tcccgtagat tttagcttga accccatgga gtaactcttt taaatccatt tacgctccag 474900
ggactattga aaaacaactt ttttcataaa aagccctcta ggggcatgct ctcactctga 474960
tttttaaaaa atgcttaaag gatttcttta ctcgtaggtc gacaaaaaat tcaatcaaga 475020
tagtatacaa gcaatcacaa aaaactgtcc ataaagttct tactcttctc tgaaatataa 475080
aatttcgctc tcctgtttct acgaacagaa gcctccttta tcccaaatct aaatccccaa 475140
acacacagcc ttacctcccc cttccataaa cttcaataga ccgtctcatc aagactttgg 475200
tctcttattt aggggaagaa agctaaagtt atttctatag tttaggacct accctagcca 475260
taacataaac tactcataga ggataaagag gagaacgggc tctctgatag agagtctttg 475320
ctcaacggta aaagtctata gcaagatgga aatagagatc ctcacctcgt tcccccttgt 475380
ttcggagaac gattccattc ttcatagaga cgctttaatg cagcagcttc ttcgtcgcaa 475440

tttcttagct tcttgtctgg aagaatccct aaatagagga gtgtgcggtc agcaacccta 475500
gaaaaaatgg gtgccgcaca acgccccccc atataatttt tcgtgccgtc ggctcgcaaa 475560
ccatattcag gatcatctat ggagacgagc atcactaaag gtgggaaatt tccctccgag 475620
ctctctacgg gagtaaaacc tataaaagaa gcaatatgac ggcgtttatc atattttcca 475680
tgaatcatct tttctgtagt tcctgttttc ccagcactag agtgatgctt aggagaggct 475740
cgaaatcccg aacctccggg taacgttgta aaacgcatgg cacgaacaac ttctctagta 475800
atttcttctg aaaagagtcg tgtcttctct ttagtaggaa gatgatattc ctctcctgaa 475860
gcagagacga tcttttttac taaagtgggc cggacggcat aacctccgtt tgcaaggata 475920
gcgtaggctt gaaccatttg tatccctgtt gccaaaatat tatatcccat agccaaagaa 475980
tatggagtag ataaggacca ttccagggaa ccattaatat ggaaacgatg gggagaaggc 476040
accaaaccag aggcctcact gggaagctcg atccctgttt ttcttccaaa tcccagagct 476100
agcaacttct gttggtacca ggccactcct aaagattgta tgatgcggtc agccagctga 476160
gctacataga cattcgaaga tttctggata gccatgtaca tattcaattg agagtttcta 476220
gaaatatcct taagcggaga tccttttcgt ccagggaaga gtgtcctggt cacatcgata 476280
ggttcttcag gatcaaaaat ctttttctgc gattttaagc tagcctcttc gttagcttgt 476340
aaagcaatcg ccacagtcaa aggtttcatg atcgacccgg gttcaaaaac atcgctcaca 476400
aaagatacct tcgtatgttc gatgcgctct ttgttattga agtattcctt ataatttgtg 476460
ggatcgaaaa acggatattg agccagtgca agaatctctc ctgtttggga gttcattaga 476520
atgagcctac ccccctgggc tttagcttct agcacgcccc gttcgagttc ttcctctgca 476580
atggtctgga tcacaggatt gatcgtaagg tagatatcag agccatcttt aggcagtttg 476640
ataacacgat tcgtatctaa acggttcaaa ggagaacgca acagctttct ctctccaacg 476700
tccccttcca gaatatgatt aaagtacgcc tccatcccgc ctgtgggaaa ggcttttcct 476760
gttttctcat ccttaatttc tcttaaggta tggagaactt gtccaaggag cttcccaaaa 476820
ggatacgagc gttggtagtc cgtaataaaa aatagggcgt ttgttggtaa gcgatgcttt 476880
gttgcatatc ctttccacca aagggatagc cggtcatgga cagaaacatc taataaagga 476940
tacagcttac aataccgaga tttcttatct aactttaggg agaggtcgtc gtaggtctgc 477000
ccctcaataa attggagaat cccttggatg atctcatcac gatgacattc gggaatagct 477060
aaaggatctg cacaaaggtg aaattttgta atatcgacag cgaaaggctg ctgaaggtct 477120
ttgtctccct tacgtactgt cgtgttagca aaaaaggtgc cccttcgaaa aggatcacgg 477180
acacaaaatt cgtgttgccc gagagcttct gcggcccagt ggtctccttc acaaatttgc 477240
nttttataat aacgcaatac tagaagagca taaagagcaa acactcctag aacaattaga 477300
gtcgaacgtt tacggtagct cataggataa aagactgata ctttcttctg agggatattc 477360
caaatattga tattcgggaa gagctgctat ttccatcaaa tgatcaggtc tttctatttt 477420
atcaattaaa aaacgtaaag aaatattttg ctgctcaagc tgacgcaagc gtacagataa 477480
acaaggaatt tcgaggcgta atttcgtcag cgagttctgc ttattaatat agaaataaaa 477540
gagacttcca caaaagcata gacagcagca taaacgtaaa aaacgacttt tgttcattgg 477600
gaagcttttt caaaacaccg tagttttgct gatctcgatc taggatttct tcgtacttct 477660
tggtaggtag gttggatcac tttctttgtg attaccttcc ccaggccaga agcttccgcc 477720
tctttaaaaa accacttcac aggacgatcc tcagagctac aaaaagaaat aatgacaagc 477780
cgtccctgag gagccagcca agatatagca gatgttagta aacttttcaa ttgtctatcc 477840
tctccattca cataaacacg tagagcttga aaaatcaagg tgagtggatg tatttttcta 477900
tgaaaacgat agtgagggaa aacgccaaga agagcttctt ttacatcctg gatcgaaaga 477960
atttttttat gcttacgaaa atggacaaca gctttagctg cagatttcca ttgtggttcc 478020
tctccatatt cacgaaaaat tctccctagt tcttcttctt ttagggagtt caggacatcg 478080
ctagcggaaa gctcttgcgt ttgatccata cgcatatcca actcttcttt ttccccttga 478140
aagctaaacc ctcgggatag agtatccagc tgcatagaag agactcctaa atctgcaaga 478200
actccgtcat aaagacgtgg agtgggttgg ttcgcaagat cttcaaaaga ggcgtgggaa 478260
aaggagactc tatcttgaaa ggtctccaaa cgtttttctg caattgccaa agcctgaaga 478320
tctcgatcgg agccatcata acaagttaga gagggatacg cctcaagaaa agcatacgca 478380
tgtcctccag ctcctaaggt gacatctcga aaagtctgtg gaggacgttg agcaaataaa 478440
gctaaacatt cttcaactaa tacgggaata tgcgcacgtt cggacataag aatttcctaa 478500
gctcttggct tcaaataagg acaaagatac aggattccat agaaagaaga aagccttccc 478560
ttatcttaaa ttctcgctag ttcgccactc taaatttttt gataggggtg agcttttttc 478620
ttaagtgcct tcgataagga gaaaaggttc aggcttctga tcaagactat tgactttttc 478680
ctagaccgca ctatcatcta gagatagaga cggtttttta aggcctcgcc atggtagaaa 478740
tttttaatta tagcacgtct atatatgagc aacatgcttc caataatagg atagtcagcg 478800
actttcgcaa agaaatccag atggaaggca tctccattcg tgatgttgcc aagcatgcgc 478860
aaattttgga tatgaacccc aagccttcgg ctttgacgtc tcttttacag acaaatcaaa 478920
agtcgcactg ggcatgtttt tccçctccaa ataattttta caaacagcgt ttttccacac 478980
cctacctggc accttcttta ggatctccag accaacaaga tgaagacata gaaaaaatct 479040
cctcattttt aaaagttctc actcgaggga agtttcccta tcgcagtcaa attactccct 479100
ttttgtctta caaagataaa gaagaagagg aagacgaaga tcctgaagaa gacgatgacg 479160
atcctagagt acaacaaggg aaagtgctct taaaagctct agatcttgga gtcaagtcta 479220
caaatgtgat gatagactat gtgatctctc gtatctttca atttgttcaa ggataatgta 479280

tgttggataa tgaatggaaa gcaatcttag gctggggaga tgatgagtta gaagaactca 479340
gaatctcagg atattctttt ctacgccaag ggcattattc aaaagcgatt cttttttttg 479400
aagctctagt gatcttagat cctttaagta tctatgatca tcaaactctt ggaggtcttt 479460
atctccaaat tggtgaaaat agtcaggcgc ttgctgtttt agatcaggca ctccgcatgc 479520
aaggagatca tctgcctaca ctcttaaata aaacaaaagc tctcttctgt ttgggacgaa 479580
ttgaagaagc tactgccatt gccacctacc tttcatcctg tcccatacca gcaattgcta 479640
atgatgctga agctctattg atgagttata gtaaagcaac caaaaaaaat gctgcgttag 479700
ttcgttaatt ttttctccta tagaaaacgt atttcgtgat tgaagaactc cggatttctt 479760
tagttctttt tccattttta ttacaaaatt tcataattat tttttcctga atttgtggtt 479820
ccttattgcc taacgaaacc aacgatccaa tttgccttaa aacctagtaa tcggtagagt 479880
tattttatcg gcagagcgta ccgcgtttac ctgcggagga agggtgtata ggatccattc 479940
ctaacaggaa cttcacccag ggaatgcagg atccaatctg aacgcgaaga gtcattgcac 480000
tcagtgcaat gaatagtcaa gatgcaatga gattttctcg ccatcactca tgggatgggt 480060
cgattgtatt tgggaaagct ttataaataa agagagcggc atgttaacct gtaacgagtg 480120
cactacttgg gaacagtttt taaattatgt taagacacgt tgctcgaaaa cggcttttga 480180
aaattggatt tctcctattc aagttcttga agaaactcaa gagaaaattc gcttagaagt 480240
ccccaacatt tttgtacaaa attatcttct tgataactac aaaagagacc tctgttcttt 480300
tgtcccctta gatgttcatg gagagcctgc tttagaattt gtagttgcag aacacaagaa 480360
accttcagcc cccgtggctt ctcaaaaaga atcaaacgaa ggaatttctg aggtctttga 480420
agaaactaaa gattttgaat taaagctgaa tctctcctat cgctttgata atttcattga 480480
aggtccctca aatcaatttg tgaagtctgc agctgtaggt attgctggga aacctggccg 480540
ctcctacaac cctttattca tccatggggg tgtgggatta ggcaaaacgc atttacttca 480600
tgccgtaggt cactacgtaa gagaacatca taaaaatcta cgcatccatt gcatcactac 480660
agaagcgttt atcaacgatc ttgtctacca tctcaaatcc aagtctgttg ataaaatgaa 480720
aaatttttat cgttccctag atttacttct tgttgatgat attcaatttt tacagaatcg 480780
ccaaaatttt gaagaagagt tttgcaatac ctttgagact ttgatcaacc tgagtaagca 480840
aattgtaatt accagtgata aacctccaag tcagctcaaa ctttccgagc gtatcattgc 480900
tagaatggaa tggggactgg ttgctcacgt cggcatccct gatttagaaa ctcgggttgc 480960
gattttacag cacaaggcgg agcaaaaagg attgctcatt cctaatgaaa tggcatttta 481020
tattgcggat cacatctatg gcaatgtccg tcaattggaa ggagctatca acaagctgac 481080
tgcctattgt cgtcttttcg gcaagtctct tacagaaact acagtccgag aaactctaaa 481140
agagctcttc cgttctccaa caaaacaaaa aatttctgta gaaacgatct taaaaagtgt 481200
tgctacagta ttccaagtaa agctgaatga tcttaaggga aactcacgct ctaaagatct 481260
tgtgttagct cggcaaattg ctatgtattt agcaaaaact cttattacag attctttagt 481320
tgcaatagga gctgcttttg gtaaaactca ttcgacagta ctttatgcct gtaaaactat 481380
agaacataaa ttacaaaatg acgaaactct taagcgtcaa gtaaatctct gtaaaaatca 481440
tattgttggt taatttaggg ggtgtcccat gttccgtaga acaggaaaag gtccttttga 481500
agatgtgcaa acactttacg aagaagaaac ttcttcacct tccagctact cgccatattc 481560
aagatccgag cgcccagaga cccctccaag tctttttgac aaccctaaag cttcggaagc 481620
tcgccctttg aatcacaatt taactgaaga atcttctctt cctcaatggt cctcaactcc 481680
aagaacagaa tctctactcc ctcttgaaga acctgaaact accttaggag aaggcgtcac 481740
ctttaaagga gaacttgctt ttgaacgtct cctacgtatt gacggaactt ttgaaggcat 481800
tttagtctca aaaggaaaaa ttattatcgg tcctaaagga gtggtaaagg cagatattca 481860
gctacaagaa gccattattg aaggggttgt agaaggaaat atcacagtat ctggaaaagt 481920
cgaactccgt ggaggcgcaa tcattaaagg agacatccaa gcgaacacgt tgtgtgttga 481980
tgagggcgta cgtattcttg gttaccttgc aattgcagga attactgatc attctgagag 482040
agaaagagac ttatagatac tagaggtgat gcactccctc caacgatagt ttgcatccat 482100
ttttgtgata aaggataggc cgacaaaaag cagatacctt ccccttttagg aagtttttct 482160
atttgtcttt tcttaggaag ccacggccac aagggaatgc gtaaaagctt ttctagttta 482220
gcaacttcta ctacgatttt cctagaaatt gtcgatgtta tataggcaat acaactcggg 482280
gtctcatcca gtgaagctag ctcaaaggcg atactcttag aaaaaaactg cagagcgggt 482340
cgcttacctt cacaagcacg agcatatacc gagagggccg tttgcgaagg aaggtacaag 482400
ctgaaagctt gaagttgtga agagggtgaa cactggctac atagacgtgt ttcggaagaa 482460
ccaagataac gaaaacaatg tagacaacgc ccttctctat cttctacgag aagtttttcc 482520
aagcagttgg aacaaaagta ggctcctgga gcttgacagc cataacataa ctttggaaac 482580
agcaacgaaa aaagtaccat ctatttttga atcatgaaaa tttttattcc taaaatttta 482640
agcagtttgc tacgtttttg aacgtgtaga agcttcaact tttctctcaa tcacaatgct 482700
caaaaaattc ataaattctc tttggaaact atgtcaacaa gacaagtatc agcgctttac 482760
tcccattgtc gatgcgatag atacattttg ttacgaacct attgaaaccc cttccaagcc 482820
tcctttcatc cgcgattctg tagatgttaa gcgttggatg atgcttgttg ttatcgcttt 482880
gtttcccgcg acctttgttg cgatctggaa ttcaggactt caatctatcg tttatagctc 482940
aggcaatcct gtgctgatgg agcaattctt acatatttct ggatttggta gttatttatc 483000
ctttgtttac aaagagatcc atatagttcc tatcctttgg gaaggactta agatctttat 483060
tcctctactt acgattagct atgttgtcgg gggtacttgt gaggtcctat ttgctgtagt 483120

tcgtgggcat aaaatcgcag aaggactgct agtaaccgga atcctctatc cccttactct 483180
ccctccgaca attccttact ggatggcagc cttagggatc gcctttggta ttgttgtcag 483240
taaagagctc ttcggaggca cagggatgaa catcctcaat cctgctctat caggaagggc 483300
attcttattt tttacgtttc cagcaaagat gagtggtgac gtttgggtag gaagcaaccc 483360
cggagtgatt aaagatagcc tcatgaagat gaactcctcg acaggaaaag tactcattga 483420
tggattttca cagtctacct gcctacaaac tctaaattcg acacctccct ctgtaaagcg 483480
tctgcatgtc gatgcgattg ctgcaaatat gcttcacatt cctcacgtcc ctactcaaga 483540
tgtcattcac tcacaatttt ctctttggac agagacgcat cctggttggg ttttagataa 483600
tctcactctt acacaacttc aaacgtttgt tacagctcct gttgctgagg gaggattggg 483660
gctgcttccc acacagttcg attctgccta tgctattacc gatgtgatct atgggattgg 483720
gaagttctca gctgggaatc tcttttgggg aaacattata ggttctctgg gggagacctc 483780
cactttcgcc tgtctgttgg gtgcaatatt ccttattgtt acaggcattg cctcttggag 483840
aaccatggca gcctttggga taggagcctt tctcacaggc tggctcttta agtttatcag 483900
cgtactcatc gtgggacaaa acggagcttg ggcacctgct cgattcttca ttcccgccta 483960
tcggcagctt ttcctcggag gacttgcttt tggtttagtc tttatggcta cggatcccgt 484020
atcatcgccg actatgaaat tagggaaatg gatttacgga ttctttatag gatttatgac 484080
tattgtgatt cgtcttatca atcctgcgta tcctgaggga gtgatgttag cgatccttct 484140
gggcaatgta tttgcccctc ttatcgacta ttttgctgtt agaaagtata gaaaaagggg 484200
agtctagaat atgtctaaag gctcttcaaa acataccgtc cgcataaacc aaacctggta 484260
catcgtttcc tttatcctgg gcctcagctt atttgcagga gtgctgttat ccacaatcta 484320
ctatgtgctc tccccaatac aggaacaagc tgctactttc gatcgcaata agcaaatgct 484380
tttagctgct catattttag attttaaagg aagatttcaa attcaggaaa aaaaagagtg 484440
ggtgcctgcg actttcgata aaaaaacaca acttcttgaa gttgctacaa aaaaagtctc 484500
tgaggtttcc tatcctgaat tagagctgta tgccgagcgc tttgtccgtc ctctacttac 484560
agatgcccaa ggcaaggtat tttcttttga agaaaaaaat ctgaatccca ttgaattttt 484620
tgagaaatat caagaaagcc ctccgtgtca gcaatccccc ctcccctttt atgtcatttt 484680
agagaatacc tctcgcacag aaaatatgtc aggagccgac gttgcgaaag acctttctac 484740
agttcaagct ttgatcttcc ctatatcagg attcggcctt tggggcccca tccatggcta 484800
tctaggagtg aaaaacgacg gtgacactgt attgggaacc gcatggtacc aacaaggaga 484860
aactccaggt ttaggagcaa atattacaaa tcccgaatgg caagagcaat tctatgggaa 484920
gaaaatcttc ctacaagatt cttctggaac tacaaatttt gcaacaacag acctagggct 484980
tgaggtagtt aaaggttccg tgcgtactac tttgggagat tctccaaaag ctctttctgc 485040
tattgatggg atttctggag ccaccttaac atgcaacggt gtcactgaag cttatgtaca 485100
atctctggct tgctatcgtc agctccttat aaatttttct aatttaaccc atgaaaagaa 485160
aacaggcgaa tgacaagtaa aaagtcctat aaaagctatt tctttgatcc tctatggagc 485220
aacaaccaaa ttctcattgc gattttgggg atttgctcgg ctctggcagt gacaacaaca 485280
gtacaaacgg caattactat gggaattgct gtcagcattg ttacaggatg ctcgtctttc 485340
tttgtttcct tattacgtaa gttcactcct gacagtgtga gaatgattac tcagctaatt 485400
atcattagct tgtttgtgat tgttatcgac cagtttttaa aagctttttt ctttgatatt 485460
tccaaaacac tttctgtttt tgtgggtctt atcatcacca attgcatnnt gatgggaagg 485520
tctgaaagtc tagctaggca tgtgactcct attccagcgt tcttagatgg gtttgcctct 485580
ggcttaggat acggctgggt cttacttgtc attggagtca tcagagaact ctttggtttt 485640
ggaactcctt atggggtttc gcatcatccc tcaatttgtt atgcttccga aacccacccc 485700
gatggatacc aaaatttaag tcttatggtg ctagcaccgt cggctttttt cctacttggt 485760
attatgattt ggcttgttaa cattcgagac tctaaagaga aaaangtagt ttatgtggtt 485820
aggtgcgtat acttggctta atgtctttgg tattcttcta caagcagcct ttattcagaa 485880
tatccttctt gcgaatttct tggggatgtg tagttacctt gcttgctcta ctagggtttc 485940
tacagccaat ggcttgggga tgtccgtagc ccttgttctc actgtaacag ggagcatcaa 486000
ctggtttgtc catgctttca tcacgggccc taaagctcta acttggatct ctccatcttt 486060
agcttctgta aacctaggtt ttctggagct gattattttc atcgtggtga ttgcggcatt 486120
cacgcaaatc ttagagcttc ttttagaaaa ggtctccagg aatctatatc tctccttagg 486180
gatcttcctt cccttgattg ctgtgaactg cgcgatccta gggggtgtgc tcttcggaat 486240
cacacgtagt tatcctttta ttcctatgat gatcttctct ttaggagcgg gatgtgggtg 486300
gtggctcgct attgttattt tagccactat caaagaaaaa ctcgcctact ctgatattcc 486360
caaaaacctc cagggaatgg ggatctcctt cattacaaca ggcctcattg ctatggcttt 486420
tatgagctta acaggtattg atatttctaa accttcagca aagattcaaa gagctcctct 486480
agagactgaa gttgttgaaa acacgaccaa tccactaaaa gaatcttcgt ccaaacacca 486540
gccaagtatt tctaaagcac gaacgcagcg tcgctctctc taggaacttt cctagagtcg 486600
agtttctagt gaacttttgt acacagagat gcttctaaac aatttcaatt cgagatatgg 486660
aaggcggctt tttgcaagct gtggcgattt tacttgcgat acaataaaca acgatcgtca 486720
gtaaaacaca aatagcaaga gctataccta tatagagagc gcagagtctc agttgagaac 486780
cttcacaaga aaacaaagtc acggcgaaag ctacagctaa cgaagtgagg agaatagcaa 486840
gcactttcgc aaaaatccga taagataaag aattctccga atggagaagg acactagaat 486900
accgttttag gaaggtcaga gtatcgggat tttgtcgaga acggggaacg tacttaggta 486960

atgtagtcat gatttttcaa tcaataaaat gtccaactca gctttcatgc gctaccttag 487020
ttttttctaa ggcattggaa atcttataac gccaacacgc acaaactaac atagataagg 487080
taatgcaact cgcagcaatc cagatcgaga gctgtagagg tgccgatccc gttaaaaata 487140
ggccacaggc taccaaagaa atcgctatca atgaaaggac catgatggct atagtaagct 487200
tcttcacacg agataccccg gaagataatg cggacaattc ttcagaagat agttgcacat 487260
gatcttgtag gacatgaggg gctaccggac tagctggatc catagaataa ttctgatgta 487320
ttttttaaac cttagacata aattttaaat gttttagcct taaaaaaata cctcttatct 487380
tctgaaaaaa atgagtatat ctaataaaaa atttactctt catccatcaa agaaagatta 487440
gaaggatccc agtcctggtc tagtcggact acagccaacc atccctcacc ttctggagct 487500
tcgttaatct tctgaggatt atccactaaa tcaaggctga tatcgataac ctctcctgat 487560
acaggactta acacctctta tagcagattt agaagattcc agaatgacta aaacctcacc 487620
ttctttacat agactcccta ctgaaggtaa atccacatgg agaatggctc ctaaattttt 487680
ctgcattttt tctgttaacc cgaggcgcac caccctctca tggacgggca aaatccaaac 487740
atgataatca gaataccaca tcaccttacg accccctgtt ctataaagcg ttccatatag 487800
gatcctaaaa gttcttcatc tagctgactt ttagctaaaa atagtttatc cacgctagca 487860
ggacgatcat acagtaaatg agagaggcat tgataataac agggcccttc gttaggactc 487920
atgaatacag aagaatagtc tcctaccttc atagagaaaa tcctatcaaa ctcttggggc 487980
agctccttgt ctcctcggga aaaagtcttc aatgagcgat ctaagctcca agagaaagac 488040
ccctcgagat gccttcccaa tctgtggttt tccactacct tccaaagacg tcgttgccat 488100
aggctagcgc cttcttctcc tggataccgt gtacgcaacg cagattctag acgctccatg 488160
tcaacaagat gaccatgaga agtaagtaac tgtgaggcca aatctctctt taaaacctca 488220
cgataaggaa gcacttcttc tttttcaaaa gaactattaa caataatagt ataataggtc 488280
tctgcatcct ggctatataa atctaaaacc tcgttttcaa gcaacacttt ggctaattgc 488340
tgaccgtcgg atattcctgg tagagcacta ttcttccctg cagataagag aacttcttgg 488400
ctctgcttag gaacttgctg tagcgattgc agaattctct cagggcgggc ccttaagatt 488460
tccttgcgtg tgaaaagaga aattttatct cgcagcgcag gcttaagatg ttggaagtct 488520
ttatacgact gacacgtctc aacgtcagga aactgctgga gaatctcctg gaagtgctca 488580
gaattttgtt gccagtgcag cacttcaacc ataggtacag tagctgctaa gtcttgcaaa 488640
gcgactctct tatagtctat agaaaaccgt ctgcctacta accgaggctc tttagctttt 488700
atggtcgcta taggaaggat ctcattagga acatccaaac tatccgattt aggtaaactc 488760
actaacttta aatagacttc gaaagctttt aactcttgtt ttgttttaaa gctatactcc 488820
ttagggagtc taaagaactc tacttgtatg gaatcttttc cttgaacgaa aaatgtagtt 488880
agaggttgga agtcaaaaga aacgcctccc tgcaatagga gaagagctct tttgcacaac 488940
aatatgtctc gatacatatt gaaaaattcg gactcagaga tctctaaaaa ctgaaaatac 489000
gagtttacaa attcttcaaa tcctaaggaa aattccttat tcttactgat cttagtatag 489060
gcatnttcgc cttatcataa aagtcgtcac gagctttctt gttttgaggg cctgggaagt 489120
acttttttct gctcgtcaat aaagcggatc aagagctcaa cagcagcaga aaggtaggca 489180
tccccaaacc agtcttgaat cgtctggtag ccaaataacc gcaagtcttt cccgcgagat 489240
aaggcttcat ctgggggaag agcaaacatt tgctgcggta ctccaacatt tgtcgaagca 489300
cataatgagg gaaccttctc tcttctaaaa agagcttggc tctagcaaga aatccttctt 489360
ttgatatggg gttctcgatt tgttgaaaga ccttcaggat ctctaaaagc tggggagctg 489420
aagatttcca aacttcttca gaggaaataa aaggagcgtc aaaacgacga tacggctggt 489480
aagctttctc cttactaaaa attttctctc ccggatggta cacttttaaa aagagttttt 489540
ctcccaccct tgtcgttaga aaataatcag taagtagccc ctcattgata aaattccaag 489600
ctctagggtt ccctgtaaat ggatacgctt cgtgagcaaa gaacttcttc atagccatga 489660
aatctttctc tacataccgc ttccctgaag cggtagtaaa aacagtccga cgtgaggtgg 489720
actctgcaga accttttcta gagaatcgtc cccaacccac tccaatacca gaaacacaaa 489780
ctacagcaat gacaatgccg ataaattttt tttgatgctt atagaagaac gataacaagg 489840
tccaccctct acccaaacaa taaaagcgta gtgtaaacga tagcagagaa aaaaaccagg 489900
caggatccct gcagagcctg cagaagagat cctgttggta gattcgcacc cagggacgct 489960
aggctggcat ataggtcagc tgcaaatgtc ccaaagtgtg gtgtacggaa tggaaatacc 490020
aagagaaaat gtcgccatga cttacaggaa tcgacaatcc gatatcttta ttgaagactt 490080
tgttagcttt tgcagcgact tctggagatt cgataactac aatactttcg taatcaaagg 490140
catcactttt ctttccaaaa ttataacttc cgatcacaaa aatttcatca tcgataatca 490200
tacacttctt gtgcaactgc gtttcccaaa tagcaaactc ataaatagaa acccgctcat 490260
aaggttttag cttttcgcaa aaccattttt tccaaagagg ataccgtttc ccatagagca 490320
aggcgaaata gttaatacgg tttccccaag catagggtcc tgtaattgca ggacttaatt 490380
catgacagcc gttcgtaatt aaactcagat gaacaccgtg attatgagaa acgtcgacaa 490440
gagcatttaa aagctcgtcc ttagggatga aatacatgtg agcaagcttc acagaagatc 490500
tagctccctg gataagtttc aaatattctt gagtccagga ttgggttgct tatcgtgggg 490560
accacctaaa actatcctga tcttggaaga gtcgacaaga acaagatctt catgtttgtc 490620
aaatccagga aatactgtct cctcggcttg ttctaaagtc agtggaggac aggcgcctgc 490680
aaactgttca ggattatcaa tgaaccacat atgatgtgca tagtagtccc acatagcaaa 490740
ttgcttatga tattcttctc tgagctgcaa accgaatgct gtagaacgca acatgatatc 490800

ctgatcacga aatgctaggg gccgacgcac tccactgaca aataaacgtg ggttatccac 490860
tttctcagga acctcatccc ctggagtgca cataaactct tcaaaattgg taccacctaa 490920
aatacaatat ttcccatcga tgatagaaag tttgatatgc atttcaatga cattaggagc 490980
gaggatgctt gttgagggtg ggcaccctgt aaaaacgtag aaaaaccggt tgggatgacg 491040
ttctttgaga gctttgagta atttttggtc ttcagcatcg gtaaacgtgg gttggataat 491100
gatattagct acagagctct ggaaccagat ccatacgagc ctcgaggtga tcttaccatc 491160
tctttaagcg ttcggcctcc tgtcatgcag ggacacagtt ctacataaaa atttgcatga 491220
tctatgcaat ccaatatctg ttgaaaggcc tctacactat tgtcataaac aagaactcca 491280
accttctcct tgtctgaagc tacgattgtc tttgctgaaa cagaattagg aaccagcaaa 491340
ataaaaaata ttccaagagc tgccaagcga aaacgcaacc gactcatcat aacccctcac 491400
cacaaaacaa tttaataaac gcctctttga cttgctgacg cgcctcctgc agcacttcat 491460
cagcagcttc caaacctaaa gggaaaaacg cgtgaatctg gatttgttca ataatttctt 491520
caagaacctc gggttttaaa aatctctggt aaaactcaga agaacttccc gataagaata 491580
aggatccttg atgtaaaaat ccctgttgca ccttgcgttg ggcagcgccc ctatcttctt 491640
gtccccaann aagaacgtca tacttcgaag tttttgccat acaaaaattt cctgaatctc 491700
tggaagaaga gttttcgtct tctggagcta acattccctg gatccgaaat actttctcta 491760
gaaccttcgc tacaaaagag tttacagtat ggtagttctc aagtaccgaa gaagaatagg 491820
aaggatgtgt cgcagacata agaacagaaa aagcataatc tcccttatgg aagacaaatc 491880
cccctcccgt aggccgcact gcggcgtcca atcctagatc cgcatagttg gaaagtaaaa 491940
atttttctgg acgcataaag tgaccgtacg tcagagaaca aggattctcc cactcataaa 492000
ggtgtaaaat gagctcccca tcttgcagag attctaataa atctctgtcc ttagccatgt 492060
gggaggccgc tgaagatttt cctgaatcta cgatacgaac tttcatataa caaacataaa 492120
actaattaag actgtttcaa aagaagctga actattgtat catatacaaa aggtttgtgc 492180
ataactttcc cttaaactca gaggaatttt accaaatttg ctggtttaga gcgaagagtt 492240
gcatcattat tttaaatttc gtatatgctt aaggaaagtt ctacccctgt cttttaggtt 492300
tttatgtttg agaagttcac taatagagca aaacaagtca ttaaactggc gaaaaaggag 492360
gctcagcgtt taaatcataa ctacctgggt actgagcaca tcctgcttgg tcttctcaaa 492420
cttggtcaag gggtagctgt taatgtatta cgcaacctcg gtatagattt tgatacggca 492480
cggcaagagg tggaacgcct gattggttat ggtccagaaa ttcaagtcta cggagatgct 492540
gcccttacag gaagagtaaa aaaatctttt gaatcagcaa atgaagaggc cagcctttta 492600
gagcacaatt atgtcgggac ggagcattta ctcttaggga tcctacatca atcagatagt 492660
gtcgctcttc aggtattaga aaacttacat atcgatccaa gagaggttcg taaggaaatt 492720
cttaaagaat tagagacctt caatctacaa cttcctcctt cgtcgtcgtc ttcttcctca 492780
tcctctcgaa gcaacccttc atcttcaaaa tctcctttag gtcaaagctt aggttctgac 492840
aaaaacgaaa agctttctgc tctgaaagca tatggttatg atttaacgga gatggtccga 492900
gagtctaagc tcgatcctgt cattggtcgt tcttcagaag tcgaacggtt gattttgatt 492960
ctttgccgaa gaagaaaaaa caatcctgta cttattggag aagctggagt tggtaagact 493020
gcaattgttg agggtctggc tcaaaaaatc attctgaatg aggttcctga tgccttacgg 493080
aaaaagcgac tgattactct agatctagca ttaatgattg ctggaacaaa atatcgaggg 493140
caatttgagg aacggatcaa agctgtcatg gatgaagttc gcaagcatgg aaacatcttg 493200
ctcttcattg acgagctcca cacgattgta ggagcaggag cagctgaagg tgctatcgat 493260
gcttcaaaca ttttaaaacc tgcgttagcg cgaggtgaaa ttcagtgtat tggagcaact 493320
acgatagatg agtatcgcaa gcacatagaa aaagacgcag ctttagaacg tcgtttccaa 493380
aaaatcgtgg ttcaccctcc tagtgtagat gagactattg agattttacg tggcctcaag 493440
aaaaagtatg aagaacatca caatgtcttc attactgaag aagctttaaa agcagctgcg 493500
actctttctg atcaatatgt tcatggacgt ttcctccctg ataaagcaat agatctttta 493560
gatgaagctg gggctcgtgt ccgtgtgaat acaatgggtc agcctacaga tttaatgaag 493620
ctagaggctg aaatcgaaaa tacaaaattg gccaaagagc aggccattgg aactcaagaa 493680
tacgaaaaag ctgcaggttt acgtgatgaa gagaaaaaac ttcgcgaacg tctgcaaagt 493740
atgaaacagg aatgggaaaa tcataaagaa gagcaccaag ttcctgtaga tgaagaagca 493800
gtcgctcagg tagtttctct acaaacagga attccctcag caaggctcac agaagctgaa 493860
agtgagaagc ttctgaagtt agaagacacg ttaagaagaa aagtcattgg tcaaaatgat 493920
gccgttacca gcatttgccg tgccatccga cgttctcgaa cagggatcaa agatcctaac 493980
cgacctacgg gctccttcct attccttggg cctaccggtg tagggaaaag cctgctcgcc 494040
caacaaattg ctatagagat gttcggtggt gaagacgctc tgattcaggt agacatgtca 494100
gagtacatgg agaaatttgc tgctaccaag atgatgggat cacctccagg atatgtaggt 494160
catgaagaag ggggccacct tacggaacag gtacgtcgcc gtccttactg cgttgttctc 494220
tttgatgaga tagaaaaggc acacccagac attatggacc tgatgttgca aattttagag 494280
caaggacgtc ttactgattc ttttggtcgc aaagtggatt tccgtcatgc cattattatc 494340
atgacctcca atttgggagc tgatctcatt cgtaaaagcg gagaaattgg ttttggcttg 494400
aagtcccata tggactataa ggtcatccaa gagaaaatcg aacatgctat gaagaaacac 494460
ttaaagcctg agttcattaa ccgtttggat gaaagtgtga ttttccgtcc cctcgagaaa 494520
gaatctctat cggagatcat ccatttagag atcaacaaac tggactcgag actgaaaaac 494580
taccaaatgg ctttgaacat cccagactct gtgatttcct tcctagtaac gaaggggcat 494640

tctccagaaa tgggagcacg tcctctacgc cgtgtcattg agcagtacct tgaagatcct 494700
ctagcggagc tcttgcttaa agagtcctgc cgtcaagaag ctcgcaagct acgagcaacc 494760
ttggttgaaa atcgcgttgc ctttgaaagg gaagaagagg agcaggaagc tgctctccct 494820
agccctcact tggaatcata ggaacgtcga taactccact accaaggcag gtatctcctt 494880
gataaaacgc tattgtttgt cctggagtta ccgccttgac gggttgtgaa aatcgcacct 494940
tgacctcgtc acctgagcta taatctatcg tgcaagcttc atcaggagaa cggtagcgga 495000
ctttagcgct acagtgacat ccggatttag gaggggtaaa ccaattgagc tctctagctg 495060
ttaattcccg taggtagagc tggggatggt cttcccccct cacaatataa atgctatttt 495120
cctctatatt ttttcccaca acataacnng gtttctcgga tcctccaaga tcaagtcctc 495180
gccgctgccc tatagtataa tagtgacttc cctgatgttg ccctacaatt tccttggtat 495240
cccaatcgat aacgttgcct gttttattgg gaagaaactt ctctaggaac tctttaaaag 495300
ggcgcttccc tataaagcaa atgcctgtac tatctttttt ttctgctgtg ggaagagctg 495360
cttgagctgc aatcgcacga acttcagtct tattcatttc cccaagagga aagagcacat 495420
tgtgaagagc acttttagga gttcctgata aaaaatagct ctgatctttt tgaggatcgc 495480
aacctctaag gagttgggtt tcttggagct cggtatttaa tcggcagtag tgccctgtag 495540
cgaggtaatc tccgccaagt tcctggactt tcttttgtag aaggtcaaat ttgatttctc 495600
ggttacaaag aatgtcgggg ttaggagtgt agcctaaaga gtattccttg aggaaacgag 495660
cgaacactct ttctctatat tctttagcaa aagatacggt gtaataaggg atatcgagct 495720
gaagacatac cctctcgaca tcttcataat ctttagtaga cgagcaaagg ccgccttcgc 495780
tatcctcttc ccaattcttc atgaagaggc caataacctt ataattggta aatttttgaa 495840
ataaataggc aacgacagaa gaatccacgc ctcctgacat tgctacaatt acagtttgtt 495900
gcataatttc tcttacttgt ctcctcatag gaagggtaag gttttaaaaa tttgaataac 495960
taagaggcat gactcgctta ctaattgact aaaaatacca tttttcttaa attatttcct 496020
caaataatag agttctcttt gtgtctagta ctttaaacgg ggtatttccc tcatcccttc 496080
cggaagagtc tgctgattta ttcattacga ataaggagat cgtagctttg ggggagaagg 496140
gcaatgtttt tctcacccac tccattccta tgcatattgc tgcgattacg atcttagtga 496200
ttgtagctct tgctggaatc gctattatct gtttgggttg ctatagccaa agcattctgt 496260
tgattgccgt tggcattgtt cttactattt tgactcttct ctgcctacaa gccttggtag 496320
gatttattaa attcatccgg cagctccctc agcagctcca tacgacagta caatttatca 496380
gggagaagat tcgacctgaa tcctctctac agcttgtaac caatgcacag agaaaaacca 496440
ctcaagatac gctaaagtta tacgaagaac tctgcgacct ctcacaaaaa gagttcaaac 496500
tgcaatcaac tctttatcaa aaacgttttg agctttctca caagaatgaa aagacaaatc 496560
aaaactagtt agcaacgatt cgaggaaaca acatggcaac ttccgtagcc ccatcaccag 496620
tccccgagag cagccctctc tctcatgcta cagaagttct caatcttcct aatgcttata 496680
ttacgcagcc tcatccgatt ccagcggctc cttgggagac ctttcgctcc aaactttcca 496740
caaagcatac gctctgtttt gccttaacac tactgttaac cttaggggga acgatctcag 496800
caggttacgc aggatatact ggaaactgga tcatctgtgg catcggcttg ggaattatcg 496860
tactcacact gattcttgct cttcttctag caatccctct taaaaataag cagacaggaa 496920
caaaactgat tgatgagata tctcaagaca tttcctctat aggatcagga tttgttcaga 496980
gatacgggtt gatgttctct acaattaaaa gcgtgcatct tccagagctg acaacacaaa 497040
atcaagaaaa aacaagaatt ttaaatgaaa ttgaagcgaa aaaggaatcg atccaaaatc 497100
ttgagcttaa aattactgag tgccaaaaca agttagcaca gaaacagccg aaacggaaat 497160
catctcagaa atcatttatg cgtagtatta agcacctctc caagaaccct gtaattttgt 497220
tcgattgctg attagaaaaa ttctagtcct ttcatccccc taaatctagg gaaatcttct 497280
tgaaagtccc tcctgagtag gaaaattatt agatagtaat ttaaaaatta cgtatattcc 497340
atttcaatct ctagtaacgt tggagaatct tacagaacat gttcaaactg ctcttccaca 497400
tcgctgcatt tgcgggacac gtactctcga ctcctatttt tattgttcaa gatgcttgtg 497460
gaattgatga agaagcatgt aaaaatcctc ctccacgtcc tttctctgct caggtacaat 497520
acctaaaggt gaacgatgct aaatttaaaa agctgcctca tcaaactata ggctatcgtc 497580
aatacgatgg aacgtttctc tgcacacttc cgattacaga gcattctggg ctactgtttt 497640
ctactggcta tataggtgcg gatattcaat ggaaaagctc acttcctatt tcggagacag 497700
atcctaatgg acttgggtgg gcgactttcc aagatacttc tttttataac tatgttctcc 497760
tctctttagg agcttataca ctctccntta aaaaattggc agtggtctat cattctttct 497820
gggcttgtgg atcctaaaaa tattgagatg ggttatggac tctatcaagg agttctttct 497880
ggaaaatacc aggccactga gaagctttct gctatttttg gcgtcattaa tgaaacaggc 497940
ctccatcaag agaaggcttg gcctttagta ggtgttagtt acaaggctac cgaccaacta 498000
actctcaatt gcatctatcc tgtgaatttt tctattgatt accgctcgac atctgtctgt 498060
aacttagggc ttgcttaccg ccttacaaga ttccgaaaaa aactttacaa aaatcaccta 498120
atttcttctc gcggcatctt tgaatatcaa ggacgtgaaa tcgaagctaa cgtgaagctc 498180
accccttggc cgggaagttt tattaaggga ttttacggtt ggtctattgg gaatgatatc 498240
tcgatagctg atgatcacaa caataataaa acgtcccata cttttaaaac ctccgcattt 498300
ttcggtggtt ccgctgtaat gaacttctaa tttccagttt ccagaaagat ttttttacag 498360
gcccatacaa aaacttcagg agattttgtc tacttgtgtc tctcattttc ctgccttttc 498420
aggaatttcg attgtctaaa aaccactatc ctcttagaat ttttacataa tttttcataa 498480

aatattctct ttcaaacgga tatgggattc aaaaatatct gcaaacaagg ctctcagcta 498540
tacctgaatg gcatttttcc ggaacgaata ctagctcgaa aattaaaaaa ctgtgcgaag 498600
agctatccca gaactgctct taccatagaa gtactggtat cctcggtctt aggagctctt 498660
aaggttatcc tgatcccttg cgcttctaca tatgctgcct tgaccctacc cctacgggct 498720
ctctttaacg ctataaaaac aaaaagctgc caacatcttg cttcgtatgc tatggcttgg 498780
ctcctccaca ttcttacgat tgctgtgatt atcggtctgg tctttagtct ggtctttatc 498840
ccccctccag ttgtctttat ctccttgggg cttctcatgt ctgtaactac tagcgttacc 498900
ctcttccaag tgcataaaaa tcttttcccc ccgtatgagc ctccaccctc acgacctcac 498960
acgccccctc catttgctga tgagtatgtc cctctcataa gcgagtctta tttcgactaa 499020
aagttccaaa taaaaataac ttaaagtttt agttaaaaat ctgttaagat tttaacaaat 499080
aaactttatt taataaaaaa gttaagactc agaagaaaag agaactaact aaatcctatt 499140
aaatcttata atagtatgaa atatatccta tgaatgaaaa ttcatgggcc taatgataat 499200
tacagtaagt tgatagtatg agccaacccc ctataaaccc tttaggtcaa cctcaagttc 499260
ctgcagcagc atccccatca gggcagccaa gcgtggtaaa acgtttaaaa acgtcatcca 499320
cagggttatt caaaagattt attactgttc ctgataaata tcctaaaatg cgctatgtct 499380
atgacacagg cattattgcc cttgcggcaa ttgcgatcct ttcgattctc ctgactgctt 499440
caggaaacag ccttatgctt tatgctctcg ctccggcact tgccctggga gctttgggag 499500
ttactctact tatttctgat attctggaca gtccgaagcc aagaaaatcg gtgaggcaat 499560
cactgctatc gtcgttccta tcattgtatt agcgattgct gcgggtctta ttgcaggggc 499620
tttcgttgcc tctagtggga cgatgttagt ctttgccaac cctatgtttg tcatgggatt 499680
gattacggtg gggctatact tcatgtcctt gaataagctc accttagatt atttccgtag 499740
ggaacacctc ttgaggatgg aaaagaaaac ccaagagacc gcggacctat tctagtgact 499800
ccatccgccg acgatgcaaa aaaaatcgca gtggaaaaga aaaaagatct ttctgcatct 499860
gcccgcatgg aggaacacga agcttcacaa cgccaagatg cccgtcatcg taggatcggt 499920
cgggaggctc aaggatcttt cttctattcg tcacgaaatc ctgagcatag acgctccttc 499980
ggcagcctct cacgttttaa aacaaaaccc tcagatgcgg cttctacacg acccgcatct 500040
ataagtcctc catttaagga cgattttcag ccttatcact tcaaagattt aagaagcagt 500100
tcattcggta gtggagcgag cagtgcgttt acacccataa tgcctgcaag ttcccgctct 500160
cctaatttct ccacggggac ggttctacac cctgagccgg tctaccctaa gggaggaaaa 500220
gaaccctcaa ttcctcgagt ttcttcatct tcccgccgtt cccctcgtga tcgccaagat 500280
aaacagcagc aacagcaaaa tcaagatgaa gaacagaaac agcaatctaa gaagaaaagc 500340
gggaaatcga atcaatctct taaaactccg cctccagacg gaaaaagcac ggctaacctc 500400
agcccctcca atccattctc tgacggttat gacgaaagag aaaaacggaa acacagaaag 500460
aacaaataag gatccgtggt ttagataacc atcttcctta ctctctcctt ctaaaattca 500520
agaaggtcta aaagcaaaac cgcttgtaca ccttctcccc tacccttaag catttccctt 500580
atgttttaag ggactcttaa atatagaaaa ataccttaaa tgcttctctc ccaagattga 500640
aacttctata actgagagtc ttctccagag catttacttg atttatttaa ctgtattctc 500700
tattggtgca ccatgctcct aaagccacat gctatgggag tatttttgat aaaaagcttt 500760
tccccaaaga cacatgaaat attctttacc ttggctactt acctcttcgg ctttagtttt 500820
ctccctacat ccactaatgg ctgctaacac ggatctctca tcatccgata actatgaaaa 500880
tggtagtagt ggtagcgcag cattcactgc caaggaaact tcggatgctt caggaactac 500940
ctacactctc actagcgatg tttctattac gaatgtatct gcaattactc ctgcagataa 501000
aagctgtttt acaaacacag gaggagcatt gagttttgtt ggagctgatc actcattggt 501060
tctgcaaacc atagcgctta cgcatgatgg tgctgcaatt aacaatacca acacagctct 501120
ttctttctca ggattctcgt cactcttaat cgactcagct ccagcaacag gaacttcggg 501180
cggcaagggt gctatttgtg tgacaaatac agagggaggt actgcgactt ttactgacaa 501240
tgccagtgtc accctccaaa aaaatacttc agaaaaagat ggagctgcag tttctgccta 501300
cagcatcgat cttgctaaga ctacgacagc agctctctta gatcaaaata ctagcacaaa 501360
aaatggcggg gccctctgta gtacagcaaa cactacagtc caaggaaact caggaacggt 501420
gaccttctcc tcaaatactg ctacagataa aggtgggggg atctactcaa aagaaaagga 501480
tagcacgcta gatgccaata caggagtcgt taccttcaaa tctaatactg caaagacggg 501540
gggtgcttgg agctctgatg acaatcttgc tcttaccggc aacactcaag tactttttca 501600
ggaaaataaa acaaccggct cagcagcaca ggcaaataac ccggaaggtt gtggtggggc 501660
aatctgttgt tatcttgcta cagcaacaga caaaactgga ttagccattt ctcagaatca 501720
agaaatgagc ttcactagta atacaacaac tgcgaatggt ggagcgatct acgctactaa 501780
atgtactctg gatggaaaca caactcttac cttcgatcag aatactgcga cagcaggatg 501840
tggcggagct atctatacag aaactgaaga tttttctctt aagggaagta cgggaaccgt 501900
gaccttcagc acaaatacag caaagacagg cggcgcctta tattctaaag aaaacagctc 501960
gctgactgga aataccaacc tgctcttttc agggaacaaa gctacggccc cgagtaattc 502020
ttcagcaaat caagagggtt gcggtggggc aatcctatcg tttcttgagt cagcatctgt 502080
aagtactaaa aaaggactct ggattgaaga taacgaaaac gtgagtctct ctggtaatac 502140
tgcaacagta agtggcggtg cgatctatgc gaccaagtgt gctctgcatg gaaacacgac 502200
tcttaccttt gatggcaata ctgccgaaac tgcaggagga gcgatctata cagaaaccga 502260
agattttact cttacgggaa gtacgggaac cgtgaccttc agcacaaata cagcaaagac 502320

agcaggggct ctacatacta aaggaaatac ttcctttacc aaaaataagg ctcttgtatt 502380
ttctggaaat tcagcaacag caacagcaac aacaactaca gatcaagaag gttgtggtgg 502440
agcgatcctc tgtaatatct cagagtctga catagctaca aaaagcttaa ctcttactga 502500
aaatgagagt ttaagtttca ttaacaatac ggcaaaaaga agtggtggtg gtatttatgc 502560
tcctaagtgt gtaatctcag gcagtgaatc cataaacttt gatggcaata ctgctgaaac 502620
ttcgggagga gcgatttatt cgaaaaacct ttcgattaca gctaacggtc ctgtctcctt 502680
taccaataat tctggaggca agggaggcgc catttatata gccgatagcg gagaactttc 502740
cttagaggct attgatgggg atattacttt ctcagggaac cgagcgactg agggaacttc 502800
aactcccaac tcgatccatt taggtgcagg ggctaagatc actaagcttg cagcagctcc 502860
tggtcatacg atttattttt atgatcctat tacgatggaa gctcctgcat ctggaggaac 502920
aatagaggag ttagtcatca atcctgttgt caaagctatt gttcctcctc cccaaccaaa 502980
aaatggtcct atagcttcag tgcctgtagt ccctgtagca cctgcaaacc caaacacggg 503040
aactatagta ttttcttctg gaaaactccc cagtcaagat gcctcgattc ctgcaaatac 503100
taccaccata ctgaaccaga agatcaactt agcaggagga aatgtcgttt taaaagaagg 503160
agccacccta caagtatatt ccttcacaca gcagcctgat tctacagtat tcatggatgc 503220
aggaacgacc ttagagacca cgacaactaa caatacagat ggcagcatcg atctaaagaa 503280
tctctctgta aatctggatg ctttagatgg caagcgtatg ataacgattg ccgtaaacag 503340
cacaagtggg ggattaaaaa tctcagggga tctgaaattc cataacaatg aaggaagttt 503400
ctatgacaat cctgggttga aagcaaactt aaatcttcct ttcttagatc tttcttctac 503460
ttcaggaact gtaaatttag acgacttcaa tccgattcct tctagcatgg ctgctccgga 503520
ttatgggtat caagggagtt ggactctggt tcctaaagta ggagctggag ggaaagtgac 503580
tttggtcgcg gaatggcaag cgttaggata cactcctaaa ccagagcttc gtgcgacttt 503640
agttcctaat agcctttgga atgcttatgt aaacatccat tctatacagc aggagatcgc 503700
cactgcgatg tcggacgctc cctcacatcc agggatttgg attggaggta ttggcaacgc 503760
cttccatcaa gacaagcaaa aggaaaatgc aggattccgt ttgatttcca gaggttatat 503820
tgttggtggc agcatgacca cccctcaaga atatacettt gctgttgcat tcagccaact 503880
ctttggcaaa tctaaggatt acgtagtctc ggatattaaa tctcaagtct atgcaggatc 503940
tctctgtgct cagagctctt atgtcattcc cctgcatagc tcattacgtc gccacgtcct 504000
ctctaaggtc cttccagagc tcccaggaga aactcccctt gttctccatg gtcaagtttc 504060
ctatggaaga aaccaccata atatgacgac aaagcttgcg aacaacacac aagggaaatc 504120
agactgggac agccatagtt cgctgttgaa gtcggtggtt ctcttcctgt agatctaaac 504180
tacagatacc ttaccagcta ctctccctat gtgaaactcc aagttgtgag tgtaaatcaa 504240
aaaggattcc aagaggttgc tgctgatcca cgtatctttg acgctagcca tctggtcaac 504300
gtgtctatcc ctatgggact caccttcaaa cacgaatcag caaagccccc cagtgctttg 504360
cttcttactt taggttacgc tgtagatgct taccgggatc accctcactg cctgacctcc 504420
ttaacaaatg gcacctcgtg gtctacgttt gctacaaact tatcacgaca agctttcttt 504480
gctgaggctt ctggacatct gaagttactt catggtcttg actgcttcgc ttctggaagt 504540
tgtgaactgc gcagctcctc aagaagctat aatgcaaact gtggaactcg ttattctttc 504600
taagattctc cgagaatctt agaaaaacat actttttata aagatgaata cgttattgag 504660
atcgcactgt agggtatcag agggggaggg catcccctct tcatcaaaga gattcttagg 504720
atccgtatga agagaagtaa aagatccgcc atccttgggg ttctgattct ccgcatcaat 504780
caattccttg cgtttccctt gatttctttt tttctttaca gtatttgcta atttaatttc 504840
cttgtttcaa aaaagtgctt acaaatgaag tcctctgtct cttggttgtt cttttcttca 504900
atcccgctct tttcatcgct ctctatagtc gcggcagagg tgaccttaga tagcagcaat 504960
aatagctatg atggatctaa cggaactacc ttcacggtct tttccactac ggacgctgct 505020
gcaggaacta cctattcctt actttccgac gtatcctttc aaaatgcagg ggctttagga 505080
attcccttag cctcaggatg cttcctagaa gcgggcggcg atcttacttt ccaaggaaat 505140
caacatgcac tgaagtttgc atttatcaat gcgggctcta gcgctggaac tgtagccagt 505200
acctcagcag cagataagaa tcttctcttt aatgattttt ctagactctc tattatctct 505260
tgtccctctc ttcttctctc tcctactgga caatgtgctt taaaatctgt ggggaatcta 505320
tctctaactg gcaattccca aattatattt actcagaact tctcgtcaga taacggcggt 505380
gttatcaata cgaaaaactt cttattatca gggacatctc agtttgcgag cttttcgaga 505440
aaccaagcct tcacagggaa gcaaggcggt gtagtttacg ctacaggaac tataactatc 505500
gagaacagcc ctgggatagt ttccttctct caaaacctag cgaaaggatc tggcggtgct 505560
ctgtacagca ctgacaactg ttcgattaca gataactttc aagtgatctt tgacggcaat 505620
agtgcttggg aagccgctca agctcagggc ggggctattt gttgcactac gacagataaa 505680
acagtgactc ttactggaaa caaaaacctc tctttcacaa ataatacagc attgacatat 505740
ggcggagcca tctctggact caaggtcagt atttccgctg gaggtcctac tctatttcaa 505800
agtaatatct caggaagtag cgccggtcag ggaggaggag gagcgatcaa tatagcatct 505860
gctggggaac tcgctctctc tgctacttct ggagatatta ccttcaataa caaccaagtc 505920
accaacggaa gcacaagtac aagaaacgca ataaatatca ttgataccgc taaagtcaca 505980
tcgatacgag ctgctacggg gcaatctatc tatttctatg atcccatcac aaatccagga 506040
accgcagctt ctaccgacac attgaactta aacttagcag atgcgaacag tgagatcgag 506100
tatgggggtg cgattgtctt ttctggagaa aagctttccc ctacagaaaa agcaatcgct 506160

gcaaacgtca cctctactat ccgacaacct gcagtattag cgcggggaga tcttgtactt 506220
cgtgatggag tcaccgtaac tttcaaggat ctgactcaaa gtccaggatc ccgcatctta 506280
atggatgggg ggactacact tagtgctaaa gaggcaaatc tttcgcttaa tggcttagca 506340
gtaaatctct cctctttaga tggaaccaac aaggcagctt taaaaacaga agctgcagat 506400
aaaaatatca gcctatcggg aacgattgcg cttattgaca cggaagggtc attctatgag 506460
aatcataact taaaaagtgc tagtacctat cctcttcttg aacttaccac cgcaggagcc 506520
aacggaacga ttactctggg agctctttct accctgactc ttcaagaacc tgaaacccac 506580
tacgggtatc aaggaaactg gcagttgtct tgggcaaatg caacatcctc aaaaatagga 506640
agcatcaact ggacccgtac aggatacatt cctagtcctg agagaaaaag taatctccct 506700
ctaaatagct tatggggaaa ctttatagat atacgctcga tcaatcagct tatagaaacc 506760
aagtccagtg gggagccttt tgagcgtgag tatggctttc aggaattgcg aatttcttct 506820
atagagattc tatgcccacc cgccatggtt tccgccatat cagcgggggt tatgcactag 506880
ggatcacagc aacaactcct gccgaggatc agcttacttt tgccttctgc cagctctttg 506940
ctagagatcg caatcatatt acaggtaaga accacggaga tacttacggt gcctctttgt 507000
atttccacca tacagaaggg ctcttcgaca tcgccaattt cctctgggga aaagcaaccc 507060
gagctccctg ggtgctctct gagatctccc agatcattcc tttatcgttc gatgctaaat 507120
tcagttatct ccatacagac aaccacatga agacatatta taccgataac tctatcatca 507180
agggttcttg gagaaacgat gccttctgtg cagatcttgg agctagcctg ccttttgtta 507240
tttccgttcc gtatcttctg aaagaagtcg aaccttttgt caaagtacag tatatctatg 507300
cgcatcagca agacttctac gagcgttatg ctgaaggacg cgctttcaat aaaagcgagc 507360
ttatcaacgt agagattcct ataggcgtca ccttcgaaag agactcaaaa tcagaaaagg 507420
gaacttacga tcttactctt atgtatatac tcgatgctta ccgacgcaat cctaaatgtc 507480
aaacttccct aatagctagc gatgctaact ggatggccta tggtaccaac ctcgcacgac 507540
aaggtttttc tgttcgtgct gcgaaccatt tccaagtgaa cccccacatg gaaatcttcg 507600
gtcaattcgc ttttgaagta cgaagttctt cacgaaatta taatacaaac ctaggctcta 507660
agttttgttt ctagattatc gaaaacgtgt taattaattg aacccaagca tctttctatg 507720
aaaataccct tgcacaaact cctgatctct tcgactcttg tcactcccat tctattgagc 507780
attgcaactt acggagcaga tgcttcttta tcccctacag atagctttga tggagcgggc 507840
ggctctacat ttactccaaa atctacagca gatgccaatg gaacgaacta tgtcttatca 507900
ggaaatgtct atataaacga tgctgggaaa ggcacagcat taacaggctg ctgctttaca 507960
gaaactacgg gtgatctgac atttactgga aaggatactc cattttcatt caacacggta 508020
gatgcgggtt cgaatgcagg agctgcggca agcacaactg ctgataaagc cctaacattc 508080
acaggatttt ctaacctttc cttcattgca gctcctggaa ctacagttgc ttcaggaaaa 508140
agtactttaa gttctgcagg agccttaaat cttaccgata atggaacgat tctctttagc 508200
caaaacgtct ccaatgaagc taataacaat ggcggagcga tcaccgcaaa aactctttct 508260
atttctggga atacctcttc tataaccttc actagtaata gcgcaaaaaa attaggtgga 508320
gcgatctata gctctgcggc tgcaagtatt tcaggaaaca ccggccagtt agtctttatg 508380
aataataaag gagaaactgg gggtggggct ctgggctttg aagccagctc ctcgattact 508440
caaaatagct cccttttctt ctctggaaac actgcaacag atgctgcagg caagggcggg 508500
gccatttatt gtgaaaaaac aggagagact cctactctta ctatctctgg aaataaaagt 508560
ctgaccttcg ccgagaactc ttcagtaact caaggcggag caatctgtgc ccatggtcta 508620
gatctttccg ctgctggccc taccctattt tcaaataata gatgcgggaa cacagctgca 508680
ggcaagggcg gcgctattgc aattgccgac tctggatctt taagtctctc tgcaaatcaa 508740
ggagacatca cgttccttgg caatactcta acctcaacct ccgcgccaac atcgacacgg 508800
aatgctatct acctgggatc gtcagcaaaa attacgaact taagggcagc ccaaggccaa 508860
tctatctatt tctatgatcc gattgcatct aacaccacag gagcttcaga cgttctgacc 508920
atcaaccaac cggatagcaa ctcgccttta gattattcag gaacgattgt attttctggg 508980
gaaaagctct ctgcagatga agcgaaagct gctgataact tcacatctat attaaagcaa 509040
ccattggctc tagcctctgg aaccttagca ctcaaaggaa atgtcgagtt agatgtcaat 509100
ggtttcacac agactgaagg ctctacactc ctcatgcaac caggaacaaa gctcaaagca 509160
gatactgaag ctatcagtct taccaaactt gtcgttgatc tttctgcctt agagggaaat 509220
aagagtgtgt ccattgaaac agcaggagcc aacaaaacta taactctaac ctctcctctt 509280
gttttccaag atagtagcgg caatttttat gaaagccata cgataaacca agccttcacg 509340
cagcctttgg tggtattcac tgctgctact gctgctagcg atatttatat cgatgcgctt 509400
ctcacttctc cagtacaaac tccagaacct cattacgggt atcagggaca ttgggaagcc 509460
acttgggcag acacatcaac tgcaaaatca ggaactatga cttgggtaac tacgggctac 509520
aaccctaatc ctgagcgtag agcttccgta gttcccgatt cattatgggc atcctttact 509580
gacattcgca ctctacagca gatcatgaca tctcaagcga atagtatcta tcagcaacga 509640
ggactctggg catcaggaac tgcgaatttc ttccataagg ataaatcagg aactaaccaa 509700
gcattccgac ataaaagcta cggctatatt gttggaggaa gtgctgaaga tttttctgaa 509760
aatatcttca gtgtagcttt ctgccagctc ttcggtaaag ataaagacct gtttatagtt 509820
gaaaatacct ctcataacta tttagcgtcg ctatacctgc aacatcgagc attcctagga 509880
ggacttccca tgccctcatt tggaagtatc accgacatgc tgaaagatat tcctctcatt 509940
ttgaatgccc agctaagcta cagctacact aaaaatgata tggatactcg ctatacttcc 510000

tatcctgaag ctcaaggctc ttggaccaat aactctgggg ctctagagct cggaggatct 510060
ctggctctat atctccctaa agaagcaccg ttcttccagg gatatttccc cttcttaaag 510120
ttccaggcag tctacagccg ccaacaaaac tttaaagaga gtggcgctga agcccgtgct 510180
tttgatgatg gagacctagt gaactgctct atccctgtcg gcattcggtt agaaaaaatc 510240
tccgaagatg aaaaaaataa tttcgagatt tctctagcct acattggtga tgtgtatcgt 510300
aaaaatcccc gttcgcgtac ttctctaatg gtcagtggag cctcttggac ttcgctatgt 510360
aaaaacctcg cacgacaagc cttcttagca agtgctggaa gccatctgac tctctcccct 510420
catgtagaac tctctgggga agctgcttat gagcttcgtg gctcagcaca catctacaat 510480
gtagattgtg ggctaagata ctcattctag ttcctacttt cctccctaaa cttttaggga 510540
ggaattctta taaaaaccct gtagattctt aacttactag tctctccttt cctcttgctt 510600
tctttaattt attgcagtat gtggtgaaat aatttgttaa accacctata gccctctaca 510660
tgaaatcctc tcttcattgg tttttaatct cgtcatcttt agcacttccc ttgtcactaa 510720
atttctctgc gtttgctgct gttgttgaaa tcaatctagg acctaccaat agcttctctg 510780
gaccaggaac ctacactcct ccagcccaaa caacaaatgc agatggaact atctataatc 510840
taacagggga tgtctcaatc accaatgcag gatctccgac agctctaacc gcttcctgct 510900
ttaaagaaac tactgggaat ctttctttcc aaggccacgg ctaccaattt ctcctacaaa 510960
atatcgatgc gggagcgaac tgtaccttta ccaatacagc tgcaaataag cttctctcct 511020
tttcaggatt ctcctatttg tcactaatac aaaccacgaa tgctaccaca ggaacaggag 511080
ccatcaagtc cacaggagct tgttctattc agtcgaacta tagttgctac tttggccaaa 511140
acttttctaa tgacaatgga ggcgccctcc aaggcagctc tatcagtcta tcgctaaacc 511200
ccaacctaac gtttgccaaa aacaaagcaa cgcaaaaagg gggtgccctc tattccacgg 511260
gagggattac aattaacaat acgttaaact cagcatcatt ttctgaaaat accgcggcga 511320
acaatggcgg agccatttac acggaagcta gcagttttat tagcagcaac aaagcaatta 511380
gctttataaa caatagtgtg accgcaacct cagctacagg gggagccatt tactgtagta 511440
gtacatcagc ccccaaacca gtcttaactc tatcagacaa cggggaactg aactttatag 511500
gaaatacagc aattactagt ggtggggcga tttatactga caatctagtt ctttcttctg 511560
gaggacctac gctttttaaa aacaactctg ctatagatac tgcagctccc ttaggaggag 511620
caattgcgat tgctgactct ggatctttga gtctttcggc tcttggtgga gacatcactt 511680
ttgaaggaaa cacagtagtc aaaggagctt cttcgagtca gaccactacc agaaattcta 511740
ttaacatcgg aaacaccaat gctaagattg tacagctgcg agcctctcaa ggcaatacta 511800
tctacttcta tgatcctata acaactagca tcactgcagc tctctcagat gctctaaact 511860
taaatggtcc tgaccttgca gggaatcctg catatcaagg aaccatcgta ttttctggag 511920
agaagctctc ggaagcagaa gctgcagaag ctgataatct caaatctaca attcagcaac 511980
ctctaactct tgcgggaggg caactctctc ttaaatcagg agtcactcta gttgccaagt 512040
ccttttcgca atctccgggc tctaccctcc tcatggatgc agggaccaca ttagaaaccg 512100
ctgatggatc actatcaata atctgttctc aatgtagatt ccttaaaaga gaccaagaag 512160
ntacgctaaa agcaacacaa gcaagtcaga cagtcacttt atctggatcg ctctctcttg 512220
tagatccttc tggaaatgtc tacgaagatg tctcttggaa taaccctcaa gtcttttctt 512280
gtctcactct tactgctgac gaccccgcga atattcacat cacagactta gctgctgatc 512340
ccctagaaaa aaatcctatc cattggggat accaagggaa ttgggcatta tcttggcaag 512400
aggatactgc gactaaatcc aaagcagcga ctcttacctg gacaaaaaca ggatacaatc 512460
cgaatcctga gcgtcgtgga accttagttg ctaacacact atggggatcc tttgttgatg 512520
tgcgctccat acaacagctt gtagccacta aagtacgcca atctcaagaa actcgcggca 512580
tctggtgtga agggatctcg aacttcttcc ataaagatag cacgaagata aataaaggtt 512640
ttcgccacat aagtgcaggt tatgttgtag gagcgactac aacattagct tctgataatc 512700
ttatcactgc agccttctgc caattattcg ggaaagatag agatcacttt ataaataaaa 512760
atagagcttc tgcctatgca gcttctctcc atctccagca tctagcgacc ttgtcttctc 512820
caagcttgtt acgctacctt cctggatctg aaagtgagca gcctgtcctc tttgatgctc 512880
agatcagcta tatctatagt aaaaatacta tgaaaaccta ttacacccaa gcaccaaagg 512940
gagagagctc gtggtataat gacggttgcg ctctggaact tgcgagctcc ctaccacaca 513000
ctgctttaag ccatgagggt ctcttccacg cgtatttccc tttcatcaaa gtagaagctt 513060
cgtacataca ccaagatagc ttcaaagaac gtaatactac cttggtacga tctttcgata 513120
gcggtgattt aattaacgtc tctgtgccta ttggaattac cttcgagaga ttctcgagaa 513180
acgagcgtgc gtcttacgaa gctactgtca tctacgttgc cgatgtctat cgtaagaatc 513240
ctgactgcac gacagctctc ctaatcaaca atacctcgtg gaaaactaca ggaacgaatc 513300
tctcaagaca agctggtatc ggaagagcag ggatctttta tgccttctct ccaaatcttg 513360
aggtcacaag taacctatct atggaaattc gtggatcttc acgcagctac aatgcagatc 513420
ttggaggtaa gttccagttc taaaagcgtt cctgatccct tagaaattct aagagatcct 513480
gagtgtatct agggacttct caaagacaat gcgccttgtt tagacgagga gaagtcgaaa 513540
gatcagagga atctaagaac tagaaaaaat cccagattac aagaggtcga agattcaaag 513600
aagtcgctag atcacaagaa agaatggcca gagtacgtga tctcacgaac tccgcccgtc 513660
ttagaatcca aagacttttt agtatactaa gactgtctat gatccaatag ccaaagactc 513720
ttgaatattt aaatgttttc tatgatccga aaactcttag agtcgataaa aactccggat 513780
tctttatccc cagatctctt tagcaatctc tttcacaaag gcgatcttct tccactgttc 513840

ttcttcagtc attctatggt ctccctcaca agaagcaaac ccacattgcg ggctcaaaga 513900
aagtctctct aagggaatgt agctcgcagc ttcataaata cgagaaacca cagcatctcg 513960
atcttcaata caagaatggt tgctggagat caatcccaag cagacgtgtt tctctccaga 514020
gacgtaagct aaaggctcag cacctcctga atacttatca tcaagagccc aataatagtg 514080
ataactatcc acatcggtct tagcaaataa aggctcctct atagaatcat aagctcgtct 514140
agagaaaaac tcggcctgat aatcaccacg acagacatgc agacttacaa aaagatcctc 514200
gggtctatcc ttcatcacta aattatggat ccataaaaac tgttctaaaa tttcctgcaa 514260
cctgtcatga gaatcaacac cataccaaga aggcgctcgt atatccaaga ggcgacacca 514320
agcacaatcg tccaactgca aattacgaca acctgcagca taaagatctt ggatgacttg 514380
gcgataataa aagacaatat catcaattag ctcttgattc gtaggataaa acttccgagt 514440
atttttcaga ttaggagcaa aaatcatctc atggaaaaat tgtgatggag aaggaatcgt 514500
ttgttttgct tttgcatttc ccttctcaaa agttttgaca aactcgaaat gttctataaa 514560
cggatgtttt gatacggaga ttttatcttt aagatacact ccaatttcag ggtcattaga 514620
gtccctgcga cgatccacgc catggaatcc ccacataaag tcgaaatccc aactatacct 514680
acggaattcc ccatcagtaa aaaagataag acctgcttct gtttgctttt ttatgagatt 514740
acgaatagca gcatcttcga caactcgcat ctgctcatag acaattcttc cttcttcaaa 514800
atcagagcgt gctcgtgtaa gactttcagg acgtaagaaa cttcccacaa catcaaaatg 514860
acattgctca ggttgttgga atgggctcat catttccatc cttagagatg atcctaataa 514920
aatacatttc cactcatgga tctctcttct cgatggttga ataaattaaa gacaagaaaa 514980
cagctagatc aaaacataaa gtacgattgt aaaagttact gcctgagggg aatctcacaa 515040
atcttaggat gggtgtaaca acaccccttc ccttaaaaaa gaaagagacg taccgtctct 515100
gcaactctaa tctttgcagc tattctctat aagaagcata acaaaaacca cttaaaattt 515160
aggatctaaa aatagtgaat tatcttattc aataagaatt aaagaaacgt attttaaaga 515220
acgggtttct attaaaaaat ttccttaatt ttaaaacgtg tttgtaacaa gaaaggcaga 515280
gtacgtgacc tcacgaactc tgccctcttg cctttttaat ccccgatctc tcctggttca 515340
aaaacaaggt gagtcgtgtt taagaaaatc ctaaatgcga tctctctcag aatttctgag 515400
acatgagaga cgctcctaga attggaactt acccccaaga tctacattat aaatccgtga 515460
ggatccacga acttcaaaga caaactggcc gagcacttca aacataggag agaaggcgta 515520
gtgactgcct gcacgcactt gcaaggcctg tcgtgctaag ttattggcat aagtttccca 515580
agaggctccg ctgattacaa gtgctgtagt gcatttggga tcattgcgga taagatcagg 515640
aacataggat aaagtcagat cataagaaaa gtcattacaa tcagagaact tctcaaactt 515700
cacccctata ggcaaagata aattgaagag gttgctgtca tcaaaagatc ttccttctgt 515760
acctttctcc gagaagctgt cctgacgtat ataggtcaga ttcagtttga tgtatggagc 515820
ataggtatca aaacaatgca ggtattcagg ataagaatga gaagaagctc ccaacatcat 515880
gttaaaagca ttattccccc aagaaccttt cacctcagga tacgcagtat actttgtctt 515940
cagatcatta ctgacgtggc tataagcgag ctgcccttct aaaacgaggg gtttatgact 516000
ccaagagcca ggaagtttat ctaagagaca acctatgaac ccactacatt ctgtaatgtg 516060
ttggatatag aaggctcctg cataggtatc agtatgattt ttagcgacta agaaatcttt 516120
atcgctacca aagagttggc aaaaggcaaa gctaattaag ttttcagaac aagtttgcgc 516180
tgcacctccg atagcatatc caccagattt atgacggtat ttgcgttttt cccctttctt 516240
atctttatct aagaaattgg cgactcccgc agcccagaag cctcgatctg aacaaagagt 516300
caaagcactt ctctctatga caccttgaat cgcttggatg tctgaaaaag atccccaaag 516360
gctattagga actaaaggtc cttgacgctc aggattcgga aggtagcccg tattggtcca 516420
agctaatgtc gctgtcttag tctttggagt gcttgcggta tcatcaaccc aagtcattcc 516480
ccaagtacct tgatacccat agtgcgtagg agttgctact gtaggaaccg ctggaacatc 516540
tgtagttgtt gcagtaccca gagcagagag ctgcacaaat gaaaagtctt gagtttttcc 516600
taagtcgtga ttttcataag cattcccttg gttatccaaa agaagaatcg gaccactaag 516660
ggctacattt ttacttgctg cagaagcagc aattacaact ttcttaccct cgcctaaaga 516720
gtctacagga atggaaagac ctgttaaagt gacctcctct gtacttgctt ttaacgttgt 516780
gcccgcatcc ataataacag aggaacccgc ggtctgagta aagcctttcg tatcgagagt 516840
gacaccacgt ttaagtacta aatttcctgc agttagagtt acaggctgct tcagcgtaga 516900
agtgaggttg tctgcaactt ttgcttcatc ttcagagagc ttttcaccag aaaaaacaat 516960
cgacccacta taatctgtac tattacctgc atcagcctta ttgagattta aagtatctgt 517020
agaatccgca gccgtattag cagtaatcgg atcgtagaaa aagatgctat gcccagatat 517080
tgcacgtaaa ttcgtgatct ttgcagtaga tcctatgtca atagaatttc ttttcgtagt 517140
ttgtggtgta gttgcaacaa tggcattccc attgaaggta atgtcccctg cttctgctga 517200
aagactacac tctccagctg ccagtataga aatggctcca ccattacctg cagtggtacc 517260
ttggactata ttgttagaaa aggagatacc cccccccccc cccggaagcc agtgtaagct 517320
tcttagcata aatggctccg ccattagcta cagcttggtt tcctgagaaa gttacactct 517380
gattcccaga tatggtaaca tcggcatctc cagaaagagc tcctccattt cctgcggtcg 517440
ctgtcacact attttcagaa aatacaagag acgtattccc tgtaattgta cagtttcctg 517500
tgctatttat agctccacct gcagcttcag caatattgtt cgagaagagg gtaggagccg 517560
tattatttgt aatatctaca gtaccagtag cacaaatagc cccacctttt ttccctgttg 517620
cgctcgattt attcccttca aaagaaatcg atcccgtgct gtttttcaaa gaaagattct 517680

tggtagaaat gtccgccatt ttcctcacag taatcttgtt taaataaaat agttccattg 517740
ttatcaaatg taagatcccc tccacattta actgcacctt ttcctgaggg ggttgtgatt 517800
accgatgatg gggccgctaa gaaagtaaga ctcgaaaatc ctgttagcga cagattttta 517860
tcagttgtaa cagaaagtnt gcgccttcag cactagactt natatttaaa aaagaaagtg 517920
agtacccctt accggcaaag cttaaagatt ccgtagtgtc agaaaaacaa cccttcgtta 517980
aagctgccga atccccaagg ttttgcagag ttatatctcc tgtcagagta tagtctattc 518040
cagtagtcgt atttttagga gtataggtgc ctgtgttagt acttccgtca aagctatcag 518100
aggggcctat attttcagca gttgcagcaa aaacagtgga acaactagta aaacatgcca 518160
atgtcgaaga gagcactaac caggaaaatt gcgatttcat aaacccactt tattattaaa 518220
ttcttacttg cgtcatataa aatagaaaac tcagagagtc aagataaaaa ttcttgacag 518280
ctgttttgtc atctttaact tgatttactt attttgtttc tatattgatg cgaatagttc 518340
tctaaaaaac aaaagcatta ccatgaagac ttcgattcct tgggttttag tttcctccgt 518400
gttagctttc tcatgtcacc tacagtcact agctaacgag gaacttttat cacctgatga 518460
tagctttaat ggaaatatcg attcaggaac gtttactcca aaaacttcag ccacaacata 518520
ttctctaaca ggagatgtct tcttttacga gcctggaaaa ggcactccct tatctgacag 518580
ttgttttaag caaaccacgg acaatcttac cttcttgggg aacggtcata gcttaacgtt 518640
tggctttata gatgctggca ctcatgcagg tgctgctgca tctacaacag caaataagaa 518700
tcttaccttc tcagggtttt ccttactgag ttttgattcc tctcctagca caacggttac 518760
tacaggtcag ggaacgcttt cctcagcagg aggcgtaaat ttagaaaata ttcgtaaact 518820
tgtagttgct gggaattttt ctactgcaga tggtggagct atcaaaggag cgtctttcct 518880
tttaactggc acttctggag atgctctttt tagtaacaac tcttcatcaa caaagggagg 518940
agcaattgct actacagcag gcgctcgcat agcaaataac acaggtnatg ttagattcct 519000
atctaacata gcgtctacgt caggaggcgc tatcgatgat gaaggcacgt cgatactatc 519060
gaacaacaaa tttctatatt ttgaagggaa tgcagcgaaa actactggcg gtgcgatctg 519120
caacaccaag gcgagtggat ctcctgaact gataatctct aacaataaga ctctgatctt 519180
tgcttcaaac gtagcagaaa caagcggtgg cgccatccat gctaaaaagc tagccctttc 519240
ctctggaggc tttacagagt ttctacgaaa taatgtctca tcagcaactc ctaagggggg 519300
tgctatcagc atcgatgcct caggagagct cagtctttct gcagagacag gaaacattac 519360
ctttgtaaga aataccctta caacaaccgg aagtaccgat actcctaaac gtaatgcgat 519420
caacatagga agtaacggga aattcacgga attacgggct gctaaaaatc atacaatttt 519480
cttctatgat cccatcactt cagaaggaac ctcatcagac gtattgaaga taaataacgg 519540
ctctgcggga gctctcaatc catatcaagg aacgattcta ttttctggag aaaccctaac 519600
agcagatgaa cttaaagttg ctgacaattt aaaatcttca ttcacgcagc cagtctccct 519660
atccggagga aagttattgc tacaaaaggg agtcacttta gagagcacga gcttctctca 519720
agaggccggt tctctcctcg gcatggattc aggaacgaca ttatcaacta cagctgggag 519780
tattacaatc acgaacctag gaatcaatgt tgactcctta ggtcttaagc agcccgtcag 519840
cctaacagca aaaggtgctt caaataaagt gatcgtatct gggaagctca acctgattga 519900
tattgaaggg aacatttatg aaagtcatat gttcagccat gaccagctct tctctctatt 519960
aaaaatcacg gttgatgctg atgttgatac taacgttgac atcagcagcc ttatccctgt 520020
tcctgctgag gatcctaatt cagaatacgg attccaagga caatggaatg ttaattggac 520080
tacggataca gctacaaata caaaagaggc cacggcaact tggaccaaaa caggatttgt 520140
tcccagcccc gaaagaaaat ctgcgttagt atgcaatacc ctatggggag tctttactga 520200
cattcgctct ctgcaacagc ttgtagagat cggcgcaact ggtatggaac acaaacaagg 520260
tttctgggtt tcctccatga cgaacttcct gcataagact ggagatgaaa atcgcaaagg 520320
cttccgtcat acctctggag gctacgtcat cggtggaagt gctcacactc ctaaagacga 520380
cctatttacc tttgcgttct gccatctctt tgctagagac aaagattgtt ttatcgctca 520440
caacaactct agaacctacg gtggaacttt attcttcaag cactctcata ccctacaacc 520500
ccaaaactat ttgagattag gaagagcaaa gttttctgaa tcagctatag aaaaattccc 520560
tagggaaatt cccctagcct tggatgtcca agtttcgttc agccattcag acaaccgtat 520620
ggaaacgcac tatacctcat tgccagaatc cgaaggttct tggagcaacg agtgtatagc 520680
tggtggtatc ggcctagacc ttccttttgt tctttccaac ccacatcctc ttttcaagac 520740
cttcattcca cagatgaaag tcgaaatggt ttatgtatca caaaatagct tcttcgaaag 520800
ctctagtgat ggccgtggtt ttagtattgg aaggctgctt aacctctcga ttcctgtggg 520860
tgcgaaattc gtgcaggggg atatcggaga ttcctacacc tatgatctct caggattctt 520920
tgtttccgat gtctatcgta acaatcccca atctacagcg actcttgtga tgagcccaga 520980
ctcttggaaa attcgcggtg gcaatctttc aagacaggca tttttactga ggggtagcaa 521040
caactacgtc tacaactcca attgtgagct cttcggacat tacgctatgg aactccgtgg 521100
atcttcaagg aactacaatg tagatgttgg taccaaactc cgattctaga ttgctaaaac 521160
tccctagttc ttctagggag ttttctcata cttttaggga aatatttgct atagggaatg 521220
ctttccttgc aaactgtaaa aaataacatt tgtccctctt caaaaaagat ttcttttaat 521280
aatttctagt tataatttta ttttaaaaac agttaaataa ttaatagaca ataatctatt 521340
cttattgact tctttttttc ttgtttatta aagttgcttc aaccttattg atttaacgag 521400
gaaaccatga ccatacttcg aaattttctt acctgctcgg ctttattcct cgctctccct 521460
gcagcagcac aagttgtata tcttcatgaa agtgatggtt ataacggtgc tatcaataat 521520

aaaagcttag aacctaaaat tacctgttat ccagaaggaa cttcttacat ctttctagat 521580
gacgtgagga tttccaacgt taagcatgat caagaagatg ctggggtttt tataaatcga 521640
tctgggaatc tttttttcat gggcaaccgt tgcaacttca cttttcacaa ccttatgacc 521700
gagggttttg gcgctgccat ttcgaaccgc gttggagaca ccactctcac tctctctaat 521760
ttttcttact tagcgttcac ctcagcacct ctactacctc aaggacaagg agcgatttat 521820
agtcttggtt ccgtgatgat cgaaaatagt gaggaagtga ctttctgtgg gaactactct 521880
tcgtggagtg gagctgcgat ttatactccc taccttttag gttctaaggc gagtcgtcct 521940
tcagtaaatc tcagcgggaa ccgctacctg gtgtttagag acaatgtgag ccaaggttat 522000
ggcggcgcca tatctaccca caatctcaca ctcacgactc gaggaccttc gtgttttgaa 522060
aataatcatg cttatcatga cgtgaatagt aatggaggag ccattgccat tgctcctgga 522120
ggatcgatct ctatatccgt gaaaagcgga gatctcatct tcaaaggaaa tacagcatca 522180
caagacggaa atacaataca caactccatc catctgcaat ctggagcaca gtttaagaac 522240
ctacgtgctg tttcagaatc cggagtttat ttctatgatc ctataagcca tagcgagtcg 522300
cataaaatta cagatcttgt aatcaatgct cctgaaggaa aggaaactta tgaaggaaca 522360
attagcttct caggactatg cctggatgat catgaagttt gtgcggaaaa tcttacttcc 522420
acaatcctac aagatgtcac attagcagga ggaactctct ctctatcgga tggggttacc 522480
ttgcaactgc attcttttaa gcaggaagca agctctacgc ttactatgtc tccaggaacc 522540
actctgctct gctcaggaga tgctcgggtt cagaatctgc acatcctgat tgaagatacc 522600
gacaactttg ttcctgtaag gattcgcgcc gaggacaagg atgctcttgt ctcattagaa 522660
aaacttaaag ttgcctttga ggcttattgg tccgtctatg actttcctca atttaaggaa 522720
gcctttacga ttcctcttct tgaacttcta gggccttctt ttgacagtct tctcctaggg 522780
gagaccactt tggagagaac ccaagtcaca acagagaatg acgccgttcg aggtttctgg 522840
tccctaagct gggaagagta ccccccttct ctggataaag acagaaggat cacaccaact 522900
aagaaaactg ttttcctcac ttggaatcct gagatcactt ctacgccata atctctaagt 522960
ctacactata attaagggaa tcccctttaa gaagattttg ggacctatct gtattcagag 523020
ataggtccct ctatgcacac atgttcacga gtctcgggcg tagcgccatt ttctacttta 523080
caggttctct aaaacatctt cgtttgggag aatttcttga gatttttcaa aaatagaatc 523140
gccattttct atcaagtatt cttctaagaa agcaatgtaa gaaatgggaa aatgcccttt 523200
aaaatatcct gtaatcttaa agctgtcaaa attaagagat taaaactgtg tcttattgta 523260
cttgtttttt tacagccttt cccttatttg taggataatc tggtttcatc tctacgtgca 523320
aatgaaaacg tctattcgta agttcttaat ttctaccaca ctggcgccat gttttgcttc 523380
aacagcgttt actgtagaag ttatcatgcc ttccgagaac tttgatggat cgagtgggaa 523440
gatttttcct tacacaacac tttctgatcc tagagggaca ctctgtattt tttcagggga 523500
tctctacatt gcgaatcttg ataatgccat atccagaacc tcttccagtt gctttagcaa 523560
tagggcggga gcactacaaa tcttaggaaa aggtggggtt ttctccttct taaatatccg 523620
ttcttcagct gacggagccg cgattagtag tgtaatcacc caaaatcctg aactatgtcc 523680
cttgagtttt tcaggattta gtcagatgat cttcgataac tgtgaatctt tgacttcaga 523740
tacctcagcg agtaatgtca tacctcacgc atcggcgatt tacgctacaa cgcccatgct 523800
ctttacaaac aatgactcca tactattcca atacaaccgt tctgcaggat ttggagctgc 523860
cattcgaggc acaagcatca caatagaaaa tacgaaaaag agccttctct ttaatggtaa 523920
tggatccatc tctaatggag gggccctcac gggatctgca gcgatcaacc tcatcaacaa 523980
tagcgctcct gtgatttctt caacgaatgc tacagggatc tatggtgggg ctatttacct 524040
taccggagga tctatgctca cctctgggaa cctctcagga gtcttgttcg ttaataatag 524100
ctcgcgctca ggaggcgcta tctatgctaa cggaaatgtc acattttcta ataacagcga 524160
cctgactttc caaaacaata cagcatctcc acaaaactcc ttacctgcac ctacacctcc 524220
acctacacca ccagcagtca ctcctttgtt aggatatgga ggcgccatct tctgtactcc 524280
tccagctacc cccccaccaa caggtgttag cctgactata tctggagaaa acagcgttac 524340
attcctagaa aacattgcct ccgaacaagg aggagccctc tatggcaaaa agatctctat 524400
agattctaat aaatctacaa tatttcttgg aaatacagct ggaaaaggag gcgctattgc 524460
tattcccgaa tctggggagc tctctctatc cgcaaatcaa ggtgatatcc tctttaacaa 524520
gaacctcagc atcactagtg ggacacctac tcgcaatagt attcacttcg gaaaagatgc 524580
caagtttgcc actctaggac tacgcaaggc tataccctat acttctatga tccgattaca 524640
tctgatgatt tatctctgca tccgcagccg ctactgtggt cgtcaatccc aaagccagtg 524700
cagatggtgc gtattcaggg actattgtct tttcaggaga aaccctcact gctaccgaag 524760
cagcaacccc tgcaaatgct acatctacat taaaccaaaa gctagaactt gaaggcggta 524820
ctctcgcttt aagaaacggt gctaccttaa atgttcataa cttcacgcaa gatgaaaagt 524880
ccgtcgtcat catggatgca gggaccacat tagcaactac aaatggagct aataatactg 524940
acggtgctat caccttaaac aagcttgtaa tcaatctgga ttctttggat ggcactaaag 525000
cggctgtcgt taatgtgcag agtaccaatg gagctctcac tatatccgga actttaggac 525060
ttgtgaaaaa ctctcaagat tgctgtgaca accacgggat gtttaataaa gatttacagc 525120
aagttccgat tttagaactc aaagcgactt caaatactgt aaccactacg gacttcagtc 525180
tcggcacaaa cggctatcag caatctccct atgggtatca aggaacttgg gagtttacca 525240
tagacacgac aacccatacg gtcacaggaa attggaaaaa aaccggttat cttcctcatc 525300
cggagcgtct tgctcccctc attcctaata gcctatgggc aaacgtcata gatttacgag 525360

ctgtaagtca agcgtcagca gctgatggcg aagatgtccc tgggaagcaa ctgagcatca 525420
caggaattac aaatttcttc catgcgaatc ataccggtga tgcacgcagc taccgccata 525480
tgggtggagg ctacctcatc aatacctaca cacgcatcac tccagatgct gcgttaagtc 525540
taggttttgg acagctgttt acaaaatcta aggattacct cgtaggtcac ggtcattcta 525600
acgtttattt cgctacagta tactctaaca tcaccaagtc tctgtttgga tcatcgagat 525660
tcttctcagg aggcacttct cgagttacct atagccgtag caatgagaaa gtaaagactt 525720
catatacaaa attgcctaaa gggcgctgct cttggagtaa caattgctgg ttaggagaac 525780
tcgaagggaa ccttcccatc actctctctt ctcgcatctt aaacctcaag cagatcattc 525840
cctttgtaaa agctgaagtt gcttacgcga ctcatggggg catccaagaa aatacccctg 525900
aggggaggat ttttggacac ggtcatctac tcaacgttgc agttcccgta ggcgtccgct 525960
ttggtaaaaa ttctcataat cgaccagatt tttacactat aatcgtagcc tatgctcctg 526020
atgtctatcg tcacaatcct gattgcgata cgacattacc tattaatgga gctacgtgga 526080
cctctatagg gaataatcta accagaagta ctttgctagt acaagcatcc agccatactt 526140
cagtaaatga tgttctagag atcttcgggc actgtggatg tgatattcgc agaacctccc 526200
gtcaatatac tctagatata ggaagcaaat tacgatttta aaccttattt aacgacaggg 526260
ttgaggcatg cctctttctt tcaaatcttc atctttttgt ctacttgcct gtttatgtag 526320
tgcaagttgc gcgtttgctg agactagact cggagggaac tttgttcctc caattacgaa 526380
tcagggtgaa gagatcttac tcacttcaga ttttgtttgt tcaaacttct tgggggcgag 526440
tttttcaagt tcctttatca atagttccag caatctctcc ttattaggga agggcctttc 526500
cttaacgttt acctcttgtc aagctcctac aaatagtaac tatgcgctac tttctgccgc 526560
agagactctg accttcaaga atttttcttc tataaacttt acagggaacc aatcgacagg 526620
acttggcggc ctcatctacg gaaaagatat tgttttccaa tctatcaaag atttgatctt 526680
cactacgaac cgtgttgcct attctccagc atctgtaact acgtcggcaa ctcccgcaat 526740
cactacagta actacaggag cctctgctct ccaacctaca gactcactca ctgtcgaaaa 526800
catatcccaa tcgatcaagt tttttgggaa ccttgccaac ttcggctctg caattagcag 526860
ttctcccacg gcagtcgtta aattcatcaa taacaccgct accatgagct tctcccataa 526920
ctttacttcg tcaggaggcg gcgtgattta tggaggaagc tctctccttt ttgaaaacaa 526980
ttctggatgc atcatcttca ccgccaactc ctgtgtgaac agcttaaaag gcgtcacccc 527040
ttcatcagga acctatgctt taggaagtgg cggagcatct gcatccctac gggaactttc 527100
gaattaaaaa acaatcaggg gaagtgcacc ttctcttata atggtacacc aaatgatgcg 527160
ggtgcgatct acgccgaaac ctgcaacatc gtagggaacc agggtgcctt gctcctagat 527220
agcaacactg cagcgagaaa tggcggagcc atctgtgcta aagtgctcaa tattcaagga 527280
cgcggtccta ttgaattctc tagaaaccgc gcggagaagg gtggagctat tttcataggc 527340
ccctctgttg gagaccctgc gaagcaaaca tcgacactta cgattttggc ttccgaaggt 527400
aatattgcgt tccaaggaaa catgctcaat acaaaacctg gaatccgcaa tgccatcact 527460
gtagaagcag ggggagagat tgtgtctcta tctgcacaag gaggctcacg tcttgtattt 527520
tatgatccca ttacacatag cctcccaacc acaagtccgt ctaataaaga cattacaatc 527580
aacgctaatg gcgcttcagg atctgtagtc tttacaagta agggactctc ctctacagaa 527640
ctcctgttgc ctgccaacac gacaactata cttctaggaa cagtcaagat cgctagtgga 527700
gaactgaaga ttactgacaa tgcggttgtc aatgttcttg gcttcgctac tcagggctca 527760
ggtcagctta ccctgggctc tggaggaacc ttagggctgg caacacccac gggagcacct 527820
gccgctgtag actttacgat tggaaagtta gcattcgatc ctttttcctt cctaaaaaga 527880
gattttgttt cagcatcagt aaatgcaggc acaaaaaacg tcactttaac aggagctctg 527940
gttcttgatg aacatgacgt tacagatctt tatgatatgg tgtcattaca atctccagta 528000
gcaattccta tcgctgtttt caaaggagca accgttacta agacaggatt tcctgatggg 528060
gagattgcga ctccaagcca ctacggctac caaggaaagt ggtcctacac atggtcccgt 528120
cccctgttaa ttccagctcc tgatggagga tttcctggag gtccctctcc tagcgcaaat 528180
actctctatg ctgtatggaa ttcagacact ctcgtgcgtt ctacctatat cttagatccc 528240
gagcgttacg gagaaattgt cagcaacagc ttatggattt ccttcttagg aaatcaggca 528300
ttctctgata ttctccaaga tgttcttttg atagatcatc ccgggttgtc cataaccgcg 528360
aaagctttag gagcctatgt cgaacacaca ccaagacaag gacatgaggg cttttcaggt 528420
cgctatggag gctaccaagt gcgctatcta tgaactacac ggaccacact acgttaggac 528480
tttctttcgg gcagctttat ggaaaaacta acgccaaccc ctacgattca cgttgctcag 528540
aacaaatgta tttactctcg ttctttggtc aattccctat cgtgactcaa aagagcgagg 528600
ccttaatttc ctggaaagca gcttatggtt attccaaaaa tcacctaaat accacctacc 528660
tcagacctga caaagctcca aaatctcaag ggcaatggca taacaatagt tactatgttc 528720
ttatttctgc agaacatcct ttcctaaact ggtgtcttct tacaagacct ctggctcaag 528780
cttgggatct ttcaggtttt atttccgcag aattcctagg tggttggcaa agtaagttca 528840
cagaaactgg agatctgcaa cgtagcttta gtagaggtaa agggtacaat gtttccctac 528900
cgataggatg ttcttctcaa tggttcacac catttaagaa ggctccttct acactgacca 528960
tcaaacttgc ctacaagcct gatatctatc gtgtcaaccc tcacaatatt gtgactgtcg 529020
tctcaaacca agagagcact tcgatctcag gagcaaatct acgccgccac ggtttgtttg 529080
tacaaatcca tgatgtagta gatctcaccg aggacactca ggcctttcta aactatacct 529140
ttgacgggaa aaatggattt acaaaccacc gagtgtctac aggactaaaa tccacatttt 529200

aaaactctaa gctctgctta gagttttctg tagccccggt cgtcttagaa tcctctatcc 529260
atcatcgaag aacttagcaa tgaaggccaa gattctcact ctatgagaac gcccccccct 529320
ctctcaagtc tatttgaaaa gaaagaatat cttttgaatc tatagtctgt tttggaaaga 529380
agagaatacg atctgctttg catccctagg gaaagcatct ttcatcaatg cgattacttc 529440
acgttgcaat ggcttcactt gtttatgcat acgttaacaa ggattatgaa tccttgaaag 529500
actcgctctc ctctcgattc tcgtgtagaa ttcactccct aggttttatc tttagagtct 529560
gcagcagatg aaactttagg attcagttcc tcatcctcat catcagaaat caaagggtct 529620
ttaccagaaa tgggcaacac gtcactgggg cataagaact ctatttggct cgctaactta 529680
tcgaatgctg cacagttcac aatgagtgta tgctcttgag aggtacggac gatactacta 529740
tcttctatga ctatgccctg agaacttgtg tacggtatca tgtctaaatt tgaaacgtaa 529800
gatactaaag catctgttac tgaagcctct agagcttctc tctcataagc aaaaagatcc 529860
ggattgcgga atctccacac agactgtttt atcacggcgt ctagagcaaa ggctagattt 529920
ttctctgtgg agactgtgtt gcagaggagt gctcctaact gctcttcagt taaagaccct 529980
tgcattgttt gttcttgttt ctctctgatc tcctccgcgt aggcaatatg actggagacc 530040
caagggagat gctcatcatc gaaagcgacc actttacatc ctgaagcata ggcatcagca 530100
gcttcttttt cacgagaaac aaaaattctt tcggatgcca actgcaaatt ccgaatatcg 530160
gggtgccctt caggaagctc tgagatcacc accgtcatag atctcccttc atcggcattc 530220
aatagcagac gttgttgaac tgtagtctcc ccggagagat cacaacgcac tacattcaga 530280
tagctatagc cttcatcgct aagctcttca ataagctgag agactgcttc aggaggcagg 530340
ccgaggatat taggttcaga atgattacaa acgacaacta cgtctttctc cttctcatca 530400
taggacacta aaagtaaaaa gcgcagccac gactctacgg aagtatcgtt agcaaaattt 530460
agagatacct cacgaagctc tttcttactt atgttcttag catccacaaa tgctgaaaat 530520
aacaaaattt tcagctcttc atgggtgaga tctgcaagat aggaatggca gtgagaccct 530580
tgagataaag gagcatggag tcgttgccgg ttctaagagc aatccctgaa atcctgggct 530640
ccaagtccca ttcccaaagt atgcaactga tggtgtgtac gagaaatctc tttataaagc 530700
tctgtcttat ccttttcggt atttataggt aaggaaagcc gagatctata cctagatggc 530760
aatgtgtgca taacattcac ccagggaaca gtacctaacg aagtccctag ggcctgctgt 530820
aaaattttac cctcccttaa gtaagaatcc ttcccataac agccgaccat aaggtcaaag 530880
tgtttcgtat cttcgccctc ggcaccctga ggttccaaga cggaaagaca ctgaacttca 530940
gaatgtagac ctagagaacg gagctgtttt tccttatcag aggagatatc ccctacccac 531000
tcctnattaa aatcgataag aagatataga gttgtctttg atttgggata gagttgaatg 531060
acctcattcg ccaagcgtac taaattctct tgttcttcag gactgacgag atctatacca 531120
acaatagcta tagagtccaa ctgaggagta ggggcataac tataggatct ggtgatgcga 531180
ttgcgaactc tagaaaaggc tttgccaagc tcgctaacat gaactccctt atcgagttta 531240
agaacctcac tagaggccat atctgcataa ggattctctt gatattgccc gtggacttta 531300
tctagatctt caacctcgat agtttcccta aggctcttta gctgattttc gattctagaa 531360
atatgatctc ggcccgcgat cttttctact ttcagtatag gagcaatatc tgaagggaac 531420
ccagggtgtg agactcccct ctcgaagcaa tgagagatca accaatcgac tcccataaca 531480
agatgcccta caatggggat ggccttaatc acccctaaag cgaagtgggt aattcgagaa 531540
tataaaggac gtgagaaatt aaacgcctgc acaaagtgat gacgaactac agaaaaccaa 531600
ccatgtaagc aatgagtcat aagaaattta cgtttagatt caaaagtaca aggaatttaa 531660
atgatgtgcc aaaaatacaa aataaacaat ttgtattaga catcaagtac actgaaaata 531720
aaacgctctt gttgagcacg ttttcctaac aaatacgtta tcgattctta ggggaaatag 531780
gttctgacaa ttagatttga gagactctgt tatgagagag agaccatagg cacaaagaca 531840
cgcctgtaaa gcagagtatc ctgattcaaa gcttagacat tgcgtatcgt tatcctcaaa 531900
ccgtcgaatt ttctttgtct ctatttgttg gctcctaagt gagcttcctc ctctggaatt 531960
ggagatattt ctgaaggagc tctgctaaga gtcgatccta gagattcaac agcttcatct 532020
aatgctgtga aatctataga caattgcgcg gattgctgcg tttctagatt cccctgagga 532080
tctttttttg cgttgttagg tagtgatgta cagtagtaaa gagttgttcc cctaacgccc 532140
gtctctcaat agagagaagt tctggcatac gggatctcca tatagactgg cggattgcag 532200
catctaaaga acaagcgaag ttctgttgcg ccgtcagctg attacaaacg taaatctcgc 532260
tctcgtcttg atccaaagca gcttcttgtt tctgttttct aacttctctt ttccaaagac 532320
catgctttct attccacaga ttagattctc gctccaaagt taagacttct tccatcataa 532380
agttcgcgca tcctgacgta tagacatcga cagcatagca ctctttacca caaagcattc 532440
cttcagacgc caaatgcaaa cagcggatat ctccttgaga tactgaatcc tcacagaaaa 532500
tcatggtaaa ggctttctct tgagaacttt catttaagat cctacgttct tctacacaca 532560
tgctattaga tctaaaggca aaaatattga agtgcgaata ccctcgacac tctagctcca 532620
taataaagtg ttggaagctt ggctcctcct tccagtcggt accccaccaa gaatcacata 532680
caacgacaac cacattgcga tcacttctgc tcatcctctc aaaaaagcca aagtgctgcc 532740
ttaagtaagt tttgctattt aagataagat ttagagatat aggttgcagt atggtactgc 532800
tcaactcctt aggattcaga aatgccgcta aaattgtttt ttgtaacccg ggatttctta 532860
agtctgcaag gtaggaatga caataatgcc cttcagataa gggagcatgc acccgttttg 532920
ggtccatcaa gatccctgac cgactatctc caaaccccaa actaagaacc gaagagcgct 532980
ctcctacgta taccaatcga gaatatagct gttctttttc taattcagag tactctagag 533040

ctttctcttt atctcccttc atctccaaag atgaaaatgg agagaaatcc cacaaggatt 533100
gtgaaagagg cttttgcccc acatgaatcc aaggggtttc ctcccctgac ttttctaact 533160
cttcctgaat ctgattcaac gtaccttgat cattctcccc atgatacacc atccaacagt 533220
cacaagttgc ttgtttgact acatcaccct gattaaagtc caaacaagca atcttggggt 533280
ttagttcgca atcactaagt aacttccgat tctttttgtc catagcattg cgaccaccag 533340
aagctgttag atatagagaa atcgtagcgt caggatagag tctttgagtt tcattacaga 533400
gacgggctag atgcaagaag tcgtcaactt caaaaggatt catatcgttt cctatgatgt 533460
aaatcttctg cagttttggc cgtggagctt gcaaatacgc ctgtcttaca cgattacgaa 533520
ttttagagaa ggcaagccct aacgttgcat cttgaacttc gggatagaga gtgagaactt 533580
ctacaggttg gattcccccg aaaggatgca caggaatctt cccatggacc ttatcctcat 533640
cttcaggagc cacagcgact ctctgcctct tcaaagtctc cactacacga gcaatgtgat 533700
cgcgaccagc aactttctct gttttcagaa gactcaccac atcagagaca aatgtcggct 533760
tggtcacgaa actctctaaa tacctagaaa cgagccactc tatcccaaca ataatgtgac 533820
ccacaacagg gatccccttg atgaccccta atacaaattc cgtaatacga gcacaaaacg 533880
gcaaagaaaa atcaaaggct cgataaaagt gctcacgaac tatagaaaac caggcagata 533940
aacaagaagc cataacacga ctaggaagaa atacttagga aagaagtatt taaaaaaaac 534000
ttgaaaaaag caatctctaa gatacttagc gactcctata gccacaagaa attcgctatt 534060
tttaaaatta gaaagaggag acctgagaaa ggttgtaaaa agcgcttaga tacaactatt 534120
tagaatccta tcctggaaat cagaaatcta cggaatacta gtgtagaaag aacaaatata 534180
gagagagtcc tctaaggagt ttgtgcactc ctggggtttg tgaaattaag atattcctag 534240
gcaaaccctt cctggaatcc taggtacatg gaccaaggat tccatctgga gataagaaat 534300
ttctctagta tttttacttt ctaactgcct tcattttgcg aggaatcagg gttactatct 534360
ggattaggaa cgggatgcgt aggatcattg cctgaatctg cagggacttc ggctgggagg 534420
acttgcacca tgcgatctag ctcttcgaag ctgataacga cctcatgttc ttcggtagtt 534480
cttttcccca tattctcatt acgctcctga ctccctaaat aggaaaatat cgcagttaag 534540
aactcctcgc ctagtgcctt tctttccata gtaagaagac ctttcgaacg gaatctccag 534600
atcgcctgtt ttaccacagc atctagagaa aatacaaaat tctgttgtgt agtgagcatg 534660
ctgctcaatt gatcctgaga ataaattact ggttctctgg cttcttctcc tgcttcatct 534720
tcgacacgtg cctcccattg tctatattca atagcctgag gactctgcat ctcagaaaac 534780
tgaatgcagg aacatccaga ggcacaaata tctgctacgc tggggagatc cttagcaacc 534840
ataccttctg cagctagctg caaacaacgg aaatctgctg cactaatggg atcctcaaat 534900
aagatcaatg tgaaagactt cccttcagaa cgatctccaa agaactgacg ttcttttaca 534960
atcattttcc gtgatttata ggagaaaatg ttcaaatagc tatatccaga catttgtaat 535020
cttctggtca gaatgttcat tgagtcagga tcaagatctt ctggacgagg agttccgtgg 535080
gcaagggtaa ctatgtttct cccttcttgc tgacccgcaa caaggcggaa aaataacgag 535140
tccaattcta atggcaatct agcgatattg aatgctacag gacgaagatc ctcgctacta 535200
agattgccag gatctaggaa aggcgaaagg actaaagttc gtagatcctc attttctata 535260
tccgcaaggt aggaatgaca ataatgccct tgggatagag gagcatagat tctatctggg 535320
tccatcagga gtcctgcatc ctctggtttt aatcccaaac caagcatata ctcatggtga 535380
tacgatcgac tcagttgtcc gtacaaggaa tccttatccc cctcactttc agggagtctt 535440
ggtggcgatg ttgggtctgt agattgtctt gtatgtgagt atgaagtaaa gtttggtggg 535500
taataaaaat cctttgtgtc ttcagtaaca tgaatccaag gaattggatc tgcactcttc 535560
cctagacact gctgaatata ctgcccgtct atattatgct gttgctctcg tgaataatag 535620
ccaatccaaa gttcggattt ttcaggagag ccagcgtcgt cttctttact gtctttgcac 535680
tggattctag agtcgagacc tgagttatgt aggtactgct tcttctctgc agtagttgta 535740
tcgcacatct gagattcgcg attcaagcct gtcaaataca gacaaactaa cgcttcagga 535800
tgcagtctct gcgtttcatt cgcgagactc acaaattgcg agaaatcttg aggagtttta 535860
agtttcgaac ccacaataga aatcgtacgt atttcgggtc ggggggcctg taagtacgca 535920
cgagtcacgc gcgtgcgaat cggagaaaat acagtatcta aagttccccc tctttctccc 535980
ggcttaagtt ttaaagtttc ctcggatttt aaacgaccaa aaggacagac aggaaacttc 536040
ccatgcacct tatcttgatt ctcaggagtt atggtccccc tttctctttg caatatctcc 536100
gccactcgag atatatgatc tcgacctaac gccttctcag tctttacaat ctgaacgaca 536160
tctgaggtaa aggaggacct agtaataatc ccggcaacac aggaagaaac taaccactcc 536220
atccccatga caatatgtcc tacaataggg atggccttga tgacccctaa agcaaaattc 536280
gtaaccctag aacaaaaggg acgagagaaa tcaaacgcct taacaaaatg ctgtcgaacg 536340
gtagaaatcc aagaagaaat atagggaatt ggcatnncaa caaaaaaaat aaaattagaa 536400
acaaacaatt ttaaataaca acatagaaaa tatcaattaa attgaataaa cagcttttaa 536460
atatttttag tattattttc tttaataaac taataaccaa ctacttttga agtcctagct 536520
tggatttaat atcctcatct aggggtaaaa taagaaacag cccttagatt cgagcagaaa 536580
tttcagtcag attctattca ggaaaaccta agattgtttc agagtcagga gaattcgaga 536640
ttctgaggga atttagtttt cagggagccc tttgcaagga acttttaagg tatcagacaa 536700
acgttactca tcttcgtgca tgtcagtcgg agatgctgtt gcccccgtag cggtcgaagc 536760
tgctgttgca tcttcgtcat catcagaaga gcatctttct tgtagagcct gctctaaagg 536820
ggtaaagtcg acagaaaact taaagctttt cttggttctc tgagatccca aaatcccgac 536880

catactctga atatattcct ctaagctgac tttgatttct cttcctaaag cctgactttc 536940
aacataaaat aaattgagtt tctcttgtga tctccaaccg gcctgtttaa ttacagcgtc 537000
taaagtaaat aagaagttct tctgtgccac gattttatca cacatatagg ctctgaaata 537060
ttctctgaaa gaaacactac cttcttctct caacttgttt gtagaagttc tccaattcct 537120
gagcccagga tgcatttgga ttttcgtaga ctaagacttt acatcccgaa gcacatgcat 537180
ccgcggcatc aagagaactg gagactaaaa tcctatccga ggctaattgt aaactacgga 537240
tatcttcgct acctaaagga agatctgtaa tcatgacagt aaaggaccgt ccttggagat 537300
ctcgccttag aatctgacgt tcctgaacct tggagactcc ttcggggctc accgagagaa 537360
tgtttagata cgaataccct gattctctta agtcttcttc taatagagac aatgcctctg 537420
gggacaatcc ttcttccaga agttttgcat cattacaaac aacagctacg tgcttttctt 537480
tctcgtcgtg cagaacacga gatagaaact ctgaccatct ctgtccaaac gaagagttgc 537540
caaagtttat agagacagaa cggaatgttt cgctagtgag atttttaggg tctagaaacg 537600
ttgacagaag ggaacgacgt agctctttgt tttgcaaatc taaaagatag gaaggacacg 537660
agtacggtga agataaaggc gcgcaaaccc tatagggatc cagaagccac cctgacaggg 537720
aattctgaat tcctagattc agcttataat gatccttaac agacataaac agagaacaat 537780
acgtggagaa ggattccagc ttattccatc ttttcttttt acctatttct ggggaatgat 537840
cgcacggaga gagcttctct tgttcttggt attgcacgga aatggaaggg gtctcttcta 537900
gagcaaagtt tagaagatgt tgtatcacct agggatcttg gacttcctga tctttcccgt 537960
aacaggtaat cataaaatcg acagtagcga cttctggcac tgaaggaaga cctgcactcg 538020
taagggatat actctctatt ttagggtcta aacctagagc tcggagttgc cctttttcct 538080
cttcagaaat ttcacagtcc cagacatctg ccaagttctt cgctaaatag agcttcactt 538140
tagtatgggg atagtgattc tgcacgccat tagcaagacg cacgaaattt atgaggtccg 538200
cagaatctcg gagaccaaaa cccacaagag caagatcttg aatcataggt ttctctacgg 538260
acctataggc ataggttaac ctactacgaa cgccttgcag tgcctcatct acagtagaga 538320
gttcttcaat cagggagaag gttggacaat ttctgtgggt gtgatatcta cgaagggatc 538380
ttctggggtt ctcccgtgta ctttgcctag atcttcttgg gaaatgggga ctctcaagct 538440
acttaaatag gcttctaggg gagctaaaca attatgaccc cgtgtttgtt ctactttaat 538500
agcactagag acatcagaag taaacattcc atgacgaacg gtgtgtctgg gaatccagga 538560
aatcaaccac tcgattccca tgacaatgtg tcctaatacg ggaattgcct taatgatccc 538620
caaagcaaaa tttgtaatcc gagaacaaac gggatgggtg aaatcaaagg cgtttacaaa 538680
atgctgtcga acgacagtaa accaagaaga aatacttgga caagccacag tagaaattta 538740
tgtataaaaa ttttaacaaa gttattttaa tcaaataaga cattaaaaca aataagtatc 538800
taattatgat aataatattt ttaaaaacgt ttttatttga aaaataaaaa gtcagtgtaa 538860
agagttcaag ctttcataaa atttttctat acaaaagaat cgtgggatac ctaagagaaa 538920
aaacctcgat accctctata gacaactaga caataagaac cctcgatacc ttcaaaaaac 538980
ccagaatgac ctaagaaaaa agaagaatct tcaaaaaaga gctctgctgg actaataaaa 539040
aggttcatca aaacaaacac cttagaaagg tgtgtctaat tagttcccat caatctgcga 539100
tttcgttcac aagattgcga tctttagtca tctgtattgt tctcctcaga aggtgcatct 539160
tgagctgagt cacaagctgc acacacagcc tcgtctaacg tagcaaaact cacattgaca 539220
ttacatgcga cttgaggctg tatagtcccc tcaactaaga gactgctgcc gatataacct 539280
tctaaagcac gtcgcatgtc atctcctaaa gcctgacttt ctacagcaaa aagatcccgt 539340
tttttaaact tccacatggc ctgttggatt accgcattga gattgaatac gatgtccttt 539400
tctaaaacta ctctcgtaga tgctgagacc cctaaaggct ctcctggaat cccctgacgt 539460
tgaagatcgc ctgccctgtc gatgtttcta tagaacgacg catactgttg cgcccactct 539520
tgctcgggat cctcatattc taagatctta cattcagaag cacaggcatc agcagcatcg 539580
agagcactaa gaaactaaga tcctatctga cgctagctgc aagttgcgga tatccgaact 539640
cccttcagga agatcagtga ggattacagt gaatgacctc cccgaaggat cagaacttaa 539700
aattctacgt tccttaacac acgtgcgttc ctgactcact gaaacgatat tcaaataaga 539760
ataacctgac tcttccagtt cgttctctaa tagagataaa gaatgtgagg gaaaactctt 539820
ttttataagt tgtggattat tgcagactac agccacatgc ttttctgttt catcatgtag 539880
aacacgagat agaaactcag accacctctg acccaaagga gagtttccaa agtttatgga 539940
cataggacgc ggatttccct gaccagaatt gttgggatct ataaaagcgg ataacaagaa 540000
acaacgaagc tcttcacttt ttaaatctaa gaggtatgag gggcaggaat actctccaga 540060
taaaggagcc gaaaccctaa gaggatctaa aaatactcca gaggcaaaat cttccattcc 540120
caagtgaaca gcatatctat cacatctagg gttagacagg gtggtatacg tttgaagatg 540180
ctcctgctca ctccaaggga tcctctgatc gggtgccgtt cctccttgag aaccaggaaa 540240
gaaatagtag ctataaaaag gatcgtcagg tctataggtt acagaaatcg cagggatatg 540300
ttcggaagaa agattgagta gactctttat agagtccacg tctctgacgg actgttgttt 540360
cccgtaataa ttaatgagaa gatcacagga aggcaagttc tcggattgaa gatatttctg 540420
gagcaggaga gcgggggccg acacacattt gattttagca tccaaaccta gagagcggag 540480
ttgctgttct ttttcttgag aaatcgtacc gtcacactga ttccatatct tttggataga 540540
gattaaataa agttttattt gagtttgggg gtactgagtt tgtatgccgt tagccagacg 540600
aacgaaattc acctgatctt cgggatctaa aatgtcaaaa ccgacgagcg taagacaagg 540660
gacttccaga ggagtgactc gttgatacgc cttggttact cggttgcgga ttccataaaa 540720

cgcgcgacct agctctccca gatattgatg aggaaccata ttcagatttt cttggggagt 540780
cctgtcgcaa acataatctc tggagacctt cccatggatt tttcctaaat ctccttcagg 540840
aagtcgaacc ctaagctgtc tcaagtactg ttccacccaa gcaaggggat tataacctcg 540900
agtttttctt attttcacga tactagcaac gtcagaagta aatgccggtt tcccaaacct 540960
tcgtgcagaa catgtggaaa ctagccaact tacccctata acaacgcatc ctaaaatggg 541020
aatagccttg atgaccccca aagcaaaatt tgtaatccga gaaccaagag gacgtgtaaa 541080
atcaaaagcc ctaataaaat gctgcttaac tgtagatatc caaatagaaa tatagcatgc 541140
cataggatca aaccaacatt gaaaatgaaa gcgtacgcag gctatataaa gatttaaaaa 541200
aagatagaaa attaactatc ttttaataag aacaacacga tagtttttat ataaaaaatt 541260
gaattctatc gcaactaatt aagagtcttc aagatccgaa ggatgatcat ctgaaggatc 541320
ctcagaggta gagggaacat cttcatcaga acctgggaag agcgcctctt taagaggaga 541380
acagtctgcg ataatctctt tttgtatgaa cgggcctttc gatgtttttt cgtgagagtt 541440
tgtgacatat tcaaggtacg atacgataga tggcaagcac tgtgttttca agtctagggc 541500
ttcacactct ttatctatca agctgggatg tttatgagtc cataccgctt gctttatagc 541560
ggcgtctaac attggaatga agtgtctcgt gacagtcact cgatcacaga atgctaagaa 541620
tccttcccag aaggtttttg aagggagcgg gagttctcca gactctagga tcccttgatt 541680
cgcctgcgta atcagtttaa attctcttga gttaaatcct gctgatacgt ctacagcatc 541740
tgcgaaatcg gcgtggcgac tgaaagttcc aggtctccaa tttgtatgat cgattttcaa 541800
gagcttgcat cttgcagcat aggcatctac aagatcaaac tctctagaga ccatgatcct 541860
ttcagaagca agttgtaaac tacgcatgtc tgtatcccct agatataaag cgcaaatcac 541920
tgtaaaataa cgccctgaag ttccctctaa ttccttacgg aataaaagct gaggactact 541980
atctccgtga caagaaaata tattgaggta ggagtagtcc ttagagttca attcctctac 542040
taatttgcag acttgggttg cgggtaagga gagaactcct ttaaaaacca cgacgacgtt 542100
ttttcccgtt tcatcacgta agaccatcga gaggaaatct ttctgttgta gaggagaaaa 542160
agaatctcca acgtttatag agaccttgag gagttctcct gctgagatat tctgaggatc 542220
taaaaacatc gataatatta aatcctttaa atgttgatcc actagatcta caagatacga 542280
gtggcaatag tgatcctcag atagaggggc gcagagcatt ttttcatcga aaaggaatcc 542340
gagttcctct tcgagactga accatagaca cggttggtaa tgctcattcc cacgagatag 542400
aaatgtgtat agctctttag catcctgccc gactccttgt ttcatcatag agtctacgaa 542460
agcattcgag gtctcatcaa taggaattcc gactgtggat atattgactc tgggagttct 542520
acagtttgga gttcccagga gctgctccgt tttttcgtag ggagaggaca gctacgatct 542580
gtgtgcacgt agatggagag atcgaagggc tcactctctg gaacagcatc ctcgctgaaa 542640
aacccacaac ggatgtgcag attttctaca tgtaattttg agagtacagc actttgattt 542700
acattcttgc ccataaagaa tcttaaatac atttgcgatt cagaatagat acaactcata 542760
taagagatta gccgctctag gatcttggaa ctttctgaat cgagaactcc tatatagacg 542820
atggcaattc tatgtaggtc gggttgccta gcacgactgt attctgtagt cacacggttg 542880
cgtagtggag caaatatacg aaataaattt agagctgtcc cctcagagaa caactgtagg 542940
aacttgagac tttggatatc aaaaaaaagg agctgaaggc agatctccgc gaaccttccc 543000
ctcgtctcta ggctctaatg agagcctttg tctctttaga atatcttcaa ctcgacttct 543060
atggtcgtga cccacgacgt gttctactcg cacgatctga tccacatcgg aaggaaattc 543120
tggctttttt atcctagaga ggagccagtc cagccctaca tacaactgcc caataatagg 543180
aagcaatgca atcactccca aagcaaaatt agaaattcgt gaacacaggg agcgagtaaa 543240
atcaaatgct gcttgaaagt gctgttgcac cttcagatac caaagagaaa agtaataact 543300
cattaaaaat gagtctttta aaaaatactt tactttaaaa aacagaaaaa atctattatt 543360
ttctaataat caaaatcagc aaaaatctat aggaatagag ataagtggct cttatgagta 543420
caagtctccc cgatggtcct ttgtttttat ctctaacaga ccctcggtgt ttcggaagag 543480
ttcctagggc atcccctagg gatataagga ggctctccga tcccaaacat cacaagctcc 543540
ctaaaggcaa acgaccctga gaaccaaaaa ctgctctaag caaagtatgc aaagagcttg 543600
atcctctgac tacttgctgc cattttccaa agtatgatgt acgtttacta aaactcttgg 543660
actcgcattt tattgtgaaa aaaggaaatt aaaatcgtct tgtcttaaat aagataactt 543720
taaactactc ataattagac actataaaac aaattataga caaaaaatct agcattgatt 543780
tattcagaat atttctttct atttgtgaac gagtatgcgc tttttttgct tcggaatgtt 543840
gcttcctttt acttttgtat tggctaatga aggtctccaa cttcctttgg agacctatat 543900
tacattaagt cctgaatatc aagcagcccc tcaagtaggg tttactcata accaaaatca 543960
agatctcgca attgtcggga atcacaatga tttcatcttg gactataagt actatcggtc 544020
gaatggaggt gctcttacct gtaagaatct tctgatctct gaaaatatag ggaatgtctt 544080
ctttgagaag aatgtctgtc ccaattctgg cggggcaatt tatgctgctc aaaattgcac 544140
gatctccaag aatcagaact atgcatttac tacaaacttg gtctctgaca atcctacagc 544200
cactgcggga tcactattgg gtggagctct ctttgccata aattgctcta ttactaataa 544260
cctaggacag ggaactttcg ttgacaatct cgctttaaat aaggggggtg ccctctatac 544320
tgagacgaac ttatctatta aagacaataa aggcccgatc ataatcaagc agaatcgggc 544380
actaaattcg gacagtttag gaggagggat ttatagtggg aactctctaa atatagaggg 544440
aaattctgga gctatacaga tcacaagcaa ctcttcagga tctgggggag gcatattttc 544500
tacccaaaca ctcacgatct cctcgaataa aaaactcata gaaatcagtg aaaattccgc 544560

gttcgcaaat aactatggat cgaacttcaa tccaggagga ggaggtctta ctaccacctt 544620
ttgcacgata ttgaacaacc gagaaggggt actctttaac aataaccaaa gccagagcaa 544680
cggtggagcc attcatgcga aatctatcat tatcaaagaa aatggtcctg tatacttttt 544740
aaataacact gcaactcggg gaggggctct cctcaactta tcagcaggtt ctggaaacgg 544800
aagcttcatc ttatctgcag ataatggaga tattatcttt aacaataata cggcctccaa 544860
gcatgccctc aatcctccat acagaaacgc cattcactcg actcctaata tgaatctgca 544920
aataggagcc cgtcccggct atcgagtgct gttctatgat cccatagaac atgagctccc 544980
ttcctccttc cccatactct ttaatttcga aaccggtcat acaggtacag ttttattttc 545040
aggggaacat gtacaccaga actttaccga tgaaatgaat ttcttttcct atttaaggaa 545100
cacttcggaa ctacgtcaag gagtccttgc tgttgaagat ggtgcggggc tggcctgcta 545160
taagttcttc caacgaggag gcactctact tctaggtcaa ggtgcggtga tcacgacagc 545220
aggaacgatt cccacaccat cctcaacacc aacgacagta ggaagtacta taactttaaa 545280
tcacattgcc attgaccttc cttctattct ttcttttcaa gctcaggctc caaaaatttg 545340
gatttacccc acaaaaacag gatctaccta tactgaagat tccaacccga caatcacaat 545400
ctcaggaact ctcaccttac gcaacagcaa caacgaagat ccctacgata gtctggatct 545460
ctcgcactct cttgagaaag ttccccttct ttatattgtc gatgtcgctg cacaaaaaat 545520
taactcttcg caactggatc tatccacatt aaattctggc gaacactatg ggtatcaagg 545580
catctggtcg acctattggg tagaaactac aacaatcacg aaccctacat ctctactagg 545640
cgcgaataca aaacacaagc tgctctatgc aaactggtct cctctaggct accgtcctca 545700
tcccgaacgt cgaggagaat tcattacgaa tgccttgtgg caatcggcat atacggctct 545760
tgcaggactc cactccctct cctcctggga tgaagagaag ggtcatgcag cttccctaca 545820
aggcattggt cttctggttc atcaaaaaga caaaaacggt tttaagggat ttcgtagtca 545880
tatgacaggt tatagtgcta ccaccgaagc aacctcttct caaagtccga atttctcttt 545940
aggatttgct cagttcttct ccaaagctaa agaacatgaa tctcaaaata gcacgtcctc 546000
tcaccactat ttctctggaa tgtgcataga aaatactctc ttcaaagagt ggatacgtct 546060
atctgtgtct cttgcttata tgtttacctc ggaacatacc catacaatgt atcagggtct 546120
cctggaaggg aactctcagg gatctttcca caaccatacc ttagcagggg ctctctcctg 546180
tgttttctta cctcaacctc acggcgagtc cctgcagatc tatcccttta ttactgcctt 546240
agccatccga ggaaatcttg ctgcgtttca agaatctgga gaccatgctc gggaatttcc 546300
cctacaccgc cccctaacgg acgtctccct ccctgtagga atccgcgctt cttggaagaa 546360
ccaccaccga gttcccctag tctggctcac agaaatttcc tatcgctcta ctctctatag 546420
gcaagatcct gaactccact cgaaattact gattagccaa ggtacgtgga cgacgcaggc 546480
cactcctgtg acctacaatg ctttagggat caaagtgaaa aataccatgc aggtgtttcc 546540
taaagtcact ctctccttag attactctgc ggatatttct tcctccacgc tgagtcacta 546600
cttaaacgtg gcgagtagaa tgagatttta acaataagtg accaaaacag aaagattaag 546660
gaacctctag tgtcaaagac tcctcctaag tttttattct atctcgggaa tttcacagcc 546720
tgcatgttcg ggatgactcc tgcagtgtat agtttacaaa cggactccct tgaaaagttt 546780
gctttagaga gggatgaaga gtttcgtacg agctttcctc tcttagactc tctctccact 546840
cttacaggat tttctccaat aactacgttt gttggaaata gacataattc ctctcaagac 546900
attgtacttt ctaactacaa gtctattgat aacatccttc ttctttggac atcggctggg 546960
ggagctgtgt cctgtaataa tttcttatta tcaaatgttg aagaccatgc cttcttcagt 547020
aaaaatctcg cgattgggac tggaggcgcg attgcttgcc agggagcctg cacaatcacg 547080
aagaatagag gaccccttat ttttttcagc aatcgaggtc ttaacaatgc gagtacagga 547140
ggagaaactc gtgggggtgc gattgcctgt aatggagact tcacgatttc tcaaaatcaa 547200
gggactttct actttgtcaa caattccgtc aacaactggg gaggagccct ctccaccaat 547260
ggacactgcc gcatccaaag caacagggca cctctactct tttttaacaa tacagcccct 547320
agtggagggg gtgcgcttcg tagtgaaaat acaacgatct ctgataacac gcgtcctatt 547380
tattttaaga acaactgtgg gaacaatggc ggggccattc aaacaagcgt tactgttgcg 547440
ataaaaaata actccgggtc ggtgattttc aataacaaca cagcgttatc tggttcgata 547500
aattcaggaa atggttcagg aggggcgatt tatacaacaa acctatccat agacgataac 547560
cctggaacta ttcttttcaa taataactac tgcattcgcg atggcggagc tatctgtaca 547620
caatttttga caatcaaaaa tagtggccac gtatatttca ccaacaatca aggaaactgg 547680
ggaggtgctc ttatgctcct acaggacagc acctgcctac tcttcgcgga acaaggaaat 547740
atcgcatttc aaaataatga ggttttcctc accacatttg gtagatacaa cgccatacat 547800
tgtacaccaa atagcaactt acaacttgga gctaataagg ggtatacgac tgcttttttt 547860
gatcctatag aacaccaaca tccaactaca aatcctctaa tctttaatcc caatgcgaac 547920
catcagggaa cgatcttatt ttcttcagcc tatatcccag aagcttctga ctacgaaaat 547980
aatttcatta gcagctcgaa aaatacctct gaacttcgca atggtgtcct ctctatcgag 548040
gatcgtgcgg gatggcaatt ctataagttc actcaaaaag gaggtatcct taaattaggg 548100
catgcggcga gtattgcaac aactgccaac tctgagactc catcaactag tgtaggctcc 548160
caggtcatca ttaataacct tgcgattaac ctcccctcga tcttagcaaa aggaaaagct 548220
cctaccttgt ggatccgtcc tctacaatct agtgctcctt tcacagagga caataaccct 548280
acaattactt tatcaggtcc tctgacactc ttaaatgagg aaaaccgcga tccctacgac 548340
agtatagatc tctctgagcc tttacaaaac attcatcttc tttctttatc ggatgtaaca 548400

gcacgtcata tcaataccga taactttcat cctgaaagct taaatgcgac tgagcattac 548460
ggttatcaag gcatctggtc tccttattgg gtagagacga taacaacaac aaataacgct 548520
tctatagaga cggcaaacac cctctacaga gctctgtatg ccaattggac tcccttagga 548580
tataaggtca atcctgaata ccaaggagat cttgctacga ctcccctatg gcaatccttt 548640
catactatgt tctctctatt aagaagttat aatcgaactg gtgattctga tatcgagagg 548700
cctttcttag aaattcaagg gattgccgac ggcctctttg ttcatcaaaa tagcatcccc 548760
ggggctccag gattccgtat ccaatctaca gggtattcct tacaagcatc ctccgaaact 548820
tctttacatc agaaaatctc cttaggtttt gcacagttct tcacccgcac taaagaaatc 548880
ggatcaagca acaacgtctc ggctcacaat acagtctctt cactttatgt tgagcttccg 548940
tggttccaag aggcctttgc aacatccaca gtgttagcgt atggctatgg ggaccatcac 549000
ctccacagcc tacatccctc acatcaagaa caggcagaag ggacgtgtta tagccataca 549060
ttagcagcag ctatcggctg ttctttccct tggcaacaga aatcctatct tcacctcagc 549120
ccgttcgttc aggcaattgc aatacgttct caccaaacag cgttcgaaga gattggtgac 549180
aatccccgaa agtttgtctc tcaaaagcct ttctataatc tgaccttacc tctaggaatc 549240
caaggaaaat ggcagtcaaa attccacgta cctacagaat ggactctaga actttcttac 549300
gaaccggtac tctatcaaca aaatccccaa atcggtgtca cgctacttgc gagcggaggt 549360
tcctgggata tcctaggcca taactatgtt cgcaatgctt tagggtacaa agtccacaat 549420
caaactgcgc tcttccgttc tctcgatcta ttcttggatt accaaggatc ggtctcctcc 549480
tcgacatcta cgcaccatct ccaagcagga agtaccttaa aattctaaaa taaaagaacg 549540
ataaaattga aatctttaga attaacaact atccgatgag ctacgttagc ccaatcggta 549600
gaggactccc tcaaaattta aatatagaaa atcattcaaa tatatgagtt tactaactct 549660
gtaatattca acatgttaat aagcatattt aaatataaat ttataaactt ctagacaaca 549720
aattgatgat tttttatgac aaactctatt ttcatatcaa agtttggatg tttatgcgac 549780
ccatttgtct cagcatttta tccactgcgc tatgttgttc cttatcagga aatgaagtcc 549840
ctaacctcgc ctcttgtcag atgtctagaa aagacatctc tgctttccac acgtctccaa 549900
gcttccgtct gaatgtaact ccagagccct tggtttcctc ctttcgtccc tctaatcttc 549960
ttaatggatt cggtcacgat ataacccagg acatcacaat tacaggaaac tctatcaatt 550020
ctgttataga ttataactac cactacgagg atggaggcat tcttgcatgt aaaaatttgt 550080
tcatttctga aaataaagga aacttaagtt ttgaaaggaa tagctcccac agttctggag 550140
gggctctcta cagtgttcgg gaatgctgga tttctaagaa tcagaactac tcgtttattt 550200
caaatgcggc ttccttagct actactacaa cttcaggatt tggtggggct atacatgcac 550260
tagatagcta tattacaaat aacttaggag aaggacaatt cttagataat gtctctaaaa 550320
atagaggagg agctatctat gttggggtga gtttatcaat cacagacaac ttaggtccta 550380
tcgttatcaa gaaaaatcaa acattagaag attccagctt tggaggaggc atcttctgca 550440
gagccgtaaa tatagaaagg aattatcaaa acatccaaat caatgataat gcttcaggac 550500
aaggggtggt atattttctg ccctaggagt cattatctct tcaaataaag aaattataga 550560
gatcagcaat cactccgcat cctcaattaa cacagcatca ggaaaactat atcccggtgg 550620
tggcggtatc atgtgtacct ccttagtcat tgagaacaat cccaaaggtc ttatctttaa 550680
caataaaacg gcagcactta gcggcggagc tatacacacg agatctttca tcttccaaaa 550740
taacggtccg acagcattta ttaataactc tgcgacttca ggaggggctc tcatcaatct 550800
ttctggtata ggaagtactc ctcaaaattt cttcctctct gcagactacg gcgatattct 550860
atttaacaat aatacaatca catcttcttc tcctcaaccc ggatatagaa atgcactcta 550920
tgctgctccg gggattaact taaaactagg agcaagacag ggttataaaa ttctctttta 550980
tgatcctata gatcacgatc agacgacaac agatcctata gtatttaatt atgaacccca 551040
tcaccttggc accgtgttgt tttccggaat caatgtagat tctaacgcaa caaatccatt 551100
gaacttccta tcaaaatttt ctaactcttc acgacttgaa aggggtgtgc tcgctattga 551160
agatcgggct gctatttctt gcaaaaccct atcgcaaact gggggcattc tacgtttagg 551220
aaacgcagca ttaatcagga cgaaaggccc gggaagctcc ataaatttta atgcaatcgc 551280
gatcaatctt ccttctattt tacaatcaga agcctcagct ccaaagttct ggatttatcc 551340
tacattaaca ggatccacct attctgaaga cacttcttct actatcactc tctcaggacc 551400
cttgactttt ctaaacgatg aaaatgaaaa cccctatgat agcttagatc tctctgaacc 551460
tcgaaaggat atcccccctc ctctacctcc tcgatgtgac tgcaaaaaaa atcgatactt 551520
cgaatctcat tgtagaagcc atgaacttag atgagcacta tggatatcag ggaatctggt 551580
ctccctattg gatggaaact acgactacaa caagctctac agtaccggaa cagaccaata 551640
caaaccacag gcagctctac gtagactgga ctcctgtagg ataccgccct aacccggaac 551700
gtcacggaga atttattgct aataccttat ggcagtctgc ctataacgct ctgttaggaa 551760
tccgcatctt acctccacaa aacctcaaag agcatgacct tgaagcctct ctgcaaggac 551820
tcgggcttct aattaaccaa cataatcgcg agggacgcaa aggcttccga aaccatacta 551880
cgggctatgc agcaacaacc tcagcaaaaa ctgcagcacg acatagtttc tctttaggat 551940
tcgcacaaat gttctccaaa actagagaac gtcaatctcc aagtacgact tcctcccaca 552000
actactttgc aggactccgc ttcgacagtc tcctcttcag ggacttcatc tctacagggc 552060
tatccctagg ttatagctac ggagatcacc atatgctttg ccactataca gaaatcttaa 552120
aagggtcgtc caaagccttc tttaataacc acactttggt agcctctcta gactgcacat 552180
tcttaccagc tagaatcacc cgcactctcg aactccagcc ctttatcagt gccattgctc 552240

tgcgctgttc ccaggcctcg ttccaagaaa ctggagacca tataagaaaa ttccatccaa 552300
aacatcccct tacagatctt tcctctccca taggcttccg ttctgaatgg aaaacttcac 552360
atcatatccc catgctatgg actacggaaa tatcctacgt acctacccta tacagaaaaa 552420
atccagaaat gttcacgaca ctactcatca gcaatggaac atggacaaca caagcaactc 552480
ccgtctccta taattccgta gctgcaaaaa taaaaaatac ttcccaactt ttctcaagag 552540
taaccttatc cttagattat tcagctcaag tctcctcgtc aactgtaggt caatacctta 552600
aagctgagag tcattgcaca ttttaaccac aaagaaaaca tcaaggaata aacagtgcaa 552660
aataacagat cccttagtaa atcttccttc tttgttggag ccttaatttt aggtaaaact 552720
acaatactcc ttaatgcgac tccgttgtct gactattttg ataatcaagc aaatcaactc 552780
acaacactct tccctctaat tgatactctt actaacatga ctccctactc tcatagagca 552840
acactttttg gagttaggga tgacactaac caagacattg tcctcgatca ccagaattcc 552900
atagaaagct ggttcgaaaa cttctctcaa gacggcggtg ctctctcttg caaatcactt 552960
gccataacga atacaaaaaa ccaaattctt ttcctaaata gctttgctat taaaagagct 553020
ggtgcgatgt atgtgaatgg taatttcgat ctttctgaga atcatggttc catcattttc 553080
tctgggaatt taagctttcc taatgcaagt aatttcgctg atacttgtac agggggagct 553140
gttttatgtt cgaaaaatgt tacaatctca aaaaatcaaa gaaccgcata cttcattaac 553200
aacaaggcaa aatcttcagg aggagcaatc caagctgcaa tcataaacat taaggacaac 553260
actggccctt gcctgttttt taataatgct gcaggcngaa cagcgggggg cgcgttgttc 553320
gctaatgctt gtagaattga gaataattct cagcctatct attttttgaa taaccaatca 553380
ggtctgggtg gtgcaataag agtacatcaa gagtgcattc ttacaaagaa taccggttct 553440
gtgatcttca acaataattt tgccatggaa gcggacatct ctgctaacca ttcctctgga 553500
ggggctatct attgcattag ttgttctata aaagacaacc caggaattgc agccttcgat 553560
aataatactg cagcacgaga tggaggtgct atctgtacac aatctctaac tatacaagac 553620
agtggtcccg tctatttcac aaacaatcag ggaacttggg gcggcgctat catgctccgt 553680
caagatggtg catgcacttt atttgctgat cagggagata ttatttttta taataataga 553740
cacttcaaag atactttcag caatcatgtt tctgtaaact gcacgcgtaa tgtctcatta 553800
acagttggag caagtcaagg tcattctgct accttctatg atcccatact acaaagatat 553860
actatacaaa actctatcca aaaatttaat cctaatccag aacacctcgg aactatcttg 553920
ttctcctcag catatattcc ggatacatcg acttctcgtg atgacttcat ttcacatttc 553980
agaaaccaca ttggactgta caacggcaca ctcgctcttg aagatcgagc agagtggaaa 554040
gtctataaat ttgatcaatt tggtgggact ctacggttag gcagtagagc tgtgttttct 554100
acaacagacg aagaacaaag tagcagtagt gtgggttctg taattaacat caataatctt 554160
gcaattaacc ttccctctat cttaggcaac agagttgctc ccaagctatg gattcgcccc 554220
acaggttcat cagcacccta tagcgaagat aataacccta taatcaatct ctcaggacct 554280
ttgagcctac tggatgacga gaacctagat ccctatgata ctgcagacct tgcccaacct 554340
atcgcagaag ttcctcttct gtatctctta gacgtcacag ctaaacatat taatacggat 554400
aatttctacc ctgagggtct aaatacaact caacactacg gctaccaagg cgtttggtcc 554460
ccttactgga tcgaaacaat cacaacttct gatacctctt ctgaagatac tgtgaatact 554520
ttacatcgcc agctttatgg tgattggaca cctacaggat ataaggtaaa cccagaaaac 554580
aaaggagaca ttgccctatc tgccttctgg caatctttcc ataacttatt tgcgacacta 554640
cgttatcaaa cacagcaagg ccaaatagca cctacagctt ctggagaagc tactcgactc 554700
ttcgtgcatc aaaatagcaa caatgatgcg aaaggattcc atatggaagc tacgggttat 554760
tctttgggaa caacctcaaa cactgcttct aatcatagct ttggtgtaaa cttctcccaa 554820
cttttcagta atctctacga gagccactcc gacaattccg tggcttcgca tacgacaact 554880
gtagcgctcc agatcaataa tccttggctg caagagagat tctctacatc tgcatctcta 554940
gcctacagct acagcaacca ccatatcaaa gcatctggat attctggaaa aatacaaacg 555000
gaaggcaaat gttatagtac gacattaagg ggcggctctc tcttgctctc tatctctaca 555060
atggcgatca cgacctctcc acttcactcc ttttatccaa gcaattgccg ttcgttctaa 555120
tcaaactgcg tttcaagaaa gtggagataa agctagaaaa ttttctgttc ataaaccctt 555180
atataacctg acagtccctc tgggaattca gagcgcttgg gaatccaagt tccgtcttcc 555240
tacctattgg aacatagagc ttgcttatca gcctgtcctc taccaacaaa atcctgaggt 555300
caacgtgagt ctagaatcta gtggatcgtc atggctccta tcaggaacca cccttgctcg 555360
caatgccatt gcttttaaag gaagaaacca aatttttatc ttccctaaac tttcggtgtt 555420
cttagactat caaggctcgg tatcctcatc aacgacgaca cattaccttc acgcaggaac 555480
gacctttaag ttttaaaagc atgttatata gacaatgcaa cctgtaaaga ccaaatagag 555540
agtagtgaac actctctacc atcatgaatc ttatgggaga agctaaggga aatccacaga 555600
tacgtttccc ccataaaaat taagaacccg atacatcctc actagagatt cgaaagaact 555660
acttaaatcc taagcattcg actctccacg aggccatcct ttttgtagta agattttcgt 555720
tgtagctaca agtcccccta agggctctag atattctgat tctccgtccc ccggctctgt 555780
tggaggaaca atactctctg cttcgaccat aacagcactc acgctctcgc taagcaaagc 555840
ggctcttact tctggtccaa accctgggct cactctaagc gaaacattag tcaatgtatt 555900
ctcgggtctt ggaagtgtag cagggggcct gccatgaatc catgggatct catcataaat 555960
aggctctaca ggagaagcgg aagcaccgcc tgacctctcc tgtaaaattc tatctatatt 556020
tctctgagta aatggattat cttcaggagt attagcatat atgggttcat ccaagtcgcc 556080

tctaggagag ggggaacggc tcatatcttc ataatcactt tcacctgcac ctgaagcacc 556140
gctcatatcc tcataaatgc tcccctcctc actcatagca ggtgaaggaa ctggaggaag 556200
aggcacaccc cgtctatttg gtgagctgct tcgtacgcga tctgaagacg gagaacgtga 556260
gcttccagac gatcctggac gtgaaggaag atcatagata gcaggagttg ctaatctagg 556320
tgtcatgtag atgttcttgt ctttagatgg ttcagcagag gttataggaa cttcataatc 556380
tccaaaaaca tcctcttcca actctcgcgc aggactccat ctaggggaac taggaacgtc 556440
atagattcca tcttttctac gaggaacatc atagaaaata tgatcatctt ccataacaat 556500
aaaactgacg gaactgtctt gactccaacc tctcactatg ggagcagaaa tcgaaatttc 556560
cggggtactt gattctgaac tctccttggt tttagcaccg tgcttatgtg cccatcctac 556620
taaggcattc atcaatggag aatcttcacg tggacttcca tttctgcgtg gaacgtcatg 556680
cggagagccc gttctttcag tttcatcaat ttctgaagga gaccccaccg acatcgagtc 556740
ctcttcagaa gaagtacact cttctcctcg gcacgtgcaa caacgaccca ctgcttcagc 556800
agctgcatga cgagcaatac gcaacctgtg caagatggac cctagaatgc ttaaaatcat 556860
tcctaaaaga gaaaccagag cctcgcgaat ctgattccat aatcttgtag accagttttc 556920
ccgagttctt tgaggatctg cagctcctcc gatttcctca taaataggct cttcttgaga 556980
aatcagagtt ggattattgt ctatcggagt tggattatcg tctaaagaaa aactcaatcg 557040
agcatgggac aaagcagctt ctaaatcgtc ggcagtctca tctaataggt tttgaagaga 557100
gtctccatca ctcatgagtt cttgaagctc aggatctgtc agttctgaac agagggaatt 557160
gatctcatca ctagtttcta cccccttaaa acgagcttct gctagagata ccgaagattg 557220
tgttgcctcc atatagccgc attggaattc tgtaatttgg atcagctgtt gtatggtaac 557280
tgtggcttct gctttttcct ctggagtcaa ctgatcatta ctttgtatgt cagagagctc 557340
ctgacgtaaa ctactcaaac ggaggtttag ctcgtagaga cttaaaggct cgctgtctgt 557400
aggtggagcc ccactaggaa gtactatgcg atcgcgaact gtctgtacca accgacccaa 557460
accgctctgt ttttttactg aaggaagaga aaataaacgc tgaggttctg gtccagaaga 557520
ggtttctgga accccctgag aaggattttc gcctgaaaca ctcgcgtcct ctgccactcg 557580
ggcttctgta gcagcagcac ttgatgaatc gacatcccgt ctctcatccg cggtgatggt 557640
ttttgataat ctaacaaaag cttgtcgcgt ttcacgcgct tgactcgtag actctgcgga 557700
aaaaaagttg ccaaatgatt ttaaggccct gtctacccca gaacgtacgc gagaaaaaaa 557760
tcccgggcta gaagctgccg aagaagagct ctgagactcc tgatcttctg gtagaaaaga 557820
ggaactgcta tgtatatgcg aagatcctga agctccttcc tctccatgtt cttgaggagg 557880
cagggaaatc tcgtggctgc caagttctcc cttaggagag ggcgatcgac ttctatcccc 557940
attatcttta ggtggaatct ttcctaatcc actagatcct ccgattcctg atgccataaa 558000
cacttcctaa aaagaataat cttttttctg aaacaattta attttattaa aaaacaacaa 558060
aaatgctttt aaatatatta aaataatcaa catattgaag agttttaatt aaaactcttc 558120
aagtttccct tgcgcataga gaaacagtga gataacgact actgtccctc tggagcaaat 558180
gatctagcta atcctaagcc ttgttgaatg acaggatcag tcacattgcc tgattccaat 558240
ttttcaagac tctgggcatt tctgttactc atttctgcag ctctacgagc gatctcttcc 558300
gcaaaagata agctaggtgc agccgatgga tttgaaacgc cctcttgctc cacaaaccat 558360
tgtgtattac tttctgatgc aagatcatct acagctgatt cacgtcgtgc tgaacttaat 558420
cttgaagcaa ttgccctaaa gaagctcgct accgcttgga atatgctaac tacaaaatcc 558480
cgtaacgctt tccatgctcc ctgcgctgca gatccctcta ccccacctgt ttcgggagct 558540
tccgcagtct ctgctggggc ttgcgatcca tcttgaggta cgatggatgc atactccaca 558600
gaatcactac ctacattgga ttctacgaaa gtaataagat cctccacagt aggaggttcc 558660
tgatgagtca attgctccat gcgtgcaaga agagattgca ccctagaagc cacctctggt 558720
gacacttgtt ctggagctac tttttccaaa ttttctaaag ccttacgcag atgttttgct 558780
gctttattca cattagaaag atcaccttcc gatgaagaac gttggaatct agttctgata 558840
ttccctggtg ctgatctgat tgcacctgca gcctgctctc cgaacgccga aaccttctct 558900
ttagcgtcac ctaaggctcc ctgaactgta gacgtcgctc ctcgaactcc tgataaaaaa 558960
gaagaactct tcgaagaagc ctctgcttct tcggaacgcc tttcttttac agtatcgccg 559020
gcagaatctt cgccctgagt tgttaaatca tcttcggaag cagaacgctg gaactttcct 559080
ttaatatcac ctagggcacc atgaacttta gacaccgctc ctcgaactcc tgataagaag 559140
gaagaactct ttgaagaagg ctctcctcct tcagaacggc tatcctctgt agaatcttca 559200
ttagaatcgt gtcctactcg atcaaggtca ccatcagaat aggaacgttt catcttccct 559260
aaatctgcct ctggattttc agatcttttc tggatacgat ctctaaagcc ctcgagtcca 559320
ggaagacgtt ggccatgttg cacatgggat ggaagacgtg gactagaaac ttcaggacgc 559380
gcttgagggg tttttttaaa gaccccttta acgcctttcc aaattctctg aagtacccca 559440
gaacgtcctg aagttggaga atctccgggt tctttgacag atggcaaagg aattttatca 559500
gtcccgccta actgggcttt tctctcttct attgtagggc cctgacttgc aagactactg 559560
tcactactgc taactgtatg ttcccctaag ttgtctttag gacttacttt tccatcctca 559620
ctattccttc tattaggagg aatgggggaa ggagatcttg agccgcctac gccaccaact 559680
gccataattg aaccttaaaa tgataaatta tttaataaaa actattaaac aaatgttaaa 559740
ataaaaaata ttttaaaaac aaaatataaa ttaaaataaa caaaaagaat gctgattata 559800
taattaagta aactactaga tgtaaaagag aaaataaaat tagagaaggc agacaagctc 559860
ccaaagctcg aggatcttac attcaaggac ctggcctaag cagctccatc tatatctaca 559920

tccacatcaa taaaatctac gggaccatca agatcaaata gatgatcgtg gctagcagca 559980
agagcctcaa caactaaatc cgtagcaatg gagcgcattt tactttcagg accatagaga 560040
agctcacggc gattacgtcg acgctcatag agcttcacaa gctcatcata gagccagttg 560100
acaaacttcc tcattaaaat cgatcccgcg attcctaacg tcgatcctcc aagagcaaga 560160
ataccaggaa cgataggagc aaatgcaggg accagggcaa gaagcatgat cccagttcct 560220
acaccaagta agattccacc aacaagggcg ctgttcactt ccccaccctt ctcaaaattg 560280
cgaacctctt cacgaattgc agcgtcagtc ttctgaatct gttttttgta gactgtcttc 560340
cacatttggt taaattctcg atccgaggca atcactccag actcaattaa gaactggcgc 560400
acccgctttt tattttttgc ttctttccaa cgtaatttac tgatattcaa tcttgctttc 560460
agttggtagg cgacgtagga aaatcctgag gaaaccatag ggagtgttac agaagcaacc 560520
gccgatccta cagaagcaac aaggagaact tgcatgactg tcaatccacc aaatcccgtg 560580
actatgaagg cagcaatact tgctaagagc aagagtgtcg caagagcaaa gatgaccttc 560640
tgtcttgtcg tcagggaaag cctcgtatcg atttcttccg cagagatcct cgtctctgca 560700
atcccctctg aatctggaac ttctatcaca gattgggaag tttcactctc ttccacctca 560760
ctggggggca atttttctaa gcgaatgtct ttcagggagg tgtctacaaa aagatctttt 560820
ttttcttgag taagcagaaa ggctctccac tttccgatac tactatggag tatcgtttgc 560880
aaaaccgtcg taagccctat agaaattaaa gggggagcta aaataatagc aatcgttgcg 560940
ggagcgcctc ctagcaatac cgttcctgca atggcaagga gaatccccaa aaccgcaaga 561000
ccaacgccaa tagtggcaat tgacaacgtg atatagtgta attttttcgc ctcttttata 561060
gtttctgctt ctgtaagctt ctccccttta gcaacttttt cagaacggaa aaacttagta 561120
taaatataat gactgaagct tgctaatgtg gtttggcttc cccgggaata cgcactggat 561180
cccaagctac ccacgagacc cccataccct ccaggaacaa aattcgcccc caccttcatg 561240
accaaaccca tgacagtaaa tccagtccct acagctaaag cacttttgat tctgctcgac 561300
tcctgcatcc tgggggacat atagtctcgg accatataaa ccaagctctt cattatagaa 561360
aatccaccaa tacccacgcc tgaaatggcg attccaagaa gaatagcctg aggaactccg 561420
ggagctaaga cggtcaaagc gacaacaaca acagaaaccg ctaccagcaa aacaaccagg 561480
ccagcaaata aagtcacgga atgggtgaaa atactttttc gcaatcctgt atcttcagga 561540
ttgctaggaa ctagggaatt tggtactggc gagagactcc ggctatttgc aagaagagaa 561600
gttttctcag ttgcattaga caaagacaaa tatcggggat cgttgctaat tggtgatgta 561660
gacataaaag aaatactaat aattattttt aattataaaa taaattatta atatcaacta 561720
caccacaaaa tagaacaatc ccacaacaat ttacaaataa agtatttaaa tttttagaaa 561780
aaagtaacta aatagatcac agtagctaaa ggagggagct ctatatccaa accccaagcg 561840
accccttggt cttgacagac cacaggagcc cgatttccct tccctgagcc tccaaaagac 561900
tcatcatcag tgttgagaag gagttcacaa tgctttacac cttcacacct taaaacatag 561960
gaaggaaaag tactcgcact gaaatgatgg acacagagaa gcgccgaaga acgattgctg 562020
cctgcaaatc tataataggc aatgacattg ttttctatat catggaagtc tacccaatgg 562080
aagcactctt gagagctctc ttgcatccat aaatagggtt ggtgaatata caacgcattc 562140
aatgcagaga cacagtttcg caaagttttg tggtagtgat gattcaaaag ctcccaatct 562200
aagggacgat caggagacca ctcgccgtat tgtccgaatt ccccacccat gaacagtaac 562260
tttttcccag gcaaacagat ctggtagctc aagagcactc tcatttgagc aaatcgggtc 562320
caggtatccc cgggaagctt attcactaag ctgcccttac cgtggaccac ctcgtcatgc 562380
gagagaggaa gaataaaaga ctcttggaag gcataccaaa ggctaaatgt cagatctttc 562440
tgatggtatt tacgatacat gggatccttc ataaagtaat gaaaggtatc gtgcatccaa 562500
cctaagttcc atttgtaatc aaaccccaga cctccctgat ctacgtcctt agtgactcct 562560
ggaaacgctg tggattcctc tgcaaaggtg agcactccag agaactcctt atgaattaca 562620
gaatttaagt gtttcaaaaa ttctatagac tctaagttct ccttacctcc atagatgtta 562680
ggcgtccatt ctccatcttc acggccataa tcacgataca gcatagaggc cacagcatcc 562740
acacgtaagc catcaatatg catcttatcg agccaaaata aagcactccc tagtaaaaag 562800
ttggtcactt catgacgact gtagtcaaag gtaaacgtat tccagtgggg atgaagagcc 562860
tgactatgcc ccgtgtactc gtagagaggc tccccatcaa aagaggcaag agcaaacgca 562920
tctacgggaa aatgtcccgg cacccaatct aaaataatac caatattttc tttatgtaga 562980
tagtctacaa aatactgaaa ctcctggaga gtcccgtatc ttgatgttgg agcataatat 563040
cccgtcactt gatagcccca agattcattc aggggatgct ccgtaatggg aagaagctcc 563100
acatgagtgt agtgcatttc cttgcaatag ctagcaaggc gatgcgccat ttcgctgtag 563160
cttaagggcc ttccctcctg ccattgccaa gagcctaagt gcacttcata gatcgtgacg 563220
ggcccttcac tctgcttcga gcgcctctcc atccaacgat gatcactcca agagtagctc 563280
tcagaatccg caacacgagc tgtaccctgg ggtggaggat caaagctctt cccataagga 563340
tctgttttta caatcacatt ccccgattgg gtaacgattt cccacttata ccgtattccc 563400
tctcccaagc ctgggacgaa aagctcccag atcccctgat cggaaatttt acgtagagga 563460
ttgacaaggc catgccaaaa attaaaatct ccgactacag agactctctg cgcatgggga 563520
gcccaaagaa caaagagcac ccctgagatt ccttgaactt ccatagggat tgcccccatg 563580
cgttcataaa tgcggtaatg cgttcctcta tggaataaaa aagaatcaat ttctccccac 563640
agaggaggaa acgcataggg atcatgagcg agaagtccat tctgatgata cacacggtaa 563700
tccccgtgtc cgattccttt gggaacggat aagaaaaaga gccccgaacg ataagctaca 563760

gcgtggtgaa gctctcctag aagttcaata gcaaccgtat gcgcccctgg acgaaaaata 563820
acaatatgat ctgaagaatc ttcagaagca aggatcccta agagtttatg gggatctttc 563880
tgtcgtcctg agacgagcag atcaagatcc caaggatgga tcagtttatc aaccatggaa 563940
ggctatctat ttatgaaaac aatcccataa atatgtggtt cctgctttta tctccagagt 564000
ttctcctaaa gaaagtctct tagatccaga gagacgtcgc tccgaccatt cccgaaggcg 564060
cgcactgcat agtgaagaac aaaactttaa aaatacctcg ccactatatt ctgcatggag 564120
ctcgacctga cggatagttt tcttagtcca gacgatagac aaagtcccaa gattaggaag 564180
ggcaacatga atcaagcggc cacaaggaaa ttcaggaggt aatgcaggaa ggatctctac 564240
tagctgtccc tggtgtctca gaaaaatatc ttggatcata ccaaagctat acgagagaag 564300
ctcaaaagga actcccccct ctggatctcc atcttggggg aggattcctt ggaactcttc 564360
atcatagctc ctaggaagaa agcactctga aaatcccgca agggctatgg agagtaacgc 564420
aggagcaatc tctgtagttt ccctatgtaa aactctttga tgggcagtct ttaccaagct 564480
accgatagcc gtcgtctcta gatcccgata ggatccctta ggagccatag cggcgaaacg 564540
gaaccatagg ggaagaattt ctttaagatc acgacgacaa cggacctttt gccaatctgc 564600
atgcttatgc accccaagag ataacagggg ccctgcttgc gctgccgaag gcagtttccc 564660
tttgatagat tgtgtgcact ctcccgaggg aaggagataa tacttgtagc gctcagaagt 564720
cactgcaagc cccccacgat gtaaatcttg gaaaacagca aagcgtttta aaggcccttg 564780
cacctgtaag gtctgctcat ttaagatttc gtgtttcgaa gaaaatagac gccacagggt 564840
aggaaatacc tgagcatata acaaagatcc aggaataggg agtttttgtc ctgggaccat 564900
agtatagggc ttaaaatgtt gcattatatc tatcatacaa tctccccaat cagcccttct 564960
tcctctaccc tatcaagacg cacagaaact agagtgttga tagctaccgt tcctacaaca 565020
gggaaagaaa ccttttcaaa ataaggagag tgacccgtag caacctgccc cgttactttc 565080
tcaacaagca cctctgtagt ctctcctaaa cgcttcatca tctctttctg gcctaccctc 565140
ttagcaacct cagcaagata cttcttcctc tcatagatca cctgattggg aatctgatta 565200
tcaaaagtat atgccttagt acgacgacga gcactgaaag ggaaactatg cactttaata 565260
aagcctacat cttcaataat tctcaaagta tcttcaaaat cttgatcact ctctccagga 565320
aatccgacaa tcacatctgt agtaaaggca tagcgaggat cagaagcacg gaacttctct 565380
acacaatcta aaaaatctcc gcgagaatac ttccggttca ttctctttaa aattgaattc 565440
gaccccgatt gaagaacaag gtgtgacgaa ggacaagtgt gacgcgatga ggtgatggca 565500
cggtgcagat cttcagtgat atcatcagga tctatagagg aaattcgaat cctctcaatt 565560
ccaggaatct ggtccacctg ttcaatcaaa gaggctaatg aacgctctcc atcgcaataa 565620
tctccaacat taattcctgc aattacaact tcgcgatatc cttggtctac aacccctgcg 565680
atttcagcta aaatcttctc agcaggacga gaaaccgaac gcccccgcaa ataaggaata 565740
atgcagtacg agcaaaaaga attacagcca tcttgaactt taataaaagc tcgagacttt 565800
ccctcaaaac tatggatctt gaactcaggg aaggtcgtat cataggaaaa aattttttct 565860
ataagtcggg atttttcttt attggaaaca agcgtgcatt gccgatccaa agaagcaaaa 565920
aactctttgt cagattcccc caaacaacct gtgacaacaa tatgtgctgt agggttctga 565980
cgacataact gacgcacagc atgacgaccc gaactctcag cagaagctgt gacagcacac 566040
gtattgatta tgcataaatc tgcagggatt tcagaatcca ggacctcttg gtaacctaag 566100
atagtcaact ggtcgcgata tgcttggacc tcatactgat tcacccgaca gcctaaacag 566160
accagcttaa atgttccttt gacttccgca accgtcatat acctctagag acttcaatag 566220
atttagcgat gagtggctat tttaaaatat cactcttttt ctcactaaca aagattcgat 566280
gtcctagaag aaaactacct aatccccgaa agaactctcc aagacggccc ctctaccctc 566340
gtcatctcca caatcttcag ggaaaacata agaaataaaa ggacgaaatt tcttttgcag 566400
ctccttagga agatacgctt caacaactaa aaaatcttct tgataccttg acgaggccac 566460
aaccccggca tcgcaaagtt ccgtaaattt tccatattct gtataaggaa aattcaaagt 566520
cacatgcaaa cttttctcct gaatgatttc cgtcataaga ctaagaagat tctggatccc 566580
ctccccagtt tttgctgaaa tcaatacagg aagaggagag agcaaacgta atttcatagg 566640
gatacttcct tgaggaagcc gatctacctt attcaacaca gtaatgatcc taggcttttc 566700
aatcttcaac tcttgaaaga gatcgtaggt cgtctgtaca tgctctaaag ctaaaggatg 566760
cgaagcatcg acaacatgca gaagaacatc ttcatggaaa gctgcttcta aagtactttt 566820
aaatgctgct accaaagtat gaggaagttt tcgaatgaag cctacagtat cagtaagaag 566880
gacatgacgg cctcctggaa gtacgcattt gcgcgttttg ggatctaaag ttgcaaatag 566940
cttgtcttca acatacgtat cagcagccgt cagcaaattt aatagggtgc tcttccctga 567000
atttgtatac cctatcaaag caaaggtagg aattcctcgt cgagatttta ctttacggcg 567060
ttccgcacgc tgtttgatca cagctttcag ctgtgctgac agcttatgga tacgctcacg 567120
gaccattcta cggtctagct cgatctgttt ttctccttcc cccttaacaa agcctccgct 567180
acctccccca gatttttgcc gagataggtg cccccaaagt ctcttaagac gaggaaggag 567240
ataacgtgct tgtgcaagtt ggacttggat atttgcctct gcagtaaggg cacggctgga 567300
aaagatttcc aaaattaact ccgtcctatc caaaacgaca aggccaaggc gtttctctaa 567360
attccgttgt tgggatggag tgatctcctc atctatgatc aaagtcccta tagagggaaa 567420
ctctttcaag atttcttcga tctcctccaa cttccccaca ttgatatagg tggaagctga 567480
gggtgttttt aaaatccaag aacgggtctc taaaacagaa ataccacagg aatccgcaag 567540
tgagatcaac tcgtctaaat gttcttcaac gacctgagaa tctgtcttat tttgatagga 567600

agccacagct aaagcttgag agggatcctg ttccttacga ggcaagtcga acctggcccc 567660
taacgaattt ccgaaagatt gagaaccctg ttccccgggc gtatctatag tgtccaaagg 567720
acctccatgc catcataagc aaatgtgacc tctggatgct ggtcacgctc tgcttctaaa 567780
cagtggctga tatgtgtaat aattaaattc tttatccctg catgattcgc aaaagctttg 567840
gcttcttcta cagtaagatg cgaagatttg tgtccctgaa aaggaatagg agtttccgat 567900
ggacccgcag acaagatcaa tgtctctaca ttatctaagt aactgaaaat ttttgcatca 567960
tagctacaga gatctgtaag ataagcaaga tttccaaaac gaaaacccgt tacatggcac 568020
gacttttgat aataggaaac ataagtatag ggaatgccct gaaattcctc ctgcccacag 568080
tcctcattca agattgtaaa ctctaaaact gcgggaagtg aagactctac attcggagtg 568140
gcgaagagat actctttagc cttgtttaaa aatctatagg tgcttgcaga aaggaccaaa 568200
ggcaacgaac gctgcgtgac tatgtaccac gcacgtaaat catcaatacc accgatatga 568260
tcgtagtggg gatgggtcag aaatacccca tcgagctcgg aaacccctgc aactaacatc 568320
tgcgtacgaa aatcagggcc tgcgtcaatc actagagtct tgttttgata ttgaatgagt 568380
accgaagatc gtaaacgatg aatccctgtg ttttgacaca ctctacatga gcaaaacggc 568440
acgggaattc cttcgggatt tcctgtgcct aaaaatacta atttccctat agattcactc 568500
tgaatatctc ttaccatacc aattgctcat ctggaaacac aagcaaacaa aaataattag 568560
tcagtctctt aactttggat cttaaagtca agaagttgct agaaccccaa tttagaatcc 568620
tctatagctt tttaaaccat agtttccaag tatctatcct taaaaatttt cgagagggat 568680
ctcgtgcagc gcagtaaaag attcatgaga aaatgtgaga aataactgac gaacacgctt 568740
agttatctct gcgtgatcca ttaggtaact ttgtaatcgg ttgagataat gcacaggttg 568800
caggatactc tgtgatcaca gaatagtttt gggaatgtgc ctgtatctga cgacaatgac 568860
attgcgttat atattcgtgt gaaagattga gccactcacg gactctccct cttcccaagt 568920
acgccatcaa gatcgcaaac aagcataaaa tggcaccaac ccctccaatg atacagggaa 568980
tcacccaagc acttaatccc gcaatcaaag gggcttcaac cattaccccg acaatcagac 569040
ctgcaaaaat acacaaccca ccaaagaccc aactcacaat agagcagatg tcaaaattcc 569100
ttacttgaga acagagctcg tacattgcag actccagctg cggagctccc ttacccacgg 569160
gctccaaagc ccataaagaa gtaatggggc catgtacggg taggccttga aatttatgtt 569220
gcttacaacg caagacctgc aagatacctg taggaggagc cccttgcaca tcaactacaa 569280
taacaggaac aacataggga ctaacgacct cagcacataa ctgacaatag aaatacctat 569340
ttttgtattc ttcaatattg ccagcaccag gataacatgg caaagaacct aacatgcaat 569400
cttgttcctt ctttgataca aattcaccta gtctcccaga gattccccaa gaaattcnnt 569460
catccgatta aagacaggaa aagattcaaa taaaggcaat gctcctaacc acattctttg 569520
gggaacggct gtcaaataca tcaaagcttt caatccttga aacgacacgc cctgtgctgc 569580
caagccatga acaagcataa ccgcagcctg ttcacttaaa acatctctag gattcaatac 569640
tttttgtcca gactgtaaac gactcgcaaa ttgagataac atagaatcca agccttgcaa 569700
acaaacggtg taatcggcct gatcttctag agagatttcg gtaccgcgaa cacagtctag 569760
caaaacagcc caatctatca aataggataa aggatattca tacaaactgc agaggaaccc 569820
tgactcattt tctaagattt cagtgttcga aagaatctgc tcacgaatct gcctacgatc 569880
tacccgtacc acagaaccat atttgtcaaa gagccgcttc atagcaggaa cagaagaaga 569940
aaaagcaata aataggttgt ctaatctttg atttgcgctc tcataacatt tttctccctc 570000
agcacagtta tatccccaat ctaaggacat tgcgtttatc aaatcgagct tctgaaaata 570060
gcgagagaga aatgtttgta tttgtgcttc atagcgggcg acatcgatcg ctgtctctgg 570120
gagctttctt tgcaagctct ctagatggcg atgtaaaaac ttctcaaaaa atgaagacga 570180
ccaaatgtag gtaagaaaag aatcctttaa gaagcgtctg tcgggatttt ccaacgccaa 570240
aagaagatcg aattgcgcca aagcatcctc aggagaacat tcttctctgc gacagctaaa 570300
taccccgtct gcgtctacac taaaggaacc tgaatctgcc caatccatca aaggagacgt 570360
tgcttgtaaa gcatctccgt agcgaatcaa tttcaaaata tcaggagagt aatactgctc 570420
ttctatcaaa aagagtagaa gttcctcggc gtgcgcagtg acttcaggac taagatccac 570480
tcccagtaag ttccactgtc tcactaaatg taaaatgata ttgatgtgga cacaatcttt 570540
ctttaacagc gaaagtgtcg tagatagggg ctcaagtttc aatatatttt gtaaactctg 570600
atcaatatct aaagatcgat ccttagaatc aacaatcaga gattcgtgga gatcccctcg 570660
taagaacaag gaaatcaact cggaacgcca atactcattc cctaagacat cttgttgtgt 570720
agtccactcc ttttgcgcaa caatttctgg aggagtcgct tctcgactag gttctaaagt 570780
cttgcctaaa acgagaccgg agacagcaaa aacgatagct aagcttaaaa gtaaacatcc 570840
aagagcaaac ccgaggacca cagcccacaa tccgacctgt gggaaaatga caatcatagc 570900
aataaaaatt aaacagctca aagcccctag gactaaagct gcgtaataca ttcgcgatgt 570960
tcgttcagta aatctttcgt tggaggagtt agcctctcct acagaagtag atgaagttac 571020
aggaacagag gttgccataa gaataccctc tattcgtaag caacttcaga aagttgccat 571080
ttcttggtta aagattgaat caccccttcg ctctttaaat ctgtaatcgc ttgttgaatc 571140
gtttgtattt cttcaggacg atctttagta cgccgagacc acagcccaac acccaacatt 571200
caggagggag ctctaatctt gttgcaacaa gattagggaa gtctttaaga acgacacgtc 571260
ctaccgaggg ttctagaacg gcaaccggag atttcccata acgaacttcc ataatcacct 571320
ccaaggtgct atcaaaagaa cggacacaaa ttccgggctg agataaaaga taatgctcct 571380
gaaacgttcc tgtctgaaca gcaacagaag aatgctgtgt taggggaagc acaggggtct 571440

ctaaagaccg cttagaaacc accatcagct cttgaacctc atcgccataa tagggaagca 571500
gggcgatttc cttctgacgc gaaggagtaa tggacattcc tgctaaaatt gcatcgatac 571560
gatgtttttt taaatttaaa attaaagcat cgaaagcgaa ttctctaact tccaattgct 571620
tgccaagttt ttcactaatt gcctttgcca aatctatatc gaaacctaca acttccccct 571680
gagcatccac atactcaaaa ggaggatatg tagcattcgt acctacaatc cagatgcgat 571740
ttcgatcgat tttagactca caacttgtta aagataaagg cattataaaa ataaatgctc 571800
taaaaaaacg gcctatttgt tttatcatga aagtttgcgc tctatacgct aaattctatc 571860
atgtgttaaa gtttacataa caaaaataaa tacatcaaac tcgaaaaagt tttaactttg 571920
aataattttg attaaaaaac gagcaatttt tgaacgtatg tttccaattc ccccaccaca 571980
ttgcccgccc aataacaaga ataattttta ccacttaacg actgatacta aagaccctct 572040
gttacttaga attctacgta ccataggata cgttctgctc catatcatta ctcttggttt 572100
gcttcttctg attcactact acaagcatca tcgggttgtc agaaaagaag gcttgccaac 572160
gcctcccact cttcccaaag gaccagagcc aaaaactata gaaattgcca aacaaccgcc 572220
taaggatggt gaagacaaaa aacccgatgt tcccaagccg ggcacgccgc ccccagagga 572280
cacacccccg cctcccccca aagctccttc accagcgagc ccaaaagtcc ctaaaacaac 572340
ctgctgataa aaagccgact ccaccaccag aggcccctcc tcctcccgta cgggtggcta 572400
cccccatgcc tctccgccca tctagtcaag gctattggca atgcttaaat cgcatggtga 572460
gcatggtact aagacgagcg cctctgcctc ttcctgccat gcaagttgat ccaatacttg 572520
gcgactttaa ccctcatttc gtagcttcct atcccaatcg gattaataac gaaccgatgt 572580
atttccaaat aaaacagttc aagaaaatcg cacaaaatcc ggatcttcct caacaacacc 572640
ggcgacttgc gcaactctct cttgaacagg ctctctatct aaatgacaat tactaccttg 572700
tgaatgtacc gggagatggg aactgctttt atcgtgccta tgctgtagga tggctatctg 572760
ctctctacga agagagcagc agaaatgata ttgtctttga gcaggaagcc acacgtctcc 572820
ttgacctgcc tttcgcctcc tcttctccgg caaatgcgaa tctttgtgca gaaatggctg 572880
aactccttca gttatgcagt acttattgct ccttcataga cctctatgac ggggtgattc 572940
tttctcagaa acacactgca actctgatag cctttctaag aaaactctct gcatatgcga 573000
ttcgccaaca aatcgcagct tcaagtaatg aagaaacagc gagagcctta tttatttctg 573060
atatgcagga cgatctcctc cccagtgttc tggaatttct tgctgcaaat cgtccctatt 573120
cggaattgtt ccaaaatctc attgatcatt ccgcacaccc tacatgcaat ctagagacaa 573180
actctttctt ctcttggaac atctgcccgc tctctttctt actgatgcag agcttcaaaa 573240
gatgtctcca gaagatcaac aacttcgaaa gcaatatgaa agagaaatac gagaggcttt 573300
tgctaagctg agtcgacgca ttgctgattc agggtgggat actgagagat tcaatgctat 573360
agtcaaagat cacctccctg aagcaatccg atgtcaatac tctcgctttc ttgcaactat 573420
agaaaacaga cgatctgggg atctcccttg gtctccagct ctttctttct ttgcttttct 573480
atgtacctgc ccctctgtaa gatttcacaa actctgcgct actttctaca aatcattaga 573540
ggatatcatt atagcgtccg cgccccccca acgctctata caagagatct tacanataag 573600
taacgcctcc ctcagctacc ttaatgaaga tttagattct tcttggcaac gagaggtgat 573660
ttcttctaac atcatgacta tccttacgac tcatgagagt ttgacgttag agagctctat 573720
gcctcaactc gaaacactac ataaacgcat agcaaaccta ttaaagaatg taatatccac 573780
atcctttgaa acccctcctt taagcaatca gccggattta ctttcaaatc ttgtaaacaa 573840
gctattagtc gcaattcata gtaagcttga attaaaagag cacttcaata ctgtctgctc 573900
ggcaagaagt ttacgtttaa cgcgtgatga aggcagtggt ctctcacaag agcaggacct 573960
cctctataca caggcagtac agctcttatt ctttatttta cagcatcctc aagtgaataa 574020
tcgtccagaa actaaagatg ccgttaaaga gttaaaaatg cttctacttc cttttctaca 574080
atatgccttt aaaaaagtag aaaacgaaaa gaaactccaa aaacttctac gttccattct 574140
agggtctcta gtactcaagc ctccagcacg ctatccttca accccttcta ataaagataa 574200
agagacgttc tgcaagttct ggtcacgaca tcctgaagtg atggttttag atcccatact 574260
tgaaaagaac tgtatgcagt ttctacgagc tactttccca aattatcaac tggaaaccga 574320
ggccatactc ttagaaaaag aaatcgaaag tacctttagg aatgggtgga acgttttttt 574380
aacacggtta aatctcttcg gatcaaaact gggttcgcct tcttctccca cagctttaag 574440
tgatcagttt tcgaaatctt ttttaatctt ttgtttcctt aacaactacc ctaaacttct 574500
acaaaaaaag actccgctag ctgctcgatt agacgctttc caaagagagg cttctcatag 574560
atttacacaa gtaaaagata agcttttact ttcgttaaaa tacggtttcc ctctagctac 574620
agcgactata aatcaatact ctagagctcg agatcagttg atttgtaatc tcttaaaaaa 574680
cacggtcaca gcatctgatg gtttctgtcg ctctggtttt agacaatcac tgataggcta 574740
cctccactcc ctaagttcta atgaactcgg tgatatcttg gatgacgtca aagagcaagc 574800
tgaggctaac gacgtcgctg ctatgactac tgtacctttg cagccgtttg ctgtttgtct 574860
gatcatgtct gatcgagata ctgtctcaga agaaaatatt gaaaactttg ttgcgatgca 574920
tggattttta aatacaattt ctccggaaag agacgctcgt atcttcttaa tccgcttccc 574980
caaccactac ggttgtctct tgcctagaaa ccctagaact gaagatcaga actcaaaacc 575040
ggacagctca aatccctagt tttgcatgag gtacttattc ttacgtttac ctatccccta 575100
ccgcgcaccc tcaaacagca tcctgacgag gtccataccg ttcctatttc tccaaatcta 575160
tcgtttggag aaggatcgcc aatactgatc gcaggcccct gcaccttaga aagttacgag 575220
catacagtct cttcagctct tacagttaaa gaagcaggag ctcaggtatt ccgaggatca 575280

atcagaaaac cacggacaag cccattttcg tttcaaggat gggagaaaga gtgtgtgctt 575340
tggcataagg aagcacagag catccatggt ctccctacag aaaccgaagt tttagatgtc 575400
cgagatgttg aaattactgc cgaacatgtg gatatcctcc gtatcggagc caaaaacatg 575460
cataacaccc ctcttttaca agaggtcagc aaatcacatc gtccgattat cctaaaacgc 575520
agtccagcag ctactcttga agagtggcta tgcgcagcgg agtacatcct tgcttcttct 575580
ccctcctgtc ctggggtaat cctttgtgaa cgaggaattc gtacctttga gcactctacg 575640
cgctacacgc tagatctcaa taccgtggct ctccttaaag agatctcttc tctccctgta 575700
attgtagatc cttcccacgc agcggggaag cgttctctag ttcttcctct cgcctctgct 575760
ggtctctcgg taggtgccga cggtctgatg atcgaagtgc atgcacaccc tgaaaaggct 575820
ctttgtgacg cgaagcaaca gatcacgccc gaggagcttc acctatttgc taaaaagcac 575880
ttctgcccat cagaatcacg ggctcatgcg atttcttgaa atcggttgat gattctcaaa 575940
taaaagagac taaaaaatct tttatttgag aactcttaaa ttaaacttaa ccaaatactt 576000
ttaaaaagtt tattagaaat tttgttttat tatatttaat ataaatattt ttattgaata 576060
gtaaatacta ttttcactat ctaacagcca attggaagaa cgccaacacg cgtctttctt 576120
tggaggttta tgataaaaca agcgtgtaaa ttttaccttt tacagtgttt actttgcgct 576180
ctgtattggc tattaaagta ttgcagaaag cttcttaagg gcactcttca ccattctgaa 576240
gagacgctct atcaagccct gctctcctct cttatcgacc tgctctatca gttaaaacag 576300
cttcccgccc ctacgaatga ataataaaaa ataaaacgaa cccctcttct tcttggaaaa 576360
gaaggagagg tatcagattc taaacacaac ttaaaaaact gacgatctct tactaagcgc 576420
aacctatcag atctaagaga aacctaaaca actcaggatc tattttagtt atatcttctc 576480
atgcagtggt tatctaagtt tgcgttttga ctttgttttt ccaagtgatc tcaataaatc 576540
ctacaaagga aaaggcgact agagaaccta cccaagccaa tagctcccca aacatcccca 576600
aagagactat ctgaacccat ccatagccca gagcaccaac gagcactcct gcccaagcag 576660
cagctccgct cacacggcga cctttaggag ctagaagata gaaacccaca gggactgaaa 576720
gacaacacac tgacaggcta tagcttaaaa tcaagacatc tacgatgttt gtaaaaccaa 576780
tagcaacaag aggagctgca accgccaaac ccaatactaa ataacgataa taaggggctt 576840
tcaacgtagg gtattcttca gcgattagct ggcttacagc attcataaga gagtccgcgg 576900
tagagagaat cgcaacgccg atggcagcag ccatcacagc tgctagtgag ggattgcaaa 576960
aatatgcaat ggtatcaatc agagggcatc ctgctttaag gcctgcttta gctcctaaag 577020
aacctaaaaa taaagggata aagttaaaaa gaagaagaac aaggcctgcg cctacagccg 577080
cccattgcaa gcgttttgga gaggaggcag ccacacacct ttgcaccata tcctgctcaa 577140
caagcataaa gagcataggc atgaatatcc aattggaaaa cttcgcacaa ggaagtgatt 577200
ggaaaggatc caacacagac aaggatttag ggacagagag ccatacagaa acaccacaga 577260
cgagcaccgc aataagaaga aatcctgctt ggatcacatc agtacgtacg accccgcgaa 577320
accctcctgt tgaggtatag gatgctaaga caatccaaaa tgctacggtt acgtacttgc 577380
caaaagggaa gctgctaaac aaccgatcta aagcaatcac ctgagcgacc aggatgaaaa 577440
ataaggaacc tgcggataat aaaaatgcga tcttacggag ctttttagaa ccataaaaca 577500
cttcaaagat agagactacg gtcgttaacg atccctctgc caaccgcttc ccgggcccca 577560
ttcctaagaa aatcaaccct aaagcgactc ctaaaggata aagaatcccc ccataaccat 577620
aacagaaggc ctcttcagca gccccaagaa gtacaccgcc accgatttgg gtggcaatga 577680
atgtcatcat caaaggaaag atttttaaac tccttcctgc aagaaaatag ctctcgcgat 577740
cttctacctt tttgctacca cgacgtccca cgtacaagca gattccctga atagctatca 577800
ggaaaaataa aaataatgaa aaattcataa aataaagaac tccacaacct aaatganaaa 577860
tatttccttt aagcaagcta cggtgctgag cttaaagata gaaatgctag gaaaattagg 577920
agtggtgatg gagataacgt ccagaataac gaaatcttaa cgagaataga gattcaaaaa 577980
acaaatctga ttttatatac aggttgtgca tgacatacag gctctgagct tacgctcttc 578040
ataaattata aaagctacca gataaaaatc tataacaaag ttttctatct ccaaagactt 578100
aaaaatgaaa gaataaagtc agtctgaaaa attattttta agaaatagaa attacaactg 578160
cgtggttttt cttctatatc ttgaataatt ttattaatat ttatttgaaa atagagaaaa 578220
gagcacccgc tgctgcactt cgcagcgaag gagcttcgtg atggcagcag tctagaagaa 578280
aaggcacagc atcaagattc tcagaaaaag caacgctctc taagattgct aatttatctt 578340
gccaacggct gtcattataa agttgagaga ccctctgtag ggaagcttga tcttttttct 578400
gacatagcga ggctaacgct ccttccaact ttgctgcaaa gcaagcatct gtaaccaaat 578460
cctcagaagt tttcacatct ccctcttccc agaacattcc agaaaaaaag ctccatccct 578520
gagcttgctg tcctgaaagg aacgttgctg ttacagcctt ggcttggcta tagcgagcta 578580
ctgccaaaag gcgaatgagc ttcctaccaa tctcacgttt aatcatatcc gaatatagag 578640
ggaaggtatc accacgtaaa ttccattgtg catcccataa gaaatactct atagcccagc 578700
acatttcagg attggagagg tagcgagcaa tcacatctcc agctctttca atatcttcac 578760
ggctcacaag aagcaaaatg gagaggttcg cagcagcctt tcgagaagaa aggctctcca 578820
aatgctcctt tgccaaaggg actccatgga ttcctaaaga gcagagagcc gccgaagctg 578880
cctcacacac aagaggttga ggagagcgca accctcaacc agagagtctc tgcccaaagg 578940
gtctccatgc agatggagaa gtgcagcagc ttgaaaacgt acttttgcaa atggagaagt 579000
gcacatcaca tggcgcactt tactaagaaa ctctttgtga ttctgtagct gatgactcca 579060
agctaaagca gaaagtaaga gagactcctg cacttcagga tgctctacag ataagagttc 579120

tgtaaaatc tcagtagtct ctggcaacat accgttacgc aacacttcac aagtgaacaa 579180
cgcttgatca atatcgtcct tagctacacc cgtctctaga aattgagaag agagttccaa 579240
gcaagccttc cacgcctctc gacgttctac actatctaca agtttgttct cagcacgctc 579300
tcttaaaaat ggcaatagct cctctatctg taaaagagcg accacctgat atgctgtaat 579360
ccgaacatga atagaatcat cattacgggc aagctctaca atggcctttt ttaaactttc 579420
agagccatag ttcacagcaa cctgaagagc caaagatcga acaatagcac tgtcatcatt 579480
acaactttgg agcaggagag ggaccaagcg aaaatctcta gcaagcccaa tagcaaggac 579540
actcacagca cgaacggtca cggaaggatt ttcgattccc tcacgcagaa cttgaatccc 579600
aaattcttct aagacatcgc gatcatgagc taactctgga taggaaatct gacacttctt 579660
aaaacttcta aaccaatcat caaaagaaga attttgagcc agagccctta aaactagctt 579720
agcctgaagt aaagaatact ctttagactc caaaaggcct atgccctctt ctgaaacttt 579780
tgcaaaatct tggatcaaca attgataacg gagggactcc gagccgaaca aggatccaca 579840
gaaacaaaag aaaattaggc tatagcacct aaaggacgcc cacctaaaag atgaatatgt 579900
aagtgaaata ccgcctgtcc tccttcagca ccgttgttga taaccacacg atacccatcg 579960
gcaattccaa attctgcagc aagctcttgc acgatctttc cagcctctgc cattaaaatc 580020
atctcatccc ctgggatatc ctgaaatcgt ggtataggtt ttttaggaat gataagaaga 580080
tgaacaggag cttgaggaaa acgatctttt atagctatga aattttcgtt ttcaaatacc 580140
ttttcacaat ctatcaatcc atcgataatt tgtttgaata ctgtcatatg atccctcgat 580200
cttgtaacgt taaccgcaaa gcacgttggc aactttctct atttgtccat acagaaagga 580260
aaagaccttt atggcagaac acagccccag gaatccctga tactttggac aactctttac 580320
ctaacaaacc tgcccaattc tcagggaaag gaacacgaac ttccatacgg cgatctaaat 580380
tcggaggaat ccctcgtaaa atccattgat cgcaggaagg aaaacaaaca aaagctgcag 580440
ggtgcttctc tccccctaaa aaaaagaaat tttcttgcca tgctaaagga cgatcaaaat 580500
ataaacacat atcctcggtt tccatggctt ctctgacaat ccccctacaa actcgatcat 580560
actgaaactt cttccttagc cgacacaaaa agtcgatggt aaaatgcaaa gcacaagaaa 580620
aatccgcatc cgaattagtt tcttcttcct cgcgaggatt ataaatttta ataatatcag 580680
aaaacgaaca aaatccctcc ttagagaaga atctgccatt atcttgttca tccacaccat 580740
gtaccaaagt gttgttaagg aaatgatatt cttcacaatc catataacca aactctttaa 580800
gataatgcag aatcatacct gcactactcc aagatccatc ataagagact tgatgatgat 580860
caaaacgctt gttttctata gaataaacac caccgacatc acaaacatat tcacatttcg 580920
ataatacgac aggatctcga gagcgtataa ttttattttc atccacaaga tcgaaaataa 580980
taaggagagc acacgctgtg acctcatccg catggaaaga accatcgtga gtaccaatgc 581040
ttcttggaat ctgcatacct actatcctcc ttagccaatc ctccattgta accagagaga 581100
aataattctc aattgcaaag actattcttt acattttact caatctctcc aagagaaagt 581160
ttctgcccta ttttctaaga aacttttttc ctaaaattcc cttccacaac acaattttct 581220
cgttccaaga ccccctcctt gacaaattgc acaaaattat aacaacaagc agaatgaagt 581280
ccttgtagga aattcttctg gaaacgctag tcttagaagc gcaaagaggg accttaccta 581340
ttgcttttaa ctttaaaata cgatgaatta tgtataacct actccacgcg catcatgatg 581400
cagcctcccc agacggacga ctcgtttccc atttgaaaaa actctcgccc cacatttacg 581460
aaggagaggt cctcattgag aatattcctg cgtactttct tggatttcat ctgcctcaac 581520
agtgtataca agtaaattta aaaagttcct tagcccaact aggtgtcgaa gccgttttaa 581580
accacttgga gctaaataaa gcccgaaaag aagctcgtct acacgttctc ttcatgagcc 581640
aagatcctat agccactgct aatgttggag ctcctaggag cctggnagtt ttgtctgcaa 581700
gctctttgct gctgatgatc gccgactcgt acgttcgcct tgttatctca acaggatgtt 581760
tacgcacaca gaccgtacag gatctccgct cctacgcttt gggaaaaaac ttgagcactt 581820
catcactcta gagatcatta atgatcggct tgttgtcttc cttccgatcc ttccaggaac 581880
aatctgttac gaagagacaa tttatgggtt ccttccctta atgagcaaat cactcacgcg 581940
tccccattta aaaatacgta agtttcttcc tttgtatcaa atggtaacag atcgtcctcc 582000
cgttcccgaa gatcataaaa ttcttctcat aaagacagag cctctgcaca tccgaaccgt 582060
atttgcaaga gtcgttcagg acttactccc ccaagggctt cgtcacaccg cagcggatat 582120
tctcgaacct accacacaag aatctggaga tatttatgaa ttctacggca gcacttcaga 582180
acctattgag agaatacctt tagaattttt tactcttgag ccttacaaag agcattcgtt 582240
tttcttctat agagatatgc tccaggaaac cttanaatct cctcaagagg tatttcgtgt 582300
ttttgaatcc ataccggaag gcgaaaatca agctgcgatg tttatctcca aaggtagtga 582360
gcttgcttga gctctcccaa gactcttgga tcatcaaacc tcgaatctcc ccatcagatg 582420
aaagacatgc tagggaaatt caaaagcaca ttgaagaccc aaccttgttt ccctttttta 582480
aaagccatgg aaacagatca tatcacaagc caaggagttt tattttcccg ctacttccct 582540
tcagcatcgc tgaagggcat gttcctctct aactactctc gctattacct gcaacatatc 582600
tattttcaga ttccctctcc cacttctgga gagtttttct cgaatcgaga tcgctctttc 582660
cttctcgatc tatattttgc aggaatttct gtattttggg cagacttaga atcgaaacga 582720
ctcttacaat acatcaaacg cagaaataaa gatgtgggca tgtttgtccc taaacatcaa 582780
gctgaacagt ttgctcaatc ctactttata ggaattcatg gttcctgcct aatcgctggg 582840
gattatgatg agtttctccg tgagctcctg acaggaatgc atactctttc tcagcaattc 582900
acgatcccag aatttccacc acagacaccg ttagcaatcc ttacaggagg gggttctgga 582960

gctatggaac tcgcgaatcg tgtagctaca gaactctcca tactctcttg tgggaatcta 583020
attagcttgg ataccacgaa tgcctatgta gaagctaaaa tgagctatgc tattcctgat 583080
cttttagaac gtcaggccga cttccatgtc gaccttgctg tatttgttat cggaggcatg 583140
ggaaccgatt tcgaactcct tctggagctt attagtctca aaacagggaa aaaagctctt 583200
gttcccgtct tcctaatcgg acctgtagac tattggaaat ccaagatcac agctttgtat 583260
aattccaatc atgctgtagg aaccattcga ggttctgaat gggtacacaa ctgcctattc 583320
tgcctatcct cagcaaaggc aggcattgca atcttccgca gatatctcaa tcatacgctg 583380
cccataggac ctgaacaccc tgtccctgaa gatggttttg ttatcgttta gaatccatag 583440
gtaagacgga aaccgtagta attacacggt tcacgaatga attggccttc tttagaaaat 583500
ccctgatgat attctaaaac agcacggatt ttccttccga tttcttgaaa tttggcccac 583560
tccatgccta aaatatagct ttgatccaag ccaaatttct gttcttccca acaacggaaa 583620
tgcatcgcga aaatcggttg tgcgtggaga tttccttctc tcagaccaaa aggtctgagt 583680
tccgcacccc attcacagta aaacggccgc tcaggaaaag taagatccct acttacaata 583740
taaccgcagc cgccatacaa gcggatctgt ggtgtgtaac gaaacgaaat gaagagatcg 583800
acgccctcat cactcaaatt aaatcttggg aaatttggat gcgtaagaat aaactcatct 583860
cctaaatggg acgagaggtg ccacaatcga aacctaaaac tccatttatc tatagcccct 583920
gaccagagtc cggcaacaaa gaaatctgaa tttaccatgc acgattcagg atgatctaaa 583980
tcaaaaactg agaagactcc tccttgaatt ccgaaatcac aatctacatg gaatcgagaa 584040
acatcaaaaa gacgcaggag aataaaatct cccccaaaga tggtagcacc tacccgattc 584100
cccacgacct tctcattaaa acgaatgcca gcactgttgg taacctgacg aggatctgca 584160
atcaaaggag aaaacaaaat ggtattttga ggtaaccata ccccttcttt gccgcagaaa 584220
attggcattc ctaaagtttt ctctttaggg agtaagggac gctccgcaga agatctcgtt 584280
atgcattcac cataaggacg ctcgcaaatc tccacagcac atatgaaggg aagatcccta 584340
acaaagttgg taatggcttc tgataaactc gaatctacag gcaaagagaa aagatacgct 584400
ctattcttct caataacaac ttgcgtacgg ctatctaaaa aatgcatgtc caaaagagac 584460
tgcacataac ctgtaagata acagtcgttt tcatagttcc agagatgatc cggcagctga 584520
tccgaacgta aaacagactt agtttcgtat ttgcaatcgg ggcacaaagg ctctccccca 584580
gcgatgccac ccacaaagag taccaagaag ctaaagagcc aacaaaacca agaatagcaa 584640
gagtgaaaag ctgtcttcat cgtaaagaca gtgtatagag aagaaagtat ttatttcgca 584700
atattgtata gcaattctta aaactaagag accctaactc ttttcattga tctttccgat 584760
ctgtgaagag aggaattcac aacagaccta caaacatctc catgtagaat cagcttgttt 584820
ccttctagaa tctccgttga aaatccactg gtcttctcct tatggattcc ctcccttcta 584880
ccgcagagac ctaaaactct gaaccctctt aatgaagaga aggagggatc tcagctctga 584940
tccggattag ctgtctttcc aatggacgtg cttgcctttg gggtctttgt ttccaggctt 585000
tttcaagatc cctttgggtg tctctataaa tgaggtctca gcatcttctt gttttctctc 585060
tacaccactc tgttttggac tgcgtgtgta agttcgcaaa cgtatgtaag ggtgcgttgg 585120
agaaaaactt gagggagaac gcttcttgct tccacgtcct gtaaacgttc taatccattg 585180
aacaaatctg ccaacgaatc ctttgcttgt cttcgaatga gaaccttctg tcttggatta 585240
tttgaaatgg gcttctgaaa tgcctttgac cttcttctct aaagaagctt tccccgtctt 585300
agaacccgag gtcaccccaa ctgaagcact cttatgcttt ctacgaatcc caccacgacg 585360
atgcaagcga gagttccctg aaaggggtac atcctttttc ccatcttctg gagaaggatt 585420
cctaccccta taacttccat aaggattgac ccttatcata attacctaaa aaattttttc 585480
aaatcttatc ctttaattat agtgagcaaa ttagaaaaga tttaaaaaac ttttaataaa 585540
cagaattata aatatatctt aataattaac cgaaactaca gagcactcct tgcctttata 585600
gaggcggtta aagnnaaatc agaaaatggc tatccaaaaa gctggggctt tcttaagatg 585660
tcttccaagt gaatcacgcc cttacctgga gcacgctatg cgtagaaatc cccacttttc 585720
tcttctcaag cctcagtatc tattttctga aattagtaaa aagcttgctc agtttcgcaa 585780
ggagaatcca gaaatctctg tcatagatct ttctatcgga gatacgacac aacctctctg 585840
ccgctctatt actcaggcaa tcaaagagtt ctgcgtttct caagagaaac aagagaccta 585900
tcgtgggtac ggcccagaaa ccggattaga aaaattacgc acaaaaattg cctctgaagt 585960
ctatgaaaat agaatctccc ctgaagagat ttttatttcg gatggtgcca aacctgatat 586020
cttccgtctc ttttcttttt ttggctcaga aaagactcta ggtctacagg atcctgtcta 586080
tccagcttat agagacattg cccacattac aggaatccgc gacattatcc ccctagcatg 586140
cagaaaagaa actgggttta ttccagaact tccgaaccaa caatccctag acattctttg 586200
tctatgctat cctaacaacc ccacaggaac agttctaacc tttcaacaac tccaagcact 586260
tgtgaactac gcgaatcagc acggaaccgt tcttattttt gatgcggcct atagcgcctt 586320
tgtctcagat cctagcctac ctaaaagcat cttcgaaatc cctgaagcaa aatattgtgc 586380
tatagaaatc aactctttct ctaaatcatt aggctttact ggcatgcgcc ttgcctggaa 586440
cgtgatccct aaagaactca cctatgacaa taacgaacct atgatcaacg attggaaacg 586500
gctctttgcg actacattta acggagcatc tctcctcatg caagaagcag ggtattacgg 586560
cctagattta tttccgacac ctcccgccat ctctttatat ctaaccaatg ctcagaaact 586620
taaaaaaagc ttagaaactg caggattctc agttcatggt ggcgatcatg ccccttacct 586680
ttgggtagaa ctccctgaag gaatctctga tgaagaagcg tttgatttct tcttacatca 586740
gtatcatatt gcagtgactc ccggccacgg ttttggttcc tgtggacaag gatttgttcg 586800

tttctccgct ctgacacaac cacaaaatat cgctttagcc tgtgaccgcc tctgtaccgc 586860
ttcactaaaa gaaacgatgg ttcttgcatg acaattctac gtaaactctc tcagtactta 586920
tttttctttt ctctgttttg ctctttcatc tatgtagcca cttgtggttc tcaaccagat 586980
agcgtctcct ctcctaaaat cgcaattttc ttatcctttc cccatcccct attagaagat 587040
tgcagtaaaa gctgtataga aaccttgaaa gattttgaga accttcctga aattgttgtc 587100
ctaaatgctg aagacagtat cgtaaaggct aggaaaattg ctcgctcctt acataccgat 587160
aaaaatgtcg tggcgattgt caccttagga actattgcta cgaaggtcat gagccacatt 587220
gaaacacaga aacctgtgat ctatgccgct gttcctgatc gcgaaagcct aacccctcct 587280
aaaaacacaa tgaatatcta cggagtgaat gacactctag acatcaatca atactgcttt 587340
gctatacaag ccgtagctac caatgcacaa tctatcgtgt atttaaaacc ctccgaacct 587400
ttcccctcag atctccaaaa agaaattgtt aagaaactcc atgcttcagg aattgaggtc 587460
attgagatct ctattacaag cagtacattc aaaacccgga tacgccaggc tatcgacaag 587520
cgcccctcag ctatcttcat tcccctctcc ccactttctc ataaagaagg caccgcattc 587580
cttcaggaaa tcctcaaaga gaaaatccct atcattaccg acgatacctc cttaatttcc 587640
gaagagcctg cattgcctgt agcgtggatt acaaaaaatc aggaaaacaa atcgcaaaaa 587700
attgtgcacc acctactcta taacaatcac gatgtggaca gcctgcgtaa aatcattgct 587760
caacgcctgt cacctacaac cacctttaat gaagatatca tcaagtactt aggaatcaag 587820
cttcataaaa cagaacgcaa ccagttctta tcttttaaaa gcaaaaaatt ggaaaaatct 587880
gagaaaggga aaaacgtagc tgtgagttag gctatacgtc tatatgcaaa gaaaaaataa 587940
tgtcttgaac tctgttagga agaagaaact ttttctttcg agaagtttct cctgcaatta 588000
aagtgacatc acgcttcggt aaggataaag cttttgctaa taaagaaatt acagcatcat 588060
tggccttacc cttttctggg ggttcggtaa cacggacctt caaagcttgt ccatcaaagc 588120
ctacaatttt gttctctttg gcttttggag tgactttaac ctctaagatc catgaatcat 588180
ccaaaacaac cctctgaaat atttaagcag ccgttcgttt cctttaacca ttccgctaaa 588240
gtatgtagac aaatccttgg agcccaatga tacttcaaga ctccttgagc aaatccaggt 588300
ccagcaagat catagctttc ccctttgaaa aactgcccga ccctgcaagc atctaaaccg 588360
ataaagggtt gactacttac aaataacacg cgtcccggaa acgcttcttg ataagaacgg 588420
aataaaagta aggtgtcctt acgattcgca accacacgat tttcctctgg ctttgctaga 588480
agaaatgtca ctctgactcc tgaagtacta tctcgccatg ctctaggtaa aagcatttgc 588540
atccaaacaa atttggcaac ctcttcttca gaagagggag taactcggtt accagattcc 588600
cagttctctt cagtagggaa aggattgtac cgagaatcaa agaaatgctc ttgttcttca 588660
atagactgat agcgccctcg ctctccacat agaaaaacga ttttcttaaa gcgcacgcca 588720
cgctgccact ctcgcaccaa aaaatctaag cgctgacgta gcgctggcaa aggcccgcca 588780
aaaactacag cacaatcata cgttgcagaa tacgaaggca cagcctgagt catatgtaat 588840
aaagacaaat cgttatagaa agcatgctca tctttaacct gacagataga gactaactct 588900
ccagaaaaac gttcttcagg agtcagtacc caagcagacg aggactcgat taaatcctca 588960
acattctcag cttcaggaag tccacaaact tctagtaaac gattgactac aggaatatac 589020
ttatcaatag aacatcttga tttcgcttgt taccaaggac aacacccaac agaaatgagc 589080
gccgtcattg cgaacaatgc cactacccat cttgcttttc ttctattcat aaagggcgcc 589140
ctccttaatc ggcaatatta gacgccacca tataacaaaa attcaaaaaa aacaataaac 589200
aaaccaccgc aatttgaact ataaaaatag gactgggtga gagtaaaagc tggcaattct 589260
gattcgatag tttctactca gaagagatca ttctttatta taactccgtc tcttcctcac 589320
tatcagagtc ttgcttatca cctcgactag gttggttacg gccatctcca tggggagatt 589380
caggatgtat agactctccc tcagctctca caatcatctc ttcctcagat aagtcagatc 589440
gccgagaaag gcgttctctc cttgtagaac gctctccgga agaagacctc tggtcttctt 589500
gagaagcatc gggattatgg aaaggcaaac accccagccc aggcaaaagg ttaatccgtc 589560
tcactatcca catcaaaaca aacacgatgt tgtcatagca actcgaaata aaacctacaa 589620
aattcgccca aagacgacgt aattcattga gcaatctttc acagcaagac aaactttctt 589680
caaccctagg atcagacgaa gattccgttg ttccctgaag agtccgcaat tcttctacag 589740
taggtagatt cactttcttg atctggacaa gcatactgtc agcagtaagc accagtcgcg 589800
gaatccccct ctcacaagaa agagaccaat taccttcagc gtctacatca agccctaacg 589860
aacccagatg ctcaagaacc ctatcattat tgtcaaacgc cagggaaaga tcttcacgac 589920
tgagtccctg tagcttgtga tgtacatctg ttaacttact taaaatgaga tgagccttcc 589980
catgaaactg gttcgcatgg cctaagacag cctcgtaatc gctacgaaat aattccagcc 590040
tcgctatatc cgcattatcc ccagcacctc gagtttcgag tcttctcgtt gaagatttcc 590100
atgccggaag acagtttttc aactctgact gaaagctagt gaaaagggac tgggcaccct 590160
taaggtcgtc taaaagctct ccaacacgct ctacaagtga tgagatctga tcagcaaccg 590220
cccgactcga agaaacttca gctacaatct cgtctacctt cggattactc gccgtttctt 590280
cagatgaggt ccccacacct tcaggaagag aaagttgagt gactatagga agagccacag 590340
catgcgtggc ttgcgcctca tctgaagaaa caacattttt gttttcttcc gtagattcag 590400
gagaagttac ctcttctgga ttcggggttc cagagctttg atttactgac gacatagtac 590460
tacaattaca aaaataaaag attaataagt atacacttaa ttataagttg agattattaa 590520
atttctaaat aaaaacaaaa atataattaa aataatacat aatcctgacc ccaattgtta 590580
agttaagaac ccaacgcaaa acattgatct agaaaaatcc ctgtgttaag atacctcctt 590640

catagctaaa ctaaatctac tagcttaaaa gactctcgat ataaaatcgc aatagcctgt 590700
aattttttct atagactctt gacgatcaat cactgaagat aacagcccta ctctatgaaa 590760
acgtctcaac tcttttataa gacttcaaaa aatgcaaata aaagcgctgc tgtgctctca 590820
aacgagctcc tagaaaaggc aggataccta tttaaagtaa gtaaaggagt ctatacctat 590880
acacccctgt tatggcgcgt ggtctccaag atgatgaaca tcattagaga ggaacttaat 590940
gcgattggag gtcaagaact tctactccca cttctccaca atgctgaact ttggcaacat 591000
acagggagat gggaggcatt tacttcggaa ggactgctct acactctcaa agaccgcgaa 591060
ggaaaatctc attgcctagc tcctacacat gaagaggtca tctgctcttt tgttgcacaa 591120
tggctctcct caaaaagaca acttcctctc cacctttacc aaattgctac aaaattccga 591180
gacgagattc gccctcgatt cggtctcatt cgctctcgag agctccttat ggaagacagc 591240
tataccttct cagactctcc cgaacaaatg aacgagcaat atgaaaaact ccgctctgcg 591300
tatagtaaga tctttgatcg tctcggtctt gcctatgtca tcgttacagc tgatggaggg 591360
aaaatcggca aaggaaagtc tgaggaattt caggtccttt gctctctagg cgaggacacg 591420
atctgcgtca gcggttccta tggagctaat attgaggctg ctgtctccat tcctccacag 591480
catgcctacg atcgcgagtt tcttcccgtc gaagaagtgg ccacccctgg gattacaaca 591540
atagaagctc tagcaaactt cttctctatc cccttacata aaattttaaa aacccttgtc 591600
gtaaaactct cctactcaaa tgaagaaaaa ttcattgcca ttggaatgag aggagatcgg 591660
caagtcaacc tagtgaaggt cgcttccaaa ctgaatgccg atgatattgc tctagcttct 591720
gatgaagaaa tcgaacgcgt tctaggcaca gaaaaaggat tcatcggtcc cctaaactgt 591780
cccatagact ttntcgcaga cgaaacaacg tccccaatga cgaactttgt ttgtgcgggc 591840
aatgctaaag ataagcacta cgtaaatgta aactgggatc gcgacctcct ccccccccaa 591900
tacggtgact ttctactcgc tgaagaggga gacacatgtc ctgaaaatcc tggccatcct 591960
taccgcattt atcaaggcat agaagttgct catattttca atctcgggac acgctatacc 592020
gatagttttg aggtaaactt ccaagatgaa cacgggcaaa cccagcagtg ctggatgggg 592080
acctacggca ttggagtcgg aagaacatta gccgcttgtg tagaacagct tgccgacgac 592140
cgtggtattg tttggccaaa agcactcgct cccttctcta tcactatcgc ctttaacgga 592200
ggagacactg tatctcaaga gcttgcggaa actatttatc atgagctaca aagtcaaggc 592260
tatgagcccc ttcttgatga tcgagatgaa agactcggat ttaaacttaa agacagtgac 592320
cttatcggca ttccttataa gcttatttta ggaaagtcct accaatcttc gggaatattc 592380
gaaattgaat cccgatctgg agaaaagtat acagtctccc cggaggcctt ccctacttgg 592440
tgtcagaatc acttagccta gctctttgat cgctgccctc ctcgtaaaaa agttagcatt 592500
cattcaattc gagtgctaaa ttctcttgac cttctcgggt tcttttccta taatgctctc 592560
agttacgatt gttctagtag gactcgagat ggctagatcc aaagtctcaa agcgagattc 592620
aaaaatcctt gatatcctgt ttgctacaac agagttgtac ctaaaaacag ggcagcctgt 592680
agggtctaaa actttaaagg aaagtttttg ctctgatttg agtacggcaa ctataagaaa 592740
ttactttgca gaacttgaag ctgaaggatt cttaaaanaa aatcatactt ccggaggaag 592800
aatccctaca gacctagcat tacgtcacta tgtagatcac caagaagaat gcccagaagc 592860
tgagatttct gcccccattt ttgataagnt cagtcngctt ccctagcgaa agtcgcaata 592920
ttatcaagga tctacaaaaa gctacggaac ttcttggaga aatcctagac ctgcctacgt 592980
ttttttcttc cccacgcttt gaaaatgatt ccgtaaccaa tattcaaatt acacaggtcg 593040
ataagcaaag agctgtcacc atcctctcta cggagtttgg tcagatcttc acagacaccc 593100
tatggctgcc tgaagcttgc gatactcttt ctatcaaacg tatagaaaaa ttcctgcaga 593160
actacatccg aaagctcccc acaaatgagg aactttcgaa aaaagaagaa cacctgagca 593220
tgtccctcta taatgaggtg gttgtccgct atctaacacg ctactgcaac tttagtgaag 593280
aagatctcta tcaaacagga atgtcgaaac tactgaaata cgaagcgttt aaagatcctg 593340
aagttctagc tctaggactc tctctttttg aaaatcgcag acaaatgtgt gagcttctaa 593400
atataggaat gcataaagga agagctacag cgttcatagg gaaggagctt tctgatattt 593460
tagggacctc gaatccagga tgttctgtaa ttactattcc ctattatatg aatcgctctc 593520
cactcggagc tttaggtatc ctaggcccga tcaatcttcc ttataaggaa gctcttcctt 593580
tgctcaaact atttgcgaat aaaataaatg aaaccctgac acaaagtttc tacaaattta 593640
aactatcctt cagaagacca ctcacctcta actgtaagct ttcgaatgag cctattttaa 593700
gaacggagta ctcttctata aaactattac cctctaagga gacgttatga cagatacccc 593760
acctgaaaat gaggaacaac acgaaagcaa tgttcaaaac gaaaatgaag ttgaacattt 593820
gcaacaggaa atcgtcaccc taaaaaccga attaaaagaa aaaaacgata agtatctcat 593880
ggctctagca gaatctgaga attctagaaa acgcttacaa aaagaacgcc aagaacttat 593940
gcagtatgct ttagaaaata ctttaataga ctttctcaat cccatagaaa gcatggagaa 594000
agccctcgga tttgctacac aaatgtccga cgatgtaaaa aattgggccc tcggattcaa 594060
catgattctc aaccaattca aacaaatctt cgaggaaaaa ggtattattg aatattcttc 594120
aataggccaa aagtttaacc ccttcctaca cgaagcggtg caaacagaag agacttctga 594180
agttcctgag gggacgattt tagaagagtt tgcaaaggga tataaaatag gagaacgccc 594240
gattcgggta gctaaagtta aagtcgctaa agctcctact cccaaagaaa ataaagaata 594300
gaaataaccc ctagagatta ggtaccaaac atgagtgaac acaaaaaatc aagcaaaatt 594360
ataggtatag acttaggcac aacaaactcc tgcgtatctg ttatggaagg aggacaagct 594420
aaagtaatta catcatccga aggaacaaga accacgccat cgatcgttgc cttcaaaggt 594480

aatgagaaat tagtggggat tccagcaaaa cgtcaagcag tgacaaatcc agaaaaaact 594540
ctcggctcta caaaacgctt tattggccgt aagtactctg aagtagcttc ggaaatccaa 594600
accgttcctt atacagtcac ctccggatct aaaggtgatg ccgttttcga agttgatggc 594660
aaacaataca ctccagaaga aattggcgca caaatcttaa tgaaaatgaa agagacagca 594720
gaagcttatc taggcgaaac tgtcacagaa gcagtgatca ccgtccccgc atacttcaat 594780
gattctcaac gagcatccac aaaagatgct ggacgcattg caggtctaga tgtaaaacgt 594840
atcattccag aacctaccgc agcagctctt gcctacggaa tcgataaagt cggtgataaa 594900
aaaatcgctg tcttcgacct tggtggagga acttttgata tctccatcct agaaatcggt 594960
gatggcgtct tcgaagttct atctacaaat ggagatactc tcctcggtgg agacgacttt 595020
gatgaagtca ttatcaaatg gatgatcgaa gaattcaaaa aacaagaagg cattgatctt 595080
agcaaagata atatggcctt acaaagactt aaagatgctg ctgagaaagc aaaaatagaa 595140
ctttcaggag tctcttccac agaaatcaat cagccattca tcacaatgga tgcacaagga 595200
cctaaacacc ttgcattgac actcacacgt gcgcaattcg agaaactcgc agcctctcta 595260
atcgaaagaa caaaatctcc atgcatcaaa gcactcagtg acgcaaaact ttccgctaag 595320
gatatcgatg atgttctctt agttggaggt atgtcaagaa tgcccgcagt gcaagaaact 595380
gtaaaagaac tcttcggcaa agagcctaat aaaggagtca accccgacga agttgttgct 595440
attggagccg caattcaagg tggtgttctt ggcggagaag ttaaggatgt tctacttcta 595500
gacgttatcc ccctatctct gggtatcgaa actctaggag gcgtcatgac gactctggta 595560
gagagaaata ctacaatccc tacacagaaa aaacaaatct tctccacagc tgctgataac 595620
cagcctgcgg ttaccatcgt agttctccaa ggagagcgtc ccatggccaa agataacaag 595680
gaaatcggaa gattcgatct tacagatatc cctccggctc ctcgaggcca tcctcaaatc 595740
gaagtctcct tcgatatcga tgcaaacgga attttccatg tctcagctaa agatgttgcc 595800
agcggtaaag aacagaaaat tcgtatcgaa gcaagctcag gacttcaaga agatgaaatc 595860
caaagaatgg ttcgagatgc cgaaattaat aaggaagaag ataaaaacgt cgtgaagctt 595920
cagatgctaa aaatgaagcc gatagcatga tcttcagagc cgaaaaagct attaaagatt 595980
ataaggagca aattcctgaa actttagtta aagaaatcga agagcgaatc gaaaacgtgc 596040
gcaacgcact caaagatgac gctcctattg aaaaaattaa agaggttact gaagacctaa 596100
gcaagcatat gcaaaaaatt ggagagtcta tgcaatcgca gtctgcatca gcagcagcat 596160
catcggcagc caatgctaaa ggtggaccta acatcaatac agaagatttg aaaaaacata 596220
gtttcagtac gaagcctcct tcaaataacg gttcttcaga agaccatatc gaagaagctg 596280
atgtagaaat tattgataac gacgataagt aatcaaaatt ttcaatttaa gtttctctat 596340
tcccatcctc ataagaggat gggaaacttc cttataaaca gaaaacagtt ccattctctt 596400
attctctgat caaggagttg caataacaga gcttctttag tacaattggc tttgaatttg 596460
agactgctcc tttcataatc acaaaaccca cttaaaaggg aaaattttgt tgagccactc 596520
agttcacgag tgaaactacg ggatttctcg ttcaatgtcc taaacttaca ggcggagccc 596580
aattgttgaa aaaaccaaaa agaaaaccag ggagaagaac atacggtaaa tccttgaaga 596640
tttttattcc aggaacccta tttgttcatg ctagaaaagg tttcggtttt gtttctcccg 596700
acaaccccga agaataccca tttgatattt ttgttcccgc ccgagattta cgcggggctc 596760
tagatggtga ccacgtgatt gtctccgtgc ttccctatcc aagagacgga caaaaactca 596820
aaggcactat cagcgaagta ctcgcaagag gaaaaacaac actcgtagga acgatcacct 596880
cactagtcag tcccacatca gcacttgcct acacaagcat gtcgggatcc caatctttaa 596940
ttccagtaga actccttccc ggacgcactt acaaaatcgg cgatcgcatt cttctgagca 597000
ctcctccctg ggtagataaa ccccaagaag gagcctctcc agccttacaa atgctcgaat 597060
ttattggcca catcaccaac gctaaagcgg actttcaggc aattcaagcc gaatataacc 597120
ttgccgaaga attcccccca gaggtcattg aagaagcaag ccttttctct caaaananat 597180
taacccaagt tctccaactc tcgcaaagat ctccgtgatc tcctctgttt caccatagac 597240
tcttccacag ccagagactt cgacgatgcc atctccctca cctacgatca taataacaat 597300
tacattcttg gtgtacacat cgcagacgtc tcccactacg ttaccccaca ttctcaccta 597360
gacaaagaag ctgctaaacg ctgtaactct acatatttcc cagggaaagt cattcccatg 597420
ttgccatcag cactctctga taatctctgc agcttaaaac caaacgttga tagactcgct 597480
gtatccgtat ttatgacgtt tacaaaatca ggtcatcttt cagattacca gattttccgt 597540
agcgtcattc gaagcaaata tcgtatgacc tacgatgaag tcgataacat cattgaaaag 597600
aaacactccc accccctctc aaaaatcctc aatgagatgg ccactctaag taaaaagttt 597660
tccgatatcc gtgaagaacg tggttgcatt cgctttgtcc tcccctcagt cactatgtcc 597720
ttggataatc ttcaagaacc cgtagctctg atagaaaacc accagacctt ctcccataaa 597780
ctcatcgaag agtttatgct taaagcaaac gaagtggtcg cctatcatat ctcccatcaa 597840
ggcgtttctc taccttttcg tagtcacgaa cctcccaatg atgaaaacct actcgccttc 597900
caagaanngg caaaaaacat gggctttgat atcacgttca ctcccacaca aagaacctga 597960
ttaccaatac cttttgcaaa ctacgtcagc aggacatccc ctagagcaag ttctacactc 598020
gcagtttgtc cgaagtatga aaacagcctc ctactctaca gaaaataaag gtcattacgg 598080
acttaagctc gactactaca cccactttac gagtcccata cgtagatata tcgatcttat 598140
tgttcacagg cttctcttca accccctatc tatagaccaa acgcacctcg aaattatcgt 598200
aagagcatgc tctacaaaag aacgagtatc cgcaaaagca gaaaattctt tcgaaaacct 598260
caaaaaaact cggttcataa ataaattttt gcaagagcaa cctaaaacta cataccatgc 598320

gtatatcatc actgcaaatc atgaaggact ctcatttgta gtgaccgaat tctgccatga 598380
agggttcatt gcagcagcag aactccctaa agaatattcc ctaaagaaaa acgctcttcc 598440
agaatctatc ccagataaaa tgaaacctgg agcttctaga aaagtcacta ttgattccgt 598500
gaatctcctt acgcaaaaaa tcgtctggtc tatagcgaca accacagaag ataaacctaa 598560
gaaaataaag aaaacgcctt ctaagaaaaa aggaacgaaa aaaagagcct cgtaacgtgc 598620
tacaagaaca tttttttcta tcggaagatg taattacact agcgcaacag cttttaggac 598680
ataaactcat cacaacacat gagggtctga taacttcagg ttacattgta gaaaccgaag 598740
cgtatcgtgg ccctgatgac aaagcatgcc acgcctacaa ctacagaaaa actcagagga 598800
acagagcgat gtacctgaaa agaggctctg cttacctcta ccgttgctat ggcatgcatc 598860
acctattgaa tgttgtcact ggacctgagg acattcccca tgccgtcctg atccgggcca 598920
tccttcctga tcaaggcaaa gaacttatga tccaacgccg ccaatggaga gataaacccc 598980
cacaccttct caccaatgga cccggaaaag tgtgccaagc tctaggaatc tctttggaaa 599040
acaataggca acgcctaaat accccagctc tctatatcag caaagaaaaa atctctggga 599100
ctctaacagc aactgcccgg atcggcatcg attatgctca agagtatcgt gatgtcccat 599160
ggagatttct cctatcccca gaagattcgg gaaaagtttt atcttaaata atcttaagct 599220
gtatagtaaa gaaacaaatg cccatttcct catatcttgc aggagaatca tgaaaaaata 599280
ctttattaca ggacttgtta ttctccttcc tctagcaatt actattgcta ttgttactat 599340
gatcatgaac ttcctaaccc aacccttcgt aggcttggct tcggaattct ttgacgaaat 599400
ttagctttta tactaaacat agagctcttc taaaattcgt attgcaaatc attttactct 599460
tcggtctctt tttcgccaca gtgctcctag gtttcctcac gagaattatg atttttaaat 599520
ccctactctc tatctacgac aaaatcttac accgaattcc catcattaaa acagtgtata 599580
aagctgcgca acaagtcatg actaccatat ttggatcaaa atcaggatcc ttcaaacaag 599640
tagttatggt tcctttccct aacgcaaatg ttcaatgcat cggtctcgtc gctggagacg 599700
cacccacagt atgctgcaca ggagaaaagg aagacgaccc cctcgtcacg gtcttcatcc 599760
caacaacacc caaccccacc tcagggtttc ttaccctatt tagaaaatct gatatcgtat 599820
tcctagatat gaaaatcgaa gatgctttca aatatattat ctcctgtgga gtcctctcaa 599880
cccccatggc atgcccctcg tctcccctcc ctgacgagct acaccaagat caaggcagct 599940
aaaagacgtc atcttcttga aaaaaaactc ttcttttact atcctttttc ttataaagta 600000
ctccgtatcc tcaatttccg ttgtctggag aaattaaaaa attcaatatc gagttattta 600060
tgacacgaat gagtaaacaa gctcggcgca gagcgaaaag tcctaaaaaa cgtaaaccta 600120
agtatgccat tgtgcatcca gcgccagtcc aagaattgta tataaattgc atacgaatgc 600180
attgagcacc agtgatagca tttttatccc gaaaataggt taatttctag agtagaatta 600240
tgtctcgaca tcgtagttat ggtaaatctg tcaaaggggt taccaaaaga aatgttttga 600300
agcgttttga gcgagtagaa gtcttgcgta agttgggccg ttggaatgat agtacagcga 600360
aaaaagtcac aggtttacct aagaccccta ttttaaaata agtttgtttt tctctatggg 600420
aatttcattc gcgtgacgca agaaaagatc aaaatacatg tttccaatga gcaaacatgt 600480
attcctattc atttggtttc tgtagagaag ctggttctta cgctcttaga gcacttaana 600540
gtaacancta atganatttt tatctacttc ctagaagata aagctcttgc agaactccat 600600
gcataaggta tttgctgatc cttctctaac agatacgatc actctgccta ttgatgctcc 600660
cggagatccc gcttatcctc atgttttagg agaagcattc attagcccac aggccgctct 600720
taggttttta gagaacacat ccccaaacca agaggatatc tacgaagaaa tctcgagata 600780
cctcgtccac tctattctcc atatgctcgg atacgacgac acctcatcag aagaaaagag 600840
aaaaatgaga gttaaagaaa atcaaatcct gtgtatgtta agaaaaaaac atgctttgct 600900
aacagcttaa catgctccat attcttttag ccatattctg tattcttcta ttcctagcct 600960
tcgggcttac gcaaccgtcc tgtcacggat cctcaaaatt cctaaaaacc ctaaaccaac 601020
gcttcttcac agataaagga agagagtatc ccccttcccc cagtgctcct acaattctcg 601080
ccacgctgct ctgcatcctc tatggagctc tcgggacaaa actctatacc ctcctccctc 601140
caaaaacagc tcacaaagat ctcctattct ggcccctata ctctctaagc gccctgatag 601200
cttacggatt cctcccccca tggatctcta caaaagtccc taaagaaacc accgcccacc 601260
tccgttttct agcttcggta ttccaactcg gtctcttccc actgcaactg ctcttttaca 601320
gacgccgccc taaccaacaa gtacgatctt caacatcatt tcaaagccag ctctccgaag 601380
ccctctccgc ttttgataac ctcattgtcc gtgaagtcat gatcccaaaa gtagatattt 601440
tcgcacttcc cgaagaaact acactacaag aagctctggt tctcgtaagc gaagaaggct 601500
acagtcgcgt tcccgtttat aaaaaaaact tagacaacat cacaggaatc cttcttgtta 601560
aagatctctt actgctctat acaagcagcc acgacctcag ccaacccata tcctcagtag 601620
caaaaccccc attctatgcc ccagaaataa aaaaagcctc ctctcttctc caagagttcc 601680
gacaaaaaca tcgccatcta gccatcatag tcaatgaata cggattcaca gaaggcatcg 601740
ctaccatgga agatattatc gaagaaatta taggagagat cgcagacgag cacgacgtac 601800
aagaaaatac tccttataag aaaatcggaa gctcttggat tgtagatgga agaatgaata 601860
tctccgacgc tgaagagtac ttcaatttga aaatcgatca tgaaaatagc tacgatacac 601920
taggaggaca tgtcttccat aaagtgggtg ctgttcccca aaaaggaatg cgtatccatc 601980
acgaaaactt cgatatagaa atcattacct gcacagaacg aaatgtcgga aaactaaaaa 602040
tcacaccaag aaaacgtaaa ttcaatatct cctaagaatg taaacatcct aggaccgatt 602100
tccttgcact ccacgatccc atcctgcctc cccccaatcg aaattctcaa acatcaccaa 602160

taaaaatctc tttgcaaaaa agctaaagat ttatcagagt gaaaacttaa gctcccgtat 602220
agttaggaga actatctaga tgagtgatat ccaaaaagaa gaacacggct caacaacaat 602280
ctttcatctc cacggaaaac ttgatggaat ttcttctcca gaagtacaag aaaatattta 602340
ccaatcccta gcagctggat ccaaaaatat cattctcgac tgtgctcacc tcgattacat 602400
gtccagtgca ggtatccgag tcctactgca aagctaccat caagtaggac aacattctgg 602460
gaaaattgtc ctgactacag tcccaaaaac catagaacaa actctctatg ttacaggatt 602520
cctttcttac tttaaaatat tcaatactgt ggatgaagcg atacaaacac taaacaaaga 602580
cggggattga gaaaaacctc actgttagta tatgatggcg cttttaagtc atataaagcc 602640
tccctcttac tatgcgacga tctgtttgtt acgttaaccc ttcgatagct cgagcagggc 602700
aaatttctac ttggaaattt ctttattccc ttgccacacc actaccagct ggaaccaaat 602760
gtaaatttga cttagcagga agtgggaaac ccacagattg ggaagccccc gcgacagatc 602820
tctcccaaac tagaaacgta atctacgcag aaatgccaga aggcgaaatc atcgaagcaa 602880
ccgccattcc tgtaaaagac aatcccgttc cacaattcga gtttactctc ccctacgaac 602940
ttcaagtagg agaaaccctc actattgtca tgggagcctc tccaaaccat cctcaagtcg 603000
atgatgctgg gaacggagcc caacttttcg cacaacgtcg caaacccttt tacctctaca 603060
tcgatcctac aggagaagga aactatgatg aacccgatgt cttctctatg gatatccgcg 603120
gaaacgtcct aaaaaaaata gagatcttta ctccctccta tgtcgttaaa aacaaacgct 603180
tcgatatcac cgtgcgattt gaagacgaat tcgggaacct caccaacttc tctcctgaag 603240
aagacccgaa tcgagctttc ctacgagcat cttagagaaa atttaaattg gcagctcttc 603300
atcccagaaa caggctttgt tattcttcct aatctctatt tcaatgagcc tggaatttat 603360
cgcatccaat tgaaaaacct ctctacacaa ganattttca tctctgcccc tatcaaatgt 603420
ttcgctgact ccgccccgaa tcttatgtgg ggtctcctcc acggcgaatc cgaacgcgtc 603480
gactctgaag aaaatattga aacttgtatg cgttatttcc gagatgaccg cgctctgaat 603540
ttctatgctt cttcatcatt cgaaaatcaa gagaacctct ctccagatat ttggaagctc 603600
atcaatcaaa ctgtctccga ctttaatgaa gaagatcgct tcatcacact atccggattc 603660
caatatagcg gagaacctca tctcgaggga gtgcgtcaca tccttcatac caaggaaaca 603720
aagtcccact cgaaacacaa agaatacaaa catattcccc tcgccaagct ctataaaagc 603780
actgtcaacc acgacatgat ttctattcct tcgttcacag cttctaaaga acatggtttt 603840
gactttgaga atttctaccc cgagttcgaa agagttgtag aaatttataa tgcctgggga 603900
tcttcagaaa ccacagccgc tctaaacaac cccttcccta tccaaggtaa agatagcgaa 603960
gatcctcgag gtacagtaat tgaaggatta aagaagaatc tccgcttcgg atttgttgct 604020
gggggtctcg acgatcgagg aatttataaa gactactttg actctccgca agtgcaatat 604080
tccccagggt tgacggctat catttgtaat aaatataccc gagagtctct tgttgaagct 604140
ttattcgcac gtcattgcta cgctacaaca ggacctagga tcgtcttaag cttcaacatc 604200
acttcagccc ctatgggctc cgaactctcc acagggtcga aacctggact caacgtcaac 604260
cgtcacatct ctggtcatgt ggcaggcact gccctactca agactgtaga aatcatccgc 604320
aatggcgaag ttctccatac cttcttcccc gatagcaata acctggacta tgaatacgat 604380
gatatggtac ccctaagttc agtgacccta aaagatccaa acggtaaagc accttttgta 604440
ttctactatc tcagggtcac tcaggcagac aatgctatgg cctggagttc cccaatctgg 604500
gtggatttaa attaagaaac tatcctatct aaggatttct tatgatgaca ctttttctcg 604560
taatctgctg tgccacagta ttgttaggtc tagggatggg tattctactt gtaggttccc 604620
atttgcttgg caggcccctc tctaaagggt gtcaaaaacc agattgctgc caaaaaaaaa 604680
catgtgacaa aacagagcac tgtgctacaa aatctcgaga gaatagtaca tcaaagtgtt 604740
catcaaatga cgacgtgcct cccacagccc cctaaaactt ccccccccta ctccatattc 604800
gaaaaactgg atgcccaaga acgattaagc agtgaagacg ctcttcatct cctcctcctc 604860
acgaataaag aagatcaacg cacactctgg aattttgcag accaagttcg caaacaacgg 604920
gttggcgaca ctgtatacta ctcctcaacc ttgtacctct atcctacaaa tttctgtgac 604980
ttcagctgca aattttgctc tttctatgca aaacctggag accctaaagg atggctctac 605040
tccccagatg atcttctaca gcaaatccaa aatataaaaa ctccaattac agaagtacat 605100
atcgtgggag gctgtttccc ctcctgcaat ctgcaatact attccgatct atttactaaa 605160
atcaaagagt acgatcctca gatccatatc aaagctctta ctgccattga atatgcctat 605220
ctctcagatc ttgataacct ttctattcgc gatgttcttc tcacattaaa agatgcgggt 605280
cttgattcca tccccggagg aggagctgaa atcctcgtcg acaaaatacg taatttctta 605340
gctcccaaac gcctttcttc ttctgatttt ctcaacatcc ataagatggc tcatcaactg 605400
ggaatccata gcaatataac catgctctgc tatcataaag aaggacctga agacctcgtc 605460
acccacatgg taaaagtccg cgacttacaa gacgaaactc agggctttaa aaacttcata 605520
cttctaaaat tcgcccaaga aaataatgtc ctaggaaaaa gattaagaaa atcaggccag 605580
ggtcatgcca tccctctaaa atctttaatg gcagtagccc gaatcttctt agacaacttt 605640
tccaatatga aagccttatg gaattaccta ggtattgagg cagctctaga cctcctttcc 605700
tgtggtgcta atgacctttc ttcaacacat atgggggaaa aggttttcca gatggcctca 605760
tctaaagaac ctattaaaat ggacgctgag ggaatggcgg ccctcatcac acaacaaggg 605820
agaacgccat gtctaaccaa ctccagccat gtataagctt aggctgcgta agttatatta 605880
attcctttcc gctgtcccta caactcataa aaagaaacga tattcgctgt gttcttgctc 605940
cccctgcaga cctcctcaac ttgctaatcg aagggaaact cgatgttgct ttgacctcat 606000

ccctaggagc tatctctcat aacttggggt atgtccccgg ctttggaatt gcagcaaacc 606060
aacgtatcct cagtgcaaac ctctatgcag ctcccacttt ctttaactca ccgcaacctc 606120
ggattgccgc aactttagaa agtcgctcct ctataggact cttaaaagtg ctttgtcgtc 606180
atctctggcg catcccaact cctcatatcc taagattcat aactacaaaa gtactcagac 606240
aaacccctga aaattatgat ggcctcctcc taatcggaga tgcagcgcta caacatcctg 606300
tacttcctgg atttgtaacc tatgaccttg cctcggggtg gtatgatctt acaaagctac 606360
cttttgtatt tgctcttctt ctacacagca cctcttggaa agaacatccc ctacccaacc 606420
ttgcgatgga agaagccctc caacagttcg aatcttcacc cgaagaagtc cttaaagaag 606480
ctcatcaaca tacaggtctg cccccttctc ttcttcaaga atactatgcc ctatgccagt 606540
accgtctagg agaagaacac tacgaaagct ttgaaaaatt ccgggaatat tatggaaccc 606600
tctaccaaca agcccgactg taaaaagatc ttcgattcca tagcgagtaa gtatgatcgc 606660
acaaatacaa tactctcttt aggaatgcac catttctgga atcgctcttt gatccagatc 606720
ctagggtcgg gatactctct cctggatctc tgcgcaggaa caggaaaagt cgcgaagcgt 606780
tatattgccg cacaccctca agcatcagta actctcgtcg acttttcctc agcaatgctc 606840
gacattgcaa aacaacacct tccccagggc tcttgctctt ttattcatag cgatattaat 606900
caactgccct tggagaatca ttcttatccc ctagcagcga tggcctatgg cctcaggaac 606960
ctctcggatc cacataaagc cctacaagaa atctcccgag tgcttatgcc ttctggaaaa 607020
ctgggcattc tagagctcac acctccaaaa aaaacacacc ctacctatag tgcccataag 607080
ctctatttgc gtgctgtcgt cccctggatt ggaaagtctg tttctaaaga tcccgacgcc 607140
tatagctatc tcagcaaaag tatccagcaa cttccaaagg accacgatct tgaagaccta 607200
ttctctaaat caggatttta tattgcgaaa aagaaaaaat tgttcctagg agcggctacg 607260
atttggctac tagagaaaca ataaaacatg gaggatctcc aagcttggtc gcaaggagct 607320
ccctaagatt cgtggagtgc tctaccccac gctaaactaa aatggtaaac ctacctctgg 607380
gaagactctc atcagcaaaa ctcccgacaa gcttcgtcct gacacaactc ttaaatcgct 607440
gttttagcat agcagtcttg ctaggttcgt tatcgataat aaaagcaccc tcttcatccg 607500
aagactcaaa gaactgcata atctcaccag aagcaactaa actctccaaa agctctacaa 607560
gctcggtacg tctttcacca actcctctaa ggacaaatct cccaaacggc tgattctccc 607620
aatatccaga aatacgtcca gcctgcacca gaccctcgag taccgaccga atctctccag 607680
cttgatcagg atggtcatac caaccttcga ttccaggctc taggacccgt agccccttaa 607740
agagaatctc ctcatcagaa gccaccaaaa attccgttaa gatgttattt ttaggcttag 607800
actcgaattc cgatttaaag aggtctagtt ttgacgtagg aagaggttta gctgtacagc 607860
tcctccctac gttaaagctt tgcaagagcc atgccactaa agaattgaat actttaatac 607920
gtgcttgtga ctgagttgcc gccagccctc gggcaagaat catattccct aactctttgt 607980
ttaagaaaat ttcttctaat ccttctttct ttgttttaaa gaaaatttta tagagcctct 608040
ccgaccaacg ccaattcgta cgccaattct caggtaaatc aggaacgtgt ttctgtattt 608100
catccttaga aagttcataa tcagctaaat agccattata caagtccaac tccatacgac 608160
gcgcacgagt agcttctaaa atcagacttt tttcttcatc tgtaaacatc acctcaggtt 608220
ctttaggcgg ctctggatag ggaacatttt gaactacaag cgcatacctc agagaaaaac 608280
tcggctcctc ctctgtccct ggtgtagaat ccacggtagc ctcagccgcc gcagcctctg 608340
aaaccccaga tgtggtgtct gtagttcccg aagctgcagg catggtatct gttgtattcc 608400
ctggagctac aggtgaagac tctacagtct cggaagctgc aggtatctct tgagtaggat 608460
tggcgttcac aggaaccatt tccgaatcca caataacagc gtccttacta cctaaacttt 608520
taggaaactt taggcgcttc attgtttctt ctaaccaaac tagccgctcc tcagaaccaa 608580
ggataggggc ccactggacc aaagtactac atagccggcc gtgggctgga gaaatttgag 608640
aaacttgtgg ctcaagagaa cgtatcccaa tagcgggccc agcctcctca taatcacttt 608700
tcacagagag gttatcccac tgcaaagcaa aaggacgcct ttgaagaata cataatttta 608760
atatgtcatc caatcccgaa attatagaaa aattcctttt agcaaactcc ttacccttcg 608820
caccggaagt gtgctgcggg acaaagaaag gccctatagg caagcgctca ttaggaacta 608880
aagtagggaa gtcacagagc ccccttgcca ccatacatac gtaataggcc ttcgcgatag 608940
ccctccccag gacggctagg ataatcttca cgaaccatga cactcggaat gatatcgata 609000
ttcttcaatc ccattttctc tataatcaac ttagagtgga tcaaccggta gcaatctacc 609060
atagcaaaat gggcgtcgta atcagagcgg aaagcctcac aaatcgtatt gccttcctta 609120
gtcctcagcg atgacccttg tttttcaata acaaagcaca aagaactgac gtgaggccta 609180
aaatcccgaa tcaaacaggc ccaatccacc aaaggctcgt tcggtggctc agggctgaaa 609240
tgctccataa ataggaaaat tttagcagct aaggcatggt taagaccatg gttctgaaat 609300
ggagagaata acgtccttac tataggaatc tcgcctgtgc ctaaagatga tcgtcctaaa 609360
tcccaatggc ggaccccctc gtagtaaacg gagcgagcgg gcttgtgaat ctcaaaagct 609420
ctcttatttt gccggtaact cgtaacagtg ctataagtaa gataacttat aattgcaacg 609480
tcaccaagaa gtgacatcac aaaaagaatt aaccccatta ccgtaaaaga ggtcgccaag 609540
gccataatgc ttggaatcaa actagagagg gccacacaaa ccaaaatgat caacaatgcg 609600
cataaaaaga tgacacttgc ccgaactaga cgtgtgctca actgattctg actcgtatcc 609660
tcaagatacg tctttaagct acaggtaacc cgagtcggct ctattcttcc gcagctcagt 609720
agtgatgaca tgatgaacta ttcactgttt cttaaattag ctttaaaaat tttaacagtt 609780
ttttaattaa aaaaatgact aataataaat agctattgtt aaaattttaa caaaacaata 609840

aatagaaata agaaagattt gtaataagat catgtaaaga agcaataaga acagcgtcta 609900
aaaataaact acttcacaaa atagaagatt aagaagattt tttgcggcct cgcttgctcc 609960
ccttcgtctt agtgaccact ttctcatcat catcaccaaa gaaatctcca gtagaaagag 610020
agtcgtaacc aagcttagcc gaaagctctt taagagaagc aaacctctct gaaagattgg 610080
taagattcag ctggtcttca acacgagtcg aaccgcgcaa gttagcaaga aattcttctt 610140
ccgtatcaaa tttttctgag aaagtaattg aagagggtga agtaatctcg ggtaagaaaa 610200
ggaaacgacg agcagcctga gggacttttg tataaatatc actcgaaatt gttgtttctg 610260
tttctacctt agcagattgc ttcggaggac ggcctcgttt cggacgaggg tttaatgctt 610320
cattggaata gtaaatctta gctttgttcg acaacaactg acgcgcttga gtgatttctt 610380
tccccacctt ggcatcaaac aaatgtattt tcaactgttc caaaacattc tttgtgaaat 610440
ctagatcttc ttcaaagaca aaatggaact tattattgcg aagccattct ataattcgaa 610500
ttcgagaacg ctctacataa aattgctgcc acttttctaa ctctgcctcg tgatcataaa 610560
taaactctaa aaactgctca cgggcattct tggattgcaa aatctcaagg aacttttctt 610620
tggtatcgat atcataaatc ttttcattga taaacgtttc catgattttt tttacttcat 610680
aaaacgtcaa cttcggaatc aaacaatacc gctcggcatt ctcttctaat tcttggtaaa 610740
tcttatttag atcctcttgg tctttatcta aatctatgta gagaataaac ccttcaacac 610800
gatctaaata aaagtccctc tcatcgtcag acttagagaa tgcatccata agacgaagga 610860
ttcttaaaag aagtgggttt tgtggtacag gatacgtcat cataaattac gattatagaa 610920
aaatcccagt tagagagcaa gatctatccc taaacaagat tttaaaatcc ttgcatgaca 610980
aaatcaaaaa tcacatctaa ttataaatct ctagttacag agccctgaag atatacccgt 611040
cccctatttt gactcacagt cataagctct ccaccccaag tatggatttg gatcgactcc 611100
ttccatccat aggagtttga cacaacaaga gcagaagcta gagcccctgt tccacaagct 611160
gcagtttccc cttcgactcc acgttcgtaa gtacgaacgc gcaactggca atgtcccagt 611220
atctgaacaa aattgacatt caccccatct ggagagaagg tctgatgata gcgaagaaaa 611280
ggacctaaga tagaaagatc taaagtagaa atctcaggaa gaattacgac agcatgaggc 611340
actcccgtat ggatacaaac gatctctttg ggaagaggat caggacgcga ctccaatcga 611400
tgaacagaag ctctccaatc tgcgagagtc atatctacaa gcacacgatc ccaagaataa 611460
aaatatcctg aatatagacc actatccgta gatacagaga tgtccgattt tcccttctga 611520
gaagctaagt gagcaatcgc acaacgcaag ccgttaccac acatcgttgg acgtgatcca 611580
tcggaattaa aaataatgag ttgcgcatca gcacaagaag agggctttaa atataaaaaa 611640
ccatcaaccc tcgtctcttg gcataagaac cgaacatctt caacctcagg aagtgtttca 611700
ccaagaagga aacgatttcc tgctccagaa tagataaaat atttagagat cgttgaagga 611760
gaataaaatg ccatccaata aaccaaaatc cttagcttcg ttggctgtca tccacatatc 611820
tctatcgata gccttttcta tgatatctcg aggttgattt gtcgcctcta catagacatc 611880
tataatgcga gcttttgttt ttaaaatctc tctcgcatga atgtctaaat cggttgcctg 611940
accggtaatc ggtccaccta ttgaaggttg atgaatcata attctagaat gaggagttgc 612000
aaatctcctt ccaggagctg cacataaact caataccgag cccatagaag ctgccaaccc 612060
tgtcacaaca gtagtgacgg gtgaggttaa cattttaatt tgatcccaaa cagcaaaacc 612120
tgcgtccaca gatcccccag gactattgat cacaaaaact ataggctttc caggatcttt 612180
taattccaaa taccaaagct ttttaattgc atcggaagca cttttctctg ttacaggctc 612240
tgagaaaaat actctgcgcg cttccaataa ctctttttct ataatatcac gtaatttatg 612300
aacttccccg tctgccataa aataatgtct ctcctaacta aactaaagct tctaaatcaa 612360
tttcagggta gagcgggaaa cgcagcaaca agttgcgaac acgatctcta gcttcctgcg 612420
ctatggcttc aggaagttcc cctttatttt tcttagaact cccttcaaca tggcaactta 612480
aacgaatatt tcgcaatact ttcacaataa tatctgcaac ttcttccatt tcatcgatac 612540
ccatacccaa agtcgttagt gcaggggttc ctaaacgtat acctgaagtg tcccacttac 612600
caatagcatc tgaaggtaat gaattccgat tcacagcaat tcctacggaa ctcaagatat 612660
cttcagcaat ttttccagaa atgcccaaag aacctaaatc aatcaccatc atgtggttgt 612720
ctgttcctcc cgtcaaaaga cgtagcccat gacttaaaaa tctctctgct aatcgacgag 612780
cattatttac aacctgatga gcgtatttct tgaaatccac agagagagct tccttcaaag 612840
ccactgtttt agcagctatc acgtgaggta gaggacctcc catcatcaaa ggacacgcct 612900
tattgagagt gctttcatac tctcgagttg ccaaaactaa tcccccgcga ggaccgcgta 612960
atgtcttatg cgttgttgtt gtcactatat ctgcataagg aataggattt tcttcatcaa 613020
caaacactcc cccagcaact aggcctgcaa aatgcgccat atctacccac aagacagatc 613080
cacaatcctc tgcaatctgt tttaaaactg caaagtttaa tcttcgagaa taggaagaat 613140
atcctgcgat cagtacctta ggtttatact ccttagctaa ccgggagatc tctgcataat 613200
caaaacactc cgtatccgga ttgacatcat aggggaagca acgcataagc ttagacatca 613260
catttaaacg tacgttccca tgggtcaaat ggcctccaga atttaatgaa ggtcctaagc 613320
aaacacaaga agacatttca gccttaagta gagtgtattc ttcttctgtt aattcgttta 613380
cagttttata acctaactta ctgacagctg ggccttggac tttgtgcgtg agaatggcca 613440
ttactgccag taaattagca tcagccccag aatgaggctg aacacaagcg caatccgcag 613500
caaaaagttc tttcgctgtc tctacacact cccactcaat agcatctaca ttttcacaac 613560
aggaatagaa acgcttaaag ggacttcctt cacaatactt atctgtgagc aagttcccca 613620
tagccaactg cactgaaagt gaagaatagt tttcagaagc aatcatcttt aaatgggaac 613680

gctggctctt caactcatca atgattctct ccccaatgga aggaaacgca tttaagagat 613740
ggtcaagagc tgctaaatac gctgtcgaag ctaaactttg tccctttttc cccgaagcat 613800
tttctaaaaa cttatgcaac aacgaaacca ccgcaaccac ctttgtaaat atttccacta 613860
ttgcgaattt cttaaatttc tcaaaaactt ttagcaagga cttctaaaga cgaagcctta 613920
atagagagaa cctctaagtc cagaaaaaac cagcctagct ttcaaagtga aaaatgtcaa 613980
tcaacgcatg atcaagaaga actaagggaa aaagaaaaat tattttcaat atatcccttt 614040
gaaggtacaa aaacttaggc agccttcctt ttacccgaaa agactcatga ccctctactt 614100
aggattgaat caaaaaaccg ctcgtaaata ccaagctcat tatttgccta ttctaactct 614160
cttcccctat gcaaaaagca ctccacaaaa taagcgtgct cttcaattcc ttccacaagc 614220
aacccatgtg attctcacaa gtccctcatc cactcaccta ttcctttcca gaatgacttc 614280
tcttctttct aaggccactc taaaaacaaa gacctacctc tgtataggag agtccaccaa 614340
agaaagactt ctctctttcc ttggacaagt gaagtacgta gtagcaactc aagaaatcgc 614400
tgaaggcatc ttcccattgc tacaggcact gccctcttca gcccgcattc tctaccccca 614460
ctcctccctc gcaagacctg tgatcagaga atttctttac aatcgattta cttttttctc 614520
ttaccctcac tacacagtga agccgcgaaa acttaaaaaa aatattttat ctaaatacaa 614580
aaaaattatc ttcacaagcc cttcaactgt aagagctttc gccaaaatct ttccgcgatt 614640
tcctgaaaaa acctactggt gccaaggaag gatgaccttg caggagtttc aaaagttctc 614700
ctctcaaaag caggtatctt tgttagaaac gcttgggaag tccaggacat ctccgtgaca 614760
aaaaagaccc cactacttta aaatgcttta tgctccgaaa aaaacttaat ttttaatagt 614820
ttatggcttc ttcagctact cccggttttg atggaacggc tccctctcta tttcccccag 614880
ctactcgtcc tcgctataat ttcaagcttg ccctcttcgt tactattgcg attgcactcg 614940
tctggatagc tctgattgct accaccatag ctattgggct atgtatccac cctttgtgct 615000
cctttatctt cctaacagca attcccttat actttatatc tcgctatatt tgctcccact 615060
acgcacgcaa tgtctacata gctctagatg tcgtccccga tcattctaaa ttgcaagaca 615120
tgcgctctca ctctccaatt ttctcggatc gataaacaaa aatttgttaa aattatagaa 615180
agtattttac aaaaaaaatg ttatgtcagg accctcacgt actgagagct ctcaagtttc 615240
tgtactatcc tatgtgcctc gggataaaga aattgctcct aaaaaacagt ttaccatagc 615300
aaaaatatcc actcttgcaa tcctagcttc tttagcttta ggagctttgg tggctggaat 615360
ctctttaacg atagtattag ggaaccctgt atttttggct cttctcatta ccacggccct 615420
cttctcagtt gtaaccttct tagtctacca ccaaatgacc tcaaaggtat cttctaactg 615480
gcagaaagtt ctagagcaaa acttcaagcc tttgggaaaa gcgtggcaag aaaaaaacgt 615540
agactgcnac tcaaacgaga tgcaatttta caataatcac ctgaacccta agttcaaggt 615600
agcgatacaa acagatgcgt ntcaaccatt tcagcctact ttcttaactg gacttagagt 615660
gatcgaaaaa aatcaatcca cagggatcat ctttaatccc gtaggcccaa cgaatctgat 615720
cgacaacact gcaacgaacc tctctactat cctttactcc accctaaaag ataaaagcgt 615780
gtgggataca tgcaagcaac gcgaaggggg tcccgcaaaa ggagaagacc ccttttcccc 615840
taccgaagtg agagtagtaa aacttccaaa cgaagctcta gatcaaacgt ttaatctaaa 615900
tttaagctct gcagaaaaga aaagtattct tccgaccttt ttaggccacg tatgcggccc 615960
taaatctgaa gagttaccaa atcagcaaga atattatcgc caagctttac tagcgtacga 616020
gaactgcctt aaagcagcta tagaaagtca tgcagcaatc gttgctcttc ctctctttac 616080
ttcggtctat gaagtgcctc cagaagagat tcttcctaaa gaaggcactt tctattggga 616140
caaccaaact caagcgtttt gcaaacgcgc tttattggac gctattcaaa atacggccct 616200
acgctatcct caaagatctt tacttgttat actccaagat ccttttaata ctatagaatc 616260
acaaagtcgt tctgaggagt aacccaaagg gtttcaccaa caacccttta taaagtttag 616320
ctaaaaattt tctcaacatt taaaagtata tttttatttt ttactaaaaa tgattaaatg 616380
cgctttggaa gtgttcttac aggaacaatt tacaagtaaa aattatatga atggctagac 616440
attagttcta agaaaagggc atttcgcttg ctcaaattta taagaatttt ccttacaaaa 616500
aaacatgctt tacttcttgc ctaaaaagag gtaaactgta tttttcttca catgtatcgc 616560
attcctaaac tccatagtga tttttggctt tcttaggaaa cttaagtaag gaataatttt 616620
catgcatgac gcacttctaa gcattttggc tattcaagag cttgatatta aaatgattcg 616680
ccttatgcgc gtaaagaaag aacatcagaa agaattggct aaagtccaat ctttaaaaag 616740
tgatattcgt agaaaagttc aggaaaaaga actcgaaatg gagaatttga aaactcaaat 616800
tcgagatgga gagaatcgca tccaagagat ttctgaacaa atcaataaat tagaaaatca 616860
gcaagctgct gtaaaaaaaa tggatgagtt taacgctctt acccaagaaa tgactacagc 616920
aaacaaagaa cgtcgctctt tagagcacca gcttagcgat ctcatggata agcaagctgg 616980
aggcgaagac cttattgtct ctctaaaaga aagcttagct tctacagaaa atagtagcag 617040
tgtcattgaa aaagaaattt ttgaaagcat caaaaagatt aatgaagaag gcaaagcttt 617100
gcttgaacaa cggacagagt taaagcatgc gacgaatccc gaactactca gcatctatga 617160
gcgtctatta aacaataaaa aagatcgcgt tgttgttcct attgaaaatc gtgtctgcag 617220
tggttgtcat attgttctaa ctcctcaaca cgaaaatctt gtaaganaga aagaccgact 617280
cattttttgc gaacattgct ctcgaattct ctattggcaa gaatcccaag tcaatgctca 617340
ggaaaattcc acagcaaaac gtcgtcgtcg tcgcgcagct gtataaagtt aatcggaaga 617400
gaaaggcaac cgctgtttat atttctcaaa aaatataaag agaggaaagt ctggacttca 617460
taagagaaga tactggagaa attccagggg ccgtaaggct acggaaagtg caacagaaaa 617520

cactccgcta taaaatttat tttatagaca ggctgaaaat tcctacttta ggagtaggag 617580
ccattaaggt gacttaatag gcatgcaaac cctatctgaa gcaagagaaa aaagcttttt 617640
gtgtctgcaa atgtgagagg aattcctccc ataggctttt tcgaaatcgc ttgagggatc 617700
tagtaatagc tcccctagat gaatggttgc ccttaggata gttcgcaaga gctatcttat 617760
agacagaatc cagcttaccc tctcttccga tatttttctc atattctcaa agtagaccag 617820
ctttggctaa agtgtgcata tgcaacaagc cagtcacatg tcggttctcc tcgttatcta 617880
aaacaggaag aacagctaca ggactactag attccataag ttgcaaagca atggcaatat 617940
ccgaatcttc agtaatacat cgaggatttg ccgtcatcac cttctccaaa gataacgaaa 618000
gcacctcccc cccataggaa gctaaagaac gccgtaaatc tccgtctgta aaaattccca 618060
taagtcggaa ttgaggatct actatacaaa cacaaccaca accgtaagca gaaaagacct 618120
ctaaagaaaa actcacttta tctccaagat gacagaaagg tacctctgtc tttgggaaca 618180
taaaatcttt aaccttacca ttagccttca tcccaacctg cccactagga tggtttttgc 618240
cgtacgtaga taaagaaaca ccacgactat gaaaaaggag catagctaaa aaatctccaa 618300
agatcatttg gcatgtcgta gaatttgtag ggattaaatt aaaaggatct aattcagcaa 618360
cagagggtaa aataacgact aagtccgaga gagccgctaa attagaataa ggcatggaag 618420
taatcgcgac aagaatcgcc cttcgactct ttagatgagg aaccgtatct agtaactctt 618480
gggtttcacc acttttagaa aataaacata caatgtctcc aggactcaca agaccaagat 618540
ccccgtgcag aagatccaca ggagaaaaga acagagcacg ttcacttaaa gattggagtg 618600
tagccactaa ttttcgtgct acacatccac ttttccctac accagaaaaa aatacccatc 618660
ccgaatgacc gagtattttt tctgctaatt gcattgcctc tttgggttga aaggcttgga 618720
aaaaaaaatc tacagcttct ttttgcttac ctagaatgtc ttggcatacg tcagtagaaa 618780
tcatcgggga aggcattcgg ttctctttgg attggctaca ctttagccca tcgtaacaga 618840
ctccaaacga tttttcaatg aggttaaaaa ctcactgccg taaataccat ccaatactct 618900
atggtcaaag gtaagtgtca catagaccat tttgcgaatg gctaaagaat cgtcatcacg 618960
gacgacaaca cgtttttgta ttgtgccaat tcctaaaata gcaacttcag gataacgtat 619020
gatgggcatc ccaatcaaag ctcccgtcat tccaaaattc gtgacagtaa cgctgccatc 619080
ttgcacttca ctaggatcca atttatttaa ccgagccctt gaagatagat ccgccaaggc 619140
ctttgcaata cttactaaac cgcgatcttg acaattgtgg atgacaggaa caacaacccc 619200
ttccttattg aggttcacgg caacgcctac attcacagat ttcttcataa caatggtagt 619260
cccatctaag gaaccattca ataaaggaaa ctgccttaaa gtctgagcta aacactgtac 619320
aatgaaactt gtaatcgtta gcttcacccc atgcgtatct aagaagcgtt ggcgttcacc 619380
agaaatcaga ttcataagat ctgtgacatc gacatcaaca accaaagatg cgtgaggaac 619440
ctcatctgaa gacttggaga gagaagaagc tattgcccga cgtagcggag acataggaat 619500
gcgattcact tctccttgaa atatttcggg aatagaaact tgttgcgatt ctgaaatata 619560
cgcttctaaa tcctgacgag tcactcgtcc ccctttcccc gtgccggcaa tcttttggag 619620
gttatcaaga ccaatgcctt cacgttgagc taaactcagc acggcaggag aaaaccatac 619680
cgaagaactg ctggaacccg cctccgactt tgtttcacaa gaagtcagag gacatgaggt 619740
gctctcatca tcagcttcgg aaatctcctc aagctctatc aatcctaaaa catccccaga 619800
agcaacctcg tctccctcat tgacgcagaa acgcaccagt cggcctgctt taggagaggg 619860
taattctgta gcaattttat ccgtagatac ttcaatcaga ggctcatctc tagctacatg 619920
atcacccaaa ttttttaacc aacggactat agatcctccg gaactcgtct ctcctatttt 619980
agggaatcgg aactcaaata tcatgtatcg ttaccttcta tgtagtttca acgctttcct 620040
gttttattga ttcataagga gaaagctctc cttctccaga agctacataa gtagcaacta 620100
cagcatcccc aagaatattc atgggtgtac ctacaatatc ccttaaccgg tcaatcccag 620160
caagtatagc gataccctgg ataggtaaac ctacagaggc taatacggaa cccagagtaa 620220
tcatacctcc tccaggaaca ccggcactac ccacggcaga gaatgttgcg gtaactacca 620280
ataataacag actgctcaag gatagcggac aattgtatgc ttgagcaatg aaaaccgctg 620340
ccataccctg aaaaattgca gttccattca tattcacagt ggcgcctaga ggcaaaacaa 620400
aaccagaaac ctcagcggac actcccaaat ttttagaaac acaacgcatc gttacaggta 620460
aagtcgcaga actactcgct gtagataccg cgcaagaaat cgcatccatc attgaagaga 620520
gaaacttcga aaaggacatc ttgcaaccaa aacgcacaag tcccccaaaa acaagcgtag 620580
catggaacaa acacgccaga tagtaagcaa tgataaattt gcctagctgc caaaggactc 620640
ctaaaccatg atttcccgaa atccatgcca tgctagctcc cacaccatag ggagcaaaac 620700
tcatgatcat atttaccatg cgcaacatga tttcagaaaa accatcaata aaacgctcga 620760
cagggcggcc acgttctcca gaaagccgaa gggcaattcc taagaaaatc gcaaaaataa 620820
taatttgtaa tatattccct tcagcaaaag agcgtacagg attcgatggg aaaacttgag 620880
ctattataga aagaaaatac gccgctgttt tgtttgaatc aatgacagta acggccgaat 620940
ccatagattg ggcctgggca aaatcacacc catttcctgg agagaaaatc caagcaaaac 621000
acaagccaat cacaatagct agtgccgtag tccctagata gaggccgaca cttttaatgc 621060
caatgcgtcc caattttttc atatcgctaa tggaagcgat tcctaaaacc attgagcaga 621120
acactaaagg atacactacc atgctcaata gatttaaaaa gatgtctcct ataggtttaa 621180
aaaagatggc tttatcttct aaaactaaac ctaaggtaac cccgacaaac aatccaataa 621240
agatcttcat ccataatttc attagttacc cctgttttta tgtatttgtt caatcatgtt 621300
gagaagagaa agtgtatcgt cttgattcac agaaaggcgc atttccacct tagctatcgg 621360

caataaactt acctctcctg ataatcgagg aagctttaca ctatcttttt ctgtacataa 621420
caacccttgt ccttggcgca tggccatttg ctgacaaaag taatttaatt ctttttttgt 621480
tattgctgcg tgatcaggca ataaatactt tcctaaaata tgaattcctt cttctctaag 621540
catattcaaa aacccttgag gaaaccctaa accacaaaaa acccccacac gcaattctcg 621600
aagagcctct ttaggaatac gctctccgtt atgggtccaa actacagaag ctattgtagg 621660
tttcacaaag atctgggggg cattcgaaac acgtttcact acagtcccag cctcttttcc 621720
accgccattt acaataatgg catccacagt cttcaatcga agaggaaaat cccgaagcct 621780
tcctttaggg aagaaggcac gtcctcctaa aggatcctga ccattcacta cagcaatttc 621840
tacgtctttg tgtagtttac ggtactgaag accatcatct aaaaggagaa taccaaattt 621900
ctctgcagcg cgtgctgcag agatcctccg gtccttatgc acccatacgg atccctctgg 621960
cagtttctct gccattaata aaggctcgtc ccctacatag gacgcagaat ggactttcga 622020
gtctacaaca gtcagtttct tttgccgact cgactggctt ttatagccac gcgacagtac 622080
accgcaagaa taccctcgaa gcctcaaagc ctctgctaac cacaatactg ttggagtctt 622140
tcctgcccct ccaacaacga tattgcctac actaatgact gtagaacgcg cacgataagg 622200
agtagaccag gaaaatcgat tccaacatgc caccaagaat gcaaaaactt tggaaagaag 622260
ggaacctaac cagccccatc ccaaaatacc ctcaaggcta atagctatgg tgacacggcg 622320
ataaaagaga aagagtgtgg aaggaaaccg ttttttcatt gttcctcttt taaacgcacg 622380
aatttctttg cacgaaaatc atgcttttta aaagatcaca atcttcgccg tattacaaag 622440
aatgctagta ttgcagaaga aaagattaaa aatcaagttt acaaagctta attaaaaaat 622500
tgtgttaaac aaaaaactaa tataattaat ttgatctcaa aaagaaaaat ggtgtactaa 622560
ttaacccaac gttgacgaac aacttcataa gctacagcag ctacagatgt agcgagattc 622620
aaagaatctg attcccctaa cataggaaga gcgatttcag aaaaatcttc agaaaaccaa 622680
tcctcagtca aaccatcttt ctctgaacca aaaaccaaag ccgtgggacc aagataattt 622740
ttagaaaaat acatagtttc agctcgagga gatgtgacaa aaacagtcca gccttcctgc 622800
ttgaacaact ccttcccttc ctctcttgaa atagagagga tgggaagaga aaagacagct 622860
cctaaagaag agcgcaccac attaggattg tacaaatcta caataggatt gcataaaatc 622920
acaccgtcaa caccagcacc atcagctatt cttaaaatag caccgacatt cccaggtttt 622980
tccacctgct caataataag atagaaaggc tgagcatttt tacgttggat caaaaaatct 623040
tctttattcc acactctctt ttgtatcacc gcgacgaaac tatcgtgatg ttctttaaaa 623100
gaaagttgag ccaaagtcga gtctaagcaa tataaaatct ttgtagaatt tctttttaac 623160
tcatataaaa attccttttc tttttctgaa agatgtgtcg aacaaaaaac atgctgacac 623220
aggtagcctg tgcgtaatgc tttctgaatc tcacgggctc cttcaactag aaaccaagaa 623280
ctttttctac aacgagaccg ttttaaagct agagcctctt taactagagg attatgtttc 623340
cctatgcaat ccattgaaca aaactcccag aaggcaaggc tcccacacct tctccacaaa 623400
aactttcccc acaagaccac gcttcagaaa caagggttgg cacctacgcc tagctatagc 623460
ccttaaaaac tctggggtat gtcctggagt atgagaggtt aagagaaaat aagaagcgtc 623520
atctgcaaga agcttcgaac acaaagaaag caaaggaaag agatctttat ctattttaaa 623580
tacttcccca tcaggtccgc gaccatagct tgggggatct aaaagaatca cttgatattt 623640
cttatttctg cggatttctt tttttaaaaa agaaatcaca tcttcaataa cccaaaaaat 623700
acgtctctca ggaaaagcat ttttctctac atttctttgc gcccaacgta ccgctgcctg 623760
cgaagcatct acatgggtca cacgagctcc acacttagct gcaaaaatag aacccgcccc 623820
tgtataagca aagagattca atacctgacg ttctttgtgc ttctcaatgg cttgtttcaa 623880
agcgggccag aaccccatat gttcaggaaa tacgcctaga tgcccaaagg gggttctctt 623940
caatagacaa cgcacatcag aaaatgcgac ctcccattct tcaggaagac gcttaaaatt 624000
tttccatgct cctctttctc cttcacgaac atactgcaat tgcgcctgag accatagttc 624060
aggtctgctt tttggccaaa cagcaatact tgaaggacga attaaagtga caggaccaaa 624120
acactctaat ttgttcccgt ccccactatc gagcaattta taatccataa ctaatctttc 624180
cctgatgagg ctaaaatacg ttttactgtg tctacctgta tctttgttga catatcgatc 624240
tcgatattga ctctttctcc ttcccttttc tttcctaaag tcgtcctttg aagagtctca 624300
ggaatcaatc ccacagaaaa cgtatcacta tcaactgaca ctagcgttaa gctgacacca 624360
tcaattgcaa taaacccttt ttcaaaaagg tactgagata attctttgga accacggaaa 624420
taataacgat ttttctttat caagaaaatc tctgctgttc caaaaacatg cccagagagt 624480
aagtgacctc caatcgagtc tcccattttt aatgcagcct ctaagttcac ttgatcgcta 624540
catctttttt ctcccaaagt cgtacaagct agagtttctg gaatcacatc aaaaaagatc 624600
ttactttcat tgcatgatgt taaagtcaaa cagactccat ctacggctac gctatcccct 624660
gtaaccagag gggtgacaaa tagtggcgtg ctcttaattc ctaaactcag accatttcct 624720
tgagcttcaa aaaaacacac ttcacctaat tcttgaataa ttcctgaaaa catcccaccc 624780
catttaacaa cagaatcttt acaacaaaag ctttcctaga gtagagtatg tttttttcgt 624840
ctcaataagc aaagcaattt tgcacagaaa tttaagataa tacgaatccc taaagccgtt 624900
tctttagaga agtatttcct ctacccttga tttcctagaa aaaaactttg caactatagt 624960
ctcttatatt tatcctgatc taaaaattcg gtttttctta gactacctta acctaggagg 625020
tagctcccat gcagtgtcct ttttgcaatc atggggagtt gaaagttata gattcaagaa 625080
acgctccaga agctaatgca ataaaacgcc gtcgggaatg cttaaagtgc tcccaacgtt 625140
ttacgacctt tgaaaccgtt gaacttactt tacaagtact aaaacgtgat ggtcgctacg 625200

aaaattttca agaatctaaa ttaattcacg gtctgaacgc agcttctagc cacacacgga 625260
ttggtcaaga ccaagttcat gctatagctt ctaatgttaa atctgaactc ttaggtaaac 625320
aaaataggga aatttctacc aaagaaattg gcgaactagt aatgaaatat cttaaaaagg 625380
ctgatatgat tgcctacatc cgatttgcct gcgtttatcg tcgattcaag gacgttggtg 625440
aattaatgga agttttattg tcagcaactc cagatatgga aaaatagttg aattttataa 625500
ggagcaaggt tgtgccgtta tcagatgacg aaatagaaca gtttaaaaaa agacttttgg 625560
agatgaaggc aaagttatcg catactctag aagggaacgc tcaagaggta aaaaaaccta 625620
acgaagctac aggatattct cagcatcaag cagaccaagg taccgacacc tttgatcgga 625680
ctattagcct agaagtcact acaaaagaat atgagcttct aagacaaatt aatagggctc 625740
tagaaaaaat taatgagtct tcttacggga tttgtgatgt cagcggagaa gaaattcctc 625800
tcgctaggtt gatagccatt ccctatgcta ccatgacagt caaagctcaa gagcagtttg 625860
aaaaaggact cctatctgga aattaagttc tatggcaact cgttttcgta gcacactatt 625920
agtgattact ctgtttgttt taatcgactg ggtcaccaag cttgttgtct tattacaata 625980
caaagatctc caaattttaa cgcaccccac cttatatact catagttggg ggcggttttc 626040
attttcaatt gctcctgtat ttaatgaagg ggctgctttc ggtctctttt caaattataa 626100
atatttctta ttccttctgc ggatatttgt gattcttggc ctcctggcct atcttttttt 626160
taaaaaaaaa tctatacaat ctacaacgca gactgctcta gtccttctct gtgcaggagc 626220
tataggaaac gtcggggata ttatctttta cggccacata gtcgatttca tttctttcaa 626280
ttataaacaa tgggcattcc ccacctttaa cgttgccgat gtattgattt ctcttggcac 626340
tctgctcctt gtttataaat tttattttcc tacaaaacaa actgaaaaaa agagataata 626400
tagatctctt caagagaagc taagatatgt ttttaaaact gttatgaacc gtcttctatc 626460
gcttttatcc gtctttgatg attttttctg gtcctatgtg gcctttattc taatcattgt 626520
tctaggtgta agtttttcct ggaagtcgcg attttttcaa ttcacgaagt tctctcagtt 626580
ttgcaagctt ttccgttatt actctcagaa tcctcaagaa agagaaacaa agcaaggtgt 626640
ccatcctcta aaagtatttt ttgcctccgc aggcggaaat atcggcatag ggaatgtcgt 626700
aggaattgtc acagcagcat gtatcggtgg acccggggct cttttctggg tgtggattgc 626760
tgggatcttt ggttctattg ttaaatattc tgaggtctat ctcggaatca agttccgtaa 626820
gttagatcgt gatggcgtct atcagggcgg gcctatgtat tttcttataa aggcgttcaa 626880
aacccctgtc gtgtctgtta ttgttgcgat tcttctctgc atttatggag tggaaatcta 626940
tcaattttca gtcatcactg acagccttgc ccactgttgg aacctaccta aagtctatcc 627000
gatgttaggt ctactcttcc ttgttttcta tgcaattcga ggaggcttgc agcgtatagg 627060
aaaaatttgt tctatagttc ttcctttctt catgctctta tactgtgccc tatccctcta 627120
catcctcgtt aaagagtttc atacccttcc acacctactt tccacagtat tttcttctgc 627180
atttaaaggt caaagtgccc ttggaggatt tgcaggctgt actgtagcca ctacgattca 627240
tcaaggaatt tcacgagcag cctattctgg ggatatcggt ataggctttg actccatcat 627300
tcagagtgaa agttctgcta aagatcctag cacccaagcg caactcagta ttgttgggat 627360
tgccatagat aacctgatct gtactctgag tcttctcatg gtgcttgctt cgggctcctg 627420
gtctctggga ttagagaatg cttcccaagt agtagaacac actctagcaa gctacttccc 627480
tatggtgaag ttcttcctcc ccaccttctt ctttgttaca ggctatacaa ccatcatctc 627540
ctacttcctg gttgggaaga agtgtgcaaa gtttctttac ggaaacacag gggcaaagat 627600
ctatactctc tatggtcttc tgattcttcc cttattttgt ttcctcagcc aaaacacagc 627660
tttgttgatc atgtctgtat ctggagccct actcctttgc tttaacctct taggagtctt 627720
catcttaaga aaagaagtta tcttccctgc aagggctgct tctctcacag aaacttctct 627780
ttctacagaa taaaaaattt accttattca tacacaatga gataagaagt tttaattttt 627840
attaataaat taaaagatta gttttagaaa aaaacacttc ataaagtaaa ataaaagaat 627900
aaaagttgta attaattttt ttacttttta tggataacta tctcctcgga agcttgattt 627960
tctgttgtgt acttctatca atagggatgt gcacgatttt cgtgatgacg atctgctttc 628020
tacgccaact caataaaatc cttaaaaaca tccatcgggt gactacaatt ttaaattttg 628080
aggctaagat cctagctcct ttgatgttag ggaaaaagct tctctgtgga tggctaaaga 628140
aaagaaaaaa tcgcggctct ctgtctgaag acattgacga actcttagat gagaaaaagc 628200
agagaagctg gaaaaaaaac ttagaccaag gaattaaatg gtgcgcacac tggtcctcat 628260
ttggaaagtg tttcgtaata aagattaaaa cattgaggga tatcgttatg ttcaggaaca 628320
accacaaacc taaaaaaact aagtgcaaac gatttcgctg gctacggggt gttttattcg 628380
gtggattcat agctacgtta ttaacatgct tgtttactcc caaaagtggc gtccaactgc 628440
ggaagaaaat cctcaaagta aaaaactctg gggcaaaaaa aagtagagtg ttctttaaaa 628500
attccaagca acataccaag tcattcgtaa aacaagcgaa gttgctagct aagaatatct 628560
cacacgaact tcaagatttt aaaaagggaa tcctcgacga caaagattag agttgctatt 628620
aagaagctct gtatgattga gatatcttct cagggctttt tttcatttaa aagcacataa 628680
atagaggctc tctccgagaa atgccattct cgagtcaact ttttatttcc gtgtatttat 628740
ttttctgttc tatgtaagtt tagatctgta taaggctata acttattagg actccgacat 628800
atgaagcaga tgcgtctttg gggattttta tttctctctt ccttctgtca agtttcttat 628860
ctacgagcaa acgatgttct cctccctcta tcagggattc attctggaga agaccttgaa 628920
ctctttactc tacgcagttc ctccccaaca aaaactacgt attctctacg caaagatttt 628980
attgtttgtg attttgcagg aaattctatt cacaagcctg gagctgcatt cctgaactta 629040

aaaggcgatc tattttttat aaatagcact cccctagcgg ctcttacctt taaaaacatt 629100
cacttaggag ctcgcggtgc tgggctcttc tcggaatcca atgtgacctt caaaggcctg 629160
cactctctcg ttctcgaaaa caacgaaagt tggggaggcg tcctcaccac atctggcgac 629220
ctttccttca taaataatac cagtgtgctt tgtcaaaaca acattagcta tggacctgga 629280
ggagcgctac tcttacaagg aagaaaaagc aaggctctct ttttcagaga caatcgagga 629340
acaattctat ttctgaaaaa caaagccgtg aatcaagatg aatcccatcc tgggtacgga 629400
ggagctgtaa gtagtataag tcctggctcc ccgattacct tcgctgacaa ccaagaaatc 629460
ctattccaag agaatgaggg cgaacttggt ggagccattt ataacgatca gggtgccata 629520
acttttgaga ataactttca aaccacaagc tttttctcta acaaagctag tttcggagga 629580
gctgtctata gccgctactg caatctctat tcacagtggg gcgataccct attcactaaa 629640
aacgctgctg caaaagtagg cggacatcca tgcggattat gttcatataa gagactgtaa 629700
aggaagcatc gtctttgagg agaactcagc aacagctgga ggggcaatcg cagtaaatgc 629760
agtttgtgac attaatgctc aaggtcctgt tcgctttata aataactctg cgttaggact 629820
aaatggtggt gctatttata tgcaggctac tggatctata ttgcgcttac atgcaaatca 629880
aggagatatt gaattttgtg gaaataaagt acgatcgcag tttcattcac atataaattc 629940
cacttcaaac ttcacaaata atgccattac tatccaagga gcgcctcgag aattttcgct 630000
cagcgcgaat gaaggacatc gcatctgttt ctatgatcct ataatttctg caacagaaaa 630060
ctataactct ctgtacatca accatcagag acttttagaa gccgggggtg ctgtgatctt 630120
ttcaggagca cgcctatctc cagagcataa aaaagaaaat aagaacaaaa cttcgattat 630180
aaaccagccc gtacgtctct gttctggagt cctttctata gaagggggcg cgattcttgc 630240
tgttcgttct ttttatcaag aaggaggtct tcttgctctc gggccaggtt ctaaactgac 630300
cactcaaggg aaaaattctg aaaaagataa aattgtcatc acaaatttag gattcaacct 630360
agaaaatcta gactcttcgg atcctgcaga aatccgagct acagaaaaag cctctattga 630420
aatttctgga gttcctagag tctatggtca cacagaatct ttctatgaaa atcatgagta 630480
tgcctccaaa ccttatacaa cttcgattat tctatctgcc aaaaaacttg ttacagctcc 630540
ctctaggcca gagaaagaca tccaaaatct catcatcgct gaatctgagt atatgggcta 630600
cggctatcaa ggctcatggg aattctcctg gtctcctaac gacactaaag aaaagaaaac 630660
cattatagcc tcttggactc ctacaggaga atttctttta gatccgaagc gccgtggatc 630720
tttcattccc acaaccttat ggtcgacatt ctctgggctg aatatagcat cgaatatcgt 630780
gaataacaat tacctcaaca actccgaggt catccccctg caacatctct gtgtttttgg 630840
aggccctgtc tatcagatta tggagcaaaa tcctaaacag agctctaaca atctcttagt 630900
tcaacatgcg ggtcataatg ttggagctag aattcctttc tctttcaata ccatattgag 630960
tgctgcactt actcaactct tctcttcttc atcacaacaa aatgttgctg ataagagcca 631020
cgcgcaaata ttgataggga ctgtatctct taataaaagt tggcaagcac tatctcttag 631080
atcttcattt agctatacgg aagactctca ggtaatgaag cacgtattcc cctataaagg 631140
gacctctcga ggatcttgga gaaactacgg atggtccgga tctgtcggca tgtcttacgc 631200
ctatcctaaa ggaatccgct atctaaagat gactcccttt gttgaccttc agtatacaaa 631260
gttagtacaa aatccctttg tggaaacggg ttatgaccct agatattttt cttcctcgga 631320
gatgacgaac ctatctctac cgataggtat cgctttagaa atgcgcttta taggctcgcg 631380
ttcttcccta tttctccaag tcagcacctc gtacattaaa gacttacgtc gggtcaaccc 631440
acaatcttca gcttccttgg tgttaaatca ctacacgtgg gatatccaag gagtccctct 631500
agggaaagaa gctctaaaca ttaccttaaa tagcacgatt aagtacaaga ttgtgactgc 631560
ctatatgggg atttctagca cccaacgaga aggcagtaac ctttcggcaa atgctcatgc 631620
aggcctctcc cttagtttct agaagttagc actatagaaa taaaagaaga cttaaaaagc 631680
gcgtggttat cattcaaaca cgcgcttttt tatcccttgc ctaatttatc catctgattt 631740
atctatcact tatcgagttt gtgaatatat ctgataggtt ttcctctatg aagtggctac 631800
cagctacagc tgtttttgct gccgtactcc ccgcactaac agccttcgga gatcccgcgt 631860
ctgttgaaat aagtaccagc catacaggat ccggggatcc tacaagcgac gctgccttaa 631920
caggatttac acaaagttcc acagaaactg acggtactac ctataccatt gtcggtgata 631980
tcaccttctc tacttttacg aatattcctg ttcccgtagt aactccagac gccaacgata 632040
gttccagcaa tagctctaaa ggaggaagta gcagtagtgg agctacatct ctaatccgat 632100
cctcaaacct acactccgat tttgatttta caaaagatag cgtgttagac ctctatcacc 632160
ttttctttcc ttcagcttca aatactctca atcctgcact cctttcttcc agtagcagcg 632220
gtggatcctc gagcagcagt agctcctcat catctggaag tgcatctgct gttgttgctg 632280
cggacccaaa aggaggcgct gccttttata gtaacgaggc taacggaact ttaaccttca 632340
ctacagactc tggaaatccc ggctccctga ctcttcagaa tcttaaaatg accggagatg 632400
gagccgccat ctactcgaag ggtcctctag tatttactgg tttaaaaaat ctaaccttta 632460
caggaaatga atctcagaaa tctggaggtg ctgcctatac tgaaggcgca ctcacaacac 632520
aagcaatcgt tgaagccgta acttttactg gcaacacctc ggcagggcaa ggaggcgcta 632580
tctatgttaa agaagctacc ctattcaatg ctctagacag cctcaaattt gaaaaaaaca 632640
cttctgggca agctggtggt ggaatctata cagagtctac gctcacaatc tcgaacatca 632700
caaaatctat tgaatttatc tctaataaag cttctgtccc tgcccccgct cctgagccca 632760
cctctccggc tccaagtagc ttaataaatt ctacaacgat cgatacctcg actctccaaa 632820
cccgagcagc atccgcaact ccagcagtgg ctcctgttgc tgccgtaact ccaacaccaa 632880

tctctactca agagaccgca ggaaatggag gcgctatcta tgctaaacaa ggtatttcga 632940
tatccacgtt taaagatctg accttcaagt ctaactctgc atcggtagat gccaccctta 633000
ctgtcgattc tagcactatt ggagaatctg gaggtgctat ctttgcagca gactctatac 633060
aaatccaaca gtgcacggga accaccttat tcagtggcaa tactgccaat aagtctggtg 633120
ggggtattta cgctgtagga caagtcaccc tagaagatat agcgaatctg aagatgacca 633180
acaacacctg taaaggtgaa ggtggagcca tctacactaa aaaggcttta actatcaaca 633240
acggtgccat tctcactaca ttttctggaa atacatcgac agataatggt ggggctattt 633300
ttgctgtagg tggcatcact ctctctgatc ttgtagaagt ccgctttagt aaaaataaga 633360
ccggaaatta ttccgctcct attaccaaag cggctagcaa cacagctcct gtagtttcta 633420
gctctacaac tgctgcatct cctgcggtcc ctgctgccgc tgcagcacct gttacaaacg 633480
cagcaaaagg aggggcttta tatagtacag aaggactgac tgtatctgga atcacatcga 633540
tnattgtcgt ttgaaaacaa cgaatgccag aatcaaggag gtggggctta cgttactaaa 633600
accttccagt gttccgattc tcatcgcctc cagtttacta gtaataaagc agcagatgaa 633660
ggcgggggcc tgtattgtgg tgacgatgtc acgctaacga acctgacagg gaaaacacta 633720
tttcaagaga atagcagtga gaaacatgga ggtgggctct ctctcgcctc aggaaaatct 633780
ctgactatga catcgttaga gagcttctgc ttaaatgcaa atacagcaaa ggaaaacgga 633840
ggcggtgcga atgtccctga aaatattgta ctcaccttca cctatactcc cactccaaat 633900
gaacctgcgc ctgtgcagca gcccgtgtat ggagaagctc ttgttactgg aaatacagcc 633960
acaaaaagtg gtgggggcat ttacacgaaa aatgcggcct tctcaaattt atcttctgta 634020
acttttgatc aaaatacctc ttcagaaaat ggtggtgcct tacttaccca aaaagctgca 634080
gataaaacgg actgttcttt cacctatatt acaaatgtca atatcaccaa caatacagct 634140
acaggaaatg gtgggggcat tgctggggga aaagcacatt ttgatcgcat tgataatctt 634200
acagtccaaa gcaaccaagc aaagaaaggt ggtggggttt atcttgaaga tgccctcatc 634260
ctggaaaagg ttattacagg ttctgtctca caaaatacag ctacagaaag tggtgggggt 634320
atctacgcta aggatattca actacaagct ctacctggaa gcttcacaat taccgataat 634380
aaagtcgaaa ctagtcttac tactagcact aatttatatg gtgggggcat ctattccagt 634440
ggagctgtca cgctaaccaa tatatctgga acctttggca ttacaggaaa ctctgttatc 634500
aatacagcga catcccagga tgcagatata caaggtgggg gcatttatgc aaccacgtct 634560
ctctcaataa atcaatgtaa tacacccatt ctatttagca acaactctgc tgccactaaa 634620
aaaacatcaa caacaaagca aattgctggt ggggctatct tctccgctgc agtaactatc 634680
gagaataact ctcagcccat tattttctta aataattccg caaagtcgga agcaactaca 634740
gcagcaactg caggaaataa agatagctgt ggaggagcca ttgcagctaa ctctgttact 634800
ttaacaaata accctgaaat aacctttaaa ggaaattatg cagaaactgg aggagcgatt 634860
ggctgtattg atcttactaa tggctcacct ccccgtaaag tctctattgc agacaacggt 634920
tctgtccttt ttcaagacaa ctctgcgtta aatcgcggag gcgctatcta tggagagact 634980
atcgatatct ccaggacagg tgcgactttc atcggtaact cttcaaaaca tgatggaagt 635040
gcaatttgct gttcaacagc cctaactctt gcgccaaact cccaacttat ctttgaaaac 635100
aataaggtta cggaaaccac agccactaca aaagcttcca taaataattt aggagctgca 635160
atttatggaa ataatgagac tagtgacgtc actatctctt tatcagctga gaatggaagt 635220
attttcttta aaaacaatct atgcacagca acaaacaaat actgcagtat tgctggaaac 635280
gtaaaattta cagcaataga agcttcagca gggaaagcta tatctttcta tgatgcagtt 635340
aacgtttcca ccaaaganac aaatgctcaa gagctaaaat taaatgaaaa agcgacaagt 635400
acaggaacga ttctattttc tggggaactt cacgaaaata aatcctatat tccacagaaa 635460
gtcactttcg cacatgggaa tctcattcta ggtaaaaatg cagaacttag cgtagtttcc 635520
tttacccaat ctccaggcac cacaatcact atgggcccag gatcggttct ttccaaccat 635580
agcaaagaag caggaggaat cgctataaac aatgtcatca ttgattttag tgaaatcgtt 635640
cctactaaag ataatgcaac agtagctcca cccactctta aattagtatc gagaactaat 635700
gcagatagta aagataagat tgatattaca ggaactgtga ctcttctaga tcctaatggc 635760
aacttatatc aaaattctta tcttggtgaa gaccgcgata tcactctttt caatatagac 635820
aattctgcaa gtggggcagt tacagccacg aatgtcaccc ttcaagggaa tttaggagct 635880
aaaaaaggat atttaggaac ctggaatttg gatccaaatt cctcgggttc aaaaattatt 635940
ctaaaatgga cctttgacaa atacctgcgc tggccctaca tccctagaga caaccacttc 636000
tacatcaact ctatttgggg agcacaaaac tctttagtga ctgtgaaaca agggatctta 636060
gggaacatgt tgaacaatgc aaggtttgaa gatcctgctt tcaacaactt ctgggcttcg 636120
gctataggat ctttccttag gaaagaagta tctcgaaatt ctgactcatt cacctatcat 636180
ggcagaggct ataccgctgc tgtggatgcc aaacctcgcc aagaatttat tttaggagct 636240
gccttcagtc aggtttttgg tcacgccgag tctgaatatc accttgacaa ctataagcat 636300
aaaggctcag gtcactctac acaagcatct ctttatgctg gcaatatctt ctattttcct 636360
gcgatacggt ctcggcctat tctattccaa ggtgtggcga cctatggtta tatgcaacat 636420
gacaccacaa cctactatcc ttctattgaa gaaaaaaata tggcaaactg ggatagcatt 636480
gcttggttat ttgatctgcg tttcagtgtg gatcttaaag aacctcaacc tcactctaca 636540
gcaaggctta ccttctatac agaagctgag tataccagaa ttcgccagga gaaattcaca 636600
gagctagact atgatcctag atctttctct gcatgctctt atggaaactt agcaattcct 636660
actggattct ctgtagacgg agcattagct tggcgtgaga ttattctata taataaagta 636720

tcagctgcgt acctccctgt gattctcagg aataatccaa aagcgaccta tgaagttctc 636780
tctacaaaag aaaagggcaa cgtagtcaac gttctcccta caagaaacgc agctcgtgca 636840
gaggtgagct ctcaaattta tcttggaagt tactggacac tctacggcac gtatactatt 636900
gatgcttcaa tgaatacttt agtgcaaatg gccaacggag ggatccggtt tgtattctag 636960
ggtatacaat taaagatttt atgaaattga ggatacggag agagtgggat tcgaacccac 637020
ggtacgcgtt aacgcacaca cgctttccaa gcgtgctcct taagccactc ggacatctct 637080
ccatatttat agattttcca ggcaaaaaga cttgccgaga acatatctta acctttccat 637140
ttttatcaac atccgtctta ctatgagaat tttttcctaa gatcaccgct tcttaggata 637200
ttcgttcttt attaaaatta tgccccaata gaataataga tcatcttatc aaactgcttt 637260
tgtcatgcat aaagtaatag ttttnatttt ccttacccta tattcgttaa aaagttatgg 637320
gaatgatgta atagataagc cccatgttct tgtcagtatc gccccctata aattcctagt 637380
tgaacaaatt gctgaagaga cctgttttgt ctatgcgata gttacgaatc actatgatcc 637440
ccatacctat gaacttcctc ctcagcaaat caaggagtta cgacaaggag acctttggtt 637500
ccgtatagga gaggcatttg aaaaaacttg tgagagaaac cttacatgcc aacaagtcga 637560
tctttcccaa aatgtctcgc tgattcaagg aaagccttgc tgtaatcaac ataccacgaa 637620
ctacgacacc cacacttggt taagccctaa aaaccttaaa gtccaagtgg agactatcgt 637680
taccacttta agtaaaaaat atcctcaaca cgcgactcta tatcaaagca atggagagaa 637740
acttctgtta gctttggacc aactcaatga ggaaattctt acgattacct ccaaagcgaa 637800
acaacgccat attttagttt cccatggagc ctttgggtat ttttgccgtg attacaattt 637860
ctctcagcac actatagaga aaagcagtca tgttgagcct tctcctaaag atgtggctcg 637920
cgtatttcgt gacattgaac agtacaaaat ttcttctgtg attcttctcg aatactctgg 637980
aagacgaagt agtgctatgc tggcagatcg tttccacatg catactgtga atctcgatcc 638040
ctatgcggaa aatatacttg taaacttaaa aaccatagcg acgacttttt ctagtttatg 638100
acaatacgaa ttcttgctga aggcctagct ttccgttacg gaagcaaggg accgaatatc 638160
attcatgatg tttctttctc tgtctatgat ggcgacttta taggaatcat aggaccaaac 638220
ggagggggaa aagcacctta acgatgttaa ttttgggctt gcttactcct acattcggat 638280
ccttgaagac tttcccttcg cattccgcgg ggaaacaaac ccattccatg atcggttggg 638340
ttccccaaca tttctcttat gatccttgtt ttcctatctc agtaaaagat gttgtcctct 638400
caggaagatt gtctcaactc tcctggcatg nnaaatataa anagaaagat tttgaagctg 638460
tagatcacgc tttggataat gttggacttt ctgaccacca ccaccactgc ttcgcccatc 638520
tctcaggagg acaaatccag cgtgtacttc tggcaagagc cttagcctcc taccctgaaa 638580
ttttaattct tgatgagccg acgacaaaca ttgatcctga caatcaacaa agaattttaa 638640
gtatcctaaa aaagctcaac cgtacgtgca ccattcttat ggtaactcac gatcttcacc 638700
atacgacgaa ttactttaat aaagtttttt atatgaacaa aactttgact tcattggcag 638760
acacttcgac cttaacagac caattttgtt gtcatcccta taaaaatcag gaattttcat 638820
gctctcctca ctaatccgtg attcatttcc ccttcttatt ttacttccca cattcctagc 638880
ggcattagga gcctccgtag ctggcggcgt tatgggaacc tatatcgttg taaaacgtat 638940
tgtttcaatt agtggaagta tatctcatgc aattctagga ggaattggcc tcaccctatg 639000
gatacaatat aagcttcatc tctctttttt ccctatgtat ggagctattg taggagctat 639060
ttttctagct ctttgcatcg gcaaaagatc cacctgaaat accaagaaag ggaagactct 639120
ttgattgcga tgatttggtc tgtgggcatg gcaattggaa ttatattcat ttccaggctt 639180
cccaccttta atggagagct catcaatttt ctatttggga acattctctg ggtcacccct 639240
tcagacctct atagcttang aatctttgat cttcttgttt taggaattgt ggtcctttgc 639300
cacacccggt tccttgctct ttgctttgat gagaggtaca cggctttaaa ccattgttct 639360
gtacagctgt ggtatttcct acttcttgtt ctgacagcaa tcacgattgt gatgttgatt 639420
tatgtgatgg gaacgattct gatgcttagc atgctcgtct tacctgttgc tatagcgtgt 639480
agattttcgt acaagatgac acgaattatg ttcatctcgg tcctcttgaa tatcttatgt 639540
tctttttctg gaatttgcat cgcctactgt ttagatttcc cagtaggtcc tacgatatca 639600
ttgctgatgg ggttangtta tacagcgagt cttgtgtgaa gaagcggtac aatccgtcga 639660
cgccttctcc tgtaagtcct gaaatcaata caaatgtata gctagggaag cgcttttgga 639720
agctttggag gcattcttcc tgttcgtcag gaagaagatc atcaatttta tttaaagcta 639780
ccagcatatc tttcttttca aaatctggct gatgagagtg gagctcgtgg atgagcgttt 639840
ctaagtcttc ttcgggagag tttctctctc ttttggagac atcgataaca aatagcagta 639900
aaagagtgcg ctcaatatgg cgaagaaaat cgagtcctag gcctttgttt tgatgagctc 639960
cttcaatgat tcctggaatg tcagcgataa tccagggttt ttgatacaaa cgatctttac 640020
aaaggactag gcccaaagag ggggccagag ttgtgaaggg ataggctccg actttcactt 640080
cggtatgtgc gagtgtatta aatagtgtgg actttcctgc atttgggaac cctaccaaac 640140
cgatatcagc aatgagctta agttctaact ctacctgacg gatttctccg ggttttcctg 640200
gggtggcttt tgtaggggct cggtttactg aggtcttaaa gaaggtattt ccttttcctc 640260
cctttcctcc ttggctcact aggagacgct ctccatctac ggtaaagtca tgaaggattt 640320
caccagtctc agcatcacga agcagggtgc ctgtagggac agaaactatt agatctttac 640380
cactgcgtcc tgtgcggtta tttgtagctc ctgactgacc gtcgggagcc ttgagaaagc 640440
ggatatttct ataagcttcg aaagaatata cacttgtggt ggcttctatg attacggagc 640500
cgccattgcc accattgcct ccgtaaggcc ctcctttagg aaggtatttt tcctttctcc 640560

aagcaacaac gccgtttccg ccctttccag cacgcaattc taaggtaatt tgatctacaa 640620
acatgattac gtttttatcc ttcttcaaac aaaaaaagct cgatttgatg cctgagcaaa 640680
aaaccgagct tttttaatgt ttctgaaaaa tcgaaaaata ggaactaaag ttgctcagga 640740
acaacagaga tataagtacg attcgtcttt ttcattacta caataccatc gactaaagcg 640800
aatagggtat catcacgacc acgacctaca ttttgtgcag gattccatcg ggtccctctc 640860
tgacgaacga gaatacttcc tgtagaaact ttttgcccag cgcccacttt aaccccaagg 640920
cgcttcgatt ttgaatctct accgtttcgg cttgcccctt gtcctttctt atgtgccata 640980
actctaaact ctctagcttc tgtttatatc aatatctcac ggattttcac acgaaggtac 641040
ttctgacgat gtccgtgttt acgatgataa tttttgcgtt ttttatactt ataggcgact 641100
actttttctc ctttaacatg agaaagatat tcggctttta cttgtgcgtt cgcaatcgta 641160
gggcttccta gagaggcttt agttccatcg aatacaaaga ggacatcttg aaaaatgact 641220
tctttatccg aagcaacttc ccctagtaac tcgacatcaa tcacatcgcc tgagcgaacc 641280
tgatattgct tgcttcctgt ttggattact gcgtagggct ccatcaattt tttccttatg 641340
aatctttcaa tgcttaaagt taagcgctgt ttgctaagtt actttagcaa acttaaggat 641400
tatacaaaat ataaagtctt ggattatagt ttttaacccc tcggagagcg ctatcctcaa 641460
gcggttatgg ccagagccgg gatcgaaccg acgacacaag gatcttcaat cctctgctct 641520
accatctgag ctatctagcc atttgtactc agttatcata ggaaaacatt cagtttaata 641580
tcaatgagat ttcgaattct tcgcctctct ttttgctatt aaggagtatt tctcaagaga 641640
gtgcttcgat gtagcgagaa agtttgacaa attactggaa taatgcgtga attttagaaa 641700
ttttgtagta tcttctgtga aagaaattct taaaaaaaac atataccagg tagttatgga 641760
tagagacaat gaggttcccc tgcccaagcc caaatggatt taccgtacag gtattggtca 641820
agacagccac cgctttctcc cagaaagttc cacgaagccc tgcattttag gtggtatcat 641880
ttttgatcat tgcccaggat ttcaggcaaa ttccgatgga gacattatct ttcatgccat 641940
ttgtaatgcg atttcctcag taactaataa aattatttta ggaaaggttg ctgatgagct 642000
tctccaaaca cggggaatta cagatagtgg gatttatctt gaggaggctt taaaatctct 642060
gaagcctaat caaaagattt ctcatgtcgc tattacgatt gaaggaagtc gacctaaatt 642120
tctctgtaag ctatctgcat tacgtcaaaa tattgcccag gttatgaact taacacctac 642180
ggatattggc attactgcga cttctggaga gggtttgagt gactttggct gtggagatgg 642240
cgttcagtgt ttctgcgtct taactgtgat ggaatactgt gactaataga cgtcaacaac 642300
gtaacgatgt tcctttctga gggaggctag agtatctttt cctaagattt cttctaaagc 642360
gtgttttact tctatgccta agactttacg tccacagaca aagaaaaacc ctccctcttc 642420
ataggccttt cttacctcat ctttttgaat cctaagaaga tcttgaacat agactttttg 642480
gtctctctct cttgagaagg ctaaaaagag ttttagtttt ccttcctctt ctgcatggtt 642540
ccagaattct cgataataga agttcacttt ttctttgcgc tcaccaaaaa atagtaaatt 642600
atttcctggg tctttattga aaaggcgttc ctctaggaag gctttataag gagcgattcc 642660
tgttccagct ccgatcataa ctaagggttt cccttcagtc tgtgtagata gagtgaagtg 642720
ttttgtaggt tgtacgaata tgtaggcaga atcgttgacc tgtagttcgc tacatagaaa 642780
ggacgagcaa actccgaagc gtttttgata ttttccagga taggaaacgt gttttaccaa 642840
tagctctata cttttgggat gtagatctgg tgaggaggct atagagtaga aacgaggcaa 642900
taaaggaaac acactctcag cgaagagttc tattgggatc tgagggcggt actcttgaat 642960
ggcatcatac agtgtaatct taggatcttt gtctgggaaa aaggaattta gctttgctgg 643020
aatttaatca agatcaacat agccttggat aaatttttgg gcagaaactt tttcagaagt 643080
tttttttacg ttgacaaggg tcgttgggga atagccttag aagctggaga acgtgttcgg 643140
agacctcttt agaattctct ggtaagactc ctaaagcatc gcctacttta taggatatag 643200
tcgtatcatt gctatcaaag accatgcggt agatgggatc gctatcgtta atggaatcgg 643260
aacaagaaag tagctcacgc aatactagag gaacttgctg agccttaaac ttttcttgta 643320
ggtacatctt ttatagacaa gcttgaatta agccgctttg attttaatat caactcctgc 643380
aggaagtgct aacattttca aagcatcgat agtttttcct gtgggatcta aaatatctac 643440
aagacgtttg tgagtacgaa tttcaaactg ctctcttgat tttttatcca catgtggcga 643500
acgcaataca gtatagactt ctctttttgt aggcagagga attggtccta caacacgagc 643560
tcctgttctt ttagcagttt caacaatatc tgctgtggaa cgatctagct gcccctgatc 643620
gaatcctttc agacgaatac gaattttttg cttttgctgc ttcatacatc ccttacttct 643680
taacaatctc ttcttgaatt ttttgaggaa ccttagcaaa gaatgctggt tccatggttg 643740
atgttgcacg tcctgaggtc aatgatctta aagatgtcgt gtacccaaac atttcgctta 643800
gaggtacctc agcattcact tgagccattc ctcgagaaga ttcttgtcct aaaattttgc 643860
ctcgacgacg gttgaggtcc ccaataacat cgccaagatg atcttctggg gtaattactg 643920
ctaccttcat gattggctct aagattacag gcttcgcttt tctacaggcg tctttaactg 643980
ccatagaccc acagatctta aatgccattt cactagaatc gacttcgtga tacgaaccga 644040
aaacaatact taccttaacg tctacaagac cgtagccagc caggactcct gtgttcaatc 644100
cctcttctat ccctttaatt actgcaggga tatattcttt aggaatgaca cctccgacaa 644160
tcttactgac aacttcgttg cctttcccag gttcgttagg ttctatttcg aggcaaacgt 644220
gagcatattg ccctcgacca ccagactgct tcacgtattt tgtttcacta tttccgctca 644280
cagtaatggt ttctttataa gaaacttgcg gttttcctac gttagcttca actttaaatt 644340
ctcggatcat acgatctcga agaatatcta aatgaagttc ccccattcca gaaatgatgg 644400

tctgtcctgt ctcttcattt gttgagacac ggaatgtagg atcttcttct gataaagaac 644460
ttaatgcttg agcaagtttt tctctatccc ctttagactt tggctcaata gccatatcga 644520
tgacggggtc tggaaattct atacgttcaa gaacaatttc ttggttatcg tcacacaagg 644580
tatctcctgt gacagaaaac ttcagaccca cgcaagctcc aatatcgccc acagtaaact 644640
catctctatc tgtacgctca ttagcgtgca tttctaaaag gcgagaaatc cgttcttttt 644700
tatcttttgt agaatttaaa atggcagacc cttttttaag agtgcctgaa tagattcgga 644760
taaatgtaat ccgacctacg tagggatctg tcatgatttt gaaagctaga gctgctagag 644820
gtccatcacg tcttggctct aaactaattt cttgatctgt tttaagattg attccgcgga 644880
tatttcctcg atccaaagga gaaggcaacc acttgacaat cacattgagc agttgttgca 644940
cacctttatt tttaaaagcg gttccgcaga gtacaggatt gattttattc tcaatgactc 645000
ccttacgcat aacctgatgg atttcatctt cagtaatgct atcgggatct tcaagaactt 645060
tcatcatgaa agcttcatta ctttcatcta tagtagcgag ttcttccaaa agattcgctc 645120
gcaattctgc acaacgctct ttgagatctt cagaaatctc tttttcttcc catttgctcc 645180
ctagggtatc atctagaaaa taaagagctt tttgagagat tagatcgacc atgccgacaa 645240
actggctttc agatccaata ggacagtgga cagggaaagc attcgctccc aatttctctt 645300
tcatggattc cacggcagca aaatagtctg ctcccatacg gtccatttta tttacgaaag 645360
caatccgtgg aacaccgtat ttatctgctt gtctccaaac agtttctgat tgaggttcca 645420
cgccagatac ggcgtcaaat acggctacag caccatcaag aacccgaaga gaacgttcta 645480
cttcaatcgt aaagtcgacg tgtccaggag tatcaataat gttgattttt gcgcctagcc 645540
agaagacagt agttgcagca gaggtaatcg taattcctct ttcttgctcc tgggccatcc 645600
agtccatggt agctccgcct tcatgacttc accgattttg tgagttcttc cagcatagaa 645660
aagaattctt tctgtagtcg ttgttttccc agcatcaata tgagccatga tgccgatgtt 645720
tctaattgca cttaaatcga attcttgatt gctcatgaac ttgttatttt ctccgtaatt 645780
taactaatct taccacttat aatgtgcgaa tgctttattt gcttccgcca tacgatgggt 645840
gtcttcacgt ttcttaattg ttgcaccctg tttgttgaag cagtcaatca gttcggtagc 645900
aagtccaact tccatagact ttccaggttt actacgagcg tgtttgatga tccattgcat 645960
cgctaaacaa ttcctacgtt cgctagcaac ttcaacaggc acttgataag tagcgcctcc 646020
aacacgacgg gaacgaactt ctaaaatagg ttttgcattt tctaaagctt ctccaaaacc 646080
ttcaagcaca ttctctaaat ttaatttttt accgaaacgc tctagagcag agtagacaat 646140
tttccttgcc acacttttct tcccatgcat cataaccttg ttgataaatt tttctaagat 646200
cacgctgcca tagataggat ccccagggat atcgcgcttt tcagcggagt gccgccttga 646260
catatacatt tacctctatc ttataaatct cgaccaagaa cttcgtatga ttacttaggc 646320
cgctttgcgc cgtaccgtga acgactttgc tttctatttt ttactgctgc acaatctagg 646380
gtgccacgaa caatatgata acgaacacca ggcaaatctt tgactctacc gccttggatc 646440
aacacaatgc tgtgctcttg aagattgtgt ccttcaccac caatataggc aatgacttcc 646500
tgcccgttag atagtcgcac ccaagcaact ttccttaaag ccgagttcgg cttcttagga 646560
gtttttgttt ttacttgaag gcaaactccc cgcttttgtg ggcacttctg caaagctggg 646620
gatttctttc tagctagact tgacttacgt cttttacgta ttaattgatt aatggtgggc 646680
atgtattctt ctcgtttcaa cctcactact acaagcatgg aaatatagag taaggctctc 646740
tttgttggca agggcttatt ttcagatgaa gagggaacgt atcctgcttg aatttaaaaa 646800
aatgctatct ttttctagac gaaccgtttt tttacttttt aaaagaaatt ttagctgtaa 646860
cgaacttatt ctaacctatc tctttggaga gaagaatttc gtattaggtt tctcctaatg 646920
aagactgtgc acaattaaag cagtattcca taagagccgc gatttatgta accaaagaga 646980
aatcctaaaa tgggctctgt taaatcttgg ttataggtaa aactactttc tttgccttgt 647040
tctatgatgt gaatagaatc gttggtataa ataaaaagca aaggtacggt gtgggaaagc 647100
aattgcgtan ttgagctagg tctgggatgc ccgcaagnat tttagcggag tcttcagata 647160
ggaaaagaac ttgaacgtag tttagaagag accaaaagcg accgaggaac gtggtcacga 647220
cttgtgagtc acaaagatca acaagcgtgt ggttattctt tcttttggtc tctttcagaa 647280
acttttctat actatccccg tagagaatca attgcccatc caggagtgtg tatccctgat 647340
tgcataggca agtatttgtt tctatgccct tttctgcttc tttttccgag tagagcaggg 647400
tatctggttg gttaggacaa cgtaaaacta atcttagatc cttgggttgt tttagagcgg 647460
gtccgttttc tttatttgcc attccctcag cgtaagtttc tccgttacgc atacataaaa 647520
tacctaaatc ggagagatgg gattgggaaa ggtttagagg ttcttttcgt ttgaaactga 647580
tagaggcttc tggaaatttt tccttatata tcaaaaaaat ttcttttatt tttgcgttat 647640
tagtcgaaga aatggttcct atttgcaggc cgcactgggc aacaaagtcc tcattcgttg 647700
tgaggataat atctatgaaa ggaatcccta gcactgttaa acaggtcgat gtttcccagg 647760
agaagctttc tgaagctctt agagggaaga cggctcttcc caagataaaa attataagga 647820
atctgaggac aacgcgccac atacaacctc agtaaaagta gaactcctat tactaaacta 647880
aaagtaagac tttattttaa gtgttttctt accttcttct ttgtataata cttgtttatg 647940
tacggtttat gtacgaagga aaatcacgca tggcatcgcc cactccagga caattgcatc 648000
tacagcaaaa agtagaatca aaggcctatg actattcacg cagcctcgct atgattgcta 648060
cagctttgtt attttttatt gttgctctta ttctttctgg attgagtctg cttcctcagg 648120
tcttcctccc cttttcagga gcgtatttta ttatcggttc ttttttagct tttattgcgt 648180
taggattctt tcttattaat tgcgtctgcg atctcaaaca gtaccttacc tcgtcttagt 648240

ttacaacctc gcaaagcttc ttttttttat tacaaaagtt ttttcctgac taagctgttt 648300
gtaataaatt agtttctcac atctataaga aatctgtgct aaagcccttg ggcttcgtgc 648360
cgatgttacg aatatccatt aacattaaat agatgttcgt aatgaaaaaa cttgtccgtc 648420
tatgcgtagt tcttctttct ttacttccga atgtattatt ttcttcggat cttttacgag 648480
aagagggcat caaaaagatg atggacaagc tgatcgagta tcatgtcgat gctcaagagg 648540
tttctacgga tatactctcg cgttctttat ctagttacat tcaatctttt gatcctcata 648600
aatcttatct ttcaaaccaa gaggttgcag tttttctaca gtctccggaa acaaagaaac 648660
gtctcttaaa gaattataag gcaggcaact ttgctattta tcgcaacatc aatcaattga 648720
ttcatgagag tattcttcgt gccaggcagt ggagaaacga atgggttaag aatccaaaag 648780
agcttgtatt ggaggcatcc tcatatcaga tatcgaagca acctatgcaa tggagcaaat 648840
ctttagacga agtgaagcag agacaacgcg ctctactcct ttcctatctt tctttacatc 648900
ttgctggagc ttcttcctct cgttatgagg gtaaagaaga gcagcttgct gctctgtgtc 648960
tacgtcaaat cgagaaccat gagaatgtat atttaggtat caacgatcat ggtgttgcta 649020
tggatcggga tgaagaagcc taccaattcc atatccgtgt tgttaaagct ttagctcata 649080
gcttagatgc acatacggcg tatttcagta aggacgaagc gttggcgatg cgaatccaac 649140
tagaaaaagg catgtgtgga attggtgttg ttctgaagga agatattgat ggagttgttg 649200
ttagagaaat cattcctggg ggacctgcgg ctaaatctgg ggatcttcag cttggagata 649260
tcatctatcg ggtggatggc aaggatatcg agcatctttc tttccgcggt gttttagatt 649320
gtttacgtgg aagtcatggc tctactgtag tcttagatat ccatcgtggg gagagcgatc 649380
atacgatcgc cttgagaagg gagaaaatcc ttttagaaga ccgtcgtgtg gatgtttcct 649440
atgagcctta tggagatggt gtgattggga aagttacgtt acattctttt tatgaaggag 649500
aaaatcaggt ttctagtgaa caagatctac gtcgagcgat tcagggatta aaggagaaga 649560
accttcttgg attagtttta gatatccgag aaaatacggg tggattttta tctcaagcga 649620
tcaaagtttc tggtttattt atgaccaatg gcgttgtggt tgtatctcgc tatgctgatg 649680
gtaccatgaa gtgctaccgc acagtatctc ctaaaaaatt ctatgatggt cctttggcta 649740
ttttagtatc taaaagttcc gcatcagcag cggagattgt agcacaaact ctccaagatt 649800
atggagttgc tttagttgtt ggagatgagc agacctatgg gaagggaacg attcagcatc 649860
aaacaattac tggagatgcc tctcaggacg attgttttaa ggttactgta gggaaatatt 649920
attccccttc tgggaaatcg actcaacttc agggagtaaa atccgatatt ttaattcctt 649980
ctctctatgc tgaagatcgt ctaggagagc gttttctaga gcatccctta cctgcagatt 650040
gctgtgataa tgtacttcac gatcctctca cggacttgga tactcaaaca cgtccttggt 650100
ttcaaaaata ctatcttcct aatctacaaa agcaagagac tctttggaga gagatgctac 650160
ctcagcttac gaaaaacagt gagcaaaggc tttctgagaa ttcgaatttt caggcatttt 650220
tgtcgcagat aaaatcatct gaaaaaacgg acctatccta tggttccaat gatttacaat 650280
tggaagagtc gataaacatt ttgaaggaca tgattttatt acaacagtgt agaaaataat 650340
tactgttgct ctttacatct gatctcgtac gtggaaagta gcatcccaag ttctaggatg 650400
cttgtgagat gaacgtctaa acgcgagcta ttttacttac taaaggtgaa agtacaggan 650460
ttccgcggcc atcattaagt actggggtan tagccctaga ggtatttcct tctgttttca 650520
catggccaaa aatcttattt gagttattaa ggatcccatc atcgagctga tcttctaaaa 650580
cacctgccca tttttggaaa accatgagtt tttcagatgt acaagcttca tcaaaacata 650640
atgatgtaac tagtaactta atcaatccta tggcagcagg aataatcaac caaaatgcat 650700
ttgcccctaa ctggctatgg aagataaaca tacatgctaa tccagcaaca accaaaagaa 650760
ttcctaaaac gagcagggta atttggcaag ctctggattg aaagagcttt gattcacgta 650820
taggttgaat aaattttgtc tttgcccatc ctatgagtag attcaccatt cctggactgg 650880
acgtaattgc ttgtagacca gcggtgacac tccccgatgc tgctatttcc tctactatgt 650940
ttagcacaga ctcaggagca gctgctcctg ttcctgttcc tcctacggga tgtagatttg 651000
atgacatacc gttctcctag tgtagttcct aaagaagcag gttacactag gaaaacatta 651060
attaaaaatt ttaattacat acctaaaaag atgggctttt aaaataaatc ttaggatttc 651120
agaaacttaa ataatatctt tgattaagaa actctacgat tggattgaag agcttataaa 651180
acaaaaaaac cgctttagta cgtaaggtac taaagcggta cttctttgaa agctatccta 651240
aagcagagcg gaatatcgct ctgctttagg ataattcctt agaatttaat acacgtgggt 651300
attttctgtg tctgatactg gtgaagtcag tgtatcagaa gaaagaatag cttcgccgcg 651360
agcatctccg ggagcaatac ctttcaaggt aacagaaaac tctacagatt ccttagaacc 651420
gagtttaggt aaagcgtcga aaacaacggt attacctgaa atcgttcctt tagttggacc 651480
tgaagaagct attggctgaa gttcttttga gaacttcaag attaaagata cgttagtatc 651540
ttcagcagaa ccacggttag ttacacagat acgatagaca gtattttctc ctacacagat 651600
aggatcattt gtgtctaata cgcacatatg ggtagctgca agacctttcc aatgtgttgt 651660
tgtttctgcg caagatgtac atgttccgca gttagactca ctagttactg caacttgatt 651720
tgtgaatctt ccaggaactt gagctttcac tacaagttta aactggaggg tttctcctgg 651780
gcacatttct ttaatacgcc aaacaacttt attacagcag atctctccac caggagcttc 651840
gagtactgta acaccagaag ggagtgtatc ttggatcacg acatcatgaa gaaccaagtc 651900
tccaggattc gatactgaga tagagtactc cacaggttta catacgtaag accaatcagc 651960
accagagata tttacttgta cacaaggctc attaacaact gtagttacat ttgcagaaca 652020
tttgtgtcca ccgcagtaag ttacagtagc aacgttagtg atttgacctc ttctttgagg 652080

gcagaactca actgtaaata cctttttatc gccaggtctc atgtctccta agttaaaaga 652140
gagaactctt tgaccagatg catgagaata gccatcggga acaggattat ctacagttac 652200
gttacgggca atagcagatc ctgtgttcac tacttcgatt ttgtagcata cagggcatct 652260
taggcaagca cagtcaggtc cttcttgctt aatacaaatg gctggttgac cgcatttagt 652320
ataagaacgg agctctgggc aagcacatac agtagcagct gtgaagcagc aaccttcttt 652380
aagaggtttt acccatacag taattttgca tttatctcct gcacccaggc gatcgatttt 652440
ccagactaat ttcccatcac ttgtaggagt tgtttctgga tcactgctta cgaattcagc 652500
ttcgcaaggt agctgttgtg taatcacaac atcaacacaa tcttttttgc ctatagcaag 652560
gatttcaata gggtaaggag atcctacagt agcgtattct ggaacggact ggcaaatttc 652620
tacgttgcaa tcatcgttta cttttacaga atacaatctt ccgtagcaag actcttgctg 652680
agcctctaca ggttgacatc gtccctcttc acagggataa aattctttat cacaaaaagc 652740
accacggctt ttttgttcaa ctggttgttt atttctacgg acaagtctaa ccttcttcgc 652800
tgtcatagga acaggtgctg gctttgtttc cgcactagcg acgatcttag taatcagaga 652860
ctctgctaca gcggcctcta tacccccgct ggcaaagcaa ctcgccatac tcgttagcgc 652920
aaggaccgta actactcgtc tgatgagttt ggacataggg atctcctatc gcatgtttat 652980
ttttttcttc tgccatcggg tagaatgttt tgtaaactct taccctcaac atttgggttt 653040
attatctgaa ttaaggtagt caggaattaa ttcatctcct aactacctga tcaatttaag 653100
taaacagtta agagaactct ccttactgtt tgcatctgcc atcaggtgat gtacaacctt 653160
taacttgtgg gctttgagag ttacactctg ttgaaccaca tggattagaa caagaaggaa 653220
cgtaggaccc acaagcatta ccgccgcaag atctttcttt ttttcttatt acttcacaag 653280
gattgcaaga agagggtgcg caaggatcct caaaacaaca atctacaatg cggcagcagc 653340
tacttaagct aactactcca caaaacattg cagcaattaa aacagctttc ttcataagtt 653400
tttaactcct ttctaattag aaaacttacg tgtcttattt attactgaaa attcaacaaa 653460
acttttaata attaataaag cacgttccca tgaacaatat tacaaatgaa aataaaaatt 653520
ttatcaatta ttttttctag ataaatcttt ttggtattaa aactcttttt agaaaacact 653580
atttactaaa agataaacct ataattttca aaaggaagcg tcatcaggaa tttgaggaaa 653640
ctgaagaata aaaagaataa tttgtttaat aactaatggt ttatttcacg agactttttc 653700
acttattttc tatattaaaa acacataaga ttcacaatca acgagttgaa gtagaatgat 653760
tgttaatccg cacaatcaaa tcattgaatt ataacaaaat aatttcgtaa cattaaaaaa 653820
taaaaacgag ggggaaatga aattatttta cttttgtaac attattttcc tgacatctct 653880
attttctgta agctgcacgc acacgcctcc tccacgtagt tatattcttg ctcaaggaag 653940
aacgcccctc gtcaataaag cgcattcctt agaattcttt ttagcaagat ctgtctttaa 654000
cacgtgttat aatacgaact tatagcctca caaatcacga tcaagagaga ggaacgcagg 654060
actactctgg gatagagctg cgttgtagat tgtcttcgca acgactcaaa caggaagtgt 654120
ggttatgaat agaaaccgcg gtggctgttt cgcttagcta gcagccacgc acattcatgc 654180
gaccgatatt tagagatcga aagtagcctc ttcgaagtct tctcgttgca tgaacttatc 654240
cacagttgca gcgagcttct taggaacgcc tcctaaaagc ttttctgcat acacaagacg 654300
tgctctcgct cgaggtttcc ctaaaatctc tatagagtca aagagaggga gcccctgctt 654360
tttcccagta attgccacat aaagcagtgg gatgatggct tttttatggt ggacattgaa 654420
tgcttgagct aaccatttag atccgaggta acacgtctct ttcgtccatt gatcggtttt 654480
ctcaagatat ttgacatagc tatagagaag gatagctgct ttctctggag aaagggcctg 654540
tggtaggagt tcctcaacac ggtactctaa caatcctgag aaaaagaacg aggtgaggtt 654600
gataaactcc gcaagagttg taatccgaga ttgacagagg ggaaggattt ttaagaaaaa 654660
ttcgtcatta agtagccaac cctggagttc ttttaacaag cactctggcg aaccctcgtg 654720
gttgaggtaa tgcttattca tccagtcgag cttttgaata tcaaaaactg ctcctgactt 654780
tccaatacgt cgaggattaa aagtttctat aatacgctct agagaataga cttcttcatc 654840
cccttccata ctgtaaccca tgagagtcag gaagttcaca aaggcttctt tgacataacc 654900
tgagtcgcgg taataaaaaa tcgaggtagg gttctttctt tttgaaagtt ttgttccatc 654960
ggggtttaga agcaggggca tatggagaaa gacgggaggc tcccagccaa aagcttcgta 655020
gagtaggaga tgcttaggag ttgaacttag ccactcttcc cctctgagga cgtgagtgat 655080
gcccatgagg tggtcgtcaa ttacattagc aaagtggtac gtggggaatc cgtcagattt 655140
tactaggacc tgatcatcga catctgccca aggaaacacg actcttccct tgctataatc 655200
ttcgaaaaca cattcccctg ataagggaac tttaagacga atggtgtagg gctgacctgc 655260
tgcctctcgt gaggctacct cttcgggaga gaggtacctg tacctgcgat cgtatccccc 655320
acgataaccg agggtacttg ccacagcgcg catttctgcg agttcttgag gggttgcaaa 655380
gcacttataa gcgcaatctg tctttaaaag tgtctcaaca tacccttgat agattttcgt 655440
gcgttctgac tgacgatagg ggccataggg gccgcctaca tcagggccct catcccattg 655500
gatcccgcac caacgaagag ctgagaaaat attttcttcg taatcttggc gactacgtgt 655560
tctatccgta tcttcgatac ggaggatcat tttcccttta aatcgttttg caaagatttc 655620
attaaacaga gccatatagg cggtacctac atgaggatct cctgtaggtg aaggagctac 655680
cctaacacgg acatttcccc aattcatgat tcttttatcc ttagaccaga gagacttcat 655740
accctgaaaa cggctatttc ttcaagggaa atcctctctg cagaaatgca taaatccaaa 655800
aataaaagaa actccttaat ttaataaaaa attaaggagt ttctcaaaaa aatcacgaaa 655860
ggatatttcg ccacccttag atcggggatc gcctttaaag tatcagaaag aagtagttaa 655920

tcaacaatgt tgtaagtaga aaatcgaaaa atcgagagcg agttagttag attcaggaaa 655980
aatttcagta ggaggttcga aatttggagc ctgatgttct ttcggttcag ctccttttag 656040
ttttttagct gcttgcttac tcagatactc attcttatat ctttcaatct cagatacgtg 656100
aattacccaa gcggctccct tacgctctcc tcgtatggtt cccgtacgag ttgcatagta 656160
gaccttctgc acaggtattc ctaaaatctg agcaacctga tttatggagt agcatccttt 656220
accgttatcg aaaacaagct ctccttgata aagagatttc tttcgagagt aacggttacg 656280
tttgtactct tccaaatctt taatatctat ttcccagcgc gtctctttag aagcttttag 656340
ttttttctgc ttaattgcca cataaattgc ttgcctagtg acgttatgta atttagcagc 656400
ttgagtgatc gaaacccatt ttgtatctga gtctttgata tcctcaattt cttctctttc 656460
ttctctttct tctaattcgt agcatccctc atgttgttcg cacgccataa gctagcatcc 656520
cctccccctc aacatattta caattaaaac aaccgtaaca gttagtttct tccttgtttt 656580
tagaagtttt taaagaagca ttttcctaaa aaaagcttta ttaatcaagc ttttttgtta 656640
atacaaagtt tatgttttca gattaaaatc ttaataaatc gtaagcagga ttatgaatta 656700
ttaatatttc tttacgttat aaaaaatagg tattcttaaa aaaacacgcc ctgaatatcg 656760
ccagggacgt ttttctaatc ataacgtttt tcttaagtag acaagacaaa ataccaagaa 656820
ccaacaaaat atctactctt tttcttttcc tgaacgaagt attttttttt aattttcctt 656880
atcggtcagc agtttacaga agccagggat aaggaaacta caaagatttc tttactcttt 656940
taaaataaag ctctcgattt ttgatttaaa tctgcgatga accttaggaa actcattttt 657000
ggtttaaaag atgagtttct aattatcgaa atgtcttcga atatgacctg caacttcttt 657060
aatcacatca tcggaaagga tatgatcgtg tcttagaccg gtctgagcta cagggatttt 657120
cttccctgaa cactcttcca agtttttagg atctaaaaat ggtgctgcga agcacgtctc 657180
ttttttgaac aatccatccc cgataagatt accttgggaa tccttgccat aaataaagag 657240
ttctgggcaa tgcaagtcct tgcttctctt ttcagaatta atattccaat gggtaagatt 657300
cgccagccaa actcctagac ttccaataaa ctgtttagca acggctcctg tagagcgagc 657360
tcctcgatct ttaacgacaa accaacggac gctatcactt ccgtctgcga tctctttact 657420
taatgcttcg gcttgaacac tagctcctaa agaatagcca taagcaacga tttgacgcgc 657480
ctgaggtcct gcgggttcat ctctaagata gcgtacgcat gcttgataag atttgactac 657540
attgtttctt gttatattcc cttggctctt catgactcct gggtaattga agattaaaat 657600
gttggattga gactcttcag caatacggaa tatccagtcc ttttcccctt gcagcactgt 657660
cctatactct aagcaatcgg agtttccatt ggagattaac atccatcgat ctggcttagc 657720
attgggaaga cgtaactcca atccgtcaat aaagacctcg tcatactgta agcaaaccct 657780
tcgcacagag gagacatgat cttggaatga agcggagaaa agacgcgcgg cgtaagcttg 657840
tcgcaataaa ttagagtccc tgcatatggg tctaaaaatc caccctcctg caccaagaag 657900
aataaaattc tgacatatct tctgaaggac ccagaaaaga cccaagggaa taaagaagat 657960
taagccgaga agaaatttca caacacccca tatgatctca agaagacgat aaaggtaagg 658020
atgagcctgt cgtttctccc aagaagttct cgcctgctcc gaagaaaaca tggcgatcga 658080
aggtttagga tgcatatcca atatagctgc gtgttgttcc cttgcaatag ctattgacat 658140
tattacctta taatttcatg cagaatgtag tgagcaaact attatagtgg aaattattga 658200
ataaaccaaa aacaaaagcc gagtttattt atattaatat aagtaattat tttaatagaa 658260
tcgacctcga accattagat agataggtga cgaccctcat ctagaaaaga acattcattt 658320
attatgataa tgaagtaaat cttttacttg caatgaacaa agaaccaatc gaatgaaagt 658380
tagcggtctt tttttagtct gaaaatctag gttctgagga aatacggaaa tcccggacca 658440
gcaggtggat gactcccgat ccagaagttt gggacaagcg gggtgtgtaa gcaatctcaa 658500
gagggtaatg ccaacttgct tttaatgcat cagcgtgtct tcccagaccg aagcgacacc 658560
ctcaagattt ctttcttttt ggcttaagta gagtttgaga tggtttccag gcaatacttt 658620
tggatagcgt acctggcgca cttttgaata gaagataggc atcagattcc ccttcccaaa 658680
aggctcgaat agttccatag aagctaggag atcataatct atagcatcaa aatccgcata 658740
agcatcaatt tcgagatgag gaagtgtgtc acccttttta agagaagagt tcacgagatg 658800
aacgaatttt tttttaaaat cttcgacttt atcttccttc ataatcacgc ctgctgcaaa 658860
gtcgtgtccg ccgtaagata aaaggagcga ggagcatttc tttaagactc cgagtagagg 658920
aaatgaccct atagttcttg ctgatccctt tccaattcct cgttggatag cgatgattac 658980
cacaggtttg ttataagtct tagcaagacg cgctgagata atagggatga cacgagcatg 659040
ccatgccgtg gatgaaagaa ctatagcagc ctgctttaaa atctcaggat tactatttaa 659100
tatctcttgg acatcttgaa atacctcagc ttctattctt tgcctttctc tatttatatt 659160
atctagctcc ataattagag catctacacg ttcatcatct tgggtgagta aaagttcaac 659220
accttttgca gggtcgtcca accgtcccaa gctattgagt tttggtgcga tcttcaagac 659280
aatatctgtc gaagtgactt cgcttttttc tactccacat aatgcgcaga gtttattcaa 659340
tccgggtcgc gcgcctctgg caatttcttt aatcccatag cgcaccataa cacggttttc 659400
ccctagcaaa acaccgacat ccgtgatggt tcctaatgtg actaaatcga gtaatttttt 659460
caggctacct tgactcttgg ggacaagatt tctggatatc agtgcgttca gtactcctct 659520
tgcgagctta aaagcaacgc ctacgcctgt gagttctcga ttcgggtagg tatgatcccg 659580
taatttagga tttaatgtaa ctacgcagtg ggaatttttt cctgtcggca tgtggtgatc 659640
tgtaatgatc acatcaatgc cttgtcttgt aatatcactc acctcttttc ctgcagtaat 659700
tccgcaatct acggtgatga ggagtgtaat tccttcctct ttcaactttg caatgagtgt 659760

ggaggtctct ccatgttgct tgagtatcgc accaagaaag aagtagctaa cgtggacatc 659820
aatatctctt aaaaattcga ccaggagagc gacgcctgtc atgccatcga catcgctatc 659880
tccataaatc atgacgtgtt ctttacgatc tctagccagg agcaggcgtt ctacagcctt 659940
tgacatatct aggaagagtc caggatcata aaggctcgac agatggctgt ataagaactt 660000
atggatttcc tgaatcgttt ggaatcctct tgagataaaa atctgagcca ctgtgggagg 660060
caagtgaaat tctttgataa tcattccaag aaacgcagga tcttccttgg gatgagccca 660120
gagcaatcct gctgcagaag cattatctga atttgtcata aatttaatac cacaggggcc 660180
tttgggacta aagtcccaaa ggagattttt atattgctac acgttagatt aagtttaacg 660240
gtacttattt tgagcgattt tctttacgga ccataaacaa caacagaggt ggtgcaatat 660300
aaagagacga taaagttcct agaagaatcc ctatggtcat aataaatgca aaattaaaga 660360
cagaggagcc gcctataaac aaaagcatta acaaaactga tagagttgta gctgttgtca 660420
ttaccgtgcg gctgaacgtc ttttgaaggg catcattaac taaaacatgc ataggggtaa 660480
acaggttcgc ttggcgatct tcacgaatac gatcaaaaat gatcaaagta ttgtttaatg 660540
aataccccaa tacagtcatt aaagcaccaa tggcttgcaa atctatttga attttcttca 660600
aaaagaaatg tgctataaac aagactgcac aggtagccaa aaggtcatga attaaagcgc 660660
atacggcact gaaagcatat tgccattcaa agcgcaaact cacatagagc aagatgattg 660720
ccaaagctcc taaaagcccg atggtcgcct gataacgcat tttcttcgat agtttgctgc 660780
ttacctttga ccaaaaattt tgcgtttcgt ttagagtttc cgtagagaaa tctaggcctg 660840
tttctgacaa caatcccaca gctaacgcca gctcatgatc gttaatttta ggagagaggc 660900
tcgtatctgc tttagtatag cttaaagctt tatcactaaa atagattttg atcttttctg 660960
aagatccaaa tgtttgaata cggaagtctc tagaagaaag accagcttcc tgtagtttat 661020
gcacaacttt gccacgcatt tgagcaacat cgctgatgcc atgctctttt ggattaaagg 661080
taaaggcata ccctccttta aaatccattc ccaaaacgga attccaggct ccaaacccga 661140
gagcaacgca acctaaaaga aaaacacttc cagaaacagc ccaaagtttt ttgcatcctc 661200
tcaagaaatc atgctttatc cccacgaact tattcatcat atgcaactgt gtatgttggg 661260
tcttattcat ccacagcatg aagaaaaatt tagtcatgaa aagagccgta aacattgaag 661320
agaaaattcc taaaatcaat gtcaaagcaa accctttaat aggccctgta tctaggaaga 661380
aaagaagtgc tgaggccaat actgtagtca agttagaatc aaaaatggct ccaaaagcct 661440
tggtatatcc tttttctaca gattttttaa gactttgaga caataaaaat tcctctcgga 661500
ttctttcgaa tacaagaaca tttgcatcta cggccatccc catagcaaga acaatcccag 661560
cgagtcctga caaggtgagt ggcgcatcca aatactgtag agctgcccag ataagcaaaa 661620
gattcagaag aacagctccc gaagcgatga cgcctccaaa tctataatat acgctcatca 661680
aaacaataag cattgccaag ccacagcatg ctgagataat gccttgtgta cattgttttt 661740
tcccaagatc agaagagatc gtctcttcac tgagaacctc gggaacaaaa gacatcgctc 661800
cagattttaa atctgaggcg agtttgctca cttcacggtg ggtaaatttc cctgagacac 661860
tggcatgatt tttcaatggg acgtttaaaa tagggctgct gaccatataa ccgtcaatca 661920
ctacagccat acgccatcca cggtttgcag aatattgtcc attagcagtg ccgctgatcc 661980
cctcctgaca atatgcggaa gtccatgtgt ggaaactctc tgtaggagaa agtttctctg 662040
ccattttctt agggcttgta tctttgactg aaaaatttaa aacataacct tcccctgcag 662100
caaattctgg acgaatgtct tttagggaag ctccatctaa cgcataattt ctaaaaacaa 662160
tgactaaagg atttgctttt tgttctgcat cttttccaat agcaatcata gaaaacgtcg 662220
tatctaaatc tgtcgaaggc gtttcgcatc ctgaaggaga gaacgccaac ccctcacttt 662280
taagcttagt aatggcctca tggacacttg gaggcacatc gacttcctca ttaaataagg 662340
cgctagcgaa ggtattgatt tcttcgggag atgtctttcc ttgagcttga gaggtaaacc 662400
aaagatagtc taaaaatctt tgcacttcgt agcgggaagc gctgtaagaa gagaacctct 662460
cattcaccac atggaaactc atttttgagg tccccaagat ctcagatgag gagatcgtag 662520
aagatcctgg cacactgaga tgaatgtaat ctccctcacg gcgaagttcg atttcagata 662580
ctccaagttt atttaatcga gcacagagct catccgaaac tttaagaata tcttccttat 662640
cggtgagctg ctttccctga tgatctttaa aagagagtag cagctgacgc ccaccaacaa 662700
aatcaatacc aagacgtagt atgttctccc cacgagaaaa ttttctcatg ttgagtttca 662760
tattttctaa gaaaagattt tgataaggaa tcggagcact caaacgttcc tgaagatcca 662820
tagaacactt tgcgtgtcgg tattgctcat gccaacgcac taaatcgctt tgacgattct 662880
tctcgatttg gtttacagtt gcaaggcgat cttgaatgtc tttaacttct aaaaaggcac 662940
agccctcttt ccctatgaca aatccctcac cccatacatc tagaaattgc tgcaaggggt 663000
gccggatttc taagacttga tcttctccta gagtccagga aatcgcttct gtatgagaaa 663060
aacagttata gagattttgt agatcttttt cgaaactttg gagttcttta ccccctccct 663120
gttgatattt cgcgacgata gaacggagtc ctttcaataa gatgtaaacg gagcctttag 663180
aaaagtgttt gcaatctgta ttgggagaaa agatgtaaca gccaaaactt cgctttctct 663240
aggttgacga cagaaaacag gaaaattttc tggaatcagg tcacaagatt cagctgcagg 663300
cctatgcaaa gttaatgccg tgagatgttc tgcgattcct tggagcaaac gctctccctg 663360
gagaataatc tttccctgag tatctttatc catccattgg aaggaaaatc cgttgttata 663420
atcttctact tgaacagtta agttcttaga gagcttttgt ttttcaacag ccaaacgact 663480
gtcaaaatct aagcgttgtt ctttagacaa agacgtacgc tgtgctaaca aatcagaatg 663540
tagtgtcagg aaaattttac gttccttagg gcaatactct atggaactaa aaaataatga 663600

ggactcacta caatctaatc gaagcccttc aatataaggg caatctaaac tacgaatgca 663660
tgcagtttga gaaagagttt catatacact cttgagtagc ttttgatctt caacatcgat 663720
tgctgcgtcg tttgacaacg aagacaggcg tgataaaaac gcaagtctat cttggttgga 663780
agaaaaggac tgttggcaaa acgctgaaag tcgcgaagaa aaaacctcaa atcctgaaga 663840
aaggttcttg gcatactgta acaattgttc tttaggtgcc gtttcccaga tagaggggta 663900
actacaagaa cagtcttttt gtttcggaca agtacatgca gaatagactc gttgcaaaat 663960
acttgaggcc atctcttgtt cattttcgga agaataggaa acaaaagaaa aatcactttc 664020
tactaaggaa gtatttatag agcttgctac ttgtattaca tggtcgtcgt gttctcgact 664080
atagccaacc acatggagtc ttgcagactt tatgggaacg ttaggctctc catgaacgag 664140
gttcccgata aagtcctctg catcttcacc tcttttaaaa cgcacactga caatatctgg 664200
aatcgcagga tgttgttgta tgtgcccacg taaatgtagg gacgaaagga tcgctgagac 664260
gcgaggaatg acatccttac gaacttgctg ggcctgcttg gtaaaagatt tgattatatg 664320
ttcggcttcg tttccatcta ttttcttatc cagaggtttg gcgtaatata aacatgtagg 664380
caaaacgtaa tacaaagcca aagcaaacac gcaaataatg atagcgaagt ttcgcttaac 664440
cttctgtttc attgcaccgc tctacttttt attaggaaat attaaaacca aaataccagt 664500
tgaaaaaaaa atcgtcaaac gaagaggagc cctccacctt caaagagagc tctttattct 664560
gtatctttta caaaatacta tttttaattt tcctaaaatc tctatctcaa aagggtcaaa 664620
ttcaataaaa actaggcaaa agtatagctc tcttgttcgc caacaaaacc agaaagcgaa 664680
ataggagtta cactatgcgg ggttgaggag tgatgagggg aaaaatacag tgaggttggg 664740
cttgtgggat ctgtgaaaat aaactttatc cgaatggaat cttgtttctc ttctctcatt 664800
cgttttgcca cctgctctgc agagtagagg aaagcggctt gtgagagcag ttttaggttt 664860
tcctgttcct ctttagggag tgcatcagga actccaagga taggagtgag tacgtagatc 664920
gtatgttttt tcactatatt aggatcgccg atttgatcta acacagtaaa aaatgcttta 664980
gtataggcat cgatataaca tcttccctga ggttcctggg ctgcgtcaga aatatttaca 665040
taaatcgcgc ttttaggatg cgtgcggtca ctagcttggt cttctgagct ccaggaaatg 665100
actttaggat ccaaatattg ctgttcctct ctttggattt cagagagcaa cgaagacact 665160
acagaaggat ctatagcaaa agttttgcgt gataaggagc ttttttcgat cccctgtctt 665220
aatgcgatct gatccatttc ttggatcaga aagatactgg tatttctatc ggattttttc 665280
tggaatatct tagtaacgat ctcctgggag ccttcgcaga tctctacttg gggacagcct 665340
ggatgatagt agcatccggg tcgtattttt ttccaagact gcaagagaga gaggcatcct 665400
aaccaattta taggtttttc aaagacctta gtttctcgaa atagaggacg ctcgaatcgt 665460
gaccatccct tagagtattg taagtacgca aaccctaaaa gaaccaatcc cagaatcata 665520
agacctatgc acgggaaaat ggtttgacat accccagcag cgaaggcagc acatcctaac 665580
aacgccaaac caaaacaagc taaaacaccg cccagtattt tcttcggcag gttttgtgca 665640
ttggggacgt agtggacatt gccntcctcg gccgccacca aatgaccatt tgagtgggaa 665700
tcaaatctga aatggatatg cgaggaaatt tcatactacg aagcatcctt ttagaatttc 665760
tttttatttt taataaaaaa ctagaatcga atagaattgc actaatatta acaaaacaaa 665820
aaattaaaaa acaaaagagt ttgtttcaaa attttcattg aaaaataaaa agaacactaa 665880
aattgtttcg actttgaaaa aatgaatgtc aacttgtcaa gatattctag tttcttctac 665940
tatgcaacct aatattttct actcctttgc tcgtcatgag taagccgagt atacttagat 666000
taggaggatg cctaagaagc ccttgtattc atttgaagta agaactctca aaaattttct 666060
tcttgagaca ccgatctgct tagagaatag aatacagcct ctccctactg ggatggctgg 666120
ggaaaaatcc tttgtgattt ttgtcttagg taagtgaacg aattctcctt tcttaaaaat 666180
aggttcgaaa cgttgttcct gagaacgaat tgtgacttac gccctaataa atgatcctgt 666240
agacttgtct ttagctacca acaatgctga atccaagttc ccctctctac agcgccttcc 666300
caaccatgtt gctatcatta tggacggcaa tcgccgatgg tacaaaaagc atagggagga 666360
gtgcggccac acacacacgt caggtcatta ttatggcgct aaagtccttc caaatatttt 666420
aaatgcggtt cttgatttag gaattaaagt tcttactctc tatacgtttt caacagaaaa 666480
ttttgggaga ccaaaagagg aaattcaaga aatatttaat attttctata ctcagttaga 666540
caagcaactt ccttatctaa tggaaaatga aatctgctta cgttgtatag gagacctttc 666600
caagctccct aaaggcatcc aaacgaaaat caaccatgtg agtcgcatga cggcatcgtt 666660
ctcgcgttta gagctcgtat tagctgtcaa ctacggtggc aaagatgagt tagtccgtgc 666720
atttaaaaaa ttacatgttg atattctaaa taaaaaaata tcttctgacg acctttcaga 666780
atctttgatt agctcatact tagatacttc aggacttacg gaccccgact tacttatccg 666840
tacagggggt gaaatgcgtg tcagtaattt cttattgtgg caaatagcat atacagaact 666900
atatatcacg gataccttgt ggccagattt tacgcctcaa gatttgtttg aagcgattaa 666960
cgtataccag caaagatcaa gacgaggggg gaaataggtg cttaattcaa ataagtttaa 667020
atcgaagacc ggtgcatacg gtgatttatt tcagcgtgtt gttgttcatt cgttagtact 667080
tacatttttg gttcttcttc tctatagttc cctatttccc ttaacttctt ttgctctagg 667140
gtttattacc gcgacttgtg gcgctgtagg aacttatgag tactcctcaa tggcgaaagc 667200
caagatgcac tatccattaa gcacgtttag tgcgatcgga tcttttttat ttttagcatt 667260
aagttttctt tccattcgtt ggggacacag tctcccagga ttcttcgatg ctcttccttg 667320
gaccttgctt attgtttggg tcgtgtggag tatctttaga gttcgaaaat ctacaatcgg 667380
cgctttacag ctatcaggag tcactctctt ttctattttg tatgtaggga ttccgatacg 667440

tttattctta catgtccttt atagctttat tcatacccaa gaaccctatc ttggaatttg 667500
gtgggcttct tttcttattg ccacaactaa aggtgcggat atcttcggtt atttcttcgg 667560
taaagccttt gggaataaga aaatcgcccc acaaattagc cctaacaaaa ctgttgtagg 667620
ttttgttgca gggtgtttgg gagccacgct cattagtttt attttctttc tacagattcc 667680
cacgaggttt gcgagttact tcccgatgcc tgcgatttta attcctttag gtcttgcttt 667740
aggaatcaca ggattttttg gagatattat tgaatccata tttaagcgtg atgctcattt 667800
gaaaaatagc aacaagctca aggctgtggg tggtatgctg gataccttag actcactgct 667860
cctgtccacg ccgattgctt acttattttt gctcataacc caatctaaag agtttattgg 667920
atgattatca ctattgatgg gccttcagga acaggaaaaa gcacaacagc gaaagcttta 667980
gccgaccatc ttcatttcaa ttactgtaat acagggaaga tgtatcgcac tttagcctat 668040
gctcgtttac aatctccctg ggcgacgctt cctttaacta aatttttaga agagcctcct 668100
ttttctttta cctttgctac aggccaacct ttagagtcgt tttttaatgg tcatcttctt 668160
acctctgaat taacaactca agaagttgcg aacgcagcat cggagctctc tcaacttcca 668220
gaagttcgtg cattcatgca agatttgcaa cgacgctatg ctcagcttgg caactgtgta 668280
tttgaaggaa gggatatggg atccaaagtc tttcccaacg cagatttaaa aatttttcta 668340
acttcaagtc ctgaagttcg tgcgcaacgg cgtttaaaag accttcctga agggactctt 668400
tctcctgagc aattgcaagc agagcttgtc aaacgtgatg ctgcagatgc acaacgcgct 668460
cacgatcccc tagtcatccc tgaaaatgga attgtaattg actcttcgga tttgacaata 668520
agacaagttc tggagaaaat tttagcttta ctatttcgaa acgagctatg attttccgca 668580
tttgtaaatt tttcacgtgg gtagcttttt ctcttttcta taagctaaaa gtttatggag 668640
tgaaaaaaaa ttttattaaa ggtcctgcta ttattgcagt aaaccataat tcttttttag 668700
accccatagc attgcacatg tgtgtccatg agtgtattta tcacctagca cgggcctctt 668760
tatttaatat cccctggtta tggaagcaat gggggtgttt tcccgtgcgt caagacgaag 668820
gaaactctgc ggcatttaaa attgcctctc ggctctttaa taaacgaaag aagttagtga 668880
tctatccaga gggggctcga agccctgacg gtcaactcca gcctggcaag gtcggtattg 668940
gtatgatggc tgcaaaatct agagttccga tcatccctgt ctatattagg ggaacttttg 669000
aagcttttaa ccgtcatcaa aaaattcctc atgtttggaa aacgatcacg tgtgttttcg 669060
gtactcccat gtattttgat gatattattc aaaatcccga gatcaaaaat aaagaaacct 669120
atcagatcat cacgaatcaa actatgaaca aaattgccga gctcaaagca tggtatgaat 669180
cggggtgcaa aggagacgtc ccctaaactt atgtcgacat tactttctat cttatctgtg 669240
atatgttctc aggcaatagc aaaggcattt cctaatctag aagattgggc tccagaaatt 669300
accccgtcta caaaagaaca ttttggccat tatcaatgta acgatgcgat gaaattggct 669360
cgtgttttaa aaaaagctcc gagggctatt gctgaggcca tagtagctga gcttcctcaa 669420
gagccttttt ctttaattga aattgctgga gcaggattta taaactttac cttctctcca 669480
gtatttctaa atcaacagct agaacatttc aaggacgctc taaaattagg atttcaagtt 669540
tccccaacct aaatnattat cattgatttt tcctctccaa atattgctaa agacatgcat 669600
gttgggcatt tacgctctac aattattggg gatagccttg ctaggatctt ctcctatgta 669660
ggtcatgatg tacttagact caatcatatc ggagattggg gaactgcatt tgggatgttg 669720
atcacctatt tgcaagaaaa tccctgtgac tatagtgatc ttgaggatct tacgagtctt 669780
tataagaagg cctatgtctg ctttactaat gacgaagagt ttaaaaaacg ctcccaacag 669840
aatgtggtag cattacaggc taaggatccg caagccattg ctatttggga gaagatctgt 669900
gagacttcgg aaaaagcctt ccagaaaatc tatgatattt tggacatcgt ggttgaaaaa 669960
cgcggagaat ctttctataa ccctttcctt cctgaaatta tcgaagatct agagaagaaa 670020
ggccttctca ctgtttccaa cgatgctaaa tgtgtatttc atgaagcctt ttcgattcct 670080
tttatggttc aaaaaagtga tgggggctac aactacgcca ccacagatct tgctgcgatg 670140
cgctatcgca tagaggaaga tcatgccgat aagatcatca ttgttactga cttaggtcag 670200
tctctacatt tccaactcct tgaggctaca gcaattgctg cgggctatct acaacctgga 670260
atattttctc atgtaggctt tggccttgtc ttagatcctc aagggaagaa acttaaaacc 670320
cgctctggag aaaacgtaaa gctccgagag cttctagata ctgctattga aaaagctgaa 670380
gaagcattgc gagaacatcg acccgaactt acggatgagg caatccaaga aagagctccc 670440
gtcattggaa tcaacgctat aaaatacagt gatctctctt cccatcgcac tagcgactat 670500
gtcttttctt ttgaaaagat gctccgcttc gaaggaaaca ccgccatgtt tctactgtat 670560
gcctacgtgc gaatccaagg aattaaacgt cgtttaggaa tttctcagct gtcattagag 670620
ggacctccgg agattcaaga acctgctgaa gagttgcttg cattaacttt gctacgcttc 670680
cccgaagctt tagagagcac aattaaagag ttgtgtcctc attttcttac agattatctt 670740
tataatctca cccataaatt caatgggttc ttccgtgaca gccatatcca agactcccct 670800
tatgctaagt ctagactgtt tctatgtgct ctagctgaac aagtcttggc tacagggatg 670860
catctcttag ggctaaagac tttggagagg ttgtaagttc ttcctgttcc atatcaaaga 670920
tctgaatttt agctccgagg cttctgagct tgcctaccca atttgtatat ccacgatcta 670980
gaagatgagt gttctcaata atagagcctc ctccttctgc aatcagtgct gccatgacat 671040
aggcaaatcc tgctcgtaaa tctggaatga ctaaatgcga ggcccataaa ggcgtcgccc 671100
catgaatcac agcgctgtga ggaaaattcc cgatagcata acgacatgcc ttggtactta 671160
agcactgatg aaaaagctga cactcagctc ccatgtgctg aagaccatga aggtagccga 671220
ggcgattttc atggacagtc tcatggatta ctgaagatcc ctgagcctgt gatagtagaa 671280

ctgcgaaggg ttgttgccag tccgtcagga accctggatg aacatcggtt tccaagacaa 671340
cacctcccac caaggggcgt tcttgaaaaa attctattcc cgactcggag accaaaaatc 671400
ctccgcctat ggagcgcaac atcttgagga agggaatcag aagttcttgt ttagcatttc 671460
ggacgaaaac acgtcctcca gagacaaccg cggccattcc aaaagaggct gcttcaatct 671520
tatctggaag gatggtgtgg tctacagagc cgagacctcc agtgccaaaa atatctatcg 671580
tacgatcatt atcggtagtg atatccgccc ctgccttttg caaaaagagc accaaatcta 671640
agatttcagc ttcgagagct acatttttta taaccgttct tcctttagca tgaattgctg 671700
cgagtatgag attttctgta gcccctacag aaggataggg tagatgaata taattccctt 671760
taagacctcg aggagccttt gcgtagtatc ccgaactatc agaggaaatc tgcacaccaa 671820
gttgttttaa cccttcaaaa tgaaagttta aggtcctttc tcctatagca tctcccccca 671880
cagtgggaac atagacacct tcagggcaac gccctaacag cgctcctaat aaaaggatgg 671940
ggatcctatt gacatttgaa aatgtgggag gaactcttgt gcattgtatt tcgggagtat 672000
agatttccaa aacctctgtt tccttatccc aagaaacatg tgctcctagc gacttgcata 672060
actctacagt taaggaaaca tctcctatat cggggacatt ccgcagtgtg cacttctgat 672120
cagaaagtaa agaagcaaca agcagcttag ttgcagcatt ttttgctcct gaaactttta 672180
cctcaccatt aagtctacca caaccaaata cttgagcaat ctgcattctc cgttcccctt 672240
tggcatcaaa atcactaaaa gaaacgttaa ccttctcgaa ggtttattgc cattcaaact 672300
ttttccttag atcaaagttc cttaattcaa aatgattaaa atattaaaac aatattactt 672360
tgatatttta aaaacaaana cagagataaa tataattaat attatttaaa atataattaa 672420
tagaattaaa aattatttat ttctcaaaat agattatggc agctcctatc aaccaaccat 672480
cgacaacgac tcagataact caaactgggc agactacaac gacaacaacg gtaggatcat 672540
taggagagca ttctgttaca acaacaggat ctggggcagc agcacaaaca tctcagacag 672600
taactctaat tgcagatcac gaaatgcaag acattgcaag tcaagatgga tccgcggtaa 672660
gcttttctgc tgagcactct ttttctaccc tccctccaga gactggaagt gttggagcta 672720
cagcacaatc cgctcaatct gcggggctat tttcattatc aggtcgtaca caaagaagag 672780
attcggagat ttcttcctct tctgacggca gttcgatatc tagaactagc tcaaacgcat 672840
cttctggaga aacaagcaga gctgaaagta gtcctgatct aggcgacttg gatagcttat 672900
caggaagcga gcgcgctgaa ggagccgaag acctgaagga cctggaggct tacctgaaag 672960
tacgattcca cattatgatc ctaccgataa agcgtctatt ttgaacttct tgaaaaatcc 673020
tgcagttcag cagaaaatgc agaccaaaag gaggccactt tgtttatgta gatgaagccc 673080
agaagtagtt tcatttttgt ccgcaatggt gactggtcaa ctgctgagtc tataaaagtt 673140
tctaatgcaa aaaccaaaga aaatattact aagcctgcgg acttagaaat gtgcatcgct 673200
aaattctgtg tgggatatga aaccatccac tcggattgga cgggacgcgt aaaacctaca 673260
atggaagagc gctcgggagc cacaggaaat tacaatcatc tgatgctcag catgaaattt 673320
aaaactgctg tagtctacgg tccttggaat gctaaagaat ctagtagtgg atatacaccc 673380
tctgcatggc gtcgtggagc aaaagtagaa acaggtccga tttgggatga tgttgggggc 673440
ttgaaaggca ttaactggaa aacgacccca gctccagact tctcctttat aaatgaaact 673500
ccaggtggag gggctcactc gacgtctcat acaggtcctg gcactccagt aggagctact 673560
gtggttccta atgtgaatgt caacttggga ggcattaagg ttgatctggg tggcatcaat 673620
ttaggtggaa ttacaacgaa tgtcactaca gaagaaggtg gtggaaccaa cataacatct 673680
acgaaatcca catctactga tgataaagtc tcaataacat ctacaggatc tcaaagtacg 673740
atcgaagaag acactataca atttgacgat cctggtcagg gagaggatga taacgcaatt 673800
cccggcacaa acacacctcc tcctccaggt cctccgccaa atctaagcag ttctcgcttg 673860
ctgactattt cgaatgcgtc cttgaaccaa gtcttacaga atgtccgaca acatctgaat 673920
acggcttatg attcgaatgg taattcagtc tcagatctca atcaggattt aggccaggta 673980
gtaaaaaaca gtgaaaacgg agtgaacttc cctactgtga ttcttcctaa aactactggc 674040
gatacagatc catccggtca agcaaccgga ggagtcactg aaggcggcgg tcatatccgt 674100
aatattatcc aaaggaatac acaatctacg gggcaaagtg aaggagcaac acctacacct 674160
caacctacta tagcaaagat agtgacttcc ctgagaaaag caaatgtaag ttccagctct 674220
gtgctaccac aaccacaagt agctacgacg atcacccctc aagcgagaac ggccagtaca 674280
tctacaacga gcataggaac cgggacagaa agcacatcta caacaagtac gggaacggga 674340
acaggaagtg tctccacaca aagtactggc gtagggacac caactacgac gactcgatct 674400
acaggaactt cggcgacaac cacaacatca tcagcttcga cacaaacacc ccaagcgcct 674460
cttccctctg ggaccaggca tgttgctaca atctccttag tgcgtaatgc tgcaggaagg 674520
tctattgtat tacaacaagg gggtcgatct caaagcttcc cgatccctcc ctcagggact 674580
ggaacacaga atatgggggc acaattgtgg gctgcagcaa gtcaagttgc ttccacttta 674640
ggccaggtcg tgaatcaagc agctacagca ggttctcaac cctcctctcg tagatcttcc 674700
ccaacaagtc cacgaagaaa atagctcttc gtaatctagt tgagaaaggc aagtccgtag 674760
aagtgaatag ctaaaatacg aggtcctgaa acttaggaca tgttgtggta gacgtcatcc 674820
acatcttcga tttgctctag ccaatcaata agagctaagt ttgcctcgcc atctttctca 674880
tcacaatcta ctagacgcaa gggaagataa atcaacctgt cttcactaca agtcgcaccc 674940
tgactgataa gtttctcttt aacggaggca agttcacttg gagcacagat cactaagaag 675000
ttttcttcat cttcggtatc gagatcctca gctcctgctt ctatagcata agaaaatatt 675060
acttcttcat ctatagagct cttagcgaca gtacacgccc ctttccttgc aaaattataa 675120

agtacgcttc caggttctac aagagaacct ccacgtttat ttatagcaat gcgcatatca 675180
gaagccgtac ggttcttgtt atccgtcatt gcttccacaa taattcccac tcccccatga 675240
ccatacagct cataggtaac ctcttcaaag ttcttttgct ctgcagaagt tgctttcttc 675300
aaattccttt cgatattctc attagggata ttattttcct tagctttctg tatcaccata 675360
cgtaatcgcg cattggactt agggtcagct ccccctaatt taacagctga aatcaactct 675420
ttaataatac gagaaaaaat cttgcctttt ttatgatctg ctctttcctt gcgatgtttc 675480
gtattggccc acttactatg ccctgccata tcctccacct actctttttt tataccctcg 675540
caataaataa gcgtttgcct tttcccaagc aacagcacga tcatacaagg ggaaacgttg 675600
ttcgtattct ttaaactttt taccatgaaa aatcgaacgt cccgatagag aatactctct 675660
agggactaca ctatgaacca tttcatgata cacaagatat tccataaaaa atcttgggat 675720
ttcctgccga tctaaagaac gatgaattcg aattaactgt tcattttcat gaaacaatcc 675780
caagacaaca ctcttgcctt ttctggtagc tttccttccg aaccaaccaa tctgtaaacg 675840
cagtgcacct tgaaataact gcgcattcaa ttcctcatag atctcctgca aatcatagat 675900
ctttcccgga cagtagtcta ctgggaatga aggacgagca agaaccctgt gcgaagatag 675960
gaacgtcttt tgacgacatg ggaagcgaaa taccctatag agatacttgc tcagataacg 676020
ttggaaatta tgaagtactg tttcaaccat aacgatagga tgtctaaaac ctctttctgc 676080
gctctataga tcccttttcc atagtagttt tagcaagata gccgatttca tctttataaa 676140
aacggttttg ccttcctact tcgacaaatc caaaacgttg gtagagatgc agagcaggat 676200
tgccctcata gacttcaaga tagaggacct caagcttaaa tcgcgtcttc gccaaatgaa 676260
taagattgtt tagcaaggcg gtccctatgc ctttattacg aaactcttct ccaacaataa 676320
tggaaatcag cgcatgatgg gaaaccttaa cataagggtt aagaaccaaa gttgccactc 676380
cagcaacatt cccattgtac acagctgtta agctagaatg ataacgatag aatcctaccc 676440
agaaatttac agtctcacga atttctgctt ccgtttggat gggaaatcca cgtaaaattt 676500
taggatcatt cagccatttt agcatatacg ttgcatcgct aggaagagtg taccgtattt 676560
ctaatcctag aattcctgta ttttgctttt ctgctgtcat gaaacnnctc caaactctgc 676620
caaatatgct ttaataaatt catccaatag ctcgccgtcg agcatcgctt ggacatttcc 676680
tgtttcatgt cccgtacgta catctttaac aagagtatag ggctgaaata cgtagttgcg 676740
aatttgagat ccccaagcaa tttccttttt atctttgcga tcaagagatt gcttctctaa 676800
acgttcttgt aaaacctgct gatacaactt tgcttgtagc attttcatac agctctcacg 676860
attctgtatc tgactacgtt cattttgaca tgaaacaacg actccagaag gtaggtgcgt 676920
gatcctgact gcggattccg taacgttgac gtgttgtcct cctgctcccg aagaacgaaa 676980
cgtatctata cgtaaatcat taggtcgtat ctcatcttaa tctntcatca atctcaggga 677040
agacgtctac agaagcaaag ctagtgtgac gtttcccatt actatcgaaa ggtgagatac 677100
gcaccaaacg atgtactcct cgctctgcct tggcataccc ataagcatac attcctgaaa 677160
actttacagt aacatgctta attccaacaa cttcaccatc taagcgatcg acaacctcta 677220
aggcccattg atgtttcgtc gcccatcggg aatacatacg aaacagcatc tctacccaat 677280
cacacgactc cgtcccacct gccccagcat tgatcgtaag gaaacaagag ttcttgtctg 677340
cctctccgga aagcaaccgt tgcgtctccc aaacagcaag tttcttctca caaaagagaa 677400
attctttctc taagtcttca caaatcgcag ggtcttcaag agcatcggca tcctcgagga 677460
aaaactctat agcatctatt ttgcttttta attcctgata ctcttggatt tgtcgtctca 677520
gacttacaat ctgttcagaa atttttccag catgaacact gtcttgccaa aaattttctt 677580
cggaactttc ttcttctaaa acttgaagtt ctttttgttt tttatcgagg tcaaagagac 677640
ctcgcagcta aagatatttc cgtgcgaagt gcttccaaac gcttgtctaa attttcctgc 677700
attactctta ccaacctacg attccaatca agattctaga aaacaaaagc caataaagtc 677760
aattagagca aggacttcct agagcttcta tgacttaaac aaaaaccaag actttttctt 677820
ctttttggag agaaagttct ttgttatatc ttttctaaag actccccttg acgctttcta 677880
aatagaaaag gtaaagagaa ttccactttt ttgttttgac gagaaacctc tctgagagat 677940
aaaaaagttg ggatgaagag ctcaggtctc ttcttaccac ctttactagg agtcaccaat 678000
gagtcaaaaa aataaaaact ctgcttttat gcatcccgtg aatatttcca cagatttagc 678060
agttatagtt ggcaagggac ctatgcccag aaccgaaatt gtaaagaaag tttgggaata 678120
cattaaaaaa cacaactgtc aggatcaaaa aaataaacgt aatatccttc ccgatgcgaa 678180
tcttgccaaa gtctttggct ctagtgatcc tatcgacatg ttccaaatga ccaaagccct 678240
ttccaaacat attgtaaaat aaggaatttg ttcgctgttg acttaggctt aagaaagcat 678300
gagctctcat tctgatatgt taagtaccct gccctttagg gcggggatat aaagtgatct 678360
ttaatttaaa atggagtttt ttctattaag aaaagaggaa tacttcaaaa gcttagtatc 678420
acctataata ggaaaattct tgggccccat aaatagtctc aagtttctcg tttttaaaga 678480
ccggaaatta gacaacaaat agttttatag tgcttatctc tatttcttta gccacccttc 678540
ctattcttgc cttttcttgg gcttccttca ttgaaccgaa ttggttaaga acaactgcta 678600
ttccatggag gcttccaaaa aaacatgcgc atttgcatgg tcttcgcatc gctcagattt 678660
cggatctcca tttccataag agagttcctg agaaatttct taataaagtt tccaaatcaa 678720
taaaaaattt ctctcccgat cttattgtat tttgtggtga cctcctttgc cgtgctcgac 678780
ttgaagataa ggaacgactt gaaaccttcc taaatacatt agaagctcct ctaggagtct 678840
ttgctattct aggcaatcac gactattctt cgtatatttc cagaaacact aaaggagaga 678900
ttacctgtat ccctgaggaa aaaagtcgtc ctatacaacg cgccatcatt gctgtaatgc 678960

aagggctatt ctcctctcct agctatcgct atgatcccaa tctgactccc caagagcccc 679020
acccagacct cttaaaactt ctgaagaata ctcccctaac tctccttcac aataccacgc 679080
atgtcattcc taacactctt aatattgtag gacttgggga tctgttcgct agacaattcc 679140
atcctgaaca ggcattcaaa aactatgatc cttctctccc aggccttctc ctttctcata 679200
atcctgatgg cataactagg ctgcaacaat accctggaga ttttgtactt tcaggacatt 679260
cccacggtcc acaagttact ttgtcctggc cgaagtttgc tcgaaaattc tttgaaaggc 679320
tgtcaggatt agaaaatccc tatcttgcac gcgggtattt cgttactaag gaaggaaaac 679380
aactctacgt aaaccgcggt ctcggcggac taaaaagaat tcgcttctgc tcccctcctg 679440
aaatctgcta catcacatgt tcctatgatt aagtcttctc taatacttct tagtggagga 679500
caaggtacac gttttggatc taaaattcct aagcagtacc tccctctaaa tggaactccc 679560
ttagttcttc actcattaaa gatactctct tctttgccac aaattgctga ggtgattgtt 679620
gtttgcgacc cctcatatca agaaaccttt caagaatatc ctgtctcttt tgccattcct 679680
ggagagcgtc gccaagattc tgtcttttca ggactacagc aagtctccta tccctgggta 679740
atcatccacg atggagcacg tccttttatc tatcccgacg aaattcatga tttattagaa 679800
acagcagaaa agatcggggc gacagctcta gcgtctccga ttccctatac cataaaacaa 679860
cgcaatcctg tgcgcactct ggaccgagac aatttagcaa taattcatac ccctcagtgt 679920
ataaaaacgg aaatcctcag agagggtcta gctcttgcaa aagaaaaaca gctcacactg 679980
gtagacgaca tcgaagctgc tgaaatcata ggcaaaccct cgcaactcgt tttcaataag 680040
catcctcaaa tcaaaatttc ctaccccgaa gatctaacga ttgcccaagc cctcctatga 680100
ctaaagtagc tcttcttatt gcttatcaag gaactgccta ttcaggctgg caacaacaac 680160
cgaatgacct atcgattcag gaggttattg aaagttccct aaagaaaatt actaaaactc 680220
gcactccact aattgcctct gggagaaccg acgcaggcgt ccatgcctac gggcaagtgg 680280
cgcatttccg agctcctgat caccctctat ttgcaaacgc gaaccttaca aaaaaagccc 680340
tcaatgcgat tctccctaaa gatattgtaa tcagagatgt tgctttgttt gatgataatt 680400
tccatgcacg ctatcttacc attgctaaag aatatcgtta ttccctatca agacttgcca 680460
aacctcttcc ctggcagcgc catttctgtt atacccctcg ccaccctttt tctacagagc 680520
tcatgcagga aggtgcgaac ctgcttatag gaactcatga ctttgcctct tttgcaaatc 680580
atggcaggga ctataactct acagtacgaa cgatctatac cctggatatt gtagataaag 680640
gagattctct ctccataata tgcagaggaa atggcttcct ttataagatg gtacggaatc 680700
ttgtaggagc ccttttagat gtggggaaag gagcgtatcc acctgaacat ctcctagata 680760
tcttagaaca gaaaaatcgt agagaaggac cttcggcggc tcctgcctac ggcctttctt 680820
tacaccacgt atgctattcc tctccctaca ataacttctg ttgtgagcaa tgctctgtta 680880
gcacgtcaaa cgaaggataa gagaaaaatt ctttgccttt aagttcaggg taatcttcag 680940
gggtgatctc cgccatcgaa ttgatacata ctaaagttgc tggaatcttg gagagcgccc 681000
gaagcccctt cacagaatct tcaaatccta taactttcat tccttcacga gcaaacgtcc 681060
gatacgcgta gtcataacta tctccgtaag gcttagggcg tgcataattt tctcgggtga 681120
cccagaacaa aaatttattt aaaattggat acatagtacg tagtgtgtgc gtggcatctc 681180
ttggagagtt agttacaact ccaaatgttt tattcaaaga caagacaagc tcgatgaaag 681240
cttcgactcc aggcatcaga gcaggacctg cgtgttctaa ggacttgtag taaatctgca 681300
gtcgttttgc aaagatctcc gccatgtact cttgtgcttg gggatattgt tctataaact 681360
ttttgctaaa aatttctgtt cctaaggtag tatgactata ataggtagaa aaatcccaat 681420
gcacttctaa agaaaattca gcacaggctt gtaaaaatgc acgataaaaa caaggttctg 681480
tatctacaag caaaccatct aaatcaaaaa agaaaacgtc ataatcctct aaatacatac 681540
cactcctcaa acacaactga aactcgtcgc ttaccaacat actataaatc caacattgtc 681600
ttaaaaatga ttttacggat ctccaccgta agccttctta caagttgctc cttctcgaaa 681660
aattctcgta cctgtttcgt cactccagaa cgcattacct cacaaaaaga ctgccccgtc 681720
cttctccatc caaaaagcac tacgatttct ccccctctct atgactggat ctccccaaat 681780
agagaggtaa tcaccgccta ttctttctac tgccgaggtc aaggaaactc tatcataact 681840
cccgaagggg ttctctatga ttgtgatgga ctccatcaca gcataactaa agaagagttc 681900
cgttatatcc atcctagatt gattgaggta gtacgactct tgcaacaaga tcaccctaaa 681960
gtctctatta ttgaagcctt ttgttgtcca aaacactttc attttttaga agcctcagga 682020
atctcactct ctcaactcca tctccaaggt actgcagcta ccttcgctct agatcctccc 682080
ctccccatgg agaaactctt ggcaactata aagaaactgt ataaaaaaaa ctccgatcct 682140
tctctctcta attttatcgt tacagaagct acactgacca atccagaact gcgactcacg 682200
caacaagatc tcggctcgca tacagaaatt actgtagaaa ttctcgataa tctacaaaac 682260
aaagaggctc tttcctccgc ataagagatt tctcttgcga taattagaac agaaaccgta 682320
cattcgtggc tcactttatg ccggtgtggc ggaatggtag acgcggtaga ctcaaaatct 682380
actcttagca ataaggtgtt ggttcgagtc cgatcaccgg cataattctt tcttttttca 682440
ggttgccaat aaattgtttt gtcgtttttc ttagggaaaa ccaaagtaac accccgattt 682500
cttatgaatg aaagaaccct cttgctcttg ttaaaaaaga agaagggcct tttccttgct 682560
attttagatc ttacgcaaac agaatcctct ctaacgactc cagaattaga gaaagtctta 682620
aagcaaaaaa aaatctttct ttcttgcatc gatagagttg atcttcaaat caaagagttt 682680
cgccatgcct tctcttccga acttccccaa gatatccaag aagagctgga agaaatccgt 682740
gatgttatta ttcgtattct agatacggat aaacgcaact atgcacagaa aaaaaaggaa 682800

tttggtattt atgaacgtcc ctgattccaa gaacctccat cctcctgcat acgaactcct 682860
agagatcaag gctcgcatca cacaatctta taaagaagcg agtgctatac tgacagcgat 682920
tcctgatggt atcctattac tttctgaaac aggacacttt cttatctgca attcacaagc 682980
acgtgaaatt ctaggaattg atgaaaatct agaaattctt aatagatcct ttaccgatgt 683040
tctccccgat acgtgtcttg gattttctat tcaagaggct cttgaatctc taaaagtccc 683100
taaaactctt agactctctc tctgtaaaga atctaaagaa aaagaagtgg aactcttcat 683160
ccgtaaaaac gagatcagtg gatacctgtt tatccaaatc cgcgatcggt ccgactataa 683220
acaactagaa aacgctatag aaagatataa aaatatcgca gaacttggga aaatgacggc 683280
taccctagct cacgaaatcc gcaatccgct aagtggaatc gttggatttg cctctatcct 683340
aaagaaagag atttcctctc ctcgccacca acgaatgctc tcctcaatca tctccggcac 683400
aaggtctcta aataaccttg tctcttctat gttagaatat acaaaatcac aaccgttgaa 683460
cctaaagatt ataaatttac aagacttctt ctcttctctt atccctctgc tctccgtctc 683520
tttcccgaat tgcaagtttg taagagaggg cgcacaacct ctattcagat ctatagatcc 683580
tgatcggatg aacagtgtcg tttggaacct agtgaaaaat gctgtagaaa cagggaactc 683640
tccgatcact ctgaccctgc atacatcggg agacatctcg gtaacgaacc ccggaacgat 683700
tccttccgag atcatggaca agctcttcac tccattcttc acaacaaaga gagagggaaa 683760
tggtttggga cttgctgaag ctcaaaaaat tataagactc catggaggag atatccaatt 683820
aaaaacaagc gactccgccg ttagcttctt cataatcatc cccgaacttc tagcggccct 683880
acccaaagaa agagccgcta gctagaacgc gttcttgaat cttcaagacc acttagggtt 683940
ctcaaagatc atctacgatg ttttcttatc cttgaaattg ctcttgctct agtctttcaa 684000
cctccgctct aagctgtgca ttttcttctt gcaatctttg aagctcgacc tctagctcaa 684060
atctcattcc agcatcttct cgaacaggag agagaaggct ctcaaggtca cagcattttc 684120
tctgagccac ttcgcaatct ttgatcgctt gtgcatgcat cttatcaaga gctcgtaatc 684180
cttctttctg ctcttctatg tgttctcgaa gcttgctgat ttctttttca gcttctacag 684240
catcggcaac cagttgagtc aattcgttag atttaaatgc aagtgctttc aggagttctt 684300
gattctcttc cacgagttcg cggatcttct tctggctgag agaagcagaa tctgtgagta 684360
cccagtcatc gtcctcaccg atctcagtag gagcagcggc accctctaaa tccgctaact 684420
tcttcttgta ggccatgttt tccttttgag ccttctcaaa gagatcggca aaatgattca 684480
tactttcttc taagattttt tcttttacct gtttctcctc ttgaagacgg ctataccttt 684540
gttgtagctc tttggaaatc tttctcaact ttttctgcct cacgcagact gtcctggtac 684600
tcaagaatga atgtattcag acgtctaatt tcttgggttt gttcgtagtc ttcctgttgc 684660
tgcttcccta attcttcttc ccaaacagct gctgctttct tcagattagc atctttttgt 684720
tcgagctgct tctcatagtc atggaccaga ctctctaagc aagccttctc acttttctct 684780
gcgttgactt cctgtaaaat cttttgcata tctagtttct gactcagata tttctcttta 684840
tattcgcgat agaacagaga ttgttcgtca aaagcaatcc cagtaagtct cagatcctct 684900
tgtaatcttt ctaatttggc attctgctta cgccaagcct tctcttgttg atggattgtg 684960
cccctcatct caatccactc ctgcatagat ttcagctgtc tccttaaatc cttctccagt 685020
tgctggcgtt ctgcacaatc catctctgtg ccctcacagg cacgctgcga agcctcgcga 685080
tcatgagccc gttgtatatc cttctgagat tggttaaata cgttgtaaag ccgtccagca 685140
tctcttctgc tcgtcatcga tccgactcct taaatcttgc accgtctgct gctgcaaact 685200
gattagatct tgtagacgag aaatctcttt ttccccctcc tctgatctaa ctgtcataat 685260
tccttgaaca aatcgctcat aacttctacg gttaaattct agccgatcgg caaccagact 685320
acgcatttcc aagagctcca gcaagaactc atacctttcc tcgtctttaa gatgggccgt 685380
cgagcgatac agaatctcct cttcacgtcg cagcttacga tccaacattc caagacgctt 685440
ttcacagtct acaaccacct ggggcaccgc agggtcctta gcgtctctat cctttaagag 685500
aacctctaaa agagtccctt caagaacttc tctggtctcg tttcttaaat cttcaatctg 685560
aatttgttgt ggttcaggaa ccggagggag ttcctcagga aggccctgag aatcataaag 685620
acataaaatc aaagccaaag caaatataac ggctcccaaa gcaatcagac ctgttccaat 685680
aaacatagga actgctggca gcaatcctac gagtaatcct ccccctaaaa tagtaagaag 685740
agcaagaata ataagactta ccttaacaat tgtatgattc catccacact gctggactcg 685800
agctattgcc atctcccctc tttcctcaag cgttagcggg gaaaagacag ggtggttact 685860
gcctagctct cttacaaaac taggatcttg aaatgtaggg gatttttgag cgggtgttgc 685920
catgttgcta ctacaatctc caattgaaaa attatttggc ctaaggattt tacgtcttaa 685980
tgatatttaa atacaacgat taaatcgtat ttcttatttt ataaaaagta tttgataata 686040
ttttttaaat tctttctatt aaattgaaag ttttgcttca tttttcaaga tttagtaaaa 686100
agaaaaaatg aatccatcca ggggagagaa catggcgatt aaaaatatac ttgttgttga 686160
tgacgagccc ctactcagag atttcctctc ggaacttctt acctcacagg gattcatccc 686220
agacactgct gaaaacttaa gaaatgctct ccaaatgatc cgaagtcgag actatgacct 686280
tgtcatctca gacatgagta tcctgacggc tctggtcttg atttaatcaa aattataaag 686340
caaagctccc cccacacgcc cgtccttgta gtcactgctt acggaagcat agagaacgcc 686400
gtagaggcta tgcaccaagg ggcattcaac tacttaacaa aacctttttc ttctgaagca 686460
ctttttgcct ttatctctaa agctgaagaa cttaagaacc tagtccatga gaatctcttt 686520
ctacattctc agacaacacc agattcacac cctctgattg cagaaagcaa ggctatgaaa 686580
gatcttcttg ccatagcaaa aaaagcagct tcaagctcag caaatatatt cattcacgga 686640

gaatcgggat gcggaaagga agtcctctcc ttttttatcc accacaactc tcctcgagcc 686700
aaccacccct atattaaagt taactgcgca gcaattcctg aaactctctt agaatcagaa 686760
ctttttggcc atgaaaaggg agcatttaca ggagcaacta caaagaaggc aggacgtttt 686820
gaacttgccc ataaaggaac cctcttatta gatgaaatca ccgaagtccc agtaaacctt 686880
caagcaaaac tcctgagagc tatccaagaa aaagaaatcg aacaccttgg aggaaccaag 686940
accctctccg tagatgttcg catcttagcg acctcaaacc gaaagcttaa agaagctatc 687000
gatgataaaa gcttccgaca agatctgtat taccggttga atgtcatccc tctacacctc 687060
ccccctctaa gagaccgaca ggacgacatc ctccctctgg cgaactactt cctaaataag 687120
ttctgccgca tgaacaatac tcctctgaaa accctctctc ctaaagctca agagctcctc 687180
cttaactacc cctggccagg caatattcga gagctctcca atgttctgga acgtgtggtt 687240
atcctagaga acacctccct actcaccgaa gacatgctcg ctttagcttg atctcctcta 687300
ggggtttttc ttgtttttct caatagcatc tggtaagctg atttgcttta atctctgtat 687360
tcttttaagc accgatagct caattggata gagtacctgg cttcggacca ggtggttgga 687420
ggttcgagcc ctcttcggtg cgttaaattc tttttttgaa taggtatttc tccttataat 687480
agagctatgg agaactattt attttatcta ctctcctaaa gcatcacgta accctaggag 687540
acaagatgag acctcatcgt aaacacgtat catctaaaag cttagcttta aagcaatctg 687600
catcaactca tgtagagatc acaacaaaag cctttcgtct ctctatgcct ctaaaacagc 687660
tgatcctaga gaaaagcgac cacctccccc ctatggaaac aatccgtgtg gtgctaacct 687720
ctcataaaga taagctaggc accgaggtgc atgttgtagc ttctcatggc aaagaaatcc 687780
ttcaaactaa ggttcataac gcaaacccat acactgcagt gatcaatgct tttaagaaaa 687840
tccgcaccat ggcaaataag cactccaata aacgtaaaga caggacaaaa catgatctag 687900
gtcttgcagc aaaagaagaa cgtatcgcaa tacaggaaga acaagaagat cgccttagca 687960
accgagtggc ttcctgtcga aggcctcgat gcctgggatt ctctaaaaac tcttgggtat 688020
gttcccgcat cagcgaaaaa gaagatctcc aagaaaaaga tgagcattcg tatgctatct 688080
caagacgagg ctatccgcca gctagagtct gccgcagaaa acttcctgat cttcttgaac 688140
gagcaagagc ataaaatcca atgcatttat aaaaaacatg acggcaacta tgtccttatt 688200
gaaccttccc tcaagccagg attctgcatc tgaggactcc acatcgcaat ctcaaatctt 688260
cgatcccatt agaaatcggg agttagtttc tactcccgaa gaaaaagtcc gccaaaggtt 688320
gctctccttc ctaatgcata agctgaacta ccctaagaaa ctcatcatca tagaaaaaga 688380
actcaaaact ctttttcctc tgcttatgcg taaaggaacc ctaatcccaa aacgccgccc 688440
agatattctc atcatcactc cccccacata cacagacgca cagggaaaca ctcacaacct 688500
aggcgaccca aaacccctgc tacttatcga atgtaaggcc ttagccgtaa accaaaatgc 688560
actcaaacaa ctccttagct ataactactc tatcggagcc acctgcattg ctatggcagg 688620
gaaacactct caagtgtcag ctctcttcaa tccaaaaaca caaactcttg atttttatcc 688680
tggcctccca gagtattccc aactcctaaa ctactttatt tctttaaact tatagctaat 688740
catccatgca gatctgtgtt accggcgttg tacttcgcag ccgcccctta ggaaaaaatc 688800
atacactcac cactttattt acccctgaag gactctttac cttttttgca aagcaaggac 688860
aaaccctcca atgtgattat cgagaaaccc ttgtccccat atctttgggg aagtatacgt 688920
tacatcgtaa tggctcacgc cttcctaaac tgacccacgg ggatatcctc aatgccttcg 688980
aagcaatcaa acaaacctac gctctcctag aagctagtgg aaaaatgatt caagctcttc 689040
tggcttctca gtggaaagaa aagccttcgc ataagctctt ctctttattc ttgaatttcc 689100
tccaccgtat tcctgaaagc agcaatccag aattttttgc agccatcttt gtacttaaac 689160
ttctccaata cgaaggaatc ctagacctga ctccagcatg ttcgctatgc aaagcatctc 689220
taccctatgc ctgctatcgc taccaaggcc ataaactatg taagaaacat cagcataaac 689280
aagccatctc catcgagaaa gaagaagaac aaatcttaca ggctatcatt catgcgaaga 689340
agttttctga acttctagct attgcagaat tcccgattgc tatagctgaa aaaatttttt 689400
atttgtttga ctcgctacaa gaggaaaaaa aatcagaaag aaattcttcg gaagatccat 689460
atcatgaaat cctaagactt tctaaagtag tccatcccta ctgatgaagg acgtgtaggc 689520
ccctagaaat tataattatc aaaggtaccg ctgcaccaaa cctttgaaaa aaggtgtgtt 689580
ttcctagaat tcaagaaaaa ttgagagaaa taaactccct aagagaaggt aattgtaccg 689640
ccctcactag atacgttaaa tattaacgtc acctcagaga atccaactaa atcattgtgg 689700
tatattttat tccatacacc ccaaccctgt tttataaacg tactcccagg acaaacgttt 689760
ccttttaaag aagtttttgt atttacaatg acgccaacat ctttaggcag ttttaaattg 689820
atagattctt tctgattaga aatggtaatg gtactacttc tattccattt gccacggaaa 689880
tccatatcca tcttgcaaga actacaagaa aaatttagag catagagaac aggatagact 689940
cccctacact ttgcacggag atgaccgaaa gaaccttgat aatttaaagt ctctaattta 690000
gggaactctc cagtcagatt gaattcgacc cgtttttttc tctgcgcctg gatttccata 690060
ttctccatat cagggtagct tttcgaaaaa tcaaaacggt gggtgtaaaa tgattcttct 690120
tcagaaagcc aacaagggac agaaagcagc tgattcccaa aggaacccga agcaaacaaa 690180
acaatgaaaa aaaataaata tcgacaagcg aatcccaaat tatgatctcc atacaaaaat 690240
agtcaaaaat aaataataaa ggagggggac atcacgaagc tattgcgaaa gaggggactc 690300
gaacccctaa ggaaagctcc actaccacct caagatagcg cgtataccaa ttccgccact 690360
tccgcaagaa gaaactagct taccgaggct gtacccttta cctcaagaaa aaaagtattt 690420
ccctaccacg aaaaatcagg tacattgtcc taatagctag aaggagtaaa gcctaattat 690480

gcgttgcact gcctactgta cagcatctgc ttataacctc catgtccttt tccacctact 690540
taaaccccgc tatcctacca tcttatctag agaatatgtt ctcgcaaacc tagatagtac 690600
acaggcaagc aaccagttag cgatcttctt ccctttcgga gttgccgtat tctggggttg 690660
ggaagagtcc gaagaaatca aacttttaca aacaatcgtt acagcatcac cagaaattct 690720
tccccaaccc gagatcgatt gctataactt tcattacgga gataaactcc aaatccggag 690780
ggaccgcctg acccttgccg acaccacatt aaatacaaag ctcgccattg cttttggtct 690840
ggcgcaatcg gtaaaactca caaccttcga aactacaatc tataaaacta tagaagattc 690900
taaacggctt ccccaagatc ttgctactaa aggaaaaatt tccatgtctc ggaaagccat 690960
cgcaaaaaag attggcaagc tcttcctaga taaggcttca gtaaaccttc attccgatat 691020
cttggatgaa cctgattttt tctgggatca tccagaaaca caggcgattt atcgtgacgt 691080
tctcagttgt ttagatattg aggcacgaat caatgttctt atcgtttgac tattcttgga 691140
gatgtgttag aaattcttaa cgaccaactc aatcaccaac actcttcatc tctagagtgg 691200
acaatcatat ggctgattat gttagaattt tctgtagctc tactcaaaga tgttttcaat 691260
gtcatttaaa cgtttcttgc aacagatccc tgtacgtatc tgtctactta ttatctatct 691320
ctaccaatgg cttatctccc ctctcttagg ctcgtgctgt agattttttc cttcctgttc 691380
gcactatgca gaacaagcct taaaatctca cggcttcctg atgggctgct ggctttctat 691440
aaagagaatc ggaaagtgtg gcccctggca tcctggaggc attgacatgg tccctaagac 691500
tgctttgcag gaagttttag aaccttacca ggaaatagac ggtggtgatt caagccattt 691560
ttctgaatga tcttatccat aggaatgcca aacctcttag ctatcaacca taaagaatcc 691620
ccgtcctgta ccacatactc ttcatcctga tcttcttgaa cacgaggagc ctctaccctc 691680
cgagtcgaca tgcaggccaa ctctctacgc ggagaatgct gtaatcgaga gaagaaattc 691740
tgactatagg gagactcttg aggcatcagg cggatgacct tctcaagatc ttcatcacaa 691800
aattcatgca aaacaacatc actatcatgg acaagcaaaa gcaaggctgc cagagattct 691860
tcacaatcta aataagattt taagaccttc tgacgttgtg tagctgaaat catggaagtg 691920
cggctctctt cattgcaaaa atgaaagaaa cgttcggatc cgcaacgaat caccatacga 691980
gctaatgagg ctactgaaga ggcctgcacg tctgcaccta caagtaacgt acgcaagtaa 692040
agaaattctg gagtcgagca gaaatgatac aggcaatctt catctaccca gccttcttgt 692100
accatctttt ctatcagcaa aaacaagccc ttagaagtat agggatactt gtggcaacgc 692160
aaatagtcca aaatcacagg aaagtcttta agatcaatat tcggcaaaag ccaccgcact 692220
gaagatcctt tcaactctgt ataggtcaaa ggcttcgaaa gcacaggagt gatgtctatg 692280
tggtgggaag ctatcgctac actcaacgcc caaagtttta tcggccgacc atacatatag 692340
cgctcatctt tgaacaacga aatcaagtcg tccaaagatg cttgagatat ctcgtttaaa 692400
aaatcttcag aaaggtagac cttacgacta cttttcgcaa tcaaaggtcc ggaaaataaa 692460
tgcagcttat agatgtcttt acgaaatatg gcagagtaaa ataagagcaa aaaaagcata 692520
ttcaatccca cactcaggat caagacttgc cacagccatc tagtttttct tttgaaagcc 692580
atacattttg cgcctaaaac tcagttcatt ctctaggatt aaaacacttg ccactataca 692640
caggtcatcg taaaatccct cgtaaaaaca tccttgcgtt tatcatccat gcggattcca 692700
ataactctac tgcaancata cttttcagaa cctctttcga caaaggaaat tttagaagcc 692760
tgtgatcata ttggcataga anncgagatt gaaaatacta ccctatactc tttcgcttct 692820
gtgattacag caaaaatttt acatacgatt ccccatccta atgcggataa actccgggta 692880
gctaccctga ccgacgggga aaaagagcac caagtggttt gcggagcccc caactgcgaa 692940
gcaggattga ttgtagctct tgctctacct ggagccaaat tatttgatag cgaaggacaa 693000
gcctacacaa tcaaaaaatc taaacttcgt ggtgtagaat ctcaagggat gtgctgcgga 693060
gccgacgagt tgggccttga tgaactccaa attcaggaga gagctctttt agagctccca 693120
gaagccaccc ctttaggtga agatctcgca acagttttag ggaatacttc tttagagatc 693180
tctctaacac cgaatttagg ccactgcgcc tccttcctag gattggcccg agaaatctgc 693240
cacgtcactc aggcaaacct cgtcatccct aaggaattct cgttcgaaaa tctcccgact 693300
acagccctag acatgggcaa tgatcctgac atttgcccct tcttttctta tgtcgtcatt 693360
acgggaatct ctgcgcaacc ttcaccaatc aagcttcagg aatctctaca agccctcaaa 693420
caaaagccca taaatgctat tgtcgatatt acaaattaca tcatgctttc tctagggcaa 693480
cctctgcacg cttacgatgc gagtcacgtc gctttagact ctctgcgagt agaaaagctc 693540
tccaccccag aatctctcac cctattgaac ggagaaaccg tcctcttgcc ctcaggagtg 693600
cctgtagtcc gcgatgatca tagtctcttg ggtcttggag gtgttatggg agcgaaagca 693660
ccctcatttc aagaaaccac aaccactaca gtcatcaaag ctgcctattt cctccccgaa 693720
gctctccgtg cctctcaaaa acttctcccg attccatcgg aatctgccta tagattcacc 693780
cgggggatcg atccacaaaa tgttgtacca gcactacaag ctgcaattca ctatatttta 693840
gagatcttcc ccgaagctac aatctccccc atctatagtt ctggagaaat ttgtcgtgaa 693900
ttaaaagagg tcgctctacg ccctaaaacc ctacagagaa ttctagggaa atctttctca 693960
atagagatcc tctctcaaaa gttacagagc ttagggttct ctacgactcc acaagaaact 694020
tccttacttg taaaagtccc ttcctaccgc catgacatca atgaagaaat agatctagta 694080
gaagagatct gtaggacaga atcttggaat atagaaactc aaaatccagt atcctgctac 694140
actccaatct acaaactaaa acgtgaaact gctgggttcc tagcaaacgc aggacttcaa 694200
gaattcttca ctcctgacct gctagatccc gaaacagtgg ctctaacaag aaaagaaaaa 694260
gaagaaatct ctcttcaggg ctccaaacat accactgtat tgagatcctc actgcttcca 694320

ggattattaa aaagtgctgc gacaaaccta aatcgccagg caccctctgt tcaagctttt 694380
gagatcggca ctgtctatgc aaaacatgga gagcagtgtc aagaaactca aactctggcg 694440
atcctgctca ctgaagatgg cgaatccagg tcctggctcc ccaaaccctc tctttctttt 694500
tattctttaa aggggtgggt agagaggctg ctctatcacc accatctttc tatagatgct 694560
ttgaccttag agtccagcgc gctctgcgaa tttcacccct accaacaggg agtgttgcgc 694620
atccacaaac agagttttgc tactttaggt caggtacatc ctgagttagc aaaaaaagca 694680
cagataaaac accctgtgtt ctttgcagaa ctcaacttag accttctatg caagatgcta 694740
aaaaaaacaa cgaagcttta taaaccttac gccatatatc cttcatcttt tcgtgatctc 694800
accttgacag tacctgaaga catccctgca aatttactga gacaaaaact tttacacgaa 694860
ggttctaaat ggcttgaaag tgtaaccatt atcagtatat atcaagataa aagcttggaa 694920
acacgaaata aaaatgtttc tctacgcctc gtattccaag attatgagcg aacattatct 694980
aaccaagaca ttgaagaaga atactgtcgt ttggtagctt tacttaacga attgctaaca 695040
gacactaaag ggactatcaa ttcatgaaac aattactttt ctgtgtttgc gtatttgcta 695100
tgtcatgttc tgcttacgca tccccacgac gacaagatcc ttctgttatg aaggaaacat 695160
tccgaaataa ttatggcatt attgtttccg gtcaagaatg ggtaaagcgt ggttctgacg 695220
gcaccatcac caaagtactc aaaaatggag ctaccctgca tgaagtttat tctggaggcc 695280
tccttcatgg ggaaattacc ttaacgtttc cccataccac agcattggac gttgttcaaa 695340
tctatgatca aggtagactc gtttctcgca aaaccttttt tgtgaacggt cttccatctc 695400
aagaagagct gttcaatgaa gatggcacgt ttgtcctcac acgatggccg gacaacaacg 695460
acagtgatac catcacaaag ccttacttca tagaaacgac atatcaaggg catgtcatag 695520
aaggaagtta tacttccttt aatgggaaat actcctcatc catccacaat ggagagggag 695580
ttcgttctgt gttctcctcc aataacatcc ttctttctga agagaccttc aatgaaggtg 695640
tcatggtgaa atataccaca ttctatccga atcgcgatcc cgaatcgatt actcattatc 695700
aaaatggaca gcctcacggc ttacggctaa catatctaca aggtggcatc cccaatacga 695760
tagaggagtg gcgttatggc tttcaagacg gaacgaccat cgtatttaaa aatggttgta 695820
agacatctga gatcgcttat gttaagggag tgaaagaagg tttagaactg cgctacaatg 695880
aacaggaaat tgtagctgaa gaagtttctt ggcgtaatga ttttctgcat ggagaacgta 695940
agatctatgc tggaggaatc caaaagcatg aatggtatta ccgcgggaga tctgtatcta 696000
aagccaaatt cgagcggcta aatgctgcag gatagtttgc ggtaatggct gatgacaccc 696060
tcattcctaa acttatgaag aactcgcttt cgcaggcgtg ttctgagggt ttactgattg 696120
ctaagtatcc tccactccag gttatcgttc actttgataa taacctagtt gttaaaacac 696180
atctttcagt agctcctgtc ttctcttgtc tttttttagg accagcagct cacaaagcca 696240
tgcaggaaat tgttttatgg tgttctcgct atgccaacaa ggaacatcct cctttttcct 696300
cgcattttgc taaagacctc atcccctcac aatatctcga aatcctaaac tgcgttgcag 696360
agattccctt tggagagcag caaacctacg ctgaaatcgc aaaaaaaact gatacgcacc 696420
ccaggactgt aggagccgca tgcaaacaaa atccgtttct gctgttcttt ccctgtcatc 696480
gcgtcgtagg aagccatgga gagcgtaatt acgtcctagg gcctgtaatt cacgagatct 696540
tattgaaatt tgagaatagc tactaatccc cagctataga tttaagagtc ttgcagacgg 696600
ggatctaaag catcacggac tccgtctcct atcagagcga tcgcaatcag caacatcgtt 696660
aatataattg ctggaggcca aagaacagca ctctctgcag ggaatcctgt aacaccctcc 696720
ctcataagat ttccccaaga cgcggaactc tcttccccca gacctaaaaa ggtcagccct 696780
gcctcacagc taatcatagc catcatagca aacggaacta aagagatcac agggacaatg 696840
gcattgggaa ggatctgatg caccataata taatagtggc tataccctaa gtttgtagca 696900
gcaagaacat aacctcggtc tcgctgtttc aacacctcaa tacggacata cctactaaac 696960
cctgtccaac taaaacagcc tagcaaaact gtgttcaata gcaaagattt ctgctgtgtt 697020
atggaaatca ccagcattaa gataaacagc acaggcatgg tctcccaaat ttcagtaaac 697080
cgagataaaa tcatatccac ggtcccaccg aaatatccag aaaccaaccc gatcataatc 697140
ccgatagcta aagctatcgt aatcccaata cctgcgacta ccaaagctat gcgaatgcca 697200
aatactaaag ccgctagtaa atctttccga gtgactctgc taagctgcca ccaaggaaca 697260
tacttgttca tttctcgaga tcccccagca tcatcttccc aatggaaact actgaaaaag 697320
gggttaatca aaatgcgaag atcttcagac tctttctcga tccacaaacg cttatcttga 697380
atgaaactga tcgcgctgcg cagcttggca tagtcttcaa gtacctgacg aatctccaac 697440
aaagattttt tatagggctc tgctcgtttc tctacattag cataagcaat acaaaggtct 697500
tcaggttgcc cccagttgtt gtaatccgcg aggcggagct catgctctat tcttgtcaag 697560
gccatcagaa acggtcggta gttgtccgta gcacgattcc aagcttgctg cgccatctca 697620
tagggacgct gcatcttgtc gactctttgc tgtaatcttt taaggcaaat gccttcattt 697680
ttcatctcta agtgacgcaa tgttggcatc ggagactgcc gtttttcttc aaaagcgacc 697740
tgatacttct ttacagaagc ctcttgtttc tttcgatact ttgccttaat gagaatcccc 697800
aactgctcat acgtactcat ataccgccgt tccatctccc aagtacgtgt atctttgggg 697860
agcagcatga ccatctcaga attcacctta ctgatgtttt ctcggacctt ctcagctcgc 697920
atttttttta aattctcagt aacgcgggat cttgaacttt cccactatag gcccaagcaa 697980
aaatcataca ttgagaaatg atgcacagcc ctaacaacca acgacgtaac caccccctag 698040
tcaacttaaa agaaagtatg aaaaagggaa acgtgaccat caaaacgttg aaaaagagat 698100
ccactggttt agtgtaatac ccagggaaaa acaagtacct cagtaaagga aaaaagatct 698160

ctccatgcca ggtgactagt aaaggtttac tactcgcaaa taaaggagcg tagatgccaa 698220
tcagagcgac agaaataaaa aatttccatg ataaagaggc taataaattt ttatagtaag 698280
cagatagaaa acgttgataa aggaaggatg cttctgcatt ttatatcctc cttccctcta 698340
gctgaactcg aggatctaag agtacgtagc aaatatctcc gagcaaatat cccactaaag 698400
atagagccga tcctacaagc acagaaaata gaactacatt gtgatctcga tttaaaattg 698460
cctgatagaa gaagttccca aagccatcta tattgaatag ggtttctaca accaacgccc 698520
ctccaagtaa cgttcctaaa tgaagaggct agagaggtca ctatagagac tgcagcattt 698580
ttccctacgt gcttatatag aatatcaaac caacgcaatc ctcgagcttt agcagcacaa 698640
ataaaatctt ggcttaatac ttctaaaaat atcgaacgac ttaatcgtga ctgtgcggca 698700
agagctccgt aactcactgc acagaaaggt aaaaacccat gcgacaccaa gtcaaagata 698760
cgtcctagag tactgagctc attaaaaacc tctgggggcg aacgtaaccc agagtaaggc 698820
ataggaattg tggtaaatgg aatcgtttta ttaataacaa agttatctaa gatccaggga 698880
accgcaacaa agacaggaat agaaaataga ataaggaaaa tgaaatttag agagtgatct 698940
atccagcggt ttcttttcaa tgccatgatc ataccaaaga tttggcataa cacaaagcct 699000
acaatcatag gtaaaattga caagaccaaa gaacaacgta aacgcttgat cacttctgaa 699060
attacagtct tatgtgcgtc atttcgtaaa gttccaaaat ctaaccgcaa cacccgggac 699120
atatagcgag caaagcgagt ctctaagaaa aaagtcttcc agaactgctt agagctatag 699180
caaaaaactt ctgttccccc atgatcttgg aaccaccctt ttaaagcttc caccttagta 699240
tcgagatctt cttcgttaag ctgtcttact aaaaaagcat tactttctgc gatctcttta 699300
ttttgagctc tttgttctgg gctaagatta gggccgacaa atccttggag gacgccacca 699360
cgaataaaca agtctgcagc aatatggcga tatttatcat ctcgcgaagc atcgtcagct 699420
tcaaataaca atgcggcatt aacaaacttc gcacaatccc cccaatagac taaggactta 699480
gcagcatttt tcgcacttgg agtagtatta tttgcatttg caagttcttg gagtgcagtc 699540
tgaatcttct tatgagtgat cttagggcga gtgttaaaaa aaatggggag cgtgaggcca 699600
taatgctctc gaaactgcaa ataacggtcc gggcccttgt aagagcgcat cttatcggat 699660
tttccagctt ctcccaaagc gtccctagac ttttcttcca agacatctcc aggagccgca 699720
tttaaaatta caaaatttat agagacaata gcaaataaag tcagggggat taaaactaga 699780
cgttttagga tgtacttaag cacggatcct ccttcttctc aagccatacc atagttacgt 699840
tgacagtctc atcctgagct tcaggaatta aatctgttct atgtgtaggt acgaaaatat 699900
tttttacata atccttataa agtaaggaac aatgtcgtga gaacaagaaa gcataaggag 699960
cttcctcatg aataatttca tggaaacggt ggtacaggcg attacgttct ttcagatcgt 700020
attcgtagct gagtctgtct atgattttat cagcttcttc attatggaaa cctacaacat 700080
tcgctgaacc cttttccata gccccttcag aatgccataa agccctagga tcctcaggag 700140
gaattcctaa acaccatccc attaaaagag catcgaaatt cttttcatca aaagcttgcg 700200
aaagatcggc catatctagt cctagaaggc tacactcgat tccgatttcc ttacaagcag 700260
tagctacgta atctgcaatg gtatgagcgg tgacactctt tacataatag cataaacgga 700320
aacggaacgg gacaatcaca ccatcgataa ctttttctcg gattccatcg ccatcggtat 700380
ctatccatcc ctcttcttcc aggagacgag ctgcttcttc tggagaataa tgccaccctt 700440
cgatctgttt attataagaa ggagaactcg aagcaaaagg cccactaatc gtatagcctt 700500
ggccatccaa gcactgttcg ataatcctct ctctatcgat tgccatgttc atagcacagc 700560
gcacctgtcg gctttggaaa aataatgaaa agcaattcca tcctatgtac gtatatgctc 700620
gatctgctga gactgtttca cggacggctc ctcccttagc tacctgtttg ttataagcgg 700680
agcttttcat aaaactatag aaattatctc tttggttggg tggaaggtaa gagatgtcta 700740
ttttccctgt cttaaaatct tggaataggg agtctgtgct ttccttaaaa tagacgaaac 700800
gcttgtcaat aagagccgca agaggatcat agaagtcagg atttctagaa aacacgattt 700860
tctcatcatc catccctgca aagtagtagg ctccacaact tacaatatag ttgtttgccc 700920
aatgcatagt gaagttttgc gcccaaatgg aattggttcg gtaggtatcg atattctcat 700980
cttcaatgat tttttccccg ttagcaaaat actgatatac aaatctaggg aggggctgca 701040
agcttaaggt attagaaaat gcagagtaga gcactttgcg ctcttccttt ccttcttcat 701100
tgattaccgt gtgtgctttc catctgacta ctaattttaa atcgttttct actgagacag 701160
aaaccacatc ttcataacaa gagcgcagag ccactgctcg catggttgct acataagggt 701220
tcataacagc gtcgtagaaa aacttaatat catgagctgt cacaggatga ggacgttgaa 701280
atacttcgtc taactgaacg tgttttggaa gggccttagg atctatagga cgccaaaaaa 701340
cattcggcct cagatagatg tgaaactctt tatccccaga accatcttca acaagatgtt 701400
cttctatttt cacagcgaga tctggagaaa attcttcgta tttccctacg tggggagaag 701460
ctaaactaga atacagagat cgtaaaagcc cacgacatat caaagccatt aaaaggctca 701520
ggntttcngg tttnccgaca tgggcagtgc gtaggatacc atgagggtgg aaatttgttc 701580
ctagaagttc aggaagagtc tgctgtttat aggggtctag ggaaagtaaa ttaggataac 701640
taggatctcc aaagagtaga gcaaaagctt cgtctctgac gagcttagga gcgagcatca 701700
ccccaggtgc cgcagggata gcttgtgatg tctgctgttg tttcactacg cgtgagattt 701760
cacgaatgtc ttcttgaata tctcttacgt taccttttat cgacttaatg tctctttcaa 701820
gtaggtctga ggaccagtat aacaaaatta aagaaccggc gacaatcccc tttaaaattt 701880
tatctagcac acatctttta tacatgtgac cactctccct ccgcttttag aggcaaggac 701940
tcatagggat ctctccccaa ccatttaatc cgagggacct tagatgctgc atagtattaa 702000

aatctctcaa aatcttatac aataagtgct gcttcgcacg attaagagaa catgtttctt 702060
ccttcaacaa agaggaaaag gtcttttgtt gatatttaga tagcaatatg atgagctccc 702120
cgtatgccaa tctgtcttgt ttgagttgta aattcactgg ctgcaaacat ggatcaaagt 702180
ggtttggcag catgttctga aatctctttg cttggcataa actgtattga aaagattttt 702240
taatcttcat gtaaccatgt tatgctttgg actatgaatg ggtcaagaaa tttcagaaga 702300
gagggataag gagcaaagac tcttttagaa attatttgtc gctacataga acaaaaacaa 702360
tctccaaaat tttaatataa gagagttgtg agtatggaaa taacttgtca actcatgggg 702420
gananagaca aatagagggc tttcaccctc ttgtttgtct tttaaaaact aaaaactatt 702480
cagcagtatt ctcttcagaa ccgccttctt catctttttc agtttgagtg tcgctatctg 702540
aagattcgtc tcgtccttca tcttcagaac cttgagcttc gtcttgtggt gctgaacaag 702600
attcgtatcc aaaagcagaa cctgcaacag gaagcacaag agctaaaaga agggctaaca 702660
ttaatttttt catttttatt ctccttattt aaaatacgac agctcaaacc tgagattggc 702720
caaataatca gtaacctatg acaaaataaa ggctgacgta aaggtttcca ggcactatat 702780
aaaaaaagaa aaataatctg caaataaaaa gtataaaaac aaatggatag tgttattact 702840
cgcgttgttg aggaaacaag taaataccat agagtaaaac tataattcct atgattagta 702900
gacctataac caatgggata ggtaaaagcc ctaaagccaa acagccatct ataaatacct 702960
gagcaagagg ggttcgcagt aaatataata cttaccgcag cagaagcaca aaaggcaact 703020
aataagccta gaattgctat agcactagca atttcgtaac gagaacttac agacaaatcc 703080
gcacgccaag aaaccctgac tctattccac agactgtctt gccttaaaag aggataatta 703140
ggtgttattt cgtccattga attaatgata tcgtcggcat gtattctagg aaattgctaa 703200
aaaattagta tttatattca acaccataaa atttaataag tttttgagat tttttataat 703260
ctcttttttt tatctcttcg taaattctta gacctttagc attcatacct tgctgaaaat 703320
aaagtattcc taactttgac atcgtttcta catcattagg ttttaatttt aggacaattt 703380
catactcttg gatttcttcc ataggcattt gcaagtcatg atagctataa gcgagttgtg 703440
cgtgtaccca agcgttacct ggagcatact catttaaaat ttggaactct tcaatagctc 703500
ttcgcgccgt tgcaaaaaac ttttcttgaa tctcagcgct atatctaccc gaagggatcc 703560
aataattagc atcaaactct gggtatttcc ttggatctgc gtaaagacca gaaagagcta 703620
catacgcatc tgctaaagag acatgcgcac taagatctac agggatggct tgaaccactt 703680
tgatataagc ttcgatcgct ttttggagta aacattcacg aaaaagaaag taatctttcc 703740
aaaaacaaaa acaactaaat ttcctaatga gatcatgctt gggcaagaat ttgaatatct 703800
cagaaagaag agagtattct tgattctgaa gatttataga taatttagta gctgctgccg 703860
ctaaatgcga ttgctcttct acaatgttct gggagcgttt attgggagga acagcacctt 703920
gaagatactc tgaagcgaga tcctcaaaaa aatcaccttt ccccgaaaga agatagagcc 703980
gtgtgacaag gcaaacaaac aatgtaagaa aaaaacacgc aagacagaat gctggaatga 704040
ctgttttccc tgagaataga aagaaatata gaaaaaaact gaactcaaga gcgatgagaa 704100
gagaggctcc acaaacaaag aggatctgct taattaaata cttaagtaac tgacgacttg 704160
ttttattaca taaagcttct aaattctcct gaaaacctaa aggtttcata aatcctgagt 704220
cctactttgt taattctaat ggaaaattat gagattaatt gttttaatgc aatgtttggt 704280
ctccctattt ttagcaaaga aagtcaccgt gacaactcca gcctatctac tagctaattt 704340
tggaggacct cgtcatgcta aagaccttca agaatttctg atttctctac ttactgatag 704400
agatgttacg ggcactttcc ttcccagagt actgcatagg catctcttta ctttcatcgc 704460
taaaaaacgt gttccgaaag ttctccctca atatcaatct ttgcaaaatt ggtctcctat 704520
ttattttgac accgaaactc ttgcaaaaac actctctgaa atcttacgag cgcctgtaat 704580
tccatttcac cgctatctac ccagcacaca tgaaaagacg ttgcttgctt tgcgtactct 704640
acatacgcgt cacgtgatag gtatcccttt attccctcac ttcacctatt ctgttacggg 704700
aagcattgta cgtttcttta tgaagcacgt gccagaaatc cccatttctt ggattcccca 704760
atttgggagt gattctaaat ttgtctctct cattacctgc cacattcggg atttccttca 704820
gaagttagga atcttagaaa aagagtgctg cttcttattt tctgtacatg gacttcctgt 704880
acgctatatc tctcaaggag atccctatag caagcaatgt tatgaatcat tttcagcaat 704940
tacgacaaac ttcaagcaat ctgagaattt tctttgcttc caatcgaagt ttggtcctgg 705000
aaaatggctc tccccgtcca ctgcgcaact atgtcaaaac atagatacgg ataagcctaa 705060
tgtcattgtt gtgccttttg gcttcatttc tgatcacttg gaaactttat atgaaataga 705120
aagggactac ctgcctctgc tacgttctcg aggatatcgg gcattacgaa tcccagcgat 705180
ttatagctcc cctctttggg tatcgacttt ggtagatatc gtgaaagaaa actctacagt 705240
agttgccgag gagttaataa agagcgggaa aaaacacaca gggattcgat aagattttag 705300
ggaagacgat accgctgttt tatagcatcg tattttcctg agcgtcgtgt tttcacaagt 705360
cctgcgttaa acccttcaag caaatctccg tttgtccctt tcagtattgc aagccgcaaa 705420
ccatctgcgt ttaagggttt tgaaataatt ttcagtcttc ctttatatgc tgtttctatt 705480
agcgcggtca cttcaattac aggagctaga agagcgtcgt aacaattcga tgttaaggct 705540
tccaatgcta ttggaacatg ttggtagagg ctaatcacag cgtcagggat attttgagct 705600
acaagaactg aagagtcaaa cttatacact ccaataagac gacctttaag atcctctata 705660
gattggtaag gagagtcttg agcgacgaca aggacaggac ctgtgagtaa aatgggatca 705720
gaaaattgat agtgttcgag catctcaaga gtaggcaata cagatgtaaa tgctccttgg 705780
gtctttttat catctaaatt ctcaaagaga tgcacccaat cttgatttac aatattaata 705840

tttagattct ctttatagtt aatctcagaa acaagatcgt ttaaaaatgc gttggtatcg 705900
gatgtataaa tgccgaattg ttttggaaac caggtggcat cacgacctac gagaacttct 705960
ctttttactc gagagcaccc gaaaaagatc agtcccacag ccagtaaaca tattaaaaaa 706020
tttaccttcc aagaaaattt tatcttcacg tttagctgtg cctcgctgtt ctgcctcttt 706080
ttgcatcata ttctagataa gaaattttta tccagcgcgc tataaagcct aattttaaag 706140
aagagggatt cgtataaata tctgaaaaaa tagaaacata taaaaaaatt atcacattga 706200
aaatcagtac tttctgatat taagaaggac tattcgatat ttcctcagtc ttaagggtcc 706260
ttttccacaa tatactcagc aagatatgtt ttcccaagtt ttcttcttcg ccttagggtc 706320
aaaccttcac aagcaatctc ttcatcggag gcattctcta ggaatagagt cccttcagga 706380
ttcaggatgt ttcccgaaac aatcttctgt aaaagggttt ctacgtaaca attacaaagt 706440
tcgtaaggag gatctatata gattagatca aaggatctct tttgtttgat aagtctttga 706500
attgccgatt gagcatcttg tctaaagatg acgacaggaa gttgttctcc gagtaaagca 706560
ctatttgtgt gtattaattg tattgctttt atagagatgt ccacgaatac gacagaagca 706620
gctccacgac ttagggcttc aaaacctatc gctcccatcc cggcaaaaag atctaaaaaa 706680
gcagcccctt ctatatcttc cctacagata ctgaaaaacg cttctttcac taaacctgaa 706740
gtaggtcgga tatgtggatt agaaaatgtt tttaaggatt tccctttgta cttacctgct 706800
aaaattctca catcacctct tgaggaacaa agtttacgta atcagcagcc actagtatgt 706860
agtggatccc tcggatatta ccgaatccat ctatagggcg ttgcacttta tgaatatgac 706920
caaataggca tagagagact cttccatcag cttctaagaa ttccgaaata ggtcctggag 706980
taccgtcact gctgattggg ggtagtgtgg tcatcacaat cacctcagtg acttctttag 707040
gaagggcagc aaaagctctc tttaaacggc ccaactctcg gagaaaaatc ttttcatcct 707100
gttctgtata agattgttcc tgagtagaag gagtaaggaa attctccttc ttcacacata 707160
ttgtaggact atcccacagt cgcactccta caacagcaag atggggtgtt aacagagcaa 707220
acccttgatt caaatagtat agagagggag gaagtgcttg tagaatttta cttgttgaag 707280
cagaactcca gtaatcatga tttccacgaa tcatatactt cgttcctggg agatccccaa 707340
taaaggcgaa atctttatga gcctctgaga ggttcatagc ccaagaaata tctcctggga 707400
ggagaacaat atcctctgga tggacaacag cttgccattc agagcaaatt ttctgatggt 707460
atcctatcca ggggtctcca aaaacttcca tagttttttc agggacgcct aaggctagat 707520
gcaaatctgc caaaccataa atatgcattt gaagggagcc agtgtaaaaa cgcaattgcc 707580
gtctactgta ggaactctaa ccataaaaag caaatgtctc tgaagttcct atctctagag 707640
cgtcacgact gaccttggga gctacacttt gccctagaag atatagttgt caggaatgtg 707700
cgtgccttga ggaacaatga taatattatc tctcacaaat agctttttat caggagaatc 707760
atatttaata tagcccttga gattctgtag tttgacaccg tttccaatac aacaattctc 707820
atctataatc gctttacgaa tctcacagtc tttcccaatt cctaaagatg gcatggatgg 707880
agatccgtac cgagcattgc ccataattat agactgatcg actacagagt tctctccaat 707940
cttactacga atccctaaga cactccgaga tacatgactc gtattaataa cacaaccctc 708000
acataataaa gaacttgaga tcatagaatc cgtaatgatt gctccaggaa gatgatgatt 708060
tttactatag atcattccgt tatcatcata acaattcagt cctcttttct ctgcatgagg 708120
cttttgagtt aacgctatat ttgcttcata ataagattct atagttccga tgtcggccca 708180
atacccatta taaaggagag tttgtacttg tccccgcttc atctgagctt ggatgagatg 708240
ctttccaaaa tcgtttcctt cttcttcgcg aagcaaagaa aacaaactgt ctcttcggaa 708300
taagtagatg cccattgatc caagaaagtc tcctgaatct tcggttaact tatgaatacg 708360
gcgatcttca gaagaaagct gaaaacgctt gagtacttct ttttcttgag gtttttcata 708420
gaaatcgatg agtttccttt cagaatcaat atctaagact cccattctat aggcatcttt 708480
ttctgggata ggctgggcaa caagaaccat atctacatga gttcgtatgg ctgtatctac 708540
aatagatcta aaatccatat tgtagagctg gtctcctgat aagattaaaa agtattcgat 708600
ttctgtatct tcgaaataaa gtaagttttt tcgaattgca tctgcgtacc ctgataccag 708660
atttggtcac cctgacgtgc ttcaggagca agaagatgta tctgatcctg caaaactcca 708720
tgataaaaat acgtcttaaa taaatgctgt tgtagagtgt aggtaaggta ctgacctata 708780
acaaaaattt ttgaaaaacc tgcactaatt gcatgagata ttggaatatc gatcagctta 708840
taccgtcctc caaaagatac ggtaggctta cagcgacaat tagttagagg agataacctt 708900
ttgccctccc ctccacacaa gataattact ccaaccttat ctcgataaaa atgagagctc 708960
tcaaaatttg aggcctccgg aaaatcgttt tctatcattt gtattcgcct gttctaattt 709020
aattttaaat tagaaacaaa taaaaaggcc aacacaacaa aacaaataaa ttttgttaaa 709080
taattaaatt ctttttaata aataggttct agaaattaag attcaatttc tagaatttcg 709140
gaaattttgt ttgccagggt tagatcacca ctgcttagct tcccataagc gatcaaagct 709200
tttatcagag tgggtacagt aaataccgaa ctgactttta ttccctgtgg accaagtggt 709260
tgacacgctt cttttctacg atctaagaat accaatgctt cacgaactac cagaccattt 709320
tcttccagtg cgactgctgt ctctattata gattttcctg aggaaaccat atcattgatg 709380
actaaacaag tttgtcctgg agtaaataac ccttctactt taatagcgtc cgaggggtct 709440
acattctgta attccttcct tcgcaatacc atagggatgt tatattttaa agagatcgag 709500
gttgctaggg ttagagcagt ataagggact ccgcagagta agctactatt gaatgagggg 709560
cggaggcgcc aaataagagt tgccactgtc tggagaactt ctggagagga gatcacaaga 709620
cgcatatcta catacagagg agtttcttct ccgctagcga gaatatgttt tccgaacttt 709680

atagctccga tttggtatag aattgctaca gcttgaccgc gtaattttgc atcttcgtag 709740
ttcatcattc tccagagcct ccgaccgttt ttgacaaaat acaaaaaact cacttataag 709800
ggaaaacatg ctaatacgcc tgtttcttgg aatttctctt cccaaaggct ttcccttata 709860
tttggagcct cctctagttc ttgcaacgtt tcaaggaact caattcgtgg gaacttatag 709920
tgaggctaca aaccctttgt atatcgataa tttgaatcta aattaccact atactcaaga 709980
actactttat aaagcagtcc cgtgtaatta taaatctata tatagagaga tacctttaat 710040
tattttccca gaagtactca taggaagcac gccaacacaa tctactgagt gacgtcctac 710100
ctaaaagcat aacaaagatt aagcaagact attctttgag tgacaacaag aattctgcgt 710160
tactattggt tttcttcaac cttcctagca agagatgcat ggcgtctata gtagtaagat 710220
ctgctatggc ctgacggaaa aggtagaccc tttctaattc actaggatga tagaggagtt 710280
cttcttttct agttccactc ttaattaaat caatagcggg gtaggttctt cgatctgaca 710340
gacgtcgatc caagacgagt tccatatttc cagtgccttt gaattcttca aaaatcactt 710400
cgtccattct ggaaccagta tcaattaaag cggttgcaag aattgtgagc gatcccccgc 710460
cttcaatatt tcttgcagca ccgaagaaac gcttaggttt gtgcaatgca ctagcatcga 710520
cacccccggt aagaattttt ccagaatgcg gctgaactgt attataggca cgtgctaatc 710580
gtgttatgga atcgagtaga atgaccacat catttccatg ttccactaaa cgacgagctt 710640
tctctataac catctcagcg acctgaatat gcctttctgg ttgttcgtca aatgtcgagg 710700
caacaacttc tccacgaact tgccgaatca tatcggtaac ttcttcgggt ctttcgtcaa 710760
ttaacaagac aataagaaca atatcaggat tattcacagc aatcgcgtgt gctatgcttt 710820
ggagaatgac agttttccca gatcttggtg ggctacaatt caatccccgc tgtccttttc 710880
caatgggggc ggtaagatct aagactctct cagccaaatg atctttcccc atttccatca 710940
cgattctttg attagggtag aggggggtca ggttttcaaa aagtacacgt tctttagctt 711000
tatctggagt agatccgttg atcttatcta ctttcaatag agcaaagtac ttttctttct 711060
ctttaggtga gcgtatcgta ccgataatcg tatcaccttt tttgagatca aatctacgaa 711120
tctgagctgg agaaacataa atatcttcag cagaaggtag gtaattatag gttggggatc 711180
tcagaaatcc aaatccgtct ggaaggactt ctaacacgcc ttcacctatc agcaattcat 711240
ctgggcgctc tgacttggct ttaacaatct caaagacgac ctgagacttt gttagagatc 711300
ctatattttt cacaccgtat tggcgagcta atatattcag ctcttcaatt cccattcttt 711360
gcagtttagc aatttttgta actgtgacag actcggcctc ttcagactca ctggcaacta 711420
cagcacactc ccctacacaa gatttttcct gcatagaaac gtaagcatgt tttttcgttt 711480
ccttcaccct aggcaagatt tctgaagaac gctcttcttt cataatgctc cctttaaagc 711540
gtaaaaatat tcttcaattt tttgatgtaa ttctttttta gttccgttgt tttcaacaac 711600
aacatctgct tgtgctaact tttcttcgac atttagaaaa cgcgaacacc tttgatcaaa 711660
gtcttcagag gaacgccctg ttttcttcat gaatctctcg cgtcgaatat cttcgtttgc 711720
cataacaagg atcactgaat caaaccactt agcatagtgt atttcgtata ataaaggcac 711780
ttctgcgaca aaaaacggat agttcccatc ttgaatactt tgatgatatt gttcctcaat 711840
aattcgacaa acttctggat gtagaatggc ttccagacct tgtaatagaa cggaattgta 711900
aaaaactttg gctgctatgg cttgcgcatc aaacgcccca tcaactacaa catccgatcc 711960
taaaagatct ataacacgac gacctatgcg tgtatgaggg ataaggaaac tatgcgaaat 712020
ttcatcagca ctaactacat aggctcccaa ttcctgaaaa acttggcaag cttcagtctt 712080
cccagaagag agatcccctg taatggaaac ttttaataat tttaacattc tgcccaattt 712140
tttccaatta agatattcac aactatagga acggataaag tcatagctga ttccatcttc 712200
tctctcacta gtctttgcat ctcttctatt tcttcttcag gaacctcaaa taataattcg 712260
tcatgtattt gtaataacat acgactcttc atttgctgtt gctttattgc ttgtgaaata 712320
tctagcattg caagttttat caattcagca gcacttccct gaatgcgagt atttacagca 712380
aaacgtcctg aagctgctct tgagccagga aattcattcc aactatcgat aattctttct 712440
cgacctaaca tcgtggtcac ccgtaaatct ttagctgctt gttgtatagt ttcttcaaca 712500
aaatgagcaa tttcgggata acgagagaaa tatgcttgaa ttaactcttg gacttcgcca 712560
atagaaattt ttaaaacttt tgccaaacca aaagcctgtt gtccatacac gataccaaaa 712620
tttactgtct ttgcctgcat tctttgttct tttgaaacct gttctaaagg cacatgaaac 712680
acttgtgatg cagtaaaagc atgaatatct tctcctgact caaaagcaaa ttttaatgac 712740
ttatcttggc ttaaatgtgc taaaaatctt aactcaattt gagaataatc ggcagataaa 712800
aaataactat ttttctcaga taaacgaaaa gccttcctaa gtaagattcc tcgttccgat 712860
cttataggaa tattttgtaa attaggatct cgacaagcta atcttcctgt cacagctcct 712920
gtctgatcaa aagaggggtg tattctctgt gtatgggaat ctacttgttt cggtaatgct 712980
tttacatatg tggataataa tttttcaatc gtccggaaag ctaaaagttt ttcgataatt 713040
gggtgctcac tacgtaaagc ctctaacacc tctgcacgtg tagattttgc cttatctata 713100
gggcgaagac ctaattcgtt atataaaata tctgataact gttttggtga ttttatattg 713160
aatggccttc cagaaagatc atagatttcc tctgtcagta cggctaattc tgtttcaaag 713220
agggcttcta aaatagccaa ctcctcaaca tctaaaggca ctccagctct ttccatagag 713280
aaaagaacct tctccaaggg catttcaata tcacttaaga tatggttgag attcttacgg 713340
ttgatttctt ctaaaatagc gtcttttatt atgggaaggt aggcaacaaa ttcaccaaaa 713400
tattgctcag gttgttctgg tagacgacct ataggcaatc ctgaatttcc ccattcctta 713460
gcaaaccggt gagcagtttc tgtaaatccg tgatttacta aaagagattg aaaggagatt 713520

tttcctcccc catttgtcaa gtgctcagct aaagctaggt cataagaaat ttctcgaatt 713580
acaattcctg catttagaag agcgtggcaa tcgcgtttta gattataacc atagaaagta 713640
agatcttctc ttaaaaagaa atcttttaat atagggagta tcttcgtgcc ttcctcttct 713700
aaggcaataa aaaatacccc tgatccctgt gtcaaagcta aaccttcaag cttcaaagag 713760
agaagatggt ttcctgtata tgctacagca aaggcaatgt cccccccttg cacaaggttt 713820
aaaatgttgg tgaggctttc cgcatctttg attatctgaa catcgactgt cgcagcctct 713880
gtttgcttgg acggcacaag agttttaaat ccttgttgta tataaaagtg tatgagtttc 713940
tcttcatcca cagggtgttg tggaaaggta agggactcta ttggtactgg gatagggata 714000
ttagaatcca agagggcaag ccgtttacta agctttaaag tctcctgccg ttcactcagc 714060
atagtttgac ttaatccctt cacggcgtct aaattttcta aaagtccctc aacacttcca 714120
aattgtttaa gaagtgctgc agctttttta ggcccacaac ctgggagccc tggaatgtta 714180
tcagaagagt ctccaactaa cgctagataa tcaggatatt tccctggagg aatcccataa 714240
cgttctatca cctcagagat ccctacaaca ccttgatctg cccaaggatt ccaagctaca 714300
acatgatcgt ttacaagctg caacagatct ttatccgcgg tgcatacgta aactttataa 714360
ttctcttctc tagccttctt agcaatactt gcaatcacat cgtcagcttc taccgactct 714420
ttttctaagt aagctaggcc tattaaagag cagtactctt taactagagc tatttgtgga 714480
gggatgtctt cgaatttttt ctgtcgatta cttttgtaat cagcataaat cgcctgacgg 714540
ctttgtttat tattaggacc gtcaaagacg gagatcatgt attctggaga gaattctttg 714600
ataagtttat ttaaagaacg aataaatcca aaaactgctt gtgttgcttg tccttgatga 714660
tttttcattt ctggcaaagc aaagtaggca cgaaagataa atcctgaggc atctaataca 714720
aacagtttct tcattgcata ttcccttcta ggtctctcta cagtgccaag tagggaggta 714780
tgtatgcagc gtcatgggat aaaggcagaa tatcgtgttt aatcatgcca gtaactaatg 714840
gacttgaagc tgcagctgaa gccacccgct tccaccaacc atcaccacca gagccaatca 714900
ctctatagtt atcttcaatc ttacaaacag caactatgtc ttgaaggact tgttctttag 714960
ttgcgcctac aacatcaata tagccttctt gtttggcctt ctctggagaa aaaatacgtg 715020
ctccgagagt gtgaactaac ttctctttag taagcagagg acggttttgt gtaactatat 715080
caacaaattg tccgtagaga aaatcaagag tcgcttgccg ttcttctcta tcatgagaag 715140
tccacggtgt ataaggattc attggagcct tatcttttcc agctgtcagc agatcacttt 715200
caactccgta gcgatttaaa ccttctttta cattgaagaa tggtccagaa cgcactccga 715260
tagaaccgat aagagaggag gaggtggcat aaattttagt tgcagcgcag gatacataat 715320
agcctcccga agcacaaaga ccattcacat aaatataaat agggaatccc ttacgttctt 715380
tccaaaagcg aagcatagag taaattctat ctatttcaaa gacctcgcct cctgggcaat 715440
ccatatcaat gacaatacct ttgacacgat ctttaagagg agctttctca aatccttcta 715500
aaatattctg aatcgttttg gccgtatttt ttgaagaagc aattacatct ttcatttcga 715560
taacagcaat aatgggggct gttttcccta gatcttttac ttctccttgg gcgtcaggca 715620
agctaacgaa ggtagcatcc ccatttccta aagaggaagc tatgagtgcg aatataacga 715680
caaaagcaag aacaactccg caacacagtc ctactatcga taaaaaggct ttggatacga 715740
agtgccacaa cgttttcata acgacctata aattctagta aattctaaaa tggataaaat 715800
aagggatgag cagaaaagct taaagatgaa aattataaaa ttctcatgct caccgagaat 715860
aacacagagt ttttctatca acctacagaa cagccatttc ttcttgttct acagattcct 715920
gggcttcacg tacgattgaa gaggaaggtt ctttaggttg tttcaaggtt gctacagcat 715980
cagcagctct agagtagtat tctttaccga tataggcaac aaccgcaatc caaacgacca 716040
taataatgag aagaactagg gcgatgacgt ttaaacttgc tgcaacagaa gagaaaataa 716100
caagcagccc ttggtaaatt aaagagcctc cagactttcc tatccttgaa acgacaccat 716160
caatcgcggc tttcccatga tttttatcct ctggagaaag tgggataaag gccatttcct 716220
tggtttgatc aaagaacgta aatttcgtcc cccgggatag gacattttgc atccctccag 716280
tccaggcagc tagagccaga ggtgtcattc caagaactcc cccaaaaata gagatgtctc 716340
tttttgcagc aaaaatagtt ccgaaaaaga gcagtcctga aactaacatt accaatggag 716400
tgactaaagc accgacagtc catccccatt tacggataca ctgtccggta aggagtacag 716460
ctgctaatac agaaacgacg ccaatgaggg tagtgattct actcatatac ccattgaatt 716520
ctacgtgaga actgtaaatc tggctaactt gatccttcca aacgacttcg aatagatgga 716580
tcaccaaatt ataggatagg acaataatag cgagccctaa taaataacga gactgaatga 716640
ggtgtaagaa aaggtttcta gctttggctt taggtttttt cttttccttt agattagcag 716700
tagctgctcc ctcttctgcc aagacacgtc tagatggagg gatcgaagta tcaatagtca 716760
aatgatgaat ccgcctatat agccagatca taattaaacc agaacaagtg atcagcatgg 716820
tcaagttgag cattacagag tgccaggaat cacatgcaaa ggagtaggca acaaatgttt 716880
gtttccccat ccaataggag atttctcctg cgcatattga ggagagattt aatcctgtat 716940
tgataagagc gtaaaaacgg cccgcttcag taattgtagt aatctgattg gctagtcccc 717000
agaacaacat cgaaagaaca accgaactcc acagctctga cattacgtaa taaatactgt 717060
aactccagta acggaccatc acaataaaac cacgaagtcc ttgaggaagg agctcttgta 717120
atttatcagc gagagagttg agatgcaggc tatcccctac aggataaatg atcacagcaa 717180
acaggaagaa aaaaccaagg aatgcggcca tgaagcaata aaaaacggta tcccgaggat 717240
accgactgcc taaccaccca tagaccatag taacaataac agctcccggg acaattcccc 717300
aaaccttaag gaagggaatc acttctgccc cagcatctga accgacaatg accagagtat 717360

ctttcatgtt tttcagcagg cagtagttaa agccaacgaa aaacgctagt agaaatagtg 717420
gaacaaactt agaaaattct gatttataaa taggacaaag atatgcccgc agccttgaaa 717480
agggtttcac ttctgatgac tgcataaact ccttcccaac tttatggcgt ataaagactt 717540
atagacattt agtttacacc cagaagacat cgcatgctta tagctcctat aatactggtt 717600
acttagcaaa cggcaaggca taaattaacg atcacgataa aaagagactc cattttactg 717660
caacgcgccg taaagttcaa gcggttcgtg gataaactta taatccttgc tcacaatact 717720
ttttctttcc gggttagcgg agaaactgtt ttttataatc cataattctc tcgaggaaat 717780
atagaaaacc gagagccacg agggggacta gtccggtcca aggaatttca ccccctgccg 717840
tgacccctag taaaataata aattgaacta ctgtaaagat cttgccccaa aataaagagc 717900
cataatcata gcctttccaa cctttaacta aagaaaggta gaacacaaag gtnataagga 717960
ataagtctcg agcgcaaatg aaaaagaggt gtgctattga taaggatcct tccatataga 718020
ggaccgtgat acaaacgaac acaaagactt tatctgtaat agggtctagg attgaaccga 718080
gacgacttgt cgctttatag cgtcgagcaa gatagccatc taagacatcg cttagcatag 718140
ctccaacaat agcaagtaag cggatatgca atttttcttg gcagaagtat agcgctagcc 718200
acaaacgtga tagagaaagt aggttgcaaa attgtctcat aattttggct aactagtacg 718260
atccttttag gaaccttata attcggcccc attatagagc atgctaaatc tacaaacaag 718320
catagagttc gctcaaacgg tttctcatct tgcaactgtt cggctctaca ttgacatata 718380
ttaggctttc tttaaaaaga atacgaaaaa gggtgcgaac tacaactagc ttgcgttanc 718440
aaggtagagg ttattcggaa agagagggat tcgaaccctc ggtacccttt aggggtacgc 718500
gtccttagca gggacgtact ttcgaccact cagtcatctt tccttattta ttcatcacag 718560
cctaacaatg aattttgatc tcactgttat tgttaggata ggttaactat caaaatgaaa 718620
aatgtcaact tgtgcaaacc tgacaagaaa aaaggaaggc aatctcttaa aattcacctc 718680
tgaactccct atagatttgg aaacttgcaa taaaaggttc tgagcagttc ctcgaaaatc 718740
tctctgagca actagcactt gagggatata tgctagataa cataacctat ctagaagtat 718800
cctaaaccct cacgaattac gaaagttcgc ttctaatgga caaatctact ggtgtacctc 718860
tcccctctcc tcctcattcg aaagaatcgg aaatgatagt tttaggttgc atgctgacag 718920
gggtacatta tctaaatctt gcagccaacc aactctacga agaagatttt tattaccttg 718980
aacataaaat tatttttcga gtcctccaag aatgccttca agcaagataa acctatcgat 719040
gttcacttag ctggagaaag aactcaaacg ggcacaacca gattactgta attgggggcc 719100
cttcgtatct aaattacttt agccgaattt gcaggtaccg nagcctatct tgaagaatac 719160
gtagacatca tccgatcgaa gtcgattcta aggaagatga tttctacagc aaaagaaatc 719220
gaaaaaagag ctttagagca gccgaaaaat gttgccgaag ccttagatga agctcagaat 719280
tcttttttta aaatcagcca atcaacatcg gtaagtcagt acactttagt tgctgacaaa 719340
ttacgcgggt taacaacaac tacagataag ccttaccttg tacaattaca agagagacaa 719400
gaattatttt tacagaatgc tcaaggagac aataagtctt tcttcactgg cattcccaca 719460
cactttattg atttagacca gctgattcac ggattttctc cttcaaattt gatgatctta 719520
gctgcccgcc ctgccatggg gaaaacagca ctcgctttga atattgcaga gaatctttgt 719580
tttcaaaacc gcctccccat tggaattttt tctttagaga tgacagtgga tcagctgatt 719640
catcgtatga tttgctctcg atccgaagtt gactctaaaa aaatctctat aggcgacctt 719700
tccggtcatg actttcaaag aattgtttcg gtaatcaatg aaatgcagga acacactttg 719760
ctcattgatg atcagccagg gttaaaagtt tctgatctac gagctcgggc tcgtagaatg 719820
aaggaaagct atgatattca atttctcatt attgattatt tacaattact ttccggctca 719880
gggactttgc gtgctacaga aagtcgtcaa acagaaattt cagagatttc ccgaatgttg 719940
aagactcttg cccgtgagtt aaacattccg attctttgtc tttcacagct ttctcgaaaa 720000
gttgaggatc gggcaaatca tcgtccgatg atgagtgatc ttcgggaaag tggaagtatt 720060
gagcaagatt cggatttagt gatgttctta cttcgtagag aatattatga tcccaatgat 720120
aagcctggca ctgcagaact tattatagca aaaaaccgtc atggttctat aggttctgtc 720180
cctctagttt ttgaaaaaga actcgcacgt tttcgcaatt attcggcttt tgaatgtatc 720240
agctagttga ttcgcaatgc gaatcagagt caagtagatt ggacaatccg atttgtctta 720300
aaacccaaga atcagtatag ttgtttggtc ggctgaggat agcaccatat ttgcaaagaa 720360
atagcgtctg catcctattg aaccagagtt ccgccggaaa aattcaaact tcggtttgaa 720420
tatgaagaat cttcgtcact tatgacgaag gggatgtcaa atcgatacgt taaccataaa 720480
gatattatgt catctgttaa gaagaaacga agactcaaga tcgccaagca caagcgtaaa 720540
aaaagacgtc gtagagatcg tcataaaaac aagtagtttt tagtaactta tgtggactca 720600
cccaattgct tatgatgtga ttgtagtggg agctggacat gcaggttgtg aggcagcata 720660
ttgctctgca aagatgggtg tctccgttct tatgctcacc tccaatttgg atactattgc 720720
caagttgagt tgcaatcctg ctgtcggtgg tatcggcaaa gggcacattg ttcgagagat 720780
cgatgccctt ggtggtatta tggcggaagt gacagatcaa tctggcatac aatttcgcat 720840
tctgaaccaa accaagggac ctgctgtccg agcaccacga gctcaagtag ataagcaact 720900
ttatcatatt catatgaaac gtcttttgga gaatactccg ggccttcata ttatgcaggc 720960
cactgtagag tctctattag ataaagaagg tgtgatttct ggagtcacta ctaaagaagg 721020
ctggatgttc tcaggaaaga ctgtagttct ttcttcggga acttttatgc gcggcctaat 721080
tcatattggg gaccgtaatt tctctggagg acgtttaggc gacccttcat cacaaggttt 721140
atcggaagat cttaaaaaac gtggttttcc tataagcaga ttgaaaactg ggacccctcc 721200

ccgtttacta gcctcttcta taaatttttc ctgcatggaa gagcaacccg gagatttagg 721260
tgtgggtttt gtacacagaa ccgagccttt tcagcctcct ttaccacaac tttcttgttt 721320
cattacccac accatggaaa aaactaaggc aatcatttca gcaaacttac atcgttcggc 721380
actttatggg ggctgcattg aaggggtagg tcctcgctat tgtccttcta tagaagataa 721440
aattgtaaag ttctcggaca aagaacgtca ccacgtcttt ctagagccag aagggctgca 721500
tacccaagag atctatgcta atgggttatc tacttctatg ccttttgatg tacaatacga 721560
tatgatccgt tctgtactgg ggttagaaaa tgcaattatc actcgaccag cttatgctat 721620
agaatatgat tatattcacg gcaatgtgat ccaccccaca ctggagagta aacttattga 721680
agggctcttc ttatgtgggc agattaatgg caccacaggt tatgaagaag ccgcagccca 721740
agggttaatt gccggcatta acgctgtgaa caaggttttc aacaggcctc cttttattcc 721800
ttcacgccaa gaatcttaca tcggcgtcat gctagacgat ctcaccacac agattttgga 721860
tgaaccttac cgcatgttta caggaagagc agaacaccgg ctcttattaa gacaagataa 721920
tgcgtgtgct cgactatcgc actatggtta tgaattaggg ttactctcag aggaacgtta 721980
cgaacttgtc aaaaagcaaa accagctatt agaagaagaa aaggttcgcc tccaaaagac 722040
atttaggcag tacggccagt ctgtagtctc tttagcaaaa gcactatctc gtcctgaagt 722100
ttcttatgac atgcttagag aagcattccc aaatgatatc cgtgatttag gagcggttct 722160
caatgcctcc ttagaaatgg aaatcaaata ttctggatat atagatcgcc agaaaattct 722220
gattcagagt ttagaaaaag ccgagagttt actaattcca gaagacttag attataagca 722280
gataacagcc ttaagcttag aagctcaaga gaaattagcg aaatttacac ctcgaactct 722340
tggttctgca tcgagaatat cgggcatagc ttctgctgac attcaagttt tgatgatagc 722400
tttaaaaaaa catgcccacc actaactgta ttttcctaga tttacgggga cactctattc 722460
ttcaccaact gcaaattgaa gaggctttac taagagtcgc gaatcaaaat ttttgcatta 722520
taaattcagg tgccaaagac tctatagttt taggaatttc tcgaaacttg aatcaagacg 722580
ttcatatttc tagagcacaa gcagaccata ttcctatcat acgccgctat agtggagggg 722640
ggacggtatt catagattcc aataccttga tggtatcttg gattatgaac agttcagaag 722700
cttctgccca acctcaggaa ttattagcat ggacttatgg catctatagt ccactacttc 722760
ctaatacctt ttctattcga gaaaacgact atgttcttgg tcataagaaa ataggaggta 722820
atgcacaata tattcaaaga catcgctggg tacatcacac gacatttctg tgggatatcg 722880
acctagataa gttgtcctac tacctgccaa ttcctcaaca acaacctacc taccgtaatc 722940
aacgctctca cgaagaattt ttgactacgt tacgtccttg gttcccctct cgcgatgact 723000
tcttggaaag gatcaaggca tctggtagtt tgttgtttta cctgggaaga atttcttgat 723060
aatgagctag aagaaattct tgctcaacct catcgtaaag caactacagt actaaactaa 723120
acgagaggtt tcgaagcgtt aacaacttct atcttactga agaagtaagc gatttcgaca 723180
gcagcatttt ctaaagtatc agatccatga acagcattca cgcctataga ttccccaaac 723240
ttagctcgaa tggttcctga agcagcttct gcaggatttg tagctcccat aagttcacga 723300
tttcgggaaa cggcatttgc cccttccaat actaaaacta caacagggcc tgagaccata 723360
aaatcaacaa gttcttggaa aaaaggacgc tctctatgca caaaataaaa cccttcggct 723420
tcagtttggg ataggtgcat cattttcata gcagctatac gtaatccaga ttgttcaaaa 723480
atagataaga tctctccgat atgggctttg ctaacagaat ctggtttaat aatggatagc 723540
gtttgttcca tggtgtatac atatctcctt taaataaaat cgttttattt taggaggagt 723600
atagcatgat ctttaggaaa gtctagtcct tgttcactcc tgaaaaattc tttttcagag 723660
cgataggaag gatatctgtt aaagaagatc cctcagggag atctttgatt gcttcggcaa 723720
tcatacgttc agcagcaatt tttgaataac ccaaagctgc taaagcctga atgccttcct 723780
ccaagcaaga agaggtggta tgtgtttggc tggtctccac tctcgagtct agaggaagta 723840
aatctggtaa tttttgttta agctcaacca tgagtttttc agcagttttt ttcccaattc 723900
ccgatacgga agctaaagca cgaatatctt cagatcggac tacagaacat agtaccttta 723960
aaggtagcgc attaagaatc gcgagggcta gcttaggtcc tattccagaa aaagaaatta 724020
aaatacggaa acactctcgc tcttctcgag aatgaaaccc atagagcaag tgttccgttt 724080
cacggaatat cacatgagtg aagacgagaa agtcttgatg taaagccctg atgcattcta 724140
ttgcccaacg ttctgtaata gcaatatggt agccaatacc ttggcattct ataacaattg 724200
caccagtatg cacataggtc agtgttccac gaatatagtc gtacatctta tctcactcca 724260
caaagagggc tacgcgctac atgagtatga catatagcaa gtgcaaaggc atcagcaata 724320
tcttcattcg aagggtgcaa aacttcggga acatttagaa tcttgcttac catcacctgg 724380
acctgtcttt tacttgcatg acccttaccg actactgctt tcttagcaac atttggggca 724440
tattcaaaaa tgaggatatc acgctgagct gcagccaata aaacgattcc tcgtgccata 724500
gctaatttca tagtactttg aggattctta tttacaaatt gcgtttctag gaccatagca 724560
ttaggttgcg tatcatctaa tactcctgag agttgctcaa acaaagtctt atagcgcata 724620
ggcagtggca tgtcggaaga taaacgaatg gcaccataac tataaggacg taattgatag 724680
cgctgttcca ctgcaatgat ggcatatcct gcgactatcg ttcctggatc cacacctata 724740
atcagttctg acacaatcga ttcctgaaat actttgaact tattgtcttt aaaagatgat 724800
cctaacctag ataatcactt ctcaagtagt gaattctcgg tttctagaaa aaaatttcct 724860
gctatttaaa acagtcctgt ttactacgta ggaatcttaa ctccctaaag atttagattc 724920
tgagaatact acgtctactt cactctcagg ttctttgcct aaaagtcctt tcaaatgatc 724980
taactttgta tttgaaggca tagccatacc tttctcttta tgtagatctc gaagctgaca 725040

atattgatcc tcaacaatat cttggagtaa ggctatggct ttcttttttt cagctagatc 725100
ggtttgcacc tctgttaact tctcaataga acaaacctca gagctagttt gagctccttc 725160
ttgtcgctgt aatttttctc ttaacttagc aatctctgaa tgatatgttt ttatataaat 725220
atctttttct tgttgcaaat ctataagact tttcttcaaa cggttgatct ctgcatatgc 725280
ggctgaagat ttgtcttctt ctatctctat agagggggct ccgtgttgcg aaacataacg 725340
ctgtagttgt tcttccagct gtttaatttt atgctcatag cataaacgac gcccttcact 725400
aatttctgtt cgctgtacac tttgaagcag ttcttttttt atctcgtcga taatatcact 725460
ttggtgtcga atttgttgta atttctcagc aaggcgattt tgcaaccagg tattttcttg 725520
ttgtaacttt tctaaatctt gatttttagc ttcttcacga cggcgatatt cttcttccaa 725580
agcagatagc tgttcccgat actgtaagtg aatacgttca tgctcctgat gacaagagtc 725640
ttgggtttcc ttctgcaaag aaaccaactt tccactcatt ctgctacaga ctcctgaaag 725700
aatattgcaa atctgctgcc attccataat atcagcatct ttccagcgac aaagcaaatg 725760
aagcatggaa cgggagcatg aatgataata gtgaggggat aatgaactgg aagatcgtag 725820
cgagaagaga tgcagcttaa gtatagataa gagccgcaca gagtacctct aaaattaacc 725880
taatcattca tcgtatagca tacgactgag ttcttgtcct aattcgtcat aagtgacttt 725940
taaaaagaca ggcaacttat ttggataact ctacagatta aaaaacttag aaaattgctc 726000
agaacaagac tcgaacttgc acgggcctac gcccaaggga ttttaagtcc ctagtgtcta 726060
ccattccacc atctgagcac gaaaagacag tctgttcaga gaagccttca attaaaaggc 726120
gtgttcttta aagatgatct ttttccataa ggatagaaaa ctaaagaaca tcttatcaca 726180
gttttttttc tagatcaatg atttttcaaa aaagggaaag gtatgcctta agttaaagtg 726240
aacttaaacc atacctttaa aaatagctat tcttcttctg tagcagaaga tgtttcgggg 726300
aagaacattg cagaagactc ttcaaaagaa gctgaggtag gttcctcttc tgtctcagga 726360
aaatgtatag catcatctgc cgatagcaaa gcattgatga gatcgctgct ctcacgaacc 726420
aaagcttgta acgactcgtt aagatccgca gaagtaacta ctggatctac aacagtttga 726480
gagataactt cggagatcaa ctttgctggg ggtggaagta aagaaaactt cttctgttca 726540
acaatctcat tttcttgaga ttcttcccaa agctcttcct taacagtatc caagatttct 726600
tgagatgctc cagttacttc agcaatctct ttatgatttc tacgacggct gagctttttc 726660
ttttccttcc acttctcccc tttaaaggaa tctttccttg cctctttctt ttccttttta 726720
ctaggagatg taggctcggt tccttcctct gggctatcag ctagaggctt ctcatcaatc 726780
acaggaagcg gagtcgattt ggtcaacttg attagagctt ctctaccacc agcaattttc 726840
actccacgat ctaaaccgac agctttcaaa ttgattttag tatcacggac ttccataacc 726900
tcatagtctc cggcagggac taaaaaaggt ttactatgat cacagttacg gaaaaaacaa 726960
atatttccta aagagatcac ttctatagct tcaaccataa aaggatcttg tgaaaaatgc 727020
tttgtattcc ttaccgacaa cttatacccc tctcgagagg taattacagt ttctatgacg 727080
gggtctcttg taaaatacac gtaattataa ccttttcatt tagagttttt ttctacatac 727140
tctaataagt ctactatacg cgtggcatat cctgtttcgt tatcatacca cgcaactaac 727200
ttgaagaacc gatcattcaa agcgatacca gctagagcat cgaatatcga ggagtactca 727260
gatcctataa aatctgaaga aactacctgc tcatctgtat aatctaaaat gccttttaaa 727320
tcagtttctg aagcctgttt catagctttg caaatgtcat cgtatgtcgt agacttatct 727380
aacctgacgg tcaagtcaac tacagacacg tcttcgatag gaacccgaaa agccattcct 727440
gttaattttc cctttaactc gggaagacat agagttacag cttttgcagc tccggttgag 727500
gcgggaataa tattttgtaa acatccacga cctcctctcc aatctttctt agaaggtccg 727560
tcaacaacta gttgagtagc agtagcagca tgaactgttg tcatcaaacc ttctgtaatt 727620
ccgaaattat ctagtaaaac tttagcaata ggagctaaac aattcgtagt gcaagaagca 727680
ttcgatataa caaagtcttt ttctggattg aaagtcttat ggttcactcc cataacgaaa 727740
gtaggaatat caccttttcc aggagcagag attaagactc gtttcgctcc agcttgaata 727800
tgcttctcag catcttcttt ttttgtgaac aatcctgtac attcaataac gagatcaacc 727860
cctaaatctt tccaaggaag attttgaaca ttgcgttcag ataaaaattg aatttttctc 727920
ttcccaacga tgaggtggtc cgcttcacaa cgtacatcct caggaaaacg tccatgtgta 727980
gagtcgaatt taaatagata ggtaagcgca tccccaggaa caagatcatt aatggcgagg 728040
acttctactg aagagtttct ctttaagatt tgtctaaaaa ctaaacgacc aattcgcccg 728100
aaaccattaa ttacaacttt catcgcttac cctcagagtt caataaattg gaaattgatt 728160
agctttagct agctaaaaac tctataatac acttttgagc attatcacca agttctattt 728220
tgcagtttta gaatacgtgt gtaaccacct tttctttcta caaaacgatt ccccaactca 728280
tcgaataact tattcacaac caaacgatcg acattatata cagaggtgtc tccaccttta 728340
gcttgtcgag cttctttgct tgttaattta ttgtatctga ccataagccg tccaatagct 728400
atacgtcgtg ctgctaagga gtttttttta gctaaggtaa tcattttatc agcatgtcgg 728460
cgtagttctt tagctttagg caaagtagtc tcaattcttt cataatgaat tagagacttt 728520
aacatgttag ctaacataca gcgattatgc gaggaagtac gaccaactct aaattttttt 728580
ctagcgtgtt gcattactta ctatcccttt atatttttag cccgaatctt ttcggcatac 728640
cacttcattt tttcttttac gttatctaaa cctacgccaa attgcgttag gtccattcct 728700
aattcaagct tcatttcttt caatttattc ttgatctcac acagtgattt ctttccaaaa 728760
tttctgaatt gtagcaatcg aggttcaggc ataataacaa gttcgccaat agtctcaata 728820
tttgcattag acaaacaatt tgttgatctg actgagagtt ctatttcatt aattcctaaa 728880

attaacttat gaagaatatc atctttgttt tctttctcaa tagaaatagc ttcttcaaat 728940
acgattttct tctcatccat attttcaaaa atggaaaaat gtttagtcaa aatttgagtt 729000
gaaaaagcca gagcttcttt cggagtcact cttccatctg tttcaactat taaaactaaa 729060
cggtcaaaat ctgtatcctg acccacccgt gtatcttcta caaagtagtt gactaaagtg 729120
actggagaaa aggctgcatc caaaacgatt tcataaacac ccttatcttc caaaacaatc 729180
ctttcagaag gtgtatatcc cctaccaaaa gcaattcgta gatcgacttc caactgtatg 729240
ggttgagtaa cagtaaaaat gacttgatct gggttaacgg cttcaaagtc cccttcttgc 729300
aatagatctt gtagagtcac ttctttttgt ccattagctg cggctaaatc ggaagcatct 729360
atagaaattg aagcttttaa gacctgagtg gttcttccta aagaactatc ctgcatgggg 729420
tactttttta atagggcacc ttttaaattc agaatgatgt tagttacatc ctcaataacc 729480
ccttcgattg ccatatattc atgaagtacg cctgtcatag caaacgagat taaatacctg 729540
gagcttctaa accaataagc aaagcacgtc ttagagcatt tcctaaagtg tggcccattc 729600
ctctttctag aggctcagca atgaagcgag catgtttgtc tattggaagc ccttcaacag 729660
gcaacatttt aactgcttca ggcagttcaa acttatcata aagtaaattg tgtgcgttat 729720
ctgacatccc tttctccttc cctaaaacta cactctgcgc ctttttcttg gccggcaacc 729780
attatgagga acaggagttt catcacggat tacagaaact accaaaccag cagatatcaa 729840
agcacgaaca gcagactctc tcccagctcc agtacccttc aaacaaacct ntanttcctt 729900
taaaccagag ttcatggcag ttttagcagc gtcttgagct gctacagtgg cagcaaaggc 729960
tgaagatttt nttgaaccag aatatcctac tttacctgct gatgcccaag aaatcacatt 730020
accagcagga tctgttatag atactattgt attgttaaag gttgctttaa catgcacaac 730080
acctgaagga atatttttta gttgttttct ttttacgctc ttttttgcct gcgcttgatt 730140
tttaaccaaa acacgactcc taattaaaaa ttatttcttc ttacctgcga ctgtttttct 730200
tttacctttt cgagtacgag aattagtttt tgtacgttgt cctcttactg gtaaagaaag 730260
tctatgtctc tgacctcgat aagaatggat ggcgatcaat cttttgatat ccgattgaac 730320
acgacgtcgc aaatcccctt ctacggtata ttctgattgt agcagagagt tcagtcgtcc 730380
tacttcttct tcagttaatt cagaggctct tgcctcagga tctaacttca actttttaat 730440
gatttcatca gaacgagctg atcctattcc ataaatatat gtcagactta tttttaactt 730500
tttctttgca ggaatatcaa ttccaatgat gcgtggcata cgttgggcct ccccttaaat 730560
agtataagca tcttaggtca aaaattgtta tttttcaatg tcttcctttc gtccgatctg 730620
ttttcagcac gctatcgtag cgacgcatta acaaaaaggc atcaacctgc ttcattgtat 730680
ctaaaacgac ccctacgacg ataagcattg cagtaccgcc taagaaatag cttacattag 730740
aatccacacg aagcagacaa cctaaaagtg aaggtaatat cgcgatcgcc gctaagaaca 730800
acgctcctaa aagggtcaca cgattcattg tatactctaa gtaatgttgc gtaggcttgc 730860
cctgacgtat gccaggaata aaggcattat tttttttcat ttcagaagca atttgttctg 730920
gatgaaattg tgtggctgtc caaaagtatg taaaaaatat aatgagcaac acataacata 730980
tagaataaac taaactgcct ggagcaagta aagctgctat acgcttcatc caagaagact 731040
ctgacgcaat aaactgtcct atagttgctg ggaacatcag tagcgaggaa gcaaaaatca 731100
caggaatgac gccagcatag ttaactttta aaggaagata ggaccctcca ccggggactt 731160
ctcttcttcc aattaccctt cgagcatatt gcacagggat ttttctcact ccctcgataa 731220
tcaaaatcgt agtaatcaga acaaaaacaa aaacaagagc aaggattaaa attgaaatta 731280
gacccaaatc agaggagtct tgagatccta aatttaactt attaacaata gatcccaata 731340
cagaaggaaa tgaggataga attccaaggg cgataattaa acttatcccg ttcccaatcc 731400
ccttatcgga gatctgttca ccgatccaca tcaataagag agttcctgta gtcatgacta 731460
caactgtagt gatataaaaa atccagggga ctccaaatag ttttgaagat aataaagtgg 731520
gcagaacaat ccccggaata gttagattca ttctaagagc aaacttagca aataagagag 731580
actgtattac agctaaagca acggtaaaca aacgtgtgag tctaccaatc ctacgtttcc 731640
cttgatcaga agactccctc atttctcttt gcagcgcagg cataaagact aggaacagct 731700
gaacgataat tgaggctgaa atgtaaggaa ccacacccag cgcaattacg gtcatttggg 731760
caaaagcgcc tccagaaaaa atatcagcta gttgaaataa attctgacct gatcccaata 731820
actgcttgaa gtaagctaca gctaattcac cattaattcc tggaacagga ataaatacac 731880
ccactctaca ggccgtaagc aaagcaaaag tatagaataa cttttgtcgt aattcggtaa 731940
tcagaaaaaa ttgtctcaat gtggtcatat acggcctaaa caatcttatt ttttaagtaa 732000
tgcctagcaa attttgtact ccttgcgata ggactacagc agtatcttgc catacaaaag 732060
tcttttccaa atctcctttg agaatgactt tgactcgtac agcttgtctc gcaatggctt 732120
tctttgcttt taatgcatct aaagtaattg cttctccttc ttggaaaagc tcggctaaac 732180
gtcctgtagt aatttcttca acgcatttat caaaacgttt atgagaaaac cctcttgtag 732240
gaactcttct atataaagga actcctcccc cttcatagcc aaaacgacgt ttgtatcccg 732300
aacggctacc gtctccttta tgaccacgac cactggtttt cccatgaccg gaagaaggac 732360
cacgacctaa taattttttt cttcgtttac gttcagaaat atcaaataat gattctaact 732420
taatcattta tagccgctcc tcttcttaac aaatccttac gtgggctgag tcctgtaaga 732480
gctttaaacg ccgctttcac ttggttcata gggttattag atccgaaact tttagctaca 732540
atatctttaa taccagccat ttctaggatc aaacgaatac gagaaccggc aacaattcca 732600
gttccaggtt ttgctggttt taacagcaac tgtgctccat cgtgatggac aagaacttca 732660
tgaggaatag aaccatcttc taaagcttct atcttcatta aattcttttt tgcagcttca 732720

ccacctttac gaatggcatc tgtgagttca ttggctttag caaaaccata gcccaaacga 732780
cccttgccat ctcctactaa aattaaagca gaaaaactga actttcgtcc tcccttaaca 732840
actttggagc aacggttaac aacaagaact ttctcttcta attgatcttc tttatgagaa 732900
ttctttgata gcgacatctc ttcctaaacc ttcattaaaa ctgtaaacca ccctctctag 732960
ccccatcagc taccatagaa acaattccgt gatatttgaa aggaccgcga tcgaaaacaa 733020
ctcgatcaag ttgaaggttt tttcccaatt cagcaatttg agttcctaat actttagcaa 733080
cttcttggtt ttttttagtc agaccttgac tcttatttaa ttttgataga gtggaaacgg 733140
gaagccaacg tcttgccgat agaatcgtct atcaactgta cataaatatg tttatttgtt 733200
ttcactacgg acaaacgagg ctttgtaggg gaacctttaa tactttacgc actcgcaaag 733260
ctcttcgtct tttcattaat gactttttac ataacgaact ttccataacc ctcgacctat 733320
tttttgcctg ttttcgcagc tttaccagct ttacgacgga catattcatt ttcataacga 733380
attcccttac ctttataagg ttcaggagga cgtttggcgc gaatacttgc agcaaattca 733440
ccaaccaact gcttatcaag ccctttcact gaaatcagag tatttttttc aactgatacc 733500
tgaagagtag atgggatagg aaatttttgt agggtgagaa accccaatgg agagatccaa 733560
aaatgcacct tgaacagagg ctctgaaacc cactccaatc atttctaaac gtttttcaaa 733620
tcctaaatgg acaccttgaa ccatatttga aatgagcgcc caatataacc cctgcataca 733680
gctaggtcta tcgacaacat ggggagctgc gtgtacaaat atactgttat cttttaaggt 733740
gatttctact tctttgaccg atttctgtgt taaggaccct ttaggacctt ttacaataat 733800
tttatcatct tgaatcgaaa cttctactcc ttgaggaagt agaataggtt ctcgagcctt 733860
acgagacatg ctttaccgtt cctatactta atcctgttac cacaccaaac agagcaattc 733920
gcccccaata ttcttagatc tggctagaga accttccata actccctgag aagtggagag 733980
gactgaaatt cccatatttc caaagacgta agggattttc gcagccgata catagactct 734040
tcgagagggt tttgataccc gtttcagttg gtgtattact ggtttacgat catcggaata 734100
ttgtaaaaac acacgcatag cgcgtttgcn gtttncttcc tntactaaat aatgagccac 734160
aaaaccttta tgcttgagga tttttacaat agcctctcgc attttactgt gctctacgtc 734220
tacatacaga tgttctgcca tcaaagcgtt acgaattcgc gttaacaaat ctgctataga 734280
atcacttgtc atgcccatat agatcctttt cctctttatt gagctttctt aaatcgtaag 734340
cccatcaact ctaatagagt ggtgcactca tcatctgttt gcgcggtagt tacccatgtg 734400
atattcaatc cctgggtgcg cttcacacga tctaaattaa tttctgggaa aatttgttga 734460
tcgtctaatc ctacagaata gcaacccctt ccgtctcctt tattagaaaa tccgcggaag 734520
tcgcgaatcc gtggagaaac aatattacag aaacgatcca taaaatcgta catacgaatt 734580
ccacgaaggg taacttttgc accgattcct tgaccttctc gtaatttaaa accggcaata 734640
gaatttcgag cttttgttac taacggtttt tgcccagaga tcatggtcaa ttcttctaag 734700
tgggcttgga ataaattttt atctttagcg gcttcagcaa gtcccatact taagacaatt 734760
tttttaagaa cagggatctg cattttattt gcatagccaa acttttcaaa caaagatttg 734820
cgaatttctt cagtatagaa tttttttaat ctactcatat tagcctttct ttcccctcac 734880
tagacgatac agctgggatg ttccgtcagg gcgtctttgc cacaactccc gcccctgttc 734940
agtcactttg acagagagtt tggcaggttc accagctatg gttaaacgta cattagaaat 735000
atggatagga gcctctatac taatccgttt acctttagga ttttgttgac tacgttttat 735060
attttttatg cgaacgttta caccttcgac aactacttta tcttcagtaa gagaaaggac 735120
ttttccttct tttcctttat cattaccagc taatataaat accttatcac caacacgaat 735180
gttttgcttt ttcataactt ccttctcctt aaattacctc aggagctaaa gaactaattt 735240
ttataaaacc tctatctcga atttctcgag ctacggggcc aaaaatcctt gttcctttag 735300
gatttccttt atcatcgata attacacagc tatttgtatc aaattttaaa gtagacccat 735360
cttttcttgt aatatgccga cgtgtgcgca cgatcacagc tttgataacg tctccctttt 735420
tttatagaac tattaggttc gacatctcta actgagcata caatgacatc tccgacagta 735480
gcataacgtc tacgagaacc tccaagaacc ttgaagcatt ttactttttt agctccagta 735540
ttatcggcaa cttttaactg actttcttgc tgaatcataa tctctatcta cctaatgttg 735600
ctaacttacc acgccaacat gttctataac gcgccatctt ttaagcttag acaaaggtcg 735660
tgtttcttga attttaactt tatccccttc agaaactttt agttcagtgt gagcgtaata 735720
tttctttgaa cttctcacta ctttaagata ctgaggatga gaaaatatcc tttctactcg 735780
aacaacgaca gttttttcca tttttgccga gacaacaaca ccaatcttaa cttttctaga 735840
gcctcttggt tcactagcca tggactttac cttttctttc ttgttttact gttagagctc 735900
gagcaatatt tttcttatgc gtagaaaaca tatgaacttt cacaactttg ttttgcagta 735960
aattttctgc tcttaaagca aacaaagcct ttttgttttc atgaacatac gcatctaaat 736020
cgtcgtcgct tttgcccctt aactgggtca ataaatcctt ttttagcagc catgtttata 736080
ccctttccac tcgcttaaca aaacgtgttt ttattcctag tttggcagca gctcttcgga 736140
gggcatcttg agcatcttct ttagagacat tcgctacttc aaacaatata cgtcctggac 736200
gaactactgc tacccaatga tctggggccc ctttaccttt acccatacga gtttctgcag 736260
gcttttttgt tacacttttg tctggaaaaa tacgaatcca tacttttcct cgacgtttta 736320
aatatctatt aatcgcaacc ctgcaggctt caattttacg actggtgacc aagccacgct 736380
caagagtttg cattgcatat tctccgaagt ctacaaatgt agctccctta cttaatcctg 736440
caaactgtcc tntttgttgc ttacgaaatt ttgttcgttt aggcattaac ataataattc 736500
actcatcctt atttacaact ttaagcagct gcagagggtg ctgccggatt gttaggagtt 736560

gtagaagagg agttctctcc aagattaatc caaactttta taccgataat tccgtaggta 736620
gtttccgcac aagctgtagc ataatcaatg tcagctctta gcgtatgaag cggcacacgg 736680
ccatttttat accattcaga acgagcaatc tcggctcctg ctaacctgcc tgaaacttga 736740
atttttacac caacagctcc agcatccatt actgattgca ttgccttttt catagcccgt 736800
ctaaaagaga ctcgacgttc aatttgtctt gcaatgttat cagctactaa ttttgcattt 736860
agttcgggac gtttaatttc cgcgatttct aaccagactt cttttccggt aagagctctg 736920
agctcttctt taagcagatc aacttcagcg cctttttttc caattaccaa cccaggacga 736980
gcggtttgga tggtcacttc aattttacca ctcatacgac ggacaacaaa tccagcggca 737040
ccttgacatg aaggtttttt tcttaaaaac tgtcgaattc ttacatcttc tatgagaaat 737100
ttaccaaatt cttgtttatt tccgtaccaa agagaacgcc atttttttgt aacccctgta 737160
cgaaatccga ttggacaacc tttctgaccc atgatccttc ttccctttta ccgttccttc 737220
tcaccaacaa taacagtcaa gtgactggta cgttttaaaa tgggagagcg tcctcctcta 737280
ctttttgatt tagatcgctt gtagacaggc ccggcatcta ctcgaacttc ggtaacgctt 737340
aaattttcac gctttatatt ttcatgcaat tcagcattag ctacagcgct atttaaaacc 737400
ttttttaaac atcttccagc tttcaactga gaaaatccca gctgttcctc agcctcttgc 737460
acacttagat ttctcataag cccagcagct aatctagctt tacgaggttg tacccgaata 737520
tatcgggcgg tcgctttaaa catgctatgt ctcctttaga cttacccttt cttcacagga 737580
tggcttttaa atatccttgt gggagaaaat tctcctaatt tatgacccac catagtttct 737640
gaaacaaata cggttaaaaa tttcttcccg ttgtgaactt caaacgtatg gccgatcatc 737700
tcaggagtaa tcattgaacg acgagaccat gttttgatag gagttttttt ctcctctatg 737760
ttcattgcac gcactttttt taggaggtgg tgatcaacaa acggaccttt tcttaacgat 737820
ctactcataa tccttatttc ctacgatctt taacaatcca tttattactt ttgttcttgt 737880
cacgtgtttt taatcccttc gtaacctttc cccaaggtgt acgtggaata taaccattat 737940
ggcgaccttc tccaccaccg tggggatgat ctacagggtt cattgcagta ccacggactg 738000
taggacgaac tcccatccaa cgtcttcttc cagctttgcc atctacacgt aggttgtgat 738060
cggcattgga aacttcacca atggtagctc tgcatccctc atttaacata cggaactctc 738120
cagaaggcat ctttaaagta acgtatcctg gagacttagc tataacttga gcagctaatc 738180
cagcagatct tacaagcttt ccacctgaag aaggacgcat ttcaatatta tgaactgaca 738240
atcctaaagg tatgcttttt aaagtcatac aacatccagg cttaaatgga cttccttcac 738300
ctgaaacaac aacgtctccc ctttggatgc ctttaggggc gagaatgtaa cgtttttctc 738360
cgtcttcgta gcttaagaga gcaatgtatg cagaacgatt aggatcgtat tctacagtaa 738420
ctactttcgc agtaatccca tctttattac gtttgaagtc aacgacccta tacaattgtt 738480
ttgctcctcc gccacgatga cggcaggata tatggcctaa attatctctt ccaccagaac 738540
tcttcttaaa gaaagagagc tttttatttg gtcgaagact tctttttgac ttcgtaccgc 738600
gcaactcacc acgcgttgtt aactcatcaa aagcgggaag gaccagctgt ctagttcctg 738660
gagttactgg tttaaatttt ttaaacatgc tgtttatctc tctaataaat tatccgacag 738720
agtgcccttg atagaaagtt acaattgctt tcttaaatcc tgaggttttt cctttacggc 738780
gccctcgaaa catgcgggcg ggttgaggtt ttacatttat ggtgttcaca ctctttactt 738840
ttacattttt atcaacgtaa attgcctcta aagcttgggc gattaatggc tttgttgcat 738900
catgagagac tataaaaaca aatttagggt ctttacaaaa gcttcccttt ttctttccct 738960
ctccagttcc agcgcttaaa tgctctaaca ttttagcttt ctctgttacg tagtgacgct 739020
taattacatc ataaggatct ttcatatcct aaaattcctt cttaatcttt cgtttcagaa 739080
acaagacgct caacaagttc ctgcaaagct tttttagaaa taacaatatt atgagcagaa 739140
gctaagtcat acccattgat attgattcca tagacaaaac ccttaactgc agtgaggtta 739200
cgcaaactta atcttaaatt ttcattcttc tctacatgat ctaagtgatc aataaagaga 739260
atgctacgac actcaacatt gcaatctttt aaaaacctta acgctgactg agttttagga 739320
gctgttaaag catctacaaa gaccgtatcg tcaactacag tcaatttgtt tgtttgaatt 739380
ttttgcgcca acaaaagcct gattgcggct tttctttctt tacggtttat acgtacgtgt 739440
tgattaaatt taggcttagg cccaaaaaca atcccacccc cacgaaactg aggagaagct 739500
aaacatccct gacgggaatt acccgtacct ttttgtttga aaggtttttt tgtagaatga 739560
ctcacttcag aacgatttct agtgcatgca gaccactgtc ttttattcgc gcgaatagca 739620
acaatataat ccttgatcaa ttgaaggcca tcgccctcat ctgcaaacaa agagtctgct 739680
acttcaactt cgcctatttt atttcctgaa aaatcaagat tttgataata aaaccattag 739740
gtcctctctg tatcacccct gtccgtatcc taagttctag aagaatgttt cactataaca 739800
atagagcctc gcgctccagg aatggcacct ttaactaaca gtactttttt ttctaaatct 739860
acttttatta cttctaaatt ttttacagta acgttctctg cgcccatatg actaggacgc 739920
ttacttccag ggaaacaacg accaggagtc gatcgcattc ctatagaacc tgcatgacga 739980
tgaaaccctg aaccatggct tcctggaccg ccacgaaaac caaatttctt catgacacct 740040
tgaaaacctt ttccttttga aattccacga acatcaactg aggaaacatc ttcaaaaact 740100
tccaatccaa aagcatctcc caaagacacc ccattaagag cctcttcaga acctcggact 740160
tctttaagaa aacgaaaaac tcgaccacca gctttacgca aatgtccgag tttaggctta 740220
ctaacacgtt ttgtaattgt atgagcgggg gcattcattt cttccgctcc tatttgcaag 740280
gagaaatatc cgtcgctttc cttagttttg atctgggtaa caacgttagg ctcaacgcga 740340
attactgagc aagcaaccaa ggatccatct ttatcaaaga tgtgaatcat cccttctttt 740400

tttcccatca cactaatatg agaccgcata aacttatcca ttaacaataa tgatttgctt 740460
tcctcttgga ggaacaaaga aacaaatctg cttagtattt tacttatcga attctctaaa 740520
aagaaaagga aattttccct taagaaaata aagggacagg ttatcagagg gggaagattt 740580
ttacaatagg aaaagtactc taggtactaa gaactcaaag ataaaagatt cttggaaaat 740640
tcctatttat agaaatagaa agtcgcttcc aaaaaatttt ttagaagcga cttctttaaa 740700
ataaagataa aactagctta tttgccagac tttgttttct gccatatgct atataagcca 740760
attcctgcgg atgctgccgt caaggctcca gaaattgcag cggagcaagc cactgtaatc 740820
ggtaaaggaa tgagtttgac tccatccact accaactcta aagctttttc aagaagagtt 740880
aaaacatttc tttttagagt agcaacgtgt tctttacgta atgccctttc cgcgcttaga 740940
atcacgtccc cactcaactg agattgttgg ttttctaagg cacatctctc attgtatagg 741000
gattccttcg cacgtttgaa cagcttttgt tctaatgcga ttccgccaac agcaccagca 741060
actcctgcca tatgattaat ggtcataatt ccagcaacag ccgtgccaaa attgcctaca 741120
gtcgctttgt ctaagaatgg ttttgctgtg agtttattga ttaacattaa cggacgtaaa 741180
acaccgaatg tcgctgagta agtcgttgca ccgactgccg tacaagcttc cctggaagct 741240
gccaataggt aatcacctcg agttaatagc attttactat actccccagg cgtttttctt 741300
tcttttgtct tgcttcctaa ctcgaaggct tgacgtgtgt attggtaaca cctctgagtc 741360
gagttaacga tagatggaac agctccattg agcacattag ctaaggcaac aacttctcga 741420
gttgttttta acataccctg ggcgtttctc gcggaggtca atgcgtcccc tgtgcatccc 741480
gcctcttcca gcgatcctac gacacagtca gaaaccgcct tacttagctt aaagcattta 741540
tctaaacttt ttgttgcccc tacaaacttt gctaacttat tcccagggtt tgtaaaaaaa 741600
ttgttgaata caataaatcc ctttctgagc aaaggagctg acagccgctg gttctttggc 741660
aactggcagc agcatcttac ttgctgcact tcctgccgat ctaattgcgt tagtcgccat 741720
ttttacctcc ttacaaggct ttttaaaaaa aaattagagc ctataataac ataaaacgac 741780
ttaatttagt ttaaatgccc taaataaaaa acacgttgta aagttaatta tttaaagtaa 741840
aaactatttt taatttcttt gctggatagc cgtttaaaaa ggattttgag ttcgtagaac 741900
ccttgccttc tacctgcact tcgtgcaagc aaatggctcc ctcagaacag gcaatagcaa 741960
gctcttgcct atctgttaca acaacagtcc cgggggctcc atatcttccc gcctctgcta 742020
aaagagaggc cttacggatc atcaagcgtt ttggcgcttt ttcagaaaaa gagaataggg 742080
tccacgctcc tggagctggc gtgactcctc gtatatgagc ataagcctct ttagcaggtt 742140
tatcccaagg aacctgtcct tcttccttag acaatttggg cgctattgtc gccaaagcag 742200
cgtcttgact gacgagttgc agttgacctg actcaatttg ttgtaaagtt tttattagaa 742260
cttctgcgcc ctgcgatgca agagcatccg ccaattctcc tgaagtcata tcaggaccta 742320
tggggacacg cgtaatattt gccatgtcgc ctgtatccat acctgcatcc atacggatga 742380
cagtatttcc agattctgta gctccttcca taatacagcg ttgtatggga gctgcgcctc 742440
ggtaggctgg caagagccct gcatgcagat tgtaacaacc ataacgagga atatcgagga 742500
ctatctgacg aagaatcgct ccataggcaa ccacaataaa aacatcagca ttaaaagccc 742560
gaagttcttc aataaattgg gggtctgacg ctttactagg ttggagtaaa ggtagaccgt 742620
gagttagagc tatagttttt actggagaag gaatgagttg tgccgatctt ttttggggct 742680
tatcaactcg ggtaacaaca gctgtaattt ggattttgtg atgcaacaaa tcttgcaaaa 742740
cggtggctgc aaatgtgggt gtgccgaaat agacaacctt aagattcaat caaaactcct 742800
tctttgtctg cgctttcttc ttcaagggct tgcttatcta tgcttcgttc gatgccgcgc 742860
ttacttgggc tttgacaaaa ctcaatgaag ttttttactt ctggaatgtc tccatattct 742920
tctagagttt cttctagaga ttcaaaaaaa catccgtcag cacgataaat ctttttaaaa 742980
gccttaatga gggctaatcg cgtagcaaaa ggaacctgcc tacgttgtag tcctacttta 743040
ttaataccag cgagttggta agggtttcca cttccaatgg tatagggagg aacatcacga 743100
cgaatcccac tgagggctcc taccatagca tgcgctccga tgcgaacaaa ctggtgcact 743160
ccaaccatcc caccaagaat agcgtaatca ccgacttgaa catgtcctgc aagttgtgca 743220
tggttactta gaacgacatt atttccaata gtacagttat gggcaacatg agcccagggc 743280
ataatcaaac aattattccc gatagagact gttgtacctt cgaatgttga agacgtgatg 743340
atagcgaact ctcgaatttc gcaattttca ccaatagtca cataggtttt ttccccttga 743400
tactttaaat cttggggttt attaccgatc attgcagagg gccatattgt tgtccccttg 743460
cctatggttg tattgccatc gatatatgcg taggacttaa caacaacgtt gtcacaaagg 743520
gttaccgtag cttttataac aacgtacggt tcaataacaa catcctttcc aattttagct 743580
cctggctcga taattgcagt tgggtgaatg ctcgccatgt ttctccgtga ttatatggat 743640
tccttatcta ccagagcaaa gctcagctct gcttcagtga ctagctgtga atctacacgc 743700
gcctgtgccc aagctttacc tcctttcgat gatattaaag aaaaatctgc ttgcaggtta 743760
agtacatctc cagggcgaac agcttgacga aacttagctt tctgtatccc taaaaataat 743820
gcgatacgct tattcctatc attttctaga actaagccta tcaatacacc agcagcttgg 743880
gccaaagctt ctaatatcaa gactccaggc ataataggcg cgttaggaaa atgccccata 743940
aaaaaaggct catttattgt gacatttttt tgtgccgtga tactgcgagc ctcgatgtca 744000
taagataaaa ctttatccac taataaaaaa ggatatctgt gggggagtaa atctaataat 744060
tcgcgtaatt tgatgacaga gggttgattc atttattctt tctttatagt tctaatgctt 744120
ccaaaatttt tttaccaaaa gcaatattgg aggagtggcc ggagccgaca gctaatacat 744180
gcgcaacaaa aggcctgcca actaaggaaa gatctccaat cagatccagt attttatgtc 744240

gtactggttc atcggcaaat cttaattgtc ctctactaat aataccatcg tccttaaaaa 744300
ctacagcatt atccaaacat ccccctccaa ttagcccctt ttccattaag aaacatagct 744360
cattgtatag agcaaatgtt ctacaaggag caatttcctg gcgaaaagac tcttcattaa 744420
tcaccaaaga tttgtattga gtccctattg ttgaactttg aggataatgc aacgtatagg 744480
aaatcttcag ctcatcagag ggaaaagctg ctaaaaaaat gtcctgatgt tgataatata 744540
caggacgtgt tagtctcgca atggaaaccn atatcttctt gttcacaaat acctgcttga 744600
tcgattaact caacaaagac atttgagctt ccatccccta tggggatttc ctctccacta 744660
cattggataa ttagattatc tatattgtta gatcgcaatg ctgccataag atgttcgaca 744720
gtggcgatta cagcactacc cctagataat gttgtacttc ttcctgtagt gtagacatga 744780
tctagtaaag cagggacatt ttcgtagtta cctgaggcag actgtctttg aaaaacaata 744840
cctgtatttg tttgtgcagg ttgcaaatgg agagttgagg acttccctaa gtgaattccc 744900
actccagaat agcgaacctc tcgctttaac gttctttgag ttcgttctaa catgtaaaaa 744960
cctgacaacg agtctccgta tattatcgaa tttctttttt agcaagcaag cgatatccta 745020
agaatcctcc tcctagataa ctgactgcac agaaagctat caaaatcata gggtaatctc 745080
cacaataccc ataaagcgtt ttataattaa atagaggcaa agaggtttcc aatacccctg 745140
agggggcttt agtttctcta gtatcataag gaagaatttt gagtattcga cctagagaat 745200
ccacagttgc tgtaacacca gtttggcaag ctcgcacgca aggcatccca aactcttgat 745260
ttctcaacat cccatggagg aaatggactt tagggagtcg tgattcagga taccatccgt 745320
catttgttaa gttaacaagg agttcggctc cttgtctctt gtaggattgc aaccgatagc 745380
cgaaagtttc ttcgtagcaa atggtgatcc cgatacgagg taaacctcgg acctgcacaa 745440
ctccagaacg tctacctgga agtctcttgc atcctagagc atatttagga aatagttgtc 745500
tacaaattag ggatccgaat ttccctcctg gtatatattc gccaccaggc acaaggatac 745560
gcttatcgta tcctacggaa attcctttgt gtgatattac ctcagcagag ttataccaat 745620
acaaaacgtt ctcttttttc acccaccgtt ctaagccaat aattactgga cactgaaagt 745680
gttgtgacag agctgtggca caatcactat tcgatagaaa tgccttacct tcgggaagtg 745740
gagcaaaaga agacaataaa tgtgcgcagg attcataggg atagacttgc ctatgcttac 745800
caaaaggcac gactacttct gggaaaatca gcaaatctat gggttgttgt attggggata 745860
cgagttggag gagttgttcc cagacgacta ttggggactt aagtttcggt cgtatggggg 745920
gatgcgcggg ttgaacaaca gcgacacgca gcgctctctt atcttgttga aacgcgtgtt 745980
taagatactc ataatgaatt gctccaaaag tatagggcaa aagaagagtg agcacccata 746040
acattttagc atgaggtttt ttcagtagta gacaataaaa gctcatattt acagctatga 746100
cagcgaagct ctgacctgcc caccccaaaa atccgccaaa ctgccgtcca taagcagagg 746160
ctgtcatagg ccaaccaaga taatcgaagg acatcccaga aaagatccca taaaatcgaa 746220
gcatctcgat agcgacccat acgccaggaa ggctccataa aaaagctgtg cgtttctgac 746280
gtacgattgc aactagaagg caagaaaatc ctgaaaatag aacggacaaa atcgtgatta 746340
atgtaagcca taccaaatag atgagtttgc ctatatattg atccgagagc atccaagaaa 746400
aatgaatccc ctctattgtg aagatccaga aaaaacagga tacaaaaaga gtccttagag 746460
gtaatgaggg tttttttaag ggttctagac tataccaaaa gaatccataa ccacaggcgg 746520
ctcctaatat ggaaacgaat ccacttaaat ctggttgagc aaaagctata aggcaccaag 746580
aaataacaaa gcaaaagatt cgtagcacag gctctcctta tttcagttca agccttgctt 746640
gacgtcgtcg atctgcttca ttataccgac gtttttcttc tggagtttct ggaacaatct 746700
gatggacagg aatgggctgg ttgtcttcgt tgacagcaac aaatgtaaag tatgcggagg 746760
taatatgacg tcgttcctgc ttataaatat tttctgccca cactttaacc ccgacttcta 746820
gggaagtacg ccatgttctg tttactgcag ctttacaaat cagattttcc cccatatatg 746880
caggagcata gaagcggagg gcatcaacaa aagcagtaac acagacggat tctgtgtgtc 746940
gttctgcgac cactaaggct aaacgatcga gcaaactcat taataatccc ccgaagacag 747000
tattattagc attaagatca ttagggaaaa ttttataaat atgtccgtca atacagctaa 747060
acgagacggg ttttttctta agcattgttg actctaccga aaggattttt atagggtatt 747120
cttggttgcg atagtatcgg tcaactgaaa gaaaatctat gatttctcnt cataagttac 747180
tgaaattttt gattattttt tagaaatcga agtgcttaca attccataaa gggattgtta 747240
acttgtgaaa atccggtccc cttttgtcta tgcttgattt catgacggat agaaaaaaga 747300
aggtctgaat cgaaatccat ttttgatgcc cattgaagat aggcaagagg aatttctgaa 747360
aaacatcgtc ccttgtgttt ccctaggggc atatatttca ttttaatagg ttttgctaac 747420
acctgtttca gttgttctaa cgttcggaaa cgtttacaaa gatgtttaaa aatattgata 747480
ttaatttcta catccttcat ggcacgatga ttcccatcat agggaacatt aaagtgtacg 747540
gctaaggatt ctagagaatt attaggacta tctccatatt cttttgctaa tcggagggta 747600
tcaataatgg tatactttga gaggaaggtc tctccgattc tttccatctc ttgagcgaga 747660
acctgcaaat caaaaccgac gctatgtcct acgatatagt cgccttcttt aaaaaatgct 747720
ttgatttgag gaaaaacttc ggcgattttc ggctgatctc tcaacatagc gttggagata 747780
tggtggactc tctgggactc cgcagataca acgcgttctg gattgattaa aaattctatc 747840
gaactaatta cactatcgaa agtaaagcga acagcggcaa tttcaataat acgatctttt 747900
tttacatcta gacctgtcat ttcacaatct aggcaagtaa aaaccgtatc ttttaataaa 747960
ctcataattt ccttctactt cctctatctt tgtttgatat ccttctgact gtagaaggat 748020
ttctgtgttt aactttatat tttgcatctg tgcagagaat gccttgcgaa cagaaaaggg 748080

ttccagggaa cctatactat tattacatcg cggctcactc ttctttatgc ctatcagtat 748140
gcaaggatac tatggagaat aaagactagc gcttctctat aataatttct ctttccatgt 748200
ttgtttgcat ggtaatgtag atatttattg tatcacaaaa tcgtggctta ggcaagcgat 748260
ccgcccattc aatacaaagg acatcgtctt cttctgcatc ttgaaaaatg tattcctgat 748320
ttttctgatc aatccggtaa agatcatagt ggcacaaccg cttaggttca ttaccataaa 748380
catgtaatat agagaacgag ggactagcaa cttcttccgc gatagtatct ccgagatatc 748440
cagagactat gccacgtaca aattctgtct taccagcccc ataatcacca aataagagca 748500
atacagctcc tggaacaagg acttgtccta actcagttcc tagtaatagg gtttcttgag 748560
aagaatggct tactcttctg tatctaccca ttggctaatg tacacatgaa aggctgtatc 748620
gtctgctaaa ctttctataa atgctgcgat cttatcttct accagcgcat cttgcaatag 748680
agaaaggaag gaactttcca attgaaactt agtttgattt tgcacttctt ccagggtcat 748740
aggacgcaag tagctagaga taaaatcctc taaaagaggg ggtaaactat ggaacaattt 748800
acctgtaatt gccgaagcaa acactgtttt tataataggt tcttttggtg ggactgcgta 748860
ttcttttatc acatcaggat cttctgatac gaggaaacgc ttgattcgca ccccaccctg 748920
tttttccata ttttgagggc aagaagacag ccagtcataa attgcgtctt gagggtttgc 748980
atagacgttg tcagcaaaga ccttaccggt aaatgggcaa atatagatac gcttcgtatt 749040
ttcgtttacc tgtggctttt cagaagaaat ttggatttct gtttctctcc aaatcttttt 749100
atcctgttcg agaatacgga ccgcatcttc nnggagtttt gaaaattatc ttgtcgcgaa 749160
caaacaccac aggacgaagg cttagagcct gttccagata gaaaagatac gttgccaaca 749220
attctggttt tttttgtttt cccaaaaact gcaaaagttt ctgtttgact gctccagaaa 749280
tatccatgcc tacccttttt cagctaatga cttaacgttc ttaagcaaac tccccataaa 749340
aggaatataa ccacatatgg gttgtcttac ctcacgttaa atcagaaata ctatcagagc 749400
cgagaattat gttgaacata aaggtatcac aacaaggttc atccaaaaaa atctgctgtt 749460
tttctttaaa aagtttctta attgtgtttt ttattatcta tagtctacaa ttttacgaaa 749520
cgcagctatg gcgaaatcgg tagacgcgct agattcaggt tctagtgagc ttatgctcat 749580
ggaagttcaa gtcttcttag ctgcaagaaa ataacaggga cagtaattcg atttttcgag 749640
aagggaaact tatggtaaag atcatatcaa gtgaaaattt tgactctttt attgcatcgg 749700
ggctcgttct cgttgatttc tttgcagaat ggtgtggccc ctgtcggatg ctcactccta 749760
tcttagaaaa tcttgctgcg gaacttcctc atgtcactat tggaaaaatc aatatagatg 749820
agaacagcaa gcctgcagaa acgtacgaag tcagctctat tcctacgctt attcttttta 749880
aggatgggaa cgaggtggct cgggtcgtag gtcttaagga taaagaattc ctaaccaatc 749940
ttatcaataa gcacgcttaa aaagacgctg caatattaaa ccgtaggatt cttttgcaat 750000
gctacggttt tctgccttac cacttcatat aaaacgatcc ctacactggt agctaaattt 750060
agagaacgga tgtcttgttg catgggaatg cgcaggcaat ttttataata tttttttaag 750120
atctcttttg gaagaccttt ggattcagat ccaaagacgt aggtgcctga agatggcaag 750180
gaaaattcag tataagatgc tgaacctttg gtacaaagac aaaaaatctg atcttcaggg 750240
acatcatgta gtgcttcttc tatagaatcc actactgtca attggagttt gtcccagtag 750300
tccatccctg cacgtttgac aaatttatcg gctaaagaaa agcccaaggg ccgaaccaaa 750360
atgagttcgg cgcctagggc tacacaagtt ctacctatat ttccagtatt ctgtggaata 750420
tcaggacaat gaagaactac tctcattcac cttgatttcc ttcttggggt acagcagcaa 750480
cttcatctgc tgaagcctga atcaagttaa tttcaaaaat taataaagag tttggaggaa 750540
gttgtcctgc ggttccgtaa gcaagatcag gatggatgta gagaactcga gtttctcctt 750600
ctttcatgcc ctgcatacct aaagcaaaac caggaattgt ttggcctaga ggaagcaaga 750660
taggctcatt gttgccttct gaactgctaa atacttggcc attgatgaag gaacccttgt 750720
agtgcaatag agctgaaggt ttacctgaaa ttgctttccc tgcaccttct ttaataattt 750780
tgtattgcaa tttacttggt tgcacttcaa caacaccagc gttcttgcta ttttctttta 750840
agaatttttc tgccaatgaa agatttcttt ttgatttttt ttcaaaaacc aacttctgta 750900
cttcagccat tttttcttca tactctgttt ctgttaaagg agcactttta caaaccaatt 750960
ccgcctgcaa ccccttagcc acttctgcaa tatcaaaaaa catatcttct gacttgcgta 751020
attggcgtgc taataaatga ccaaatgttc tggataactt ttgattatcg gataattcta 751080
tgtcattggt atctttgtta tctttatatt ccactaacga cccctgatcc ttgtctttat 751140
ccttagaccg tacgtcacaa gaagcgacgg agagtgccag agctactgtt gctaaaacta 751200
aattccaccg tctgttcatt ttccttctcc tatctttgat cttaaggcaa cgactatgct 751260
acatgcccga agcaattaca acttatatca aaaagctacc ttaatactta gctcttttaa 751320
ttgggaagac ataatttctg agggagcatt catcataaga tccgatgctt tctgtgtttt 751380
aggaaacgcg atgacctcgc gaatgctctc tgctgctgtt aaaaccataa ccaatcgatc 751440
taaccctaag gcaatcccta gatgaggtgg ggttccaaaa ctcaaagctt tgataaagaa 751500
tccaaatttt tcttggatgc tctcaggact tatttttaaa atagtaaata tttgactttg 751560
caagtctggg ttatgaattc tttgagatcc cgaagcaatt tcatatccat ttaaaaccaa 751620
gtcatagctt gacgaacgca ctgctagagg atctgtttct aacagaggaa tatcctcctc 751680
caaaggtgct gtaaacggat ggtgttccgc cacaattttt ccatcttcta aagagaaaag 751740
agggaaatct gtaatccaaa caaagttata ttgattgtcg ctatacaatt cgcgctcttt 751800
tgcgatcaat ctacgcagat gatcgagaga ctgattcgct actgattcag gagctgctat 751860
caagagcaaa atatcctgat ctttggcatc aaaataagca aagagttcgt ggaaaacctc 751920

ttcatccata aatttagcaa tattggaagc aactttcccc tcttgatttt taatccagac 751980
aagccccata gccccataac gctttacaaa ctcggtataa ccatcgagtt gcttgcggga 752040
catagtagcc ccacctggaa cacaaaaacc tttgatcgta cctccgtgag ccaactgatc 752100
taagaaaata gagaatgagg aacgttttgc atagtctcga caatctttta atttaagatc 752160
gaatcttaaa tctggtttat ctgtaccata ggaatcctta gcttcttgat aggtcatttt 752220
agccaaaggc aaaggaatct ctataccttg cgtagcaaac agtgttgcga ccaattgttc 752280
tataatggga agtaggtcct gggtatcccc aaagctcatt tcaatatcta tttgagcaaa 752340
ttcaggttga cgatctgctc gcaaatcttc gtctctaaag cacgtggcga tttggaaata 752400
acgatccaag cctccaacca ttaaaagctg cttaaaaagt tgtggtgatt gcggtagagc 752460
ataaaatttt ccaggataga ttctagaggg aacaagataa tctctagcac cttcaggagt 752520
ggattttcct aatacagggg tcacgatttc tgtgaatcct tgagcatcca taaagttgcg 752580
gcaagcaagc atgacctgat gacgacaaag caacttctca ataatatccc cgcgacgcat 752640
atctagataa cgatactcta aacgcagctc ctcattcaca ttgatgtggt catcggcaat 752700
ggaaaaaggg agattttgcg acttggatag cacttcaaag cttgcaactt caacttcaat 752760
atgtcctgtt gctaagttag gattttccat tcctgcaaga cgtggacata ctttccctcg 752820
tacagaaaga acccattcag aacgcacagc atccaaacgt tggtgcagtt ctggttgctc 752880
atcttcacgg cacacaattt gagtaatacc aaaacgatct cgcaaatnta tgaagacaac 752940
acccccatga ttacgataac ggtgcaccca tcctgccaat tgaacattct cacctatatg 753000
attacttgtg agttcattac aacggtgtgt tctgtatttc atataacaac ctttgctcta 753060
cttcttcttt tgttccaaag aattctttac gcagagacat atttttaata actaactgtt 753120
gagaaattaa ctctcgctca ccgattaagc aaacgaaaga aacctgttct gtactcgctg 753180
cttttagagc tccttttact tttttatgag accaatcgac ttccgtaggg attcctaacc 753240
gtcgcaaatg ttgcgaccat tccaaacaaa actgatccgc atctggttcc attggaatca 753300
aacgcagttt atgagggaac tgtggctcaa tacgcttttg agctaataac gtttgaatcg 753360
ctctttcaag gccaacaccg aaaccacagg caggaagaga agctcctcca aaagctgaaa 753420
tcaagccgtc atagcgcccg cctcccccta aggcataaga gacctcttgg aatgtggtag 753480
tcgcttcaaa gactaagtct gaataataat ccaaaccacg cactaaacga ggattgatag 753540
catatgggat ttctaaaact ctcaaagcgt ctaaaatttc attaaaatac ttaagatctt 753600
catcagaaac atagtctaga atcgggggcg cttgacggat aatttcttga tcttcaggct 753660
cctttgaatc caaaatacgc aaaacattcg ttgaaaatct ctgctggctt aatgccgata 753720
attctcccat ggactctttc aaataagcgc gtagaacctt atcgtatcga aatcttgtct 753780
cacttcctcc taagaaattg agttgaattt gcatatgttg caaaccgaca cgagagtaga 753840
aatcccaaag caaagcgaga acttctgcat ctcttagagg gtgacgcaca ccaatagcct 753900
caacaccgaa ctgatggtgt tgacgatatc tacccgcttg ctgacgttcg tagcgaaaca 753960
tgggaagaat ataatagaac ttattatcac ttcgatgaga ggccccgtgt tcaagaaaag 754020
aacggacaac agcggcagtc ccttcgggac gcaaagtcat ggaacgacct tttctatcta 754080
aaaacgaata gacttctttt ttaacaacat cactttcttc ccctacatgt aaaaacactt 754140
ctgatttttc aaaaatagga gtacgaattt cacaaaatcc ataaagcatg caaacggtat 754200
gaattgcctt ctcaacacta tgccaaagtg aagtgtgacg ccacaattgt ttagcatctg 754260
caagataagg aaatatatca aagacccctt tggggagagt tacagtcacg tgatgtcttc 754320
gctcaaaatg attacttttt tagataatgg aatatctgga gcttagtgac tcctgcctaa 754380
gcctaagtca tattcacact cgaatctacc ctctttttaa tgaagggcat ttcgcacgac 754440
cttgctagag ataatccagg agcgcgctac gcgcacgacc ataattatat gctagcttat 754500
ggtttttgta actaagtatt ggagaaaaac cgcgccctat gaatgctata aactattctt 754560
aaaattcttt attaagaagc atgtttacag acgaaaacaa aagataattt tgcttttttc 754620
tagatcgtta taacctggaa cttgttatag tttacatccc ttcattcaac aatcaaaccc 754680
acccttcata atgtaccagg caatcgggag aaataggctc gataacttcg tgtacaggga 754740
aaacatcctg aacaaagaac aaattatact ttgctctggc cgcttttaaa acagtcgcgt 754800
gagcgtcgtg ttccttattg gtatttcctt ttattggaac aagaaatatc cctatcaaag 754860
caatcaattt tttcattgca tgcgcctccc aaagttaatt atgtcacgat gagactttca 754920
aatctggagc agtaaaaata cgctgtttta gtatttctta tttaattaaa aataaccaag 754980
aaaaacactc tactaatgca tctttactct cgcaacctga aacgattccc tccttgtttt 755040
ttcatccttg atatggtaaa gtaggctttt cttcgcattt catctcagtg aaaaagattt 755100
tacacatgga aattgaaaga tgaacgtttg gactaaattt ttccaacctc caaagcacat 755160
taaagaaatt gaagaccaag aagtggtcaa gaaaaaatac aaatactggc gtattcgtat 755220
tttctatagc atgttcatcg gctacatttt ctattatttc acaagaaaaa gctttacctt 755280
tgcgatgccc acgctaattg ctgatttggg ttttgataaa gcgcaattag ggatcatagg 755340
aagtacctta tatttttctt atggaatcag taagtttgtt agcggagtca tgtccgacca 755400
atccaatcct agatatttca tggctatagg attgatgatt acagggctca ctaacatctt 755460
tttcgggatg tcatcctcta ttgtattatt tgctctttgg tggggactaa acggatggtt 755520
ccaagggtgg ggctggcctc catgtgctcg tctactcacc cactggtatg cgaaatcaga 755580
acggggcact tggtggagtg tgtggagtac ctcccacaat attgggggag cacttattcc 755640
tattctcaca ggattcatta ttgattatag tggatggcgg ggagccatgt atgttccagg 755700
cattctttgt attggaatgg gtttagtttt aattaatcgt ttacgagaca cgcctcagtc 755760

cttagggcta cctcctatag agaagtacaa gcgtgatccc catcacgcac atcacgaggg 755820
caaatcagcc tcagaaggaa ctgaggaaat cgaacgcgag ctatccacta gagaaattct 755880
ttttacctat gtccttacaa atcagtggct ttggttttta gctgctgcct cgttctttat 755940
ttatatagta cgaatggcag tcaacgattg gagcgcttta ttccttattg agacaaaaca 756000
ttatgcggca gtgaaagcca atttttgcgt atctctattt gagattggtg gtttattcgg 756060
catgctagtt gctggttggt tatctgataa gatttctaag ggcaatcgtg ggcctatgaa 756120
acgtcctctt ctctttaggt ttgctgtttg ctattttagg catgtggttt tcacgtagtc 756180
ataatcagtg gtgggtggac ggaaccttac ttttcgttat tggttttttc ttatacggcc 756240
ctcaaatgat gatcggtcta gcagcagcag aactctctca taaaaaagct gctggtactg 756300
ctagcggatt tactggatgg ttcgcttatt ttggagctac ctttgcaggg tatcctttag 756360
gaaaggttac tgatgtttgg gggtggaaag ggtttttcat tgctctctta gcctgtgcat 756420
ccatagcttt attgctcttt ttaccaactt ggaacgctac ggagaaaaac actcgtagta 756480
aagcctagcc gttcttgagg attttttttg acctggatac cccttcactg tcattctcaa 756540
tactctgttc ttgatgcaat gagctccatc aaagatttcg ttgcgaaagg tcaggaattt 756600
ggaattcccg ctctggctct aacagaccat gggaatcttt atggagctgt tgatttctat 756660
aaagaatgca ctcaaaaagg gatccaaccc atcattggtt gcgagtgtta tattgctcca 756720
ggatcacgtt tcgataagaa aaaagagaag cgtagtcgtg cagcacacca tctcatttta 756780
ttatgtaaaa atgaacaagg gtaccgcaac ctttgtattt taacctccct agcatttact 756840
gagggtttct attactttcc tcggatagac aaggatcttt tgagacagta ctctgaaggc 756900
ttaatctgtt tatctggttg tttatctagt tctgtttcag atgctgcctt aaaatctccg 756960
gaagctctgc ttcttgaatt gcaatggttt caagacctat tcaaagatga ttatttcaca 757020
gaagtacaac tacacaagat gtccgaagag agcattgcag gctttaaaga ggaatggtta 757080
aagcaagaat attactctct cattgaaaaa cagatcaaag tcaatactgc agtgttagaa 757140
gcaagtaagc gcttaggcat tcctactgta gctacgaatg acatccatta catcaatgca 757200
aacgattggc aagctcatga aatcctgttg aatgtccaat ctggggagac tgtgcggatt 757260
gcgaaacaga atactcatat ccccaatcct aaacgaaagg tctatcgcag tcgcgagtac 757320
tattttaaat cccctgcgca aatggcagag ttatttaaag atattcctga ggtcatttcc 757380
aacacattag aagttgccaa acggtgtgat tttacttttg atttttccaa gaaacactac 757440
cctatctatg tccctgaatc tttaaaaacc ttaaacagct acacggagga agaccgttat 757500
caagcttctg cagtcttctt aaaacagcta gctgaagaag ctttgcctaa gaaatactct 757560
tctgaagttc ttgctcatat tgctaagaaa tttccacatc gggaccctat cgatattgtc 757620
aaagaaagga tggacatgga gatggccatc atcattccta aaggaatgtg tgactatctt 757680
ttgattgttt gggacattat tcattgggcc aaagcaaatg gcattcctgt aggccctgga 757740
agaggttcag gagctggatc cgtattacta tttttgttag ggatcacaga aatcgagccc 757800
atacgatttg atttattctt tgagagattt atcaatcctg agcgtttgtc ttacccagat 757860
attgacatcg atatttgcat ggcaggacgt gaacgtgtca ttaattatgc aattgagcgt 757920
catggcaaag ataatgtagc tcaaatcatt acttttggaa ctatgaaagc caaaatggct 757980
gtcaaagatg tgggaagaac tttagacatg gccttatcta aagtgaacca cattgcgaaa 758040
catattccag atttaaatac tacgttgtct aaagctttag aaacagatcc tgacctacat 758100
cagctctata ttaacgatgc cgaatctgca caagtgattg atatggcgct ttgcttagaa 758160
ggctccatac ggaatacagg ggttcatgct gctggtgtga ttatctgtgg agaccagctg 758220
accaatcaca ttccgatttg tatttctaaa gactccacaa tgattacaac acaatactct 758280
atgaaacccg tggagagtgt tggaatgctt aaagtcgact tattagggct caagacttta 758340
accagtatca atattgcaat gtctgcaatt gaaaagaaaa caggacaatc gctagctatg 758400
gcgacactgc ctttggatga tgccaccaca ttttctcttt tacatcaggg aaagactatg 758460
gggatatttc aaatggaatc caaggggatg caagaattag caaaaaacct acgccctgac 758520
ctctttgagg aaatcattgc tatgggtgct ttataccgcc caggccctat ggatatgatt 758580
ccttctttta ttaaccgcaa gcatggcaaa gaaattatag aatacgacca tccccttatg 758640
gaatccattc ttaaggaaac ctatggaatt atggtctacc aagagcaagt catgcagatt 758700
gctggtgcat tagctagtta ttctcttgga gaaggtgatg tattacgacg tgccatgggg 758760
aagaaagact tccaacagat ggagcaggag cgcgaaaagt tctgtaaacg cgcctgcaat 758820
aacggcatag atcctgagtt agcgactgtc atctttgata agatggaaaa atttgctgcc 758880
tacggcttta acaaatctca tgctgctgcc tatggcttga ttacttatac aacggcgtat 758940
ctcaaagcaa attatcctaa agagtggctt gcggccttac ttacctgtga ttctgacgat 759000
attgagaaga taggaaaact gattcgagaa gctcagagta tgggcattcc gattcttcct 759060
cctcatatca atgtctctag caatcacttt gtagctactg atgaaggcat acgctttgcg 759120
atgggagcta ttaaagggat tgggcgtggt ttaattgaga gcattgtaga agagagagat 759180
catcatggtc cttatgagag catccgcgac tttatccaga ggtctgattt aaaaaaagtt 759240
tcgaaaaaaa gtatagaaag tttaatcgat gcgggttgtt ttgattgctt tgattctaac 759300
cgagatttgc tgttagcctc tgtagagccc ctctatgaag ctattgccaa agacaagaaa 759360
gaggctgcat ctggtgtgat gacgttcttt actttaggag ctatggatcg aaaaaatgaa 759420
gtccccattt gtcttcctaa agacattccg actcgctcta agaaagaact tttaaaaaaa 759480
gaaaaagagc tcttagggat ttaccttaca gagcacccta tggataccgt gcgagatcat 759540
ctttctcgtc tttctgtagt tcttgctgga gaatttgaaa atctcccgca tggttctgta 759600

gtccgcaccg tgtttattat tgataaagta acgactaaaa tttcatcaaa agcgcaaaag 759660
aagtttgctg tccttcgtgt tagtgatggc atcgattctt atgaactgcc gatctggcca 759720
gatatgtatg aagaacaaca agaacttcta gaagaagatc gtcttatcta tgctattctt 759780
gttttagata agcgcagtga ttctctacgt atttcttgtc gctggatgaa agatctttct 759840
attgttaatg aaaacatcat ttatgagtgt gatcaagctt ttgatagaat aaaaaatcag 759900
gtgcaaaaaa tgtcatttac aatgtcaacc tctggcaaag aaactaaagc taaagggaat 759960
aagcctaatg agaatgggca tacacaagct ttagctcctg tgactctatc tttagatctc 760020
aatgaactcc gtcatagtca tctatgtatc ttaaagaaga ttgtgcaaaa gcaccctggc 760080
tcacggacat tagttttagt ttttactcaa gataacgaaa gagttgcctc gatgtctcct 760140
gacgacgcgt atttcgtttg tgaagatatt gaagaactcc gtcaagaact tgtgactgca 760200
gaccttcctg tgcgtgtaat tactgtttga gattttctag acgctaggtt gcatgcttga 760260
agttctcaaa gacatgaggg catgcctatc tatctataga tcttgaaaag attttcccgt 760320
gactgcgttg atgtattcaa cacggacact attatctgca gaaactaggg agatcttata 760380
gacaggtaca tagaccatcg agctacgtac aattaaaaaa ttatctccaa agatcatctt 760440
cactaaatga cgcacacgat cttcggaata ctgcgcaggg aacacggctg cgtgacgtgg 760500
tttttgcaac cacacaggat tgttattgag tgttgttgta caccctcgga taggctggaa 760560
ctgttggaag tgcgttccta tttgattccc ggaggcaaag atcagttttt tcttctgaca 760620
ttccttaatt actcgctctg cacggatatt ctttagggct aaggcttggg ctaaggattc 760680
ccgggatgtg gttcccccta aggtagctag cgtttgtatc acccgcatat cttgcttacc 760740
agcgtgcaaa aggaggcact ctcgaaatcc cttagagcat gtccaagtcc ctgtatgcaa 760800
taccatctca ccattaacta agctccaaag gatttctccc tcttggctga taatatgctt 760860
tttgacaggt tcttctttag agaagcgtac gtgcataagg gtatgtggga tgaaggcgag 760920
ctctacaaaa gacttgccat cacgatattc tgggaacaat gccaacgcct gttctggaga 760980
gacgtgacgt tcaaaaactt gtaactcttc aaatcctgag atttttcgta caaagcggca 761040
gctcttagaa aatacttctg agctatggtc agcagcaagc cgcataattt tttgtgcaca 761100
aggaactcga tcccacaaaa aaacaccaca aaaaagagaa gcaacaaaag cagcaaaata 761160
tagaattaac ttcttcataa agtacctttt ttgtatttta cacaataaag aaatgtttta 761220
tcaaataaaa aaaataacaa gttataaata aaacaaaaac aaggcatttg acaaattctg 761280
tttttctttt ttatgatggc gttttgttgt tgtaagcccc cgtctaatta tgaaatttct 761340
attatacgtt ccacttcttc ttgttctcgt atctacgggg tgcgatgcaa aacctgtttc 761400
ttttgagccc ttttcaggaa agctttccac ccagcgtttt gagcctcagc actctgctga 761460
agaatatttt tctcagggac aggaattctt aaaaaaagga aatttcagaa aagctttact 761520
atgctttgga atcattacgc atcacttccc tagggacatc ttgcgtaatc aagcacagta 761580
tcttatagga gtctgttact tcacgcagga tcacccagat ttagcagaca aggcatttgc 761640
atcttactta caacttcctg atgcggagta ctctgaagag ttgttccaga tgaaatatgc 761700
gattgctcaa agatttgctc aagggaagcg taaacggatt tgtcgattag agggcttccc 761760
aaaactaatg aatgctgatg aagatgcgta cgcatttatg acgagattct aacagcgttt 761820
cctagtaaag acttaggagc tcaggccctc tatagtaaag ctgcgttact tattgtaaaa 761880
aacgatctta cagaagccac caaaacctta aaaaaactca cgttacaatt tcctctacat 761940
attttatctt cagaggcctt tgtacgttta tcggaaatct atttacagca agctaagaaa 762000
gagcctcaca atcttcaata tcttcatttt gcaaagctta atgaagaggc aatgaaaaag 762060
cagcatccta accatcctct gaatgaggtt gtttctgcta atgttggagc tatgcgggaa 762120
cattatgctc gaggtttgta tgccacaggt cgtttctatg agaagaagaa aaaagccgag 762180
gctgcgaata tctattaccg cactgcgatt acaaactacc cagacacttt attagtggct 762240
aaatgtcaaa agcgtctaga tagaatatct aagcatactt cctaagatag aaatcaatat 762300
gagattgttt tctttaggca cgatttatct ttttttttct ctagcacttt cgtcatgctg 762360
tggttactct attttaaaca gcccgtatca cttatcgtct ttaggtaagt ctttattaca 762420
ggaaagaatt ttcattgctc ccataaaaga agatcctcat ggtcagctct gctcagctct 762480
aacttatgag cttagtaagc gttcttttgc tatctctgga aggagttctt gcgcaggcta 762540
tactcttaaa gtagagcttc tgaatggtat tgacaagaat ataggtttta cgtatgcccc 762600
aaataaactc ggagataaga ctcacaggca ttttatagtc tctaatgaag gcagactatc 762660
actatctgca aaagtacagc ttatcaataa tgacactcaa gaagtcctta tagaccaatg 762720
tgttgctcga gagtctgtag actttgactt tgagcctgac ttaggaacag caaacgctca 762780
tgaatttgct ttaggccaat ttgaaatgca tagtgaagcc ataaaaagtg ctcgccgtat 762840
actatctata cgcctagccg agacgattgc tcaacaggta tactatgacc ttttttgaag 762900
gagaaaccgt ttttcctgca gtacttagtg aacttcatag catgttggac ttaatcaaac 762960
gtgcaggaaa acaatctaag tgcccccaag agaagttgtt aaagctcgag cttgcttgtg 763020
aggagcttct cgtcaatatc atttcttatg cttatcaggg cgaaaattct ccaggaacga 763080
ttgcgatttc ttgcatctcc catagaggag acttagaagt tgtgattaaa gaccatggac 763140
cttctttcaa tcctcttgct gtttcaatca acattcagga agatcttccc ttagaacagc 763200
gtaaactcgg gggcttaggg atttttctgg ctaaaagttc tgtggacgag tttctttatg 763260
ctcgtgaaga tcattgcaat attgtgcatt taaaaatgct caatggccaa cattcctaaa 763320
cctagtgatc gttattaaag cggaaagaac gcagagcatt ttcagtatct ttaatatcct 763380
taagatataa ctgcagggct tcgagaatca gcgacgactg attgtattgt ccagaggctt 763440

cgaaagccat ttctggttca tagtttcctt ctccttggga aaccgcaata tatccccgag 763500
tgactgcctg tccttgaaca agctcaggaa cgagccaaat agcaaaatca gcgttaaata 763560
atgtgagttt atctttagat tctgcaataa taaaatcgtt gtcagactgg ctacagagaa 763620
agtcagtgtt agcatcagtg caatgaggat aagagagtat ttctgcaata tttaagtatc 763680
cttcggggaa ggacgtccct gtattgagta ttgactgatg atagaactcc tcgaaaaaat 763740
ctaaactcat agtatatttc ctctgattta tggtaattct ttattttcag agccgtcaag 763800
tcctttctat tctgttgaat ttcctaataa cgtaagtaat aaacaatcaa aagtccgcat 763860
atgaaaagac ctttttttac ctatctatgc atcatcttct acggatcttg tgcatcgtta 763920
tctttacatg caggactctc tttcccagaa gtacgtggag ctacggctgc tgttgtccat 763980
gccgactctg ggaaggtatt ctatgataaa gacatagatg ctgtaatcta tcctgccagc 764040
atgacgaaaa tcgcaactgc cctctttatc ctaaagcact atcccacagt cctcgatact 764100
ctcatcaaag tcaaacaaga tgcgatcgct tccatcactc cgcaagcaaa aaaacaatca 764160
ggatatcgta gtcctcccca ctggttagaa actgatggat ctacaataca gctccatctt 764220
cgagaagagc ttttagggtg ggacctgttc cacgccttac tggtctgttc tgctaatgat 764280
gctgcgaatg tcttagctat ggcatgttgc ggatctgtag agaagtttat ggataagctg 764340
aacttcttct taaaagaaga aatcggctgc actcataccc attttaataa tccccatggg 764400
ttacatcatc cgaatcacta tactacaacc cgtgatctta ttagcatcat gcgttgcgct 764460
ctgaaagaac ctccatttcg aggggtcatc tccacgacaa gctataaaat aggggctaca 764520
aacctgcatg gcgaacggat cctatcccca acaaacaaat tgcttcttcc tgggtctacc 764580
taccactatc cccagcttt aggagggaaa acaggaccca ccaagactgc agggaaaaat 764640
ctaattatgg ctgctgaaaa aaataaccgc ctcttggtaa cgatcgcaac gggctattcg 764700
ggtcctgtga gtgatctcta ccaagatgtc attgctctat gtgaaacggt atttaacgag 764760
ccgctattaa gaaaagagct cgtccccccc tccgactgtc tccaattaga aatagcgaat 764820
cttgggaagc tttcttgccc tcttcctgag ggactctact atgacttcta tgcctccgaa 764880
gatcgcgaac ctctttctgt atcttttatt gcacatgcgg acgccttccc tattgaacaa 764940
ggagatcttc ttggtcattg ggttttttat gacgatgaag gcaagaaaat ttcttcccag 765000
cctttctatg ccccttgtcg ttttgagcgc actatcaagc cttggaaact ctatatgaaa 765060
cgtgtcttca catcgtatag aacctatatg tctataacca tgctgctcat gtattttcgc 765120
atccgcaagc accgcaagta taaaaattta aaacactatt ctaaaatcta actttttctt 765180
ttaatttata aaaaaccaaa ggtttatgta agatttgcgc ttttcaatcc aacaagaatc 765240
ccttgtgcgc acattacttt gctgtttact tgtcttcact tgtacttctt caagttatgc 765300
tcagatatcc tttcctaaaa aggattctct cctctctttt ttattgtctt ggaactctta 765360
gggaaaatct acagaaggaa aagcatacca ctgtttcctt aagcaagtgt ctattgcctt 765420
aaaccgagaa gaagtttggg ataatcccca tcacttaatg tttatcttaa tgcaattcca 765480
acaattttca ggggaacagg atcgttttgg aagtttctta gaagcaacca tccgtgatcg 765540
ggtctctttt ttagtcttac aagaaaagat tgccacttta aagtagcagt ttaaaactct 765600
gcataccaaa gatagagctt cttatcgtta ctttagggaa cggtaatgtt tgatttcttt 765660
tgaaagattc gggcgctcta cagcgcctct attctagatt ttttgaaaat gagaagtaaa 765720
aaatgcgtcg cccttaccta cttgtaaggg aagcgttttt ctgtggactt ctttccaacc 765780
taaagagtgc atgtaggcaa catgagcctc attttcttct tttaaaagag aacaggtaat 765840
gtagacaagt cgtccccgag gtcccacata agcacttgct tgttttaaga tgctcttttg 765900
cactcgcaca tagttcaaca acaatttctt agaaaattgc cacttatgtt cgggatgtcg 765960
tcggaaaact cccgttccag aacaaggagc gtctacgata actacagaaa aggatcctaa 766020
acgtaattga tccgctaaag aaaaattcct agctccagca cgtaataaac gatgctttgc 766080
agtttgcaaa atagctttac gactgtcatt gatcacaaca tgttttgctt tctgcgcaaa 766140
gataaggctt ttcccgcctg ctcctgcaca aaaatccaag acgatatctt tatctgttag 766200
ggaaatgccc tgagaaattc tctgagagtt ttcatcttgg atttcgaaaa acccacgacg 766260
aaatgcttct gtagattgta agggatgacg tttggaaaag tgcaatgcct caggaagctc 766320
tccaggagaa cttggatatt ctaacttctc ttggagttct ttgacggaga ttttatctgt 766380
attcacacga atcgtaatcg gagcttccgt taaccaaatc ttggcgatct cctcggcctg 766440
ttcttctcca taatcttgaa ctaagaagtg cgcaagatca tcagatatag agtaacgcac 766500
aggccaggga atagcgctgt aactgtccaa gttctctaga accccttcgt ttactttcgc 766560
aacgagagct tctggagtta cttgttctcc cgaatcaaga attaaagttt caagaagacg 766620
gcgatgacgc aaaatattaa aaataatatt ttgaatccac tgacgatcct tagaccccaa 766680
agaacggttt tgtttaaaat agtacgaaac tctatctgct tctgaaattg cagacgtgtg 766740
tagctgtttt aacagctgat aagcatgatg ctgacgaaaa ggaaccatag tgacatagag 766800
tatacctcgt catcacctat tttgcttgga aaatcttaca gaagacaaaa taaagagagc 766860
catcagtata gtaaaaaata tgcattacgg acgacgttgc cataatctca agaattgtct 766920
ccaaaaagaa ggatgcggat tatagtattc cgctgctttt cttaaggcta taccgatatc 766980
tctatcatag tctaacaacc aacgcataag taacatggta atcattttat cccacagggc 767040
gggattttgg tctcgaatac tataaatatt ccccacctcc tcaggagtgg tattgtcgga 767100
cttagataca tggagtttct tttctacgat gactttccgc aatacttctg caagaaagcc 767160
cagggctaat ttagaatctt caggggaggc tagggcagtc tcttgccata gacctacaaa 767220
agtcttccca tgaaatgtta aaggctggtt cccaataaga gagtttgcca atacctcaga 767280

tagagaatct gagataatac tcataataat cgtttggaaa tcctcggcac tcaaagaaaa 767340
aagctggatc agctcgcgta acaatacgta gctcttttct gataatgttt ttaggggttg 767400
cgctttttct cctataaact ggttgagcaa ttctaaactt aagggaagat acgccgtatg 767460
cccttcaagt aatgtggtta acgcttctat gatcatttgt agagaggcat ctgctgccaa 767520
tcttctttgc acctctccag ccagaggcgt ctgaacttct gataccgtaa cctcatgagt 767580
aaaatcttcc catgaggaaa aattactttt ccctttagag aacaccttat ttgcagcttg 767640
cacaatttta tcgacaggac gcttggactc aaaaagaatc aaagacttaa acatacatag 767700
tttgtctaca atggcttttt tgatgtgata cttggagggg aagaacagcc ggtagagcca 767760
tcctgtttgg ggtttttctt catgcaaacg caattcaata attgctaaaa gtcgactcga 767820
tatcgaataa gaaattgctg accaggaaaa gagctcgtga attacctgac gaatcgtgga 767880
tacgcttgta tcatagttgg taggtaaggg gtcattgacc ggccatgcat ctaagagaag 767940
tcgttttctg tcatatgcga gtatgcgcgt gccatcccta attgctagtt caagaacccg 768000
taaataatat cctatagaaa attgaaaatc taaaatcata gatagtggta cttagagctt 768060
ttctatgttt tattcttcag gagaaatatt atttcatcat atttatttcc tgaagttact 768120
ataggcaatc gttaacaatg agaactttta ttaaaaagtc ctgaatacta gtctttgggc 768180
tctattgatg tcagacgctc gctacagaaa aactgacaga tcacatagtt taatntcttc 768240
tgatatgcta ggggatgcga gcgatagtct tcttctaaga tagggagatt tacttctaag 768300
atagcgagaa cgacatcnta gtttacttct gaggcgtgct cttctgacca catcattaaa 768360
aagtctccga gctctttgct acgatcttga cgtactaaat agaggatgtc tttcataacg 768420
agagcttcgt tctctccttt atcggcaaac tcatcaattt gggaggcaca ggcaagtacc 768480
tgagtatagt tacgaccaag atcatatgtt tttaggttgt caacaacgct gacgcattgc 768540
ccagctccta aataaggaga ggtaaattaa tcttagtaat cccacggacg ttagctaacc 768600
catcaatacg tttgtctgag tataatagtt cggagagcct tttggagact tcttctactg 768660
ttgttgtagc ttctaaagaa aacgcattat acattcctgc ctcttcgatg acttctaaag 768720
gcagtccttc ttgaactgcg tccgtagcta cccacttgca gcaagaacgt aagattaaag 768780
caaatgcaac tgcagcttct ggacttatag gatctgtacc acaaaggtct aaaggagcct 768840
ctgggtgttg acgacgtaaa agagtgaata gccttaaaaa tacatgaaag aaaaagcttc 768900
ggcatgaagc tagtgctgtt tctaaagcat aaaagtatgg aggttccgca gtttctttag 768960
ggagtcgcgt tgctttgaga agatcacaag ggatgtcccc caaggaacgc actccctgtt 769020
cttttggatg gcgttttgtt ttaacatgca tccaaatctg cgatactgct ttacgaatcc 769080
gcgcaagcat cgtagttaac aggcttccgc gagcttcata ttctactcta tctacaaggt 769140
cgctatttaa atcactattc gtaccttcag atggagttgc ttctgggatc cccgtatccg 769200
ctatggtttc tatcacttgg gtagcacttt ccgatacccg atcctctaaa tctgaatccc 769260
cctcttctgc ggcactttct tctaaaggat ctgatatcgt attacggtca ccatcattgc 769320
cagatatagg agttcccatc gattaactac tccacaacaa tgcttctgcc ttttttattc 769380
aaaaagatcc ccgattaaaa ctgtcattgt attcgcccaa gctccgaccg ataacacttt 769440
cataccttga agtagtctac tcgactcatt ggccattcgc acaacgtcac gagtgtgatt 769500
ctttcctgca tttgcgattc ggacggaagt agcatgagag tgtacagccg caccgagttc 769560
tcgtgctaat gctatccata ttctaggcat ctcattagga tgattttcgc gtacccgatc 769620
taaagctgac cctaatcgag ctatatcact tgtataatct atgtcgctat catctccacc 769680
gaaatccgaa accctagttt ctaaaaagtt ttggatagat tctcctgggt ctaatgtagg 769740
tccttcgata cctaagctta taaccacttg catgagtaca ccatcaggat cattacctgg 769800
cttaggattt tctaaattct taattgcgcc tctaactgtc tgagttacag catccttagc 769860
tacagtcact gcttgcggaa ctggtcctgt agatgaagtc ccttcgataa gcatagattg 769920
ttctgtgtat gtttgctgca aagctcgagc aatgacacga taaccatgca aaagatagtc 769980
tggaccccta ttatttcccc aatccgtcca ctttgtttgt agctcttgag ataccttttt 770040
aagtactttt tgagactccg catgactatg gactgcatct tttaattcac tcacaagttt 770100
agcgacttca gaactcatgg cagtaggttg ctctgatcct gaagaagacc ccgccgtagg 770160
aggttgaggt gctcgacctt tcccatgcgt tgcgggacgt ttcgcattag ccccacccgt 770220
tgtaggaggt ggaggtgccg gacgcgtagg ccgtattgta ggtcctgaag gactttgcat 770280
tgcaggtccc gaagcaccct gagaagaatc gggtgatttc ttccctaaaa agaactccct 770340
taccgtctgc cataactgct ttgctttaga agcaacttgc ggctgtgagg ttgatgtagt 770400
tacgttgtga gcaccaagat ttgagtttgt agctcctgta ccttgggtac tggagctatc 770460
gccttgagct cctctaaccc atacatcatc tggtgatcta ttacccgaag gattgattcc 770520
cattgcaatt ctctatttat ttttcttttt taaatttaat taattttaaa aagaaaataa 770580
ttatgtataa acttttttaa attaattaat aattaactat tacaaaaacc actaaactat 770640
aaagatcgct tactaagacg tagaatgtgc gtactacctc tgtaagttcc gtaataccca 770700
agcccaatag agagaaagca ccccgccaat ctcttagaga gacaggcctg agtggttctc 770760
ttgttcaccg ccttttaaag gaaaacctta gtggctacat ttaatcttcg tcagaccagc 770820
tgacacgctt ctttcctgaa gtcccagact gcccaggctg tttcaaaatg cccttaggag 770880
gttgaggtgc tggacctttc ccatgcgttg ctgcgcgctt agcattggtc ccacctgttt 770940
taggaggctg gggtgctgga cccttcccgt gcgttgccgg acgttttgca ttagttccac 771000
ccgttttagg aggtgggggt gccggacgcg taggctttag tgtcgtgcta cctgatgaac 771060
gttgggcccc tcctccagga acctgtggga tagtgacttt catccctggt tggtaccctt 771120

ttttaattaa gttcctacca gctccctcgg tagcgcgagc atcccctccc ggcaacgggc 771180
tacgtacagt atctgcagat ggagcagagg gttgttggct acctcttgga gctcccgatc 771240
tgaagaagct catccgacta aaaaacccgg ttactgcttc tttgattcgt gctaataacc 771300
cttggcgtcc tcctgaggca gtcactctat gacttcctag gttagcactt gtaaccccgg 771360
attctttaac atcgggatgc tgatcatgag ctcccgtaat ccagagatcg ttcttggaat 771420
ttcctgatgg attaacagac attataattt tctattttta tttttcttta ttacttaatt 771480
ttaagaatta aattaattta tctataaaca ttttaaataa aattaattcg caattagaaa 771540
atataactat tctatttcta agagaaaaaa gacgttttta taataaaaaa catctaaagt 771600
tcagggagtt atctccagag gagtcctctg gtgaaaattt gaaaaatcgg aatgtattaa 771660
ggtttggaga attacaacct aaagatagac caaagcctgg gggaaagagt tacccacaca 771720
tagacaagca ccgcagctct ccatgttaag agagaagaag agcgcgatgc atcgtgttta 771780
cctaaacgac aacaagatac atctcttaac acgaattctt aagtatgttg atttaagagt 771840
actttagctt ttggatggag ataaaacttc tgtacctggg aggaagccct gctctagaaa 771900
ctctaaagaa gcccctcctc cagtagaaac atgggaaact tttgtagagc agcctgccaa 771960
agcaaccaca gctgccgcat ctcctccacc cacgacagta acagctgaag ggtgattgcc 772020
taaggcattc gctatagcaa tagatccaga gtcaaaagga gggacctcat aaacacccac 772080
aggaccattc caaaacacag tcgctgattg gtttataata cggataaatt cttcggttgt 772140
tctaggtcca atatcaaagc cttgaagatg cggaggaatg ccttgatcta tagaaatcac 772200
agaatattcc ttagattgga gattttcggc tgctttcaca tcgctaggca aaactatggt 772260
aacattacga cttttagcaa ttttcaatac atttctagca agatccaagg cagatttctc 772320
cacaagagag ttccctaggg attttcccaa ggcttgtagg aaagtaaatc ccatacctcc 772380
agctaataag aggtagtcta cttgattcag tagagcctct ataactccaa ttttagaaga 772440
aatcttagct cctccaagga tggcagtgaa aggcctctta ggggaggtca atagatgtct 772500
tcctaaaaat tccaattctt tttccataag caggcctgct gcggctctac ctgggaaagc 772560
ctgcggcact acatagactg aagcatgttt tctatgcgaa gttccgaaag catcgttgac 772620
atagaaatcc ccgtaggaag agagttctgc ggcgaacgtc gggtcttttt ctggatgttc 772680
ttctcctata tggaaacgca agttctcaag aagcaaaacg cgaccaggag aaagctgagc 772740
tacagcttga cgtgcaacct cacccacaca atctggagct agaggcacat gatgtcctaa 772800
gtatccttcg agaacatcca caacgggttg cagagaatat tcctcttgga acccctgtcc 772860
tttaggtcgc cctaaatgac tcattaaaat cactgcagca tgtttcttaa gtagatagtt 772920
gattgtaggc atcgcactgc gaatacgaat gtcatcgagt atcttgccat cttgcatggg 772980
gacattgaaa tctacacgta cgaggacttt tttttcttct ggagaaagat cttgtactgt 773040
tagcttatcc atatatcctc aagaacctac tatttgattt ttcttatttt taatggaaca 773100
aagctcttaa agcaaagaaa aagagtatgg ataataaagc tcctgcagga agcgtaataa 773160
accaggagag tacaatatct ttgataatgt ttaagttaat ggcacggatc cctcgtgcta 773220
aacctattcc taaaacagct ccaacaacaa catgtgtcgt agatatagga agtcctaaaa 773280
tagaagctaa agcaattgtt aatgctgagc ccatccccac ggaaaaccct cgagacgggg 773340
ttaactcggt aattttacag cctacagttt ctataacacg ccatccccaa atcgcaaggc 773400
ctatgaccaa gcctatgcct ccaaatgcca tgagcctaat taatgtatac gacgtatagg 773460
aagcaggata tgcctgacgc aagactccag ctacaggagc aatggcatta gcaacatcat 773520
tagatccgtg agcaaacgcc ataaagcaag ctacgataat ctgtaggtag gcaaagattc 773580
tttctacaac aagatacttt cttccataat ttccgcctcg ttctttcaaa cgataggtaa 773640
gactgccttt ttttggtgta tctgaaatgt aggaacagtg cttggtatgg acgtagtaaa 773700
acgtaatgat ataacttaga agtccacaaa ccagaacccc actaactgcc cagggagttg 773760
aagaaacctt aaggatcacg cccccagaga tcatcacggt tcctaaagtc atgatcacca 773820
aagctgctaa aaacggagca acacgaacca tagcaagaac aggatcattc ttataaaaaa 773880
tatggcgccg aatgaaagaa aagatcaggt aagcaacaca cccacccata aaaggggaga 773940
gaatccagct aattaaaata atgcctacgg aattccagta aatgattgtt cccttaccaa 774000
ggaccaatcc aaagccaatc acagctccaa ctatagaatg cgttgttgag acgggccaac 774060
caaaaaaaga ggccagctgc aaccacacgc ctgttgctag taaggctgcc gtcatgccgt 774120
acatatagtc cccagaggca atcataggat tggtcacaga aacgatacta ctttctatag 774180
tccctgcaac acgatctcca agaaggagag caccaaaaaa ctcaaagata gcagcgatga 774240
ccacggcttg tcgcaatgtc aataccccag atcctacact agggcctaca gcattagcga 774300
catcattagc tcctatattc caagaagtat aaaagccaca tagaaggaca aaaatgatta 774360
atggaagcat ggagtattac ttttcttcta gggtcatatt aattctatgt gcgagctttt 774420
cagaactatc tgaaatcccc gcagtgcgtc gaattacttg taaccaaaga taaaactctt 774480
tttcaggaat tataaaatca tcagaaaaaa atatttgcat aagttctcgt tgcaaaacat 774540
ccgattcatg ttcagattta gccacacgcc ctacaagcaa gcgtgcttta tctgccttcc 774600
tccccccaaa tgaactttca agcaattggt tgaattcatg tagcaatgtc atagttaact 774660
caaaagcttc tagatttttt tccaaaaatc ggaaaaaaag cgtttccata gatggataaa 774720
agtttaatcg tctgatggtt aataagatag caacatcttc agcagtatcc gcgatgctat 774780
cttgtataga aataatttct agaatccccg ctcgagatat cggcatgaat aatcctgcag 774840
gaagatgatt cctcatatca ttttttatac aatctgcttg atactcttta tcagaaacaa 774900
gttttgccat ttctaataat tcttcatatc ttccatctcg gagagcagtg aatataggaa 774960

gcatgtattc cacacaagag accaccattt ccagatgagc ttgtaaagga gcaaatggag 775020
attggccaaa tagacgagca agggtttgca taagaatagc ctttttcgca ataataactt 775080
gcctaaacga tcttgtaaac gacttatggc ttctaatccc attttacaga tagaggatct 775140
atccataacc ttggcaaaac aacgccaaca gtaccccatc gtccaatctt tatcgtttac 775200
tatcaatgaa ggacaaacct tagcaatcat tggagaatca ggatcaggaa aatctgtctc 775260
tgcgcatgca atccttcgat tacttccttg ccccccattt tctgtttctg gccaggtcaa 775320
cttccaaggc cacaacttac ttacggcttc gcgctctata caaaaaaaga ttatagggac 775380
agaaatttct atgatctttc aaaacccgca agcatctcta aaccccgtgt ttactattga 775440
acagcagttt cgagaaatta ttcataccca cctagcctta actgcagaag ttgctaaaga 775500
aaagatgtta tacgctcttg aagaaacagg gtttcatgat cccaggctgt gcttgaatct 775560
ctacccccac caactctctg gagggatgct tcaaagaatt tgcattgcca tggcgctcct 775620
ctgttctcct aaacttctta ttgctgatga acctacgact gctttagatg tttctgttca 775680
gtatcagatt ctacaattac taaaaacact acagaaaaaa acgggaatga gccttcttat 775740
tattacccat aatatgggag tcgttgcaga aactgctgat gacgtgctcg tgctctatgc 775800
aggacgcatg gtagaatgtg cccctgcggt tcaaatgttc cataatcctt ctcatcccta 775860
tacccgagat cttttagcat ccagaccctc tctacaaccg caacaactag gttccttcaa 775920
ccccattcca ggacagcccc cacactacac ggcctttccc tcgggatgtc gctatcaccc 775980
tagatgctca aaaattttaa atcgatgttc tgcggaagct ccagaaatct atccggtacg 776040
cgaaggtcac aaagtaaggt gttggctgta tgacgactaa ttttccccaa cctttaattc 776100
aagcaacctc attaacaaag cactattaca agcgttcctt ttggtttcag ggaaagacaa 776160
ttgccagtcg tcctgttgac gacgtctctt tttcactata ctccagacgt gctgtcggac 776220
ttattggaga atctggatca gggaaaagta ccctggcgtt agctctcgca ggtctcctac 776280
ctctcacctc tgggttctta acttttaacg gcaccccaat caagttgcat tctaaacacg 776340
gacgccatca attacgatct caagtacggt tggtctttca aaatccacaa gcttcattaa 776400
acccgcgaaa aactatccta gatagtttag gccactctct gctttaccat aaactcgtcc 776460
caaaagaaaa agtactagca acggtaaggg aatatttaga attggtaggg ttatctgagg 776520
agtattttta tcgttatcct caccagcttt ctggaggaca acaacaacga gtctctatag 776580
cgagagccct attaggagtc cctcagttaa ttatttgtga cgaaattgtt tctgctctag 776640
atttatctat tcaagcacaa attctgaata tgcttgccga gctgcaaaaa aaactcagcc 776700
tcacatatct cttcatttcg catgatcttg ccgttgtacg ctcgttctgc acagaggtat 776760
tcattatgta taaggggcaa attgtagaaa aaggaaatac aaaacgcatt ttttctgatc 776820
cacaacatcc ttatacgcgc atgttgttaa atgcccaact tccagagact cctgatcaaa 776880
ggcaatctaa acctatattc caagaatatc acaaagattc tgaagaatct tgctctacag 776940
gatgctactt ttacaatcgt tgtccacaaa aacaagaagc ttgcaagtca gagatcatcc 777000
caaatcaagg agacgcgcac catacatacc gttgtatcca ttgattcgtc ctctacgcta 777060
ttcttaagct accattaagg aatcccaagg gagaggtctg ctctatatat cctgagtgat 777120
gtttgacttt atgttagcta gagcattccg agctgcaaac acaattgtaa atacaaaaca 777180
aaagccattc catatccatg gaatggctac tttaagacct aacgcaacat ccattcttaa 777240
tataaaagaa gacgctctta gcctaggcac cgtgggatct ttcgcaagta ccgctagtat 777300
taagatagac tctcactgag tgtcccatgg ctggaaagcc aagcttccaa cttttgaaga 777360
tcctgagtat tttgcaaatg aaaagagact gtagcctttg atccccgggt ttttatctgg 777420
accttatacc cacaaagatc actgagacgt tgttgcaatt cttcatgctg ctttgacgac 777480
tctgccatgt ctaaaggagt gggcttcaac tctatcgaag agccctcttc acttataagc 777540
tgttcgctat cagttccgct tcacgtactg ccaaatgctc ttgtatgatg atctcattca 777600
gcttttccct aagtatagga tcttccagag tcaagatgac tttagcatgg ccgagagtaa 777660
tctgaccctg caacaagctt tcctggatcg tcttagaaag agcaagtaac cgcaaataat 777720
ttgctactgt agaacgtttt ttccctactt tataagcaac tntgtcctga gtgagtccaa 777780
agacatgaat taatctttta aaggcctcag ccatttctat agggtttaaa tttacccttt 777840
ggatgttctc aatcaatgta gcttctgcag cagtaccatc agcaatgaca tgcttgagaa 777900
tcacaggtat cgtagttgct cctgcgagct gcatggcccg ccagcgggct cgccagcaat 777960
aagctcataa tataacaccc gatctccagt gcaaatttca cgcactacag gaggatgaat 778020
caaacctaca gcttttatcg acgctattaa ttcttgcagc tcctcattag agaatactcg 778080
acgaggctga aaaggactca cacgaatatc atctatagct acttctataa ttgtatcctt 778140
actgatttcc tcagtcacaa tatctcctga tttttcctaa tagaattttg gatttgcaag 778200
catttgaaat cctggacgta cagggaatgc ttacagatca acgtaagcat atccaaatgc 778260
tgcataaaca taacagcatt gaaatttttc tatcaaacat ggttgtagaa gtgaagctct 778320
tttttaaaac ccttaagtaa aaatctgggc ttttctatcg ggaagtttag gtgctgtaac 778380
gacgggtaga gcttcctagg tcttctttgc tcgcagacat aaacagacga aaagaaaaat 778440
aactcttgat aaacacgaaa caacctcccc ccacgcatca tcgagtcttt agcttatgtt 778500
ctggaaaaga gaaaaaaatt gaatgcgaat gctaaattgc gataacataa agtgaaagct 778560
tcaaatcaaa ataggtttaa gccataaaac tccaaacgga agagcacaaa ctctagttag 778620
aggaagatcc ataaaaatct tcggcatgct tatagatttt caacgccaaa ctgtcgttcc 778680
tttatgcaaa agctagtcca taatatttgg aaaaaatttt attctttttc ttcggcaatt 778740
gccatttgta tcgttcttgc gtccttccta tccctaaaga ttgtttctaa cacgtataaa 778800

cattcccaag ccaaacgtaa tagcattctt ctacttacga gagcagctga agtcgctgtt 778860
tctcaaggat tccttccatc taaatctgcc ttgtcgtcat tggaacaagc ctatcatctt 778920
ggaggagaat ccatgaagcc ctatgcaggg tttttagctt cgtgcttcta tattcataat 778980
gagcctttac gtggagccta ctacgcagga ctcgcttata acaatagtca agcactgcag 779040
ctgccccacc ccattcaaaa actcctcaag gaaatttcag aagcacaagc tgatcaattg 779100
tatgatgttg ctttaagtaa atcctatcag ctcttacaaa ctgctaacag ctctcctgaa 779160
tatcctactc tatctttttt aaccctacta cgtgtgatcg aactcaaaga actcctccac 779220
caagatgtaa gtcaagactt tgcagcattg aaaagctccc ccctatttca ccaatttgaa 779280
cgcatgtata gcgatggaga atggacatta agcaaacgtt ttggcaaaaa aggataaaac 779340
tcttaaggac ctctatgtca gaatctctag aaatcccgga acttactgaa gtgctttctg 779400
agcagccgtc tctttctact cccgactctc cccctaaagt aatcacaggg accttaaccc 779460
tatacttcca agaagatatt gaccctgctt cttaaagcct tatgctcata gttcttgctt 779520
tccgacaggt ctttttttcc cactctcgtt cccagttaga ccgtctaaaa aattacctac 779580
ggctcctaaa acaaaacttt gctattaccc tccccaaaga acgaacctca aaaggacatt 779640
cgctaatgct cacttttgac ttcgcctcct ttgacttcta tacaaatatc tttcccttcc 779700
ttgaggaaca aaagattcct gctgttgtag gggtagcttc ccgatatatt ccatcaaatg 779760
ctgctcaaga ccttcaccct tcacatcgtt taaaaccctc tgaaactcta gcattccaag 779820
acgagatctt ctctaactac atgccctttt gttgccaaaa tgaactgata gaaatggcaa 779880
agtctcccta tatccaatta gcatcctcag gattcgcaat tcggaatctc atgaataatc 779940
ctccgtatct cactacagaa attttacttt cgcgacatca catagaaaca ataacaggag 780000
ccaagccctt ggcattcctc ttccccttcg ggaagtcaga tcctacaagc cggaagcttg 780060
ctgcagatca ctacccctat tctttcctgt tagggaatac cattaacaga aaattaaaaa 780120
ctcataacat ctaccgctta gacataaaac ctatgcagta cgtctgcccg agtttatttc 780180
agagctctag gtatttaaaa aactggatta aagagaaaag taaacagctg tatctcaaaa 780240
aacaacttcc aaaaagataa ccttaccacg tccaaaaaaa tagaagatcc tatcgcagac 780300
gggtcatcta cgaatcttat ctaaagaatc ctgcaaaaca aggatgaatt gatcgatatc 780360
atcctcatca ttatagattc ctaaggacac tctcaacaca tgacccacat tccatcgctc 780420
catagcaggt tgggcacatt gatgacccgt ccgcacagca attcctctaa gatctaataa 780480
aaaacctaga tccaaaggat gggctccatc gattgtcatg cctataagag ctcccctggg 780540
ttcctctata gaaggtccga gaatctctac acctggaatc tcaagcagct ctttatgtaa 780600
atatgtagtt agggcaatct ctttgtcgta gataaactta gctgacaagc catcgagata 780660
atctaaagca gcccctaagc ctaaaactcc agcaatattt ggagtcccag cttcaaattt 780720
cataggtgca ggaagatatt caggattctg atgatcgtag atagcaacca tatcaccacc 780780
tccttctact ggaggcaact gatctaatag atcttttttc ccatataaga ctcctatgcc 780840
cgtgggtcca taaatcttat gtgacgaaaa cacatagaaa tctacatccc aaagctgaac 780900
gtctatagga agatgaggag ctccctgagc accatcaaca gcaaggtaag cgtcatagcg 780960
gtggacaagc tcagcaactt gttggagagg ttggacacaa cccgtaacat tactcacatg 781020
aggaatgctt acaaattgag caccttcatt tagaagcttt tccaaatcat caagatctat 781080
aagccctgaa tcatgaactc tgatcttttt tactaaagaa cctcgccgcc gacaggaatc 781140
tcccaagata aaacattcgc atggtgttct gcctcagaaa ccagaacaac acccccctta 781200
gggatccaga ggtcattaac agaaatggct aataaattta accctgcagt tgtcccacgg 781260
gtgaatacga tttcactatc agaggctgcg gatacccact tacgcacttt ttcgcgaaca 781320
gctgcgtatg cttccgtgac gttcctagag gagctataaa tcgcacgatt tacagttgca 781380
tatgaagaag tataaaagtt agcaacggca tctatcacct gttgaggttt ctgagtcgtt 781440
gcagctgaat ctaaataaat aaaaggctcg ttctcttttg ctttagcagc aaaaatcgga 781500
aaatcttctt ttaaattctt cactgatcat ccccccccct aaatcctaaa cctaggaggt 781560
ttgatttagc tgaaaagaag atcccaaaaa cgtatcggaa actaatcctt gttttaggaa 781620
cccatgtatg agtttttcct gagcctctgc ctctgtcatg cctcgagaac gcatgtaaaa 781680
gatctgctga ggatctaaag gtcctactgt agcgccgtgg gatgccttca cctcgtctgt 781740
ttctatctct aaacgtggaa atgtagatac acgagcttct gaacttaata acaacgtatc 781800
atgcttttga ttcgcatccg acaagtctcc ttgagaagaa atagaaattg tcccttcaaa 781860
tagaaaatgc cctgaatata aaatcgattt aatattctga cgcgataccg tctcttcagc 781920
atcgtgagac attaaattat tgacccaagt ttttcttggc gattggacta acactagaga 781980
ttctgcgtgt cccttcttcc cgactatgta gctcgtattg tcaaaccatc cgaaaccttg 782040
acaactttca agcaaattct gagtcatcct gcagatagca tctttctcaa ccgtcgcaat 782100
agtggaccaa ctcaaggtat cctcttcaga atatcctgga accataaaca ctgtaagatc 782160
cgccccttct ccaacgaaga gctcggtaac cccattgact atagttttac tagaacccac 782220
catctctaaa tcaacatcat gtgatatttg tatttgagcg gacgcccgtt gtcctaaaat 782280
aacgacgatc ctaggagaga aaatcacatc atgatccgaa actgtaggaa aactaatatg 782340
acgtacaaaa ataggatcgc tcgtctgcat ctcttcagga atgtaaatca ctacaccccg 782400
atcttcagaa caaacagcat ttaaaaatgc taagggatgc ttatttacat caaacccttg 782460
catgaatgaa gacaatgatc ccctagcttc gtctatacca cagacaatca ccccctcagg 782520
caactgggat aacgaaggtt cgtattttcc attaattaaa atacactcaa aggctaaaga 782580
atggttattg tgtagccaat gttgcttaat tagttctgaa gctccagtcg caagattatt 782640

agcgatcagg aaaaagagaa agctcctgaa tccaagaaaa gctactaagc acctcttttg 782700
aagaaggctg cttgctatat tgagtgtagc aagcttccgc agctttttgc acaggagaac 782760
ccgaagcaat agaagaaaat gtctctattg aaactaacac cttatctcca cgcaacacgc 782820
ttagtcacct cttggtagct ttttgcttct aattcatgca tcaaagaaac gtctcctgaa 782880
agcgctactc gaccatctaa taaaagatga acaacatcag ggcgaatgag gtttcctaat 782940
ttggggttgt gagtcacaat gcataaggaa ctggtaggat gtaactctcg gtatttctcc 783000
aagactctac aaatcaaacg taatgcatct acatccaaac cagaatcagg ttcatctagt 783060
aagaccattt cgggttctaa aactagcatc tggcaaatct cattgcgctt tctttctcct 783120
ccagaaaaac cctcgttgac attcctatct aaaaatagat ctgtagtcgc gttatactca 783180
tatgtctcta atacagtcga aagcagagta ttaaactcat caatagaaat atctccctct 783240
tgattcgcac gacggcgggc attataggcg tctcgcaaaa acatcttgtt attgactcca 783300
ggaatctctg gaggcatttg aaaaccaaca aatagccctg ctcgggaacg ctcttctggc 783360
aacatagaaa gcaaattttg ctcctgtaat gcaatctcac ccgaagatac caagacactc 783420
tcatctcccg ctaaaatttt agcaagagtc gattttcctg ccccattagg tcccataatg 783480
acatgcatag ntccaggttg gatattcaaa ttgaaatcat ccagaatctt cacatcatta 783540
cagctagcat gtaagtgctt tatttttaac attgaaccta acccacgcta ttttctaatt 783600
taattaacaa taacttcgat gcttcctgag caaattctaa aggtaattgt tctataattt 783660
cccgacaaaa accatggatc actaagctga ctgcttcctc aggactcagt ccacgactac 783720
gtaaatacaa taactgatcc tcacgtaatt ttgaggtcgt ggcttcatgc tcaattgaag 783780
atgttgaatt ttctactaca atcttcggat ccgtataggc tccggaagcc ttgcctatca 783840
acatggagtc gcattgcgta tagttactac tatgttcagc ctttttccct aaggagacca 783900
aacttctaaa cgtgttctta gactcgtcag aagaaattcc cttagagatc accgtggatg 783960
tggtgcgttt ccctacgtgt agcattttgg tgcctgtgtc ggcctgcatt ttcccactag 784020
taagagctac agaataaaat tctccaacac tctcgtcgcc ctttaaaata caactagggt 784080
atttccatgt aattgcagca ccaacctcaa cctgtgacca ggagatctta gaacgatagc 784140
ctgcgcacag acctcgtttt gttacaaaat tataaatgcc gcctttccct gttttcttat 784200
caccagcata ccaattttgc accgtggaat accttatgac cgcatgctca tgagccacca 784260
attcaacaac cgcagcatgt agctgattag aagagtatgc cggcgccgta cacccctcaa 784320
gataactcgc atagccgcca tcctccacaa caatgagagt acgctcaaat tgacccgctt 784380
ccttgttatt aatccgaaaa taggtagaaa tatccatagg acatttcacc cctttaggaa 784440
cataaacaaa agagccgtca ctaaaaacag ccgcattcaa agcagcaaag aaattatccc 784500
gatgcgaaac aacggagcct aaatattttt ttaccaaatt cggatgttct tgaatcgctt 784560
cgcccaaaga acagaaaata actccggcct tttccaacgc ttctttaaat gtggttccaa 784620
tagagaccga gtcaaaaact aaatctacag caacattctc gacatttagt aagcgcttct 784680
gctcatctaa aggtatacct aattttttga acgtatctaa aatttctgga tcggcatctt 784740
ctaaacgtcc aagaggtttt ttctgcttag gagatgaaaa atagactata tcatcatagg 784800
ctataggacc ataatgcagg cgtgcccagg ctggctcatg caactgcttc caataacggt 784860
atgcttgtaa acgaaaatct ataataaact gaggttcatt acgtagagca gcgatttctt 784920
cgattgtctc ttcactaagt cctcgcgtca atccttgaga ctctatagga gtcacaaaac 784980
cgtaaggata gtcctcacgc tcttctaaaa aaacctttac tgattcgccc ataaccttta 785040
atcccgtcgc gcaaaactct tgaatttatc gtattaaatt acacgaggac aaagctaaga 785100
tagcacacag attatttttt cagtataaca aagaaggctt gaaagacgaa cctacccctt 785160
tcaagcccag aagaaaagag aaagaatttt ctaaaaatcc taactccctt ggagatcaca 785220
ccatcagcat ggaatacact aaggcgctta catgcaaagc cattctaggg aaataaaaaa 785280
tttttgtctt ctaatttcaa ttgaaaacac tatttcccta gtttattgct gtgacgtttt 785340
ttctacttaa catgccttaa gaaatagtat ctaaacgcct ttcaaattgc attgaggagg 785400
gatctgacac cacagtcttc tgcaataagg gagcaaacgt agaatccttt ttttgcaaaa 785460
ataaaagctc ttttgttgcc ttatcggtct ctccttgcat atgatgcaaa tagcccagaa 785520
gatagtgagc gcgctcatgt tctaaattaa tagacaacgc gctattaaaa gcctcatacg 785580
cttcccgcat ttgcttaaga tccaagtgag caagtccgac ataaaaatgt gcatcagcat 785640
cttcagcatt gagaaacaac gcttcttgaa aagccttcaa agctaatcga gttttatcta 785700
aagtgagata acataaacct aaattgtaat gaccatcaga caaatcaggt cgcagctgaa 785760
caacacgttc ataagcctca gtagccttgt cccatcgttt gcttcgagaa agcaaaaaac 785820
ctaactttac ccaagctttc caatataagg gattcttcgc tacagcaact tcaagcaaac 785880
gaatggactc cgcttcatcg tccatttcag aaaggatcac tgccttatta tataaacttt 785940
gtggattcca aggatcaagt gcaaggatct tatcaaaaca atctaaagcc tcttgaagtc 786000
tcttcaaacg atgatataca cttccaagac taaaccagca ctcaacatca tcgggatgca 786060
aagcaacata cgcactatac tgttcgatag ctgcttcata ttgattcccc cggtctaacg 786120
ctacaccata acaataacgg agatagctgt ctcccggctc ggatgctaaa cctttagaac 786180
accagttcaa agcctcggag actcttccag tctctaaagc aataatccct aaataacaat 786240
aagcgagtgc tgctgtagag tctaattcta aggtttcttt cagtcttttt tccgcttgct 786300
catattcacc gctcaaaaaa aggttaattc ctgaacacag aaattctttt gccaagtgtt 786360
tcgcagcttc ttccatggat atttctccca gcacatgatt cgttgatctc aactaaatga 786420
ggagcgaaaa ccatgccaaa agaaaactat ccccttaaaa aagattctct ttgttgaatt 786480

ttttcccaca tatcaatcat ttttaccgcc ataggagccg catctcgacc aaattctcct 786540
aagcgtaaat agactatgac tacgatcgta ggaagagata aatcttgatc agaaaaaccc 786600
accgcagcaa accagatgtc tttcattttc atggtaccat attcccgatc cagtcccaca 786660
cgcataatgg actctgctgt acttgtcttt ccaataatac gagacaaaag ttgtggagga 786720
aattgacttt gtattgctcg agctgttccg tattgacccc agataacatt gcgcatgcca 786780
gtcttaagca cctctactac agcatcaggc atgaaaatcg ttcgcttctt tttagaagag 786840
agataagaaa catgctcccc ctcccattct ccaagcaata acttggggac ataaaccaca 786900
ccgccattaa ctaaagaagc taacatcact gctgtctgca aaggagttac aacaagagta 786960
tgctgtccaa tcgctgttgc gtataaaccg gaacggttat acgccaaatc atgaggcacc 787020
ctacccgcat actctcctgg caatcctaaa cctgtttctt cgccaaaacc aaataaagaa 787080
gccgcgtctg ctaaatcttc aggatcccca agaccttccc ctaccaataa tgaaaagtac 787140
gggttgctag acatctctaa tgctgagact aaatcaataa agcctcttcc catgaaatca 787200
tttcccggca aactcccccc acggaaaaac gtgggaatcg gtgtgccatc tttaaaaaag 787260
cccacgtgag gcttagaact cctatagcca aaggaatttt tatcaataat gaccaaggga 787320
ttcgcaggct cctcattatg tccccataag atcctctgag ataacacaga atatgcagat 787380
actaacttaa aaatagaacc taaggtagct gcttgcccgt aggcatgagg acgtaggtat 787440
ccgtatccat atactggata aaaagaagca gctaaatctt gttctgtctg cctcttattt 787500
ctcacaatcg agatgggata tttgcctaat agaggacgtt gcaattcatt gaattcacga 787560
aatgtagaaa aaagtgcagg gagatgttct gaaagatgcg acacacgttc ttttagaaaa 787620
agataatgtt cgttccaaga taacgctcta tgcgctccat tatctaattc atttatccat 787680
aaatctaaaa tatcatagta aggctctaag ccttctttat aaggggtttt agaaaataaa 787740
tacgcaagaa atgtatccaa atgttcttgg cagaacattt tatattgcct tgttttttct 787800
tcctctaagt aatctacata aggtgtagga tacctctgtt tccttaatgc ttcctcttga 787860
cgtttcgcag caagatattg aagaaattca ctcttacgcc acgacttaaa atgcacctca 787920
ataaaagcat cttctaaaat agtagagaat gcagagcgaa gcaccacata acgtccttga 787980
agctctgtaa attcagataa cgagagccta tgaacctctg aaggaagtac aggagaaaaa 788040
cgctctggat ctacaatcag cctaaggata tccgtataca agattttatc gtaattcgca 788100
ggaagctcat taaaaacttg gtctaatgct tccttaagct cttcaatatc tgctttatgc 788160
tgattcagac attccatgat ccatttttgt tcctgaagag aaatgacttc ttggattaag 788220
atatgcccct cttcattagg gaaaactgca tcaaaaatag cggaacaagg acacgtcccc 788280
tcttcataag gaaataaaga aagcaaacga gtcaccaagt tttgaacctc tatcgcctgt 788340
cctacaaagc tgtttctttt taactgcaat ttaatcacag agttttcagg gaaaagaaaa 788400
tcaaggaagc aatcaaaggt taatggcaaa atctcttcat aacataatcc agtaagagga 788460
ttccttcttt cccgaattag agggactttc ctatcataaa tttctgcaat atgctcttta 788520
ttttctaacc atccagataa atagacgatc ttaccgcttt agaatcttcc gcaaccttcg 788580
cattcacaaa atcattgtta cgataacggg gagaagaagc catggctaaa atctctccgt 788640
tattaggatc taacgcaata atggctcctc ctttaatcca agggaacaaa ggaggaagct 788700
tctctcgttt cttcaatgac ttagcactac gaaacgtctc cgttttttca tattctaaaa 788760
gtaacgcatc cgcatacgct tgtagctcag cagacaaagt caactgcaat ttagttccag 788820
gagcttcagg aacagcaccc tccatttctt gaatgaagtt cccacgacga tctactaaaa 788880
tcggtttttt tccgatctta cctcgtaatt ttgagtccca acagtgcctc aacacccatt 788940
tttcctacta aagcatttaa actataagcg ttgctctcca cagactctaa taaagcacgc 789000
acctgatcta tacttgctaa tccttcaggc aacttaggat cttcaccctc ttcataagca 789060
cgcacacact cacgcaattg actcagctcc tgagtgactc tcttatactc ttgaagactg 789120
ataggtccta cataacctaa aatatctgaa gccacgcttt cttgaggata atgacgacga 789180
actacagcct ctacatgcaa tccaggccaa tctttagata acattttgag tttcaaatag 789240
gtgcgctcag aaacattagc agccactaaa taagggaccg aacctaatac agaagctttt 789300
gcatgaattg catcttcgat cgcctcgcga tctaaatgca attcctgaga taaaagctca 789360
gacaaacaca ttatataatg cttacgcaca ggaatgagct gtttatgccc atgctcatcg 789420
acacgccaag cccgagtagg caaatcacga atggccccat aagcaacgct cacatcatat 789480
tgcaactgat tcacagccaa tgtctttcca aaacgatcac aaatcgttgc tctttccaca 789540
tattgaggaa gcactcgaat ctgtggcttg tatgcctctt ctaacttttg ttcatgttca 789600
acaacagcaa gataccataa acgcaatgca atcacagcaa atgcaataac aatcccagac 789660
aacagtctgt tggtcttttg agcaatggaa agataaatag gaaatttttt cggtcgtttc 789720
atgctatgga tattatgagc ttcactctcg tctgaaaatt acagagaact atgctctcac 789780
attatttcca atcatagtct gctaaataca ataataaaaa gaagcacgat atttaagaag 789840
aatagtcaat ctaatcgaac aaaatctaga gaagatacag ttcgcaaaat acaactaatt 789900
ttagtccaat caaaacatat taaaaccaaa aatcatgttc taaataataa aaataaacaa 789960
aaaaatttct caagagaaaa agacttgaga aggtagtgag gagccatttt taaggggaac 790020
taaattatat atataatgaa aagaatatac aaaaaagcta tagctttcct atagctcata 790080
acagaagttc ttggttgaaa tatgcggcta aaaacactta atcttcttat cgtctttact 790140
ataataagaa aagtttgata tgttttcgac taatgagctg tatgttcata tttaaggccg 790200
tttttcaatg ataagagctt cctaaatttg cctgcaggat atcttgtctg gctttaattt 790260
ggacgtcgtg tcgccaaaat atgagtaata gcgagcacat aaataaaaga tactaagcat 790320

aatctttaga ggtgagtatg aaaaaactct taaagtcggc gttattatcc gccgcatttg 790380
ctggttctgt cggctcctta caagccttgc ctgtagggaa cccttctgat ccaagcttat 790440
taattgatgg tacaatatgg gaaggtgctg caggagatcc ttgcgatcct tgcgctactt 790500
ggtgcgacgc tattagctta cgtgctggat tttacggaga ctatgttttc gaccgtatct 790560
taaaagtaga tgcacctaaa acattttcta tgggagccaa gcctactgga tccgctgctg 790620
caaactatac tactgccgta gatagaccta acccggccta caataagcat ttacacgatg 790680
cagagtggtt cactaatgca ggcttcattg ccttaaacat ttgggatcgc tttgatgttt 790740
tctgtacttt aggagcttct aatggttaca ttagaggaaa ctnntacagg ttcaatctcg 790800
ttggtttatt cggagttaaa ggtactactg taaatgcaaa tgnactacca aacgtttctt 790860
taagtaacgg agttgttgaa ctttacacag acacctcttt ctcttggagc gtaggcgctc 790920
gtggagcctt atgggaatgc ggttgtgcaa ctttgggagc tgaattccaa tatgcacagt 790980
ccaaacctaa agttgaagaa cttaatgtga tctgtaacgt atcgcaattc tctgtaaaca 791040
aacccaaggg ctataaaggc gttgctttcc ccttgccaac agacgctggc gtagcaacag 791100
ctactggaac aaagtctgcg accatcaatt atcatgaatg gcaagtagga gcctctctat 791160
cttacagact aaactcttta gtgccataca ttggagtaca atggtctcga gcaacttttg 791220
atgctgataa catccgcatt gctcagccaa aactacctac agctgtttta aacttaactg 791280
catggaaccc ttctttacta ggaaatgcca cagcattgtc tactactgat tcgttctcag 791340
acttcatgca aattgtttcc tgtcagatca acaagtttaa atctagaaaa gcttgtggag 791400
ttactgtagg agctacttta gttgatgctg ataaatggtc acttactgca gaagctcgtt 791460
taattaacga gagagctgct cacgtatctg gtcagttcag attctaaaga tttgcttaga 791520
atttctcctc accttgttat cagagtctac atgttaggct ctgatttatg ctcagagctt 791580
cttaatttct gagcaatttt tattcccccc ctacttcaca tcacatcaag acaaatgaat 791640
tatttactta tgctattttc taatagcttc ctgtagatta cacgcttgcg ttaaaagcat 791700
tattacacta ctatacccct taatccagtt tgcgcccgta gctcaatggt agagctgtag 791760
ccttccaagc taccggtgtc agttcgattc tgatcgggcg ctttctttac acaaccaaga 791820
ctgaaattct ggcttttatg tcagaatgcc gttgttaacg tattctaatt ttgaaataga 791880
ggtacaaagc ttggaatccc aatcctgcaa acttacaatt aaagacctta tgagtgccgg 791940
tgctcatttt ggacaccaaa ctcgaagatg gaacccaaag atgaaacttt acatctttga 792000
ggagaaaaac ggtctttaca tcatcaatct agcaaaaact ttacagcaat tacgcaatgc 792060
tcttccccac attcgcaaag taattcaaga caataaaact gtcctattcg taggaacaaa 792120
aaaacaagca aagtgtgtca ttcgagaagc tgcaatagaa gctggcgaat tttttattgc 792180
tgaacgttgg ctaggcggaa tgttaaccaa catgacgact atccgaaatt ccattaaaac 792240
gttagacaaa attgaaaaag atttatctag aaatcaggcc tatcttacta agaaagaagc 792300
agctctttta gctaaacgtc atcaaaaatt attgcgaaac cttgaaggga ttcgttacat 792360
gaagaaggct cctggtcttc tagttgttgt tgaccctagc tatgaaaaaa ttgctgttgc 792420
agaagcaaaa aaactcggaa ttcctgttct tgctctcgtc gatactaact gcgatcctac 792480
tcctatcgac catgtgatcc cctgtaatga tgactctctt aaaagcattc gattaatcat 792540
caatgtgatt aaagaaaata ttatcgaggc caaacataag cttggtatag aaattgtttc 792600
tccagtgaaa tctttagaag tgcccgatct ctcagctttc gaatctagcc aagatgacga 792660
atctgacgaa gagaatcgag aagaagatct attagcaaaa aaatttgatg gcgaggcaaa 792720
ctaatgagcg acttttctat ggagacccta aaaacattaa gacaacaaac tggtgtaggg 792780
ttaacaaagt gtaaggaagc tttagaagct tgcggtggta acctagaaga agctgttgtc 792840
tatttacgta agttgggatt ggcatctgct gggaaaaaag aacacagaga aactaaagaa 792900
ggcatcatag cagctaaaac tgacgccaac ggcactgcat taattgaagt gaacgtagag 792960
acagattttg ttgcaaacaa cgcagtcttt agagaatttg tttccaatct acttaatgac 793020
attctcaaat acaaagtaga taccgttgaa gccctatcgc aagcagcctc gtcccaagat 793080
ccctctcttt ctgtagacga actcagagca gtgactatgc agactgtagg agaaaacatc 793140
cgtattagta gagtggcata ctttcctaag gctacaaatt ctactgtagg aatttattcc 793200
catggcaacg gcaagacagt agctctgact atgctttcag gctcctctac tgctgacagc 793260
ttagcaaaag acattgcaat gcatgttgtt gctgctcaac ctcaattcct cagtaaagaa 793320
agcgttcctg ctgaagctat tgctaaagaa aaagaagtga ttgcttctca aattcaagga 793380
aaacctcaag aagttattga gaagatcgtt acaggaaaat taaacacatt cttccaagaa 793440
gcctgtttat tagaacaacc atttattaag aacgccgacc tttctattca aagtttaata 793500
gatgatttct ccaaaacctc tggaagctct gttgcaatag aacagttcat tttatggaaa 793560
ataggagcct aataaaaaca tggctaagca aactagacga gtcttgttta aaatttctgg 793620
ggaagcatta tctaaagatt ctagcaatag aattgatgaa atgcgtttat cccgactggt 793680
atcagagcta agagcagttc gtaataatga tatagaaatc gcccttgtaa tcggcggtgg 793740
caatatttta agaggactcg ctgagcaaaa ggaacttcaa attaatcgtg tatcggcaga 793800
tcaaatggga atgctggcta ccttgatcaa tggtatggca gtagcagatg ctttaaaagc 793860
tgaggatatc ccttgtcttt tgacatctac cctatcgtgc ccacagttag ctgatcttta 793920
tactccacaa aaatcaatag aagctttaga ccagggaaag attcttatct gcaccactgg 793980
agctggttct ccttatctga ctacagatac tggagctgct ttacgagctt gtgagcttaa 794040
tgttgacgtt ttaatcaaag cgactatgca tgtagacggt gtctatgata aagatcctag 794100
gctctttcca gatgctgtaa aatatgattt tgtttcctat aaggattttt tgagcaatca 794160

actaggggta atggatgcat cagcaatttc cctatgtatg gattctcata ttccaattcg 794220
tgtctttagc tttttacagc actctctaga aaaggctcta tttgacccta cgattggaac 794280
attagttagc gaggatgtaa accatgtctg ttctccaaga cactgagaaa aaaatggctg 794340
cggctttaga tttttttcat aaagaagtaa agtcctttag aacaggaaaa gctcatccag 794400
cattagtaga aactgttgta gtcgatgttt atggcactac aatgcgtttg tctgatatcg 794460
cttcgatttc tgttgcagat cttcggcaat tggttatttc tccctatgac gggaacaatg 794520
cttctgccat tgcaaaagga attattgcag cgaatttaaa cttacagcct gaagtcgaag 794580
ggtctattat tcgtattaag gtccctgagc ctactgctga ttaccgacaa gagatgatta 794640
agcaacttcg ccgcaagtgt gaagaagcta agatcaacgt tagaaatatc cgcagagaag 794700
ctaatgacaa gttgaaaaaa gactcggctc ttacagaaga tgttgtcaaa ggtaacgaga 794760
aaaaaattca ggagttaact gacaagtttt gcaagcagct tgatgagtta acaaagcaaa 794820
aagaagctga aatagcttca atataagtat acttagggtt tttcttttcc ctctgacttt 794880
tttagtcata gagagggaaa aagattgctc taaagagaga aaattagtaa catttattcg 794940
tcttggcccc atcgtctagc ctggcccagg acatcggatt ttcattccgg taacaggggt 795000
tcgaatcccc ttggggtcaa agtataaaat taacaagata tttcgggtct ttagctcagc 795060
ggttagagca cctcactttt aatgaggggg tcgaaggttc aaatccttca agacccattt 795120
aatgattctt gttaacttta tcttttctaa aaaaaatctt tttcccctta gttctatttt 795180
tgctatgtac tgagtaccga gcttaatgga acttaactca tggttcactc acctacccac 795240
caatgttatc attgtcaaca gcctgccacc atatgctata cagaaataga taaggataag 795300
gttatacgct cttatgtatg cgcaacatgt ccttgtccta gccattacta taataatgag 795360
cacctgagtc tatctaaagg ggttggggtt ctcactttag agtgcggcaa ctgtaaaacc 795420
gtatggcatt caaagcaaga cgacgaacaa ctgttaggct gccaccaatg ttatacaaat 795480
ttcaaaaatc agattaccag caaactcaaa agtgagagag tggtatcttc atcctttact 795540
atggagaaag gccaaggctc tcttcatata ggtcgagccc ctggggaagc ttccaataca 795600
aatcctcttt taaaacttat agcattaaat gaagctttac aagatacctt agaacgagag 795660
gactacgagc aagcagcagt aatccgagat cagattaatc atttaaaaac caaaaatcca 795720
gatgaccctt cctaatgatt tactagagac cttagtaaag agaaaagaaa gtccacaggc 795780
aaacaaagtg tggcctgtaa ctacattttc tttagctaga aatctctctg tatctaagtt 795840
ccttccctgt ttatctaaag aacagaaatt agagattctc caatttatca cctctcattt 795900
taatcatatt gaaggctttg gggaatttat agtgcttcct ctaaaagaca ctcccctatg 795960
gcagaaagag tttctacttg agcatttttt actcccttat gatttggtgg ggaacccaga 796020
aggtgaggca ttagtagtta gcagatctgg agacttctta gcagctataa attttcaaga 796080
tcatcttgtt ttacatggaa ttgatttcca aggaaatgtt gagaaaactc ttgatcaact 796140
tgtacaattg gatagttatc tccatagcaa gttatctttt gctttttctt cagaatttgg 796200
atttttaaca accaatccta agaactgtgg gacggggtta aaaagccaat gttttctgca 796260
tattcctgcg cttctatatt ctaaagaatt taccaatctt attgatgaag aggtggagat 796320
aattacttct agtttattac taggggttac aggatttcct ggcaatattg tggtattatc 796380
gaatcgttgt tctttagggc tcactgaaga actgcttctt tcttctttaa ggattactgc 796440
ttccaagctc agtgttgctg aggttgcagc aaaaaaacgg ctttctgagg agaattctgg 796500
cgatttaaag aatcttatcc ttcgttcctt aggtctactt acccattcct gccaacttga 796560
gctgaaagag actctagatg ccttgagctg gatacaactg ggtatagatt taggcttgat 796620
taaagtaacc gaaaatcatc ccctatggaa tccattattt tggcaaatac gtcgagcaca 796680
tcttgccttg caaaaacaag ctgaaaactc ccgggatctg caaaaagata cgatttcaca 796740
tttaagagct agcgtattga aggagttaac taaaggatta tctcctgaga gtttctgata 796800
aaattctgaa gatatatttt taaagagaga tgcccaaagc cgggatcgaa ccgacgacct 796860
acacgttacg aatgtgttgc tctaccaact gagntattta ggcatgtcgt aggaagtagg 796920
gaacaaaaaa gccaagaaaa cataaagtta tacggccgac tcttacatct tcttggcttt 796980
ccccctgaat gcaacagagt cagaagctat tccacggcgg agaatatcat cttcctcttt 797040
tatataccac tactgagctt ctatttctat tattttaaag tcactttcag cctgtaaaga 797100
aggaggtcct tggcattcag gagcatggcg aggggccact tgcatggatc cttcatcagc 797160
ttcccaattg tatctgtatc cttccttatt ggtaacacga gtgccttctt caaagctact 797220
aatcacctta ggcttaataa acatcatgat attgcgtttt tgcctttggt cgatggtacg 797280
gctaaataaa ccacgaatta atggtatgga gtttagcaaa ggcactcctg aaaccacttt 797340
tgtagtttta tctctgatat gcccactcat aactaagaaa caaccgtcgg gaatttgtaa 797400
gcgtgtggct gcataagttt tatctgtgac aggtgttagt gatccagacg cggaatgtaa 797460
ttctgagatc gtctgttcga tttgtagtgt aactacattg ttgggagcaa ctgtagaggt 797520
aacgacaagg ttcactccaa tatcttcata atcgatattt tgcgttacag ttcctgtttc 797580
ttggataata gtatttgtag tttggtaagg gaccgtttgc cctacaaaaa acgaagcttg 797640
ttgcgtatcc tgagccatga ttctaggatt caagacaatg acagtatctc catcttgatc 797700
taaggcactt aataagcctc ccaaagtaag gaaagacttc cctttatgac ttaggacatt 797760
tccgatgatt cctagaccga atgctgacga agagttcagc atatctgaga accctgtcaa 797820
ttgtcctggc gtaggaagag ggatcgaacc aggatttggc gtgccgggag ggacagttgc 797880
ttttgtaggt gtggctatgc cagtattatt caatagtcca gaagcataag ctactttact 797940
ttgttcatca cctagggcta cccattgcac tccaaagtcc caggatttct ctaagctggt 798000

atctagaatt aaaacttcga tgtaaacctg tttaggaggt aaatctaaac cgtttaagag 798060
gccaataact ctgtcgacat tcccttggtt tccgataata actatggagt tattgacctc 798120
taaccactgg atactattga gagtgttaat gaaatcttcg tccatagctg tggttacata 798180
tagattgtaa ccgatatctt ggagggcatt agcaatcact tctccatttt gatacttcag 798240
cttgtacata aagaaccgca aactcttagg gctcgtggtt cctgttcctc ccaaagccaa 798300
ggcagtactt gcaggatcat ctagggtatg tgccatttct gggacatcta aggacttcag 798360
gagctgctct gccttatttg caagacgtgg tgaagagacg acgaaaattt tgttcgttcc 798420
aggttggatg aacatttgga aagcatcatc ttcggccaga gtaccaagaa catcttggca 798480
gtagctaaca agagctgcgg gattggcata tttaacttcg tattcagtca tgtccacaga 798540
tgtgcctggg caatctagag ctgctagcaa atcactgact ttatcgacat taccagcaat 798600
atccgagatg ataacatgac gagtagcttc tgaagcacta acgatagcat catgggaaag 798660
taaaggttga ataatattta ctgctgcaga gggctgacgc tgtaaagacg gaacactcgg 798720
gtaaccacaa cagcttcaca cgtttctttt aaggagctgt ctgtgactac tgtggatagc 798780
ttagaaagat gaggattacg atagataagg acgttattgc cttgttcaac aaccttcaag 798840
tcatgcattt ttaagacttg tagtaagatt gtagataaat catctacaga agtaggatcg 798900
tgggaaacga tcgtgacatt gaattgcaaa tcgttgctat caaagacaaa gttcgttcca 798960
gaaattttac ttacgaactg caacaactct aaaatagaaa tgtcttcaaa attgacagta 799020
tagccgttat ctttaaggtc ttcacaggta agttcccgct ttgtcaatcg ctctttaact 799080
ttttcttctt cagatttgtc ttgaggtaca gccactgttg ttgctgcttt ctcgattcct 799140
ggcatagagg tcgaaggact tgcaacgttt ttcttcgaag cgtgtggtgt tgcttgtgtt 799200
ttaggttgga cgtctttctc tgtaactgtg gaggtttgct cttcgagttg tttttttata 799260
ctcagattga tagcatttac ggctctgcga gcataatttt gtttttcttc ccaaacctgt 799320
atactatcta attgttcttt agaggcggca ggtacaggct cttcgacttc ttgtgttggt 799380
aagaagcgtg gggatctttt tacatcggca gtagttctac gctctggtcg aggttttttt 799440
ctctcttcca gttctttcag ggtggtaggt tttgctggga atgcggatcc tgaagtcttt 799500
tggaaagttt tatcttgggt tgcgtcgaat ttagagaaga ctttactggg gatagaacct 799560
ggagtggttt tcttggtatg acttttcttt gctgaaagtg aggctgctga atttttagga 799620
caagcagcta acttttcatc tctaagattg tgttttacat ttgccgaggt ctctgtaggc 799680
ctctgggnac ttacaccaag taaaactaaa tctaaaaaaa agagaccact naaaatcccg 799740
atcttttttt tctntttttt gatgccttgc aagatttttc ttccaatgtt caatatcaca 799800
gttttcaccg gattcttttt aagctaatga gcgttgaggg agagttactt ccgtgctgac 799860
gactttgtat tccaccatgt atgctctcta tgggacagaa tatagctttt tgccttctat 799920
cttgcaactc tgcgagtgat agggttttta aggatgcaac agattcttct aattccagcg 799980
tacacactac agcttcatca cctatttccc atgcaacctt ggtttctcga atttgtaaag 800040
aagtaatttt ccctaatcct ggggaaaagc tctcaaagtc ttggacaacc cttccctgtc 800100
tttttatcca gaaaacggtt ttcccacaag ctatccaaga gagttcctta ttttctttcg 800160
aaatttcgag gcagagtata gagataccca tttcatctat gggtattttc atgcgaatca 800220
agcactcatt gatttcgcat actctatcta aaagaggtcg ctgtagatct tgttggcaga 800280
ctaaactttt tacaacagac aaagctatag ttgcattact tataggactg taacctaacc 800340
acagattaaa aacatctcct tcaagcaacg tatcatatgc atgaggatac aagggatatc 800400
cttgatgata gagaactccg gatataaaat ctggaaacct cagagtttct ggggctaacc 800460
aaaatcgttg tgtttgcagc tgttcataga gagctaccgt gtgatcttta atacgtagat 800520
cttcttgcat gtctccagac atgcgatagt gatgtatatc ctggataaac tctcgggtcg 800580
agctgtagcg attattagga gaaggctgta acgcttttgc taaaatttta cttattcttt 800640
cgggcactaa tgaaagaaag acgcgtccta aagaaaggtg ccctaagatc aactcataag 800700
ctaacaaacc taaagcataa atatccgaag cgggagaatg agactcccct tggcgttgtt 800760
ctggactcat ataataagga gttccgataa cgctaggatg tgctctctgt atttccgtat 800820
cccaatcagc gagtccgaaa tctatgagtt tgattttacc ctgaggagtg atgagaatat 800880
tttcaggctt gatatcttta tgtagaatat tccgactatg gaggtgctcc aaagcttgag 800940
caatatcaaa gataatatct atggcttgtg gcaaagagat aaattgcgcg aggatatact 801000
ctcttaaaga gatcccttct atatactcca tagcaatgta gaggcaatct tgccatttgc 801060
catagcgatg gaacttaaca atattaggat gagtaatttg atggaggctc tgtgcttcct 801120
taagaaaatt atagacagag cgactcgtga acgaggggga aggagaaaaa acttttatga 801180
ctgtagaatg gcgtgtttca ggatgtagac catgaacaac tctacttctt aattttttac 801240
tcaatatttt tttaacatga tatccgccaa tcacctgagg ttcaggaaga gggatgccac 801300
cacgacaatc cataaatcca atctttcttt atacctctaa aacgcgaata cctaaaacat 801360
ctcctagagc gataatttct cccctaccga ccttagctcc atctaaaatg atatccacac 801420
cgtatgctgg atggtttccc aagctcaata tacttcccaa gttcaatttt ataaattcac 801480
tgactgctaa ggaatatctt gcaacttcga ctacgagtct actgtatcca gggaggggag 801540
ctgcggaagc ttgtggattt tcaggaagag gagggtcttc atgagttagg ttagggtaac 801600
tggtaatttt aaattctcca gaggagggcg ttaagaaacg gccaccaaaa aactgatgtt 801660
tttgcactgt gagtaaagcg ccgctttctt cggtttcagg atcataaaga cagctatcta 801720
acataatgaa tgatcctggg actacctgat gccattcttc ttgagttagc tgagaatacc 801780
cgacttctac agaaagagag atctgctgcg tttgatctat attatgaaga tccgactcat 801840

catggagacc tgagaaaaac ttctgacaac tttggaaggt atcttctggc aataacagac 801900
gacatcgaac attttttcca tctaggcgca gagaaatatc tacgacttgg aaagagcctt 801960
gcagacttgt agctgtaaat atagcatccc ctccgacttt ggcagacaaa gagggcaccc 802020
actggagctc ttcaaataat ttacaggctt cggcgacaaa ataataatgg aaccctagga 802080
gcttatcttt ttcatagaaa tatgaagcaa ggctggcatc atcaaatact gctaccatga 802140
gctcctgaag gtcttcttct gatgttagga acaacagatt ttctacttcc caaggttgga 802200
ctaccatagg ttgtattaga agatgcacgc cgaattcttt cgtagcttct acagcagtta 802260
tagatccacg aaactttata gaaacctgga catcttcaag tcgaaatttc tctctaattt 802320
tatgttggca cagttcctta ggaaactcag gagcagcaac ctgctcctca gtcttcccta 802380
gggaacttaa aaaattattc cgagatttta gccaacttgc actagaatcg gctgctactg 802440
ccataaagta cctttataaa cgtgcttctt cgattttata agaatcttgt tctttatcgt 802500
cttgtttttg tttttgattc tgatctcttt gatctttctc ttctctatga cggatcgtag 802560
atgcaatcat atgtagagga gtttgtacct cttcaatttt aggaagctgt actaaaagat 802620
ttccaactga gaattctttt aatgtgagtt gatgaccttt taaagcactt accaatgaag 802680
aaagttggct agggttattc gttacaaggt ctgcagcttc tgccatttgt gtagcatcta 802740
caaaacttga gaacttaacg gaaagatcct gtccagattg cactaatgta agattagctc 802800
caacaaaggc ttcaggcaca ctagaactag catctaatac caactctaca agttgctcac 802860
cgttgatttc tgaaatcacc atagattcta cagtagaaag tatgatattt tctatccact 802920
gtgtatcgat actactaaca gctatgggag cgacttctac aacagcttct gctgatccag 802980
ctgctgcgat atctataaga gacatacctg caaaggcgtt ctcagcaagg caaaattctt 803040
gagattcttg ctcttcctct ttggatccta cttcagcaat ctcatcatca actcgcttct 803100
cttcatcagc aaattttcta gattctgtac gtgtatttcc tttacttgag gttgttttct 803160
cttgtcgtgt ttgctttcct tccaaagaaa atacttttac atcacgacta tctctaggtt 803220
caggagagtt ctggtataca gtgtgattgt ctgttttagc gctatataat gattctgctg 803280
ttttctttaa ttccatgaac tagcttcccc ctgattcacg tttttttttc tggcgcagtt 803340
ggaaaagcag ctgccccatc tcgtcttgtt ctttttcttc agcgcgactt cttctttgag 803400
agcttctttc atccattctt ctttatggag tcgtgttttc tcttcttctt ttctacgctt 803460
cgctaaattt acttctgctt tttcgagttc tttagaagca gcaagcacaa cttctttttg 803520
tttgttgact ttctcttctt cttccgaaag ctgtactgca accactttaa tgtacgattt 803580
tatctgtaag acggcgtcgc ttgttgtacc ttcgtccaac aaatcgcgga gttgttggat 803640
tttttgcata tagtgatttt taactttatc gcgttcagct tctttctctc gtaatttctc 803700
ttgttctatt tctaaaagac gtcgtttttc tttaacaact ttttctgctc tatctacacg 803760
atccttttta atcgctaaaa caggctctag tggatatttt gccacagcat actactcttt 803820
atttaccgga aaatggcgcg cagttgttgt gctgcttcct catagtttgt cttttcgtgg 803880
atatcttgct ttaaaaaccg gttcaatttg tcaatatggt cgatagcaaa atctatctca 803940
cgatcagaac ctcgtcggta ttctccaata cggatcaaca tctcgttggc tttatattta 804000
gctaaaactt ctcttgcttt ccctatgatc cgtcgttgtt cttcaggaac aatagcagtc 804060
aggagtcggc taatcgaagc aagtacgtca attgcaggat aatggtatgc ttgagctagt 804120
gcattggaga gaacaatatg cccgtcaaga atcgatttga cttcgtcagc aacaggctcg 804180
ttcatatcat ctcctgctac caagacggta taaaatgctg taatggttcc tttatcggag 804240
gctcctgatc tttccaatag acggggtaga gtggaaaaaa ccgatggagt gtatcctgct 804300
ctagcaggag gctctccagc ggctaacccc acttcccgca atgcacgagc aaatcgtgtc 804360
acggaatcca tcataagtac gacagttttc ccttgatcac gaaaatactc tgcaattgca 804420
gtccctacat aggcggcatt aagtcgcaat tgcgacgatt ggtcagaagt agaaacgaca 804480
attacggaac gtttcattcc ttcttctccc aaatccccct ctataaactc acgtacttca 804540
cgcccccttt ctccaataag cgcgattaca ttaacgtcgg cttcttcagc gtttcttgcg 804600
atcataccta agagcgaaga ttttcccact ccagcaccag caaaaattcc aatacgctgc 804660
cccctagcga ccgtgagcat accgtctata caacgcacac ccgtagacag gatctgtcgt 804720
aatttcgccc tatgcagggg atctgggggt gcacgaaaaa taggaaatgt ttgatccaca 804780
ttttgtaatg gacctttagt ttctacatct atgggttctc ccaacccatt gagaacacga 804840
cctaagaggc cgtttcctgc tcgaatgtgt aaggggagtc ctgtagggat gacttcggaa 804900
gaaggactca ctcctgataa ctctcctaaa ggagagagaa aggcaaaact ctgggtaaaa 804960
ccaacgactt ctgttacgag aggttccatg ccgttacgtt tcactaagca tacttctcca 805020
acacgcacat taggaactac ggctttgatt aacatgccga ccacttctgt aatgcggcct 805080
actacagtcg taagattcac atcaccaagt tgtgacatga gagtgtcgaa atccgttgtt 805140
aactgatcca tgttaaccta attgcgttgt cataatcgtt ttcacgctac ttacagaggt 805200
accgacaata ctcgcaaaca gttctgctct ttgtgtagct cgctgcacag catattgtac 805260
tttaaggaag tttgctaaac tctcacgatt cccatctcta tcagagaagt agagcaacgc 805320
acgattgagg acctcttctc cggaggagac ccaatcaatg attttcctag ttaccgaacc 805380
ctcttcttta tcttctttcc gagcatcttg ttgtgtgtgc tcagcaatcg tattcaagtc 805440
ttcgttaatt aaatctatga tgactcctac agcttggaaa tgctcgtctg caaactgtgc 805500
tgttgtctga ctatcttcga aagttgagcg gaaatcccag aggcgccctt tcaagttgtt 805560
aatttgctct tggagctctt ctgattccaa ggcctcttgc aactcaggat cgattaaatt 805620
agaattttga tcctgcatca ttgccatcgg atttggttta aatccttgaa caagggtcgt 805680

atctttagga gcagcataag atcccaaagc aaatggagaa atatcttttt ttaattcact 805740
tgctaagggg gtgccagaat tctgagttat agattgcgct tctgcatctc catcaaggtt 805800
ggggaaacat tctacagggt ctatcataaa gcaaaaaaac cgttttactg ttttaaaagc 805860
ctagcccaaa ggacctgcac tttttgccac taaagtgtca tgccaatcta agacagattg 805920
agcaagagct cttgtggact ctattttaca atttgctatc acttgatcag caaattttag 805980
gcaactttct agactctctc tgcgcacttc aaaagaactt ccttgatgca agacgatgag 806040
catatgggta agagataaaa atgcttttat actccaatta tcttcattgc cttttatcag 806100
cgcactgagg cgttcttcag catcgaaaag atccatttta tgcaaagaaa tcaatgcgag 806160
tcctagatca tgaccataat gattagggtt tagaatatgg agagattgaa ataactttct 806220
tgcgctatct tcatctccct gtttaatggc caaaaggcct gcttcaaata acaaagcaaa 806280
gtctgcttga aatacttcca aatctgccat gatctctctc ttatattatt aacttccttt 806340
aactgctcta gccattgtga tcatctctgt gttcacagcg gttaggatgt tggacaccga 806400
ttccatatac tgtgataaga tctgcatacg gaattgcaaa ttaaacatgg ttcccaaatc 806460
gacagtgcct tgtgttgatg tctctaactc agttaaatac tgttgaacac ccttcacgta 806520
agtacatacg ccgtctagca tcttattaaa atcgaatgct gtgcaacttt tatttgtagc 806580
catagatttc tctcctctgg acctttatat catcttgcgg ttgatccgac ccagaacttt 806640
ctgtagagct acatagcctg ctaacaacga ctgttgttgc ccaaaagatt ctttgtcaga 806700
accttctcga agcagcgcat gtaacttatg aactttatct tgcacactag ctttaagttc 806760
ttgagctctg tcatgatctt gcatatcttg ttctaaatct aacagcggtt gagaattttt 806820
ttcttctttc gctgtatttt ccatattaaa catagaatat ctacctaaat ataaataatt 806880
aataaacccc aatacttggt tatttattgt agtctatttt atatttcaac ccntccttct 806940
ctaaaaagat cgcgttaggt tgtatacttg ttactgtcat accatcaatc acgtcccctc 807000
ttgtgaggat tctgccattg acaactacat tgatacttat ttctccgtat ctagaatagc 807060
ctgtaacacg atagcgattg ggtaacgtag ggtttaaatc tatgattccc tcttcagctg 807120
ggagtaagac agcaaaattc ttgaccaacc tcacaccagg aatccccgac agctcttgca 807180
ctacagcacg gaacttctct gcatcatcgt tattgacgta accagtaagg ataacttcac 807240
cgttcacaaa ggccacatgg atgtttgcaa aacctccttg aagaagatgg cctgcaattg 807300
cttttaacat ntgggtttna acaacaactt tattctctag taacgagagg gaattaaaat 807360
gtatatttaa ataatcaacg aggcaagctg cttgctcctc agtcttgaca tagcctgtga 807420
tgatgaattt cccaggttct ggggaatgca tgctgatgcc tttaaactcg ggtcgctttg 807480
ataacaggat gttcatctcc tgccaaacag cttcatcatc aataacatta tcatctacgg 807540
atttcacaaa ggaaagggcg tctactttat acagcagctc gcttttgtcc gtactatttt 807600
tgacatgtcc gattaagaaa agttggctgt tcgttttatt aaacgtataa cgcaccgtag 807660
ggaactgatt gataacctgg gcaagatctt cttgataatc aatattttct aaaggaacca 807720
cttctttggt atggaaaaga gaagctgttc ctataccaaa gagaatagcc aatcctccaa 807780
caaacagggt aagaatgaaa gatcctgcgg gtagtgtagc gcgtttttgt ttttcttctt 807840
cttcttgggc ctcttgtctt tctaaggctt cggcgtcttg ctgtctccca aacaaactgt 807900
aatcgtctgg ggatagagaa gcaactatag tatcagcggg ggcatgatga tctataagta 807960
aaaataatgt cgttcctaaa gccacaactt gattcgagct caatgtagag gtcttatcaa 808020
tttttcgtcc ttcaacaatg acaccgtttt tactatcgag atcctcgata agaatgcccc 808080
cgtcattacc gacagtaatt ttagcatgtt gatgagaaac acttaagtca ttaaatacta 808140
tgtcacaagt tgtaggatcc gtacctaaaa tataggtttt tcctgagtct aaatggaact 808200
ctgctccaaa tattagctcc ggctaaaact ttgagtaaaa aacgagaagg ctgcgtcaag 808260
tctacagaga tatttttttt cgcaatatca tcaatctctg ctggaaaaat tgtttgatcg 808320
aatcgaaata agtcttgaac atgaaatggg gatagaactg cgctcttttc gttctcgact 808380
tttttaggag tttctttgtt atcctctgca gtatggtcac ttgccgtgtc gtcatttgca 808440
tcagcggcac tgtcagaatc ttctttatta tcttccgtaa ggttctcttc ttcttcttgt 808500
acaaaggctt cttctgcttc cttatcctcg gattcttttt gtgaatccaa aggtgttgct 808560
tctattttag agccttcctt tttagaacct tcctcaggtt gatctttttt ttctggagaa 808620
tccggtgttg ctttcttatt agccttctct tctacaggtt tattttcttt caagggagtc 808680
tcatccctgg ctgtgttttt aagagagggt tctgctgact ttggttgcgg atcttgccta 808740
ggcgaagtcc gttatcttcc ataatggcgt tctggggttt gttggttctt tcctctcctt 808800
tcggagaatc ctttgcattt tgttcctttg gattcaaaga ctcgtttgaa gattctttta 808860
aacccttctt agcaactttg gctcttggtt gatttttttc cgcttttgct gatgctaaaa 808920
aagcatcagc aagctcctga tccccacttg taattggatc gctacttccc tgatctttgg 808980
tcagcttttc cttaggcttc ggtgaatgat ttgtttccga agtttgccga ggctcaagat 809040
cttttccctg ttcattacta tcggacaaat cccctgaatc attagaaaaa ttttcttcgg 809100
gaatatcaaa atcataaaca agatcttgag gatcaaattc atctgataag aaagaatact 809160
gattgcttcc taataagata gtatcttcat tttttaactg tgtagtttct tggatcgcta 809220
cgccatttac aacaatagga attgtatcat ctaaatttgt gatgtagtag cttccgtcag 809280
tcttattgat aatggcttgc gatgcaccga gtttaggatc ttcaatagga atgtcattag 809340
cactagagtc gcgtcctata gaccagctta tcccatcttc cagaacaaaa attacaccag 809400
acaaggggcc ttcatcaaca attaatcgta ctgccattta ttccaccttg ttactgttta 809460
cctaggccaa gatctgacag ccatgtctct gaaaaattca taaaactctc aacatgtcgt 809520

acaaaatcat catatgtagt atccccagaa aatctgcgga ccatgacaac attgccctcg 809580
gaatccaaac ctaaagcact gcctcctgtt tctctaccga ataaattgcc aatcatcatt 809640
tgcaagtata attttgctgt atcagcagat ggaggcaaag ctcctaacga agcacttaaa 809700
acaatttcgt tatcagcatt ttgctgagca cgcactttaa ctacctcact tataggcagg 809760
acataggctc cgtcagcatc taactcgaga gttgacgtta tacccatata cgtggcaaaa 809820
ttttttatta atttttccaa catactcgtg ttttccaacg cagcaatggt tgtctttgtt 809880
ccctgaggaa tggaaacaat ctcatttcta aaacctgctt tttcttcctt taaatcgagt 809940
tatgccaata cgaactttaa aaataaaaac gatttcttat ttgcagaaac agcattgagc 810000
gcttttcttt gctcatcttt ttcattataa agaacaagag gccacaagaa aaatcgagct 810060
gaatccataa caagaataat tataatctat caattaaccc gagcacgccc attccctcac 810120
agacatcaga ggatattcgt acttctaatg aaagcttaga gtattttcga aagctgtata 810180
tgtcaagcga tgaattaggc attaaagcat caatagctta gaacggaagg aatacgtggg 810240
gatgaaattt gcctctgggt tatatgtgag ctcttttttt cataaatttc ccattgtttt 810300
ttcgcggcac aagttaacaa aagttttgct ttatttactc cttctttggt gcattgcagt 810360
cttttttgca cgcattcact gatgaaatct atatctaaat aaacaaaacc tgtaggggtt 810420
tctttccata aaaaggttcg tggcacgtta aaatcaaaga caatacgttt gggaatgcta 810480
gcaagactct cacaggaaag atcggaaaat tgtgaagctg attctgaaga gccaaagaaa 810540
atcacatcat agggttgtcg aaatgagagc gtttctcgag acaaggttct atagggggcc 810600
gtgacttgtt gtctagagca aaaggtaatc ctatgatacc catgttgata taagtaggca 810660
gcgactttcc tattgatatc cgagtatcct acgaataaaa aattcgtata tatcgacttg 810720
tcgtagctga gtagaatttc ttgaacgaca gattctatgg tcacttgatg atcgggaaac 810780
ccgatgcgtg agcgatactc cttcccttcc tttagagctt tttgaaataa aaaatgaaga 810840
tcaaaaggca actctctctc tttgcttcct tttaaatagg ctcgttttac ctgtccttga 810900
atttccgttt ccccaaagat caaactatct ataccactgg tgacctgaaa cagatgagta 810960
aaacaagaca aacctctatg acggtaagga cgtattccct gggatgtcaa ttctgaaagt 811020
aaggcggctt gagcaatttc aggactttct gaataataat aaagttcggc tcgatgacaa 811080
gtaagtaacg ggataaacgc tcctcctttg cctaaaaaac gctgtgcaag gaaaagattt 811140
ttttcaaaag attgcagata ttgaatcgca cgctctcttt ctttcaaagc ggcttcacga 811200
taactgattc caacaactcc taacaccata agcacgattc gataatttca gaacagtatg 811260
aaggaaactt atactaaatt tataaatgaa aagtaactta gctgtataga cgaagagaaa 811320
attagaagcc atcaaataat ctattttctc tgctcattgc gttttgaagg cggttgccta 811380
tcccatatac cagaggatct ttacgttgct ttaaaaattg aaaggtaatt ttgactacga 811440
tctctaggag aaatcaatga agaaggcagg cctatgtttt gcagttcccg tgttttgttc 811500
ttccttgatt aggaaaaaga ggttcgggta agatgcgttt tgagctcctc ggggagttcc 811560
aaggattctg tccttaaatg ataagacttt agcggatgga tatgcagttt aaagctgagt 811620
tcatgtaata gcgatacatt cccaagacta agatccataa gtttcgtctt agtacgaaac 811680
ttatatgttg agcttgtccc ttttcttggc gcaaaataga taagcaatca aaagattgtc 811740
ctatccgctc ttcttaaaaa ttcaacaaaa tatcacacat ggcggcatac acagaagcaa 811800
gcattctttc tttggcctct cttgatcaca ttcgtcttcg ggcggggatg tacattggaa 811860
ggcttggcaa tggttctcaa aaagaggatg ggatttacac tctttttaaa gaagtggttg 811920
ataatgggat tgatgaattt atcatgggtc atggtaaatc tttaaaaatt tctgctagtg 811980
acaagcagat ctccattcaa gatcaaggtc gtggcattcc tttaggtaaa cttatagatt 812040
gtgtttctaa aatcaatacg ggagctaaat atacccaaga tgttttccat ttctctgtag 812100
ggctgaatgg cgtgggactc aaagctgtga atgcactttc agaaatattt tctgtacgtt 812160
ctgtaagaaa gaaaaaatac caccttgcca ccttccatcg aggagttctg caagagtcta 812220
agcaaggttc taccaaagat cctgatggaa cttttgtttc ctttactcct gatcctagta 812280
tcttccctga gtttactttt aaccacgact tcctaaaaga taaaatccgc caatacacct 812340
acctacattc gggattagag atccgattta atgatgaggt gttcatatct cacaacggtc 812400
tcaaagatct tttcgatgca gagatcactg agcccccttt atactctcct cttttttttc 812460
aaaatgagga tttaactttt atcttttctc accttgaagg aaatacggag cgttattttt 812520
cttttgtcaa tggacaagag actcttgacg gaggaacaca cctgactgcc tttaaggaag 812580
ccatagtaaa aggggtcaac gagttttttg gaaaaacatt tgtttccaat gacattcgag 812640
aaggcattgt gggctgcata gcaataaaaa tagcctcgcc aatttttgaa tcgcaaacga 812700
aaaataagct tgggaataca cagattcggt cttctttaat taaagatgta aaggaagcga 812760
ttgtacaggc cctacgtaaa gataaagtgg ctcctgagct tcttttagaa aaaataaaat 812820
tcaatgagaa aactcgaaag aatatccaat ttataaaaca agatcttaag agcaaacaga 812880
agaaagtcca ttataaaatt cccaaacttc gggactgtaa attccattat aacgatcgct 812940
ctctgtatgg tgaggcctct tcgattttcc ttaccgaagg gagtctgcgt ccgcatcaat 813000
tcttgcttca agaaatcccc tcacacaagc tgtcttttca cttcgaggaa agcctatgaa 813060
tgtcttttcc ttagaagaaa ccaaaatgta taaaaatgat gagttatttt atttagcaac 813120
tgctctaggc atcacgcaaa acgagattca gcatttacgt tataacaaag tcatcctggc 813180
tactgatgcg gatgtagacg gtatgcatat tcgtaatctt ttgattactt tcttccttaa 813240
aacactcttg cctcttgtag aaaataatca cctctttatc ttagaaaccc ctttgtttaa 813300
agttagaaac aaaacgacta cgctctacta ctattctgag caagaaaaga tgcaggcgtt 813360

acagcaattt gggaaaaagg actcctcttt agaaatcaca aggtttaaag gtttaggaga 813420
aatttctcct aaggaatttg ctgcgtttat aggtcctgag atccgcctca ccccagttac 813480
gattacctct ttagagagca tttcttcgat cttacaattc tatatgggga aaaatacaaa 813540
agagagaaaa caatttatta tggataacct tattactgat ttttaattta tgcgtgacgt 813600
ttcagagctt tttcgaacac attttatgca ttacgcgtct tacgtaattt tagagagagc 813660
gattcctcat attcttgatg gcttaaaacc ggtgcagcgt cgacttctat ggactttatt 813720
ccttatggac gacgggaaaa tgcataaagt tgccaatatt gcaggaagaa ctatggctct 813780
ccatccccat ggcgatgccc ctattgttga agctcttgtt gtcttagcaa ataaaggcta 813840
cctcatcgac acgcaaggaa acttcggaaa tccccttacg ggagatcctc acgctgctgc 813900
ccgttatata gaagcacgac tcagtccttt agctcgagaa acgctcttta ataccgactt 813960
gatagctttt catgactctt atgatggaag agaaaaagaa cctgatattt tacctgcaaa 814020
gctccccgtg cttttacttc atggtgtgga cgggattgct gtggggatga ccacgaaaat 814080
tttccctcac aattttgcag aacttttgaa agcgcaaatt gcaattttaa atgataaaaa 814140
attcactgtg tttcctgact ttccttcggg aggattgatg gatccctcgg agtatcaaga 814200
tggattggga tcgattacac tgcgtgcatc tatagacatt attaatgata aaacgcttgt 814260
agtgaaacaa atttgtcctc aatctacgac tgagactttg atccgttcta tagagaacgc 814320
agcaaaacgt ggcacaatta aaatcgatac catccaagac ttctctacag atgtccctca 814380
cattgaaatt aagctgccaa aaggctctcg agccaaagag atgcttccct tgttattcga 814440
gcatactgaa tgccaggtga ttctctattc taagcccaca gtcatttacg agaataagcc 814500
tgtagaatgt tcgatatccg agattctcaa actgcatact acagctctac aggggtatct 814560
tgaaaaagaa cttttgttgc tccaagaaca acttactttg gaccattatc ataaaacctt 814620
agaatacatc tttattaaac ataagctcta tgattctgtc cgagaagtcc tagccataaa 814680
caagaaaatt tctgctgatg acctacatca agcagtgctc catgctctgg agccctggct 814740
tcatgagctt gcaactcccg ttacaaaaca agacacctct caacttgctt cactaacgat 814800
taagaaaatc ctttgcttta atgaagaggc atgcactaag gaactgctag ccatagaaaa 814860
aaaacaagca gcgatacaaa aagatcttgg aagaataaaa gaagtcaccg tcaagtacct 814920
caaaggactt ttagaacgcc atggacactt aggagagaga aaaacacaga tcacaaactt 814980
taagacggca aagacatcta tcttgaaaca acaaacctta atttaaaaaa ctaagtttat 815040
ctaaaaaact tctgattaat aagagatggt aaaatatttt ctttttaaat tagaattaaa 815100
tttatagaca ctataaccat tgtagtacgt atggaaccac gtcacattta tataagaaaa 815160
ccagagactc caaaagctcc tgacgtagaa aagcctggtg tacctgagta catgacgatg 815220
gcaaacactc ctaccttcga gggtcctgta aaaactcttg atcactacgc cgagctctta 815280
tcgagcaacg aggagctgag gaagggcaaa aaatgtatga taatttcatt cagtctattt 815340
taatttcaac atttgggctt gtacataagg atatggaccg agcacaaaaa gcttctaagc 815400
gtatgagatc tgtctataaa gagcagtaat gtcgtttacc tatttcctag cgcttcccgt 815460
agataggctt atgcaagaac ggttcctctg ttctcccaaa cgttgggctc cttttatcaa 815520
ttcgccttta taccttactc tcattgctga ccacgatact ccttatttgg ctaagaatct 815580
tgataagttt cccttacctg tagagcaatg ggaaaaaacg gtcctgcacg tctctagcct 815640
attgaagtct atatttttat gttcagacct ttcctcttta aggttgctgg cctgtacaaa 815700
attcgaaatc ttgactttga acgaccttta ttgcgcccaa aatatctaaa aaattgcttt 815760
acaaagacac tcgcatcttt cccttgacta aaaattttgc ttgtcaaact gccatcgaag 815820
tttgtcatga tgatgtttga gctacatagg ttagcctcag ttgattccag cattttatct 815880
taattcaacg catttctatc tttttagact cattaaatta tttttgaaaa attctaagtg 815940
tctaagattc aaaaagagac tctttctttc aataaaatcg ctagatcctt aactgattcg 816000
atattaaact agtaaagaaa gttatcaaga tttcaatgaa aactgtgact tcctttactg 816060
tatgtaaaga aaactcgggg cgtttagaca agtacctgac tgaggtgcat cccaaatatt 816120
ctcgagcttt ctaccaagaa catatcttaa gtggtcttgt ccaaatcaat gggcaaataa 816180
acaccagggt ggcaacgcgc ttaaattgtg gtgatatagt cactatagat atccaagaaa 816240
aggaagaact tcttgagctc ctacccgaag ccatccctct agataaggtt tatgaggatg 816300
gaatgatctt agtgatcaat aaacctcggg atatggtggt ccatccagca cctggtcatt 816360
tccatggaac cctggttcat gctcttctcc atgaaatagg agagagactg aaggaagaat 816420
tccctgagga accttggaga cctggaatcg tacatcgact tgataaagat acctcgggat 816480
tgattattac tgcaaaaacg cggcaggcca agaaggtttt cagcgagctt ttttcaacca 816540
agcggttaaa gaaaagctac ttagcagttt gtatagggaa acctaggagt actacgatcc 816600
atacacatat aagccggcat caaaacaaac gtaaagaaat gactgtaagc tctcaaggaa 816660
aagaagccgt tacccactgc caagtccttg cttttaatgg aaaactgagt tttgttgcct 816720
tgtctccaga gacaggaagg acccaccagc ttagggttca tatgaaacat ttagggactc 816780
ctattcttgg ggatcctgtg tatggaatcc cctctatgaa ttcgagttac ggtcttgata 816840
aacaacaatt gcatgcctat agcgttgatt tcactcatcc agaaacccgg caattttgtt 816900
cattaaaggc gggtttaccc gaggatatgc gttccctctt aataaaagaa ttccgcaatg 816960
aaacaactat attaaataaa aatttattgg aatcgatttt aaaagaacaa taattcatta 817020
aaaagttcat ttattttagg aaacgcatta aaattaatta aaattgaatt tcatttttta 817080
acatctcttt ttaaagacaa cgcgaattag ttaaggatta ctatgaaaga atttttagcc 817140
tatatcatta agaatctagt ggaccgccct gaagaagtcc gtattaaaga agttcagggg 817200

actcacacga ttatttatga actaagtgta gctaaacctg atatcgggaa gatcattggc 817260
aaagaaggcc gtacgatcaa agcgattcgt actcttctgg tttctgtagc aagcaggaac 817320
aatgtaaggg tcagtttaga aattatggaa gaaaagtagc cttaagccta gcttaagtac 817380
ttctcattga aattgctagc ctaagggaaa ccgaaggcta ccacttcaat aaaaaataat 817440
tgcacgcata aagataaact cggaccgagc aggactcgaa cctgcgacca ttcgcttaga 817500
aggcgaatgc tctatccact gagctatcgg tccctattct tgtacaacct tggggtattg 817560
agcacgaaag cgagcaaagg aaatccctca atcagtatag gtctaccaag aaaaaagtca 817620
attctgacac aagattttct tgttccctaa agaacttctt tcaaagctat ataaaaagcg 817680
aattccacct ccttacctgt aattttatag gtaaaattaa ggagcaactt ggattgtatt 817740
ctccgatctt ataacctgga aagttagaac caaaagttgc tttattctaa aaaaggatgg 817800
tcatgttcaa taacaaaatg atcctaattg ctggcccctg tgttattgag ggggaagata 817860
ttacattgga aatcgcaggg aaattacagt ccatactcgc cccttattcg gatcggatcc 817920
aatggttttt taaaagcagt tacgacaaag caaatcgctc ttccctaaac tcatttcgag 817980
ggcctggttt gacagaggga ttgcgcatcc ttgccaaagt caaagaaact tttggcgtgg 818040
gcattcttac agatgtccat acgcctcaag acgcttacgc ggctgccgaa gtctgcaata 818100
tccttcaggt acctgcgttc ctctgcanac aaaccgacct cctcgttgca actgcagaaa 818160
ctggcgctat agtaaattta aaaaaagggc agtttctctc cccttgggat atggaaggcc 818220
caataaataa agtactctct acaggaaata acaaaatctt acttacagaa agagggtgta 818280
gcttcggtta caataacctt gtttctgata tgcgctcgat tcctgtttta tcccgttcag 818340
gatttcctgt aatttttgat gccacgcact ccgtgcagct ccctggagct ctatctacag 818400
aaagcggtgg tctgacagaa ttcgttccta ctctttcacg agctgcttta gctgcaggag 818460
ctcatggcct ttttatagag acccatacca atccaaaaat cgctaaaagt gatgcagctt 818520
ctatgttgag cttagaagaa ttcgcagctc tcctccccac ctgggatcaa ttatttactt 818580
gcgtcagttc ctttgatatg gtctcagcat gacaaaattt ctatactgcg ggctctttta 818640
ttctctagga ctacttgtct tggcttttgg gactatggta gccattattc aagtggacca 818700
gatttgcgat gtttcctgta tgaacaagca cttccaagaa tccccccctt ttttaaaaat 818760
aaaaaaggtg aatgtctcca aacaaatttg ctctcctgaa gaacgattct tccattgtaa 818820
aattgataaa tcgtgtatgg aactgcattt tcctcagtct agttattcct gtaaagaata 818880
cctcacccgg atctcagggc atattctaac acaaaatttt gaaaagcaaa tgcaattccg 818940
aggaaactca ggattactaa attaccaaga tggttcctta catgtgtatg actgccgttt 819000
ccaagtagat cctgtacctg ggtatgggtc tccagataag gaggacagtt cttcaggagg 819060
tatgaaaacc ctctatttat ctttattcag gaattaagcc tctatgccta tactttctgt 819120
gtgtaatctc gtaaagaagt ataacaagaa gcccgtgaca aatgatgtgt ctttccaaat 819180
caaccccggg gagattgtcg gcctactcgg ccctaacgga gcaggaaaaa caacagcatt 819240
ttatcttact gtaggcttaa ttcgccctga ctctgggaag attatcttta aaaatgtcga 819300
tgtcaccaaa aaaactatgg accatcgtgc acgactggga atcggttatc ttgctcaaga 819360
acccacaatt tttaaagaac tcacagttca agataacctg atttgcattt tagagatcat 819420
ttacaaagcg cgtaaacaac aatcccatct tttaaacacc ctggttgatg atttgcaact 819480
aggttcctgc ctccataaaa aggcaggaac cctatctgga ggggaacgac gaagattgga 819540
gatcgcctgt gtattagctt taaatcccag cgtattgttg ttagatgagc cttttgcgaa 819600
tgtagatcct ctcgtcattc aaaacgtcaa gtacctaatt aaaattctag caggacgtgg 819660
aatcggcatt ctaattacag atcacaatgc taaagagctc ctttctattg ctgataggtg 819720
ttatttgatt attgatggga agatcttctt tgaagggtct tcaagccaaa tgatcagtaa 819780
ccctatggta aagcaacatt acctgggaga ctcgttctca tactaatgga tctcacaaaa 819840
gtctctaggg aaaggagcgc tactgtattc tgtaagaatc cttgctaata cctcaaagcc 819900
atcttcttct aggatagtga ctcctggttt gactcctcgc tcagctaatt taaatgcctt 819960
aaatgtattg caaatacgtc ctgtgagtct gatgcccaca ccctttaagt tcattacgga 820020
ggcataatct ccaaacattt ttaaggaaag ttcacaagca gtaactgcaa agagaatttg 820080
atatccaggg tagcgctttt ttaaagtgtg taaatgtttt gcgatatcta taaatgtagg 820140
gatgatcaca gtagtgtatc tacgagcatt cagccgcatc atggtcccta tagaacatga 820200
ggcacatgta ggatgtgaag gatcataccg acaagcatca ttgaaacgcc cttcaggaca 820260
tgccttaggc ttttgacaat aagaaaatcc taacaaaagg attctatgag gacgacgcat 820320
ctcctctaaa atatcctcaa tattactaca gccataaaaa aataggttgc cctcctgtaa 820380
ggcctcctta ggagctatca aagccttagc taaccgagct aaagaaccag gatctttcag 820440
aaaatcatag gcaagctgtc tagcatcttt taaagaggcc agataagcaa gcgtcttcat 820500
acgcagaccc cgtcttgtag ctttagtaat ataaggaata ttaggtttgt gctctggtct 820560
agacataaat acggaatcat aacaattagc aacggccgca cactgccacg aagcacaggg 820620
gcagtcacac aaaaacccta gaagcatttt ccatagctca gggaatggca atattttaga 820680
tagtatacag cctgaacgat aatctaatct agtagaggtt cgtcatcaga gaggacttcc 820740
tctgcgattg ccttgattgt agtgagacct tcagggaaac ctaaactaca taataattca 820800
tttacatgtt ctaactctgt agtcagctgg tcgttgataa actccaagcg agcgagttgc 820860
tgttgcatat gagatgttgg atgcataaga cccctccgta acgattcata aagaagagga 820920
gcgaaaaaca tgccaacatc tccgcaagat taaaattttt ttaagattta gaaatccaag 820980
tttagaaatt gaagaactat cctgtcaggc ttcgtgctaa acttaagtac atttgattta 821040

aaagctgtag agaagtcgcg actacagtcc actcctgctg catagaagtc aggtgcatct 821100
gcaaatctaa ctggaagttc tgtcccatat ccgcaaaaga ttgctggtct gactgtattg 821160
tagattgcag tgggaacata cccccattaa tcatatttcc tactaacccg gacactaaag 821220
cttcttcaag aatctgcaat cgcgcttgcc attgctcttg cccctcttta acttcaaaag 821280
ttcctgctac agaacctcca gctatagaca aaggctgtaa gtaattttgc aataacacta 821340
aagaacctga gatagaattg atgttgtcct cgtagttcct taaggaatct aaaatcgtgg 821400
agcgctgctc attcgtgatt ttatcatctt taagcactct agctctttgt tcttcaataa 821460
ctgtaagagc tccccgtgtt tcttggagat acaaagcagc ttgctttcgc tcctgatcca 821520
actttgcctg agcgctttct tgagatcctg ggaaggcatt cgctccccca gcacctactg 821580
ccggctgttg tcccacataa ctagcaaaat tgaaatacgt agctccattg acatattgag 821640
aaatcgcatc aataatggaa tttccgacag aagatcctaa gttgctataa tatagcttct 821700
tatagatttc atttagataa tcgacctctt taggcatata acgatcgatc aacacagagg 821760
cgactgccga aagtaggggg agagcccgta attcactagc ctgtgataac aattgcccat 821820
caaacagttc tgttttcata ttggtgaatt gttgcaattg actctgaaga tcttcgattt 821880
tgttctgtac attttctgat tctacagcag caagagcctt aaaccgaaaa taacttcgtg 821940
ccgtagattc ggcgtttgtt tttgctagtt tcgtaatttc tggcaaaaat cctgtagatc 822000
cataatttaa caggatttga ttaatctcaa ctttgccgtt tttctcagaa tatatagtat 822060
attgtaaggt acttgagttc accgttacgg aaaaggaacc gcttgaattt acaattgaag 822120
tcatcgcgga ttttatagat gatgctaact gaaaactaga gttatctaat tctttagctt 822180
cttcaaggat agcagcttta acgtggtcca tggttgcagt agggtttaaa gcaactacag 822240
tagatgctgt aaaataagcc cagatagcac caaggtgctc accacactta aaggtaacca 822300
aagatttata cactcattaa tcagtttctg ttgctcagca gttaactcat tgaaacgaga 822360
atttaaccct cgggctgttt gtagagctcc agctaaagcc gcactactaa tatcaccttg 822420
agaggaactc agatcaattg gagcaaaagt tccaattaaa gagccaacaa agctagacaa 822480
attggaaaac atcgttcctt gatgctggtt aacatagata cttgcatcaa gtaccgtatc 822540
aaaggatcct aaagccgcaa cttgatctga gtataagtct gttatatgtt gacagaacat 822600
aattttatca tttctagtca ggtcagaaga attaatgact gcggcgagtt tttctccagc 822660
atcctcaatc cgttgtaatc ctgcttcatc aaaattacct acatgatctg ctaaagcctg 822720
cagctctgtc gcaatctcag caaagatagt aaactgctct ggtgtaagga ctccttgtac 822780
cgctgtaagg atgtctttga acccttcgac tctaccgtct gcaaacgttt gagttaaagt 822840
agcttgattt ccatatttgc ctgccaactg gttcgtaaag tctgttttca ttcccccagt 822900
aaacgtatct gctcgttgga ttgcagtgaa gatctcctct gggaaattgt agagagtctg 822960
aaaattctct tctgttaaat tattccccgc agcgactaaa gcctgaagtt cagtatttaa 823020
tttatcaaag acatctttat ttgcaggatt cttactatta actgaggcca tgatagtggt 823080
cataaccgta ttaagctcaa cgataagatc ttgcgcccac ttctgtttta ctgcgttata 823140
ataggccccg actccagatc ctgtagctgc tggcgtactt gctgtaacct gaacagatgc 823200
atgtgtagag gaaagaggtt tctcatgaat ggctgccaca ggaatagact tcgctactgt 823260
cgcacgctcc acttcatatt tgtgtagctt ctcgacggca gagcggtagc gctcgctcgc 823320
cctctgatcc aaaacttcga tcaaccgttt tagtatgtca cgttccgctg tagtcttctc 823380
ataattagcg atatgttcta aagattccct gtgtctttgt gcaaaagaat gcattgaaga 823440
cactagagag agttttttat aaaatgttga aatagaagtg ctataaatca tatcaatgtc 823500
tgcttagata agaacagtgt tatttttaat taacattaac attaataaat tattttttta 823560
attcaaaaaa ttcgtcaaat gtttaaaaag aaaactatca atattataga aaacaaatta 823620
tttttatttt gaaaagcttc atagaaaaaa acaaaacaaa gtacgaaaat aaaataaaaa 823680
tattatttat tttctatgtc taccttttct atccaaaatc gactaagaac catttcaggt 823740
gaaagtactc gaatcatcaa gctggaccat aagtactctg gttttgatcc cagatcagtg 823800
cctgcgataa atttagaaga gttaaattca gggatttatg ctctaaggca tttaatgaac 823860
gccctgcaat cagaaaatac caatgttgct gctttattaa acccaaacaa tacgatcttc 823920
cccacaacat cttggacaga ttacaagcat tcgcgtccgc aagctagctc tccaagagca 823980
ccctcatcac aaactcccac agatatcgta tcagcagcag ctcttgcttt agttcttgtt 824040
attgacggag gtctagcgga attagtggcc tccgttacag aaattgatct cggagcttta 824100
tccactatat ccacagttcg tcagttaatg gcgagctacc tcggtttgac aactctaaca 824160
gctgaacaag aaaaggttgt attttccagc tcctatgttc cttcagaaaa aatctccttt 824220
gaacatgtaa aacaagaaaa agctgctgaa atccaagcta agcaagaaga aataaaagca 824280
gtattagaag ctaaaggagt ctctactgaa gagatcgaag cgatacttaa ggaatatcct 824340
gatatctatg cagcagattt cttcaaagag tttatagaag agcctttaca tacatatcgt 824400
gcaaaagtcg gtgcaccgat ccaagagatg aatgagaacg cgattcagct gcttcctaca 824460
cctcctgcga tcactcctga caatgtcaat gaagtcaacg gaatgaacac cctcagcact 824520
attttacaag ctatagatga tgctattaaa caagctcctg cacttggtgg ggatcaggaa 824580
atcattacta tactacaaac tttggttccc ctagtcgata agaccacgtt tacaaaagct 824640
gaattcgatc ttatttacac agcaacacaa cttcctaata cagcatcttt aaaactctac 824700
cttacggata gacaaattgc tgagtatcga gggaaaatca cgaaagtata tcaaaattct 824760
atccaaaatc tctctgagac aaaacgtgta gttgaaaaca accgaagcat gctagaaaca 824820
caactctcca tgttccaaca agcacaaaat tgctttgtta cttggattag tcaagccaat 824880

gcacttaaca tagccatcac taataaatat atttctgctg tacttacgac ttctatggag 824940
atgtacggag gtctcctttg cctttcttat atgtacgaaa ggttagccga tgatgaaaaa 825000
gcaatttttg acaaaagtgt gaatgagtat ttaccgattc acatcgttgt tggtggttca 825060
tgggtaaatg gctggatagc aaaaatggca gcctatcaag aactcgcgga atactcttta 825120
ggaaccgcag ttacaagtca agatcaaatc aaagcttatt tacaaacacg agggaatgag 825180
tttaaagcta cgcgtcattt tttccataat attggggatc aaatgtacca atttgctaat 825240
gagactgtct ttggaaattg tcttacaaca gcaaatggtg cgatacagcc cgatttaggt 825300
ggttttatca gagaagcaat gacgaatgtt ggaactgttg aagccgatta tgtaagcaat 825360
gctcagagga tcctaaatga atttaatacg gctgcaactg cgcatgtttt acaattacaa 825420
ttacaaatag ctgagttaca aaagaaagca gatgacttag acccaggaaa agcctctttc 825480
actgagaacc gtaaatttgc tgttgccgct ttggatcaca tcggagagct taggagatgc 825540
tttaatttct atgattttna actctcagct accaaagcaa gaggcttttt taaaaccttt 825600
gatcgaagaa attaacttca ataacctcgc agcgaatgcc ttaaacagct tgctacagat 825660
taccaatgaa ttttctacga cttctgtcta ctatagcctc tcttcctatt tagttcagag 825720
taaaactgga caaaacctgt ttgctggtga ttactatgaa acacttctag ctgcagctag 825780
agaacgggag tatatttatc gcgacactgc gagatgtaaa caagcgatta atctagtcaa 825840
tggacttctc caaaaaatta actctcttcc aggggctacc tcagcacaaa aacaagaaat 825900
gcttaacgca actacctatt atcaatacag cttatcagtc actttaaacc aacttactgt 825960
attagaatct ttactcgcgg gtctcaaaat gactcttcag acaactagta ataacaaata 826020
cgacaaaagt gtgtttaaaa ttgaaagttt tgatgactgg attccaactc tagctgcttt 826080
ggaaagtttt ctaactagtg gattccctaa tatcagtgcg acaggaggcc taggtccttt 826140
atttacccag gtgcaatccg atcagcaaac gtatacttct caaggccaga cacagcagtt 826200
gaacctacaa aaccaaatga ccactatcca acaggagtgg acattagttt ccacatccat 826260
gcaagtatta aacggtattt tatcacagct tgctggtgcc atctattcca actaattgca 826320
tccttaggag tttttagagc tcctaaagga tcttttcttc tcctttaccc tatacttttt 826380
ctttatccat ctgcagctta gaaagaacat ctcctaagct gctgatcaat taacaagatt 826440
ggaatcaatc atggaagaga aactctaaaa aagtatagag gaccttgcaa accattctct 826500
aaaatcaaat aaaagcttag aaaagaagtt taaaactggt gtcttttatt tattgaagat 826560
cgttctagtg tgttggtaag gcctcaacaa aggccttacc aaagcacata ataatctctc 826620
caaacgaaca cttaggtgtt gttattggag ataaccagaa tatagagagc cgatattgac 826680
cagcacctgc tgaataaaca aggagttcgt ttcaaaggaa agtgctttta tttcagctgc 826740
tgtcctagat ccttcagcaa acaaactttc taatttagct atgaacttct gtgtagagtc 826800
attagaaagt tgcacataag gatagccaaa ctgtggaggc tttgtcactg ccgatgtaag 826860
cttttgtctg atctcctcat tattcgcttg aggattcgac tggatgatct gcattacaga 826920
ttgtagtgcc gaaacttgac tatagacatc tccaagagtt ctgctattgc cagaaatcac 826980
cctagcgagg gcttgatctg ttttttctgg tcctcgagct tctgttcgtg ctctaggaac 827040
ggatcgtgtg agagcggggg tacttacatt gtttatcaca tcacgagtcg catcatttcg 827100
tgccctacca taggcatcat tgatggattt gtaagcatca taacctgctg atatctgtgt 827160
tttataatca gaacctgtag attttgaggt cttgtaaagc tggtttacag atgaccctat 827220
agaacttgct gcagcgggag gaactcctgc gctcacaaac agcagcagaa gcgatctgtc 827280
ctaaagcatt gagtatgccc tgttgttgcc cagctttacc tagagccgct tctaaagctt 827340
tctgagcatc tgccagcgct gcagcagcac tgtcatctcc agcggctttc gctgctctag 827400
cttgtgctgc gagctcctgt tgggcagctt gagaatcagg attttccgta ttgaacatgt 827460
gaatcatctg acgaaaccca gacatcaaaa tggaagcggt ctcattttca gcatcatcta 827520
acagcatgga aacacgaata ctaccaatac tacttccttg ttgcttggag cctccaactg 827580
tagttcctgg atttggaaca tcagaaccat ctgcaggttt gatattttta agatctttct 827640
cagcctgtat taccatttgt tccgcttctt gaagaattgg agagtcgggg aactttttct 827700
gagcttcagc tatttgtgtc ttagcagtag cgattgctgc tttagctgaa tctatgttgc 827760
ttatactgtt attagattta gcattttcta cagctccact agcgttctgt cctgcaaaat 827820
atgcatccct aatcgcattt ccatctttct ctatctgtgt cgctgtagca tctgtctgat 827880
caactaaaga ttgagcaatt gcaggcgttt tccctgggac tactgggtta tcttgcatct 827940
ctttaagaag ctcagctgct ttgttattgt ttgctacaga ttggagaaga gcagcctgta 828000
agaggtcgaa ggaagtcagt ttacctaaag agtcaagaat cgcttggtta tccgaagcat 828060
atttcgctaa ttctgtaatt tgcgcgccaa ctttaactgc atcggcattc ttagtttccc 828120
actccgcagc gattgcggtt tcctcatcag tagccgctgt atcctttata ttagtgacag 828180
catcctggag gctcaccaaa gcagcctgta tgtcagctag tgatgttgag gtaaagatag 828240
tatcgtaagc tgtttgcgct tgagtcttat aatcatcaaa cgtgggtgga ggaggcgtag 828300
gtgcggtcgc tgtcgttgag tccacgtctg cagatctgct agtagaagac gagctgttac 828360
tagaagcaat acccagctta tctgccagac tcatgagagc attcactgca gaacgcaaga 828420
tgctccatcg ctctacagaa tcggtcttag attctttagg cttagcttcc gcacctgcta 828480
ttctttgagc ttccgcactc ttatttgctg cactcgcctc caatccttga gcagaaagat 828540
ctgcgggagg tgtgcgttct gtttcgtcta taggacctgg accaatagga ttaaccataa 828600
aaactcaaaa aataataaaa atcttttagt tttattataa aaaataaaaa aaaagctctc 828660
actaataatt aatgcgagcc tgtttttaaa gattttatta aaatttgact taaaacgctg 828720

aaattatacc tagatcaaac tttcggaaag taaaatcact gccgagttta tcgtttgctg 828780
gtttggagta agccccataa gctctgaatg atagagaatc tgtgatgcca tacatatata 828840
gagcggaaaa tcctttataa tttgtaaaac cattagcctc tttaggatca tagttagcag 828900
caattgcttg ggcgaaccaa aactttaata aattaccacg gccaatccct gaaacatcta 828960
tttctggaac cgacaaggct tcgacatact cataacgtac tgtggcagac cagtctccag 829020
cttttctgag tccccctaaa gttcctccaa taaaccaagc taggttttct tttccattta 829080
acgtagtctt cgtagccttt gctaaagggt tcattaagaa agctccataa agatatagag 829140
gctttttctg tccattgatc caaggaacct gactatgctt tccgacgagc cactgccaaa 829200
cacagtattt gtatttcata gcgtttgtag cagctttttc tgtagtggag gtttctgaag 829260
gaacgaatgt gttccagtcg acaacactac atttcacaaa aaactgttta ggcaaacgat 829320
tgagaatccc ttctacaacc caagcataat gtttttttgt catgttcacg acgaaaggac 829380
ctccatgaac aatcacttga taaggatagt ccttagaaag ttctcgagtc caatatatat 829440
gtagtccgtc aaaattactt tggaattgga cttctgactc aaagagatct cctaaaccag 829500
aacgtccgat ttccataaag aaatctgtac gtgtttcggg attcttataa aaacgatatc 829560
ctagaaatgc tctgttgata tcaacaccag ctgcagtgtt ttcccctcct gcaattgctg 829620
tccaattcat ctttgaagac agccagttcc tctcagcgcg ataatcaata tagagataaa 829680
attcactacg ataacgattt actggtaagg gattgtattt atctttatct gaggggtttt 829740
tgatatcttc tctgaaatac aaccaccggg ctctaacatc tcctgctatt cttaaaacgc 829800
catcttgctt tcgcgtttct acaaaacccc gtttacttaa gtagtctttg acttcgtcta 829860
aagttgtata agtgtgactt tttacttttt gttccgagag aggaagttca gcgtatcctg 829920
aaattgtcag cgctaaaaga accacactcg ctaaccattg atatacctgt tttttcatag 829980
tatccttagt atcaatatta agtgtagaaa cagaagacca agcaggctat caaaacttaa 830040
agctaaaaga aagcgactga gctacaagaa acaaaacatg attaattttg agtgtcttgc 830100
atcacgtgtt gtctttgcaa aagaccccgt tgcccacgcc agtattcaaa agaagccaaa 830160
ttaataagat cttctactcg aagcagtttt gcaaaaccaa acaaaaaagc caagaaaatg 830220
cagctctcag ataaaaaagc aattgcttga gccgttatgg aggataaggg ccaagcaagt 830280
ggtgtgaggg ggtttaagaa aattacatat gtagtttgcg taaggatatt taagcctaga 830340
gtaatcatac aagcaagcat agtggttccc ataactttta tggaacgccg gatgctctcc 830400
caaagtaact tagagtacat agggagtctt ttcgaagaat aataccagag gaaatataac 830460
tgcacccaag cagttatgga tgtagcatag gaaatgcccg agacgtcttt taaaacccaa 830520
cgacctagaa ccaagcttaa aacaatattg gccaaagccg taccgattcc tataaagagc 830580
ggaacagcat actgccgctg tgcataaaaa agaacagaga ctaaaggagc caaggccata 830640
gggataatac tggcaccata acctcgcaat acacgaacaa tagcgtagac agcactctga 830700
gggaaaagtc cgtgttcata aaggacacgg actccaggta aagctaagag caatagccct 830760
gctgtcataa tgatcattac ggacatggtt agggtgagaa cgaacttcat aagtttcaat 830820
cccctctcat gatcttctcg ctgtacacaa cgagaaattg ctgggaggag aacggtaaac 830880
acaccaaagc caaagagatg tatggggagc tgataaatct ttaaggagta cataagatat 830940
agagggccta tttcatgtac atagcgagcc aagcagatat cagaaagaag gttcagctgg 831000
aagatgcttg aagttaaaat acccaaagat aagggagcta ataaagctca aacactatcg 831060
tgttcttgag gtgggctctt cgcttctaat agaaatttcc atactccagg aaccgtgatt 831120
aaccattcga agaaaaaccc gataactaga gccacggata aaccgataat acgctctcta 831180
ggatctgaat gacgagccgc tataacaaag aaaatccaaa tgatatttac aactacggga 831240
gctaatccca ccccgaaaaa cttattttca cagtgaagca aagcgccgtt tacattgtac 831300
atcattaaga aaatgccaca gggcaagagt atcatagtaa ggagaatcat atcgtaagtc 831360
ccctcttcaa cgtattgaag aactacccac aatactgctt caataagcag agtgaatata 831420
atagtgctgc ctttaatcaa tctagaaaag cgtcggaaaa aaaacgccgc acgatcgaga 831480
ctttgagcac ggagaaattc aaaatgaggg atgaaggctt gttctagaat gagccctcct 831540
aaaatttttc ttaagaaaaa aacagtacgg aaacctaacc agaaagcagc tacaattgga 831600
tcagctccaa aataggttgc cattgcaatt tctcgaaata tccctgtaat acgactacag 831660
aaagttccgg ataatatatt aaaaattgaa cgagctaagg aaacctcatt gtcttttctg 831720
ctcattaaac cgttttcccc tcctccttta aaacaagact atctcttcga catttccccc 831780
tatacttctt cagaaatcac aattggagga agctatttca agctaaataa agcatcttta 831840
cagagctcta cattgcgttt gagaagcata agtatcattt cataatctct atgatatcga 831900
catagaaaga agcctttgtt tcataacacc gtaaacgata cctgtcagca gcttttttct 831960
atataaaact gatgctttct tcttataaaa agagcttttt atggatgatt ctttaaaata 832020
tattttttct gtattattgc gatactgcgc attcggtttt gttcaaaact caagaatatg 832080
aaattctgtg ggggaagaca tacgaaagtt attattaaaa tttttgaaaa attgaaaaag 832140
cctcaagaaa tgagcagctc aatcaaagga cctaggtttc ctctgaaact gggtagttaa 832200
gaaaagacct tgaaatttta tgaaagtact tcctcctccc tccattccct tactaggggc 832260
tcacacttca actgctggtg gactcaaaaa tgcgatttat gaaggccggg atataggggc 832320
ttctacagtt cagattttta cagcaaacca aaggcagtgg caaagacggg ctctaaaaga 832380
agaagtgatt gaagatttca aagcagcgct caaagaaact gacctttctt atattatgag 832440
tcatgcagga tatctgatta atccaggagc ccctgatccg gtaattttag aaaaaagtcg 832500
gattggcatt tatcaagaaa ttctggactg catcacttta ggcatttctt ttgttaattt 832560

tcaccctgga gcagctctca aaagctctaa agaagactgc atgaataaaa ttgtcagcag 832620
ttttagccaa tcggcccctt tatttgatag ttctcctcct cttgttgttt tactggaaac 832680
cacagcgggt cagggaacgt taattgggag taactttgaa gaattgggtt acctcgttca 832740
gaatttgaaa aatcaaattc ccattggcgt gtgtgtagat acttgtcata tttttgctgc 832800
ggggtacgac attacctctc cacaggggtg ggaagatgtt cttaatgaat ttgacgagta 832860
tgtcggttta tcttatctac gagcctttca tctcaatgat tctatgtttc cattaggagc 832920
gaacaaagac cgccatgcgc cccttggaga gggctatata ggtaaggaat cttttaaatt 832980
tttaatgaca gatgaacgaa ctagaaaaat tcctaagtat ttagaaaccc ctggtgggcc 833040
tgaaaattgg caaaaagaaa ttggggaact tttgaagttt tcaaaaaaca gagatagtta 833100
ggaagttttt taagtgcttt tagatcccga agcaatccag tagatcttct gaaatcaaaa 833160
aaaacgccat actgatatac agttggcgtt ttctagaaaa gattctaaat caactacact 833220
tatgttctat gagataggaa ctcacaaaca acagaaatgt tgataggtag aggcaattga 833280
gcctcgattt gatcttgctc aggagataca agtaattccc ctttaaagcc tgtcttatct 833340
aaagaaatgt aagaaggcag agaactttcg tctttgcttt ctagggcatc ttttacagac 833400
tgaagtcgtt tagatttttc cttttaagag aaatctgcat tccaggacgt aaaaagaaag 833460
agcgtctatc gactcttcgc ccattaacta agatatgtcc gtgagcaaca agctgctgag 833520
cagcaaaaat tgtttttgca aagcccatac ggtataccat gttatcaaga cgacactcaa 833580
atctttcaag gaacatttga gcaacatttc cctgcttatg tataacttct ttgaaagcct 833640
taaccagctg cttttccata atcatgccgt agcaagcttt aagcttctgt ttctcttcca 833700
gttggagccc atagtcagac tttttctttc tctgcatacc atgttgacct ggaggatgag 833760
gcttctttaa caaaggattt cggcttctgc caaagatgtt cgcaccaaaa cgccttgcca 833820
ctctattttt agggccacaa tatcgagcca tgtatttcag tccttatatt taatccttgg 833880
aaatcatcta tgccccttga ttttcatcct agggcaattt tggcactatt ttacaaagct 833940
caaggtgcgg agtaaaataa aaataaaacg cacactcata acttgacaaa ataccaaaga 834000
gcattttagt tctaatggca attttctaca atgcaattat ttccaagatt atgttagagt 834060
atttatcatg gaaaaaaaat attatgcact agcctattat tatattactc gtgtggataa 834120
tccacatgaa gaaatcgctt tacacaaaaa gttcttagaa gacctcgatg tctcttgtcg 834180
tatttacatt tcagagcagg gtatcaatgg acaattcagt ggttatgagc cccatgctga 834240
gctctatatg caatggctta aagagcgtcc taatttttct aagattaagt ttaaaatcca 834300
tcatattaag gaaaatatct ttcctaggat cacagtaaag tatcgaaaag aacttgctgc 834360
cttaggatgt gaggtagatc tttccaaaca ggcaaagcac atttctcctc aagaatggca 834420
cgaaaaactc caagaaaatc gttgccttat tctagatgtc cgaaataact atgagtggaa 834480
aattggtcac tttgataatg cgactctacc tgatattcaa actttcagag agtttccaga 834540
gtatgctgag aagcttgctc aagaatgtga tcccgaaacc actcccgtta tgatgtactg 834600
tacgggggga attcgttgtg agctttactc tccagtttta ttagaaaaag gctttaaaga 834660
agtctatcaa cttgatggtg gtgtcattgc ttatggacaa caagtaggca ccggcaagtg 834720
gttaggaaag ctctttgttt ttgatgatcg cctagctatt cccattgatg agagtgaccc 834780
tgatgtggcc cctatagcag aatgttgtca ttgtcaaact cctagtgacg cttattacaa 834840
ttgtgcgaac acagattgca atgctctatt tctttgctgt gatgaatgta ttcatcaaca 834900
tcaaggatgt tgtggtgaag agtgctctca aagccctaga gttcgtaaat ttgatagttc 834960
acgaggaaat aagccttttc gacgtgctca tttgtgtgag atcagcgaaa acagtgaatc 835020
agcaagttgt tgtttgattt aatacctatt ctggtttctt catctgggag ttgtaaaact 835080
tagaaaagtg aagaaaccag aacacatgtc tcttaaaaaa aaattactta gagttgacca 835140
tataatacgt ctcatcgctt tccagggcat tctctaaatg atttttaatt ttctgtgaca 835200
aaatatttgt tactacgttt tgtcggtaat ttccatgtac aatgatatca gcatatttcc 835260
gagtcggctc tataaatttc tcatgcatag gctttaccat agaaagataa cgagacatga 835320
tgcagtccac gctatctcct tgttcttgaa catctcgaac catacggcgt agtatccttt 835380
catcagcatc ggtgtctaca aagatcctaa tatccataag atctctaagt tcttgatttt 835440
caaagaccag aataccttca acaagaataa ctttagatgg atagatcgtt tctatctccg 835500
ttttagatcg attacctaaa acaaaatcaa aaactggggc ttggacaatc tcattatttt 835560
ttagacgttt tatgtctgaa attaataagt cattatcaaa ggcgtccgga tgatcccaaa 835620
ttaaattggc acgttcttca ggagtataat gagatctatc tttgtaataa ttatcttggc 835680
agataacact cacatcctca ccgaaaattt ctttaatgtt ttgggttagg gtggttttcc 835740
cagctccaga acctcctgta attccaataa tcatcataag catcatcaac ataaattttt 835800
ctccttagta cttctcttgt aatcagcaca caacaaaatg cgggtccttt gagaatttat 835860
tgatttaaaa ggagacaaaa gaaaaagctt cgtgatcaga aattgaaatc tttagatata 835920
gtcattttct gatcacgaag tttaggatag aattctatcc aaaaaaatat ttcatccccc 835980
aataaacaag ggatgttata caaaacccca attcacgtat agatagattt aaaaaagcaa 836040
aaacactgtc agaatgcctt ataagaacga tttacggtat cttagatgtt tcctaaagga 836100
gtacaagact ccttatgcat gaggaaacac tagagtataa atcactcttt ttttcccttc 836160
tttctgaaca agttgtatcg gatgcaagtc gataccttgt tcttgtagta tggatgtaaa 836220
tctctctaca ttcttgggta gtcccaaaca tacaacactg acatcaggaa attttttcaa 836280
agatgttttc aggtgatttg cttgtagaac agcccaggca tcttgggcct cagaatcgtt 836340
atctctaaca ctagagacaa ctgtgggaac acaccaagac aataaccacg ctctccatcc 836400

tggcactaat ttttggtcga gaatgatgac tctgcttctc actgttttgg ttagagcagc 836460
taaaacacgt cggatttccc tataactttg tacggcaaca aaataacatt tcgggctcga 836520
agactggtgg atttttaaat agtaacctag ctgccgtctt aatatgatac ccaacaacag 836580
gcgatagacg tgttctctcc atatccataa tgtccagata gagacagcga gcacaaacca 836640
accgatatag aaaaaacttg tttcaggaga caagcctaag ttacttccca aaactcggat 836700
cactcctgca gcgactaaca ctccaaagaa atctaaaaag ttattggcag ccaaaatttg 836760
ccctctctta tgttccgggc ttgcatattg tacataagca tggagaggca cttgatacac 836820
cccaccaaga aatcccagtg cgagtaaaaa aaacaacacg aatagtatcg aacaagcaaa 836880
ggcatacagc cccataaata cgagagctag gccgatagcg gccaaaggga cgtatcctat 836940
tttgatatct tttcctgaga ttttcccggt gatgtacgac cccgtgccta cacctaacgc 837000
cactatagga aacaagtaag cgccataatg cttaggatat tttaaaataa attctacaaa 837060
agggatgatc tctagctgag tataagcacc tatcaataag aagaaagatc ccaaaaaaat 837120
cgatacagtc aaataatgaa tcatccgggt atctttcaaa actttccata aatctttgaa 837180
actcaccaag gtgattttct gtttcacatt tttcacattg ctaggtcgga tacagaagga 837240
aataagagta ctgataatag aaacaataac acacattaag gtaggccaga catagctatt 837300
tacacctaaa cgatgagtta catcgacaag aagaggagca aggcaagaac ctaaaatact 837360
tcctgtatag gtggctgctg tcataatccc gttggcttgg gagagctgtt ctgagggtag 837420
catttcggga agaatcccga gctttgctgg cccaaagatt gtggtgtgac atgccattaa 837480
aattaagact acataccccc caactacaga ttggataaag aaaaagtacg ttccgagaat 837540
tgtacataaa atctctataa atctggttgc taagataata ttccgtttct gaaagcgatc 837600
tgctaaactt ccagctagcc ggagctagta aaagaaaggg aagggcaaaa aagaaactta 837660
cacaggagag aatctttgca ttctctgtta gagttttgcc ttccaatagg aaaaacgcta 837720
aaagaaattt atataaatta tcgtttataa tcgtcaggaa atgtgtagtt actagtgccc 837780
taaacgattt tttttaaccc gaaacagtca tgaatgccac caaacattgt cgagcaagct 837840
tcagtaactc gccccgtcac ctcctagctc agcttgcgga ggatattacg tccacacatc 837900
aaaaaccttt taccaaaagg tggattcttg ttgcaaatgc tactacaggg cactggataa 837960
aaaaccaact tgtacatgtc ttgtcagacc acatctttat gggatcaact attttcactg 838020
cttcggattc cattgtcaaa cacttattct tgggttcggg ttgctcgcag cccaatattc 838080
cggactacct tacccttccc ttgttaataa ataatatttt agaagaaatc tcgaaggctt 838140
ccaaatttga aaatggaagg gagtttttat ctccacccac atacggnaca acaaaaaaac 838200
ttgctgctgc gtttaagcag ttccatacgt tttcacaacg cccgaccaaa aacgcctccc 838260
attaccaaga attatttcaa atcttggaaa gccatttttc ttcttatgaa gagatgttca 838320
ccactatttt aaataatcga acgcaagagg aggactgctc ccttcatatt tttggttatg 838380
ctcatcttcc caaacatctt gcggaatttt ttattaattt aagtacgtat ttccctgtat 838440
atttctattg tttttctccc tgtcgagaat attttggtga tttactttcc gatagagcta 838500
ttgatttctt ttggaatcaa cttcccgact ccccaataaa aaatgcctgg gaacactatg 838560
tattatcaga caggcaagca cttcttgcaa acttagctca taaatctcag tcgtcgcaaa 838620
attttttcct agatagggaa atagactatc aagagatgtt tctcccttca aaacacgata 838680
gttctttagg ggtaatacag aactctattt tagacctcaa gcccacgtct cctcaagatt 838740
tctctcaaac aaagcagacc atttgtattt atagagctct aaatattccc agagaagtcc 838800
aggaggtatt ttgtaaagtt acagaacttc tgcatcgcgg agtgtcacct gaggagattt 838860
ttattctctc ttctcacata gagagctaca aggtacatct aaacgctatt ttcaatcctc 838920
atgtgcctat atactttact gatgaagtag atccacgggc tgaagatctc agaaataaaa 838980
atcctcctac tttcttctat tttacaaaca caaggggatt tacattacat tcttcaactc 839040
cttacgcacc cacaactaca acaacctata gatcaaaaca aggttcccta tctgattaaa 839100
aagctttcct cagaatgggg aaaaatttct tcaaaagaac gagcttcggg tcaacaaatg 839160
aaagctctag gtgatctgat attagaagaa tacccattcc atcaagaggg tgggcgtgtg 839220
agccaagtag aagtttggga aacaacagta cctttaattt atttcattca agagcgtatc 839280
aacctttatc tttccagctc ccaacatagc tatgaagatc tatttcaaaa cgtgttttct 839340
tgtttagaaa agatttttgt tttatctccc gaagagacct ctttcattac aacgttaagg 839400
aactctcttt tcccaacctt tgctacatct tcctgttctc ttcttttttt cactgatttc 839460
tgtttagact ttttgcttca tttccataaa cccagtcccc tgtatgacaa gccaggacct 839520
tacataggta gtttgagtag cctcagctta attcctaaag gctatgtctt tattttagga 839580
gctaataaaa caacatcgtc tgacattttt gatcttttaa ataggacaac aacacatgaa 839640
gagcttgcat tttcttctac agaagacgag gaaaatttcc acttcctaca aattttagtc 839700
tctacaaaac atgaacttca tattagttat atatcatcgg cagcgcaatt caaccttcct 839760
agtccctttc taaaccatat taaagacacc ttagacctgc ctgtagaaac gttacctaca 839820
caaccctacc tctctgcttt cttcaagaat aaagcttgtt tacacacctc tcaagaatac 839880
aactactctc ttgctcatgc tttctattct aaaaaagctc tcctcccttc cttgtttatt 839940
ccaactgtaa agcaggtaaa tcttcctcaa catctctctc tcaatgaaat tatcaaagga 840000
atcttttctc ctttagacct ctttttaaaa accaattaca atctcagaat ttcctaccca 840060
gaacacctta aaaaacaaca gaaactcttt ccaacaaaac atcaaatcga agacttttgg 840120
aatgaatgtt ttgtagacaa agagcatgac ctgatcccta gtatctctcc tcatgctgaa 840180
gagcttttta cttactatag ggaaaagaca atcctattgc gtaatggatt ggacaaagat 840240

ccgaaacatt caccttatac agtcacattc tcttcctcaa ttttcgaaga gagaccctat 840300
catgaaagtt accttttccc gcctctttct ttatctttcc aaggaaatcc ggtccaaatc 840360
catggaacaa ttcatggggt atgcaatgag ggactttatt tatgttctat agatcctaga 840420
gattccctaa aaaaaacaac cagaacccta gggagtctcc cagaaacctc ttctgaacaa 840480
aaacagctct tagaaagata tgtagcgttg gcggtgttac aaatgtctca gcacctctct 840540
tcagattcgg ctttaataaa gcttacatcg ttcaatacta aagaaaacca ccatcctcct 840600
ttttcggatc ctgaaggtta tctccgtaaa gttttagaag tctatcacct gatgtcttcg 840660
caacccattc ccctactatc tccattatgt tggaaaacct tagacgatga agaaaaattt 840720
catcaggcag tactttctgc tataagtgaa gaagctaaaa atccttctct tcctattttc 840780
tggcagtttc acaatcgtaa tatcgaggag atcttaaatc acgtgggtgc atccgaacgt 840840
ttgaaaattt tatctctttt cagaggtccc tgtgaagccg tttaatattt ttgactcaaa 840900
ctcttcgatt cagggaaaat ttttcctaga agcctctgca ggcacaggaa aaacatttac 840960
tatagaacag atcgtgttgc gagccttgat tgaaggctcg ctaacacatg tagaacatgc 841020
cttagcaatt acatttacga atgcttctac caatgagctt aaggttcgca tcaaagacaa 841080
tcttgctcaa actttaagag aattaaaagc ggttctaaac tctcagccgg cttctttacc 841140
cacatattta gatatcaatt gcaatgtaaa gcagatttac atgcaagttc gcaatgctct 841200
tgctactcta gatcagatgt ctttgtttac gattcatggc ttttgcaact ttgttctaga 841260
acaatatttt cctaagaccc gtcttattca caaaaaccct gctctgaccc actctcaatt 841320
agttcttcat cacatcacta actatttaaa acaagacctc tggaaaaatg tactttttca 841380
agaacagttt catctattag cagttcgcta caatgtaacc tcgaagcata catcttctct 841440
ggtggataag ctacttgcca gctataccca accaatctcc tcctactttt cctcacgtgt 841500
tgaaagactt gagcaaattt ctctttggca tcaacaaata tacaactctc ttttagaaat 841560
tccaaaacag gtttttctag atcagctaac tgcccacatc tcaggattta aaaagcagcc 841620
cttttccatt cttgatgatc tccatcattt tgtggatctc ctttacactt cggagaccca 841680
tagctcttta ttttcattct ttaaaattgc agaaacattc aacttcaaac accgtcttgc 841740
acgttacaag ccttgtgctg cctttactgt tttagaaaac atgtcttggg tagagcgtac 841800
tttagagttt tgtaacttgg atcgaatctt caatactttg ttagtcgatc tccaagagta 841860
tcttaaacaa aattataccc cttggctctc tcccgatgaa agcgtctttg ccctcgagaa 841920
actactctct tcatctgaag ctcaacctgt agttcaagct cttagagagc aataccagct 841980
cgtattaatt gatgaatttc aagatacaga caagcaacaa tggagcatct tttcgaatct 842040
ctttatttct ccgaaattta caggatcgtt atttcttatc ggagacccca agcaatctat 842100
ttatgaatgg agaagtgcgg atcttcctac ctatcttaca gccaaatctt cgttttcaga 842160
agacaagcaa ctacagcttg tcaattaatt accgctctac acccaaactc atggaagcca 842220
tcaaccaaat attcgggaaa atctctccat ttttagagat ccctggctac ctacctatag 842280
aataccatgc gctaaatcct cagagtagtg agacatttga aaatcccccc cacgctccta 842340
ttcatttctt cttttatgaa actattaaag accaggcatt atggatattc tcagaagcgc 842400
taagactaca aaaagaacaa aagattcccc tagggaatat ggttgtcctg gtctcagact 842460
caaatcaagc ttttgagtta atttcctatg cgactattcc tgtttccttt tctaaaaaca 842520
aatctatatt tcatcttaca gaaactcaca tactgactac agctctacta gaagccattc 842580
ttcacccgga gaattatgaa aaaatcagca agatattgtt ctcatctctt tttggacttt 842640
ctttagacga agtnacaaca aaaaaagaag actttacgat ttatttccaa tcactacata 842700
gctacatctc acatcatgga cttctggcta cattttaccg agtgatgact acgcaaggaa 842760
acgtattgtt ctcatctcct agaggggatc ttatttttca ggaaatggag aaactttgtg 842820
gttacctaga tacaatttct tcttatccct accaccaact tcttcacctg aaaaactttt 842880
ctgaaacagg acggtgggaa gaagaactcg ctatatcttc ttattctgag gacttggaaa 842940
ctttaaaaat cactaccatt cactcttcta aaggtctcga atacgatata gtcttttgtc 843000
caggaattga gaaaagtaaa aaaaataaaa gctcttcaga attactaaga gaaatgtacg 843060
ttgcttgcac gagagcaaaa aaacagctgt acctacctat aagcacgcaa ccgccttctc 843120
ttcagagaag ctccgcatta acaaattacg tgaaattaga aggtacgcag agttcggctt 843180
atgacttagc tatccaccta catcaagaac atcctgattt attttcgtat tcgctaccta 843240
aggaccatgg acatgctacc acagtgttga atctgccact tttagagacg ttcgctctaa 843300
aagtgacacc cccgaaaact attttttcct tctcatctac aaaattccta ttggacactc 843360
acaaagactc gcaatcgatc ccatattcca actcccgatt tcaaaacaac agcttcctct 843420
gggagaaaaa acaggaattc ttatacacaa aattttagaa tctattcaat tttctctatt 843480
acaagacact gagtacttga tgtctacgat catgcgtttc ataaaacaca ctcatcttga 843540
aggattcgag gaaacgattc ttaaactact tagtaagacg tttttttctc ctttaacatt 843600
ttcatcgcag acattttctc tatctcaagt tctaccgaat aagatatttc gagagacttc 843660
ttttttattt ttagagaacc aagagctgtg gcaaggggtg attgatcttt tttttgagca 843720
tgaaggaaag tactacatta ttgactggaa aacatcgttt ttaggagaga caaactccga 843780
ttactcaaaa agcaacctat ctatctacat aaaacaagaa aaattagatt accaaggcag 843840
gatctacgtc aaagctgtca ggaagttttt aaatcaattt gaaattgatg atgatgtaga 843900
gcttggagtt atctttatcc gtggcataga cacccaagga aatgggtttt tcgctttaaa 843960
tagcagcgaa gacattccta acttcaatcc caaagcaatc caaaaatgtc aggcctatca 844020
ttaggattaa attctagggg aggtaaaata aagagctgat ccagctccat aagcttttct 844080

tccaaagact gagcttctat cttgaaagta caggctccat cacgaggagc ttttaccttg 844140
gtgactctag ccaccaacag tcctggggga aataccccat ctaatcctgt tgtcactaag 844200
atatctccag gtagcagagt ttttccctcg ctaaagtaga atccttctcc ctctaaacat 844260
aaagaccctt ctttccataa agccccaccc actccagaaa gtattcctct taggagagct 844320
tggttttctc cttctccctg tattaaagaa tctaactctt gtagctgaga gattttttca 844380
tacttatcct tttcaaggat ataggcgtgt gaaatctgct ctacttgtct gattagttct 844440
ctaagactgt gttttatcca ccaagactga atatcaccgc gcatagcaac tacagagggc 844500
ttcattccta catctgtgat taatcgtata cgggattggt gttctccaac ataatcgaca 844560
agtcctacta gaacatttcc agaaagcacc ggagaatttt tcttgattcc atgagttttt 844620
cctacattca cccaacaaga actagaccaa tgtgtgtaat ctcgatagac aacctttccc 844680
tctaccaact tatgaaaata gggagttaag atctctggaa ataaaggagg agtgtgatta 844740
gagacctcat aaagtttgag tttctcttct aaagaagcca ccctctcctt aagaacgagg 844800
ttctctaatt ctaaattagc aagatgagaa gaaggagctt gtttaatttt agggaaaaat 844860
ttgacgtgta gagagacaaa actgcttctg atcttatcat agacctcttg agggatgctt 844920
cgaaaagaga gaatccctaa agcaataata atatagacgc agatttttgt ttttttattg 844980
cgtaggctat agctcataag cttgaataaa actttgcact acatgatcta tatttttttc 845040
cgtaattcca tttaaattca ttcttcctcc cgctgttgta taaacagcgt gttgttctct 845100
taaaaagagc acctgtttat cggaaaaccc agggtatgca aagaatccat gttgggacaa 845160
taaaaagtca aatgtatgac cggcaacctt acgcaaagct tgaacaaatc ttgttctcat 845220
tttacccaaa gactctctta taaaattgag ttctgattgc cattcttctt tcaaatatgg 845280
attactcaaa attgtagaaa caatttctac accccaacgt tgtggtgaag agtattcccc 845340
tcgaattttt tcttctaaga aactgtgaat tttaaccaac tcatcagtaa aagtactgtg 845400
aacagcaaaa tatcctacac gctcaccata aagagcaaag tttttgcttg atgaagcagc 845460
gacaagaacc gtatttccct ctgatataaa aatttctata ggttttctat ccaactctat 845520
gccgtgagca aaaccttggt atgcggtatc aaaaaatgga attaactctc tttctttcat 845580
taagatggcg agttctttcc acatatcttc agtaaaatct acacctgtag ggttgtgaca 845640
acaaccatgc aataagataa cggaattctt ttctacttcc ttcaaaaatg cgatcagggg 845700
ttcaaataac agctgttttt gttctttgct ataataagga tatcggatta cctcaagacc 845760
ctcttgagag aaaatgcgta tatgattgct ccaagtctgc tcgggaacat aaaccttacc 845820
agatcctttt gccacggaaa gaagccgggc tcccaagtgt agcgctcctg tacctcctaa 845880
agactgaaac ccgactatag cactcggatc tacagcacca aaaaccagct cacgcatctc 845940
atctaaaaat atttgcaacc ctgaaatagg aagataactc ttattttgtt cttcttccaa 846000
aatgacagtt tgtgccttac gtatacaaga caagccgccg taacgctttt gcggatgctc 846060
ataaacacca atcacaaggt taaccttttc gggacgctta tctgcaaaga aaacgttctg 846120
caaacctaaa atagcatccg gagaaaatgt tggtatgtga ttaaaaaaac tcatatatga 846180
ctacacctaa ttataacaaa gattgattct aaaccctgga acatgatact tttttgtatc 846240
ttttggggta ttagctcagt tggtagagcg caacaatggc attgttgagg tcagcggttc 846300
gaccccgcta tgctccatgt tcttcccaaa tggattgtat ccgagaaatc ttatactcct 846360
ttccttgcag tattaccacg tcattcaact tcttccctaa catgttctgc gcaagcttgg 846420
attgtaaaga aagtatacaa ctatctggat cggcatccca aggtcctaaa attgtatact 846480
ccacaacctc tccagcatcc ccttttaaag taactttaca gccaacacca actttatctg 846540
taaagacaag gtcttttgta agaattctcg cccgattaat ttcttccgat agcacgcgaa 846600
tttcttcttg taagcgagct ctcttctcta aagcaaactt gtactcagaa ttttcccgca 846660
aatcccctaa agaacgagca tcttcaattt ccttagcatt atcaaccatc tccttgccaa 846720
caagagattg tagcttggct ttcattcttg aaaaactctc agaagtcgac cataaaacat 846780
tttcttcttc tacattagac ttgtgctttt ttagcgttgg ctgaacaact tcagcaaggc 846840
tttgtaaaac gttcaaatca gaagaagaaa actgcgggca tttggtggat agtaatagta 846900
attcttttaa aaagggaagg gatgcgcctt ctatcatctg acgcacagcg agataccttt 846960
gccccaccaa gtagtggtgt aacttttttc ccagttcttt atgaggtgta gangctactt 847020
gatacataaa attcaaagct gactctaaga acaatcttaa gacttccttg tcttcaggat 847080
caaagagacc atcttcatgg ttcccaagct tcaagaaaaa ccaaacaaac agctcaggga 847140
acatcattgg ttgatgggca ctatctagaa gtcttttctt taaaacctct acgcttgatg 847200
gatcgttttt aatggtttta tatacaaaat ccctcatggt tggagaggtg gtataaagaa 847260
gaatttgcat aaaaacttgt tgccagaagg aagagtactt tcttaccaaa gacaaaaagg 847320
acttttgcaa agcaacaata ggcatgtttt ccaaaagacg actggtatca tcttctgata 847380
aggatgttat atactctttg tctatgctcg catccttaat tcccaaatac tcagaaagaa 847440
gcagttctct ctgcaaaatt aaagacttat taccttcctc aacatcaaga tcttgcaaag 847500
ctttaactag actcttgcga atctctatat ttttcaattc actatgcaaa tctctaataa 847560
aatgatagat cagagagatc ttttctgcgc tattgagact caatcccaac ttacgctcca 847620
actgtcccat atgagaacac cctgcatcag ataaaacata gggctcttta ggattgtctg 847680
gggatataat tctagttccc tttttaatct tagtcttagc tgattgccac caccgattcc 847740
aatctgcctc aggaatcaca agatcaacta actcgtcttt gatttccttt gcggtctttg 847800
gtccaagatc tctaaggaga atctcaacaa cttcaatagg gttttcctta gcaaaagcct 847860
caaacccatc gggatctcca aaccgtcgtg aaaggaagtg atcgccgctc aaaggagtta 847920

aacttttaaa tgccgtctcg aaagaaatat ccttggcact cataattcct tcaaactcta 847980
tcaacacctt ctgctgaaga aacgagaccc ccataacctc gccaacaccc caaccccctt 848040
gatggaagac aaagtttccc ttatgcatat gcatcaaaaa atcaaaacgg ctgaggctaa 848100
actgaaaatc acgaccatca cgaagcccaa caacacgcaa agcctcgtta aagttttctt 848160
ctccactgta ctttttattc acatattcag tggctatatc aaaaaacatc tggctgttag 848220
atgtttgcaa atctaaaatt aattgaagaa ctcgatcttt atcctttccc tcgggtattt 848280
tttcccaaag aggaacgacg gtatccacaa tctttccaaa taaagatgct aaagaggaag 848340
actttacttt ttctaggatc tcaacaagct cccgcccccg cactacatcg ttaaaacaat 848400
actcctccca aaggcttaaa aagttcgcag attggccttc ttctattaag acttgcaact 848460
tttctaaata gtccacgatg tctccagttt ttaacctaaa aatatactct tacgtatgaa 848520
aaagagcaat atcacaatac aaaataatca aacttatttt ataataaact aaaatgatgg 848580
tgattgttat gaatagtaaa tctgcgcaaa aaataataga ttctataaaa caaatcctaa 848640
ctatttataa tatagacttc gatccctcct ttggatcctc gttatcttca gattctgatg 848700
cagattacga atatctaatc acaaaaactc aagaaaaaat ccaagagcta gacaaaagag 848760
ctcaagagat tctaacacag acaggaatgt ccaaagaaca gatggaagtc tttgcaaata 848820
atccagataa cttctctcca gaagagtggc tggccttaga aaaagtccgt tcttcttgcg 848880
atgagtatcg aaaagagaca gaaaatttaa tcaacgaaat taccctagat ttacatccca 848940
caaaagaatc aaaacgccca aaacaaaaat tgtcatctac caaaaaaaat aagaaaaaaa 849000
actggattcc tctataaaat cacttataat cccgattgaa tgaaacaccc aaggacagag 849060
tccaagggac ttccctttta aatttttatg aaaattacag tcaatcgggg tttagattta 849120
tccttacaag ggtctcccaa agaatctggt ttctataaca aaatcgatcc agaattcgta 849180
tctatagact taaggccatt ccaaccttta tctcttaaac ttaaggtaga gcaaggagat 849240
gcggtctgtt caggagctcc tatagcagaa tacaagcact ttcctaacac ctacattacc 849300
tctcacgttt caggagtagt taccgctata cgacgtggaa ataaacgttc tcttttagat 849360
gtcatcatta agaaaactcc tggtcctaca tctacagaat atacgtatga tctccaaact 849420
ctctcacgtt cagatctttc cgaaatcttt aaggaaaatg ggctctttgc attaatcaaa 849480
caacgtcctt ttgatattcc cgcaattcca acacaaactc cgagagatgt tttcatcaat 849540
ttagctgaca atcgtccttt tactccaagc ccagaaaaac atctggctct cttttcctct 849600
agagaagaag gattttatgt atttgtggta ggagttcgag ctatagctaa actttttgga 849660
ctccgtcctc atatagtttt cagagatcgt ttaactctac ctacgcaaga actaaagaca 849720
attgctcacc ttcataccgt ttcgggacca ttcccctctg gatctccatc gatacatatt 849780
cacagcgtag cccctattac caatgagaaa gaagtggtat tcacactgtc atttcaagat 849840
gtccttacta ttggccatct tttcttaaag ggaagaattc tgcacgagca agtcacagca 849900
cttgctggta ctgcactaaa aagttctcta agacgctatg tgattactac aaaaggagct 849960
agcttctcta gtttaatcaa tcttaatgac atctcggata acgatacatt aattagcgga 850020
gatcccttaa caggaaggct atgcaaaaag gaagaggaac cctttttagg atttcgagac 850080
cattcaattt cagtcttaca caacccaaca aaacgggagt tgtttagctt tttaagaatt 850140
gggtttaaca aaccgacatt tacaaaaacc tatctctcag gatttttcaa gaaaaagcgt 850200
acctatacga atccagatac taacctccac ggagaaactc ggccgattat tgatactgac 850260
atctatgata aagttatgcc gatgagaatt cctgtagttc ctctgattaa agctgtaatt 850320
actaaaaatt ttgatttagc taatgaacta gggtttttag aggtttgtgg tgaagatttc 850380
gccttaccca ctcttataga tccatctaaa acagaaatgc ttaccatagt caaggaatcc 850440
ttaatagagt atgccaagga atcagggatc ctaactcccc atcaagattg attttttgtt 850500
cttcaaagaa cttaaaattc aaatccctga tgcagtaatt ccaaaataaa aggagcggag 850560
taactaataa tcatatctgc gccagtctct tgattgctat caaagactca tgaaatagtg 850620
tttctttatc taaccaacct tgttgaaagg ctgataagat catagcgtat tccccactga 850680
cctgatacgc agctagaggc aaacaggtat tttgtcgaat ccgatagatc acatcaagat 850740
agagtcctgc aggcttcacc attaagatat ctgctccctc ctcctcgtct aaagaagatt 850800
caagcaatgc ctctaatacg tttttagggt tcatctgata ctgtttttta tctcctgaag 850860
ttacgtgaga actcagcgca tcacgaaaag gagaatacaa acaagaagcg tactttacac 850920
tataagacat tatagaggtc tttgagtagc cagactggtc taacttagag cgaatgtagc 850980
caatccttcc atccatcata tcactcggag ctacgatatc cgctcccatt tcagcatgta 851040
aagttgcaat atttccaaaa attctaacac tttcatcatt aaggacctct ccattaagga 851100
aaatcccatc atgaccgtgt gtcgtataag gatctaaagc tatatcacta atcagacata 851160
ggtgaggaaa tgcgtttttt atttcatgaa tgctatgaca taagatgttt ttaggatttg 851220
aggagtaaga accgtaagca tctttaagat catcaggaat aatgggaaac agcattacag 851280
ctcgtaaccc gtaggtacac aaacgctcta tttcctttaa tagcaaatcc aaactccatc 851340
ggaacactcc aggaagactc gggatctctt cctttatgtt atttccatac ttcacaaaga 851400
atggtgctat gagatctttt gggcttaagt gagtttcggc caacaaatct cttatagctg 851460
ctgtctttcg gtttcttcta ggacgtctac ttagtgttaa agaactcatt tctcagatcc 851520
tttcaacagt ttccacaggt cctaaatata tattgttata tttatacata atattctctt 851580
cttctttagg gggtgtggaa actgtttaaa actttgtttt tcgagggaag tttatgtggt 851640
tttgttgttc aaaactttgt taaaacattg ttcattttgt tgataagtga ggattttgca 851700
tgtttataaa cacaagatca accctttttc tacatcaatt tttcaaagaa aaataggatg 851760

agggaatttt tgttagttgt gtagaaactg tgatcttttg gctttgattt ggataaaaaa 851820
tttctgtttt ctctctgttt tttgtatgca ataggttgta gaattacaaa aatttctctt 851880
gtgatttcgg aggtttgagg tgaaattttt tcaccgatac ttggttacaa aaaccataag 851940
ctaaagtatg cttggggtca taagcgacgt tatacgtcgt gctcccctgt agggatgagg 852000
cgcgccaatc gtcgaagcag gaatccactt aagcaagttt atattatccg agtatattag 852060
tagttttcgg gaagatgtcc gttcagtttt gaggttttca tgttgggcaa agaagaagag 852120
tttacgtgta aacaaaagca gtgtttgtca cattttgtta ccaatctgac gtccgatgta 852180
tttgctttaa aaaatcttcc agaagtcgtt aagggagctt tattttctaa atactcccgt 852240
tcagttttag gtttgcgagc acttttgtta aaagaatttc tatctaatga agaggatgga 852300
gatgtttgtg acgaagccta tgacttcgaa accgatgtac agaaagctgc ggacttttac 852360
caaagggttc ttgataattt tggggatgat tctgtaggag agcttggcgg agcacctggc 852420
tatggaaaat gtctctattt tggctgctaa agttttagag gatgctcgaa ttggcggatc 852480
cccgctagaa aagtccacaa gatacgtcta tttcgatcaa aggtacgggg ggagtattt 852540
atattaccga gaccctattt tgatgacttc ggcctttaaa gacatgtttt tgggtacttg 852600
tgatttttta ttcgatacct attctgcttt aatccctcaa gttcgtgcct attttgaaaa 852660
actgtatcct aaagattcta aaacacccgc atctgcctat gccacatcat tacgagctaa 852720
agttttagat tgtatacggg gacttcttcc tgcggcaact ttgacaaatc taggattttt 852780
cggtaacggt aggttttggc aaaatctgat tcacaagtta caaggtcata accttgcaga 852840
gttgcgacgt ttaggagatg aatccctaac agagcttatg aaagttattc cttcatttgt 852900
aagtagagcc gagcctcatc atcaccatca tcaagctatg atgcaatatc gaagagcttt 852960
aaaagagcag ctcaagggac ttgctgaaca agcaacattt agtgaggaga tgtcttcttc 853020
accgagtgtt cagttggtat acggagaccc tgatggcatt tataaagtag ctgctggatt 853080
tctttttcct tattcaaatc gttctcttac agatctcata gactattgta aaaaaatgcc 853140
tcatgaagat cttgtacaga ttttagagag cagtgtttct gcaagagaaa accgccggca 853200
taagtctcct cgtggtttag aatgcgtaga atttggcttt gatatacttg ctgatttcgg 853260
tgcataccgc gatttgcaac gacatcggac gctgactcaa gaacgacagt tactctctac 853320
acatcatgga tacaattttc ctgtggagct tctagatact cctatggaaa aatcttatcg 853380
agaagctatg gagagggcga atgaaaccta taatgagatt gttcaggagt tccctgagga 853440
agctcagtat atggttccca tggcttacaa tatacgttgg tttttccatg taaatgctcg 853500
ggctttgcaa tggatttgtg agttacgctc acagcctcaa ggtcatcaaa attaccgcac 853560
tatagctaca ggtttagtgc gagaggttgt caagttcaat cctatgtacg aattattttt 853620
caaatttgta gattattctg acatagattt aggacggtta aatcaggaaa tgcgaaaaga 853680
accaacgacc taagtctcta gatctgtaat tttagatatt gtaaaaacat aatctttgca 853740
tttacaggaa cattctctac tggtattttt attctttttt catgaagaga tcataaaatg 853800
aaagatctta tatataataa cttggttagg tttaaaaaca tttctaaaac caaaacttca 853860
aggtaaaggg tgtcaaagga ggagcctcat ttacagcttc tatagctgta gatatagact 853920
ctgcggattt atcttccgag gattctatgg aggttattat agaaaaatgt gctcagattg 853980
gcgttgctga attccaaaaa tgcgaattcc aatttgaggg tattacctgt ttgtagaaat 854040
ctagtttcct gggtgtagcg cacctggtag cgcacttgca tggggtgcaa gggggcggag 854100
gttcaaatcc tctcatccag atctctctcg gggatgaaga ttttatcttc atcccttttt 854160
tataagaaat tttgttagta aagatagaag ccaaggtggc tgtaagttta aaatagcacc 854220
ttagaatatc cttgaagatt taaaaagatc ctaaactcga gataatcgtt tcgaattcag 854280
aactattctt taatccacca gcactgagac gatccaagag ctctaagcaa ttgttttgag 854340
atcttgctaa tagctctaga gcggcctttt ctccaaatag taaagcataa tttagcccaa 854400
tttgttggga gtctttttgt aaatctgaaa aatcatcttt tatttgaaaa agtaagccaa 854460
aattatttga gaaacttgta attataggtg caaattgtgg gtctccgcca ccaaataacc 854520
aaccagaaat acatgcaatc tcaaatagag aacccgtttt tttgatcata atagattgta 854580
cgtgttcttg acctcggtta gaaaagaaca tatcatcata ctgccctcct aataccccag 854640
aacatccaat atttttgtct gtaatatctc cgataatgtt gtaagcgata tctatttctc 854700
taggatcaca gccctgttct tttaacttct ttgcgtttaa gcgaaggtgg gagtaagcag 854760
caggaattag ggcgtaagat gcgagtaagg cggttgcttc atcgaaagct ttatgtaccg 854820
tcgggcgtcc cctacgctca tcgtcgttgt ccatgcaagg aagatcgtcc gcaattaggg 854880
tggaagtgtg gacaaactct acagctaaag ctgagtccat aacgtcatga tttaagccca 854940
aaccttgagc catcatgcag actaaaccag gtcttaaacg ctttccaccg ccctgtaaag 855000
cgtattctac aggagagcga atcgggtgac ctataggacc aaatccttcc aaagcctttt 855060
ctatagcact ttctatagaa ggtcgatacg tatctaaagc atgtaacaca agagtcccct 855120
atttttaaat gacttgacca ggacgaattc tagtatgagg gagaatatgt tggcctggat 855180
tgatgacaac attgcatcct atagcaaccc cttttcctaa aaaggcacca agtttacgac 855240
gccctgtatc gatttttttt gatttatctg aggtagaacg aacatagatg ttccttccat 855300
ctagacggaa attagcacaa cgtactccag cacctagatt gacttctgaa cttaacacag 855360
aatctccgag gtaagcaaag tgagcggctt tcgtatgatg acctaaatag ctattcttga 855420
tttcagtaca gtgacccaca acacagcgac tgcctgtgat gacattgcct cgcagatagg 855480
ccccatgacg aacttctgtt tgtgagccaa gaatgcatgg tcctacaatg taagccccag 855540
actctacgta ggcatcttca gcaatctcaa tcttttctat attttttaaa gttacaccag 855600

actctacagt accatgaatc ccagaaaaca cgtggttttc cagcatctga tccattaaat 855660
caagaatatc ccaggtatag tgagctttgg agattatctc aggatagaga aagtcctcag 855720
gagaaaatat agacgaggct agataagtca tatagcatac tcaagctatt tgcctcagta 855780
ttttcatact ctgaagattt acttacaaga tataaagaac ttactttact cttcgattgg 855840
atgcgctgta ttgtcatgat tctggagagg aatactgtca gcatctgaaa atagatagcc 855900
tacaccacga atcgttacaa ttttagatcc gtagggacct aatttttttc ttaaagaagc 855960
aatatggaca tccacgttgc gagcaatgat ttctttagta tttcctttaa tctctgctaa 856020
aagattcttt cggagacata ggtgtccacg gttgataagg agttttttaa gaatacctgc 856080
ttcagaaggg gtaaggtata cgcttccttc tggagattct atcacgagat tcaagactcg 856140
gaatgtatga tctccaaaag tcattgtatc tggaatgcta tgctctagaa cttcgtgttg 856200
gcgtaggaaa gctctgatca cggcatctaa tactttcgct gtaataggac ggagaagata 856260
ccctgttgct ccttgattta acacctttgt gatcgcctct tcttgaaatg tatcgaatag 856320
aacaattaaa tcttcttctg gaaagatccc aggagagaaa atttgctcag gtagcaatag 856380
gtattcacaa aatatagcaa cagattcaaa agatgtcgga aacaccggag atacgagtat 856440
ttggtaatca gatctttgtg atgccaagtc ttttaattgc gaagataaac tgagatcctc 856500
agtaacaaac aatatgattt tatcaccgat catatgaaaa caaaatagat tatatgaatg 856560
aatacgtaat ataaaatcag ttatttttca aaggtcactt acactttttt acttttttct 856620
tttttgaaaa atagttttgc ataaacacaa cactatatat ataatttaaa attaataatt 856680
tagaagcgac atagtttttt aacttctttg aaggccgttc aggacgtaaa tgtttcgttg 856740
catattgttt ggtattttcc tactcacgtg tttttcttct ggcggggtgt tatattactt 856800
attctgttcc catgattttt ctatagggcc taaggaaaaa tcacgatccg tgtggattga 856860
ggaagaaaaa gagttcacgg attccgtatt acatcatctg ccatcgcaac atcagcattt 856920
gcatattctt tgtttccaag ggtttttact acagaagcaa caaaagtttt ctcaagcaga 856980
aaagattttc tctaaagttt acgacgaggc tcaggacggt ccttttcttt ttaaggagga 857040
aatttagga tcccgactga tcaacagttt tttttagaa aaacagacg tcatggagac 857100
cattctttgt cttctgaatc agcgctgtcc caactcccct tactaccact tatttaaggc 857160
tctagtatgc tataagcaaa agctataccg tgaggtcata gagcaactag cctactggca 857220
agaagagaaa actcgagcgc ttgctccttt attgaatata agtattgaac agctgctaac 857280
agattttctg ttagattata tttctgcgca ttctctgata gaacagaaaa tgttccccga 857340
aggcagagta attcttaatc gcaatatcaa taggttatta aaacacgaat gtgagtggaa 857400
tgcgaagaca tacgatcgta ttgcgattct tcttagccgg agttattttc tagagttggt 857460
agaatctaag tctgcagata tttattttga ttattatgag atggtgcttt tctatctcaa 857520
aaagatctat attttagagc agtgtcctta tgcagaactt ctccccgagg aagagcttgt 857580
ttccttgatt atggaacacg tgtttatcct tcctaaagat aaattatatc ctttaattca 857640
gctcctagag atgtggcaga agcattatgt tcacccaaat agttctttag tagttcagat 857700
attggtagac cgcttttcta cacatatgga aggggctatt cggttttgtg aggctttagt 857760
ttctttctct ggattggaag aattacatca gcaaattatt accacttttg aagagctgct 857820
ttcaaataaa gtacagcaga taaaaactga agaggctaaa caatgtgttg ccctacttca 857880
tattttggat ccttctattt ccattagtga aaaattagct ctttcttcgg atacattaca 857940
aaatatagtt tctggggacg acgagcagca tacaaaactc cgcaattacc tagatctttg 858000
ggaagccata cagtcttatg atattgatcg ccaacagctc gttcatcact tagtttatgg 858060
tgcaaaagat ctttggaaaa aaggaggatc tgatgaaaag gcattgaacc ttcttcagct 858120
ggtcttgagg tttacaagct acgatataga atgcgaaagt gttgtgtttc tttttataaa 858180
acaggcgtat aagcaagcac tgtcttccca tgccattgct cgtcttttaa agttagaaaa 858240
atttatatcg gaagcgaata ttccctctat agtgattagt gaggctgaga aggccaattt 858300
cttagcagat gctgaatatc tttttgctca tgaagactat gacaaatgct atttgtatag 858360
catgtggttg actaaggtgg ccccctcccc tcaatcctat cgcttagcag ggttatgcct 858420
gatggaaaat aagcgttacg acgaagcttt agaatttctc tgtatgctct cacccaatga 858480
tagtatcaac gactataaga cgcagaaggc attagcattt tgccaaaaac atcaatctaa 858540
ggaccgagct gcctcttagc attctccccc catcttttca ctcttaaagt aaagagtttt 858600
tgtgtagtaa attttttata gttttgctta ggaactattc tccgagtgtt taggaaaaga 858660
ttttcgaatc ttcatgcatt catgttatgt tagactctat aacggaatca aagtagggat 858720
gggcattgca cacagaattt gctccttttt tagaagactt agtacatcag caggtgatat 858780
cccctttaga catcgctttt gcttctaagc acatctcttc ggactttgaa gagtcttttg 858840
tttttctcgc ggtctcctca gcgctttggc gttatggtca tccttttctt tcccttgagg 858900
aaaatcgcat tagaccttct ctaggaggga tctcagaaac agatttgtat cggggatttc 858960
ataaccttcc taaggaagtt cgagataaat tatttgtcgt tgtttcagga cgtttgtatt 859020
tacggtctct gtatacgata cgatcgaaac tcttagacaa gctttcgttg ctttgttcag 859080
caaccccgaa ttattttcct ccttctatag attcttcgat cctttcagaa gagcaaaact 859140
ttatttttaa taaaataact caaggatgtt tttctatagt ttctggaggc ccaggaacag 859200
gaaaaacttt tttagctgca caactcatcc tctctttagt gaagcagcaa cctaagttac 859260
gtattgctat agtatctcct acaggaaagg ccacgtctca tattcgtcag attcttatga 859320
aatataatat atttgacgac atggtgttga tgcagacggt gcaccacttt cttcaggagt 859380
atgcgtaccg tcgctataac tctatagatg tccttttagt agatgaaggc tctatggtaa 859440

cttttgactt gttgtatagt ttggtacaaa ccctacaggg atatgagaaa gacaaaaaac 859500
tttatacctc gagtttaatt attctcggag ataccaatca attgcctcct attggcattg 859560
gggttggaaa tccccttcaa gatctcatag gatatttccc atgaaaatac gtttttcctg 859620
aagacatcgc atagggcaaa gactggagtt gtggatcagc tgactcaatc tgtattgcgt 859680
ggcgaaatga tttctttttc tcctctccca tcgatatcct cagctataga agtcttgaaa 859740
aatcgttttg taaagtcgtt acgtcaatca gaagcacgtt tgtgtgtatt gactcctatg 859800
cgccatggcc cttggggggt tctgaactta aacacaatga tacatcaaag attggcgaga 859860
agcgatcctg atttacgtat tcctattatg gtgacgagtc gttatgaaac ttggggacta 859920
tttaatggag acacaggatt actgtgttta aaaactcaga aattgcattt ccctcaacat 859980
gaacccatag attctagggc tctatcacaa tacgtctaca attacgttat gtctgtacac 860040
aagagccagg ggagtgaata cgatgaggtt attgtaatta ttcccaaggg aagcgaagtg 860100
tttggggtgt ctattctcta tactgcaatt acccgagcta aatatagagt ttcagtttgg 860160
agagatcccg agacgttaca taaaacaatt aagaagtcta attactagat tcttatcaaa 860220
ataaaaaaac cctcaaagaa tactctaaga gggttttttt gctgcgaatg tcgttaaaaa 860280
ctatcctatt ttagcataat ctttaacaaa gtttatgaca cagctaatca atccaagaat 860340
agccataatt agaacagcat ggactccaag aatagcagga aggaatagag aaagagtgcc 860400
aagagcatca caaactaaat caactacatc tccaagccat gcaatgaagg cgtttcttat 860460
agctttggaa cgtgctgcga attcggcaga aggcttgttt ctgttttcgg ggtcggagat 860520
tgtttcaggc ctggtgctta aaattctgta aagtgaaatt gaactctcag ttaaagagca 860580
tcctgtagcc actaagttta ggcaggaagt caccttacat ccaattttgt ttgcattggc 860640
tcctaaggaa accacgtcca tttcatgtaa gaatgtagct gttccaagag tctttgatgc 860700
taaacgagca acttttcctg tgatagttag ggctgatcgt cgttgtagtt tttgagtcat 860760
acaaccctca gcatctgctt cgttgcaacg tctgagttct cctgtttcct catcagtttc 860820
gaagatcata gaaccattaa gtagttggcc ccacaacata gctcctgcca ctgcagtgtt 860880
cactccgtca gcggcaccca gaaacccttc agttttctgc aaagcagcat gtgcataatg 860940
ggagcttcct aaagcatgtc tagagattgc tgtggaattt ttaattgctc cgacgatgtc 861000
gccggcaaac aaaacgttat tgtgtagaga cattgattgc tgtctgaata aattgcctga 861060
agctctgatt gccggagtcc aagatcttaa atgcagaagt gcttgtctac caagatgtgc 861120
tgttgccata atctacctaa ttattagatt gttttaaggt cacgatgctc tcgcgttaac 861180
ttttaaggtt gctttagatt taatgcgtgc tgccttatta tctttgaaga tacctcgctt 861240
tacagcctta tctacaacac tgtagacgga ttgtaagttg ctaagagtgg cttgagtgtc 861300
gtcgagtttt aaagatgctt caaacttttt gactattgtt ttcactttag atttgaagct 861360
gtgattgatt aactctcttt tttgagcagt tagaatgcgt ttttcagcag aagtcttctt 861420
tgtataacgt ttttttattt cggtttttta ggtgccataa tatctccaga caagacaaga 861480
actttaagat ttaaaataaa ctgctattaa ttgcagatca attgagagag aaaggaaagc 861540
tttttcttat tttcgataca catattaaga aaaaagagaa tttatgagaa ataaaaaaag 861600
ttttatgcca acataacaat aagaatggtc ttaaaaaaat acttttctaa tcaaatagaa 861660
cttatggcta tgaagagcaa tttattctta tttttgttga tataaggatt tattttttat 861720
gttattggta aggaaatggt tgcatacttg tttcaaatat tggatttact ttcttccggt 861780
ggtaacgcta cttcttcccc tagtgtgtta cccttttctg tcgattagtc aaaaaattta 861840
tggatacttt gtttttacta caatttcttc tttaggctgg ttttttgcat tgagacgtag 861900
ggaaaatcaa ttaaaaacag cagctgttca gcttcttcaa acaaaaatta gaaaattaac 861960
agaaaataat gaagggttaa gacaaattcg agaatctctt aaagaacatc agcaagagag 862020
tgctcaactg caaattcaaa gtcagaagct taaaaatagc ctatttcatc ttcagggttt 862080
acttgtgaaa actaagggag aggggcaaaa attagaaact ttgttacttc atagaacaga 862140
agagaatcga tgtttgaaaa tgcaagtaga ttctttaatt caggaatgcg gagaaaaaac 862200
agaggaagta caaactttaa atcgagagtt ggctgagact ttagcctacc agcaagcttt 862260
aaatgacgag tatcaagcga ccttctctga gcaacgcaat atgctggata agcggcagat 862320
ctacattgga aagctggaaa acaaggttca ggatttaatg tatgagatcc gtaacttgct 862380
tcagttagag tcagacatag cagagaatat tccttctcaa gaatcgaatg ctgttacggg 862440
aaatatttct ttacaattgt ctagtgagtt aaaaaaaatt gcttttaagg ctgaaaacat 862500
agaggcagcc tcttctttaa cagcatcacg ttaccttcat acagatacga gtgtgcataa 862560
ctactcttta gagtgtcgcc agttatttga tagcttaaga gaagaaaatc tcgggatgct 862620
ttttgtctac gctcgtcaat cccaacgtgc ggtttttgct aatgcgttat ttaaaacgtg 862680
gacggggtat tgtgcagaag attttttaaa atttggtagt gacatagtga tttctggggg 862740
caaacagtgg atggaggatc ttcattcctc tagagaagaa tgctctggta gattagtgat 862800
taaaacgaaa tcacgaggtc atcttccttt ccgttattgt ttaatggctt tgaataaagg 862860
ccctctttgc tatcatgttt tgggggttct ttatcctctc cataaagaag tgcttcagag 862920
ttgatactat ttcttcttct atgaggaaag ttgtatcaaa ttgttggtaa gatattgatt 862980
ttcgtcagcg tataagataa aaatccctag atttcctatc ttcctttgac taaactgtcc 863040
tctaaggcta gaaatcccct tgttttattc caagtaagaa agttgttcat gaatacacag 863100
aatagccaag ctacagaagt ttcatcagaa gaagaatctc aaaagaagtt agaagagctt 863160
gttgctcttg ctaaggaaca gggtttcatc acatacgaag aaatcaatga aattcttcct 863220
atgtccttcg acactccgga gcaaattgac caagtgttga ttttcttaac tggaatggac 863280

attcaagttt tgaatcaaat tgatgttgaa aggcagaaag agaagaaaaa agaagctaaa 863340
gagcttgagg gtttagctag gaggactgaa gggactcctg acgatcctgt tcggatgtat 863400
ttgaaagaaa tgggtacagt acctctcctt actagggaag aagaggtaga aatttctaag 863460
agaatagaaa aagctcaagt acagattgaa agaatcattt tacgcttccg ttattctgct 863520
aaagaagcga tttctatagc ccactatttg attagcggca aggaacgttt tgataagatt 863580
atttccgaga aagaagtaga ggataagact cactttctta agttacttcc caagctaatt 863640
accttgctta aggaagaaga tacgtattta gaaaacttat tattgtcttt aaaacagcct 863700
gatttatcca agcaagaagc agctaaatta aatgacagtt tagagaagtg tcgtattcgg 863760
acgcaagcct acttgcgttg tttccattgt cgtcataatg tcactgaaga ttttggcgaa 863820
gttgttttca aggcttatga ttctttctta cacttagaac agcaaattaa tgatttgaaa 863880
gttcgtgcag aaagaaataa gtttgctgct gcaaagttgg cagcagctaa gcgtaagttg 863940
tataaaagag aagttgctgc tggaaggact ttagaagagt tcaagaaaga tgtacgtatg 864000
ttacagcggt ggatggataa gagccaagaa gccaaaaaag aaatggtgga gtccaattta 864060
cgtctagtga tttctatagc caaaaagtat accaaccgtg ggctttcctt cttagattta 864120
attcaagaag ggaatatggg cttgatgaag gctgtcgaga agtttgagta tcgccgtggt 864180
tataagttct cgacgtatgc cacctggtgg attcgtcaag ctgtgactcg tgctattgcg 864240
gatcaggcaa gaacgatccg tattccagtc catatgattg aaaccatcaa taaagttctt 864300
cgtggagcga agaaattaat gatggaaaca ggaaaagagc ccactcctga agagttagca 864360
gaagagttag gattaactcc tgaccgtgtt cgggaaattt ataagattgc tcagcacccc 864420
atctctctac aagccgaggt tggagagggt agtgaaagtt cctttgggga tttcttggag 864480
gatactgccg tagagtctcc cgcagaggct acggggtatt ctatgcttaa agacaagatg 864540
aaagaggtct taaagacgct tacggatcgt gagcgatttg ttttgatcca tcgttttggc 864600
cttcttgatg gcaaacctaa gactttagaa gaagtgggtt ctgcctttaa tgttactcgt 864660
gagcgtattc gtcagattga agccaaagct ttaaggaaga tgcgtcatcc tattcgatcg 864720
aaacaattga gagcattctt agacttatta gaggaagaaa aaaccggaac tagcaaagtt 864780
aagagtttga aatccaaata gtctttgagt aaaaggttcg ttttttatag ccttgtataa 864840
aaaatattgc tctggtgatt gctatagaac gttatcagtt aattatatcc aagtttcgta 864900
tgtggttgtt tttagggtgt tctgttgaag agcgtcattt taagcagcct gttcttattt 864960
cagtgacttt ttcttataac gaagtcccgt ctgcttgttt atccgacaag ctttcagatg 865020
cttgttgtta tctagaggtc acctctctta ttgaagagat tgcgaataca aagccttatg 865080
ctttaataga gcacctggct aacgagctat ttgatagctt agtgatatct tttggagata 865140
aagcctccaa gatagatcta gaggtagaaa aagaacggcc acctgttccc aacctattaa 865200
atcctataaa atttacaatt agtaaagagc tatgtccgag ccccgttttg tctgcttaag 865260
tttaggatca aatttaggaa atcgttttaa aaatctacag attgctcgta ctttattagg 865320
cgaacaagct gttttaggtc tacgtagttc ggtaattcta gaaacagaag ccttgttatt 865380
accgggatct cctccagagt gggaccttcc ttattttaat tcggtacttg taggggaaac 865440
caccctatct ttgcgagaac tactggttac tatcaaacag atagagaagg tggtaggtag 865500
agcagaggag tcgcccccat ggtctcctcg aaccatagat gtagatattt tgctttatgg 865560
tgacgagtct ttttgttgtg atcacaccga gataacgatt cctttgtcca atttgttatc 865620
acgtcctttt ttgattgctt taatagcatc tctttgtcct tatcgtcgat tttgcactca 865680
aggttctcct tatcacaact ttacatttgg agagttggcg catcaccttc cctcacctcc 865740
agggatgatt cgtaggagtt tatctccaga tacgatgttg atgggggtgg taaatgtgac 865800
taacgactct atgtctgatg ggggcatgtt tttagatcca gaaaaagcag tggctcaagc 865860
tgagaagtta tttacagagg gcgctgcagt tatagatttt ggagctcaag caacaaaccc 865920
taaagtaaag cagtttttat ctgtagatca agaatgggag cgtctggagc ctgttttaag 865980
gttgttaaaa gagacttggt ccaatagaaa acaatatcca atcatctctt tagatacgtt 866040
ttatcctgaa attattctta gggctatgga tatttatccg atccagtgga ttaatgatgt 866100
ctctggggga tcacagtcta tggctgaggt cgctagggat tgtgagctat ccttggttat 866160
gaatcactcg tcttcgcttc ctgtggatcc taaaaatatc ttgtcgtttt ctgtccctat 866220
tggagagcaa ctgttgagct ggggtgagaa gcaacttaag atgttttctg atgttggtct 866280
gaacgcaaat caggtgattt ttgatcccgg tataggtttt gggaaagggg ctgcgcaatc 866340
tttggctact ttgtatgaga ttgcgaaatt taagcgtttg ggatgcccta tccttattgg 866400
acattctcga aaatcgttct tatctttatt tggtaatcat gatcccaagg atcgtgattg 866460
ggaaaccgta ggtctatcta tactcttaca acaacaaggt gtggactact tgcgagtgca 866520
taatgttgct gctcatcaaa aagctttatc agtagctgct tgtgaagcct gtgcacccat 866580
ctaattttga aaatcctcta ggtgtcgaga tgtgtaaaaa tagaggggtc cgcgggatcg 866640
tggcttgtga tcctagaggg gtgataggtt tagaaggaaa gcttccttgg cattaccctg 866700
aagatctcca atttttttct gaaaccatac aaaaatttcc tattgttatg ggaagaaaga 866760
cttgggaaac acttcctagg aagtattttg ttgatagagc agtcgtcgtg ttttctcatg 866820
aaaaacgaca gggagtgcac ggggagatct gggtaacttc tttagaagaa ttcctgctct 866880
tagatctttc ttcgccgaca tttttaatcg gtggtggtga gctttattct cttttcttag 866940
aaaatcaaat tgttcgagat ttttttattt ctcatatcaa aaaagaatat gctggtgata 867000
catttttccc tttgtccttg ctagagacat ggaccaaaac tgtgcttaga gatacccaaa 867060
agatcacaac gtgttactat gaaaatcacc acagtcaaaa caccaaaaat atatccttat 867120

gatgacctat attctattct agagtcttca ttgcctaagt taaacgaacg ctctattgtt 867180
gtgattacgt ctaagatagt ctctttatgt gaaggtgctg ttgtagaact tgagaaggtt 867240
tctaaagatg aattaataaa gcaagaagca gatgcctatg tttttgtaga gaaatacggc 867300
atatatctaa ctaagaagtg ggggatactc attccttcag cggggattga cgagtccaat 867360
gttgaaggtt attttgtgtt gtatcctagg gatgttttgc tttccgtgaa tactctaggg 867420
gattggttaa ggaatttcta tcatctcgag cattgcggaa tcattatatc ggatagtcat 867480
acgactccgt tgcgtcgggg aactatgggt ttaggcttat gttggaatgg ttttttccct 867540
ttatataatt atgtaggaaa accagattgt tttggtcgtg ctttgaagat gacttatagc 867600
aatttattag atggtttatc ggcagctgcg gttctttgta tgggagaggg agacgagcag 867660
actcccattg ctattataga ggaagctccc aagattacct tccattcttc tccaactaca 867720
ttacaagata tgagcacttt agcaatcgct gaggatgaag atttatatgg tcctctgcta 867780
caatctatgg catgggaaac tcccgcacca acctcctgag gtattatgac atcctggata 867840
gaattacttg ataagcaaat tgaagatcaa catatgttaa agcacgaatt ttatcagcgt 867900
tggtctgaag gaaagttaga aaaacaacaa cttcaagctt atgccaaaga ttactattta 867960
catattaaag catttccttg ttacctttca gcgctgcatg ctcgctgtga tgacttgcag 868020
attcgtagac aaattcttga gaatctcatg gatgaagaag ctggaaatcc taatcacata 868080
gatttatgga gacagtttgc tttatctctt ggagtttctg aagaggagct tgccaatcat 868140
gaattcagtc aggctgctca agatatggta gcgacatttc gccgcttatg cgacatgcca 868200
caacttgccg tgggtttagg cgctctctat acttatgaga ttcagattcc tcaagtctgt 868260
gtagagaaaa tccgtggttt gaaagaatat tttggagttt ctgctcgagg ctatgcatac 868320
tttactgtac atcaagaagc tgatattaaa catgccagcg aagagaaaga aatgctacaa 868380
actttggtag gcagagagaa tcctgatgct gttttgcaag gatcacaaga agttttagat 868440
actctatgga actttttgag ctcttttatt aattcaacgg agccttgttc ttgtaagtag 868500
tatcttggca ggtctagaat ttttggatct tattagctta aaaaatagga tcatgcatct 868560
gtaaacagaa tcccccctcc taaagtatta gaagggggga ttctttgtct caaggtaatt 868620
tgtagaatct ctatgttttc tatttagaaa ttacaattta agcttctact gtttgagcag 868680
gaacttcctg aggtgtttca ttagcatgaa cagagggagt tttattcgct gcaattacat 868740
cgtagatgcg cttctcaatt tcctcaaaaa gctttctatt acgtttaagt tcttcacgaa 868800
caaattctct tccctgtcct aacttcttct cttgatagtt gaaccaagaa ccttttttct 868860
caataatatt atattcgaca gcaagatcta ggatacaacc tgcagaagaa atcccttcat 868920
tgaataggat gtcaaattct gcgattctga atggaggagc aagtttattt ttagctacct 868980
tcactttaat tcgatttccg atgtcagagt tatcactgcc ttttattgaa cctatacggc 869040
gaatatctaa tcgtattgaa gagtagaatt ttaaggcacg tcctcccgta gtagtttctg 869100
ggtttccgaa gctaacaccg attttctctc ggatttggtt aatgaacact gcacaggttt 869160
ggctacgtga tagggtagcg gtgagcttgc gtaatgcttg agacatcata cgagcttgta 869220
ggcctacgtg tacatcaccg atgtctcctt cgagttcgct tttaggaact aaagcggcta 869280
cagagtcaat aacgataaca tcgacagctc ctgaacgcgc gagcaattct gctatgctta 869340
atgcatcttc accacagtcg ggttgagaaa tcataagatc atcgatattg acgccaataa 869400
gagatgcata actaggatct aaagcatgtt cagcatctat ataggcagca acaccgccca 869460
ttttttgagc attcgcgaca atatgggtag ctagtgtcgt tttccctgag gattcaggac 869520
caaagatttc gatcacccgt cctttgggga ccccatgaat tccaagagct aagtctaaag 869580
ataaagctcc tgttttgatg gtggagattt catgtgtggc agagtgtctt cctaaactca 869640
tgatggaccc agcgccgaat tgcttttcaa tataagcaac agcagcttct agagcctttt 869700
ttctatcagg taaattcatg taaatgctcc tcttggtttc catattcccc agagaatagt 869760
tctgtttctc tgagaatcga gagaaataga gaagttcttc tcatagttag ttgagagatt 869820
tgggggtgga aggttttaga tgatgtgtat cattctgtat tgttttgctc tctatgcata 869880
cttgaatttg ccatggcagt caaggcaaaa agtaacttca agatggcttg ttatttctca 869940
ataaatttaa gaaaagcact agaatacaag gaccttagtt tcagatagta tatctaaatt 870000
atgaaactga aaaaatagat ctattggatg ccgagattcc tttaacaagg gaaactattt 870060
tcaaaaaatc aaaagaactt cttcttattt ctaacataaa tagatttggg aaagggggat 870120
gtcatgactt tcttggggaa gtctatcgat tttttgttcg cagtagccga tgccgatggt 870180
tcgtatagag ggatagggat gttgtgctaa ccagcgatcg tagaaaccgt gaccataacc 870240
aagccgatag ccctgctgat caaaggcaag gcccggaacg agcacgtggg taatcttatc 870300
actcgagatc ggtgtttgtt tcgagaaggg atctttggga tgcacaacgg aaataagatc 870360
gtctatcgag gggataagaa caggatagag gttttcctga tcaatcttgg gaagagctag 870420
ggtacatttc tggataagta tgcgatttgc ttcttgcatg tctatttcgt gattgaaaga 870480
gacaaaagag agaacgacgc tctctttaga aaagctgcga acgaaagagg ccactgcaga 870540
agaggcctca tgcttgcgtt cttcagagag atccctgcgt atagagataa atagtttacg 870600
tagtgcggat ttctctattt taggatcagt catagggaag ttctagttga gcaaaggttt 870660
gccgtcctga taggggattt ttttagtaat agttcccgcc gacgagaaaa atactgcagt 870720
cccacaacca cgatctattt tagagtaggg atgacggtct ccagggcgga agtactctcc 870780
tttaattaga agatcattat catactcttc ggtcgccatg atctgtcctt cagggtagta 870840
aatggtcagt aacccggatt ttttgttatt tacgagttct ttacaacttt ctaaggttcc 870900
tccgggatac caagttttta ctatcccatt taaaattcct tcatgccaat taagaaagca 870960

gcttgggttt ccctgtctca ggataaaaga aaattcttct ccgtgcttcg cgccttgcaa 871020
aangttatac gtttggacaa tctgtgttcc ggagttgtcg aatctggtaa cttttccata 871080
aggttcccct cggtaaaatg ccctagtttc tataacggca tacttgccgt agattgcttg 871140
aatgccgttc ccttcgtgta tagtcgcata gatttcgtga gtttgaggat ctaagtactc 871200
tgcttttagg agtcgtccct catgatattc ttcccaggct aaaacatctt cttcggaatc 871260
ttcgctgtag cgaatcgaaa gaccgtgtct tttgccttgt tggtaattct gttctttgag 871320
cagtttcccc gaagatgtgt atgtcaggaa tttaccttga ggaactccct tatgataggg 871380
acactctttc caaatattcc cattagtatg gtaatacacc gaagatcctt cgagcagccc 871440
tttttcatag acgatagcgg cttctaagat accttcatca ttataggcaa atgtagtttg 871500
atcaaatagc cagccagact ctgctgaggg atgaagatcc gcaatacctc cgataacctc 871560
agcttggatt ttgatattcc cgttgacgtg ccattcacga tatcttccat aagcacgatt 871620
attgagacac tccaggtact gcttaatttg cccgttagtg tgataggctg ttaaacaaga 871680
aacgttatct ccgcgtttgt ttttatacat cctcatgacc ttttgatagg gctggggagc 871740
aagaaagtct accttggtgt atttctttag cttctcttta gagcaaatag tttctgacag 871800
gccgtttcta tcaatgatat tgatccctgt aagggtgagt ttctcatagt cacctgtttt 871860
cccataaatg ggactcatgg caattagaga agaacataga aataagcaaa agagtttttt 871920
tatatccatc gacttatagc ctcagcatgt gttaaccaga cctcatttcc tagaggcgta 871980
gtttgttttg tcattttcca acaggtgaag aaaactaggg gcgcgacagg gttctctggg 872040
ttgaaaagag aaaacaagga ctcgatatct tcattatcca tttcagtagc ttgctctaga 872100
cgcacaaaat gcaaagtcat tgtgcatttg ttcgcagttc cagacgagct ggtgatttga 872160
tttttctaga gcacgctttc tttcccaaac ttctttactt tgtgctagca aagaatttga 872220
gtttaatttg tttaaacgtt cgcgttcttt ggatagaggg cgtaattgct tgcaagaggc 872280
atttaaactt tctattgaga ggttattgcg atccttagaa atgcgagcat tgtgtttgat 872340
cacctgatct tcatgatctc ttataacttt cagtgtgaga atctgagaat ttagatcaga 872400
ccaacgctga gagatcttca cgtgatttat ggtaatcgct aggacgggaa gtatactcaa 872460
aagagaaaga aaactaagaa ttaaaataga aatccatttt ttcataagac gtcctaagag 872520
cttaatgtaa attgtagttt aaaagatcgt tgatcttcta gagactctga aacatgttgt 872580
agcttggggt gactcgatat tttttttaag aattgaggga tgtcttcagg ttgtccttgt 872640
cccttgactt caactaaggc actgtagggg agagaggggt tatcttttga gggataactt 872700
gtcatggtat aggagaaata cgagaacttt attgaggggc tgcttttccc aagggcaaga 872760
agaaatttta atgtttgctc acttgtggga attgtaggca agagtgggta gttggaagcc 872820
gagttcttct ttccgattgc ctttactgtt ttttctgcgg cctttaaaga tcttgggagc 872880
acgccctctt caggacaagc gaaagcaaaa tggttcgaag ctgaggaaga aagagattta 872940
agttttaaca cggatcccag acttacgact accgtagcca tgagggcata cttccctatc 873000
aacagagagg agcgtagcag ccaatgtttt tgggctgctg ggaaactgaa ggtagcgtca 873060
tagggaaaag ttagaggtcg tcttgaggcc ccatggtggg cagcagcaat agtatctcca 873120
taaatttccc aatcctcgtc ttccactcca taagtcatag attgacagac cacaagcggg 873180
agagacagtt tttgttctaa aatttnttgt agattcgggg atatctgtgc gacgtgaata 873240
gcaggaagca cagtttgtgg gaatgtttct tgtatatact gcaacgttgc atggatgtcg 873300
tcgcagcttt ttttcgttga gtggttgcta aaagaacgag ctacagcaat ggcatgattt 873360
tttacaaaaa tgcaggtaac ttcttcagaa ccgccataga taagaagata tgcgggcagg 873420
ctctttaaag ggctctgttc tgctagaaaa aaaatatcgg cagcacgaca agagagttta 873480
tcagggaaaa tctgagcctg ggacagaaaa gaaagttctt ttttgagtgt atttttttga 873540
gcaatccata gggtcagagg agtttctcct ctatccgtcg gtttgcctag ttgtggctgt 873600
acaatcagag attcccaggg tagggcaaga cttgcttcta gatttgtgag agctactttt 873660
aaaatatttt ttctattttt aagggatgag gaagaacttt tgactaagat gtcagagccc 873720
tgcagagaga aagtcgttgg tgctgcaaag tatttttttg ggagagacca ggtctttcct 873780
tcgggaattt gttcacagtg acagacgatc catcctttac atgttttctg taaaattgct 873840
attttaatag tattgttttc agctttggtt agtccaatat gataaacagg cagcttgaaa 873900
ttcatagctc aagaattctt aaaatataaa ggcagctatt ttaatggata gagggctctt 873960
tttcaagaaa aacacatatt aattataatt aagagagtaa aatataatgc tatcttattt 874020
gttaagaacg ggctattaat gtttatagct ttctaatttt agcctatatc tttgcttctt 874080
gggtccctga ttgccagtct gcgcgctggt accagttggt ttccaagtgt gttgaccatt 874140
ttttgatttc ttcgtcgctt tgttcctaga attggattta tagatcccag tccttttgtt 874200
ggtctgcttt gccttggaat ccttcctttt gttatattaa gagtcctacg ttttattatt 874260
cttaatattt ttcattctcc atggctgctc caatatttat aaaaaatatt ttacttcgtt 874320
cctctatagt ctatgctcct ctagcgggat tttcagatta tccctaccgt tgcatgtccg 874380
cattgtatca accagggttg atgttttgtg aaatggtgaa agtagaaggg atactctacg 874440
ctcctgagcg tacttcgaag cttctagatt ataatgagaa catgcgtccc ataggagcgc 874500
agttgtgcgg tagtaatcca gaaactagtg gggaggccgc taaaatttta gaaggccttg 874560
gtttcgacct tatagaccta aattgtggat gtcctacaga taaaatcacc aaagatggca 874620
gtgggtcagg tctttttgaa gacgccagag cttattggga ggattttaga taaaatcatc 874680
aatagcgttt ccattcctgt aacagtaaaa attcgctcgg gttgggatat ggaacatatc 874740
aacgtagagg atacggtacg tattatacgt gatgctggag ctagcgcagt ttttgttcac 874800

gggagaactc gtgctcaggg ataccacggt cctagcaagc aagagtatat ttctagagcc 874860
aaggctgctg caggaaaaga attcccagtt tttggtaacg gagatatttt ttctccagaa 874920
gctgcgcaag caatgctaac tacaggatgt gatggtgttc tggtagctcg aggaaccttg 874980
ggagcccctt ggattggaaa acaaatccaa gactatctca ctacaggaag ctatgagaaa 875040
attcccttta tcaaaaggaa agctgcgttt ctggagcata tgcgcctagt agaagactat 875100
tatcaaagcg aaacgaagtt cctttcagaa acacgtaaat tatgtggcca ctacctaatt 875160
tccgcggcta aggtgcgttt tcttcgttcg tctctagcaa aagcgacatc ctaccaagaa 875220
gtctaccagc ttgtgaatga ttacgaagaa gccgacgact cgtcattaga gacctttgtt 875280
aaatgctgac ttaggtgttt cgaaagttgg aacatatcga taggattcgg accaatgatc 875340
gtagctaaat tgttatcagg aactaaaagt tttttatttt ctggtgcctg taattgatgt 875400
tccttgatgt aatcccagat ttttttggtt gcttctcccc gagatacggg nttcgtttcc 875460
gatcattttt gctagatctg gagagggaag gaataaagga cctgttttct tttctgaaga 875520
ttttttaacc gagctttttg cttttccctt tttagaaggt gttttggctg cttttgttgt 875580
ttttgctgaa gattttttct tggtcggagt ttttttctta tagggaattt tttctgttcc 875640
tgagtacttt gtgattacag catctataga atttccaatc acactacatt caggatactc 875700
tgaacaggaa tagaaaatct tgttgtaacg ggagcgtttt ttgaaaattt tcccattaca 875760
gcctattgca gggcagggga taggctcttc ctgttcgatt tcctctccct ttttatggat 875820
tgatatagtg ccacggcatt caggatactt ctcacaccct aaaaatgttc catagcggcc 875880
gtgacgtact ttcataacgc ctccacaaag aggacaagga ctgtcccagg gggtgtcttc 875940
agcatagtct tctttgttga aagcgagctc ttcttcagaa gtgcggtaat cgcattcagg 876000
atattctgag cagccataga aataactgtt tttagaccag atttttacta gttttccttt 876060
atggcactta gaacattcta tatttgtgag aattctagga atgacagctt ctttttctgc 876120
tgtaatcact acaggaagga atgtagtcca gaattcttga agtaagagtt tccaaggttt 876180
tttattatct gcaatgagtt caagctcgtc ttccatgaga gctgtgaacc cgatatccat 876240
aattcttgga aagtttgttt ctaagaactg tgagataatc tttcctaatt ctgtaggacg 876300
taaccgttga ttttctttag tcgtatattc acgactttga attttgttca ttatcgtggc 876360
atacgttgaa ggacggccga tcccagattt ttctaactct ttgactagag aagcttctgt 876420
gaatctagga aggggttttg taaatgcctg ttcttgggat acttcctctt tgattaaggc 876480
atcttgggca tgtagggggg ggagaggatg gtcttcttct tgatcatttt catcatcttg 876540
cttctcttca tagacagcga gaaacccttt aaattttagt aaggatcctg aagctcggag 876600
gtctatttct gtatccgtag taatttgaac agctaaagta tcataaattg caggggtaat 876660
ctgtgaggct acgaagcgtt tccagattaa gttgtatact ttaaattgat catcagaaag 876720
cttattcttt aatttgtcag gagtcagatt aatatcagtg ggacgtatgg cttcgtgagc 876780
atcttgcgtc atcttttttg tagtatatac gtttgctttc tcagggagat attctttacc 876840
gaaagtctgt tggatgtact ctctaactgt agttaatgct tcgggatcta cacgtacgga 876900
atccgtacgc atgtaggtaa tcaaacctgt agaatcttca ctatctaaat cgacgccctc 876960
atagagggtt tgcgctatag acatggttct agaagcagaa aaacgaaaat gccggcttgc 877020
ttcctgctgg agagtggatg taatgaaagg aggaggagca aaacgtcgtt ttgccttagc 877080
ttctacacga gtgattgtat acgaggattt ctctaacagc tcggcatagt gacgggcttt 877140
ctcttcagag ttaataagaa ggacatcatt ttcggttttc ccttcaggga tttctttctc 877200
ccactttttt ccttgcacag cgtataaatg cgcccaaaac gtttttgtcg ttttgggatc 877260
ttgcattaaa acgcgtaaat tccagtattc aacaggaaca aaagcatcaa tagccttttc 877320
tcgatctacg acaagcttca aagctacaga ttgcacacgc cctgcagata tccctgagcg 877380
ttgttgtaac tttcgactta ggataggaga aattttatat cccacaatgc ggtcaagaag 877440
tctccgcgct tgttgtgcgt tgactaaagc catatcgatg gttcgagggt gttttaaggc 877500
ctctgtaacc gcatttttgg taatggcatt aaacgatacc ctctggatca gaggagagtc 877560
aggaagctga ttcgcgatgt gccaggcaat tgcctctcct tctctatcag ggtcagggga 877620
aagatagact ttttcacact tcgcggctag cttgcggatg tgattgatga cctcttgttt 877680
atcgggaagc acttggtatt gtggttcgaa atcatgatcc acatcaatgc caaattcctt 877740
agcagggaga tctacaatat gtcctataga tgaggcaaaa acaaattcac tccctaataa 877800
tttttgtagc gttttaattt ttgcaggtga ttctactata attaaggact ttttcattaa 877860
tctaattgcg tgaggaccct ggatacttta ccgagagaaa acgcgactcc cttttattaa 877920
tttaattttc taattcctaa aactttgttt tcatgcaaat atatgttatt tatttcaagg 877980
tttcgctgaa ataataacac gcatgccaat agcaaagctc attaacagtt acaataagtc 878040
actctagcag cttttcatga gtcggtgctc ttagtttctc gatcatggaa aaataatcaa 878100
ctggatcgtc caacctatac tgttgcgttt tcatacgcta aataacagga acgctggtct 878160
atatgaaggc tagcatagag aattcttgat atgtccctat ggttattgag taagaagcaa 878220
attcttttca agggaataat atgatagaaa ctatttaggt ttcatcttga acctgcaact 878280
gtcattcttt tctgattagt aaaaagtttt aaaataacac aacattaaaa gagacgagat 878340
ttcttattgc caagatgcta aaaccttttc aatttttntt gttaacgatt gtaatttctt 878400
ttaacaatga gaaaaaaggt ttgaaaaaga gccgagcata gatagaaacc taggacacga 878460
aacgtggaaa aacttgagtt tgtcaccagc ctttcttctc ctgatgatga tttgattact 878520
ttcaataaac agggattgat tgcaggccca gaagaagaaa aggtagcgtt tcttgtacgt 878580
agcaatgcta tgctagatgc aggacccgaa acccccgcgt cgtttcctga atctttaagg 878640

gaacaattcg atattttccc tgagtatgtt gaagtgctct actctaatga aggattagat 878700
gtctgggaag caggatgtac gtggattcta aataatgaag tgaccatcca actgcgtaaa 878760
catcaccgga aagcttcgcg atggctagga atgtattcca gagatgaggt actcgctcac 878820
gaagccgtgc atgctgtgag aatgaaattt catgagcctg tctttgaaga ggtgttagct 878880
tatcaaactt ctcgttgggg ttggagaagg tttttcggtc ctctatttcg ctctccagga 878940
gagagctact tgctattatt cttcaccatt ttaggtttag gaatctcctt atggtatcct 879000
gccggtatac tgattatgct ggttttacct atgtattttt tgatgcgatt gtgcatggcg 879060
cagagctatt tgtatcgggc catgaaaaag attcgtaaaa tgctcggagt acctccctta 879120
tgggtgctgc taaggctgac ggataaggaa ataaaaatgt ttgctaaaga gcctattcct 879180
gttttggaac actatgctag aaaacgaaag cttgaaaatg tccgttggaa gcaaatttat 879240
caatcctact ttgtttaact ttaactagaa ctgcctatct ctaaaatgac tgtttgatga 879300
tcttatgtaa aacagctttt ttcttttatt agcaggcagg atctttagtt gtgcacggta 879360
ttttgctact gtacgtcggg cacaaggaat cccttttgca gtgattctgt cactgatcac 879420
actatcagat agaggagttt gttccgttgc gatccattgg cggatccatt gtagaacatt 879480
ctctttagaa tgcgaggaat cttgatggat tcctcggggg aagaggtgct ttagagggaa 879540
aatccctata ggagctgcaa ccgctttgtt ttcaatggca cggaagattg ttgactcatg 879600
aaaagagaga tcttcagcca aatctttaat gcctaaagga tagggggctg gaatttttcc 879660
taataaaaag tcttcttgtt tggggagaag tgtctccatc acttgaagga gcgtttgttc 879720
tcgttttctg agatttttga ttagccactt tgctgataaa atttgttgag agaggttttt 879780
ctgctcttct ttaggaaggt gttcatagaa gtgaaacgtt tctttattca gctttataga 879840
tggcaagcct cgagtactca cttcaatttt ccaagatcct gaggaataaa aaagataaat 879900
atcgggaaga ggagttgata ccatgggctt cacagtgcaa gctgctgcag gacaccaagg 879960
tatagatcct aatgcttttt ttaaaatatt tcgaagttcg gataaagaga gactgaactt 880020
tttcataata ggcgcaaact cacagttagt catcaaggga tagcaatcac ggacgatgct 880080
ataggcttgt tggtgggagg agttgcggag gagcttcatc caatagcttt gtagcgaagg 880140
agaggcaatg ccttcaggac ttaggttttg tatagtgtcc caaactttat gaattttttc 880200
taagggaagc tcaagttctt gagcaaaatc ctcaggattt cttagaaaga gtccttcatc 880260
cgagagattc ccggcaattt gatgggcaat gaatcgttct tctgcagtag aaaaagcctc 880320
ctcgatttga ggaaggagac gagtatataa agactcttga ggtccaggag tctgattcaa 880380
ataggaaaac gtagagtttg taggtcgata acaaggagac cattcttcct cttctagtga 880440
agagagatca aaaaaaggat tatcaatgat ctcttgaact acatacgatg ataactcagt 880500
aagtggcgat tgcagcatct gcaggccttg ttgcatcctt agtgagggta gatactttag 880560
agacaacttc tgcttttgct gaaacatgtc taacgcactt gaatcatata atcttttgga 880620
atttccttta aaaatctact gggcttcatc atccgtacgg ttccccagag gctgcgaact 880680
tgtgcggcag taagatagag gagatcttga gctcgagtaa ttcctacgta gcataaccgt 880740
cgttcttctt caatattttc ataagtgccg cccagagagt tcgcatgtgg aagcaattgt 880800
tcttctagac ctacaagaaa tgatacacgg aactccaacc cttttccatt atgaagggtc 880860
atcaaattca cgcgatccgc agttaaattt agatcatcat cagagccttt taaggcaaga 880920
tcatcaagga aaagttccaa atgtgtcttt ggattttgtt gttcggattc caaagcttta 880980
tgatagagtt cctctaaatt gcttttccga tctttgaagg tatccgcatc ttcttttaag 881040
atctcaaggt aacccgtgat cctaactaca gactctataa aatctctaag ggaaagagta 881100
ttgtaggcat gttcaatttg agggaaaagt gcaagatact cttgaaggcc ttcttgttgt 881160
tttttagata atttgacgtc tttagtatcc aaggcttgtt ggcatgcttt gaggataggg 881220
agaccttgag caattgcata ttgcgtgagt gcaaatatcg ttgttgaacc gatccctcgt 881280
ttgggtagat ttacagttct atcaaaagca acgatgtcgc ttttggaaat aaagatacgg 881340
agaaaggcta ggatatcttg gatttcctta cgcttgtaga aggagagacc cccgataatt 881400
tcatagggaa tgcgcctgcg aagtagagcg tcttcaaatg tccgagattg ggagttcgtt 881460
ctatagaaaa tacagatgtc acgtagtttt atattcccga ctctatgtaa ttgaagaatt 881520
tctgcagcga caaagtctgc ttcttcgcga tctgtgcttc ctaggaaaag acgaatcttt 881580
tctccaggtc ctttgacgct acgcaattct ttttctaacc ttgatgcgtt atttttaatc 881640
agagcattag cggcatttag aatattgcca taactgcggt agttttcttc gaggcataag 881700
actttagcat tagggtaatc gttttcaaaa tttaagatat tgtgaatatt tgctcctcgc 881760
caggagtaga tagactgatc aggatcccca acagcaaaga cattgcgatg ttgctttgag 881820
aggagctgca ttaaagtata ttgtgcatgg ttggtatctt gatactcatc gatgagcaat 881880
gctttccata attggttata taattcctgt gcttcgggac tttctctaag aagtcttacg 881940
gttaaaaaga gaagatcatc gaaatccaga gcattcgctt cgataagttt cttttggtat 882000
tcttggtata tcgagactac aggatcgata tagtcattgg gatccaagtc ttcgggaaag 882060
agtaaacggt tctttgcttg tgagacgtga gcttgtattt tgctcgcaag attaggtttg 882120
aggttgtgtt gttgcaaggc atgcttgatg agcttttccg cgtcactttg atcataaata 882180
gtaaaattat tttcacgatt tagcagattt atagaacgtc ggagaataaa aactcctaaa 882240
ctatgaaatg tacacaccat cggaacatca aattcattag tggaagcaca ctgattgaca 882300
atacgttctt taagttctcg cgctgcttta ttcgtaaaag ttacagccag aatttctcga 882360
ggcgcgatgc cttggttaat taggtgtaag attctatagg taaccacacg agttttacct 882420
gctcctgctc ctgctagaac gagtacagga ttgagaggag ctgttacagc tttgcgttgt 882480

gcttcgttaa gttctgagat acatgtcata ataagtccta atttttagct ttacacctcg 882540
agactgacaa tctctgtcga gctaaaataa aaagcgagta tactttcatc acaattatag 882600
aaaggtgatt tatgcagaat gctactatag atcagctccc tgtgtcttgg caagaacagc 882660
ttcctttatg ttggcgtgag caacttaagg aagagtggtc caaaccctac atgcagcaac 882720
ttcttatttt tttaaaacag gagtataaag agcatactgt ttaccctgag gagaattgcg 882780
tattttctgc tttgagaagc acgccctttg atcaggtgcg tgttgttatc ttgggtcaag 882840
atccttatcc aggaaagggg caagctcatg gattgagctt tagtgttccc gaaggtcagc 882900
gtttgccccc ttctttaatt aatattttcc gagagttaaa aacagatttg gggattgaaa 882960
atcataaggg gtgtttgcag tcttgggcaa accaagggat cttattattg aacacagtat 883020
tgacggtgcg tgcgggagaa cccttctctc atgctggtaa aggttgggag ctgtttacag 883080
atgccattgt gacgaaactg attcaagaga gaacccatat catctttgtt ttatggggag 883140
ctgctgcaag aaaaaaatgc gagcttttat ttaattcaaa acatcaacat gcggttctat 883200
cctctcctca cccctctccg ttagctgctc accgtggttt ttttggttgt tcacactttt 883260
caaaaattaa ctatctcctt aataagctga ataaaccaat gattaattgg aagctcccat 883320
gaatgaaggt atccactctg tctgttttca aaaaacacct cggcttactg cgaagtccgt 883380
agtgagtatg gagatgctct taactactca acagcttcct tccgcagaag ggatgccctc 883440
ggttgctaat ttggaagcgg attttttacg agcagaagct ctgttagcag aaatgcgaga 883500
aattcgtggt tgcttggagc aatctttgcg aacactagtc cctagtgagt aggtgttttt 883560
caaataagct ttgcaggtga ggggcgagtt tttgcaatgc ctttgctctg tgagaaactt 883620
gatttttcac atcttcacta agctcggcaa atgtttgttt gtaatcatat tttacaaaga 883680
tagggtcgta gccgaaccct gaagaacctt tttcttgatg gctgatgtag ccctcgcata 883740
tcccatacgt tttaaaaatc tcctgattag gggagactaa aactacacaa cactcgaagt 883800
acgcagaacg gtctacgagg ctttccaaag acgacataag atcaagcagc ttttttcgat 883860
gatctttatc atacgcacct acaccagcaa agttcgcaga taaaggaccc ggaagaccat 883920
ttaaagcggg gacgcgtaac atcgtatcat ccgcaatgac ccagcaaccc aaatgattgg 883980
cagcgtgaat ccctttagta agggcgttcg ccgttataga atcttcctgt tcttggggaa 884040
gtttatagtc aggaaaatca gaaagagaaa aaatatcgaa atcacctaaa cgctttaaaa 884100
aagtcttggt ttctcgtatt ttataaccat gagaactagc aatcacaatt ttcatgaatc 884160
tttcttaatc ttttgaaatt ataaagtttt aacaagatag agtcagcaag cgtaatgcgc 884220
aattattagc cgatgaaaat ctttgtagtt aaagtacaac atacattgta tctacgcaat 884280
cgctagtaag gtagacattc tagtttattt ttgttctgaa gaacttttat gattgcctgt 884340
tgatgttttt tcaatttttg agtttcacaa tgaagaaaat tttttactct tttgtattgt 884400
taagttgtat tttcccttac gtagggtgtg ctcaagtttt tgtaggctta gatcgtattt 884460
tttctgaagg ggagtataca cgttgcattc aaggcaagaa aatcgctcta atttctcata 884520
gcgcagctat caatagtcgt gggcaggatg ccctctctgt attctattct cgtaagcatg 884580
attgtaccgt ggaaatcctc tgtacgttgg aacacggcta ttatggagcc acacctacag 884640
aaacggtggg gaatcagcca tccagatatc caaatttacg ttctgtatcc ttgtatggag 884700
tgaaagaggt tcccaaagag gttgccgaac attgtgatgt atttgtttat gatgttcagg 884760
atatcggagt gcgttcttat agctttgtta ccgtgctgat gcaaatagta aaggcttctg 884820
aacggtacgg aaaacagctc attgttttag atcggccgaa tcctatggga ggaaggattg 884880
ttgatggacc tcttcctaat cccacaactt caggttccct agcgattcct tattgttatg 884940
gcatgacacc tggggaatta gcgttgtttt ttaaaaagac atacgctcct aacgctaatg 885000
ttgtcgtgat ccctatgaaa gggtggaatc gctcgatgac ctttgatgaa acaggattga 885060
tttggatgcc cacaagtcct caaatgccag atccacaatc accgtttttc tatgctgcca 885120
cagggatttt aggtgccttg tctgtagcaa gtatcggtgt aggttatacc ttacctttca 885180
aagtgctcgg agctccttgg atggacgggg aaaaagttgc cgacgagctg aatcgcatga 885240
agcttcccgg tgttctgttt cttccttttt tctatgagcc ttttttcgga aaatacaaaa 885300
tggagatgtg ctccggggtt cttcttgttc ttcaagatcc taagattttc tatccagtag 885360
aaacacaatg tacaatttgg ggtgtattaa aagcattata tcctaaacag gttgagcaaa 885420
cgttaaaatc catagagcgc attcctgcac gtcgatcttc catatgcaat ttatttgggg 885480
gggatgaatt tctcagcata tcgcacaaag agcgctatat tgtatggcca ttgcgtaggt 885540
tatgtaaaga gtctcgagag agctttcatc aactgcgtag ttcatgttta ctctcagagt 885600
atgcagaatc ctaacaagaa cacttaagat cctctacggt ttgacaggaa tatttcttgt 885660
ttctaagatg accttttcat ggcagaagcc tttcctgcat taagaataga catcagagga 885720
tcgagtggtt tgggtattta aaagtcaatt tgagggactt tcagcattaa aacgaggagt 885780
gcatgctctt actaaagctg taaccccagc atttggacct cgagggtata acgtagtcat 885840
caaaaaagga aaagctccta ttgtcttaac gaaaaacgga attcggattg ctaaagaaat 885900
catacttcaa gacgcattcg aatctcttgg agtaaagctt gcaaaggaag ccttgctaaa 885960
agttgtagaa caaactggag atggctcaac aacagccctt gttgttatcg atgctctttt 886020
tactcagggt ctaaaaggca ttgctgcagg tctagatcct caggagatca aagcaggcat 886080
tctcttgtca gtggagatgg tctaccagca attacaaagg caagctatag agttacagtc 886140
tccaaaagac gttttgcatg tcgctatggt tgcggcaaac catgatgtta ctttaggtac 886200
cgtggtagca actgtcatat cccaagccga tcttaaaggc gtcttctcta gcaaagactc 886260
tggaatttcc aaaacacgtg gtttaggaaa aagagtaaaa agtggatacc tttctcccta 886320

ttttgttacg cgtccagaga caanggatgt tgtgtgggaa gaagctttag tgctcatcct 886380
atcccatagc ctagtgtctt taagtgaaga actgattcgg tatttagaac tcatctctga 886440
acagaacacc caccccttag tgatcatagc agaagatttt gatcagaatg ttttaagaac 886500
tctgattttg aataagctta gaaacggtct tcctgtttgt gctgtgaagg ctccaggatc 886560
tagagaactg cgacaagtcg ttttggaaga tcttgctatt ttaacgggag ctacccttat 886620
aggacaagaa tcagaaaact gtgaaatacc agtttcctta gatgttttgg ggcgtgtgaa 886680
acaggtcatg attactaaag aaacgtttac cttccttgag ggagggggag atgctgagat 886740
catacaagct aggaaacagg agctctgttt agcgatagct gggagtacct cagagagtga 886800
gtgtcaggaa ttagaagaac ggttagcgat ctttatagga agtatcccgc aagtgcaaat 886860
tactgccgat acggatacag aacaaaggga acgacagttc cagttagaat ctgccttacg 886920
tgctacaaaa gctgccatga aaggtgggat agttcctggt gggggagtgg ctttcttacg 886980
agcagcacac gctatcgagg tgcctgcaaa cctatcttcg ggtatgactt ttggttttga 887040
gactctccta caagcggtac gaactccctt gaaggtttta gctcagaact gtggtagatc 887100
ttcagaagaa gtcattcata ccattctctc tcacgagaac cctagatttg gctataatgg 887160
catgacagat acattcgagg atcttgtaga tgcagggatc tgcgatcccc tcattgtaac 887220
aacctcttca ttaaaatgcg cagtttcggt atcatgcctc ttgctaacga gttctttttt 887280
tatcagctca aggacgaaaa cataatcagt tgagttctta ataaggctgc ctaaaacatg 887340
cgttgttgat tgaggactct cttctaaaac atccttgatt tgggatggta taacggaaat 887400
ttctaagaaa taaaaatttt tagtaagatt agttcattaa aaatttccac agcatttctt 887460
tatagacaga gaaaatgttg atcatttgat ctttctggga tacactatgt tgagcgaaaa 887520
taggcaccag tagctcagtc ggatagagta cctggctacg aaccaggtgg tcagaggttc 887580
gagtcctctc tggtgcggaa caataaaaga gttgaagaag aggtttttat gacactctcc 887640
ctagttggaa aggaagcccc tgattttgtt gcgcaagctg ttgttaatgg cgaaacgtgt 887700
accgtatctt taaaagatta tttaggaaag tatgttgtgc ttttcttcta tcctaaagat 887760
tttacttacg tgtgtcctac ggaattgcac gcatttcaag atgctttagg agaattccac 887820
acccgaggag ctgaagtcat aggctgttcc gtggatgaca ttgccaccca tcaacagtgg 887880
ttagctacta agaaaaagca aggtggtatc gaaggtatta cctatcctct tctctcagac 887940
gaagataaag tcatttcaag aagttatcat gtgttaaaac ccgaagaaga attatctttc 888000
agaggagttt tcctgattga taaaggtgaa atcatccgtc atcttgtagt gaatgatctt 888060
cctctaggcc gttctataga agaagaactt agaaccctag atgctttaat cttctttgaa 888120
actaatggct tagtctgtcc tgcaaaattg gcatgaagga gagcgagcga tggctccaaa 888180
tgaagaagga ctgcaaaatn atttcgggac tatagactag anaggctgat tgaaagtcag 888240
caagtcataa agatcgtgat caaagaacaa taaaaggcta ttgtgttttt ggcataaaga 888300
ccgagaagct tcaatgatgt gttgatttcc aacaccagga agtccgatag caatgatgtt 888360
ttttaacgtc gtttgtgtat ttaagaaatg caaaccaaaa aagtaactat ctacacagta 888420
gccttcttct cgaactctga agtacactac attataatca cttaataatg ttttaatagc 888480
taaagtgatg cctggactgc tgacatctcc aagataatta tgtagatcta caagactacg 888540
acagaagtac gcagtatcgt aacttccttt ttctgcttct ccaaataaag caactgtaaa 888600
tttcatttga aatatgagat cgaatcttta tttctgatcg tacaggactg tttgtttatt 888660
atctacagag agaaacttag ttttgaaaag atttgcgaat ctaagatggt tattattgct 888720
tatttctttg taaacaaagt cgttgcccag catagatagc atcggaatct aatttattaa 888780
ttttctttaa ttcagttact gaaagtttat attttttagc aattttacta agactatcac 888840
cttcacgtac tatataaata ttctcaggaa caggatccga gaagtcagca taggctccag 888900
gagaagagct gtctactaaa gcaagtaaag aacgtcgtac tagacgcaaa tcctgagcta 888960
aagctcggtg atctttttga atttcttgta gtttactttg taagttcgtc tgtaagtctt 889020
taacagaagt tgttagtaca gctaatgttt tcgcaagcgc cttttgatcg gactctagct 889080
cgcggatttt ttgagccagg gtttctggtt ttgctgctgt ccatttttgg aacttagagt 889140
cttgttcatc caagcgctct gacaacatga caatctccac ttcgtgagag gctaattttg 889200
ccgagatatc ttcgatttct gcaaggactc cttgtagaga aggagacctt ccagcagcat 889260
gcaaacgact gcaatctatt cctgaaaata acagacttag aagaataaaa aagcttagct 889320
tagcgtgcat gaatcttaaa ctctgtacgg cgattttgtt gccatgctag ttcgttgtgt 889380
cccgaattta aaggatgttc ttttccgtag gaaatagtag atagacgatc tgcagagatt 889440
ccctgctttc ggagatgctc tttaatcgca ttggctcgtc gtgctcctaa agcaaggtta 889500
taggatgcag ctccacgctc gtcagtatgc ccttcaatgt acagtgtagc tttcgggttt 889560
ttcttcatgt agtgaaccaa gttcgtgaga atcgcaaggt tctcttcacc tttaattgta 889620
tagctgtctg tagcaaaggt gatattacga aatgctgcaa cttggcttga cttgtattgt 889680
ttttcttctt tggaatcata ctcaccgaag gtaaaattag ggttaaagtc ctcttcggta 889740
tagagaggaa caaagccaaa agaagaaggc ttttttcgtc ttgtatgatg gcatgtatta 889800
caggaatcct cccagccata attaggggaa aggctacatg ctggcaatgc aagtaaagcc 889860
aataaagtac aaagtttcca tagggaatgt atattcatag tgttctcttt atcggttgct 889920
gagggaaagc accccaggag gggaaccgtt tttctcctac tcctatagca attttgttag 889980
tttttttggt gactagactg attaaatata actctgattc ttcagcattc cccgcactaa 890040
agacaagatg acggctgtct atagcccaag aaggactctc tttatttgtg ggagacgtag 890100
tgagttggta atcctctcca gaggagagat cgtaaataca aatttgtcgc acccctttaa 890160

ttacagagca gaaggctatt tttttaccat ctggagacca tgcagggcaa ctgctatttc 890220
tgtatttttt tgtcagcaag cgaggtgctt ggggttcagg atcgagggac ataatataaa 890280
gacgcggacg gccgtctttg ttcgatataa agacaagctg ggatccttca gggttgaagg 890340
agggattccc ttgagtcccg aaattctcat taaggaggcg acgtgggcga cccataggtc 890400
ctgaagttag tgagaacggt tgaataaata aatcaggatt tccatacgta tcagcaacga 890460
aagctaaaag cttttttctt ggagaaaacg taggcatgag ttggttgcct tttaacggaa 890520
ggactttttt accttcagtg ttctctaggg aaccaagaaa aattttaggc acaccatact 890580
tatacgaaac atagagatag ggaaaatttg atcccacacc cacccatttt ggagttatag 890640
agagcgaaca ttctgtggtt aaaggggcga ggtttttccc atcgtaatct gtagtccata 890700
attctccttg cttgagcttt tgatcttttc ctaaagaact tagagcaaaa acaattttcc 890760
cagcactgat tccaggaatc cctgtgaggg cgtaatgaac tgtatcagca gcgtgatgga 890820
ttttttgacg atctacagaa agattttgag aaatagtaaa agaacataag gtttgaggag 890880
tttttgaaga ctgtaaaagc actacagata gctgaggtac atgcaaccgt aaagatattg 890940
ctaaaggaga tgacgattct ttagaagccg ctgtgggttg tagacaatct cctagggcaa 891000
tgtccttgca aaatatctcc gttagcgagc tgaggtattt ctgtattttt ggatctttcg 891060
tatcggtctg gcaagagacc tcaataggga gcgtgatatg ttcggaacgg acaacaactt 891120
ctaattcttc agcatagact agcgatgcga agcaaaagaa aaaaacttgg aagcatagtt 891180
gccgtaacat gccgatatct cctcacccta gggatagagc gttaggattc attactgacc 891240
agtttaatat gaaaagatta tatttttcga gactttgtat ttttcgagaa atttttgaaa 891300
tgggagtgca tgaatcctct gagtgagcag ctgtttgtca gcagcactca cctcagagag 891360
aaaactgcat tcttgaatct ctccattcgg agaaaggact aatttaatac gcacataccc 891420
tttagagggg agagctatgt gagtgcgaaa taactcacat agctcgtctt cttgagttgc 891480
tttaagttct gagtgcatgg tcaattgggc tgtagatggc caggagatat ttttgagaga 891540
agtttcactt ttctcaattt tatctacatg tagggaaagg gcttgggcaa cttcggagag 891600
tgtttgtagt tgggtttttg ataactgtgt atttttttta gttgtagagg gaggaggttt 891660
ctcagttgcg gtcgtttttt ttgctacagt aggggctggt gatggtttag gaggttctgt 891720
ttttataact tttggaatgg gtttttgtag agccttctgg acgttctctt gaggagggct 891780
gcatttagct tgtttttgtg gctgcgtagt acagaggggc gtatcgtttt agctggatct 891840
acaactactg agggagtagg gacaggaggt ttcggttgga tagtgacgag tttttcttga 891900
aaggctttgg gttgtaaacg ttttttagga agaggagaag caaagacaag gagaagaatc 891960
cccccatgaa tacatgccgt gattgctata tagggaagat acttcatcat aattcaattc 892020
tgtagggcta cgtgaagttc gtgaaacccc gcagcttcta tagcattctt tacgttttga 892080
tatgtccgaa aggaggtttc tccatcttgt agtaataggg gggtcttttc aggataggct 892140
ttatggagga gggtaaggcg aacagtgagc tcttgtagtg tgatcggatg ctcgttcaaa 892200
gttaacgaat ggtccgcaaa tactttaatt accgctatag aatcgttttc gctgctcagc 892260
acttcttgct cttgtgtgcc aggagcaaga gctatagaat ccagttttat taagggaaca 892320
gcaacgataa atgccattaa aatgacaaag acaatatcaa tcaacggcgt taagttcacc 892380
aggggctctt cttctatttc ttccgtgaag cggtatttca taagtttgta ttgcggtatt 892440
tgacttctat agaattcagt agtaaatatg ctgtctgctc tatctcagaa atcaattccg 892500
aagaatgtgc tttaagatag ttaaaagcga tcagtgaggg tatagcaaca aagagtccta 892560
taatcgtcgt tcccagggct gtggcgagtc cttccataat ggctgagttt ccactgcttc 892620
ccgagctaat gtgggtaaaa gcgactaaaa ttccccatac tgtacctaaa agtcctagaa 892680
aaggagctaa gctgatcgtt gtcgccggaa taaagctgtt tttatgtaaa agagctttat 892740
atttcggcat gatggctccc aggagcgtct ccaaagattg gatatcttca gaagaaagga 892800
tgggacctcg atctggagcc gattgccgat ttttatctaa gagctccaaa gttccccgtt 892860
ttatagtaaa atacaagtcc gcaaaggggc tgagctctgg atggatatcc agagacagag 892920
gtgcgtgacg gtttttaatt aagaaatctt taagagactt tcctgctttt aaaaaatttt 892980
tttgaatggc aagcttttga tgtagtactg tccaagtaca cacagaaaga ataagaagac 893040
aaaagaatat acttttacca aaaaaatctg cttccgtgta ggcctgaatg atggggttat 893100
gagagaagtg taccatggaa taaaaatctt tatttacaga gagtgtctca aagtaaagat 893160
gatcctaaca aattttcgtt atagagccaa gcgggttttg taaagaagat ttataaaatg 893220
gaatataaaa attaatgtat aattagtaac ttgatttata aaatcacggt gttattttga 893280
ataaattcaa aacatattta caaaccgcat tgatagcacc tttcttttct ttcccagcgt 893340
tatctggaag tttttcctct atccaggcgg aagaaattnc acaacaagtg aatcatccag 893400
gtgcagaact cctttctgag ggtagctaca tccctggact acagacattc cgattgggga 893460
tcaagattta cagcttccaa agggagccat atctactgga agaatcccgg agaaattgga 893520
agtcctctca aaatttcttg gcagttgccg aaaggtttcg tggttgaaga agagcattgg 893580
cctaccccca aagtatttga ggaagagggc actacatttt ttggatatga agattccgct 893640
cttattgtcg cagatgtccg tgctcctgaa ggatacactc ctggtcagga ggtcgaatta 893700
cgagctcagg tcgaatggct agcttgtgga gatagttgct tgcccgggaa cgtcgatcta 893760
aaattgacac tgccctacga agagaaggag ccttctcttt atcctgatac acacgcagaa 893820
tttactaaaa cgctgcatgc gcaacctcgt gttttagaaa atgatcactc tgttcaagtg 893880
gcgcaaggaa aaggaaatga gatcatttta aatatctcta agaagatcaa cgctacgaaa 893940
gcatggtttg tttctgaaaa agccgataag ctttttgctt atgcagagac ctcttatagc 894000

gggggaacag gaactgcatg gagattaaaa gtaaaaaatc tctccggagt tcagaagaat 894060
gagaagcttc atggatactt gctgttagcc gaccacacag gtcgtcccgt agaatcactc 894120
accattcata gtgaagttct tggtcaaaca ggatctgctg tagcaggact gtcacaatat 894180
atcacaattt tgatcatggc ctttctcggc ggggtcttgt tgaatattat gccttgtgtg 894240
cttcctttag tgaccctgaa ggtctacggt ttaataaaat ctgctggaga gcaccgctct 894300
tctgtaattg ccaatggctt atggtttact ttaggggttg taggatgttt ctggggattg 894360
gcaggtgttg cctttatact taaggtttta ggtcacaata tcggctgggg cttccaactc 894420
caagagccta tgtttgttgc cacattgatc atagtattct tcttatttgc tttaagttct 894480
ctagggcttt ttgaaatggg gaccatgttt gcaaacctag gagggaagtt acaatcttca 894540
gagatgaaga gctctaataa taaagctgta ggggcctttt ttaatggtat tttagctacg 894600
ctagtcacga ctccttgtac aggacccttc ttaggttccg tattgggatt agtcatgtct 894660
ttatctttcc tgcagcagct cttgattttt actgcgatag gcctggggat ggcttcacct 894720
tacctagtct tttctgtatt tccaaaaatg ttgtccgtac ttcctaagcc tgggggatgg 894780
atgagcacct tcaaacagct aacaggattt atgttgttag taacggtaac ttggttggta 894840
tggattttgg gttctgaaac aagtacaact tctgttgttg ttctccttgg aggactgtgg 894900
cttgcaggat taggagcttg gattttagga cgttggggaa cccccgtctc tcctaaaaaa 894960
caacgtgttt gtgcttcttt attgttcttt gcattcctag gtggagccat ctctgtaagt 895020
ggtttagctt ctcattactt tgctgaacct cagcagacag tcagtgtgaa cgaagatagt 895080
ttatggcagc ctttttcctt agagaagctt gcccaattgc gagcccaagg tcgtcctgtt 895140
tttgtgaact tcacggctaa gtggtgcttg acttgtcaga tgaacaagcc tgttttgtat 895200
ggtgatgctg tgcaaaagat gttcgaaact catggaattg tgactttaga ggcagattgg 895260
acccgtaagg atccagggat tacagaggag cttgctcggt taggtcgcgc aagtgtccct 895320
tcgtatgtct actatcctgg agataactct gcacctgtcg tacttccaga naagattaca 895380
caaaatcttt tagaagacgt cgtaagtcga tttgtaaggt agctgttaag tgtagaacca 895440
acacaagcat tcgtaggtgg aatcatccac gaggtacctt aaaaattgtt agggcagcat 895500
atgctgccct ttttatttgt aacaagaata ccaaagttca cctgccttta aaagcagagc 895560
cgcttcaatc ttgtgtagaa tcttgagtct ggggagctta aattttcctt agtaggacag 895620
agtctcggga gttcgctgtg ttttaaggat tcttctttag tgaagatatt ttcaagagaa 895680
tgtattctat agggaattct tgccctgaat gttctacgac tttgcttaaa ctaggagaca 895740
acccgtggat ttggctgatg ctcatgttca tctttctgat gatgcttttg aagaagatat 895800
taacagcgta ttacagcgcg ctcaagattc tggagtgtca ctagttgtta atgtaaccac 895860
aacagaaaag gaattaaatc gctcgtttgc gtatgccgaa cgttttccta aaattcgatt 895920
ttgccatgtt ggagggactc cccctcaaga tgtagatcag gatatcgaag aagactacag 895980
gaattttcat gctgcagcac atagtaagaa actcgccgca atcggagagg tcggtttaga 896040
ttattgcttt gccacggaag agggaatagc aaggcagaaa gaggttctcc aacgctattt 896100
ggctttatct ttagaatgcg aactcccact tgtagtgcat tgtcgaggtg cttttaacga 896160
ttttttccgt atgctagacc aatactacca taacgatcca cgttcacgtc cagggatgct 896220
gcattgcttt acaggaacct tggaagaagc tcaggaactg atctctcggg gatggtttat 896280
ttctataagt gggatcgtga cttttaaaaa tgctcaagat ttgcgagatc tggttgtaga 896340
acttcctctt gagcatcttt taatagagac ggatgcgcct tttctggctc ctgtacctta 896400
tcggggaaag aaaaatgagc ctgcacatgt gctccatacg atcaacgccg ttgccaatgt 896460
aaaagggatg ttcccacaag agcttgcagc tcttgcttac aagaacgtct tacgctttct 896520
gcacggttaa tttgatggag ttcatagaac tccataacct ttctattttg tcatataagc 896580
ttctgtttct tatgaaaaat tactttccat aattttattc tagcctctat cttgagttga 896640
acttaaagtt tagtaaaaag tcttagaatg tcacgacatg aaatctgccc agaagtgtca 896700
cacaagaaag gcaagtatta tagcaccttt atcttccgtt gtattcactc cttagcgggt 896760
atagcgttta cttttttcct atgtgaacac ctatttacga acatgctagc ttcttcttac 896820
ttttcccagg ggaagggttt cgttgctatg gtcaatggtt ttcataagat cccagggctg 896880
aaaattattg aagtggcggg tttagtcctt ccttttctct gtcatgcgat tatcggcatc 896940
gtatatctct ttcaaggaaa aagtaattgt tattctggtg acggaagtcg acctcatttg 897000
cgttatgcta aaaattatag ctatacgtgg caaaggtgga ctgcctggat tttactcttt 897060
ggaattgctt tccacgttgt gcatttgcgt tttatccgtt atccagtcca tgttgatatc 897120
catggaacta cctattatgc tgtagacatt caaccttctc gctatgacgt gattgttaga 897180
gggactaaag gctttttaac tttgaatctt cccaatacag aagcttcgag tatcgaggtg 897240
tctcgtcatg atttaggtgg tgctgatgct gcgttattgt cggagaggaa ctcctattta 897300
ttgactccaa gtgcaggtac cgcatttctt tatgtagttc gtgatgcctt gggatcacta 897360
ttcatagctc ttctctatac tattttggtc attgctgcag catttcatgg gtttaatggg 897420
ttgtggacct tttgttgtcg ttggggtgtc gttgtttctc tgaggatgca aggggtattg 897480
aggatagtat gttacctcgc tatgattgtt gtgactttca tgggagtgag tgcggtttgg 897540
aatttgtata gtgtggcata gcaaatggat gagaatcgaa aagtaatcgt tgttggtggg 897600
ggattggcag gattatccgc agctatgcag ttagccaacc ttgggattat tgtagagctc 897660
gtatctctga ctaaagtcaa gcgctcccat tctgtatgtg ctcaaggggg aatcaacgct 897720
gccttaaatc tgaagcctga ggaagaggat tctccctacg tgcatgccta tgatacgatt 897780
aaaggtgggg attttcttgc agatcagcct cctgtcttgg aaatgtgtct tgcagcaccc 897840

agaatcatta aaatgttaga taactttggt tgtcctttta accgtggtcc ttctgggaac 897900
ttagatgttc gtagatttgg aggtacgtta taccaccgca cagtattctg tggagcttct 897960
acagggcagc agcttatgta tactttagat gagcaagtgc gacggcgaga acatgcgggt 898020
agggtgataa aacgagaaaa tcatgaattt gtacgtttag ttaccgacca ttccggacgt 898080
gcttgcggca ttatattaat gaacttgttt aataaccgtc tggagatttt acgaggcgat 898140
gctgtcatta tagctacggg aggccccgga gtgatcttta agatgtctac aaactcgact 898200
ttctgtacgg gagccgcgaa cggaagactc tttttacaag gcatggccta tgcaaaccca 898260
gagtttatac aaattcaccc tacagcaatt cctggaaggg ataagctacg attaatttca 898320
gagtctgtgc gtggtgaggg cggtcgtgtg tgggtgcctg gggattcttc aaagcgcata 898380
gtatttccag atggttcgga acgtccttgt ggagagacag gagctccttg gtatttctta 898440
gaagatatgt atcctgcgta tgggaatctt gtcagccgag atgtaggagc gcgtgctatt 898500
ttacgtgtat gtgaagctgg attaggaatt gatggacgca tggaagcgta cttagatgtc 898560
actcatcttc ccgagaaaac acgtcataag ttagaagtcg ttttagatat ttataagaaa 898620
tttactggcg aggaccccaa tacggttcct atgaggattt tccctgccgt gcactattct 898680
atgggaggtg cttgggtaga ttggcctgct gccgatgatc ctgatcgtga tagtcgcttc 898740
cgtcagatga cgaatattcc tggatgtttt aattgtggag aatctgattt ccaatatcat 898800
ggagccaatc gcttaggtgc taattcttta ctttcctgtt tgtttgcggg tttggtttct 898860
ggagatgaag cttctcgttt tatagaggct tttggggcat cacaggcaac gtctagtgat 898920
tttgatcgtg ctctacagca ggaaaaagag gagaacgcgc gtcttttatc tgcatcagga 898980
aaagagaata tttttgtttt gcatgaggaa atcgcaaaga ttatggtgcg aaatgttacg 899040
gtaaaacgaa ataatcgtga tctccaagaa actatggata aattgaaaga atttcgtgag 899100
agattaaaaa atgtctctgt attggactct tcaccatttg cgaataaatc cttccatttt 899160
gtacggcaga tgggacccat gttagaactc gcactggcga ttactaaggg agctcttcta 899220
cgcaatgagt ttcggggttc ccattacaaa ccagaatttc ctgagagaga tgacgagcat 899280
tggctgaaga ctacagtcgc tgtttatgct cctgaagaac ctgagatttc ctatcttcct 899340
gtggatactc gccatgtagc cccgactctc cgggattaca caaaatcttc aacaggaaaa 899400
atagaactca cgaatattcc tgataatatc cgtctaccca tatagaaaaa gagagatgat 899460
ggagaatcta gagactttta ttttaaaaat ttacagaggc gttccaggga agcaatactg 899520
ggaaagcttt gaacttcctt tacatcctgg ggaaaatgtt atcagcgctc ttatggaaat 899580
cgaaaagcga ccggtaaata tcttagggga aaaggtcaat cctgtagttt gggagcaggg 899640
ttgcttagaa gaggtctgcg gatcctgttc tattcttgtg aatggagttc ctcgtcaggc 899700
atgtactgct ctgatccaag aatatatcga tgcaacgcaa tcccgagaga ttgtccttgc 899760
tcctcttact aagttcccgt taatccgaga tttaattgta gatagatcga ttatgtttga 899820
taatctcgaa aggattcagg gttgggttgc tgcggatatt gaaggagaga cgtttggtcc 899880
tcaagtcact caggaacagc aagagcttct ctatgcattg tcgcagtgta tgacgtgtgg 899940
ctgctgtaca gaagcatgtc cccaaattga taataaaagc gatttcatag gtcctgcagc 900000
aatttcccaa gcgcgttatt ttaatacgta tcctggagat aagcagtcta agaaaagatg 900060
gcgggctctt atgggtaaag gagggattga gggttgtggt caagcgcata actgtgtccg 900120
tgtctgccct aagaaacttc cccttacgga gagcatctcg gccgtgggac gtgaaatttc 900180
aaagttctct ttaagaagtt tattttcagc tctttttaaa aagaaaaaat aatctagaaa 900240
aattctttag ggaggcggct gcggtagcct ggaggcggtg gcatgccata atctatagga 900300
tttgcataga gggtccaggg tcttgacgtt gcgaacagct cctggatatt gtcaagaggc 900360
tgtccttgca gccctgcata gttaaatttc caaagatcga tctctgtggt tcctggattt 900420
aggatgaatc caaaataaat agaaggccag ttactgtctg caaagagcaa aggagcggga 900480
taggcaagat tatgatgcct cattgccgtg gtgaggcgga ggtacgtatc ttcttcggtg 900540
tagatctttt gataactttg catgaggaga cctttataga tatgcctcag gtctgctgag 900600
gagagtaagg tcattttagg gatggtttcc tctatcaggg agcggaattt ctcataggta 900660
atacgagagg aaatcccgag atatgaagag acgttatcta agacttctgg aagctgttgt 900720
tctgaaacat aagggacttc acggaccata aggtagagaa gacggcgtat atagataaga 900780
gctacggtct tatctttggt gaataaggag cttagaaaac gcgatccttt gtcatagagc 900840
tccggaagag tcaaggagtg gtcggagcag aaatcatgaa agtcatgaac tacatgttgc 900900
aaagcatatt tgttacaaaa attctctatg aaagcataga tacttagctg aggtaatata 900960
gtatcttgaa ggaaatcttg gtgttgtttc acccagacat cacgaagcca ggtatagctg 901020
taccaatcat tatcccaagc ttcccgaaat aaaggagatc ctgcgattat agagaaaacg 901080
tgggtgggtg atgagctaag tagagagtgg gatccttctt ctagataact tttaattcct 901140
gtagggagat ctttaagggc gtctgcgtag aaagctgcaa gctcatgagg attttcagga 901200
tgcttttctg taagtgtcag aggttctgag ctttcaaaat aatccaaaag aagagtgtcc 901260
actgttcctc cagaaacata aacccaggga gtttgtgaca gctgatctaa gtggtttaag 901320
atggagggag gcacaggaag ctgataggct tctaaaattc ttgtaaggag agcttcttgg 901380
aaaacatccg tgtgtagcat ggcagtgatg ttgtggacga gccgagatgt ttctttctct 901440
aaattgatca cggcatgttt ccccagaagt tctgactctg tggaggtgaa gaattcagaa 901500
agaaaacgta taaattcatt aatcgaatag atgggggacc atgtgttcgg atgggtgcgt 901560
ccatgcgtga aaagaatacg gaagccagcg ggagcattag catagagatg agcaaattct 901620
tgaatgaagg catcgtaaga actacgaaag tataagggaa tttgctttgt atagaaagaa 901680

agtaagaatt caggaagatg tagaaatttc tttgcctttt cttgagcact atcccactca 901740
taaagagctt tattgagttc ttgacggaag cgcatgtgat ccatcgtcaa aatctgactg 901800
tcttgattat ttagtgggtt gcgcatccgc ccttcaatat attctagttg ggagcgtgct 901860
tcgtgatagg tctgttcaca ttgttggact aaaattcgga tgttttctac ttcctcttca 901920
acaaagtgtg taactagaga tacaagactg tgagggtctt cacttttcca tcctaaggca 901980
aggcggatat ggtttgagat ggtaggttgg ctagcatccg caagagtcgc taaagtatac 902040
tcccaggctt tcagtaaggg attttgagtg tcatggataa aagcagattt tgcttcttca 902100
taggcatgta agtagtggta tacccgttgt atttctgaga gctctctggg gtgttgcgtc 902160
gagaatgcca cttgttcttt gctgaacaac ccttctttga agaaaatagc tcgtacagta 902220
ctttcttgga gctgatagta gtgcagaagt gtcgatttga taatgtcgtt agcagttaag 902280
gtctcatgga cattttgtag tttttgcatc aaatattgat gcgagagcaa ctgttggatt 902340
tgtgcttcag aatccccaag agtttcaata agattggcag cagaaaaggc ttttttgagt 902400
cctggagatg aggagagctt aaccagagga tcaggataaa gatctagaat ccttaaaggc 902460
ttgaatagct ctccaatgca tcccgaaagg tttataggaa ccgcaatttc cctttggttt 902520
acgattctag agagtttgcc actgctaatg agatcattga gatctttaag gaatcgttct 902580
ggatattctt ggtgaatgag aatggcagga gccgtagcaa aacaggaacc tacatcttgc 902640
cgaaggtagg tgaagagcgc tgtgagtgct gcttgacgca catgaatcgt agagagaatt 902700
gtctgtggat tcaatgctag tgtatggcga attaggtttt ggattgtaga gtatgaaggg 902760
acaaagagag ttttgatgct ttcttttaat ttaggatttt cctttagagc ttttagcatt 902820
ttaaggaggt gttcacgatc ttgagcttca ttatggcgat gaggccctaa aggataggta 902880
cattgtgaga gatggtgaat ggcttcgaca agctttactg tatcgatttc cccattgtca 902940
gctaagatat aatttgcgac tttacgggcg atatttaggt tcttagcagc tagaggggat 903000
tgtagagccg tagtacggta ggctttaaat aaaactgtgt cttcagaaca gaaaatttcc 903060
tcaagaagat tttgatcctg gtttgcgatc agagaatcga aaacaaaaat atccaaacgt 903120
gacataggat gtagaagata gttatgggat attcctttgc tgtaacacga atacatgttt 903180
ctgtcactga tatcctccac tactattttt ctagagctcc gaaggcaagt ttatgaaaat 903240
gaaaggatgg tcagagttcc atctgcagaa tgttcggttt cgttgtttaa catcatggtt 903300
agagaatcaa taagattctc tgtatttaga gggtttaaag gatcttttaa gagctcttca 903360
ataggaagct gagaaaagta cttgaggatg tcttctcctc cagtgaggca gatcaaccga 903420
tctccaggct gtagagcttg gtgagtctct aaggggagac gtacgaaaga ttctcctcgt 903480
tgtagaaaca tggtaggagc tccctcgctt aacgagagga gctctagaga tcgatctttt 903540
tctacatatt taatgaaagt catagcaact acagcctcat tgccttctgt tgtttttgag 903600
aagctgtcgg cagtatcctt gctgattttt tgtaacgaaa cgtccgagga agcataggca 903660
agaaaaagac tccgtgcgga taaagcatag agataggaag gaagaccaat atcgccagcg 903720
agccctatga tccctaaaag ggtatcgcca ccatcttgaa ctgtccaacc attaaaatga 903780
ccggaaagtt gccttctccg gagatgttgg gaactaaagg taactttagg gaacgtaggg 903840
aaatccggac ttagtaacgc actttgtagt gaagataaaa tccctaattc tttttgtaat 903900
ttttcgcctg aatggtaatc gatatctgct ttctcaatgg aattcaataa gagtaggaga 903960
gtgcaattga aaatatttcc tagttcattg aattcataac cgtaaggctg gggttcaaac 904020
ctcacgttat ggtttcctcg ccaggcagct tccatacaga aggtcagttc ttgaagaggc 904080
ttgttaagtt tggtgttgat cttagagaaa atccaccaca tgaggaggaa agcaagtaca 904140
tagaaaaaac aaatattgag aggaactttc aaggcggatt ggatgagatc agaaactgga 904200
actaaagata gagtgtaggt cccttggata ggaattttat tcagtaccaa tcctaggtag 904260
cgtttcttgt tgatactcac tgtgattagg ttctccccac caagaattcc agaagctttc 904320
tcaatttcta tggcagaggg gcttcttgct tggaattgcg gtaaattagg gagatctaga 904380
gaaaatacaa aagaagattc actgtcctga gcacagaaga ggacctcgcc atacttattt 904440
acaaggcaga tatttccttt ggtgatgtgt aaggattgga ataaatcttt ctgtaaaaaa 904500
gacatgggat agaaacttac aagcagtcct gaagtcgttg tagaatccca agatgcgaca 904560
tcttcaacta gaataagata atgtaaaagt ggtttacctg gaatggtcaa taaaaaggct 904620
ttccctacag ctgcggatag ctttttcttc atttcaggat gctgttttag atagcgaatg 904680
aaagggtctc caggattttt cgtccttaca gatccatcaa agggatctat aaggcataag 904740
gaaaagtctg tattggagag tgccatcatc tcattgtatg cctgtgcata gggctctgca 904800
gaaggagatg cataggattt taaggctaat gtgttggcaa gtctatcgag gaaaagcttg 904860
tgtatcgtca gttttttttc gaattctata cttaagttcg tagcacgggt atggaggacc 904920
tgtactaaat ttgctttagc ggcagaaaat gagaaaaaac ctacgaccat aagattgagg 904980
agtaggggaa tgggaatcac taaaaagaaa aaaaatagaa cacgcttggt aaaggtatgt 905040
ttcatgatgc taataccttt aatattaata aggtgatgtc gtcatgttga tgggagttcc 905100
cgacaaaggt ttttacactt aacatcaacc tgtggacggc atcagcagca cttttccctg 905160
tcaatccttg aattgcagct tgtaggcgct cttctccaaa catgtcgtta ttgttattat 905220
gggcttctgt aataccatca gaatacaaga caaagagaga ccctggctta ggatgaaata 905280
gctttgaagt gatgttcgca acttcgggaa ggaagcctaa agccattcca ggatggaata 905340
gccaagaagt ctcgccatca ggatctaggt agcaggcagg aggatgtcca caagaataat 905400
attccatggt gttggaagtt tgatgataac agtacacaca tagagtgaca aacatccctg 905460
agttttttgt attgttataa aataagcgtg aggtttcttg gattgcctgt tgaagagacg 905520

aagagcgaga aaggaatgtt ctgagcatat tttttagaaa tagcgaatac ccacaagcat 905580
taacaccttt ccctgaggcg tcagcaacaa tcaggaatag gcgagccttc gaaccctctc 905640
ctacaacaaa aacatcaaag aaatcaccac ctacagtaat ggcagggata taggcttttg 905700
cgagttctat atgaggatag ctggggagag tattaggaag aagtcgctgc tgagcctgct 905760
ctcctaaatg tagagcgttc tgtgcatttt ctttcatctc aaagttcgtc ttagcgaggt 905820
gttgctgttt gtggagattc tccaccatag cattaaaaat atggccaagt ctgttgatct 905880
caaaccctaa ggagtcgtca gtatagaggc agtttttgtt tttcctagat tctatcatcg 905940
cagtggcaag ttttctgata ggtaacgaca atcgtcttgc tacaataaag gctatgaggc 906000
tccctaagag aatgcaaaag aaataggcag tgtacatgcg agctctgcgc cataaaggcg 906060
caaagctctc ttctttttta gcataggaaa ggactgcaat atctatactg ggaacatttt 906120
caatacagcc ccaaatctca gtatctttga ttttaaaaga atagaaattt tctccaatat 906180
ccagagggga gagagttaaa ggacctaatt ctgagtctat agggcaagga tcatcattga 906240
gaaaaacttg gcagaatttt tctttcgtca tgtcagggta gacagtatgg agatggagag 906300
caggatcaga agcttttaag ataacgccgt atttggaaag gatcgcagtt tttacagtga 906360
gataggattg cttgtttatc aggagatctt tgagtaagct ctcagcactg aacgtggtgt 906420
ataagatccc ttggagttct tgagtttttg catcaaaaac attagcttgc attacagaaa 906480
agacttcctg atttttagga gattgtttta gggctgctaa aaatggagtg ttcttaggga 906540
tgtctatttt gtgattatag ttttccccta agtgttcagg aatgctagaa gcaacaacaa 906600
ttttatctcc atttgggaat accttgatta aagagatttc attatagatc ccttggaata 906660
ctttctgcat ttcattgcta aggagaacgt tcggagtctc tggaatacca gcatctaaat 906720
ctaagacatc agaaaataga gatagaacat cgacattcaa agggacaatc tgagtgagag 906780
tattggcttt gaaagcagcg ttttctttca gtgcagtaga aatagcagag actatggtgc 906840
ggtattgatc taggtttaac catacgatgt tgatccctag aggtgcaatg atagcaacgg 906900
cgcaacaaac cacaaacgga accctattgt tttagtaaaa gggatcatgg gtattacggc 906960
gctctattct taaggcttgt catccttgag aaggaacgtc tttcctctag tgttttgttt 907020
cttacgcagt ataaaaaaat ttccttaagg agacacctac tatttctttt attcttgcta 907080
tatcttagta aatcaattgc ttgcaacgaa gatcttattt tttcatctcg atcttctaat 907140
gaatgaaaat attattctct aacttctttt aaatcacatg gatgtttgag aatatagaca 907200
ataaaaattg ttaaagcttt tgttttttac tcacaatact tatgtagaaa tcttctaaac 907260
acagaagctt tctactaaaa aagagcggag ggaatcaagt gagtctatat caaaaatggt 907320
ggaacagtca gttaaagaag agcctctgct attcgactgt tgctgctcta atatttatga 907380
ttccttctca agaatccttt gcagatagtc ttatagattt aaatttaggt ttagatcctt 907440
cggtcgaatg tctgtcagga gatggtgcat tttctgttgg gtattttact aaggcgggat 907500
cgactcccgt agaatatcag ccgtttaaat acgacgtatc taagaagaca ttcacaatcc 907560
tttccgtaga aacggcaaat cagagcggct atgcttacgg aatctcctac gatggcacga 907620
tcactgtagg aacgtgtagc ctaggtgcag gaaaatataa cggcgcaaaa tggagtgcgg 907680
atggcacttt aacaccctta actggaatca cgggggggac gtcacatacg gaagcgcgtg 907740
cgatttctaa ggatactcag gtgatcgagg gtttctcata tgatgcttca gggcaaccca 907800
aggctgtgca gtgggcaagc ggaggnctac agtaacacaa ttagcagata tttcaggagg 907860
ctctagaagc tcttatgcgt atgctatatc tgatgatggc acgattattg ttgggtctat 907920
ggagagcacg ataacaagga aaactacagc tgtaaaatgg gtaaataatg ttcctacgta 907980
tctgggaacc ttaggaggag atgcttctac aggtctttat atttctggag acggcaccgt 908040
gattgtaggt gcggcaaata cagcaactgt aaccaatggg aatcaggaat cccacgccta 908100
tatgtataaa gataaccaaa tgaaagattg aggaacttta ggaggggcga attcttcagc 908160
aactggagtt tcttcagacg gttctgtgat tgttggtcag gcgcagacag ccgataaatc 908220
cgtgcatgct tttcaatact ataatggtga gatgaaagat ttggggactc ttgggggtac 908280
ctcttctaca gcaaaaacag tgtccccaga tggtaaagtg atcatgggta gatcacaaat 908340
tgctgatggc agttggcacg catttatgtg tcatacggat ttctcctcta ataatgtact 908400
ctttgatctc gataatacgt ataaaactct aagagaaaat ggccgtcagc taaattccat 908460
attcaaccta caaaatatga tgttacagag agcctcagat catgagttca cagagtttgg 908520
aaggagtaac atcgctcttg gtgccgggct ttatgtgaat gccttgcaga atctccctag 908580
caanttagca gcacaatatt ttggaatcgc atacaaaata cgtcctaaat atcgtttggg 908640
ggtgtttttg gaccataatt tcagctccca cgtttcctaa taattttaac gtaagccaca 908700
atagactctg gatgggagcc tttattggat ggcaggattc tgatgctcta ggatctagtg 908760
tcaaggtgtc tttcggatat ggaaaacaaa aagccacgat tacaagagag caattagaga 908820
atacagaagc cgggagtggg gagagccatt ttgaaggggt cgctgctcag atagaagggc 908880
ggtatggtaa gagcctcgga ggacatgtca gggtccagcc tttcctagga ctgcagtttg 908940
tccacattac aaggaaagaa tataccgaaa atgcagtgca atttcctgta cactatgatc 909000
ctatagacta ttctacaggt gtagtgtatt taggaattgg atctcatatt gcacttgtag 909060
attctttaca tgtaggcaca cgcatgggaa tggagcaaaa ctttgcagcc catacggaca 909120
ggttctcagg atctatagcg tctattggaa actttgtgtt tgaaaagctt gatgtgactc 909180
acacaagggc atttgcggaa atgcgtgtca actatgagct tccctatcta cagtctctga 909240
atcttattct acgagttaat caacagcctc tacaaggggt tatgggattt tccagtgatc 909300
ttaggtatgc cttaggattc taaagaagaa aatttaattt tcttgcgaac atttctatcc 909360

atagttgata gagataaaca gataaaatat agctcagaat ataatctgag ctatattctt 909420
cttacttgca gccatgtctg aatatgagta tagtcagaaa ttctgcattg ccacttccgt 909480
gtttaagcag atccgaaacc tttaaaaaag ttaggtcgca tatgaaattt atgaaagtcc 909540
ttactccatg gatttatcga aaagatcttt gggtaacagc attcttactg acagcaattc 909600
caggatcttt tgcacatact cttgttgata tagcaggaga acctcggcat gctgctcaag 909660
caacaggagt ttctggagat ggtaaaattg ttataggaat gaaagttccg gatgatcctt 909720
ttgctataac tgtaggattt caatatattg atgggcattt gcaaccctta gaggcagtac 909780
gtcctcaatg ctctgtatac cctaatggta taaccccgga cggaacggtt attgtgggta 909840
caaactatgc catcgggatg ggtagtgttg ctgtgaaatg ggtaaatggc aaggtttctg 909900
aacttcccat gctccctgac accctcgatt ctgtagcatc ggcagtttct gcagatggaa 909960
gagtgattgg agggaataga aatataaatc ttggcgcttc tgttgctgtg aaatgggagg 910020
acgacgtgat tacacaactt ccttctcttc ctgatgctat gaatgcttgt gttaacggaa 910080
tttcttcaga tggttctata attgtaggaa ccatggtaga cgtgtcatgg agaaataccg 910140
cagtacaatg gatcggggat cagctctctg ttattgggac tttaggagga actacttctg 910200
ttgctagtgc aatctcaaca gatggcactg tgattgtagg aggttctgaa aatgcagatt 910260
ctcagactca tgcctatgct tataaaaacg gtgttatgag cgatataggg accctcggag 910320
gtttttattc tttagcacat gcagtatctt cagatggttc tgtgattgta ggagtatcca 910380
cgaactctga gcatagatat catgcattcc aatatgctga tggacagatg gtagatttag 910440
gaactttagg agggcctgaa tcttatgctc aaggtgtgtc tggagatgga aaggtaattg 910500
tgggtagagc acaagtacca tctggagatt ggcatgcgtt cctatgtcct ttccaagctc 910560
cgagccctgc tcctgtccat gggggaagca ctgtcgtaac tagccagaat ccacgtggaa 910620
tggtagatat caatgctacg tactcctctt tgaaaaatag ccaacaacaa ctacaaagat 910680
tgcttatcca gcatagtgca aaagttgaaa gtgtatcctc aggagcacca tcttttacaa 910740
gtgtgaaagg tgcgatctca aaacagagcc ctgcagtgca aaatgatgta cagaaaggga 910800
cgtttttaag ttaccgttcc caagttcatg gaaacgtgca gaatcagcaa ttgctcacag 910860
gagcttttat ggactggaaa ctcgcttcag ctcctaaatg cggctttaaa gtagctctcc 910920
actatggctc tcaagatgct ctcgtagaac gtgcagctct tccttacaca gaacaaggct 910980
taggaagcag tgtcttgtca ggttttggag gacaagttca aggacgctat gactttaatt 911040
taggagaaac tgttgttctg caacccttta tgggcattca agttctccac ctaagtagag 911100
aagggtattc tgagaagaat gttcgatttc ctgtaagcta tgattctgta gcctactcag 911160
cagctactag ctttatgggt gcgcatgtat ttgcctccct aagccctaaa atgagtacag 911220
cagcaacttt aggtgtggag agagatctga attcacatat agatgaattt aagggatccg 911280
tctctgctat gggaaacttt gtcttggaaa attctacagt gagtgtttta agaccttttg 911340
cttctcttgc tatgtactat gacgtaagac aacagcaact cgtgacgttg tcagtagtta 911400
tgaatcaaca acccttaaca ggcacactaa gcttagtaag ccaaagtagc tataatctta 911460
gcttctaagc tattgttcaa taaataatca gaacatgtgc agtccatagt tggaaatgct 911520
gattaagtac ccaactatgg actcttcttt ttagttttaa aaatacacag atacaacgtg 911580
tagtgccttt aaaggttcag agactaataa aaatattttt aatttcttct ggcaattctt 911640
ccgccatatc cttataacta taagccgttt aactaagtgt tgattttaac ttggatcaac 911700
gtacttacaa agttaggttt aaatatgagt aagaagataa aggttctagg tcatttgacg 911760
ctctgcactc tgtttagagg agtgctgtgt gcagcggccc tttccaacat aggatatgcg 911820
agtacttctc aggaatcacc atatcagaag tctatagaag actggaaagg gtataccttt 911880
acagatcttg agttactgag taaggaaggg tggtctgaag ctcatgcaat ttctggaaat 911940
ggcagtagaa ttgtaggagc ttcgggagct ggccaaggta gtgtgactgc tgtcatatgg 912000
gaaagtcacc tgataaaaca tctcggcact ttaggtggcg aggcttcatc tgcagaggga 912060
atttcaaacg atggagaggt ggtcgttggg tggtcagata ctagagaggg atatactcat 912120
gcctttgtct tcgacggtag agatatgaaa gatctcggta ctctaggagc tacctattct 912180
gtagcaaggg gtgtttctgg agatggtagt atcatcgtag gagtctctgc aactgctcgt 912240
ggagaggatt acgggatggc aagttggtgt caagtgggaa aaagggaaaa tcaaacaatt 912300
gaagttgttg cctcaaggtc tctgggtctg aggcgaatgc aatctctgag gatggtacgg 912360
tgattgtcgg gagaggggaa atctctcgca atcacatcgt tgctgtaaaa tggaataaaa 912420
atgctgtgta tagtttgggg actctcggag gtagtgtcgc ttcagcagag gctatatcgg 912480
caaatgggaa agtaattgta ggatggtcca cgactaataa tggtgagact catgccttta 912540
tgcacaaaga tgagacaatg cacgatctcg gcactctagg aggaggtttt tctgtcgcaa 912600
ctggagtttc tgctgatggg agagccatcg taggattttc agcagtgaag accggagaaa 912660
ttcatgcttt ttactatgca gaaggagaaa tggaggattt aacaactttg ggaggggaag 912720
aagctcgagt gttcgacata tctagcgaag gaaacgatat cattggctct ataaaaactg 912780
acgctggagc tgaacgcgcc tatctgttcc atatacataa ataaaagcat cgtagagaaa 912840
agataaacga agtaaatcgt aagcttggaa ttcatgaagt gattcataat tccaattttc 912900
atacttgttg tcttttctct atgtagataa agttaagtgg tttttgaaat tatttttgtt 912960
gttagagtcc ctatgaaaaa gacatgttgc caaaattaca gatcgatagg cgttgtgttc 913020
tctgtggtac ttttcgttct tacaacacag acgctgtttg caggacattt tattgatatt 913080
ggaacttctg gattatattc ttgggctcga ggtgtatctg gagatggccg cgttgtcgta 913140
ggttatgaag gtggcaatgc atttaaatat gttgatggtg agaaatttct gttagaaggt 913200

ttggtcccga gatccgaggc cttggtattt aaagcttctt atgatggctc tgtaattata 913260
ggaatctcgg atcaagatcc gtcttgccgc gctgtgaagt gggtaaacgg tgcacttgtt 913320
gatcttggaa tattttctga gggaatgcaa tcttttgcag agggtgtttc cagtgatgga 913380
aagacgattg tagggtgcct atatagtgat gatacagaga caaactttgc tgtgaagtgg 913440
gatgaaacag gaatggttgt tctccctaac ttaccagaag atcgacattc ttgcgcttgg 913500
gatgcctctg aagatggctc tgtgattgta ggggacgcca tgggtagcga ggaaattgcc 913560
aaggcagtgt actggaagga cggtgaacaa catctgcttt ctaatatccc aggagctaaa 913620
agatcgtcag cacatgcagt ttctaaagat ggatctttta tcgtaggcga gttcatcagt 913680
gaagaaaatg aagttcatgc ctttgtttat cacaacggtg ttatcaaaga tatcgggact 913740
ttaggaggag attactctgt agcaactgga gtttctaggg atggtaaggt catcgtgggt 913800
cattctacaa gaacagatgg tgaataccgt gcatttaaat atgtggatgg aagaatgata 913860
gatttgggga ctttaggagg ttcagcatct tttgcttttg gtgtttctga cgatggcaaa 913920
acaatcgtag gaaaatttga aacagagcta ggagaatgtc atgcctttat ctaccttgat 913980
gattagggtt cttatggaag tcgtcctata ctcagaaaat tgctaacaca tcaacgtaga 914040
taaatgtaga taactgacga ttcttattct gattggactc caacaataat ttctccatca 914100
atagaaacag cgtttgcaca ggcttctttc cagctatact ctcctaagtc gatcattctt 914160
ccaccgacat atttaaaggc atgcaatttc ccatttgctg ttgtggacat accgacaata 914220
acttttccag ttgcagagac tcccttagct gcagaataag atcctccgag ggtgccgagg 914280
tctgacatga cgccattctt atgacagaaa gcatggacct ctccataata tgtttcggat 914340
ctccctacaa tgaccttgcc attattggat acagcttttg caatggcaga atagcctcct 914400
aaagttccta gatgtgtaat agtgttccct tcccacttta cagcataaag aattccagag 914460
gcgtcttgta cagaccctac aattatagat ccattggcag atacagaatt cgctacagat 914520
ttgacagccc agcctactgg taacacctga gcctcgtgtc ccgtccactt cacagcgaaa 914580
gttcgcgacc aagcatcctt agcactccct acaataacag agccatcaga agaaattcca 914640
tacgctttag attgcactgc ccatgtggaa gggagcaaat acatttcatt gtttttccat 914700
gtgactgcga aactttgaga aagctcattc tcgacaatcc ccgcgattat agacccatca 914760
gaggagattc ctgttgcttt tgattttttt acccatgagc tggtagggag ttctttggtc 914820
ccccaataat tccatcttac tgcgtatgtt cgagaccaag tgtcttttat aaagccgaca 914880
gttgtagcac cagttcctga aacagctttt gcagaggctt ttttatttga taatcttgag 914940
agagttgtct ttccccaatg atcccatttt actgcggatg aagaatggaa gtcatcttct 915000
ggtttgtctg taattacata gcaataacca gatagagaat ataatgaaaa taggaccatc 915060
cataagctac tctgagatag catagaacgt aaaatttgtt ttatagctgc catattttcc 915120
tctcgttttc aaggtttgca aatgaatttt tttttaatcc aagaaactat accaagatat 915180
caaaagagct acaagcaaga tcctaggagc ggttttttaa tgaaaataga ataagaaagg 915240
atttatagac gtgctgatga agatgtcttg aaagagggaa ataaagaaca cattgcagat 915300
cagaaagata aggcccatag atctgcgatg tattcttgat ttggagataa gatagaaata 915360
gagaaaatcc ctctattcct cagaatcctc gtaagaaaat acattagaat ctgtgaaaat 915420
tgcttcggat ccaagctctt cttcaatttc catgagtcta ttgtattttg caacacgctc 915480
agaacgtgat aaagagcctg ttttgatttg accagcgttg aaggcaacag caagatctgc 915540
aatcgtagtg tccgtagttt ctcctgagcg atgagaaata attgtagtat agccagccat 915600
ttgcgcaact tgatagcata cactgtttca gtaagcgtcc ctatctgatt tggtttaatc 915660
aacacagagt tcgctaatcc attgctaata ccctctaata ttaattccgg atttgtaaca 915720
aataggtcat cacccacaat ctgtactttt tctccaagaa cttcagttaa caaggcccac 915780
ccgtcatagt cttcttcagc aagaccatct tctatggagt ctataggata gcgatcacat 915840
aaattagaaa ggattgcgat ttgctcttca tagtgcctcc catcatacgt gcctgttttt 915900
acgttataga atgaggatgc tgcgcagtct aaggctagcg atatatcttt tcctggagta 915960
aagcctgctt tttcaatagc cagcaatagg agctctagag cttcttcatt agaagcaaga 916020
ttcggggcga agcctccttc gtcacccact ccagtagata agcctctttc atggagtaat 916080
tttttcaaag tatgaaaaac gtcagcaccc atgttgacag cttctttgat ggaagaggct 916140
ccaataggac ggatcataaa ttcttggaac cccaagccgt tatcggcatg catgcctcca 916200
ttgatcagat tcatcatagg acagggaaga ctgcaggcaa aacaccctcc taaataacga 916260
tacagaggtc tgcgtagtgt tgctgctgct gcatgtgctg tagctagaga gactcctaaa 916320
atagcattgg cccctagagt ttctttgttc ggagagccgt cagaatccat catcagagaa 916380
tcaattaagg attgctcata aacactacat cccttgacga ggggaaaaag aatttctttt 916440
acgtttttta cagcttgcaa aacccctttg ccttgataac gaggagaatc tgtatcacga 916500
aactctaagg cttctttttt ccctgtggat gctcctgaag gaacccgagc ttctccaaca 916560
gaacctgtgc tagtggttac tttaacatgt aaagtgggat acccgcgaga atccaagatt 916620
tccctagcct ggatatcggc aatgacagct tcaaacataa tttttatctc ttttctttaa 916680
gactttgcta agagctcaat agcatgtgaa cctactgatc ctgctctgct tcttgaactg 916740
tagatatgta ttctaaaata cgtagctacg gattcacatc aaactcataa tgacaaaaat 916800
agataaattt ggggaggaat attcttgaat atcgagatca gaaattctct tagaacaaat 916860
agagaagctg ttctttgcaa gcttgcattg catcgcggta tttcgcagct tcgttgaatc 916920
gaaattcttt agcagcacgt tgcatgagag cttcgtattt cttaatttgc tcttcaagat 916980
cttctttaga tagggggcgt tgagattcct tgggagactc cgagtcttta gaggtttgta 917040

gaataggatt ggcaaaaata gcttttataa tgggcttagg aacaatattg tgctctttat 917100
tgtaatctaa ctgtatctga cgccggcgtt cggtctctcg aagagtctcc tctatagaac 917160
gggttttttg atcagcatag aaaatgactt taccattgat attcctagca gctcttccac 917220
aaaactgaat cagagatgac gtgcttctta aaaagccctc tttgtcagca tctaagatcg 917280
caactaaaga gacctcggga agatcgagtc cttcacgtag gagattgact ccaatcaata 917340
cgtcaataac gcccgagcgt aggtctgtaa ggatttgcgt gcgttctgca gtttctattc 917400
cagaatgcaa gtatgccgca gggatctcta attctgaaag aaaacccgcc atatcttcgg 917460
caagtttctt tgttatagaa atcactagga ttttttcatg tttctgagac agccgcaaac 917520
gaatttcttc aagaagatca tccacttgtc ctgtagcagg acggatttca ggcataggat 917580
cggggatccc cgtcgggcgg atgatttgtt gtacaatatg acctgagctc tcttggactt 917640
ccgtatcacc tggtgttgca gagacataga tgactttacg aaagtacttc tgggcttctt 917700
caaaaggtta gcgggcggtt atcgaaggcc tgaggggaaa cggaatccat attctactaa 917760
agattgcttc cgagattggt ctccacgata catagcacgt atttgaggta gtgtttgatg 917820
agactcatcg ataataagga gaaagtcctc aggaaaataa tctaagaggc acgtaggcgg 917880
tgctcctggg ggcgcccctg taaaatgccg tgaatagttt tctatgccct tgcagaatcc 917940
gatttccttg atcatctcaa tgtcatgagt cgtacgatgg aaaatgcggt ccttttctat 918000
aggacggtcg tcaaaaaagg ccatgcgctc ttctaactcc tcctgaatgg ttcggatcgc 918060
ttgctcgcga attgcctcag gaatgacgta atgagatcca ggatataaag tagccgaagg 918120
gacactttcc ttgggaatca tagtgagggg atcgctgtat tctatggagg taagggtgtc 918180
atttagaaat tctaatctta gggcaagttc actttcgtat gcagggaaaa tatcgattac 918240
actcccacgc tctcgaaatg cacttctctg tgggatggga gatgcttgat agtgcatttt 918300
aacaagctga gctgtcagga tattcctagg gtactctttc cctacctcta agactaacgc 918360
catggatgta tagttttcag gagaaccaat accataaatg caagatactg aggaaacgat 918420
taaagtgtct cggcgctcta aaatagaccg tgttgctgat aaacgtaact tatcgatttc 918480
atcattgata aggaggcttt tttctatata ggtatcacta cgggcaatat aggcctcagg 918540
ttgatagtaa tcgtagtaag agatgaaata ctcaacggca ttattaggga aaaattcacg 918600
aaattcttga tacagctgag cagctagcgt tttgttatgg gctaaaacca gggtagggag 918660
atttacgttg gcaacaacat tcgcaatagt gaatgtcttc ccagaacctg tagttccaag 918720
aagtacttgt gattttacct gattacgcac acccgcagac agccgagcaa tcgcctctgg 918780
ctggtcgccg caaggagcaa aaggagcatg aagttggaat gtcatagtaa aaattattta 918840
agcaatgagc gccatttatg gctaaaccca aatttgtcat gaacttcttc catagtgact 918900
ttggctactt cacgcatttt atgcgtgcca tcctctaaga cgttttgtaa tgccagaggc 918960
ttggatagaa attctgagcg tctttctttt atgggtttta aaaagtgaat cagctcttca 919020
gcaaggcgtg cttttacttc gatgtcttta atgcaacctt gacgatagcg tgctttaaac 919080
tcttcaacct catctttatg gggattgaag atgtcatgat aaataaagag gggatttcct 919140
tcaactcgac ctggtgtggt tgctcgaatc cggttgggat ccgtgtacat cttacggact 919200
ttctcagtaa ttgtagcgtc gctatcggaa agatagatcg cgttgttcgc ggacttactc 919260
attttccctt gcccgtcaat tcctacaaga gaggtaagct cgccttgtag tacctcgggc 919320
tctggaaata cctgcccata cagacggttg aaatttctag cgatatcccg ggtgagctcc 919380
acatgcgctt cattgtcttt ccctacaggg acaaactgtg ctttcgctag aagaatatcc 919440
gcactttgta aaatagggta cccaataaga ccataagaaa gacttccctc ctcgattgag 919500
gcatttcggg ccatatcttt gaggctggga atgcccatca ctctattgat tgagatgagc 919560
atcgaaaata ggagatgtaa ctcgtagatc tcagggatcg cagattgtag gtaaattata 919620
gatttcgttg gatcaatccc tacacttagc caatcagcaa gaacttcata gatgtggtta 919680
tctacatcta aaacctcttc cttgcggatt tttgtagtta gggtgtggag atcggcaata 919740
ataaaaaagc aatcgtactc agggctattt tgaagttcta atcggttttt tatagaacca 919800
acccaatgcc ctaaatggag tttccctgtg ggacgatccc cggtaagtac gcgctttttt 919860
ttattcatga cgtccgctat tgaagtgctt tagttagctc atcaaagtga ttttttaatg 919920
atgtaatttc ttcttgaatt tcctgaagat ttttctctcc aacaggattc ttttgtgctt 919980
ctaaaagtct tgctagtaaa tgacgaatca agtggcaggt attctgcaca tcagtcatca 920040
ataagaactc ttgaccatcg acacgagcta atcgatttaa gaaaatacaa cgctcttgtt 920100
gggtaagcat atctgaagtg atgaaattaa taaattcccc gcgttcatgg atgtctttag 920160
cgacacgaat aggcatcata aattcggcaa ttaaactatg cgcagcttgc ttttgctgag 920220
gagcaagttc ttgtgttaac ttgccattaa ttaacatctc gaaaaagaaa tttgcaatct 920280
cttgctgacc attggcacag ttgaaaacga cattttgaaa ttctcgaaag gttgtgtaac 920340
caataggagt agcaaaaatt ctttttaaat ttccttcaag aaggagaaaa acattgtctt 920400
caagctctaa tgtttttgtc tttgctgcca taaatcatac ctttgtatcg ggaaagaggt 920460
tacttttaac tttttctagt ttttttatca atttttccaa aatgatctgc tcatcagaag 920520
agagactcgt acacttagtt agtatttgag agcctttggt aagcatagca agagcctgct 920580
ttactggtga tgtttttcta caatctgtct caactaaggg gaactctttg cgaatgcgat 920640
ctaaaagctc gctttttctt tcccctcggt agtttcggat aatctcttct tttttttctt 920700
ggggcccttg acgagatgct aaagtataga cagcttgtct aggcatcttt tccatctcta 920760
tttttaaagg ttcaggaaga agggtaaaga gttcgtagta aactaaaaaa ttataggggg 920820
tttgtctatt cccataggtt aagagaagcc aagaagaaaa agcaccttca cgatagcttt 920880

tcagtaaatc acggactttt gtgatgcgct ccccatgtaa aaggacagat tgatgatgga 920940
tttgcttcac ttcagcagaa agagtacaga gctgggtaag atcaggttct tcaatctgtt 921000
ctcgaaagtt gtagtgctga agcagatgac ggagttgctc tttttcttta gaggaaagcg 921060
taggattgga aaggcgaacg gcaagaggag agggatctcc ttccatgcgt ttacgggcaa 921120
gcgcttccat tttagtggga gtattctttt tgaagcgact ttctaagagc gtttttagat 921180
tccccataga gctaaatatc tcttaataaa attagcaatt ctttagtgag gttgaaataa 921240
tcttcagagg cacgtgcact gggagatgta gcaaagacag gtttgccatg aatcgcagct 921300
tctgaaacag tgatgtctct acgtattttc gtattcaata acttcccggg gaaagttttg 921360
tgaatcagct cagcaaatgc ggaattgttt ttccctctgc aattccaaaa ggataaggca 921420
actcctaaaa tcgttagggg atgtcgtgct gaaattcctt ggatgaaacc agccagacgt 921480
tctagacctt tgacactata aaattctggg gtagcgcaaa taagcgcata atcagcagca 921540
attagggcag attctgtaag ccaacaaagt gaaggaggag tatcaataat cacatagtcg 921600
tacttgtctt ggacagaacc aagtacatat ttcagtcttt catgagaata gcgatccgca 921660
gcaagatttc ctgacacttc aatacgctct aaccaggtgt cagcaggaat taaatccagc 921720
tgagtgtctt ggatgggtcg gatgacttct tgaatttctt tttcgccttg taacacaaca 921780
gcaaggctgt catagcaatc aggatctaag cctaatccag aggtgagatt tgcttgagcg 921840
tcaaaatcaa taagcaatac cctggcttga tggtactggg caagagccgc accaagatga 921900
agggttgttg atgtttttgc tgttccacct ttaaagctgt ttacagcgat ggtcttcatt 921960
cttgatgcgt atcctcaaaa atccttagca actacaataa tgcagttaaa cttaaagagt 922020
tcttttcttc aagtatagtg tttaagaatc tctctacaga gacatcatta atcactcggt 922080
tatctcgggt gcgtactgcg agtacatttt cattaatttc atgatctcca agagtgatca 922140
tgtaattgac ctgcatgttt tgggcattgc gaatcttttt acttacagac tcactagaat 922200
cgtctaaggt gacgactaag cctaaacgtt tccacgcttc ctcgagctct ttcgctctag 922260
gaatatggcg atcagcaact gtgatgatac gcacttgctc aggacttaac cataagggga 922320
atcttccttt gaagttctca atcagaattc ctaaaaagcg ttctatagaa ccaaaaagag 922380
cccgatgtaa catcacagga acactcttag ttccttgagc tgtggtgtat tctaattcga 922440
aacgctctgg aaggaacatg tctaactgga tggttccaca ttgccatgtt ctttgaatcg 922500
catctttaac atggatatca attttaggac cgtaaaaagc accttctcct ggacgaacaa 922560
tgaagggtgt cccggactga actaatgctc gatttaaggc atctgttgct agctcccata 922620
gagaatcatc cccaatcgta tctttttctg gacgtgtgga gagttccagg tgatactcta 922680
atccaaatgt accgtacagt gtagaaacta actgaagaat attcaaagtt tcctcttcga 922740
cttgctctgg agtaagaaat acatgagcat catcttggtg gaacgcacgg acacgcatta 922800
accctgaaag ggctcccgaa gcttcttgac gatggacatg accaacttct gctacacgta 922860
gagggaactc cttataacta tgcaagcggg ttttataata gagcatgcat cctgggcagt 922920
tcatgggctt aatggcataa tcttcatcgt cgatttgcag tgtgtacatg tttgctttat 922980
aattgtccca gtggcctgaa acttcccata actgacgatt catcaactgt ggcgttagga 923040
tttccttata acccgcagct gtgtgcagct gcttccaata acgaatcaat gcatcccaaa 923100
caatcatacc gcgaggatgg aagaagggca ttccaggaga actttcttgc tgagaaaata 923160
gatctaactt ggcccccaaa acacggtggt cccgtttttt tgcttcctca atttgctcta 923220
aatgtgctcg aagttcctta gatgtaggaa acgaagttcc gtaaatacgt actaaagatt 923280
ctcgagaagg atcccctcgc caataagctg cagatgtgcg taagacttta aacgctttta 923340
catgagctgt agagggaagg tgaggacctc ggcaaagatc aaaaaactcc ccttggctat 923400
aggcagaaat ttcttcattt tcaggaagct cacgaatcaa ctctgtctta aatggattct 923460
gtgggaattg tgctaaagct tgttgcttat cgccataagt aaagcgagaa atcgcaagct 923520
tttcatcaac aatttgtttt actgtatctt caattaacgg gaaatcactt tcgctaatgg 923580
aaagattggc aaaatcatag taaaagccat ggtcgatgac agggccgatt gtaggaattg 923640
catcaggcca taaacgcaat acggcttggg ctagaagatg ggcagaagta tgaagaaaaa 923700
tttctcgtct tcgggatctt cggaagtcag aaatactaag gtgtcgcctt cgtttaggtg 923760
ggtggaaaga tctctagggc gttcgttaat gagaacacca atgaattgat gagaattttt 923820
taattgttta gcgagttccg ctgctgtagt accttcgagt acttcataat ttttttgatc 923880
acaagttact tgaatcatct tgttccacgc ctccatgtta gggggacttt cgttgttagc 923940
attttagtgt tttttttgat caaagcaaga agcttttttt tataaaaaaa cttttatttt 924000
ttaatacaag ttccaaagcg ccaggagatt tagattggaa ctaataaaaa catcttttat 924060
actatctgct taataaacat tacaaactgg tttgcatatg tcgctttttc tagtttttct 924120
tacggcattt atttggtctt cttccttcgc tcttagcaaa ctagttatga atgcttcagc 924180
tccgatattt gctacaggag ctcgcatggt aatcgctggt gcgatcttgg ctcttgctgc 924240
atggtttcga ggcggttttg ttggtatatc gaagaaaata ttcttatata tcgtcctgtt 924300
agctttaaca ggtttctatc ttaccaatat ttttgagttc ataggattac aaagtctaag 924360
ttcatctaag acatgcttta tttatggact ctctcctcta atgtcagcac ttttttccta 924420
tattcagctg aaagagaaag tgactctcaa aaaggtttta ggattatccc taggcttggt 924480
gagctatatt tgttacttaa cctttggtgg gggaggagac gattctcagc cttggacctg 924540
gcaaataggt cttcctgagc ttctaatctt aggggcagca agtttagctt cttttggctg 924600
gactcttctt agacaaatcg aaaagcagtc tacgttatcg gtcacagcaa ttaatgcata 924660
cgcgatgtta atagccggaa tgctatcaat catgcactct gcagtcgtgg aaccctggcg 924720

tcctttacca gtgcaagata tatcgcagtt tctatacgcg actttggctc tagtggtaat 924780
ttctaatttg atttgctaca acctgtacgc caaattatta agaaagtatt cttccacttt 924840
cctttcattt tgtaaccttg tcatgccact ttattcaggc ttttatggtt ggatattgct 924900
tggggagaag ggagtctcct tgggcttggt gttagctgta gccttcatgg tggcgggctg 924960
tcgtctcatc taccatgaag agttccgaca gggctacatt gtttcttaaa gtaaagccgc 925020
tttacatgaa aaagctcggt atcaaaccga gctttttttg atcagatctc taattgtgat 925080
taattaaaga tagcagatgt tgtttgtgtt tgagagtccg caatattttg tagtgtttgt 925140
aaggcagtat taaatgattg catagcttgc tcttgtaatt gtcctgcttg tcctgcttgc 925200
tgaccataaa ccgaagacat ttgtttcaag acttctgctt gagcctctgc ttgtcctgct 925260
aaccgctgat gatgggcgac ttctattcct gtctgccccg cagaaagacc ttcaacagcc 925320
gctgtgagtc catgcattaa ttgactcatt tgcatagagg aagaaagagc atttcctgca 925380
aatgatgcaa cacgcgctcc ctgagtttca ctacgttcat acctctggag actttttcag 925440
accagttagg tttatttaag acttcgccaa agagtcctcc tgaagctttc gaagctccat 925500
cagaggccat ttttgaagct gctgcggcca tatcatcggt taggttcgct gcagctttgg 925560
tggctgctga tcccgcagaa ctcgctgctg ttgttgccgc cttggcagtc gaagccatag 925620
tctgttgaac agaagaactt gcagaagtta aagctttgga agcagcacct ccagcagctg 925680
aggcacctgt ttctttggca aaggaagcag attttaaagc cgaggtagct ccttttgcag 925740
cagagaaaat acccgctcct acagaaactg taaatcctac aatgtttaca ataccactaa 925800
tcaaggactg ttttgcctgt gcttctgtag ctgatgcttg atggttcgct tgccttttaa 925860
tagcctctcc aatagcagga gcggattcga cttgtgaccg gatcgcttgg ttttgtgatt 925920
ggaatgaagc tttccaagac tctccggcag cattggccat caaggtcata actaaaccga 925980
ggagtgctaa cgtacccatg ccttttttaa gtacaatacc attgatggtc tgtgcctcag 926040
gagtaggaag gctgggtgag tcataattat tttgtcttaa tcccttagga ccttgtacgg 926100
cttgtgccgt tgcagtttta cttgcgcttg ctaccgaagt tgaggattta gatttcccag 926160
atttttcgct tttttctccc ttactacttt tagtagttga agatgtagct tctttttttc 926220
ctgtactttg cgctgaagcg tcttgaatga gatcctcaaa gcctccagca gcgacttctg 926280
cttgagcccc ttgctttgtt acattcttag tatcgtgacc gctctgagca tttcctgctt 926340
tttgagagga ttgtgccaat tgcgcagcca atgtgggatc ctgacttgaa cttccactaa 926400
ctcctgatgt catagcaata catgctcctt taaaaattat tggttttatg ctgcgccacg 926460
atggctccgc tgattgcggc atacgcttta aggatttgag cgcccagctt ggtagctttt 926520
tgagtcattt cattagactc gcctgtttgt tttgaggcaa ttttacttgc ctgttgccaa 926580
aattgagtga acatagaaat catatcagcc gcagcctgca gttttccgac ttctttctga 926640
aattgagcga cgttttgttg catctccgag agctgcattt gcataatccc tttaccgaga 926700
gcaggcgccg caactacaac cccaacccca accgtgaccc atttactggt aagagacgag 926760
atgactttcg agagcttggg gaagttcttc gcaatcattt gagttccctt agcgaaaacc 926820
ttagagattc ctttagaaat ggctttggca atcgctttga ctaaagtttt gataaatgct 926880
tttattccag atttgacagc cgcttttata gccgcggtga tcgcttgtct gacagctgtg 926940
ataacagctt gtttcaccgc ttggacaaca gcttgaactg taatttgtgt tgctaccgtg 927000
gttgcggcag cagctcctgc tgcacctcca gctgccgctg cacctacagc agctcccgca 927060
gcgagtccag cgagtccagc tccgcatgta aaaatagcag caacaataga aataactgta 927120
atggcaacag aaaccgcgat catcacagta ttgacagtat ccattgttcc ttcgagatct 927180
ttagacttct gttcggcagc cttcatctct tggtattctt ctcgttcttt atcgattttt 927240
atcgcttgct tttctagacc tagtttattt gtttggtctg cttgtgcttg tgtacttgca 927300
tagttagata aggcagattt tgtggcttct cccaatgtct gaattgcttt agcaagcgcg 927360
agtccgattt cgataacctc tgatcttggt gtcaccccgg gcttggggag ctcaggtgtt 927420
tccaattttg cggaacccga acttttccct gagagggcag caacaacaac cgcttcgact 927480
tctttcattt gcgcggcact gaggctttga agtgactcta aggtagactc catacttttg 927540
ctcgcctctt caatagaggt ctgcatagca atttttgttg cagtatttga tgctgtagta 927600
gcagccgctc ctgatactcc agtatcagca cctgcctttt gactttctga ggattctttc 927660
ccagcagcaa ctccctgttg tggagctgtt tctgtttttg tagtcgagga agttttgtct 927720
tttccagaag caccagcaat agtggcatcg ctttccatct cagtgttttt accctgacgt 927780
gtttgctgta tttgcttcgt ttcgttgcca gacagcttat cttgttgggg cacgccctgg 927840
ggtgtcgatg tcagaacttg agacatgata tttttttgat tgtcaggtcc tgaagaagat 927900
gaaatagaca tgtttgtatc ctaaggggtt tgattctgca aagggcttta ctcagcgcct 927960
ttaaccattt gtttattaac tacagtggtt aggtttttta atccgtgtac tactgaagat 928020
aactcctttg cgaagttggc atcttcttgg gcagaaggga ataatccttg agttaactct 928080
tcgagatgta aagattctag agttccagaa agagccgcga tgatgctatt aatctgattc 928140
ttgtcagtaa gactggcaaa tgtctgagct aagggaagct ctttatatag atcagatacc 928200
gcaagttcta actgctggat agcagcttct tgagattctt ctggtacgcg ctttacagga 928260
gctgccttag actgaactgc cttttttgtt ttcttgggct ttttattcat gatttctcct 928320
tagaatctta ctaacgtttc tttccgcttt tcttatttgt agttgttttg ctttttcccg 928380
caggtttttt tgtcggtgct ttcttagttt ctccagccat ctgcttctca atagactgtt 928440
tcataatttg gcaacgttct tttaagattt tgaattctgg gttattccca cagatatcca 928500
tggtgacgtc taagaaattg ttagattctt cgggttgctg tagcttcaat aagctatcag 928560

caatgtagta aggaggaatt gggttgtcag gttgagcatc gaaagcaagg aaaaatccaa 928620
aagccgcttc attatataaa tgcaattggt ggtagcagga gcttaaccct aacatatact 928680
tgtagttctg aggttgtgct gctgccaata actggaagag tcctacagct tcgttgtact 928740
tcccttgaga atagaatgta taagctacag tatagatctc ctctaagaga tagtccgaga 928800
gacctagaat ctgttgtagg tctagtccat tgctaagacc ttcgaagata ttgcctaaag 928860
cttttttaat ttcttcttcc gtaggtacgg gatggacttg ttctaagtca tcagcctttg 928920
ctttcttttg agcagccagc tctgctagac ggttcgcgtt tttttattga aagaggctga 928980
aggtttttga ggttgattgg cattacgagg agagggcttg ctcatcgatc taattcctaa 929040
aataaaatta ttttgtattt atgaatcaat tttaaaatta atctttttct aaaaacaagc 929100
ctattgataa taatattttt attattttaa ttaatctttt tctaacccgt tcatttttaa 929160
ggaaaaatgg aaattagacg gaagctttct tgacattagc tttagggtat tttaatttgc 929220
tggctcggaa atttaacgaa ggcacctatg tctacaagaa ggcctattca gttacttgat 929280
cccctgacca tcaatcaaat tgctgctggt gaggtcattg aaaactccgt ttctgttgtt 929340
aaagaactga ttgagaattc cttagatgct ggcgccgatg aaatagaaat cgaaacttta 929400
ggagggggac aaggcgcaat cattatcaga gataatggtt gtggcttcag agccgaagac 929460
atccccattg ccctccaacg tcacgccact tcaaaaataa gagaattctc tgatattttt 929520
tctttaaata gctttggctt tcgaggcgag gctctaccct ccattgcctc gatttctaaa 929580
atggaaatac aatcttccat tgagggggac gagggtgtac gtaccgtaat tcatggggga 929640
gacatcgttt cttgtgagcc ctgtgctcgg caactaggaa ccacagtgat tgtgaactcc 929700
ctgttttata atgttcctgt gcgtcgtgga ttccaaaaga gcatgcaatc ggatcgctta 929760
gggattcgca agctgataga aaatcggatt ttatccacag caaacatagg gtggtcctgg 929820
attagcgagg gacatcatga aattcagatt gctaagcagc aaggatttca agaaagagtc 929880
gcctatgtga tgggagacca cttcatgcag gatgccctca ccatagataa agaagcaaat 929940
ggtgtccgta ttgtaggggt gttagggtct cccagcttcc accgtcccac acgtcaagga 930000
cagaaaatct ttattaacga tcgccctata gagtctttat ttatttctaa gaaggttggg 930060
gacgcctatg ccttgcttct gcctctacac aggtatcctg tttttgtgct gaagctctat 930120
cttccttcgt catggtgtga ttttaatgtc cacccacaaa aaatagaggc tcgaattctt 930180
aaggaagaac ttgttggaga ttgtatcaaa gaagctatcg tagagactct agcatgtcct 930240
cctggcatct tatgtcgtac gcatcaagaa atagaagaat ctgattcggt gcccttaccc 930300
atgtttcgta tgttggaaac aagcgatgtg caagaagaag agagtgtaga gtttgatcaa 930360
aatctttttg catatagttc agaagatgtt tccttagaga aacaagaata tacatctaga 930420
ggacctaagt cccaaatgga ttggatatat tctagcgacg ttcgtttttt aacttcttta 930480
ggtcgtgtgg tcctggctga ggatcttgag ggtgtgcaca ttatttttac agctgcagcg 930540
cgaaagcacc tgttttttct gtctttgatg caagagaatt ctcgcatgta tcaatcacaa 930600
gcattactga ttcctctacg ccttcaggtg actcctgagg aggctttttt cttctctcat 930660
cacggaagaa cgttatgcga cttaggaata gaaatatcac aggtaggacc ttgtgttttc 930720
tctattgaaa gtacccccac tgtcattggt gaagaagagc taaaagaatg gttattgcta 930780
ttggcagcaa ggggctctac tgatataaac tcagaggctt taacagcatt gatgaaagaa 930840
actttgacgc aggcaacgtt ttctaaacat cagcatgttt ttgatgtttc ctggctcaaa 930900
ttgctttgga gtgtagggaa acctgaaaaa ggatttgacg gtgcacgaat tcgtcggtta 930960
attttagact ctgattttat ggaaggataa tcatatgtca cacgatcgta ttttacgtgc 931020
tcaaagagcc ctctcagaac ataatcttga tgctattctt gtggaaaaaa gcgaagatct 931080
tgcttatttc ctgcatgatg aagcgattgc agggatctta ttgatagggc agcaagaagt 931140
gatgttcttt gtctacagaa tggataagga cctctattct catatccaac gtgtgccttt 931200
gacttttctc actcaggatg ttgttgcaga cttatcgctc tacgtacaaa aacagaggta 931260
tcagaaaata ggatttgata gtgcctcaac agtgtatcac aagtttgcac agaggcaagt 931320
acttccctgt ctttgggagc ctttagagtg cttcacagag aaaattcgta gtataaaatc 931380
tgaagaagaa attagacgca tgcaagaagc tgcagctttg ggatccgcag gatatgatta 931440
cgtattgacg ttacttcgag agggaatcac agagaaagag gtcgtgagac agctgcgagc 931500
tttctgggct gaggcaggag ccgaaggacc ttcttttcct cccattattg cttttggaga 931560
gcattcagcg tttccacact cgatccctac agaccgtcct ttaaagaaag gagatattgt 931620
tcttattgat attggagttc ttctgaacgg gtattgttct gatatgaccc ggatgacggc 931680
attaggaact ccgcatccta agcttttgga aagctatcct gtggttgtgg aagctcaaaa 931740
gcgcgccatg gctctttgca aagaaggagt gctttgggga gacattgatg cagaagctgt 931800
gcgtgtactg cgagagcatc acctggatac ttattttatc catggaatag gacacggggt 931860
ggggagacat attcatgagt acccttgttc tccgcgggga agtcaggtga aactggaatc 931920
tggcatgacc attactgtgg agccaggggt ctattttcct gggattggtg ggattcgcat 931980
cgaggacacc ctatgtatag ataaaaataa aaattttagt ttgactgcac gtcctgtaat 932040
ctcagagtta gtttgtttat aaattaaatt ggatttagtt tttaaattta aattgaattt 932100
aatttgtttt tataaattga ttttttttgt tttttaagtt atcttataac tttattttta 932160
acctgcccct cactatgtac cagcttcttt cgataggtta tagttttgtg agtttcatcg 932220
ctctgctttg gatgctgtgt tattcaccga actatgtaac agatttatat aggatttctt 932280
tgagtgccga ggaaagctta ggggggattc gagcgtttcc tcaggcagag agcctcctgg 932340
gcggagcctg tgctttaaat tttccagatc tagaagagag attgcctgat ttaaggaaag 932400

agctgctttt tctgggcagt aacgatagac cagacgcttg tggtgggaag ttttcgctac 932460
aactagcctc ttcaaaagag tgctacatcg cggctcttaa ggagagagtc tatttgaacg 932520
tcaccaactc ttctcgaggc cctgtgtatt cattcagccc taaaggggta cccacagagt 932580
tgtggattga gtgcttttct gtgagcgtgg atggtagagt agaagttaag gtgcgcctcc 932640
aaggtttaca taaggagtta atttcgaagc cgcgagattg tgaaacctta tttttaaacc 932700
ctccagctaa taaactagat tgctgggaga ttgcgggatt tagagtagat gcaagctttc 932760
ctgtaaaaca aaagatacgt cgtatcggtg tggataagtt tctcttgatg catgggggag 932820
ctgagtacgc ggataaggcg acaaaagaac gtgtcgattt tgtttcctct gatgaggaga 932880
attatagtcg gtaccttgct gttggagatg ttctcctttg ggatggcaac tgctggcaga 932940
cctgcggaga gtttcaagga gcgagctcgc gagcgcctct ttttgaggtg aagaggatcg 933000
acgataaggt catgattgcg gatctatgga atgtcggggg tacgcaacgt cagacgataa 933060
gtcttgtgaa aggggtgcct tctcctatcg aaattaacga agtgatacgt gaaatcgagt 933120
tcactgggat gcgctcatgg tcaaagccta tcgttttggt agggggacaa aggctgattc 933180
tttctcccga cgattggata ctaagaactg ctaagggttg ggagaaactt tcaagggcag 933240
accagattca agattacgtt acaggaaagg taacaggacc tcttttggtc tttgaaaagt 933300
tagaaaagga tcttcgaggg tttgtcttgc gagggcatat gtttaatgca cagcgaactc 933360
tcgtagagac aatcagttta ccgttaaaac aaggatttga gcctgctgtg gcttctcaag 933420
aagtgtcttc aaacacacgt agcgcacagc acatccaggg gcgaccaatc gtgggggatc 933480
atagatggtt tttttccgta attctttact gcatttagtt gccctatccg gaatgctctg 933540
ttgttcttct ggagtggctt taacgatagc cgagaagatg gcttctttag agcactcggg 933600
gagaggagca gacgattatg aggggatggc ttcgtttaat gccaatatga gggagtatag 933660
ccttcagctg agcaagttgt atgaggaagc acgaaagcta cgcgcttctg gaactgagga 933720
tgaagctctg tggaaggact taattcgacg gattggtgag gtgcgaggct atcttcgaga 933780
gatcgaggag ctttgggctg cagaaattcg tgagaaaggg ggcaatctcg aggactacgc 933840
cctctggaat cacccagaga ctacgattta caatcttgtt accgattacg gaaccgaaga 933900
ctctatttat ttgattcctc aagaaatcgg agcgattaaa atcgcaacct tatcgaaatt 933960
tgtagttcct aaagagtctt tcgaagactg tctcactcag atcctatctc gcttaggtat 934020
tggcgtgcgt caggtcaatt cttggattaa ggaactttat atgatgcgta aggagggctg 934080
cagtgttgct ggagtttttt cctccagaaa agatttagag gcgctcccag aaacagccta 934140
tattggtttt gtattgaatt cgaacgtaga tgcgcatacc aatcaacatg tcttaaaaaa 934200
gttcattaac cctgaaacaa cgcatgtaga tgtgattgca ggacgtgtgt ggatttttgg 934260
ttctgcgggg gaagtcggcg agcttctgaa gatttataat tttgtgcagt cggagagcat 934320
acgtcaagag tatcgggtga ttcccttaac taagatcgat ccaggggaga tgatttccat 934380
tctcaacgca gcatttcgtg aggatctgac taaagatgtt agtgaagaat ctttaggcct 934440
tcgtgtagtt cctttacagt atcaagggcg ttcgttgttt ttaagtggaa ccgcggcgtt 934500
agtgcagcaa gcgctgactc tcattcgaga gcttgaagaa gggattgaga accctacgga 934560
taaaacagta ttttggtata acgtcaagca ctccgatccc caagagttgg cggcattgct 934620
ttcccaagtc catgatgtct tctctggcga gaataaggcg agtgtcggag ctgcagatgg 934680
atgtgggtcg caattaaatg cctcgatcca aattgatact acagtaagtt cttctgcgaa 934740
agatggctca gtgaagtacg gaaacttcat cgcggattct aagacaggaa ctctgattat 934800
ggtggttgag aaagaagttc ttccacgtat tcagatgcta cttaagaaac tagatgtccc 934860
taaaaagatg gtccgtatcg aggtgctgtt atttgaaaga aaattggcac atgagcagaa 934920
atctgggtta aatcttctac gtcttggtga ggaagtttgt aaaaaagggt gcagtccttc 934980
tgtgtcttgg gccgggggta ctggcatact agaattttta tttaaaggaa gtacgggatc 935040
ttcgatagtt cctggttatg atctcgccta tcaattttta atggctcaag aggacgttcg 935100
gattaatgcg agtccttctg tagttactat gaaccaaacc ccagcacgga ttgctgttgt 935160
tgatgaaatg tcaatagcgg tgtcttcaga taaagataaa gcgcaataca atcgtgcgca 935220
gtacggtatc atgataaaaa tgctccccgt aattaatgtg ggagaggaag acggaaaaag 935280
ttacattact ttagagacag acatcacctt tgatactacg ggaaaaaatc atgatgatcg 935340
tcctgatgtt acaaggcgta atattactaa taaggtgcgc attgctgacg gagagactgt 935400
gattattgga ggtttgcgtt gcaaacagat gtcagattct catgatggca ttcctttcct 935460
tggagacatt cctggtatag ggaagttatt tggaatgagt tccacatcag acagtctcac 935520
ggagatgttt gtatttatca ctccgaagat cctagaaaat cctgtagagc aacaagaacg 935580
taaagaagaa gctttactct cttcgcgccc tggagagaga gaagaatact atcaggcttt 935640
agcagctagt gaggctgcag cacgagcagc tcataaaaaa ttagagatgt tcccggcatc 935700
aggagtatct ttatctcagg tagaggcaa agaatacgat ggctgctagt attttatctc 935760
aggagctttt ggatatcctt ccttatactt ttttaaagaa acactgtctt ctccctattg 935820
aagagagtag tgaggctatt actatagccc atgctaccgc gacttcagtc attgctcaag 935880
atgaagtcaa attgttaata aaaaagcctg tgcgtttcgt tctaaaagag gaatcggaga 935940
ttctgcagcg cttacagcag ctctacagca atcgggaagg taatgtttcc gatatgttgt 936000
taacaatgaa agaggaagat ggcactacga tttcggaaga agaagatctt ctggagacta 936060
cggatacgat cccagtcgta cgcttgttga actggattct gaaagaagcg attgaagagc 936120
gcgcttcgga cattcatttt gagccttgtg aggattctat gagaatccgc taccgcattg 936180
atggtgtgct tcacgatcgt cattccccac cttcccacct gcgttcggca ttaaccactc 936240

ggcttaaagt cctcgcaaag atggatattg cggagcaccg tcttcctcaa gacgggcgta 936300
ttaagatcca tattggtggt caggaagtgg acatgcgtgt cagcacggtt cccgtgattt 936360
atggcgagcg tgttgttctt cgtattttag ataagcgcaa tgtcattttg gatatcgcgg 936420
gcttgcatat gcctaagggt accgaaatac tctttaaaga taccataaca gctcctgaag 936480
ggatccttct ggttacagga cctacaggca gtgggaaaac tacgaccctc tacagtgtat 936540
tacaagagct taagggacct ttaacaaata tcatgacgat cgaagatcct ccagagtata 936600
aactgcctgg aattgctcag attgctgtga agcctaaaat tgggctgact ttcgcacgag 936660
ggttacggca tttactgcgt caagatcccg atatccttat ggtcggagaa atccgagatc 936720
aggaaactgc agaaatcgca atacaagcag cattgactgg gcatttggta gtgagcacgc 936780
tccatacgaa tgacgctatt tctgcgattc cccgtcttct ggatatgggg atagaatcct 936840
atttgttatc ggcaacgctc gttggcgtgg ttgcccagag gctggtgcga accatttgtc 936900
cctattgtaa ggtcgcttat actcctgaga atcaggaaaa atcttttcta gcttctctag 936960
ggaaagatac agaaatgcct ttatatcggg ggcaagggtg cgtgcattgt ttcgttccgg 937020
atataaagga agacagggaa tttacgaatt tttacgcccg aatacactat ttcgttcaga 937080
agtagcctca aaccgcccct atcatatttt acgagaaact gcagaacaaa acggattctt 937140
accgatctta gagcacggca tcgctcttgc tgtatctggt gagactacct tagcagaagt 937200
cttaagagtt accaagcgct gtgattaggg agggcggatg cctcgatatc ggtatacata 937260
tttagatccc aaagagcgaa ggaaacgagg atatttggaa gcccttcata tacaagaagc 937320
tagagaaaag ctcgcccagg aaaatatcca agttttggat attcgtgagg tcgccttacg 937380
aagaatgagc attaaaagta ccgagctcat cgtgtttaca aaacagctcc tcctcctcct 937440
acgctctgga ctgccgctat atgaaagctt ggtatctctc cgagatcagt atcatgagca 937500
gaaaatggga cttttgctca catcgtttat ggaaactcta agatcgggtg ggtctttatc 937560
tcaagctatg gcagcacatc cgaatatctt tgatcacttt tattgtagtg gtgtcgctgc 937620
tggagaaagt gtggggaatc tcgaagggtg tctgcaaaat attattgttg ttctggaaga 937680
gcgtgcgcag attaccaaga agatggtcgg cgcattaagt tatccttgtg tgttgttagt 937740
attttctttt gccgtgatgc ttttcttttt gttaggagtg atcccttcat taaaagagac 937800
ctttgaaaat atggaagtca aaggactaac aaaaattgtt tttggagtta gcgactgtct 937860
ctccgcatac cggtatctat ttttaggatt tgcgagtgct ttgattaccg ttggaatttt 937920
gatgcgccat cgcattccct ggaaaaagat cctagagaag ctcttatttg ctttgccagg 937980
aaccaagaag tttgttgtta aggtagcggt gaatcggttt tgttccgtgg catcggcaat 938040
cttgaaggga ggggggaccc taatcgaagg tctcgacttg gggtgtgacg caattcccta 938100
tgacagactg aagaccgata tgagagatat tgttcaggct gtaatcggtg ggggatcttt 938160
aagtcaggag cttgctcagc gctcttgggt tcccaagctc gctataggga tgattgcttt 938220
gggagaagag tcgggggatc ttgccgacgt tttaggatat gtagcccaca tttataatga 938280
ggatacacaa aaaacgttgg cttcgataac gtcgtggtgt caacccgtga ttctgatttt 938340
tcttggtggc ctgatcggtg tgatcatgtt ggcaatattg atcccactca caagcaatat 938400
ccaaacatta taaagtgtgt actcagagga gtcggtatga aaagacaaaa gagaaagcag 938460
tccatcacat tgattgagat gatggttgta atcaccctca tagggattat tggtggtgct 938520
ttagcattca atatgcgagg cagtatccat aaaggtaagg tatttcaatc tgagcaaaat 938580
tgtgcgaaag tatacgacat cttgatgatg gagtatgcca cggggggatc ttcgttaaag 938640
gaaatcattg ctcataagga gacagttgtc gaagaggctt cttggtgtaa agagggtagg 938700
aaattactta aagacgcttg gggagaagat ctgattgtgc aacttaatga taagggtgat 938760
gatttagtca tcttctctaa gcgtgtacaa agttcaaata agaagtaact cttgagtaac 938820
atcatggggt ctcgacgtaa acttaaacgt agctttttac ttatagaagt cctgatggcg 938880
ctttctttgg tttgtgcagt gctcttgccc tgcatcagat tttactacgc catccacagg 938940
tcttttgagg aagatatttt taatttgcaa ttgccagccc tgatcgacca ctgctttcta 939000
tctgtngaag aaaagatgcg tcagcaaatg gcagaaggaa ctgttctcac ntcagggaaa 939060
gggcagacag tttctttagc atataccagt caggggatag gctatcggat cccttatggt 939120
tacaatgtag atatccgtca ggaagtccgt ggtgataatc ttaagatgaa agtttgcctt 939180
gccgatgttg ttgtggaact tttcccagat cagaaacaag cagtatccgt acagagatgc 939240
ctatgtgtaa ctctatagct atgaaaaagc aaaagcgtgg ctttgtgctt atggaattac 939300
tcatgtcgtt cactctaatt gctttgttat tagggacttt aggattttgg tatcggaaaa 939360
tttatactgt acaaaagcaa aaagaacgta tttataactt ttatatcgaa gaaagccgag 939420
cctacaagca gctcagaacc ctgtttagca tgtccttgtc ttcatcttac gaggagcctg 939480
gatcattatt ttctttaatc tttgatcggg gtgtttatcg agatcctaag ctggcaggtg 939540
cggtacgagc ttctctccat catgacacca aggatcagag attggaactt cgtatttgta 939600
atattaagga tcagtcttac tttgaaacac agcgactgct ctcccacgtg acccatgttg 939660
tactttcctt ccagagaaat cctgatcctg aaaaacttcc tgaaacaatt gctttaacta 939720
taacacggga acctaaagca tatcctccaa ggacgttaac ataccaattt gcggttggga 939780
aataagccta tgcaaccttt tatctttact ttactgtgct tgacatcttt ggtttcttta 939840
gtcgcctttg atgctgcgaa tgctcgtaaa cgttgtgcct gtgctcaaac tatagaacgt 939900
ggagagaact tcttttccat aaaacgctct gcttgtgctg aaatcgaata tcaagaaaaa 939960
tctcgccacg cctcagcaat tgaaagaatc tcaaaagata aaggcaaagt cactccaaag 940020
cagattgcga aagtagctac taagaaaaag caaagatacc gtttattgca ggttcctttt 940080

tcaaggcctc cgaataactc aaggtataac ctctatgctt tgcttagtga acctcccgaa 940140
tgctatagcg atacagcatc atggtatgct atttttattc ggttacttcg acgtgcttat 940200
gtagacacgg gaaatgtacc tcctggatct gagtatgcca tcgctaatgc tttgataagt 940260
aacaaacaag agattttaga gaggggagcg cagcttggac ccgatgttat tgaaactcta 940320
acattgcctg aggaacaagc cgagattttt tataaaatgc tcaaagggtc gtcaaactct 940380
cagtcgctac tgaattttct gcattatgaa gagaaaagct taggccactg taagctaaat 940440
ctgatcttca tggatcccct actgttagaa gctgttctag atcatcccga tgcttatagg 940500
gaaacgtcgc tcctgcgcga tggcatttgg gaagcggtga agcgtcaaga acatgccatc 940560
caagaacatg gccaggcagc tgctttggag ctttttaaaa cacgcaccga cttccgcctg 940620
gagctgcgag ataagatgca gttacttcta agtcgatacg atttgctccc cttattaaat 940680
aaaaaaatgt tcgactacac cttaggaagt gccggagatt acttattttt ggtagaccca 940740
gatactaagg caatttctcg atgtcgctgc ccttcaaaga gtattaaatt ataatttaat 940800
tttaatattt attttaaata gttttttttg ataattgtct taataagtac tataaaaaat 940860
atttctatag gtaggaccat ggcagacgag accccgaaag agaactcctc caaagaatcg 940920
tcctcacaat ttgactcttt gaagcgtaag gtgaaagatt tacactccaa tcctaaagta 940980
gggaaatgga agaagtttct ttctcatcga gcttgcgaan tatcggtggt tgcttggtgc 941040
tggttggtat catcgctgat tttatttcat gggctggagg actgtttatt gcttgtggtg 941100
tggtcctagg ttttcacgtt gaaattcgta aaatgcttag caatctccag agctattcga 941160
ttgctaatgg ccctattaag aatgcaattc tctgtggctt gattttattt tttgtattaa 941220
acatcccttc ctttgcagtc tcgtttattg ttctctgtgt cattctttct tttattacaa 941280
cagcaccgtc atgttcgacg tgttcgaaag atcattgtga caaacatcaa gatacttcta 941340
ataaaccttc ttaaaactac tttttcccta aaggcaaaat gttgaagccc tcctttcctc 941400
aactttttgc cttgttttca ggcatgttcg tcgagagctc acaggctgga acgtatctca 941460
tgttttaaga gatgcatcct tttctatagt cttgaattta tagattctta gattacaaaa 941520
gaatatgcga gagattccct aagataatcc ataaacagtg cgctaagatt cgtaagtgag 941580
cgcatctcca gataagagtc acgataagag cctaggggat tagagtactt gagggttgga 941640
acctaggagc ataatgggga cttctttgaa ccaagcaaga gtgaaataat ctatctgctt 941700
aattatgaac caccacgcca gggtgagaaa gagaagaccc atgaaagcct taagggcaga 941760
gaggaggtag atgacctgaa cttgaggtgc catacggtta ataatcccta agaataggtc 941820
ggacattaac atcgccaaag ctgcaggagc actcagctgt atggtcatca cgagacagag 941880
ctggcacatc ttgatcatag taatccaaat cggggcactt aagctcatca tctcggcagg 941940
aaagaaacta tggatcggaa tgacttcaag agtttgcaat aacaaagaga ttacaatacg 942000
gtgaccaccc actaaccaaa aaataatagt cacgaagtaa tggtataaaa tgccatgcgg 942060
agaggtctgc tcaatggaaa tcagggatgt cgcgccctct aaaccctgaa tcccttgttg 942120
gttagtgatg aaagatcctg ccgattgtgc agcataaaag ggaaatgcta aaacaaagcc 942180
tatcacaatg cctatgatca tctccttcac aagtaaaaca taaaagagat tgttatccat 942240
gtaatttgtg atctgcgtat ccgccaagac ttttggaaag atgattgcaa gccaagagag 942300
actaatcccg attttaatag gggagggaaa gagctttgct cctaagaagg gagctacagc 942360
aaaaatagga agcagacggg ctaataaaag aagaaaaact gaccaaacat aggccggagg 942420
atgttgaaag atataatcta agtaagcaga acctaggttg gaaaaaagct ctggtagaga 942480
gattcccata agctctttat ttccatttat agaagttttg gaaaatctga cctgcaaagc 942540
gtaaaatcat attgctaagc caccctccag agatcattaa ggttccaaaa atcacgacta 942600
gtttgactgc aaaagcgaag gtctgttctt ggatttgtgt tgcggcttgg aagatcgcaa 942660
ccataatccc gactatggaa gctaagatga tgggaggtgc cgaaacaatc aaaataagta 942720
ataatgattg gtaggagtac tcaaaaagaa cagatttgaa actagttgcg aaaaatgcta 942780
acacggcacg tgtccttatt taaagctgat cataagccct tggagcagta atgtccatcc 942840
gtctaccatg acgatcaaaa gtaactttaa aggtaacgaa atcgatagag gggataacat 942900
catcatctgc atcgctacaa gaacgttagc agtcactaaa tcaataacaa agaaaggtag 942960
atagatcaag actccaattt cgaaagcatt ttttatctga cccataataa aagcaggaat 943020
aatgattaca aagtcggagg cagtgaggtg cgctcgaatt tccgaaggaa ggttttctgt 943080
gagatcttgt aaaagctttg aatttgtgct tttggagtgt tgcgaattaa gaaagagcgc 943140
aaaggttctt tagatttgtt taaagcgaca aacactgttt cagcaccttc tgcagtgaag 943200
aggctttgag gaatggtatt ggcttcgatt tccttgcgag catctttata catagccact 943260
cccgtgggga acatcacata aatagatagg atgagtgcaa tcccattgag gacttgactg 943320
ggaggtgttt gttgtactcc taaggcgtta cgtaataaga ctaaagtaat gataatcttt 943380
agatacgaag tgagcaacat gaccaggaaa ggcgataggg ccaagaaaat taagatgatc 943440
gcttgcgttg taatgtctgg atacgtatct gagaaacttc catctgacag atggtctcta 943500
gggagaacat catcagcatt cagtgggggc atgtaggaag gcactgacga tgcagcaaca 943560
ggctgttgta ctacgttcaa aggattagaa ttcgagggag gaggggtagg ttgacaacgc 943620
gagggacagg agttttcata tagactggcg tcagaaaaca gcatgagacg ctcagcgtaa 943680
aaaaacaaag ggaaaatcga aaaatggatc gcataatagt gaataccttt attctttctt 943740
atcctgatca ttagataaag aagaagaatc cgtggatgat gaagtctcag atggctcgtc 943800
tacagggttc ttcgctttta gtatagtcga gaaagctttt tctaaggcat ctaattgtac 943860
atcaagctgc gcattgatga tccctgcttc agtctcgata atgcaacccc caggagtaac 943920

atcaggtttt gctgtaagaa ttaaggagtc agcatactcc acgatgttct tgagttcagg 943980
acgacttttc tcaacaagag gtaaatcttt gggattgaca gagagtaatg atatgtttat 944040
tttgtgtgag ctctttcaat gcttgagaaa taatagagac aatagtttca ggatgtaatt 944100
cgagttcctt cccaatgatt ttcctcacac tcgcaattgc cagaggaacc aaggcctcgc 944160
gtactcttat gcgtagattt ttagtttctt cttctaagaa agcaatttgc ttgctccagg 944220
attcagatcc ctctttaaat ccttgatctt tagcttcttg acgaatttgt gcacactttt 944280
gttctgtctc tgcaacatag gcttcgctat cggcttttgt tttttctaac agctctttgg 944340
catcaaggaa agcagacgaa agcttcagga gataaaacct tcttatttgg ggagacatca 944400
tcatctttaa aaattaagct aaaaaacttc atctttacta tggcgtagta tctttgaggc 944460
tgatttacct cagaagagaa aacaccgctc cttttattgt cgtgttattt ttctatactt 944520
tgtataaagc taaatcttta cttataaagc ttttatttca ctaagacttt catgcattgt 944580
tctaggcggg acttaaaata atccacatag ggatggtcat accatgtttt taaagtctgc 944640
tcgacgatat atgctcgacc gacatctaac ctacgtagaa aataccatag aaaagaagcg 944700
ttttcttttg ttaatgcttt acctaaaaac tctaaccctt gcttatgaac gaactgtcgt 944760
aattctgcat cagtagtcca agaagaaaga aaattcgtag tttctaaatg tttcatcgga 944820
tgagattggc agtaggtaag aaagagtttc tctgtagggg agagagcatt cttcactcgt 944880
tcaatcacaa ccttatccag aatgtgcttc aactctttag caatagaata aagacctagg 944940
cagttgatta aagcgatctt tacaggacct gtatagtaaa gtatagcatt tgctgaggat 945000
gcaggaagaa agatttcttc tgtaattcca caaggacgga tctttttact tagcatatct 945060
agaagataga aggctccgaa aggtgcacag cgatgtgggg ctatagagat gccaggcagt 945120
aagggtagaa tttcttgaac taaaggctct ggcaaccacg ctaataattg accttggatt 945180
tcaggaggga actctttcat ggcaatggta atccatgaag gatgaattgt aggtagccaa 945240
ctcatcgtaa aagataaaga ttttaaaggg atttcttcgg gatgaggaga ttcaacaaga 945300
agatttttag gaagaaacct ggagaggtca tcttccttgg agtgcttcat caagatgtct 945360
agagttccaa aagtgttggc agtcactaag caccctcaat ttcattgctt ccttctggag 945420
catctttatc actagagtct ttatcactgg tttccgcatt tttgctttcc ccctgtgaat 945480
ctgcatcttc ttttttctct ttgtcagcag ctgctccctc ggctttcttg gcttccaagg 945540
cattctttgt atatggtgta gggttgaaga acccttttgt acctcccata gtcataatga 945600
gagtatgagt tttccaaatg acccaaagga gaccacaaga aataacaaat aaaatgagaa 945660
tcaagacata aaaaatgaga cggaatttgg tgagcgaaga cttcgcaaga ataatacccc 945720
aaacagaaac ataatcgatt tcttctgtta atccccaagg accattaatt gtaatatcac 945780
tataantgcg cgatcgctca ctacagagac gttctctggc acaagtcctg gaacagcact 945840
tgcaataagg cgcttaattt tggaaaccat aatgctgttc ggattgtcca aaacccctcg 945900
atgcttaata tacacagagg ctgttaaagg aagattatct tcattttctg tagtgaagga 945960
aatctgtact gaggcatcga caacgccatc catttttcta atcgtagagg ccatctgttc 946020
tgataagcct tcttgataac ggattttttc ctgaagctcg gaaggaacaa gaccttgttt 946080
tgcaaaaaga tctaacaggc ttgtcccttt catacgtgga agacccgctt gatttagaat 946140
ggcaagggcc tctgtgattt gtgctgacgg aaccgcgata tcccacattt gctcagtagc 946200
tgctccggct gtagccgctg cagcttgagg caatttttgt gcagccaccc ctttgcttac 946260
caaaagcacc acaatctcat tcgcttctct gccaggaaga ccgtgcacaa ttagagacct 946320
gctgttacag cttgtacagc acagcaatgt cattagaaag aacaagcaaa aagaaataga 946380
tcgacgaacc ataatccacg catacctttt tattcaccat aacaaaaggg gggattagtg 946440
ccaatcgcga acataaaatg gtaatccttg tcttcttttt cttcaagcca cgatattaaa 946500
aaatgatttc atacatacct ctcggcatgg tgttccaatt atgaaaggga tgtttttttg 946560
aaggtaagat aaatgctctc tacctaatgt gatatttttc agagttttct ttcctatcgt 946620
tttatgaagc tctctacctt tgttgcaata agagtaattc acaaactccc ttacttaagt 946680
cttaggcaaa ctttttggtt tgagaatccg tgttcatttt aagcatcgtt gttgcggtac 946740
ctacgttacc tttcgagttc tttgatggta taagagttag gggtaggacg ttatattttg 946800
gatagtcact cggaccattg tggtttccct tgcctctatt ttgagataga aatacatatg 946860
tttcgtgcca actttggcga aatcctttta tataaatata agagattcaa ggttttattc 946920
atggctttgc tttataatga aggaaacgta ctaccgcgac tttcttcatg agaactattt 946980
gaaaaacaag aagagcatgt ttatgaaaat ttataaaact gcaggggagt tttttttagc 947040
aaatgcaaaa tggcccttgg taccggctgg gtatcgacgt gttcgaggaa aagattttgt 947100
tctatccccg ctcgtggact tagtgattct atttccttgg gtaaccaaag actcccgata 947160
ttcaccttgc agcatgacat tcacttgtat ttgtaggagt atagtagagt gtattcctgt 947220
tgtaagtaca ttatttggta tcggacgatt ttgtgctgtg tggtgcgttg aaggtttctc 947280
aggctctacg tttgataaga tctatcatac aattgtcgcc gttctaggaa ttcttggttt 947340
gggaattctt acgttcattt taagaattat tttttctgtg cttatgttgc ccgtctggtt 947400
cttatttaag tgttattctt agcgtacaag atggcgggac tgcagatcat agccactagg 947460
attcttgaca gttttctcct cccctgcttc gaggtagaag cccagacatt tcctcaagtc 947520
tttagcaaag ttgttgtata taagtacaag agttctagaa ttttattgat agctttgctt 947580
tataatataa ctctcgtctt aggcttattt tttattcata agaaatactt aggacaaaag 947640
gggagggtaa ttctgaaaat ttatcaaaat gaagaagaat tttttcgagc aactgaaagg 947700
tttccatcaa taggggcggg gtatctacgt gttcgaaaca aaaactctgt attatttcca 947760

tttgaggatt taatgcttgt atgcccctca gtacctaaag actttccact ttcagctttc 947820
aaagtaacaa ctaagcttat ctattggagt gtattagaga gtatccccgt cgtgggagca 947880
ttttttttca gtataggaag actctttgct atgtggtgca tagaagattt cccaggctct 947940
attttttcta gaatctatca taccactgtt ggtgttttag gaattcttgg tttaggaatc 948000
atcatgttca ttttaagaat tatctttact ttgcttacgc tacccttctg gctcataagt 948060
tgtctaaaat caagtgctgc ttgaccaaga tcgagtcccg gtgattgaag acggtttccg 948120
atagccacta ggatcactga ggttccttcc tctctgagat aaaaactcat atttttcgtg 948180
taaagactaa caaatcagtt ttataggaat gcaaagattg tataaagatc gagcatcttg 948240
gtcgctctac tctacagtga agcacgtgtt ttatcttgac tgttagaaaa gaaaatattt 948300
ccagaattga ctcttttaca cgtagttgaa cttagttcta ggggatctag gttttattta 948360
tatttaataa aacaaaagga aaaataatgt gcttataaga aaatctgaat cagaaggagc 948420
tttttttgaa gcaactcaaa attatcctac aatacagcaa ggatatcagc tggtacggat 948480
tcgtgagcac aatctttctg tccgagcaca ttttgactta tctctatctc ttgatgcatc 948540
agtccatccc gcggcttaat agatagggta ttgaattcta gatgcttatt tctattttta 948600
aagagtagat ttctttcggt gtcttgtcta aatggaggct atcaggcgta tttatcaact 948660
cttcttttga gtgggccggc tttggatagt tatttgtaag cgtatctaaa attgtaatta 948720
tttatagcgt acggaattgt ttgttggagg ggctaagact acggagcgca gttttaagaa 948780
ttttcctaac aagtttcttc atcttggtat catcggttag tgtcttgtct ggatgagaag 948840
atcgcaagga tgacagtttg aaaaagcgtt agaatgtagt ttttaagatg tgaatccatg 948900
atctttcaat atcaagattc gatgagatgg atcgtactat gcttgttaac tgacgctttg 948960
tcttgtacag aagctaggat catatcggca ttaaaactcg agcgtacaaa aggtcctgcg 949020
taaacaaaaa gacccatagc ttctccgact cgacgatagt aatcaaaagt ctcgggagtc 949080
acataacttt tgacttggag atgcttacga gaaggacgta agtattgacc tatagtgaca 949140
atacgaactc ctatggaagc cagatcttgg agagtttgtt tgacttctcc ttccatctca 949200
cctaatccta ccatgatccc ggatttgatt ttaagatcgg ggaggtagtt ggcagcttgt 949260
tctaacatga acatagatcg ggcataggtt gctttgtgcc gtactagagg cgagagcctc 949320
gctacagttt cgacattatg attataaata gtaatgccag aatctaacag ggtgtggaga 949380
gcagaaacat tcccttggaa atcagaagct aaaacttctg tagtcgcttg agggagttct 949440
tcacgtaact tttgaatgat atcgactaaa ccttgtgcac caccatcctc aaggtcatcg 949500
cgagccacca tggtgattac cacatgcttc aaccccagtt ctttcgctga caaagcaatg 949560
cgctcgggtt ctgtaggatc tagcgcgggt ggggttttag aatgcccaat attgcaaaaa 949620
ccgcaacttc ttgtacagac atcgccgaga gcaaggtagg tggcagtttt acgagaccaa 949680
cattctgctc gattggggca gagagcttct tcgcataccg ttggcattcc tgagcgtttt 949740
attgtagcgt ccgtagcatg aaatgcagaa ccttgtggta agggcctctg taaccacttg 949800
gggaaacgct cgggaagctt cttccgcact ctaggttggt ctgtatttaa agttggtcta 949860
catttcatga cttaggaggg aagtgtaaag ggtgatttgt agcaagtaaa gcaccttcag 949920
cccaaacttc agagagtgtg ggatgagcat gcacggtttc atatatgcaa ggtagggtca 949980
gctcattgcg gatcgctaag gtcatctctc caattaatga tgaggcgtga ggtcctatga 950040
cataagctcc gagtatttgc tgggtaattt catgactcac aatagcagca aaaccatcag 950100
atgctcccaa agcaacagct tttccaatcg ctttaaaagg aaatttggtg agctttgcag 950160
gaagattttg ttgttctgct tcttgtagag atagacctac catagcaatt tctgggtggg 950220
taaagatcac agaaggtatg gcagaataat ccataacttc gtgatgtccc gaaatatttt 950280
tcgcggcaat aacgccttgg tgcgaagcca catgagcaag tagccacttt ccagtgatgt 950340
ctccaatcgc atagatattt ggaacattag tgcgcatggt ttcgtcaaca ggaatcacgc 950400
cacgatcgtc ccggatcact ccagcattat ctagccctat acttgctgta ttaaattggc 950460
gaccaatagc caccaagaca taatcaaact cttccacttg atcgttcaca gtaatgcgaa 950520
cttggttttg ggattcttcg attgcagaga tcgaggcttt ggtaagaatt cgaattcctt 950580
gtttcgtaaa tttattcgtt acggtttgag aaacttcttt attgttaacc gcaagaatat 950640
gatccaaagc ttctataacg gtaatctcaa cgcctaaagt gtgaaataga gacgcaaatt 950700
cacagccaat aacgccgcca ccaataatag cgagcttttt agggaggact tcaagttcta 950760
agatccctgt ggaactcaaa attctagagg agaagggaac ccctgggaaa ggacgaggct 950820
cggatcctgt agctaggata atatgattgg ctttgattat agtcgtgtct tggccaataa 950880
ctttaacttc tgtagaagat actagagatc cggttccttt taagacagta atcttgttgc 950940
tgcggatcaa tccttctaat ccttgacgga tcccctggac gactgtattt tttctttttg 951000
ccatcgcagg gtaatcgatt gtataaccat caacatggat gccgaactct ccgcatgctt 951060
aatgtgagat acaacattgg ctccagcaat gagggctttt gaagggatgc atccgcggtt 951120
taagcaggtc cccccagcct ggtcttcttc aataagagcg gtccgtaatt ttgattgcgc 951180
agcagtgatt gcggcaacat agccactagg tcccgcacca ataacaacac aatcaaattc 951240
ttgggtcata ttctcactca ctgtaatcaa gatttcaaaa agaacccccc ttcataaatg 951300
catgcatctc attaagaaga ggaccctgtg cttattttag acctaaagat taattttcag 951360
cattgctttt ttcttcgctg attctgtatt gaacctcgcc tatatatccc agaaaaagca 951420
aacgttccct cggtaaatat tgttttcttt tttaataccc agaagtatac ttgcattaat 951480
ggcattttag cgacctaaga ggtcgtgatt tcataattga caagaacgta ttcgttataa 951540
acaagcatgt ttaaatggat gagtttatgg gggaaatgcc ataacttaac cctacagagg 951600

gcatagttgg atagcaattg acgaggaaga atgtatttct cctcatgatc atgaaaaaag 951660
tgaaagatac ctatgccatt tgctaaagag acagaaatgc aaaggacgtg ttggaagtgt 951720
gaaggcagtg tatctatgca cgtgcctcaa tgtccctatt gcagcgcctt tcttcaagat 951780
cctccagtag cctcaggagg gttttcttct tgccacatct cgttcccaga aggagcttct 951840
aaagaagaag ccgaagacct atttgccgtc tcttcagaag attgggaagc cgtgcttgga 951900
gatcaaaacc ctactcaaga aacgaataaa caagtgatcc ctgagtggac atggttacaa 951960
agttggcctc ttgcagcctt atttttaggc ataggcttgc tagcgtttgc ctttctgatt 952020
cttcttttct ctacagacag tggattggtt ttgacttggc ctaaaaatcg ggcctatttt 952080
tacggtatta taggtgctgc agtcgcctat cgtggatacc gtaaacttcc tctttaatta 952140
gcactaaata caatttcttc gttaggaatc ctcaggggga agcacacctg tatcatcttc 952200
tacgggtgca tctacagggg actcttcact gtctgaagtt ccatgttcgt ctttgtagga 952260
tagtatagtg aggagatctt cccgattcaa catgtgtagg atattagaat cttgagaggc 952320
aatgacctta tccaataagc gaattttctt ctcaattaaa taatggatgc gctcttcaag 952380
tgtatcttca gtaatcagct tatagataaa gactgtattt ttctgaccaa tccgatgcac 952440
acggtctaga gcttggtttt ccttggcagg attccaccaa cggtcataca taatcaccac 952500
attgcctgca gtcaggttaa ttcctgttcc tgccgcaagt aacgacccaa caaacacctg 952560
acaattagga tctgtagtaa atgtttcaat ttcttccttc cgattcagag attttccttg 952620
aaatcgaagc atacttgatc ccaatttcct caagatagag agtaatgatc cgaatcatgt 952680
ggatatattg cgagaataca acaactttgt atccagcgtt taaagattcc ttaagtagct 952740
taacgaaagc attccacttt ccagattcgt aatttttata ttgatccggg tctttgaaaa 952800
atactgcagg gtgatcacaa atctgcttga gatgatttaa gagagcaaaa atgtgtaaga 952860
aattcgtagc aggttcttcg ggagtttcaa gcttttgaat atgacttttc tctctttgca 952920
aggtcgccat atataacttc tcctgatccg gagacaacga acaagcaatg atagattcga 952980
ccttatcagg aagctcggga agtaccagtt tttttgtccg tcgcaagata aaaggacgag 953040
tcaactttaa taaaagatcc tgggagggga taatctcttc caattcttca gaagaacatc 953100
gtttggtaaa caactttttg aatagagcgt cagagggaag ataattgggt aagataatgt 953160
ctaaaagccc tttaaactct aagagattgt tctctatggg agttcccgta agtcccagct 953220
tcatctgagc gtctatccga cagaggattt tgtgaatttg gctactcttg tttttagcca 953280
tgtggatctc atcgaaaacg acaattgtga atgctatttt gtaaaactta tcgtagtttt 953340
gtcgtagcgt tccataagaa gttaacaaga tatcagcagg aggtagctca ctcggtttgt 953400
ttggcccatg gaaagaaaaa atactcacgc caggaagatg attacttaaa atatgctccc 953460
agtgtggtaa cacactttgt tggacataca attaaggaat ttcgggcgcg ctgagggctg 953520
tgaagactga aatacaatat ctagttaagg ctgtagcttg gtgagtcttt cctaatccca 953580
tttcatcaca gagaagccct gagagtctgt gattgtataa gaaccacatc caaagtaacc 953640
cgctgttttg atacgggcgg agctggtggt ctgaagaaaa gagattttgt ggaatcggag 953700
gtaaacacgc tgcttttaac tgtgagaaaa attgcaagtc ttcgggattg gctatagtat 953760
catcagttac tgatagtgga gctagagcat ctaatttaaa gacgtcagta atattcgcta 953820
tgacagtgtt ctcagcgatc acacatttct gcgtagatag gaactgtttt aaaaattgaa 953880
acaggttttg tttcaaatct aaaaagccag cctgtgtaaa tagaaaagta tgtttacttt 953940
tcaatccttg taaagcgatt cctatgggga cagatccgag gtttgtcttc agctccaatt 954000
gcaaatggag aggagaggca ggatgaggcc tatgaatcga ctggatcacc aattcataac 954060
tttctggagg gcgtgtctgc ggatttggaa ataaaatacg ctcatgctgc gcatactgtg 954120
taagaaactg tgggacattt tcaggaggaa tgactctagg tagaaagcat aacccttgta 954180
ggggagtggg aatcaaagag aatcctatat cttcgcgata tagaaataca ccaaatagcc 954240
aacaacggct ttcccctaaa ccaacaagaa taggcttgag gtcaagtcct tttccacgag 954300
attgcctgtg aacttcaata agaaggtctt ttaaattagg gggagaggaa aagaaattgg 954360
ggagacgtcg tagtgccgca tcgttcttta caatgaaagc agggatgtct tgaggttcta 954420
caatgagccc atcttgaatc ggcaaatcgt ttttcttctc tagaaagaaa ccttgatttg 954480
tatagtaggt ccatgtcccg aaacgtattt ctgtagaaga gggatctccg acatcatagt 954540
ggaataataa aacaccttgt tctgtgacat tataagtaag atgaccttcg ggacgttcgt 954600
ttataaatgt ttggaaaccg ggctcttgaa ttagatgccc cctctcattg agaaagtctt 954660
caacctgttc ggatttaact ataaacgctt gttttggaga tagcatcccc acgacttgca 954720
tcagaccttt cgtaggacta tagcaatagt ttgggtaaat gagactgcca ttgtctaaat 954780
ccccaggagt gacgaggtaa gcagaaaatg agaaggaagc gtcacgtagt agacggatat 954840
catagcgtag tcgatattct agagaatcat agcagagcaa agaaagcagc tgatctttga 954900
atttatcagc gagcaaagga atcgcataga tgggaagggc tatggcttta gggcctgtga 954960
tcatttcatg cgtttttgca acgtattcta cagaacctat tctagtctta ggatgattct 955020
ctctgtcttt tttatggata acaggactta atgtgaaatt taccttagct tcttctgcgc 955080
agaccgtaac atttgtaata ctgatgtcgt gagatacatt ctctaaagat gtatgcgcga 955140
gttccaactt aggaaatatg tcttctaatg taggaaaatc caagatttcc gctttaaaga 955200
ccaggccttg ccattgaaga gaaaaatgag aaggaaagcc ttgagaattt tctcctatcg 955260
taagctcagc cccttcttca tttaagaaaa agaatttttt tagcagtgcg atagagagcg 955320
ganttcaaga aagtcttatt tgtaaatact gtaggttctt cagaagcatg aatggtgcgg 955380
agccaatctt ggaatacttc ctcggacaga cactctattg ttaacgtgat atgtggggat 955440

tctagtgtat aaaccatctc tccttgagct tgtaatggaa tcgagtcgag gaaaaagtga 955500
gagaagacgg cataccaaaa ggaatgacgg aatttatcgt gtaaagggtg caatcctaaa 955560
gcgtcataga cagcaaaata tgcagtcata aggtggagac aacactcacc gtcaggacaa 955620
ctgcaagaag caaaagttag cctatcaata tcttgaagtt ttaaagtgct taaccaatag 955680
ccctctggag cttcttcatc aggaatgcgt atggtgtaac tatcctcaca aaagtctaca 955740
acgatctcct tgcgatgttt aaggagatgt tgcatcgcat cctgtcgaaa gatggctaat 955800
gcttctaaaa ccataatatt atgctagcaa cccgtatgtt attttaattc actaacttta 955860
aaacaattgt aagagagagg gttaaataaa acaagactgg ggagtatcgg aaattccata 955920
acttgtaggc aatgtttcca caagccagta caatcaaagc tggatagcta agttgtagca 955980
aaggcaagag taggtgactt atagtttcaa aattcaaaat agaaattaaa taggtgggga 956040
taagagtaca aattactgct gaagcatagt tcagtttctt aaaggaaact acacgagcta 956100
gaaaatctgc aacaatacct actaaagcaa tttccgtagt tagacaggca atgaaaacgc 956160
tgactcctgc gagaatgcta tttggcccta atgcaatcgc agagatcctt cccagaatat 956220
gacctttact tacattcaca agtaagcccg catgacgtgc agcagataaa acaaatccaa 956280
gataggtcat tcctagtaag atcgcagcaa gaaagaaacc taaagctaag gaacgcttat 956340
tttttttgct aataccttga aaactaagag ggatttcttc ctctgtagga tgtttctctt 956400
cagctacgag ctgacgtaga gagatcaaaa ctatggagca gaagaaaaac gctgcaagta 956460
aatccatagt attgaaccct tcaatgaatc ctgccaacca agcttgacgt gcgttcggaa 956520
taaattcttg aaccatgggg tgggtaggaa tcataaaact gcggatgatg acccaaagta 956580
aggtaaccaa cataatcggg aaaaatacag atcctagcca ttgaattaaa cggctgagct 956640
tgcatgagaa gatatagata agcacacagc aaatcgcact aaaaataggc aaagagggaa 956700
tgaaagcact cttatgctcg gatagtgaaa tcaatgtggc atgagatact gcaatggctc 956760
gagggattcc accgaagggg cctatcaaaa gtataatagc cgtaataaaa atcatccctg 956820
gaattcttcc tatcgagaaa aagaacttct ggtagtctcc agaatagaat agcatactga 956880
ctagacctaa aagaggaacg catacagcag taagcatcat gccgaaatag gcagaccagg 956940
gatgcgcatt gtagtggtat cccagagcta agggaaagac aatattgcca gctccgaaga 957000
acatagcaaa aatagatcct ccaatagacc aaatggacaa acttttttta tcatttgttt 957060
tatgagatgc gttttttttc atcttttaaa taaagtagag atagtaaagt gctgagtgaa 957120
tgagtttaaa gaatcatgat cttagaaatc aaacgatttt cagtagttag tttttattgt 957180
actgtgggac aaaaggtaga gttacgaatt cttaaaaagc taacaactct aaatttatac 957240
agtttttttt ataacaaagc gattttctaa gtacgggtac tatttgcttc ttttgcaaga 957300
tatgaacata tagggcaatt gtcaatttta tgatgtaaag caggacaata ctgtcgtgca 957360
taatagatga gttgcaagtg caatttcggt gtgttctcat ggccaaaaaa gcgagccaga 957420
tccttttcag cagcggaggg gctttttttt tcagaaattt tccatcgctg cgctaaacgc 957480
aaaatatgag tgtctacagg gaatgtaggt tttccatagg ctatcccaag aaaaacagaa 957540
gcggtttttc tgccaactcc cggaagttgt gtgagaagag ccatgtcatt agggggttct 957600
ccatgaaaat cacgcactaa aatttgagat aattgataaa tataggcgga ctttctctct 957660
ccaaggccac aaggggcaat gagctggtat agcttccctg gaggcaaatc taaaatagat 957720
tgggcgtctg gggcttttgc aaagagttgt ggtgtcacgg aatttactgc tttgtccgta 957780
gaattcccag ataagagaat agcaataagt agttgaaagg gagaggacca cccttctaaa 957840
gatggttttg gattgggaaa tagcgcgttg agtgttctga gaataaattg cttcatggtg 957900
agctattttc caatgcaaaa tttactaaaa atttccccta aaatactttc agtaacttct 957960
ttgccagaaa gcatcccaat cgagtgaagt gcctctctta actccaaagc aatgatttct 958020
ggaggttgca gatagaggtt tttctgcgct tctttcagac agcgagccac ctcttgtaag 958080
atcatgtgat ggcgagaaga aactaaaaac actttagagg tttttccagc ctcttgtttt 958140
tgcatccatt ggattaaagc ttgtttcact tgagtaagtc cctcaccagt ttttgctgag 958200
atcgcaaatt gagggagcga agtgtcaagg aatggaggag gggtgaggtc agctttattc 958260
cataggagga atgaaggttt tgtaaaaaga attttaggaa gatcttctag aggttgcgtc 958320
gcatctatta cccagaggat cccatccgct tcttccatag cagaaagagc tcgttcaatg 958380
ccctcttttt caatgtcatt gtctgttgtt ctttgtcctg ctgtgtctag cagtcggatg 958440
cgtttgcctt gcaagagcca ctgctcctct aagatatcac gagtggttcc aggaatatgt 958500
gttacaatcg cccgattttt ctgaagaagc gcattgagta gggaggattt ccctacgtta 958560
ggtttccctg caaggatcaa acttgttccc tgagcaagcc tctgcccctc atcaaaactg 958620
gaaataaaat cttccacgat atgcagagca ttttgaattt tttcttgagg gacgaggagg 958680
tctggttgtt cttcttcagg gaagtcggct aggacttcca aaaacgccaa tgcttcgata 958740
atcagagtat gtatttcttg aattttctta gaaaaatttc cttgaaaatg cgtttgagca 958800
atccgaaagg cgtctatatt ttccgcaaca atgagatttt ggattgcctc tgcttgaacc 958860
aggtcaattt ttccatttag aaacgctcgt tgagaaaact ctccagggag tgcaggacgg 958920
gcgcctaaag caatcaaagc gtctaaaatt tgggagcaag cgaaaaatcc tccatgacac 958980
tgaaattcga ctacatcttc tccagtgaag gagcgaggag agcgcattag aagaagaaga 959040
gcttggtcaa ttaatgtctc ttcaaaaatg acttgtccaa gatgtatcgt atgggaggca 959100
aagctagcca cagatccaga aaaaatacga tcggcaatga caatcgcttg tgggccagag 959160
agtcgtacaa cagcaatact tccttcccct ggaggagtgg caatggcagc aatggtatcg 959220
tgctttagca taaaaataga aaagttaaag gaactttcgg atagaatacc aagttttaga 959280

ttaaagaggt gaaaggagtt tggataaata ttaagactaa ttcaattgga atattttcat 959340
taatcttttt ataatttctt tttatcgttt ctagaggtct tgtgcagaag ccccaatata 959400
ttgatcgaat cactaaaaaa aaagtaatag aacctatatt ttatgaaaaa acaatgctct 959460
tcctatacaa ctctaagcta gggaagaagc tctcagtatt tctatcaaca caccccatct 959520
tttctcgaat ttatggctgg cttcaaaggt gttcgtggac acgtcggcaa attcgccctt 959580
tcatgaatcg atataagatt tctgaaaaag aattgacaaa gcctgttgca gacttcacct 959640
catttaatga tttttttacc cggaaattga aacccgaagc acgccctatt gttggtggga 959700
aagaggtatt catcactcct gtggatggac gttaccttgt gtatcctaat gtttcggaat 959760
tcgataagtt tattgttaaa tcaaaagcgt tttcattacc caagctttta ggagaccatg 959820
agttaacaaa attatacgct catggcagca ttgtttttgc ccgccttgcc ccctttgatt 959880
accatcgatt ccattttccc tgtgattgcc ttcctcaaaa gacacgttgt gtgaatggag 959940
cgctgttctc tgtccatcct ttagctgtta aagataattt cattttattt tgtgaaaata 960000
aacggacggt tactgtactc gaaacagagc agttcggcaa cgtcctctat ttagaagttg 960060
gagctatgaa tgtaggctcc attgtacaaa cattttctcc gaaccaaacc tatgctaaag 960120
gtgacgagaa gggctttttt gcttttggcg gctctacagt tatcttgctc tttttgccta 960180
acgctatacg gttcgataac gatcttttga agaactcacg tatgggtttt gaaacacgct 960240
gcttgatggg gcagtcatta ggtagatctc agagagaaga aatttaaaga ctttaaagac 960300
tttttttggt attgcaaaga gagataaaag tcaaaaatgg cgaattatgt ggttagtcat 960360
cttgtgggct ctagctgcaa gtttagcaat agcgctggtc gctaaaggtt attaccgctt 960420
tgtttatttt cgtcgctatg ctgtgcaagt tatacgagaa gtacgcctaa gtatggagct 960480
caaagaatgg gcgcttgcag aacagcaact cctacctatt ttaaaaaaac ggtcgtatcg 960540
acggcagtgt ttatttgaat atatgcgcat tctacgcaag atgcagcgtt tcgaggaatc 960600
cgagaaactg cttgccgaag cgaagaaatt gggattgcgc ggtccctatt ttttcttaga 960660
aattgcctat aaagcctaca ggtttggggc ttttaaagaa tgtgcacagg cgtttgcttc 960720
cgttcctcaa gatttgtttg aagaagaaga cgctgcaaaa tatgcttcag ctctggtgcg 960780
gttaggcgat ttagatgctg cctgcagttt gattgaacca tggatttctc ccctatctca 960840
ccaagaaact tttgtgacta tggggcacat ttatttcact tccaagcgtt ataaagatgc 960900
tatagatttt tataatcgtg cgaatgcttt gggagtctgc cctgttgagg tcacctataa 960960
tttagcacaa gcgtatcgca ttacctcaag ctatgcaaaa gcaggcaaat tattccgcaa 961020
actcttatca aatcctgtct acaaagaaga agctttattt aatatcgggc tttgtgaaca 961080
aaagctaggc agaccaggga aagctctact tatttatcag agcagtgatc tttggtctcg 961140
tggggatgcc ttgctgatga aatatgcagc tatggcagct atggatcaac gagattatgt 961200
attagcagag ccctgctggg aattggcttt gcgctgttcc acatttgcta aagattacaa 961260
atgcgggcta ggctatggct ttagcttgtg tcgattacgc aagtatggag acgcagagcg 961320
tgtgtattgt aatctgattc aaaatttccc tgaatgttta acagcgtgca aggctttggc 961380
ttggctttgt ggagttgggt atgcaacact acttggttca gaagaaggct tgatgtatgc 961440
gaaaaaggcc gtagaactcg accatagttg tgaaacttta gagttattaa gtgcatgcga 961500
agcacgttgt ggaaattttg atgctgctta tgaaattcaa tcgtttcttt cttctcgaga 961560
tacctctttg caggagaaac aacgacgttc gcagattttg cgaattttac gtaaaaaact 961620
acctctcaac gatcatcata ttgtggaagt ggatgctctg cttgccgctt aaatctgatt 961680
tttttaaatt ttttattcta tggactacaa agccctcgtg gctcgaagat aacaaaaagc 961740
agaaatcagc ttcttgggaa cttttattcg ttttattaag taagatcgag tatggtattg 961800
agaaacgttc tcgtaaaaga tggtattcta gtaattacag tgaattaaac gacatatgtt 961860
aggttttctt aaacgcttct ttggttcctc tcaagagcgt attctaaaaa aatttcaaaa 961920
acttgtagat aaagtgaaca tttatgatga aatgctcacg cctttatctg atgatgaatt 961980
gcggaataag actgcggaat taaagcagag atatcagaac ggagagtctc ttgatagcat 962040
gcttcctgaa gcttatggtg tcgtgaaaaa cgtttgtcga cgtttagcag gcaccccagt 962100
cgaagtctcc ggataccatc aaagatggga tatggttcct tatgatgtgc agattctagg 962160
ggccattgct atgcacaagg gatttattac agagatgcag accggggagg ggaaaacact 962220
cactgcagtg atgcctctgt atttaaatgc tttaacaggc aagccagtgc atttggttac 962280
tgttaacgac tatcttgcac aacgagattg tgaatgggta ggatcggtac tgcgctggtt 962340
aggacttaca acgggagttt tggtttcagg aactctttta gaaaagcgta agaaaattta 962400
tcaatgtgat gttgtctatg gtacagcatc tgagtttggt tttgattatt tgagagataa 962460
ttctatagct actcgccttg aagagcaggt aggtagagga tattactttg ctatcattga 962520
tgaagtcgac tcgatcttaa tagatgaagc tagaacaccc ttaattatct caggtcctgg 962580
agaaaaacat aatccagtct attttgagct taaagaaaaa gtcgcaagtc tagtgtattt 962640
gcaaaaagag ctctgcagcc gtatcgcatt agaagcacgt cgcggcttag atagcttttt 962700
agatgttgat attcttccta aagataaaaa agttcttgaa ggcatctctg aattttgccg 962760
cagcctttgg ttggtaagca aaggaatgcc tttgaatcgt gtgttacgtc gtgtacgtga 962820
gcacccagat cttcgtgcta tgatcgataa atgggatgtt tattatcatg ctgagcagaa 962880
taaagaagag agcctagagc gtctttcaga gctctacatt attgttgatg agcacaataa 962940
tgattttgag cttacagata aaggaatgca gcagtgggtt gagtatgctg gaggctctac 963000
cgaagagttc gtgatgatgg atatggggca tgagtatgct cttatagaaa atgatgagac 963060
cctatcacct gcagataaga tcaataaaaa aattgcaatt tctgaagaag acaccttaag 963120

aaaggctcgt gctcacggat tacgacagtt attacgagcc caacttctca tggagcgtga 963180
tgtagattat attgtccgcg acgatcagat tgtgattatc gatgaacata caggacgtcc 963240
tcaacctgga cgacgttttt ctgaaggcct ccatcaagct atcgaagcta aagaacacgt 963300
cactatccgt aaggaatctc agacgcttgc tacagtcacg ttgcaaaatt tcttccgtct 963360
atatgaaaag cttgcaggga tgaccggaac agcaattacg gagtctcgag agtttaaaga 963420
aatttataat ctttatgtcc tccaagtacc cacgttcaag ccttgcttac gcatagatca 963480
taatgatgaa ttttatatga cagagcgtga gaagtaccac gctattgtta atgagattgc 963540
gactattcat ggcaagggga accctattct tgttggtaca gaatctgtag aggtctctga 963600
gaagctgtct cggattttga gacagaatcg gatagagcat actgtattga atgctaagaa 963660
tcatgctcaa gaagcagaaa ttatagcagg agcagggaaa ttaggtgctg tgactgtagc 963720
tacaaatatg gctggtcgag gcacagacat caaactagat aatgaagctg tgatcgtagg 963780
cggtctccat gtgatcggta ctacacggca tcaatcccgt cgaattgata gacagttgcg 963840
tgggcgttgt gctcgtttag gagaccctgg cgctgcgaaa ttctttttat cttttgaaga 963900
tcggctgatg cgactattcg cctcccctaa attgaatacc cttatccgtc attttcgtcc 963960
tccagaagga gaggcgatgt cggaccctat gtttaataga ctcatagaaa cagcacagaa 964020
acgtgtcgaa gggagaaact atactatccg taagcatacc ttagagtatg atgatgtcat 964080
gaataagcag agacaagcga tatacgcttt ccgccatgat gtcttacatg cggaatctgt 964140
tttcgatctt gcaaaagaaa ttctatgcca tgtgtctctg atggtagcat ccttagtgat 964200
gagtgatcgt cagttcaaag ggtggacatt gccaaatctt gaagaatgga taacctcatc 964260
tttcccaata gccttaaata tagaagaact cagacagctt aaagatacag attctattgc 964320
tgaaaagatc gctgctgaat tgattcaaga gtttcaagta cgctttgatc atatggtaga 964380
agggctctcc aaagctggag gggaagaatt ggatgcatct gctatttgta gagatgtcgt 964440
tcggtctgtc atggtcatgc atattgatga gcagtggcgg attcatcttg tagatatgga 964500
cttactacgg agtgaagttg gcctacgtac tgtagggcaa aagatccttt tgttagaatt 964560
taaacacgag tctttcttac tgtttgagag cttgattcgt gatattcgta ttacgattgc 964620
gcggcatctt ttccgtcttg agctgaccgt agagcctaat cctcgtgtca acaacgtgat 964680
tcctactgta gctacgtctt ttcataataa tgtgaattac ggtccgttag agttgactgt 964740
agttacagat tctgaagatc aagattaaga aataacctag aaggaggagt ccttctaggt 964800
atttgtttca caggctacat agtttttatt tttaaataaa tggggtccat tctgaccaaa 964860
cttggtcatt atagtagcga atccagaatt ttctgcgatt gccggctatg aggaactggg 964920
catagtggct cagtgtattg tttgctatac agaaaagaat gtgcttttgg ttcttggagt 964980
taggaaaatc cttaggggcg ttgagcaaag tatcaacgtt tgtagtaagg agagactgtg 965040
tttttaggag ttggttgcag tcgtattcag gttctgaaat tgtcttcagt gcaatgttag 965100
accagggaga cctacttatt gttcttggtg tatagtattt ttaaatacag attcaccaag 965160
aacaataagt aggtctccct ggtctgtatt ttcttcaaca tacaccccag tttcttgata 965220
tttgtctagt gaagctgctt cttcagagtt gtcaagtaag tcttcgaaaa tctgatcgtg 965280
gtcggtgtcg ggcgtatcgt attggtcata gttcatgact ttaaatctcc aagaaataat 965340
taatttattt ttatttcatg gtaataatat tttttattat tttttaacaa attaattatg 965400
tctttttggt ttttctttaa attctaaatc gaaaggtatt ccatataaat taaacgacga 965460
ttttaatgtg ttttttaaat aatattcata atgttttgtt aatagagact ttgcatttat 965520
aaataataaa aactgcagag gtgtcgttgt cttttggatt gcataataga tcctcaacct 965580
gcgtccttga atcacctgag gatggtttct atgtagagca gaagcaagag ttttatttac 965640
tatcggtgtt ggaactttat tcgatactac gtgatgtagt tcatcaattg cagagaagat 965700
ctttttaaga ttgcgtttcg tagtagcaga gatacaaagc attttagctt gtcctaaata 965760
aggatcagta gcgcgtagat ctttgcagta atgctccatg cggacttctt caagtaaatc 965820
ccatttgtta attaatatga tgtgaggctt ttttcgttta gaaattaaag agaggatgcg 965880
cttctcataa gaagagagtt tttgtgtagc atcaatcaca agaagacaga tatcagcacg 965940
agaaatagct ttttcagttc gagatgaaga aatccattct atggaattct taacgctttt 966000
catttttcta agacccgcag tatcaatgaa aagatactgg cggtccttat gggaatacag 966060
aatatcgata ttatcacgtg ttgtcccagg agtattatca ataatacaac gctcttcatt 966120
gagaagaccg ttaataatag aagatttccc tacgttagga cgtccgatga gcgctatttt 966180
taatgtcttg ggagcttgct gaggagactc tgggatagtg caggttcttt caggggagaa 966240
gccttccgtg aatacttctg aaaaatcagg gaatgtattt gaagggagag ccgcctcaga 966300
ttcttcgtgt tcatctacag aaagctcctc aaggccttct tcttcttctt cgcgaggttc 966360
tggtaggttg gctaccaact taattctttg aagaagggta tcaatgtgct tatcgtgagc 966420
tgttgaggtc actacaatat ctcgaattcc taatttatag gtctcatgaa tttgtaattc 966480
ctcttggcga ctgtctgctt tattggcgac aaggatgaga ggtttcttta agggaaggag 966540
gagtttggct aggtgagcat cttcttcggt aataccacag cggatatcaa tgactagcaa 966600
taggacatca gcttccttag cgccggtgag ggcttggtta tagatatgct tttggaagta 966660
atcttcagaa ttgtgatcaa cacctccagt atcgatgacc tgagcaggaa caccaaacgc 966720
atggagctcg ccgtagaggc gatctcgagt tgtcccctct tgagaattta caattgctaa 966780
agagcgttta catagacgat tgaaaagtga tgactttcct acattgggtc ttcctaagat 966840
ggctattttt agcataatcg tctctattct attttttcag taattaatga gaaaataata 966900
ggagttaaaa aattaaagct aaagagataa atttctaagg gttgtaaaag taggattcta 966960

tttaaattaa tctacttcca aaagcctttt tcttgtaaaa gcaataaaat tttttctttg 967020
tcgagacatt cattttctat agagagaatc tcagcttctc tgagtagatc gccaagaagt 967080
cttccgggag agatgccctt agctatcaga tcaggagccg aaacaacggg ggaggatgtt 967140
tttattctta atatgaattg ctctaagcgt gattcgagtt cttgcactcg agagataaag 967200
tgctgctgtc tgctcggatc tttttgtaga gccgagaaga gctccaaaaa tagaggtgct 967260
gtcggagacg ctaggaaatg cgcccaaaat acacgattgc cggattggtt ttggaaatga 967320
gggagtgcct cgtaccaaga ttctattaat ttcaattctt tattagaaat tcgtaatctt 967380
ccaaacgcta ctgttgcggc ttcctcactg accccttgga atagaggcag taaaaaaaga 967440
atttcaggga aatgtgttgg attaaactta cgggcaaatt ctatggttgt tcttaagaga 967500
ctgtagggaa tatcacgtag ttcagggaaa ataaaaatta agactttaag cttgagcaga 967560
agagagaggg ctccataagg ctgtctttta agcatttttt ttagctcttg ccagattctc 967620
tccggggata cggaatttac taatgccggg gcttctttaa taatggcgcg ttctgtagtt 967680
gggtctaggg tgaagccgag ggaggaagaa aaacgtatgg cacgtaggat acgcagttta 967740
tcttctgaga atcttaaacg tggatggcct atagctcgaa tgactttctt ttcaatatca 967800
cgagttcctt cgacaaagtc gaaaactttg tcttcaaaag gatcgtagta catcccgttt 967860
actgtgaagt ctcttcggag ggcatcttct cgcatagaag aaaagatgat acgatcggga 967920
tgccttccat ctttgtattc accgtcagag cggaaagttg caacttcaaa taggcgtccg 967980
tcctgtttta ctacgatgat accaaaggca acaccaatac tgatgacgtc tgggaagatc 968040
gtagatacaa tcgttgggga tgcattggta gctatgtcaa tatcctcgag agggcgattc 968100
attaacatat ctcgaacaca accaccgaca aaatatgcct gatatcctgc attacgtagt 968160
ttgataagaa ctttttttgc agcttctatg gcaattgttg tcatgacacc cctaaaatag 968220
gaactaaaaa gagctaagcg atagcttctg gggtccttct gataattatt ggcgctttat 968280
tttctgcgat agtgtcttct tggatatgaa tagcttctac tgtaggatct gaaggaattt 968340
caaacataag gtctctaagg agattttcta ggatcatccc tagagcacga gctccagttt 968400
ttgcttgctt ggcttttttt gctatagcat atagggcttc ttttttgaag actaacttga 968460
cgttttcttc tgcgaatagc tccatatatt gtttcacaat cgcatttgta ggttctgtaa 968520
ggatggctac aagctcatcc aaagaaagct cttcacagtt tacaatgcag ttgaatcttc 968580
cgacaaattc agggatcatt ccgaaggcaa tcaggtcttc ggtttcaact ttagcaagta 968640
gatggtctct ggttttttga gagaggtctg cttgatcatc agaaaaccct atggtagttt 968700
tccccaatcg ctttgcgata atcttatcta ggttgacgaa agtccgccta cgataaataa 968760
gatattttcc gtattgactc ggatatactc ttggttagga tgcttacgtc ctcctttagg 968820
aggaacgttt gctgtggttc cttcaacgat ttttaacaat gcttgttgaa ccccttcgcc 968880
agaaacatct ctagtaatgg agacgtttgc tgttgtcctt ccaattttat cgatttcatc 968940
gatatagata atgcctcgtt ctgcacgggc gacatcgtaa tcagcagctt gtaataaacg 969000
taagacaatg ttctctacat cttcaccgac ataacctgct tccgttaggg tcgttgcgtc 969060
ggctatggtg aagggaacat ctaaaatttt tgccaatgtt tttgcaatta atgtttttcc 969120
agatcctgta gggcctagga gaagcacgtt agatttcccg tagcttacct gtttgttatg 969180
tagtagagca cgtatacgtt tatagtgatt ataaacagca acagcgattg tctttttagc 969240
tctttcctga ccaatgacat attcatcaat atgctttttg atttccttag gggtaagcac 969300
cctgagatca gaaggctgtg aaggtgtttc agaaactgga gctgaggata ttgtagagga 969360
gggtttctta tctaaaattc cagagcataa tttgatgcag tagtcacaaa tgtataccga 969420
aggcccagca atcagtttct ctacatcttt ttcagaccga ccgcaaaatg aacaaatagt 969480
tagatttttt ttattcatat gttctctcta agtgctactt gtatccttat tagtttcttt 969540
cgcagaagtt accaccttat caataagtcc gtaggagatg gcttcctcgg ctcccatgaa 969600
gaaatctcgt tcagaatctt ctataatttt ttctacaggt tgtcctgtgc attcagagag 969660
gatattggca aggtgttttt ttagtgttag aatttcagct gcttggagtt ggatgtctgc 969720
ggatgttccg ataatgcctc cagaaggttg gtggatcatc atacggctat ggggaagagc 969780
gtgacgcttt cctttagttc tgcggataat aagagggctc ccatggatgc agcttgaccg 969840
atgcagtagg tatttacatc acaacctaaa aagcgaatgg tatcatagat tgccagtcca 969900
gcggtgatgt agccgcctgg ggaattgatg aaaatttgaa tatccttttt aggatcttcg 969960
gacatgagga aaaggagctg ggcaattact gtgtttgcga ggggctccgt gatttcctga 970020
ccgatcatta caatacgatc tttcagaaga cgggagtaaa tatccatggc cctttcacca 970080
cggcccgtat cctcgacaac atagggtacc agtgtcattt gcgtttcctc atcaaataat 970140
tttcagttgt gtccatacgt tcccgatcac gaaatgaccc taggaacgta gctaagactt 970200
tttaggctgt ctacgtcagc aacaagctta gacattaaaa tctcgaattc tatgtttaag 970260
actttcttgg gtcaagtatt ttcctctaac taaaatttat gcagaaggag ttgaagccaa 970320
cagttctgct ttgcgaagaa catgttcgat agctttgcta taggtcagcc gatcgcgggc 970380
tgacattaca agttcttgta aggtgtcatt ggaaatatct ttaggaggtt gttgtccgaa 970440
gcgctctctt gaacatacat ccatcatgta ttgcagttct tcgcggctaa tggtgagttt 970500
ttcatcagag aaaattttat gagtcaaaaa cagtaacttt aatgcttttg tagcatcttc 970560
ttctgcttcc ttgataagtt ctgatttctt ttttcaagtt cttcatcaga acaatattgg 970620
ataagacgtg cattaagaag tttttctctt gtgattaagg aaatacgttc ttcaagcagg 970680
gaggtaggaa gttcaaaatc tacgagcatg gcaagagcat cttcagcttc agaaaagcgt 970740
ttttgcaatt gtttatcttt agcttgcttc tctagttgta tgcgtaattt cgcttttaaa 970800

tcgtctagag attctgcttg tagttgacga gctttttcgt catctatctc ggggatcgag 970860
acttcgatta cagcgtttac tgtaaatgtt aaagtatctc ctcttaagaa ggattgaatt 970920
tcaggggagg taattgtctc aacaacacga tgtccagtag aaattcctaa gaatttttct 970980
ttaaaggcat cggtcatttc ttcttcagaa agcttaaaat atttgttttc aaaaatcgct 971040
gcagaggaag cattctcatc attactttta gagacgtgca aggaaataga aatgaaatcg 971100
ccttcttggg aaggacgttc tacaggagtt tttgttgcaa agaacatacc aatgtttgtg 971160
agtcccttct cgatatcact atctgaaatc tcactagcag cttcttcctg aggtaaagaa 971220
aggttttccc aaggaagatc agaaattgca gggaaagctt ttcataagag aactctactt 971280
tggctccttc ttgaagatcg aattgagtga ttgagttcga acggacagct ttaggcgaaa 971340
gaggacgacg atctccgact gtagataagg catgataagc gtcttgagtt actagttctc 971400
ctaactcttt tcttacgtta gtaggatatc gggatgcaat tacatcatca ggggcttttc 971460
ctttgcggaa tcctggaagt gtaatttctt ttttaatctt tttaagagct tgcttgttga 971520
gtttgttaag tacttctggg gagactttta ctaaagctga gacaatacaa ccaggagatt 971580
cttctaaatc gacggagaac tgctcattgg agagactacg tggcacaaga caaccctttt 971640
tattagactt gaaaggaaaa gcgggtgatg aggcttgaac tcacgacctt cacgttggca 971700
acgtgacgct ctaccactga gctacacccg caaagaaaag aatagtttag atttttgaag 971760
agttttnngc aacgactaga gtctttagta tcatagaaat tcgtgtctct tccccctgag 971820
aagggctttg ataaaatttt tcattgattt agcatgggta aacaggtaca agtaagcaga 971880
ttatattatc cactcttatt ccagaggaga aatgcttaat tttcgcaagt tacgccggga 971940
tttttcagcc aatattttac aagatggtaa aaaacttttt gagcaggggg ctgtgattga 972000
tgcgaaaatc ctttcgatga atggagagac tgtctgcatc agcgctcagg ttcggggctt 972060
gtacgacaac atttatgagt gtgagattga agttgatcgc tcggaatccg atactgtgga 972120
ttccaactgt gattgttcgt ataactacga ctgccagcac atcgtcgcac tattattcta 972180
tttagagcaa tattttaatg agatggtagt agcctatgct cgtagtgctg atttagaaac 972240
ggatcacgag atcaacgagg aagtaaaaaa ggagctcaag gaaacttttg tcgctgctgc 972300
cacaaaggaa gaagagcgta aagatcgtga gcatcaaaaa gagattttaa gagagtatgt 972360
tcacgctgca aatgctttaa gtgcgaatcc tttttcccta cctttagaat atttagaaaa 972420
ggattctgct gagcttgctg tattatttgt ttctgtaaat gaggatacgt ttgctcctgc 972480
caatcagcct atagagtttc aattagtact tcgtttaccg tgtcgttcca agccttttta 972540
tatctctaat atccgtacct ttttggaagg ggtgttgtat caggagccaa ttgtattgaa 972600
tgggcgtcgg tttttcttta cgatgcaatc gtttaatgct tccgatcgca agctaataga 972660
tttattgatt cgctatgtcc gttaccccaa tcatacaacc gaagagaagt tattaaaatc 972720
tgcgtatttg atgcctcctg cgttaggtgt gattcttgca aagatgtttg aacatcaact 972780
ggcagatcgt ggaggaggaa gtttagggga aaaagagagt ttttcagggt tattctgtgg 972840
aaatcttgaa gagcctctgt gttggtcatt aactccggct aagatgaagt ttaacttaga 972900
cttctttgac atgccctaca aagcgttgtt aatgactcct gtgattcttg ttgatgatga 972960
tgaagttcag cctgagcaga ccatgttatt agagtcggat gctccaggga ttattcatca 973020
ttttgtttat catcggtttt ctcctcagat caagcgtgcg catttacgtt cctttagtcg 973080
tttgcgagat atagcaattc cagaggcttt gtttggttcg ttccgtgaga atgctcttcc 973140
tgtatttcag gaatatgctg aaattgcgaa tgttcacctt ttgaattcct ttgtgacact 973200
tccttatgta gatgaggtcc gggccatttg tgatatgagc tatttggacg gggaattaga 973260
ggcaaaatta catttccttt atggttcttt acgggttcca gcagcatctt tggctttgca 973320
atatcaggat gttcgtgcct ttattagtga tgagggaatc ttagccagaa atctggttga 973380
agagcgtaag atgttggaag aggtcttctc aggctttatt tatgatgaac gcgatggagc 973440
ttttcgtgtt aaaagtgaga agaagatcgt ggaatttatg acggagacga tccctgcgaa 973500
tcaacatcgc attactttta actgtccgga aaatctttca ggtcagttta tttatgatga 973560
gacgatcttt gaattatcgt tccgagaagg gagcgacatt aattattatg aggcagacct 973620
taaggttcat ggtttattga aaggagtgcc tttagattta ttgtgggact gcattagtgc 973680
gaaaaagcgc tttttagagc ttcctaaagc gggtcagcaa tctaagggaa cgcggcgcgg 973740
taaggtgaat tcgggtaagt tgccttgtat tttagtctta gacttagaaa aaattgctcc 973800
tgtggtgcag atttttaatg aaataggatt taaagtttta gatgacttag ttcagaagtg 973860
tcctttatgg agtttaacgg gaatttcgtt agatcagttt gaagcacttc ctgtgaactt 973920
ttccatgtct gaaaggctta tagagattca gaagcaaatt cgtggtgaga tcgagtttga 973980
tttccaagat gttcctcagc agattcaggc aacgttacgt agctatcaaa ccgagggcgt 974040
acattggtta gagcgtttga gaaaaatgca tctcaacggg attttagctg atgatatggg 974100
acttggaaag actctccagg cgattattgc tgttactcag agtaaactag agaaaggcag 974160
cggctgttct ttgattgttt gtcctacctc tttagtttat aactggaagg aagagttccg 974220
taaatttaat cctgaattca ggactttagt tattgatgga gttccttctc aaagacggaa 974280
gcagttaacg gctttagctg atcgcgacgt cgcgattact tcgtataatt tattacagaa 974340
agacgtggag ttatataaga gctttcgttt tgactatgtt gttttagatg aagcgcacca 974400
cattaagaat cgtacgactc ggaatgcaaa atcggtgaag atgattcaat cggatcatcg 974460
gttgatatta actggaacgc cgatagagaa ctcgttagaa gagttatgga gtctttttga 974520
tttcttaatg cctggtttat tgagcagcta cgatcgcttt gttggaaagt acatacgtac 974580
gggcaactat atgggcaata aagctgacaa tatggttgcg cttaagaaaa aggtctcacc 974640

ttttattctt cctcgtatga aagaagatgt attgaaagat cttcctccag tctctgagat 974700
tttatatcac tgtcatctta cagaatctca gaaggagctg tatcagtcct atgcagcttc 974760
tgcgaaaaaa gagctttcac gtttggtcaa gcaggaaggt tttgagcgta tccatattca 974820
tgttttagca actttgactc ggttaaagca aatttgctgt catcctgcta tttttgctaa 974880
ggatgctcca gagcctgggg attcagcaaa gtatgatatg ttgatggatc tactttcttc 974940
tcttgtggat tctggccata agactgtggt ctttagtcag tatacaaaga tgctgggcat 975000
tattaagaaa gatttagagt ctcgaggcat tccttttgtc tatctagatg gttccaccaa 975060
gaacagacta gatttagtga atcagtttaa tgaagatacc tagcttgttg gttttcttaa 975120
tttccttaaa agctgggggc acgggcttga atcttgtcgg tgctgataca gtgaattcac 975180
tacgacatgt ggtggaatcc tgctgtagag aatcaagcga ctgaccgagt ccatcgtatt 975240
gggcagagcc gttctgtctc ttcctataaa ttggtaacct tgaacacgat tgaagaaaaa 975300
atccttactt tgcagaacag gaaanagagc cttgtaaaga aagtgattaa ctctgatgat 975360
gaggttgtat ccaagttaac ttgggaagaa gtattggaat tgctgcagat atgattttat 975420
gagtccacat cgcaatctgt ttaaacttaa gaatttttcc aatcgccttt acaacagggc 975480
tttgggtcgt ttcgacaagg tctttaattt tttttctggt aatgttggca ttgatttagg 975540
cactgcaaac actttggttt atgtccgagg tcggggtatt gttcttagtg aaccttctgt 975600
agttgctgtg gatgcacaga cgcatgcagt gcttgctgta gggcataagg cgaaagcgat 975660
gttgggtaag acgcccagaa agattatggc agtgcgtcct atgaaggatg gggtaattgc 975720
tgattttgag attgctgagg gcatgttaaa ggctttaatt aaacgtgtaa ctccttctcg 975780
tagtgttttt cgcccaagaa ttttaattgc tgttccttct gggattactg gagtagaaaa 975840
acgtgctgtt gaagattctg cattacatgc tggggctcag gaagtaatct taattgaaga 975900
acctatggct gctgcgatag gtgtggatct gcctgttcat gagcctgcag cgagtatgat 975960
tattgatatt ggaggaggca ctaccgagat tgctattatt tctttgggag gaattgttga 976020
atctcgttcc ttgcgtattg ctggggacga atttgatgag tgtatcatta actacatgcg 976080
tcgtacgtac aatttaatga taggtcctcg tactgctgaa gaaatcaaga ttaccattgg 976140
ctcggcctat cctttagggg atcaggagtt ggagatggaa gttcgaggtc gtgatcaggt 976200
ggcgggcttg ccgattacca agcgtattaa ttctgtggag attagagagt gtttggctga 976260
acctattcag cagatcatag aatgtgtacg tttgacatta gagaagtgtc caccagaact 976320
ttctgctgat ttagtagaac gtggtatggt tttagctgga gggggcgctt taattaaggg 976380
attagataag gctttgagta agaacacagg actttctgtg attacagcac cgcatccttt 976440
gctggcagtt tgtttaggaa ccgggaaagc tttggaacat ttagatcagt ttaagaagcg 976500
taaagggaat ttggtatagt catggtatgg agcaccaaca tcaagcatga aggtctgaaa 976560
tcttggattg atgaggttgc taaattaacg actccgaaga catacgtctt tgtgatggtt 976620
cggataccga gtatgatgag ctctgcactc ttatggagag tacagggacg atgatccgtt 976680
tgaatcctga gtttcatccc aattgctttt tggtccgttc gtctgctgat gatgtcgctc 976740
gggtagagca atttactttc atttgcactt cgacggaagc agaggcaggc cctacgaata 976800
attggagaga tcctcaagag atgcgtaggg agttgcatca actttttcgt ggatgtatgc 976860
aggggcgcac tctgtatata gttcctttct gtatgggtcc tttagactct ccatttctta 976920
ttgtaggtgt tgagttaaca gattctcctt atgtcgtttg ttctatgaag atcatgactc 976980
gtatgggtga tgatgtttta cgttctttag ggacttcggg gaagttttta aagtgtttac 977040
atagtgtggg taagccttta tctccaggag aggccgatgt ttcttggcct tgcaatccga 977100
aatcaatgcg gattgtacat tttcaagatg atagtagtgt gatgtctttt ggaagtggtt 977160
atggaggcaa tgctttactg ggtaagaagt gtgtggctct tcgcttagct tcttacatgg 977220
caaaatctca gggctggctt gctgagcaca tgttgattat tggaattacc aatcctgaag 977280
ggaagaaaaa atacttttct gcttcgttcc cgagtgcctg tggtaagaca aatttagcta 977340
tgttgatgcc taaacttcca ggttggaaga ttgagtgtat tggggatgat attgcttgga 977400
tccgtccggg tcgtgacggc aggttgtatg ctgtaaatcc agaatatgga ttttttggtg 977460
ttgctcctgg gacttctgag cgtacaaatc ccaatgcttt ggcaacttgc aggtcgaatt 977520
ctatttttac gaatgttgcc ttgactgccg atggggatgt ttggtgggag ggattaacgg 977580
agcaaccacc agagccttta acagattggc ttggaaagcc ttggaagcct ggagggagtc 977640
ctgctgccca tccgaattct cgatttacag ctcctttacg tcagtgtcct tctttagatc 977700
ctgagtggaa tagtcctcag ggtgttccct tagatgctat tatttttggc ggacgtcgtt 977760
ccgaaaccat tcctttagtt tacgaagctt taagttggga acacggagtg actataggag 977820
cggggatgtc atcgacgaca actgcagcta ttgttggtca gttaggtaaa ctccgccacg 977880
atccttttgc catgcttcct ttctgtgggt ataacatggc atattacttc cagcattggc 977940
tttcctttgc tgagaataga agtcttaagc ttcctaagat ttttggagtc aattggttcc 978000
gtaagaataa tcaaggggag tttctctggc ctggttttag tgaaaactta cgtgttctag 978060
aatggatatt ccaaaggact gatggtctgg aagatattgc agagcgcacg cctattggtt 978120
atcttcctaa cattcaaaaa ttcaacctca atggcttgaa tcttgaccta caaactgtac 978180
aagaactgtt ctctgtagat gctgagggtt ggcttgctga agttgagaac attggggagt 978240
atttgaagat ctttggttcg gattgtccgc agcagattac tgatgagttg ttgcgaatta 978300
aatcagaatt aaaagaaaaa taaaaatcaa agtcattagt tatttataag ttagttttaa 978360
taacaattat ttcttatttt ttataaatta ttttttaaat aaaattaaga ataattgatt 978420
attattatct gataaatacc gtgacgctac aaccgagcta cattaatttc acccccaatg 978480

tgaccactgc tttatctggt ggtaagattg atacgtcagc tatcgaactt tcttgcagcg 978540
ctctattttt ccaagagttg caagataaag ctcagggttt aaaacatgct ttgggtttag 978600
ttcaggagtt aagtgcagag gcacttcgtc ccgcccaagt tcaaacctca atttcctatc 978660
ttcctacaga agaatcctca cgtccaggga tttctgctgg aatcatagat aggacaatgc 978720
cgacctttac tgatgacgag gtaaaggcta tcctccaaaa ccctaatttt gaaacgtcta 978780
aaattttcgt tgagggcttg gataaggtct tcaagagtta tttagattct gttacccctc 978840
ccgagggtat agatccttcg aatcctgaga gtgctattat acttaactat ataacgctct 978900
tgaataatct taagcctaaa tttgctgctg gttcgacacc aacagacgct gattataacg 978960
ctctctatgc gttgcctggt gattttgtga aggaaatcga ggctttgaaa gcagcggatg 979020
ctcctcctaa aagtaaggtt catgcttttt ggcaggagat catgacaatt tacaataaca 979080
tgcaggtact ttcctatcct gttacggatt accttaatgt tcagattgcg gatctttctc 979140
ttaatattac cgctgctcag gaggtccaac agtatctaaa gaacttttat agcatattaa 979200
aggacatttt gaatccagga tggacggatc cacaggcgac gcactatcca gcagatgcag 979260
agtataatgc tcgcgatgct ggggtgattc aaagtttatt aaatttgagt gggaactatc 979320
gtcagctgac tgagaatatg ctccccaata cagatacaag tcttccccag gaaattattg 979380
ctcagattcg tagtttccaa aatggtgtga atgggacgat aattgcatcc aacactcttc 979440
tacctactac catgagactc gatactctcc ttggtgttat ctatacctat cagtgttgtg 979500
ccactatttt tggaatgagc tatggaacta gcactccggc taagcaaaat tacatagatg 979560
ctattaatca agaaaaaagt tactggcaag cgcgagcaaa tggatttgat gtaactagtg 979620
atcaggtttt tgaccaattt gctactaata tacagagcgg cacttcatat agaggcatag 979680
atcttttcaa aaataataaa gtaaacgaga tcaatcctat tttcttaagt caggctgcat 979740
ctttcttacg gtatccgtat aatttgatgt cacgtagtat gtatcaaact atagaggatg 979800
ctgctaatag atctattacg gctttggatg ggctaatttc cggttggagt acacagatag 979860
caaccttcca aacgcagaaa aattctttgg atccttcttt gttgaagtat tttgatacta 979920
tgaaagccaa caaagagtct tttgtaacca cagctccttt gcagatggtc tactcttcat 979980
tgatgttgga taagtatttg cctacgcagc agaacgtgat tgcctcctta gggatacaga 980040
tgacgtattc taataaggct gctaagtatc tcaacgaact aatcaaagag atcactacgt 980100
ttcaatctgc cgatatttat tattctttat ctatatattt gaaacagatg aatttgcaag 980160
cggtagcgga tcctattggt aaggctgtgg gtgttttgaa tgatgaaaaa acacgagcga 980220
tggcagatat tacccgctgc aataagataa aagcggctat tgataagatg ctcgttgaga 980280
tcaaggcgga tgcagagtta tcaaagtcac aaattcgtga gcttgtggat acgttaacaa 980340
acttcaagtc tcaaagcgac gatctgattc gcaatttatc ttgtttactg ggtttcttat 980400
cagggctcac ccttaaagct gtgaatgacc ctaatgctac gtatgaggca tttactgcag 980460
aaatcttcac agaacctttt aataattgga aacggcagtt agctacattt gagagttttg 980520
tgattcaagg tgggcaaaat ggaattaccc caggtggtca gcaacagctc ttacaggcta 980580
tggagtcttc acaacaagat ttttcaacgt ttaaccagaa ccaacagctt gctctgcaat 980640
tagaatcatc agcaatgcaa caggaatgga ctcttgtaag tgctgccctt gctttattaa 980700
accaaatggt ttcgaagatt gcgagaagga ttaaatctta gtacttagga aatagaatat 980760
tatgcatccg aaaatagaaa aaagaaatag ccttccactt acggcagtcg ctcctgtgtt 980820
tgaagaatcg tatcatcctt ctgtagctac aactgtagat tatgtagatg ccacgacact 980880
ttcccgacat cttacagtct taaaagatgt gataaaagaa gctcgaaact tagatttagg 980940
gaaggcattc ctgacatcta tgaaacaggg ttttataaat acgggtacgg aacttgccat 981000
tatacaagca tctctggcag atcagagtag tcgcgagtcg cgtaagaagg aagagaagat 981060
cttccatcag cacttaggaa aggcagcccc acaagcggca acagcaactt caggagtgca 981120
gcctactgcg gatcctgttg ctgataagat gcctttacaa tctgcatttg cctatgttct 981180
ccttgataag tacattcctg ctcaagagga agccctttat gctcttggaa gggagttaaa 981240
cctatcagga tatgcgcaaa atttatttag tcctctttta gatatgatta agagctttaa 981300
ctctgctcct atcaactaca atttaggatc gtacatatct cagacgagtg gcactgcgaa 981360
tttcgcgtat ggttatgaga tgattttatc gcgctataac aacgaagtct ctcaatgtcg 981420
cctggacata gcaagtacag taaaagctaa agctgcgtta gcgaacatgt cggcttctgt 981480
taaagcaaat gtgagtctga ctgatgcaca gaagaaacaa attgaggata tcattgccag 981540
ctatacgaaa tctttagatg tgattcatac acagttaact gatgtgatga caaatttagc 981600
atccataacc tttgttcctg gtttaaataa atatgatcct tcgtatcgca ttgttggtgg 981660
ggatttatct atcattgcct tgcagaatga cgagaaggta cttgtcgatg gtaaggtgga 981720
tatcacgact gctgtgaatg aaggaggcct acttaatttc ttcactacag tccttacgga 981780
tgtgcagaat tatggagact tagctcaaac gcaacagctg atgttggact tagagcttaa 981840
ggcgatgcaa caacaatgga gtttagtatc tgcatctttg aaattattga atgggatgta 981900
taccacagta atttctggat ttaaaaacta aaactgcaga cctgacgtaa tgttgatagc 981960
acgctgatat ccccaacggc cttctacact atagtagaag ttatttgaga tgcagcaggt 982020
agttccgaag cagaagttta ctctgtcgaa gtttgtgatt ttacgaattt taaatttaaa 982080
attcgtaaat tgcttttcga gttctgtgaa gctatcagaa ggagcttttc ttgaagtatt 982140
tcctatagat acggatgcat agggaagcac atagtcatta agatacgtag agatgccgat 982200
gcttgcagac cattctttat agcttaggtt tccatcagta gcatcgaaat agatctcggg 982260
gttggccttg ttgtaaacga tgatatagtt gatgggactg gaaccgtgac ggtagtcagc 982320

gctcacccct acaaaagaca ctccatcttt ccacaatact ttttgtaaac tcagacccca 982380
gacaattcca tagtctgact ggacttcaat gagaccatct gtaacttcag attctgcatt 982440
taaaggattt gaagtgaagt ctctgtaagc attgagaggg aggcggtagt actgcttaag 982500
tcctccgaca cgtgcagtga aggctatatc taaaagggga atggcagctg gggatgtttc 982560
ctgtagagct atggttgcaa aaacacagct cgagctgatg gagctgttgt taagatcaaa 982620
gtctacgttt ttagttgtag aggtaatggt tggcgttgtt cctgtgcctg aagtcgtaac 982680
ggaggtaatg acagggacat tggtaatatg ggcactttct gagaagacat aatctccata 982740
gaacccaaat ttgaggcttc ctgcaagagc agcaaaaaga tcgtaactat tacaaagttg 982800
gaaggcacac catcccgttt gctcaggatt cacacctggg agtactggag ctgcagggtt 982860
tccagcccct agggcatata ctgcgggaga agatacaatc ccgaagaaca tagagaagga 982920
aagggttgct aaacgtaaat gttttagcat cttgctattc atgtcaaagg ggccttattt 982980
gcttttaatg attaagtcct ctttaccaga tggtataaaa aatgtacaaa tgaaaagaca 983040
agtgacgggt aggatcttag aatgttgtag gtctcgggtc tttttttcac ctggattctt 983100
taattttctg aaagattata ggaactcttc ttttgtattt ctctgtaaga ggagagcaat 983160
gccctctttg agatcaagat tttcatagag aacgcgatag attcccgtag tgatgggcat 983220
atcgatttta tgatgtttgg cgacttggta tgcagaaagt gctgtgtagg ctccttcgac 983280
taccatccca atttttgctt ttgcttgttc gaaagtaaga ccttgagcaa gaaggtgccc 983340
gaaccgtaaa ttccgactcg actctgagaa gcaggtaaca caaagatctc caagacctgc 983400
aaggccattt agagtttctg gtttacaatc catgatagcg gcgagtttac gcatttcatg 983460
caatccccgg gtcacaaggc ctgcttttgc attgtttcca aacgataatc cctcagcaat 983520
cccacaggca atagcaatca catttttcaa agctcctccg agagcagcac ctttaatatc 983580
ggtattaggg tagactcgga aggtggggag ggaaaaagct tcgtggattt gtttgagagt 983640
ttgtgaatcg taagcgctga ctacgacaga acagggagaa ccgtttagga cctctttagc 983700
gatggaagga ccactgagat accctaaata tggagtcacg gaatctccaa gcacttcgag 983760
catgatttca ctgaggagga gtcctgtatt ttgctcgatt cccttagagg taattacaaa 983820
ggggaccgag aggtcagtga tttgttttaa ttgctctgct acgggacgga ttcctgcgga 983880
ggtcactcct tctacaatca tgaaagcatt gtggatcgct tctttcatgt ctgtagtgaa 983940
tgaaaggttt ggagagatga caacattggg agctaaagga tgacgtcgtt cttcttgtaa 984000
ttgcttgatt aggtcagggt tccgggacca tgccacaaca ggatatccct tattcgcaag 984060
tagggaggct agacaaaacc cccaaattcc catacctaag tagccgatgt gttgtttcat 984120
gaggcctcaa aaaatggttc ctcgaaaaat gctttgtttt cccaatgtag ggaagttgat 984180
gttgaaggat aataaaaatc agcttctaat tcaaatgttg tgttaggaga gagctttttt 984240
cctgtgactt tgtggaagag ttggcgctct cggtcagaga gagcttgtct tactgtatcg 984300
gggctatgat tgccctccag attttttagg ggagcaaagc actcttgtct aggatacaca 984360
agagtttgac aatggtcgct ataacaaaat agatcaaaga taaactcttc aaatttccaa 984420
gcatttttct catttaagga ggtatggcct agttgcttgg catgcttgtg gactttatac 984480
aggggcagct gttggtaggc ggcgtggcgg ataaagtcca tggagagaca gtaaagaccg 984540
atatttgcaa ggcagtattt taattttcca tcttcattaa gagcgaatct ctcgttttgg 984600
ggaatttcag agtattcgat aacagaagtt tttccagaat catgagattt tacaagaata 984660
cccacatctt caatagcagt ttgacgtaga gccgctttta tggttacctc gttgttagac 984720
atcgcatgga atccacagag ttctacgtca aaaggaagcg ctaaaggatt gtcaatgggg 984780
atcacactta ccatttctat gccagcattt ttccatttct cccatactcc tgaagtatag 984840
agaagggtag ctatacaacc gttgccattc ggaccaagag ctagagtgtc catatcttca 984900
agaaataggt ctccagatag ggtgaggagg ggccaaagtg gttggcagaa aaaatccacc 984960
tgatttggat ctaggtggaa gtagtcgttg gattcaaaaa aagaacgtgt ttgtcgggta 985020
ttcaaaggag aggtcatgaa tgctagagga agaggttgac ctgcaagttt acttgcagca 985080
cgtacctttt ctgctaccaa ctgaaacagc ggcttctttt taataggaga aacagggaat 985140
aaccctttag ggccatcgca cttcaatctt gagccttgtc cccctgccag gactacacag 985200
gcaacttttt tctctttaag tagcgtagtt cctgcatgag ctcgttctgg atcttctcct 985260
gaagaggcaa atgacgttat cggatgaaaa tctttaagaa ttgctgttgg tgaggaaagc 985320
agttgctgtt gcttacgaaa aaaatcaata tctacagagg tgagctgctg aaaaagtctt 985380
tgttgttgtt ttggagaaag ggagggccag atatctaata tatgttcttg atttatggct 985440
ttgagcttat ctgctagaga gtttacatgc atagcagagg gcgaatatac cgattcagtc 985500
attacagtta gccttacgtt cctatctctt gatccttaga atacttattg ttcttgatct 985560
tttcgatgat ttttctgcgg tttattaatt cctggagttg ttggtgatgg atttcaagaa 985620
agcgaagaga ttcgctctgt tgtttataaa gagatgattg taatgttttt aagtattcta 985680
ggagtatatc ataatgtttt atgttatctc gagaaataaa atgctcataa cgttccaggt 985740
gtaaatcagc aatcctttta tttgtagctt gaatatttcg tgatactgtt aaagctaagc 985800
gcttctgatt atgaagggta tctaaatatt tatggatttt tacttttaag attaaagata 985860
ggctcccaag tcgatctctc atgagtctgc taatgcctcg agctgtttta atgtattgtc 985920
taagtaacag taactagaca agggttgcgc taggaaggct ttgatgctgg gaaggagctt 985980
cactgcttta tctaactctt catcttgacc gggagtgtaa gctcctagat gaatgatatc 986040
taaagcttca ttatatacct taagtagtga acgaagtctt tctgcagctg cataatgatg 986100
gggaagtgct agtgcttgtg ctgaccgaga aagacttgat aaaatatcga ttggggggga 986160

ggctaaggct ttaccttggc ttgttaggaa aaagtgtcca tccaacagcg attttaaata 986220
gtctgtaaaa atatcaggat gcttcgggta atataaaatc gcatacagag ctgttatgga 986280
ccccttgtca ttgtttccgg cacgttctgt aaattcagat acatggtgaa agacagaggc 986340
ggcatattgg tgcgcagaga gtgtttctcc tcgcgctaaa gcgacttctt ggagtgcagc 986400
gatccatcgg gataaagagt ccataataaa taagacttca tggccttgct ctcgaaaata 986460
ttctgctatt gtcatagccg ctcgccctgc gattactttt gttggtgcag tctcatgggc 986520
aggtgctgca atgatgatcg tacgctgctg tttcagggca ttgctatgct tctctatgtg 986580
ttctcggact tcgcgtcccc gctctccaat taaagcaatc acattgattg tagattttga 986640
tcctaaagcg attgctgaga gcagtgaaga cttcccactt cctggctcag agaaaactcc 986700
aatacgctgt cctttcccta aggtaagaaa tgcatcgatt gctttaatcc ctgtagggaa 986760
gatctgatcg ataggttgtc tcatcatagg ggatggcggt agagacagaa gaggttttcg 986820
atgtgtttta ggaaggtcct ctttcttgtc aatagggttg ccgaaagcat ctaagactct 986880
ccctaaaaga tgatctgaga gatgtagaga gggggggcgg cgtaaaggaa ggacttctgt 986940
tccaagagct acagagtgta acggagatag ggacatgaga agtgtcgtgt gattgtgaaa 987000
gccaatcact tcagcaagaa gattcggatc tttggttgag gaaattttgc agagctcccc 987060
caggcatgca gagagcccat cgacctcaat aagatttcca gagaccttag ataatagacc 987120
acaggcacgg tagggctgcc agttatgaat atgaagtttt tctttattta gatgattcat 987180
gctgtcaaaa cagaaagtag atggtctagt tcttcgctga tttcttgtct cagaattccg 987240
ttaggggttt ctattttgaa tcctgatcgt ctacaagaag tgtcagggaa aaactcagca 987300
tgcttaatca tggggagttc gtgggtggag atccaatctg taagtgtttt gagatcctcg 987360
ggatggagaa atactttgat gggagtcaga gatcttaatg tcgtatgtct ttggagcgcg 987420
gtagagagga gcagggccag ttcttgagga ttttctagct tcttatagag aaatttttca 987480
cagatgagaa gagccagttc tagtaggtcg ggttttagct gttgtactgt tttttcgact 987540
tcactaagta ggtgaatgga tagcttgcga aagagatgaa ctaacgaatg gagttcttgg 987600
cttgccttat cattcgganc ttcgngaaga cttgtaggtt ctgtatccca aggatcatgt 987660
ggatgaggaa ggtgagattg agaaagcgaa ccaggagatt gtggtgttgt cacaagaaat 987720
acctaattct taaagtttat atttcagcac ttgatgtttg agatcttcgg gtaatctgtt 987780
taataaggct tctgctttct tagggtccag gtacgaaaga attaaagcta ttttttctgg 987840
agattccttt tgtataatct ctaacagctt tgagatattt atgcctcgtt ttattttcct 987900
aggttcgggg gagactctct cataagcgtg tcttgcgagg tagaaactag ctaaagcaac 987960
aatcatcaag cttatgacaa gaatcatact cccgattaga acttttgctg agaacgattt 988020
tttattctgt aaacgagcga aagggagggt ttctatgaca atatcatagg agtcgtcana 988080
gtttgataag aggtagtgtt tagtatgggc tacaatcttt tcggattctt ctttggttaa 988140
atgagcaata taattttcat ttaaagtgag ctgtaatgtg ggcttctctg cttttgcgtg 988200
ataccgcaag agcaaagtgt tccttgggga aaatttcccc caggtaattt tctaatgtat 988260
ggatgaacag agaatttaca gttatagatt caggaatata gaggttccct aagttgtcag 988320
atagggatat atgttctcgt ttcaacccag gaagactgct gcatagatag tctgtaattg 988380
agaagagtag tgaaggagtt agggactctt cttttcttaa agtaagaatg acagagagat 988440
gcagaggaga catgacgtca tcttctgtag acagagcaat ggcaactttt gcagatgcaa 988500
tgggatggaa ggaggtcagn ctttttctaa ctgtcttttt ttgttagaga ttctatgagc 988560
ttggggttcc ccatttgtgt gagcttcagc caattgccag aagttttttc tgttttaact 988620
tgagtagggg ctaaggatgg gttcgaggat cttccaaata aaatagcaca actcaccacc 988680
ccacctatta ataggcatcc taggggagaa atccccaaag cggtaagttt ttttgccaaa 988740
ttttgaaaaa acacaagagt ccttcataaa aataaacgaa acctatggca ttgttgccat 988800
cgtattcttc tctatcaaaa agtaggaatt attgacaatt gcatctcctc ttaggattta 988860
ggacttggaa aattttgaat gaaaatttta gagcctacat tccaaagaat ggttagagaa 988920
ggattttaag ttgctagagg ttgaatttgt tggaatcaag aacacgtgaa atgaaagcct 988980
ctacaatgat agagactcgt atttaggaat gtagggattt ctactaaaag aagttggggg 989040
gatggctacg ggtgagaaag atttaatgat gattcatcta ccttcacttg aagttcaggg 989100
tagatatagg ctcttaggag ctgccctatg gaatttaaag tagatcctaa tgacgaagga 989160
catccagaac aattcccaga ataggcaatt gtgacaataa agttttctag ggattctaca 989220
gttacttcac ctccgtccat agcaatgtaa gggccgattt tttctgctat tgtagcgcga 989280
agagcataga gcttttgttc gtgagtgaga gcttcccaat cactctggct atagggattc 989340
gcgtcttcga aatcaaggtt catgggagaa ttttgcaaag gaagcgagcc atcctctaag 989400
gggatttcaa gacattgctc aacagctgta tctaaggcat caataacaaa atggtatagc 989460
gagatgctat cttcaggaag agcgggttgg tgtgcgtgta cgcgtaagga tttatcaata 989520
tcatcaagag tcatcttgta agcttcgcta tagcttttcc cacaaacaag attgcagaca 989580
gcctccgcta gtgggattag ataagggtga ccaaagtatt ggaattttgc atccaggatt 989640
acgccatttt tcttatccac aagccaataa aaagtgacgc agttgcccat cagtctgtgc 989700
ccctgttttc cagtgaccag atgtgcttct ttagcttcag catcctcttc agaaaacgtg 989760
cccgcacaat gtggtgtaag gaattttttc ataacttttg ctgatagcga ggaccaaaaa 989820
atcattggtt ctagaggtag ggtcatgagg aacttccaag taaaggggta agatgcttaa 989880
tggcatcatg catggcacga gcaagtttgg aaaactcgag gtctttactt ctttctgtaa 989940
gagagaaatg taaggcactg tgacataaga acggggagat cccacaattt tgtaacactt 990000

gagctagggg ctggaatctc tcgtatccta aggatggata gatgccttgc tggtggagat 990060
ggaaggcaag actctctgct gggatgtcag gaattgcagc aacaacgata ttaggtaatc 990120
ggttctggac ctcggaaaat gccaattgga ttgaggggag gacgctttgt aactcttgaa 990180
ttagcttttt acacaaattg gaggtgtgga atgtgaaaag cggaagagcg gaaatacgtt 990240
cttcacacgc agtttgcatt gctgccaccg cactgaaaca taaagatgct gaagtgtgag 990300
gtggaaacca ggaagaaaag accctctcta aagatttacg aatgaagatc cccccgatag 990360
agcccatacc tccaagagct gctgaagaga aagtaatgat gtcagcgttg aggatctcag 990420
gagttagagg agctcttcct aaaatgtcag aaatatccaa gtgaaggagg attctgcggt 990480
ctttgcataa ggagagcaga ggatctaagg gttggattac ccctgtcaat ccatgagctg 990540
cagatagtga gaataacaaa ctacgtggac tcaaggtttc tataagctgt tcttcaacaa 990600
tcctaccttc gtgatttact gttacccagt cataggtagt tccgagtcct tgatggcgac 990660
ataggctatt aattaaaagc tgttgatcat gagcaggaag aatgatgtga tttcttcctt 990720
gaaacatgga cagattttct acaaggctgg ccaggactat atgaaccaca tgagggaaat 990780
gagggacaaa acgaaaaata tgagagtctt tcaatccgac caactgtcgg atactctcct 990840
ctgttttctc agctaatttt aacgcggagc cggggggtag agaaaatatg tccgagtgta 990900
gggcataact ttctttaacc cgctcagaag gaggaatagc gacttggttg tttaaccaaa 990960
aaattctggg tgcttttcga ttttgtggtt tttccattga tatacaactg gttttcccgt 991020
cggtaattct aaagatagta cctcttcctc acttaatttt tctagatcca taatcaaaga 991080
gcgtaaggaa tttccgtgcg cagaaacaaa aacgtttttt ccattttgca gttgaggcaa 991140
aatatttttt tcaaagtaag gcaaggtcct ttgtttcgta tcatagaggc tttctccctg 991200
aggaggagct gttttgtagc tgcgcctcca cagcttgacc cgctcttctc cgaactgttc 991260
ggctgtttgt tttttatttt ttccttgaag ttcgccgtac attctttcgt tgagggcact 991320
agattgatag agaggaatca tattgttttc ttcttccgca ctgtagatcc tcgacatctc 991380
ttttgctttg ggatcttcat gaacaatata ggggatcttc ttagagtgat gatttgtcat 991440
ggcgagtaag gcggtcatca gactacgtac taaggtagag gtaaagatgc agtctatagg 991500
caaattttga atagctcttc ctgcggaaaa agcctcttca attccctgtt ggcttaaagg 991560
aatgtctacc catccagaaa ataaattttt ttcattccat acagattgtc catgacgtaa 991620
taaaataaga agagccatat gttcctacta gaatttaggg tgagaagcta agcataagca 991680
gaaatcaact tttgtctagt agaaaatata gaagtaaaaa aagtttttaa gcaaagaaaa 991740
gtagaccagt attgccttgc ttaggcacaa taggtgcttg atatggggta aacgtgacaa 991800
aagttcgtct taataaattt ttagcttctg cgggagttgc ttctcgaagg aagtgtgatg 991860
aaattatttt ttctggatcc gtgactgtga atggtcgtgt tgctgagggt ccctttgttc 991920
ttgtagatcc agaagataaa gtacaggtgg gaggaacctc tgttcatctc actaagaaag 991980
tatatttcat ggtgcataaa gctatcgggt atctgtgttc ttctgagaag aagtttcctg 992040
gaactaagtt ggtgatcgat cttttcgcac atcttcccta ccgtgtgttt actgtgggac 992100
gtttggataa agagacctcc ggattaatct tagtgactaa tgatggggaa tttgcaaata 992160
aaatcattca cccctcctca ggaattacca aagagtatct tctaaaagtt agccgcgatg 992220
tctctgcaaa agatttggga aaattaatgg aggggacttt catagacggc aaacatgtgc 992280
gtcctgtctc tgtgactaaa attcgtcgtg gtacagtaaa gatcgtcgtg agtgaaggga 992340
aaaaacacga gatccggttg tttgcagatg ctgcaggact tcctatttta gagctaaagc 992400
gtatccgtat agggagtttg gttttaggag gcttgcgtta tggcgaatat cgcgagctta 992460
cagatgcgga actcgggacc tacatgaaat tgtctgacta acttgttttt ttatatacta 992520
acctgcaata gatagagccc tatgggatat gttttctatg tgatcgctgg cagtatattt 992580
cttggcatca gtttgggtgc ctattgtcaa ctctactatt cagtaaaaag cgtactattt 992640
tcttggtact tgttaacagt atatgctttg gaaaagcgtc atgcgttgct cgctctctct 992700
caacttgtag gcgaagaaga cgcccagtcc caaaaggaaa tcgactttct ctcgcagtgt 992760
gacaagctct cttggcgtgc gttcctcaaa aatagctacg agatcatccc aacatttcaa 992820
agagatggaa gaccttcttt ctgaaagagt gcagggattc ctagagtcta tagaaacgat 992880
tgcagagcac gatcgagcga tcttgtgtat cgaaaacttt tgggcaagta aaaatctatt 992940
tgattttgag attgcagctt acgaagaagc tgtggagaag tatcttaagt tgcgacagcg 993000
agcccctttg agacttgcct caaagttgtt ccgcttttta gatgttccct cgattcgatt 993060
tagtagctaa gctatcggga agattctcct gcaacactcc taggagatgg tgtataagaa 993120
gctccctggt agtctcaagg tctatgggat ggccaagaat ctcttgaagg gatgttgcag 993180
gttgtccgac atcttttaaa gcttgtttcg tagtattgcc attgaggccg atcccaagaa 993240
cgactccaag aagtccttct acagggagag tctctggaag aactccacag agcttttctc 993300
catgaaccag gacatcgtta ggccatttta tttttgcttc tgtaattcct aaatccttac 993360
ataaggcaac aacagcttct gtgcctaagc gaaagagacg tgatacatcg atgtggaggt 993420
ctgtaataaa aaaacagaag gtattaagaa gatccccctt tgaagatttc cagcttttac 993480
caaattttcc tgtgcccgca gtttgacatt ttgtggagat tacagttaat gcataaggat 993540
cccacaaatg catataggat tttgccattg tgttcgtgga gggaatttct tctatttcgt 993600
aataaataac tttcatattc tagaaattta cagtctgctc aatagtaata cgattatgag 993660
atatcataaa tattttcggt atgtgaattc ttgggttttt cttgtcgtac ttaccttaat 993720
gctattaagt gttgtggtca tttcttcaat ggatcctaca gcgatgctgg tgacctcctc 993780
caaaggcctc ttgaccaata aaagtatcat gcagctcagg catttcgctc taggatgggt 993840

cgtttttttt atctgtgcct acttcgatta tcacttattt aaacgatggg catgggtact 993900
ctactttttc atgatttgtg ctctcgtggg cctttttttt gttccgtcag tccaaaatgt 993960
ccatagatgg taccgtattc ctttcatcca tatgagcgta cagccctcag aatatggaaa 994020
gcttgtgatc gtgataatgc tcagttatat cttggaatcc cgaaaagcag atattacatc 994080
gaaaacaaca gcattccttg cttgcttagt tgtcgcactt ccgttctttc taattttaaa 994140
agagcctgat ttaggaaccg cattagtctt atgtcctgtg acattgacga ttttctattt 994200
aagtaatgtc cattctttac tagtaaaatt ttgtacagtg gtcgctacca tcggaattat 994260
aggctcgtta ttgatttttt caggaatcgt ctcacatcag aaagtgaaac cctatgctct 994320
gaaagtcatc aaggaatatc aatacgagcg actcagcccg tcaaatcatc accaacgcgc 994380
gtctctcatt tctatagggc tgggaggaat tcgaggtcgt ggatggaaaa ctggggagtt 994440
tgcaggtcgt ggatggctac cctacggcta cacagactct gtattctcgg cattaggaga 994500
ggaattcggg ttgctggggc tactctttac tctagggcta ttttattgtc ttatctgttt 994560
tggttgtcga actgttgcag tcgccactga tgactttgga aaactcctcg ctgctggcat 994620
taccgtatac ctagcgatgc acgtcttaat caatattagc atgatgtgcg ggctgctacc 994680
tatcacagga gtccctctga ttctaatttc ctatgggggc tcttcggtaa tctctacaat 994740
ggcatccctt ggtgtattgc aaagtatcta tagccatcgc tttgctaagt actaactttt 994800
cagcaaacgg agagcattta accccacaat caccgtactt ccctcatgaa gaattaccgc 994860
gagccataga ggaatgatcc ctaacgatgc aggccaggag actaaaagaa taatagcaag 994920
cgctagtgca aggttctgcg aaacaacttt tttcgtttgt tttgcttttt gaatgatcca 994980
agggagggac gacaaggagt cgtgcaggag aacaatatca gcagcttcta ttgctgtagc 995040
gcttccagct tctcccatag caatgcctac agtagcttgt gctaaagcag gagcatcatt 995100
aattccatcg ccgaccatca taatttgacg ttgtgttgca agctcgcgta ttttcgctaa 995160
cttatcctca ggggtgagat cgaaaaatac ctcagaaatt cctaaatttt ccgctgtatt 995220
ttcagcactg actttatgat ctcccgtgag catgctcacg ggatagccga gatctttgag 995280
atcttgaatg atttctttag cttgaggacg aggaatatct cgaaaataaa atagagcaaa 995340
actattccca acatacgcta gagaacaaat ctccccatgt tgcttcgcct gataaatttt 995400
ttgttctata tcttcaagat actcagaagg caccttccct agtcctgttt ctacccgacc 995460
tacaaaagct tcttgttcat tgaaatagcc acgaaccccc tctccaggaa ctgtaagata 995520
ccgatctgca ggaagcgagg ataccttctg ctccataagg taagaaacaa tagcctcagc 995580
aatcggatga gatgaagact gctctaaagc taatactgaa gggaaaaagg tctcattttt 995640
agaaccgaag tagtcgcaac ctatacaggt aagctcgcca gtcgttaacg ttcccgtttt 995700
atccatcact acagaattac aaganactaa acgatctaaa acacaccgcc tttcangaga 995760
accccatgtt tcgcacaggc attgatagca cttaaatagg caataggaat cgcaatgatc 995820
aaagcacacg gagacgctgc aataagaaac gctagggcac ggtaaaatgc actctgaggt 995880
cctaacaatg gaatggaagt aaataaagga accaaaaggg caattccaca ggcaattgca 995940
aaaatagaga gagcatatac tgaagaatat ttatctaagc gttgctgtaa cctaggttta 996000
gagttctgtg cctgaatgac taaattgata atatgagcga tggtagaatc cgatcccgta 996060
cgtaagacac gaagatcgaa actcccttcc atattatggg ctcccgcagg aacaatcgac 996120
cctggatgac aggatttagg aactttttct ccagtaagat gcatgaggtt gatagaagaa 996180
gaaccatgaa ggatttctcc gtccaaaggc acaacctcgc cacttttaat gcgtaggata 996240
tttccaacct caatcttgtt gattgcgact ttttgtaagt tgccatcttc taagacaagc 996300
caacccgtag taggagcgag ctgcttcaaa gagactaaag tgctctttgc tttccccgat 996360
accatctgtc ccaacgcttc tgaaatcgca aacaagacaa gaagcaaggc tccttctaaa 996420
gctcctccaa taaaaataga gccgaagggt gctgatgtca tcaaaatatc aatgttcact 996480
accttttgac atatgttaac taacgactta atcagagcag gagtgccagc aagaaaaaat 996540
gtaaatacta caaataagtt agagagattt tttgcatgga gccagaaact tagaagagca 996600
atcaagtagg tccctaaaga taaatacgcc gattttaaag gaagattgtg actcaatttg 996660
cgattttgtt tagatagaag gggactcgta tcttcagaca tccctgattc aaaaaatgta 996720
ttcactactt ctgcagaaaa tgaagtgaaa aacaaacggg agaacacgta gcgacctgtt 996780
ataataattt cattaatgat gagataatac tcgcggaagt gatgaagatc cctaacaccc 996840
aaaccatttc ggaaatttta tcgtttttta gaattttagc ttttaggaag gaaagaacca 996900
gaaccataag agcagaaaag acccctgcaa gagtgtagct gatgcatagg actagtggga 996960
ggaaaatggc aagcccacat agacctccta atattctaga tcctccttga atcaggggat 997020
gaggaagatc ttcttttcga atgtaaagct cttctcgaat catcgtatct agtagtaggg 997080
tagaatcgga acagacatat tctaccatct cttggaggag agggtcttta aacccttgat 997140
tctcaaacaa aatccggagt tctatttttt cttgttcaaa attctcttca atctcgtttt 997200
tctcttccag catagaacga tgcgagagtt ccatataggc ccaagctttt cttgctttta 997260
agcatccgtg gtaaaatgtc cagccaacgc ctaaagatat taaggatttt acttgaagcg 997320
cacgattcgt aggaattaag aaaaacaacg tccgaatgaa aaaaataaaa acgccagtag 997380
ataaagcatt attagctaag tgatagaaaa accctttaaa ggttgtatgg ggctcccctt 997440
tacagactct gtgcttgtct ctaacatgct ttatgtggtc ctctggagtg cgtgatttaa 997500
agtggtcatg tggatcctga gtcataattt tccttagaat ttgatgctgt ctacagtctg 997560
atgtgttctc actggtcttt gggaagaaga atctccaacc ctcctaaata aggacgaagg 997620
acttcaggaa taaccacaga gccatcagct tgttggttat tttctaaaat agctaccaag 997680

agacgtggtg ttgccagacc tgaaccgtta agagtatgaa caaactgtag tttcccttgg 997740
ctatctttat agcgtgtccc agagcgccgc gattggaaat ccgtacactg agaaatagaa 997800
gacacttcat aaaaagcttt ttgtcctggc aaccagacct cagcatctat agtcttggat 997860
nctgtaaaag acatatcccc cgtagaaagc aaagaaagac gataaggcaa cttgagctct 997920
gtcagcattt cttcaacaat actaagcatt ttttcataag caatatcatc ttgattgggt 997980
gttgtaaagg caaacatttc taccttatgg aattgatgca cccgcacaag tcctctctct 998040
tgagctcctg cagcacctgc ttctcttcgg aagcacggag tgcacgcagc atagtaaaga 998100
ggaagttctt tttctgttaa aatgtcttgg gaacgaaacc cattaagaac gacttccgca 998160
gtggggatca gatagagata ctgctctccg tcctcaacac gatagtattg tccatcaaat 998220
ttcggaatct gcccagagcc aaataaaatt tctttcttca ctaataaggg gggaagccag 998280
agctgaaatc catgagctgc ttgcttctgt aacatatacg taagaagagc ccattccaga 998340
agaacaccac gatttttata agcaggccat cctgatcctg tcgtttttgc tgctgcttgg 998400
aaatctaaga tgtctaactc ctgatttagc tctaagtgat gcttcggagg aaaggaaaat 998460
ataggaaggt ccccaacact tttaatgact tgatttcccg ctttgtcctc ggatacagga 998520
atgtcgtcag cagggtaatt aggaagatgg ctcagcaatt catgtagttg agcgtttttt 998580
tgatcgaggt gctgctcaat tttttcaaga tccgcagcaa gagtctctac ctcttgaatt 998640
aagtttgtcg cgtctacccc ttgcgtttta gctttatgta tatcttgaga taagaggcgt 998700
ctttgtgctt gtaaagtttc tgaatccgtc ttgagttgac ggacctcttt atccaaggag 998760
agtacaggtt ctaaagaaat tttggatctt tttttacgaa gtcgagtttc acattcttcg 998820
ggagtcttgc gtataatttt tatatccaac atgcttgtct cattgttaag tagaagtgtg 998880
caaaagtgag agtatatgga agatttctct gagcaacaac tcttttttat gcggcgtgct 998940
atagaaatcg gagaaaaggg tagaatcaca gcccccccaa atccttgggt agggtgtgtg 999000
gtcgttcaag aaaatcgcat cataggggag ggatttcatg cgtatgctgg aggaccccac 999060
gctgaagagc tcgccataca aaacgcctct atgcctatat caggatccga tgtctatgtt 999120
tctctagagc cttgctctca ttttgggtca tgccccccat gtgcgaactt actaatcaaa 999180
cataaggtct ctagagtttt tgttgctctt gttgatcctg atcccaaagt cgcaggtcaa 999240
ggaatcgcca tgctacgtca ggcagggatt caggtctatg tcggtattgg agagagcgaa 999300
gcacaggcgt ctctacagcc gtatctatac caaaggaccc acaacttccc ttggacaata 999360
ttgaaaagtg ccgcaagtgt tgatggtcaa gttgccgatt ctcaagggaa gtctcaatgg 999420
attacttgtc cagaggctcg tcatgatgtg ggaaaattgc gagcagagtc ccaagccatt 999480
cttgtcggtt ctcgtacggt tcttagtgac gatccgtggc tcacagcaag acaacctcag 999540
ggaatgctct atcccaaaca gcctctacgt gtcgtcttag acagtcgtgg gagcgtccct 999600
cctacatcaa aagtctttga taagaccagt ccgactctgt atgtaaccac agaacgatgt 999660
cctgaaaatt atataaaagt tttagattcc ttagacgtcc ccgttttact cacagaatct 999720
accccatcag gagtggatct tcacaaggtc tatgagtatc ttgctcaaaa aaagatacta 999780
caggttcttg tagaaggagg caccacacta catacgtctt tgctaaaaga aagatttgtt 999840
aattctttgg ttctttactc tgggcctatg attcttgggg accaaaagag acctctagtc 999900
ggagtattag gaaatttgtt ggaatctgcc tctcctctaa ctctgaaaag ttcccaaatt 999960
ttaggaaatt ctttgaaggt cgtatgggag atttcccctc aggttttcga gcccataagg 1000020
aattgaaagg aaaggatttt tagagtagca tgcttagcat cggaatccgt taatgcgagg 1000080
gagtcaatga tagaaactcg agaagaggta ggctctgcaa attttgtttc tttagaacga 1000140
gctattgagg atttaagagc aggaaaattt gttattgttg ttgatgaagc ttcgagagaa 1000200
gatgaaggcg acctgattat cgcgggagaa aaaattacag ttgaaaagat gacgtttctt 1000260
ctccagcaca ccacaggagt ggtttgcgct gctttaagcc aagaacgtct cttaagcttg 1000320
gatcttcctc ccatggttaa ggataaccgt tgccgtttta aaactccctt cactgtatcc 1000380
gtagatgctg ctcacggggt gactacagga gtttctgccg cagatagaac caaagtcgtt 1000440
cagttattag cagatcctaa gagcaaaccc gaagatttta ttagcccagg acactttttc 1000500
cccctagcaa gttctccagg aggagtgtta aaacgagcag gtcatacaga atctactgtt 1000560
gacctaatgg agttggcagg actgcagcct tgtggtgtac tcgcagaatt agtgaacgaa 1000620
gactactcta tgatgcgatt gcctcaaatt ttagagttcg caaggaaaca taatattgca 1000680
gtgattcccg tgacatcgat cattgctcat cgcatgctct ccgatcgttt ggtttctaaa 1000740
atctcttcag cacgcctccc tacaatttac ggagacttta cgattcatgt ctatgaatcc 1000800
ttattggaag gaatgcaaca tcttgctttg gtaaaaggca atgttgctgg aaaaagtaat 1000860
gtcctcgtac gtgtccactc agagtgtgtc acaggagata ttttaggatc taagcgctgt 1000920
gattgtggag aacaattgag ttcagcaatg tcctacattg ctgagaaggg aactggtgtt 1000980
cttgtttact tgcgagggca ggaaggccga gggatcggtt tgggccataa agtacgtgct 1001040
tatgctttgc aagataacgg ttatgatact gtagatgcaa acttagctat gggatttccc 1001100
gtagactcaa gggaatatgg cataggagca caaattcana ttgatctcan gttgacaacc 1001160
ataaaattaa tcactcataa tcctcaaaaa tattttgggc ttcaaggttt cggacttagc 1001220
atcacagaaa gagttcctct tcctgttcgc atttctgaag acaatgagca gtatttaaga 1001280
acaaaacagg aacgtatggg acattggcta gatctcccat gctgtaacaa tcgggtacaa 1001340
taattttgag gagtatatga aaacattgaa aggacatttg tctgcaaaga atctacgtat 1001400
tgctattgtc ggctcctgct ttaatcaagc tatggctgat gccctagttt ctggtactca 1001460
ggaaactttt ttgaagtttg gagggagcga ggacggcttg atgactatcc gtgttcccgg 1001520

agctttgag attccctgta cgatcaaaa actttatct tctgaagaa agttcgatgc 1001580
tattgttgca tgcggcgtcc taattcaagg agaaacagac cattataacc aaattgtaaa 1001640
tcaagtagcg gcaggtattg gtgctctctc tttggaattt tgtcttccca taaccttgtc 1001700
catagttgca gctccttctg cagaaatcgc ttggcaaaga tcaggtatta aaggacgtca 1001760
tttgggagtt tctgggatga cgacagctat agaaatggca acgttattca ctcaaatcta 1001820
gttcttgtaa gaacgtatac gtcccacaaa atttgtggga ctcttccttc tacagattga 1001880
tgctgacagc tccatttaaa tggatgcgaa ctgcgatact ctcagcaaag acatccaagc 1001940
ctcccgaaga caatgtcgtc tcagaaaaat ctaacacagt cccttgcggg agaagttgtt 1002000
tatttagcat ttctctatga gaacgtaagc tttttaccaa taaggatagc attgtcgagt 1002060
ataattgacg tagacgattt tctggactta ccgtagatcc atgagtcgcg accgaaagtc 1002120
ccgcaactaa actgggaaga ccacgaaatg ttaaaggaat atgctctcct tgtactgtat 1002180
agaaactgtt aacaagattc tcagcttcat cagaaggtaa atttttaaaa ctccacgcca 1002240
aagtactcgc ttccgacgga gagagattca ttaatgttgc taagcgaacc aaagtcatgt 1002300
ccgttcgaga ggttccctca aaacctaaac gattcgctcg ataagtaaaa taacgagaga 1002360
atgcctcttc tatagtcgca tgctgctgta acatggaaag tagaggagca aagtttatat 1002420
agaactcttg agaacagttc ttgtctccac aggtatctaa gcaatacagg tacaaatcta 1002480
agatcgcaag agaccgacgt gttctccaag cagaaagcag ctgcgcacta ggaacctctg 1002540
ttaagttcag tagagaatcg tagttgccca aacgtcttag agataacaag acttcttgct 1002600
gttctggaga aagcctcgca taagaaggac aaagttcagg gaaagtacgc aacacatgta 1002660
acgcctttgc gtaaaagctc tgtgggcacg agtctgtgaa ataaggctta aacgtggtga 1002720
cagcttttgc agagcagcct acatgtctga gcacgatcgc tgtttctaaa attctcaagg 1002780
tattatcgaa actcttagga gaatttaaaa agaacaataa ttgtttgtgc gccgtctgga 1002840
attgcttaaa tgtcatacct ccttcttcag aagggaggat ctgagagagc tgcatgtaag 1002900
attgacgaga cccctgtatc aaaagatgta ggtgaatcat actgcgaatc gcaatgtcta 1002960
aagcggggac tttttattta aagagctttt cagaataggc catatcgata ggagtcgatg 1003020
tgagaagagg agcccctcca gattccgtaa gccaaagaag ctcaggatac tgacgtagtt 1003080
tttgttctat ccaaacctca gaaccaattt taacctttgt ctgcgagttc gtctgtagag 1003140
atccttgggg gccagcatag ccgatagaat tgcatcccat aagtaaagct aatgggaaaa 1003200
ccaagacttt gattttaaga atttttaaca tggaagcctc ttctctatca cgttgctttg 1003260
ttaatatctt taaatttaaa gagattaact aaaaacccca ttttgataac aaaatgccca 1003320
ataacaaatc aacttgagaa acgcaaggac gttttcctcg gaagaattaa agattccaac 1003380
ataaggattt agttatgtta ataacttaat aatttatttt tgtaataaat aggccttttt 1003440
aaaaggaaaa tgtgttataa tctttaaaat taaattttta ttttaactaa aaataatgaa 1003500
aagagtcatt tataaaacca tattttgcgg gttaacttta cttacaagtt tgagtagttg 1003560
ttccctggat cctaaaggat ataacctaga gacaaaaaac tcgagggact taaatcaaga 1003620
gtctgttata ctgaaggaaa accgtgaaac accttctctt gttaagagac tctctcgtcg 1003680
ttctcgaaga ctcttcgctc gacgtgatca aactcagaag gatacgctgc aagtgcaagc 1003740
taactttaag acctacgcag aaaagatttc agagcaggac gaaagagacc tttctttcgt 1003800
tgtctcgtct gctgcagaaa agtcttcaat ttcgttagct ttgtctcagg gtgaaattaa 1003860
ggatgctttg taccgtatcc gagaagtcca ccctctagct ttaatagaag ctcttgctga 1003920
aaaccctgcc ttgatagaag ggatgaaaaa gatgcaaggc cgtgattgga tttggaatct 1003980
tttcttaaca caattaagtg aagtattttc tcaagcttgg tctcaagggg ttatctctga 1004040
agaagatatt gccgcatttg cctccacctt aggtttggac tccgggaccg ttgcgtccat 1004100
tgtccaaggg gaaaggtggc ccgagcttgt ggatatagtg ataacttaac cttctaaagc 1004160
tctctacaac taagcttcct ttccccaaaa caatagggaa gggaagccca gaagtttttc 1004220
tctttggcct tcctgaattt tcataatcaa tcgcaacagg agagacaatg ttatatttta 1004280
tagaacagct aaataaactg agtacgtcgt tttgtgtatt ccctatgatc ttattgttag 1004340
gggggttctt gacatggaaa ttacgcggtt tacagttcca cgggttaaag ctcggcttta 1004400
acttgatgct tcaaaataaa ttggatgata gttcatcaaa agctaacgaa gtttcttcgt 1004460
acgaagctgt agccggaatc ctagcaggaa attttggcac gggaaatatc gccggaatgc 1004520
tgtcgcctta gcttgtggag gtccaggcgc cctggtctgg gtctgcttgc agccctcctc 1004580
ggagctatcg tccaatatgc tggctcctat ttaggttcaa aatataggaa acctgaagga 1004640
aatacaggag aatttatagg aggacccata gcctgcctcg cttttggcat gcgtaaaaaa 1004700
atactcgcag gattctttgc tttattcact atcatgacag ccttctgtgc aggaaactgt 1004760
gttcaggtaa gttgtatcgt tcctctctgt gcagaaggaa ctccaggaaa actcctcgtt 1004820
ggaattctac tagctctcgt agtgatcccc gtgttagcag gaggaaataa ccgtatatta 1004880
agattctctg ctcgtgtgat tcccttcata gcaggatttt actgtatttc ttgcgggatc 1004940
attctcttcc aacatgcctc ggctattctt cccgcaatca aactgatatg ctcttcagca 1005000
ttcggcatta aagccggact cgctggaatc ggaggctata ctctttcgca agtcatctct 1005060
acagggatta accgtgctgt catggctaca gattgcggca gcggaatggt atctattttg 1005120
caagcaaata caaaaagcaa aaatcctgtt gtagacggac tcgttactct agtcccccca 1005180
gtcattgtga tggttgtttg ctctattaca atgctcgttc taattgtctc aggagcttac 1005240
agctcaggag cccaaggaac tctaatggtc atgagtgcct ttaaaaatag cctcggctct 1005300
ctaggtagtg tgattgttat tctcgctatg gccctattcg gatatacaac aatattgaca 1005360

tggtttgctt gcgcagaaaa aagtttacaa tatatgatcc caggaagacg agcaaatcta 1005420
tggttgaagg ctatatacgt cttgatcatt cctctagggg gtgttatcga tatgcgtatg 1005480
atttgggcct tatctgacac aggtttttct ggtatggtca ttctgaactg catagctcta 1005540
atcgccttac tgaaagatgt actatccaca aaccgcgatg ttgctttgct taaagaacgc 1005600
gagtgctctg ttgcagatcc tgtgcgtaat ctagatgctt aaaggagaag aatcatgcaa 1005660
ttattgtccc cagcgtttgc ttatggcgcc ccaattccta agaagtatac atgccaaggc 1005720
gcagggattt cccctccctt gacttttgta gacgtccccg gtgcagcgca aagtcttgct 1005780
ttgattgtcg aagaccctga tgttcctaaa gaaattcgta gcgatggcct gtggatccac 1005840
tggatagtct ataacctatc caccacaatt actaatcttg ccgaaggagc tgaaatcttc 1005900
gctgtgcaag gattaaatac ttctggaaag cctgtctatg aaggtccctg tcctccagat 1005960
aaacagcacc gctaactttc tttactctat ttgctcttga cgtagttctt ccagaagaag 1006020
aaaacgtcac ccgtgatcag ctatatgaag caatggaatt ccatattata gaacaagcag 1006080
agttaatggg aacttacgag aaaagttaga atttattcct cttcaggaat aaattcctct 1006140
cgcttcttcc tgtgtttcca atatagcggg ccatagtgat agcaaatttc ttggcccgca 1006200
tatatcggtg ctattgtcct gataatcacg tgaaacaatc cctcactgaa aaccccgata 1006260
gcttccgcat taggttgttc gctgtgattg ataaaacgtg tgacgtttcc ctgcttccca 1006320
ctatcaatag taaaatatct taaagtgaat aaaggcatcg gataacgaaa acaataatcg 1006380
ttctcatcca tccaaattgc ctgccggtga cgcaaaattc ccgtgtattc tccgatatat 1006440
gtccaaggag caatttcatc acgagcaaaa actccatacc ctacgtgcgc attaatccaa 1006500
cagacagaaa ccggtggagc tggaggacat aatagatctt gcttgtgtag ttttcctaac 1006560
cacttggcta gaggagaaat caaacgacgt ttttccgatt tgtgacaaag agtctcaatt 1006620
tgctgttcta ccttccagtt agaaaaaact aaagaaggaa gaaaccgaaa atgaagaagt 1006680
tcactcgctc ggtctaaaga atacggctgg ctatctcgcc aatcattgtt caaagaaatg 1006740
tgtatagaag aacaaggttc cgtagttacc gtggacatgc aacctcaaga cgagaaaagt 1006800
atattggaga tgtaatgccc tgcgctaatg caatttctgg tgctatagaa gtgatagaag 1006860
ctatcgattc agatactgta gaaagtgcta gctccgcagt gttacactcg gtagacaggt 1006920
gagcaagata taacttcttt aacttcggag tgataatctt ttgtaaaagc tgaccacact 1006980
cttggttaga aatatgacct aatttactca atacacgctt tttgtaaaca tcaggacgtt 1007040
gagattgacg taccaattca ggggaatgat tggactcaat taataagtaa tcacaatcat 1007100
agagttcatg tgtgatccaa gaggtgaccc aacctaaatc tgtgcaaaaa cccagtttct 1007160
cttcgcgata atgaaaaata aaagccacag gatctacagc atcatgaggt acattgaacg 1007220
tctgtacttc gagatcttga aaacaaaatg aagaccctgt ggaaaatatt ttgaattctg 1007280
gatggctatc tagtagatgg cataaagcac gagccgtctc caagttgcaa acaatgggag 1007340
tgttatacgc cttaacaaaa cttttaatcc cggagatatg atcagaatgt tcgtgcgtaa 1007400
caaaaattgc ctgaatatct tcaggatcga tattcataga gagtaattcc cgagtgacga 1007460
cttgcttgct cactcctaaa tcaataagaa tcttacaaga atccgtgcct agataagcag 1007520
aattcccttt ggatccagaa gctaaaggga aaaaaccttg catgctaacc ttccagagga 1007580
ttctgtatca ggatttgacg aggcttagct ccttccgaag gcccaataat tctagcttct 1007640
tcaagttgat caattaaact agctgctcgg gcataaccaa tttttaattt tctttgaagg 1007700
aaggtggttg aagcattccc cgtctgtaaa attaaggttt tcgcctgagc aaataaagga 1007760
tctttttctc cagagttatc agaatcagaa tcatcaaaag catggaaaga aggaatcaca 1007820
tattgtgtag ggaatctaga acatagatct tgaatgactt tgtttatgtc ctcatcacaa 1007880
atgtaggcac cctgagctcg tatagttcca aaaacagaag gaagaagtac aagcatatca 1007940
ccatttccca tcagattctc tgcaccaggt tcatcaatga taatctgact attcacttta 1008000
ttggagacct taaaagaaat tcgagagggg aaatttgctt taattaaacc tgtaattacc 1008060
tctcttgaag ggcgttgcgt tgctaaaatc agatgaatcc ctacagctct agccatctga 1008120
gctaagcgaa tgataggagt ttcaatatct tgggatgacg agagaagcaa atcagataac 1008180
tcatcaatga tccctaccat aaagggcata gtttcacgaa tctccctgtc gtaagaagcc 1008240
tcaatcgttt tattgcgagt gcgggagtta aacgcttgta tattgcgcaa acccaaatat 1008300
ctcaaaatct cataacgaga ttccatttcc ttaactaacc aaactaaagc attgtacact 1008360
tctcttgatt cggtaatcac aggagataac atatgaggta attgcgaata cccagtgagc 1008420
tctacttttt taggatcaat aatgacgagt ttaatttcag aaggtaaggt cgtcataatc 1008480
atagacatga caatcgtgtt aatacataca gatttcccag atcctgtagt accagcaata 1008540
atgagatgag gcatcgtagc taaatcagcc caaagattgt caccatttgc tttcttccct 1008600
aacaacaagg gaatctgcaa tttacgattt gtcttctgat agtcttctaa taaatcacga 1008660
aaattcacag cttgaggaaa aggtgtggga atttcaatac ccacagcagc tttccccgga 1008720
atcggagcaa taatccgtat gctcgaagct tggagtttta aagctatatc attttctaaa 1008780
gatttgattt tctgaacctt aactccagaa tgaggtaaca cttcaaaagc tgctaatgtc 1008840
ggtcctgaac agatgttgcc aaggtccgca tcaattccaa aactcgtaag agtctgtttt 1008900
aaaatcagag cttttctctc tagctcagct tgtaaggact cgggacgagc ttctctgttt 1008960
ttactaagta aatgatattg cggaagatca ttttcaggga ctgctaaact ttttaattta 1009020
ggaagagccg ctctttcctt gcctttggat tccctcacta cagtgggagt cgaagatagc 1009080
gcaattgttt tcccttcttt cgctttattc tcttgaggct caacaaattt tgtcaggaaa 1009140
cgcttgcaag gatgaggagt taggaaaaag gactctttct tagaaccggg gatctcttcc 1009200

tgaggtaagg gagaaataga cccatcttaa atgatagtct cagaaactcg acgtggcgaa 1009260
ggttgcgatt tcgtacaaga aaaaggattc ttggaaacaa aaggaaccga aggcttagga 1009320
agatagtttc gcctatttat taattttttt aaatttttga aacaagtttg gaaaaaagag 1009380
caaaaagcct ttttgacccc gtcttgaaag gtnttttttt ttaataaagc aatgcctgcc 1009440
gcaaagataa aggacggaga atagcatcac gaatccaaaa attagagcag ttcctacaga 1009500
cccaattaaa tgttttagac aaaaagattg gccttcgtaa aacagataga aagggattcc 1009560
acccacataa gagacaggag gaatattccc taaaataaac tttggaagac gtgtatctaa 1009620
taaagcagga agcgttccta ctggagataa catggataag agaatcgcag aacaaaaggg 1009680
gagagaaaga aatgccgcag ctttatagaa gaaaagcggt cgaggagtcc ttctgaagta 1009740
caaaaaagat aaccaaagaa aatatanagg gataaaaaat gccgcggcac caaagaaata 1009800
aagtagaaaa gaactaaatg accaacctaa tagacctatc caattctgtg tgcacggctg 1009860
atctctatga aaactccata gactgagacc agaaaaacac gcgaaaaata aatataaact 1009920
ggctttagct gctaaaggca aagtcggaag acgagggtgc ctgctctttt ttctttctct 1009980
tatcatgggc tacataagat tctaagtatt gtcgagcttc gttatagatt tcgttatatc 1010040
aagaaaaaca aaagaaacaa agaagataaa gagggcgaac gacggggctt gaacccgcga 1010100
ccttcggaac cacaatccga cgctctaacc agctgagcta cgttcgccaa accatataaa 1010160
attaaggaaa atattagctt cagtctgaaa tttagccaag ataaaaaggt cagaatagga 1010220
aatctcaatg ttctcactaa gataagtagt atgaatttct tgaaaaacag caaacataac 1010280
gttctttgaa ttcaaataga aaaccaggac agggaaaacc ggagctcatt gcgaatagaa 1010340
atagggttta tgaataaaaa tgcattattt ttttcgcgaa attttagccc ttacagcccc 1010400
ttctggagca gaaaatggtt ttttgcaaaa aaagaaaaaa ataaaaagca aacgaaatgc 1010460
gttgcgaaaa aaatgccatt ttttatagga tgttgctttc ttcttaagtg aaacaaacaa 1010520
agaagaagag tccacacttc gatgtggata cgcaacgaaa gtttgagtat ctgtagttta 1010580
taggttttta aatttttctt cttaacaatg caaatgagat agaatgcagg ccagtataaa 1010640
atgcttgtga ggatcttttg aaggatctgt taattttttt tattttttct gagaatttga 1010700
tcttagttca gattgaacgc tggcggcgtg gatgaggcat gcaagtcgaa cggaataatg 1010760
acttcggttg ttatttagtg gcggaagggt tagtagtaca tagataatct gccctcaact 1010820
tggggataac ggttggaaac gatcgctaat accgaatgta gtgtaattag gcatctaata 1010880
tatattaaag aaggggatct tcggaccttt cggttgagga agagtttatg cgatatcagc 1010940
ttgttggtgg ggtaaaagcc caccaaggcg atgacgtcta ggcggattga gagattgacc 1011000
gccaacactg ggactgagac actgcccaga ctcctacggg aggctgcagt cgagaatctt 1011060
tcgcaatgga cgaaagtctg acgaagcgac gccgcgtgtg tgatgaaggc cttagggttg 1011120
taaagcactt tcgcctggga ataagagaga ttggctaata tccaatcgat ttgagcgtac 1011180
caggtaaaga agcaccggct aactccgtgc cagcagctgc ggtaatacgg agggtgctag 1011240
cgttaatcgg atttattggg cgtaaagggc gtgtaggcgg aaaggaaagt tagatgttaa 1011300
attttggggc tcaaccccaa gtcagcattt aaaactatct ttctagagga tagatgggga 1011360
aaagggaatt ccacgtgtag cggtgaaatg cgtagatatg tggaagaaca ccagtggcga 1011420
aggcgctttt ctaatttata cctgacgcta aggcgcgaaa gcaaggggag caaacaggat 1011480
tagataccct ggtagtcctt gccgtaaacg atgcatactt gatgtggatg gtctcaaccc 1011540
catccgtgtc ggagctaacg tgttaagtat gccgcctgag gagtacactc gcaagggtga 1011600
aactcaaaag aattgacggg ggcccgcaca agcagtggag catgtggttt aattcgatgc 1011660
aacgcgaagg accttacctg gacttgacat gtatttgaca actgtagaaa tacagctttc 1011720
cgcaaggaca gatacacagg tgctgcatgg ctgtcgtcag ctcgtgccgt gaggtgttgg 1011780
gttaagtccc gcaacgagcg caacccttat cgttagttgc cagcacttag ggtgggaact 1011840
ctaacgagac tgcctgggtt aaccaggagg aaggcgagga tgacgtcaag tcagcatggc 1011900
ccttatgtcc agggcgacac acgtgctaca atggttagta cagaaggtag caagatcgtg 1011960
agatggagca aatcctaaaa gctagcccca gttcggattg tagtctgcaa ctcgactaca 1012020
tgaagtcgga attgctagta atggcgtgtc agccataacg ccgtgaatac gttctcgggc 1012080
cttgtacaca ccgcccgtca catcatggga gttggtttta ccttaagtcg ttgactcaac 1012140
ctatttatag gagagaggcg cccaaggtga ggctgatgac tgggatgaag tcgtaacaag 1012200
gtagccctac cggaaggtgg ggctggatca cctccttttt aaggacaagg aaggttgttt 1012260
ttaacaaccc gactaggttg ggcaagtatt ttatattccg cattctattt cttttgcatt 1012320
gttaaggttg ttttcaaaac attcagtata tgatcaagta tgttatgtaa ataatcatgg 1012380
taacaagtat ttttcacata taataataga cgtttaagaa tatctgtctt taggtgaagt 1012440
taacttgcat ggatcaaaaa tttacagacc aagttgttaa gagctattgg cggatgcctt 1012500
ggcattgaca ggcgatgaag gatgcgttta cctgcagtaa tcttcggtga gctggtatag 1012560
agctatgacc cggaggtatc cgaatggggc aacccgatag actaatagtc tatcattata 1012620
tgttgaatac ataggcatat aaggcgacac ccgctgaact gaaacatctt agtaagcgga 1012680
ggaaaagaaa tcaaagagat tccctgtgta gcggcgagcg aaaggggaac agcctaaacc 1012740
atatttttaa tatggggttg tagggtcgat aacatgggat cttaagtttt agttgaatac 1012800
ttctggaaag ttgaacgata cagggtgata gtcccgtaaa cgaaaaaaca aaagacgcta 1012860
atcgatacct gagtagggct agacacgtga aacctagtct gaatctgggg agaccactct 1012920
ccaaggctaa atactagtca atgacctata gtgaaccagt actgtgaagg aaaggtgaaa 1012980
agaacccttg ttaagggagt gaaatagaac ctgaaaccag tagcttataa gcggtcggag 1013040

acctataact cttcggagta atggttgacg gcgtgccttt tgcatgatga gccagggagt 1013100
taagttaaac ggcgagatta agggatttac attccggagt cgaagcgaaa gcgagtttta 1013160
aaagagcgtt ttagtcgttt gatttagaca cgaaaccaag tgagctattt atgaccaggt 1013220
tgaagcattg gtaagacttt gtggaggacc gaaccagtac atgttgaaaa atgtttggat 1013280
gagttgtgaa taggggtgaa aggccaattc aaacttggag atatcttgtt ctctcngaaa 1013340
taactttagg ntagcctcgg ntattaagtt ttttgggggt agagcactga atttctaggc 1013400
ggggcctacc ggcttaccaa cggaaatcaa actccgaata ccaaaagcga gtccgggaga 1013460
tagacagcgg gggctaagct tcgttgtcga gaggggaaca gcccagaccg ccgattaagg 1013520
tccctaattt tatgctaagt gagtaaggaa gtgataattc taagacagtt ggaatgttgg 1013580
cttagaggca gcaatcattt aaagagtgcg taacagctca ccaatcgaga atcatcgcgc 1013640
caataatgat cggggctcaa gcataaaacc gacatcgcgg gtgtatatta tgtatacgcg 1013700
gtaggagagt gtagtattca gcagtgaagg tataccgaaa ggagtgctgg agcggatact 1013760
agtaaagatc catggcataa gtaacgataa aggaagtgaa aatcttcctc gccgtaagcc 1013820
caaggtttcc agggtcaagc tcgtcttccc tgggttagtc ggcccctaag tcgaggcaca 1013880
aatgcgtaga cgatggagca acaggttaaa tattcctgta ccacctaaaa ctttagcaat 1013940
ggaatgacgg agtacgttaa gcacgcggac gattggaaat gtccgtatca caatgagact 1014000
ggttagtagg caaatccgct aacacaaggt cgggttgtgg ttaaggaaaa tcttcggagg 1014060
aactgatagt gtggcgcaag gctttcaaga aataatttct agctgttgat ggtgaccgta 1014120
ccaaaaccga cacaggtggg cgagatgagt attctaaggc gcgcgagata actttcgtta 1014180
aggaactcgg caaattatcc ccgtaacttc ggaataaggg gagcctctta aggtgattac 1014240
ccagcggtat gagcctcggg gggccgcaga gaaatggccc aggcgactgt ttaacaaaaa 1014300
cacagcacta tgcaaacctc taaggggaag tatatggtgt gacgcctgcc caatgccaaa 1014360
aggttaaagg gatatgtcag ccgcaaggaa agcattgaac ccaagccctg gtgaatggcc 1014420
gccgtaacta taacggtgct aaggtagcga aattccttgt cgggtaagtt ccgacctgca 1014480
cgaatggtgt aacgatctgg gcactgtctc aacgaaagac tcggtgaaat tgtagtagca 1014540
gtgaagatgc tgtttacccg caaaaggacg aaaagacccc gtgaaccttt actgtacttt 1014600
ggtattgatt tttgatttgt tatgtgtagg atagccagga gactatgaac actcttcgtt 1014660
aggagggtgg gagtcattgt tgaaatactg gtcttaacaa gttgggagtc taacattact 1014720
ccatgaatct ggagaatgga cattgccaga cgggcagttt tactggggcg gtatcctcct 1014780
aaaaagtaac ggaggagccc aaagcttatt tcatcgtggt tggcaatcac gagtagagcg 1014840
taaaggtata aaataggttg actgcaagac ttacaagtcg agcagagacg aaagtcgggc 1014900
ttagtgatcc ggcggtggaa agtggaatcg ccgtcgctta acggataaaa ggtactccgg 1014960
ggataacagg ctgatcgcca ccaagagttc atatcgacgt ggcggtttgg cacctcgatg 1015020
tcggctcatc gcatcctggg gctggagaag gtcccaaggg tttggctgtt cgccaattaa 1015080
agcggtacgc gagctgggtt caaaacgtcg tgagacagtt tggtctctat cctttgtggg 1015140
cgcaggatac ttgaaaggag ctgttcctag tacgagagga ccggaatgga cgaaccaatg 1015200
gtgtgtcggt tgttttgcca aaagcatagc cgagtagcta cgttcggaaa ggataagcat 1015260
tgaaagcatc taaatgccaa gcctccctta agataaggta tccctatgag actccatgta 1015320
gactacgtgg ttgataggtt gggtgtgtac gcacagtaat gtgtttagct aaccaatact 1015380
aataagtcca tagacttggt ttttatcata taaaaagctg aataagcttt tttgttgaat 1015440
tagtcgattt atgcaagttt actaaagact cttcttaagc gtctattagt atacgtgaaa 1015500
atacgttaca agatttagct tggcgatact ggagaaaggg atacacctga taccattccg 1015560
aactcagaag ttaagccttt tatcgctgat ggtactatac acaagagtat gggagagtaa 1015620
gtcgttgcca agctttctat tatatgtttc ttaatacata attaagaatt tcaaaagagg 1015680
ataatttttt tatcctcttt ttttttgctg ttttttgata aacctattta cccagcttgt 1015740
ttcatattta aaagtgattt cattgttttt aaaataaaaa attcgctgta cacatgcccg 1015800
ttcctataga taattcctct cgcaacctac aagaagttcc agaaagccta gaagacctcg 1015860
aacaacacgc agaagaatct cttactcatc aaaagtgcag aaagcagttc tttgcaactg 1015920
tctctagcct cctcagcaat ttctagtaga gtagaacaac tatcttccct cgtcttagga 1015980
atggaaaatt cagatttctc ctctttaaga gacgttccta tcttctcagc tatctacgaa 1016040
tcttcaacac acacacctgt ccccactcct ctagttggcg tgggatatat caacggaagt 1016100
caatcaggat actacgatac acaaagagaa tctcttcacc tcagccaatt gttaggaagc 1016160
cgaagagttg aagttgtcta taaccaagga aacttcatgg aggcctcttt gctaaatctg 1016220
tgccccagaa gacctcgaag agatccctct ccaatttctt tagctctatt agagctctgg 1016280
gaagcatttt ttttagaaca ccccccaggt agcactttta atccaatatt tttttggtaa 1016340
cggagctttc tatgttcgcg aagctctccg cctaactccg catgcacaga atatagtgct 1016400
cgttggtatc tgtccttctc tatatccaga acatcctcgc tccttttatt atcgtgtttc 1016460
tggagatata ggctcccgat tcgacgatag aggatttgta aactctggag tcgaaaccct 1016520
gccatactct tcaggcagct ttgggatttt ttggatctcg tttacggatc ccacatttaa 1016580
ttttgctatc gtaaatacct ttatgcgaac tgcagggatc aatgaagtct ctagacccat 1016640
gacacaagat acagaaactt cattgataga aatgagagac ctaagtgaac aacaagaagc 1016700
gaataacaca gattctttag agcaagaaga gagcttaatg ggtattgtag gacatactgt 1016760
gggaggagtt tccatgaccg tgacctccag tccaaatatc ttttatcgta tacaaacact 1016820
tctgggactg ccagagactc ttgcagaagc tgaagaaaat cctaccttcc caaattctac 1016880

tatagatagc cttgcagaaa taatgatgaa cctcgtaagg atctctgatg ctgtctctat 1016940
tttctggatt tttcctatcg tagatactac atataatgga gttttattag ccgtctgtat 1017000
cggcttcttc ggaatcaatg ggatttgttc cacgttcctt atgcttacga atccacgctc 1017060
tcgtcgagat agatggagga atttacgcat catggttctt tgctatcgtt ctttgggaag 1017120
cggaatgaat ctctttgatc ttagcaataa tgtgcgcatg gcagcacgta ggcatgtgac 1017180
atcatgtaca gtagctctct atgctatggt cactctattt ggatggacag tagcaataca 1017240
agatgctttg caatatggtt tccctagcgt tcgggatgcc ttctatagat attgcttacg 1017300
ccacagatat tgcttaactc aaagaaacga agactctctg caaactacag gaacgcgctt 1017360
tcaggttacc cgtacacatc tagaagatca acagatggtg gcttctattt tgaatttgag 1017420
tgtttttggg ctcttttttg gattcgtagg gctaatgacc acgtttggag gattagaaat 1017480
ctcaccatct tgtcggtggg atgcagcaaa taaccgaacg gtaggtattt tttagactcc 1017540
aatgttacct tttctcaagc tttaagtagt ggtcgtgtct atgcagtcag ccaatcggtg 1017600
catatgatct tttgcttctt ctctattatc ttatatacga tgtctctact ttctcttcta 1017660
agaagacgtc gtcgtcaata aagaaaaacc agatccacta gcttccaaaa tcatcaagaa 1017720
taatcgaact gcgctcgacc ccatagtcac caagaagttt aagaatactg ctattgtgta 1017780
gcggtggacc acatacgtag taaagatagt cttcaggatt atctaaccta ctcaattgac 1017840
ctagattaaa tgcccgaaat aggaagtttg tctttgtagg gtcatcttta tcccaacctg 1017900
cagcaatatc ttcaggaaga ggctcagaaa ggactaggtg ataatggaaa ttgggaaact 1017960
gtcgttctaa attttcatat tcttcttggt aaatgttctc ttttaatgag cgcgccccat 1018020
accaaagatc aatttctctc ttggaatgct tgtttaaaag taaatctagg atgtggctcc 1018080
taccaaaaga tgaacctgct cctccaatta ggaagattaa agggcggtcg tcatctttca 1018140
taaaagattc tccataaggc ccagaaactg taattttgtc tccaggtttt aaggagaaca 1018200
cataggagga acagactccc caagggatct ctgaattagg ctttccatta atgaaaggag 1018260
gcgtagcaat acgtatatta aacttaattg taggaagctc ggcaggatag gaagctaaag 1018320
aatacgcttt gttggcagaa tccgcaggaa gttgactgtt gtctataact tgatcaaata 1018380
gatgaaagtg ttcccagtcg ctgtaatact caggagccat agtttgcttc caatcggaag 1018440
agttcgtttt atagctcggt actgtaattt gtaagtagcc cccaggctta aagggaatag 1018500
gtttatttgg gtctacagca acaacaagtt ctttaataaa ggtagccaca ttgtcattag 1018560
agataacagt gccctcccaa gaagaagcat ttaaatatct ctcttcaatt tctaaactca 1018620
tgtcgtgctg aactttgcac tggcaggaaa ggcgccagcc ttcctcgagt tgtctttttg 1018680
aaaatgtaga acggtccgtt tctaaaggct catcagcatt tttaacaacg cgaactttac 1018740
attgcttaca ggtggctttt cctccacaag gggagggaat aggaattcct gaacttaata 1018800
atgaaactaa gagagtctga ccactttcga cagtttttgt cagttcttca ttgtcgttaa 1018860
tctttaattt acaagggtgt accttaatga aaagcttgcg agatagaagg atcacacctg 1018920
caagaatcac gccaatggcg caaaagataa gactagcaat acaaatgaaa tagaggcctg 1018980
aaagccaagt cataacgata aaaatcccag actttatgaa aagctcatag agtcaaatta 1019040
taacttagcg cataaaaaag caataaacct taagacaata aataagaaat cccacgatnt 1019100
taatcgtggg atgaaagtct caaaagaaat tccttatgat ttgttatcgt taggcttgag 1019160
gatttcagaa atagctcctt ttaaaacttc tacttttcca gaagcaatat ttaaaattac 1019220
cgtgtgctca cggatatcgt caacagtgcc gatgattccc atggcagtga ctttatctcc 1019280
tttagcgaga tcattcttac gcttttccat ggcctttctg cgtttttgtt cgggacgcca 1019340
taagataaaa tagaaaaata gaatagcaat tgccaacatc acggcaggtt gcacaaaagt 1019400
attttttgat tgagctgctt cttcttctgc aaacaggggc aaagagctca gtaaacaata 1019460
gaaaacatgt cactatacga gaaagcatgc aatcacctta acttgggtta attcgaagta 1019520
agattatcat aataggaagc gagcattcaa tgcttttcct agagatcgat ctctctttct 1019580
agtaaaataa tattttctag gtgcgtcgaa tagggaaatt ggtcaatagg ctgcatcttt 1019640
tttatgcgat atcccccaga gattaggtcc gcgcactctt gaaactgtgt tttagggttg 1019700
caagagatat agacaatttt tggagatcct atacgtaaaa tatatttaag tactttactt 1019760
tgcataccac aacgtggggg atcaataata atgacatcag gagctttaca attttcattc 1019820
cttttgcaga acgctttcgc atcttctaaa tagacttcta cgcaatcttc tttgttattc 1019880
gctttgatgt tctcctgagc cgaagctaca gcatcaggaa taatctcaac gccaatcaca 1019940
tttttgacat agggagagag cataatccct atagttcctg ctccacaata gagatcaaga 1020000
agcgtttccg aaccctcggg gtttataaac tctttcgcag tttctataat tttcgctgcc 1020060
tgagtaatct gaggttggaa gaaacttctg ggacgcaaac taaaagaggc agagttaccg 1020120
tcactaggta aggacagttt ttgctgtatc gagggggctc cgtatagcag tttagtttca 1020180
taatatgtag aaatcccacg tgcagctacc ttttcttccc aatagatcga tgctatgttt 1020240
agagaagacg ataggagaat ctctttccat tcatctatac aggcttcgtt taccctatat 1020300
tctggagttc ctgatgttgt taggatcacc ataaagtttt gctgcgggct ccccgtacgg 1020360
acggtgagtg tgcataacga gcctttgttt ttagggggga agtacgccat aagctctggg 1020420
tgcttatccc accattcgcg agtgagtttt aaaatatcca tagtctgctc atggataagc 1020480
agacatgtag tcactggaat tcctttcttt ggttttgtag agctgatgaa tcctaaactt 1020540
ttttctcctt cataagtttg aaaaaaggag aattccattt tatttcttcc tcttaaagag 1020600
ggagaacatg ggatgatagg agcaatcata tccgagggaa ctaaaggagc gaataactga 1020660
tgaaggagtt cttccttttt ctttaaggag tcagaataat tggactgagg aaacgagcat 1020720

cctccacata caccaaaatg tggacaattt tgcatggtag acatagttaa gatagaagct 1020780
tagtttttgt tagagaggta atctgagaac tagacaacaa ttcacaagga ccttcatttc 1020840
gaatccctag atgagatact tgaaaggttg tttgagaata tcaaaagcaa aatggaaatt 1020900
ttatcacgaa ggaaaattat atctctacct ttttaaggaa gaacncctag gtagcacaga 1020960
gaaaaatttg ggcataaaaa agaggatgag tagaaaatta cccaccctct aaacgaaatc 1021020
aagctaaggc gaattatttt ctaaatccgc gtgctttgga aggtttttta actttttttg 1021080
cggctgtctt tttagatgca ggtttagaag ctttgactgc tttgctagtt ttagctgcag 1021140
cagcagagct ttttttaggg gctttttttt ccgcagtttt ttttactttg gcaggagcct 1021200
tagttgatgg cttacgtttt aataacccag atttctctgc ttttatagat tcttttctgt 1021260
aaagtttcgc aactttttct aattttatag agtctgtgcg tactctttga gctgctgcct 1021320
tgtttccttt ctctgcttta gctaagtcgt gttggatgct atccagcagg tctttcattt 1021380
tttttgccgt atcttttagc gccatgaaaa gcgtcctcta gtcgctgttt taaactattt 1021440
ttacttgttt taatttttaa ttagtttgtt tgttcaaatt catgcaacca ttttttagag 1021500
aaaataagac ttttgtaagt tattgttatt ttgttatgag aacacacttc tttgtttatc 1021560
aatgtgttaa tttataaaat gtttggaaaa tccattggaa aaagcaaaaa actttaagag 1021620
aggatttggg tttttttttc gtgttttgag taaaatggag aacgtatttg tatcaatttt 1021680
tgtagcatgt agagaaaaac atattttctt tgatcattgg gaacgtagtt tctatagatc 1021740
gaatgtagtt caaagaatgc taccccccaa atcctttact ttcgagaaat gtctgctata 1021800
aaaggaatag aagtatttag atgaagaagc tatatcaccc taccttattt ctgagacctt 1021860
tgattcggct gagcctcatt tttgcgttaa gtttgacact gatttcagga aatttcccac 1021920
agcagaaatc ctttggtcat tgctgtgcag atatgcactc agcattaata tcagggaaaa 1021980
attgtgaaga actctttgca gattttattg agcgggttct tgccgacaga gagactttga 1022040
ctgcccgtga ttggggaact gttgttgtct tagtccgcga gtatctcttg aagtgtattc 1022100
gtaaagggga ttgtgactat ggagtgaaaa ttcttcagaa actcctagcc ttacgcttgc 1022160
cgaaggatgc taggaaagac ttgcagatct tatggcaccg gttgaatcca gagcaggctc 1022220
ctttgcgaga tgttgttgat cagcttttca ctataggctg tcacgaatct ttgcaagatc 1022280
acttgctttt tgagctctac accgtgacat tgcatagcgg ttatgaaaac cgtaaacagg 1022340
atatgctttt agctaaagaa caaggggatt acaaaaaagc tatagaattg gcaaaagagc 1022400
tggttgcagc attggaaaag ggatcgtgta gccctcaccc agagatagtg caaatagaaa 1022460
aaactttttt gcaaaaaaca ctgcttgctt tgcaaataaa agtagcacaa gaagctcaag 1022520
aatcttgcga tgctttactc acaccctact gtttgtctga aattgcctat actgaagcta 1022580
tggatgcttt ggtactgcgt atagctagag gtgaagtctc tcgtactaat gaggtggata 1022640
gtgtcttact gagccatgcc ttacaacacc tccctttcgc acgggaaaaa gcgattcctg 1022700
aacttgaagt tcttattgat cacggagcct atctagaatc cacattacta tactatgcat 1022760
acttctcttt attagaactt tatcatcaaa ataaagactt tgcatcgcta gagcgtttgc 1022820
tagagaaggg tgatgctgtc tttgtccctg agcatcccta tttcccagaa tatgggtttt 1022880
ttctaggtgc atatttctat gctaaaggga aatacgagag cgctgagaaa gttttcttac 1022940
agattataga tcccgctgta aaactaggag ctacttttgc tagagcatat gaatatctag 1023000
ggtgtattgc ttatgttcag aatcattacg agaaggcgga agaatacttt ctccgtgctt 1023060
ataagagttg gggacgcgaa gagtcaggta taggattatt tttagcatac gctgtgcaaa 1023120
agaaaaagac tgcatgtgag gatatgctct accatcctaa gttttctttt acctaccgtc 1023180
atttgctaga ttctctctgt tctttatcgt atccacatgg ggaaaataag ggcagctctg 1023240
cgatccaaag agtccatagg gctgtacccg agctttcaga gatttatagc cgctgtatct 1023300
atgatatgat taaatatagg aatgttacct atacgcaccc tattatagag cttgcttaca 1023360
accaagtccg caatttagaa aaacgtaatt tagaagaaat ttgtagggat gcgcaagatc 1023420
cagaatatga taaagcttta gccttttggg gagcccttca atcgggggcc tctgtgccaa 1023480
ggtctctcat tgaaagttca gatgtggatg aagccggaat cacaatacgt tgctatgagg 1023540
ctttatattt tcacaatcca gatgctatag ctatgcttcc tcaagcgttt tctgaagaat 1023600
gtaattcttg gcaaacggca ctgcgactcg tttggacgtt agtacgacct aaaggagctc 1023660
ctaatcacgc taagtattgg gatcaccttg tgctccgtcc ccatggggat agcctatatt 1023720
tttttggtta tgaccttcaa gaatatctca ttggcaaaga agatgccttg aagcatctgt 1023780
ctgtatttgc ggaactcttc cctaaatctt cgctgctttc gcttgtatac tatttacaag 1023840
gctatagcga atcctcggct ttgaggaaag tcggatggtt tgtaaaagct cttgaagagt 1023900
tcactgagat ttcttggtcg ggagagcata tgaagacatg ggcgtatatc tattatatgg 1023960
tgaagttaga tcttgctgat acctacatct ctttaggcaa cttttctcag gcagttcata 1024020
ttcttgagga ggttaaggaa gattggcaag tcgccagtca tcccaaacta catttcctta 1024080
agggagagga ctgttactta gctatggaac tgcgatgggt agagggattg gcctatgcgt 1024140
actttcagtt acatgagacc gcacacttgt ccaatcatct tcttgagcat gtagaaaaga 1024200
atttaatttc cccacgttct tacagagact attatggcga gtctctacag agaactctgg 1024260
ggttgtgcca gcgtttcctc ggtgtgtaaa tcacagatag catgatatgc agaagctata 1024320
catcgattca gtcctgggcc tgcaatattt tgacctacaa tttttaaatt tcctggtaag 1024380
tgaggtagga tacgctcttt cctttctaga aagccaacag catgttgtgg catgccatcc 1024440
tgagatgaaa ataaagcaaa agcatctggt ttttgattga tattcaggta ttctgagaga 1024500
gctgctatcg caaaggcatg agcttctgat tcccgccatt tgccttcaat gagaagggag 1024560

agcactgttt tccctggcgt acttgcggga agatttgaga attccaaacg attcccaaga 1024620
ggggaagctc gtctgcaaat agcatgccat aacctttggg aagcgaaaag ttggcgtgat 1024680
gccaacctaa gctgatgcta gataaattcc aagggagaac tcttttagat aaattttcaa 1024740
ttccatagtt aggaagcaat acaggaagct gctggagagg tcctgtataa attaccatat 1024800
cggcaaaaaa cgtctctgaa ggtgtggtta cacaagcttc ttttggtgaa caatcgatgt 1024860
gagtcacact agtagaaaac ttccatgttg cggggagttt ttcttggata gtagtaatca 1024920
aagtccccat ggaaggagat agagatgcaa gatatctgtc ggtcttagac tttttggggg 1024980
atctattttt aagataactg cgtaatagag acccgctgga agcttctctc tttgctagtt 1025040
caggaaatgc catgtgtgtg gagagaatgc tgctatggcc tgcgcgtatg gctgtgatta 1025100
gtggatctaa tatatagctt gtaaaatttt gtgaactatg tcgctttaag aaatcctgaa 1025160
cggaactatc ttgagtatag caaggtgcac ggaagtcctt aattaaagaa ggaagcaggc 1025220
cctttcttag gagagtccag gtggatattt tacgggcctt ccctctgtag tagacaaagc 1025280
ggttttttgc tgcacgatca ctgaagatca aggaattttg gagaccaagt tcatggataa 1025340
gttttagagt atactctccg tctccccggg tgaggaatcc tttaggtccg aggtcaaaag 1025400
aaaacccttg aggggattcc gtgcggacga agcctcctgc gtaggcttct ttatctaaaa 1025460
caaggatttc cgcttggggg aatttcttat gaagccacca acctgcagca agaccagaaa 1025520
ttcctgcgcc tataatgatt gctctcttca cacaaaacct cctctctgct cagtttctca 1025580
acatttttat atagaagctg agaaacattc ttttttagat accttattga gaaaatagtg 1025640
atcgaaagct gtggcaatga ctctgacaaa caattctcca agaggagtta ctttcaaaga 1025700
gccaggactg ttatggatga gacctgtagt ttccatactt atcaagcgat cacgactttc 1025760
tataaaataa gtatcaaact catatccaaa aaggttgaaa aactcttcct tattgatcgt 1025820
aaacgtgcac atcagcttat ggattgccca ttttctaatc cgatcatcct cggtaagaat 1025880
tttacttttc acagtggcaa atgttcctcg aagcaccgta ttgtgatatt cctcaagggt 1025940
ttttgcattt tgtagataaa ttccacgaat gaagcttgta gaagtcattc ctaacccgag 1026000
cagatcttct tctgggggta gagaataccc ctgaaagttg cggattagag ttttgttttt 1026060
gaaagcgagg gtaagaggat catggggaag agagaaatga tccataccga tggcctgata 1026120
tcctgctttt gtaagtaaat gccgggattg agaataaatc gcgaatttct cttccataga 1026180
aggcatatcc gaagctttca tggctttttg gtgcggcttg atccatggaa ctgaggcaaa 1026240
agaaaataaa gcaagacgat ctggatacat cgctaagata tcttgaattg ttttagaaaa 1026300
ggactccttg gtttgtttgg gaagaccata aattaagtct atattgatac tttggaaggc 1026360
gagttcctta aatttttcgt atgcctttaa agattcctca tgcgattggc gtcgtcgtac 1026420
agcttcttga acatcagctt gggtatcttg aacgcctaag ctaaccctat taaaacctac 1026480
gttctgaaag aaatctgctt tttccatgtc gtttcttaaa gaacgaggat ccacttcaat 1026540
agcaatttct tcagcatgtg aaagatcaaa aagcttatgg atgtggtcaa aaagcagggt 1026600
aaacaactcc ctggagagtc tgctaggggt gcctcctcca aaatgaatcc tggatacctg 1026660
aggccggaat cctatggtct caacgacaag tttcatctct tggattaggg tgttgatata 1026720
agcttctaca atatcttcac gacgattcaa aacaacagaa caaccgcaat acaaacacat 1026780
ggattggcag aaggggatat gaaaataaag agagagaggc tgagnattnt ctctaantct 1026840
ttggaacgct agaagagctg gagccgcatc ggaaggttcc cattctaaag ctgtagggta 1026900
gcttgtgtat ctgggcgcgg gttgatgaag tccttctaag aatttaaagt tgacgttgaa 1026960
cataagaaac taccaattgg acgttttcta aaggtgtttc aggaagaatc ccatgtccag 1027020
aattaaaaat gaagttagga tatgttctta aaggaacaag gaaggcttct acatagtgta 1027080
ataatttctc ttggggcaat aaaaatattg ccggatccaa gtttccttgt agggagagca 1027140
taagattttt ttgtatgcga tgaaggtcca catgataatc aggatggagt gtatcggctt 1027200
gcgtggcttg cagagtataa aaattttctt caaaacaacg acaaaataaa ctcacaggaa 1027260
tggcctgctc tttaagtttt gctatcagac gacggtttgg ttcagtcaca tagcgtgtaa 1027320
agagtgctga agggagacgt agactcgaag attcaaaaag ctgcacagca gcagctcctg 1027380
catccatttg tgtttttaga taaatagcag tcccttcgat aattgtagag atgagctgat 1027440
caaatttttc aggatacaca tagagaaaag acatggtctt agaaaaatct ttggaagcac 1027500
ctccatcaat taagtagcag gcgagtgtga agggagatgc tgcaaaaaca atcagcggaa 1027560
caggaagttt ttgcttaaga gttcgaatcg cgtccaaaag atagctaaag atggtttgtg 1027620
gatcggaagt gaaagtgaaa ggttgctctg gagaaaattg tatgcgcgga ccaggagcaa 1027680
agtcataggt cacagcaaaa ccatccaaga tggataggat atcagcaaag agaatagcag 1027740
catctacatg tagcaacgag ggccctaaaa gcgtagctct acaatagctt cagtattatg 1027800
gaaaaaggtt ttaaagattg ggatcctttc agctcttggt atggaggcat gtaccttcct 1027860
acctgtcgta atagccatat gggaggatga gaggcagttt gagattttaa aagatcaaaa 1027920
aaagcagaca tagagtccca attatgaagc gttgattaag aagccccttt ctatggactc 1027980
aatgacttct ttgactagaa gttcaggagt tggagataga gcatagggca aggttttctt 1028040
tgtctgttca gatttgctaa ggcatttttg cacatataaa gcattcgcag ttcccttaat 1028100
gctagaaatg ccatgctgca aagcaaatag gcgtatttca gcaagggcaa aaagccagca 1028160
gatctcttga ggtaatgggc caaagcgatc tcgcatttct tcttgtattg cggtaagctc 1028220
ttcagagctt tcagcattac caatcttttg gtaaaactca atgcgcatcg atccagtttc 1028280
gatgtaagta tcaggaatac gcgaattgta aggaaattct attttcacat cgtcgttgaa 1028340
aagtaggggg gacgtgtgtt tttttaaagc tgaaacagct ttttttagta atttgcaata 1028400

caaattaaac cctatagttc cgatatgtcc cgactgatcg gttcctagaa tattccctgc 1028460
accgcggatt tctaaatcat ggagggcaat cttcattccc cctccatatt cctgcttatt 1028520
taaagcagcg agtcgcttcg ctgctggccc agacaacctg tctaagtgag gaactagaaa 1028580
ataacaatag gcctttttat tccatcgccc gacacgtccc ttcatttgat ataaatccgc 1028640
cattccaaac ttatcggcat gatctatcaa aatggtattg gcgtttggaa tatcaatccc 1028700
gttttctatc agtgcagtag caacgaggat gtcggttttc tgatttttga atttcgtaaa 1028760
gatattagag aggtcctcag ctcccatttg accatgagct acgccaatac gagcctcagg 1028820
aatcagattg cgaatggtct cagcaagagt atagatgctc tcaatccgat tatgaatgac 1028880
ataggcttgt cctcctcgaa ggagctcgtg ccttaaagcc gctgtcaatg tttctgtatt 1028940
atgctccatg acaaaagtac ttacaggcaa cctatccaag ggaggcatgg caatcacaga 1029000
tagatcacga gctcctgata gagacatgtg caatgtcctt gggatgggag tcgcagatac 1029060
tgtaagacag tcaatcatgg gatagcgctc cttcagattg tccttaactt taactccaaa 1029120
gcgttgttct tcatcaataa ttaataaacc agggttctta aactctaggc ttttgttaat 1029180
gagtttgtga gttccaatga taatgtcaat ttgtcctgaa gctacttgct cacagatgag 1029240
tttttgcact ttggcttggg agaaacgtga aagcacagca atttcgatcg gcaatcccgc 1029300
cattctttct ttaaaagttt catagtgctg agttgctaaa atcgttgtgg gaaccataac 1029360
aatgacttgt cgatggccat cgcaaacagc cttgacagca gcccgcatga tgacttcagt 1029420
tttcccaaag ccagcatctc cgcaaattaa tctatccatg agttttggag acatcatgtc 1029480
attgtaaatc tgatcaatag tctttaactg atcgggggtt tcttcatagg gaaacgtttc 1029540
cgcaaacttg attacggact ctccgtgagg agggtacaca aaagcaggag ttgtcgaacg 1029600
ttgtgcttct aactgtaaga gcttctctgc gtagacaatc aaagattttt cagtaagatc 1029660
tctagagcgc ttccatttcg aactatttaa atgatggaga tcggcagctt tatcagaagt 1029720
cccaacatac cgagagatca gataggcttg gttcgaagga acataaagcc gagctttatc 1029780
tgcatattct aaaacaagat aatccgtttc aatattcaga tggttcggtt ttttttctat 1029840
tcctaggaat tttccaattc cattatgaat atggacaaca gtctctcctg gaatcggaac 1029900
aaaaacttct tcagtagtca ctgaaaagtg agtgcgttgt ttttgcctac gcaatacttt 1029960
tgtagaagca aactcggata gggaaatcgc tgcaaaggct tcgtttacta atgcaaagct 1030020
ggaagttaga ttccctgttt tttcatagat ttccacatcc ccacgagcta cagtctctgc 1030080
tagagcacgg gcctctttta aagatttcgt ttttgtgctg tagatggcta attttaaggg 1030140
ctttccatga ggaggcatat attcttgaag atgctgtagg aaagcaagta ggggattctc 1030200
atcgttctga ataatttgct caggatacag tataggaatc gcttggcgac tcgcttccat 1030260
gtttcggtga aatgcttcga tgatgacgcg gttttccttg agattcttga cgttaggaaa 1030320
cggagtctcc gagaaataaa cttgattaga cgttgaaatg cgatcgtaga gagtgccaat 1030380
agagaaaaat ctatctggaa gggacgaaag tgttccagaa atatccgcaa agtcatcttc 1030440
tagaatttct aagttatcaa agagatagag aggaggggtg ctgaaatagt ctaatagtga 1030500
atgagaatag tttcctccag aggcctcttc tgtgtacgct ggagagatag aaattttaga 1030560
gacttttcct gtcgatagct gatccgaagg attgtaagat ctgatagaaa tgatcttctc 1030620
tccccaaaat tctatcctaa aaggctctgg cgaggataac gggaaaatat caacaattcc 1030680
tccgcgacaa gaaaattctc ccttttcgct agttagcatg acctgagaat atcctaaact 1030740
tttacagagt tccgtagttg cttctggatc taagacatct ccaactgcga gatcaagatg 1030800
ttgttgactt gtagcttgtg gagaacgggt tttttctaaa agagctttta atgtagtgac 1030860
acagaatata ggagccctgt gctgattcaa gctgtaaaga agatgatctc gcttccccac 1030920
agcatctatg ttcactaatt ttggagagag atcaatttca gaagagggaa attctacagg 1030980
agcttggtct aaaaaggtcc ttaaattttc aaagagatca tcaagacgtg cgggtgtcgt 1031040
aatcataatt acagaagcac gacagtcatg aaacatcttt gctgcaagga atgccgtggc 1031100
tcctggatga atattttcta atagtaaagg aagtgtttcc tctttgaatt ctttggaaat 1031160
agaaaaatct aaattaactg ggttgaaatc cattgccata aagtttcatt taatacttct 1031220
gttgctggga gagcgggagc actaccttgg gccgattgat cttttcctcc ccagcgacca 1031280
ccgcaaggag taagaacagc tttcaataaa tcctgagcat ggacgccttg tgtgataagg 1031340
tcgtcagaga ctcgagatag tacaatatat ttcccatttt tctctgttgt ccacagagaa 1031400
attagttttt ctggaatcct ttgatgcaaa cattgtgcgt actgctgcaa acggtggttc 1031460
tcatgttctg cgagatgatg cacgaggcaa gtgattcctt gacgttgatg acaattgtgg 1031520
attaacttgt ctaattttgt ctgaatcaga ctgttttcta actcatttaa ccgtttgtct 1031580
tgttgtttgc gctcatctaa agttgcagtt agcctggaga caatctgatc cctagggact 1031640
tgtaatagcg tacaatctct tctaatactt cgctttgttg gtgtactgta gcttcggctt 1031700
tttctccagt gaccgcctca atacggcgga tgcccatggc cacagcatgt tctttggtaa 1031760
tccggaagaa tccgatatcc ccagtagctt ccgcatgcgt gcctccacaa agttcatgag 1031820
agtgccctgc agaaactaca cgaaccacat cgctatactt atccccgaag aattgcttga 1031880
tttctgatga gttcatcaca tcagaataga gagcttcacg aatatctaca ggctcattct 1031940
cccggatact ttcattgact agagtctcaa tacaaagaag atcttcaggt gaaattgcct 1032000
gggggtgagt aaagtctaaa cgaatttttg tatcgtcaac ataggagcct gcttgacgga 1032060
tgtgatcacc caaagtgatt tctaaagctt tgtgtaatag gtggcaggca gtgtgattgt 1032120
tggcaatcct ctttctacga taacgattta cctgagcagt cactgcagct tctacagtca 1032180
gacttccttg ggaaattctt ccatgatgta cgattaaccc ggcttttgga gatgtcgtat 1032240

gggtaacaat aaaggtcccc tcactacaaa agatctcccc agaatctcca acttgtcccc 1032300
ctttttctgc ataaaaagga gaaactttta atacaatagc accctcttgc ttctcttgaa 1032360
gcgaagaaac tatgtgatct ttagaaatga tagcttcgat gaacgtatca caagagagat 1032420
gatcataccc tataaattct gaagttaaat gtagttcatt gtagatagat tccgaagtcc 1032480
cttgggattg aactacgttt tttctagaac gttctttagc ttcttgctct aacttatgga 1032540
aggtatccat gtcgacacta tagtcgtaat ctttagctaa caaagaaatc tcatcaatgg 1032600
gcatgccata ggtatctttg agcttgaaag catcttcacc tgaaatgcaa gaggaggaag 1032660
aagaactttt caaaacttgc tgtagaaggt ttcctccacg gtcaagagtt ttaaagaaac 1032720
tttcttcttc taaagtgagt actttttgaa tttgggatag cgaattcttt aattcagggt 1032780
aggcctctcc catagcatct gctaatgagg gaacaatttc tgctaagaag ggattgcgaa 1032840
accctaaacg ccgtccatag tttacggatc gtctcaaaat ttttcttaga acatagcctc 1032900
gctcagtgtt tcctggaagg agaccatcag cgatagcaaa agataaagaa cgtacatgat 1032960
ccgcaattac ccggaaagct gcaccactat catcgggatg atagactttc ccagagagct 1033020
gctcagtctt tgcaatcagt cgcgcaacac atccgcttca aaaacagtgt gcgtcccagc 1033080
aattaaagag acaagtctct ctagaccagc tcctgtatcc acatgtttat taggtaaagc 1033140
aagtaaagag ccttcgctgg tacgattgaa ttccatgaag actaaattcc aatattctaa 1033200
gaaacgctct ccatcagtat cgtcaagggg agaagaggcg tttccaaaac tggggccacg 1033260
atcaaagagg agctcggaac aatagccaca ggggcctgtg tttgccatgc tccagaagtt 1033320
gtctttgtct gtaagacgga aaatacgatc tgtaggaaga tatgcttccc aaagagcaaa 1033380
tgcttcatcg tctttttcat gtacggtagc gtaaatcctt cgggattaaa attaaaaaca 1033440
gataaagaga cttcccaagc aaaggcaatc gcctctgctt taaagtagtc tccgaaagaa 1033500
aaattcccta acatctcgaa gaaggtgagg tgccttgaag tatgacccac attatctagg 1033560
tcgttgtgtt tccctccagc tcgaatacat ttctgcgatg ttgtggctcg agagtagctc 1033620
accttctctt tatttaggaa aatatcctta aactgattca ttcctgcatt cgtaaaaagg 1033680
atcgagggat cattgtgagg aaatactgga gaagagggaa gaatggtatg gtggcggtta 1033740
gcatagaatt ttaaaaaatt ggagcgaata gtattgctta acatgaaaaa gaactctcaa 1033800
cggcagcggt tttgcagatt ccttgccatt ataggagagt tctttatttt cgtctctatc 1033860
cctaattttt agttaaaaaa ctttcctgtt tattattgag atttgatcga aaattcattt 1033920
gtttttgaaa gaattacaag gtaagagttc cttgcttttt gcttggggat ttcctatagt 1033980
tgctgctttt acatagaagg tctccagggt ttacttatga tcaataaaga attagatatt 1034040
ggtattttag gaaaaattgc tggggctatt aaacaaatta gtattgaatc catccaaaag 1034100
gcctcttctg gtcatccagg acttccccta ggatgcgcag aacttgctgc ctatttatat 1034160
ggttatgtcc tgcggcaaaa tccacgtgat ccccattgga ttaataggga tcggtttgtc 1034220
ttgtcagcag gtcatggatc tgctcttctc tattcttgtt tgcatcttgc cggatttgat 1034280
gtttccttag aggatcttca ggaatttcgc cagcttcact cccgaactcc cgggcatccc 1034340
gagtacggcg aaactgtggg agtcgaagca acgacaggtc ctctgggaca gggattaggc 1034400
aatgctgtcg gcatggcgct ctctatgaag atgttggaat cccgattcaa tcgcccagga 1034460
catgagattt ttaacggaaa aatctattgt ttggcagggg atggctgttt tatggaggga 1034520
gtcagccacg aagtttgtag ctttgcaggc tctttaaatt taaataatct tgtggtcatc 1034580
tatgactaca ataatgttgt tctcgatgga tatcttaatg aaattagtgt tgaggataca 1034640
aaaaaacgtt ttgaagccta tggctgggag tattatgaaa ttgatgggta tgattttacc 1034700
catattcatg agacattctc gagcatcaaa cgggggcagg aacgtcctgt attagtgatt 1034760
gcacatacaa ttattggtca tggttcgcct aaggaaggga caaataaggc tcatggttct 1034820
cctttaggag tcgaagggac tcatgaaaca aaacagtttt ggcatctccc tgaagaaaag 1034880
ttttttgtcc ctcctgcagt aaagaacttc tttgctcata aaatacaaga agatcgaaaa 1034940
gcacaggagc aatggctgga tgaagttcgt gtttggtcaa aacagttccc agaattacac 1035000
gaagaattcg ttgcgttgac ctctcataag ttacctaaaa acttagaatc cttggtgcag 1035060
agtgtagaaa tgccagactc tatagctggc cggctgcttt caaataaact gatccaagta 1035120
ttagtacagc acattcctta tttgattgga gggtccgcag atctttcaag ctcagatgga 1035180
acttggattg cgaatgagaa agtcatccat acgtatgact tctctggaag gaacattaaa 1035240
tacggcgttc gtgagtttgg tatggccaca atcatgaatg gtttagctta tagccaggta 1035300
tttcgtcctt ttggtggaac atttttagtt ttttctgact atatgcgtaa tgcaattcgc 1035360
ttggccgcat tatctaaatt accagtcatc tatcaattta cccatgattc tatatttgtt 1035420
ggagaagatg gacctacgca tcagcctgtg gaacaattga tgtctttgcg cgcgatccct 1035480
ggattgtatg taatacgtcc tgccgatgct aatgaagtta gaggagcgtg gattgcagga 1035540
ttaaagcaca caggtcctac agtcattgtc ttgtcgcgac aagcattgcc cacactgcct 1035600
gctgcgcatc ggccttttaa agatggtgta ggtcgtggtg cttatattgt cttaaaagag 1035660
tcaggagaaa aaccagacta tactctcttc gctacagggt cagaagtttc tttagctctt 1035720
tctgtagcta aagaactcga acacttggat aagcaagtgc gtgtagtttc tttcccttgt 1035780
tgggagcttt ttgaagctca agatgtggac tacaaacaga gtattgtagg cggagatctt 1035840
ggaattcgtg tctctataga agcaggatct gctttggggt ggtataagta tatcggatct 1035900
gaaggtttac tatcgctatg gatagattcg gatactcagg agcttctgat gatgtatcag 1035960
aagaatgtgg ctttactaca gagcaaatcc ttcagaggat tctctctcaa tagtcactgt 1036020
cggaagtttc agagccgcta gccatagtgt catcggcttc tccaacttcc atatgaggta 1036080

atcctcgata ctgttgatcc ttcttatggt cagaagctgc tcgttttagc atgacttttt 1036140
caaggttctc tatgacttct tgtcctgtta agatgtggtc ttccgtataa gtattaaaga 1036200
tgaagttccc actggacttc gttttgattc cctccttcct taagggaaga tctgaaatca 1036260
ataataacgc tccaatgggc aggtttctac ggtatccggc agcaaaaagt gtcgcacact 1036320
ccatttcagc ggattgagct ttggtttcgt acagtttttt tctaaatttt ttgttaaatt 1036380
cccaaaagcg aatgttggtc gtgtgggtaa tgccaatatg gtagtttgcc ttcttatctt 1036440
ctaaaacttc agttgttgct ttctgtacaa caaaatttgc aagagccgga acttcaggag 1036500
ggaaataggc gtctgaagta ccctctccac gtatgctagc tacggggaca aagtaatctc 1036560
caacctgata atgagagcgt aagcccccac acattcctaa cataagcgct gctttgagat 1036620
caggaagaaa tgaacataag tctatagtta atgcagctcc tggagaccct agtttaaaat 1036680
ctaaaattga agttttaaga tgaggagcat gggcagcaga aaacatagaa ccctcaaaga 1036740
cgggcacccc atgaagtttt gcaaaggttt ggatatagta agagaagttc gtgagtaaga 1036800
gataaggaca aaattgcttt acgctagagc cagaataacg ttctaacata tcttgagcaa 1036860
tcctggattc agaagtatgt ttggacacac gctctccttt gtaatgtttt cttctcagat 1036920
tcttagcatt cttatcgttt ctcggtcatt aaaaaaatcc tagaaatcaa aagatagaat 1036980
tctgataagg gacagctgca atcatttaca tagaaggaga gtccttcgat tcctgatgta 1037040
cattcttctg gacaaaggag aaaaagctcg ataacataaa tcgaagaaaa ttaagaaata 1037100
gattgcttta tggttcgtgt aagtactagt gaattccgtg tgggattaag aatagaaatt 1037160
gatggtcagc cctacttgat tttacaaaat gattttgtaa agccaggaaa aggccaggct 1037220
tttaatagaa tcaaagtaaa aaatttttta actggcagag tcattgaacg aacctataag 1037280
tctggagaat ctgtagagac tgcagatatc gtagagcgct ccatgcgcct tctgtatact 1037340
gaccaagaag gggccacgtt tatggatgac gagacctttg agcaggaggt tgtcttctgg 1037400
gagaaactcg agaatattag acagtggtta ttagaagata ccatctacac cctagtttta 1037460
tataacgggg atgtggttgc tgtagagcct cctattttta tggagcttag cattgcagaa 1037520
acagctccag gagtgcgtgg agatacagca tcagggcgtg ttttgaagcc agccgtgacg 1037580
aatacaggag ctaagatcat ggtccctatc tttattgatg aaggggaatt ggtcaaagta 1037640
gatactcgta cgggaagcta cgaatcccga gtttctaaat agattgccgt tgtaggaaaa 1037700
gtatttcttt tttacagtgc gtaatatgga agctaagaaa atcaaagagc tatctaaaga 1037760
agctcaattg ctaaaaaaat taagagagaa gtctcgggtt cttgatgaaa agaacaagcg 1037820
caaagcttgg gttgctaagc ttgtagctat gccagagtct atccgagaga tagagaaaga 1037880
agagcgcgta gaaactcctc aattatttca agctatagca gagaagattt tagaagaagg 1037940
tgtttagtca cctgaatctt cctgcatcat tctataactt ctgacagtcc caagttgctt 1038000
cattagcaat ttctaaaggt gcatcaaagt ctaagagatt tttcaaaatc atagtgtgca 1038060
cctgatattt ttcaggatac aagtctataa catggaaacg tgaattcgtt ggtagagaga 1038120
tcgacccgct attgagaata taagagggcg aagtgtccgc acaattgtaa acagcagctt 1038180
ggtgttcgtg accgtgtaga taaaggcgta ctttgggata tttcttcaaa acgttttgaa 1038240
gatgtgtatt gttgataaga tcatgactgg gattttgtga agagagcaga ggataatggt 1038300
ttgcaatgat gacattctct tcaggggaaa gagagagcaa aaaggtttca atagccgaaa 1038360
tctgtgctaa atgcaccact ccatttgctg aaaaccatcc attcaaacaa gaacaatcta 1038420
ataaaattaa ccaccaatga tcagtgatct tgtgaaagga caccttattc tgttggagct 1038480
ggtcatttgg gaagtgggtg taaaacgttt gttgtgcaag agatttcagg gtgtagacat 1038540
cgtggttgcc aggcaaaagg taaaccgagg aatgcttagc gagagtttca acgaaatgct 1038600
tagcgagtaa gaactctcca tccatagccg tgagggagaa atctcccgtg atgcacacgc 1038660
tatccgctcc aagagatcgg actaccttag ggaaacgctg ccctatagtt gtagcttgaa 1038720
aatggactaa cccaaatacc ttgcgcagaa gtcctttgag ccttttatta aagcagtgca 1038780
cgggattgac agggagaaca tgaaaatgta catcggaaat gtgaataata cgatgcacat 1038840
gtcggggctt ttcttgcata ggacgtgggt tacttttgtt tttctgatct actgtattag 1038900
aatctaggga aaaattctaa caacaaagat cgatctctaa ggagaagccc ttagagtcaa 1038960
gattctaagg ggctatccca aatgattgat accaaggggt ttattaattt tataagagaa 1039020
gatcagagcg tagaaatctc atattgattt tctaaaatta aaattttaga agagagaacg 1039080
agaatcgctg tatctaaggc ttgggctagg attgtagatg tcgtagctaa cgaatctaag 1039140
attccaccag caataagatc ttcaatttct ctagaaaaca cactgattcc taggcttgtt 1039200
gttccaagtg aggagagctt agcaatcacg gcatccccat ccaagtctgc attggttgcc 1039260
aagagcttca gaggagcaca acaggccttc tgtaagagag aaattgcaat actattctca 1039320
tcagcatcat ctttaggagt ccctaaagtt aaagatgcat agaacaatgc aacgcctcct 1039380
ccaggaacat acccacgact taaagcagat tccataatct taagagcgag ggtatataaa 1039440
ggttcgttat cttcatcagt aggaagaatg gctaccgaac tttggagcct atttgtgctt 1039500
ttgattagcc tttttttgt ttcaaggcat gaggtcgtgc gtatttcttc tgctaattgt 1039560
cgcgtcttta aggtcaggac ttccgggata tgtaggcctc caatgagtgt cgtttgtgac 1039620
tctgagattt ctatagatag acaggaccct aaagtgacca tctcaggagc taaaacatga 1039680
gatgcttctt ggcaaggaca aatgtgggtc ccagtaaata aagcaatatc ctcggctaat 1039740
tcttgatttg tagtagagag ttgaggaatg gtgactacag ttacttgcaa gagcccttgc 1039800
aatttgttaa caacaagggt agcaagtacg tctggatcga tatcttcaca gaaaatgatg 1039860
aggtgctggt tttgttctga aatttcttgg agtaggggaa gtaaggagtg aatcatagag 1039920

atctttctat ctgtgatcag aattagagga tgagcaattc tagttagacg agacgctgta 1039980
tcagaaacaa aatacgtcga agcataccct gcaggaattt taaatccttg aaagacatcc 1040040
atggaagttt tgtcgttttc tctctcctta gtgatggaga taagaccctc agggcctacc 1040100
acagaaaaag cattgtaaaa atgatcggca atggtgggca tgtgtagaga agaaaaaata 1040160
atattgcgaa cttttagagc atctttaata ggccaagatt gttgttgtaa ggcttcctga 1040220
agcttttctc cctgcaattt taaagaggca atgagcttgt gtgtggagat tcccttttct 1040280
aaagctgcat agctttcctg taaaattgca tggagtaaaa taagtccagt agtcgcgccg 1040340
tcgctgtgtt ccttatgaat tttattcacc atagctttcg caaaatctac accgagattt 1040400
tcataggaat tcgaaagttc tgtttgtgaa atagcgtaga agccacgctc tttaaagaaa 1040460
gaagttgggg agagggattg cttgggacca tacgatccct ttactatctg gaaaagttta 1040520
tctatccctg aaaaaagttt tttatcagca ttgtaattag ataatttttc ctgttcggac 1040580
atgcaaaccc ccagtagacg taaaattttt acactaccaa gtcgtttttt agttcacaat 1040640
accttcagta ggaaaggtgg cacccggaat cgaaccgggg atagaggctt tgcaggcctc 1040700
ggccttaccg cttggctatg ccaccaaagg ggaggatgag aatccttagt ttaagcgaaa 1040760
gaagatttta tggtaagcga gaagtgcgca taattctaga gactagggaa atcttagtcg 1040820
tttttgagag atgcaattgc attttagttc tcttaaaaaa gaggttatgt aatcaaccta 1040880
ataagggtac ttgcattctt gtctgcattt taaatatagt actttttagt gtaggtccat 1040940
ccttctggta gggagttcag actttctaaa accatagatg ctgtaggcaa aattatatgc 1041000
gagctatgtt gcttgaagat tgggtctctc tgatgctatc agatgtctct tgtccgaagt 1041060
gtgataaaaa aattacagga tttgctatag atagccagaa agtgcagcct ggggatctat 1041120
tttttgccct tcctggaaat gcaacagacg gtcatcaatt tttaaaacat gccgcaaccg 1041180
caggagctgt tgccgccgtg gtttcccatg actaccaagg agatagcttt ggtctagaat 1041240
tgatccgtgt tgatgatacg aaatctgctt tgcaagaagc aggatccaat caatgcaatt 1041300
tattccaagg cacccttgtt ggaatcacag gatctgtagg gaaaacaaca accaaagaat 1041360
tctcaaaaac aatcctaagc tccatctata aaactcacgc aagccctaaa agttataatt 1041420
cacagttgac ggtaccttta agcctcttga tggcggaagg ggacgaagat gtgatgattt 1041480
tagagatggg ggtctctgaa ccaggaaata tgcaagatct ccttcggatc gtccagccag 1041540
agatcgcagt gatcacgcat attaatgacc aacatgcgat gcatttcccc caaggcatcc 1041600
aagagatcct aaaagaaaaa agctatattc tacaaaaaag taaactacaa ctgctcccca 1041660
aagattctcc atattaccta gatttaaggt cctgttctcc tactgccgaa aaattttctt 1041720
tttcttttaa cgatcccctt gcagatttct gctacaaggc aattagtgga gactctgtag 1041780
tcatccaaac tcctgaagaa aattattgtc ttccgatagc tttttcttac aagcctgcat 1041840
ataccaactt attaattgct gtagcactct cttggatctt agaagttcca gaagagggag 1041900
tgatacgctc tctacctgaa ctgaagttgc ctcctatgcg ctttgaacat agcatgagaa 1041960
atggaatgca ggtcatcaac gatgcataca atgcatgtcc agaagctatg atcgctgctc 1042020
tcgatgctct acctttacca agtgatggag ggaaaatcat cttaatttta ggccatatgg 1042080
ctgaattagg caggtattca gaagaaggac atgctttagt agctgaaaaa gcagcttctc 1042140
gaggagatat gatatttttt attggggaaa agtggatccc agttcaaagt gttttaaaaa 1042200
gctattcttg tgaagtgagc tttttctcct cagctcaaga tgttaaagac attttgaagc 1042260
aagtggcacg ctatggggac gtgattctac tgaaaggttc tcgtgccctc gctctcgaat 1042320
ccttattagc ttgtttttaa tttcttagga gcaagtatga tccccttaat tccaatgttt 1042380
ctaaaacagt ctctattttt ttctttggct ttgacaggga tgaccaccct tgtgcttaca 1042440
gtatctctgg gtgttcctgt aatgaaatgg ctgaaaagaa aaaattatcg tgattacatc 1042500
cacaaagaat actgtgagaa acttgaaatg cttcacaaag ataaagccga ggttcctacg 1042560
ggaggaggag tcttactttt tatctcgtta attgcttctt tacttgtttg gttgccttgg 1042620
ggaaaatttt caacatggtt ctttattata ctcctgacat gttatgcagg tctgggttgg 1042680
tatgatgata gaataaaaat taaacggaaa caggacatgg gactgaaggc aaagcataag 1042740
ttcatggtcc aaattgccat tgcagctttt acactcattg ctcttcctta catttacgga 1042800
agtaccgaac ccttatggac tctcaagatc ccttttatgg aagggatgct ttctcttccc 1042860
ttttggctag gaaaagtctt ttgcttagga ctcgctcttg tcgctattat agggacaagc 1042920
aatgcagtaa atcttaccga tggtttggat ggacttgctg caggaaccat gtcctttgct 1042980
gctctgggct ttattttcgt agctctaaga agttctacaa ttcctatagc tcaagatgtg 1043040
gcttatgttt tagctgctct tgtaggggct tgtatcggat tcttatggta taatggtttc 1043100
ccggcccagc tcttcatggg agataccggg tcactacttc tagggggcct gctagggagc 1043160
tgcgctgtta tgctgcgcgc agaatgcatc ctagtcgtga tcggaggagt ttttgttgct 1043220
gaagcgggat ctgtcattct acaagtactt agttgtagat taaggaaaaa acgccttttc 1043280
ttatgctctc cattgcatca ccattatgaa tatcagggcc tcccagagac taaaatcgtc 1043340
atgcgctttt ggatctttag ttttgtatgc gcaggtctag gtatagcggc tgtcttatgg 1043400
agatagatat gtgtcagcgc attcttattt tagggactgg aatcacagga aagtcggtag 1043460
caaggttttt atatcagcaa ggacactatc tcataggagc agacaattct ttagaatctc 1043520
tgatatcggt agaccatttg catgataggc tactcatggg agctagcgag tttcctgaga 1043580
atatcgacct tgtgatccgc tctccgggaa tcaaaccgta tcatccgtgg gtagaacaag 1043640
cagtatctct taagattcct gtcgttactg atatccaagt tgctttgaaa acaccagaat 1043700
ttcaaagata tccctccttt ggcatcacag ggtctaatgg caagacaaca acgacattat 1043760

ttcttaccca tctcttaaat actttaggga tccctgctat agctatgggc aatatcggct 1043820
tgcctatact agaccacatg ggacaaccag gagttcgtgt tgtagaaatc agctcatttc 1043880
agctagcaac ccaagaggaa catatcccag cactttctgg atctgtgttt ttgaactttt 1043940
ctcgtaacca cctggactat catcgcaact tagatgccta ttttgatgct aaacttcgca 1044000
tccaaaaatg cttgcgtcag gacaaaacat tttgggtgtg ggaggagtgt tccttgggga 1044060
attcttatca aatttactct gaagaaattg aggagatttt agataaaggg gatgcattaa 1044120
aaccaatata cttgcatgat agggataact attgcgcagc ttatgctttg gctaatgaag 1044180
ttggttgggt ctctccagaa ggttttctga aggcaattcg gacatttgaa aaacctgccc 1044240
atagactaga gtaccttggg aaaaaagatg gcgtgcacta tattaatgat agcaaagcca 1044300
caaccgtgac agcagtagaa aaggctctta tggctgtagg gaaagatgtt attgttattt 1044360
tgggtggcaa agataaaggt ggagatttcc ctgccttagc ttctgtattg tcccagacaa 1044420
ccaaacatgt cattgctatg ggtgagtgtc gacagacaat agcagatgcc ttatcagaaa 1044480
agattccttt gacgctctct aaagatttac aagaagcggt gtccatagct caaactatag 1044540
cacaagaggg agatactgtg ttattgtctc caggatgcgc aantttgatc agtttcaaag 1044600
ttttaaagaa cgcggantta ctttaagctg ttgatcagaa gaaatgcagg cagtgaggta 1044660
aatatgaatc gtagagacat ggtaataaca gctgtcgtag tgaatgctat attgcttgtg 1044720
gctcttttcg tcacatcaaa gcgtattggc gtcaaggact atgacgaggg attccgtaat 1044780
tttgcttcta gcaaggttac acaagcagta gtttcagaag aaaaagtcat agaaaagcct 1044840
gtagtcgcag aagtgcctag ccgtcctatc gctaaagaga ctctagctgc acagtttatt 1044900
gaaagtaagc cggttattgt aaccacacca cccgtgcctg ttgttagcga aaccccagaa 1044960
gtgcctactg tggcagttcc gcctcagcct gttcgtgaga cagtaaaaga ggaacaagct 1045020
ccttatgcta ctgttgtagt gaaaaaagga gattttctcg aacgcattgc gagagcaaat 1045080
catactaccg ttgcaaaatt gatgcagatc aatgatctta ccaccaccca acttaaaatt 1045140
ggtcaggtca tcaaagtccc tacgtctcaa gatgtcagca acgaaaaaac tcctcaaaca 1045200
cagaccgcaa accctgaaaa ttattatatc gtccaagaag ggatagcccc gtggacaata 1045260
gcattgcgta accatattcg attggatgat ttgctaaaaa tgaatgatct cgatgaatat 1045320
aaagcccggc gccttaagcc tggagatcag ttgcgcatac gttgatctaa gcagtccctt 1045380
agcatgaaat ggtttgttat ttcctgttta ttaggaatct tttctctagg gctgattatg 1045440
gtttttgaga cttcttcagc agaagtatta gaccgctctt tagaatgtag tacacataaa 1045500
gctctcatcc gccaggtgac ctatcttatc cttggcttgg gagtcgcatc gcttctctac 1045560
atgatggaat ggagagattt cttaaaaatc agtcctgtgt tgctttcagg agctgcccta 1045620
gccttaatct gtgtgtttat tccaggatta gggatatgcc gtaatggggc aagacgttgg 1045680
ttggggttcg gtcagctaac gattcaacct tcggaatttg tcaagtacct cgtccctata 1045740
gtcgctctat atttccttac attctcttcc ctctatcaga aacaactgaa aatgtttctt 1045800
aaacttacag caattttatt tattccgatt cttttgattg ctatagaacc cgataacgga 1045860
tctgctgctg taatttcagc atccctgatt cctgtgttta tcatgacctc agtgcgcctc 1045920
cgctactggc ttctaccact tctgtgcgtc ctcatagctg gaggagccct tgcttataga 1045980
atgccctatg tgcgctaccg cttaaacgtc taccttcatc ctgaactcga catcaaagga 1046040
agaggacatc agccgtatca agccaaaatt gccgcaggat ctggaaagct actaggaaaa 1046100
ggtcctggag ctagccttca gaagcttacc tacctcccag aagcccaaaa tgactatatt 1046160
gccgcaatct acgctgagga gttcggattt ttaggtatgc tcgtcctcat acttttgtat 1046220
atgtgctttg tttacggagg gtatgcaatc gctataaaag catcatcact agaaggtgct 1046280
gctttggcaa tggtcattac tttgattatt agcatgcaag catttatgaa tttaggagtc 1046340
gtttcaggcc tgcttcctag taaaggagtc aaccttcctt tttttagcca aggagggtcc 1046400
tctcttatcg caaatatgtg tggagtcacg ttgttattga aggtatatga tgaagaaaat 1046460
tcgaaaagta gccttggctg taggaggttc aggaggccac attgtcccag ctctctcggt 1046520
aaaggaagct ttttctcgtg aaggaataga cgtattacta ctagggaaag gtctcaagaa 1046580
ccatccttct ttgcaacagg gaatcagcta tcgggaaatc ccctcaggac ttcctacagt 1046640
ccttaatccc ataaagatca tgagcaggac cctttctcta tgttcaggat acctgaaagc 1046700
aagaaaggaa cttaaaattt ttgaccctga cctggtcata ggatttggga gctaccactc 1046760
tcttcccgtg ttgctcgcag gactgtccca taaaattccc ttatttctac acgaacaaaa 1046820
tctagttcct ggaaaagtaa atcaattgtt ttcccgctat gctcgaggta ttggagtgaa 1046880
tttctccccc gttactaaac acttccgctg ccccgcagaa gaggtcttcc ttcctaaacg 1046940
aagcttctcc ttaggaagcc ctatgatgaa gcgatgtaca aatcataccc ctacaatctg 1047000
tgttgttgga ggttctcagg gagcacagat attaaatact tgtgttcccc aagctcttgt 1047060
caagctagtc aataagtacc caaatatgta cgtccatcat attgtaggac ctaaaagtga 1047120
tgttatgaag gtgcaacatg tttacaatcg tggagaggtc ctctgctgtg tgaagccgtt 1047180
cgaagagcaa ctcctagatg tcttgcttgc cgcagatttg gtcatcagta gggcaggagc 1047240
cacaatttta gaagaaattc tttgggcaaa agttcccgga attttaattc cctatccagg 1047300
agcttatgga catcaggaag ttaatgctaa attctttgta gacgtcttag aagggggaac 1047360
tatgatccta gaaaaagaat taacagagaa gctattagta gaaaaagtaa cgtttgcttt 1047420
agactcccat aacagagaaa aacaacgcaa ttccctagcg gcgtatagtc agcaaaggtc 1047480
aacaaaaaca ttccatgcat tcatttgtga atgcttatag gttcattata tgaagggaac 1047540
tcctcagtat cattttatcg gtatcggtgg tataggaatg agcgctttag ctcatatttt 1047600

gcttgatcgt ggctatgagg tctctggaag cgacttatat gaaagctata cgatcgaaag 1047660
cctgaaagct aaaggtgcga ggtgtttctc aggccatgat tcctcccatg ttcctcatga 1047720
tgccgtcgtt gtttatagct caagtatagc ccctgataat gtagagtatc ttaccgctat 1047780
tcaaagatca tcacgtcttc ttcatagagc agagctcttg agtcagctta tggaggttta 1047840
tgaaagcatt ctggtttcag gaagccatgg gaagacaggg acctcatctc taattcgagc 1047900
gattttccag gaagctcaga aagatccctc ctatgctatt ggaggactcg ctgcaaactg 1047960
cctgaatggg tattctggat catcgaaaat cttcgttgcc gaagccgatg aaagtgatgg 1048020
gtctttaaag cactacactc cccgtgcagt agtcattaca aatatagata atgaacattt 1048080
gaataattac gctgggaatc ttgataacct ggttcaggta atccaggact tctctagaaa 1048140
agtaacagat ctcaataagg tattctataa cggggattgt cctattttga aaggaaatgt 1048200
ccaagggatt tcttatggat attcaccaga atgtcaattg catatcgttt cctataatca 1048260
aaaggcatgg caatctcact tttcctttac ctttttaggc caggagtatc aagacattga 1048320
gctcaatctc cctggacaac ataacgctgc aaatgcagca gcagcctgtg gagttgctct 1048380
tacctttggc atagacataa acatcattcg aaaagctctc aaaaaattct cgggagttca 1048440
tcgacgtcta gaaagaaaaa atatatccga aagctttctt ttcttagaag attatgctca 1048500
tcatcctgta gaggttgcac ataccctgcg ctctgtgcgt gatgctgtgg gtttgcgaag 1048560
agtcatcgca atttttcaac cacatcgatt ctctcgttta gaagagtgct tacaaacctt 1048620
ccccaaagct ttccaagaag ctgatgaagt catacttaca gatgtctata gtgccggaga 1048680
aagtcctaga gagtctatca ttctttccga ccttgcggaa cagattcgta agtcttctta 1048740
tgtccattgt tgttatgttc cccatggaga catcgtagat tatctacgaa actacattcg 1048800
cattcatgat gtctgtgttt ctctaggagc tggaaatatc tatactattg gagaggcttt 1048860
aaaagacttt aaccctaaaa aattatccat aggactcgtc tgtggaggga aatcttgcga 1048920
acacgatatt tctctacttt ctgctcaaca tgtctctaaa tatatttctc ctgaattcta 1048980
tgatgtgagt tacttcatca taaatcgtca gggcttatgg agaacaggaa aggattttcc 1049040
tcatcttatt gaagagactc aaggggattc gccactttct tctgaaatcg cttcagcttt 1049100
agcaaaagtc gactgtttgt ttcccgtgct ccatggccca tttggagagg atggtacgat 1049160
ccagggattt tttgaaatct taggaaaacc ttatgccgga ccctcactat ctttagcagc 1049220
aactgcaatg gataagctgt taacaaaacg aattgcatca gcagtgggtg ttcctgtagt 1049280
cccttaccaa cctttaaatc tctgtttctg gaaacgcaat ccagaactat gtattcagaa 1049340
tcttatagag acatttcttt tccctatgat tgtaaaaact gcacatttgg gatctagtat 1049400
tgggatattt ttagtccgtg ataaagagga attacaagaa aagatctcag aagcatttct 1049460
atatgacacg gatgtgtttg tggaggaaag tcgcttaggg tctcgtgaaa tcgaagtgtc 1049520
ctgtatcggc cattcttcta gctggtattg tatggcaggg cctaatgaac gctgtggtgc 1049580
tagtgggttt attgattatc aagagaaata tggatttgat ggcatagatt gcgcaaagat 1049640
ctcttttgat ttacagctct cacaagaatc tttagattgt gttagagaac ttgcagagcg 1049700
tgtctaccga gcaatgcaag gaaaaggttc agctcgaata gattttttct tggatgaaga 1049760
ggggaattat tggttgtcag aggtcaatcc tattccagga atgacagcag ctagcccatt 1049820
tttacaagct tttgttcacg caggatggac gcaagaacaa attgtagatc actttattat 1049880
agatgctcta cataagtttg ataagcagca gactatcgaa caggcattca ctaaagaaca 1049940
agatttagtt aaaagataat aaaaaaacta gagatctttt tattaaaatc tctagttttt 1050000
acatgtaaac aaaagcagac gaataactac gattaatggt ggttactaca ttcgcaagaa 1050060
tgatgcatat tgccttcttc ttgttcttct tgattaagat cttcgtcgta atgacagtgt 1050120
ccactgcagc aggtgtcaga ctcgccgaaa gtatcttcag tagagttaga agagtcatta 1050180
tcacagatgt aatcttgatc agcaatcaca tcagttccat tttcaggctg aaccaaatct 1050240
atctgcactt tcttttttaa tctagaacat acacgtctaa gaatcgttct tagcgcatgt 1050300
attgtgttcc ctcttcttcc aataattttc ccaatatcct ctgctgcgac acgaatttct 1050360
aacttaatag attcgttatc ctcgtcttca atagaacgaa tttcaacagc ttcaggatta 1050420
gtaactaaat tcttaacaat atatgctaca aactcttcca taaagtccaa attaaattga 1050480
gatatagaaa gaaaaaaaca taactctatg tttttaaagt tcctgaagaa aggatagata 1050540
ttcaaaatga aaaagtcaat gtctaatctg aaagcggagg cttctctgga tagctgagaa 1050600
tcgttaacca attcaagtac ctcggatccc cattttcaat aggaaatagt aagacttcag 1050660
gaacctcata gccagagaac tcctgaatag caagacaaat ttccgagaag cgtatgtcta 1050720
tcgatttgat ttgtatatga tgttcttcag actcacatag cttgccttcc catagatatg 1050780
tcgatgtgcc tttagggaat acatgcacac aggaagcaag acgctctgta atcagatgtc 1050840
tagctaagga gcgagcactt tcctccgaag ggaaagatgt aagaataaga acagcagtca 1050900
tagagctttt aataataaga aatttggaaa acgcaaatca tagacatatt gtttttcgct 1050960
ctcgattaca ggactttttt tcatgtgctt atacaggtct aaggcacgat ctaaggtctt 1051020
aattggnagt cttaacagac tgcctgaaga gagcgtcact ataatttctc cagggtatgc 1051080
atcagatnaa gataaatcaa tgattttcgg agactccata gcaagctcct taagaagaat 1051140
cttggtaaaa agcatttttt ctttagggag tttttgcatt tttaaatctt cttgagagaa 1051200
aaaaatctga gggagattca gagaggggaa gtacggttga ccaagaaagc agctcccctc 1051260
gagattgcat aacgtattag atcggttccc aacataagca ataggtgttt gtaaggtata 1051320
gaaaattgta atgcccttat tatcaggaga tttttctata actaaagagg aaaaaatccc 1051380
aagagcatgc aagctcgact cagcttcttt aatagagaat tcatggagat atgtaggtgc 1051440

atccgcagaa agacgtaaat gttctgcaat cgcagtagga gggactttgg atagagtgga 1051500
ggaaactaaa aataggttcc taatgggaga aggattgaat ttagataaag acagttcagg 1051560
gagaaataac caacaaaatg taggaacaca aacaagggaa ctcaaaacga tcaggatcga 1051620
agcataacat aaagagtatt gaggacccgg agggaaaagc gttttgaaaa aacgtcttat 1051680
catgagctca ttgtttttta ttatgatcat aaataatatc gctaatattt acgagattcc 1051740
tcttggagag attactgatg aatttatcag ctaaagaata cggagatatc attgttattt 1051800
atttacaagg atctttagat gccgtctcag tgcctagtgt tcaagagtat ttagagcagt 1051860
ttatacagaa gaaacatctt aaaattgctc tgaattttac agatgtctcg tatatcagta 1051920
gcgcagggat tcgtctactg ctgtctaatt ttaaattggt tcagagcttg ggaggaaaaa 1051980
tgtgcctatg ctgtgtcaaa gagagtgtaa ccgaagtcat gcggattgcc aggtttagac 1052040
aaatgatctt gctctgccag gtctgaacag gaatgtttaa gtaagttata ntttctctat 1052100
atgcttcctt ttgaattcga gtttaataca acttcttctc ctgaatgcga cgtatgcctt 1052160
gatcctcaga agttatttgt aaagctattt aagcgtacga tcgtcttgct ttcaggacct 1052220
acaggatctg ggaaaactga tgtttcttta gcattagccc ccatgattga tggagagatc 1052280
gtctcagtgg attctatgca agtataccaa ggcatggata tcggaactgc aaaagtatct 1052340
ctaaaagcta gacaagagat tcctcaccat ttaattgaca tccgacatgt tcaagagccg 1052400
tttaatgttg tggattttta ttatgaagca attcaagcat gccaaaacat tttatcaaga 1052460
aataaagttc ctattttagt tggtggctca gggtttattt ttcatgcctt tctttcaggt 1052520
cctcctaaag gtcccgcggc agatcctcag atacgagaac agcttgaagc gatagcagaa 1052580
gaacacgggg tttctgctct ttatgaggac ctacttctta aagatccaga atatgctcaa 1052640
acaatcacta agaatgataa aaataaaatc attcgaggat tggaaattat ccaacttaca 1052700
ggaaaaaaag ttagcgatca tgaatgggat atcgttccca aagcttcaag agagtattgt 1052760
tgtcgtgcct ggtttctttc ccctgaaaca gaatttttga aaaataatat tcaaatgcgt 1052820
tgcgaagcta tgctgcaaga agggctgcta gaggaagtca ggggattatt gaaccaggga 1052880
ataagggaaa acccttcagc attcaaggcc attggatatc gagaatggat agaattcctt 1052940
gataacggag agaaattaga agagtacgag gaaacaaaaa gaaaatttgt atccaatagt 1053000
tggcattata ctaaaaaaca aaaaacatgg tttaaacgtt attcgatatt tcgagaactt 1053060
ccgacattag gcctctcttc ggacgcaatt gctcagaaga tagctaaaga ctacttactg 1053120
tacagctgat gtgttgataa aaggtatttc tctccacagg aatgaaacct tcagatcgaa 1053180
tgatattaca gatttcttct tcagagctct gtatcgacca tcctgtagct ttatgcacgc 1053240
tttcgtctaa aataacccct ccaaaatcat cagctccgta atggagagct ttcgctccta 1053300
agcttttacc ctcgccaaac catgaggctg ctacgtgatc aaaattatcc agaaagattc 1053360
tgcccaaagc taaaatgcgg taataggttt caatagaagc ttgttgagga acgttacgtc 1053420
ttagggccgt attcccaggt ttataactcc aggggataaa actataaaag ccagggcagc 1053480
tatcttgagc atcacggagt gtttgaagat gtataaggat atcttctggg ttctccacat 1053540
gcccaaacat cattgttgct gttgtgcgaa agcccatgag atgggctaac ttatgtagat 1053600
tgatccagcc cccaggctgc attttttttg gagagatgat ttttctaact ctctctgaaa 1053660
gaatctcagc cccgccccca gggatggtac gttgccccgc atcccataac ctttggagac 1053720
cttgctcaat actgatgcca gagacccggc aagcatgttc gatttctact gcagaaaaga 1053780
aatgaggatg gatggaagga aattcttgaa cagtaatgcg tacgagttct tcaagataat 1053840
cgatgcctaa ccccgggtgc acaccacctt gcagtaaaac agttttgact cctgaactta 1053900
cataacgttg taataggctg cgaacttcat caaaagataa tagataagcg tcgggagatt 1053960
taggctttct gtagaacgca caaaacgtac aatctatttt acaaatatta gtatagtttg 1054020
gattggcatc caaaacataa gtaacttcat ttgaaggata tcgttcttta cgaatcgcgt 1054080
ccgcacgctc ttgtaatctc tcgatagggg acgaaacaaa tagctctaat ccttcttcaa 1054140
atgaaattct tttacataaa ttcataaaat aattgacgtt aaagattgct agcagcagac 1054200
tttaagtttt aacaatttta tttgtcaagc cccacaccta agttgttatt atggataatt 1054260
cagacaacag ctttcatact ttggaaacgg agcagggatc gtttttaaac gatgaattag 1054320
cagtcgaaga agtggcgtct acagaaagca cagaaatttc tgatgcgaca ttatgtttcg 1054380
ctgatgagat tcaagagctc ccctccccag aaaagaaagt tgcttttatc ttgaataaga 1054440
tgagggaagc cttaacagga tcatctcaag gctcagactt aaggttgttt tgggatttaa 1054500
ggaagcaatg cctcccctta tttaatgaaa tcgaagatac cgcgaaacgg gcagatcatt 1054560
ggcgttgtta tatcgagttg actaaggaag gccgtcatct caagggtctt caggatgaag 1054620
aagggtcttt tgtcgttggt cagattgatt tggcaatcac atgtctagaa aaagatattc 1054680
ttaagttcca agaaggaaca gaagataaaa ttttcaaaga tagagaagat aatttcttag 1054740
aaagccaagc tctagataaa catcaggctt tttataagca gcatcatacc tcattgctat 1054800
ggttaagtag tttttcttca aaaatcatag atctccgcaa ggaactgata aatgttggga 1054860
tgcggatgcg gttgaagagt aaattttttc aacgtctctc taatttagga aatcaggtgt 1054920
ttcctaaacg aaaggaactg atcgaaaaag taagtcaaac atttgcagaa gatgttgatg 1054980
catttgtcgc taaatatttt attggatccg ataaagaaac attgaaaaaa acggtttttt 1055040
ttttaagaaa agaaatcaaa aaccttcaac acgcagcaaa aagacttttt gtctcatcgc 1055100
atgtttttgc tgaaacacga ttgaaactca gtaagtgctg ggatcagctc aaggggatgg 1055160
aaaaagaaat tcgccaagag caaggccgct tacgtgttgt atctgctgaa aattcaaaag 1055220
aagttcgcca gatgttagct gaggtctctt ctcttcttat tgagggaaat gatctgagta 1055280

aagttcgtaa agacttggag ggcatttcta agaaaattcg agctcttgat cttacgcacg 1055340
atgatgtcat ctctttgaaa aaagaaatgc aacaattatt tgatcaatta cgagagaaac 1055400
aagacgcggc agagcattcc tatcaagagc aattagctaa agataaacaa gtcaagaaag 1055460
aagcggctcg ctctcttgct gagcgcatca caacattctc aaaaacttgc tccgaaggaa 1055520
cattacttcc gaatctcgag aagaatggca gacattgaaa gagctcttag gtaaaatgtc 1055580
ttttttacct cctcctgaaa aaatttcttt agataatcaa ctcaatcttg ctcttcaaac 1055640
tattgttaac ttctttgaag aacaacttct ctcttctcca gattctcgag aaaagcttgt 1055700
gaatatgcgc caagtattga agcaaagacg agagcgtcgt caagaactta aagataaatt 1055760
agagcaggac aaaaaattat tagggtcttc aggattagat tttgatcggg ccatgcagta 1055820
tagtgctcta gttgaagaag acaagcgtgc tcttgaagaa ttggatgcaa gtattttaga 1055880
attgaagcaa cagattcagc aattgctatg agaagaaact gtatatatgc ttttgattta 1055940
gacggaacct tattgaaggg gaatagcagc tggagttttt attgctacgg cttacttcaa 1056000
ggtttgtttt cctataaaac gttgcccccc tgcatttacc gcttttttag atttaagttt 1056060
ttttttggga tcttccatcc ttctattatt cgatagtaac tcgtctactc tcttctgttc 1056120
cctgtgatga tctttatgaa gttgccttga attttgtatc gactctgacc ggctccgatt 1056180
tttatgctcc tgttttagaa aaactagaag aagcttttgc agataccaca ggacaggcga 1056240
tccttttttc ttcttctcca gactttattg tccaccccat agcgcagcaa ctcgggatta 1056300
gttcttggta tgcgtcgtgt tatcgcgatc agtctgcaga acagacgatc tataaaaaat 1056360
gtcttacagg ggataaaaaa gcgcaaattt tgagttatat taaaaaaatt aatcaagcaa 1056420
gaagccatac cttctccgac catattttag atcttccttt tcttatgctg ggagaagaga 1056480
aaaccgtcgt tcgccctcag ggacgactca agaaaatggc aaaaaaatat tactggaata 1056540
tcgtttaatt ccatttaaaa aaaagctcga ccttatcttg gataatcgag tatcctaaag 1056600
ctggttttat ggattcattt tgttttgatc tattgaaagt agctgctaaa gctattgacg 1056660
acaaaaaagg gaataatctg gttgttctag atgttagaac aatttcggaa tttaccgatt 1056720
attttgtttt tgttgaaggt agtgtgaatg tgcatgttaa ggctttagca aatactatcg 1056780
tagaagagtt aaaaaagcag aaagttagtc ctcttcatgt ggaggggata acggatggta 1056840
attgggtggt gatagattac ggatttatcg tcgtccatgt atttgtctct gaaattcgtg 1056900
gaaaatatcg tttagaagag ctatggaagg acggatttat tgtcacatcc aagcttttag 1056960
cttcttaatg gagtaagggt ctacatgagt aaaaaacgcg tagttgttac aggattcggt 1057020
gttgtatctt gcctcggtaa tgaagtagat accttttacg ataatcttct tgccggtgtc 1057080
agcggcgtac gaccaattac atcctttcct tgtgaggatt atgccactcg ttttgccggc 1057140
tggatcccag aattcaatcc cgagccttat gtagataaaa agcaagcacg tcgtgtcgac 1057200
ccctttatta cctatgccat ggtagctgct aagaaagcga tcgctatgtc acgttgggat 1057260
aaagaccatc tgccttccga tcccgtgcgt tgtggagtga ttgtcggctc tggaatgggt 1057320
gggctctcta ccctagacca agggatggaa agactcttag ttattcataa aaaattatct 1057380
cccttcttta ttccttatat cattacaaat atggccccag cacttattgc tatggacttt 1057440
ggtttgatgg gccccaatta ttctatatca actgcatgtg ctacaggaaa ttattgtatt 1057500
gatgccgcct accaacattt agtatctggg cgcgctgata tgatcatctg tggtggtacg 1057560
gaagctgcag tgaaccgtat tggtttagag ggttttattg ctaatcgtgc tctctctgag 1057620
agaaacgatg ctccagatca agcttcacgt ccttgggata gagatcgtga tggctttgtc 1057680
ttaggagaag gagcaggaat tcttgtttta gaaaccctag agagtgctct acgtcgcgat 1057740
gctcctattt ttgctgagat gttaggaagc tatgttacat gcgatgcctt ccatattacc 1057800
gctcctagag atgacggtga ggggattact gcatgcgtgc ttggtgcttt aaattccgca 1057860
ggaattccta aagaacgtgt caactatgtg aatgctcacg gaacgtcgac tccattaggg 1057920
gatctttctg aagttctagc tgttaaaaag gcttttggct cccatgtacg aaatctccgc 1057980
atgaactcca ctaaatcatt gatagggcat tgtcttggag ctgctggagg tgttgaagcc 1058040
gtcgttgcaa ttcaagctat ccttacggga aagcttcatc ctacgattaa tttggataac 1058100
cctatcgcag aaattgaaga ctttgatgta gttgcaaata aagctcaaga ctgggatatt 1058160
gatgtagcga tgtccaactc atttggtttt ggtggacata attcaacgat attattctcg 1058220
aggtatgtac cctagttatg atgaagacaa aatatgagta ttcttttggt gttattccta 1058280
taaaattttt tggcaccccc gataagaaca cattaaaagc ttgttttatt tgccatactc 1058340
gaggaaaaca ttggggattc cctaaagggc attctgaaga taaggaaggt cctcaagagg 1058400
ctgcagagag agaattggta gaagaaaccg gactaagtgt tgttaatttc ttccctaaag 1058460
ttcttatcga acagtattcg tttaataatg aagaacaagt cttcgttcgc aaagaagtca 1058520
cctattttct tgctgaagtt cgtggtgaca tccatgcaga tcctatggaa atttgcgata 1058580
gtcaatggtt atctttgcaa gaaggactcc gcttattaag ttttcctgag ctacgagatc 1058640
ttaccgtaga agcagataaa tttattaata actatctttt ctcttcttga ttttcaagga 1058700
gagggaaccc ctctccttag acattaattc acttcagcag tgtctccaat ataggataag 1058760
taatcctcgt gagcgagctg tatcaccttt tgtgcttcct tcttcccgta aatccccacg 1058820
atttcaattt ttgcagggct acctttaatt aaatgattgg gagtcgcttt atatgttagg 1058880
aaatagtgtt ggatcatatc taatactgtg cctggacagt ctgaaatatc ttcgatctca 1058940
gcaaagacta aatcgtcctc taaaacagca ataatcttat catcagcttc tccagaatca 1059000
atgatgcgaa gacctcctat aggacgtgct tgaagtaaaa tatttccatg atggatattt 1059060
ttttctgtaa gaacacagac atccagaggg tctttatcac cttggattcc ttcgcgacgt 1059120

gtttgttccc cgctatagtt cccagaagca gtgccacagt atgtttgagg cagtaaacca 1059180
taaaggcagg gacaaaagtt agaaaatttt tgaggtctgt ctactttgag caagcctgta 1059240
gccttgtcta attcaaattt tacagagtca taaggagtaa tttctatata acaacacagg 1059300
gactcataat tatcctgggt taatgtaggg ctgtgccaag gatgtgcaac atataatggt 1059360
tttttagaca taagtaactc tcaatataaa atttcctata caatcgggtt tagtgaattt 1059420
agactaacac ggagaaagtt ttaagagaag tgtctgctca ggcctaattg acttttaatt 1059480
aaaagtagtc aataatttta ttttctaatt aaacctcact aatttatgaa atactcactg 1059540
aactttaaag agatcaaaat agatgattat gagcgtgtta ttgaagtcac atgttcaaaa 1059600
gttcgtcttc atgcaattat tgctattcat caaacggcag taggacccgc cttaggtgga 1059660
gtgcgagcct ctctatattc ttcttttgag gatgcgtgca cagacgctct tcgcttggct 1059720
cgggggatga cctacaaggc aatcattagt aatacaggaa caggcggggg gaaaagtgtt 1059780
attattcttc cccaggatgc tccttccctc actgaagaca tgctgagggc ttttggccag 1059840
gctgtgaatg ctttagaggg gacctacatc tgtgctgaag atcttggtgt atctataaat 1059900
gatatttcta tcgttgctga agaaactcct tatgtgtgtg ggatcgctga tgttagtgga 1059960
gatccttcta tatacaccgc acatggcgga tttttatgca taaaagaaac cgctaagtat 1060020
ctttggggat cttcctctct tagagggaaa aaaattgcga ttcagggaat aggctctgta 1060080
ggacgacgtc tattacaatc gttgtttttt gaaggcgccg aactttatgt tgctgatgtt 1060140
ttagaaagag ccgttcagga tgctgcaagg ctctatgggg ctacgattgt tcctacagaa 1060200
gagattcatg cattggaatg tgatattttc tctccttgtg ctcgtgggaa cgtgattcgt 1060260
aaagataatc ttgcagattt aaattgtaag gcgattgtag gtgtcgcaaa taaccaactt 1060320
gaagatagct cagctggaat gatgctccac gaacggggaa ttctttatgg tcccgactat 1060380
ttagtgaatg ctgggggatt gcttaatgtg gccgcagcaa tagaaggaag agtctatgct 1060440
cctaaagaag tgcttcttaa agtagaagag cttcctatag ttctaagtaa gctatacaat 1060500
caaagtaaaa ctacaggaaa agaccttgta gctttatcag attccttcgt ggaagacaag 1060560
ctcttggcct atacgtcata gagcaataag cttatccgtg gggacaacat tgagttggtt 1060620
ttgtaacgct gctaatgttg tctcatgggt ttcctggtct ccagaagcaa gaatcactgc 1060680
gtgattattt aagaccaaac tttcttttct atattctaaa ggggctccta aagcatccgt 1060740
gactctaccc ccagcttctt caacgaggaa ggctccaggt acgtgatccc aagcacgagc 1060800
gggagaatca ataaaagggt agcgaatgaa aaaatctacg gcgccttcag caactaaagc 1060860
atacttatat tggctttcta cacgacgagg actcggagtg ttggggagac ccaggcttag 1060920
cttacgtgtt gcatgatgct gttgattcaa tgcagctaac gaagcctcac agaattgttt 1060980
tgtttgtttt ctatcagcat aaacaaagcg tctatctaga ttttgagaat gaacaataga 1061040
aagaccatga cctttagctg ctgaatatag tttaaatgtc tgattatagg cagggcatgc 1061100
catgaccgac aaaatcggtc gatactcata aattagtgat atagcaacgg caaaagcacg 1061160
atgtctgata aaacctgcag taccatcaat agggtcgaca agccaaaata aagaagtcgg 1061220
agatggagga gggaccaggg tagaaattaa gtcatctctt gagactgaag aagttaacag 1061280
gcgtgtaaat tttaagattt cagggatttt ttcgttgtct tgatcaggat atagagtttc 1061340
ttctccaata aaaggaatat tgggaaaggc ttttgcaagc tgttgtttta gataatattg 1061400
actgccgtaa tcagcagcgg tgataaaaga accatcggat ttttcccaaa aaggaaccaa 1061460
acggtgctcg cttcgatagt ttagtagttg tgtagtgatt tccgtaacta cagactcaac 1061520
gatgttctga tagttaggca actcggagtg catagaattt tcctcaagta tctagataag 1061580
aatgttagca aaagttagga ttatgttaaa cttcgggagt agcactataa tagatagcat 1061640
agtgcttaca aaaaattctt tttccctaag taaggggaac ttatgctaat caagctatgg 1061700
cgagctactt atgaagggat gtatacattt cttgtgggtg cgttgctaaa gttgcgctac 1061760
cgtatgcaag ttgaagggtg ggacacctta aacataaatc ctaagcaggg gtgtttgttt 1061820
cttgccaatc acgttgcaga agtggaccct attatcttag aatatctatt ttggagtcga 1061880
ttccatgttc gtcccatggc ggtcgagtat ttgtttcata gtcgcgttgt tcaatggttt 1061940
ttaaattctg taagatccat tcctatccct caacttgttc ccggtaaaga gagtaagcgc 1062000
tctttagaac gtatgaacgt atgttatgaa gaagcctcac gagctttaaa tagaggggaa 1062060
agcctccttc tttatccttc aggaaggtta tcgagaacag ggaaagagga aatcgttaat 1062120
cagtattctg cttatgtgtt attacataga gttatggaat gcaacgtggt tttagtcaga 1062180
gtttcaggct tatgggggag tgcgttttcg cgctataagc agaactctac acctaagtta 1062240
ggccctgcgt ttaaagaagc ttttcgagct ttactgcgtc gtgggatttt ttttatgcct 1062300
aaaaggtttg taaaaattac tctatgtcaa gtagatcatc tttttttaaa gcaatttcca 1062360
acaaaacaag acctaaatac ttttttggct tcttggttta aatcaaggag atgacaattt 1062420
gcccatagaa gttcccttac gcataacgag gaagttacga cgtatgcacg atcaacggaa 1062480
taggggtcat aacaaccata atttaaggtt gcgacctgga tctacattat tggaagcttt 1062540
tttaatatta tgttccgaac atgaagaggg aattgcctgt tttgacgaac atctaggctc 1062600
actctcttat cgagaacttc gcaatgctat aattgctgta gcaattaagg tctccaagtt 1062660
ttctgaagat agagttgggg tgatgatgcc cgcatctata ggagcattca ttgcctattt 1062720
tggcattctt cttgcgggga agactcccgt aatgatgaac tggagtcaag gactcagaga 1062780
gctacgtgca tgtacaaaaa cagtagaggt tcgacgtgtt cttacttcac agcagttcat 1062840
caaacattta actgaggtcc aagggttcgt agaatatccc tttgatctta tgtatatgga 1062900
agatgtgcgt aaacgccttt cctggtggga gaagtgccgg atagggttgt attctaaatg 1062960

ctccgtccct tggttgctca gaatctttgg agtttcaggg gttgagagcg atgatactgc 1063020
tgtcattttg tttacttctg gaacagaaaa acttccaaaa gcagtccctc taacccataa 1063080
gaatttaatg gaaaatcagg aggcatgcct caaatttttc gatcctaata cacaagatgt 1063140
catgttggca tttctccctc cttttcatgc ttatggattc aatagctgtg gcttgtttcc 1063200
tttactgatg ggcgttcatg tagtattcgc ttcgaatcct ctaaacccta aaaagttagt 1063260
tgagtttatc gatgataaaa aggtcacctt ctttgggagc actccggtat ttttcgacta 1063320
tattctgaaa acagcaaaaa aacaaaattc ctgtttggag tccctacgac ttgttgtgat 1063380
cggcggggat gcattgaaag ataccctcta cgaagaaact aagaaattac aaccacaaat 1063440
tgctctctat cagggctacg gtgctaccga atgttctcct gtaatttcga ttactacgaa 1063500
agaaagtcct aggaaatccg agtgtgtggg aatgccgatc gaagggatgg atgtgctgat 1063560
tatttctaaa gagactcata ttcccgtatc ctcgggagaa cagggattga tcgttgttcg 1063620
tgggaactct gtattttcag gatatcttgg gaaccatgaa catcagagtt ttgtctcctt 1063680
aggtggggat cagtggtatt tgactggaga tttgggtcat ataggtccta gcggggatct 1063740
atttttagaa ggtaggctaa gccgatttgt aaaaatcggt ggagaaatgg taagcctaga 1063800
agctttagaa agtattttgc atgagcattt tactgaaaat caaaatgaag acgcaggttc 1063860
cctagtggtg tgtggtattc ctggggataa ggtaaggctg tgtttattta ctactcttgc 1063920
tacaacaata catgaagtaa atgatatctt aaaaagcgct gaaaccagta gcatagtgaa 1063980
gatatcgtat gtgcatcagg ttgaaagcat tcctatctta ggcattggga aacctgatta 1064040
cgtttcatta aatgctctgg ctgtttcatt atttgggtaa gtagccgttt ctctgggtag 1064100
gttgatacat agtgagtttg caaggagagt ttcttgacta ccagtgatgt tatagatttt 1064160
gtaacaaatg attttctagg tttcgctcgt tctcccacaa tatactgtga ggtaagtaag 1064220
cgtttccaaa tacattgtca gcagtttcct catgagaagc tcgggatccg aggttctcgg 1064280
cttatggtag ggccttcttc agttatcgac gatcttgagt ctaaaatcgc aagctatcat 1064340
ggagctccta atgctttcat agtcaatagt ggctatatgg cgaacctagg cttatgtcat 1064400
cacgtatcac gatctacaga tgtccttttg tgggatgaag aagtgcatat gtcagtagtg 1064460
cacagcctat ctgcaatctc tgggcaacat catacttttc atcataacaa tctggaacac 1064520
ttagaatctc tattacagtg ttacaggata agctctaagg gaagaatttt tatctttgtc 1064580
tcttctgtat attcttttag gggactttta gctcctcttg agcaaatcat agcactatca 1064640
aagaagtatc atgcccactt aattgtagat gaagctcatg ctatgggaat ttttggagac 1064700
gatggtaaag gactgtgcca tgccttaggt tatgagaatt tttatgctgt attggttact 1064760
tacggaaaag ctttagggac gatgggagcc tctttattaa cgtcatcaga agtgaagtac 1064820
gatttaatgc aaaattctcc gcccttgcgt tattctacat ctttatctcc tcatactcta 1064880
atttctatag gcacggcgta tgattttcta gcctctgaag gggaaatcgc acggaagcaa 1064940
gtctttaagt taaaagagca ctttcatgag tgtttcgact ctcatgctcc aggatgtgtg 1065000
cagcctatat ttttaccaca cacctgcttg gaagaagcaa tttctgtttt agaaactaca 1065060
gggatccatg taggcgttgt tgcctttgct aagcatcctt tcttacgtgt gaacttgcac 1065120
gcttacaata ctgtcgatga agtgaacctg ttggctcaag ttatgaagcc atacttagaa 1065180
aaaagtagtc atagggtcca catcaatcat gaatttcacc tttggcgaga gctttgccag 1065240
cattaaggcg tgatggagtt tcttattcac tggaatgaca taagcactct taattaaaaa 1065300
ctgatagcgg aacgtatcct tgattttaaa atgaccgcaa ggagtcactg gcatcagtgg 1065360
attcgtgctt tctagctgct cttttagaat gttatggact cggtgggcct cttcccaagt 1065420
ctgcttggga catttcccca tgaaaataca acggataaga cggataaatg gaggatactc 1065480
acagagttcg cgacctgtga tttcctgact ataaaatgcc gagtagtcct gacgcatagc 1065540
actatgaatt gtgggatggt caggaagaaa ggattggatg agaatttctc caggtaggtg 1065600
gctccgaccc gacctacctg ctacctgtgt aatgagctgg aagacttgct ctgaagctcg 1065660
gaaatcaggg atatacaatc cagaatctcc atttagaatg accgcgagtg tgactgcaga 1065720
gaaattcatg cctttggcaa tcatctgggt gccgatcaaa acatctgctt ttcctgttgc 1065780
aaattgcctg agtaacgttt catggctccc cttgaatttg gtagtgtctg aatcaatacg 1065840
gatggtacgt atctgaggaa aaatctgctg gagaattttt tctatttttt ctgttcctga 1065900
gcctcgatat tgcaacgtca tagttccaag acattttgga caagattgtg ggaggtcttt 1065960
aggtgaggag ttgcatagat ggcagagaag cacatttgca tatttatgga aggtgagcac 1066020
catgtcacaa tgagggcatt tcaatgtatg cttgcaaaca gtacaagaga cgttggtatg 1066080
atatccccga cgattaaaga aaatcaaaac ctgctctccc acttccagac gttcggctat 1066140
tttctttaac acaggctggg aaaatagaat cttggttttt gacttttccc tctctaagtt 1066200
catgttgata agagaaattt ttgcgggatg agcagcagct gctcttgaag agagccgaga 1066260
cagaacgtac ttgccagata gcgcattagt atagctttct aagctgggag tcgcacttcc 1066320
taaaaccaca gtagcatgag cgagtttgcc tcgcattaca gcgacatccc tggcatggta 1066380
gcaaggagga ctttccgttt gtttataggc gggatcgtgt tcttcatcta caatgatcag 1066440
tcccagattc ttcatggggc agaaaagagc agaccgtggt cctatgagga tacgtaggga 1066500
tcttcggaag cttggcgcca cgtgcgactt ttgatcgctg tcgctaagct tgtgatggag 1066560
aacacctaca tccttgccaa agcgcgcttt aaataatgag actgtctgca ctgtgagagc 1066620
gatctctgga acaagaagaa ttgtactttt cccttgttta agagcctcgc ttgttgctcg 1066680
aagatagatc tctgttttac cacttcctgt aattccgaaa agtaaatgtg tatggaattg 1066740
tgaggttttt agtgaggaaa aaattttatc tatagcactt tgctgttcag gatgtaaatc 1066800

cttaggagca ggcgggaaaa aggttaggag gtcttcttga agctctaact gcgctgcatc 1066860
tacaatatca agaatgccca gcttttcaag agaatgaatt ggagattgcg atactttggc 1066920
agtctccata agggaagata aacccggtgg ggacgcatgt tgtagtagaa tttttaaaac 1066980
agccccttga gaaggatgta aaacttctaa ttttgcaaga atctctttag tttttgcttt 1067040
actttgcttt aagacgacac ggtagtgctg cttaggctga atcacattcg aagagatagc 1067100
gggaagaaat aattttagag ttttcccaag aggagcaaag tagtattggc tgatccaaaa 1067160
tagcaagtct agaagatctt gaggaaggac aatctctgaa tcggataatc ctaagatagg 1067220
taaaattttc ttacattggg tcgttgtttt tatttgataa ataactccaa cttttttccc 1067280
tccccgtaaa gagatagtaa cagcagttcc tttagtaatg tgctctagat tttcaggaac 1067340
gccataatcg agtaccttgt tgatgttaga gcctacgatg acttcggcgt ataggcgaaa 1067400
ggtagacgat tcaatatagc ccatagattt cgttttagct gtgaatcttt ttcatactct 1067460
agtgaagaat agaggggaag aaagatacat ccattttgtg tcagagattg atgataatca 1067520
gttgtaggaa ttttataacg gatcacattt aaaggggcta gagctaaaaa aaaattgggg 1067580
ttgtttacga agatattcgt cttcgcatga atcaaagtca actttgtagg gaaaagttgt 1067640
tgcgtaagaa tttttgctaa tctgttaaaa aacagcactt cttcgttgtt ttcttcataa 1067700
acaaaaatag agcaggggat cttcggagca ggaagactac aatcttttaa agcgggatat 1067760
ttctctttta aaatagcatt ttgtgacagg tctggatgca gaggaacaca tttccaatct 1067820
gaaggcttag tcttttcttg agaaagattt gtcggtggag gaggggtgat tggttggggt 1067880
tctgcattgg ggacttctac agctttctca ggtatagaag aaacacttgc agagattgca 1067940
gtattcggaa gagttacatt caggagagat gcaagtgttg gatagagttc ctcagaataa 1068000
cgatccaaaa gttgacagag agtgtcatga aaattttgag agttttccat aaatagccaa 1068060
tgtttctaaa aaagagaggt acgttttggt acccataaaa ggagagctct ctgtggataa 1068120
gaatttctat tcttatttta tcctaaatca gataatagtg tcatcatact caacaaactt 1068180
attcgacacg tttttttaag gatagtgaca tgaaattttg gttgcaagga tgtgcttttg 1068240
tcggttgtct gctattgact ttaccttgtt gtgctgcacg aagacgtgct tctggagaaa 1068300
tttgcaacaa actcgtccta tagcagctgc aaatctacaa tgggagagct atgcagaagc 1068360
tcttgaacat tctaaacaag atcacaaacc tatttgtctt ttctttacag gatcagactg 1068420
gtgtatgtgg tgcataaaaa tgcaagacca gattttgcaa agctctgagt ttaagcattt 1068480
tgcgggtgtg catctgcata tggttgaagt tgatttcccc caaaagaatc atcaacctga 1068540
agagcagcgc caaaaaaatc aagaactgaa agctcaatat aaagttacag gattccccga 1068600
actggtcttc atagatgcag aaggaaaaca gcttgctcgc atgggatttg agcctggtgg 1068660
tggagctgct tacgtaagca aggtgaagtc tgctcttaaa ctacgttaag ggattttata 1068720
acttattttt gagaccttcc ttttttaagt tgaatgtgag aactttctta caaacccttg 1068780
gattaggata aggtctcaaa aaagtgcgta ggatattaat caggttgctg agactttctg 1068840
ttttcagaat ctaaataatt agagatcact gctgctaatc tgtctactac atttggactg 1068900
agaggattta gatggagaag atccctttcg ggtataggaa ttttagtccc cgaggttttt 1068960
acttcaggaa gctctagaaa aggtgttnct acgcaatttt ctctttcgaa gaggccgtcg 1069020
ctaatgagtt cttgatcatg attagagttg taaatgaaaa tttcgggaca acgcaatctt 1069080
tcgctaggtt tcacagagtc tatgttccaa ccaacgagtt ttataatcgc ggaagctatg 1069140
ggcttacaaa tttgattcgc gacatctgct agagagcgag ggccccgatc ttttacaaca 1069200
atccatgaag ttccatcact gccatcagtg acctcacgat ctagagcagc agcttggaca 1069260
ctagttccca aagagtatcc gaaagctatg atttgattgg ctttaggacc tgtctcttca 1069320
tctcgtaggt agcgtacgca tgcctgatac gatttaacca gattttctcg tttcgcttct 1069380
cctttgctgg acataattcc aggatagttg aacacaagaa gattcgagcc ggttgctttg 1069440
gctagctggt gtagagagga atcgccccga tcgaacaggt tttccatcaa tccagagttt 1069500
ccttgagaat aaagaatcca cctgtgggga agagcatgag gtatctttat agctaagcta 1069560
tcaaccacca aatcgtcata ttgtataacc acacgatcgg cagctgagat ttccttaagg 1069620
cttaggagtt gtttaatatg ggacagaaag gtcattcgcc gaattgccac agctgttttt 1069680
gacatgctct gagatatgat agaagatggg agaatgaaaa actgacaggt tttcttgagt 1069740
acccacaata acgctaaggg aaggaacaga atgattgaga taataaactt cacaatattc 1069800
caaatcgttc ctagaacttt aaataaggtg ggatgggcct tccgtctttc agaagctatc 1069860
tctgtttttt ttgaagagaa catgattaaa gacggctttg gatccgtctc tagaatcgta 1069920
tcatcacgaa agtttattgg ggtaggggat gggatcatac aagagtccta aattttaggt 1069980
acacagagta taaagagaac aaaagaaaaa gaatacgcat tataattttt taattttttc 1070040
tagagagact ctcaaggatt gcttcagcaa gcttttgtat tgtagggtgt ttcagagggc 1070100
ttgagtggag tagatttacc tctcctataa attttttact cctggcaaat ggggttcgtt 1070160
ttattgcatg agcaagagta aactccggga gcaatgcggt atcatcacca acttctgaag 1070220
ggcgaaatcg atccgcagaa taaacaagaa tttctgggca aggaagctct cggctgtttt 1070280
tttccgcatc cattttccat cgagcgagga ctgcgatgag ttttcctatt ggcccgaaga 1070340
aactattagc agctgcgggt aaagagtggg gagcacggtc tttgactgct acccaagaag 1070400
tctcgctatt tgtaaaagga ttttttgcaa agctgcagac tggactaccc ctcctaagga 1070460
atatcctagg tgatgatttc gttagcccca gggccctgaa ttttatcttg taggtacttt 1070520
gcacatagat tatgactgtc gctaggttct ctaagttcaa ttttcctgtg ctcgacttaa 1070580
ccccaggata gttgtaaatt aggatattag cgcctagcag tttagctaac tctttccagg 1070640

agagaaacaa ggaatctttc aggcctatca tgtcttcgag gaaatctcca cttcctagag 1070700
aaatcagcag ccaacgttta ggccttgccg tactgaaatg tattgctaaa gtatcaatgg 1070760
taagatggtc ttgttggatc gtgattcttt tactttccga gactgctggg tctttgaaag 1070820
agggatctag attctttaca aaataatctt gtatttcctg ttccaaacga tactttttaa 1070880
agcaggtttt caagacgctg taaagcatcg aagaacgggg gaaaagagcc aaggaacata 1070940
ctaattgaca taaccaataa attcccaggg gcggaataat cagaccgatt aagagtttga 1071000
aaattttaac aatgatcgta acaattttgg tgagtatggg gtgcttctta ctaaagtcac 1071060
ttaactgctt ggcgtgtgtt gacgaaaaat aggaaactgg atgaggattt gaaaatattc 1071120
ctgacaaaat atcagacctg tttgaaagat tcaaaagagt gaatgcgaaa tccttacgtc 1071180
tctaatctca agggactggg gacgtcaggt caaacaaagt tcagtgttat cggcggctgt 1071240
ttttcgcgct tatattataa cggtgtcctt agaaagatta aatagttaag tgcctttaaa 1071300
tctgattctt aaggagcatc ccttctttct aagagagctt tttcaaaatt ggtgtggcaa 1071360
gagccactct tgttttgctg tcgatgggat cgatatcaga cgataatcgt acttctataa 1071420
attctttatt ttgaacatag ggactatttt ttatcgcatg agcgagagta agttcaggtg 1071480
ctaagagctt gttctgtctg actgttgatc ttcgtaagga atccgtagga tagagaaaaa 1071540
tttctaggca gggaaggtct tggcttctct ctacggcttt ggtcccccag ccaaaaagac 1071600
gagctactag ctttcctatg cgtctgcaac tgtggaaacc ttctggagat ataaagagag 1071660
gacacctatc tttgactgct atccaagtag tatcatcgtt tgcaacaatc ttctggtctc 1071720
gcaatgcttc tgcttgtatc aaacctccta gggagtaccc ataggtaatg atttcttttg 1071780
ctccagggcc ctgttcttta tctttaaggt atcttgtaca aatattatga gctgatgcta 1071840
ggtcctttag gctgctgctc cctgtgctgg acatgactcc ggggtagtta taaacgagta 1071900
tattggcccc tatcaacttg gcaaatcttt gccaagaatc aaagatctcc ttacaagcga 1071960
tttcttccaa gctacagtca cttcctaaag aaatgagcat ccaacgattc gtaggtgctt 1072020
gtgaaaggca tatttccaaa gtgtcgatga gaacattatc ctggaggata ggaactcgtc 1072080
tcatggaagc aacgcggttt ttcgaggaat aatcttgcaa agcacgcaaa taattagttt 1072140
ttaaggtttt agtgttgggt tgcttcttga aaatttttaa taaattcttg gaagggagaa 1072200
tcgagtttgt acaaagcgtt tgacatagcc agtagattcc taagggaagg atgatcagcc 1072260
ctatcagaac tttaaaaatt ttaataatta cagaagcaat ccgtgtaagc acgggatgcc 1072320
tgtctttaaa ctcatgaagt ttttgggcgc gtgttgaaga aaaataagtc gcttgtggag 1072380
aagggtggga atgtaaaata tcctcaacaa acgcattact tccgtgaatt ggagccatga 1072440
acttttccta taaaatttaa gttcttttta cggaactact tggtttttga tttgttcgta 1072500
tagagggggg gggttcaggt agaatgttgc cgatatggag tcgtctagag caggcttcaa 1072560
tgtctgtcca caaaagtttg tggtcttttc tttattgcca tccaagaact gatctagctt 1072620
tacagttagt aagattttca ggaccgaatt ctaagagaat aaattttaag caataaggga 1072680
ataaaaaaga atcatatctt cactcggttc tgactttgta aagaagttta tcctcattct 1072740
tgattacacg aatctaacca actataaaca aatttttttg aggagaaaat ttcttctact 1072800
gttttggcct gaactcaagt tgtttatcta aaagaactta aaaaatacaa cagctctttc 1072860
tttagaggat acagaaagtc gagttataga tagagatttt actaacttaa ggataaatat 1072920
taaaatattt atttaatatt ccaagagttt ttatttgata gtttctattt ttaattttaa 1072980
tttattaatt ctttaaaata agatgagcga gctcgccccc tgctcgacag gattgcagat 1073040
ggtcccccat acgcaggtcc atcatgccct tgatacgcgg agagtcattc taacgatagc 1073100
cgcctgtctg tctttaattg caggaatcgt gttggttggc ttaggtgctg cagcaatcct 1073160
gccctcgctt tttggagtca ttggaggaat gattcttatt ctgttttctt cgatcgccct 1073220
catttattta tacaagaaga caagggaggt ggatcagatt gctctggagc ctcttcctga 1073280
gatgatttct aaagatcaaa gcattataga ttttgtaaag acacgagact atgcatcttt 1073340
agaaaagaaa gcgacctttg cttatactca tactcattat tacgatggaa gcatggtctt 1073400
ctatagggag atccctagat ttatgttagg ctcttatctc gcgcttcgca aagacatgga 1073460
ccgccaagct cttttttgaa gaaggacatc catgttatgt tgctcgccag gcttttaatt 1073520
cagggattcg actctatcgt cagttatttg acatagaata tttaacgaag ctatacaaaa 1073580
acaaggatta cgatcctgag catcgacaaa gtttaaaaag cctaatagat tggatgaaat 1073640
gcgaagacca tggggaagac tttttgcgtc aacatcgggg ctgtgactac ccatcttctg 1073700
gctatacggg aatgtttttc aaacattctt aagtctttgt ccgcatcctg aatcttgcat 1073760
ctagaatttc tttttcataa tctcatcctt catggtttag aagatttgtt ttggtgtgtc 1073820
ttagttcacg caaaaaagtg ggagagactt gtaagggaga cttctttaac taaatgacta 1073880
tcgattgaaa gaaggagttt tgtatttaga aaagcgtatt cttcttaaat tttttactga 1073940
tacatacata gaatgtatag aagtatcaga atcttcaaaa gactcagagt gatcaacaac 1074000
agtggatcca tttatagaag taaaacaaga gctgtactac catttagaat ataggccttg 1074060
gttttgctcg gggaaattcc atgatatctt tctttatgaa tctaaagttt tcctagagcg 1074120
aagagagtca aaaactcagt aagctatata aaatttgccg tgtaattaaa gcatcagata 1074180
ctctgtctgg tgggatcggc ctgcttttta caactttaga tagatagatt ttagagtctt 1074240
gggttggaaa agcgatatct atacaaacat actagaggaa agaatgacgg cgagagcaga 1074300
atatttagac catgaggatt ttttatatag gagtcacaaa cttcaggaac tttctgagtt 1074360
gggtgtagtc ctttatcctt acgagtttcc tggagttttt tcatgcgaag atattaaaaa 1074420
aacgtttgct agtcaagagt tagggaatag tgaagctgct atgagtaggt ccactcctag 1074480

agtgcgtttt gctgggcgtt tggttctttt ccgtgcgatg gggaaaaatg cttttgggca 1074540
aattttagac cataatcaaa ccatccaagt gatgttcaac cgtgagttta cctccgtaca 1074600
cggactttct gaagatgctg agattactcc gattaagttt atagaaaaaa agttagattt 1074660
aggagatatc cttgggatcg atggctattt atttttcacc cattccggag agcttaccgt 1074720
tcttgtagag actgtaaccc tactttgtaa atccttactt tcgttgcctg ataagcacgc 1074780
aggtttgagt gacaaagaag tccgctaccg caagcgttgg ttagatctaa tttcctcaag 1074840
agaggttagc gatacctttg tgaaaagaag ctatattatt aagcttatcc gtaattatat 1074900
ggatgctcat ggatttttag aggtagagac tcctatatta caaaatatct acgggggagc 1074960
agaagctaag cctttcacaa caacaatgga agctttacat tcggagatgt ttttaagaat 1075020
ctccctggaa atcgctttga agaaaatcct agtaggagga gctcctcgta tttatgaact 1075080
cggtaaagtc ttcagaaacg aaggaatcga tagaacacat aatcctgaat ttaccatgat 1075140
agaagcgtat gctgcttaca tggactataa agaggtcatg gtctttgtag aaaatcttgt 1075200
tgagcatctt gtccgtgctg taaatcacga caatacctct ctagtctatt cttattggaa 1075260
acatggcccc caagaggtag atttaaaagc tccttggatt cgtatgacaa tgaaagaaag 1075320
tatcgcgacc tatgcaggta ttgatgttga cgttcacagt gatcagaagc ttaaggaaat 1075380
cttaaagaag aaaactacct ttcctgagac tgcatttgcg acagcttccc gggggatgtt 1075440
gatagcagcc ttatttgatg aacttgtctc tgataattta atagctcctc accacattac 1075500
agatcaccct gtagagacaa ccccgttatg taaaactctg cgttccggag ataccgcatt 1075560
tgtagagcgc ttcgaaagtt tttgcttagg gaaagagttg tgtaatgcct attcggaact 1075620
caatgatcct atccgtcaaa gagaactctt ggagcaacag catactaaaa aagaactcct 1075680
tccggatagt gagtgtcatc ctatcgatga agagttttta gaagctctgt gtcagggaat 1075740
gccccctgca ggaggatttg gtataggtgt cgatagactt gttatgattc ttacaaatgc 1075800
tgcatctata cgcgatgtgc tgtatttccc tgtaatgcgg cgcttcgatg cagagaagac 1075860
gaattaaagg ggcttaactt taggccctga cttagagtct tcgacaagga agcctgcggc 1075920
aagtatctca tcacgtaagg tgtctgccat cgcccaattt ttagtttttc tagcttcctc 1075980
acgttctgca acaagttgca ttacggtttc tgggatgcac acgcttgtgg ttagaggaag 1076040
cacaccaagt acagtatcga cttttttaag agtgtctaag atatagagag agtcggcttt 1076100
agaaaagtgc ccttggtcga tcaaggtgtt gatttcgtga acaaaatcga acagagatgc 1076160
aaatcctgta gaaacattta agtcattagc aagagctctt gagaaagctt ctatgaattg 1076220
actgctagag tccaaggttc tgggaagggg agattcccca gggagatcta cgccttcaag 1076280
tcgagaaaca aaatctttta agcggcgtaa ggcatgccta caggcaagga gagcttcttc 1076340
tgtaaagttg agttgcgtac gataatggct ttgcagtagc atatagcgta cttcttgacc 1076400
agtaaattcc tggtggagaa ggtctcgtag agtgaggaaa ttccctaagc tctttgacat 1076460
tttcttccca tcaataagca gatgttccga atgcagccaa tagcgtgcaa agggtttccc 1076520
tgaaagagcc tcagattggg cgatttcatt ttcgtgatga ggaaatatat tatctacacc 1076580
tcccgcatgg atatccaaag aatctccaag aagttccatc gccataatcg aacattctaa 1076640
atgccatcca ggtcttccta ttcccgaaag gactttccca ataaatgacg ccatcacgtt 1076700
ctggattgta tgctttccac agtacaaaat cacttgggtt ttctttgtca tactcatcag 1076760
cagaaattct ggagcagcag cgtaagctac ttagatccag atgagagagc ttcccataat 1076820
ttggaaaacg gtttagagaa aaatatacgg aagcatcctg acctatatag gcgattcctt 1076880
gctctaagag ctttgtaatc gcttggatca tttgagggat gtagtgtgtt gcgtgtggat 1076940
agaaatctgc tctagcaata tttaaagtat cgagatcttc aaaaaaggct tcggtatatg 1077000
gctgcgtgta ttcttgcagc ggaatatttt ttttcgaggc tcctgctatg gttttgtctt 1077060
ctacatcagt aatattcatc acatgggtta cagagtagcc aaagaatact agagtgcgtt 1077120
tgagaatatc ttcaaagaca taagttcgga agttccctat gtgagcgtag tcatacacgg 1077180
taggaccaca agtatatagt cttacagggg tgtgattggg gaaaaaaagt tctttttttt 1077240
gagatgctgt attgtagaaa tagagaccct cgatatgaga aaatgccata actgtatcac 1077300
tcttgactca ggagtttgtt gttatataga cagagagaat ttaacgtcag tctcttcggc 1077360
ctatcctaac aaaataaatt ttctagatct atgatttttg ctttcgaaaa tcttatagga 1077420
gaaaagatag caaatttcct tcttttcaaa agaaataaaa aatcgcataa atattcttag 1077480
aatttcatat ttattcatgc aatagaagct aaactttttt tagtagcgaa gttcaacgtt 1077540
tttaagtagt gtttttttatt tcgttttaat aaaaaaaaga ctatacaaca agagtcgcta 1077600
gaaagatgtt ctcgaggcat atctggcccg ccctgaatag aataaattgc gtcctaatct 1077660
taggaaagga ggaggtagcc cgtgatcctg ttacaaaata tcaaaagatg ttccttaaaa 1077720
cagttgaagg tcttggctac tcttttgttg agcttgagct tacctacgct agaagcagcc 1077780
gagaacagag attctgattc tattgtttgg cacctggatt atcaggaggc tttgcaaaag 1077840
agcaaggaag cagagcttcc tttgcttgtt attttttctg gttccgattg gaatggccct 1077900
tgtatgaaaa tccgtaaaga ggtgttggaa tcacctgaat tcatcaaaag agttcagggg 1077960
aagtttgtct gtgtggaagt ggagtatctg aaacatagac cgcagttgaa aacattcgtc 1078020
agcaaaatct tgctttgaaa tctaaattta aaattaatga actgccctgc atgattttgc 1078080
tctcacatga ggagagagaa atctacagaa taggttcttt tggtaatgag acgggttcca 1078140
atttagggga cagtctttgt catatcgtag agagtgattc tttactaaga agggcgtttc 1078200
ctatgatgac gtcgctatct ttatcggaac tgcaaaggta ttaccgactt gctgaagagc 1078260
tctctcataa agaattcctc aagcatgctc ttgagcttgg ggtgcgcagt gacgattact 1078320

tctttttatc tgagaagttt cggttattgg tagaagtggg caaaatggat tctgaggagt 1078380
gtcagagaat taaaaaacgc cttctcaaca aagatcctaa aaatgaaaag caaacccatt 1078440
ttaccgtagc cctgatagag tttcaagaac tggcaaaaag atctcgagct ggtgtgcgcc 1078500
aagatgccag ccaagtcatc gctcctctag agagttatat ttctcaattt gggcagcaag 1078560
ataaggataa cttgtggcgt gtagaaatga tgatagcgca gttttactta gattctgatc 1078620
aatggcatca tgctttgcaa catgcagagg ttgcctttga agctgctcct aacgaggtgc 1078680
ggtcacacat ttctcgctct ttggagtaca ttcgccacca gtcgtagctt tcgtttttcc 1078740
aagacgatgt aacccctctg ggatttgatt tataaaatcc tggagaagtt ttgaaaagac 1078800
agggcgctgt ttatgtccct tggggaaaac aacaatttga caattgggaa gttggtggcg 1078860
gacatgccga aatacttcac gaacgacacg tttaaaagag tntctttcgt gagctttgcc 1078920
aaatttttta gatacagtaa tacccatcct acaagtcccg ggatgcctag aagggaccac 1078980
gtagaaagtc gcctgactac cacgacaaca aaaacctgaa cgcgtaatgt aaagaaattg 1079040
ctttcttttc aaaacgcggg attgttttgg taaggtgagt gggtgcacga aatactaaat 1079100
tataaatcga ctagggaatg cctaccgtgg cggcggcgac gattcaaaag ttttcttcca 1079160
tttcttgtgg ccatacgagt acggaaaccc acagaatttc ggcgtttcct tttgctaggt 1079220
tgataagtcc gtttcacagt gtcttcctat aattccttgc ctcattcacg gttgagtctt 1079280
actaaagagg ctttgcccga aattttagaa gattatatca ataaagatca ataagaagac 1079340
atcggctttc ttccaactta tctaagaaaa ttcgagagat gacagtctgg tattgtcaaa 1079400
aaagctccta ggagattaaa ttggttcctt gttgtaactt agtatgcttc tcctttttat 1079460
gagaagtgaa ggtaatatct catgaaagtt agttcatctg ttaaggctga tccatctaag 1079520
ggggacaaat tagtccgccg taaaggacgt ctttatgtaa ttaataagaa agatccaaat 1079580
cgaaagcagc gccaagcagg acctgcacgt aaaaaataat ttaaaaattt aggtaaaacg 1079640
catggcgaaa aaatcatcag tagcaagaga agctaagcgt cgacgattgg tagaggctaa 1079700
ttttaaaaaa cgatccgacc ttcgtaaaat cgtgaaaagc ttgtctgtta gcgaagaaga 1079760
aaaagaaaat gcccgtatct ctttaaataa aatgaagaga gatacttctc ctacacgttt 1079820
gcataatcga tgcttattga caggccgtcc tagaggctac ttaagaaagt ttgctatctc 1079880
tagaatctgt tttagacaaa tggcttctat gggagaaatc cccggcgtta ttaaggctag 1079940
ttggtagtct attctgatta gagccccaac cctatttaag gacgtggggc tttattttga 1080000
tgcttctttc aaagcctgtt gtagtgatgg aacatccttc actcgttgcc aatctttcat 1080060
tccctttttc catacccaaa tttcttctgg atacgttttt ccctttaaaa gtacgaccaa 1080120
ctcctcaaaa gatatcggac ctacgttttc tctatcttta tttaaataaa accacttctc 1080180
tgtatcgata acgatttctt gaagatctcc cgatgagggt atctcgtcat tacctgctaa 1080240
actttttttc aaatcatcaa aaagatcgga gttatcaaaa ggatcgtttt gtggcttttc 1080300
taaagcgttt cgacgagaag gaagaagaag aaggacaact agaccaataa atccaaaaaa 1080360
tgctcctgca aaaaaccagc caataacatt tcgttttttc ttatctgcta tgtaggcaga 1080420
tagacaacct agaatcacat aaaataataa aatcgaaata gggagcatag tgcgtagcgt 1080480
acctgaaaga ttaatagtct gtcactaacg cctattattt ccatgtgttg tatttctcgc 1080540
tagagaaata gggcacgttt cataaggaaa cgtagaaaat acaagtctta aattaaaaat 1080600
atcagttcaa gaagaacaat ttcagaactt ttctgatgcc atatagtagt gaaaaagaga 1080660
aggtagatct atagacgtta aagtggataa aaaaactcat aggcaagagt gcgacttttt 1080720
ttctttacta gcagagcggt aggggaacct agaagttgga tctaatggat gtagcatatt 1080780
aaccgctgga cgattagact gtcgctaaca cttattattt ccaccgtgtt atatttcttt 1080840
agtgaagaaa tagagctcat tggaggagga aaaatggaaa aacaaaattt aaaattagat 1080900
gtcaaagaga ttgagtttcc tgaaacggta ttcagccgtg atatcgaaac tcgtgttatc 1080960
caagtaatta ttttgcattg tttagcaaaa attaacggtg tttccctcct cggaggaaat 1081020
ctaatagacg ctctgttcgg tagagatatc gaaagaatga aggggatcta tgtagaacag 1081080
gattcaaaaa atcatctggt caaagttcgt gtcgaagtga acgtagatta cggtgtttct 1081140
ataccagaga aaacagaaga aatccaggga tgcattgttt cagaaatttc agaatataca 1081200
ggacttcatg tggccgctgt ccacgtgatc attaaagggt tgacacaacc aaaagatcgt 1081260
attgatgaag aaattgaaga ggaagtctct gttcaagatc ttccttcacc tgaagacttc 1081320
ttacttgaga attctgaagg gtagacttaa tcagatttgt tgaagtcttt ttgccttgca 1081380
agaagcactg caatatcttt cttagttagc ccaggaatag cttccagctc ttcctgggag 1081440
ctaagcatta cctgtttcca acttttaaat ttctgcaaca atcgttttcg ctttacctct 1081500
ccgattccag ggattttttc ctgttcaaat aaagcctttc ctcttttttt cctgtgctta 1081560
ctaatcgcaa agcgatgtgc ctcatcacga aggatctgaa aaaattgtaa taaattagaa 1081620
gtcgggggaa gagaaaaacc ctctgggaac gtctcgcaga aaatcttctc cttatttaac 1081680
cctcgactat gattactttt ttcttttgcg atagtaacaa cctgaattcc tgtaagattg 1081740
agagtttgga ttattttttt tgtcttgtta tagtgcgttt tccccccatc aaccacaatc 1081800
atatcaggaa gagctgtggt cagtgaatga aaccttcgta agagcacttc ttctaataga 1081860
gccaagtcat tctgagtctt ttcagaatct atggaaaacg tacgatattg cttgggatcg 1081920
aacccattat tttcaaatac aatgtacact ccagtagcgt gagcaccttg catatgagca 1081980
ttatcgtagc attctatccg atacggatat tgtgacatcc gcagtattat tcttaaagtc 1082040
ttggtagggg agcttgatga aggaagtgta gttgctgcat acgccttggc attgcgatag 1082100
gctagatcaa gaagttcttt tccatatcct gtttttggag aacgtaggcg gggaggagac 1082160

tccgcattca atacgtagga gagagtagga aattctaggg gaagaggtgt cagaatttct 1082220
ttaggaatgt agggttggct aacataatat tgcaaaataa aagaggagag caagtcttga 1082280
tcttcttgag cattctcaaa gaaagagaaa tggcgtgccc caagaagctt ccctgagcgg 1082340
acagtaagta gcgtgaggat ggtgcgttgt ttatgtctgt aaagaccaag agcatcaata 1082400
ttttgaaaat gaaatttttc cacctgttgc tttgccatag cttgcttaat tagggacaac 1082460
gtgcggtagt aattcgcagc ttgctcaaat tctaagttat cggaagcctt ttgaatcact 1082520
ttttctaagt ccttgaccac ttcttcgatc tttcctttaa gaaagaggat cgccttgtct 1082580
aaggttcctt gatattcttc aggagtgcaa tagccgacac aaggagcaag acagcgcttc 1082640
atgtcataaa gaatgcaagg gcgtttcctt aaggcaaact ctcgatctga acaagtcctc 1082700
agaggaaacc attggctgat aacctctaag agagtgtggc aggcttcagc acttacataa 1082760
gggccaaaaa tcaattgtcg ttgtgaagag gttatcgcct tcgtgcggat agcttccact 1082820
ttgggccacg aatgtgacag ggaaatcgca aggcagaaaa aggttttatc atctttgagc 1082880
aacacattgt atttaggatg gtgctgcttt atcaaattgt tttctaaaag gagggcctca 1082940
gtttcattcg acacaacaat agtttcaata gacgcggtct ttttcatgag aaaagggatg 1083000
cgttctcgag agtccccttt ctcatgaaaa taggatgcta agcgattttt gagattttta 1083060
gccttgccaa tgtagaggac ttgatcatgg acatccttca tcaggtagac tccaggagag 1083120
gaaggaatga gtttgagaga aaaatcctca atgcgcataa ctagaataat gtcagttgtt 1083180
gcatcttatc ttgagcaggg cgtgtaatgg attccgggcc ttctaattgt cttaggatct 1083240
gctgagctct cgataccaca caaaggggaa agccagcaag cctggcgaca tgaatgccga 1083300
aacttttttg tgaatgtcct tgagaatctc atataagaaa acaggctgcc ctgctttatc 1083360
tttaactccg gcatgaaaat tttcaacgtg cggacaatgg tcttctaaag tcgtcaattc 1083420
tttataatga gttgcaaaaa gcgtcttagc tttctttttg tcagtgaaaa ggaggtactc 1083480
tacaacagct tgagcaatgg caaggccatc ataagtactg gttcctcttc ctacctcatc 1083540
taggatcact aaagagcggt ccgtggcatt gtgaaggata ttcgcagttt cggccatttc 1083600
taccataaaa gtagacattc cttttgagag gttgtctcca gcgcctatcc tggtaaaaat 1083660
tttatcaatc actccgatgt gagctgattt tgcagggata tacgacccca tttgagccat 1083720
aatcacaaga agagctatct gacggatata ggtagatttt cctgccatat taggcccagt 1083780
aagtaagatc attcgagttt gagagccacg catttcagtg tcattgggaa taaatttccc 1083840
tgtgtctaca agagtttttg ctacaggatg acaaccacga taaatacata aggtgtcact 1083900
catatctaca cgaggacgac aatagccctg agcatgtgca agatccgcaa gagaaatgat 1083960
gtagtctaga tcagcaagac tttgagatag agccaaaatc tcagtgcgta actgtaggat 1084020
gtgtgagcaa agatccttga agaattgcgt ttctaaagtt tgaagttttt cagagatatt 1084080
agacatatca tcttggaatt gctgcagctc aatagtagta aacctttctg catgcaatcg 1084140
tgattgccga cggatgaaat ctttaggaag ttgaggagca aactcgctgc tcacttcaat 1084200
ataatatcct aaagcctgag caaagcagat tttgagtttt ttgatccctg tttctttacg 1084260
aatgcgttct tggtattccc aaatccattc ttgagaatgc tcttgattgt ggcgtaagcg 1084320
ctttaaatca ttgtggaact catccacaaa aatatttcca tcagaaactc ggagagggag 1084380
gtcgccgttc aaggactttg aaagcagtgc aattaaggag gctagctttg tatctaacga 1084440
gcatttatct ataaagaatt ctggaagagt cgcagaagcc agttgttcgt agatttgagc 1084500
tcctgcagaa aaagaatctc tgagtgtccc gatatctcta ggccctgcca atcctgtggt 1084560
cactttggtc atcagtcgtt cgatatcacg tacttgacaa aggtacgttt tgatattttt 1084620
tcttaaagtc acttgccgaa gaaaaattct acagcatctt gacgtacaag gatctcctta 1084680
gggttataga aaggactgat caaaatttga cgtaaaagtc ttccccccat aggtgtgctt 1084740
gtatgatcca tgatacgtaa gagagaattt ttcccctgag gatcattcaa gggagcgagg 1084800
agctcaaggt tcacttgaga tgccgtatct attaataact tttgctgttt tccacgcgtt 1084860
tggggaatcg caatatgttt cgtaggtaat aaaagtttat cttggatata agagagaagg 1084920
cctccagcag cgttgatcgc tgggacgagc cctttcaggc caaatccatc tagggaggcc 1084980
acttgaaaat gcgttgtaag tttttgagag gcgaacttat gttcaaaggc ccagtccgca 1085040
tacgtagata aggtcagctt gaggtgttgt tgcaactgca tgacaatagc agtttcttta 1085100
ttataaaatt tgttacacga gagcacttca gaaggagcaa gacgacagat ctcgtcaaca 1085160
agctctttgg tattctcaca ttcttcaata aagaaagatc ccgtagaaag atctaaacaa 1085220
gcaaaaccaa acagagaccc gatacgagtg atagcaacaa tatnattatt gaatttttcc 1085280
tggagcaggg tggaggatag caaagtgcca ggagtgacaa atctctgaat atcccgggcc 1085340
atgggaccga tttttttact ctctttttct tttgcaggct cgccaaattg ttctgcaaca 1085400
gcaactttga atcccttgcc gatgaggcga tccacatagg tatcaacagt agatacagga 1085460
atcccactca taggaattcc ctgtcgttgt gttaaagtaa gttccaaatg ttgggataaa 1085520
agaaccgcat cgtcataaaa agcttcgtaa aagtctccca tccgaaataa aagcacagaa 1085580
tctccagctt tttctttaca ctgatgccat tgttccatca tcggggtagg ttttttttcc 1085640
gtcataactt attcgcaatg tacgttattg ttttcatatc cattaggatt caaatgtatt 1085700
gagctaattg attactgctt tattaccgtg gcttttgcta acggttgctt tgactataca 1085760
agaaagtaga gttttcgaac gagcctctaa aattcctgag agacagcctc agttgtaaat 1085820
ctagagtggg agttaagaat gctgcattac gagccggtcc ccttgagaaa agataaacag 1085880
gatgggatat taattccaag gcaattctcg gtagtaaatc cttccctggg atgctgtaaa 1085940
tagaaaattt ctttttataa gaattcccta atataaaaga aattcatcca gaaagaaaag 1086000

ctgcattaga caaaatacag aggtaaatta ataattgggg agagtctctt tattttatag 1086060
agcctcagtc tttttaaact aagaaatctg taagttaaaa ttgcagcata accaagttgc 1086120
gtaccgctat gtatacggaa gagagcttag ataacctgag acacagtata gatattgtgg 1086180
atgtcctttc ggaacacatc catttgaagc gctccggtgc aacatacaag gcatgttgtc 1086240
cttttcatac agagaaaaca ccttcgttca tagtcaatcc tgcgggtgca cactaccact 1086300
gctttggatg tggtgcacat ggagatgcca ttggcttcct catgcagcac ttgggatact 1086360
cttttactga agccattttg gtattatcta aaaaatttca agtagaccta gttctccaac 1086420
ccaaggattc cggatacacc cctcctcaag gactgaaaga agaattacgt cacatcaaca 1086480
gtgaagctga aacttttttc cgttattgct tgtatcacct tccagaagcg agacacgctt 1086540
tgcagtattt ataccatcgg ggattttctc cagatacaat cgatcgattc catttgggtt 1086600
atggaccaga acaatctctt tttctacaag ccatggaaga aaggaaaatc tcacaagaac 1086660
aactgcatac tgcagggttt tttgggaata aatggttttt gtttgcacga agaatctctt 1086720
tcctgtccac gatgcgctag gacataccat tggattttcc gctaggaaat ttttagaaaa 1086780
ctcccaaggg ggtaagtatg tcaacactcc agaaactcct atattcaaaa agtccaggat 1086840
cctctttggg ttaaattttt cacgtaggag aatagccaaa gaaaanaaag tcatcctagt 1086900
ggaaggacag gccgattgcc tgcaaatgat agattcagga tttaattgta cagtggcagc 1086960
tcaaggtaca gcatttacag aagaacatgt gaaagagtta agtaaattag gagttttaaa 1087020
agtcttccta ctcttcgaca gtgatgaagc aggaaataaa gcagcattac gtgttgggga 1087080
tctttgtcaa actgctcaga tgtccgtatt tgtctgtaag ctaccacaag gccatgatcc 1087140
cgattctttt cttatgcaac gaggaagctc aggactcatt gctttattag agcaaagcca 1087200
agactatctt acgtttttga tcagtgaaaa aatgagttct tacccgaagt ttggccctag 1087260
agaaaaggct cttctagttg aagaagcgat tcgtcagatc aagcattggg ggagtcctat 1087320
tcttgtatat gagcatttaa aacaactagc ctccttaatg atggttccag aagacatggt 1087380
attgtcttta gcaaaccctc aggtaacagc cgaaccacaa aatattccca taaaacaaaa 1087440
agttcccaag atacatcctc atattgtgat ggaaacagat atcttgcgtt gtatgctttt 1087500
ttgtggatcc aatactaaaa ttctctacac agcgcaattc tactttgtcc cggaggattt 1087560
caaacatccc gaatgtagga aattgtttgc gtttatgatt tcctattacg aaaaatatcg 1087620
gaaaaatgtt ccctttgatg aagcctgtca ggtactttct gattctcaga ttcttcaact 1087680
gttaaccaag cggcgcctaa acacagaagc tctcgatact atcttcgtac aatctcttca 1087740
aaaaatggca gataggagat ggcgagagca atgcaaacct ctctctctta accaaaatat 1087800
tcaggataaa aagcttgaga ttttggaaga ctatgttcaa ttgcgtaaag atagaacaat 1087860
aatcacactt ctagatccag aaagtgagct cattccttaa cccagctctt cttttgtaaa 1087920
gaattcttgt ttttctaaat cccccggatt tcttagatgt ttcctaattt ccttggagga 1087980
ggaacctgca atgatgtgcc tttcaataaa ccggaaagcg gcgtaattcc tattaaaagc 1088040
aacttactta taatttattg aaattttaat aaatataata taaaatgttg tttcgttttt 1088100
atttttatta ttagtttttt atgaaatctt ttaagttttt gttgccattt ttaagtgtta 1088160
ttctttgctg tggaaaccta ctctcttctc cacgctccag agcaatttca gtgaccgaat 1088220
ccattggaat gtcggcagtg aagactcttg ttctatctga gaaggctcat gaattcttag 1088280
agggaatcgg atatggagtt ggagcatcta gtattctccg cgactggcaa acacaacagt 1088340
ggttagaaat agaatcctta ttagcacaaa atgaggtgat gtaaatttga gatattttca 1088400
cttacaaagt atctttgcct atttatatcg gcgacctgtt ctctatagct tagcaagtat 1088460
gattttctct actgtagtct ctaaaattca tagcttgacc gcgatctagc tttccaactc 1088520
tcatatcctt atctatctcc aaaaaataga ttttataaaa ttataagttt aattctatct 1088580
gtttgcaatt tgataaaatt gattatctgt gataacttac tttaacttta aataaagatt 1088640
tttaaataaa aattatgatg caccgttatt ttattccttt attagcactt ctcattttct 1088700
ctccttcttt agtcagggca gagctacaac caagtgaaaa cagaaaaggg gggtggccta 1088760
cacaactttc ctgtgcagaa ggttcgcaac tcttctgtaa attcgaagct gcctataata 1088820
atgcaattga ggaagggaaa cctgggattt tagtcttttt ctctgagcga cccacaccag 1088880
aatttgccga cttaacgaat ggttcatttt ctctctctac gccaatcgcc aagggcttta 1088940
atgtcgttgt gttatgcccc gggcttatca gtcccttaga ctttttccac caaaatggga 1089000
tcctgtgatt ctctatatgg gaagttttct agagatgttc cctgaagtgg aggcagttag 1089060
tggccctcgc ttatgttata tcttaataga tgaacagggt ggggctcaat gtcaggctgt 1089120
cctgccttta gaaacaaaga attagagtat atttaaaaac aaaattttgc catcctactt 1089180
cggacataat tttgatcaat taaggaggca ttacatgcgt atcgcactat ctctactttc 1089240
attattaatg atattcccta tcttcggaga ggaaagtcgc cctggttcag aagacggcaa 1089300
cagtaatacg caggagatag tcggatctca ggatacacag gtatgtcttt atcattccta 1089360
tgaacagggc ttgcaagcat cccgaatcga aggaaagccc ctggttattg tagtactttg 1089420
caattctggt gatgatggcc aggcatgcac catagggtta agtgaaacat gtgaggaggt 1089480
cctttctgta ctttcaggat ctattttttc cgagttagct aacttcgtcg tacttgtccc 1089540
ttcaggagtg aacccactca tttatcctcc aattgaagat ccgattctcg cagagattgt 1089600
aaagtttaag gagttgttca aagatgagtc tttccctaca ggattaagta ttattgttgt 1089660
tggtgtcact ccagaaggac ctggtgatat catagaagtc agtccggttt cgttaaccgt 1089720
agaggaagaa gagacgctac caagtgaaca aactacagaa gtagagagca cgtctgaact 1089780
tcaatcagaa gatccagcta tagcataacc aagatcttcc cttaataaaa gaaagcgctc 1089840

gtaggattcc tcccacgagc gctttcttta tctaaatgtt atgaaattat acagctacag 1089900
attcccaatg acacaaggaa aacttatcca tagcagtaag taatagcgaa atccgtaagt 1089960
tgcggattgc accatcatca ttagcaatat gcacagtatt cagaaaatcc tgaatatcat 1090020
tggaaagatc agcaagggat aagaaatatt ctaaaaaggc atgtgctgaa gtttccttag 1090080
gaaatccagg gaaagcatca agaacttgct taaaattaga ttcacgatcc ccaagaactt 1090140
caatagggga cgaagtcatg gagagtttta aagaggaaag aatcttcttt aaacgattgt 1090200
gagtggtcgt aatcactgct aatttttctg tatgctcttc ctttaatagc tgaagggctt 1090260
cagcagtatc taaaatctca ataggatttt ncgttgcaga atctataaga acagcagcaa 1090320
tctcatcctt acgaaactct agggatccca taaatgtttt taaccgaccc caaatgaatt 1090380
caaggatttc gtgtatagtc ttggacttat cccagacctt ttcttcaatg gtgctaggaa 1090440
aatggtccgc tagacgatct aaaagagaag caagatctat agggagccga gaggcggata 1090500
ctagtgttaa cacttctaag gattgacgac gtagtgcata aggatcatgc gatgacgtag 1090560
gcttaagtcc taaaataaag caagcaagca agttatccaa acgatctaaa agagaaagaa 1090620
gagttcctat ggttgagagc ttttgaccca tagtaatgtg tcgtaggtgc tctcctacag 1090680
caaccgcaga cgctgtggga agattcgcat gcttcagata atactctccc atgatcccct 1090740
gaagttcagg gaattcattg acgacagccg acactaagtc agctttgcag tattggatgg 1090800
caatgtctag gtcctcggaa gccgctaaag aggaaaaagt agagaacact ctttggtgtg 1090860
cttttaaacg ctctaccttg tcatataaag aacccaaagc ttcaaagtat gtcacagact 1090920
tgagcttttc aataaaggtt gttaaaggag tctgtaagtc ttgtttgaaa agaaattctc 1090980
cgtcagtgag acgaggagtg agtgcctttt catttccttc aatgattgta tcattcggag 1091040
aattatcaca aactacaata aaaaagttag aaatagctcc agaagaggtt tcgtgggtag 1091100
ggaaatactt ctggtgattt accatctcag ctataagtaa ctctttcggt aaagcacaaa 1091160
attgctcgga aaattgccca caagagacaa aagggtgctc cgacaggaag gtagcttctt 1091220
caattaaacg ggaagaggaa atcgcagaaa ttgtatcaga actatgagct cgtagccctt 1091280
gttctataat catgcgacgc tctttttgtg aaactacaac acacgcttgc cttaaggttt 1091340
ctacataatc ttgaggagag gaaatcgaaa tttttctcgg atctaattga cgatgaccaa 1091400
aagaatttct tgaagctatt atagtaccga gagtgattgg taagatgtgt tctccataaa 1091460
gagcaactag ccaacgtata gggcgggcat actcgactcc gctgttatcc caaaccatct 1091520
ttttagggaa tttcatcctc tgaattaata aaggaagttc ctgcattaag atatcagcag 1091580
ttcgtaacct tatctcagga tgtaatagga ataaatattc agaaccgttg actgtacgga 1091640
tagctaagga agcgtgacgg gaaagatctt gataatgaga aatatccaca ccctgagaag 1091700
caaaaaactg ctggccttga ggagacacat ctccatcagg agaaaataaa gacgtcagca 1091760
taggaccttt tttctcaaaa gccttctgca cgacctcagg agctacgttt ttaacaagca 1091820
aagccaaccg tcgtggagag ccaaggacct ctaacccctc ataaacaata ttatgatcag 1091880
taagaacctg gcgagctaac gattctagtt gttgtattcc aataggaaca aaagtcgcag 1091940
gaagttcctc agaaccaatt tctagtaaga gatcctctgt agatgaaatc ataggaacta 1092000
cagattctga tgtttcctta ggctcagatg ttgaagaaag actaagtaaa ggataattta 1092060
aagaagcacg ccactctaca tagctatccg caactaaacg agttaattga cggatacgag 1092120
caatataacg tgtacgctca gtaacagaaa tcgttccccg agcatcaagg atattaaaag 1092180
catgcgacgc tttgatcacg aagtcataag caggaacaga aagaccattt ttaagagttc 1092240
ttaaagcttc ctcagcgaaa tcttcgaaat gcttaaacca catctcggtg tttgcatagt 1092300
caaaattata ttcactccaa gctttttcag aagcttgtgt aatttgtcca tacgttaaag 1092360
tgtcattcca taagacatca taaatagagg ttttcttttg caaatacatc gcaattcttt 1092420
caatgccata ggtgatctcc ccactgatag tatccaaagg tttactccca atggcttgga 1092480
aataggtcaa ctgggtaatt tccatcccat tgagccacac ttcccaacct aagccccaag 1092540
ctccaatggt agggttttcc caatcgtcat gaataaaacg gatatcgtga tcacgaaggt 1092600
ctaaaccaat ggcccgtagc gattccgtat agagtgagag aaaattttca ggcacgggct 1092660
ttaaaatgac ctggagttga tgatagtttt gtagccggtt cggatgcacg ccataccgac 1092720
catcttgcgg acgccttgaa ggttctacat aagcagcctt atagggctca ggtcctaagg 1092780
ctcgtaagaa cgttgcagga ttgaatgttc cagcaccaac ttctaaatcg tatccttgat 1092840
ggatgacgca cccttgctcg ctccaaaatc gtaagatagt cgcaatcata gactgtaagg 1092900
tgaggggatg ttctgacaca aatgactcca aggtataaca tttttctttt tgacattctc 1092960
cttagctaaa gcgggagatt cttgtttcat tgaaggtagc atagatttta aagctacagt 1093020
cacaccttct ccacaagcgt agatttccgt gtgtctcacg gtacgtgtta ggatgagtat 1093080
ataccttttg aaaaatactg cagtacagaa aaccaaaaga aaataacgac atagatttaa 1093140
caagatcaga gaacaagttt ttaaaacatc atttccattc taattaaatt atatgaagat 1093200
tttttacctt ttgacctaaa aactctcttg agtaatctta agaacaatcc tgtgagcaga 1093260
tgtttggatt ataacactca attttgagtt attttcatag tgcttgacaa tattttcaat 1093320
aatttagaaa aattagaaaa ttttctattt agcttagagc aagagggttc tcgatataca 1093380
aaaatggatc atgcgagcaa ttgattttaa ggaaaacacg acttcctttt aaattcaaag 1093440
ttatcctcga tcttgtgaat aaaataacta tagggtttcc ttaagtaagc tagaacaaaa 1093500
aatacgcaga ctgtgatttc agtgctttcc tcttagtaag gcactcatct catggacaga 1093560
caagaaccat atattagggg agtagggtgg ggctacctaa ttacatcact ttttccagac 1093620
tctttattac accgattttc atgatcctgt atttaaaagg aaaatggttt ggaatcactc 1093680

cagtagtgtt gccttacgtg ctgttagctc tcctggcaat ctctgagtta acagacgcta 1093740
tcgatggtta tgtcgcaaga aaattttcac aggttaccga tttaggaaaa ctcctggatc 1093800
ctatggcaga tagtatctac agaatttcta tctacctaac ttttacacag cctccagtca 1093860
atttaccttt gcttctggta ttcatcttcc tagcacggga ttctgtaatt agtactttgc 1093920
gtactgtatg tgctttccgt gggcgtgttg tcgctgcaag ggctagtggg aaactaaaag 1093980
ctatactaca aggagtcagc ttcttcttaa ttcttttggt tatgattcct cactccctag 1094040
gacttctttc tcagaatgga ttggaaatct ttgcctcagt tacggtttca atcatagctg 1094100
tgtattctat agcctcagga atcgaatact tctggatgaa caaaaacttt ttatcccaaa 1094160
gagctaaaac aaaagattca gaaaagaatc atgagagtaa agattgataa agatttacgt 1094220
aatgcttagc catggcatct aagccagagg cccgaagcat tcccgactca atcaaattca 1094280
accaaacgtc aggctcctga cgatacgtcg ttacagcgtt gctaagcata gcccgaaatt 1094340
cattaaaatt gtttgtatca aagaaagtga aaccatttac cccaggaatc actgtatcag 1094400
caagccctcc agttttacga actaaaggaa ctgtgccata acgcatcgct atcagctggg 1094460
taagtccaca agcctcccta tgtgaaggga tgcagatcat atcggcagca gcataagtta 1094520
gcctggctaa aggatcatta aagtccaaga tcaaacgaat gttgggggag ctcgctaaac 1094580
aatcttgtaa gttacggaac tcattaagaa gaacctcatt ttgacttgtc ccaatcaaga 1094640
taaaggcata actgtgctcc atagcatgga gaataatctc tttcataaat tcaggaccct 1094700
tttcctcaac aatgcgtgag atcacacaaa tcaaaggaaa atagtctgaa ctgatcccca 1094760
acttctcata taataccgct ctgttctctt cttttttagt aaagagaacg tcaggttcgc 1094820
ttaataggct tgcatcgtac tgtacagcta aagcaggatc tgtcttcggg ttccaaacgt 1094880
cttcatcaat gccattgatg atcccagaaa atacagaatt tcttgctaga atcgcatcat 1094940
gaagttcgta atcagaatag tcgtttataa tttcctgcac ataagtaaga gacactgtcg 1095000
taatgtaatc cgaacaatag agagctccct tcattagaac agaagtttgc ggatcgcgaa 1095060
atagttggta gtgactcaaa tgaaaatcat caatttgcga cgctgctaat agctgcgtac 1095120
tacaataccc tcgataacca aaattatgga tagtaaagac aatcttcgaa tgcacagggt 1095180
ttaaagggtt ttttaataaa cccgcaagta aacctacatg ccagtcatgc aagtgcacaa 1095240
tgtcagcagg atccgcttct tgaagataag ctgcagctgc agctgcaaaa gcagagaaac 1095300
gtacaacatt attctcagag tacacggacg tggttgagaa aagctctatt tgtgaatcca 1095360
acgtaattat agtaagcgta agaccctcgt aagaataaga aattgcagag gcttgctgct 1095420
tgcctaaaaa ttcataatag aaagaacgct cggaaagaac ttgagacgaa gagaatttgg 1095480
aaattaaagg ataatgaggg agaagtactt ccacatcatt ttgtttcgct aactccttag 1095540
atagactagc tacagcatcg cctagaccgc ctactttaac gattggagtg aattctacag 1095600
cgacttgtac gattctcata gactctccct aaagctataa aatatcccat atcaagggcg 1095660
aaggtaaaat ctcaagacca aaaaagagag ttttttatca ttttcctatt ttcaaaaact 1095720
gaagattcgt taaaataatg cctttctgtt ggggtgtggc caagcggtaa ggcagcggtt 1095780
tttggtaccg tgcatcggag gttcgaatcc ttccacccca gagtcctttt cttctccttt 1095840
tggtaactgt attctttagt tgtctcttct tacaagaagg atatacgttc aattctttaa 1095900
ttagggaaag tattgacttt ttccaaactg gagatataat tttgcctcga aaactgtcga 1095960
tagacgtcca agacatgaca gtgattggcc ctctaagaaa atatgtttgg actctcagag 1096020
tgaatgcata gcgcacagct cttttagctc aagattacgg agaaaacaac attatggagc 1096080
ttgtagttac aagtcgagag actggtaaga aatcttttct taagaaaatt cgtcagcaag 1096140
gtggaatccc tgctgtagta tattctgcag gcaagagcct tgcgaatatc actgtggatg 1096200
cacttgtgtt taaaaagttt ttatcgaatt tagaaagcgg agccctatct tctacggtct 1096260
tttctttgtc ttatgaaggg cgtataatta aagctctagt taaagatatc caatatcaaa 1096320
tcaccaccta cgatgtaatt cacctcgatt ttgaagaact cgtagaagat cgtcctgtaa 1096380
agttaaatat tcctatccgt tgtatcaatg ctgtagactg tattggagtg aaactcggtg 1096440
gatctttacg acaagtgatt cgtgccgttc gcgtagtatg caaacctaaa gatattgtac 1096500
cttttctaga acttgatgtg cggtctgttg gactttctca aacgagaaaa ctatccgata 1096560
taaagatccc tgctggaata gaaacaatta cacctttgaa agaagtcgct ataaccgtct 1096620
ctagaagata atatggctaa gctcattgta gcgataggga accctaggca tgggtatgca 1096680
aatactagac ataacgcagg gttcctattg gctgataggt tagtggagga gctccaaggc 1096740
cccccattta aaccgttatc aaaatgccat gctttaatga ctctcgtaga gtcttcttca 1096800
gggcctttgg tttttattaa accaacaact tttgtcaatt taagcggtaa agccgtggtt 1096860
ttggctaaaa aatattttaa tgttgctctg agtcacattc tagttctcgc tgatgatgtg 1096920
aaccgttcgt ttggtaaact tcgcctttgt tttaacggag gaagtggggg acacaatggt 1096980
cttaagagca ttactgccag cttgggttcc aatgaatatt ggcaattacg gttcggtgta 1097040
ggaagacccc tcgaagaggt gttgagctat ctaatttcgt tttaggaaag ttttctgaag 1097100
aagaaaatct tcagttggga tccatatttg ttgaggcatc tactctattt accgagtggt 1097160
gttcgaaatt ttaaatggct tgagaagttt ctgaaataat tttagtggac tgagatatct 1097220
taaggataat aaactactct atctgtctgt gtttaggagt ttttaatggg aaaaaaagaa 1097280
aatcaacttt acgaaggcgc ctatgtgttt agcgtcactc ttagtgaaga agctaggcgc 1097340
aaagctttgg ataaggttat ttcaggcata actaattacg gcggtgaaat tcataaaatt 1097400
cacgatcaag gacgtaaaaa actagcgtat accattcgtg gagctagaga aggttactac 1097460
tattttattt atttttctgt ctctcctgga gccattacag agctttggaa agagtatcac 1097520

ttaaatgaag atctacttcg tttcatgact cttagagcag attctgtaaa agaagtttta 1097580
gaattcgcct ctctacccga ataatttgtt aaggagaaaa tatgaataag cctgttcata 1097640
ataatgaaca cagaaggaag cgttttaata aaaaatgccc ttttgtttcc gcaggttgga 1097700
aaacaataga ttataaggat gttgaaacct taaaaaaatt cattaccgaa agaggtaaag 1097760
tattacctag aagaattaca ggtgtttctt cccgtttcca aggcgtgcta tcccaagcaa 1097820
tcaaaagagc tcgccattta gggttgttgc cttttgttgg agaagattaa tttaaaggaa 1097880
gaagaatgaa acaacagcta cttttacttg aagatgttga tggattagga cgtagtggtg 1097940
atttaattac cgctcgccct ggatatgtcc gtaactatct tatccctaag aaaaaagcag 1098000
tgattgctgg tgcaggaact ctgcgtttac aagctaaact taaggagcaa cgcttaatac 1098060
aagcagctgc tgataaagca gattccgaaa ggattgctca ggctctcaaa gatatcgttt 1098120
tggaattcca agtacgtgtt gaccctgata acaatatgta cggatctgta accattgcag 1098180
atattattgc agaagctgct aaaaagaata ttttcttggt tcgtaaaaac tttcctcatg 1098240
cccactacgc tattaagaat ttgggcaaga aaaacattcc tttaaagcta aaagaagaag 1098300
taaccgcaac cttattggtc gaagttacct ctgacaatga atacgtcact gttttggctc 1098360
aaggaaaaca aactgaggaa aatcaagaag gctaatacgt agggtagaaa agtttgttat 1098420
aaggatatca tgcaatactt ttctcccgca aagttaaacc tttttttaaa aatatgggga 1098480
aagcgtttcg ataattttca cgagctcaca accctttatc aagctataga ttttggagac 1098540
acactttctt taaagaatag catgaaggat agtttgagca gtaatgttaa cgaattgctt 1098600
tccccctcga atctcatctg gaaaagcctc gaaattttca gaagagaaac acaaattcac 1098660
caaccagttt cttggcacct caataaatct attcccctcc agtctggctt aggaggaggc 1098720
agtagtaatg cagccacagc cctctacgct cttaacgagc atttccaaac ccatattcct 1098780
ataacaacat tgcaactttg ggctcgagaa atcggaagcg atgttccttt tttttttctt 1098840
caggaacagc attagggaaa ggtcggggag aacacctctt ctctataaaa aaactcaacc 1098900
ataaacataa atatgttctc tatctcgatc atcaaggaat acccacagaa aaggcatacc 1098960
aatccttact tccacaagac tatagtacag gaaatcataa cgcctgtttc tatggtgaaa 1099020
atgatctaga aaaatccgta tttcgtatac ggacagactt gaaaaataaa aaacacatgt 1099080
tagagaggat gtggagtcct ttcgaaagcc atgtgcttat gtcaggttca ggagctaccc 1099140
tattcgtttg ttatctagaa gagttagaac aagattcaaa agtatcctcc caaatccata 1099200
gcctaatcaa acaaacccaa ggaattcccg ttagccgtct ttatagggaa ccgcattggt 1099260
attctctaaa gcaatctact tacaaaaaca gcccgctaga gtgttttcag ccacagatat 1099320
gattttattt gcctctacag aaagtgagtt ttctgcttgg caaaatccat gaacattacc 1099380
taaactcgcc ttttccttca gcacagagaa tataagagta gaagcttcgg aaattttcgt 1099440
tactttttta gcagtcgttt catctgcttt aggaagtgta atcttgtaag acataaataa 1099500
accctcacat tctcttccta cttaaaggat ttttttattt taaacaattt cttttataaa 1099560
tgtgttttag cttcttaatg tgaaatttct atttctctac gagaagaaaa ccagagatct 1099620
tcctaaattt tttgaccctt tctttttact gtgaatacct ttatgtctac gcataagtat 1099680
taacttagct cgtctggttc tctatgatcc tgcctccata ctcttattct ttgaaaatag 1099740
gcgccgcagt gctctttttt tgttccatac tgcacacatt tctgactccc tggctctaca 1099800
ctctatgtca gtcttacgaa cataaaaaac ttgttttccc agaatgctgg aaacgctatg 1099860
cccggttgtc agaactcttc agaatcctaa gtagagtaga aattgtattt ttcttgtggg 1099920
cagtaccctt gttcttctgg tttctatata ccgaaggata caggatttcc atggcctatt 1099980
ttaatagtag aaattatgga ttcgctgtct tcattatggt gatcctcatt ttactagagt 1100040
cccgccctat agtatatttc gcagaacttg tgctctcctc aattgcaaaa ttaggaaaaa 1100100
cctctcccaa atcctggtgg tggacattga tgatcgcacc accacttctc tcctgtctcc 1100160
ttaaagaaac aggagctatg attatcggtg cgactctact tatgagacat ttttatgttt 1100220
ttactccgtc aagacgcttc gcatacgcta ctattggact tctgttttct aatatttcca 1100280
taggaggact cacaagctat gtatcctcaa gagctctctt ccttatcttt cctgcactaa 1100340
aatgggaaca ctcgtttttt ctttcccact tcgcatggaa agctatcgtc gctatcctaa 1100400
tctctacaac aatctattat tttatctttc gaaaagaatt taaaaagttc ccagatatcc 1100460
ctagtgacaa agatcctagc gttgaaaaag tgccctggtg gatcatctgt gttaatatta 1100520
tttttgtcgg ttctataata ttatcacgat ccacaccctt gttcatggga gctctactac 1100580
tcttttactt agggtttcag aagttcacta tcttttatca agatccaatc aacctttcca 1100640
aggtatgcta cgtaggactt ttttatgccg gattggtagt cttcggagat cttcaagaat 1100700
ggtgggtgct gaatctaatg caaggtctgt cagatttcgg atacatgaca gtttcctata 1100760
cgctgtccat attcttagat aatgcacttg tgaactatct agtacataac ctctccgtag 1100820
ctacagattg ctaccactat cttgtagtcg caggctgcat ggcagcaggg ggacttaccc 1100880
tagtctccaa tatcccaaat atcgtgggat acctcatctt aaggtccgca tttccctcat 1100940
cgacaatcca tatgggatgg ttgtttctcg gagctttagg cccctccata atttccttgg 1101000
gagtcttctg gttattgaaa aatgttccag aattcctcta ctgttttttc aggtaaacta 1101060
aagataaaac aaaggcataa gatatgatct tatgcctttg tttttaagag gtcttctgag 1101120
tgccacgtat atagacgcta gtttgcgtcc ctagttcatt gctaaggaaa cactgacagt 1101180
attttaagaa ctcttcgtat gttaacgttt ccgcaatagc aatcttcaag tcgggagtcg 1101240
aaaactccac aaaaggccgc tcaaatgcta gagaaaataa cgccgagttc atcatgtcta 1101300
aggaatgctc aggctccaag attttattga tatacgcttt gcgtatgttt gcgaatttct 1101360

cttgcgatat tccaaatttc tcaggagaag ctgagacctt gttaaggaac agagaagttt 1101420
tagcaagaag ctcttcagga gaatacgcat ccgaacggat atagagaaat ccaaacggcc 1101480
tggaagcaaa ctctcgatag cgcgcaccca ccatataacc caattgctgt tgcgttctaa 1101540
gctcctcaaa agtaatgtga tgcaaccatt caaagagcat ctccgcacaa accttccctt 1101600
gtatagacgg ggaactctta tcttgaagta ataagagcat cccgtttgca gttaacggat 1101660
agtcatgatg gatctcagaa atttcctgag actgtagctc gtaataaaag ggctttgtag 1101720
catgcgaaga tcgtgacgca gtgaaaactt gtagcatctc aagataatct ttcttctgct 1101780
gctcagaaag gttccctaag accataactt caagatgtac actgttgaaa aggttcgagg 1101840
caaacgcttg gaattcagaa aaacttaact tctcaagagc tgatagctta gtagtattag 1101900
aatacgtctc cttcataact tgtgaggcaa gctcatcaag cccagaacga accggacagt 1101960
tgagcaaagc tccttgataa agctctaaca actgcttttt atatactaag aatgtctcat 1102020
acctaatctc taaattaggt aatgaggtta aaattgagtt taacaatgca gggactgttg 1102080
ttgtgtaccc tgaaactctt aaatcaatac catcaccacc taaagcagaa gtaaaagaaa 1102140
aaccagcttg cgttgcggga taatactccc tcaaaagctg atggttcaca ggctaagcaa 1102200
tagagctccg tagcaactag aaattgaggg gaggacctcg aaatctgagg agagcggatg 1102260
cgaatctgac tggagagttt cggtgctgta tagtagtggt cctcgcaatg gtacagagtt 1102320
aatttatcat cttggtaact gagtgcagga gcaaaaggaa attcttgttt tttcagtagg 1102380
tgtacaccag gaagagtcac ctctttagga ataaacagat tcggctttgg aagagctatg 1102440
ggctttagtg actgcacctt tccataatcc tgaacaccgt ccagagcttt tacatagtag 1102500
gtcatgtcaa aaataggatc gtggagctga gtcgcttctt cccaatgctc agagttctta 1102560
ctagataaga caaaacgtgc ttgttcagga tcagagacta aattaagaag agcagactcg 1102620
tcttcagaag agtatttagg atacacaagg ctatgataag gatacgtaga tagatcctca 1102680
ttgcccaagg atacaatctg cttacaaagc agatcaaaca atggactttt ggaactgtaa 1102740
cagtagttta aagcattaat tgtagaaatt tcttctaacg tatagttggg aatcccgtgt 1102800
tcctgaatat atcgaagata ttggaaggta ctatcaataa cctgagaata gtgtttatcg 1102860
cctttctccg taagctcata gctaatatag aattctccag tatttaaaga acttctaaag 1102920
aattccacgt ctaaatccgt aattagctgc tcgtttttca ataaagagac taaactgttc 1102980
ttactctcat ttcttaaaac ttcagcaaga gccttgtaac agcctaaagg aatcggatgg 1103040
gaagattcat aaatatgcca gtaaatttct agattagagg taggctgaat cgcttggtta 1103100
atatagagat tctttaatga cgaggtgtca ccagaaggaa gaaaaggttc ctgtctttca 1103160
taattttttg atctaggaat ctgagaaaaa atctttgaga actgtttctt tgctttagag 1103220
agcggagctg atgtgtaagc aatagcacac atattctcag gagaataatg tagcttaaac 1103280
cattctgcca ttttctctgt agtcactggg gtgagggtgg aagcattccc acaaccaaaa 1103340
cgtgcgcagg gatggccctg aggagcaaca agctgctgaa tgcgatgcac acgtctccca 1103400
tcagaaagag gatgagcagc gaattcttga tgtactgcgt acttttctct atcaagatct 1103460
tcttgacgaa acttcggatt aataaataga tgaacaaatt ggtctaaagc atcagaaaac 1103520
gcagaatgtt ctactgaaaa tacaaagact gttttatttg ggtaagtgaa agcattatgc 1103580
accccattat tttcgcttaa aaatccaggg aaaccagaga cctcaggata cttttcattt 1103640
ccaagaaaga cacagtgttc tgtgaagtgc gccatcccag gatactcttc aggatcggca 1103700
ttatttcctg ttttcacaag gagtgctgct cccgaagtag gaagattagg gtcggaaata 1103760
ataagaagag ggagcccgtt actacaaata atcttttcaa ttttctggtc cgcagcggca 1103820
ggagtggaaa cttgtaaagg gcactgattg gggacgactt tgaactgctg ttcacacgat 1103880
gttatggata gggaagtgca aattaaaata gggacaaaaa agtttccaaa acatcttaca 1103940
attcctcgta tagatttttt acaaatagaa aatacttttt ctgctcttaa tgttctttgg 1104000
gcgtgtttat cgcaatgaat aagttcctct tttgaacata atgcatcaaa aaatagttct 1104060
gctccgaagt tgaagaaaac aggagcaaag aaaatggctc gctgctcccc aattgcattc 1104120
tctattttcg gaggaggggg gagaatgtca taggttttta aagcaaaggg ataaaaatgt 1104180
gtcgtttgat tagaagcctt agctaataaa cgaaacactt caatgctttc tggagagaat 1104240
tctgaaggat ataatcttcc ttccgcattt tttctgtcac gacctcctgc aggggctacg 1104300
taaataaatt tgcctccttc atttaacaag gtctttaata tttgcatgct tttctgatta 1104360
tgaagaagct tttcttcccg gagttctggt ggagtggcaa tatgacgctt agaataaata 1104420
cataataaat cacatcccat gctaaatggt cgtgctaggg gatcagaagt gactcggtct 1104480
ccagctacaa aaatcatgtt ctccattaac tcaggatgcg tcttgcctaa ggcataatac 1104540
atgagttgtg gatcgcattc tgtctggtga tttgcaagga gaacaacatt atctccccta 1104600
gcaatgtatt cttctatttc cttaaggcgg tgtaggttta aaattcgaga gtttttatcg 1104660
tcaatgacta gagaaaaaaa atctatactc aaacgaaata gatctatagg agctcgaatt 1104720
tttttatgat acggagggaa aatgaaaggg tttttcaagt cctcgatgat aaccttgacc 1104780
cattgtaaac atagcacctc agcttgatca gctgcagctt tttttgtagc tgcatcgatg 1104840
taattctgat gaaaaacaga aaatttctga tataaaggct ctgggagata ctggttatca 1104900
aaagcatagc gtaaatacct agaaaactgc atgtatattg ccctataaat ctatactttc 1104960
ccctgtaatg agagagaaga attggtagtg gtttaaatga accgagtccg atgtattgat 1105020
ctgaagttta gcttttaatt gcttagccaa attgatcatt tcattgtcat cgttttcttg 1105080
tttcatatag cttgcaattt ctggatgcac aacaagacaa agatgagagt gttctttgtg 1105140
attaataacc tttttcaaat ccctctcaat ctcgatgacc acactttcag gtgttttaat 1105200

aatggcgttg ccgctgcagt agggacatag agtgaataat gtctgcatta gagattcccg 1105260
atttctctgt ctggtcatct caacaagacc aaattcactc atgcttaaaa tagtacagcg 1105320
agcagcatcg tatttcatat gttctttcaa tcgttccaaa acacgtcgct ggttcttacg 1105380
agatttcata tcgataaagt caataatcac taatccgcct acattacgta aacgcaactg 1105440
tctggcaatt tcttcagctg cttctaagtt gatttggacc agagtttctt caactccact 1105500
ttctaactgt gtgcttcttc ccgaatttac gtcgatagtg tgcatggctt ctgttttgtc 1105560
aaaaaacaaa taaccaccgc tggacagcca aatttttctt cttgtcgcct tatcaatttc 1105620
tttctctata ttgaaacgct caaacatcgg aatagaatcc cgataatact ctatcttgat 1105680
ggaagcatcc ggagagtatt tttttaacat atgtttgcac ttttgatacg tagcataatc 1105740
atcaataagg agtcgcttgt agttcttatc gatacaagta atcacagctt tttttagtat 1105800
gtccgtctca gaataaagca agcagggttg ctctgtggaa tagaattttt ctaaaattgt 1105860
tttccaagtg agtaacaagt cgtgggcctc attaatgaga gcttcagtag aggctgtagt 1105920
gctcgctgta cggcaaatca aacccatgtc ttgaggcatt tcaaaagaac gaatgagctg 1105980
ttttaactgc tctctcatat ggggatcttc aatttttcga gaaacacctc ggtgaggtga 1106040
gtttggtaaa agtaccaaat aacgtcccgg aatggagatg ttagaagtta agcgagctcc 1106100
cttacttcca ataggctctt tgactacctg aacgagaacg gggctgtcta atttgagaaa 1106160
ttcttcaatg ggagcttcct cagaagaaag aagaggagct tcagaagctt cttcgggaag 1106220
agcatctaca tccatatcga acatctgttc aaacttcttc gaattctcta aaatatcaga 1106280
gatatgaatg aaaccattct ctctctcatc aatattaata aacgcagatt ggatatttct 1106340
cagaatgttg gtaacacgac ctcgataaat attgcctttg agctgacgaa ctttctttct 1106400
ctctatagtt agatcaaaaa gttgaccatt tttcagatgg gcatagcgaa tttctttcga 1106460
ttctatgttg agtaaaattt cattttccat gactttcctt gtgcctatac catatccttg 1106520
agcttactta ttctgtttgg gaaaagagtg gtttctagac gtcgaaactt aaatttctaa 1106580
ctgctcgtga gataagttac gttggtgatt ataaagaaaa taggtaaaaa attctacaga 1106640
aatggctttt tgattctttt taaaaataat taggttattt taatttaatt cttttcaact 1106700
gtaggtcttt gttttctata atcattgtct ttggtaagct atctttctaa tcctcaaaaa 1106760
gccttggttc taggtagtaa aggttttagt atggactgtg tcgataattt aaaattatat 1106820
atttttcgtt tgaaactacc tggagatacg gaacgcatca gctattctat tagccctgaa 1106880
tacattcgtg agaaggggga agaagagctt ttgaattctc ccatcgaggt ggaagggtct 1106940
ctgggacgca tagatagtga ccaatggatt ctctcgctaa gcctaaagac tcagctaggt 1107000
ttatgttgcc cagtgtgtaa caattttttt tcacactccg tctgcttgcc agatcttcaa 1107060
cgtgtgatat ctcatgatga ggtaggttcg ggagtctttg attgtagacc tttgattcgt 1107120
caggagctcc ttttagaaag cgattgtttc gaagagtgta gtgggcaggg ctgccccgaa 1107180
aggaaaaata tcttaaaatt tttggaagac agaaaaaaac atgaggggaa taatcccttt 1107240
gagtatttat aaaaggtaag gaacatggcg gtaccacgca atcgacatag taatgcaaga 1107300
aagaatattc gaagaagtca cgatgctaag aaggcttgtc acgcagcaaa gtgcagcaat 1107360
tgtaagcatg cccttcttcc tcatactata tgcccttctt gtggatttta taacggtaaa 1107420
gccgttatga ctgtagaaaa gaaataaatc ttatctgatt ttatggaagt gcaaattggc 1107480
atagatttaa tgggagggga ccattctcct cttgttgttt ggcaagtgct ggttgatgta 1107540
cttaaatctc aaagttctac tattcccttt gcatttactc tttttgcttc cgaggagatt 1107600
cggaagcaaa ttcaagaaga atttatatca gatctgcctc aagagaagtt tcctaagatc 1107660
atttctgcgg aaaattttgt ggctatggag gactctcctc tagcagctat ccgcaaaaaa 1107720
tcctcatcca tggctttagg attggattat cttcaggaag ataaactcga cgcttttata 1107780
tccacaggga atacaggggc cttagttaca ttagcacgtg ctaagattcc tctatttcct 1107840
gccgtatctc gtcccgcatt acttgtttgt gttcctacaa tgcgaggtca tgcagtcatt 1107900
ctagatgttg gtgccaatat ttctgtaaag cctgaagaaa tggtaggttt cgctcgtatg 1107960
ggacttgcct atcgtcagtg tcttggcgat tctaagattc ctacgatcgg attgcttaat 1108020
attggttcag aagaacgtaa aggtacggaa gcccatcgcc agacattccg tatgctgcga 1108080
gagacatttg gcgaactttc ttaggcaata tagaaagcgg tgctgtcttt gacggtgctg 1108140
cagatatagt tgtcaccgat gggtttacag gaaacatctt ccttaagact gctgagggtg 1108200
tatttgagtt cttgcagcgt attctagggg ataaacttga agcagacatt caacgtcggt 1108260
tggattacac attttatccc ggctctgtag tctgtggtct ttctaaactt gtgatcaaat 1108320
gtcatggtaa ggcgtgcggt tcttctttgt tccatggcat tttgggctct ataaatctgg 1108380
ctcaagcacg cctatgcaaa cgcattttgt ctaatttgat ttagttaact acattcaatt 1108440
ttttgcttct ctgtatagct ttttctttat tgacgggatc ttgttgatag tgctacatat 1108500
tttgcaaaat actttataaa cacctctcag gtttaaggtt gcaatggtag cgaaaaaaac 1108560
agtacgatct tataggtctt cattttctca ttccgtaata gtagcaatat tgtcagcagg 1108620
cattgctttt gaagcacatt ccttacacag ctcagaacta gatttaggtg tattcaataa 1108680
acagtttgag gaacattctg ctcatgttga agaggctcaa acatctgttt taaagggatc 1108740
agatcctgta aatccctctc agaaagaatc cgagaaggtt ttgtacactc aagtgcctct 1108800
tacccaagga agctctggag agagtttgga tctcgccgat gctaatntct tagagcattt 1108860
tcagcatctt tttgaagaga ctacagtatt tggtatcgat caaaagctgg tttggtcaga 1108920
tttagatact aggaattttt cccaacccac tcaagaacct gatacaagta atgctgtaag 1108980
tgagaaaatc tcctcagata ccaaagagaa tagaaaagac ctagagactg aagatccttc 1109040

aaaaaaaagt ggccttaaag aagtttcatc agatctccct aaaagtcctg aaactgcagt 1109100
agcagctatt tctgaagatc ttgaaatctc agaaaacatt tcagcaagag atcctcttca 1109160
gggtttagca tttttttata aaaatacatc ttctcagtct atctctgaaa aggattcttc 1109220
atttcaagga attatctttt ctggttcagg agctaattca gggctaggtt ttgaaaatct 1109280
taaggcgccg aaatctgggg ctgcagttta ttctgatcga gatattgttt ttgaaaatct 1109340
tgttaaagga ttgagtttta tatcttgtga atctttagaa gatggctctg ccgcaggtgt 1109400
aaacattgtt gtgacccatt gtggtgatgt aactctcact gattgtgcca ctggtttaga 1109460
ccttgaagct ttacgtctgg ttaaagattt ttctcgtgga ggagctgttt tcactgctcg 1109520
caaccatgaa gtgcaaaata accttgcagg tggaattcta tccgttgtag gcaataaagg 1109580
agctattgtt gtagagaaaa atagtgctga gaagtccaat ggaggagctt ttgcttgcgg 1109640
aagttttgtt tacagtaaca acgaaaacac cgccttgtgg aaagaaaatc aagcattatc 1109700
aggaggagcc atatcctcag caagtgatat tgatattcaa gggaactgta gcgctattga 1109760
attttcagga aaccagtctc taattgctct tggagagcat atagggctta cagattttgt 1109820
aggtggagga gctttagctg ctcaagggac gcttacctta agaaataatg cagtagtgca 1109880
atgtgttaaa aacacttcta aaacacatgg tggagctatt ttagcaggta ctgttgatct 1109940
caacgaaaca attagcgaag ttgcctttaa gcagaataca gcagctctaa ctggaggtgc 1110000
tttaagtgca aatgataagg ttataattgc aaataacttt ggagaaattc tttttgagca 1110060
aaacgaagtg aggaatcacg gaggagccat ttattgtgga tgtcgatcta atcctaagtt 1110120
agaacaaaag nattctggag agaacatcaa tattattgga aactccggag ctatcacttt 1110180
tttaaaaaat aaggcttctg ttttagaagt gatgacacaa gctgaagatt atgctggtgg 1110240
aggcgcttta tgggggcata atgttcttct agattccaat agtgggaata ttcaatttat 1110300
aggaaatata ggtggaagga acttctggat aggagaatat gtcggtggtg gtgcgattct 1110360
ctctactgat agagtgacaa tttctaataa ctctggagat gttgttttta aaggaaacaa 1110420
aggccaatgt cttgctcaaa aatatgtagc tcctcaagaa acagctcccg tggaatcaga 1110480
tgcttcatct acaaataaag acgagaagag ccttaatgct tgtagtcatg gagatcatta 1110540
tcctcctaaa actgtagaag aggaagtgcc accttcattg ttagaagaac atcctgttgt 1110600
ttcttcgaca gatattcgtg gtggtggggc cattctagct caacatatct ttattacaga 1110660
taatacagga aatctgagat tctctgggaa ccttggtggt ggtgaagagt cttctactgt 1110720
cggtgattta gctatcgtag gaggaggtgc tttgctttct actaatgaag ttaatgtttg 1110780
cagtaaccaa aatgttgttt tttctgataa cgtgacttca aatggttgtg attcaggggg 1110840
agctatttta gctaaaaaag tagatatctc cgcgaaccac tcggttgaat ttgtctctaa 1110900
tggttcaggg aaattcggtg gtgccgtttg cgctttaaac gaatcagtaa acattacgga 1110960
caatggctcg gcagtatcat tctctaaaaa tagaacacgt cttggcggtg ctggagttgc 1111020
agctcctcaa ggctctgtaa cgatttgtgg aaatcaggga aacatagcat ttaaagagaa 1111080
ctttgttttt ggctctgaaa atcaaagatc aggtggagga gctatcattg ctaactcttc 1111140
tgtaaatatt caggataacg caggagatat cctatttgta agtaactcta cgggatctta 1111200
tggaggtgct atttttgtag gatctttggt tgcttctgaa ggcagcaacc cacgaacgct 1111260
tacaattaca ggcaacagtg gggatatcct atttgctaaa aatagcacgc aaacagccgc 1111320
ttctttatca gaaaaagatt cctttggtgg aggggccatc tatacacaaa acctcaaaat 1111380
tgtaaagaat gcagggaacg tttctttcta tggcaacaga gctcctagtg gtgctggtgt 1111440
ccaaattgca gacggaggaa ctgtttgttt agaggctttt ggaggagata tcttatttga 1111500
agggaatatc aattttgatg ggagtttcaa tgcgattcac ttatgcggga atgactcaaa 1111560
aatcgtagag ctttctgctg ttcaagataa aaatattatt ttccaagatg caattactta 1111620
tgaagagaac acaattcgtg gcctgccaga taaagatgtc agtcctttaa gtgccccttc 1111680
attaattttt aactccaagc cacaagatga cagcgctcaa catcatgaag ggacgatacg 1111740
gttttctcga ggggtaccta aaattcctca gattgctgct atacaagagg gaaccttagc 1111800
tttatcacaa aacgcagagc tttggttggc aggacttaaa caggaaacag gaagttctat 1111860
cgtattgtct gcgggatcta ttctccgtat ttttgattcc caggttgata gcagtgcgcc 1111920
tcttcctaca gaaaataaag aggagactct tgtttctgcc ggagttcaaa ttaacatgag 1111980
ctctcctaca cccaataaag ataaagctgt agatactcca gtacttgcag atatcataag 1112040
tattactgta gatttgtctt catttgttcc tgagcaagac ggaactcttc ctcttcctcc 1112100
tgaaattatc attcctaagg gaacaaaatt acattctaat gccatagatc ttaagattat 1112160
agatcctacc aatgtgggat atgaaaatca tgctcttcta agttctcata aagatattcc 1112220
attaatttct cttaagacag cggaaggaat gacagggacg cctacagcag atgcttctct 1112280
atctaatata aaaatagatg tatctttacc ttcgatcaca ccagcaacgt atggtcacac 1112340
aggagtttgg tctgaaagta aaatggaaga tggaagactt gtagtcggtt ggcaacctac 1112400
gggatataag ttaaatcctg agaagcaagg ggctctagtt ttgaataatc tctggagtca 1112460
ttatacagat cttagagctc ttaagcagga gatctttgct catcatacga tagctcaaag 1112520
aatggagtta gatttctcga caaatgtctg gggatcagga ttaggtgttg ttgaagattg 1112580
tcagaacatc ggagagtttg atgggttcaa acatcatctc acagggtatg ccctaggctt 1112640
ggatacacaa ctagttgaag acttcttaat tggaggatgt ttctcacagt tctttggtaa 1112700
aactgaaagc caatcctaca aagctaagaa cgatgtgaag agttatatgg gagctgctta 1112760
tgcggggatt ttagcaggtc cttggttaat aaaaggagct tttgtttacg gtaatataaa 1112820
caacgatttg actacagatt acggtacttt aggtatttca acaggttcat ggataggaaa 1112880

agggtttatc gcaggcacaa gcattgatta ccgctatatt gtaaatcctc gacggtttat 1112940
atcggcaatc gtatccacag tggttccttt tgtagaagcc gagtatgtcc gtatagatct 1113000
tccagaaatt agcgaacagg gtaaagaggt tagaacgttc caaaaaactc gttttgagaa 1113060
tgtcgccatt ccttttggat ttgctttaga acatgcttat tcgcgtggct cacgtgctga 1113120
agtgaacagt gtacagcttg cttacgtctt tgatgtatat cgtaagggac ctgtctcttt 1113180
gattacactc aaggatgctg cttattcttg gaagagttat ggggtagata ttccttgtaa 1113240
agcttggaag gctcgcttga gcaataatac ggaatggaat tcatatttaa gtacgtattt 1113300
agcgtttaat tatgaatgga gagaagatct gatagcttat gacttcaatg gtggtatccg 1113360
tattattttc tagttcgatg tgacagggct tcaatcaaaa aaaagggtta cttttagtaa 1113420
ccctttttta tttctcttaa tgcttatagt tcgatgatct ttaatacata gagcaagtag 1113480
gcgatacaag ctttattagg ttcataggtc tctgggtcca ttaagagtgt agaaaagacc 1113540
tcggtatgag cggaagtttg aggaagccgt aaggatgcat ataatatatc tagagcaaac 1113600
tcacggatta aagctttggt ttctggatct gctttttgca tggatttaaa gacgctatta 1113660
acaagttgat ttccagaatt taaatactta tttaggaaaa gagcagctga caaatcagcg 1113720
tcttctctgc cttgatgtgt gtccactgta gtttgacgta aagttagggc gccgagagta 1113780
tcttgtaggt gttctgtaac tagaccgaga tttttcccat ggaagaattt catagtgttg 1113840
atcacatgcc attgtagttt ggctgctgac tcgtttcgta gaacttgttc tattgcctct 1113900
gtgtaacgct caccgtgttg ctgagccgca aaccgatact cttcagcatc atcaccatct 1113960
tggatggaca tcagctctcg ttctttaaac tcttgaaccc aaatcagaag ctgattgtct 1114020
ccttcattag ctatactata gagtctgtta agagatctgg ttatgacttc tccccaagtc 1114080
ggcttacaag tcttgcaaga acttgcaata aataaggccg cttgcatttt ttcctttgta 1114140
ctaggagttt tgggtaaaga ggactttcca gtaagagctt ctatgaggag cttgatcaat 1114200
attgggaacg tctttttatc aggattacta tcatgttcgt tttcagtccc ctcaaagtac 1114260
ttcagcatgt gcttccattc tggagttagc acgcctagag actttaatac ttttttcagt 1114320
tgtgagcata gatccggaat cttccgcatg gcttcttggc tntcttgcgg cgaaattgct 1114380
actagtctat ttaaaaaggg attaggtaag ggtgctttgc ctacactttg ttcaacaatt 1114440
ttaaaagagc gaagtttgtt atcctcaaaa agcaacgctt gcaaagcgaa aatgaagggt 1114500
tgaatgacct cattcgtttc ttcagtagta ggttcactta aatcctctac gattttttta 1114560
gggggaagag gcatgaagag acgaagacct gctacgatga gattcaccgg taaaagaatg 1114620
ataaacagta ctgaagtaat aataaatgct aggatgttga gaatcaccca acataaggga 1114680
ccaccagagc acccgcaggg attcagtatt ttttttgaca tactgaatag gctagaagga 1114740
tgtaggacct gagccacgtt ggttaggcgt gtttgacacg cattttcttc caagataata 1114800
agttccgtcc tccgaggata aatcggcgaa gtttttggaa ttaaattgta ctttgctatt 1114860
aactctgcag ggaaaaacaa agagatgggg ttagacatat gcatcttgga gttatggatt 1114920
aagtagaaat attttattat actttctaag attgattgtt attcaattaa tttttattta 1114980
gaaatactat aaaaaagtag ctcaaaacga attgttttat acttatttat gagttcttaa 1115040
agaacttgtt ttaagaaacc taaatattca tacagcggga agtgtgtcaa aaattctttt 1115100
taaaaactct tcagaagcta tttgccctgt cgtcatcact ttacataatt tgcggcagtc 1115160
ctccttttgt ttttctttag agccatgttg atttagcaaa tctaaagcgg tagcagtttc 1115220
ttctggatga aaatcttttt tccctccaat gaactctgga aaaatcacgg agttcataat 1115280
gatatttggt aaggaatacg cgggcaaaag aattttaaaa atatattttg ctaagaatgt 1115340
gtcaaaaggt cgaagtcgac acatgactat tgtgggtgtt tgatttaagg cagtttctaa 1115400
gactatagtg ccacattttg ctagtgcaca atcacagctt ctcatgagct catagcggaa 1115460
attcatgggg atgatttgac tatgctgaca tccttctgct tttagagtat cttcaatgat 1115520
ttcgtcgtac ttagcgcttg atgaggaaac cacaaattga tgtgtttgag atagcgatga 1115580
atttagaaaa gcttgtacct ggatccgtaa atttcggctg atgtcaccac gacgacttcc 1115640
aggaaacgca gcaacaatag gacggtcaga attaaggaat ttctctttcc aagaggcctg 1115700
ttctttgtag tcagagatct cctcaactag ggggtgtcct aaatacacag tctcaagaga 1115760
tgtgtttttg aaaaggcctt cttcaaaagg aaggatcagt aggagcatat caagatgttg 1115820
ctctagtatg cgttttcttt tggggcgcca tgcccaaata ctcgggcaaa cgtaatggat 1115880
gatttttcct cgatacccat gttttctcag ctttttaatt aagagaaggt gaaaatcggg 1115940
gaaatcaatg aagataagag tcgctggctt gtgtttgagt atcgttttta ggattttgcg 1116000
gtaatttcga tataatctaa ataatgaccc cagaacttca gcaaatcctg aaacttgaaa 1116060
ctcctccatg tttagaatgg gctggagacc ttcttgtcgc attgcaggac ctccaacacc 1116120
ccagaaccgt atgtttggat agagagactt tatggattgg attaacttac cccccaaaat 1116180
gtcaccgcta gcttctccag cagagagaaa gcagtgtgta tccaaataag gagtcgaacg 1116240
ttgttcttta taaaagagac gtaaatttgc gatggacggg aataatccac agccatagca 1116300
caggatgttt ataggatctc cgatgcgaat gaagtacacc agcgccagca atcccccaag 1116360
aagtccaatt ttccaaaata gtaaggggaa atctttagta ttattcgatt ctatgtagaa 1116420
ccattggatt aaaaaacgac ccgaaaaaat agcaagccct aaacagccta taagatgcca 1116480
agaaagttgt gcaggaggaa gaggaagatg gaagatatta ggagaggcca tccattccat 1116540
gttcacatag aggaaaggca gagtcacgaa caccacgctc agagccatca gaactaaggt 1116600
cgctcgaaag gaaatcggtc gagaggaagt aatatttagg tttctgaggt agataattaa 1116660
gttaatgaca tgaagtacag tcacaggaaa ttggctttga attgtcccgt ggacaatcat 1116720

caaagtggct cctattgaag agagaatcca aaagcttcga ggagcataga cctctttgcg 1116780
ctttttactc agccaccact gtattgagaa ggcactccca aaaaaaagac tagcaagaaa 1116840
tcctagggga taaagcagat aaactaggcc agaagggatc atgtttagac ctacttttga 1116900
gaatgagaat cctttttaca cttaagtgtt tgatgatgac gtatccaagc aacatacact 1116960
ttatcgagct taggatacac tatactacga atttccaata aagataatgc ttctaagaac 1117020
cttgtgtgat gtaaaagttt cttattaaag aagagcgctt tttttgtagg gatcagggga 1117080
gtgagtctgt actgcatttg taaaattaat gctgtcagga taaaattctt ttttgagcaa 1117140
cttgtgaacg agtcagcaaa gaattgttct aggaaatttt tgatataatc aaaaacggat 1117200
gtcaaagaca aatacgggtg cttctgatgc ttataacgga cattaaagtt cactaaagga 1117260
aaaaggaata tagccatcag ctgatgacga tcatactctg cctctttttt tagtattttg 1117320
tcatctagag cttttaggta ggtggctgtt tgctcttcta gtgcgcgatt taagcggaat 1117380
gccttatcca tatagggaaa aaggatctca agaagatggt tctctattag cagttggaaa 1117440
aagttctttg cggctccgga atttaacatt ttgataagtt cttcgaagac ccgtgcccga 1117500
gaacttttaa ttagctcttg gcgacaagca attaaagctt cttgagtttg cgtttctaca 1117560
gtaaacggcg accgtgataa aatctttaac agtctcagca tgcgtactgg atcttgcttg 1117620
aatcttgtaa agggatcgcc gatagtgcgc aaataacggt tccttaaatc attaacgcca 1117680
ccagtatagt ctataatctc ttcatgctcg ggatcgtaaa acaatccatt gattgtaaaa 1117740
tctcttcgca agacatcttc ttcaggagtc ccccatagat tatctttagt aatcaagaca 1117800
tcttcatcgg tgcttcccga acgaaatgtt gaaacttcaa taatctgctt agagaatcta 1117860
atatgggcaa gacggaaacg cttaccgacc aaaatacaat ttttaaagat agctttaatt 1117920
tcttcaggtt tcgctgaagt ggagatatca aaatccttcg gcgtggtatt taataacaaa 1117980
tccctaatgc aacccccaac aatataagcg atataccctg ctttacggag ggttttgatc 1118040
actgacaggg cgtgaggaga gaaatctttg agtttgatgt tatggttaga aacagaatag 1118100
atcgtaggag ttaaagtgat attagatttt ttttttaaca attctaggcc tctaccagag 1118160
agaatattgt tttcgcagac cattattata gttattaaga gttaaaagtc ttggaagaaa 1118220
aagagtgtgt agtttgcgtc tacttccatt ttaaaatccc aacatagcaa aactagggaa 1118280
gttttcaagg ataaaaatga aaaacaaaag atgttttcta tagagattcc tcttcaattt 1118340
ttaattatat atggatcatt cttaagactc tagcatccac agaacccggt agagagctct 1118400
taaatgcctt gatcctgggg agcagaaagg atgtaaaaag catggaataa aaaatatcta 1118460
gtttctgaca gaattcaatt atagaacttt acatccattg gagagttctc tatagaagat 1118520
cgaagtattc ttcgagaaga gtactacagt cagaggctac tttcttttac ttatagttta 1118580
ctgcttataa attcgctttt atttgtgcgt gtagaagtga aaagatacgg agaataggaa 1118640
tagattttag gagaaacatg caacaaagtg tcagaaaact ttttggtaca gatggtgtgc 1118700
gaggacgagc aaattttgaa cctatgacag tggaaacgac tgttttatta gggaaagctg 1118760
tagctagagt acttcgtgag ggtagatcgg gcaaacatcg tgttgtagta ggcaaagata 1118820
ctaggctatc gggatatatg tttgagaacg cattgattgc tggctcaatt ctatggggat 1118880
agaaactttg gttcttggtc ctatccctac gccaggagtt gcgtttatta cacggcctta 1118940
tcgagcggac gcagggatta tgatttctgc ttcacataat ccttacaggg ataatggaat 1119000
taagattttc tctttagaag gatttaaaat ttctgatgtt cttgagcagc gtattgaaac 1119060
catggtgagt gaagctgact ttggtccatt acccgaagat cacgcagttg ggaagaacaa 1119120
acgtgtgata gatgctatgg gacgctatgt agagtttgtg aaagcaacat ttcctaaggg 1119180
acgcacttta aaggggttaa agattgttct agactgcgct cacggagctt cttataaggt 1119240
agcaccttct gtatttgaag agctggatgc agaggtcatt tgttatggtt gcgaacctac 1119300
aggaatcaac atcaatgaac actgcggagc tcttttccct caagtcattc aaaaagctgt 1119360
aatcgaacat caagcacatc ttgggattgc gctagatggg gatggagatc ggattatcat 1119420
ggtggatgag aaggggcata ttgtcgatgg agatatgatc ctcagtatat gtgctggtga 1119480
tcttaaaaaa agatccgcat tacctcataa ccgtgttgtt gccacgatta tgacaaattt 1119540
cggagtcttg aaatatttgg aaggattagg cctacaggta ttcacctctc cagtagggga 1119600
ccgtcatgtt ttgcacgcca tgttagaaca tgaagtgacc ttnggaggtg aacagagcgg 1119660
acatatgatt ttcttggact acaataccac tggagatgga attgtctcag cgctacaagt 1119720
tttgcgcatt atgatagaaa gtgaatctat gctctcagat ttgacagccc caattgtaaa 1119780
aagtccccaa acattgatca acgttgctgt aagagagaaa attcctcttg aaaccatccc 1119840
tttaattgaa agaaccttga gagatgttca agatgcctta ggcccctctg gacgcatatt 1119900
attaagatat tctggaaccg aaaatatatg cagagttatg gttgaaggtc ataaaaaaca 1119960
tcaagtagac tgccttgcca aggctcttgc ggatgttatt gatgcagaat taggtacagg 1120020
tagtagagag taggatcgta tgtgcgggat atttggatat ttgggaaacc aagatggtgt 1120080
gtctattgtt ctagaaggct tggcaaagtt agaatatcgt ggttatgatt ccgcaggtct 1120140
tgctgctgta gttgaacaag agctttttat tagaaaaact gtaggtcgtg ttcaagagct 1120200
ttcaaacttg tttcaagaaa gagaaatccc tacggcatca gttattggcc atacccgttg 1120260
ggcaactcat ggagtgccta ccgagattaa tgctcatcca catgtggatg agggaaggtc 1120320
gtgtgctgta gtccataatg gaattataga aaatttcaaa gagttgcgac gcgagctgac 1120380
tgcgcaaggc atttcatttg cttcagatac cgattcagaa attattgttc agctgttttc 1120440
tctatattat caagagtccc aagatcttgt gttcagcttt tgtcagactc tagctcaact 1120500
ccgaggtagc gtagctgcgc tttgattcat aaagatcatc ctcatacgat tctttgcgct 1120560

tctcaagaga gccctttaat tcttggttta gggaaagaag agacgtttat tgcttcagat 1120620
tcgcgagctt tcttcaaata tactcgacat tctcaagcct tggcctccgg agaatttgct 1120680
atagtttctc aagggaaaga acctgaggtt tataatttgg agcttaagaa aatccataag 1120740
gatgtacgac aaatcacctg tagtgaagat gcttcggata aaagtggcta cggctattat 1120800
atgctgaagg aaatctatga tcagccagaa gttttagaag gtctgattca aaaacatatg 1120860
gatgaagaag gacatatttt atctgaattt ttatcagatg ttcctatcaa gagttttaaa 1120920
gaaatcacga ttgttgcttg cgggtcttcc tatcatgctg gttatctcgc taaatatatt 1120980
atagagtcct tagtttcaat tcctgtacat attgaagtgg cttccgaatt tcgctatcga 1121040
cgtccctaca taggtaaaga tactttgggg attttgatca gtcaatcagg agaaacagct 1121100
gataccctag ctgctttgaa ggaattacgt cgcagaaaca ttgcttatct cctaggcatt 1121160
tgcaatgtcc cggaatcagc aattgctctt ggtgtggatc actgtctgtt tttagaagcg 1121220
ggggtggaaa tcggtgtagc tacgacaaag gcttttacct cgcaactctt gttgcttgtg 1121280
tttttgggtt tgaaattagc aaatgtacat ggtgccttga ctcacgcaga acaatgttcc 1121340
tttggccagg gattacaaag cttaccagat ctctgtcaaa aacttcttgc ccaacgagtc 1121400
tctccattct tgggcgcagc cttactccta tgaagataag tttctttttc taggccgtag 1121460
gttgatgtat ccggtggtta tggaggctgc cctcaaactc aaagaaattg cttatattga 1121520
agcgaatgcg tatcctggtg gagaaatgaa acatggcccc atagctttaa ttagcaaagg 1121580
tacccctgtt attgcatttt gcggtgatga tattgtctat gaaaagatga taggcaacat 1121640
gatggaggtt aaagctcgtc atgctcatgt gattgctatt gctcctgaat ctcgtgaaga 1121700
tatcgctgca gtttctgatc aacagatctt tgtcccagat tgtcattttc tcgctgctcc 1121760
tgtgttatat actatagttg gtcaagtgat ggcatatgct atggcgttag caaaaggaat 1121820
ggagattgac tgtcccagaa atcttgccaa gtctgttact gtagagtaag tacttcacag 1121880
tagtagacct cgcaatccta tgatgaaact ctttagaact tgtcttaaga gtgcatatga 1121940
aatttaggta tacgttatgt caaataaagt tctaggtggg tccttgctta ttgcaggttc 1122000
tgccatcggt gctggtgttt tagcagttcc tgtattgacc gcaaaaggcg gttttttccc 1122060
tgcaactttt ctctatattg tgtcctggct tttttctatn cctcaggcct ttgccttctt 1122120
gaagtcatga cttggatgan agaatcaaag aacccagtga acatgctttc tatggcggaa 1122180
tctattttag gtcatgtagg caagatttct atatgccttg tctacttgtt tctcttttac 1122240
tccctactga ttgctatttc tgcgaagggg gaaacatctt atgccgcgtg tttaattgtc 1122300
aaaatttagg aatctcatgg attcgtcacc ttggccctct aggttttgct atattgatgg 1122360
ggcctatcat tatggcggga acaaaagtga ttgattactg taatcgtttc tttatgttcg 1122420
gcttaactgt agcttttgga attttctgtg cccttggatt tttaaaaatc caacctagct 1122480
ttctggtgcg ttcctcatgg ttaactacaa taaacgcatt tcctgtgttt ttccttgctt 1122540
ttggattcca aagtatcatt cctacgttgt actactacat ggacaaaaaa gttggagatg 1122600
ttaaaaaggc aattctcata ggaacgttga ttcctcttgt tctctatgtc ttatgggaag 1122660
ttgtggtttt aggtgctgtc tctcttccga ttctttccca ggctaagata ggtggatata 1122720
ctgctgtaga agctctcaag caggcccatc gttcttgggc attttatatt gctggagaac 1122780
tttttggctt ctttgctttg gtctcctctt ttgtaggagt tgctctcggt gttatggact 1122840
tcctggcaga cggtttaaaa tggaataaaa aatcacatcc caattttcaa ttttcttttt 1122900
aacatttatt attccccttg cttgggctgt ttgttatcct gaaattgttt tgacctgtct 1122960
taagtatgct gggggattcg gggccgccgt gattatcggg gtattcccaa cattgattgt 1123020
gtggaaaggg cgttatggca aacaacatca cagagagaaa cagttagttc caggaggaaa 1123080
gtttgcttta tttttgatgt tcttgttgat agtaataaat gtagttagca tttatcatga 1123140
gctttaaatt tgttttgctc tatttttcaa tttcaagtga gcaaatagga nnnttctatg 1123200
ggactatatg atcgtgacta tatacaagat tctcgagtgc agggaacttt tgcttcaaga 1123260
gtctatgggt ggatgacagc agggctaatc gtaacttcat gtgttgccct gggtctttat 1123320
ttttctggat tatacagaag tttatttctt ttttggtggg tgtggtgttt cgctacgcta 1123380
ggcgtgtctt tctttatcaa ctctaaaatc cagacactat cggtagttgg tcaagtgatg 1123440
gcatatgcta tggtgttagc aaaaggaatg gagattgact gtcccagaaa tcttgccaag 1123500
tctgttactg tagagtaagt acttcacagt agtagacctc gcaatcctat gatgaaactc 1123560
tttagaactt gtcttaagag tgcatatgaa atttaggtat acgttatgtc aaataaagtt 1123620
ctaggtgggt ccttgcttat tgcaggttct gccatcggtg ctggtgtttt agcagttcct 1123680
gtattgaccg caaaaggcgg ttttttccct gcaacttttc tctatattgt ctcctggctt 1123740
ttttctatgg cctcaggcct ttgccttctt gaagtcatga cttggatgaa agaatcaaag 1123800
aacccagtga acatgctttc tatggcggaa tctattttag gtcatgtagg caagatttct 1123860
atatgccttg tctacttgtt tctcttttac tccctactga ttgcctattt ctgcgaaggg 1123920
ggaaacatct tatgccgcgt gtttaattgt caaaatttag gaatctcatg gattcntcnc 1123980
cttggccctc taggttttgc tatattgatg gggcctatca ttannncggg aacannantn 1124040
attgattact gtnancnttt ctttntntnn ggcttancng tnncttttgg aatttnctnt 1124100
gcccttggat ttttaaaaat ccaacctagc tttatggtgc gttcctcaat ggttaactac 1124160
aataaacgca tttcctgtgt tttttcnttg ctttttggat tccaaagtan cattcctacn 1124220
ttgtactact acatggacaa aaaaagttgg agatgttaaa aaggcaattc tcataggaac 1124280
gttgattcct cttgttctct atgtcttatg ggaagttgtg gtttnaggtg ctgtctctnt 1124340
tccgattctt tcccaggcta agataggtgg atatactgct gtagaagctc tcaagcaggc 1124400

ccatcgttct tgagcatttt atattgctgg agaacttttt ggcttctttg ctttggtctc 1124460
ctcttttgta ggagttgctc tcggtgttat ggacttcctg gcagacggtt taaaatggaa 1124520
taaaaaatca catccatttt caatttcttt tttaacattt attattcccc ttgcttgggc 1124580
tgtttgttat cctgaaattg ttttgacctg tcttaagtat gctgggggat tcggggccgc 1124640
cgtgattatc ggggtattcc caacattgat tgtgtggaaa gggcgttatg gcaaacaaca 1124700
tcacagagag aaacagttag ttccaggagg aaagtttgct ttatttttga tgttcttgtt 1124760
gatagtaata aatgtagtta gcatttatca tgagctttaa atttgttttg ctctattttt 1124820
caatttcaag tgagcaaata ggagggttct atgggactat atgatcgtga ctatatacaa 1124880
gattctcgag tgcagggaac ttttgcttca agagtctatg ggtggatgac agcagggcta 1124940
atcgtaactt catgtgttgc cctgggtctt tatttttctg gattatacag aagtttattt 1125000
tctttttggt gggtgtggtg tttcgctacg ctaggcgtgt ctttctttat caactctaaa 1125060
atccagacac tatcggtttc tgctgtaggg ggccttttcc ttctctactc aacattagaa 1125120
ggaatgtttt ttggaacctt acttcctgtc tacgctgctc aatatggcgg aggggtgatc 1125180
tgggccgctt ttggatcagc agccttggta tttggcttag cagcagtata cggagcgttt 1125240
acaaaaagcg atcttactaa aattagtaag attatgactt ttgctttgat aggacttctg 1125300
ctagtgactc tagtctttgc tgtggtttcg atgtttgtat ctatgccttt aatctactta 1125360
ttgatttgct atctagggct cgtcatcttt gttggattga cagctgctga tgcgcaagca 1125420
attcgtcgga tttcttctac tataggggat aacaatacct tgagttacaa actctctttg 1125480
atgtttgctc ttaagatgta ttgcaatgtc atcatggtat tttggtatct gctgcagatt 1125540
ttctcatctt caggaaaccg agactaaaca acgacttaga tcttttctac ttcaggaaat 1125600
aatttgttta gaaaaagatc taagtcgaat tcattaagat cctttaaaga ttccccataa 1125660
ccaataaact ttgtggggat tttcagtcgt ttagctattt gaaatagggt gccacccttg 1125720
gcagagccgt ctacttttgt gaagataaga ccagaaaggg gaacaacatc atgaaatacc 1125780
cgcacctgct ctatagcatt attccctaag gtagagtcca cagtcataaa aatttcatga 1125840
ggagctccct ctagagcttt gccgcaaacc gagactattt tggaaagctc tttcataaga 1125900
ttgccatgta catgcaggcg acctgaggta tcaataatga ctctagagta cccccgggcg 1125960
attgcagatt gaatcccatc aaaggcaata gcagcagcgt cccccccggg ttgtccagag 1126020
acaaagccac agccaagttc gttggcccag agtcgtgcct ggtccatgcc agcagctcga 1126080
aaggtatccg tggctacaag catgacgctt tcagatcgct ctttgtagta atgagcaagt 1126140
ttggccgctg ttgtcgtttt tcctgagccg ttggtcccaa gaagtaaaga aacaatcggt 1126200
ctggtctggg aactttgtga agcttgagaa ggaagacctt ctagagactc gcgaagtaaa 1126260
actgtaatca gatctttgat agtagatgcg tcagcttttt tagtccgacg taatcgtgca 1126320
cacaattctt cagtaagctc agtaccaaaa tctgcttcat aaaacaaact ttcagcatct 1126380
tctataagat ctaaagatat atttttttta aataaagatt gaagcttgtt tctaaagaat 1126440
ttgaacattg ctaatagcaa agttagttga agtaaaagaa atgatacaag ataagaagga 1126500
aaaagcaaag agttatttat gcatcttcat gagtaccaag ctaaagatct cttagcttct 1126560
tacgatgtgc ctattccccc ctattgggta gtgtcttcag aggaagaagg ggaacttctt 1126620
atcacaaagt cgggattaga ttccgccgtg gttaaagtac aggttcatgc tggaggtcga 1126680
gggaaacatg gtggtgtcat tgttgcaaaa tcctcagcag gcattttgca agccgtagct 1126740
aagctcttag gaatgcattt tactagtaat caaactgccg acggatttct ccccgtagag 1126800
aaagtcttga tttcaccact tgttgctata caaagggaat attatgtcgc agtgattatg 1126860
gatcgaaaac accgatgccc agttcttatg ctttcaaaag caggaggtat ggatatcgaa 1126920
gaggtcgcac actcttcccc agaacagatc ctgacattac cactgacctc ttatgggcat 1126980
atctatagtt atcaacttcg tcaagccact aagttcatgg agtgggaagg agaagtgatg 1127040
caccaaggtg tccaattgat caaaaagctt gcgaagtgct tttatgaaaa tgatgtctct 1127100
ttattagaga tcaatccttt ggtgttgact ttagagggtg agctacttgt cctcgattct 1127160
aagattacga tcgatgacaa tgccctatac cgtcatccca acttagaagt attgtatgac 1127220
ccctctcaag agaatgttcg ggatgtccta gcaaagcaaa tcggattgtc ctacatcgct 1127280
ctaagcggaa atatcggttg cattgtgaat ggcgccggac ttgccatgag taccttagat 1127340
attttaaagc tccatggtgg gaatgctgcg aatttcttag atgtcggagg aggggcttct 1127400
caaaagcaaa tccaagaagc tgtatcttta gtattgtccg atgagagcgt gaaagtgctt 1127460
tttatcaata tcttcgggnn gattatggac tgttctgtag ttgcctccgg gctcgttgca 1127520
gttatggaaa caagagatca agtggttcct acagtcattc gtctcgaagg aacaaatgta 1127580
gaattgggaa aagaaattgt tcagcaatct gggattcctt gccaatttgt ctcttctatg 1127640
gaagagggcg ctagacgcgc tgtagaactg agtatgtaga tttaggagat acatgttcca 1127700
ctcactaagt aaaaatactc ccatcatcac acagggaatt acagggaaag caggttcatt 1127760
tcatactgag cagtgcctcg cctacggtac gaatttcgta gggggtgtga ctccaggcaa 1127820
agggggaacc ctttggctag acctgccagt ctatgattct gtacttgagg cgaagcaagc 1127880
tacagggtgt cgcgcgacca tgatttttgt tccaccacct tatgccgccg aggccatcct 1127940
agaagccgaa gaggccggaa tcgaacttat tgtttgtatt accgaaggga tccctgttcg 1128000
agatatgctt gaagtggcta gagttatgga caacagtact tcacagctta tcggcccgaa 1128060
ttgtccagga attatcaagc ctggagaatg taaaattgga attatgccag gatatatcca 1128120
cctgccggga aatattggag tggtctctag atcaggaact ctgacatacg aggctgtttg 1128180
gcagctcact cagctcaaaa tcggccagag tatttgtgta ggtattgggg gagaccccct 1128240

caatggaacc tcatttattg atgtcttgca ggcgctggag gaggatccct atacagaact 1128300
gattttaatg atcggggaaa tcggcggtag tgccgaagaa gaggccgcgg cctggatcca 1128360
agcacactgc acaaaacccg tggttgcttt tattgctggt gttacagctc caaaagggaa 1128420
acgcatgggg catgctgggg ccatcatctc tggaaattct ggggatgcca aaagtaaaat 1128480
acaagttcta cgtgaatctg gagttactgt agttgagtct ccggcccata tcggaaaaac 1128540
tgtagacgct gtattacggg caaaagaact ctaatgctta atcatctaac ctacaagtac 1128600
aaggttcgct taatcctttg gattgggagc tctctacttt aatgaagatc ttgtgcttgg 1128660
ggactcattc ttgcgcctag ttcttattca tgtcttacgt cgctatttcc ctgtgggtaa 1128720
ggaaaatcag aaaaagagac ctattcgcaa gttataaagt atttacttga aatattattt 1128780
ccctcatcta ctagctcttt aagaactagc caagggtgga tagaagtgcc ttcaaggagt 1128840
gctctggcag ccatatctaa gtgtgtgttc aaaccctatt ctggcaacac aaataagata 1128900
gatgttgtgg gatactatat cataagagta tactgtttcc aatttcttca aatccgtaag 1128960
cttttttctt tccttacaca cgactttatt ttttgaaata tcttatcaat accttagaac 1129020
gaagttctaa ttaaaataaa atcaggatgt tatgcaggtc tacgagttct ctcgtcaagt 1129080
tcaacccaca tttcaaaatt tactcatgga aaaattagat gcctgctttt tctttggagg 1129140
aaagcgggta aaaattatcg gtttagatga acctaatctg gcttgaggaa tagaagaaca 1129200
ggttgcacta tccattgcta taaagatcct taaaattatc cttgcactta ttttgttccc 1129260
attagtttta ctcgcctggg tgattcgcta tcaattgcac gcaaatttcc attgtagtgt 1129320
agtgcccttc ccaggatttt cggtaaacca agcatacaaa tgtagtgagg ctaagataga 1129380
agaaatgcta gatcttctag acttagaaac cctggaatgg tcatcaagat gccttcgtca 1129440
agatatgaca tttgcaaatc ggctagagga agagctgatt caagaacttc gtgtgtcaga 1129500
gaccgaggag ttgattagtc ttggtggtaa acgaaacctt gtgcgtttac ttctcacgca 1129560
ctctttttaa tcctccaaaa gagatcgcgt gtagagtctg taggtcatga ggttgtcttt 1129620
cctgtatttg ataggctaaa gagagaagag gaaatcattg gggatggacc catcactcga 1129680
tccaatgagg aactgtgggc tcttctagat catggaactg ctcgcgggat ccataagacc 1129740
ctttggtttt cgattttttt taagtatcta acacaaatcg aactgttcta gaagcaggta 1129800
ttggagggga gattttcaag agacttaacg ctatattcca gacttctacc ggtcaaacat 1129860
tgactttagc tccgaagctc tcgaatcttt gcttttctgt tcatttctct gggttgctgc 1129920
gttgggaaaa cattcaagtt gagtagagag aaattgttna ttttgtttct tnncaaanat 1129980
tttatcttac cagtctcttc tatttgtctg agttacttga gtgatgcgtg aatttttaca 1130040
tgtgactttt ggatcatgga taactggaac ttttataaaa gatatcgagt tcagaaaaga 1130100
agaaaaaaac ctcttttatt taatgattta ttttttaaag tatataaata atttataatt 1130160
ataaaataat ttacttttta ataattaaaa tgtaaactgt ttgcacttta ataataatta 1130220
taattataga ttttgatgct tgtctattgt tttgatccct cggtaccaac ttcaccagaa 1130280
caccgcctga tggcggcatt agaccgttgg tttttcttag gaggacatcg tgtaagaatt 1130340
ctcacgcttg agggtaatca ctatcgggct tttcaagaaa atatgtctat ctcaacagta 1130400
gagaagatat tgaaactaat ttcctatcta ctcatcccta ttgtccttat tgctttgcta 1130460
atccgttgtt tcttacactc taggtttaaa tgtaattgga aatgcgattc tctaagtgac 1130520
gcaagagtcc cccacgacgt tcaacctttc aatgatttcc aacttttcaa taatcaggag 1130580
aggctaaata tatggaaaaa tcggagatat gtctctggaa tagatgtgct tatggtgccg 1130640
gtcgattatc tcagatctca gtttcccggt tttaaggaga ttccagaagc tatccgttgc 1130700
gaaaactatg taagtgatgg tcagttttct gaagaaagta aaacaagcta tcttagagcg 1130760
atgctgaccg acattgttgg gtatatcttg tcattagatg agacctattg gacgaatgtg 1130820
atcctcaaga tccgtgcgat gtgcatcacc ttcgaaagtt tccctggaaa agaagcagac 1130880
cctaactatt cccctcgcgt aactcaccac tattttgatg aatcttggaa ggcacttgcg 1130940
cgacatgtat taggagaagg taacatggtg aatagactag atgaggcgct aattcgtaca 1131000
gagaagccgg gcaaagaagg ggaatgtatt acgaaacagt ttttaaaaga ttactgtaaa 1131060
aagcatctcg aagttatgag ttgtccagat ttcattgaat ctctggtaga tgaaaaaatc 1131120
cgagagtttc gttgtccaag tattttaaac tcagcagtat gtgatgtaat agatcgcaaa 1131180
tgccaagaac atttgttaaa ggcaataata aacgaagcaa accgcaggct ccctgggatg 1131240
aaaaatagct cattcactat gcgtggtaac caagttttat tttataccat cttttctcca 1131300
ccaaaattgc cacctgctgc gagttccgta tatttttaaa gaaaggagat ctcttggtct 1131360
cctagtcctg ctataaggtc ttgatataca tctttttgga gtaatttcat cttttatatt 1131420
attttattaa aatctttata attcgtttct tttgaaatat ttattgtatt tcttaataac 1131480
tgtatataaa ccaatttgct aatattttaa aaagtagttt tttgatggaa gtttatagtt 1131540
tttccccttc agtaagaact tcgtttcagc accgtgtaat ggcggcacta gataattggt 1131600
tttttctagg agggcgccgt ttaaaagtag tttctctaga tagttgtaac tcagggcagg 1131660
cttgtgaaga atacgtgcct atttcaacga cagaaaaggt cttaaagata ctctcttacc 1131720
tactcatacc gattgtcata atagctctgt taattcgtta tcttttgcat agcaatttta 1131780
cggcaaaggt atcacagaaa ccttggttaa agaccctgca gttaggaatt gatataaaaa 1131840
gcttcatact tcccggttct catgtaaaca cgatggattc agctactttg tttaaagcaa 1131900
ttcgtttgga agggaagcgt gttgatgtag aatatcatag gctacatagc agcgataagg 1131960
tggtttttta tatccctgct cagaaacttc cagatgatct gcgtttgact cattggcttc 1132020
cagaaaaaga aacaagaaag actgagtatg tgagacatat gctggcccat gtcatgggtt 1132080

atctaacatc acagggtaag gaacggcttc aacaggtagt gcaagactct cgaagcagta 1132140
cttccttggg ggctgaaaaa gtccttcaat acagattcat tgatcatcca cagagtcaag 1132200
gagaatttca acgtctgctt aatgaaaata taacgaccaa aggttccgag gataaggaag 1132260
ttgtacagag tgatttattt gacatggctt ttcagtgttg gtggccacag tttatttcag 1132320
ttatacaatc tccgaccttc agtgaagaat tagtacacga aatgagtcag aaacttgatt 1132380
tagattgtat atacccagaa gatgatgaat ttgagcagaa gttccttaat acccttctga 1132440
aagcagtctt gcaccacggt tttgaaggaa tcagtgttgc gagtatgggt gttatcttcc 1132500
tgatttgtcc ggactctctt gcattacaga ttcccttctt aaggaatcaa aaatgatgca 1132560
ggtgtcgagg ttcttagttt taaggtagta agacctcccc gcggtagaat ataggatctt 1132620
caagctcaaa gtctccggct ttgagcgatg ttcccctcaa actttaattc tacatcatga 1132680
agtattctca atctaaagat aaaaaatcct tttgtagtgc tgtgttagtt acatatgatt 1132740
tgttttttta atatgcattg tatttttcaa taaataatga taaacacttc gcgaatgttt 1132800
tgaaaatatt tttttatgga agtttatagt tttcacctgc ggtaaggact tcgtttcagc 1132860
accgtgtaat ggcagcacta gatgcttggt tttttctagg agggcaccgt ttaaaagtag 1132920
tttctctaga tagttgtaac tcaggttggg cgtatcaaga acttgtgtct atttcaacga 1132980
cagaaaaagt cttgaaacta ctctcttacc tactcgtacc gattgtcata atagctctgt 1133040
taattcgttg tcttttacat agcaatttta ggatagacgt agagaaggaa cgttggttaa 1133100
aaataaggga gttaggaatt gatatagaaa gctgcaaact ccccagttct tatgtaaacc 1133160
aggtttcctc gtttatttgg tttgaaaaag ataaatccaa acggccacgt attgatgtag 1133220
attatcatac gctacatagc aaagactggg tagttttccc tatcgttttt cagaaaattc 1133280
caaagacctc gcgtttcagt tattggttct cacaaaaaga aacaaggaag agggattatg 1133340
tgagaaatat gctggaccac gtcattggtt atctaacgtc agaaggtggg gagtggttgc 1133400
agtatatatc gaaaacctct tatcaaagcg ctacttcctt ggatcctgaa agagttcttc 1133460
aatattgctt aactgataac caggagctcc agggagaagt gcaacgtttg cttaatgagg 1133520
agagtgcgac caaaagctct ggggataagg aagttttgtt aagtcatgta tctgacatta 1133580
tttgccagtg ttggtggcca aagtttcttg aagttataca atctccggcc tttattgaag 1133640
aattagtaga agaagtgagt ggtaaactta atttagattt tttatgccta gaaaaggcta 1133700
atacattaga tcaggagttg agaaacagtc ttctaagagc agtcgtacac cacggttctg 1133760
aaggagttga tattaagaaa gttggtgccg gcctcattat ttatacggaa gctattcaat 1133820
tacagattcc cttctcaagg agttaaaaat gatcgtagtg tcggggtgct tagttttaag 1133880
gtaacaagac atcctcgcag tagaagagag gatcttcaag ctcaaagtct ccggatttga 1133940
gcgatgtttc cctcaaactt taattctaca tcatgaagta ttctcaatct aaagatataa 1134000
aatccctttg tagtaatgtt ttcgtcacat atgatttgtt tttcgtaatt actaggtagc 1134060
agaacctgtg ttagctagta tacgatggta ttctgcttca tcgaaagaca accttcttga 1134120
gggatagata tgataactaa gcaattgcgt tcgtggctag ctgtacttgt tggttcaagt 1134180
ctgctagctc ttcctttatc agggcaagct gtcgggaaaa aagaatctcg agtttccgag 1134240
ctgcctcaag acgttcttct taaagagatc tcgggagggt tttctaaggt cgctaccaag 1134300
gcgactcccg ctgttgtgta catagaaagt ttcccaaaga gccaggctgt aacacatcct 1134360
tctcctggac gccgtgggcc ttatgaaaat ccttttgatt attttaatga tgagtttttc 1134420
aatcgttttt ttggtctacc ttcacagagg gaaaaacctc aaagtaaaga ggcggttcga 1134480
ggaacaggtt tcctagtatc tccagatggc tatattgtga ctaataacca tgttgtcgaa 1134540
gatacaggta agattcacgt aactcttcat gatgggcaaa agtacccagc aactgtaatc 1134600
ggactcgatc ctaaaacaga ccttgcagtc attaaaatta aatcccaaaa cctcccgtat 1134660
ctttcttttg gaaactccga ccacttaaaa gtcggagatt gggcaattgc aattggaaat 1134720
cccttcggtc ttcaagctac ggtcaccgta gtgtcatcag tgctaaagga agaaatcaac 1134780
tccacattgc agattttgaa gattttattc agacagatgc tgcgattaat ccaggcaact 1134840
ctggaggccc tcttctaaat attgatggac aggtcatcgg tgttaatact gccattgtca 1134900
gtggtagtgg tggctatatt ggaatcgggt ttgcgattcc tagccttatg gcaaatagaa 1134960
tcatagatca gctgattcgt gatggtcaag ttacccgagg attcttagga gtgactttac 1135020
aacctataga tgcggaactc gctgcttgct acaaactcga aaaggtttat ggcgctttag 1135080
tcacagatgt tgttaaagga tctccagcag ataaagcagg gctaaaacaa gaagatgtga 1135140
tcattgctta taatgggaaa gaagtcgatt cactgagtat gttccgtaat gctgtttctt 1135200
taatgaatcc agatacacgt attgttctaa aggtagttcg tgaaggaaag gttatcgaaa 1135260
tacccgtgac agtttctcaa gctccaaaag aagatggaat gtcggcttta cagcgtgtgg 1135320
gaatccgtgt gcaaaaccta actcctgaaa ctgctaagaa gctgggaatt gctccagaga 1135380
ctaaaggcat tttgattata agtgttgaac cagggtctgt agcagcttct tcaggaattg 1135440
ctcctggtca gctgatcctt gctgtgaata gacaaaaagt atcttcgatt gaagatctga 1135500
atagaacgtt aaaagattct aacaatgaga atattcttct tatggtttct caaggagatg 1135560
ttattcgctt cattgccctg aaacctgaag aataaaaagc ttggagtcat acatttgatt 1135620
cataaagagg aatgaagaga gttctctctt cactctttta ttttaggaag cttatcaaga 1135680
agctgacaaa tagaaaggaa tcccttttta agttggtcca tcgagaaatg ctcttctgca 1135740
gcgtgaatat catcactaag atacgaagtc ccacaaatga taggtgaggt ttgtgctgct 1135800
tcccctaata ggggacctat cgggatcgta gcaggcatca ctaatctcag gcattcttca 1135860
ttgtagaggt cactatagat ctcttgaagc actttgacta ttgggaggtt cgcagaactt 1135920

ctccaacctc gagatcctcc tggaagaatt tcataagaga acctttaacg aagaagggac 1135980
ttgttgtttc aagtgatgga taacttggtg agcagcttta tcaggatctt gattaggaac 1136040
aagtcggcag gagaggtagg cagtggctct ataggggatc acagtcttaa atcctggtcc 1136100
cgtataacct ccagaaattc cattgatttc tacagtagga cgtaacgcag attcttcagg 1136160
gctataagaa gcttcgtaac cctgaggtcg aaaccctaag ttttcttcgc attcccgaag 1136220
ggtatccgac ttcggaagat caggacgatc gctatccgag gggagagcaa gatcatcata 1136280
aaatccttca atagctatag aattgtcagg gtgatgcaga gagctcagaa tttctgataa 1136340
agcacgattc gtattgtagg caattcctcc taaaactcct gagtgcatgt ccttgttccc 1136400
ctcttcaagg gagattttca tggaaacaat accccgagct ccaatgctta cgtaggggtg 1136460
tttttcagaa aggaaacccc catctacgat cagaagatag tccgcgcgta aagcttcttt 1136520
tttcttttct aaccaagtaa ataatgcgag actcccactc tcttcttcac cctcaattaa 1136580
ccaaataata tttagaggga agtttccttg agattcgtaa tagtgctgta atgcctttaa 1136640
ggtgtaaaaa cattgtcctt tgttatcaga ggctcctcgg gcatagagat tgccattctc 1136700
ttctctaagg ataaagggat ctcccttcca accatcagat agctgtgctg gctgcacatc 1136760
atagtggtta tagagcataa gagtagggct aagaggatct tcgcttttat aggatgcata 1136820
gatgataggg ggatgcccag gcgtttccca gagctctaca tcgaaaatct tattcacatg 1136880
atctactaaa aaatgtgcac aattttcaca atcctgaaga tgatcactat ccgcagatat 1136940
cgaggggaag cttataaatt ttgcaaactc ttccaggaaa tttgcactat ttatatcaaa 1137000
atgtttcgaa tctagattca taggtttccc agagcagttg ttgaaaataa tatcacagta 1137060
acttaacttg tcttttgtcg aaaagaaaat aagaacatag ggtttagcgt gctttttagc 1137120
atgattaagc atgacaaatt agggattcag ccctaactaa tcagatgagt tacagaaagt 1137180
atgaaagctg gtgatacgta tagaaatttc attattaagt catgcaaaga tcttcctgaa 1137240
atagaaagta agctccttga ggcggaacat aagccgacag gagcttctat tatgatgatc 1137300
gtcaacaacg atgaggaaaa cgtattcaat atttgcttta gaacatgccc acaaacttct 1137360
aacggtgttg cgcatgttct agagcatatg gtcctttgtg gttccgagaa ctaccccgtg 1137420
cgggatccct tcttttcgat gacacgtcgt agtctgaata ccttcatcaa cgcgtttaca 1137480
ggtccagatt ttacatgcta tcctgcagct tctcagattc ctgaagattt ttataattta 1137540
ctcagcgtgt atattgacgc cgtgtttcat cctttgctta ccaagcaaag tttccttcaa 1137600
gaggcatgga gatacgaatt caattccgag aatcatctct gttatacagg agtagttttc 1137660
aatgagatga aaggtgcgat gatgtcggga gaggcacggc tctctgaggc tctaaatgca 1137720
gctattttcc cttctgtgac ctacggcgta aactcaggag gagaacccag agagattgta 1137780
actctatctc atgaagacgt ccgagcattc catcaaagcc agtatagtat caatcgatgc 1137840
ttgttttact tttatggaaa tatcaaacct tcccgtcatt tagatttctt ggaagaaaag 1137900
ctactcagac aagctacaaa gctagaaaag caggcagtat ccgtaccttt gcaaaagcgg 1137960
tttaaggaac cagtaaggaa catcttaacg tatcctgtcg atcatcaaga agaagataag 1138020
gtccttttcg gaatttcttg gcttacctgt tccattttag aacaacaaga acttcttgcg 1138080
ctgcatgtgt tagaaattat cctcatgggt acagatgcct ctcctttaaa atctcgattg 1138140
ctgaaatcag ggttctgtaa gcagacagaa atgagcatcg agaatgatat ccgtgaaatc 1138200
cccatgacct tggtatgtaa aggatgttct ccagcaggag cccagaaatt agaagctttg 1138260
attttcgctt ctcttgaaga aatcatccgc gaagggatat cagaaaatat tgtagaggga 1138320
gcagttcatc agttagagtt atctagaaaa gagatcacag gatattctct accctacggg 1138380
ctgtctctat ttttccgctc cggactattg aaacagcacg gaggctctgc cgaagatggc 1138440
ctgagaatcc ataacctctt ttctgagctc cgtaattcgt taaaaaattc tgattatctt 1138500
gccaagttga tccgcaagta cttcttagat aatccacatt ttgcaagagt gattttactt 1138560
cctgataccg aacttgttgc taaggacaat aaagatgagc agcagctttt actcagtgtt 1138620
agtgaaaaac tcacagacga gaataaagaa aaaattcaac aaaatgtacg agaactcaca 1138680
gaatctcaag aacaaaagga agatctgaat ggtattcttc ctaaccttgc tttggataaa 1138740
gtgcccacgt ctgggaagga atttccttta attaaagagg ggctgagtca aggagaagtt 1138800
cttcatcacg aatgcttcac gaatgatatt gtcttcatag atgttgtctt agatatccct 1138860
ccgctatctg gagaagaact tccttggttg cgtttgctcg tatttttaat gttgcagctg 1138920
ggatgtggag gcaggtctta taaagaacat ttagagtttt tgttagaaca tacaggtggc 1138980
gtggacgtct cttatgattt ctctcctcat gcaaataaaa atagtttcct ctctccatcg 1139040
gtaagtatcc gaggcaaagc tctgtcatcg aaatctgaga agctctgtgg tattgtcagc 1139100
gatatgttaa caagcgtgga tttcacggat attcctagga ttcgtgagtt gctgatgcag 1139160
cataacgaag ccttgaccaa tagtgtgaga aatagtccca tgagctatgc agtgagcatg 1139220
gcctgctctg ggaattctat aacaggggcg atgtcttact tgactacagg gcttccctat 1139280
gtgaaaaaaa ttcgcgaact cacaaaaaac tttgatcaga atatagacga agcggttgtc 1139340
atcctacaac gattatacac caagtgtttc tcagggaaac ggcaaattgt cattagtgga 1139400
agcgctcata actatcagca attaaaagat aataagtttt acggtctttt agactacctg 1139460
attgtgattc ccgaaccttg ggaaaatcca agtataaact tatatgtaac ctctcggggg 1139520
ttgcatattc ctgcacgtgc agcattcaat gctcttgctt tccctattgg cgatatagct 1139580
tatgaccatc ctgatgctgc agctctaaca gtagcagcag agatcttaga taatgtggtc 1139640
ttgcatacta agattcgaga gcaaggagga gcctatggct cgggagcagc tgcaaaccta 1139700
tcacgaggat ctttctattg ctatagttat cgagatccag aaattgcaac tacctacaaa 1139760

acgtttctaa agggagtgtc agagattgct tctggtaatt tcactaaaga agatatctat 1139820
gaaggagctc ttggagttgt ccaaggtttg gatatgcccg tagcgccagg aagccgtgct 1139880
tccgtagcgt tttataggct aaaaagtggt aggattcccg tgttgcgcca agcattccgc 1139940
cgttctgtcc ttgaagtgac taaagaacac atttgtatgg ttatggataa atatctagag 1140000
tcaacagttc aggagacaac tttgatatct tttgcagggg aagagatgct tcgtaataat 1140060
gttcttactc tagataaaga tttccccata gtaccagcaa tttgagactt agtcttgttt 1140120
ttctaaagac tcaataacag ctaagttcgt atctatatca catgtgtgtg gaaacgaggt 1140180
ggctaagctt tctatagggg taggctcttc aatctgatgg gatgacgagg tttccaagcc 1140240
ttcgaacttc cttaatgtag gcagtacccg gtattgaaag ctagaggtca tatcgttgta 1140300
actttgtacc gtctggttta aatttttgcc tatcttctga aaatgtgtga acactacctg 1140360
taggcgacga tggagctctt tacctaaaag actgacctct tgaatttgtt tttgcagatt 1140420
ctcttgcttc cacatgtaag caatcgtctt aagtagagca agtaaagtca aaggactgga 1140480
taagattacg tttgaagaag ctccaatctc catcaattca ggagctaacc gaattgcatc 1140540
attgaataag ctctccccag gaagaaaaag aatcacgtat tctggtgatt gatgaaactt 1140600
ttcccagtaa ctcttggatt tcagagtctt aatgtgctct ttaattttat caacaagatc 1140660
ccccttgtca atctcctcta cagaaaaata ggagtctgaa atcggagctt tagcatcaat 1140720
aattaaacag cgatcttgag gcagccgaat aataatatcc gcccgaaatg ctccttgggc 1140780
acttgtggtt tgactgtcgt agtcacagta ttttaacatc ccggcaagct ctaaaattct 1140840
ctccagttgg atttctcccc aacgtcctcg agatcctggg tgctttaaaa tgtccgtgag 1140900
aacatgagtt tcatgctcca attttttctc tacagcaagt aattgcgata tctgttcttt 1140960
taaccttcca cgatcttcgg catgcttagt ttcaaatgtc tctaaacttt gcttaaatgt 1141020
cgtgagtgtc gtttggatag gagaaagtat agactgaaaa gatttagacg tatcaccaaa 1141080
ataattctga gcctcttctt tcatatcttt gatgagtttg tgagaagaaa gtgctagacg 1141140
attgctgaaa tcttcaatta actgctcttg atggcggcta agatttaaag aagtctgtag 1141200
cagttggttt tcatgttcta atttctgaat cttctctaga aacgcacgct tcttccttgc 1141260
atagagagaa gacgatacga agactcccag aaaaaacaca cacccagata aaagtaggca 1141320
cgctagggaa acagggaggt tcataacaga tgctagctct tttttctata gatgattgaa 1141380
aagatcggaa agcctactaa agtatatagc caagatacaa ggaaaaagac ttcaacaaaa 1141440
tgatctacaa gtcctgaaaa gaaaagacat gctgctaaac ctatagtcac taccagcaga 1141500
aaagaagaaa cattaaagcg gaaatgcttc actccaggaa atttccatgg agaaatcatc 1141560
agacccccaa tgaaaagaag ggcaaatgat aatagaccta cccgaagttg ggcagggagg 1141620
tctggaaaga aatccgaagc aagaaataaa gctaaggaga caatacttgc agcagcagca 1141680
ggaatgggca gaccaataaa acaataaggc tttgaaacat ctacagtctt ttgagaaaag 1141740
aggttgtaac gtaccaaccg caacactcca cacagagaat aaataatcga ggtgatgagt 1141800
agtagcgaag agaaaaaatt cccaacataa atcccatcaa gactttttat agcaatcaaa 1141860
ggtggggcaa tgccgaaagt gaccgcatca gaaagagaat caaactgagc accaaaagcg 1141920
ctctcggctt tcatgattcg agcgatcgca ccatcagaaa aatccgcgat catagcacta 1141980
ataagcaaga gagataagcc ctgcaaacga tggaataact ctacagagga cgacgttctt 1142040
aatacgcttt tgaaaataat aaataatcca caacaaagtc caaaagcagt aatagcgtta 1142100
ggtgtcacta cgcgacgctt gcctcgtgct tctagatcta atcccgccat gtcaatttgc 1142160
cagagtttct tttgctcgta gcataatgga ttcttctaaa atcaacaagt ttttgcagtc 1142220
ctcttgtcta ttaagatctc tatctccaaa agctcctaga gtatgatatt tttaccgaga 1142280
aagggagcat ttttcaattg caggaaacca tgacaattcc tatatatggt agataagcgc 1142340
ataacgtttc actacatatt gggtttttta aagaaaaatc cttcttatgt ttaaaggcgt 1142400
ttttctttct actttttaat tataacaaag gaaaaagtac tccaatattt atggttttgg 1142460
agtcctgatt ttctagtgcg caaagaataa nagaatctaa tatttanata tcattttagg 1142520
gaaaagttat ggtcgaagtt gaagaaaagc attacaccat cgtcaaacgt aatggaatgt 1142580
ttgtcccatt taatcaagat cggattttcc aggctttgga ggcagctttt cgagatacgc 1142640
gtagcttaga aactagttct ccactaccta aagacttaga agaatctatt gcgcaaatta 1142700
ctcataaagt cgtgaaggaa gtcctcgcta aaatttcaga aggtcaggta gtcactgtag 1142760
agagaatcca ggatcttgta gaaagtcagc tctatattag cgggttgcag gatgtggctc 1142820
gcgattatat tgtttacagg gaccaacgca aggcagagcg cggtaactct tcgtccataa 1142880
ttgccatcat acgtagagac gggggaagcg ctaaatttaa tcctatgaag atctctgcag 1142940
ctctcgaaaa agcattcaga gcgacgctcc aaattaatgg gatgactcct cctgcaacac 1143000
tatccgaaat taatgacctt acccttagga tcgttgaaga tgtcctaagc cttcatggtg 1143060
aagaagctat taatctggaa gagatccaag atattgttga aaagcaactt atggttgccg 1143120
gctattatga tgtggccaag aattatattt tatatagaga agctcgtgca cgagcccgtg 1143180
ctaataaaga tcaagatgga caagaagagt ttgtccccca agaggaaacg tacgttgttc 1143240
aaaaagaaga cggcaccacc taccttctga gaaaaacaga tttagaaaag aggttttctt 1143300
gggcatgcaa acgctttcct aaaactacag attctcaact gcttgcagat atggcattta 1143360
tgaatttgta ttcaggaatc aaagaagacg aggtcaccac agcatgcatc atggcggcac 1143420
gtgccaatat cgagagagaa cctgattacg cttttatcgc agcagaactc ctcacgagtt 1143480
ccttgtatga agagacctta ggatgcagct ctcaagaccc caatttatca gaaatacata 1143540
aaaaacattt taaagaatac atcctcaatg gagaagagta tcgcttgaat cctcaattaa 1143600

aggattatga tctcgatgct cttagtgaag tcctagacct ctctagagac caacagtttt 1143660
cctatatggg agtccaaaat ctctacgatc gctattttaa tctgcatgaa ggacgacgtt 1143720
tagagactgc gcagatcttt tggatgcggg tttctatggg cttagcctta aatgaaggag 1143780
aacaaaagaa tttttgggca atcactttct ataatctgtt atccacattc cgctataccc 1143840
cagcaactcc tacattgttt aactccggaa tgcgtcattc ccaactcagt tcatgctatc 1143900
tttccacagt aaaagatgac ctaagtcaca tttataaggt gatttctgat aatgctttgc 1143960
tttctaaatg ggcaggggga attggaaatg attggacaga tgtccgtgct acaggagctg 1144020
taattaaggg aaccaatgga aagagtcaag gcgtcattcc cttcattaag gttgccaatg 1144080
atactgcaat tgcagtgaat caggggggca aacgtaaagg tgctatgtgc gtatatttag 1144140
aaaactggca cttggattac gaagactttt tagaattgcg gaagaataca ggagatgagc 1144200
gtcgtagaac tcacgatatc aatacagcaa gctggattcc tgatctcttc tttaagagac 1144260
tagaaaaaaa aggcatgtgg acactcttta gccccgatga tgtcccaggt ttacacgaag 1144320
cctatgggtt agagtttgaa aagctttatg aagaatatga acgtaaggtt gaatctgggg 1144380
aaatccgtct ttataaaaaa gtagaagccg aagtgctgtg gcgtaaaatg ttaagcatgc 1144440
tttacgaaac agggcatcct tggattacat ttaaagatcc ttcgaatatt cgctcaaacc 1144500
aagatcatgt tggcgtcgta cgctgttcta atctatgtac agagatttta ttgaactgtt 1144560
cggaatcaga gactgcagtt tgtaatttag gttccataaa cttggtagaa catatccgta 1144620
atgacaagtt agatgaagaa aaattaaaag aaactatctc aatagccatc cgtattttgg 1144680
ataacgttat tgacctgaac ttctacccta caccagaggc taaacaagcc aacctaactc 1144740
acagagctgt ggggttgggg gttatgggat tccaggatgt tctttacgag ttgaacatta 1144800
gctatgcctc acaagaagct gtcgaatttt ctgacgagtg ctcggagatc atcgcatact 1144860
acgctattct agcctcgagc ttactcgcga aagaacgagg tacatatgct tcttattcag 1144920
gatctaagtg ggatcgtggg tatctaccct tagatactat cgagcttctc aaagaaactc 1144980
gcggagagca taatgttctt gtagacacat caagtaaaaa agattggact ccagttcgtg 1145040
atactatcca gaaatacgga atgagaaata gccaggtcat ggcaattgct cctacagcaa 1145100
cgatctcgaa tatcataggg gtcacccaat ctatagagcc catgtataaa catctctttg 1145160
taaagtccaa cctttccgga gagtttacga tccccaacac ctacctgatt aaaaaactta 1145220
aggaattagg actttgggat gcagaaatgt tagatgatct aaaatatttt gacggatctc 1145280
tattggaaat tgaaaggatc cctaatcact tgaaaaagct tttccttacg gcatttgaaa 1145340
tcgaacccga gtggattata gagtgtacct ctagaagaca gaaatggatt gatatgggag 1145400
tttctctaaa tctgtatctt gctgagccag atggtaaaaa actctccaat atgtatctca 1145460
cggcttggaa aaaaggatta aagactacct attatttaag atctcaagct gcaacatcag 1145520
tagagaaatc atttatagat atcaataaac gcggcattca gcctcgttgg atgaaaaata 1145580
aatcagcgtc cacaagtatt gtggtcgaaa gaaaaacaac ccccgtttgt tcaatggaag 1145640
aaggttgcga atcttgtcaa taacggaaaa aagaggagct aaaatggaag cagatatttt 1145700
agatggaaag ctcaaacggg ttgaggtaag taaaaaagga ttggtgaatt gtaatcaagt 1145760
agatgtcaat cagctagtcc ctatcaagta taaatgggct tgggaacatt acctcaatgg 1145820
atgtgcaaac aactggcttc ctactgaagt tcctatggca agagatatcg agttgtggaa 1145880
atcagatgaa ctgtctgaag acgaacgcag ggtcattttg ttaaacctag gatttttcag 1145940
taccgcggaa agcctagtcg gaaataacat cgttcttgct atcttcaaac atatcacaaa 1146000
ccctgaagca agacagtatt tactgcgtca agcttttgag gaagccgtac atacacatac 1146060
atttctctat atttgcgaat ctttaggact tgatgaaggc gaagtattca atgcctataa 1146120
tgaaagagcc tcaattaggg ctaaagatga ttttcaaatg acattaacag tcgatgtcct 1146180
tgatcctaat ttttctgtac agtcttcaga aggccttggg cagttcatta aaaacttagt 1146240
aggatactat atcattatgg aaggaatctt cttctatagt ggttttgtaa tgattctctc 1146300
tttccataga caaaataaaa tgacaggaat tggagaacag taccaataca tcctcagaga 1146360
tgaaaccata catttaaatt ttggaatcga tcttatcaat ggaattaaag aagaaaaccc 1146420
cgaagtttgg actacggaac tacaagaaga aatcgtcgct cttattgaaa aagctgtaga 1146480
gcttgaaatt gagtacgcta aagattgctt acctcgagga atcttgggat taagatcttc 1146540
gatgtttata gattacgttc gtcatattgc agatcgtcgt ttagagagaa ttgggttgaa 1146600
gcctatctat cactccagaa atcctttccc ttggatgagc gaaaccatgg atctgaataa 1146660
agaaaagaat ttctttgaaa cccgggttac cgaataccaa accgctggta atttaagttg 1146720
gtaatttcac ctctttttca agggagtttt gagagatggt cttagatcgt ctctcaggat 1146780
ttttcagctt tcccttccga agaaagtcct tagaaaaaac ttcaagaagc tttatacttt 1146840
cctaaagcaa acagtctctg atttctgctt tttatgaaac cccaagatct atccccgcca 1146900
tttttatgga aagaacgccg tccttgtatt caagatggag ttctctatgt tcctagacat 1146960
tactttgaac accagaattt ttcaacaagc taccatcaag aattttttca aaatcatact 1147020
tctatagctt gtgaactatg ctctggtaat ggggattggg tagttgctca agcgcaaaaa 1147080
gatcctcaag tactttggat cgctgtggaa cagcgttttg atagggtaag gaaaatttgg 1147140
tctaaaatga tcaaccacca gatccaaaat ttaaggattg tctgtggtac tgccgaaacc 1147200
tttttccagt actatgttcc tgatcagttt ttgcaacgcc ttgttgtgaa ttttccagat 1147260
ccctggccaa agatgcggca tcgtaaacac cgtcttctcc aaccctcatt cgttcaagaa 1147320
atctcccgct ctctccaaga ttccgcagtt tttgctttgg ctaccgatga taaaacatac 1147380
ctattggaat ccatagaagc gctgcaaaca catttagctc caagaatgga aacaccatat 1147440

tatataaaaa tgacagatac ttatggaaat tcttggtttg aaaacctttg gcggacaaag 1147500
ggacaggaaa tttttatac agaatttata aaaaaggctg ggatatagct gacttgaaca 1147560
gctgaccttc acgatgtcaa cgtgacgctc taaccaactg agctaatacc ccgagttcag 1147620
ctactatcct aactagaaaa tggtatcttt gcaataggct tcttcatgtt tgcatatcgt 1147680
accctgctta cacataatgt agtccaggta tcacacgaaa tttttaaaac cacagttgtt 1147740
cccggagata cagtcattga tgcgacctgc ggaaacggta acgacagcct ttttttagcg 1147800
cgcttgcttc aaggagaggg aagacttgtt gtctacgata tccaaaaaga agcgttgtcc 1147860
aatgctttac tattgtttga aacacacttg tcagagcaag aaagaagtgt tatcgaaatg 1147920
aaagagcagt cgcacgagca tattttagag aaagatgtga agctcattca ctacaacttg 1147980
ggctatcttc cgaaagggaa taaagaaatc accacattgg caagaactac agaaataagc 1148040
ttagaatatg ctttaaacat agtccgcccc gatggactca ttactgtcgt atgctatcca 1148100
gggcatccag aaggagaaaa ggaaacacat agtgtcgaga gcctagcaca acggttacat 1148160
cctaaggaat ggtgcgtgag tcattttatg tagcgaatcg gtgtcgagcc cctagacttt 1148220
ttatttttca gagacagggg agtgaatctt cggttgataa gggataatgc gaatctcgtg 1148280
ttccagatca atgccttgcg tttttaacgt tgattggatg atagctatga gttgcttgac 1148340
ctcatcagaa gtggccttgc cagtattgat aatgaagttt gcatgcaacg gagaaatttg 1148400
tgcccctccg attgctaatc ccttcaaccc aggctgcgtc aataagcttg ccagcagaag 1148460
ttccttcagg attacggaaa atacagccag cagaaggctg cgtatagggc tgcgtcatga 1148520
gccgatgttg cagaatggat ttcgagtggt cggcagaaac ttgctttttt gaaagctgga 1148580
aggtcgctga taaaataaac tcttgttgtc tatggaagcg cgaggagcga tagctcagct 1148640
ctaattcttc tacagaatag gaacaaagct ctccttcaga gttgattgtc tcaacatttc 1148700
tcaccacgga agatatgtcg ctttcatttg tacctgcatt cataaaaatg gccccgccaa 1148760
ccgacccagg gatgcccgca gcaaactcaa gtcccgaata tccattatag gcagtcgctt 1148820
tacctaaagc tgcaaaggaa agacccgaat acgctttaat acgagcatcc tctaggaact 1148880
gtttaccata gattgcattg tagagtacaa agccatcaaa accacggtca tcaaataaac 1148940
aattagagcc tttccctatg atgaggaagg gatagttgat tgaatgtagg aaacggataa 1149000
cttcacgagc ctcctcaata gtatggatgg ctttaaagta atttgcaggt cctccaatgc 1149060
ggaaagtgga atacctattc agccacacac tacgacgaac aggaaaagga aaatgcatag 1149120
gcgcagcttc tttcattttt agaatccctt ggtctaaatt aatcagattg ataaagaatt 1149180
ttcatttaga gatgaatctg tgaaaatgtc attcaaaatc gcctgaatga aaggacaggc 1149240
ttctgaataa ctaaactttt taactagacg aattgcttct gcaataagaa tggccttgtt 1149300
aatgggtggg ctgtaaaaat gctcaaagag agtcaggcgt aggacatttt tttctacaag 1149360
atctaaacta tcaaaagact tatttttaag agcattccca ataattagat ccaattcttg 1149420
tgatttttct aaaatgcttt tagtctggtt taaagctacg agtacgtgtt tttgagacac 1149480
cgcagtttgt gacataagca aaggaactaa actgtcctct gctgaaggag ccatatctag 1149540
ggcatacaac atctgcaaga tgatttctct catcttttgc tgagggaatt ccttcgagat 1149600
cgaaataggg gaccccgaga atttttcagg actcaatgtg gccatgacaa gcaaccaaaa 1149660
aatagttaat aatacacatt taactgctaa agaggtcctt agctaaggag ttgtgagaaa 1149720
gaaacaaggt tatctattag aaagttttaa ttgcaacaaa aacttcttag ttgaaaaatg 1149780
tactttgttt tcggaactca cttattttct gattcagaga aagagttgct ggagattttt 1149840
tatataaaaa tttttaattt ctaaagacga caaggataat tatgagaaag ggatttccgt 1149900
tgcttttctt ttcctaatac cctaagatag ccttcttctt gtctagaacg gataaaagat 1149960
cggattgtct tttctggaac actcagtgct ttcagaccat agcaattgtc ttaacaggaa 1150020
attgagatag agtgtggcat taaattttaa gattaacagg caaataagag ctcctaaagt 1150080
tcgtctcatt ggttcagccg gagaacagtt aggaatactt gctatcaaag atgctttgga 1150140
tttagcccga gaggcaggtc ttgatttagt tgaagttgct tcaaatagcg agcctcctgt 1150200
atgtaagatc atggactacg gtaaataccg ttatggtctg acaaaaaagg aaaaagatag 1150260
taaaaaagct caacatcagg tgcgcataaa agaagttaag cttaagccta acatagacga 1150320
aaatgatttt tcgactaagt taaagcaagc gcgtacgttc gttgaaaaag gaaataaagt 1150380
caaaattaca tgcatgttcc gtggtagaga attagcttat ccagaacatg gttttaaagt 1150440
tgttcaaaaa atgagtcagg gtttagagga tattggtttc gttgaagctg aacccaaact 1150500
agcaggtcgt tccttgattt gtgttgtggc tccaggaaca gtaaaaacaa agaaaaaaca 1150560
ggaaaagtct catgcccaag atgaaaacca ataagtctgt ttcagcacgt tttaaattaa 1150620
cagcctcagg ccaattaaaa agaactcgtc cagggaagag acataagttg tctaaaaagt 1150680
cttcgcaaga aaaacgcaac ctatctaagc agcctcttgt agataaaggt caggtaggta 1150740
tgtataagcg aatgatgctt gtttaaaggt aaggaaagtt agttatggta agagcaacag 1150800
gttcggtagc ttctagacgt cgtcgtaaac gtatattaaa acaagctaaa ggtttctggg 1150860
gtgatagaaa agggcatatt cgtcagagtc gctctagtgt catgcgcgct atggcattca 1150920
attacatgca caggaaagac cgtaaagggg atttccgtag cctttggatt gctcgtctta 1150980
atgtcgcttc aagaattcat agcctctctt atagccgttt aatcaacggc ttgaaatgcg 1151040
cgaatatttc tttaaacaga aagatgcttt cagaaatagc tatccacaat cctgaaggtt 1151100
ttgcagaaat tgcaaaccag gctaaaaaag ctttggaagc cacagtttag ggattagaat 1151160
ttctatggaa atgaaagaag agattgaagc tgtaaagcag caatttcact ctgagttaga 1151220
tcaggtgaac tcttctcagg cacttgcaga tcttaaggtc cgctacttag gaaaaaaagg 1151280

aatttttcgg agtttctcag aaaaattaaa gcaatgtaca gataaggcaa aactaggttc 1151340
ccttatcaac gactttaaaa cttatgtaga ggatctttta caagaaaaaa gccttgtgct 1151400
tcttgcttca gaacaagccg aagccttttc taaagaaaaa atagatagct ctctccctgg 1151460
agattctcaa ccttctgggg gaagacacat tttaaaaagt atcctcgatg atgttgtcga 1151520
tatttttgtt cacctaggat tttgtgttcg agaagcccct aacatcgaaa gtgaagccaa 1151580
taattttacc ttactcaact tcactgaaga tcatcctgct agacagatgc atgatacttt 1151640
ctatctaaat gctacaacag tactgcgaac acacacatca aacgtacagg ctcgtgaact 1151700
aaagaaacaa caacctccaa tcaaagttgt tgcccccggc ctatgttttc gtaacgaaga 1151760
tatttctgca cgttctcatg tgctcttcca tcaagtggaa gctttttatg tagatcacaa 1151820
tgtaacgttt tcggacttga ctgcaatcct cagtgcattc taccattcct tcttccaaag 1151880
gaaaacagag ttacgtttta gacatagcta ctttcctttt gttgagccag gtatagaagt 1151940
agatgtctct tgtgaatgtt gtggcaaagg atgtgccctt tgcaaacata caggttggct 1152000
ggaagttgcg ggagcgggaa tgattcatcc ccaagtacta cgtaacggaa atgttgatcc 1152060
tgaaatctat tctgggtatg ctgttggcat gggaatcgaa agattggcaa tgttaaaata 1152120
tggcgtctcc gatatccgac tttttagtga aaacgattta aggtttttac aacaattctc 1152180
ttaaggaaga atggcagagc ggtttaatgc acctgtcttg aaaacaggag acctgaaagg 1152240
gtccgggggt tcgaatccct cttcttccgt ttccttttta ttcacgttta ttccgataga 1152300
ggaactttgt ggctaagagc cacgagtttc tggtattcaa cgagttcttt cttttttctt 1152360
ttgatttctt gccttttcag gtttcatagt ttttattaac ttttaatttt ctctttatta 1152420
attatttata tctaaatcta ttatttttat ttttaaacta attgatggtt aatttgtttt 1152480
tataataaat tagaaagacg atctcataaa aaataatcta acttttttgg ttccatcgag 1152540
gaggtagaat gaaaaggtct cgacgtaact tcgaacaggc tttagaaaat ttagaaaagc 1152600
tcaaagagat ttctttagct acctcgaatg attcctacct aaataatcca gcacgcttca 1152660
accagagaaa acaaacaggc tcttctgtca tggaaatgaa agaggctctg aaaaatgttg 1152720
aaaattatct actcgaaatc agctgtgttt cgaaaagtca tgcagataaa gctcttaaag 1152780
aatcagattt tttaattgca ggtgtgcaaa acgtcttttc ctttttagag aaccaagaag 1152840
acctctataa gtctttacta gacgaatatt ctgaagttac taaggcttat gatgaggtaa 1152900
aaaagaatct taaagaagtc ccaacttatg acctctcgac agatgaagaa accgaggaac 1152960
acaaagaacc cgagtgcttt ttaaataacc ttgttgaagt taagcgggac cgttcctatg 1153020
agcttttcta catgctagat gagcaagaca aacgctttta taacgatgct cttgtccaga 1153080
tcatctataa gcaaaataaa ctacacgaaa ctgtaaatga aggtgacccc ctaactaaaa 1153140
cactcctctg gaatagtgaa gaagttaaaa acattgcctc ttcgctggtt atcgtaaatg 1153200
atatgccctt gcgattgttt tatcagcgtg ctttaagcca tttagacatc gaagctgtgg 1153260
tcaaagttca taacgcagtc atggcgttgt tcttctcaag atacgaagct acaatggtct 1153320
tcaaaagccc taagaagcat aatatatggt actttaatga ctttttgctc tttttgagag 1153380
aagcatggaa agacttaaat aataacgtga ttgattctca agagagaaaa cagacgaaac 1153440
ttcttgcttc cgctttaagt ctcgggatct ttgaaagcaa actagtattt gaagaggcat 1153500
ctcgttatct ctacttcaat attcaaacga aattagaaaa cgccaacggg aagaagcctc 1153560
tttcaccagg acaatatctt acagacgctt atgaagaact tcatcgtctt atctctaaat 1153620
atcctaacgg gcctcttttt aaagctatgg atagagtttt agaacatgaa tcccgtccct 1153680
atgatcctat gattttagga atcctcccta gccttgaagg aacgttaaag ctccacggga 1153740
aatctataga tatcatacga tctcccagtc ctgtgaccca aagctcgatt ttgtatgcta 1153800
attgtaatga ggaattctta gggtttctta atgctaaagc acatcgcagt gaggtgactt 1153860
tagttctaaa tattcagaat cgtatatcaa gaaaagaacg tgcgcgaagc cgtgttatcg 1153920
aagaagcttt agaacaagaa gaacacgccc cctatgtcca cgccttctct ttccctgaac 1153980
ccgaagaact cctccagaat ttggaaagta ttcatggaga tattgaaact tttgctgatt 1154040
tcttttctat tctgcaagaa gagtttcata aaccccttct tgcttcatca ttttttctaa 1154100
cgaaagaatt aaaagagttc gtaggtagct tcttaaaaga aaaactcacg gcgttgaaag 1154160
acattttctt tgctaagaaa aaaattctat tcagaaatga taagcttttg cttctacacc 1154220
tcctctcgta cctaattgtc tttaagttaa tagaacgtac caatcccaac tctattgtcg 1154280
tcgtatctaa agatgggctg gattatgttt ctgtctttat tgcgggattt gcgttctttt 1154340
ctagggaagc gttttgggat gaacatagct tgaaactatt acttacgaat gttttatctc 1154400
ctacactcgt agctagagat cgtctggtct tcgtatccca tattgaactc ttaagtaaat 1154460
ttgtaaactg tctaaagaaa aatcgtcagg gattttctag tctgaagtct ttcttcaaag 1154520
atgacattga aggttgggag ttcaccgggt acttgcatga actcactgaa gtttcgcata 1154580
agcataattt gtaagtagaa agagtactat aagaggaaaa gccatcacag gaagtgtggg 1154640
aagaacgctg ctactggcta agacgattcc cgcctttagg aagacaaaga aaatatttac 1154700
cgtccctaaa gggatcaaat aggctagagt gaccgttggt gttctactaa atctcaaaca 1154760
gagataggca gataaaatga tagccgccat acaggcaagt ggtgaaatta agacgtaata 1154820
gaattgagcc agaagagaaa gaatcctttg tggcacttgt gtcgagagtc ctaaacctgt 1154880
agcgttccaa gggatggcct tgaaaaattc tgaaagacgg tttttattcc ctgcagaaaa 1154940
tagcttggaa aaaggatttt catagaaatt aaactcgatc tcaggaaact ccttcatatc 1155000
gaaaaactcc ttaagctcaa gattttctga atcattagcg aaaaattgtg tgacatttaa 1155060
accaatcgga agagagaggg tggtgaacgc taacttttcc atggtgtaaa tggttttagg 1155120

atctttgatc cagaagacat tatttaaagt aagcgtctta ggctcaatag aagaatataa 1155180
gagtactgtt tgatctttga ggtaaagagc aggaattttt ccctgctctt tatctgtcgt 1155240
gcctctatcc atattttctt tagtaataga gattttctcg caaataggat gcaaccactg 1155300
gaaattcgca tagagcacca tcatgatcac agctcccgaa aggagaagag gatgcattag 1155360
agattttaag gagagaccag aagcttgtag aagaataatt tctcgcttat tttgcatagc 1155420
aaaaagcgtg cttgttgttg ctactgcaac aagctgaggc atgaggaact ctgcttttaa 1155480
cgaaatctgt gctaggtagt acaatataga gagcttgaca gaagctccag aagcagctgt 1155540
agaggcccct ttaagggtat gcaaagagtg atgaatggaa gcatagaata taagagcaag 1155600
taccaaaaga gaggtaagtg caaaccaaaa tctagtgagt aagtggcgtt tccaaatcaa 1155660
catacgttaa gcataccctc gactttccct atacgcgcga gcagcaaaga caacccaaga 1155720
gaccaattgg gggaagacaa atagcatgaa ggctaggggt agattttttg tatttttccc 1155780
aacgataagc agaataaggt ccaaaatagg gaaaatgaaa tataaagcta tagattttcg 1155840
aaatctaggt ttatgaattc caaggatcat gccagcatac gtcagcgtga tacagagaaa 1155900
accaatagca actcttcgga gcgtttcagg aagatgcgag tgctttaggg actgtttaac 1155960
aagctgcttc caaggcagat agtctgtccg tgtctttaaa taagattttc ctgcaaacaa 1156020
tgttgaagta atcttgggaa ttagcaattc atctaaggtt tcaatataga atctttgaga 1156080
agatggtgag gaagattctg ttaacgagtc tggaagcttt gagataaaga ccacatcctt 1156140
agcttttaca gtatctttag tagtgtcagg gatgatactt tttataattc ccacatggga 1156200
gatctcatta tttcctttta gagctacaat gacattgtca aacttgcttt tagcacaatg 1156260
atctacagcg atgaaaatac gattgttttc ttttttctgt agagtctgta agagcagggc 1156320
tggtgatgtc attgccatgt ttgcaatttc cttgcatgtt tgatagcgac aaattgaagc 1156380
aagctctgag catgtgtaaa agtttaaaca gcaaatggct ccagaaacca tcaaaacagg 1156440
aaacatgatg atgctttgag aagctccaga cgctcttaga aacgtcatgt ggttgttatc 1156500
tgataattta cggaaaagag agaatgctga gacaaaacaa gagccaggga gaataaaggg 1156560
aagaaggtag gggatttggt aagccattaa acgtaagact gtatcgtaag gaacgtcttt 1156620
agcaatgtag gcaacaattt cctgtagaga gctaattata gaaatacaaa ttaagcttaa 1156680
ggtacagaaa gctgctgttt ttaagtaacg gaaaatgaga actttccata aaataggcat 1156740
ggcttacctg gtaattacaa tttcttagat gccttgatag agtgaaagag tcacgatctg 1156800
ttttgaaaaa tccctagcaa cactattctt ttcaatgatt tttatatagg cgtataaaat 1156860
ggtactaagc tcagatttac tccgtgatga taagcaatta gatctttttt ttgcttcttt 1156920
agatgtaaaa aaaagatatt tgcttgctct ttctggagga agcgactctc tttttctttt 1156980
ttatctactt aaagagcgag gggtttcctt tactgcggta catatagatc atggttggag 1157040
gtctacctct gctcaagaag ctaaagagtt agaggaactt tgcgctcgtg aaggtgttcc 1157100
cttcgttcta tatactttga ctgctgaaga gcaaggagat aaagatttag agaatcaggc 1157160
aagaaagaaa cggtatgctt ttctttatga atcctatcga caattagatg cgggaggaat 1157220
tttcctcgct catcatgcca atgatcaagc agaaacggtc ttaaaacgtt tgttagaaag 1157280
cgcccatttg actaatctca aagctatggc ggaaagatca tacgttgaag atgtgttact 1157340
tttacgacct ttgttacata ttcctaaaag ctcgttgaaa gaagctctag acgctcgcgg 1157400
tatttcttat ttgcaagatc cttcaaatga agatgaaagg tatttaagag cgaggatgcg 1157460
gaaaaaactt ttcccttggc ttgaagaggt ctttggaaaa aatattacct ttcctttact 1157520
aactttaggt gaagaatcag cagaactatc cgagtatctt gagaagcagg cacaaccatt 1157580
tttttctgcc gctacccatc aagattctca aggagagctt ccctgtccag actgcctcat 1157640
tcaacaagcg tttttatgca aatgggttat gaagaaattt tttaataatg ctggaattgc 1157700
tgtttcaagg cattttttgc aaatggttta tgatcattta tctcggagtt cttgcgcaac 1157760
tttgcgcatg aggaataaga tagtaatcat aaaacctgga gtagtagtga tagattagaa 1157820
agtatatact cattatcttc tctatatgtg aaaaaaagag agaacactag gtcattgtat 1157880
tagtgcctgt tcatattttt tatttggctg ttatagttta aaatattttt tctaaaacaa 1157940
aataataaaa aatatgttat attgaataat tatccgtctg attgattagc ttagttgtga 1158000
agtttatgtc gaaagataag aaaatgaaac ctgaaccgaa aaaaaatttc cctacagtct 1158060
ttttttttct tttgtttggc gtggtttttg gtgtagtcgc atttcaaaat tttctagctg 1158120
gaaagaaggc tagagtcggt ttcagtcatc aaatcgagca ccttgttaac ttacgtctga 1158180
ttgttccaga agatagccat aagatagctc ttaatgacaa cttagtttct tttggcggac 1158240
gttttcgtga tgtccaaact caagaaggac aactacgtta tcattatcta gagcttattg 1158300
atcagggcca tcggttagac cttgatcttc aagagacgag caagagtctt acgaccttag 1158360
ggaaagaggt cacgaattcc atactttggt tttctgcaat ttctggatcc ccgattcctg 1158420
aacaagggta tgccatttct tatccgagtg aagtcagcgg ctctgtattg acagaacctc 1158480
tcgttgttac aggacctgca actccacagc taatcaatct ccactctctg caggagcgtt 1158540
atcctacact atcacgatct ccagaagctt tacgcaccta tggatccgat ctttatgagt 1158600
taataggaaa atatctatct cctgttttag gaataggatc tgagacgcta aaaagagaac 1158660
ttaaagatct ctaccagcaa gtggaagtct ccttaactca ggagacggat acggaagcag 1158720
cctacaccct ttatgggcag gttcttagta ctttgaacag gatctcttct tctttagtcg 1158780
tgtctgaggg aggagaacgt ttttctcaac tccgttctgt acgtctctat cgggaggagt 1158840
ggaataagta tcataaattg gtggaagcgc gcgatctgaa tcaagcgcaa ctagagaagc 1158900
tccgtggtga attaagccag acagtttggt attttaataa ccaagagctc tcctcccgta 1158960

gtttagagaa acaagatcct gaagtttttg gccattggtt tgccggggct aaggaagagt 1159020
ggactgcctt taaatttaac cactccctat catttaaagc tccagaccaa ccaagaaatc 1159080
tcgttttaga gaaaacattt aagagccaag agccttcgcc acatnattta ggatatcttt 1159140
tcacantctt acctatcatc ttagttctac tctttgtcta cttggtgttc tctcgtcaga 1159200
tgagaggcat gagtggttct gcaatgtctt tcgggaaatc cccagcgcgg atgctcctta 1159260
agggacagaa taaggttacc tttgctgatg ttgctggtat cgaggaagct aaagaagagc 1159320
tcatcgagat tgttgacttc ttgaaaaatc ctaataagtt taccagttta ggggggcgta 1159380
ttcctaaagg agtcctcttg ataggacctc caggaacagg aaagactttg atagctaagg 1159440
ctgtatctgg agaggcggac cgtccctttt tctccattgc tggttctgat tttgttgaga 1159500
tgttcgttgg agtcggtgct agccgtatcc gtgatatgtt tgagcaagca aaacgtaatg 1159560
ctccctgcat tatctttatt gatgaaattg atgctgtagg acgtcatcgt ggtgccggta 1159620
ttggtggtgg tcatgatgaa agagaacaga cattaaacca gcttcttgta gaaatggatg 1159680
ggtttggcac caatgaaggt gtcattctta tggctgcaac aaaccgtcct gatgtcctag 1159740
ataaggctct attgcgccca ggacgtttcg atcgacgtgt tgtaatgaac cttcctgata 1159800
ttaagggacg ttttgagatt cttatggtac atgccaagag aatcaagtta gatcctactg 1159860
tagatcttat ggccgtggct agaagtaccc caggagcttc tggagcagat ttagagaatt 1159920
tattaaatga ggctgcctta ttagcagcac gtaaggatcg aactgcagtg actgctgttg 1159980
atgtcgctga agctcgtgat aaagttctct atggtaaaga gcgtagaagc ttagagatgg 1160040
atgccgaaga gagaaaaact acagcgtatc atgagtctgg tcatgccgtt gtagggcttt 1160100
gtgtacagca tggagatcct gtagataaag ttacaatcat tccaagaggg ctctctttag 1160160
gagctactca tttcctacca gagaaaaaca agctcagcta ttggaaaaaa gaactttacg 1160220
accagctcgc agtgttgatg ggaggtcgtg ctgctgagga gattttcctc ggagacattt 1160280
ctagcggtgc tcagcaagat atatctcagg ctacaaagtt agtgcgtagt atggtttgtg 1160340
aatggggaat gagtcctcag ctaggaaatg tgacttatga tgagcgttcc gatggcttga 1160400
caggctacgg tggttatcat gaaaagagct attcagaaga aacagcaaaa actatcgata 1160460
cagaattgag aatgcttcta gatgcggcat accaacgtgc gttagatatt attaatgagc 1160520
ataaggcaga aatagagctt atgactcaga tgctcattga attcgaaact cttgattcta 1160580
aagatgtgaa agaaattatg gaccatactt gggatcctga gaagaagaga gcacgtctaa 1160640
aagaagaagg gatgctattt aaaaagtctt ccgacgatct tcctcctcct cctcccaagg 1160700
aagacactct acctggtttg gggtttaatg ctacgtaggt aggaatacct caagatgcat 1160760
tctgatccga aaagaaccta ggaaatcctc ctaggttctt tttttgtctc aattgaatca 1160820
gattagagtt agatgtaatc ataaaaaaag ctagtctttc taagaagact agcttttcat 1160880
gttaagcgta tagaatagaa tgtatgtgaa agattattct agagttgctt tatggctcaa 1160940
cttaagttga cctttttcat tgatgcttaa gagttttaca tcaatgatat cgccttcctt 1161000
aacaacatca cttatatttt ctattcgctg tctagaacac tcagagatgt ggcagagtcc 1161060
ttcttttccg ggagaacttt caacaaaggc tccaaacgca actacggaag ttacgcgacc 1161120
tctgtaggtt tttccaactt caacttctcc aactaaacct tcaatgattt ctttagcttt 1161180
attgatagct gatgctgagg acgcagagat gctaacaact cctaagtcgt taacatcaat 1161240
ttggacgcca gtctcttcaa tgatctgacg gatttgcttg cctcccggcc caattactga 1161300
agcaattttt gttggtttaa tttgcattgt ttcaatacga ggcgcgtatt gagataggtc 1161360
tgctttggga gctgaaaggg cttcgttcat aatatttaaa atatcattac acccttgctt 1161420
cgcttgggaa agagctttct tcatgatagc tggagtgatt ccttcaactt tgatatccat 1161480
ttggaaggct gtaattcctt taccactccc tgctatttta aagtccatgt ctccaaggtg 1161540
atcttcaaga ccggaaatat cagaaaggat aatcgctcct tgatcatcaa ggatcaatcc 1161600
catagcaatt cctgcaatag gagaagaaat agggacgcca gcgtccataa gagcgaggca 1161660
accgccacat acagaagcca tggatgaaga accattagat tctgtaatat tagattctat 1161720
acggatggta tagggaaatg tagcgctatc aggaagagca tgacttaaag ccttctctgc 1161780
caatttgcca tggccaattt ctctccttcc tggagagccg attcttccta cctctccaac 1161840
agagaaggga gggaagaagt attgtaagta aaattttgaa agtccttcac cattcaaatc 1161900
ctcatagcgt tgagccattg cctcactgcc taaagtacat accgcaagtg tctgagtctc 1161960
tccacgagtg aagaggcagc ttccgtgagt acgaggaagg taagaagttt caatagtaat 1162020
aggacggact gtagttaaag aacgtccatc tgcacggatc tctctatctc ttatgagagc 1162080
acgcatcgtg tcagatttta aagttttaca tgccgctttg atgttaaaag aagagaaaag 1162140
atcatcatct tcgcgctgta atttttctaa aatattttct tcaatctcat gcgcagtagc 1162200
tgcatgtact tttttatctt taatattaaa gagttcagta aacttgtctt gcgcacactc 1162260
tttcacagct gttaaaactt ctgcaggaag aggatataca gcacttagat ttttagattt 1162320
tccaacttct tcttgccaca actgtaatct tttacagatg gtaacaatat gcttgtgacc 1162380
aaattctatg gcatcaagta cttgctcttc ggtaaagaaa tcgcaatgac cttctatcat 1162440
tagaatagcg ttttctgttc cagctaatac aagatctaaa gtagaggatg ctaactcagt 1162500
ttttgtagga ttgatgaccc attggttatc aatacatcca atccgtactc cagcaacaat 1162560
gttgctctgt ggaatatctg aaattgctaa agcagcagat ntgcgcagat agcaagagga 1162620
tcaggtaaaa cttgaccgtc atacgaccat acataagata aaacttgcac atcttgcatg 1162680
agtcggtagg ggaaggaagg acgtagagac cgatcaatca atcgagaaac taaaatttct 1162740
ttttcagagg ggcgtccttc tctttttata aatccaccaa gagttttccc tgtggaagag 1162800

aatttttctt ggtagtccac tcgtaaagga agaaagtcta ctttgtcatc aagatctaca 1162860
gcgcatgcac ttgcaaaaac acaggtttcc cctgaacgga caagaacagc tccgttagct 1162920
tgacgagcaa ttttccctgt ttcaaacacc aatattttac ttcggtcaga ttaatagaaa 1162980
tagtttgaaa attcatgaag gtctcctgtt agatatttgt aaagggaacg aaaaaactac 1163040
ttacgtagat tcaatcttgt aattaaattt ttgtaccttt cggtatctgt agaattaaga 1163100
tactccaaga gctttctcct ttgaccaaca agcttaagca atgctaagcg ggagttttgg 1163160
tctttagggg atcttttaag atgttctttc aattccttgc ganatgttcc gtgagaatag 1163220
caatttgtac atctgccgat cctgtatcct tttcatgaag ttgaaacttc tttgtgattt 1163280
cttccttagt tcctttatcc aaagacatcg gatgtctcct taaaataatt gccgcaaaag 1163340
ctgattatac ttgagaacat ggttaatgta caccttttgg tagggtaaga cttttttttc 1163400
tataaacaaa gaaaaaatta ggggctgtgg aaaggtctga gattgcttca taaaagcgtt 1163460
ttattgatat tgtatattgt tatttttttt gtttttagaa ctcataatgg aataaataat 1163520
attatttaga attgggtgga gagaagctta tcaatatgga aaaagatatt ttttttatgc 1163580
aacaagcctt caaagaagcg cgtaaggcat atgatcagga tgaagttcct gtgggctgtg 1163640
tgattgtcaa agacgataag atcattgctc gtgcgcataa ttcagtggag aagcttaagg 1163700
atgctacagc ccatgctgaa attttatgta tagggtctgc ggcccaagat ttagataatt 1163760
ggcgtttgtt ggataccgtc ctttactgta cgttagagcc gtgtttgatg tgtgcgggtg 1163820
cgattcaatt ggctagaatt cctagaattg tctgggcagc tccagacgtg cgtttaggag 1163880
caggaggaag ttgggtcaat atctttactg aagagcatcc tttccatacg gtttcttgta 1163940
ctgggggcgt gtgtagcgag gaggcagagc atctaatgaa aaaatttttt gtagaaaagc 1164000
gcagagagaa aagtgaaaaa taaaattgtt acattattag atcagctcta cgaggatcag 1164060
gagtcacgac ttcagaagtt aggagaggaa attgttccta acctcactcc tgaagattta 1164120
ttgcaaccta tggattttnc tcaattggaa gggaatccgg catttcgttt tgaagagggt 1164180
gtcttatcag gaattggtga ggtgcgagct gcgattttta acggcgctct ctcaagagaa 1164240
ctagaatctc aaagaagtag tataggggtt ggggatcttt tcttttttac aaaataggat 1164300
agccagaacg atcgcaagca tcgatgcgag gagtagaagg aaggcccctg agaaaataag 1164360
aatgcaacag tagaacccta agacgaacaa gaatccccac tttattgaga cggatagggg 1164420
agtgggtcct atggtaaagc gcttttctgg atctaagaag atctgtactg tcaaaacacc 1164480
acagataata ctttccggac cgaagaaaga ttgggagctg tgatgagagc catgaatccc 1164540
cagagaacag ctcctataat gagtgcctgg cctgagatta caacgagaac actgaaggct 1164600
cccaatttgc ggatgaggtg ttggatagct ttatagaaaa gaaaaaaatc caagacattg 1164660
cgcaaaagaa gtctttgggt gatttcgaag ctttgatctt tatttaggga tagggaatct 1164720
gctgtgatta gggggtaggt gagaaactgc caaaaatggt gtttctgtat ccctttaaca 1164780
gagagggcta gaatttcgag gagcccagga attccaaaaa atttatttat gatataagag 1164840
aaaaacgggg cgatacacga agtaactaat ataactgaag gaagtttttt taaaagcttg 1164900
gataggttgg ggaagttatt atgtttatcg gggaaaatca ccctcatgga ggctttacct 1164960
gcaatctgac ggtcaggccc ccatagtaga ggatgaattg ttttattaga agtctattgt 1165020
catagtttag tcagagggtg agttcgttag atttgccttg attgttagtt cctttagggt 1165080
agagagcatg gagctggtgg cttgtagaat ttgcatggat tctatggttg ttgagtttac 1165140
agacttttgg tttgcctgtg ctctttgttg tgccgaggag agctcctgtt gtatcatttg 1165200
acgatttgct gagatctgtt ggtttgagct ttggtaggct tggatttcat tttggttctt 1165260
atattcgtca tcgcttccat cttttttctg tagatcgggt actttaagta aggggaggtt 1165320
aatgagctct ttcactcttt gctgttggac tattgtatta tcttctagta cggctaagat 1165380
tgatttgccg tactctgttg tggattcaga gagccgaagc atgacggaga tgcaaaagta 1165440
gaatgcagac tgtttgttaa ttgtgatgga gtcagaaatt acttttgtag aatcaggaat 1165500
cactttatcg gctttagttg ttgtattaga gacgggttgt atcgacatga gttcctctat 1165560
acttagatgt tagcaattag gcttacaatt tgagagaagg tctggataag agcctgtcct 1165620
acctgtaagg attgttggat gatgttatta tttgtgttca agttactgga gattacctga 1165680
gccgcgtttc ctaaactgga aatttggttt tggatagctt gccgagaagc tcctatcgct 1165740
tggttatcgg attggatgtt ttgtagatag ctagaggagt tatcgttcag tttattttta 1165800
ggaatactga cgtattggta taaggcttct tggttattta ggtaggtttg agctgaagag 1165860
tttgcttgga gttcttgggc aatgatggag aggttatctt gagcaactaa tacggattga 1165920
tagacgtagt aaacagtaac aatcagaggg tttgagtctt tggagaattt tgcaatgata 1165980
tcgtcagcag ttgctgtatt aggtcctcct gtaattggag tgccaggaac atttatggct 1166040
gcttgtagag gttnttttgc tgatagaggg tctatgatcc acatattcaa cctaactggt 1166100
tttcgatttt aaaatgttta cggatggttt taattcctta tcctaggggt ttattcagtt 1166160
ggtttacggt gcttcctatg gagtttgttg ttttgataaa tgaggagtct tgtgaggatt 1166220
gctgttggat gatgttaatg tttgtagagg cgtgtgagag gataatttga ccgttttgtc 1166280
ttgttgttac gagttcgtct tggatattag atctttgtgc tgagtagttt tggttctgat 1166340
tttgtactct agtgatctcg tcttcttttg ctccagcact tactattgca tacttgattt 1166400
gattagtttc ttggtttaat tgttgttgaa tattagtatt gtcgtttaat tgttgtgatt 1166460
gagtcagaac tgtttgttgt cgaatttcaa ttgcttgtaa aagaagctcg taaatgctaa 1166520
aaagcacagc acctatttta ggtgtattta gaggctcaag gggaggacga gaactgttta 1166580
tagtattaac tgtgggaaga gtccctaagg tggttataga cataataaaa aaattcactt 1166640

ttgtttagtt ttattctaac aagaagaaaa acgtttatta aaataaaatt tttatgattt 1166700
ttctttatta gttgttatta agtgttttgt ttttttataa ttaataagaa attttggcgg 1166760
cccagagaat attgggtact agattggagt aagaagagct taatatgggt cctaatggag 1166820
gcatttgtag actccctagt tgtggtataa atctttagct ggagaggaga aggacgtgtc 1166880
tgggaatgag tgcaatgact atccagaagt ttttaaagat gacgtaagtg cttacgtatt 1166940
ggtaacttgt ggtcagatgt cttctgaagg caaaatccag gtggagatga cttatgaagg 1167000
agatccagct gtgatcagct atttattaac aaaagcacga gactctttag atgagtctta 1167060
aactattcat ttattgggat gttgaaaccg atgtacgtgc ttagcaaacg tctttatcga 1167120
tgggtaaatc aactaattaa attaggggat ttggtaaaga actcacgatc attttctgtg 1167180
gaatgggttt ttatcagcgc tctgttgttg atttttggat gtttaggctg cgcatctgtt 1167240
gttaaagttt ctttagttcc ctttctatta ctattttcgt ttttagcgtt tcctctgatt 1167300
ttatgttttc gtgggaaggg ctatgctttg cttctggggg tttttgtaac tctttatgtc 1167360
gctaagtatg ttgttggaga gaccttatat gtctcttttt ggttatctgg cttaggagtc 1167420
tcctttttgc ttgcttttgg gcttttcctc caaggagtgt ggcttgctca agaagaagag 1167480
atggtaaagg ggaaggagca actcaggctt tctgaggatt tggatgcaca acgtagtgct 1167540
tatgaagatt tgctgctgac aaagagtcag gagaaggagt ttttagatgc tcgtgctcag 1167600
ggtttagatc gggaacttac tgagtgtcag gagcttttga aagcggctta tcagaaacaa 1167660
gagtatttaa ctatagattt gaaaattcta gcggaccaaa agaacagttg gttagaggat 1167720
tacgcggaac tccataataa gtatatcgag ttggtttcca agaatgggga tgttgtattt 1167780
ccttgggtag cagagccttc agtaggtgaa agtcagggct cagagagggt ggatgtttct 1167840
agatgggtat cggctttaca ggagaaggaa gagagtttag agcgtctacg aaatgagatt 1167900
ttagtagaga agcagcgttg ttctgattat gaacaccgtt gtcaagagtt gggccttttg 1167960
ttgcaaaatt tcactgctct tgaaaggaga tgtgaggagc ttcaaaacct tttaaatcag 1168020
aaagaaacgc agataaatga gctccaccaa ttggtctgta aatctgaaga gaaggtctct 1168080
gtggaacctt ctgcacatgc tgaaacgagt tgtgtggaag aaaaacagta taaagggttg 1168140
tattctcagc ttcaggagca gtttctagaa aagagtgaga ctctttcttt agtaagaaag 1168200
aagctttttg cggttcagga gaagtatttg actttgaaga agaaggaaga actcacaaag 1168260
caagacatat cttttgatga tatctctatg attcaagggc tcttagagcg catagagatt 1168320
ttggaagaag aagtcagtca tttagaagag ttagtatctc gtagcctgtc tctgtaattg 1168380
ctatagtgtg ttcccattgt gcgctgggtt gattatcgca tgtgcgtgct tcccattgat 1168440
ttttggggtc tacaacgcct tctttttttc ctacattaat cataggctct atagtgaaga 1168500
tcatccctgg tgctagagga atcatggagc ggtttctata atgtggcacg taggggtttt 1168560
cgtgaaactc aattccaacg ccatgtccta caaattggtc tactacagaa aatccatagg 1168620
tgtctgcacg tgcttctata gcttctccaa tttcacagag gggaatacca ggttttaaaa 1168680
tggctataga atcgttaaga cattccaaag ctgcttggca tatcttcttt ttaatttctg 1168740
gcacctctcc gatcatgacc atgcggctac agtctccata gtagccatcc acaatgcagg 1168800
acacatctat attcatgata tcaccgtctt ttagtgggat atcgttagga atcccgtgac 1168860
agatcacttc gtttaatgat gtgcagattg tttttggaaa tggtggtgag ccgtaatgga 1168920
atggagcggc aatcgcgtca tatttcttat ggagctcttg ggagagctca tccaactcat 1168980
ttgtagtgac gcccttttga gatgctttgc atagctcatc tagaattctt gctgtaattt 1169040
ggcaggcgtt atagattttt gctttctgtt ctggggtttt gagtaggata ttatattgcg 1169100
aggcgtagtg ttgttttaag gcttctggag acatttttgg gggttgaggg tagtggcact 1169160
gtttccattt acgtccactg ccgcaccagc aagggtcgtt tcttttcata aggatcaccc 1169220
gatgtaaaag cctatattaa acgctattga gatccctttc aacataaggt ttaccgacat 1169280
aagtagtaaa gcaataccga agagtctctc gagtgcgagg agtccgaaat ttccagaaag 1169340
gcggtcaaag aagctagagc atagcagagt aaataaagaa aatgcccagg cgataatcat 1169400
agcagtaaaa ataatttccc tagagtagat tccctcttcc atgtagctaa ggagtgctgt 1169460
aatcacagca gggcctgtga ttactgggaa agctaagggg aaaaatatag gttctgtttt 1169520
ggaagtatca tctttagctt tttctggcat gggtgccagc atcattttta tagagactgt 1169580
aaagaggaga aagccgccta tgatctgaaa agcgtagaga gaaatatcca agaattggaa 1169640
gaagcttctt ccaaaagtaa cgaagaggat gagggctccg agagcaaaaa ggcattctct 1169700
taggatcacc cgttgttgtt tctttctaga aaagtttttg agcaaagcaa caaagacagg 1169760
aatggagcct ggagaatcga acaggacata aaatagtagg ctaagattga gtaagatgag 1169820
cataacttat tctataaaaa tgcttgttgt aatcctgaga ctaggagttg ggtacccaca 1169880
attgttacaa aaagtcctaa aacggtttgt gtagctagga gtactttgtt ccctttgcag 1169940
aagatctgaa gagaaaaggt ggctagggta atgagtagcc agctgagaat taggatttgg 1170000
aaattgatca gagtattgtg atttctcgct atgagtgtac agcaagctgc taaccagagg 1170060
gtccaaacat taaagggaga gctattggag aaatgcacgg agaatatgag ggggacatat 1170120
tgaatttata agggatccag gattcttctt ttcccaatcg caaaacagct cggactcctg 1170180
ctagtgttac cgcaattcct ccgacaactt ctatagcgca tacgggggta ttcaatactt 1170240
tcaggcctcc gagagcgagt ccgtaaagag caaacatagc gatgaatgcg aagaagcttt 1170300
ctctcagtag aacaagcatt cgctgtttta ctgaataatt cgcgaggaga tggtgtagcg 1170360
ctaggatatt agttaaagag tctgaggcta gcagcagaat gcatgtttgg ggcaagaaaa 1170420
aagaaaactg tatcatgggg taatttttta acctcatttc ctattatttt caagggaatc 1170480

ctctagagga tacttcggaa ttgggagagt ttatcgaggt ttgtataaat tttatttata 1170540
ggtaatattt tttagaacag ggtgtgagtt acacactctg ttcttctggt agtgacggat 1170600
tgtagaggtc gtttagagaa aacttcgcga cttctgaagc aagttcggga taccgtttaa 1170660
tgattgtttc gaggagtttg tctacaaggt ctttgcttaa gggatcttgg gactgagcta 1170720
ggagggggaa agtttcctcc cattggagat actcctgata gagattttta ggtatgcttt 1170780
tagtttcttt agcgtagagt ttaaaagcat cttcggggtg gtcttttgag aatatgatgc 1170840
tttcttgaag agctttttgg aaagcttcaa caatttcggg ttcgctagct ttcgttccct 1170900
ttttagtaaa gacaatcaac tgggggcctg tgggaagatc acaggtatcg gagagaaagc 1170960
atttcacggg catgcctaaa gtttgtaatt ttactccttc aatattgtag aaagcgccgt 1171020
agaggaaatc aattttgttt aataacatcg gagagatgag atcagaactt acatttttga 1171080
cttcagaggg aaccacaccg ttacgattta aggtttcaag tagacgattt agatctctgg 1171140
agttgtttaa gcagaaacct aagactttgc catttaggtc ttcaaatttg tagatggggt 1171200
cctgacttct gtagagaaat cttgcaggga actgtctatc aatctcccta cgatttgtat 1171260
tggcatgcct tttatggagg tcttcatgat gccgagagca tggtaaaggg ccatatctac 1171320
ttgttcaaat agaacgtggg gaacagcaga acttgagtct gtattttttt gaagttgtag 1171380
atccaaaccg tgttgtttga agtatccttt ggctactcct gcatagaggg gaatatggtt 1171440
aggattgggg gtccaatcga gtaaaagtgt tagtgaagta agattttttt tgtcaggagg 1171500
agatttagag tttcctttcc agggaataag cattatagga attaaggcga gtacggataa 1171560
agctttttta gctacggatt tgtgcttaag gctcatccgt tttactcgga atagtgaaaa 1171620
aatcaatttt tcaattaaca aggtgatttg gaagaggctg agggtgagaa ttgagagagt 1171680
cgcgagtcct gcgaatgcta attccatttc ataatttctg cggctttcga gcataaggat 1171740
gccgagaccc gattgggagg ctacccattc tcctgcaatg gcagcaaatc ctgcagatcc 1171800
tatagcaatt tttaatcccg agaatatgtg ggggagagcg tggggaatcc ggagtttgat 1171860
caggagttgg aattttgtac tccgcaaagg acgaattgct ctataagttc ttctggtgtt 1171920
gataagatgc cctgatagat agtcagggtg agcggaaaga atatggtgag tgctgtaggg 1171980
acgattacag caccgattcc ccagccaaac caaagtacaa tcagtggagc cagagcaaac 1172040
ataggagtgc actgtagcag gatgaaaaga ggttgtagga gatctttagc tgatttataa 1172100
gacaacataa ttgttgctag aacaatggag agagtaatcg caaggaagaa ccctcctaga 1172160
atcgctttta aggtgtgcca agcagatgtt aacaagagtg gaagggattg gagagtacta 1172220
gaggcaatgc tcgagggggg agggcagaaa aaggaaaacg tgggtcggtg gcgtgacgtc 1172280
atctcccaaa gaaatagcag ggaaaaaacg ataacaaagt agaaaataaa tttttttttc 1172340
atttttgaag taattaacga gaaaatagcc taaatcatag gggaaatata gatatgcgac 1172400
aagaaaagga tagtttagga atcgtagaag ttcctgagga taagttatat ggagctcaaa 1172460
ctatgcgttc taggaatttt ttttcttggg gacctgagtt gatgccttat gaggtaatac 1172520
gagctctcgt atggattaaa aaatgtgctg ctcaggcgaa tcaagattta ggatttttgg 1172580
attccaagca ttgcgatatg attgttgctg ctgccgatga gattttagag ggaggttttg 1172640
aagagcattt ccctttaaaa gtttggcaga cagggagcgg cacacaatct aatatgaatg 1172700
tgaatgaggt gattgcgaat cttgccattc gtcatcacgg aggggtgtta ggcagtaagg 1172760
atcctatcca tcctaatgat catgtgaata agtcccaatc gtccaatgat gttttcccta 1172820
cagcaatgca tatcgctgct gtgattagtt taaaaaataa gttaattcca gctttagatc 1172880
atatgattcg ggtgttagat gctaaagtgg aagaatttcg tcatgatgta aagataggac 1172940
ggacccatct tatggatgcg gtgcctatga cgttgggtca ggaatttctt ggttatagca 1173000
gtcaattgcg tcactgctta gagagtatag catttctttt agctcattta tatgaacttg 1173060
cgattggagc tactgctgta gggactgggt tgaatgttcc tgaagggttc gtggaaaaga 1173120
tcatccatta tttaaggaag ganacagatg aaccgtttat tccagctncc aattattttt 1173180
cagcactgtc ttgtcacgat gctttagtag atgcccatgg gtctttagca actttagcat 1173240
gtgctttaac taagatagct acggatttga gctttttagg ttcaggaccc aggtgtgggt 1173300
tgggtgagtt atttttccct gaaaatgaac caggatcttc tatcatgcct ggtaaagtca 1173360
atcctacgca gtgtgaagct ctccaaatgg tttgtgctca agttcttggg aataatcaaa 1173420
cagtgattat tggaggaagt cgaggaaatt ttgagcttaa tgtgatgaag cctgtgatca 1173480
tctataattt cctgcagtct gtggatctcc tttctgaggg gatgagggct ttctctgaat 1173540
tctttgtgaa aggattaaaa gtaaataaag ctcgtttaca agataatatc aataattctt 1173600
tgatgttggt tacagcttta gctcctgtat taggttacga caagtgttcg aaagcagcac 1173660
tgaaagcatt tcatgaatct atatctttga aggaggcgtg tctagctttg ggatatcttt 1173720
ctgagaagga atttgatcgt ttagtggttc ctgagaatat ggtgggaaac cattagaatt 1173780
ttttaaagag agcgaacgct gagtgtattg taaagtaccg tagcgtacgt tttctcttcc 1173840
cttgttatga aaatctttaa ggttgctttt ctaggattta gactaaatga cgagttgaag 1173900
ttttgctctc tagatttgtt agagcttggg caaataggag agcactttta atatttgaga 1173960
agatatggtc caccccaatc agttcatcta gatgataacg tttcaggtca gcaaggggag 1174020
ttttcttaac tccagcgagg agaagtaggg tcccttgacg atcacattcc aagaaaaatt 1174080
cttcgagagc atgcatagct gaggcatcta ttgtggggac tcgcgtcatg catagaataa 1174140
agattttagg gggtttttct atatcattaa gaagattctt taatctatct gcaattccga 1174200
agaagaaagg accattaatt tcgtagatct ctgtgttttg aggaacttca gctttgctta 1174260
aaaagtctga atccttatcg aagtatttgg ctgtggagat cacatcggaa agatcactca 1174320

tttgtttcat aaataagaat gccgctagca tcattcccac ttgtacagct gctgtaatcg 1174380
ttgtcattac cgtgaggatg aagacagtga ggagaaccac aatatctttt ttaggagcgg 1174440
taaagagatg gataaagtgg tggatttcgc tcatattcca tgcaatcaaa atcaaaacgg 1174500
cagcgaggca ggtgagagga attttgacag tgagtggggc cagaagaagt aaaataaagc 1174560
aaataaagat ggagtgaacg attcctgcta tgggagttgt tgcttcagac ttgatgctgg 1174620
ctgctgtgcg agatagcgac cctgtgacgg ggattcctga aaataatgag gttccgatgt 1174680
ttgctacccc ttgggcgaca agctgacagt tggattggtg acgccatcct gtcattccgt 1174740
ccgcgaccac ggcagatagt aaagtctcca atcccgataa gactgcaatg gtcagagcgt 1174800
ctggcatcag ttgaagaatt ttggtgatgc tcagttgtgg gattttaggt aaaggaatcg 1174860
cggtaggtag agttccgtaa cgactgccga tggtaggaat atcgatttta agtaaccaaa 1174920
cgagggtggt cgccgttaca attgcaatca tgactccagg atagcgaggc ttgtagtttc 1174980
ggaagtagat catgatcaat agggtaaaga gacccaccgc gaaggactta ctgtcccaag 1175040
tccataaatg atcccagtaa gctatccatt tagggaggaa atctgcagga atattagctc 1175100
ccatttgcag acccaggaaa tctttaattt gcgaggagaa tataatgatc gcaagtcctg 1175160
tgggtgagtc ctgtgacaac gggatagggc atgtatttaa tgaacgtgcc taagccagtg 1175220
agtccgaagg cgataagaaa gacgccggcc agcaatgtga cggtaaaaag cgcctcagcc 1175280
ccgtattttg cagataagca gtataaaata gaaataaagg cactggatgg ccctgaaatc 1175340
agaacgttac ttcctcccat agccgaagcg agaaggcctc cgataataga agctaacagt 1175400
ccctgaattg gggagactcc gacaccaatg gctatggcaa tagcaaaagg aaaggctagg 1175460
attcctacag taattcctgc ttggaaatcc tttttaaaag tattaaacga atagccttct 1175520
ttgatacttg tgtagagttt agggatgaag tttttaaatg cccaagggac tttcacgatg 1175580
caataaccta aagtagaagc cagtgcttac cattttttcg attcctttga atctttgtca 1175640
aatggttcgt gcaatttttt aatctttttt cttcatattc tctgtctagg gatccgtaaa 1175700
agaataatta caaaaatttt tccttcttta cctctgtaag agttaggagt aaagtagatc 1175760
acgaaatcat tttagaggtg accatgctga aactacaatt gtgtgcgcta tttttattcg 1175820
gatatctcgc aattgtcttt gaacatattg ttagagtgaa taaatctgca attgccttag 1175880
ctatgggagg actgatgtgg ttagtatgct tctcccacat tcccatggcg gatcatatga 1175940
ttttagtcga agaaattgcg gacatgtccc aagtcatctt cttcttgttc tcagcaatgg 1176000
ctattgtcga gcttattgat gcgcataaag gattttctgt gatcgttaag ttctgtcgta 1176060
ttcagtcgcg aaccctgctt ctctgggctc ttatcgggct ttctttcttc ttatccgcgg 1176120
ccttagacaa tcttacatct atcattatca ttatctcgat tttgaagcgt ttagtgaagg 1176180
ctagggaaga tcgcttgtta ttaggagcta tttgtgtcat tgcagtaaat gcaggtggtg 1176240
catggactcc tctaggtgat gtaactacaa caatgttatg gattaacaac aagattactt 1176300
cttggggcat tatacgtgct ttatttgtgc cgagtttggt ctgtgtgttg gtcgctggtt 1176360
tttgtggtca atttttcctt cgtaaacgag ggagtactct cattgccaag gatgtagagt 1176420
tacaatctgc gcctcctaag agtctttgga ttatttttat aggtttaggt tccttactca 1176480
tggttcctgt gtggaaggca tgtttaggat tgcctccttt tatgggagcg ttgttaggtt 1176540
taggtcttgt ctggttaacc agtgactgga ttcactctcc tcatggtgag gatcgttacc 1176600
atttgcgagt tcctcatatt ttgactaaaa tcgatatctc ttcgattacg ttctttattg 1176660
gaattttgct tgctgttaac gcgctatctt ttgccaattt gcttacagat ttttctctat 1176720
ggatggataa gatcttttct aggaacgtgg ttgcaatcgt tatcggattg ctctctagcg 1176780
tattagataa cgtaccttta gtagctgnta ccatggggat gtacactctt cctcttgatg 1176840
atactttgtg gaaattgatt gcttatgctg cagnaacggg aggaagcatt ctgatcattg 1176900
gttctgcagc tggtgttgcc tttatggggc tcgagaaagt agacttttta tggtacttca 1176960
aaaggatttc ttggattgct ttagccagtt atttcggcgg attgtttctt tattttgttt 1177020
tagagagcct caattttttc atttaatttt ttatagggaa acaacagaga ctgtgcgttc 1177080
ggtttttcta tgattttgtg tattttttca gataatgttt ttaaaaaaat gttttaaaac 1177140
cctaaaatcc tacctccttg taaccattct cggtagaaaa gagaggtatt tatgaaaaaa 1177200
gggaaattag gagccatagt ttttggcctt ctatttacaa gtagtgttgc tggtttttct 1177260
aaggatttga ctaaagacaa cgcttatcaa gatttaaatg tcatagagca tttaatatcg 1177320
ttaaaatatg ctcctttacc atggaaggaa ctattatttg gttgggattt atctcagcaa 1177380
acacagcaag ctcgcttgca actggtctta gaagaaaaac caacaaccaa ctactgccag 1177440
aaggtactct ctaactacgt gagatcatta aacgattatc atgcagggat tacgttttat 1177500
cgtactgaaa gtgcgtatat cccttacgta ttgaagttaa gtgaagatgg tcatgtcttt 1177560
gtagtcgacg tacagactag ccaaggggat atttacttag gggatgaaat ccttgaagta 1177620
gatggaatgg ggattcgtga ggctatcgaa agccttcgct ttggacgagg gagtgccaca 1177680
gactattctg ctgcagttcg ttccttgaca tcgcgttccg ccgcttttgg agatgcggtt 1177740
ccttcaggaa ttgccatgtt gaaacttcgc cgacccagtg gtttgatccg ttcgacaccg 1177800
gtccgttggc gttatactcc agagcatatc ggagattttt ctttagttgc tcctttgatt 1177860
cctgaacata aacctcaatt acctacacaa agttgtgtgc tattccgttc cggggtaaat 1177920
tcacagtctt ctagtagctc tttattcagt tcctacatgg tgccttattt ctgggaagaa 1177980
ttgcgggttc aaaataagca gcgttttgac agtaatcacc atatagggag ccgtaatgga 1178040
tttttaccta cgtttggtcc tattctttgg gaacaagaca aggggcccta tcgttcctat 1178100
atctttaaag caaaagattc tcagggcaat ccccatcgca taggattttt aagaatttct 1178160

tcttatgttt ggactgattt agaaggactt gaagaggatc ataaggatag tccttgggag 1178220
ctctttggag agatcatcga tcatttggaa aaagagactg atgctttgat tattgatcag 1178280
acccataatc ctggaggcag tgttttctat ctctattcgt tactatctat gttaacagat 1178340
catcctttag atactcctaa acatagaatg attttcactc aggatgaagt cagctcggct 1178400
ttgcactggc aagatctact agaagatgtc ttcacagatg agcaggcagt tgccgtgcta 1178460
ggggaaacta tggaaggata ttgcatggat atgcatgctg tagcctctct tcaaaacttc 1178520
tctcagagtg tcctttcttc ctgggtttca ggtgatatta acctttcaaa acctatgcct 1178580
ttgctaggat ttgcacaggt tcgacctcat cctaaacatc aatatactaa acctttgttt 1178640
atgttgatag acgaggatga cttctcttgt ggagatttag cgcctgcaat tttgaaggat 1178700
aatggccgcg ctactctcat tggaaagcca acagcaggag ctggaggttt tgtattccaa 1178760
gtcactttcc ctaaccgttc tggaattaaa ggtctttctt taacaggatc tttagctgtt 1178820
aggaaagatg gtgagtttat tgaaaactta ggagtggctc ctcatattga tttaggattt 1178880
acctccaggg atttgcaaac ttccaggttt actgattacg ttgaggcagt gaaaactata 1178940
gttttaactt ctttgtctga gaacgctaag aagagtgaag agcagacttc tccgcaagag 1179000
acgcctgaag ttattcgagt ctcttatccc acaacgactt ctgctttgta aacgggacgt 1179060
aatagaataa tttttattat tgctttaata tgcgcgcttc caatataagc attgtgaagc 1179120
gcgtttcata tgtcttttat ctttaggtaa ttattatgag aaaacttatt ttatgcaatc 1179180
ctagaggatt ttgctctgga gttgtgcgcg ctattcaagt tgtagaggtt gctttagaaa 1179240
agtggggagc tcctatctat gtaaaacatg agattgttca caatcgccat gttgttaatg 1179300
ctttacgagc caagggagcg atctttgttg aagaacttgt tgatgttcct gaaggtgaga 1179360
gagtcattta ttcagctcat ggaattcctc cttcagttag agctgaagca aaagcccgta 1179420
agcttattga tattgatgct acctgtggtt tggttactaa ggtgcattct gctgcgaagt 1179480
tatacgcaag taaaggatac aaaatcatac tgatcggcca taagaagcac gttgaggtga 1179540
ttggtattgt tggagaagtt cctgaacaca ttactgttgt cgagaaggtt gctgacgtcg 1179600
aggccttacc ttttagttct gatacacctt tattttatat tactcaaacg acgttgagtt 1179660
tggatgatgt tcaggagatc tcatcggctt tgctaaagcg atatccctct atcattactc 1179720
tgcctagttc ttcgatttgt tatgcaacca cgaaccgtca aaaagcattg cgttctgttt 1179780
tatctcgcgt gaattacgtc tatgtggttg gagatgtcaa cagctcgaat tccaatcgtc 1179840
ttcgcgaagt ggctttgaga aggggagttc ccgctgattt gatcaacaat cccgaggata 1179900
ttgatacgaa catcgtaaat cattctggag atatagcaat gactgcagga gcctcaactc 1179960
ccgaagacgt agttcaagct tgcattcgaa agctatcatc acttatccct ggtttacaag 1180020
tggaaaatga tatatttgct gtagaggatg tcgtatttca attaccaaaa gaactccgtt 1180080
gttcttaggt ctttaggctt acttgccaag tttttctcga gattgcttta tagagtcttc 1180140
ttctcgttca gagagggtat ttaccttttt agttctctgt atttgaaata tcctagatta 1180200
tttttttatg atcttggtaa gtatgtctat tctttaaggc attgccctta tgcaaagctc 1180260
ggtcgcttgc caggagcctc tttattgaaa gaaggaaacg tctacggaga gactccatgg 1180320
tctgttcttg caaagatctg tcaggctttt gatattactt ctcaagacat tctctatgat 1180380
ttgggatgcg gcctaggaaa ggtatgtttt tggttctctc atgttgtgcg gtgccaagtt 1180440
atagggattg ataatcaacc ccacttcatt cgtttttctt caaacatgca ccgcaagctc 1180500
tcttcaggat tcgcgttatt cgatactgaa gagtttaaga acgtagttct ctcacaagct 1180560
tcttatgtct atttttatgg ttcttcgttt tcacgacgcc tgttaaatga gatcattctt 1180620
aaattatcgg agatggctcc aggaagtgta gtcattagta tttccttccc tttagactct 1180680
ttctcaaggg ggaaggaatg tttctttact gaaaagagct gctcggtgcg cttcccttgg 1180740
ggaaagacaa tagcatataa aaatattcga aaaggctctt aattctactg atacagactg 1180800
cgagcagcgt catgttctgt ttggagaatg ttcagaagga aattgtccat acttttccag 1180860
ttgtctttga cttcctcaat ggtacgtgtg acttcatcct gtctcattct gaagacttct 1180920
tttctgtact cagcaacagc tcgtacagca ctctctgata actcatgtac tttagaagcg 1180980
gcttcagtaa gttgtgatag agaggtgaaa acttttccta ttcctttaaa gaaggttttc 1181040
gcagtcgcat ctttgaatct tccagaaatc ctttggacaa accctaaaat gctatctccg 1181100
gaaatttctc caaccatagg agcaatcgct cctaaaatac ctaacgttgc tgtcgcgatc 1181160
cctagccagc gggcgtattt agcttgacgt gaataactcg ccgccaattc atgagcttcc 1181220
ctttccatga gctctctttc ttcaagacgt gtcattgtgt cgttagctct acttttgagc 1181280
atcaacttca ttagctctgt acatattcta aggacatcaa ggttggtcac acgctttcct 1181340
gcaagcacgc ttagctgtga ttcagacaaa gcaaactcca caataggatc aggaataata 1181400
ggagtataag actcgtacac ctggttcgaa cttgaagtct gttgtagaga ttccacagtg 1181460
caccatggtg tctttttctt gccttcttct tgctcttgtt cttgctcgta tccttcttgt 1181520
tgcctttgat ctcgatgttc ttcatctttt tgttggcttt ttgtagagga tagtgcttga 1181580
ggtacttcct tatgtaaggt gctatagagt gacatcggac taagagtcgc atcagactta 1181640
gtttgtgtgt gtttttgtgt ctctgccgac ttggtgcttt gtgagacttt ttctttagca 1181700
ctatgttctt ttatggtgct gctagaatag gaccttacat gaaattgtgc ttggctttga 1181760
gaagcgcttg cttcttgttg ctcttgtgct tttggtgttt ggacagacac tgtgaacgtt 1181820
ttgtccacac tggagtctcc ttcaggagct ttgcatgaga aaaacggaaa ggaagacgag 1181880
gttgtttctg ttgatgcttt ccctgtggaa ttcgaagatg tggattctgc gctaagggat 1181940
ttctatgtga agctaagcta gtctgctgcg attgtcctgg ttttaggttc ggcagagatg 1182000

ttgatcgcat taatatgcca ttttttacta gaaattgctc ttcttgtaat ggttttttcc 1182060
ttgggaggca gtgcttgtgt caaagcacat tccgattgtg tnaaaatccc aaaacatagc 1182120
tcggaagttg ctctcagcat cgcatattgc tctcctgcag ggagagctac ttgtacttct 1182180
gcagcaacaa cagcttcttc agtggcattt ccatctacaa aaatatcaaa ctctccttca 1182240
ggaggaggag tcgatatgga ttggtaagat acagtcataa tatntcttct gtggatttat 1182300
cctcttaagg agtggagata cacttgatct agttcacgca tgtaatttaa gattcttgaa 1182360
agggcttcga aagaactctc catgttatct aaaagttctt gagaacgttc gtaatgatcg 1182420
tccctatcaa aacttttcaa ttcaatgtcg agttggattt tagtgagttt tcctttgagt 1182480
ttatagattt ctgcttgaga aaacagtata gcgcatctta aagcagccat cgtagattct 1182540
atcgcgggtt ggattccttt aatagctcct tctataatag gggaaaaacc taaattttct 1182600
acttttaatg tgcatatgga gagaatgaca ggggttaaat agacgaccca ctggattatg 1182660
gttataattt ttcgtcttag ttcttcatta tttataggca gatgtttttc tagccaatcc 1182720
cagaacttta gtttctctaa acacttgata acaagagaaa ttagccctgc aaaaaaacct 1182780
gcaaaagcaa agataccgcc tccactcaag atagcaacaa tgccgatccc tatagaaaca 1182840
aacggagcta gccaaccgat aatgtcagaa agagatcccc ataatctttc tcgtttttct 1182900
ttctcgatgc tgaccttgat agtctcgatt ctcttttcat gtagagcatc gagctctcgt 1182960
tgtgtcgact ccacggaatc tttgtggcat ccatacgaag aaaagtctga aagaatccct 1183020
tgttttgtga gtagatagct caatgtaaac actcctatgc ttggaggagg aattacagga 1183080
acagcttgtg ctgctttggc tattctattt ttacgtgatg tatcttgttg gaaaggggat 1183140
ttcttttgtt tttgattgga atcctgatcc tgagaaaaga ggtctttaca gagatctttt 1183200
atctgaactt tggtttctat ttttttctca gaactctgct ggtttgcata ggtttcactg 1183260
cgctcttctc ttcgtgattg gaggagttct tgctgttcac tatctaggct ttgagaatat 1183320
agcgagtcaa cggttccaaa caacttgaga tgttgttgta ggtgcagcgt atcaaaattt 1183380
ttgcaagagc cttcttgttt agaactcagg tttttatcta gagccttctc tgaagttttc 1183440
tccggagatt ttggcatagt cagttcttgc atcagagctt tacttgcttc tgtgctattt 1183500
ttttgagaaa aaagattcca aggagatgaa gtttgagctt gcgatgtgtt gtgaagttga 1183560
gattcgaaag agctgccgaa acgcaagatt ctctctcttg gtgttgcaat aatgaatgtt 1183620
ttatgatggg tgataggagg ctcagactta cattgtgact gaaatttctg agtgaaaaac 1183680
ttgggaagag acttttgagc ttcttgaggc gctacagtga tagaatagct aatcttagga 1183740
accgaagatt cttgtgacct aggagcatca gggatataag gatcttgatt gagaagaacc 1183800
tcacttgctt gagataacca agaggacata gttgagtttc cacattattt atgttttcga 1183860
atatctagaa tttcttcttt caattcatta tagaggggct tgtgttgtgc acgtacccac 1183920
gccatttcta aagctttttc agcttcttca tgttcatttg taagagtata gcatatgtag 1183980
gcatagtaat gaggataggg atctttatct cgaaggactg cagtgactcc ataggcatgc 1184040
aaagcttgtg aatattgttc ggacatatgt agagaggcac ctaaagaaaa ccaaaactta 1184100
gagacaaaag gattgaagaa aactaaccag cgaaaaacgg tgatgctttt agcatagtct 1184160
ttgtcgagat aggcgtgata accctcttta tatacttttt caagatcctc ggaggatatc 1184220
ttgaagatct tttggtaggt atctaaagct atatttttat ccgggacata tccttccaaa 1184280
tagctttcca aatcatctgg gaaagggaag tcttccttgg aagatgcagc gattttttct 1184340
agtaaataat ttaaatgtga cattaggggc gtaggttgta aatgaaggtg tccataagtt 1184400
ctttcaataa cttcaataca ttagagcgcg cttgatgaca ttgagaaatc tcttggaggt 1184460
gcctttgcat gtccgtcctt tccatttgag tgattttctc catattctct ttgcgcattt 1184520
ggacattctc ttttagaagt cttttttcct cttctgtcca agtatatttt tctttgtcta 1184580
tagtgactcc aatctcttta gctcgattta aaagagcttt catctcttca ttttcactcc 1184640
aatcaatgtc tttcttttca ttattgatct tagagataag gactgtgagt gtgtctacat 1184700
catcggtacg ttgtttgact cgcatgtaga gttcattagc ttcggcttcg gcttggccta 1184760
agattcttgc catgagcttc atgaacctta agaacacatt ttctactttt ggagtttgta 1184820
taggcncgtg tattgaagag cctacagata acggattctt actagggatg aatctgctaa 1184880
acacagacat tggagaaggt agcttctttt tgaatgtcgt ttctttntta gaagtttctt 1184940
cagcaggagg tcgcatttga tctgagaaga ttaaagcggc aatatctaga ttttctccgg 1185000
gttcctcagc gactgcctct acaccgagac cacgtttctt ctttttcttt ttagattcag 1185060
cgtcttcttc ctgatcgtgc tttctgtcgt gctgctctct ttgttggcga tcttgatcgc 1185120
gttctttata gagttcatga gattttgagg tcgtcagggc ctctttttgt ttctctgcag 1185180
agaactggga tagaggagag aaggagcttg ctttttgttg ttctaatagt gttctttctg 1185240
gcgcactttc ttgcggaggt aaggatctct gagaacttga gcttgcctga gataaggaag 1185300
cgcgagcttg tgtctcaggt gacggtcttc gctgtgtttt cttacagcag agtcttttgg 1185360
tgtttgaaga gcttgctgtt gtttagaatc agaaatttca ggttttaagg agcgtgggga 1185420
gagagcagat tgtttactgc tgctttgttg agggagggtc tgctcactcc tactttgcgt 1185480
ttgtgtttct tgtggttcac ttgtgattgt ctctgcatgt tccaattgca ctagcaagtc 1185540
aacaacttgg atttctaatt ctacaacttc aggagggagg aagaggagtt gatgaacatt 1185600
tgtgctttgt aagagcagtt cttgttgggt cgcaagaatg atttgtccta aagctaagct 1185660
aattaaagca tctgtagaag agcttccttc tagcgctgta caagagacta agacttcacc 1185720
tgttgcagtc tttaatattc cagaggctgt ggaaactgtt gagctgtctt cttttagagt 1185780
tatatttgca gagggaactg cagaaggaac aggcatattc atgatttgta atttgagtgc 1185840

gtagaaaaaa aaggaaaatt agctaaaaca tcattttcct gcaagtttga ttgtaagcgg 1185900
tttagaggga aagagaaaat aaaacaaaaa tccaagggtt tttgtttgca aaaaacactc 1185960
taactataga gaataaatgg gtcagtttca tgtctttgac aaagatgctc tgcccaggtg 1186020
gtgaaattgg tagacacgct ggatttagga tccagtgctt tcgggcatgt aggttcaagt 1186080
cctatcctgg gcatctcctt ttctaatctc tttaaatcca cctaaaattt gctattcgta 1186140
gtaaaataaa atctaagggt tagatttttt tagagatctt tattatgaag aaagtcgtaa 1186200
cactatccat tatatttttc gcaacgtatt gtgcatcaga gcttagtgct gtaactgtag 1186260
tggctgtgcc tttatcagag gctccaggga agattcaagt tcgtcccgtc gttggtctgc 1186320
aatttcaaga agaacagggt tctgtgccct atagttttta ttatccttat gactatgggt 1186380
attactatcc agagacttat ggctatacta aaaatacagg tcaagaaagt cgcgaatgtt 1186440
atacccgatt tgaagatggc acaatttttt atgaatgcga ttagagattc ctggggtgat 1186500
gggcaagaaa cttctcgatt agagaatccg cggctacgtg agtatacact tctgtagagg 1186560
cgatgcgtgc gtgacctaac atctcttgaa tgacgcgtag gtctgcctta ttgtctaata 1186620
gatgtgtagc aaaggcgtgt cgtaaggagt ggggagatac gggctttgag gtcacttgct 1186680
ttgcgtaatt gtgaatacgg cgccacacac aagagcgttc gagcttgtgt ccccgggtag 1186740
agaggaaaag atgatcttcg tgaggattct ttttctggta ttggtctcgg aaaggacaaa 1186800
ggtacgcatc gatagcttct cttgctcgag atcccagagg caccagtcgg gttttagaac 1186860
ctttcccggt gacgcggatg caatcatcag agacatggcc taaacgtagg tcacaaagtt 1186920
cagaaacccg gacaccagtt gaatatagtg tgtggagaat ggctgtatca cgaaaggcta 1186980
ggtgtcgggg gtttttctcc atttggagag gaaccgcaag tagagcatcg acttcttgag 1187040
gagtcagtac tgaaggaagg cgtttccata tttttgggtg ctctattatt gggggatagg 1187100
gaagaagttg ctgatctttt aagaagagaa aaaatacttt taaagcaatg agcctacgcg 1187160
ctaacgtagt ctctgcttct ttacgtctat atagttcttc agcaaagata tatacgctgt 1187220
tttgtgaaat atcttggggg gagctaatgg cacttatggt aaggaaggaa gagatgtctt 1187280
ggcggtaagc cgctatagat tgttggcaaa ggccgcgatc tacagataaa aacaatgaga 1187340
attgttctag aatcgttgtg tgaaactgag tcgagggcat agtcatactc gctaacgaat 1187400
gtaactttag tatagggagc ggagaatttt caagctacag agagaaaacc atggaaagaa 1187460
aaagatttat agattgcgat tcaacaaaga tactccaaga gcttgcttta aatcccctag 1187520
acctaacagc tcccggggtt ttatctgcag agaggatcaa gaagttttct ttgctaggag 1187580
ggggatttac ctttagcttt gctaccgagc gtttggacga tgctatttta gctgcattga 1187640
tctcattagc agaagaaagg ggattgcatg agtctatgtt agcgatgcag caggggcagg 1187700
tcgtgaacta tattgaaggg tttccaagtg aaatgcgacc tgctctgcac actgcaactc 1187760
gggcatgggt aaccgacagt tcatttacag gagaagctga agatatcgcc gtaagatctc 1187820
gggtggaggc gcaacggctt aaggattttt taaccaaagt gcgcagccag ttcacgacca 1187880
tagtgcagat aggaatcgga gggtcggagc taggccctaa ggcactctat cgggcccttc 1187940
gtgcgtactg ccctacagat aagcacgtac atttcatatc caatatagat cctgacaatg 1188000
gcgccgaggt gttagatacc atagattgtg ctaaggcttt ggtagttgta gtatcgaagt 1188060
cagggactac aatagagact gcagtgaatg aggccttttt tgcagattat ttcgcgaaga 1188120
agggcttgtc atttaaggat cattttatag cagtcacttg tgaaggcagt cctatggatg 1188180
atacgggtaa gtatctagag gtctttcacc tttgggagag catcggagga agatttccct 1188240
ctacctctat ggttggaggt gttgttttag gttttgctta tggctttgag gtttttttgc 1188300
aattacttca aggagcctca gctatggatc aaattgcttt gcaaccgaac gctagggaga 1188360
atctccctat gctttcagct ttgattagca tttggaatcg aaatttctta ggctatccca 1188420
cagaagctgt cattccttat tcttcaggtt tggagttttt cccagcgcat ttgcagcagt 1188480
gttgcatgga atctaacggg aaaagcattg tccaagatgg tagaaggtta ggattttcta 1188540
cgagccctgt catttggggt gagccgggaa ccaacggaca gcattctttt ttccaatgcc 1188600
ttcatcaggg tacagacatt attcctgtag agtttatagg ttttgaaaag agccaaaagg 1188660
gtgaggatat ctcatttcaa ggaactacat cttcacaaaa gctttttgcc aatatgattg 1188720
ctcaggcgat tgccttagca tgtggctctg aaaatacaaa tccgaataag aattttgatg 1188780
gaaaccgtcc ttcttcggtt ttagtgtcta gccagctgaa tccgtattct cttggggaat 1188840
tactttctta ctatgagaat aaaatcgtat ttcaagggtt ctgttggggg attaattctt 1188900
ttgatcagga aggggtctcc ttgggtaagg cattggcaaa ccgtgtttta gagttgcttg 1188960
agggggcgga tgcttccaat ttccctgaag ctgcatcgtt gttaacgctt tttaacatca 1189020
agtttaggta aaaattacac tatcctttca taagtctacc tcagagagag gtttttctta 1189080
aggttcttta ttgtccgtct gtgatataat gctcctttgg atttggaaaa atttgtgagg 1189140
ataggatgtt tttcattgca gtacgctctc gtggattttt agatattcat ggtattttag 1189200
ccgctcgtaa gggtaagcaa gtagtgaaat ctactgcggg cgcatggata gggtctcgtg 1189260
gcgccgtatt ctacagcctg gtttcgtaat tttaaataaa atattccgta tctttgttga 1189320
aatatgccag gttctgtgtc atcacctcct ttgtctcctg taattgtccg tgaaagggtc 1189380
ccatcctctt caggatccga cctcatacag cctcatgctg ttttaaagat ctccatccta 1189440
atttttgcgc ttgtgacaat tttaggaatt gttcttgtag tgtctagtgc tttaggagct 1189500
cttcctagtt tagttttgac ggtttctggt tgtattgcaa tagctgtagg cctgattggt 1189560
ttagggattc ttgtgacacg gctgattctc tctacgatca gaaaagtaga tgccatgggt 1189620
tatgatgctg cggtcaaaga agagcagtat ttgtcacgta tcagagaatt agagtctgaa 1189680

aatagagaga ttagagatag aaatcgtgct gtcgaagatc agtgtgccca tttatccgaa 1189740
gagaacaagg accttaggga tcccgaatat ctacatggaa tgactgaaag gctcattgcg 1189800
agcttagaaa tagagaatca agctctcgta gctgagaaca ttcttctcaa agactggaat 1189860
gcaagcctat ctagagattt ccgcgcatat aagcaaaaat ttcctcttgg ggcattagaa 1189920
ccctggaaag aagatattgc atgtatcatg gaacaaaatc tctttttaaa accggaatgt 1189980
atcgcgatgg ttaagtctct tccattagag acgcaacggc tgtttttata tccaaaagga 1190040
tttcagtctt tagttaatcg atttgctccg cggtctcgct ttttccagac tccaaagtat 1190100
gaatataaca gtaggaatga aaatgaggac ggaaaggtag ccgcagtgtg cgcccgtttg 1190160
aaaaaagaat tcttcagtgc tgttttagga gcctgtagtt acgaagaact agggggcatt 1190220
tgtgaaagag cagtagcact taaagagacg ttgccattgc ctgaagctgt ctatgatacc 1190280
ctagttcagg agttcccaaa tcttcttact gctgagagtt tatggaaaga atggtgcttc 1190340
tattcctatc cctaccttcg tccctatctt tctgtggatt actgtaagag gttatttgta 1190400
caactttttg aggaactctg cctaaagctt tttacaacgg gatctccaga agaccaagct 1190460
ttggttcgcc ttttctctta ctataggaat catattcccg cagtcttggc ctcatttggt 1190520
ttgcccccgc ctgagacagg ggggtctgta tttgtattgc taccaaaaca agaaaacctt 1190580
ctttggagtc aaattgaggt gctggctaca aggtatctca aagatacctt cgtgagaaac 1190640
tcagaatgga cgggctcttt cgagatgatg ttttcttata acgagatgtg taaggagatc 1190700
tccgaaggaa ggattcgttt tgctgaagac tatgaaacga ggcattccga agaattccct 1190760
ccttcccctc tctctgaaga aggagagggc gaagaattcc ttcctccttg ctctgaagaa 1190820
gaggtttcgg ttcttgagcg cccagatcta gatgtagact ctatgtgggt ctggcatccg 1190880
tccggtccct aagggacctc tttaatcacc tagagttcta ttttcagaat cttcatagta 1190940
gtaattttcc taagatataa ggtcttaaat gagttctaga ggttttagga tctttttgga 1191000
gagcttgntg tgggtttaaa atcttgataa gaagatatcc tagtgtttag aaacccaaaa 1191060
atttagggtt tgatgaattg gagtctcgaa acgacttttt tttcaatttc ttgttttgtt 1191120
tctacttaaa gactatagtg attttttttg aagggggtgc gtatggcatt caaagaggtc 1191180
gttcgtgttg ctgtcacagg aggcaaaggg cagattgcgt ataatttttt atttgcatta 1191240
gcccatggag atgtttttgg agtggatcgt ggtgtagatt tacggatcta tgatgtgccg 1191300
ggtacagaga gagctctctc aggggtgcgt atggagctcg atgacggtgc atatcctctt 1191360
tnacatcgtc tgcgtgtgac gacatcgtta aacgacgctt ttgatggtat cgatgcggcg 1191420
tttctgatag gtgctgtgcc tcgtggaccc ggtatggagc gaggagatct tttaaagcaa 1191480
aatggtcaga tcttttcgtt acagggggcc gctttaaata cagcagcaaa aagagatgct 1191540
aagatttttg ttgtagggaa ccctgtcaat acgaattgct ggattgctat gaaacatgct 1191600
cccagattgc atcggaaaaa tttccatgcg atgttacgct tggatcagaa tcgcatgcat 1191660
agcatgctcg ctcatcgtgc tgaggttcct ctagaggagg tctcccgtgt tgtcatctgg 1191720
ggaaatcatt ctgcaaagca ggttcctgac ttcacacaag cacgtatctc agggaaaccc 1191780
gcagccgagg ttatcggaga tcgagattgg ttggaaaaca ttttagtaca ctccgtgcag 1191840
aatcgtggaa gcgctgtaat tgaagcaaga gggaaatctt cggcagcatc cgcatctcga 1191900
gcacttgccg aggccgcgcg atctattttt tgtcctaaaa gtgacgagtg gttttcttct 1191960
ggagtgtgtt cggatcataa tccttatggt attcctgaag acttgatttt tggttttcca 1192020
tgtcgtatgt tgccttctgg agattatgaa atcattcctg gattgccttg ggagcctttt 1192080
atcagaaata agattcaaat ttccctggat gaaattgctc aggaaaaagc tagcgtgtct 1192140
tcgttataag cacaatgttg tggggagtaa gtatgagaca atcattcgat gaattaagtc 1192200
aaaatgcatt taaaaatatt tttaataaac agaggttctg ctttattttc tgcagtctct 1192260
gttgctttgg ttttgtgttt gcattgtttc tgaagctctg ctcacggctg gctcctgaaa 1192320
tttctttgtc gacattaggt ttgggagctt ttttctgtgc ctttagtgtg atttgcgctt 1192380
cagcgattat cgtgcaattt ttattgcata aggagtctca aggagaaacg agtaagctct 1192440
gctgtgctat taagaacacc tggtcttcct tatggctttc tcttcttgta tcgatgccgt 1192500
tcttcattgc tatggtcgcc gtagtcactg tagctatgct ctcttctttt ttaggatctc 1192560
ttccttgggt gggtaagtta ttccatacag tgttgatttt cattccttat ctatcggcaa 1192620
cggcactgat tttacttttt ttaggttctt ttagctgttt gtttttctgc attccggttc 1192680
tccataatca agagtctata gactatagga aattgcctag agtgttttcg tgggaatatc 1192740
cttcggcagt ttataggggt ggtgattgct ttggttccnt tagccctatg cagttggtta 1192800
gctttagatt ctttttattt gatgacacat cttgttgaaa ttgcagatat acatacctgg 1192860
tcatttctag ctcagatgtt tgtgcttatt gttcctattg ctttgatttt aactcctgcg 1192920
gtttccttct tctttaactt ttcttttagc ttttaccttg caaagcaaga agaagagaaa 1192980
gcccttgtta aatagtaaat agaaattctt tagcaatata agctgtagat tttcgtagaa 1193040
ctgcctgagc gagcatgtct cctgtaatcc cgtgatacag gagtcctttc gacccgagtc 1193100
ctcctaagaa ccagagtttt tccctaatcc tagagatgac aggaagacgt gatttgctag 1193160
aagagcgcat gccagcataa cagtgcagga cttgagcatc tttaagtcct ggaaatagcg 1193220
agagcacagg aggcatgatt tcctgatagg caatagcggg atcaggagtc tcttcgggtt 1193280
ggttgtgttc gaaagtcgcc cctaaaatgc aggtattctt ttgtgtattg gcgaccatat 1193340
atttatgagc attgatactg aatgagagca tggcaagatc tttaggccaa ctgatctcca 1193400
gcaactgtcc ttttacttta tttaccggca tgtctttaag ttcaggaaga atcgaggcgt 1193460
tggctcctgg agtcactatg atatgatcat agaactcttc aatatcggca aggtcctcaa 1193520

tgagctcatc atagaattgt gtccctaatt tcatgcaggc gtcagccaat ccctgaatat 1193580
agagatcatt atttagagtt accccactct ttatgaatag tgctccgaga tttgggggga 1193640
tgaccatact gggaatggag atttcgcaac gagccntctc ccaccattcg acttctttgg 1193700
ggaattcttc aacacgctcc gtgaatagct gagcttgatc ctcgtcaatt gccggtctta 1193760
agatcccttg agaaatcaca ataggtacgt tgagggcttt actagcgcan tgtgattaac 1193820
gcgtgtgtag cattgattcc ttgatcctac caggggaggc ttgagagctt ttttccccgt 1193880
gaaagcatgg aggagtcctg aagacattcc agaggctcct tctcctaagg gaataggatc 1193940
aaagagatcg atagttgcag tcccctggga gtggagaagc agatgccaag ttacagagag 1194000
tcctgcatat cctgctccta aaacggctat acgcataaag tgtaccttaa atcaagtatc 1194060
gttacataga ccttgtcttg agaataagag aataagggat aaaagaacag gggggaatct 1194120
ctctgattgg gagataaaaa tgtagaagat gaaaacctga ttcttaaggg tgaatttttt 1194180
actatagaga agagaattag ggagaccccc acggcataag ccgtggggaa ggatgaaagt 1194240
ttaattaaat tttgattctt cctgttgaga ataggaagat cgctgtcagt gcaagaaggc 1194300
ctatgaaagt cattcctaca atttcttttt tagcaaagaa tgtcttagca tttttcttct 1194360
ttttccctgc gtctatatag aaggggatgc caagagctaa aagaactaag gccataaaga 1194420
ggtattttag acctcctgca tagataagcc atagagagta aaccactccc aggataccag 1194480
ttatcattgc caaaggggct ttaatagacc cttttttagg atatgttttg cttttgctaa 1194540
gtttaaagag aaaggcagca ctcgctagat acgcagggag aaccatgacc cccgtgatgc 1194600
tgagcatagt attccaagca ttcgaagaaa agtaaacaag gagcatggcg agctgcatca 1194660
cagagctcgt aatgtatagg gagacgctgg gagatttctc tttattttct atagtgaaga 1194720
tctcagggaa ggtgccattt ttagctgctg agaaggggat ttctgcaacg atgatcgtcc 1194780
aggataacca gctagataaa acagcaataa tcaggccgac attcatgagc acttctcccc 1194840
atttccctac gaggatgtcg aggacaccag ctgtagaggg attggggatg ttcgctagtt 1194900
gatgttggaa gagcgagcca aaaggtaata aagaaaataa gatgtagatt gttaggcaac 1194960
ctaaaaatcc taagactgta gcttgcccta cagagagagg attttttgct cgtccagaca 1195020
tcaccacggc accttcgatt cctataaatg cccatagggt gaccaacata gtccctttaa 1195080
gttgtgaact tacggatcct aaactcggtt gggcttttgt tacggcatgt ccccaaaaat 1195140
ctgttttgaa aacagcgagc ttgaagaaga acgcagtcag gataatgaag atgattagag 1195200
ggatgatttt aaatatagtt ccgatgacgt tgattatcga tgcttgacgg attcctttca 1195260
gaactatgaa gttgaacacc cagataagaa tggagcctcc taaaatagca ggtagggtat 1195320
ttcctccttg gaagtaggga gggaagaagt agtttagagc atccatagta attacggcat 1195380
atcccacatt accgaagatt tgacaaagcc aatatcccca gccgatggtg aatcctatgt 1195440
aggggccgaa accttctcta ctgtacatgt agatcccttc cttaaggtcg ggacgtattg 1195500
tagagaggat cctaaacgta tttgcgataa agaacatgcc gaagccagtg agtatccaag 1195560
ataggattac ggcaccagct cctgctgtcg ctgccatgtt ttgggggagg ctgaaaatcc 1195620
ctcccccaat tatggaactg actaccatac ccgcaagggc tatggtccct aaattttttg 1195680
aggatttagt ccttgaggtc atgaaagtct ccttaattta ccttagctgg ttcagcattt 1195740
tcgaaattta ggaatcctaa tgcagttaag caaaaaccga atttttgttt gatgttgatg 1195800
taattatgga agaattggaa ttcgctatgc tttgctatag agcgtaggtc aagttcgtgt 1195860
tgtagggatt ttttcaacca cattttagca tgggtttctg caatttcgtc atttatccat 1195920
gtagggaaga attcaacata ttctgctgcc catccgccaa taagctctcc atttttatct 1195980
ttaccccagc aaatgccgat tccggtggcg atcgcatggg ttccgtcaga gagagcagca 1196040
ccacgacccg ccatgattac ttcaaggaca gcgccgtgtt tgaaagattt tacacaggta 1196100
tctacaggaa cgatattccc aaagagctct ttaggaagta cagatgtata agggacgata 1196160
ttaaaatttt cgatttttgc ttgtagaaga gcagaatcgt agcagaaagt ttcaaagggt 1196220
tgtgggggca taccgtcatc agattcacca atgccccctg tatggaatgc tagcgtggga 1196280
taacgagttc cgtaagccat aagttactcc taaataatat tggtgtggtt ttgttataag 1196340
gaaagatttg cacgcaggcc atacacttga gaggttctcc tctctgggcg taacgctggg 1196400
tggatataga gttggaaatc tggagttaga gatatataag gtccaaagcc aatagtagcg 1196460
aaagcttcca taacagactc ataacgtcgt agcttgttca cattggaaat cgctttagca 1196520
tttaccttat ttgtagcaaa tcctatgcca agaagatctt gagagtggcg gtttaaggga 1196580
ttctcagaga caagacctaa aacatacgaa cggtttatgg ggagtgctgt tcccgtagct 1196640
ccgttgattc ttccgaatag atagagcttt tcatgaatat gttgagcagc attcagggac 1196700
catcctgtga cctgggagtt ttgctcagga actttgcgtg tgctatagag caatacagag 1196760
tactgtccat ctccacacga aggttttgga gtccaggagg cgtagccgta aaagttgtat 1196820
gttgcttttt gtcagattat aaatcgaaaa gttggttcca tcaatattat aggaatcttg 1196880
aaaacctagt tgaaccttga tttcagaatt tggagtgaac tgcagataag cacctgtact 1196940
tcccaatgag taggtagcac ttgcgttttg tgataaggcg tagctaataa acccagagta 1197000
ctgatcatta tcgtaaagcg tgccgtctat agcatagagg ctgtattgtc ctatagctag 1197060
ggtcaaaaag tctccaggga aagtctgaga gaaggtaagc tgtgctaggt tattttctct 1197120
attagagtaa tcattcatac tggaagcggt tcccgcggct tgattggcgt caacaccgtt 1197180
agtttgccag taatgtatca acgtatagct aaagtctacg attccttgac ctgctgttgg 1197240
agaatcataa agagtccagg tagtgctagg actgaagtag aactgccacg aaggaatctc 1197300
tagggcttgc gtttggccaa gagctttgtg agggtaaaac cactggggaa gaatactgaa 1197360

gtccaaggat atctgagtat ttgtcgcttt ttctacagat gttaataggt tggatacgga 1197420
aatgccatcc ttccaaggat gtgaacagaa gatattagta actggagaga gaaggctgtt 1197480
atgatgtgga gtttccgaag gggcgctttc ttttatggac tcgggatgtt ttttttgaat 1197540
tcttgcttta taacgatggt agtggcccgt cgtttcttta ggagtttcgg cataagagga 1197600
aattccaaga gcgcagagtc ctgaaagtaa aagaaancga aaggntatca tgaattcccc 1197660
gtagaagaag gcatagtgtt tttttttgac tctaaaaaaa agaattgttt ttatgtagca 1197720
aaaattttct tctgaggaga ggaatgctat ggaatccaca gtatcagtcg ttaagaattc 1197780
tcctgagtag tcttttttct tattattcta aaattatttt ttattgagat cgagaaccca 1197840
caaaagtaaa cgtcagggag tgcttatctt taagaaggat tattcgtgca tttggtggcg 1197900
cagaagaaga gccgccgctt cattgctgag taatttttta gagtagttgt actgtacttg 1197960
tgcaatgtta tcaaaataga tcggggggtg aagttcctga gctttatagg tgattccgaa 1198020
ataagcaatt ccatattttt ggcatagatc accgatgcga aggacatttt ctttttggtt 1198080
atcaatatag ataatcttag cgggtaaggg ggtgcatatt tccaagaaga gatcgagtcc 1198140
cggtcctttg tggtagtctc cagaaaaaag aattcccgat gtgtagagaa ggttcttggg 1198200
taggggagct tgaggttgtg gagcagtatc ttctaaggag acgttgagca tatgaagttg 1198260
ctttagggta agatctttag ctgttttagg acgttccgtg tagacaaagg ttgtctttcc 1198320
ttgcttctgg attttctcaa tgagtagaaa aatagcagat tctataggct gaacggttcc 1198380
catttcttga atttcaatcc agaaaggaac tacagcctcc caagcttctt gttctggggt 1198440
tccctgtttc tgtaacccct ggattgcttt gcttttccat atagaatgag agagagcctc 1198500
tcctccttga agaagcgtat catcaagatc taagatcagc cagaagttct cttcatcata 1198560
gaggatatca ccagcaactt catgaatgga cctgacctcg gcataacgac aagaagcata 1198620
gaacgacgag caggagaata agaaagtgaa gaacaaccag gttttcataa cgtaccttgc 1198680
aatcttttgg acaaggatag agtaagacaa gtatttttta tacttttgcc atgaagtttt 1198740
ttacatagtt tcggaaagag tcacaagact ctggagttag gaaatctggg aaccaaagag 1198800
caaattgtag cagggcttgt tctatgaaca tttcgtagcc atggataatg agagagccat 1198860
gtttttgagc acgttcaagg tagggagagg gatgcggttt ggtattgatg tccatgacta 1198920
taggagggaa tctccaaggg aaggtcactt ccggagggag acagttgatg attatgtcta 1198980
tagttttaaa attctctaag gagcctaagg gataggcttt gcctttacag caagtagcta 1199040
aagcagctgc tgaagaaaga gttctattga agatgtggag attagcacct tgcatcgcca 1199100
gtgttgcagc gattgcttta gcagcacctc cagcacctac aatggcaatg tgtttgttgt 1199160
taacagagat attcttttgt tttaggagtt tggctactcc ctcaccgtct gtattgtaac 1199220
ctagaatttt ctgatttcgg aagaccaggg tgtttataga ctcgcagagt tgcgccgatg 1199280
catcaagagc atcgacatgg tcgaagattg cggtttttag tggcatagtg acactaagtc 1199340
ctgaaaaagg aagatctcgg atcgcagaga aaaaggtgac gacttcccct atagtaacag 1199400
ggaatttgat atatgttgca ttgagagaaa gcttagagag taagaagttg tgggagaggt 1199460
gactgatgct acggtctaca ggatctccga tgagtccgta aatgtgtgat ttttcagata 1199520
gcttcgagta gttataggag agcagctcct caagtttagg ttgtcccggg gctacttgag 1199580
gcgcactgat tccagcagcg taattcatag cgttgctgat cagtggggag agaactctag 1199640
aggggagccc atgggttccc atacaaagga ctgtagatgg ttttggtaac aggcgagctt 1199700
tttttatata attcagagct tcactggagt tctctgggga gagtacgatt ttgtagatct 1199760
cagcgggagt tgcaagcatc tcattataga ttgcatcgag gtcttcattt ttgtctgtat 1199820
gataggaaag gatgagcttt attttaggat ggctcttgcg gatggtttga agggcggtct 1199880
taggaagact cacatcgata tccatccact tgggttcaag ttttgctagg gaatatagtt 1199940
tttgaatcca tagcgctgtc gacatctctt tgtgttgtct aaaggtgagg atggggtttt 1200000
gggctgtggt aatgagggta tgcagttctt gatcatcaag ttcgtttatg agatcgagtc 1200060
gtagttctat aatatctaca aggtgtagag actttagaat ttgttgtttt gcttcacaaa 1200120
atgagggacc gctaacagtg gcgcataaca tgacactcac tccatagtat atcatagaga 1200180
atctccatat ttggagatgc acaataggta ccattaaagg gagctgcacg acctagatgc 1200240
tctatcatga tcatttttag ttcgtgttga gaaagatttt ttttatcgta tcctaaagtg 1200300
tagatgatat tttcaggact atacaagctg ttgtggagat gttccggaac aatagattgt 1200360
aaatctttca aagtagaagg gagattaaat cgttttagga gtctttcaag ttggtctatg 1200420
agttgtgggg ttttcataac tccttcagca agagatattc ttgtttctat catcataccg 1200480
acacttacgg cttggccatg atttaccgtt ccttttgcca gggtttctat agcgtgggct 1200540
atggagtgac cgaagtttag gatttttctc agacttcggt catagggatc ttcagcgaca 1200600
atcgccgctt tgatttggca gtttcttttg atgaactcgt ggaggatctg cgatgaagaa 1200660
aatagcattt tactatggct atttagaaat tcccagagat aagcatccgc gatgaatcca 1200720
tgttttattg cttctgcaat tccatgatac cattcttctc ttgggagtgt agagaggaac 1200780
tgaggacaca tccatacttc cttgggtaag tagaatgtcc ccagtcgatt tttgattcct 1200840
cgtaagttga ttccattttt ccccccgata ctcgtatcta ccattgcggt gatcgtcgtg 1200900
ggaattagat atagagggag ccctcgacaa tatgtagcag ctaaaaatcc tgtcatatct 1200960
aaaacagtcc cccctccaat tccaataatt gaagatttcg gagagatatt ctgatctaca 1201020
agctgatatt gtagagatat gaatgtttcc caggttttgt taggttctcc aggaggaaag 1201080
gttaggacaa tgacttgata tcctaacatt ttaatatggt ctaagatagg acctaggaga 1201140
tgctgttgta ctgaaacgtc tgtaataatg actaagggat acgctgtgga tatagaagag 1201200

aacaacttct tctggaagaa gttggatatg agtttcacta catggggagt cgtaattatt 1201260
gtctctgaca tgatagtttg gagcatcgtt ggtataatac aagatctgca agaacaaggt 1201320
ttatcatagc ctcaacaaca gggacagcgc gtatggcgac acaaggatca tgacgtcctg 1201380
tttgaggtgt cctataggta gtttcttttt ttgtctttgt cactgtagca cagggtcgct 1201440
ttatcgaaga ggtaggttta aatgctatgc gcccttctat aggaactcct atagttatgc 1201500
ctcctagtgt gcctccacag ttgttagact tcaaggtaat gttttctcct tccatgacga 1201560
agggatcagt atattgtgaa cctctcattt gagcagaggc aaaccctttt cctatttcga 1201620
atcctttagc ggcggggatg ctcattaaag cacttgctaa gagggcgtgc actttcccaa 1201680
agaggggttc ccctagaaag tcgtggattg gagacgttat gaaagaaatc accccaccta 1201740
gagaatcaga gtcgtcgtgt agagaagtaa ggatctcttg gattttctca ttaggtaacg 1201800
gtgaatagaa tggcgaggtg tgaatcttgt ggatgagctc gggggagatc ttcaggtagt 1201860
gagggagggt tagagatcct aacgaggaga ggtaggctaa agtaaaaatg ttttgattcg 1201920
cgaggaattt ctctgcaact acgccagcag cgacgcgaca tgccgtctct cgagctgagg 1201980
agcgacctcc tccgttagga tctacaattc cgaatttctt ttcataggta tattgggagt 1202040
ggccaggacg gtagagcctt tcactgtttt cataagggga gctatctacg tcagtattga 1202100
ggatttgcag ggatagggga gtgcctgtgg tctttccttt ataaactcca gagaggattt 1202160
gcacgatatc gttttctttg cgcgatgagg ttcctggatt tcctggacga cggcgcttca 1202220
tggcaggaac aaaatctgat tcatggagct cgagtcctgc gggacaacca tcgattacaa 1202280
ctccgattga gggaccgtgg gattctcccc atgttgtaaa agaaaacaaa gagccaaagc 1202340
tatttttcat gactttaaca aggtaatgag atcttgagac gcttgttcta aggagctctt 1202400
cgaggaatga tctacgtgat ccacgggaaa aatgtaatca gcaatttctt tcattctgtc 1202460
gatgcgctca gttaaaatct cgcttagagg ttttgttttc atggcttctt tcagccgttc 1202520
tggtaaccct cgtttctcga gtctttcata aattaaaggt agttctacag agagaaatac 1202580
aagagctccc ctagtttgaa tcgctcggta agaggcttca tacatcaagg tccctccacc 1202640
aagggagatc aaggcatctt ctggaggtaa agtttcaaga atacgagctt cacattcact 1202700
gaatttctga tccccataag ctttatagat ctctgcagat gaggagtata gtgcactgct 1202760
gtagttgctt acaattaaat catcaagatc ataaaaagga aggtttagaa atttagccaa 1202820
agctttcccg agcgaagact ttccgcttgt gggtagacca catagaataa ttgtcatgac 1202880
attcctcaat tcgagcttcc attatattta gagtctgaac gaagttagga aaggttttcc 1202940
taacgcacgc agtgttgtga atacgactat cgccagaagc atatagagct gctatggtca 1203000
aggccatggc aattctatga tcatcgtgag agtccaaaac agcaccgtag aggggactgg 1203060
gattcaccag taagccatca tgagttggct gtatgcatgc tcccattttc tgtagttctt 1203120
cggtgattgc aagaatgcga tcgctttcct tgtctttaga actgcgtgca ttgtagaggt 1203180
gcgaagggga atctgcaaaa caacaaagga ctgtgagaat gggaagagca tcgatacaac 1203240
catccatatc tatagagcca ccagagaacg acgaagggaa tactaagatc tcttcgttgt 1203300
cgtactgtat agaggctcct aagttttgca tgagagaaaa aaagattttg tctccttgga 1203360
tatctaagat gtccaagtta cgaagacgta tcggttggag tgattttgaa agtagagcag 1203420
ctgcagcaat gaaagctgcg ctgctgaaat caccagttac gtggtaagag aaaccttgag 1203480
gatgtgaact tcccgggaaa gaataggtag tatcagaaca agaataggga agatggagtt 1203540
tctctaacca ccagagacta agatcaaacc atggacgctc tttcggttct atgatagtaa 1203600
aggaacaggg accttcggct aacgaacatg ctacggctag tgctgaggca aattgagaat 1203660
cactaccttc aacatcgcta taagcagagc gtaacggtcc tgacattgtg aaaggtagaa 1203720
cgcttttatc tgaagagaaa tgaaacgaag ctccaaagtt tctcaaggct tgaagtagag 1203780
gggccatagg acgccgttgc aactgtgacg atcctgtgac ggtgatttct ttagagaaga 1203840
cacaggcaag ggcagtcata aaacgcaaca cgataccaga atttccagcg tctatgagag 1203900
tatattttgg aaatatagcc aagggattgc ccacaatttc caaaatttga gggaatttct 1203960
ttatagaagc tcccatctgc ttgcaagcac aaatcatagc ctcagtatca ggagaatcta 1204020
aataattata aattgtagat tttccctcgg caacagaagc ccatagaatg gctcttagag 1204080
tatgggactt tgaggaagga atgaaggcgt tcccatacac agaagaaggt gaaactttat 1204140
aagtaagcat tgtaaagcaa acttatctct ttttttttag gtaatttgcc tgacttctga 1204200
tttgtcgtct aagaaaaatt ctagaacttt gtaaaagtcg aggaaggatc gaagatcatt 1204260
tgttctaatc tctctacaaa ctgcaagcaa agctcccctt cgaattggca agcatagcct 1204320
aagagttccc tccccgtggg ggagatcttt aagttcacgt tccatctctt gcagatggcc 1204380
ttgctcttct aagataatgg attttaccgt tattttactt tgagcttctt tcagaatatc 1204440
gtgatacaga ggataaagtt cagaagcacg aagttcgatt gcgtaggtaa ctaaaatata 1204500
cgctgcagtt tttaacgttt gtcccgatag ggagtattca ttttccagta ctcggcacgt 1204560
ccttaaatct agaagatgga ggtaatattt tgtaagtaag cctcccagaa gatttttaga 1204620
tgtatagtca gggagagaag tctctgagat tctagaaatc tgagttttta gatagtgacc 1204680
atgacgaaat tcttcagcag catgttttaa aacttcttcc tttacctccg taggatgttc 1204740
acttgcggag attttttttg ctccactatt ttctaaaaag gacagggtat ttatccattg 1204800
tgcatgccaa tagttggaag ctacgatctc tttgagaacg ggctcccaac gagaggctgt 1204860
gtggataaag tgttttactg tggttgtaga catagggcat cctgtaggtg agaataaata 1204920
atccggagat cttcttcttg gatacagtag gggggcagca catacagtgt gttccctaaa 1204980
ggacgaagaa ggactccacg ttctaagaaa aagcgattga gatggtctct atattgtgaa 1205040

aaatatcctg tagcttctgc agggtaatct agagcgagta ccgtgcccag aacctcacac 1205100
cgttgccata gggaaccatg agcttcttga aactcttgat gacaccgttc tatcatttgc 1205160
ctttgttgta ggcattctgg agatagggtg agatccaaag aagcgagggc agcactacag 1205220
cctaaaggat ttcctgtgaa ggtatggcca tgaagcagtg ccttcatccg atcttgggag 1205280
acaaaggcat catgaatttc tttagtggtt actgtcaagg ctagagggag atagcctcct 1205340
gtaagacctt tagaaagaca gataatgtca ggaggaatgt ctgtaaattc agaagcaaac 1205400
agtggacccg tacggccaaa gccagtaaga atttcatcag caatacagag aaccccgtaa 1205460
tgcttggcaa gcttgagaat ctcctttagg ccttcgggat tatacattaa catccctcca 1205520
gcaccttgca atagcggctc atagataaac gctgcgatat tgctttcaga aaagactgtt 1205580
tttgcttggg caatggcaag ctcttccttg ccataatagg gagcagcaat tgtactggaa 1205640
ggaagaaaaa gatcatgaaa gggaactgta gtagggctcg tgccagctat cgacatagct 1205700
ccaaatgtat ctccgtgata ggcattgctg agtccaacaa aatggctctt agccttgttt 1205760
tgattgtagt aatattgcac agcaattttc attgctattt cgatagacgt tgatccgttg 1205820
tcagagaaaa agaaacgttc tagaccttca ggaaggaggg gagcgagttt cgatacgagc 1205880
tctagagccg gttcatgggt gaaatttgcg aagatcacat gttctaactt ctgtgcttgc 1205940
tcacataatt tttttgtaat gtagggatgc ccatgaccgt ggaggttgca ccaccatgaa 1206000
gatatcgcat caagatatct tgttcctgat tccgcataga ggtaagcacc ttctcccctt 1206060
acaatcttta tgggtgtaga atctaatgca gattgagtga aggggtgcca gatacaccct 1206120
gaattccctg atgattgctt gtccatactt ccttccattg ttcggcataa cagcttatga 1206180
ttgtctttgt gatttctttt tccttggcaa gagtcccgat tataggaagc ttgatttctt 1206240
gagttagcca gtgctcttcg tcctctggat acccatttac caccatacct aagatattga 1206300
ggtttcgtga gcgcattgct tctaccgtta aacaggtgtg attgatactt ccgagatatg 1206360
cttggctcac taaaatccaa gaacatgacc aagaagaaaa cacatctccc tgaagtcttt 1206420
ttgatgtgca gggggataaa aatcctcctg aagtctcaat aatcagattc gaagttgttt 1206480
ttggcgcaca aatatgactc tcttcgatac ttacattatc gatttgcgct gccttgtgtg 1206540
gagacaaggg cttatgcaat cgataagctt cgggatgaca gtaggctccc gatagctcat 1206600
gaacaatatt gctatctgaa ttttctagat tccctgcttg tataggtttc cagtattctg 1206660
cgttaagtgc tctagcaagg atagcactga caatggtttt tcctacgcca gtgtcgattc 1206720
ctacaatgat gatacgttgc ataaaaaaat ttgctctaaa gtatgtaaaa ggtgatctat 1206780
ttcatttttt gtattgaagg catgaaggca aatacgtaac agttcttctc tttgttttac 1206840
tgttgggctc acgatagggc gaacatcgta ccctgagttt tgtatttgca atgcggcttg 1206900
ccgagcacga tggctccctg aaacacaaat agattgtata ggtgtagtag tattgtcttt 1206960
cattagctga agtcccaagt tttgagcttt ttcgcgaaaa tgatgaatga gagcagaaag 1207020
atgttcacgt tgattaaaag cacgttggtt atgctcataa gcgagctcta tagctgtgag 1207080
cgcatgggga ggttgggctg tggtgtatat gaaaggtcta caaaaattaa ttaggtagtc 1207140
tttgagtata gaacttcctg caatagcagc gccatgagtg cctagagctt ttccaaaagt 1207200
atagacggtg gctaggactt tatcttgaag acctaatgca gagacaaggc cttcgccctg 1207260
atctccaaaa actccgactg catgggcttc gtcaacgatc aggtaagcgg aatatctttc 1207320
acagagttca ctgatagctt gtaaaggagc cacggagcca tgaagcgagt atacggattc 1207380
cacgcaaaca aaggttcttc ctagatggga gcttgctaaa cgtttttcta agtgattcag 1207440
atcattgtga ttaaagggaa agctttgagc tttgcttaat cggatgccat cataaataga 1207500
agcatggata taaaggtcgt gaagaatacg atcctggtcg gtggcaagag catagagcaa 1207560
gcctaagttt gctgtgtaac ctgtattgaa gatcaaacag ctttcaaaat tatgataagc 1207620
ggctagttgt tcttctatac gctgacagag ttgcgagtgc cctgtgagaa gccgagaccc 1207680
tgtagctcca agcgattcta tcgcatggag cttggtaatg tattctttcc ttaattctgg 1207740
agaagaggca aatccaagat aatcattaga ggtaaaatca atgagatgag agtttaagct 1207800
tagcgagcgg taggtatgtt tggacttacg tcttgccaaa gcctcaatta ggaattgttg 1207860
gcataacatg ggttacctct ttctattcca aatgaagggc gagggattaa gcctaaaagt 1207920
ttgatcattt cagcatcttc atctatatca ttgttttcta cagtcaacag tttatctcca 1207980
tagaatatgg agttggcacc ggctagaaaa cataaggttt gttgttctac tgtgaggaaa 1208040
gcgcgtcctg cagcaagtcg taccatggat ctggggaaaa caacccgtgc cgttgctatg 1208100
gttcgcaaga cttcccagaa agaaatcgga ggctggtctt gcaaaggcgt gccgtcaatc 1208160
ggccaaagta aatttacagg tacggattct gggatatgat ctcttgttgc aagaacatga 1208220
agaagcttta tacggtcttc ttcagattct cccataccta caataccacc gcagcatgta 1208280
ctaatgccag atttatttac tacatcaaga gtgttgaggc gatcttcata agaacgtgtt 1208340
gtgattatag tttcatagaa ttccggagaa gagtctaaat tatgattgta ggcataaagt 1208400
cctgcatcat acagtttttt agcttgctct tcggagagca tgcctaaagc acaacaaacc 1208460
tcggctccga gatctgtgat acttttcacc atagcgagga ctctatcaaa gtatcgatcg 1208520
tccttagcat tgcgccaggc agccccaaga cacacacgag tggcgcctag ctctacagca 1208580
cgttttgccc tttccacaac gtctacaatt ttcatcatag gttctggtgt gacgtgggta 1208640
tgatagcggg aagattgggc acagtaggcg caatcttcaa cgcatccacc agttttaatc 1208700
gaaatcagat agcaagtctg cagttctgaa tggaggaaat tactacgcaa tatggcattg 1208760
gctttgtgaa tcagctcaaa tacgggagtg tgataaattt cgcggatgtc ttctaatgac 1208820
caggatacag tttcttcacg catgtgtttt gcctaggggg taaattcaac gaccgagcct 1208880

aagatattta catggaattt agagaaagtc aataggaacc aatttttggt tcctatnttt 1208940
tatggtatga tttgagaatt attctaataa aataagagaa attacgtttt ttaataaagc 1209000
agctctattt ttaaaaaatt agaaataatc atgaattatt tcttgttgtc taaggttgat 1209060
ttatcagagg aactgtattt gcaattttag tagaaggcat ccctaaaaac ttgcggattg 1209120
tattcacagc gagataaaag agcggtgtcg tgagaacaca aaaagtaatt ttataaatgt 1209180
aagagtagaa cataatattt agggtttggg ggaaagaaag ccccatgccg aaatataaaa 1209240
tacaggtgtc gacaatgaaa gtatctggna tttgtgagat ccatgtagac ccattgctac 1209300
gtagccatag agatgaattg ggagttcgat ttttaaaaaa agtgtaaagg acgatgtcta 1209360
attgctgaga gacaatgaaa gcnagtagtg aagctagaaa acggagagga cttaaatcaa 1209420
acaagcagtg ccaggcagtt tgcatctcag gagaggctac tgggaagaac ataaaaattt 1209480
gtacaattga agaagctaag aggttggcga taaaagctga aaatatcatg acacgagctt 1209540
ttttagggcc aaagatttcg ttaacaacgt ctgagatgag aaacgtcagg ggataaagaa 1209600
tgagtccccc aggaataatg aagttaaaaa aagtcgttgg aatgagcttc gaagagagga 1209660
caagattgga gagaacaaga agcaaagtaa aggttgaaga caagtaggaa aatactaatg 1209720
tttttctgtg ggatgtattc attggcaaag tacctcaaaa ccagaaagat atttctcttg 1209780
attgtaaagg ggaattgatt ctaacagacc ttggtcgagc atccattcta attttgaagt 1209840
gagttctacc agttcatcaa aatgtcttat tgaaaataaa gtctcttgtg gagttgatgt 1209900
attgaaggga agacgaataa tctgatccaa ttctaaaggg agaacacgta cgttatcaat 1209960
gaaagcgtgt ccaagttcct gaggagaact gataagaacg gctccatatg cttttcttcc 1210020
ttcatggttt tcaataagtc cgctttcaac agtaaaccaa aagcagcgta caatagcgat 1210080
cagattgctt tgtagggttt gtatgcgttg ttttttacta ggaagagctt gtactttttc 1210140
tatgacttta gtgaagagtc ttcccatgtt tatgaaaaat tcagaaaatg agggatgtag 1210200
aagccaaggc acgtgcccta aaaggtcatg gatgagatca ggagttaagg agaaattatc 1210260
tttatcgaga gttcgcatta cagaggcaat gggaaagtaa cggtcctgca acagagacaa 1210320
gtattgatgg ggagctacaa atcctgaaac gggataatag gaaaaatgtg tttctaattc 1210380
gaagaattta atgactgctt gatggtctaa gaaatcagaa aggagaccga aagcttctaa 1210440
gtaatcaaga aaaatcttgg acaatagctt ttccatagag aaaaccgtga ggaaaggagt 1210500
cgataccata aattccgatt aaaaaaggga gtgcttacaa cagccatatc agtggagcaa 1210560
ccttcccttt gattctttga taatgacaga agatgaacaa agagtaagtt tttgaaaaat 1210620
tctaaaatag aaatgcattt gtgtcgagct aaagcttgct tctctttatt ttccttttgt 1210680
agaatgattc ggtagtagga atatggggtc gagtatgcac gttggagtga ttggctgttc 1210740
tggaagaaca gggaaagtga ttgtctcagc tttagagcaa tcttcagaat atactttggg 1210800
tccagggttc tctcgcagta gtgcactcac tctttttcaa gtgattgcac ataacgatgt 1210860
tcttgttgat ttttcccacc ccttactaac taaagaagtc gttgcccatc tcttaatctc 1210920
tcctaaaccc ctcattatcg gaactacagg gttccctggg aagtgtaaag aagctcatga 1210980
cagtctcgaa gagctgactc acatcgttcc tgtagtggtt tgtccaaatg caagtctagg 1211040
agcttatata cacaagcggc ttgtaatgtt gctatcgcaa ttatgtaacc cacagtttga 1211100
tattcgcatt cgcgagactc atcatagata taagaaggac tctctctcag gaacagctca 1211160
agatttactt gatactatac agcaagtaaa acaagaggat tggggagagg agtatgaggt 1211220
aggccagcga gattcttcta agaagacgat agaagtgcaa tcttcacgag ttggagatat 1211280
ccctggagaa catgaggtcg cttttatcag ttctggagag caaattttgg ttcgccacac 1211340
agtattttct cgcaatgttt ttggtcgggg aatcctttct attttagatt ggctaaaaac 1211400
attgaatccc caacctggtt tgtatagtct tggggatact ttggagctag tcttacgaaa 1211460
tgaacattgc ttgcttaaaa agacaacgga tcattaatat gcagatataa agatcttttg 1211520
cgtcgtaaaa tcctgatctt caaattagga atgtagggtg ccgcaattac acaacttcgt 1211580
gtacaaaatt ttaagcgagg actgttttga tttttagcat cgataggacc atcggttaaa 1211640
ttaggaagtg ttgacaggca attcaaaagg aatcattgca tgtctgtaaa cactagagat 1211700
agggttatcg tgcattccaa tctgaagtaa ataattgaga tggagaaagg aaaggaatgc 1211760
gaatcgctgt tttaggcgtt actggacttg ttggccagaa attcgtagct ttgcttcata 1211820
agtggtatcg tgattgggtc attgctgaag tcgtagcttc aaatagtaaa tatgggcaat 1211880
cgtatggaga tgcctgtatt tggcaggagc aatcggccct atgcctgaga tggtacgtga 1211940
tttgcctata cgtaagatcg aggaagtgca gtccgacatc gttgtttctt ttttgccttc 1212000
tagtgctgag tccatggagg cgtattgcct ctctcaagga aaagttgtct tttctaatgc 1212060
ctcaacatat cgcatgcatt cttcagttcc tattatcatc cctgaagtca actcagatca 1212120
ttttcaactt ttagaagagc aaccttatcc tgggaaaata atcacaagtc ccaattgttg 1212180
tgtctctgga attactttgg ctctagcacc cttaagaaag ttctccttag atcacgtaca 1212240
cattgtcacc ttacaatcag caagtggtgc aggctatcca ggagtcccgt cgctagatct 1212300
tcttgccaat acagttcctc atatcgttgg agaagaagaa aaaattctta gagagactgt 1212360
gaagatttta ggaagttcta agcaaccttt accttgtaag ctctctgtga cagtccatcg 1212420
agttcccgta gcctacggtc atacgctctc tctgcatgtt accttttcta aagatgtaga 1212480
tcttgacgag attctgtact cttaccaaga aaaaaataaa gaattcccca atacctacca 1212540
actctatgat aacccttggt ctcctcaggc tcgaaagcat ctttctcacg acgatatgcg 1212600
cgtacaccta ggacccatta cctacggagg agattttcgc accataaaga tgaatgtttt 1212660
aatacataat ttggtgcgag gtgctgcagg gactttactc gcaagtatgg agaattattt 1212720

tttcgactat cttaagaggg aaatgtgtct aagatagttt ataaatttgg tggcactagc 1212780
ttagcaactg ctgagaatat ctgtttggtt tgtgatatca tttgcaaaga taagccttct 1212840
tttgttgttg taagcgcaat cgccggtgtg acggacctcc ttgtagactt ctgctcgtct 1212900
tctttaagag aacgagagga ggtcttaaga aaaatagagg gaaacatgaa ggagattgta 1212960
aaaaacctag cgattccttt tcctgtctct acatggacgt ctcgactcct tccctatcta 1213020
caacatctgg agatctcaga tctcgatttt gctcgtattt tgtctttagg agaagatatt 1213080
tcagcttccc tagttcgtgc tgtttgtagc acccgtggtt gggatttagg atttctcgag 1213140
gcacgtagtg tcatcttaac agacgatagc taccgacgtg cctctccaaa cctagatctt 1213200
atgaaagcac attggcatca gctcgaacta aatcagcctt cgtatattat ccaggggttc 1213260
atcggatcta atggtttggg agaaacagtt cttcttgggc gcggaggcag tgattattca 1213320
gcaactttga tcgcagagct tgcaagagca acagaagtgc gtatttatac cgatgttaat 1213380
gggatctata ccatggatcc taaagtgatt tccgatgcac agcgcattcc tgagctcagt 1213440
ttcgaagaga tgcagaattt agcaagtttt ggtgctaaag tcctttatcc tcctatgctc 1213500
tttccttgta tgcgtgcggg aattcctatt tttgtgacat caacatttga ccctgaaaaa 1213560
ggaggaacat gggtctatgc tgtcgataag tctgtgagtt atgaaccaag aataaaagct 1213620
ttatccttaa gtcaatacca aagcttctgt tctgtagact atactgtcct aggatgtggg 1213680
ggattagaag agattttagg cattttagaa tcccatggga tagatcctga attgatgata 1213740
gcacaaaaca acgtcgttgg atttgtaatg gatgatgata tcatttctca agaagctcaa 1213800
gagcaccttg tagatgtttt atcgctatct agtgtcacac gcttgcatca tagtgttgca 1213860
ttgattacca tgatcggaga taatctttct tctccaaaag ttgtctcaac aattacggag 1213920
aaactcagag gttttcaagg acctgtattt tgtttttgcc aaagttcaat ggcattaagc 1213980
tttgttgttg cctcagagtt ggcagagggt attatagaag aattacataa tgattatgta 1214040
aaacaaaaag ctatagtcgc cacgtaggga gaatcatgca tttacttaca gcaactgtta 1214100
cacctttttt ccctaatggc actatagatt ttgctagctt agaacgtctt ttatcttttc 1214160
aagatgcagt tgggaatgga gtggttcttt tagggagcac tggagaaggc ctttcgctaa 1214220
caaaaaagga aaaacaagca ctgatttgct ttgcttgtga tctacagctt aaagttcctc 1214280
tttttgttgg gacttcggga acccttctag aggaagtctt agattggatt catttttgca 1214340
atgacttgcc tatctcggga ttcttaatga caactcccat ctatacgaag cctaaactat 1214400
gcggtcaaat cttatggttc gaagcagtac tcaatgctgc aaaacatccc gcgattcttt 1214460
ataacattcc atctcgtgca gccacgccct tatatttaga tactgtaaaa gccttggctc 1214520
atcatccaca atttttggga atcaaagatt ctggaggctc tgtagaagag ttccagagtt 1214580
ataagagcat cgctcctcat atccaacttt attgtggaga cgatgttttt tggagcgaga 1214640
tggcggcatg tggagcccat ggtttaatct ctgtcttatc gaatgcttgg cctgaagaag 1214700
cccgagagta tgtgctgaac cctcaagaac aagactatcg ttctttatgg atggagacgt 1214760
gtcggtgggt atataccaca acaaatccta ttggaattaa agcgatcctt gcctacaaaa 1214820
aagctattac tcacgctcac tgcgcttgcc cttgtctata gaagattttg atttggagaa 1214880
tgtatctcct gctgtagaaa gtatgcttgc ctggccgaag ttacgtacct ctgtgttctc 1214940
ctattcttaa tgcagcaaaa ccttatcttc tgcatctatc ttgttctaac ttattacttt 1215000
acaaataaaa tttttgcaga ggttttttct agagaatagt gcttttgttc tggattttta 1215060
acaagatgcg tcgcattttt ttcttctttt tacatcgctt atgatttttt atgactccga 1215120
aatcgattca acaattgcac ttaataaaga ctattgatcc agtaagaaaa atttctcctg 1215180
taacaacaaa aaaatcttcg ttctttagac agtctcttct tagattttta gaactatttt 1215240
ggatgtttct ttactgtata cgctctatta gattccattg tgttcatatc gccaccttta 1215300
tctgtcgagg tcttattctt tttctgacta ccttgttctt atctatgata tgcatcttgc 1215360
atttcattac tcttccttgg atctgtaagg aagaccctcg gattataaga aaaaataaat 1215420
aatcttgttt catcaccaag aatcaggtca ttttgtcttc atagtttctg attttttttc 1215480
catttttgtc gccatattca agattttcaa aatcgtgctt cagctatact gaggagttta 1215540
ttttgaataa tttttcattc aaaaaatgaa atataattca agagaaaaaa taaaaagcgc 1215600
tttacggatt tgctcttcat attgtatcac tgtctttcga aataatttca gtttaagttg 1215660
ttatgataaa attttctatt cgttgtcttg ttatgttttc aatggcccaa acagtattgg 1215720
gcgctgccga agtttttgtt tcttcagagg aaaaaaaact gaagtagaaa ctaaagaagt 1215780
gaaaatcaaa gatgagattc gtccttcatt ggaaggaaat gatcctgtaa aagttgcgga 1215840
gtcattccct aaaagaagag cagctcttga gtctttaagt tctcaaagtt ccatcggcaa 1215900
tctttgtgca attagtaatt tcttagattc tcaaatgtta tctagaaatt tctcaaaaga 1215960
gatctgggga tctacaatct tcacacgatc taaatctaca tgtgatgccg aaggatctga 1216020
gcctttccgt tatactgcat gtggatacct agctggtctt agatctaaac tagcaggttc 1216080
ttatgaatta ggtgtaactg caggattgct acagggacgc cttaaagatg tttcagatag 1216140
ccatagaact cgtgcgacat cttcaatatt gtccgtgcat ggtagtatgg ttacaagacc 1216200
gttgagttgt acgaagtata tcgtaggaaa agcgcgtcct ttacttttct tcttcagatt 1216260
aacatcggat gtaagaagag atttgaaaaa aaagttcaga cttgaatttt gcaaagattg 1216320
atcacaatca tctctacctt acatgtttgg gagatcttgg tgtagcttgt cctatacttt 1216380
ctacagattg tctacctaat tatagcgaga aagcatctca tgaggttctt gtttatagta 1216440
aatttagatg catttctgga gagccatctc gacttgcaac ttcaggaaat gacacatatt 1216500
attctatagt aagtttacct ataggactcc gttacgagtg acttcaccat caggacgtca 1216560

tgatttcaat attgatatgc atgtagctcc aaagataggt gcagtactct ctcatggaac 1216620
acgagaggct aaagagatcc caggatcttc aaaagactat gcatttttta gcttgactgc 1216680
tagagaaagt ttaatgattt ctgaaaagct tgcgatgact ttccaagtta gcgaagttat 1216740
tcagaattgt tattcacaat gtactaaagt aacgaaaact aatttaaaag aacagtatag 1216800
gcacttatcc cacaatacag ggtttgagtt aagcgtcaag tctgcattct aaaaatagtt 1216860
cttgttctct tggacttgaa aaagccattc ctatttgaaa aaatgggaat ggcttttttt 1216920
ataataggtc gaaccaaaga ggaacttgtt tcattaaaat tacttaaaaa gtgatatcgt 1216980
tttctaaata caaagataaa aatactcacc aatatactcg cacaaccttg acaaaggaag 1217040
ttttcttgaa gatctttttc ggtttttata agaaaattct tctttagaag gaagatgaat 1217100
ctctttgaat aaaatactat atattagtag cttagtgggt ttaatttatg tgtttgatcg 1217160
cgatggcacc acagattcat aatgcaagta cttctatctc cgcagctacc ccccccccca 1217220
acactctgta gggtcgattt cttctccatc taaacttcgc gttttagcga ttactttttt 1217280
agtttttggt atgctcttac tgatttcagg agctctcttt ctgacgttag ggattccagg 1217340
attgagtgca gcaatttctt ttggattagg catcggtctc tccgcattag gaggagtgct 1217400
gatgatttcg ggactactat gtcttttagt aaaacgagag attccgacag tacgaccaga 1217460
agaaattcct gaaggggttt cgctggctcc ttctgaggag ccagctctac aggcagctca 1217520
gaagacttta gctcagctgc ctaaggaatt ggatcagtta gatacagata ttcaggaagt 1217580
gttcgcatgt ttaagaaagc tgaaagattc taagtatgaa agtcgaagtt ttttaaacga 1217640
tgctaagaag gagcttcgag tttttgactt tgtggttgag gataccctct cggagatttt 1217700
cgagttgcgg cagattgtgg ctcaagaggg atgggattta aactttttga tcaatggggg 1217760
acgaagcctc atgatgactg cagaatctga atcgcttgat ttgtttcatg tatcgaagcg 1217820
gctagggtat ttaccttctg gggatgttcg aggggagggg ttaaagaaat ctgcgaagga 1217880
gatagtcgct cgtttgatga gcttgcattg cgagattcac aaggtggcgg tagcgtttga 1217940
taggaattcc tatgcgatgg cagaaaaggc gtttgcgaaa gcgttgggag ctttagaaga 1218000
gagtgtgtat cggagtctga cgcagagtta tagagataaa tttttggaga gtgagagggc 1218060
gaagatccca tggaatgggc atataacctg gttaagagat gatgccaaga gtgggtgtgc 1218120
tgaaaagaag cttcgggatg ccgaggaacg ttggaagaaa tttaggaaag cagtcttttg 1218180
ggtagaagaa gacgggggct ttgacatcaa taatctcctt ggagactggg ggacagtgct 1218240
tgatccttat agacaagaga gaatggacga gataacgttc catgagttgt atgaaaaaac 1218300
tacgtttttg aaaagactgc acagaaagtg tgcgttagcg aaaacaacct ttgaaaagaa 1218360
nagatctaaa aagaatttgc aggcagtcna ggaggcgaat gcacgtaggt tgaaatatgt 1218420
aagggattgg tatgatcagg antttcagaa agcaggggag agattagaga aactgcatgc 1218480
tttgtatcct gaggtttcag tctctataag agagaacaaa atacaagaga cgcgctctaa 1218540
tttanagaaa gcctatgagg ctatcgaana gaactatcgt tgctgtgtcc gagagcaaga 1218600
ggactactgg aaagaagaag agaaaaggga agcgganttt agggagaggg gaaacnagat 1218660
tctttctcct gaggagctgg aaanttcttt ggagcaattc gaccatggtt tgaaaaattt 1218720
ttctgagaaa ttaatggaat tggaagggca tatcttaaaa cttcagaaag aagccacagc 1218780
agaggtggag aataaaatac tttcagatgc agagagccgc cttgagattg tatttgaaga 1218840
tgtcaaggag atgccctgtc gaattgagga gatagagaag acgctgcgta tggcgganct 1218900
gcccctactt cctacgaaga aggcgtttga gaaggcctgc tcacaatata atagctgcgc 1218960
agagatgttg gagaaggtga agccttactg caaggagagc ctcgcctatg tgactagcaa 1219020
agagcgttta gtgagcttgg atgaagattt acgacgagcc tacacagagt gtcagaagag 1219080
attccagggg gattcgggtt tggagtcgga agtaagagcc tgtcgagagc aactgcgaga 1219140
gcggatccaa gagtttgaaa ctcaagggct ggacttggtg gaaaaagagt tgctttgtgt 1219200
gagtagtaga ttaagaaata cagagtgcga ttgtgtatct ggtgttaaga aagaagcacc 1219260
tcctggtaag aagttttatg cccagtatta tgatgagatt tatcgagtta gagttcaatc 1219320
ccgatggatg acgatgtctg agagattgag agagggagtt caagcatgca acaagatgtt 1219380
gaaggcaggc ctaagcgaag aagataaggt tcttaaagaa gaagagtatt ggttgtatcg 1219440
agaggagaga aagaataaag agaaacgttt ggttggtact aagatagtag caacgcagca 1219500
gcgagttgca gcatttgaat ccatagaagt tcctgagatt cctgaggccc cagaggagaa 1219560
accgagtttg ctggataaag cgcgttcttt atttactcgc gaggaccata cctagcataa 1219620
ctccaagagt tgtgtttctt taaaaattct ttgaataaaa tactatatgt tagtagctta 1219680
gtgggtttaa cttatgtgtt tgagcatgat ggcgccacag attcataatg caagtacctc 1219740
tatcaccaca gctacccccc ccccccccag accactctgt aggggctttt ttttgtctgt 1219800
ctaaatttcg tgttttagca atcacttttt tagttcttgg tgtgcttttc ttgatttcag 1219860
gagctctctt tctgacgttg gggatttcag gattgagtgc agcaatttct tttggattag 1219920
gcataggtct ctctgcgtta ggaggagtgc ttgttgtctc aggacttcta tgtcttttag 1219980
caaaacgaga ggttccgaca gtacgaccag aagaaattcc tgaaggggtt tctgtggctc 1220040
cttctgaaga gccagctcta caggcaactc agaagacttt agctcagctg cctaaggaat 1220100
tggatcagtt agataggtat attcaggaag tggtctcatg tttaggaaag ctaaaagatc 1220160
ttaggtgtga agatcaaggt cttttaaaag atgctaagga gaaacttcaa gtttttgact 1220220
ttgtctggaa agacatgatg accgagtttg tagagctaca acagatcatg gatcaagagg 1220280
ggtggtatct aaagtgcctg attcaggaga tgcgggatat aggaagcaca ctttttatga 1220340
gtcaggttag tttatttaaa ttatgggaat ggcttgggta tttaccttct ggggatgttc 1220400

gaggggagcg gttaaagaaa tctgctcgtg aggttgtgga tcgctttatg agaaggattt 1220460
gcgatacgcg gaaggtggcc atgacttttg ataggaatgc ctatggagtg gcgaagacgg 1220520
cctttgaaaa ggcttttgga gctctggaaa cgtgtgtata taagagtatg acagagagtt 1220580
atagagaggc cttttgtgag tataagaaga cgaagatcct tagggatgag gagaagatat 1220640
taaggatatg ttatctcgag ttgaggagat agagatgatg cttcgtgtca tagagcttcc 1220700
actacttcct ataaagcaag cgttggagaa ggcttttgta caatataata gctacaaagc 1220760
gaagttaacc aaggtagaac cttgctttag agagagccct gcctatataa ctagcgaaga 1220820
gcgactcnag agtttggatc agactttaga acgtgcgtac aaagagtacc agaagagatt 1220880
ccaggagcct tcacgtttgg aatcggaagt aagtggatgt agagagcatc ttagagagca 1220940
ggtaaaacaa tttgaaactc aaggactaga cttgatcaaa gaagagctta tttttgttag 1221000
tgatgtgtta ttccgaaaaa tggtcagttg tctagtgtcg acagtgcatg ttccctttat 1221060
ggagttttat tatgagtatt ttgagttgca tagattgagg ttgcgggccc aatggatggc 1221120
gaatgccgag atttatagca aagttagaaa agcattccca gagatgttga aggagacctt 1221180
agaaaaagct aaggctccca gagaagaaga gtattggtta ctttgcgagg agagaaagag 1221240
taaggagaag cgtttgattc tcaacaagat agaggcagct cagcagcggg taaaagattt 1221300
agaacctcct cctattaaag agacagggaa acagaaacgg aagaaagaat attcgttttt 1221360
cattcgatta aaatcgtgat tcgaggcaac cctgcattct atagattttt tctcagagtt 1221420
ctagtgatct aagtacgaag aggaagaaag acttttgtat tttttgcatc ctagaatttt 1221480
ttgttttgca tagtattcgt tatagaagtg ttttaattat tactaactat tttatttata 1221540
aaatggttag ctcttctgtt catcataaga attgtaaaat gtgtattaaa agacgagaat 1221600
tttatttaaa ataaattata cacttttact tttttaattt tgctatgcct gagcctctat 1221660
atacgaataa gctaatcaca gagaaatctc catatttgct tctttatgct catacccctg 1221720
ttaattggta tccttgggga gctgaggcat ttcatattgc agctatcgag aataagcccg 1221780
tcttcttgtc tataggatgc aagcattctc gatggtgtca ggtgatgttg caggagagct 1221840
acacgaatcc tgaaattgct gctatgctca atgaatactt cgtgaatgta aaagtagata 1221900
aggaagaact tccctatgta gccaagctgt atggtgatct tgctcagatg cttgccgttt 1221960
ccggagatca ccaagaaact gtctcctggc ctttaaacgt ctttcttact cccgatctcg 1222020
ttcctttttt ctctgtaaac tatttgggga acgagggaaa actcggtctt ccctcattcc 1222080
cacaaattat tgataagctt cattttatgt gggaggatgc tgaggaaaga gaagctctcg 1222140
tcgaacaggc aatgagattt ctggaaatcg cgtcgttttt agaggggtgt gtaagaaaag 1222200
agatcttaga cgagagctct ctaaagcgaa ccgtagccgc gttataccaa gatatcgatc 1222260
cccattatgg aggagtgaaa gcatttccta aacgattgcc aggtttgctc ttacaatttt 1222320
tcctccgata cagcctcgaa tatcaggaga gccgaggctt attttttgta gatcgctctt 1222380
tgagtatggt ggctttggga ggagttcgag atcacattgg cgggggagtt tattcataca 1222440
ctatcgacga taagtggttg atccccgctt ttgaaaaacg gctgattgat aatgccttaa 1222500
tggctttaaa ttatttagaa gcttgggcat gtctgggcaa agaagaatac cgtggtatag 1222560
gaaaacagat actttcctat atcttgagtg aattatactc tccagaggtg ggtgcctttt 1222620
atagctctga gcaagcagaa aattggggag caggaggaca aaatttttat acatggtctg 1222680
tcgaagaaat ctccaatgct ttgggagaag atgctgagat tttctgtgat tactacggga 1222740
tttctaggga gggcttcttc aatggcagga atatactcca tattcctgta catagagaaa 1222800
tagaagagct gtcagaaaaa tatcatcgtt ctatagaggc tattgaagat atcgtagata 1222860
gatctcgaga tattttaaag gggattagag cccaacgttc ccatcgttct aaagatgatc 1222920
tatctttaac ttttaacaat ggctggatga tttatacttt tgcctacgca ggccgacttc 1222980
ttggagaggt tgagtatatt gagattgaga aaaagtgtgg ggaatttgtc cgaaattccc 1223040
tctataaaca ccatgaactt taccggagat ggagagaggg agaggcaaaa tatcgagcga 1223100
gtttagaaga ttatggtgct cttattttag gggtgctagc tctttatgaa tcaggatgtg 1223160
gatctttctg gttaagcttt gctgaagagc tgatgcaaga agtcgtcctt tccttccgtt 1223220
cagaagaagg aggtttttat agtgatgatg gtcgggatag caccttgctg atcaagcaat 1223280
ctcccctttc tgatggagag acgatctcag gaaacgctct aatctgtcaa tgcttgctct 1223340
cactgcacct aatcacagaa aaaaaacact acctgactta tgctgaagac atcttgcaga 1223400
tagctcaagc atgtgctcat acccataagt tctcttcctt aggattgctg attgcatcac 1223460
agaactattt ttctagaaaa catgtaaaag ttttaatccc tttaggggat caagaagatc 1223520
gcagtcctgt tttgaaatgt ctctcaggtt tattccttcc ttatctatct ctaatttgga 1223580
tgactcagga gaaccaagaa catttagaga ctgtgcttcc agaatatgag cactgtctta 1223640
tccctaaaag ggggattgca cagctacgac aatttatgtt ttagaagtcg atcaatgcaa 1223700
aagatttaaa gacttagaat tgtttcgtcg ttatctaatc tctctataga acgctatact 1223760
agttattgat aaagtgtttt ttactaatta aaatttattc tttttaggaa agaaaacacc 1223820
ttctctcagt ggtcaaaatt accacaattc cttatagtaa agtcccctgg gcaggtcaaa 1223880
atctataggg taggaagtca tgaaattata tcagaccttg cgaggtattg ttttagtaag 1223940
tacgggatgc atattcttag gaatgcacgg aggatatgcc gctgaggttc cagtgacttc 1224000
atctgggtat gagaatcttt tagaatctaa ggaacaggat ccttcaggtc tagcgatcca 1224060
cgatcgcatt ttgtttaagg tagatgaaga gaatgtagtg actgccttag atgtgatcca 1224120
taaattaaac ttactatttt ataattcgta tcctcatctt atagattctt tccctgcacg 1224180
atcccagtac tatactgcga tgtggcctgt ggttcttgaa tctgtgattg atgagttttt 1224240

gatggtggca gatgccaagg caaagagaat cgctacagat cccaccgcag tgaatcaaga 1224300
aatcgaagag atgttcggaa gagatctctc tcctttgtat gcgcattttg aaatgagtcc 1224360
caacgatatt tttaatgtga tcgatcgcac tttgacagca caaagggtga tgggtatgat 1224420
ggtgcgctct aaggtaatgt tgaaggtgac tccagggaaa attcgagaat attaccgaaa 1224480
gctagaagaa gaagcctcta ggaaagtcat ctggaagtat cgtgtgttga cgattaaagc 1224540
caacacagaa tccttggcta gccagattgc tgataaagtg cgtgctcgtc taaatgaagc 1224600
gaaaacctgg gataaagatc gtttaactgc tcttgtgatc tctcagggag ggcaactcgt 1224660
ctgctccgaa gagttttctc gagagaatag tgagctctcc caaagccata agcaagagct 1224720
ggacttgatt ggctatccta aagagctctg tgggttgcct aaggcacata agtcaggata 1224780
taaactctat atgttgttag acaaaacctc aggttctata gagcctttag atgttatgga 1224840
gtccaagatc aaacagcatc tttttgcttt agaagctgag agtgtagaga aacaatataa 1224900
agacagatta cgcaagcgct acggctatga tgcttctatg attgcgaaac ttctttctga 1224960
agaagctcca cctctatttt ccttattata ggggattgag tgacaagaag ttctcctgca 1225020
caactctccc ggtttctttc tgaaattcaa aataagccga agaaaagcct ctctcaaaat 1225080
ttcttagtgg atcagaatat tgtcaaaaaa attgtggcaa cctctgaggt gattcctcaa 1225140
gattgggtgt tggagatcgg cccaggattt ggagccctta cagaagaact catagctgca 1225200
ggagctcagg tcattgctat tgaaaaagac ccgatgtttg cgccatcttt agaagagctc 1225260
cctatccgct tagaaattat cgatgcctgt aaatacccgt tagatcagct tcaggaatat 1225320
aagactttgg gaaaaggtcg agtagtagca aatcttccct accatattac cactccattg 1225380
cttaccaagt tgtttctgga agccccagat ttctggaaaa cagtgacggt tatggtccaa 1225440
gatgaagtgg cccggcgtat tgtagcccag ccagggggta gggattatgg gtctctaacg 1225500
atctttttgc agttttttgc tgatatacac tatgctttta aagtttctgc ttcatgcttc 1225560
tatccaaaac cgcaagtgca atctgcagtc attcatatga aagtcaaaga aacacttcct 1225620
ctatcagacg aggagattcc tgtatttttt actttaacaa gaactgcatt tcaacagcgt 1225680
cgaaaagtac tcgctaatac tctaaaaggc ctctatccta aagagcaagt ggaacaagct 1225740
ttgaaggaat taggattact tctaaatgtg cgtcctgaag tattgagtct taatgattat 1225800
cttgctctct tccataagat gcaagcgggt taaaggtact ttcccttcta aggaaattac 1225860
aatctctagg gataattaaa ctctgacgtc tccaacagtc tgcttttttg atctgaagtt 1225920
gaaatgagtg agaatgcggt tggtcatact actctcatca aggcctatag attttgtgag 1225980
ggcttcctta ctcccatggg ataggaatgt atcggggatt gcaaaattta agatatcgac 1226040
cttaaaattg aacgtagcta caaaattatt aaactcggac gctaatcctc ctcgaatgga 1226100
gtgctcttct atggtaatca ccttagagtg actcatcagt aaaagactga aaagatcgtt 1226160
atcaaaaggt tttataaaga tcgggtctac aacagttgcg gagatgccat aagcaagcaa 1226220
ctgatgtttt atagataggg ccgtgaagca gagggttcca agagctatga tgagaacgtc 1226280
ctcaccttgt gagagggtct cagcattgcc tggggatctt aggaaatttg gatctccagt 1226340
gagtgggtct ccatgaggag ctgggatatt ggggtagcgg atagcagaag gagaggacca 1226400
gtgtagagaa gaatacagta gctgttggaa taccacctgg ctacgtggct gacagataat 1226460
catctggggc atcgcacgta ggaaactcat atcatagatg ccgtgatgac tacgtccatc 1226520
accataggca agtcctgcac gatctatagc aaaaatcacg ggaagatctt gcatgcaaac 1226580
atcgtggaaa acattatcga gagcacggtg taaaaatgta gaatatatag aacagatcac 1226640
aggattgccg gcttttgcaa tgcctgcact gaaagtcact gcatggcctt cagcaatccc 1226700
tacatcaaag aagcgttctg ggaacttctg tttgaaacct tccaaacgag atcctataga 1226760
cattgcagga gtcaccacat ggagacgtga ggaaacctct ccaagttcac atagcgtttg 1226820
gccaaatata tcagggaaag aaggcttagg cttaatcgca ggaagatgtt ttgcggattc 1226880
tcgcttattg aagtttgctc tgactccgtg atactttgca gggttatttt gggcttggtc 1226940
taggcccttc cccttagttg tacagacgtg gacaagaata ggaaaaggga ggttacgaac 1227000
ggactgaagg atggggatca gttttttaac attatgaccg tctatagggc cgacatacgc 1227060
taatccgaat tgttcaaata aaggagtggg acagaagaga tttttaacac attgtgaaag 1227120
tctccgactg tgctttgcta agctatcccc atagcgtgga attttagcga gccatttttc 1227180
cacttgctta gtgagtttat tggttgcagg gtggtgtagc catcgggaaa agattcgaga 1227240
catggctcct acgtttttag agatcgacat attgttgtca ttcaaaataa caacaaactt 1227300
cgataaatct gttgaaatat tgttcaacgc ttctaaggta agaccacaag agaatgcagc 1227360
atctccaagg atgggaatga cgtgtgtgcg tgattctaaa ggggttgttt gagccattcc 1227420
tagagctaaa gacaatgccg tccctgcatg tccagagaaa aataaatcgt ggtcactctc 1227480
cgtagggttg gtaaaaccac tgaggccgtt gtcattgcgt atatggtcaa atccttcatt 1227540
atttcttcct gtcagtagtt tatgaggata ggtctgatgt cctacatcaa aaataaattt 1227600
atcttttggg gaagagaaca cgtaatgtaa ggctatagta agctctacga ttccaagatt 1227660
tgaagataag tggcctcctg tttgtgataa tacagagatg atgcgataac ggatttcttc 1227720
agctaaacca ggaagctgag aaatagagag tttctttaaa tctgcaggag acaatatcag 1227780
gtctaaaagg gggcaagaag acgaagtcat gaccttagtg atgtggatgt ataggaaacg 1227840
tttgtactat agggtctcca gaaggacttt tgaggagttc tcctttggag ttaacagaaa 1227900
gaagataaca tagttgatct tttcttaatg ctttatttaa tccttcatag atcaaagaaa 1227960
acatggcctc atatgtagct acaatatcac tcaaagagtt taaggtttct aaagattgtt 1228020
gtaaggcgaa taactttttt aatgaggggt ctttgtgatg gatttctacc ataagactac 1228080

cgaactacag cttgttcctc aaagagagaa ctttcatgac gcttttctgc caactcacgg 1228140
actctttgct ctacttgtcg aatacgagat tcacaaatac gcattaaggc atcagcttct 1228200
tcatataggg ctagagatgc atccaaagaa gtcgtgggtt gattcataag gtcaacaatt 1228260
tcttctagtc gttgcattgc attttcgaag gggacctctt ccatgattta gccttttata 1228320
agtttacaaa tttcaatatt tgtgacagtt aaaatggcct cgccatcttg caactgaatc 1228380
cttacacgag cattttcttg taagctgtct acggaaatca tagcggaatt ttcattaaag 1228440
tcaaagagca ttgcataccc gcgttttaaa acatttttcg gatttaaaga gtgaagttgt 1228500
tcttttagcg cagagtagcg ggcaacagaa ttttcgtaac gacgttctag aattgttgct 1228560
aaagtttcct tgacatttgc ataggcgatt ttttggtttt gtagatttag ctcgcatcct 1228620
ctcgatagac gtctgcctaa tagttccagt ttctgcacat ggctacggag ctgttgctct 1228680
aagacattgt gagcatgctt gagtcgtgtg tgtttatgaa aatattcttc tttttggtag 1228740
tggaggcgtc gtgaaattag ggtgtctagc tgttgacgcc aaggggataa tttctgtgat 1228800
gcctgttgga tttggcgagg ataggtaagg cttttcttta gttggtggca acgtacttgg 1228860
agagaaagtg ctttatggga tagggcttgt gaaagcattt ttttcaaact ttgcagtcgg 1228920
caggtcatgc gggaaacaag atctccttgg agccaccgcg agatgttgtc atagcgttgt 1228980
ttgctttcgt gaattttccc ttggacacct ttttgaatag cgatctcaat agagtcgagt 1229040
tgctgttgtg ctgtagtata gaattctgcg cgatctaaaa agcgtctcca gggaagcaat 1229100
tgctgttttt ttgatgtgag gagttggcga gagtgtgaga gtagatggcg gaggtatccc 1229160
tcgaacacct gaacttgctc ttcgctactt ttacagacga tctctgcggc tgcagaagga 1229220
gtgggagcac gcacatcaga agcaaagtca cataaagtgt aatcagtttc atggccgaca 1229280
gcagagacaa tagggattgt gcttgcatga atggctttaa ctaagatttc ttcgttaaag 1229340
gcccagagat cttcaatact tcctcctccc cgagcaataa taagaacgtc agcgaggttc 1229400
tcagcgttca tcacttcaat agccttggag atttcatgag ctgcagagtt cccttggaca 1229460
gtgacgggat aaactaaaat tttatagttg cgagcacgcc gggagagtac acgtaagata 1229520
tcttggatca cagctcctgt tgggctagta atgacgccaa tgcattgagg agcaaagggg 1229580
aggggcttct ttttttcagt tgcgaaatac ccttcagcag tcagacgtct ttttgtttct 1229640
tcaaattttt gtaggagatc gccctctcca gcgtaaacca aagcatgggc tacaatttgg 1229700
tactgtcctc taggagcata gaccgcaagc ttcccatgaa taataactgc atccccatct 1229760
ttgggtttgc ggtcatagta cttactttta aaatgaaaaa aggcaccatt aagaaacgct 1229820
tggctatcct taatcccaaa atagagatga ccacttggtt gtagggagac gttgctaagc 1229880
tcgcccttca ctatgatctg acaaaaattg gactcaagaa gagtcttaat gcgttcagtt 1229940
agggatgcaa cagcctgtgg aggcgatgac ataacaggaa agcctctcta gagaattcag 1230000
tgactttaga gtaagtgatt ttctg 1230025

## Claims

1. - An isolated polynucleotide having a nucleotide sequence of a *Chlamydia pneumoniae* genome, comprising:
(a) the nucleotide sequence of SEQ ID No. 1;
(b) the nucleotide sequence contained within the *Chlamydia pneumoniae* genomic DNA in ATCC Deposit No. 1360-VR;
(c) a nucleotide sequence which hybridizes with the totality of the nucleotide sequence of (a) or (b) under conditions of high stringency wherein the hybridization is carried out at a temperature of 65°C in the presence of SSC buffer, 1 x SSC corresponding to 0.15 M NaCl and 0.05 M Na citrate, the washing steps are the following:
2 x SSC, 0.1 % SDS at room temperature followed by three washes with 1 x SSC, 0.1 % SDS; 0.5 x SSC, 0.1 % SDS; 0.1 x SSC, 0.1% SDS at 68°C for 15 minutes;
(d) a nucleotide sequence exhibiting at least 99.9 % identity with the sequence of SEQ ID No. 1; or
(e) a nucleotide sequence exhibiting at least 80 % identity to SEQ ID No. 1.

2. - An isolated polynucleotide of claim 1 comprising the nucleotide sequences ORF2 to ORF 1297.

3. - A method for the detection and/or identification of *Chlamydia pneumoniae* in a biological sample, comprising:
(i) contacting the sample with a polynucleotide of Claim 1 or 2, under conditions which permit hybridization of complementary base pairs; and
(ii) detecting the presence of hybridization complexes in the sample in which the detection of hybridization complexes indicates the presence of *Chlamydia pneumoniae* in the sample.

4. - A DNA chip containing an array of polynucleotides comprising at least one of the polynucleotides of Claim 1 or 2.

5. - A screening assay, comprising:
(a) contacting a test compound with an isolated polynucleotide of Claim 1 or 2; and
(b) detecting whether binding occurs.

6. - A kit comprising a container containing an isolated polynucleotide of Claim 1 or 2.

## Patentansprüche

1. Isoliertes Polynukleotid mit einer Nukleotidsequenz des Genoms von *Chlamydia pneumoniae,* umfassend:
(a) die Nukleotidsequenz von SEQ ID No. 1;
(b) die in der genomischen DNA von *Chlamydia pneumoniae* in der ATCC-Hinterlegung No. 1360-VR enthaltene Nukleotidsequenz;
(c) eine Nukleotidsequenz, die mit der Gesamtheit der Nukleotidsequenz von (a) oder (b) unter Bedingungen hoher Stringenz hybridisiert, wobei die Hybridisierung bei einer Temperatur von 65°C in Anwesenheit von SSC-Puffer ausgeführt wird, wobei 1 x SSC 0,15 M NaCl und 0,05 M Na-Citrat entspricht, die Spülschritte die folgenden sind: 2 x SSC, 0,1 % SDS bei Raumtemperatur, gefolgt von drei Spülungen mit 1 x SSC, 0,1 % SDS; 0,5 x SSC, 0,1 % SDS; 0,1 x SSC, 0,1 % SDS 15 Minuten lang bei 68°C;
(d) eine Nukteotidsequenz, die mindestens 99,9 % Identät mit der Sequenz von SEQ ID No. 1 aufweist; oder
(e) eine Nukleotidsequenz, die mindestens 80 % Identität mit SEQ ID No. 1 aufweist.

2. Isoliertes Polynukleotid nach Anspruch 1, umfassend die Nukleotidsequenzen ORF 2 bis ORF 1297.

3. Verfahren zum Nachweis und/oder zur Identifizierung von *Chlamydia pneumoniae* in einer biologischen Probe, umfassend:
(i) In-Kontakt-Bringen der Probe mit einem Polynukleotid nach Anspruch 1 oder 2 unter Bedingungen, wetche die Hybridisierung komplementärer Basenpaare gestatten; und
(ii) Nachweisen der Anwesenheit von Hybridisierungskomplexen in der Probe, wobei der Nachweis von Hybridisierungskomplexen die Anwesenheit von *Chlamydia pneumoniae* in der Probe anzeigt.

4. DNA-Chip, der ein Array von Polynukteotiden enthält, umfassend mindestens eins der Polynukleotide nach Anspruch 1 oder 2.

5. Screening-Assay, umfassend
(i) In-Kontakt-Bringen einer Testverbindung mit einem isolierten Polynukleotid nach Anspruch 1 oder2, und
(ii) Nachweisen, ob Bindung stattfindet.

6. Kit, umfassend einen Behälter der ein isoliertes Polynukleotid nach Anspruch 1 oder 2 enthält.

## Revendications

1. Polynucléotide isolé ayant une séquence nucléotidique d'un génome de *Chlamydia pneumoniae,* comprenant :
(a) la séquence nucléotidique de SEQ ID No. 1 ;
(b) la séquence nucléotidique contenue dans l'ADN génomique de *Chlamydia pneumoniae* du dépôt ATCC N° 1360-VR ;
(c) une séquence nucléotidique qui hybride avec la totalité de la séquence nucléotidique de (a) ou (b) dans des conditions de forte stringence dans laquelle l'hybridation est réalilsée à une température de 60°C en présence de tampon SSC, 1 x SSC correspondant à NaCl 0,15 M et citrate de Na 0,05 M, les étapes de lavage sont les suivantes :
2 x SSC, 0,1 % SDS à température ambiante suivie par trois lavages avec 1 x SSC, 0,1 % SDS, 0,5 % x SSC, 0,1 % SDS, 0,1 % x SSC, 0,1 % SDS à 68°C pendant 15 minutes ;
(d) une séquence nucléotidique présentant au moins 99,9 % d'identité avec la séquence de SEQ ID No. 1 ; ou
(e) une séquence nucléotidique présentant au moins 80 % d'identité avec SEQ ID No. 1.

2. Polynucléotide isolé selon la revendication 1, comprenant les séquences nucléotidiques ORF2 à ORF1297.

3. Méthode de détection et/ou d'identification de *Chlamydia pneumoniae* dans un échantillon biologique, comprenant :
(i) la mise en contact de l'échantillon avec un polynucléotide selon la revendication 1 ou 2, dans des conditions permettant l'hybridation de paires de bases complémentaires ; et
(ii) la détection de la présence de complexes d'hybridation dans l'échantillon, la détection de complexes d'hybridation indiquant la présence de Chlamydia pneumoniae dans l'échantillon.

4. Puce à ADN contenant un jeu de polynucléotides comprenant au moins un des polynucléotides selon la revendication 1 ou 2.

5. Méthode de sélection, comprenant :
(a) la mise en contact d'un composé à tester avec un polynucléotide isolé selon la revendication 1 ou 2 ; et
(b) la détection d'une liaison potentielle.

6. Kit comprenant un récipient contenant un polynucléotide isolé selon la revendication 1 ou 2.
